# EUROPEAN PATENT SPECIFICATION

(11) **EP 3 442 947 B1**
(45) Date of publication and mention of the grant of the patent: **07.06.2023**
(21) Application number: 17720921.0
(22) Date of filing: 17.04.2017
(51) Int. Cl.: C07D 231/38, C07D 401/12, C07D 239/48, A61K 31/505, A61K 31/506, A61K 31/44, A61P 7/06, A61P 35/02

(54) **AMINE-SUBSTITUTED ARYL OR HETEROARYL COMPOUNDS AS EHMT1 AND EHMT2 INHIBITORS**
AMINE-SUBSTITUIERTE ARYL- ODER HETEROARYL-VERBINDUNGEN ALS EHMT1 AND EHMT2 INHIBITOREN
DÉRIVÉS D'ARYL OU HETEROARYL SUBSTITUÉS PAR UNE AMINE COMME INHIBITEURS DE EHMT1 ET EHMT2

(30) Priority: 15.04.2016 US 201662323602 P; 10.06.2016 US 201662348837 P; 30.09.2016 US 201662402997 P
(43) Date of publication of application: 20.02.2019
(73) Proprietor: Epizyme, Inc., Cambridge, MA 02139 (US)
(72) Inventor: CAMPBELL, John Emmerson, Cambridge Massachusetts 02138 (US); DUNCAN, Kenneth William, Norwood Massachusetts 02062 (US); FOLEY, Megan Alene, Somerville Massachusetts 02144 (US); HARVEY, Darren Martin, Acton Massachusetts 01720 (US); KUNTZ, Kevin Wayne, Woburn Massachusetts 01801 (US); MILLS, James Edward John, Worth Deal Kent CT14 0DF (GB); MUNCHHOF, Michael John, Corvallis MT 59828 (US)
(74) Representative: Russell, Tim
(86) International application number: PCT/US2017/027918
(87) International publication number: WO 2017/181177

(56) References cited:
- WO-A1-2007/053452
- WO-A1-2010/129802
- WO-A1-2012/044936
- WO-A1-2013/140148
- WO-A1-2015/192981
- WO-A2-03/032994
- WO-A2-2013/070852
- WO-A2-2014/058921
- US-A1- 2007 021 446
- US-A1- 2011 230 478
- US-A1- 2014 128 391
- KANCHAN DEVKOTA ET AL: "Analogues of the Natural Product Sinefungin as Inhibitors of EHMT1 and EHMT2", ACS MEDICINAL CHEMISTRY LETTERS, vol. 5, no. 4, 10 April 2014 (2014-04-10), pages 293-297, XP55271475, United States ISSN: 1948-5875, DOI: 10.1021/ml4002503
- YUNLONG HE ET AL: "Targeting protein lysine methylation and demethylation in cancers", ACTA BIOCHIMICA ET BIOPHYSICA SINICA, vol. 44, no. 1, 22 December 2011 (2011-12-22), pages 70-79, XP55260982, US ISSN: 1672-9145, DOI: 10.1093/abbs/gmr109
- JING CUI ET AL: "EHMT2 inhibitor BIX-01294 induces apoptosis through PMAIP1-USP9X-MCL1 axis in human bladder cancer cells", CANCER CELL INTERNATIONAL, BIOMED CENTRAL, LONDON, GB, vol. 15, no. 1, 4 February 2015 (2015-02-04), page 4, XP021211599, ISSN: 1475-2867, DOI: 10.1186/S12935-014-0149-X
- Aline Renneville ET AL: "EHMT1 and EHMT2 inhibition induces fetal hemoglobin expression", Blood, 28 August 2015 (2015-08-28), XP55376947, DOI: 10.1182/blood-2015-06- Retrieved from the Internet: URL:www.ncbi.nlm.nih.gov/pubmed/26320100 [retrieved on 2017-05-30] cited in the application
- JOHN L. BUCHANAN ET AL: "Discovery of 2,4-bis-arylamino-1,3-pyrimidines as insulin-like growth factor-1 receptor (IGF-1R) inhibitors", BIOORGANIC & MEDICINAL CHEMISTRY LETTERS, vol. 21, no. 8, 1 April 2011 (2011-04-01), pages 2394-2399, XP55376699, AMSTERDAM, NL ISSN: 0960-894X, DOI: 10.1016/j.bmcl.2011.02.075
- SAVICKIENE JURATE ET AL: "Euchromatic histone methyltransferase 2 inhibitor, BIX-01294, sensitizes human promyelocytic leukemia HL-60 and NB4 cells to growth inhibition and differentiation", LEUKEMIA RESEARCH, vol. 38, no. 7, 2014, pages 822-829, XP028850194, ISSN: 0145-2126, DOI: 10.1016/J.LEUKRES.2014.04.003
- DAS ET AL: "Dithiocarbamate and CuO promoted one-pot synthesis of 2-(N-substituted)-aminobenzimidazoles and related heterocycles", TETRAHEDRON LETTERS, ELSEVIER LTD, AMSTERDAM, NL, vol. 49, no. 6, 8 December 2007 (2007-12-08), pages 992-995, XP022417901, ISSN: 0040-4039, DOI: 10.1016/J.TETLET.2007.12.022

## Description

### RELATED APPLICATIONS

### BACKGROUND

Methylation of protein lysine residues is an important signaling mechanism in eukaryotic cells, and the methylation state of histone lysines encodes signals that are recognized by a multitude of proteins and protein complexes in the context of epigenetic gene regulation.

Histone methylation is catalyzed by histone methyltransferases (HMTs), and HMTs have been implicated in various human diseases. HMTs can play a role in either activating or repressing gene expression, and certain HMTs (*e*.*g*., euchromatic histone-lysine N-methyltransferase 2 or EHMT2, also called G9a) may methylate many nonhistone proteins, such as tumor suppressor proteins (*see, e.g.,* Liu et al., Journal of Medicinal Chemistry 56:8931-8942, 2013 and Krivega et al., Blood 126(5):665-672, 2015).

Two related HMTs, EHMT1 and EHMT2, are overexpressed or play a role in diseases and disorders such as sickle cell anemia (*see, e.g.,* Renneville et al., Blood 126(16): 1930-1939, 2015) and proliferative disorders (*e*.*g*., cancers), and other blood disorders.

### SUMMARY

In one aspect, the invention provides a compound for use as defined in the claims.

In one aspect, the invention provides a compound of Formula (IIa1) for use in treating or preventing a blood disorder: 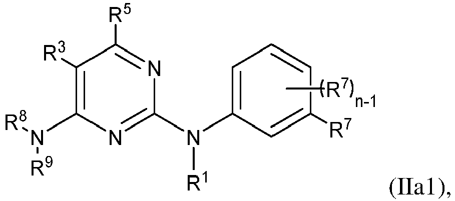 or a tautomer thereof, or a pharmaceutically acceptable salt of the compound or the tautomer, wherein
R¹ is H or C₁-C₄ alkyl;
R³ is selected from the group consisting of H, halo, cyano, C₁-C₆ alkoxyl, C₆-C₁₀ aryl, NR^{a}C(O)R^{b}, C₃-C₈ cycloalkyl, 4- to 7- membered heterocycloalkyl, 5- to 6-membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl, or R³ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, amino, mono- or di- alkylamino, or C₁-C₆ alkoxyl;
R⁵ is selected from the group consisting of F, Br, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 12-membered heterocycloalkyl containing 1-4 heteroatoms selected from N, O, and S, C₁-C₆ alkyl optionally substituted with one or more of halo, OR^{a} or NR^{a}R^{b}, and C₂-C₆ alkynyl optionally substituted with 4- to 12-membered heterocycloalkyl; wherein said C₃-C₈ cycloalkyl or 4- to 12-membered heterocycloalkyl are optionally substituted with one or more of halo, C(O)R^{a}, OR^{a}, NR^{a}R^{b}, 4-to 7-membered heterocycloalkyl, -C₁-C₆ alkylene-4- to 7-membered heterocycloalkyl, or C₁-C₄ alkyl optionally substituted with one or more of halo, OR^{a} or NR^{a}R^{b}, in which each of R^{a} and R^{b} independently is H or C₁-C₆ alkyl; or
R⁵ and R³ together with the atoms to which they are attached form phenyl or a 5- or 6-membered heteroaryl;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, cyano, OR¹⁰, OR¹¹, C(O)R¹¹, NR¹⁰R¹¹, C(O)NR¹⁰R¹¹, NR¹⁰C(O)R¹¹, 5- to 10-membered heteroaryl, C₃-C₈ cycloalkyl, or 4- to 12-membered heterocycloalkyl containing 1-4 heteroatoms selected from N, O, and S, and wherein the 5- to 10-membered heteroaryl, C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R⁷ is not H or C(O)OR^{g}; or optionally, one R⁷ and R⁵ together form a C₃-C₁₀ alkylene, C₂-C₁₀ heteroalkylene, C₄-C₁₀ alkenylene, C₂-C₁₀ heteroalkenylene, C₄-C₁₀ alkynylene or C₂-C₁₀ heteroalkynylene linker optionally substituted with one or more of halo, cyano, hydroxyl, or C₁-C₆ alkoxyl;
each R⁸ independently is H or C₁-C₆ alkyl;
each R⁹ is independently -Q³-T³, in which Q³ is a bond or C₁-C₆ alkylene, and T³ is H;
each R¹⁰ is selected from the group consisting of H and C₁-C₆ alkyl;
each R¹¹ is -Q⁶-T⁶, in which Q⁶ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T⁶ is H, halo, OR^{g}, NR^{g}R^{h}, NR^{g}C(O)R^{h}, C(O)NR^{g}R^{h}, C(O)R^{g}, S(O)₂R^{g}, or R^{S3}, in which each of R^{g} and R^{h} independently is H, phenyl, C₃-C₈ cycloalkyl, or C₁-C₆ alkyl optionally substituted with C₃-C₈ cycloalkyl, or R^{g} and R^{h} together with the nitrogen atom to which they are attached form a 4- to 12-membered heterocycloalkyl containing 1-4 heteroatoms selected from N, O, and S, and R^{S3} is C₃-C₈ cycloalkyl, C₆-C₁₀ aryl, 4- to 12-membered heterocycloalkyl containing 1-4 heteroatoms selected from N, O and S, or a 5- to 10-membered heteroaryl, and R^{S3} is optionally substituted with one or more -Q⁷-T⁷, wherein each Q⁷ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁷ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{j}, C(O)R^{j}, NR^{j}R^{k}, C(O)NR^{j}R^{k}, S(O)₂R^{j}, and NR^{j}C(O)R^{k}, each of R^{j} and R^{k} independently being H or C₁-C₆ alkyl optionally substituted with one or more halo; or -Q⁷-T⁷ is oxo; or
R¹⁰ and R¹¹ taken together with the nitrogen atom to which they are attached form a 4-to 12-membered heterocycloalkyl containing 1-4 heteroatoms selected from N, O, and S, which is optionally substituted with one or more of halo, C₁-C₆ alkyl, hydroxyl, or C₁-C₆ alkoxyl;
n is 0, 1, 2, 3, or 4.

In one embodiment, R³ is not H.

In one embodiment, n is 1 or 2.

In one embodiment, R¹ is H or CH₃.

In one embodiment, n is 1 or 2, and at least one of R⁷ is -Q²-OR¹¹ in which R¹¹ is - Q⁶-R^{S3} and Q⁶ is optionally substituted C₂-C₆ alkylene, C₂-C₆ alkenylene, or C₂-C₆ alkynylene linker.

In one embodiment, n is 1 or 2, and at least one of R⁷ is -Q²-NR¹⁰R¹¹ in which R¹¹ is -Q⁶-R^{S3}.

In one embodiment, Q⁶ is C₂-C₆ alkylene, C₂-C₆ alkenylene, or C₂-C₆ alkynylene linker optionally substituted with a hydroxyl and R^{S3} is 4- to 7-membered heterocycloalkyl optionally substituted with one or more -Q⁷-T⁷.

In one embodiment, Q⁶ is C₁-C₆ alkylene, C₂-C₆ alkenylene, or C₂-C₆ alkynylene linker optionally substituted with a hydroxyl and R^{S3} is C₃-C₆ cycloalkyl optionally substituted with one or more -Q⁷-T⁷.

In one embodiment, each Q⁷ is independently a bond or a C₁-C₃ alkylene, C₂-C₃ alkenylene, or C₂-C₃ alkynylene linker and each T⁷ is independently H, halo, C₁-C₆ alkyl, or phenyl.

In one embodiment, Q² is a bond or a C₁-C₄ alkylene, C₂-C₄ alkenylene, or C₂-C₄ alkynylene linker.

In one embodiment, at least one of R⁷ is

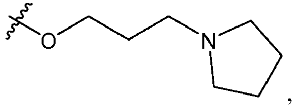

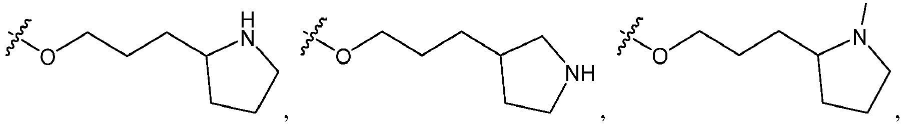

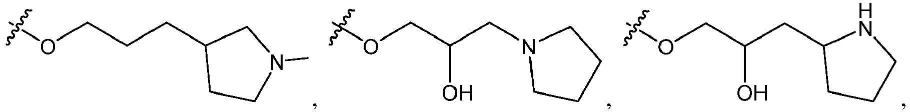

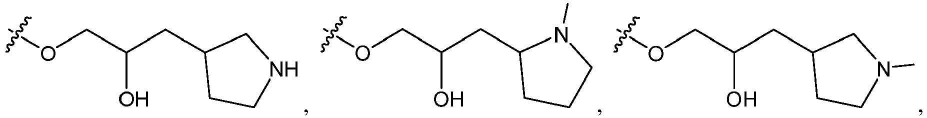

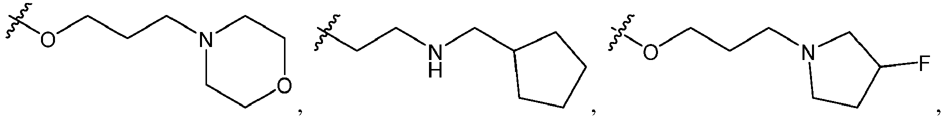

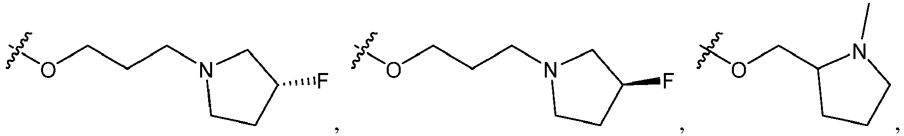

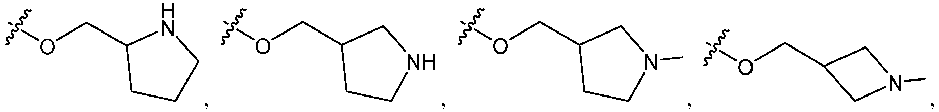

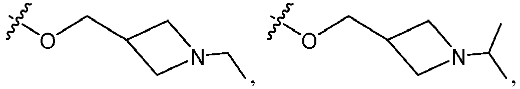

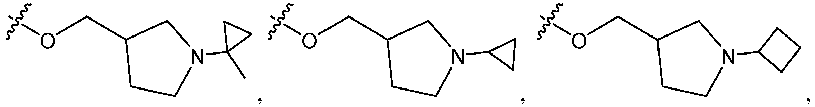

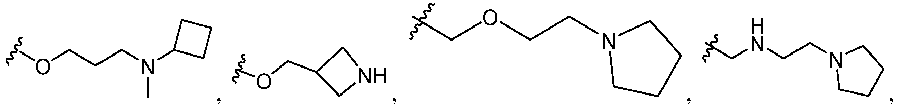

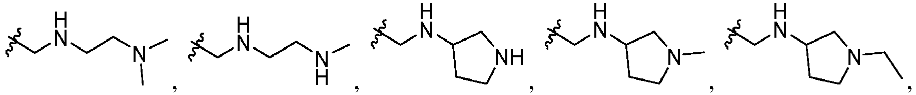

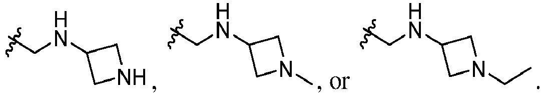

In one embodiment, n is 2 and the compound further comprises another R⁷ selected from halo and methoxy.

In one embodiment, the compound is of Formula (VI): 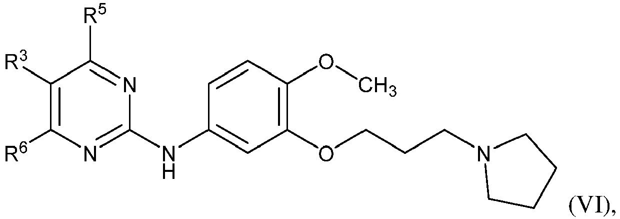 wherein R⁵ is selected from the group consisting of C₁-C₆ alkyl and NR⁸R⁹; and R⁶ is NR⁸R⁹.

In one embodiment, the compound is of Formula (VIIIa): 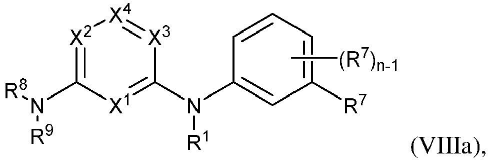 wherein
X¹ is N;
X² is CR³;
X³ is N;
X⁴ is CR⁵;
R³ is H; and
R⁵ is independently selected from the group consisting of C₃-C₈ cycloalkyl, and C₁-C₆ alkyl optionally substituted with one or more of halo or OR^{a}.

In one embodiment, R⁵ and R³ together with the atoms to which they are attached form phenyl or a 5- or 6-membered heteroaryl.

In one embodiment, the compound is of Formula (VIIIb): 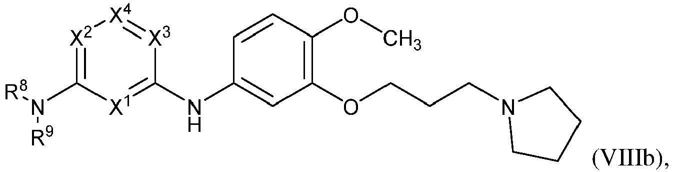 wherein
X¹ is N;
X² is CR³;
X³ is N;
X⁴ is CR⁵;
R⁵ is selected from the group consisting of C₃-C₈ cycloalkyl, and C₁-C₆ alkyl.

In one embodiment, R⁵ and R³ together with the atoms to which they are attached form phenyl or a 5- or 6-membered heteroaryl.

In one embodiment, the compound is of Formula (VIIIc): 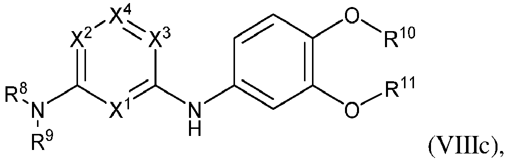 wherein
X¹ is N;
X² is CR³;
X³ is N;
X⁴ is CR⁵;
R⁵ is selected from the group consisting of C₃-C₈ cycloalkyl, and C₁-C₆ alkyl.

In one embodiment, R⁵ and R³ together with the atoms to which they are attached form phenyl or a 5- or 6-membered heteroaryl.

In one embodiment, R⁵ is C₁₋₆ alkyl or R⁵ and R³ together with the atoms to which they are attached form phenyl or a 5- to 6-membered heteroaryl ring.

In one embodiment, the compound is selected from those in the following table and pharmaceutically acceptable salts thereof:

| **Compound No.** | **Structure** |
|---|---|
| 186 | 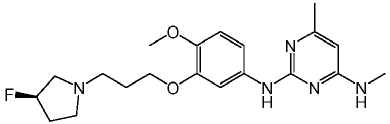 |
| 205 | 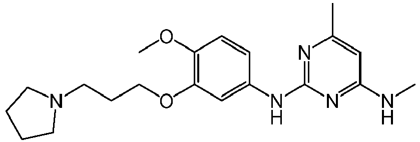 |
| 232 | 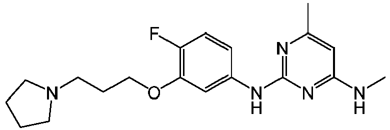 |
| 236 | 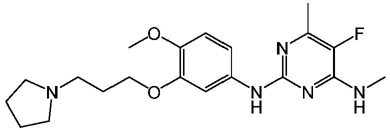 |
| 238 | 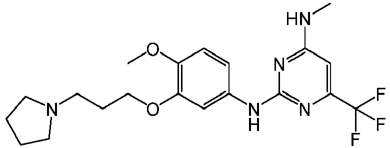 |
| 240 | 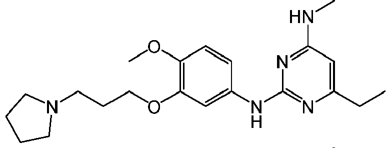 |
| 261 | 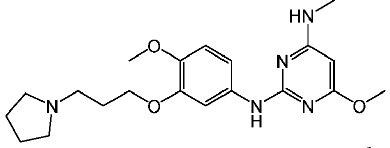 |
| 263 | 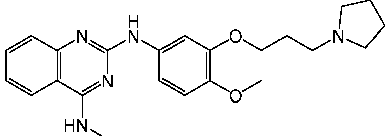 |
| 301 | 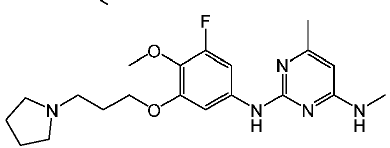 |
| 302 | 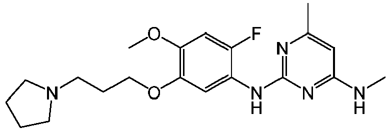 |
| 311 | 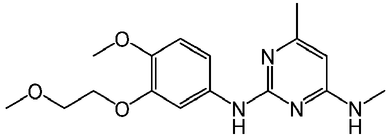 |
| 312 | 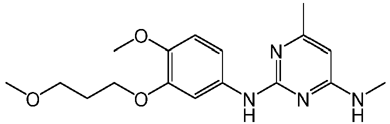 |
| 313 | 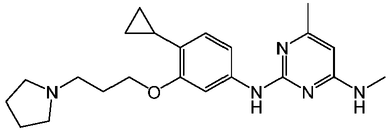 |
| 316 | 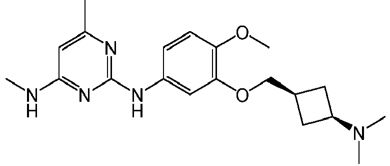 |
| 317 | 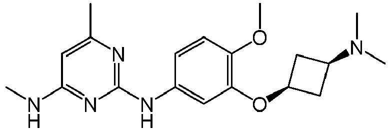 |
| 318 | 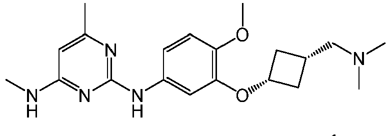 |
| 319 | 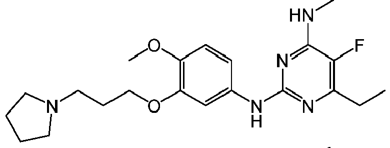 |
| 320 | 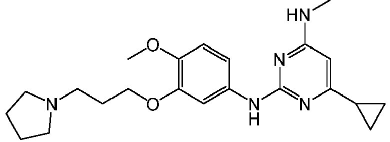 |
| 328 | 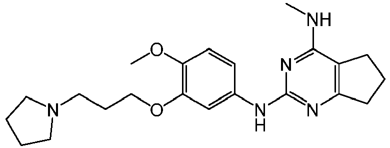 |
| 329 | 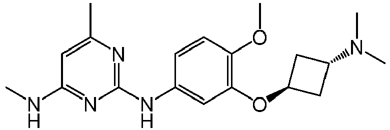 |
| 330 | 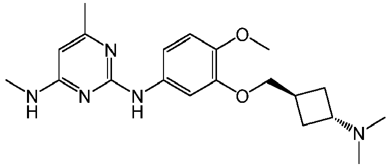 |
| 331 | 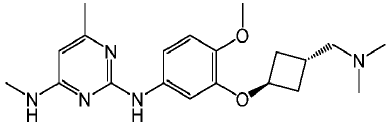 |
| 334 | 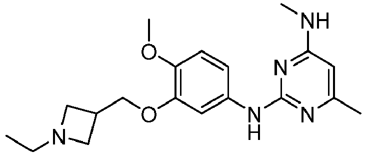 |
| 337 | 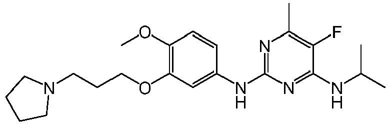 |
| 356 | 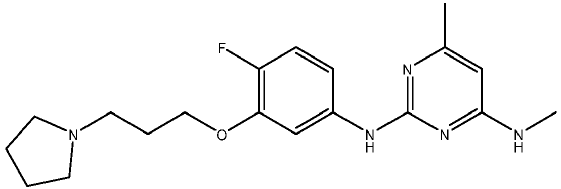 |
| 360 | 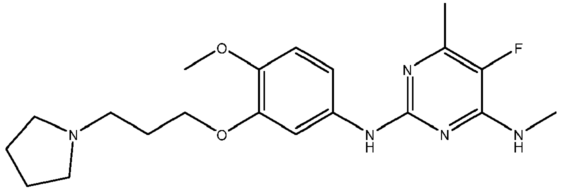 |
| 362 | 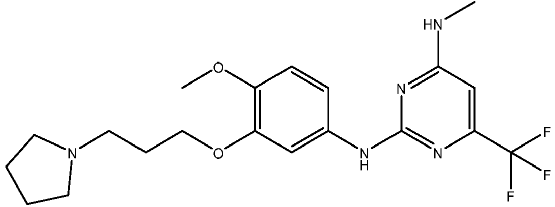 |
| 364 | 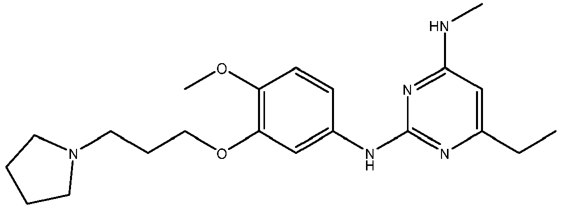 |
| 380 | 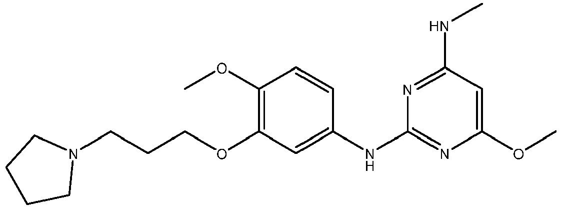 |
| 384 | 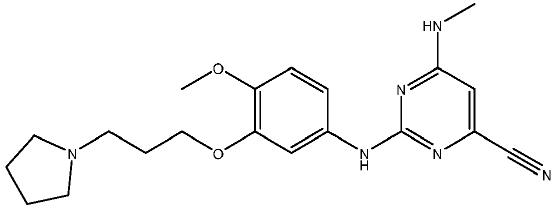 |
| 385 | 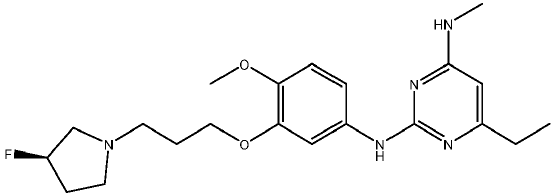 |
| 391 | 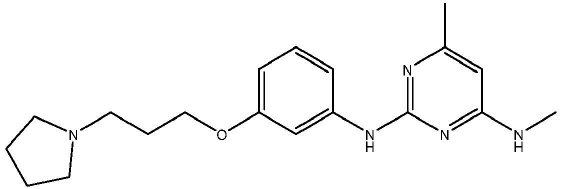 |
| 413 | 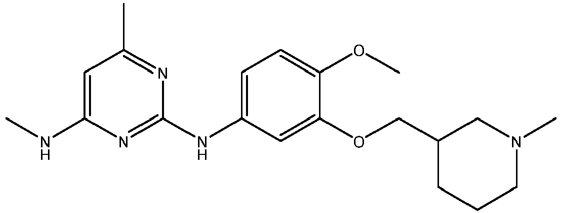 |
| 417 | 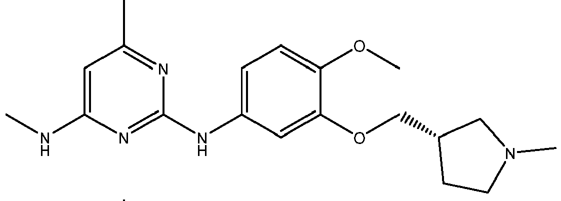 |
| 418 | 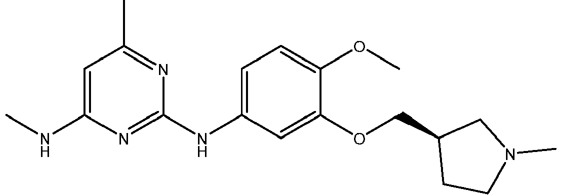 |
| 419 | 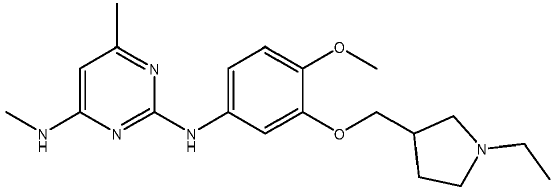 |
| 421 | 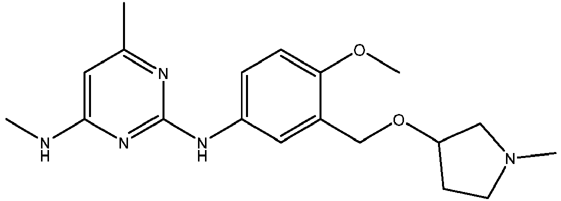 |
| 428 | 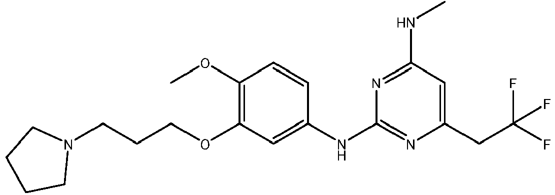 |
| 432 | 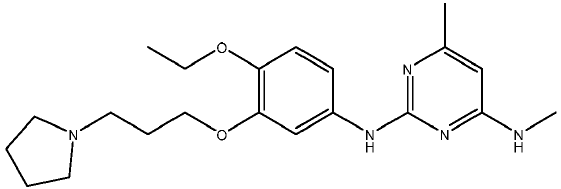 |
| 435 | 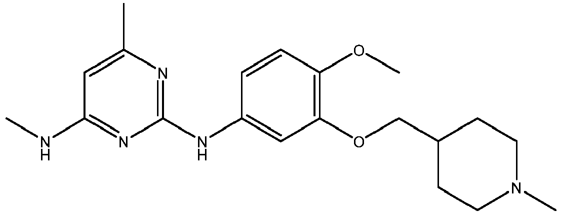 |
| 436 | 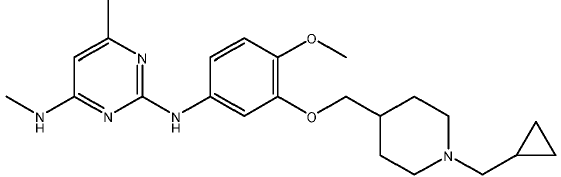 |
| 437 | 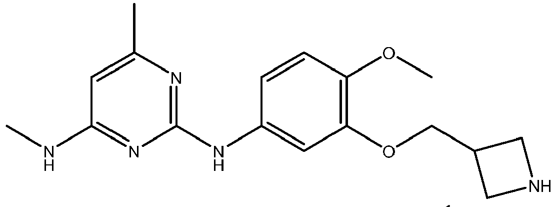 |
| 441 | 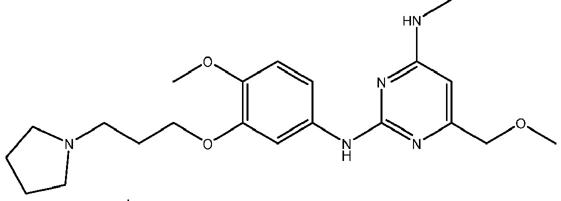 |
| 442 | 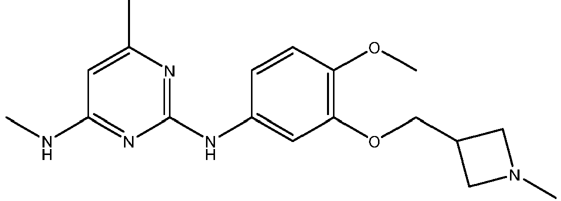 |
| 443 | 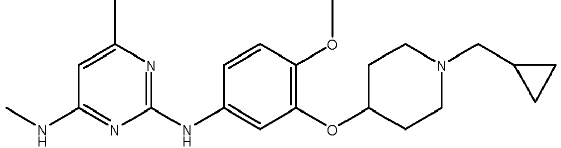 |
| 444 | 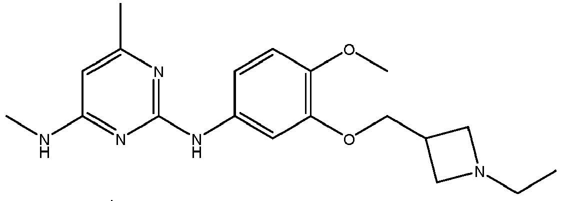 |
| 445 | 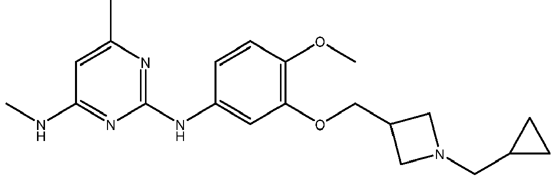 |
| 446 | 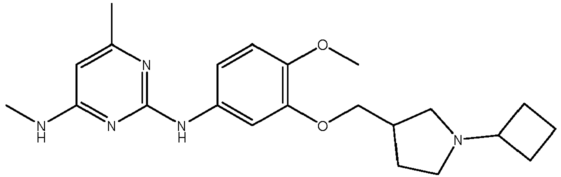 |
| 449 | 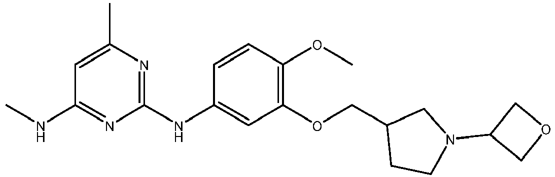 |
| 450 | 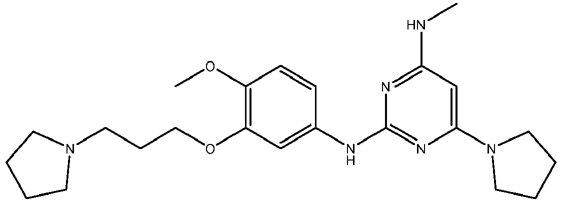 |
| 452 | 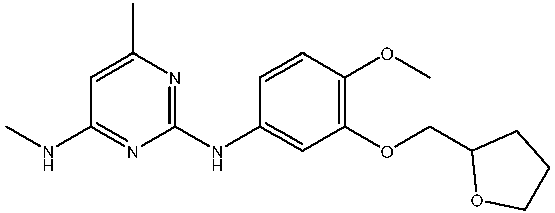 |
| 455 | 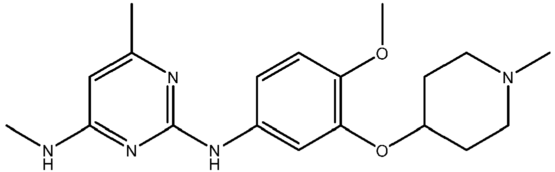 |
| 456 | 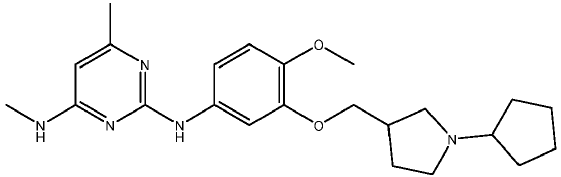 |
| 457 | 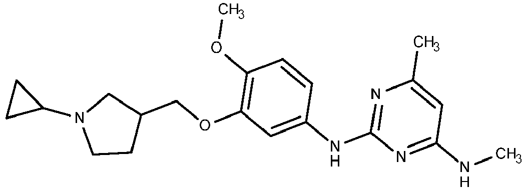 |
| 458 | 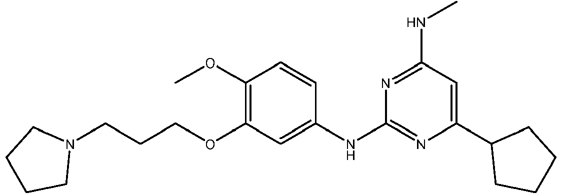 |
| 459 | 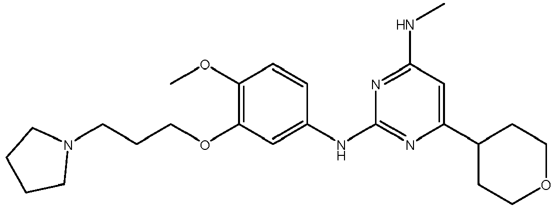 |
| 461 | 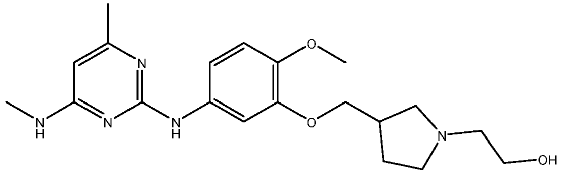 |
| 463 | 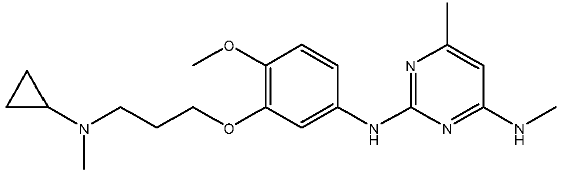 |
| 465 | 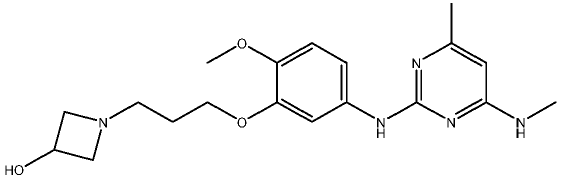 |
| 466 | 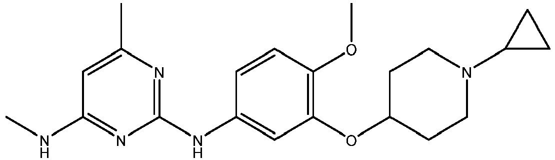 |
| 468 | 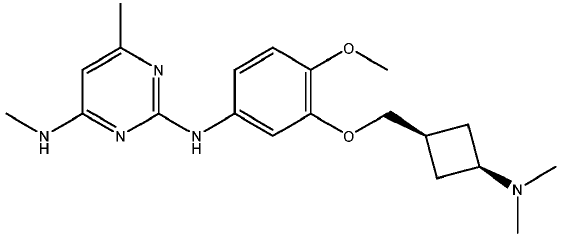 |
| 470 | 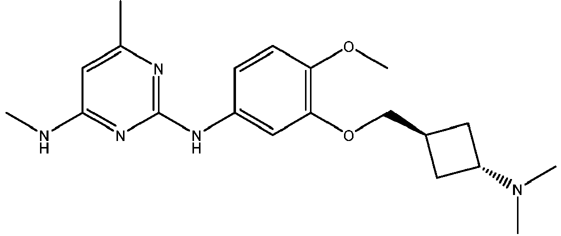 |
| 481 | 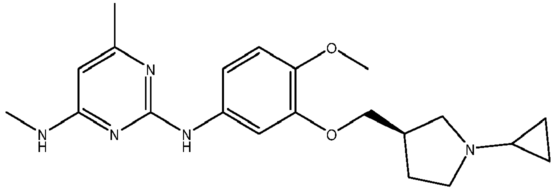 |
| 482 | 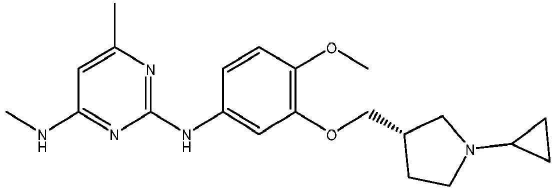 |
| 483 | 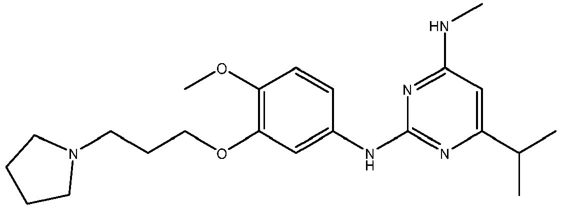 |
| 484 | 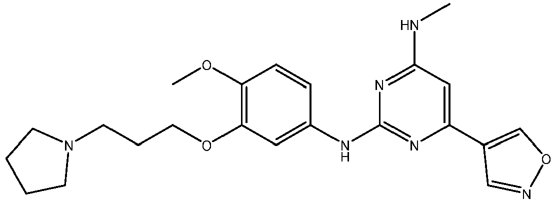 |
| 487 | 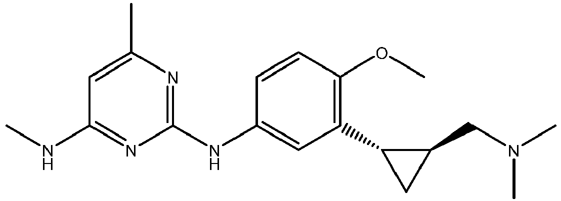 |
| 488 | 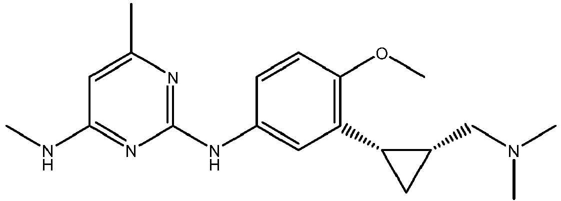 |
| 489 | 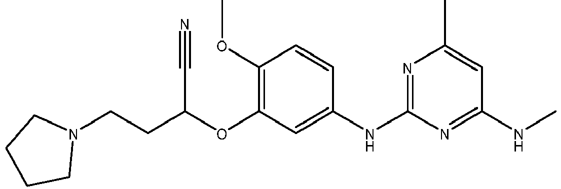 |
| 490 | 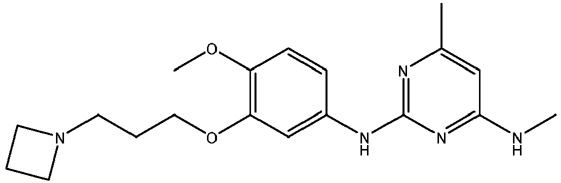 |
| 491 | 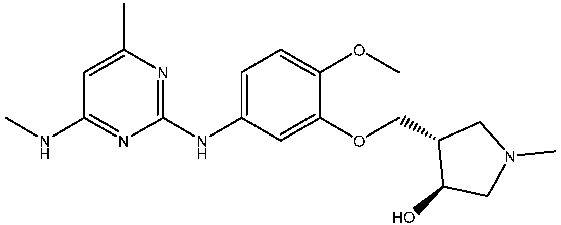 |
| 492 | 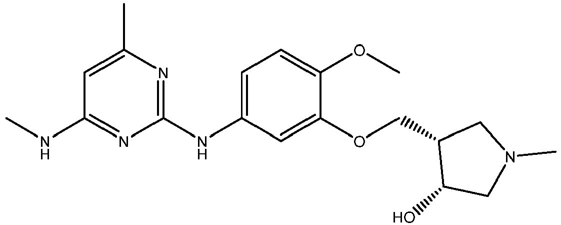 |
| 493 | 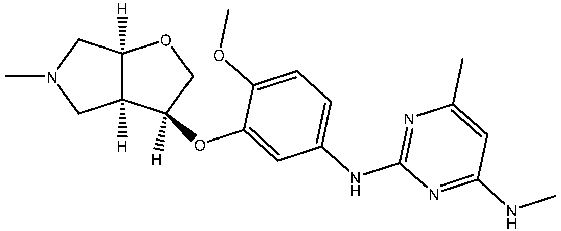 |
| 496 | 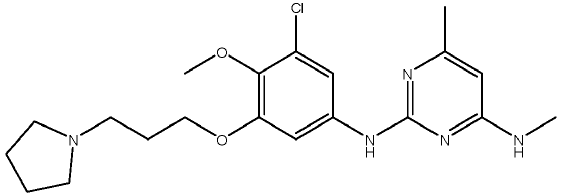 |
| 497 | 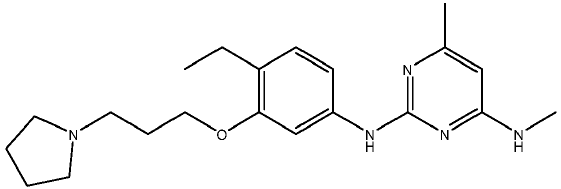 |
| 498 | 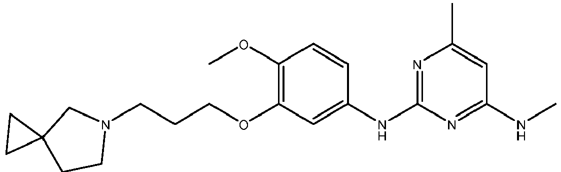 |
| 502 | 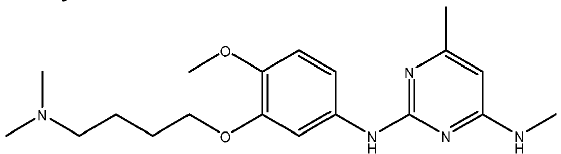 |
| 503 | 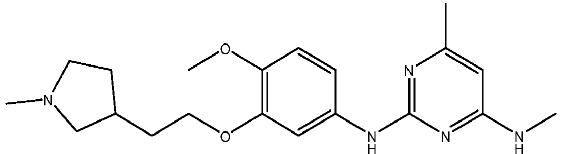 |
| 504 | 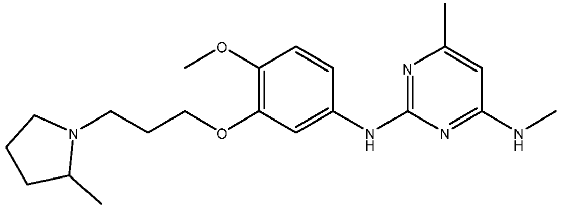 |
| 505 | 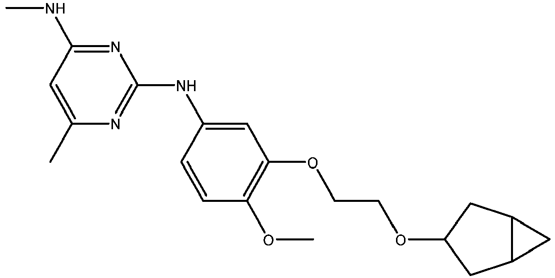 |
| 506 | 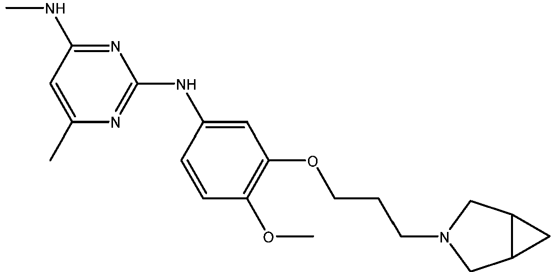 |
| 508 | 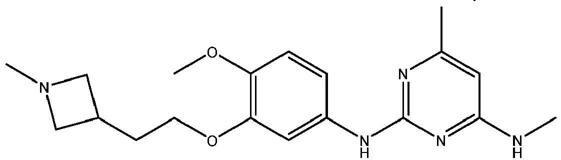 |
| 510 | 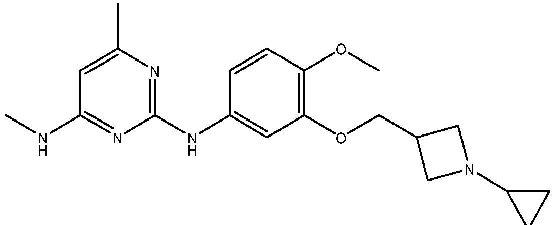 |
| 517a | 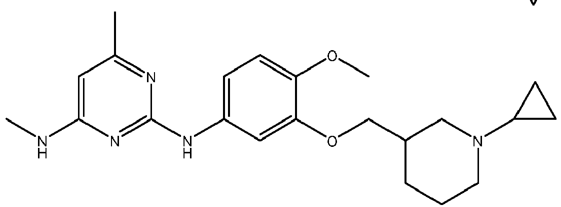 |
| 517b | 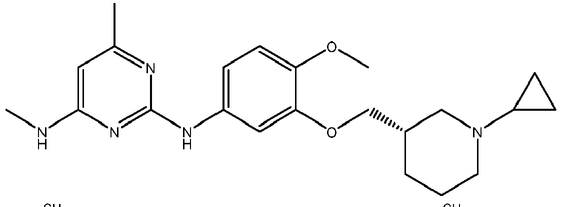 |
| 551 | 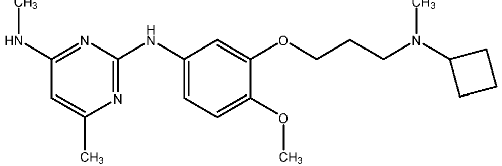 |
| 559 | 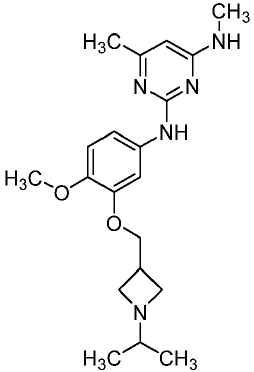 |
| 560 | 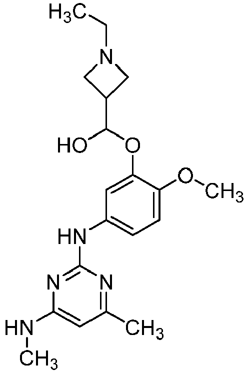 |
| 561 | 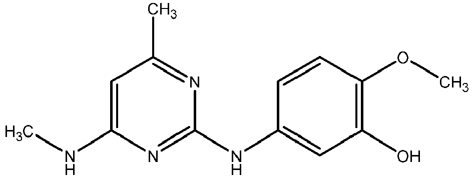 |
| 562 | 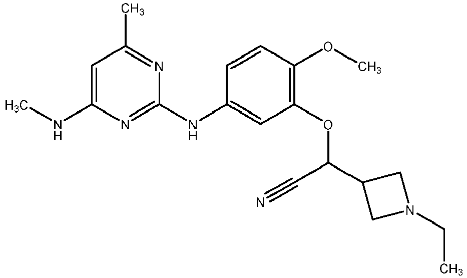 |
| 563 | 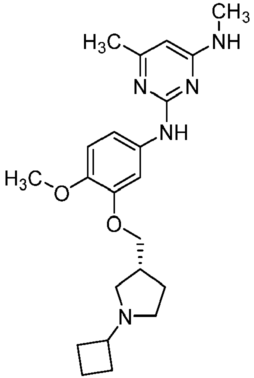 |
| 568 | 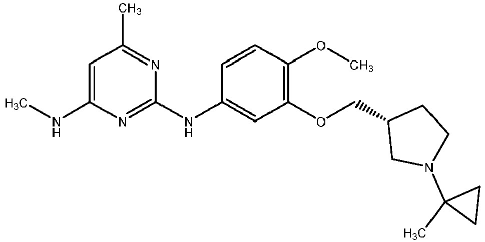 |
| 570 | 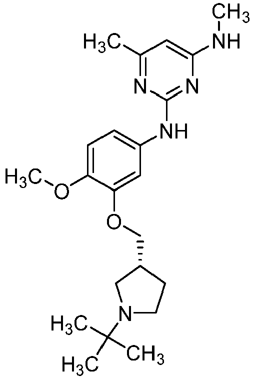 |
| 571 | 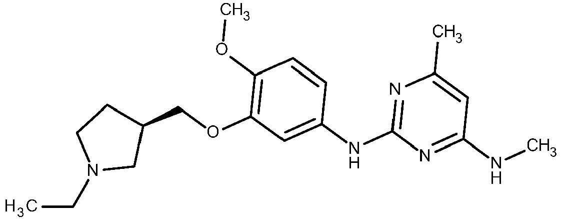 |
| 574 | 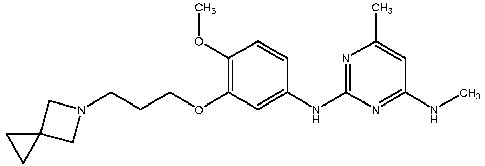 |
| 575 | 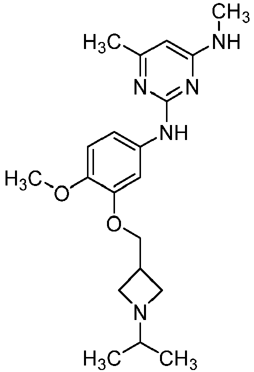 |
| 576 | 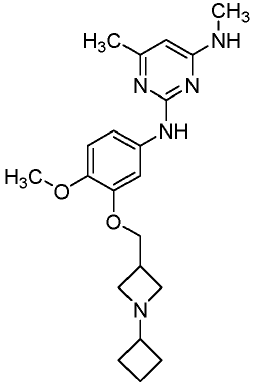 |
| 577 | 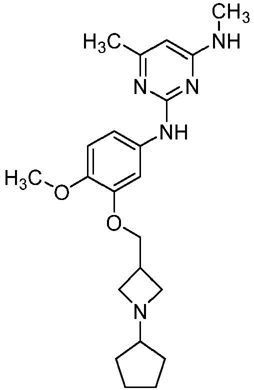 |
| 580 | 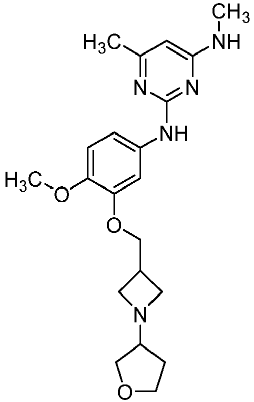 |
| 600 | 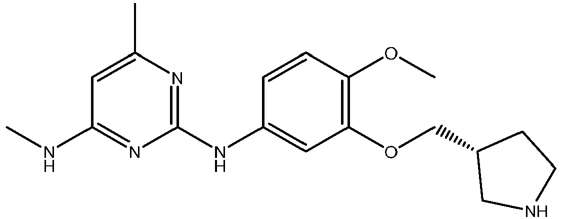 |
| 602 | 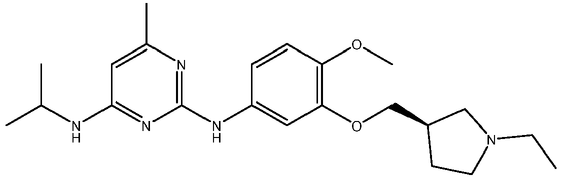 |
| 603 | 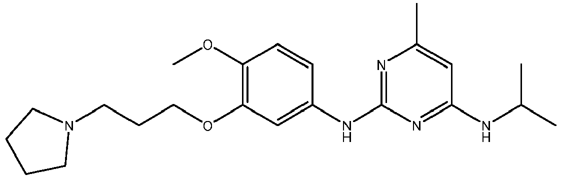 |
| 606 | 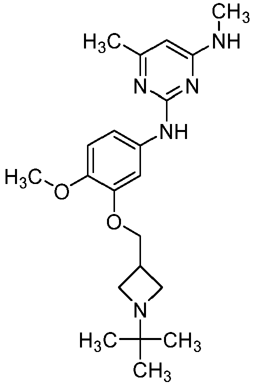 |
| 610 | 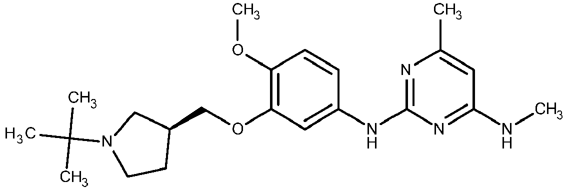 |
| 614 | 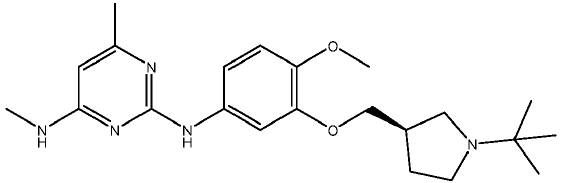 |
| 624 | 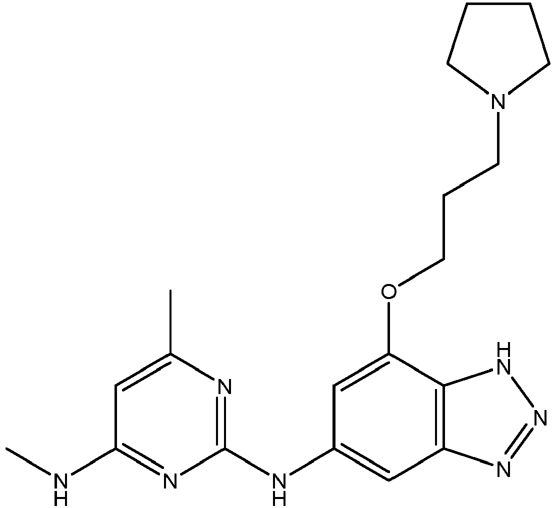 |
| 644 | 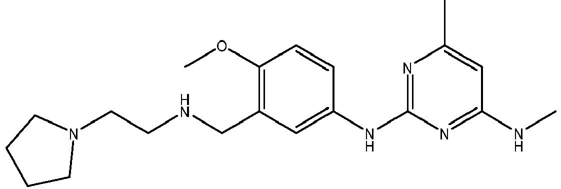 |
| 645 | 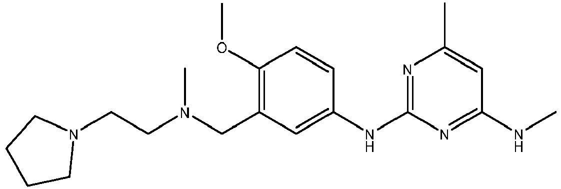 |
| 646 | 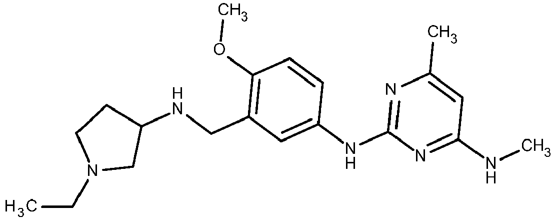 |
| 647 | 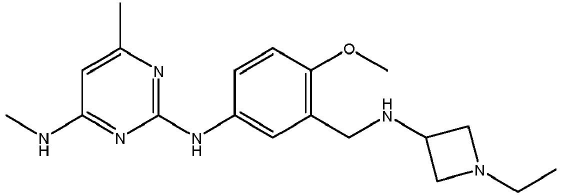 |
| 649 | 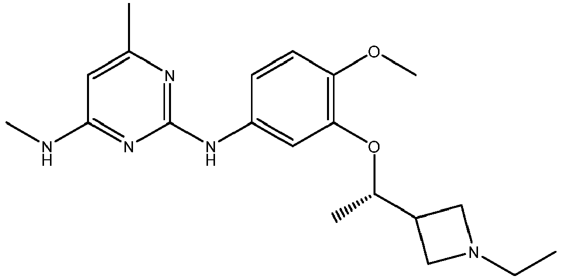 |
| 650 | 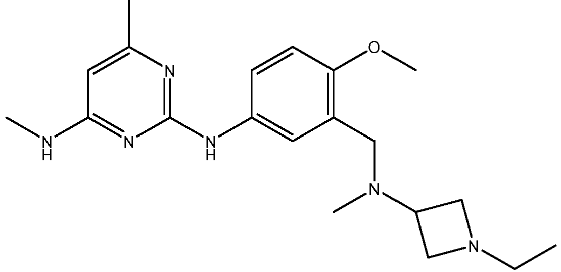 |
| 651 | 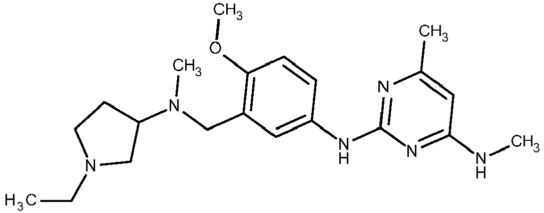 |
| 671 | 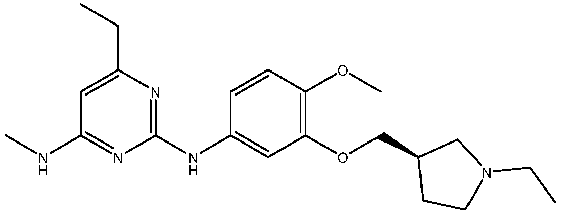 |
| 672 | 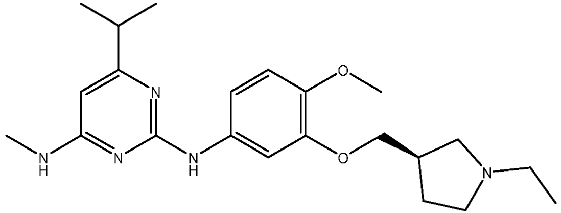 |
| 680 | 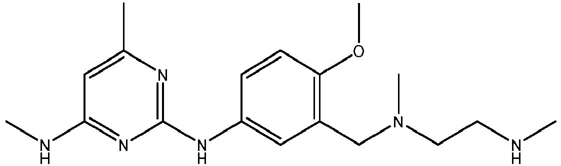 |
| 687 | 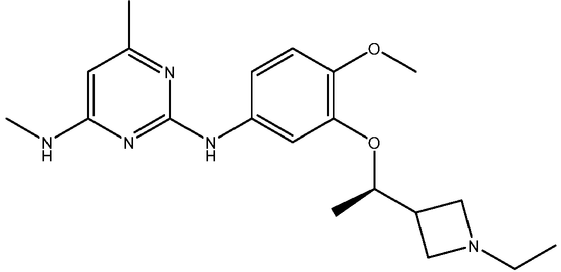 |
| 765 | 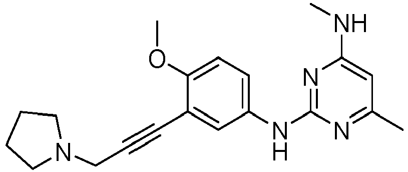 |
| 786 | 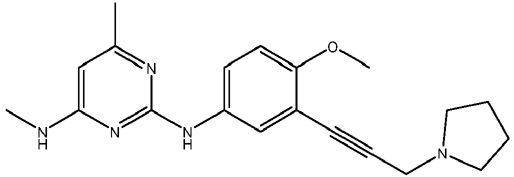 |
| 791 | 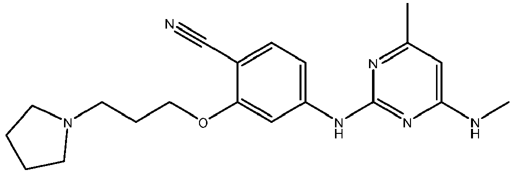 |
| 793 | 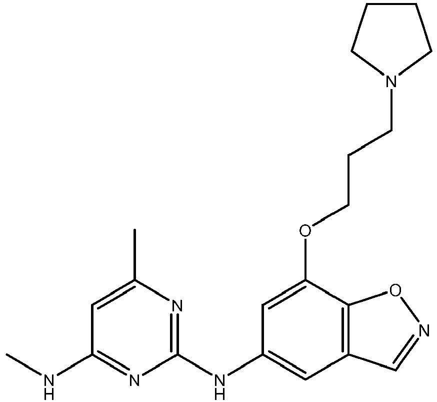 |
| 799 | 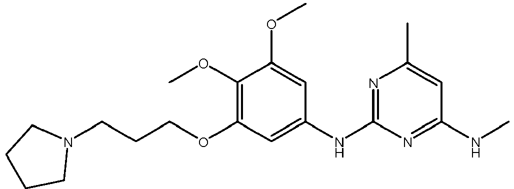 |
| 800 | 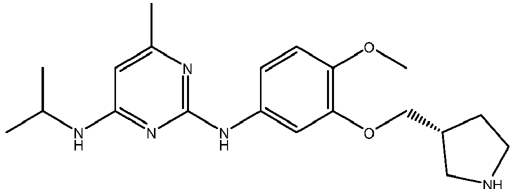 |
| 813 | 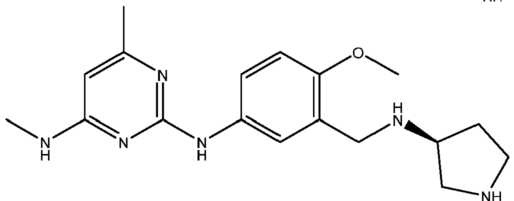 |
| 814 | 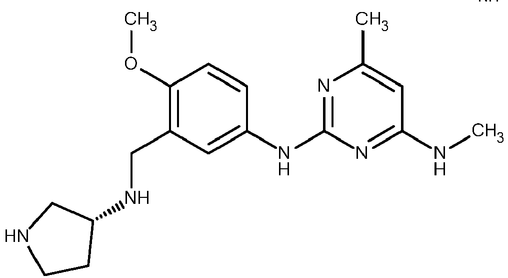 |
| 845 | 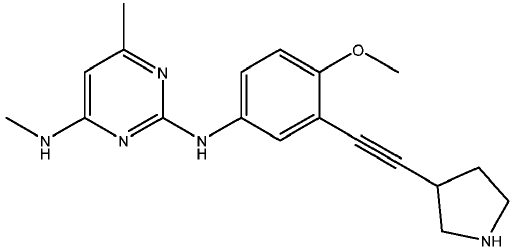 |
| 846 | 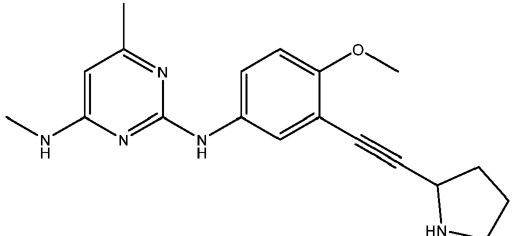 |
| 847 | 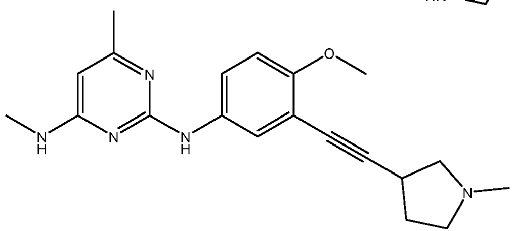 |
| 848 | 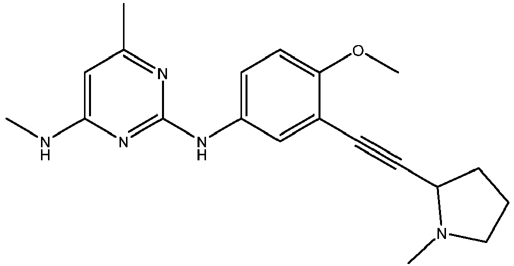 |
| 855 | 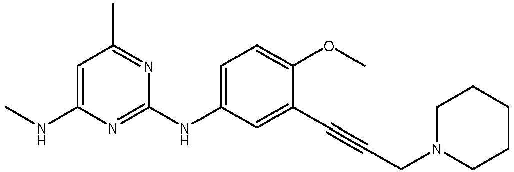 |
| 860 | 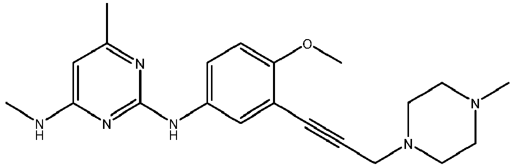 |
| 871 | 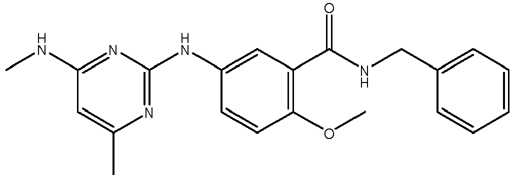 |
| 872 | 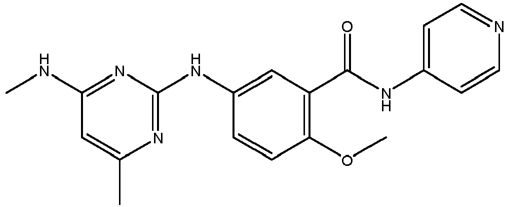 |
| 873 | 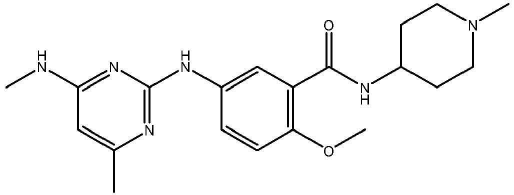 |
| 874 | 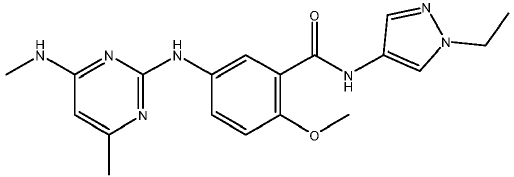 |
| 876 | 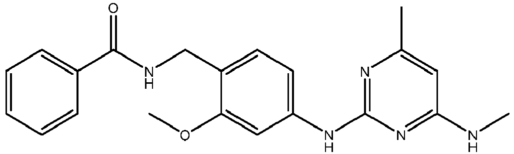 |
| 877 | 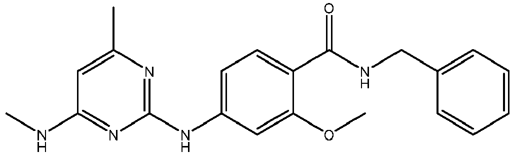 |
| 878 | 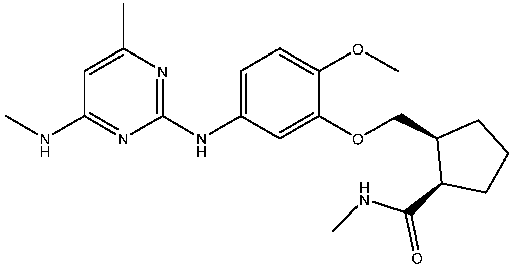 |
| 879 | 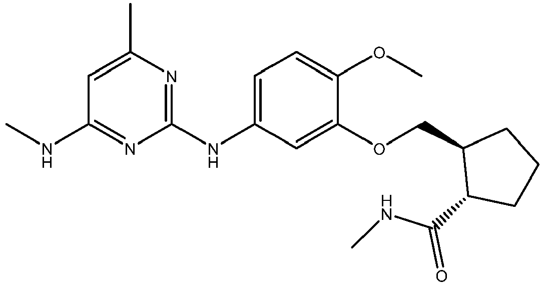 |
| 890 | 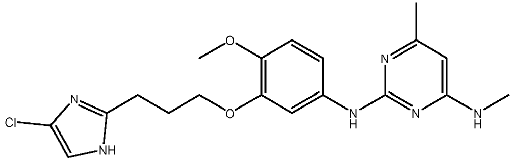 |
| 891 | 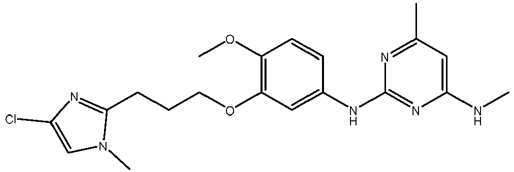 |
| 904 | 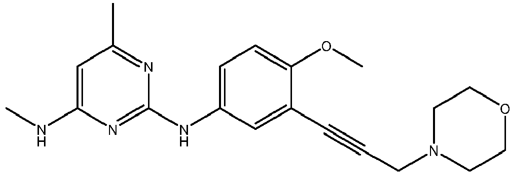 |
| 905 | 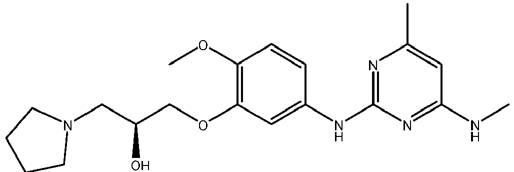 |
| 906 | 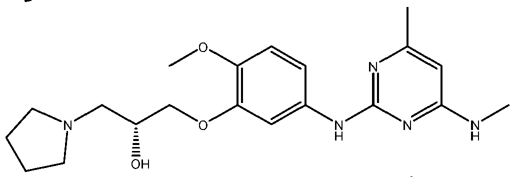 |
| 962 | 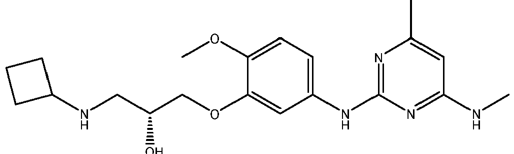 |
| 964 | 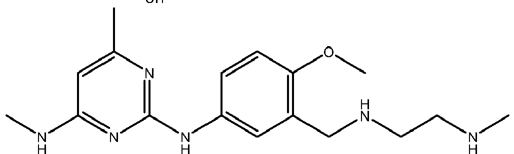 |
| 965 | 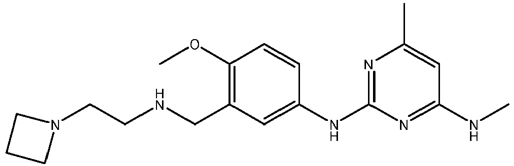 |
| 966 | 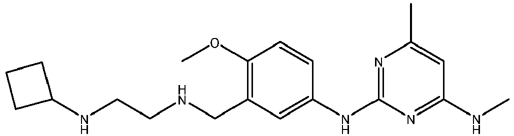 |
| 967 | 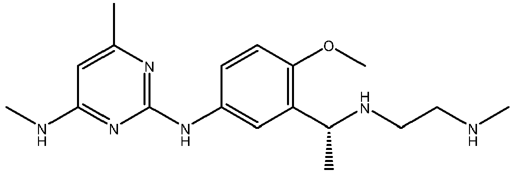 |
| 968 | 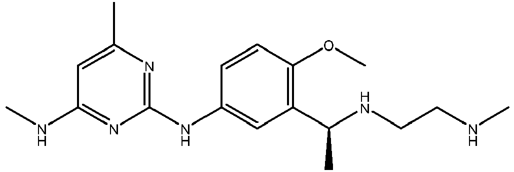 |
| 969 | 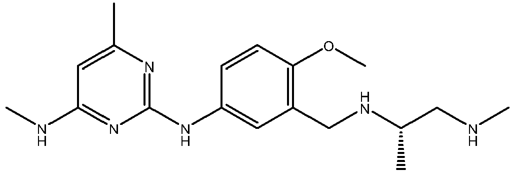 |
| 970 | 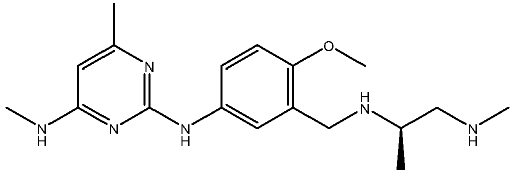 |
| 971 | 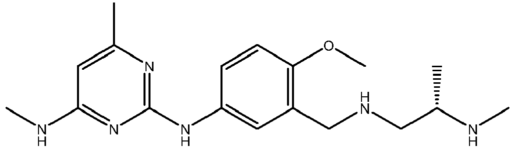 |
| 972 | 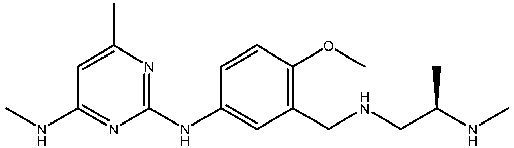 |
| 977 | 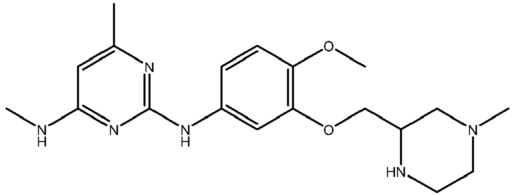 |
| 985 | 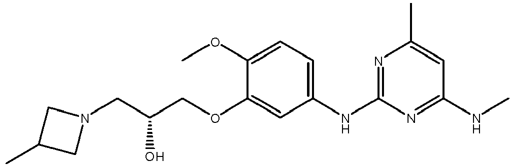 |
| 986 | 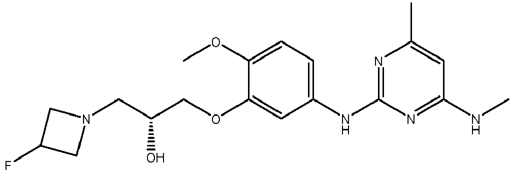 |
| 989 | 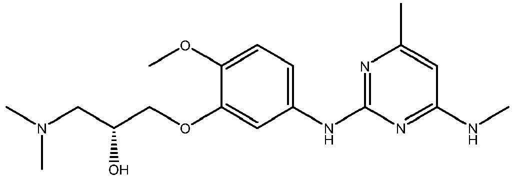 |
| 990 | 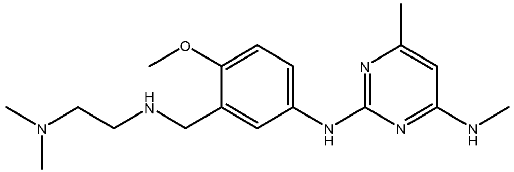 |
| 991 | 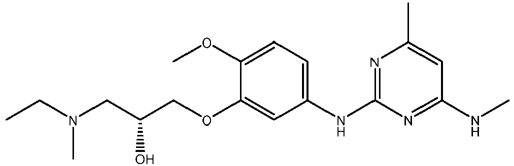 |
| 992 | 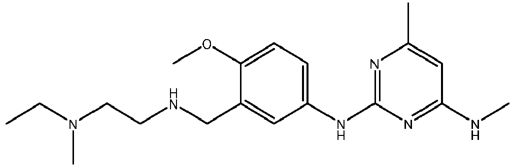 |
| 993 | 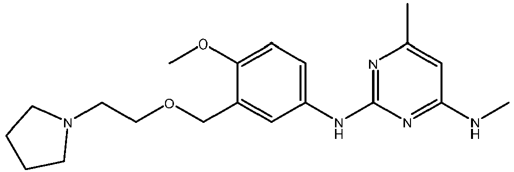 |
| 994 | 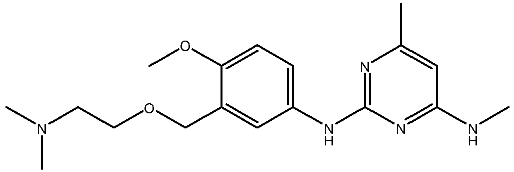 |
| 997 | 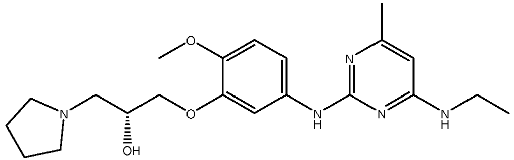 |
| 998 | 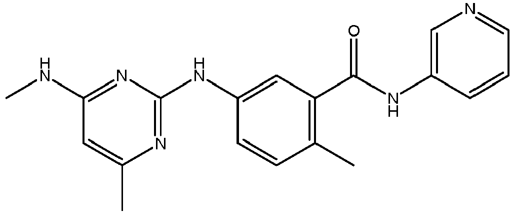 |
| 999 | 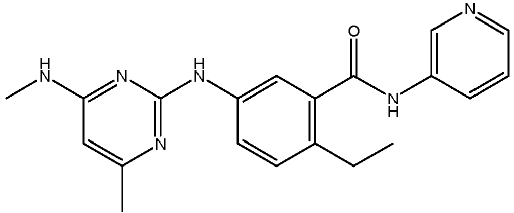 |
| 1000 | 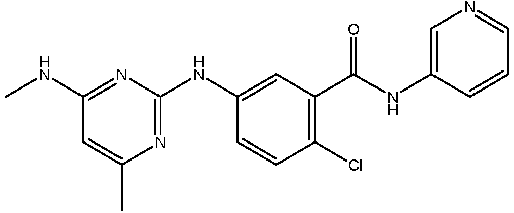 |
| 1001 | 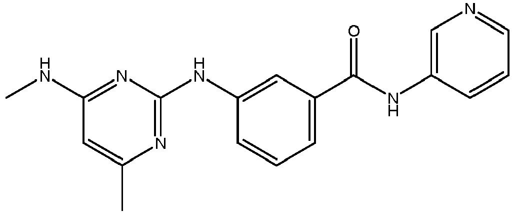 |
| 1004 | 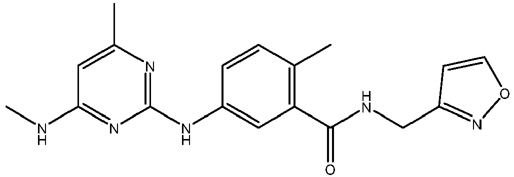 |
| 1029 | 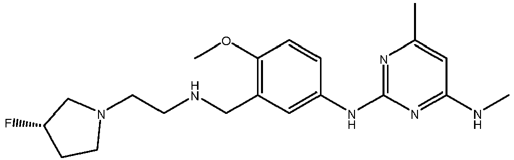 |
| 1030 | 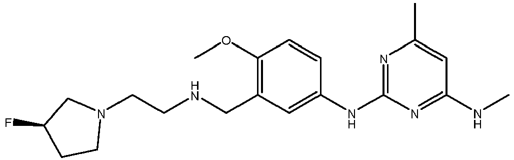 |
| 1036 | 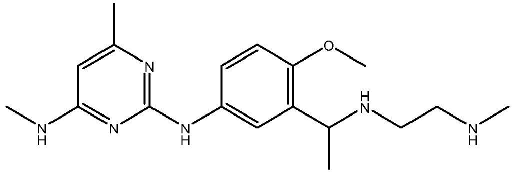 |
| 1037 | 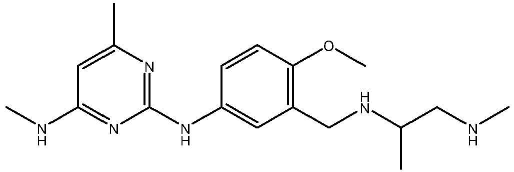 |
| 1038 | 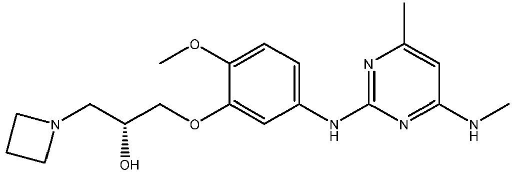 |
| 1040 | 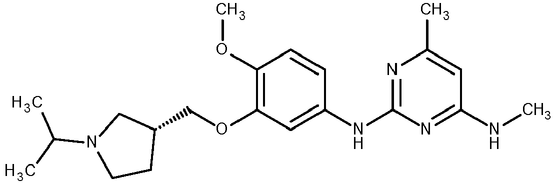 |
| 1044 | 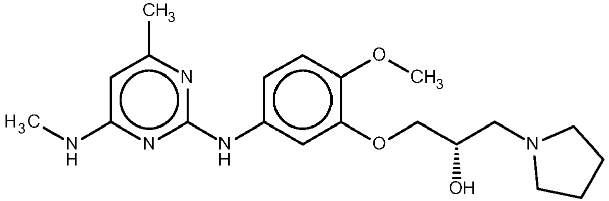 |
| 1045 | 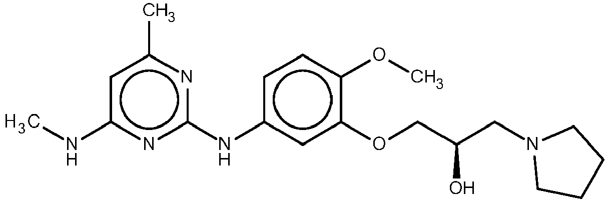 |
| 1078 | 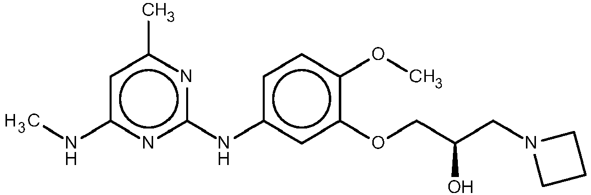 |
| 1079 | 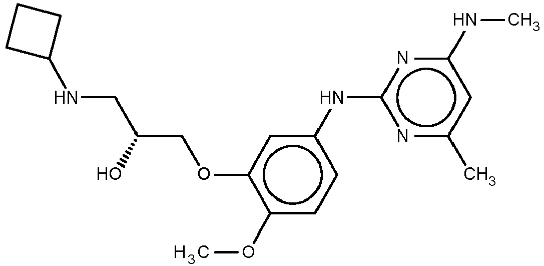 |
| 1080 | 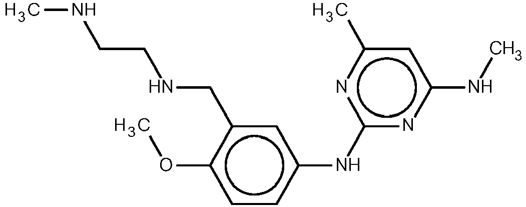 |
| 1081 | 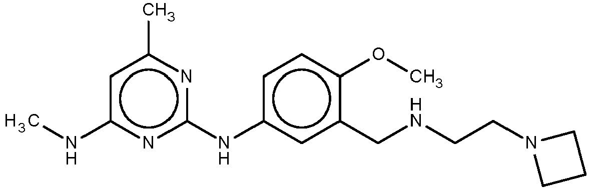 |
| 1090 | 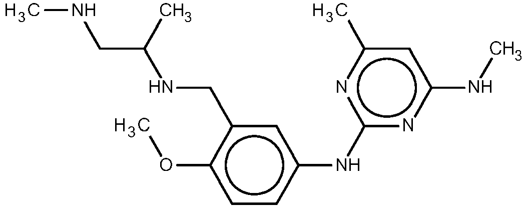 |
| 1094 | 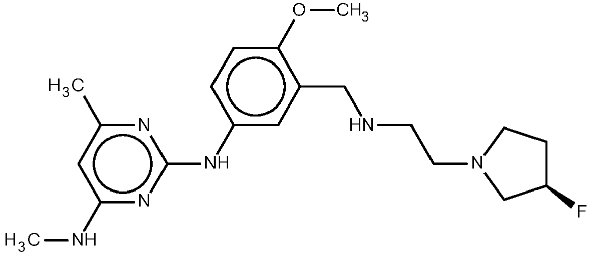 |
| 1095 | 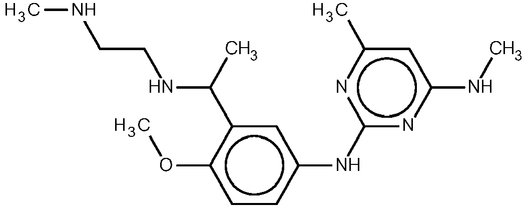 |
| 1099 | 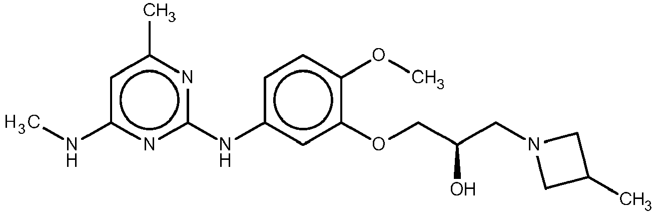 |
| 1100 | 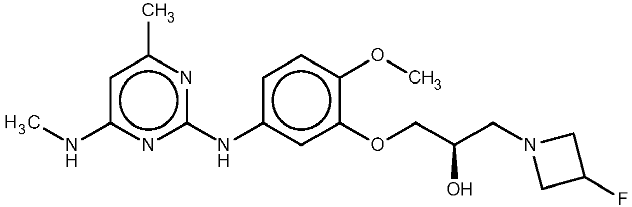 |
| 1101 | 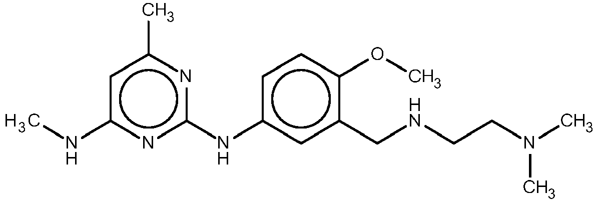 |
| 1102 | 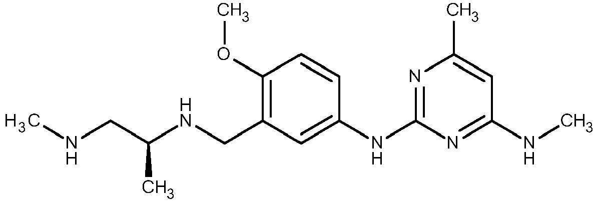 |
| 1103 | 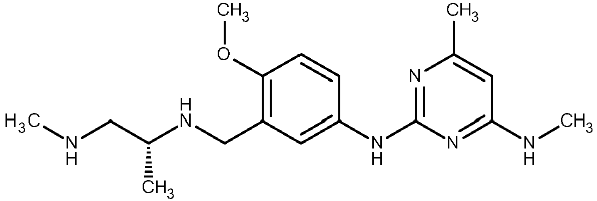 |
| 1104 | 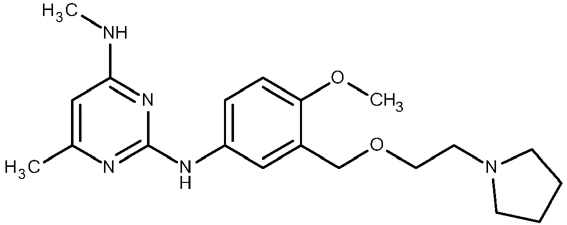 |
| 1105 | 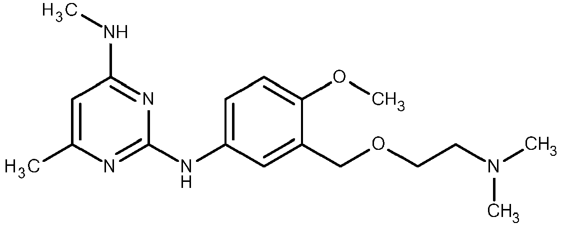 |
| 1106 | 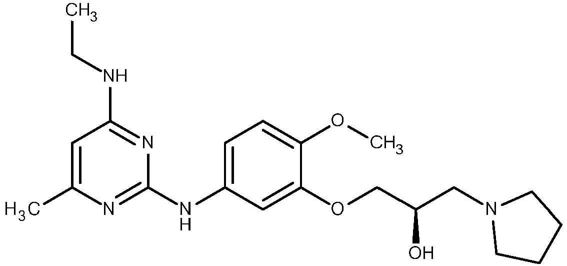 |
| 1109 | 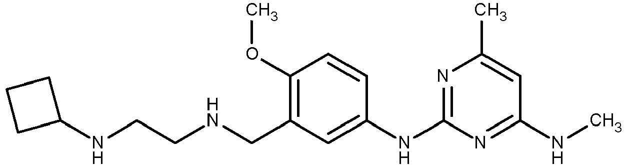 |
| 1112 | 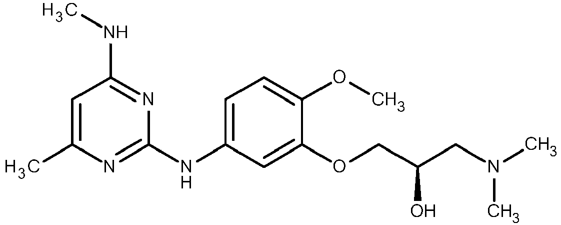 |
| 1113 | 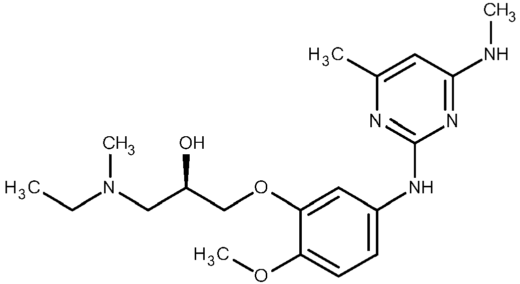 |
| 1114 | 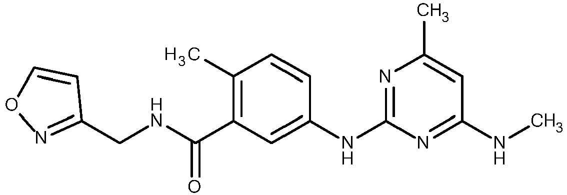 |
| 1117 | 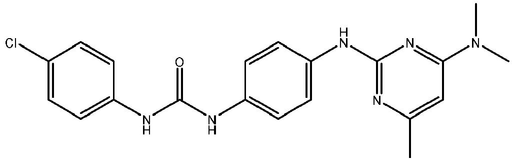 |

Also provided herein are pharmaceutical compositions comprising one or more pharmaceutically acceptable carriers and one or more compounds described herein.

Another aspect of this disclosure provides compounds for use in a method of preventing or treating an EHMT-mediated disorder. The method may includ administering to a subject in need thereof a therapeutically effective amount of a compound of any of Formulae (IIa1), (VI), (VIIIa)-(VIIIc)or a tautomer thereof, or a pharmaceutically acceptable salt of the compound or the tautomer. The EHMT-mediated disorder is a disease, disorder, or condition that is mediated at least in part by the activity of EHMT1 or EHMT2 or both. In one embodiment, the EHMT-mediated disorder is a blood disease or disorder.

In certain embodiments, the EHMT-mediated disorder is selected from proliferative disorders (e.g. Cancers such as leukemia, hepatocellular carcinoma, prostate carcinoma, and lung cancer), addiction (e.g., cocaine addiction), and mental retardation. Unless otherwise stated, any description of a method of treatment includes compounds for use in such treatment or prophylaxis as is described herein, as well as use of the compounds to prepare a medicament to treat or prevent such condition. The treatment includes treatment of human or non-human animals including rodents and other disease models. Methods described herein may be used to identify suitable candidates for treating or preventing EHMT-mediated disorders. For example, the disclosure also provides methods of identifying an inhibitor of EHMT1 or EHMT2 or both.

For example, the EHMT-mediated disease or disorder comprises a disorder that is associated with gene silencing by EHMT1 or EHMT2, e.g., blood diseases or disorders associated with gene silencing by EHMT2.

For example, one or more compounds of formulae (IIa1), (VI), (VIIIa)-(VIIIc) are used in a method comprising the step of administering to a subject having a disease or disorder associated with gene silencing by EHMT1 or EHMT2 a therapeutically effective amount of one or more compounds of Formulae (IIa1), (VI), (VIIIa)-(VIIIc) described herein, wherein the compound(s) inhibits histone methyltransferase activity of EHMT1 or EHMT2, thereby treating the disease or disorder.

For example, the blood disease or disorder is selected from the group consisting of sickle cell anemia and beta-thalassemia.

For example, the blood disease or disorder is hematological cancer.

For example, the hematological cancer is acute myeloid leukemia (AML) or chronic lymphocytic leukemia (CLL).

For example, one or more compounds of formulae (IIa1), (VI), (VIIIa)-(VIIIc) are used in a method further comprising the steps of performing an assay to detect the degree of histone methylation by EHMT1 or EHMT2 in a sample comprising blood cells from a subject in need thereof.

In one embodiment, performing the assay to detect methylation of H3-K9 in the histone substrate comprises measuring incorporation of labeled methyl groups.

In one embodiment, the labeled methyl groups are isotopically labeled methyl groups.

In one embodiment, performing the assay to detect methylation of H3-K9 in the histone substrate comprises contacting the histone substrate with an antibody that binds specifically to dimethylated H3-K9.

Still another aspect of the disclosure provides one or more compounds of formulae (IIa1), (VI), (VIIIa)-(VIIIc) for use in a method of inhibiting conversion of H3-K9 to dimethylated H3-K9,comprising the step of contacting a mutant EHMT, the wild-type EHMT, or both, with a histone substrate comprising H3-K9 and an effective amount of a compound of of formulae (IIa1), (VI), (VIIIa)-(VIIIc), wherein the compound inhibits histone methyltransferase activity of EHMT, thereby inhibiting conversion of H3-K9 to dimethylated H3-K9.

Further, the compounds or methods described herein can be used for research (e.g., studying epigenetic enzymes) and other non-therapeutic purposes.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice or testing of the present disclosure, suitable methods and materials are described below. The references cited herein are not admitted to be prior art to the claimed invention. In the case of conflict, the present specification, including definitions, will control. In addition, the materials, methods and examples are illustrative only and are not intended to be limiting. In the case of conflict between the chemical structures and names of the compounds disclosed herein, the chemical structures will control.

Other features and advantages of the disclosure will be apparent from the following figures, detailed description and claims.

### BRIEF DESCRIPTIONS OF THE DRAWINGS

Figure 1A is a graph indicating the effect of Compound 205 on histone H3K9 dimethylation (data illustrated by triangles) and on fetal hemoglobin-containing cells (HbF+; data illustrated by squares).
Figure 1B is a graph indicating the effect of Compound 418 on histone H3K9 dimethylation (data illustrated by triangles) and on fetal hemoglobin-containing cells (HbF+; data illustrated by squares).
Figure 1C is a graph indicating the effect of Compound 642 on histone H3K9 dimethylation (data illustrated by triangles) and on fetal hemoglobin-containing cells (HbF+; data illustrated by squares).
Figure 1D is a graph indicating the effect of Compound 332 on histone H3K9 dimethylation (data illustrated by triangles) and on fetal hemoglobin-containing cells (HbF+; data illustrated by squares).
Figure 2 is a series of graphs indicating the effect of Compound 205, Compound 642, Compound 332, or Compound 418 on the ratio of Hbb-γ to total β globins.
Figure 3 is a series of graphs indicating the effect of Compound 205, Compound 642, Compound 332, or Compound 418 on the ratio of Hbb-γ to total β globins as measured by mass spectrometry and PCR.
Figure 4 is a graph indicating the effect of Compound 205 on the rate of growth of MV4-11 cells over 14 days. 0.2% DMSO was used as negative control (containing no compound of the disclosure).
Figure 5 is a graph indicating the effect of Compound 205 on the inhibition of growth of MV4-11 cells over 14 days.

### DETAILED DESCRIPTION

The present disclosure provides amine-substituted aryl or heteroaryl compounds, synthetic methods for making the compounds, pharmaceutical compositions containing them and various uses of the compounds.

In one aspect, the compounds disclosed herein may be used to treat a blood disorder, e.g., sickle-cell anemia (i.e., sickle-cell disease). Non-limiting examples of sickle-cell anemia forms that may be treated using the contemplated compounds include hemoglobin SS disease, hemoglobin SC disease, hemoglobin Sβ⁰ thalassemia disease, hemoglobin Sβ⁺ thalassemia disease, hemoglobin SD disease, and hemoglobin SE disease.

Without wishing to be bound by any theory, it is believed that sickle-cell anemia describes a group of inherited red blood cell disorders in which at least some of the red blood cells of a subject having sickle-cell anemia contain hemoglobin S ("HbS"). Hemoglobin S is a mutated, abnormal form of adult hemoglobin. Without wishing to be bound by any theory, it is believed that the contemplated compounds may treat sickle cell anemia by inducing fetal hemoglobin ("HbF") expression. *See, e.g.,* Renneville et al., Blood 126(16): 1930-1939, 2015.

In some embodiments, one or more complications of sickle-cell anemia may be treated or prevented using the contemplated compounds disclosed herein. Non-limiting examples of complications that may be treated or prevented using the contemplated compounds include anemia (e.g., severe anemia), hand-foot syndrome, splenic sequestration, delayed developmental growth, eye disorders (e.g., vision loss caused by, e.g., blockages in blood vessels supplying the eyes), skin ulcers (e.g., leg ulcers), heart disease, chest syndrome (e.g., acute chest syndrome), priapism, and pain.

The present disclosure provides compounds of Formula (I): 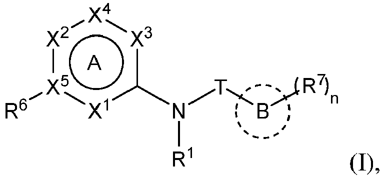 and tautomers thereof, and pharmaceutically acceptable salts of the compounds or the tautomers, wherein
ring A is phenyl or a 5- or 6-membered heteroaryl;
X¹ is N, CR², or NR²' as valency permits;
X² is N, CR³, or NR³' as valency permits;
X³ is N, CR⁴, or NR⁴' as valency permits;
X⁴ is N or CR⁵, or X⁴ is absent such that ring A is a 5-membered heteroaryl containing at least one N atom;
X⁵ is C or N as valency permits;
B is absent or a ring structure selected from the group consisting of C₆-C₁₀ aryl, C₃-C₁₀ cycloalkyl, 5- to 10-membered heteroaryl, and 4- to 12-membered heterocycloalkyl containing 1-4 heteroatoms selected from N, O, and S;
T is a bond or C₁-C₆ alkylene, C₂-C₆ alkenylene, or C₂-C₆ alkynylene linker optionally substituted with one or more of halo, cyano, hydroxyl, oxo; or C₁-C₆ alkoxy when B is present; or T is H and n is 0 when B is absent; or T is C₁-C₆ alkyl optionally substituted with (R⁷)ₙ when B is absent; or when B is absent, T and R¹ together with the atoms to which they are attached optionally form a 4-7 membered heterocycloalkyl or 5-6 membered heteroaryl, each of which is optionally substituted with (R⁷)ₙ;
R¹ is H or C₁-C₄ alkyl;
each of R², R³, and R⁴, independently is selected from the group consisting of H, halo, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 7- membered heterocycloalkyl, 5- to 6-membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl, or R³ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, amino, mono- or di-alkylamino, or C₁-C₆ alkoxyl;; or when ring A is a 5-membered heteroaryl containing at least one N atom, R⁴ is a spiro-fused 4- to 12-membered heterocycloalkyl containing 1-4 heteroatoms selected from N, O, and S;
each of R²', R³' and R⁴, independently is H or C₁-C₃ alkyl;
R⁵ is selected from the group consisting of H, F, Br, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 12-membered heterocycloalkyl containing 1-4 heteroatoms selected from N, O, and S, C₁-C₆ alkyl optionally substituted with one or more of halo, OR^{a} or NR^{a}R^{b}, and C₂-C₆ alkynyl optionally substituted with 4- to 12-membered heterocycloalkyl; wherein said C₃-C₈ cycloalkyl or 4- to 12-membered heterocycloalkyl are optionally substituted with one or more of halo, C(O)R^{a}, OR^{a}, NR^{a}R^{b}, 4-to 7-membered heterocycloalkyl, -C₁-C₆ alkylene-4- to 7-membered heterocycloalkyl, or C₁-C₄ alkyl optionally substituted with one or more of halo, OR^{a} or NR^{a}R^{b}, in which each of R^{a} and R^{b} independently is H or C₁-C₆ alkyl; or
R⁵ and one of R³ or R⁴ together with the atoms to which they are attached form phenyl or a 5- or 6-membered heteroaryl; or R⁵ and one of R³'or R⁴' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl or C₁-C₃ alkoxyl;
R⁶ is absent when X⁵ is N and ring A is a 6-membered heteroaryl; or R⁶ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, C(O)R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, NR⁸R⁹, or C₁-C₆ alkoxyl; and R⁶ is not NR⁸C(O)NR¹²R¹³; or
R⁶ and one of R² or R³ together with the atoms to which they are attached form phenyl or a 5- or 6-membered heteroaryl; or R⁶ and one of R²'or R³' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl, oxo (=O), C₁-C₃ alkoxyl, or -Q¹-T¹;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, cyano, OR¹⁰, OR¹¹, C(O)R¹¹, NR¹⁰R¹¹, C(O)NR¹⁰R¹¹, NR¹⁰C(O)R¹¹, 5- to 10-membered heteroaryl, C₃-C₈ cycloalkyl, or 4- to 12-membered heterocycloalkyl containing 1-4 heteroatoms selected from N, O, and S, and wherein the 5- to 10-membered heteroaryl, C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R⁷ is not H or C(O)OR^{g}; or optionally, when B is present, one R⁷ and R⁵ together form a C₃-C₁₀ alkylene, C₂-C₁₀ heteroalkylene, C₄-C₁₀ alkenylene, C₂-C₁₀ heteroalkenylene, C₄-C₁₀ alkynylene or C₂-C₁₀ heteroalkynylene linker optionally substituted with one or more of halo, cyano, hydroxyl, or C₁-C₆ alkoxyl;
each R⁸ independently is H or C₁-C₆ alkyl;
each R⁹ is independently -Q³-T³, in which Q³ is a bond or C₁-C₆ alkylene, C₂-C₆ alkenylene, or C₂-C₆ alkynylene linker optionally substituted with one or more of halo, cyano, hydroxyl, or C₁-C₆ alkoxyl, and T³ is H, halo, OR¹², OR¹³, NR¹²R¹³, NR¹²C(O)R¹³, C(O)NR¹²R¹³, C(O)R¹³, S(O)₂R¹³, S(O)₂NR¹²R¹³, or R^{S2}, in which R^{S2} is C₃-C₈ cycloalkyl, C₆-C₁₀ aryl, 4- to 12-membered heterocycloalkyl containing 1-4 heteroatoms selected from N, O, and S, or a 5- to 10-membered heteroaryl, and R^{S2} is optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or -Q⁴-T⁴ is oxo; or
R⁸ and R⁹ taken together with the nitrogen atom to which they are attached form a 4-to 12-membered heterocycloalkyl containing 1-4 heteroatoms selected from N, O and S, which is optionally substituted with one or more of -Q⁵-T⁵, wherein each Q⁵ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁵ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{e}, C(O)R^{e}, S(O)₂R^{e}, S(O)₂NR^{e}R^{f}, NR^{e}R^{f}, C(O)NR^{e}R^{f}, and NR^{e}C(O)R^{f}, each of R^{e} and R^{f} independently being H or C₁-C₆ alkyl; or -Q⁵-T⁵ is oxo;
R¹⁰ is selected from the group consisting of H and C₁-C₆ alkyl;
R¹¹ is -Q⁶-T⁶, in which Q⁶ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T⁶ is H, halo, OR^{g}, NR^{g}R^{h}, NR^{g}C(O)R^{h}, C(O)NR^{g}R^{h}, C(O)R^{g}, S(O)₂R^{g}, or R^{S3}, in which each of R^{g} and R^{h} independently is H, phenyl, C₃-C₈ cycloalkyl, or C₁-C₆ alkyl optionally substituted with C₃-C₈ cycloalkyl, or R^{g} and R^{h} together with the nitrogen atom to which they are attached form a 4- to 12-membered heterocycloalkyl containing 1-4 heteroatoms selected from N, O, and S, and R^{S3} is C₃-C₈ cycloalkyl, C₆-C₁₀ aryl, 4- to 12-membered heterocycloalkyl containing 1-4 heteroatoms selected from N, O and S, or a 5- to 10-membered heteroaryl, and R^{S3} is optionally substituted with one or more -Q⁷-T⁷, wherein each Q⁷ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁷ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{j}, C(O)R^{j}, NR^{j}R^{k}, C(O)NR^{j}R^{k}, S(O)₂R^{j}, and NR^{j}C(O)R^{k}, each of R^{j} and R^{k} independently being H or C₁-C₆ alkyl optionally substituted with one or more halo; or -Q⁷-T⁷ is oxo; or
R¹⁰ and R¹¹ taken together with the nitrogen atom to which they are attached form a 4-to 12-membered heterocycloalkyl containing 1-4 heteroatoms selected from N, O, and S, which is optionally substituted with one or more of halo, C₁-C₆ alkyl, hydroxyl, or C₁-C₆ alkoxyl;
R¹² is H or C₁-C₆ alkyl;
R¹³ is C₁-C₆ alkyl, C₃-C₈ cycloalkyl, C₆-C₁₀ aryl, 4- to 12-membered heterocycloalkyl containing 1-4 heteroatoms selected from N, O, and S, or a 5- to 10-membered heteroaryl, each of which is optionally substituted with one or more -Q⁸-T⁸, wherein each Q⁸ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁸ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or -Q⁸-T⁸ is oxo; and
n is 0, 1, 2, 3, or 4.

The compounds of Formula (I) can have one or more of the following features when applicable:

For example, the compound of Formula (I) is not 4-(((2-((1-acetylindolin-6-yl)amino)-6-(trifluoromethyl)pyrimidin-4-yl)amino)methyl)benzenesulfonamide,
5-bromo-N⁴-(4-fluorophenyl)-N²-(4-methoxy-3-(2-(pyrrolidin-1-yl)ethoxy)phenyl)pyrimidine-2,4-diamine,
N²-(4-methoxy-3-(2-(pyrrolidin-1-yl)ethoxy)phenyl)-N⁴-(5-(tert-pentyl)-1H-pyrazol-3-yl)pyrimidine-2,4-diamine,
4-((2,4-dichloro-5-methoxyphenyl)amino)-2-((3-(2-(pyrrolidin- 1-yl)ethoxy)phenyl)amino)pyrimidine-5-carbonitrile,
N-(naphthalen-2-yl)-2-(piperidin-1-ylmethoxy)pyrimidin-4-amine,
N-(3,5-difluorobenzyl)-2-(3-(pyrrolidin-1-yl)propyl)pyrimidin-4-amine,
N-(((4-(3-(piperidin-1-yl)propyl)pyrimidin-2-yl)amino)methyl)benzamide,
N-(2-((2-(3-(dimethylamino)propyl)pyrimidin-4-yl)amino)ethyl)benzamide,
2-(hexahydro-4-methyl-1H-1,4-diazepin-1-yl)-6,7-dimethoxy-N-[1-(phenylmethyl)-4-piperidinyl] -4-quinazolinamine,
2-cyclohexyl-6-methoxy-N-[1-(1-methylethyl)-4-piperidinyl]-7-[3-(1-pyrrolidinyl)propoxy]-4-quinazolinamine,
3-(1-cyano-1-methylethyl)-N-[3-[(3,4-dihydro-3-methyl-4-oxo-6-quinazolinyl)amino]-4-methylphenyl]benzamide,
6-acetyl-8-cyclopentyl-5-methyl-2-[(5-piperazin-1-ylpyridin-2-yl)amino]pyrido[2,3-d]pyrimidin-7-one,
*N*-[2-[[4-(Diethylamino)butyl]amino]-6-(3,5-dimethoxyphenyl)pyrido[2,3-d]pyrimidin-7-yl]-*N*'-(1,1-dimethylethyl)urea, or
6-[[2-[[4-(2,4-dichlorophenyl)-5-(5-methyl-1H-imidazol-2-yl)-2-pyrimidinyl] amino] ethyl] amino] -3-pyridinecarbonitrile.

For example, when T is a bond, B is substituted phenyl, and R⁶ is NR⁸R⁹, in which R⁹ is -Q³-R^{S2}, and R^{S2} is optionally substituted 4- to 7-membered heterocycloalkyl or a 5- to 6-membered heteroaryl, then B is substituted with at least one substituent selected from (i) -Q²-OR¹¹ in which R¹¹ is -Q⁶-R^{S3} and Q⁶ is optionally substituted C₂-C₆ alkylene, C₂-C₆ alkenylene, or C₂-C₆ alkynylene linker and (ii) -Q²-NR¹⁰R¹¹ in which R¹¹ is -Q⁶-R^{S3}.

For example, when T is a bond and B is optionally substituted phenyl, then R⁶ is not OR⁹ or NR⁸R⁹ in which R⁹ is optionally substituted naphthyl.

For example, when T is a bond and B is optionally substituted phenyl, naphthyl, indanyl or 1,2,3,4-tetrahydronaphthyl, then R⁶ is not NR⁸R⁹ in which R⁹ is optionally substituted phenyl, naphthyl, indanyl or 1,2,3,4-tetrahydronaphthyl.

For example, when T is a bond and B is optionally substituted phenyl or thiazolyl, then R⁶ is not optionally substituted imidazolyl, pyrazolyl, pyridyl, pyrimidyl, or NR⁸R⁹ in which R⁹ is optionally substituted imidazolyl, pyrazolyl, or 6- to 10-membered heteroaryl.

For example, when T is a C₁-C₆ alkylene linker and B is absent or optionally substituted C₆-C₁₀ aryl or 4- to 12-membered heterocycloalkyl; or when T is a bond and B is optionally substituted C₃-C₁₀ cycloalkyl or 4- to 12-membered heterocycloalkyl, then R⁶ is not NR⁸C(O)R¹³.

For example, when X¹ and X³ are N, X² is CR³, X⁴ is CR⁵, X⁵ is C, R⁵ is 4- to 12-membered heterocycloalkyl substituted with one or more C₁-C₆ alkyl, and R⁶ and R³ together with the atoms to which they are attached form phenyl which is substituted with one or more of optionally substituted C₁-C₃ alkoxyl, then B is absent, C₆-C₁₀ aryl, C₃-C₁₀ cycloalkyl, or 5-to 10-membered heteroaryl.

For example, when X² and X³ are N, X¹ is CR², X⁴ is CR⁵, X⁵ is C, R⁵ is C₃-C₈ cycloalkyl or 4- to 12-membered heterocycloalkyl, each optionally substituted with one or more C₁-C₆ alkyl, and R⁶ and R² together with the atoms to which they are attached form phenyl which is substituted with one or more of optionally substituted C₁-C₃ alkoxyl, then B is absent, C₆-C₁₀ aryl, C₃-C₁₀ cycloalkyl, or 5- to 10-membered heteroaryl.

For example, when T is a bond and B is hydroxyl-substituted phenyl, then ring A is not pyrazinyl.

For example, when ring A is phenyl and B is a 5-membered heteroaryl or phenyl, then T is not C(O),

For example, when ring A is phenyl, B is absent, and T and R¹ together with the atoms to which they are attached form a 4-7 membered heterocycloalkyl, the heterocycloalkyl contains at most one N ring atom or the heterocycloalkyl is not substituted by oxo,

For example, when one of ring A or B is pyridyl and T is a bond, then the pyridinyl is not substituted at the para-position of N-^{R1} with -Q¹-T¹ or -Q²-T², in which T¹ or T² is phenyl or heteroaryl, or

For example, when T is a bond or C₁-C₃ alkylene, ring A is a 6-membered heteroaryl and B is optionally substituted phenyl, pyridyl, or piperidinyl, then R⁶ is not H and at least one of R², R³, R⁴ and R⁵ is not H.

For example, ring A is a 6-membered heteroaryl, wherein at least one of X¹, X², X³ and X⁴ is N and X⁵ is C (e.g., pyridyl, pyrimidinyl, pyrazinyl, pyridazinyl, and triazinyl).

For example, ring A is a 6-membered heteroaryl, wherein two of X¹, X², X³ and X⁴ is N and X⁵ are C (e.g., pyrimidinyl, pyrazinyl, pyridazinyl, and triazinyl).

For example, R⁶ and one of R² or R³ together with the ring A to which they are attached form an optionally substituted 6,5- fused bicyclic heteroaryl; or R⁶ and one of R²' or R³' together the ring A to which they are attached form an optionally substituted 6,5-fused bicyclic heteroaryl. For example, the optionally substituted 6,5- fused bicyclic heteroaryl contains 1-4 N atoms. For example, the 6,5- fused bicyclic heteroaryl is optionally substituted with one or more of halo, C₁-C₃ alkyl, hydroxyl, or C₁-C₃ alkoxyl.

For example, T is a bond and ring B is phenyl.

For example, T is a bond and ring B is pyridyl.

For example, T is a bond or C₁-C₆ alkylene, C₂-C₆ alkenylene, or C₂-C₆ alkynylene linker optionally substituted with one or more of halo, cyano, hydroxyl or C₁-C₆ alkoxy when B is present.

For example, T is a bond or C₁-C₆ alkylene, C₂-C₆ alkenylene, or C₂-C₆ alkynylene linker when B is present.

For example, n is 1.

For example, n is 2.

For example, n is 3.

For example, at least one of R⁶, R², R³, and R⁴ is not H.

For example, when one or more of R², R³, and R⁴ are present, at least one of R⁶, R², R³, and R⁴ is not H.

For example, the compounds of Formula (I) include those of Formula (II): 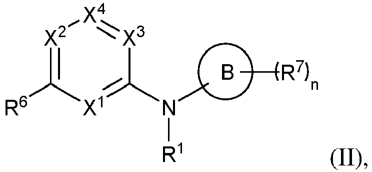 wherein
ring B is phenyl or pyridyl,
one or both of X¹ and X² are N while X³ is CR⁴ and X⁴ is CR⁵ or one or both of X¹ and X³ are N while X² is CR³ and X⁴ is CR⁵; and
n is 1, 2, or 3.

For example, the compounds of Formula (II) include those of Formula (IIa1), (IIa2), (IIa3), (IIa4) or (IIa5): 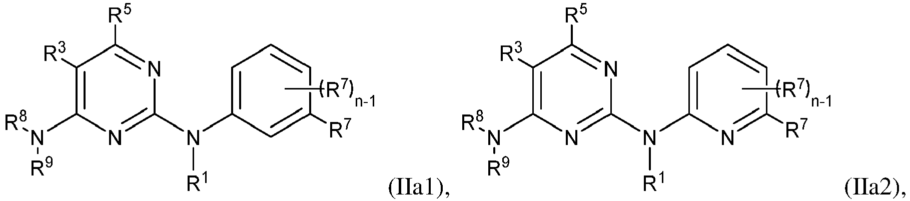 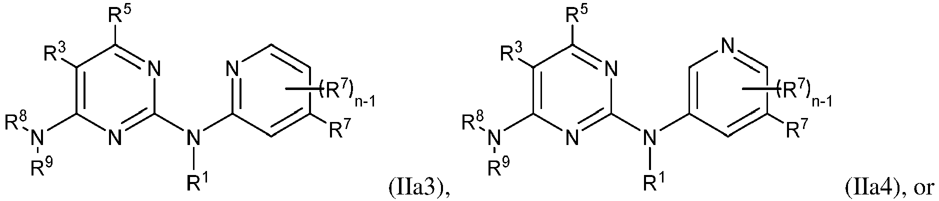 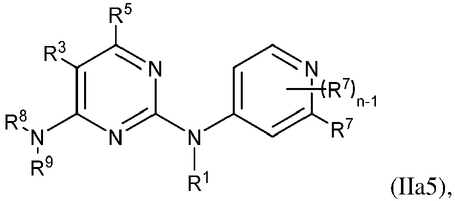 or tautomers thereof, or pharmaceutically acceptable salts of the compounds or the tautomers.

For example, at most one of R³ and R⁵ is not H.

For example, neither of R³ and R⁵ is H.

For example, each of R³ and R⁵ is H.

For example, the compounds of Formula (II) include those of Formula (IIb1), (IIb2), (IIb3), (IIb4) or (IIb5): 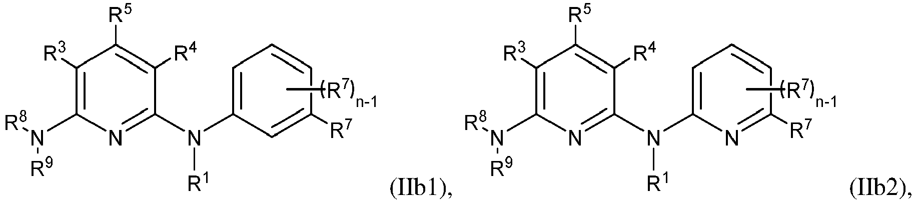 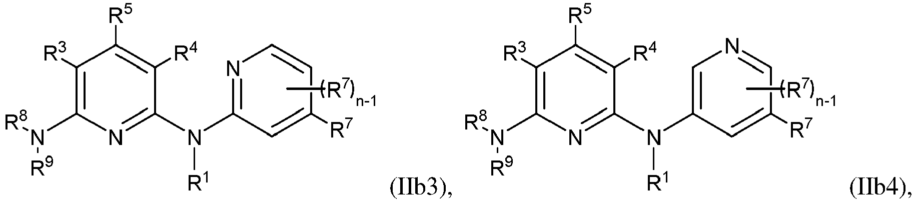 or 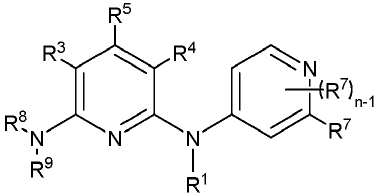 (IIb5), or tautomers thereof, or pharmaceutically acceptable salts of the compounds or the tautomers.

For example, at most one of R³, R⁴ and R⁵ is not H.

For example, at most two of R³, R⁴ and R⁵ are not H.

For example, none of R³, R⁴ and R⁵ is H.

For example, each of R³, R⁴ and R⁵ is H.

For example, the compounds of Formula (II) include those of Formula (IIc1), (IIc2), (IIc3), (IIc4) or (IIc5): 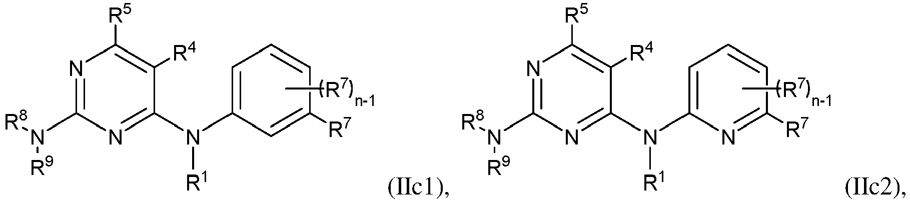 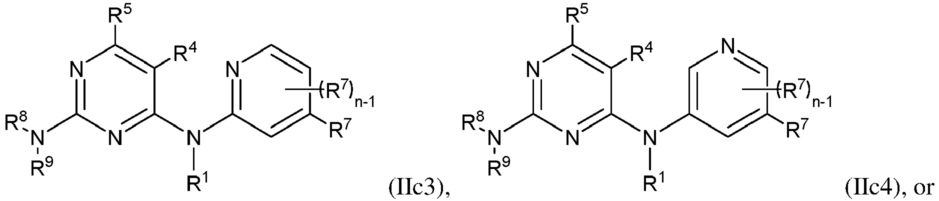 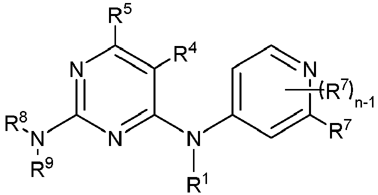 (IIc5), or tautomers thereof, or pharmaceutically acceptable salts of the compounds or the tautomers.

For example, at most one of R⁴ and R⁵ is not H.

For example, neither of R⁴ and R⁵ is H.

For example, each of R⁴ and R⁵ is H.

For example, the compounds of Formula (II) include those of Formula (IId1), (IId2), (IId3), (IId4) or (IId5): 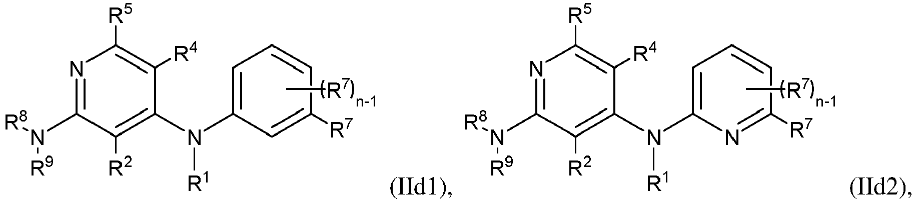 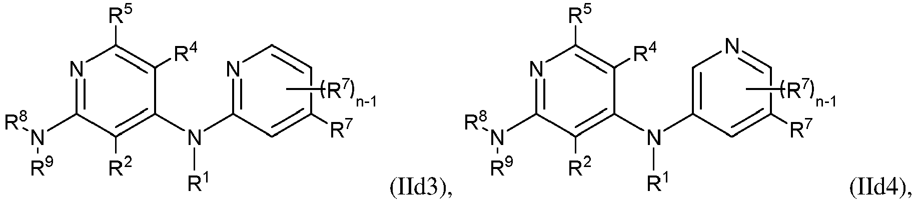 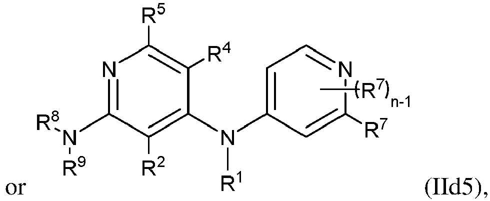 or tautomers thereof, or pharmaceutically acceptable salts of the compounds or the tautomers.

For example, at most one of R², R⁴ and R⁵ is not H.

For example, at most two of R², R⁴ and R⁵ are not H.

For example, none of R², R⁴ and R⁵ is H.

For example, each of R², R⁴ and R⁵ is H.

For example, one R⁷ and R⁵ together form a C₃-C₁₀ alkylene, C₂-C₁₀ heteroalkylene, C₄-C₁₀ alkenylene, C₂-C₁₀ heteroalkenylene, C₄-C₁₀ alkynylene or C₂-C₁₀ heteroalkynylene linker optionally substituted with one or more of halo, cyano, hydroxyl, or C₁-C₆ alkoxyl.

For example, one R⁷ and R⁵ together form an optionally substituted C₂-C₁₀ heteroalkylene linker, e.g., -NH(CH2)₂O(CH2)₂O-.

For example, ring A is a 5-membered heteroaryl (e.g., pyrrolyl, imidazolyl, triazolyl, tetrazolyl, or pyrazolyl).

For example, the compounds of Formula (I) include those of Formula (III): 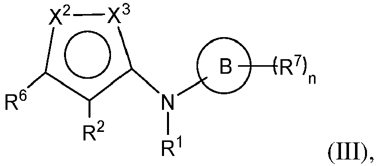 or tautomers thereof, or pharmaceutically acceptable salts of the compounds or the tautomers, wherein
ring B is phenyl or pyridyl,
at least one of X² and X³ is N; and
n is 1 or 2.

For example, the compounds of Formula (III) include those of Formula (IIIa): 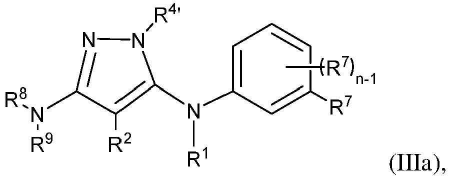 or tautomers thereof, or pharmaceutically acceptable salts of the compounds or the tautomers.

For example, at most one of R⁴' and R² is not H.

For example, neither of R⁴' and R² is H.

For example, each of R⁴' and R² is H.

For example, the compounds of Formula (I) include those of Formula (IV): 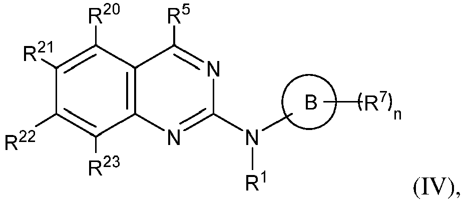 or tautomers thereof, or pharmaceutically acceptable salts of the compounds or the tautomers, wherein
ring B is C₃-C₆ cycloalkyl;
each of R²⁰, R²¹, R²² and R²³ independently is H, halo, C₁-C₃ alkyl, hydroxyl or C₁-C₃ alkoxyl; and
n is 1 or 2.

For example, ring B is cyclohexyl.

For example, B is absent and T is unsubstituted C₁-C₆ alkyl or T is C₁-C₆ alkyl substituted with at least one R⁷.

For example, B is 4 to 12-membered heterocycloalkyl (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, and the like) and T is unsubstituted C₁-C₆ alkyl.

For example, the compounds of Formula (I) include those of Formula (IVa): 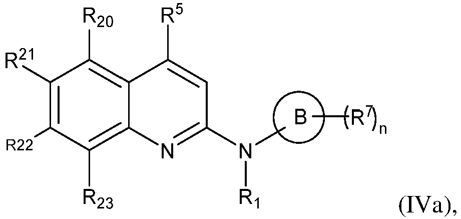 or tautomers thereof, or pharmaceutically acceptable salts of the compounds or the tautomers, wherein
ring B is C₃-C₆ cycloalkyl;
each of R²⁰, R²¹, R²² and R²³ independently is H, halo, C₁-C₃ alkyl, hydroxyl or C₁-C₃ alkoxyl; and
n is 1 or 2.

For example, ring B is cyclohexyl.

For example, B is absent and T is unsubstituted C₁-C₆ alkyl or T is C₁-C₆ alkyl substituted with at least one R⁷.

For example, B is 4 to 12-membered heterocycloalkyl (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, and the like) and T is unsubstituted C₁-C₆ alkyl.

For example, the compounds of Formula (I) include those of Formula (V): 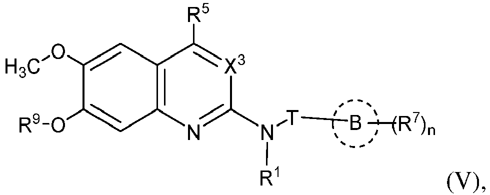 wherein
ring B is absent or C₃-C₆ cycloalkyl;
X³ is N or CR⁴ in which R⁴ is H or C₁-C₄ alkyl;
R¹ is H or C₁-C₄ alkyl;
or when B is absent, T and R¹ together with the atoms to which they are attached optionally form a 4-7 membered heterocycloalkyl or 5-6 membered heteroaryl, each of which is optionally substituted with (R⁷)ₙ; or when B is absent, T is H and n is 0;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, OR¹⁰, OR¹¹, C(O)R¹¹, NR¹⁰R¹¹, C(O)NR¹⁰R¹¹, NR¹⁰C(O)R¹¹, C₃-C₈ cycloalkyl, or 4- to 12-membered heterocycloalkyl containing 1-4 heteroatoms selected from N, O, and S, and wherein the C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R₇ is not H or C(O)OR^{g};
R⁵ is selected from the group consisting of C₁-C₆ alkyl, C₃-C₈ cycloalkyl and 4- to 12-membered heterocycloalkyl containing 1-4 heteroatoms selected from N, O and S, wherein the C₃-C₈ cycloalkyl and 4- to 12-membered heterocycloalkyl is optionally substituted with one or more of 4- to 7-membered heterocycloalkyl, -C₁-C₆ alkylene-4- to 7-membered heterocycloalkyl, -C(O)C₁-C₆ alkyl or C₁-C₆ alkyl optionally substituted with one or more of halo or OR^{a};
R⁹ is -Q³-T³, in which Q³ is a bond or C₁-C₆ alkylene, C₂-C₆ alkenylene, or C₂-C₆ alkynylene linker optionally substituted with one or more of halo, cyano, hydroxyl, or C₁-C₆ alkoxyl, and T³ is 4- to 12-membered heterocycloalkyl containing 1-4 heteroatoms selected from N, O, and S, optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or - Q⁴-T⁴ is oxo; and
n is 0, 1 or 2.

For example, the compounds of Formula (V) include those of Formula (Va): 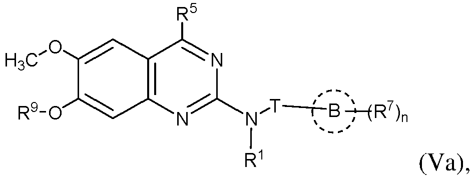 and tautomers thereof, and pharmaceutically acceptable salts of the compounds or the tautomers.

For example, in Formula (Va), ring B is absent or C₃-C₆ cycloalkyl;
R¹ is H or C₁-C₄ alkyl;
or when B is absent, T and R¹ together with the atoms to which they are attached optionally form a 4-7 membered heterocycloalkyl or 5-6 membered heteroaryl, each of which is optionally substituted with (R⁷)ₙ;
each R⁷ is independently oxo (=O) or -Q²-T², in which each Q² independently is a bond or C₁-C₆ alkylene linker optionally substituted with one or more of halo, cyano, hydroxyl, amino, mono- or di- alkylamino, or C₁-C₆ alkoxyl, and each T² independently is H, halo, OR¹⁰, OR¹¹, C(O)R¹¹, NR¹⁰R¹¹, C(O)NR¹⁰R¹¹, NR¹⁰C(O)R¹¹, C₃-C₈ cycloalkyl, or 4- to 12-membered heterocycloalkyl containing 1-4 heteroatoms selected from N, O, and S, and wherein the C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, and R₇ is not H or C(O)OR^{g}; each of R^{x} and R^{y} independently being H or C₁-C₆ alkyl; or R⁵ is selected from the group consisting of C₃-C₈ cycloalkyl and 4- to 12-membered heterocycloalkyl containing 1-4 heteroatoms selected from N, O and S, optionally substituted with one or more of -C(O)C₁-C₆ alkyl or C₁-C₆ alkyl optionally substituted with one or more of halo or OR^{a};
R⁹ is -Q³-T³, in which Q³ is a bond or C₁-C₆ alkylene linker optionally substituted with one or more of halo, cyano, hydroxyl, or C₁-C₆ alkoxyl, and T³ is 4- to 12-membered heterocycloalkyl containing 1-4 heteroatoms selected from N, O, and S, optionally substituted with one or more -Q⁴-T⁴, wherein each Q⁴ independently is a bond or C₁-C₃ alkylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or - Q⁴-T⁴ is oxo; and
n is 1 or 2.

For example, the compounds of Formula (V) include those of Formula (Vb): 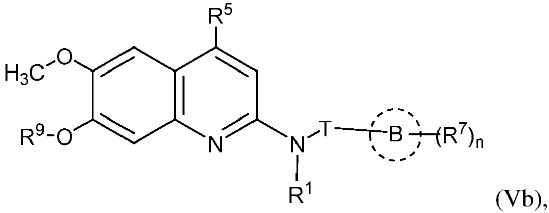 and tautomers thereof, and pharmaceutically acceptable salts of the compounds or the tautomers, wherein
ring B is absent or C₃-C₆ cycloalkyl;
R¹ is H or C₁-C₄ alkyl;
or when B is absent, T and R¹ together with the atoms to which they are attached optionally form a 4-7 membered heterocycloalkyl or 5-6 membered heteroaryl, each of which is optionally substituted with (R⁷)ₙ; or when B is absent, T is H and n is 0;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, OR¹⁰, OR¹¹, C(O)R¹¹, NR¹⁰R¹¹, C(O)NR¹⁰R¹¹, NR¹⁰C(O)R¹¹, C₃-C₈ cycloalkyl, or 4- to 12-membered heterocycloalkyl containing 1-4 heteroatoms selected from N, O, and S, and wherein the C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R₇ is not H or C(O)OR^{g};
R⁵ is selected from the group consisting of C₁-C₆ alkyl, C₃-C₈ cycloalkyl and 4- to 12-membered heterocycloalkyl containing 1-4 heteroatoms selected from N, O and S, wherein the C₃-C₈ cycloalkyl and 4- to 12-membered heterocycloalkyl is optionally substituted with one or more of 4- to 7-membered heterocycloalkyl, -C₁-C₆ alkylene-4- to 7-membered heterocycloalkyl, -C(O)C₁-C₆ alkyl or C₁-C₆ alkyl optionally substituted with one or more of halo or OR^{a};
R⁹ is -Q³-T³, in which Q³ is a bond or C₁-C₆ alkylene, C₂-C₆ alkenylene, or C₂-C₆ alkynylene linker optionally substituted with one or more of halo, cyano, hydroxyl, or C₁-C₆ alkoxyl, and T³ is 4- to 12-membered heterocycloalkyl containing 1-4 heteroatoms selected from N, O, and S, optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or - Q⁴-T⁴ is oxo; and
n is 0, 1 or 2.

For example, the compounds of Formula (I) include those of Formula (VI): 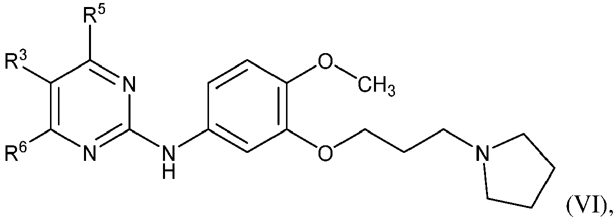 tautomers thereof, or pharmaceutically acceptable salts of the compounds or the tautomers, wherein R⁵ and R⁶ are independently selected from the group consisting of C₁-C₆ alkyl and NR⁸R⁹, or R⁶ and R³ together with the atoms to which they are attached form phenyl or a 5- or 6-membered heteroaryl.

For example, the compounds of Formula (I) include those of Formula (VII): 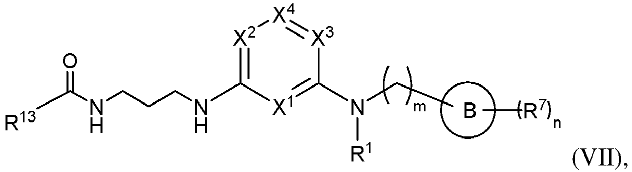 wherein m is 1 or 2 and n is 0, 1, or 2.

For example, the compounds of Formula (I) include those of Formula (Villa): 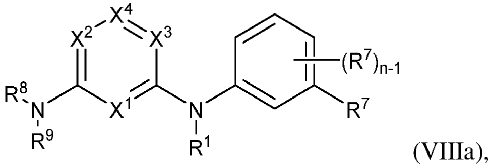 tautomers thereof, or pharmaceutically acceptable salts of the compounds or the tautomers, wherein
X¹ is N or CR²;
X² is Nor CR³;
X³ is N or CR⁴;
X⁴ is N or CR⁵;
R² is selected from the group consisting of H, C₃-C₈ cycloalkyl, and C₁-C₆ alkyl optionally substituted with one or more of halo, OR^{a}, or NR^{a}R^{b};
each of R³ and R⁴ is H; and
R⁵ are independently selected from the group consisting of H, C₃-C₈ cycloalkyl, and C₁-C₆ alkyl optionally substituted with one or more of halo or OR^{a}; or
R⁵ and one of R³ or R⁴ together with the atoms to which they are attached form phenyl or a 5- or 6-membered heteroaryl; or R⁵ and one of R³'or R⁴' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl or C₁-C₃ alkoxyl; and wherein at least one of R₂ or R₅ are not H.

For example, the compounds of Formula (I) include those of Formula (VIIIb): 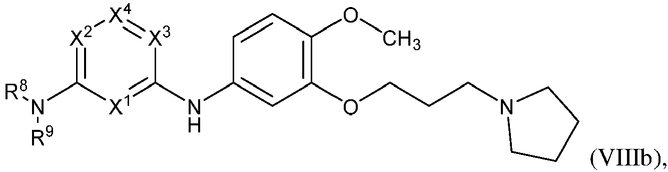 tautomers thereof, or pharmaceutically acceptable salts of the compounds or the tautomers, wherein
X¹ is N or CR²;
X² is Nor CR³;
X³ is N or CR⁴;
X⁴ is N or CR⁵;
R² is selected from the group consisting of H, C₃-C₈ cycloalkyl, and C₁-C₆ alkyl
each of R³ and R⁴ is H; and
R⁵ is selected from the group consisting of H, C₃-C₈ cycloalkyl, and C₁-C₆ alkyl; or
R⁵ and one of R³ or R⁴ together with the atoms to which they are attached form phenyl or a 5- or 6-membered heteroaryl; or R⁵ and one of R³'or R⁴' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl or C₁-C₃ alkoxyl; and wherein at least one of R₂ or R₅ are not H.

For example, the compounds of Formula (I) includes those of Formula (VIIIc): 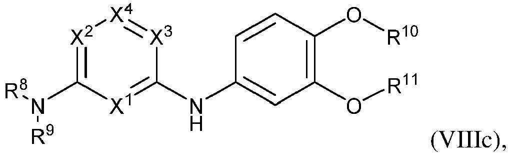 wherein
X¹ is N or CR²;
X² is Nor CR³;
X³ is N or CR⁴;
X⁴ is N or CR⁵;
R² is selected from the group consisting of H, C₃-C₈ cycloalkyl, and C₁-C₆ alkyl
each of R³ and R⁴ is H; and
R⁵ is selected from the group consisting of H, C₃-C₈ cycloalkyl, and C₁-C₆ alkyl; or
R⁵ and one of R³ or R⁴ together with the atoms to which they are attached form phenyl or a 5- or 6-membered heteroaryl; or R⁵ and one of R³'or R⁴' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl or C₁-C₃ alkoxyl; and wherein at least one of R₂ or R₅ are not H.

For example, at least one of X¹, X², X³ and X⁴ is N.

For example, X² and X³ is CH, and X¹ and X⁴ is N.

For example, X² and X³ is N, X¹ is CR², and X⁴ is CR⁵.

For example, R⁶ is NR⁸R⁹ and R⁵ is C₁₋₆ alkyl or R⁵ and R³ together with the atoms to which they are attached form phenyl or a 5- to 6-membered heteroaryl ring.

For example, both of X¹ and X³ are N while X² is CR³ and X⁴ is CR⁵.

Further, the compounds of any of Formulae (I)-(VIIIc) above can have one or more of the following features when applicable:

For example, R¹ is H.

For example, R¹ is CH₃.

For example, R² is selected from the group consisting of H, halo, cyano, C₁-C₄ alkoxyl, phenyl, NR^{a}R^{b}, C(O)NR^{a}R^{b}, NR^{a}C(O)R^{b}, and C₁-C₆ alkyl optionally substituted with one or more of halo, OR^{a} or NR^{a}R^{b}.

For example, R³ is selected from the group consisting of H, halo, cyano, C₁-C₄ alkoxyl, phenyl, NR^{a}R^{b}, C(O)NR^{a}R^{b}, NR^{a}C(O)R^{b}, and C₁-C₆ alkyl optionally substituted with one or more of halo, OR^{a} or NR^{a}R^{b}.

For example, R⁴ is selected from the group consisting of H, halo, cyano, C₁-C₄ alkoxyl, phenyl, NR^{a}R^{b}, C(O)NR^{a}R^{b}, NR^{a}C(O)R^{b}, and C₁-C₆ alkyl optionally substituted with one or more of halo, OR^{a} or NR^{a}R^{b}.

For example, R⁵ is selected from the group consisting of H, cyano, C₁-C₄ alkoxyl, phenyl, NR^{a}R^{b}, C(O)NR^{a}R^{b}, NR^{a}C(O)R^{b}, and C₁-C₆ alkyl optionally substituted with one or more of halo, OR^{a} or NR^{a}R^{b}.

For example, R⁵ is 4 to 12-membered heterocycloalkyl (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, and the like).

For example, each of R^{a} and R^{b} independently is H or C₁-C₄ alkyl.

For example, R⁶ is -T¹, in which T¹ is H, halo, cyano, NR⁸R⁹, C(O)NR⁸R⁹, OR⁸, OR⁹, or R^{S1}.

For example, R⁶ is -Q¹-T¹, in which Q¹ is a C₁-C₆ alkylene, C₂-C₆ alkenylene, or C₂-C₆ alkynylene linker optionally substituted with one or more of halo, cyano, hydroxyl, oxo and T¹ is H, halo, cyano, NR⁸R⁹, C(O)NR⁸R⁹, OR⁸, OR⁹, or R^{S1}.

For example, R^{S1} is C₃-C₈ cycloalkyl, phenyl, 4 to 12-membered heterocycloalkyl (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, and the like) or a 5- or 6-membered heteroaryl (e.g., pyrrole, furan, thiophene, thiazole, isothiazole, imidazole, triazole, tetrazole, pyrazole, oxazole, isoxazole, pyridine, pyrazine, pyridazine, pyrimidine, and the like), each of which is optionally substituted with one or more of halo, C₁-C₆ alkyl, hydroxyl, oxo, -C(O)R⁹, -SO₂R⁸, -SO₂N(R⁸)₂, -NR⁸C(O)R⁹, amino, mono- or di-alkylamino, or C₁-C₆ alkoxyl.

For example, R⁶ is NR⁸R⁹.

For example, R⁶ and one of R² or R³ together with the atoms to which they are attached form phenyl or a 5- or 6-membered heteroaryl (e.g., pyrrole, furan, thiophene, thiazole, isothiazole, imidazole, triazole, tetrazole, pyrazole, oxazole, isoxazole, pyridine, pyrazine, pyridazine, pyrimidine, and the like), in which the phenyl or 5- or 6-membered heteroaryl as formed is optionally substituted with one or more of halo, C₁-C₃ alkyl, hydroxyl, C₁-C₃ alkoxyl or -Q¹-T¹.

For example, R⁶ and one of R²' or R³' together with the atoms to which they are attached form a 5- or 6-membered heteroaryl (e.g., pyrrole, furan, thiophene, thiazole, isothiazole, imidazole, triazole, tetrazole, pyrazole, oxazole, isoxazole, pyridine, pyrazine, pyridazine, pyrimidine, and the like), in which the phenyl or 5- or 6-membered heteroaryl as formed is optionally substituted with one or more of halo, C₁-C₃ alkyl, hydroxyl, C₁-C₃ alkoxyl or -Q¹-T¹.

For example, n is 1 or 2, and at least one of R⁷ is -Q²-OR¹¹ in which R¹¹ is -Q⁶-R^{S3} and Q⁶ is optionally substituted C₂-C₆ alkylene, C₂-C₆ alkenylene, or C₂-C₆ alkynylene linker.

For example, n is 1 or 2, and at least one of R⁷ is -Q²-NR¹⁰R¹¹ in which R¹¹ is -Q⁶-R^{S3}.

For example, R¹¹ is -Q⁶-R^{S3}, in which Q⁶ is a bond or a C₁-C₆ alkylene, C₂-C₆ alkenylene, or C₂-C₆ alkynylene linker (e.g., C₂-C₆ alkylene linker) optionally substituted with a hydroxyl and R^{S3} is 4 to 12-membered heterocycloalkyl (e.g., a 4 to 7-membered monocyclic heterocycloalkyl or 7 to 12-membered bicyclic heterocycloalkyl such as azetidinyl, oxetanyl, thietanyl, pyrrolidinyl, imidazolidinyl, pyrazolidinyl, oxazolidinyl, isoxazolidinyl, triazolidinyl, tetrahyrofuranyl, piperidinyl, 1,2,3,6-tetrahydropyridinyl, piperazinyl, tetrahydro-2H-pyranyl, 3,6-dihydro-2H-pyranyl, tetrahydro-2H-thiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, 3-azabicyclo[3.1.0]hexanyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like), which is optionally substituted with one or more -Q⁷-T⁷.

For example, Q⁶ is C₁-C₆ alkylene, C₂-C₆ alkenylene, or C₂-C₆ alkynylene linker optionally substituted with a hydroxyl and R^{S3} is C₃-C₆ cycloalkyl optionally substituted with one or more -Q⁷-T⁷.

For example, each Q⁷ is independently a bond or a C₁-C₃ alkylene, C₂-C₃ alkenylene, or C₂-C₃ alkynylene linker and each T⁷ is independently H, halo, C₁-C₆ alkyl, or phenyl.

For example, -Q⁷-T⁷ is oxo.

For example, Q² is a bond or a C₁-C₄ alkylene, C₂-C₄ alkenylene, or C₂-C₄ alkynylene linker.

For example, at least one of R⁷ is

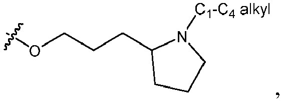

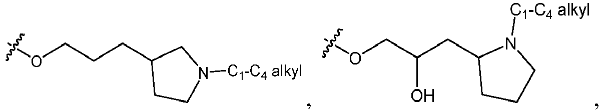

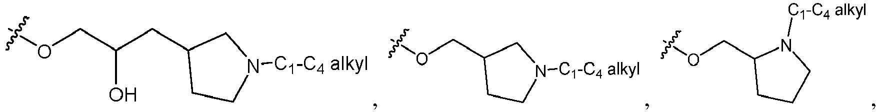

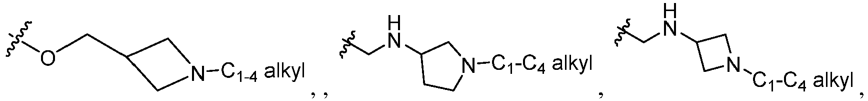

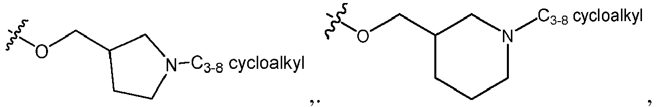

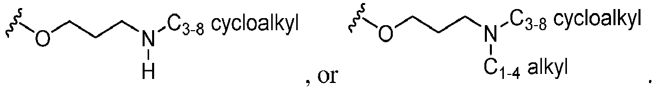

For example, at least one of R⁷ is

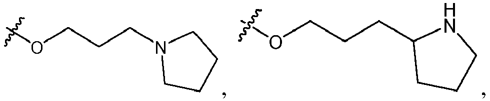

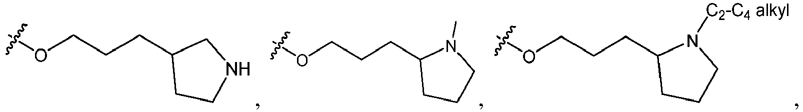

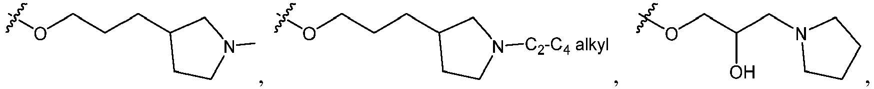

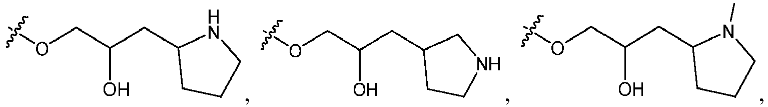

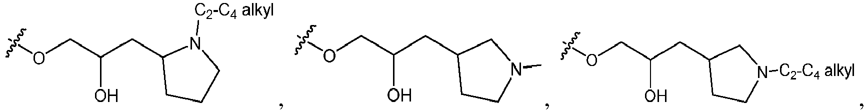

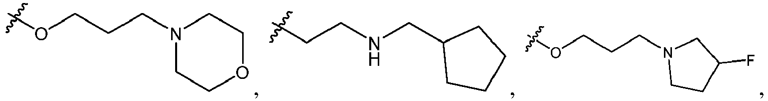

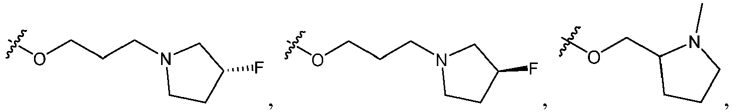

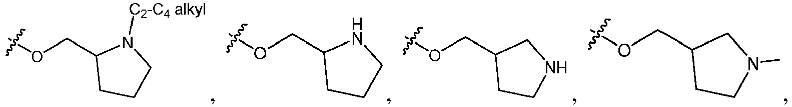

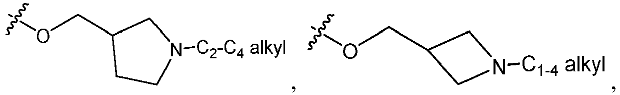

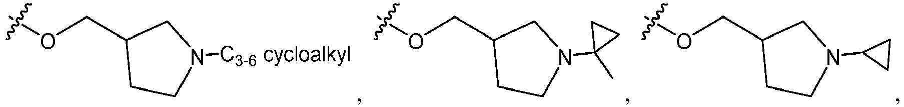

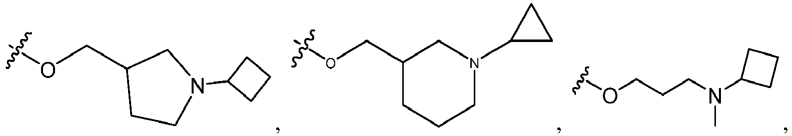

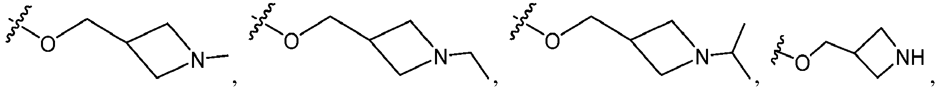

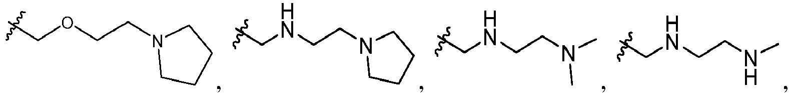

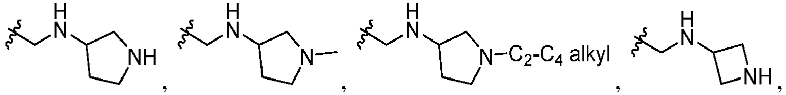

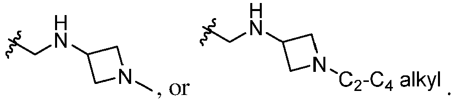

For example, n is 2 and the compound further comprises another R⁷ selected from halo and methoxy.

For example, ring B is selected from phenyl, pyridyl and cyclohexyl, and the halo or methoxy is at the para-position to NR¹.

For example, R⁶ is NR⁸R⁹, in which R⁸ is H or C₁-C₄ alkyl and R⁹ is -Q³-T³; or R⁸ and R⁹ taken together with the nitrogen atom to which they are attached form a 4 to 12-membered heterocycloalkyl (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, and the like) which is optionally substituted with one or more of -Q⁵T⁵.

For example, R⁹ is -Q³-T³, in which T³ is OR¹², NR¹²C(O)R¹³, C(O)R¹³, C(O)NR¹²R¹³, S(O)₂NR¹²R¹³, or R^{S2}.

For example, Q³ is C₁-C₆ alkylene, C₂-C₆ alkenylene, or C₂-C₆ alkynylene linker optionally substituted with a hydroxyl.

For example, R^{S2} is C₃-C₆ cycloalkyl, phenyl, 4 to 12-membered heterocycloalkyl (azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, and the like), or a 5 to 10-membered heteroaryl (e.g., triazolyl, pyridyl, pyrimidinyl, pyrazinyl, pyridazinyl, and triazinyl), and R^{S2} is optionally substituted with one or more -Q⁴-T⁴.

For example, each Q⁴ is independently a bond or C₁-C₃ alkylene, C₂-C₃ alkenylene, or C₂-C₃ alkynylene linker optionally substituted with one or more of hydroxyl and halo, and each T⁴ is independently H, halo, C₁-C₆ alkyl, or phenyl; or -Q⁴-T⁴ is oxo.

For example, R⁶ or NR⁸R⁹ is selected from the group consisting of:

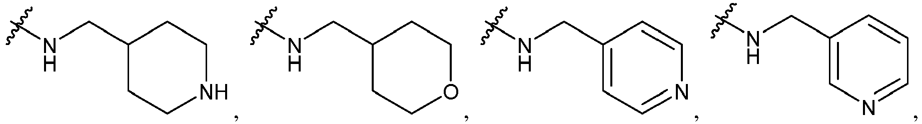

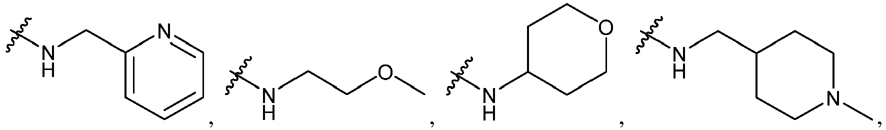

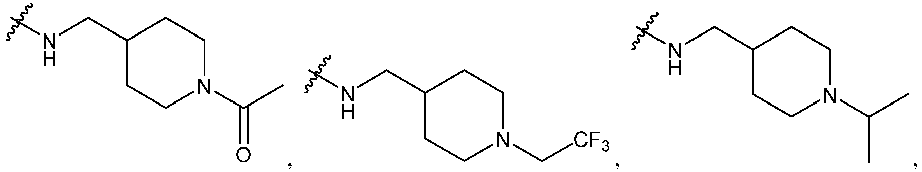

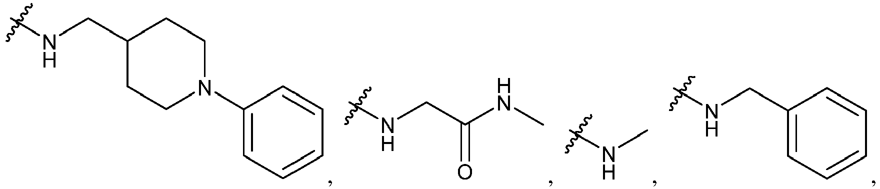

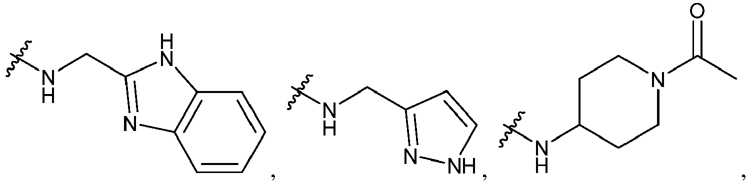

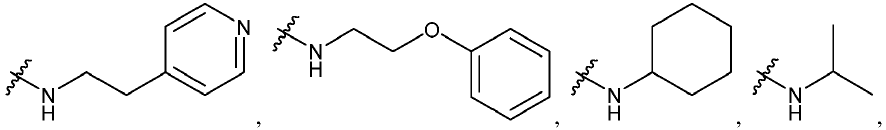

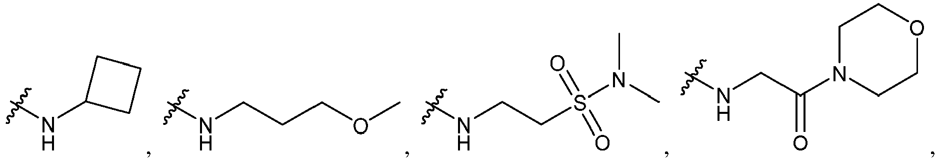

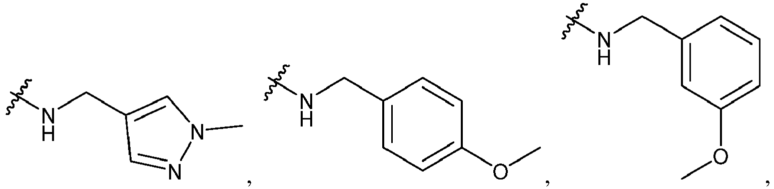

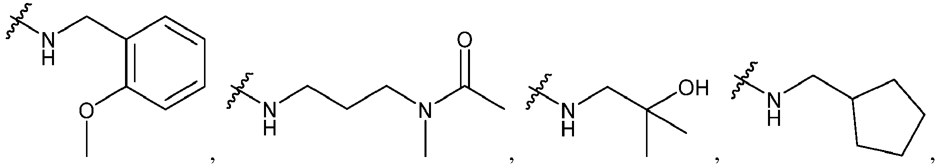

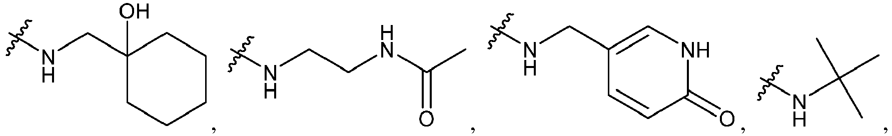

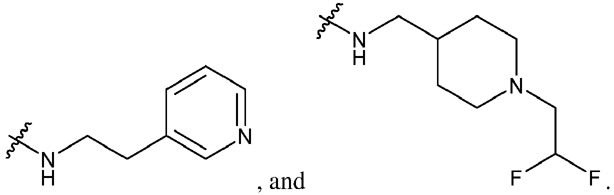

For example, R¹² is H.

For example, R¹² is C₁-C₆ alkyl.

For example, R¹³ is C₁-C₆ alkyl optionally substituted with one or more -Q⁸-T⁸.

For example, R¹³ is C₃-C₈ cycloalkyl optionally substituted with one or more -Q⁸-T⁸.

For example, R¹³ is C₆-C₁₀ aryl (e.g., phenyl) optionally substituted with one or more -Q⁸-T⁸.

For example, R¹³ is 4 to 12-membered heterocycloalkyl containing 1-4 heteroatoms selected from N, O and S (e.g., azetidinyl, pyrrolidinyl, imidazolidinyl, pyrazolidinyl, oxazolidinyl, isoxazolidinyl, triazolidinyl, piperidinyl, 1,2,3,6-tetrahydropyridinyl, piperazinyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, morpholinyl, 3-azabicyclo[3.1.0]hexan-3-yl, benzo[d][1,3]dioxol-5-yl, isoindolinyl, indolinyl, 2,3-dihydrobenzo[d]oxazolyl, and the like) optionally substituted with one or more -Q⁸-T⁸.

For example, R¹³ is 5 to 10-membered heteroaryl (e.g., triazolyl, pyridyl, pyrimidinyl, pyrazinyl, pyridazinyl, and triazinyl) optionally substituted with one or more -Q⁸-T⁸.

For example, Q⁸ is a bond.

For example, Q⁸ is a C₁-C₃ alkylene, C₂-C₃ alkenylene, or C₂-C₃ alkynylene linker.

For example, T⁸ is halo, cyano, C₁-C₆ alkyl, C₃-C₈ cycloalkyl, phenyl, or 4 to 7-membered heterocycloalkyl (e.g., e.g., azetidinyl, oxetanyl, thietanyl, pyrrolidinyl, imidazolidinyl, pyrazolidinyl, oxazolidinyl, isoxazolidinyl, triazolidinyl, tetrahyrofuranyl, piperidinyl, 1,2,3,6-tetrahydropyridinyl, piperazinyl, tetrahydro-2H-pyranyl, 3,6-dihydro-2H-pyranyl, tetrahydro-2H-thiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 3-azabicyclo[3.1.0]hexan-3-yl, and morpholinyl, and the like).

For example, -Q⁸-T⁸ is oxo.

The present disclosure also provides compounds of Formula (IX-1) below: 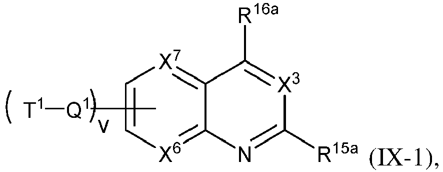 or a tautomer thereof, or a pharmaceutically acceptable salt of the compound or the tautomer, wherein,
X⁶ is N or CH;
X⁷ is N or CH;
X³ is N or CR⁴;
R⁴ is selected from the group consisting of H, halo, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 7- membered heterocycloalkyl, 5- to 6-membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl;
each Q¹ is independently a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl;
each T¹ is independently H, halo, cyano, NR⁸R⁹, C(O)NR⁸R⁹, C(O)R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, - SO₂R⁸, -SO₂N(R⁸)₂, -NR⁸C(O)R⁹, NR⁸R⁹, or C₁-C₆ alkoxyl; and -Q¹-T¹ is not NR⁸C(O)NR¹²R¹³;
each R⁸ independently is H or C₁-C₆ alkyl;
each R⁹ is independently -Q³-T³, in which Q³ is a bond or C₁-C₆ alkylene, C₂-C₆ alkenylene, or C₂-C₆ alkynylene linker optionally substituted with one or more of halo, cyano, hydroxyl, or C₁-C₆ alkoxyl, and T³ is H, halo, OR¹², OR¹³, NR¹²R¹³, NR¹²C(O)R¹³, C(O)NR¹²R¹³, C(O)R¹³, S(O)₂R¹³, S(O)₂NR¹²R¹³, or R^{S2}, in which R^{S2} is C₃-C₈ cycloalkyl, C₆-C₁₀ aryl, 4- to 12-membered heterocycloalkyl containing 1-4 heteroatoms selected from N, O, and S, or a 5- to 10-membered heteroaryl, and R^{S2} is optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or -Q⁴-T⁴ is oxo; or
R¹² is H or C₁-C₆ alkyl;
R¹³ is C₁-C₆ alkyl, C₃-C₈ cycloalkyl, C₆-C₁₀ aryl, 4- to 12-membered heterocycloalkyl containing 1-4 heteroatoms selected from N, O, and S, or a 5- to 10-membered heteroaryl, each of which is optionally substituted with one or more -Q⁸-T⁸, wherein each Q⁸ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁸ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or -Q⁸-T⁸ is oxo;
R^{15a} is CN, C(O)H, C(O)R¹⁸, OH, OR¹⁸, C₁-C₆ alkyl, NHR¹⁷, C₃-C₈ cycloalkyl, C₆-C₁₀ aryl, 4- to 12-membered heterocycloalkyl containing 1-4 heteroatoms selected from N, O, and S, or 5- to 10-membered heteroaryl, wherein each of said C₁-C₆ alkyl, C₃-C₈ cycloalkyl, C₆-C₁₀ aryl, 4- to 12-membered heterocycloalkyl, and 5- to 10-membered heteroaryl is optionally substituted with one or more -Q⁹-T⁹, wherein each Q⁹ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁹ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5-to 6-membered heteroaryl; or -Q⁹-T⁹ is oxo;
R^{16a} is -Q¹¹-R¹⁶ in which Q¹¹ is a bond, O, NR^{a}, C₁-C₃ alkylene, C₂-C₃ alkenylene, or C₂-C₃ alkynylene linker each optionally substituted with one or more of halo, cyano, hydroxyl, or C₁-C₆ alkoxy; and R¹⁶ is H, C₁-C₆ alkyl, C₂-C₆ alkenyl, C₂-C₆ alkynyl, C₃-C₈ cycloalkyl, C₆-C₁₀ aryl, 4- to 12-membered heterocycloalkyl containing 1-4 heteroatoms selected from N, O, and S, or a 5- to 10-membered heteroaryl, each of which is optionally substituted with one or more -Q¹⁰-T¹⁰, wherein each Q¹⁰ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T¹⁰ independently is selected from the group consisting of H, halo, cyano, C(O)H, C(O)R¹⁸, S(O)ₚR¹⁸, OH, OR¹⁸, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or -Q¹⁰-T¹⁰ is oxo;
R¹⁷ is H or C₁-C₆ alkyl;
each R¹⁸ is independently C₁-C₆ alkyl, C₂-C₆ alkenyl or C₂-C₆ alkynyl;
p is 0, 1, or 2; and
v is 0, 1, or 2.

For example, R^{15a} is CN or C(O)R¹⁸.

For example, R^{16a} is -Q¹¹-R¹⁶ in which Q¹¹ is a bond, NR^{a}, or C₁-C₃ alkylene linker optionally substituted with one or more of halo, cyano, hydroxyl, or C₁-C₆ alkoxy.

For example, each Q¹ is independently a bond or C₁-C₆ alkylene or C₂-C₆ alkynylene linker optionally substituted with one or more of halo, cyano, hydroxyl, or C₁-C₆ alkoxy.

For example, each T¹ is independently NR⁸R⁹, OR⁹, or R^{S1}, in which R^{S1} is optionally substituted C₃-C₈ cycloalkyl or optionally substituted 4- to 12-membered heterocycloalkyl.

For example, one subset of compounds of Formula (IX-1) is of Formula (IX): 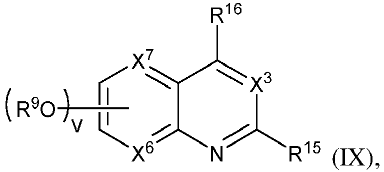 or a tautomer thereof, or a pharmaceutically acceptable salt of the compound or the tautomer, wherein
X⁶ is N or CH;
X⁷ is N or CH;
X³ is N or CR⁴;
R⁴ is selected from the group consisting of H, halo, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 7- membered heterocycloalkyl, 5- to 6-membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl;
each R⁹ is independently -Q³-T³, in which Q³ is a bond or C₁-C₆ alkylene, C₂-C₆ alkenylene, or C₂-C₆ alkynylene linker optionally substituted with one or more of halo, cyano, hydroxyl, or C₁-C₆ alkoxyl, and T³ is H, halo, OR¹², OR¹³, NR¹²R¹³, NR¹²C(O)R¹³, C(O)NR¹²R¹³, C(O)R¹³, S(O)₂R¹³, S(O)₂NR¹²R¹³, or R^{S2}, in which R^{S2} is C₃-C₈ cycloalkyl, C₆-C₁₀ aryl, 4- to 12-membered heterocycloalkyl containing 1-4 heteroatoms selected from N, O, and S, or a 5- to 10-membered heteroaryl, and R^{S2} is optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, NR^{c}R^{d}, C(O)NR^{c}R^{d}, and NR^{c}C(O)R^{d}, each of R^{c} and R^{d} independently being H or C₁-C₆ alkyl; or -Q⁴-T⁴ is oxo; or
R¹² is H or C₁-C₆ alkyl;
R¹³ is C₁-C₆ alkyl, C₃-C₈ cycloalkyl, C₆-C₁₀ aryl, 4- to 12-membered heterocycloalkyl containing 1-4 heteroatoms selected from N, O, and S, or a 5- to 10-membered heteroaryl, each of which is optionally substituted with one or more -Q⁸-T⁸, wherein each Q⁸ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁸ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or -Q⁸-T⁸ is oxo;
R¹⁵ is C₁-C₆ alkyl, NHR¹⁷, C₃-C₈ cycloalkyl, C₆-C₁₀ aryl, 4- to 12-membered heterocycloalkyl containing 1-4 heteroatoms selected from N, O, and S, or 5- to 10-membered heteroaryl, wherein each of said C₁-C₆ alkyl, C₃-C₈ cycloalkyl, C₆-C₁₀ aryl, 4- to 12-membered heterocycloalkyl, and 5- to 10-membered heteroaryl is optionally substituted with one or more -Q⁹-T⁹, wherein each Q⁹ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁹ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or -Q⁹-T⁹ is oxo;
R¹⁶ is C₁-C₆ alkyl, C₂-C₆ alkenyl, C₂-C₆ alkynyl, C₃-C₈ cycloalkyl, C₆-C₁₀ aryl, 4- to 12-membered heterocycloalkyl containing 1-4 heteroatoms selected from N, O, and S, or a 5-to 10-membered heteroaryl, each of which is optionally substituted with one or more -Q¹⁰-T¹⁰, wherein each Q¹⁰ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T¹⁰ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, and 5- to 6-membered heteroaryl; or - Q¹⁰-T¹⁰ is oxo;
R¹⁷ is H or C₁-C₆ alkyl; and
v is 0, 1, or 2.

The compounds of Formula (IX) can have one or more of the following features when applicable:
For example, each T³ independently is OR¹² or OR¹³.

For example, each Q³ independently is a bond or C₁-C₆ alkylene, C₂-C₆ alkenylene, or C₂-C₆ alkynylene linker optionally substituted with a hydroxyl.

For example, R¹⁵ is C₁-C₆ alkyl, NHR¹⁷, or 4- to 12-membered heterocycloalkyl.

For example, R¹⁶ is C₁-C₆ alkyl or 4- to 12-membered heterocycloalkyl, each optionally substituted with one or more -Q¹⁰-T¹⁰.

For example, each T¹⁰ independently is selected from the group consisting of H, halo, cyano, C₁-C₆ alkyl, and 4- to 7-membered heterocycloalkyl.

For example, each Q¹⁰ independently is a bond or C₁-C₃ alkylene, C₂-C₃ alkenylene, or C₂-C₃ alkynylene linker optionally substituted with a hydroxyl.

For example, the compounds of Formula (IX) include those of Formula (X): 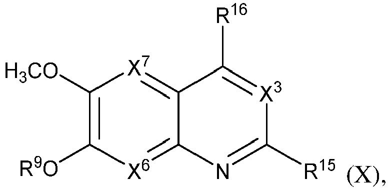 and tautomers thereof, or pharmaceutically acceptable salts of the compounds or the tautomers, wherein X³ is N or CR⁴, wherein R⁴ is selected from the group consisting of H, halo, and cyano.

For example, the compounds of Formula (X) include those of Formula (Xa), (Xb), (Xc), (Xd), (Xe), (Xf), or (Xg): 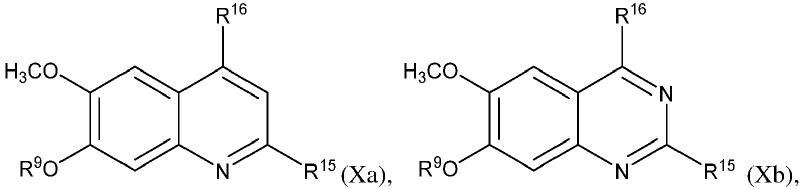 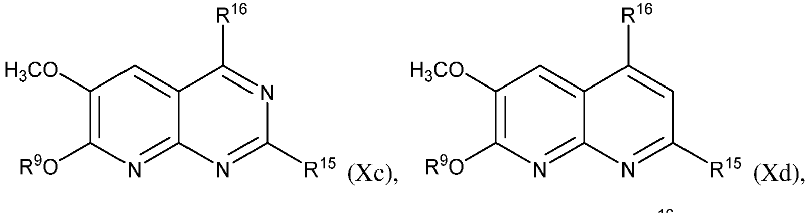 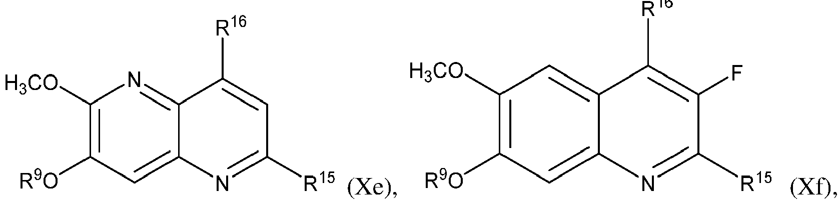 or

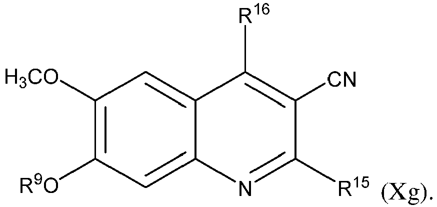

For example, X² and X³ are CH, and X¹ and X⁴ is N.

For example, X² and X³ are N, X¹ is CR², and X⁴ is CR⁵.

For example, R⁶ is NR⁸R⁹ and R⁵ is C₁₋₆ alkyl or R⁵ and R³ together with the atoms to which they are attached form phenyl or a 5- to 6-membered heteroaryl ring.

For example, the compound is selected from those in Tables 1-5, tautomers thereof, and pharmaceutically acceptable salts of the compounds and tautomers.

The present disclosure provides compounds which inhibit a kinase with an enzyme inhibition IC₅₀ value of about 100 nM or greater, 1 µM or greater, 10 µM or greater, 100 µM or greater, or 1000 µM or greater.

The present disclosure provides compounds which inhibit a kinase with an enzyme inhibition IC₅₀ value of about 1 mM or greater.

The present disclosure provides compounds which inhibit a kinase with an enzyme inhibition IC₅₀ value of 1 µM or greater, 2 µM or greater, 5 µM or greater, or 10 µM or greater, wherein the kinase is one or more of the following: AbI, AurA, CHK1, MAP4K, IRAK4, JAK3, EphA2, FGFR3, KDR, Lck, MARK1, MNK2, PKCb2, SIK, and Src.

The present disclosure provides a pharmaceutical composition comprising a compound of any one of the Formulae described herein or a pharmaceutically acceptable salt thereof, and a pharmaceutically acceptable carrier.

The present disclosure provides compounds for use in a method of preventing or treating a blood disorder via inhibition of a methyltransferase enzyme selected from EHMT1 and EHMT2, the method comprising administering to a subject in need thereof a therapeutically effective amount of a compound of Formula (I): 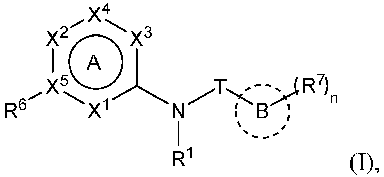 or a tautomer thereof, or a pharmaceutically acceptable salt of the compound or the tautomer, wherein
ring A is phenyl or a 5- or 6-membered heteroaryl;
X¹ is N, CR², or NR²' as valency permits;
X² is N, CR³, or NR³' as valency permits;
X³ is N, CR⁴, or NR⁴' as valency permits;
X⁴ is N or CR⁵, or X⁴ is absent such that ring A is a 5-membered heteroaryl containing at least one N atom;
X⁵ is C or N as valency permits;
B is absent or a ring structure selected from the group consisting of C₆-C₁₀ aryl, C₃-C₁₀ cycloalkyl, 5- to 10-membered heteroaryl, and 4- to 12-membered heterocycloalkyl containing 1-4 heteroatoms selected from N, O, and S;
T is a bond or C₁-C₆ alkylene, C₂-C₆ alkenylene, or C₂-C₆ alkynylene linker optionally substituted with one or more of halo, cyano, hydroxyl, oxo; or C₁-C₆ alkoxy when B is present; or T is H and n is 0 when B is absent; or T is C₁-C₆ alkyl optionally substituted with (R⁷)ₙ when B is absent; or when B is absent, T and R¹ together with the atoms to which they are attached optionally form a 4-7 membered heterocycloalkyl or 5-6 membered heteroaryl, each of which is optionally substituted with (R⁷)ₙ;
R¹ is H or C₁-C₄ alkyl;
each of R²', R³' and R⁴' independently is H or C₁-C₃ alkyl;
each of R², R³, and R⁴, independently is selected from the group consisting of H, halo, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4-7 membered heterocycloalkyl, 5-6 membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl, or R³ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, amino, mono- or di-alkylamino, or C₁-C₆ alkoxyl; or when ring A is a 5-membered heteroaryl containing at least one N atom, R⁴ is a spiro-fused 4- to 12-membered heterocycloalkyl containing 1-4 heteroatoms selected from N, O, and S;
R⁵ is selected from the group consisting of H, halo, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 12-membered heterocycloalkyl containing 1-4 heteroatoms selected from N, O, and S, optionally substituted with one or more of -C(O)C₁-C₆ alkyl or C₁-C₆ alkyl optionally substituted with one or more of halo or OR^{a}, C₁-C₆ alkyl optionally substituted with one or more of halo, OR^{a}, or NR^{a}R^{b}, and C₂-C₆ alkynyl optionally substituted with 4- to 12-membered heterocycloalkyl; wherein said C₃-C₈ cycloalkyl and 4- to 12-membered heterocycloalkyl are optionally substituted with one or more of halo, C(O)R^{a}, OR^{a}, NR^{a}R^{b}, 4- to 7-membered heterocycloalkyl, -C₁-C₆ alkylene-4- to 7-membered heterocycloalkyl, or C₁-C₄ alkyl optionally substituted with one or more of halo, OR^{a} or NR^{a}R^{b}, in which each of R^{a} and R^{b} independently is H or C₁-C₆ alkyl; or
R⁵ and one of R³ or R⁴ together with the atoms to which they are attached form phenyl or a 5- or 6-membered heteroaryl; or R⁵ and one of R³'or R⁴' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl or C₁-C₃ alkoxyl;
R⁶ is absent when X⁵ is N and ring A is a 6-membered heteroaryl; or R⁶ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, C(O)R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, NR⁸R⁹, or C₁-C₆ alkoxyl; and R⁶ is not NR⁸C(O)NR¹²R¹³; or
R⁶ and one of R² or R³ together with the atoms to which they are attached form phenyl or a 5- or 6-membered heteroaryl; or R⁶ and one of R²' or R³' together with the atoms to which they are attached form a 5- or 6-membered heteroaryl, in which the phenyl or 5- or 6-membered heteroaryl as formed is optionally substituted with one or more of halo, C₁-C₃ alkyl, hydroxyl, oxo (=O), C₁-C₃ alkoxyl or -Q¹-T¹;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, cyano, OR¹⁰, OR¹¹, C(O)R¹¹, NR¹⁰R¹¹, C(O)NR¹⁰R¹¹, NR¹⁰C(O)R¹¹, 5- to 10-membered heteroaryl, C₃-C₈ cycloalkyl, or 4- to 12-membered heterocycloalkyl containing 1-4 heteroatoms selected from N, O, and S, and wherein the 5- to 10-membered heteroaryl, C₃-C₈ cycloalkyl, or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R₇ is not H or C(O)OR^{g}; or optionally, when B is present, one R⁷ and R⁵ together form a C₃-C₁₀ alkylene, C₂-C₁₀ heteroalkylene, C₄-C₁₀ alkenylene, C₂-C₁₀ heteroalkenylene, C₄-C₁₀ alkynylene or C₂-C₁₀ heteroalkynylene linker optionally substituted with one or more of halo, cyano, hydroxyl, or C₁-C₆ alkoxyl;
each R⁸ independently is H or C₁-C₆ alkyl;
each R⁹ is independently -Q³-T³, in which Q³ is a bond or C₁-C₆ alkylene, C₂-C₆ alkenylene, or C₂-C₆ alkynylene linker optionally substituted with one or more of halo, cyano, hydroxyl or C₁-C₆ alkoxyl, and T³ is H, halo, OR¹², OR¹³, NR¹²R¹³, NR¹²C(O)R¹³, C(O)NR¹²R¹³, C(O)R¹³, S(O)₂R¹³, S(O)₂NR¹²R¹³, or R^{S2}, in which R^{S2} is C₃-C₈ cycloalkyl, C₆-C₁₀ aryl, 4- to 12-membered heterocycloalkyl containing 1-4 heteroatoms selected from N, O, and S, or a 5- to 10-membered heteroaryl, and R^{S2} is optionally substituted with one or more -Q⁴-T⁴, wherein each Q⁴ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁴ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{c}, C(O)R^{c}, S(O)₂R^{c}, S(O)₂NR^{c}R^{d}, NR^{c}R^{d}, C(O)NR^{c}R^{d}, and NR^{c}C(O)R^{d}, each of R^{c} and R^{d} independently being H or C₁-C₆ alkyl; or -Q⁴-T⁴ is oxo; or
R⁸ and R⁹ taken together with the nitrogen atom to which they are attached form a 4-to 12-membered heterocycloalkyl containing 1-4 heteroatoms selected from N, O, and S, which is optionally substituted with one or more of -Q⁵-T⁵, wherein each Q⁵ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁵ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{e}, C(O)R^{e}, S(O)₂R^{e}, S(O)₂NR^{e}R^{f}, NR^{e}R^{f}, C(O)NR^{e}R^{f}, and NR^{e}C(O)R^{f}, each of R^{e} and R^{f} independently being H or C₁-C₆ alkyl; or -Q⁵-T⁵ is oxo;
R¹⁰ is selected from the group consisting of H and C₁-C₆ alkyl;
R¹¹ is -Q⁶-T⁶, in which Q⁶ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T⁶ is H, halo, OR^{g}, NR^{g}R^{h}, NR^{g}C(O)R^{h}, C(O)NR^{g}R^{h}, C(O)R^{g}, S(O)₂R^{g}, or R^{S3}, in which each of R^{g} and R^{h} independently is H, phenyl, C₃-C₈ cycloalkyl, or C₁-C₆ alkyl optionally substituted with C₃-C₈ cycloalkyl, or R^{g} and R^{h} together with the nitrogen atom to which they are attached form a 4- to 12-membered heterocycloalkyl containing 1-4 heteroatoms selected from N, O, and S, and R^{S3} is C₃-C₈ cycloalkyl, C₆-C₁₀ aryl, 4- to 12-membered heterocycloalkyl containing 1-4 heteroatoms selected from N, O and S, or a 5- to 10-membered heteroaryl, and R^{S3} is optionally substituted with one or more -Q⁷-T⁷, wherein each Q⁷ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁷ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{j}, C(O)R^{j}, NR^{j}R^{k}, C(O)NR^{j}R^{k}, S(O)₂R^{j}, and NR^{j}C(O)R^{k}, each of R^{j} and R^{k} independently being H or C₁-C₆ alkyl optionally substituted with one or more halo; or -Q⁷-T⁷ is oxo; or
R¹⁰ and R¹¹ taken together with the nitrogen atom to which they are attached form a 4-to 12-membered heterocycloalkyl containing 1-4 heteroatoms selected from N, O and S, which is optionally substituted with one or more of halo, C₁-C₆ alkyl, hydroxyl, or C₁-C₆ alkoxyl;
R¹² is H or C₁-C₆ alkyl;
R¹³ is C₁-C₆ alkyl, C₃-C₈ cycloalkyl, C₆-C₁₀ aryl, 4- to 12-membered heterocycloalkyl containing 1-4 heteroatoms selected from N, O, and S, or a 5- to 10-membered heteroaryl, each of which is optionally substituted with one or more -Q⁸-T⁸, wherein each Q⁸ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁸ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O and S, and 5- to 6-membered heteroaryl; or -Q⁸-T⁸ is oxo; and
n is 0, 1, 2, 3, or 4, provided that
   (1) the compound of Formula (I) is not 2-(hexahydro-4-methyl-1H-1,4-diazepin-1-yl)-6,7-dimethoxy-N-[1-(phenylmethyl)-4-piperidinyl]-4-quinazolinamine, or 2-cyclohexyl-6-methoxy-N-[1-(1-methylethyl)-4-piperidinyl]-7-[3-(1-pyrrolidinyl)propoxy]-4-quinazolinamine;
   (2) when X¹ and X³ are N, X² is CR³, X⁴ is CR⁵, X⁵ is C, R⁵ is 4- to 12-membered heterocycloalkyl substituted with one or more C₁-C₆ alkyl, and R⁶ and R³ together with the atoms to which they are attached form phenyl which is substituted with one or more of optionally substituted C₁-C₃ alkoxyl, then B is absent, C₆-C₁₀ aryl, C₃-C₁₀ cycloalkyl, or 5 to 10-membered heteroaryl; or
   (3) when X² and X³ are N, X¹ is CR², X⁴ is CR⁵, X⁵ is C, R⁵ is C₃-C₈ cycloalkyl or 4-to 12-membered heterocycloalkyl, each optionally substituted with one or more C₁-C₆ alkyl, and R⁶ and R² together with the atoms to which they are attached form phenyl which is substituted with one or more of optionally substituted C₁-C₃ alkoxyl, then B is absent, C₆-C₁₀ aryl, C₃-C₁₀ cycloalkyl, or 5- to 10-membered heteroaryl.

The present disclosure also provides compounds for use in a method of preventing or treating a blood disorder via inhibition of a methyltransferase enzyme selected from EHMT1 and EHMT2, the method comprising administering to a subject in need thereof a therapeutically effective amount of a compound disclosed herein, e.g., any compound of any of Formulae (I)-(Xg).

For example, the blood disorder is sickle cell anemia or β-thalassemia.

For example, the blood disorder is a hematological cancer.

For example, the hematological cancer is acute myeloid leukemia (AML) or chronic lymphocytic leukemia (CLL).

The present disclosure also provides compounds of Formula (I) which are selective inhibitors of EHMT2.

Representative compounds of the present disclosure include compounds listed in Tables 1-5 or tautomers and salts thereof.

**Table 1**

| **Compound No.** | **Structure** |
|---|---|
| 1 | 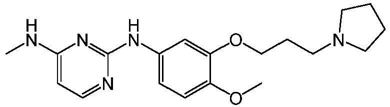 |
| 2 | 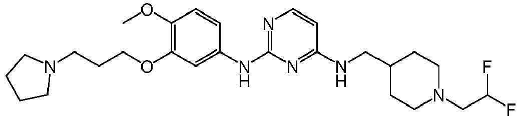 |
| 3 | 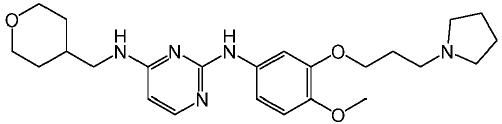 |
| 4 | 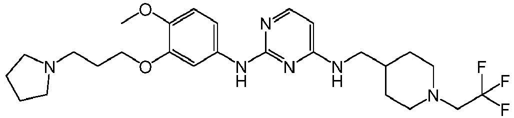 |
| 5 | 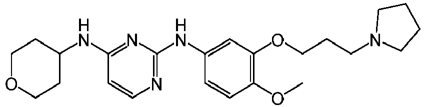 |
| 6 | 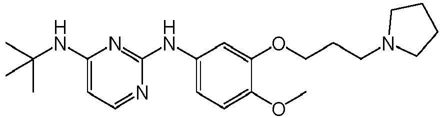 |
| 7 | 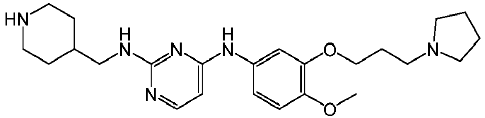 |
| 8 | 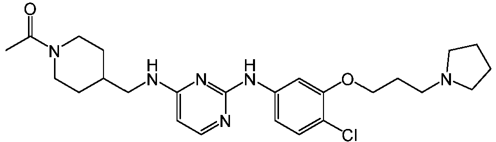 |
| 9 | 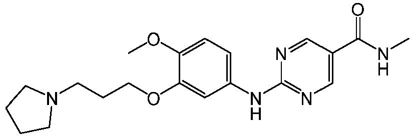 |
| 10 | 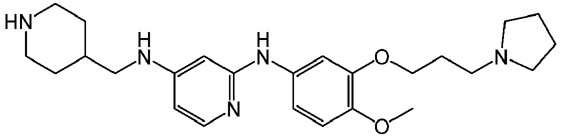 |
| 11 | 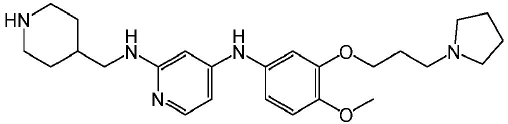 |
| 12 | 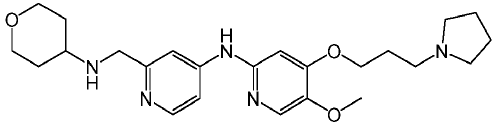 |
| 13 | 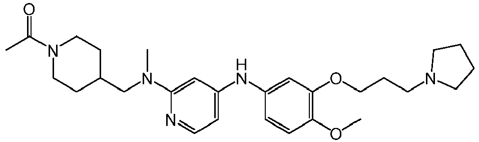 |
| 14 | 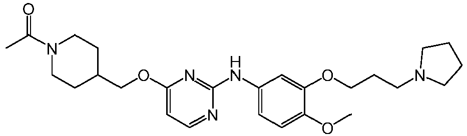 |
| 15 | 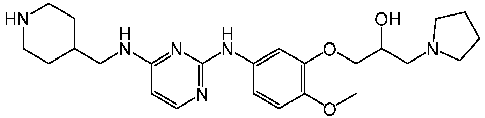 |
| 16 | 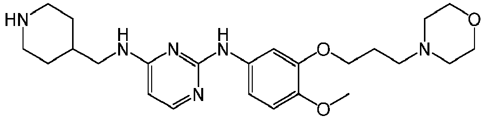 |
| 17 | 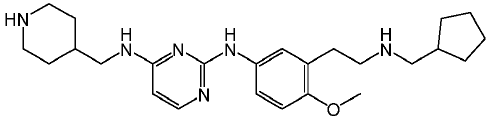 |
| 18 | 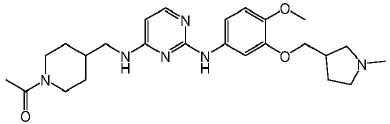 |
| 19 | 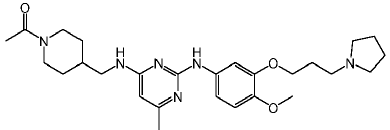 |
| 20 | 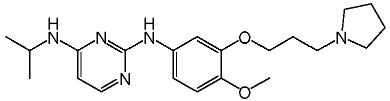 |
| 21 | 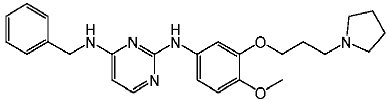 |
| 22 | 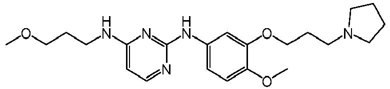 |
| 23 | 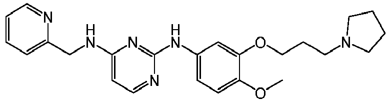 |
| 24 | 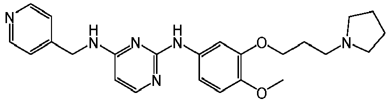 |
| 25 | 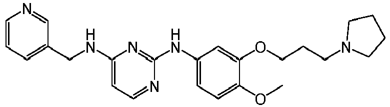 |
| 26 | 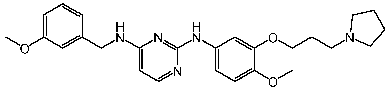 |
| 27 | 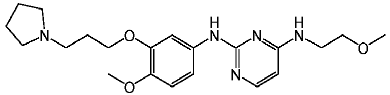 |
| 28 | 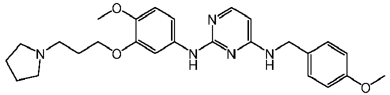 |
| 29 | 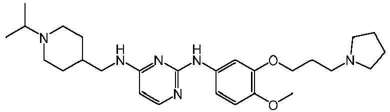 |
| 30 | 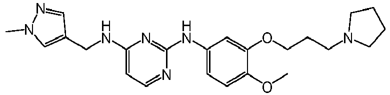 |
| 31 | 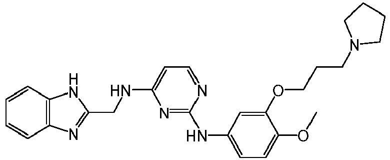 |
| 32 | 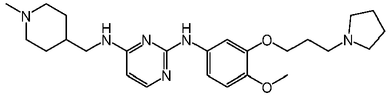 |
| 33 | 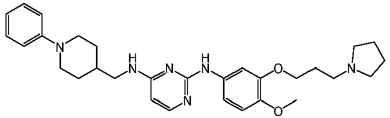 |
| 34 | 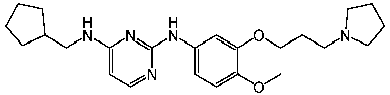 |
| 35 | 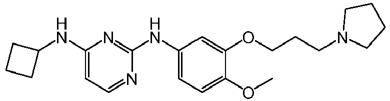 |
| 36 | 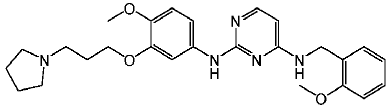 |
| 37 | 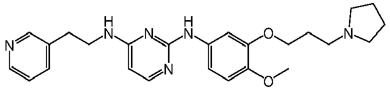 |
| 38 | 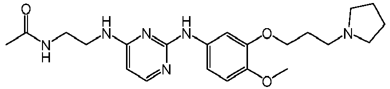 |
| 39 | 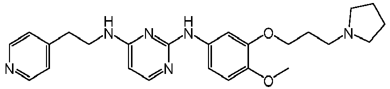 |
| 40 | 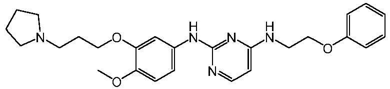 |
| 41 | 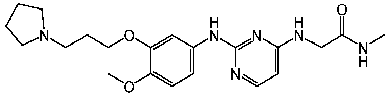 |
| 42 | 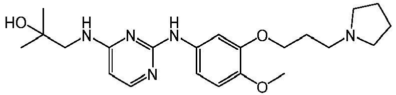 |
| 43 | 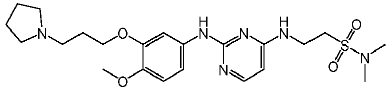 |
| 44 | 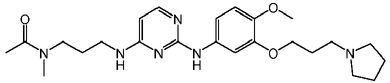 |
| 45 | 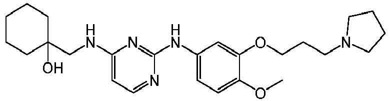 |
| 46 | 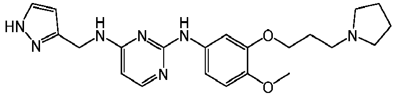 |
| 47 | 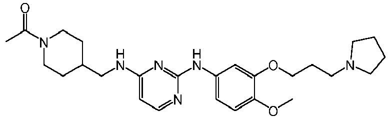 |
| 48 | 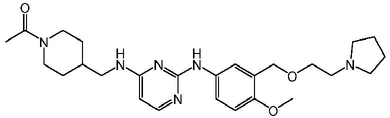 |
| 49 | 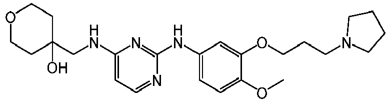 |
| 50 | 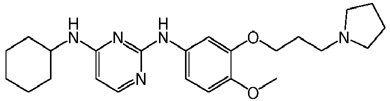 |
| 51 | 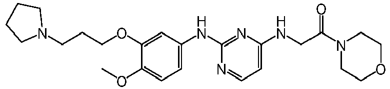 |
| 52 | 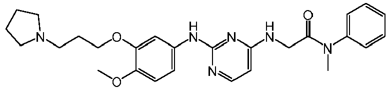 |
| 53 | 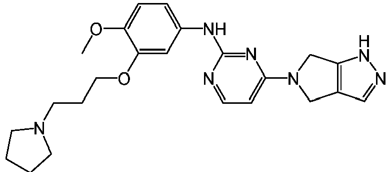 |
| 54 | 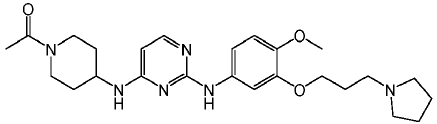 |
| 55 | 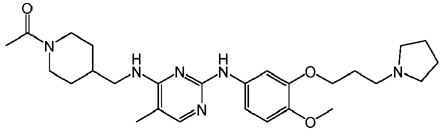 |
| 56 | 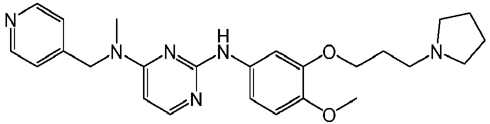 |
| 57 | 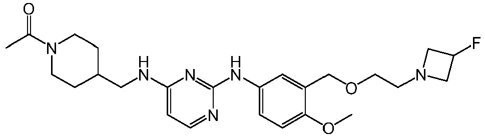 |
| 58 | 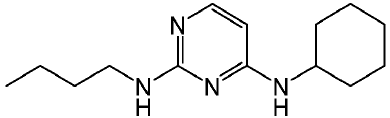 |
| 59 | 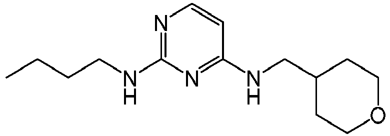 |
| 60 | 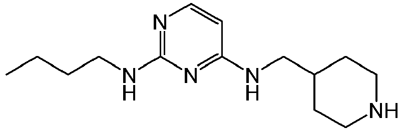 |
| 61 | 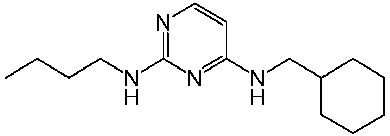 |
| 62 | 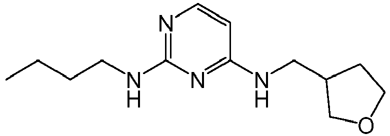 |
| 63 | 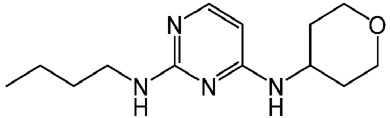 |
| 64 | 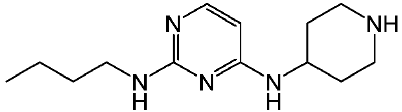 |
| 65 | 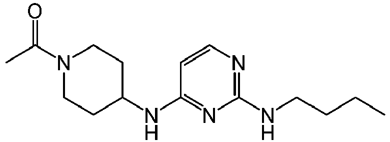 |
| 66 | 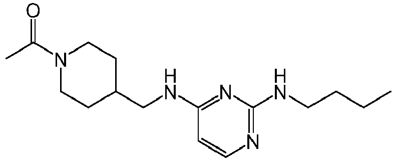 |
| 67 | 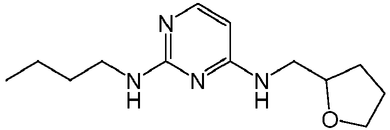 |
| 68 | 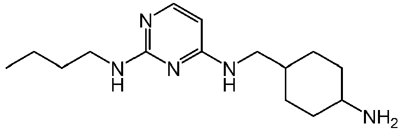 |
| 69 | 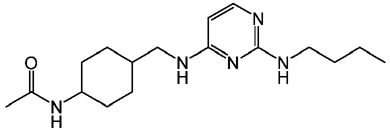 |
| 70 | 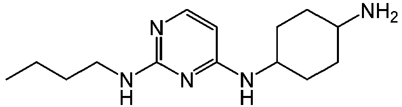 |
| 71 | 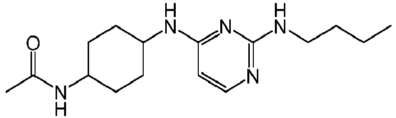 |
| 72 | 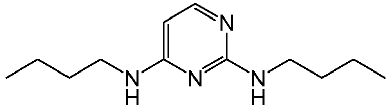 |
| 73 | 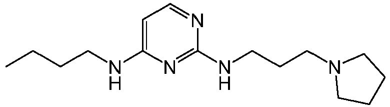 |
| 74 | 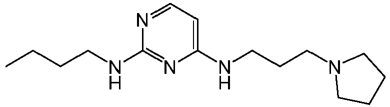 |
| 75 | 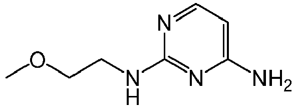 |
| 76 | 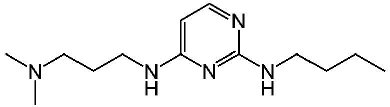 |
| 77 | 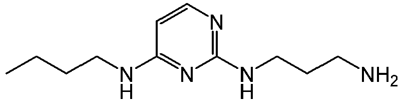 |
| 78 | 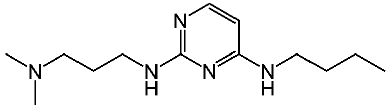 |
| 79 | 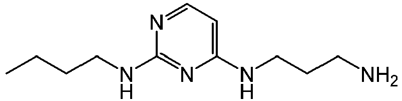 |
| 80 | 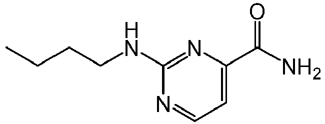 |
| 81 | 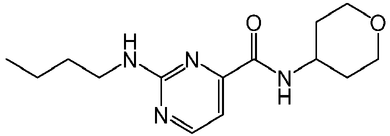 |
| 82 | 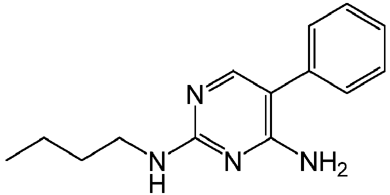 |
| 83 | 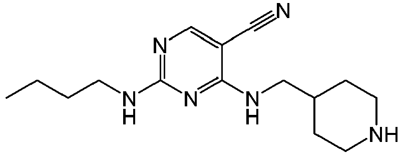 |
| 84 | 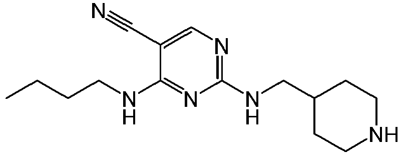 |
| 85 | 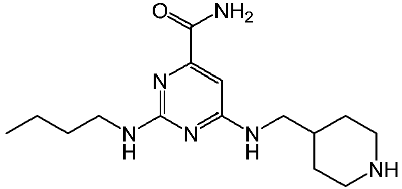 |
| 86 | 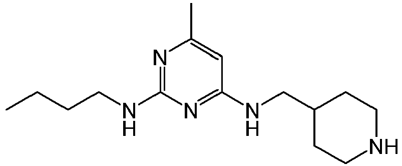 |
| 87 | 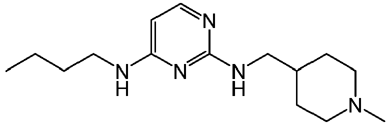 |
| 88 | 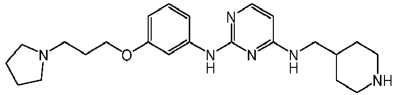 |
| 89 | 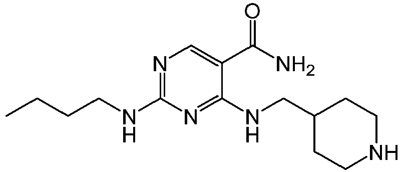 |
| 90 | 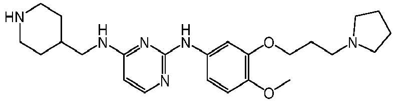 |
| 91 | 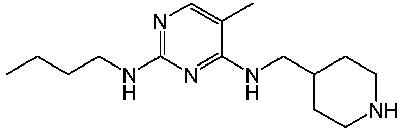 |
| 92 | 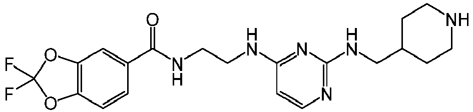 |
| 93 | 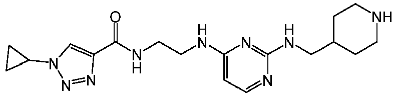 |
| 94 | 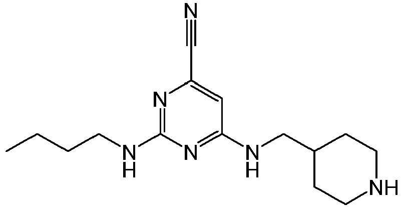 |
| 95 | 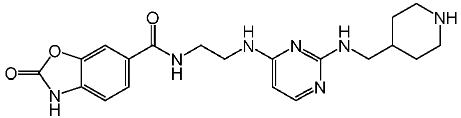 |
| 96 | 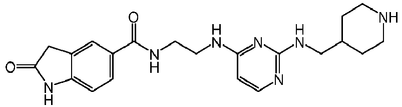 |
| 97 | 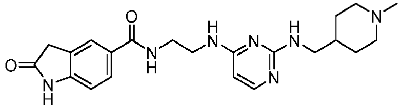 |
| 98 | 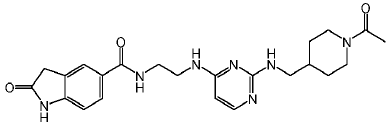 |
| 99 | 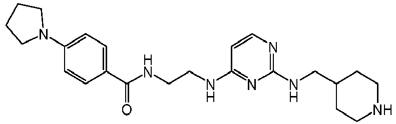 |
| 100 | 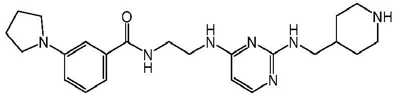 |
| 101 | 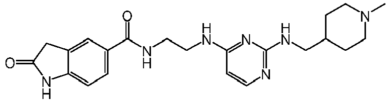 |
| 102 | 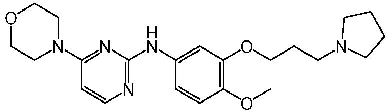 |
| 103 | 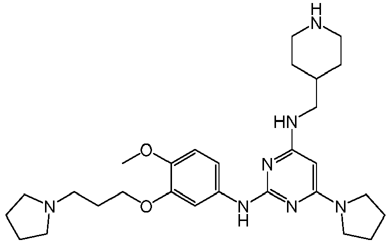 |
| 104 | 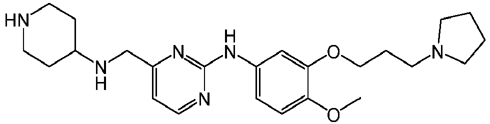 |
| 105 | 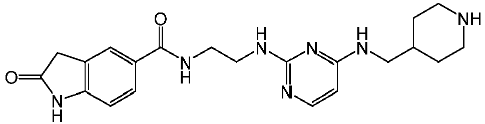 |
| 106 | 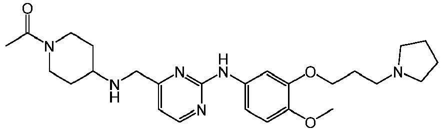 |
| 107 | 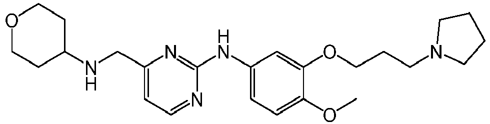 |
| 108 | 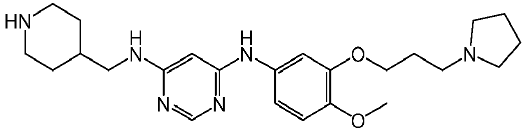 |
| 109 | 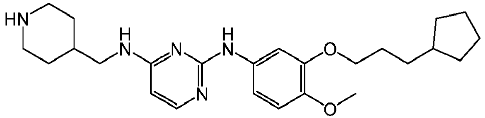 |
| 110 | 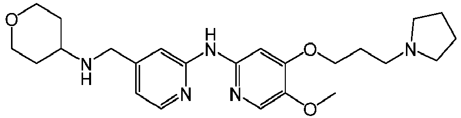 |
| 111 | 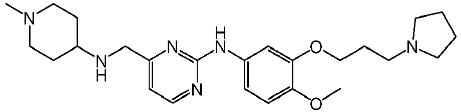 |
| 112 | 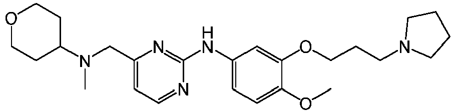 |
| 113 | 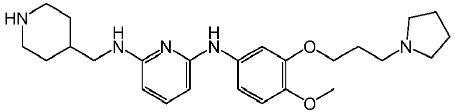 |
| 114 | 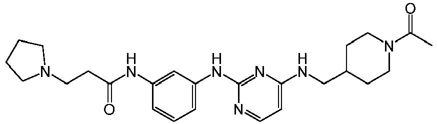 |
| 115 | 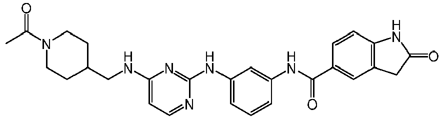 |
| 116 | 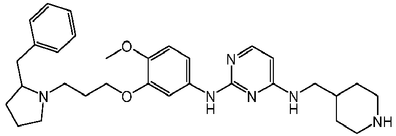 |
| 117 | 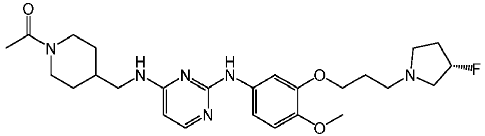 |
| 118 | 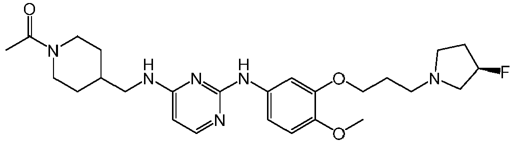 |
| 119 | 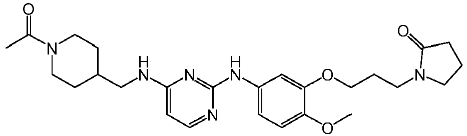 |
| 120 | 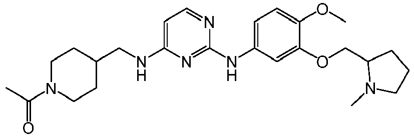 |
| 121 | 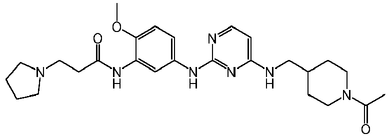 |
| 122 | 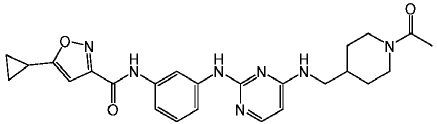 |
| 123 | 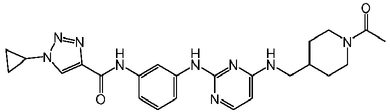 |
| 124 | 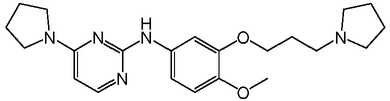 |
| 125 | 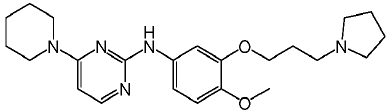 |
| 126 | 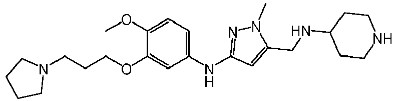 |
| 127 | 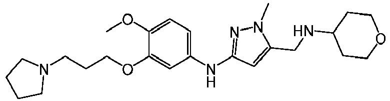 |
| 128 | 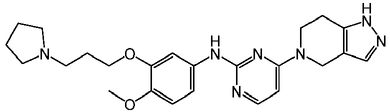 |
| 129 | 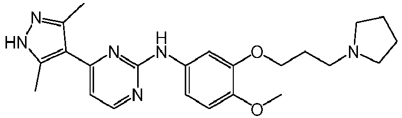 |
| 130 | 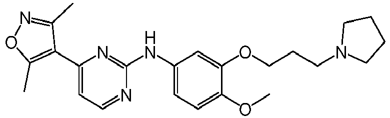 |
| 131 | 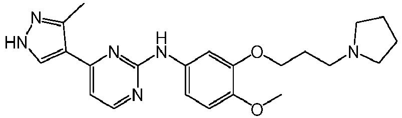 |
| 132 | 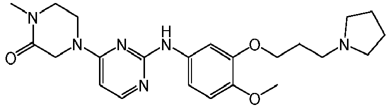 |
| 133 | 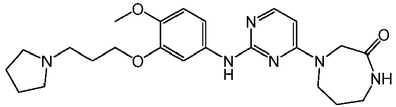 |
| 134 | 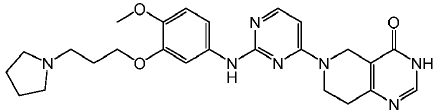 |
| 135 | 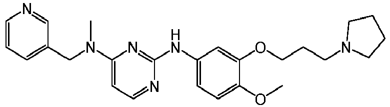 |
| 136 | 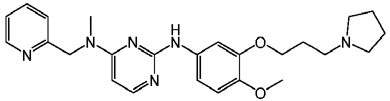 |
| 137 | 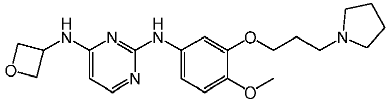 |
| 138 | 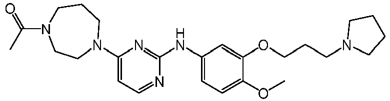 |
| 139 | 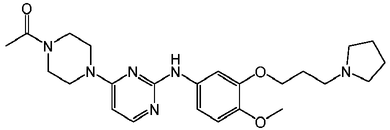 |
| 140 | 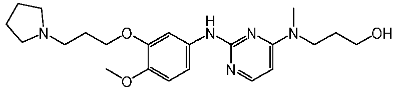 |
| 141 | 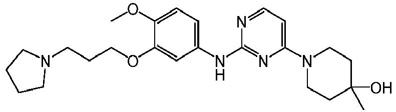 |
| 142 | 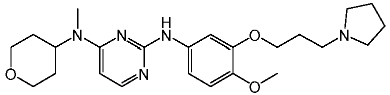 |
| 143 | 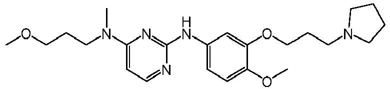 |
| 144 | 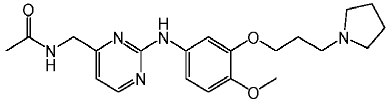 |
| 145 | 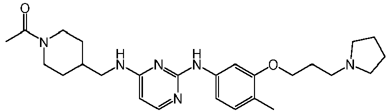 |
| 146 | 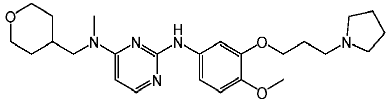 |
| 147 | 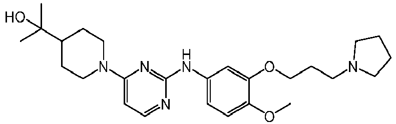 |
| 148 | 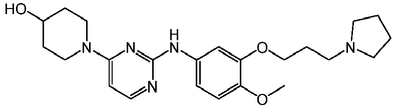 |
| 149 | 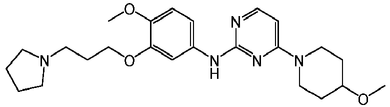 |
| 150 | 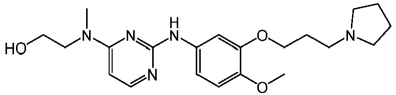 |
| 151 | 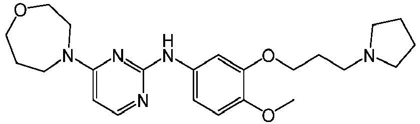 |
| 152 | 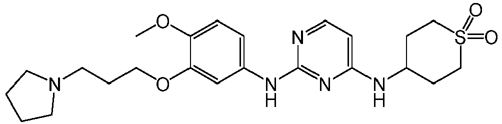 |
| 153 | 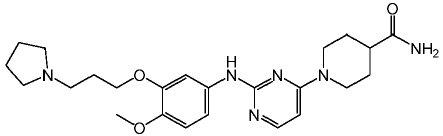 |
| 154 | 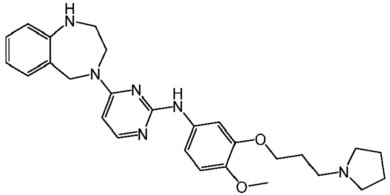 |
| 155 | 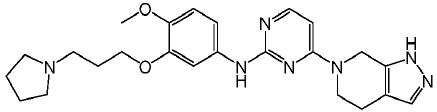 |
| 156 | 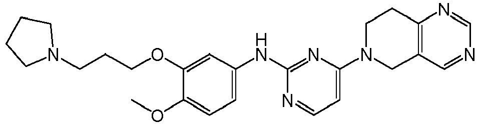 |
| 157 | 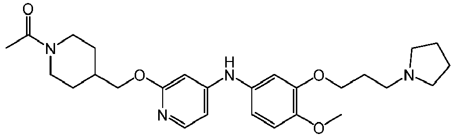 |
| 158 | 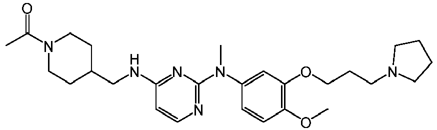 |
| 159 | 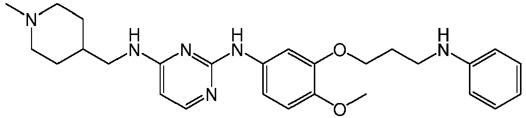 |
| 160 | 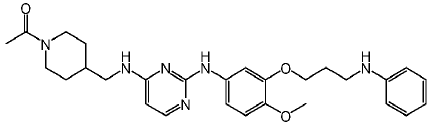 |
| 161 | 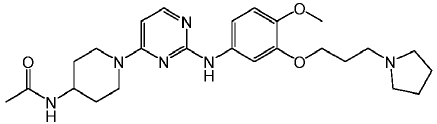 |
| 162 | 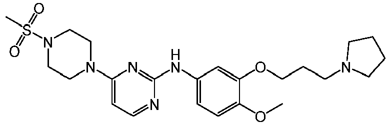 |
| 163 | 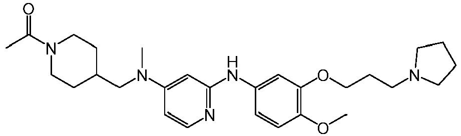 |
| 164 | 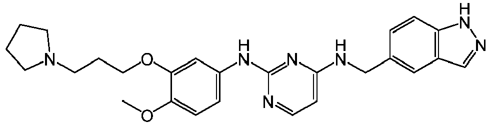 |
| 165 | 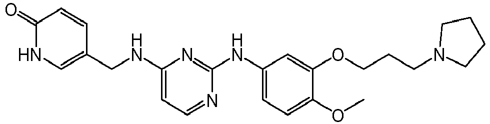 |
| 166 | 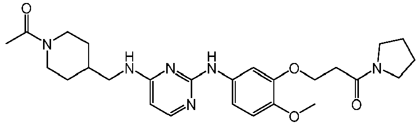 |
| 167 | 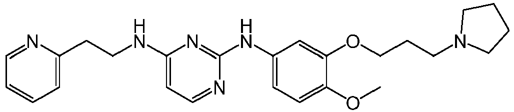 |
| 168 | 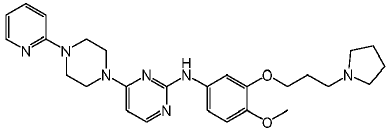 |
| 169 | 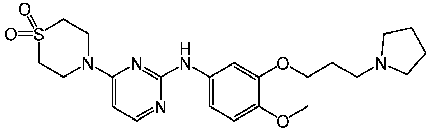 |
| 170 | 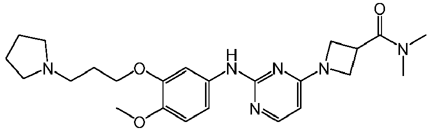 |
| 171 | 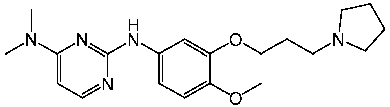 |
| 172 | 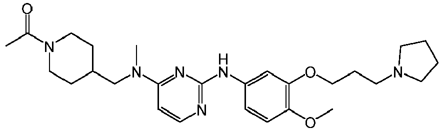 |
| 173 | 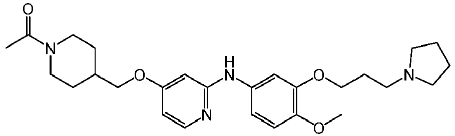 |
| 174 | 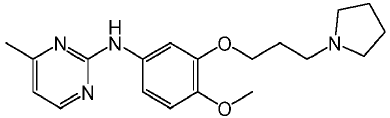 |
| 175 | 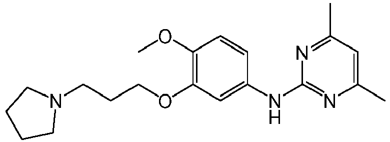 |
| 176 | 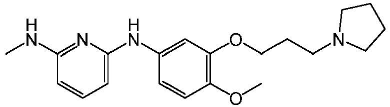 |
| 177 | 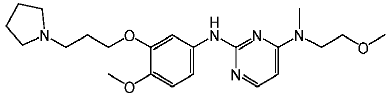 |
| 178 | 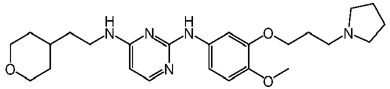 |
| 179 | 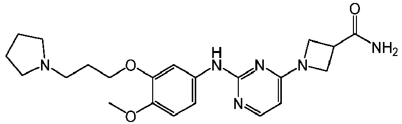 |
| 180 | 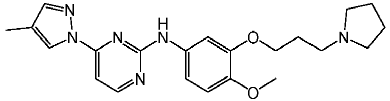 |
| 181 | 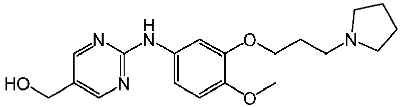 |
| 182 | 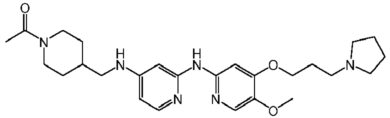 |
| 183 | 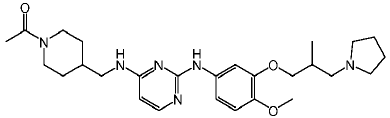 |
| 184 | 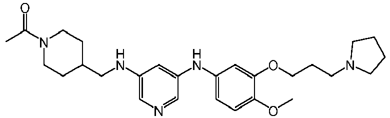 |
| 185 | 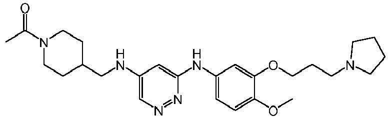 |
| 186 | 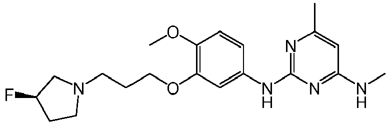 |
| 187 | 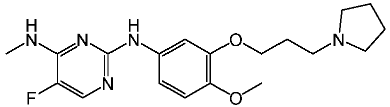 |
| 188 | 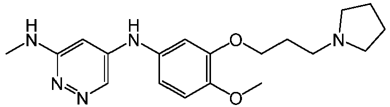 |
| 190 | 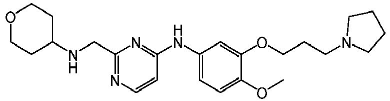 |
| 191 | 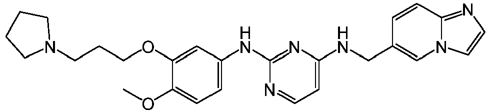 |
| 192 | 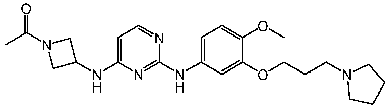 |
| 193 | 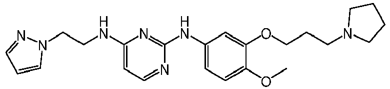 |
| 194 | 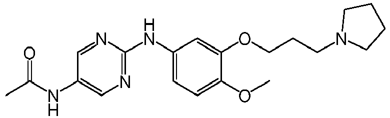 |
| 195 | 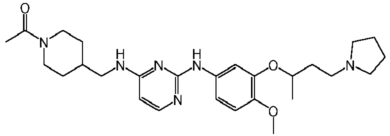 |
| 196 | 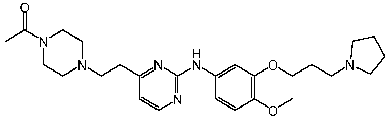 |
| 197 | 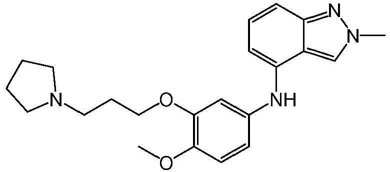 |
| 199 | 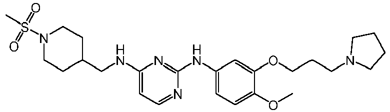 |
| 200 | 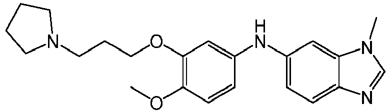 |
| 201 | 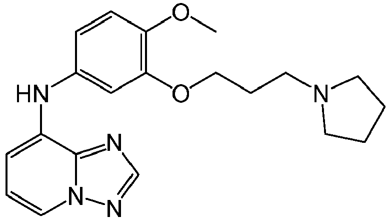 |
| 202 | 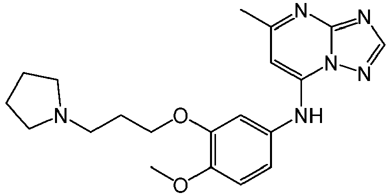 |
| 203 | 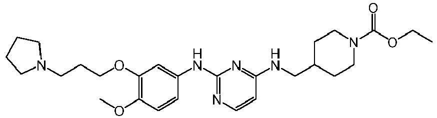 |
| 204 | 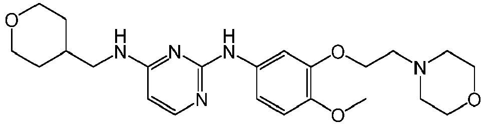 |
| 205 | 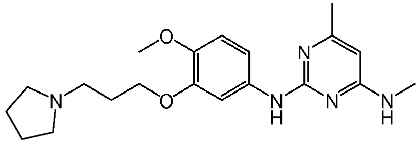 |
| 206 | 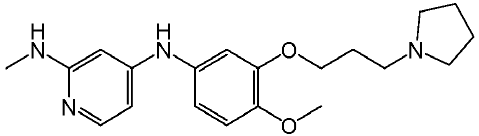 |
| 207 | 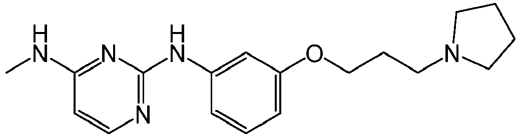 |
| 208 | 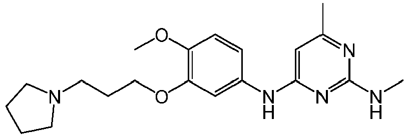 |
| 209 | 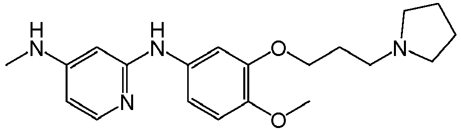 |
| 210 | 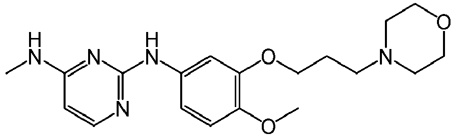 |
| 211 | 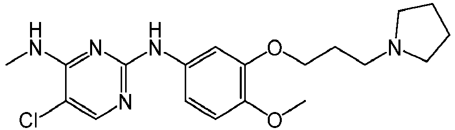 |
| 212 | 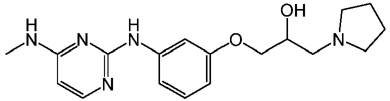 |
| 213 | 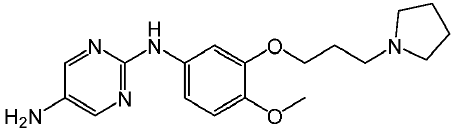 |
| 214 | 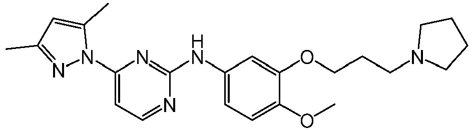 |
| 215 | 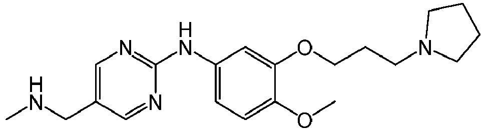 |
| 216 | 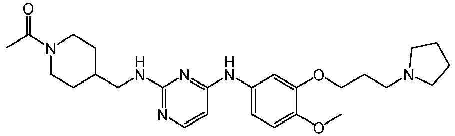 |
| 217 | 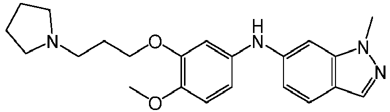 |
| 218 | 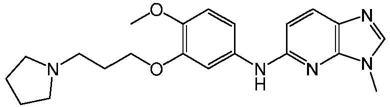 |
| 219 | 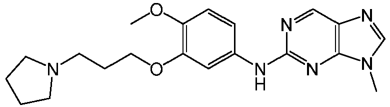 |
| 220 | 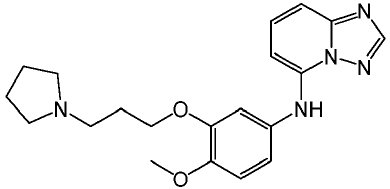 |
| 221 | 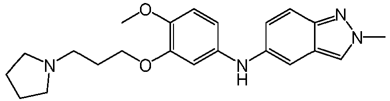 |
| 222 | 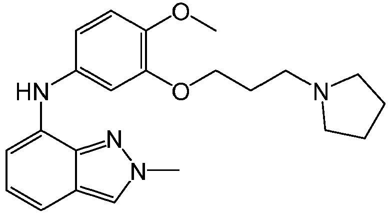 |
| 223 | 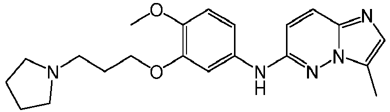 |
| 224 | 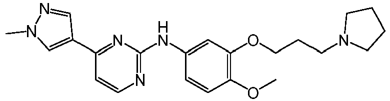 |
| 225 | 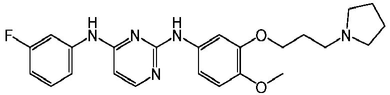 |
| 226 | 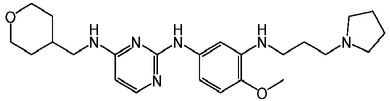 |
| 227 | 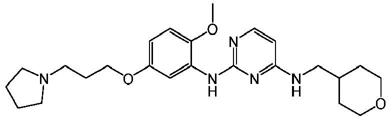 |
| 228 | 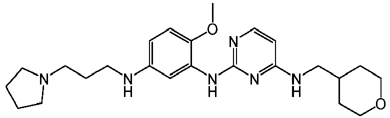 |
| 229 | 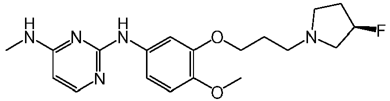 |
| 230 | 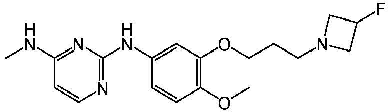 |
| 231 | 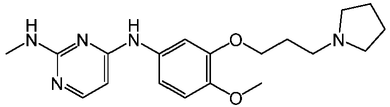 |
| 232 | 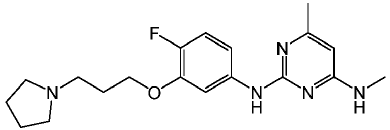 |
| 233 | 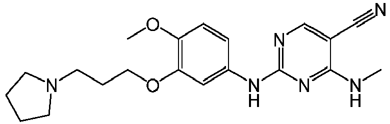 |
| 234 | 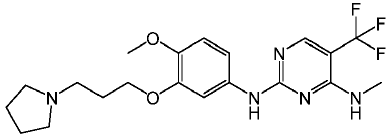 |
| 235 | 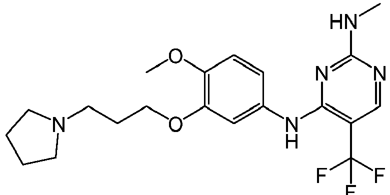 |
| 236 | 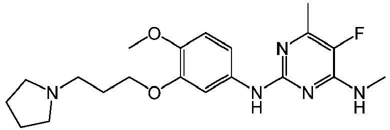 |
| 237 | 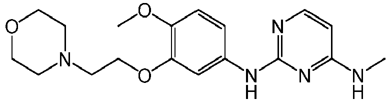 |
| 238 | 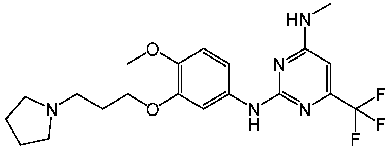 |
| 239 | 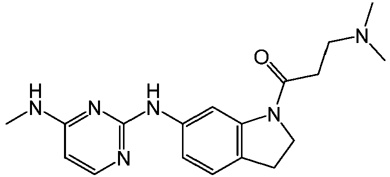 |
| 240 | 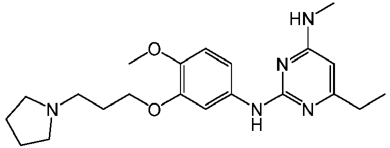 |
| 241 | 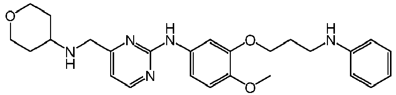 |
| 242 | 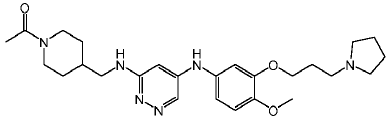 |
| 243 | 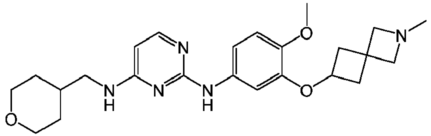 |
| 244 | 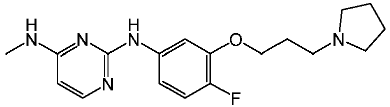 |
| 245 | 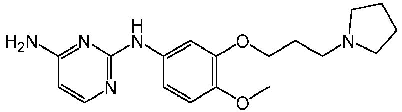 |
| 246 | 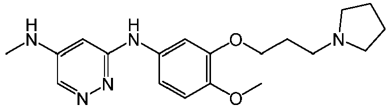 |
| 247 | 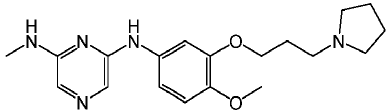 |
| 248 | 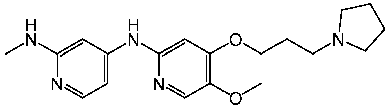 |
| 249 | 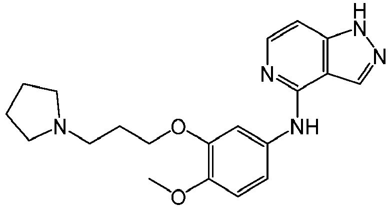 |
| 250 | 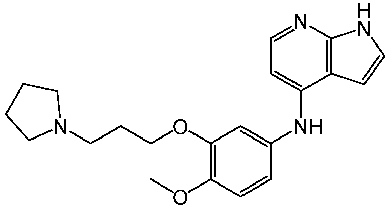 |
| 251 | 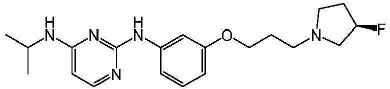 |
| 252 | 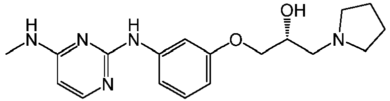 |
| 253 | 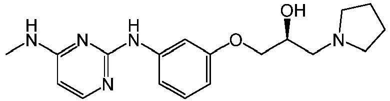 |
| 254 | 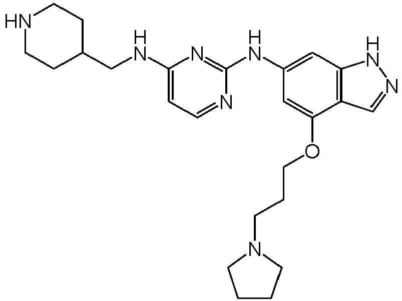 |
| 255 | 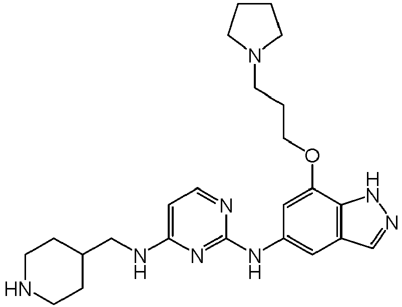 |
| 256 | 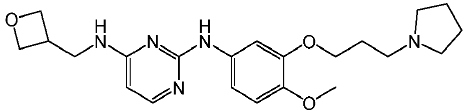 |
| 257 | 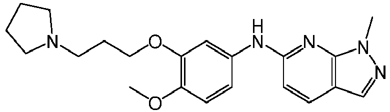 |
| 258 | 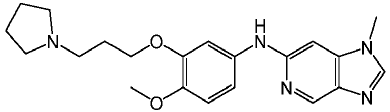 |
| 259 | 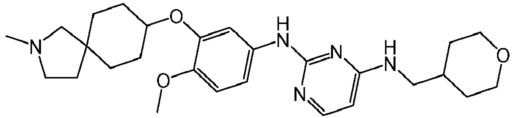 |
| 260 | 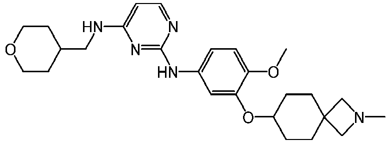 |
| 261 | 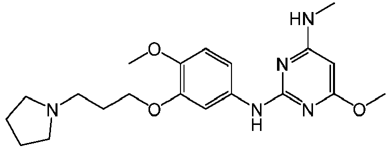 |
| 262a | 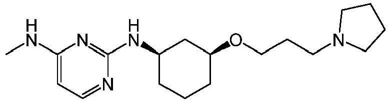 |
| 262b | 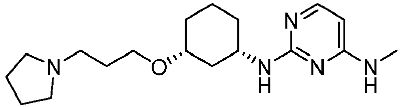 |
| 263 | 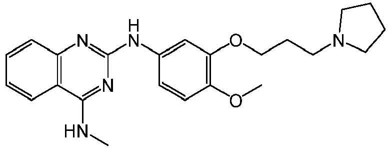 |
| 264 | 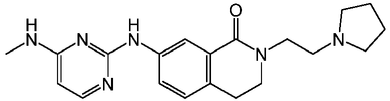 |
| 265 | 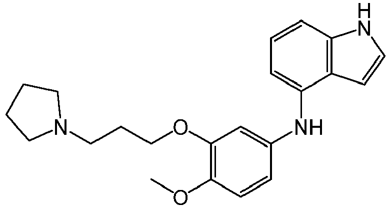 |
| 266 | 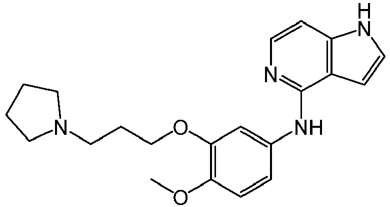 |
| 267 | 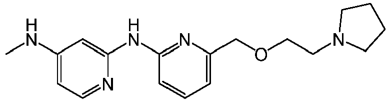 |
| 268 | 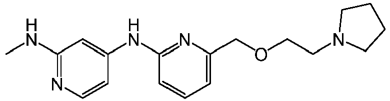 |
| 269 | 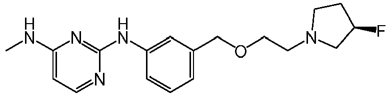 |
| 271 | 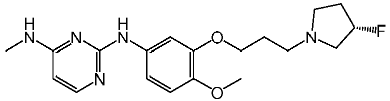 |
| 272 | 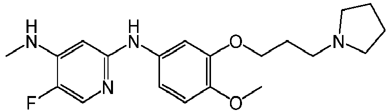 |
| 273 | 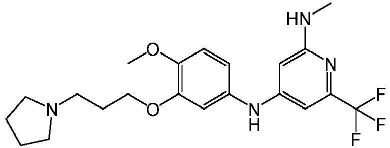 |
| 274 | 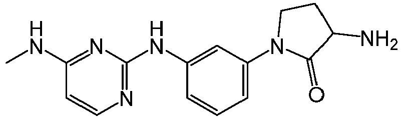 |
| 275 | 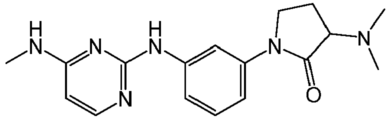 |
| 276 | 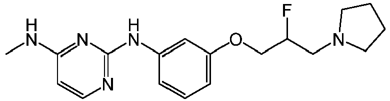 |
| 277 | 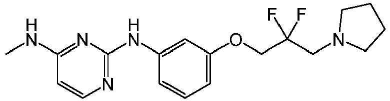 |
| 278 | 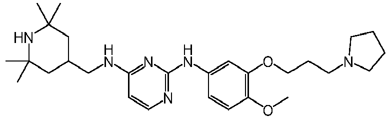 |
| 279 | 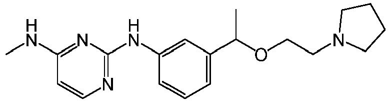 |
| 280 | 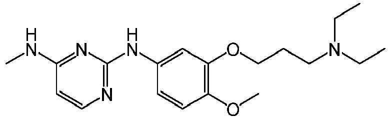 |
| 281 | 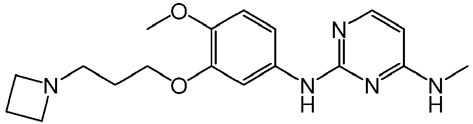 |
| 282 | 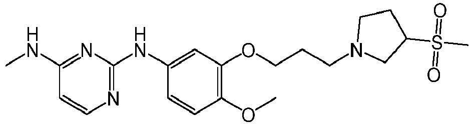 |
| 283 | 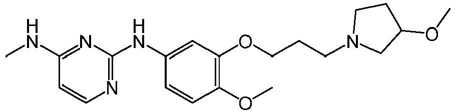 |
| 284 | 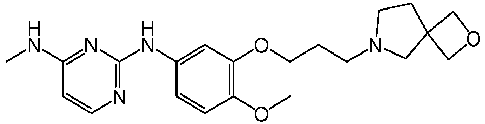 |
| 285 | 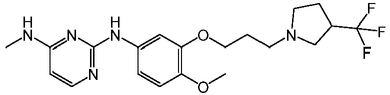 |
| 286 | 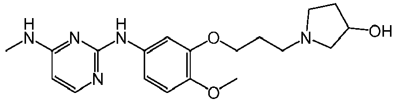 |
| 287 | 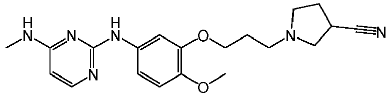 |
| 288 | 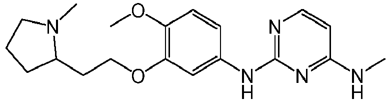 |
| 289 | 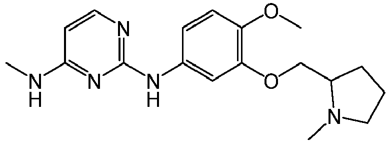 |
| 290 | 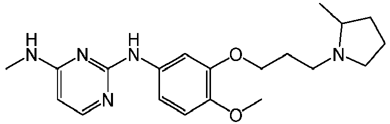 |
| 291 | 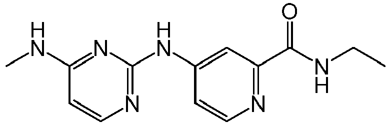 |
| 292 | 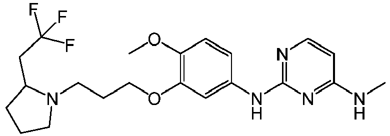 |
| 293 | 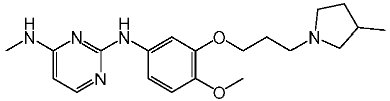 |
| 294 | 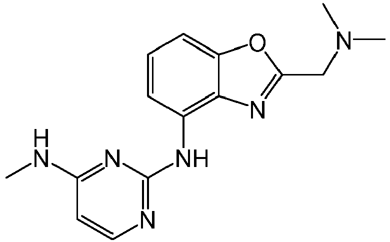 |
| 295 | 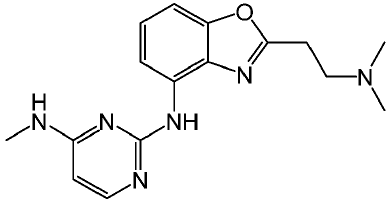 |
| 296 | 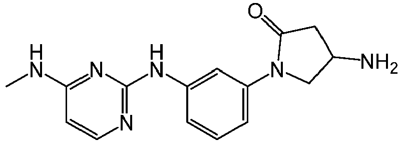 |
| 297 | 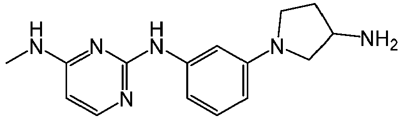 |
| 298 | 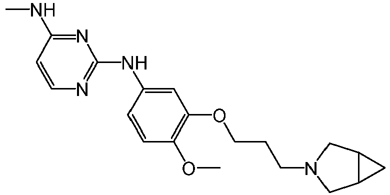 |
| 299 | 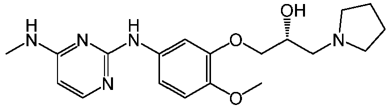 |
| 300 | 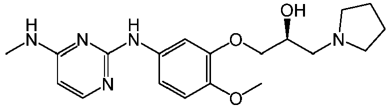 |
| 301 | 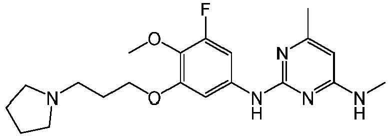 |
| 302 | 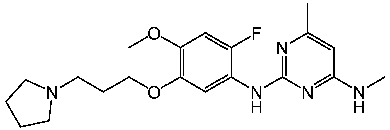 |
| 303 | 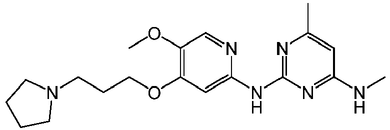 |
| 304 | 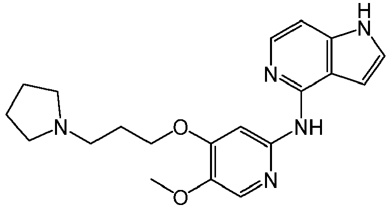 |
| 305 | 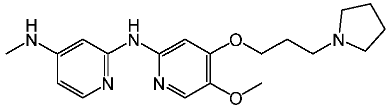 |
| 306 | 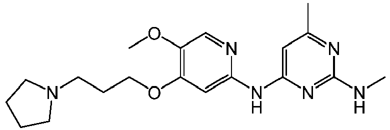 |
| 307 | 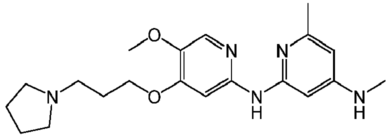 |
| 308 | 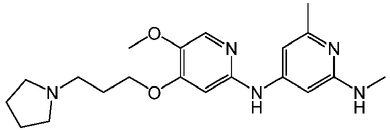 |
| 309 | 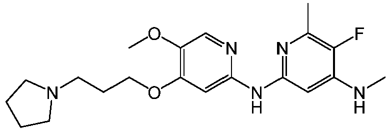 |
| 310 | 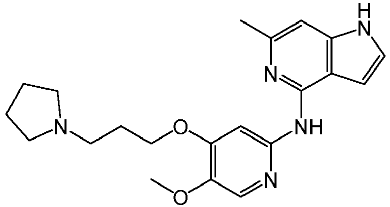 |
| 311 | 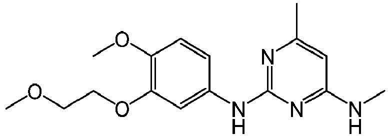 |
| 312 | 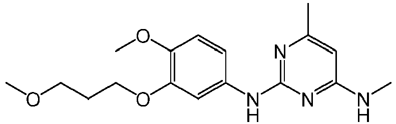 |
| 313 | 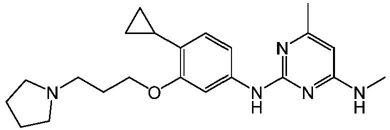 |
| 314 | 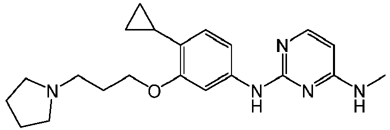 |
| 315 | 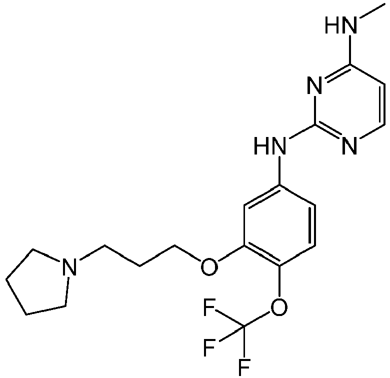 |
| 316 | 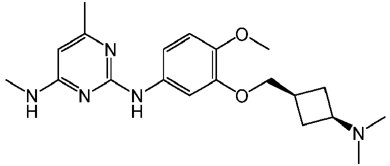 |
| 317 | 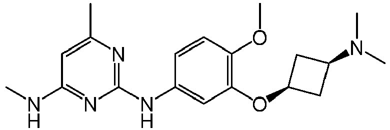 |
| 318 | 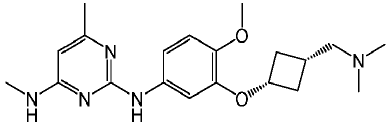 |
| 319 | 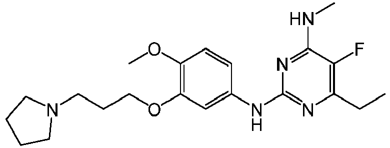 |
| 320 | 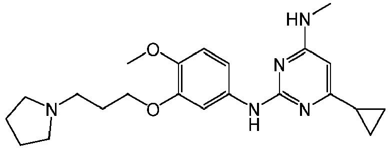 |
| 321 | 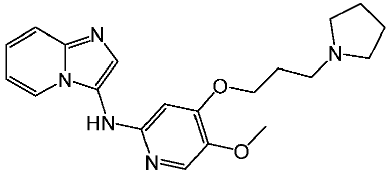 |
| 322 | 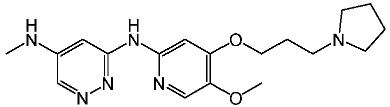 |
| 323 | 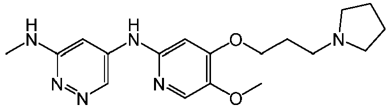 |
| 324 | 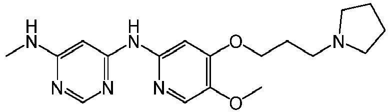 |
| 325 | 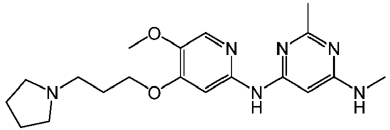 |
| 326 | 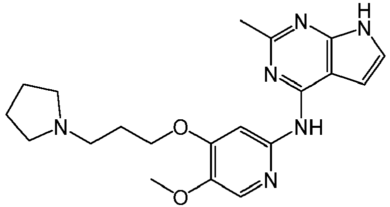 |
| 327 | 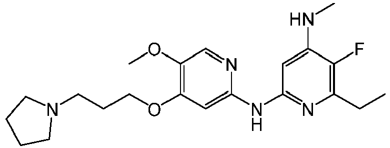 |
| 328 | 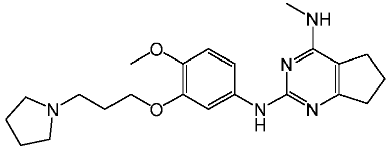 |
| 329 | 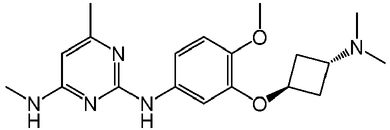 |
| 330 | 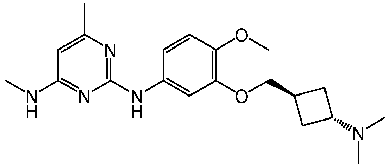 |
| 331 | 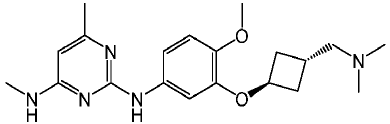 |
| 332 | 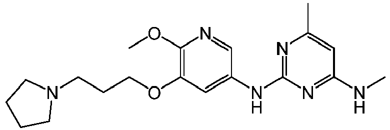 |
| 333 | 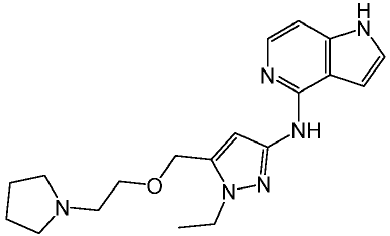 |
| 334 | 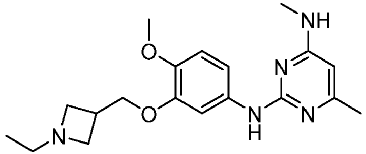 |
| 335 | 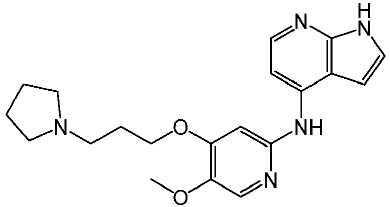 |
| 336 | 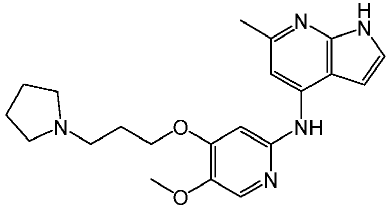 |
| 337 | 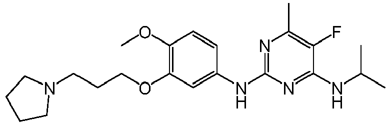 |

**Table 2**

| **Compound No.** | **Structure** |
|---|---|
| 338 | 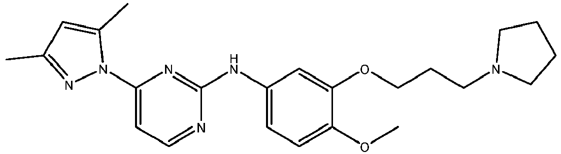 |
| 339 | 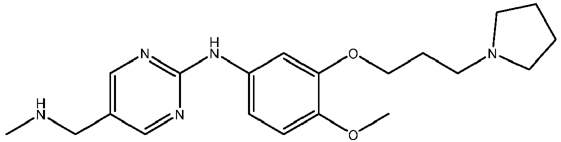 |
| 340 | 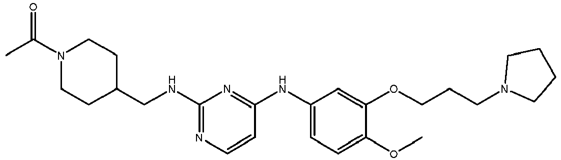 |
| 341 | 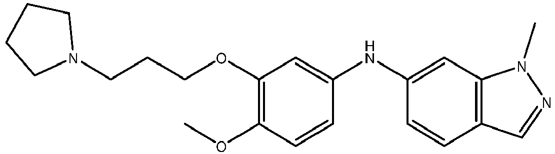 |
| 342 | 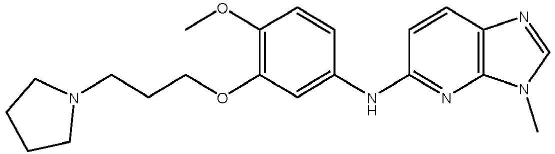 |
| 343 | 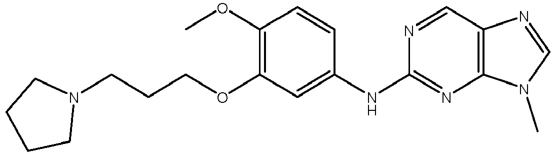 |
| 344 | 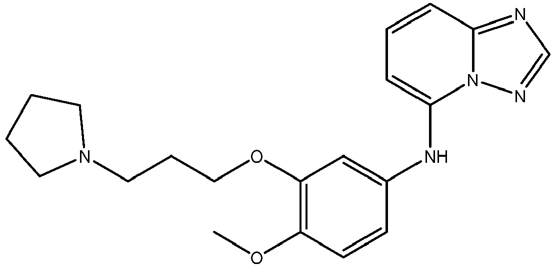 |
| 345 | 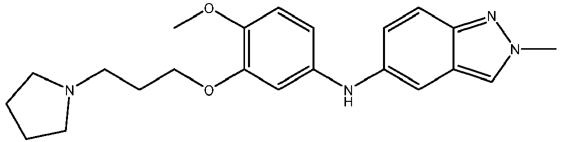 |
| 346 | 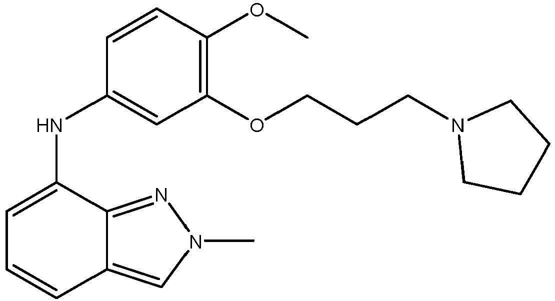 |
| 347 | 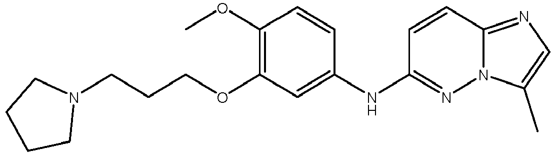 |
| 348 | 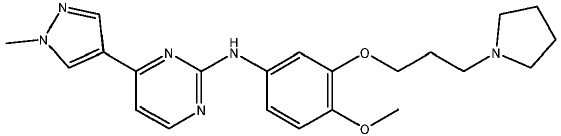 |
| 349 | 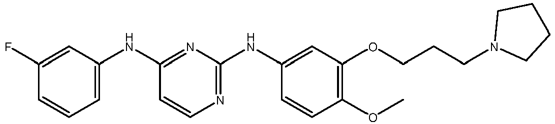 |
| 350 | 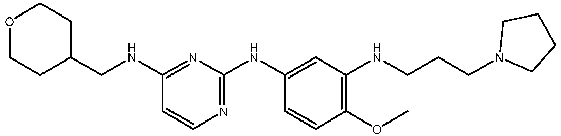 |
| 351 | 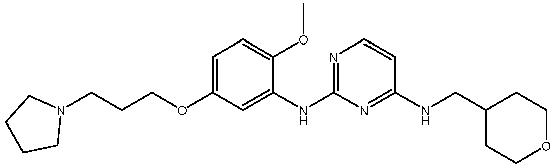 |
| 352 | 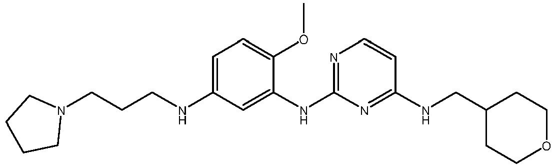 |
| 353 | 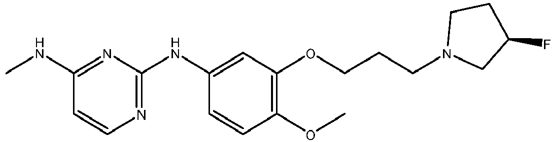 |
| 354 | 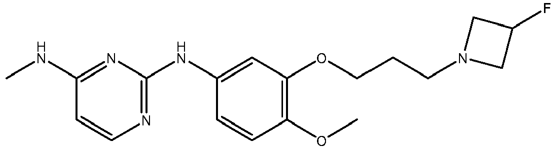 |
| 355 | 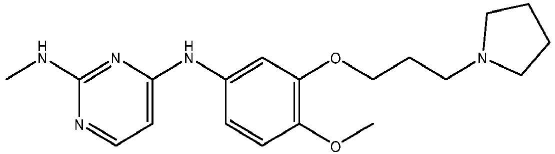 |
| 356 | 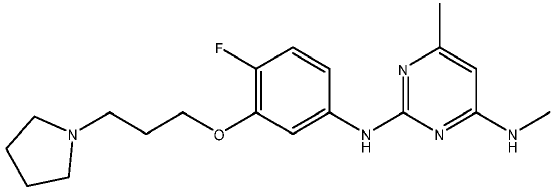 |
| 357 | 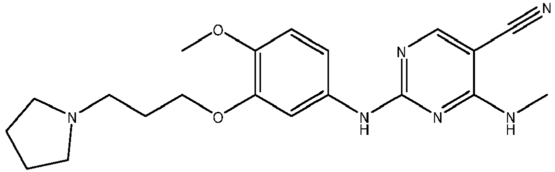 |
| 358 | 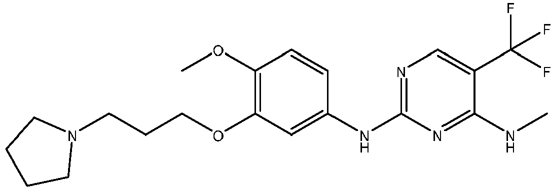 |
| 359 | 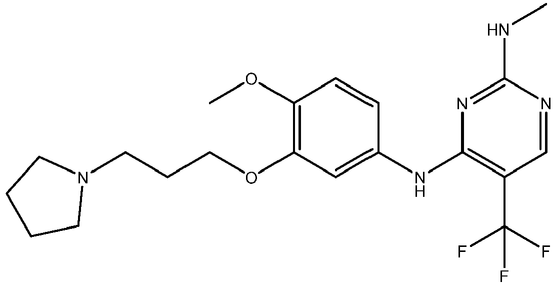 |
| 360 | 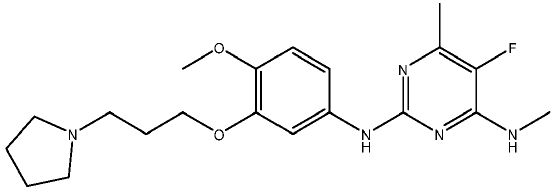 |
| 361 | 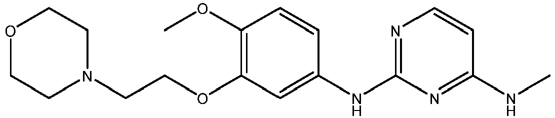 |
| 362 | 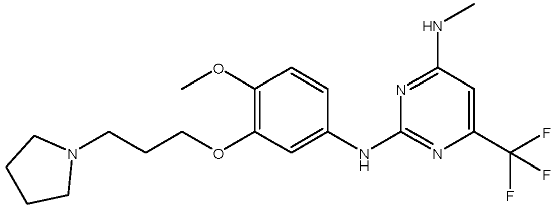 |
| 363 | 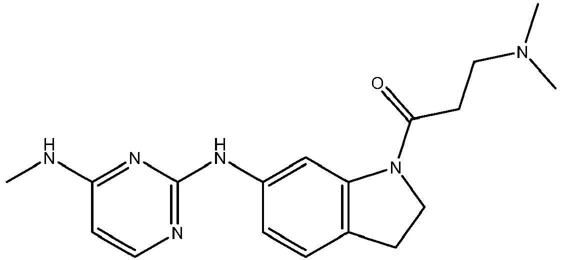 |
| 364 | 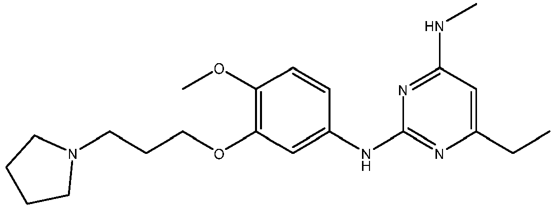 |
| 365 | 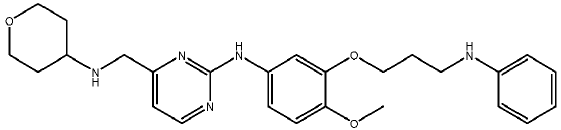 |
| 366 | 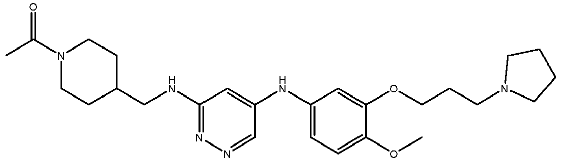 |
| 367 | 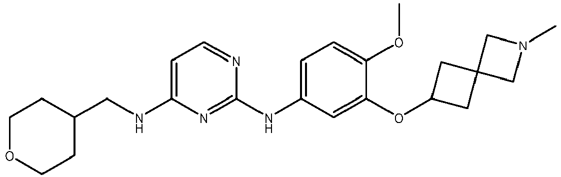 |
| 368 | 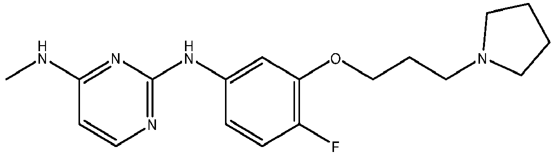 |
| 369 | 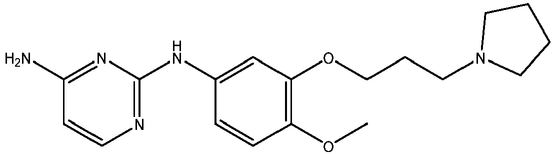 |
| 370 | 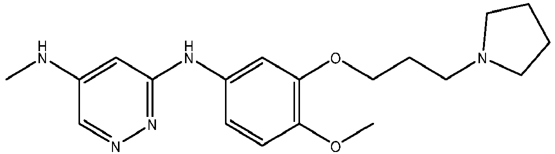 |
| 371 | 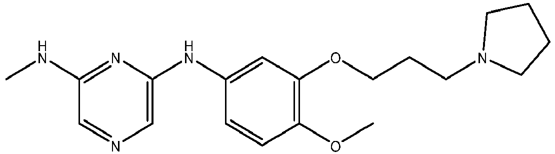 |
| 372 | 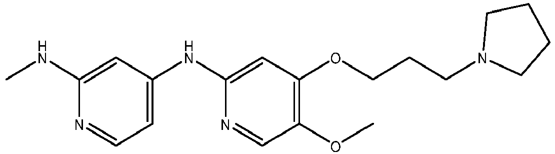 |
| 373 | 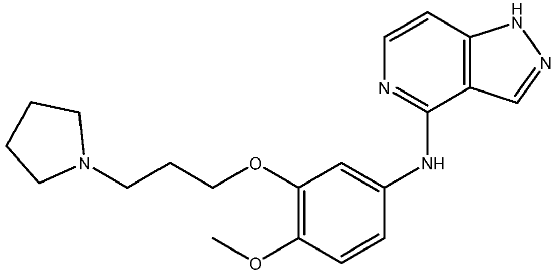 |
| 374 | 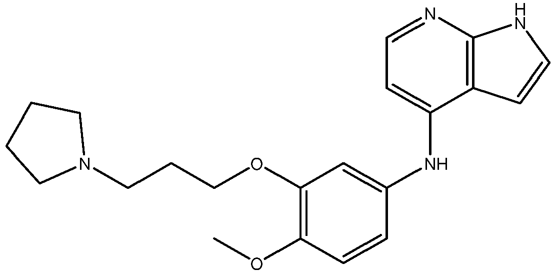 |
| 375 | 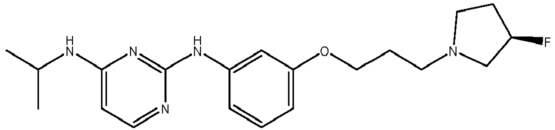 |
| 376 | 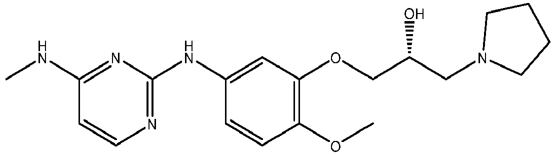 |
| 377 | 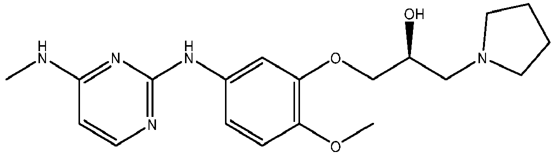 |
| 378 | 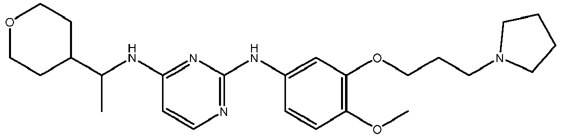 |
| 379 | 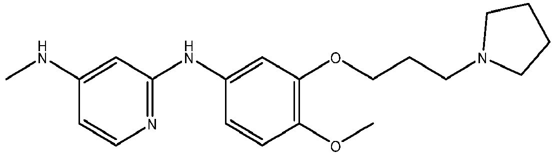 |
| 380 | 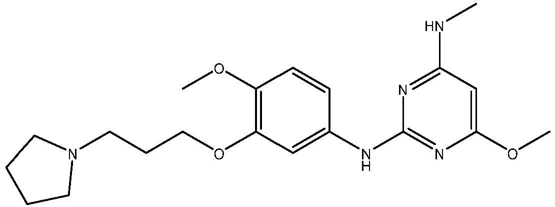 |
| 381 | 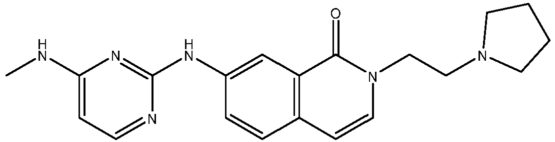 |
| 382 | 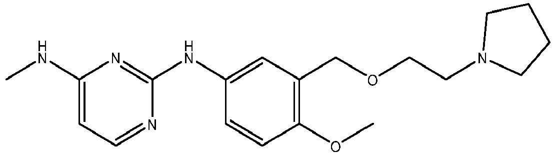 |
| 383 | 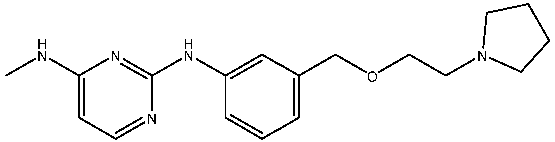 |
| 384 | 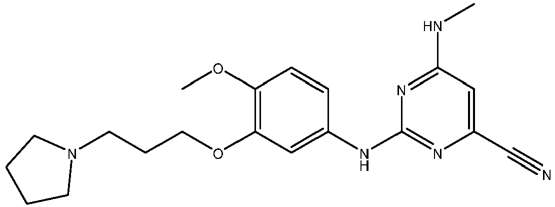 |
| 385 | 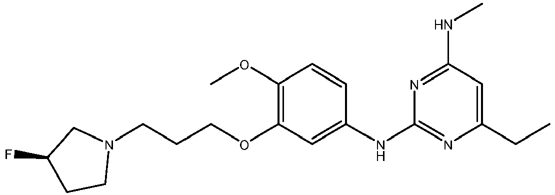 |
| 386 | 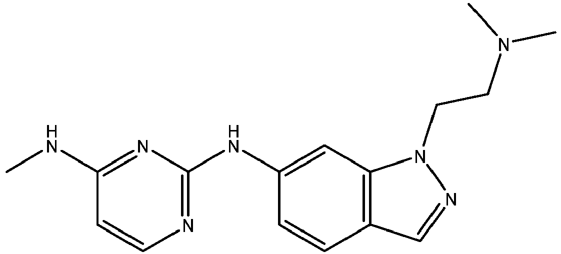 |
| 387 | 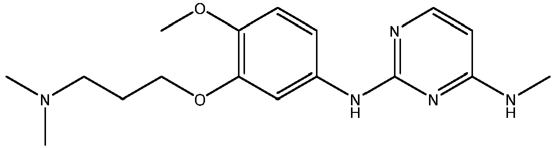 |
| 388 | 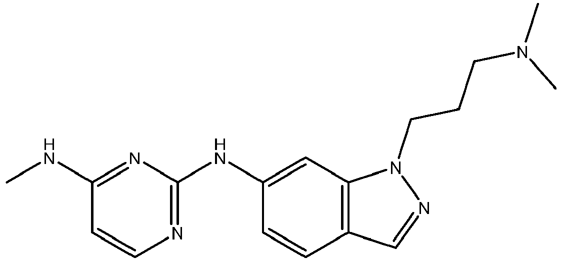 |
| 389 | 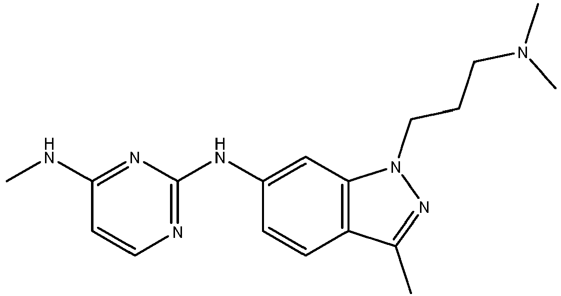 |
| 390 | 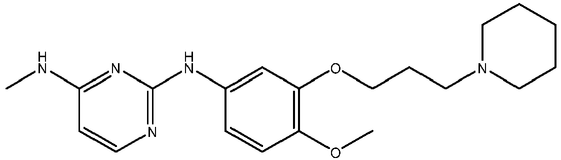 |
| 391 | 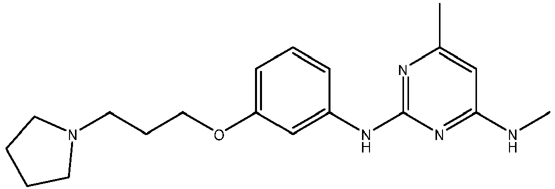 |
| 392 | 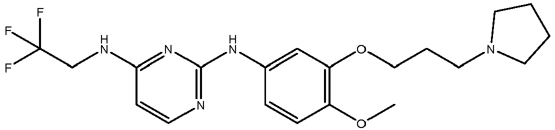 |
| 393 | 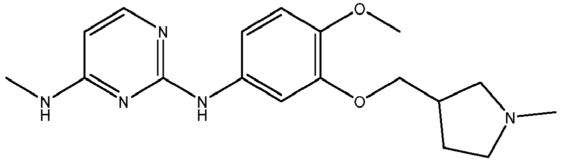 |
| 394 | 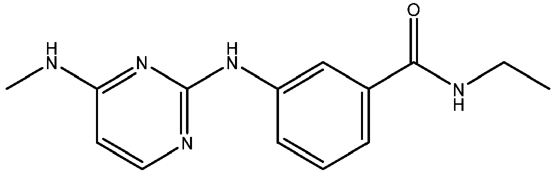 |
| 395 | 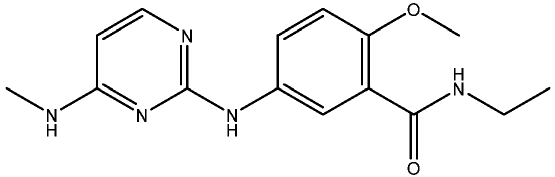 |
| 396 | 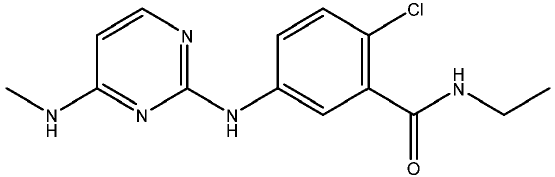 |
| 397 | 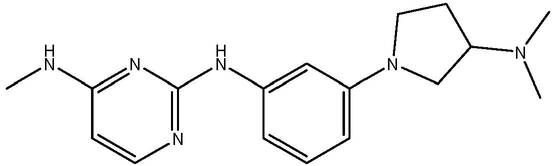 |
| 398 | 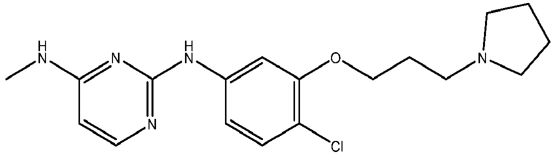 |
| 399 | 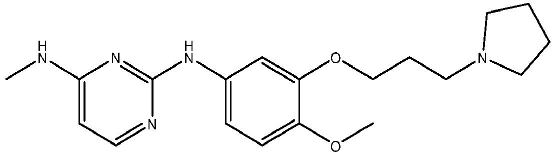 |
| 400 | 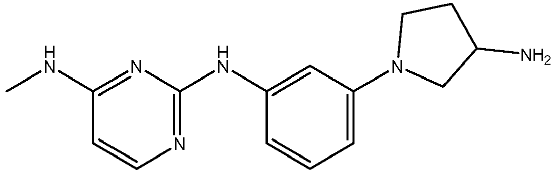 |
| 401 | 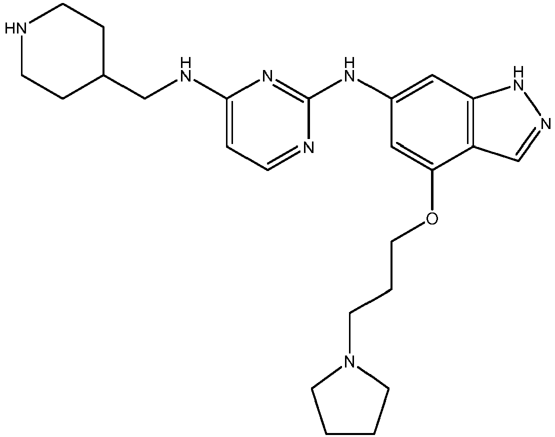 |
| 402 | 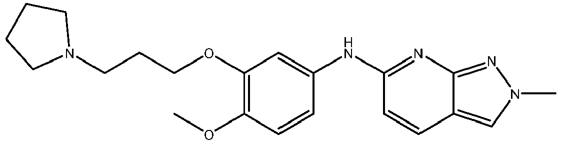 |
| 404 | 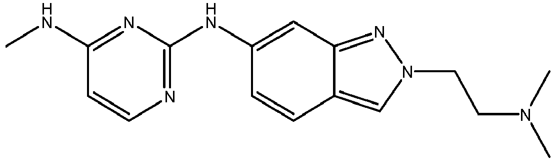 |
| 405 | 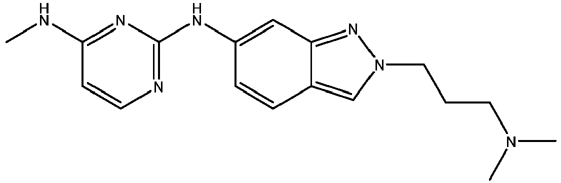 |
| 406 | 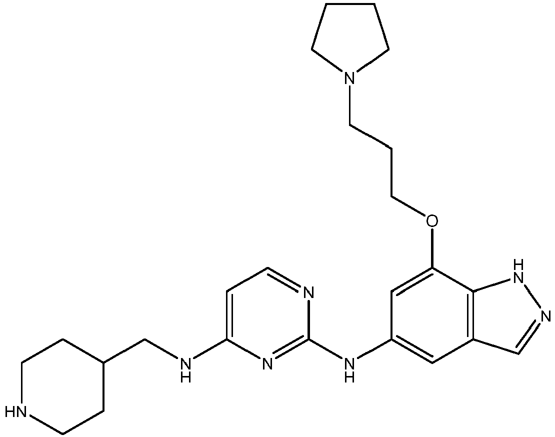 |
| 407 | 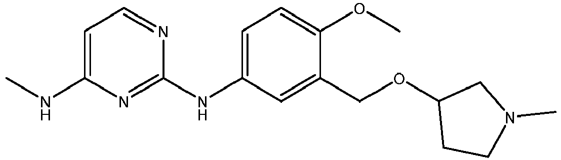 |
| 408 | 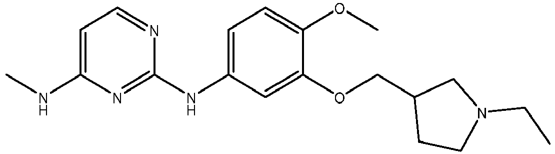 |
| 409 | 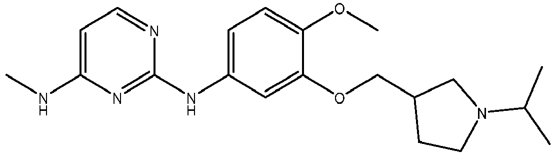 |
| 410 | 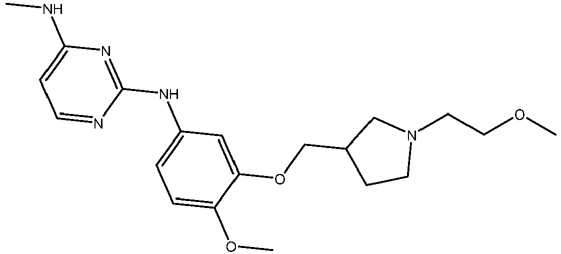 |
| 411 | 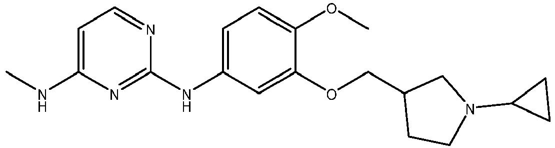 |
| 412 | 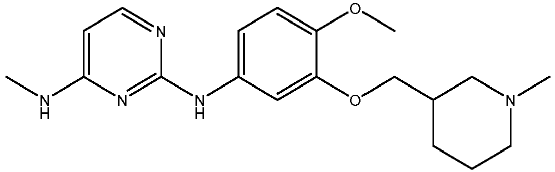 |
| 413 | 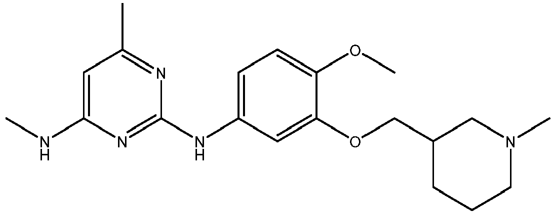 |
| 414 | 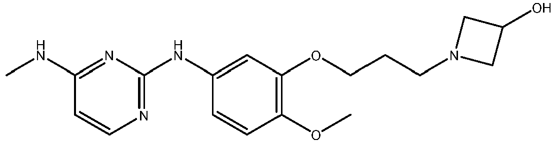 |
| 415 | 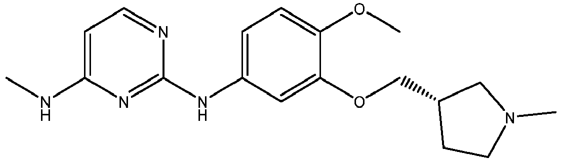 |
| 416 | 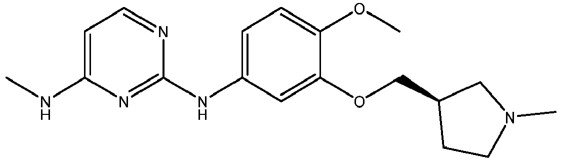 |
| 417 | 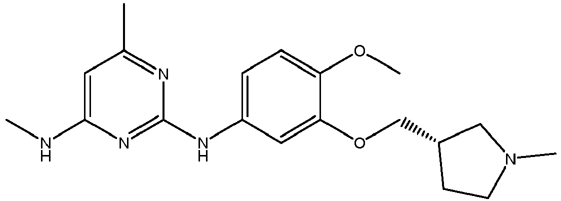 |
| 418 | 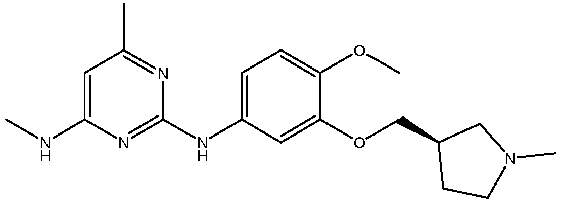 |
| 419 | 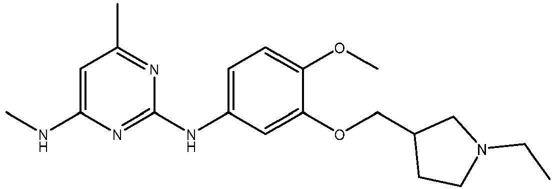 |
| 420 | 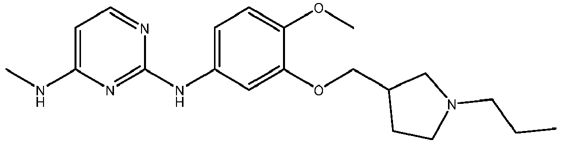 |
| 421 | 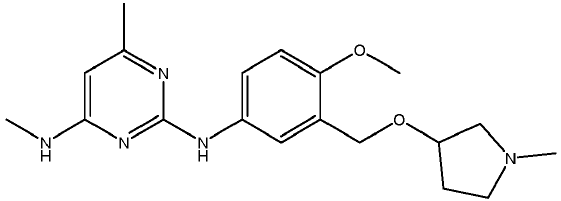 |
| 422 | 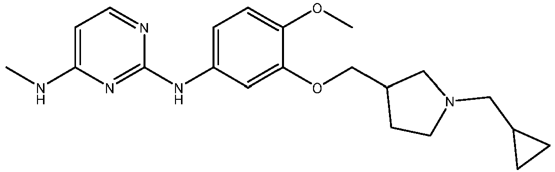 |
| 423 | 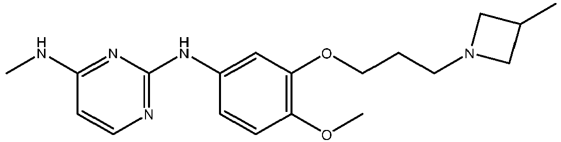 |
| 424 | 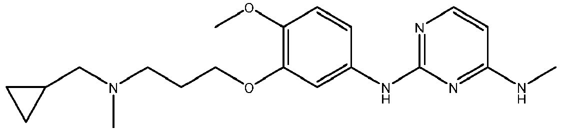 |
| 425 | 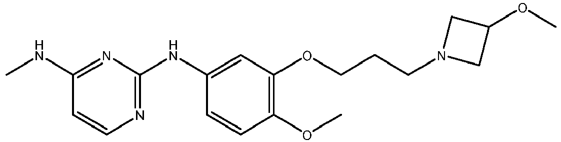 |
| 426 | 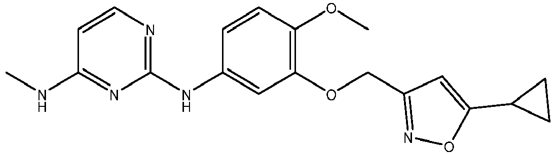 |
| 427 | 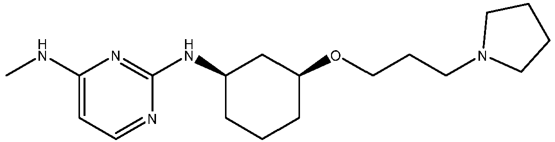 |
| 428 | 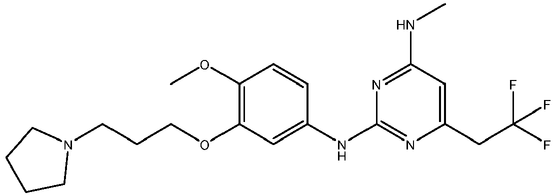 |
| 429 | 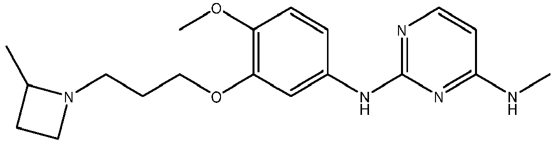 |
| 430 | 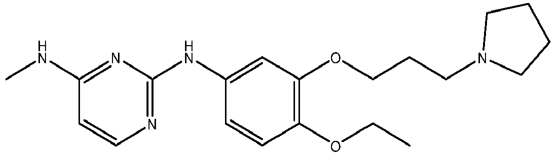 |
| 431 | 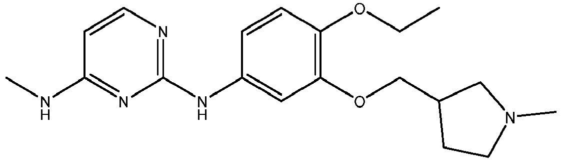 |
| 432 | 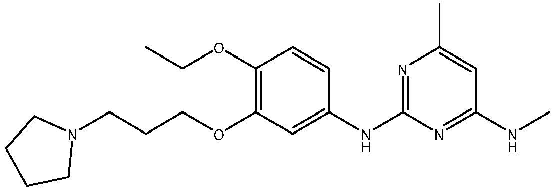 |
| 433 | 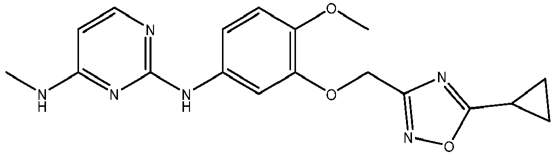 |
| 434 | 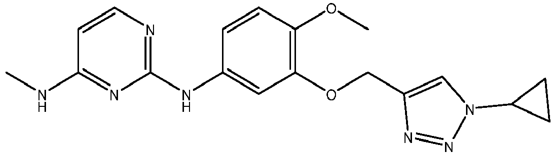 |
| 435 | 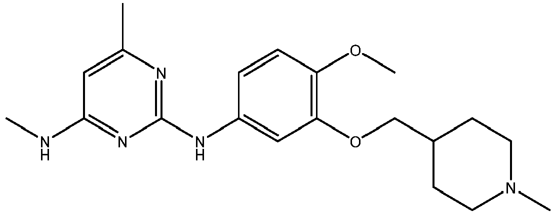 |
| 436 | 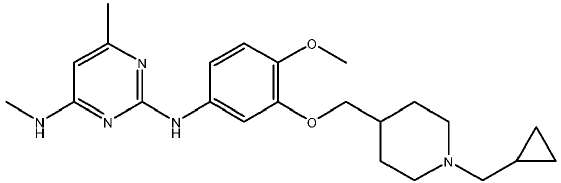 |
| 437 | 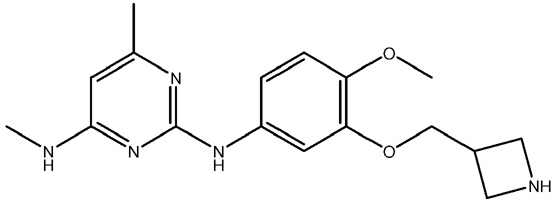 |
| 438 | 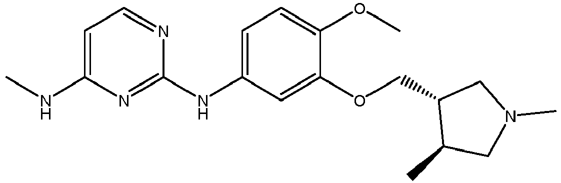 |
| 439 | 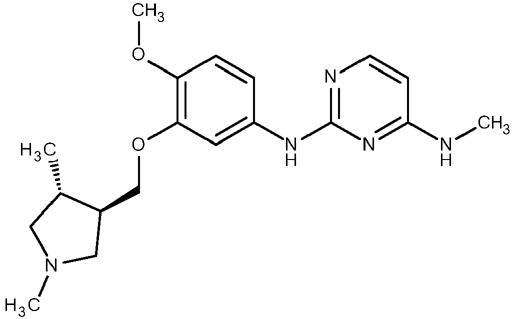 |
| 440 | 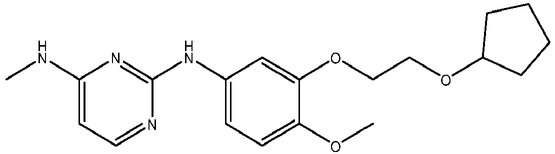 |
| 441 | 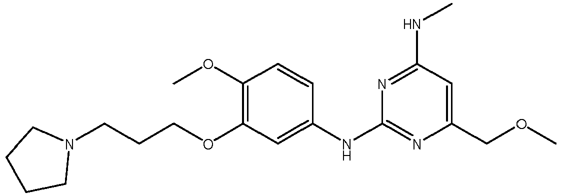 |
| 442 | 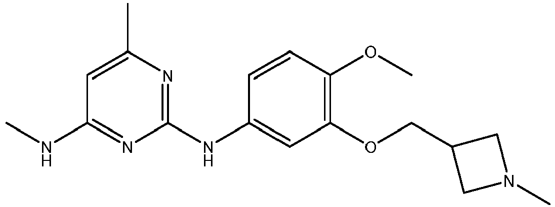 |
| 443 | 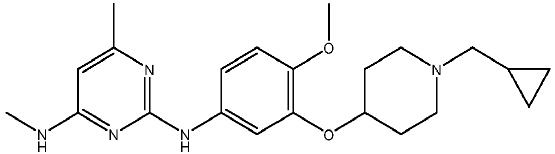 |
| 444 | 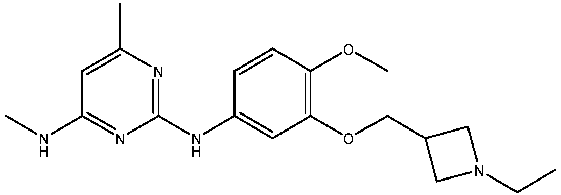 |
| 445 | 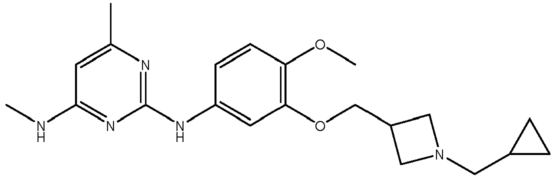 |
| 446 | 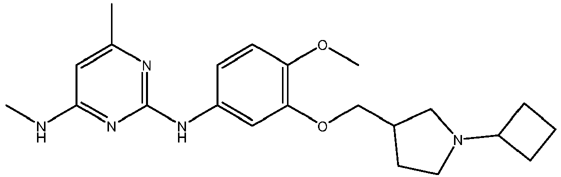 |
| 447 | 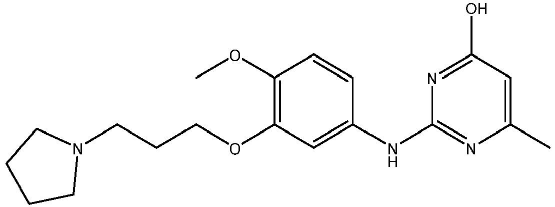 |
| 448 | 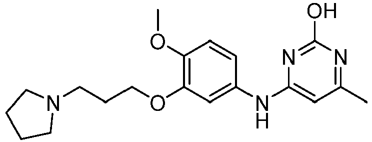 |
| 449 | 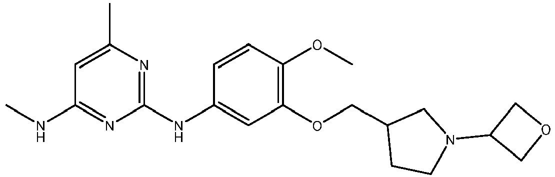 |
| 450 | 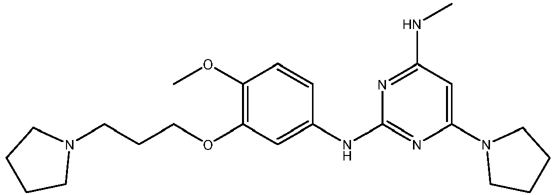 |
| 451 | 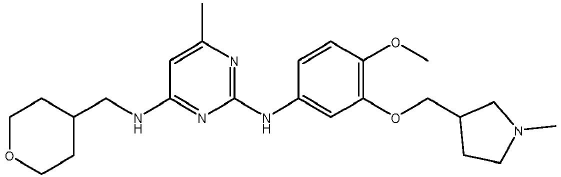 |
| 452 | 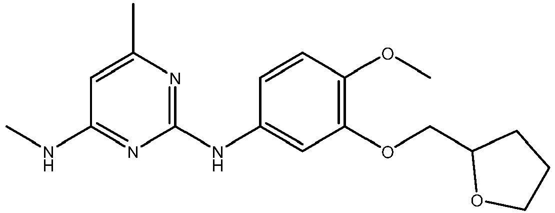 |
| 453 | 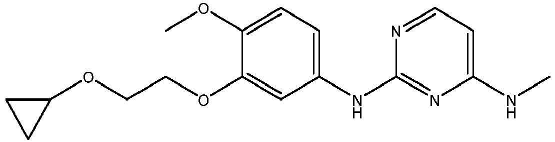 |
| 455 | 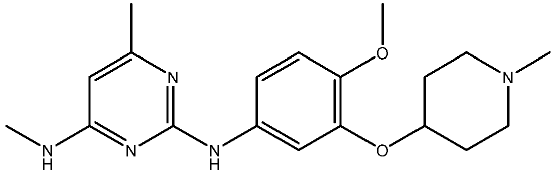 |
| 456 | 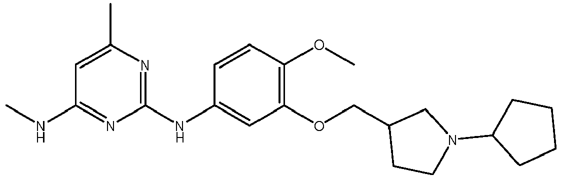 |
| 457 | 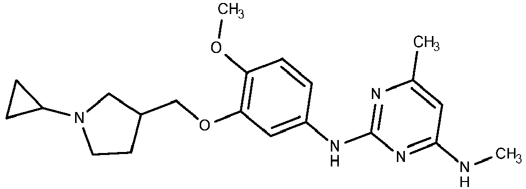 |
| 458 | 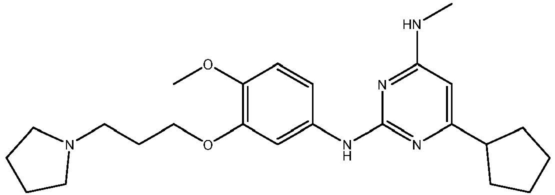 |
| 459 | 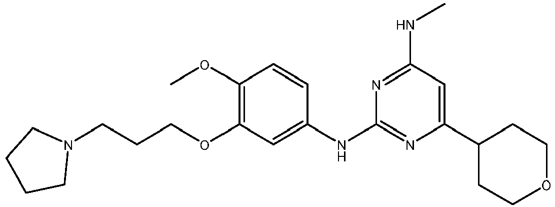 |
| 460 | 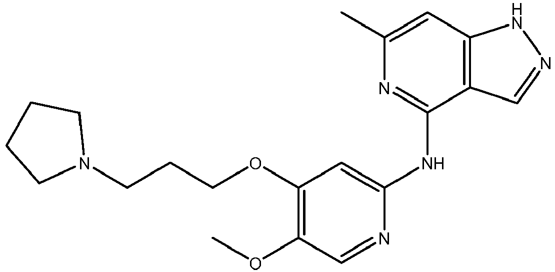 |
| 461 | 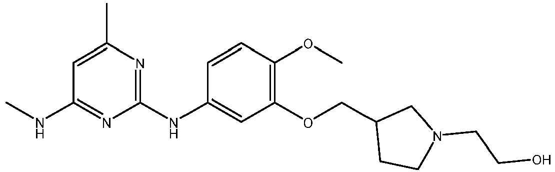 |
| 462 | 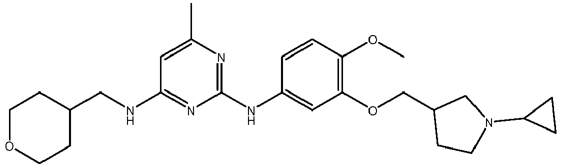 |
| 463 | 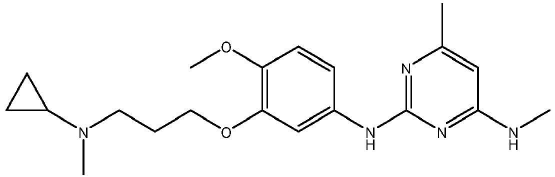 |
| 464 | 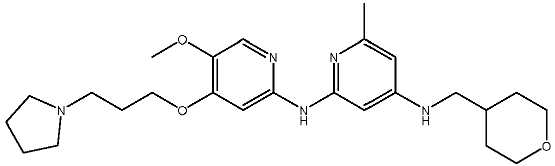 |
| 465 | 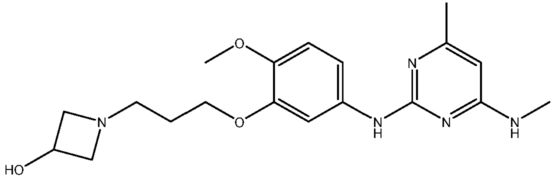 |
| 466 | 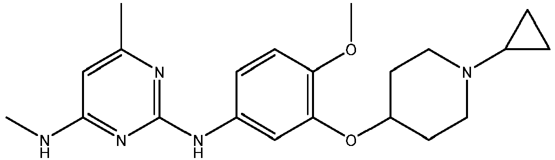 |
| 467 | 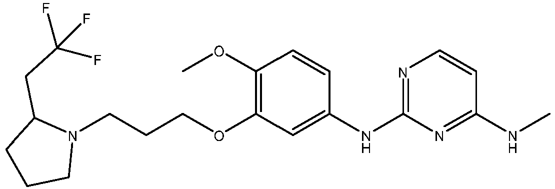 |
| 468 | 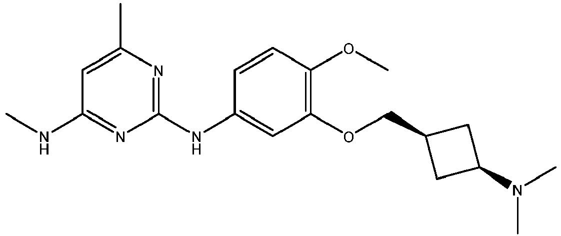 |
| 469 | 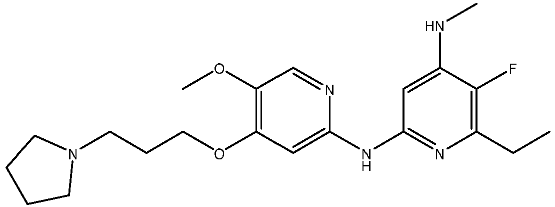 |
| 470 | 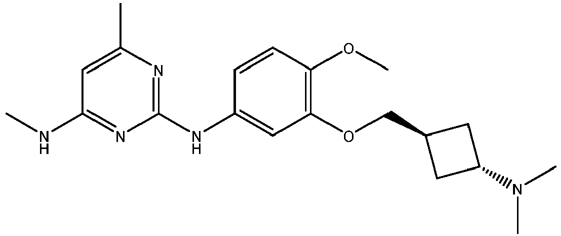 |
| 471 | 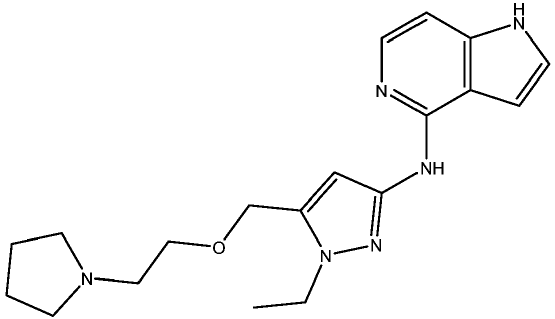 |
| 472 | 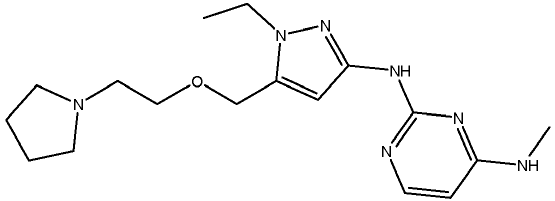 |
| 473 | 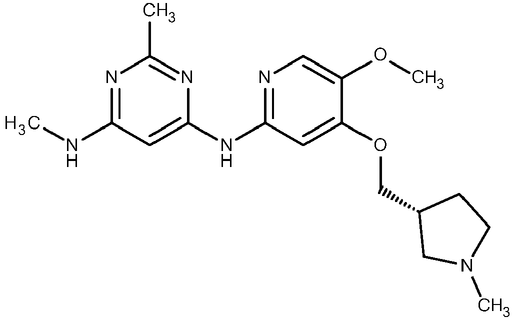 |
| 474 | 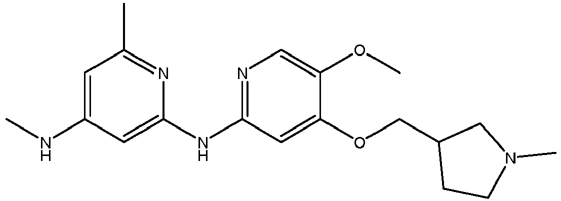 |
| 475 | 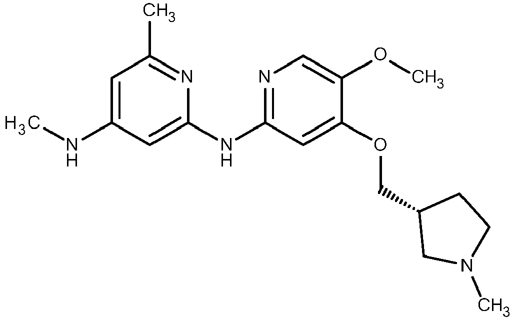 |
| 476 | 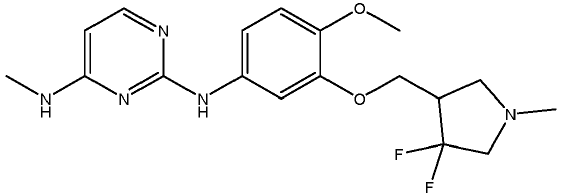 |
| 477 | 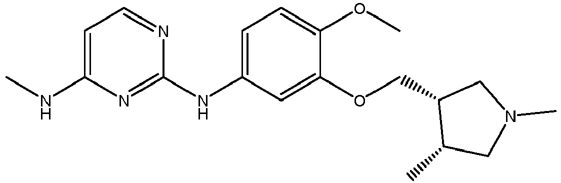 |
| 478 | 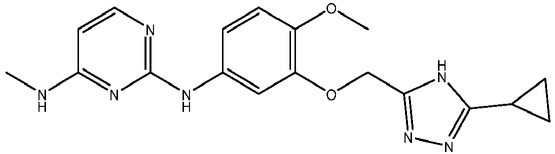 |
| 479 | 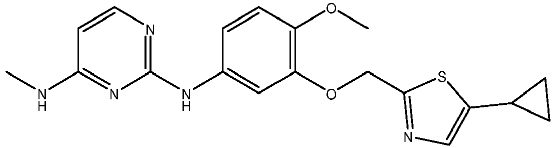 |
| 480 | 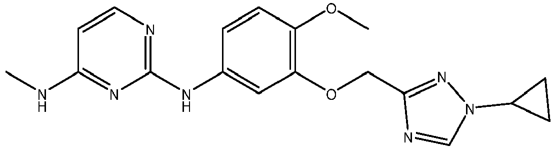 |
| 481 | 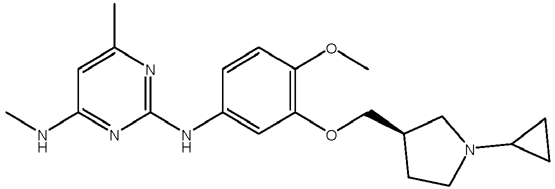 |
| 482 | 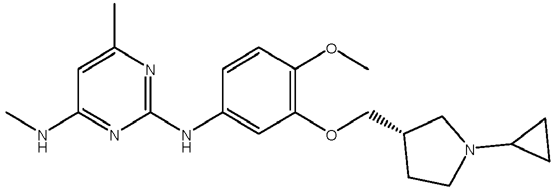 |
| 483 | 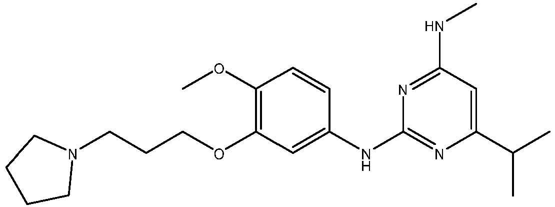 |
| 484 | 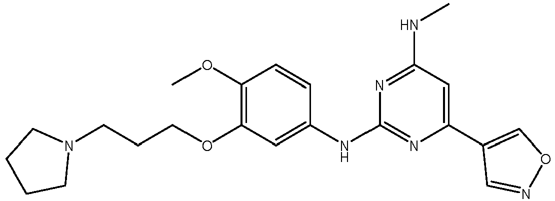 |
| 485 | 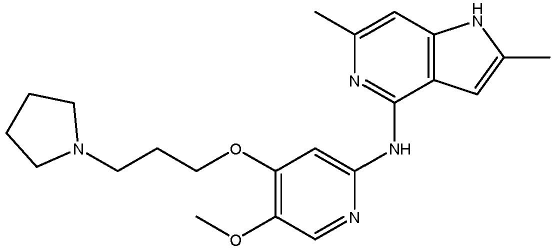 |
| 486 | 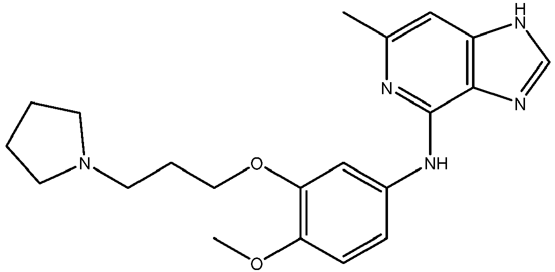 |
| 487 | 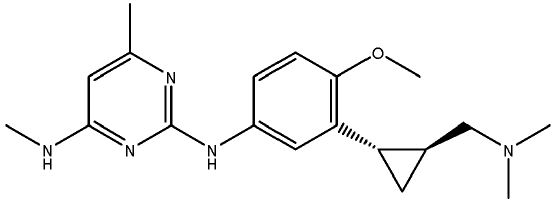 |
| 488 | 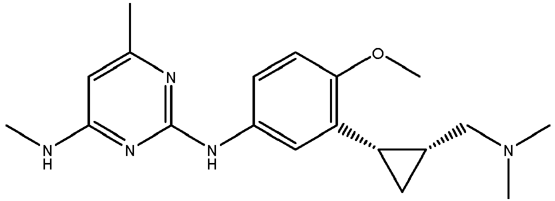 |
| 489 | 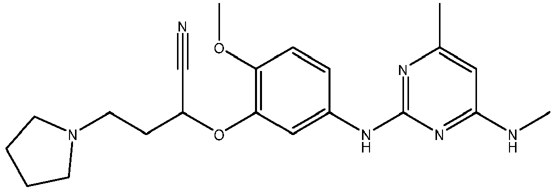 |
| 490 | 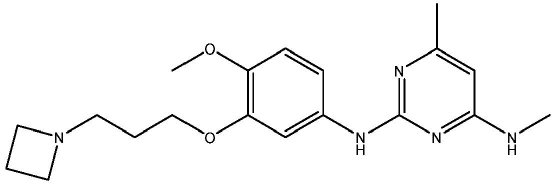 |
| 491 | 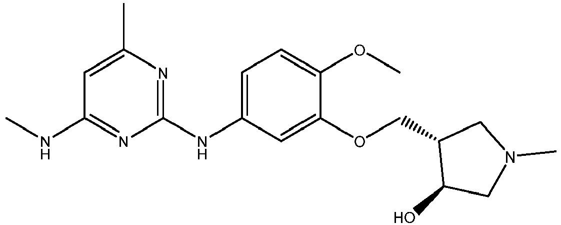 |
| 492 | 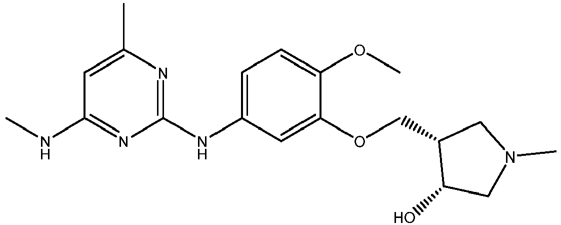 |
| 493 | 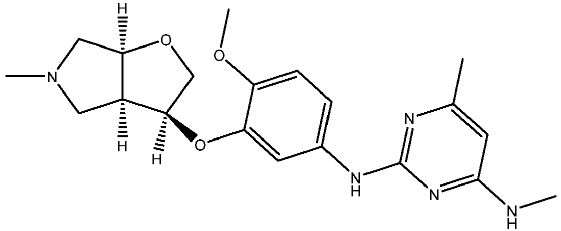 |
| 494 | 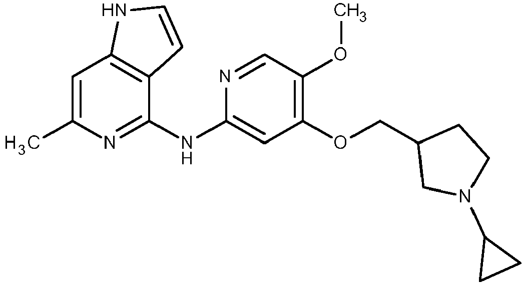 |
| 494a | 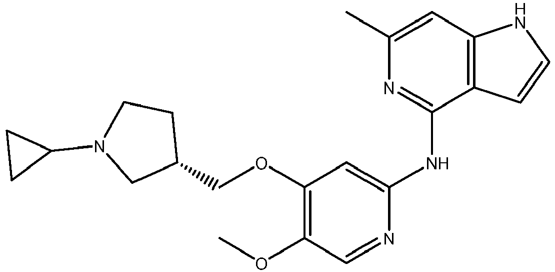 |
| 495 | 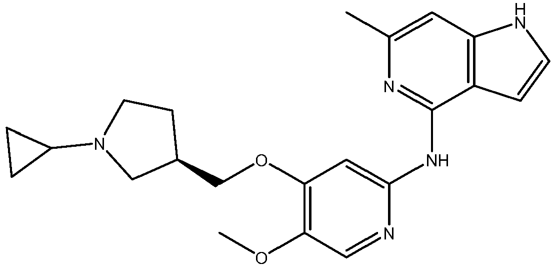 |
| 496 | 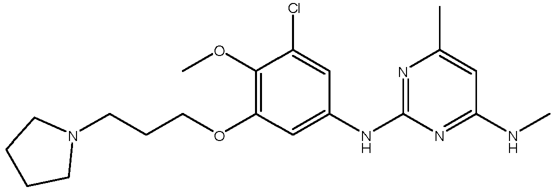 |
| 497 | 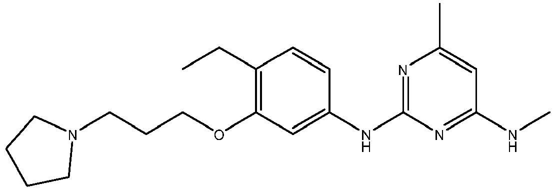 |
| 498 | 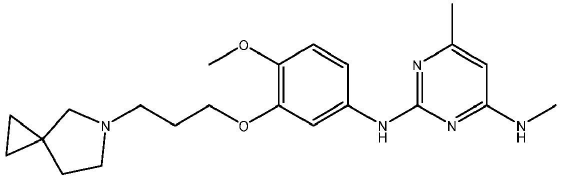 |
| 499 | 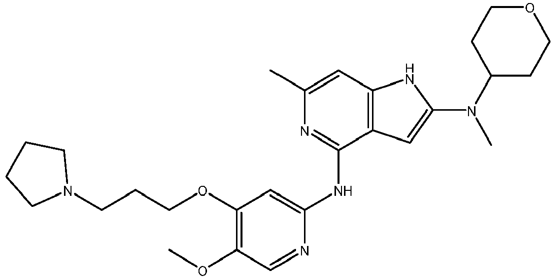 |
| 500 | 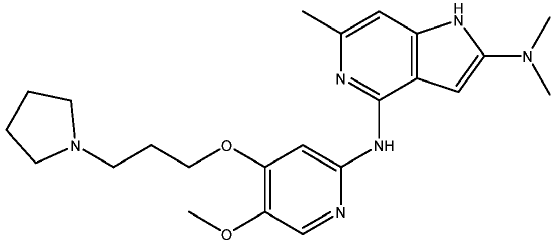 |
| 501 | 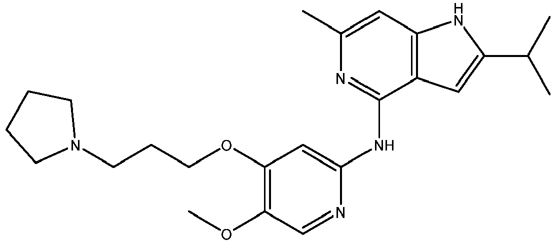 |
| 502 | 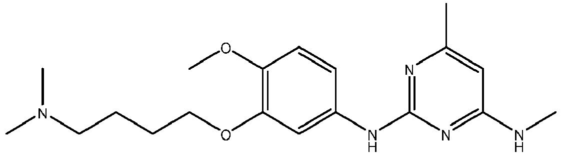 |
| 503 | 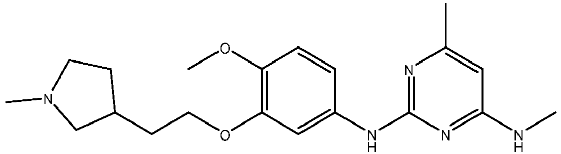 |
| 504 | 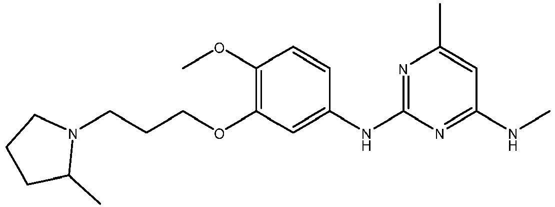 |
| 505 | 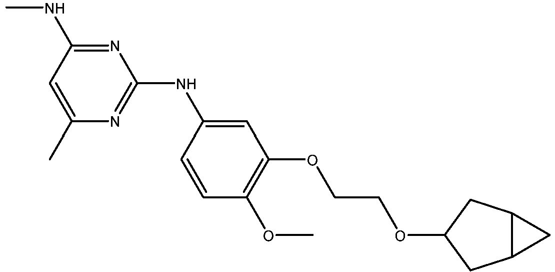 |
| 506 | 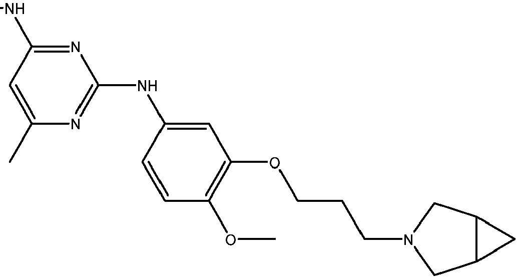 |
| 507 | 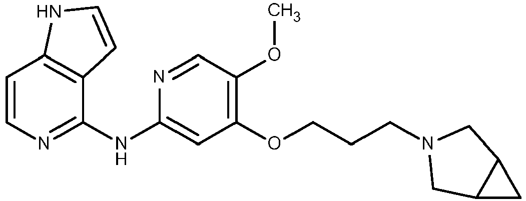 |
| 508 | 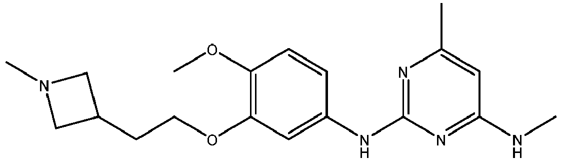 |
| 509 | 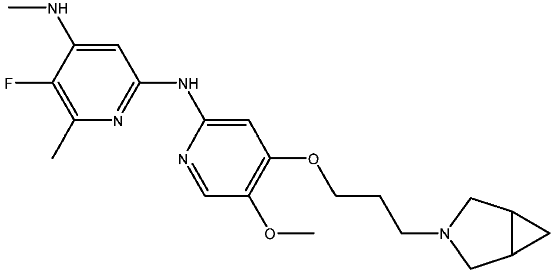 |
| 510 | 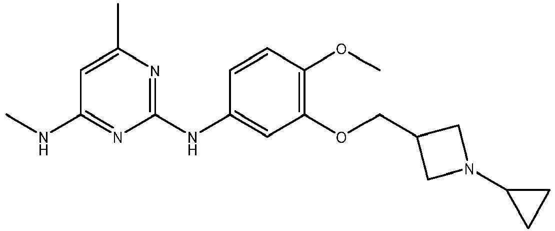 |
| 511 | 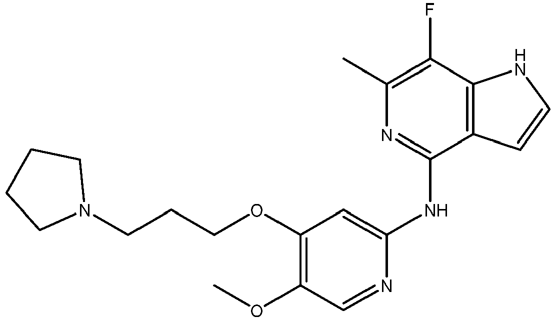 |
| 512 | 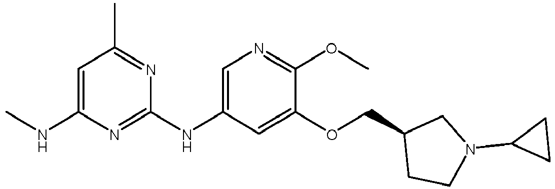 |
| 513 | 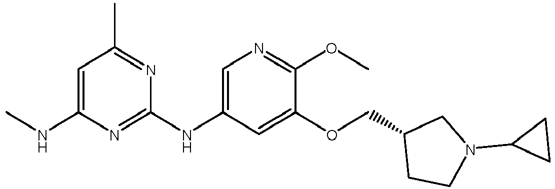 |
| 514 | 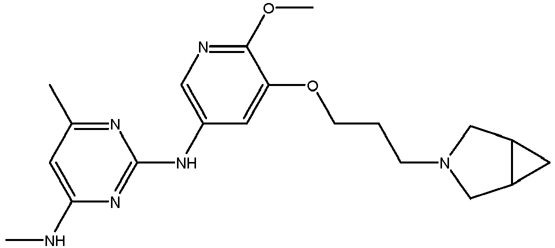 |
| 515 | 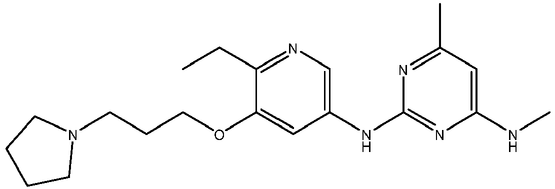 |
| 516 | 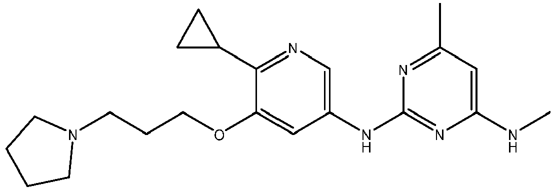 |
| 517a | 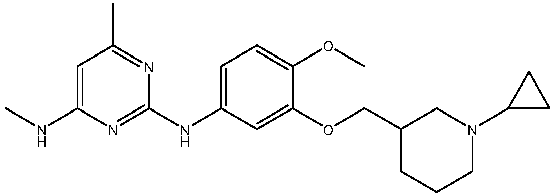 |
| 517b | 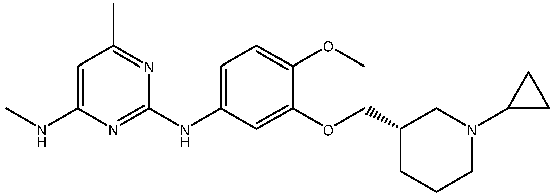 |

**Table 3**

| **Compound No.** | **Structure** |
|---|---|
| 270 | 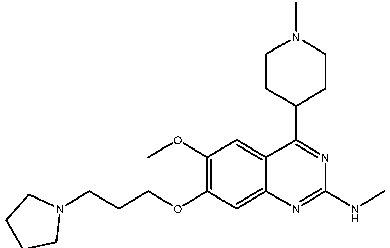 |
| 518 | 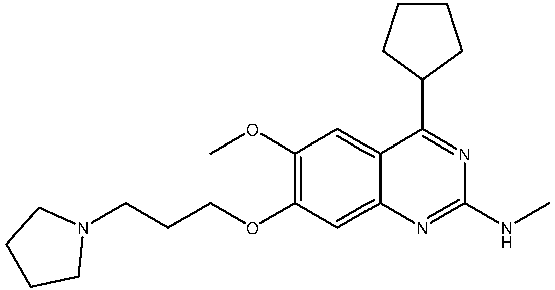 |
| 519 | 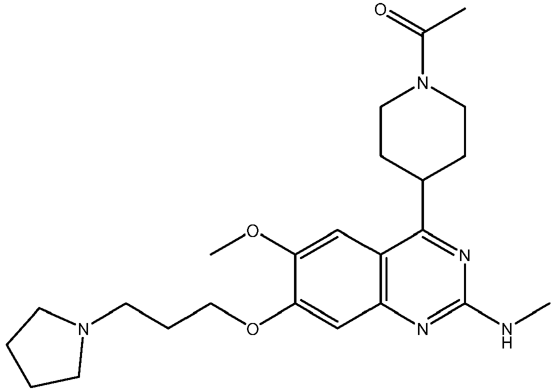 |
| 520 | 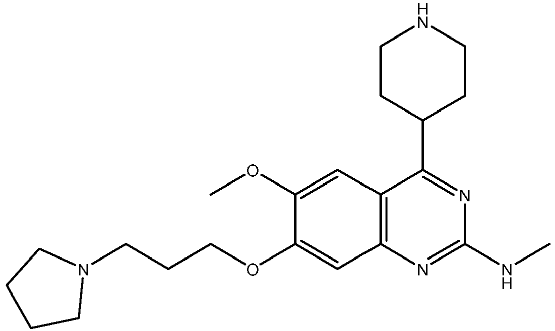 |
| 521 | 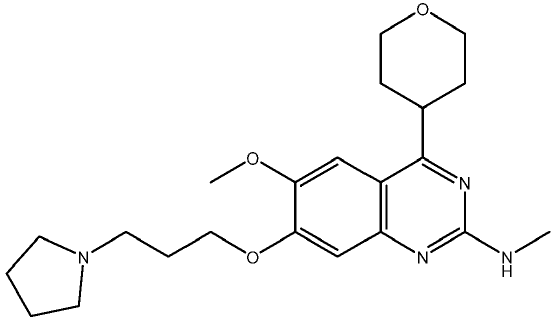 |
| 522 | 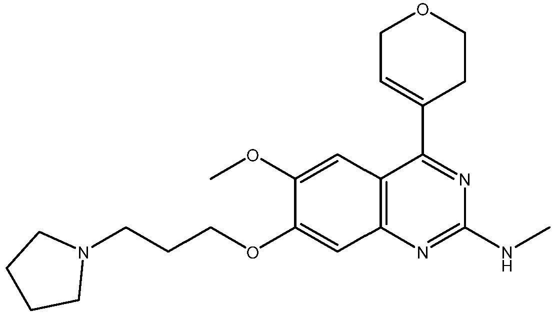 |
| 523 | 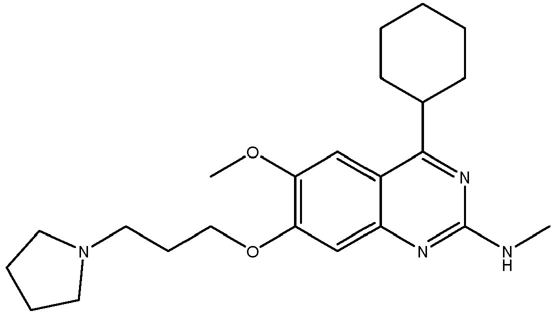 |
| 524 | 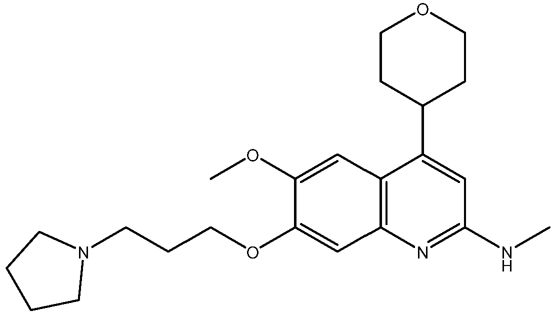 |
| 525 | 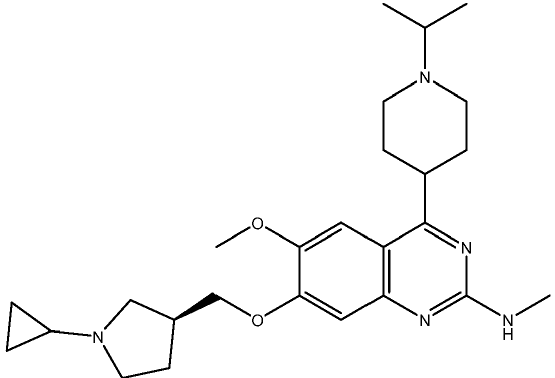 |
| 526 | 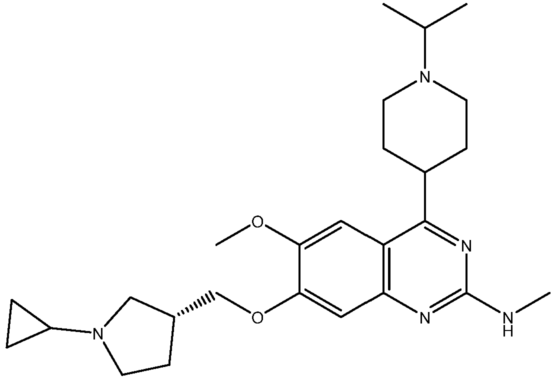 |
| 527 | 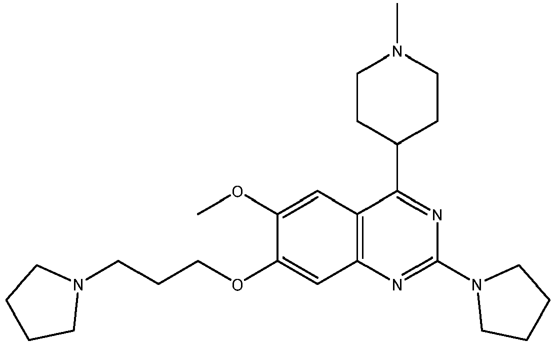 |
| 528 | 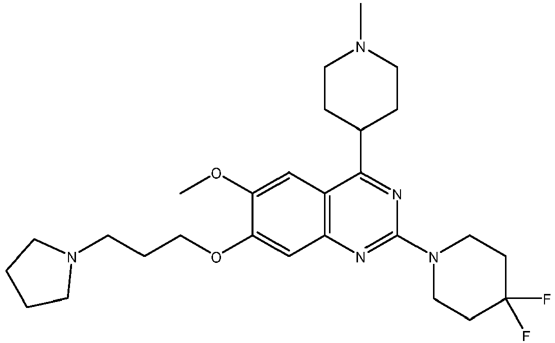 |
| 529 | 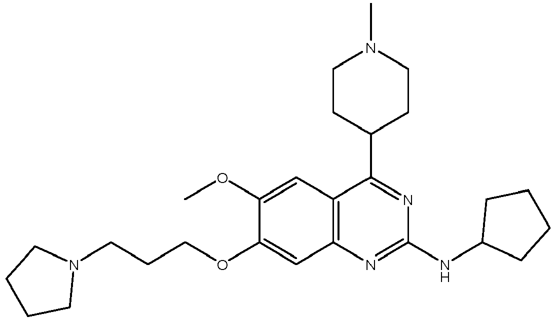 |
| 530 | 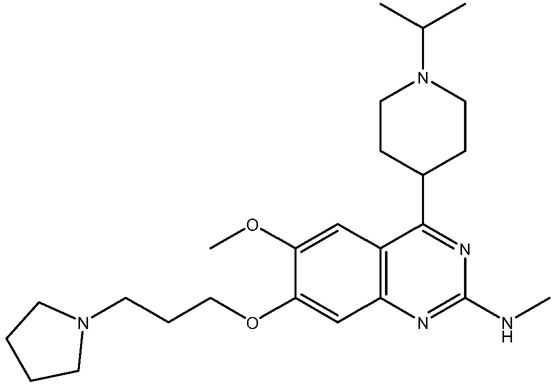 |
| 531 | 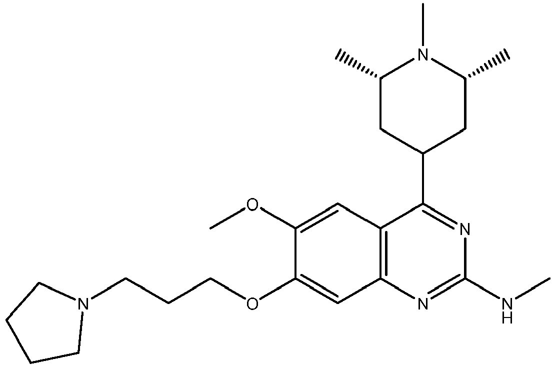 |
| 532 | 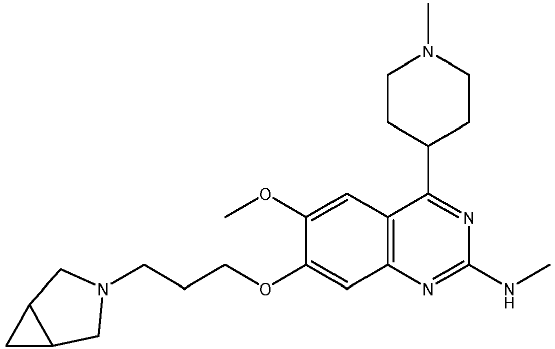 |
| 533 | 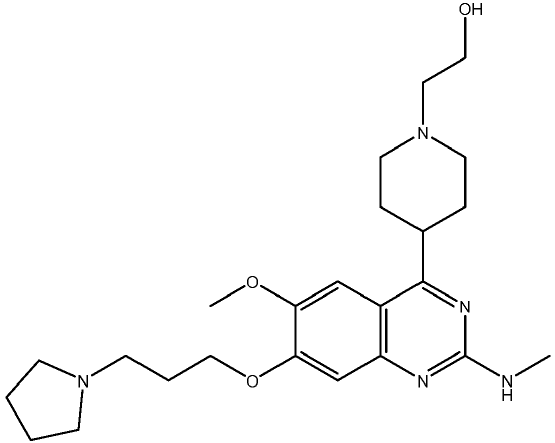 |
| 534 | 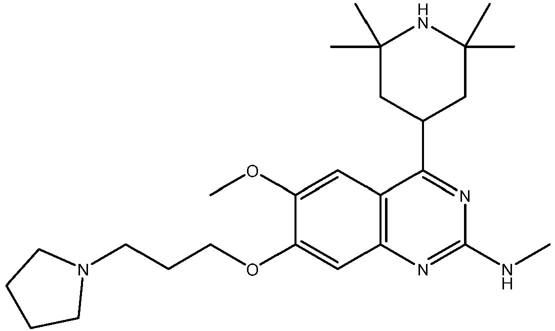 |
| 535 | 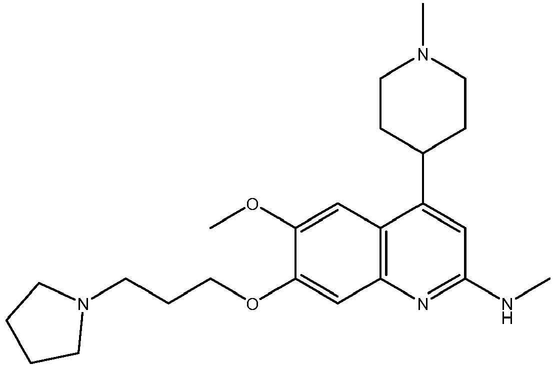 |
| 536 | 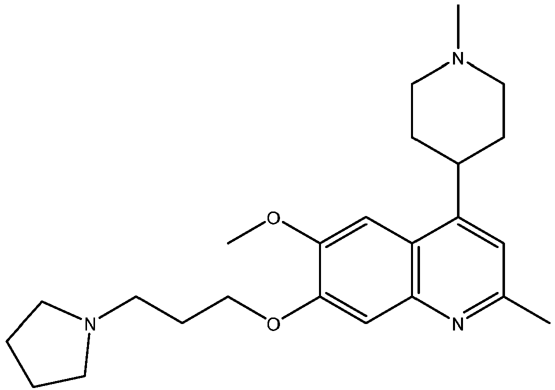 |
| 537 | 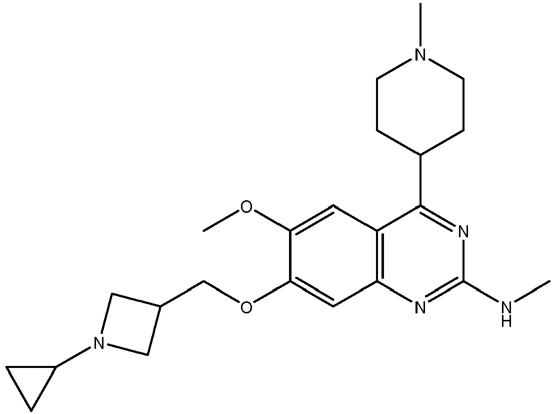 |
| 538 | 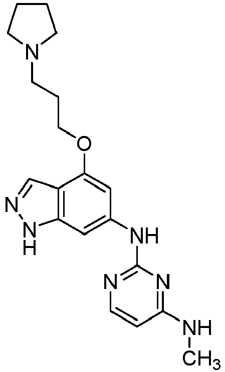 |
| 539 | 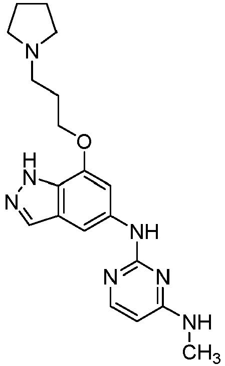 |
| 540 | 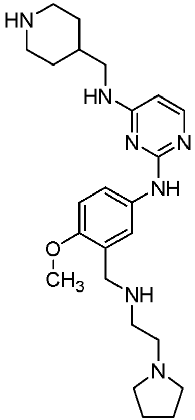 |
| 541 | 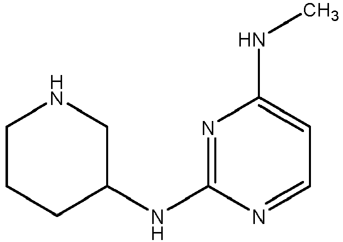 |
| 542 | 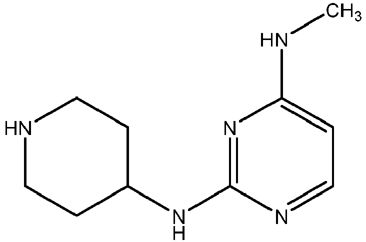 |
| 543 | 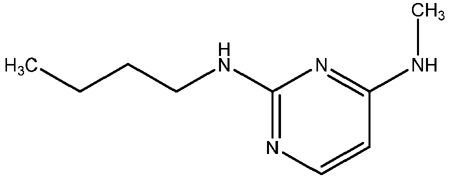 |
| 544 | 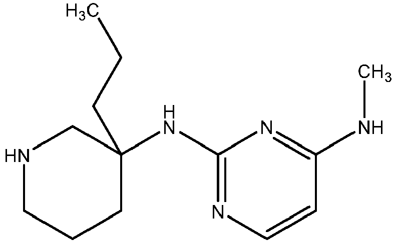 |
| 545 | 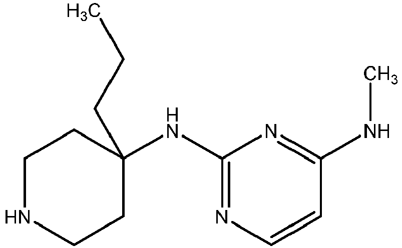 |
| 546 | 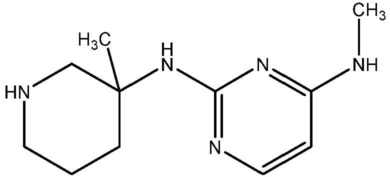 |
| 547 | 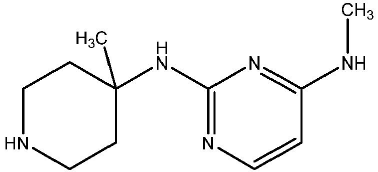 |
| 548 | 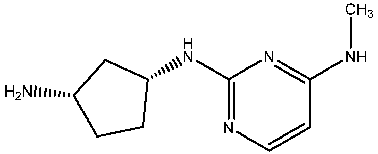 |
| 549 | 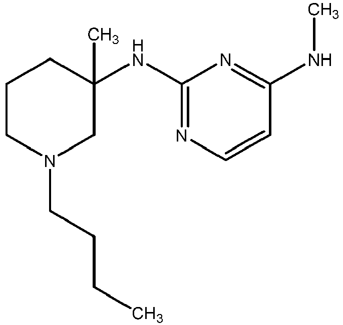 |
| 550 | 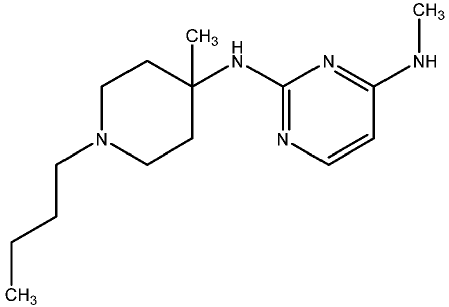 |
| 551 | 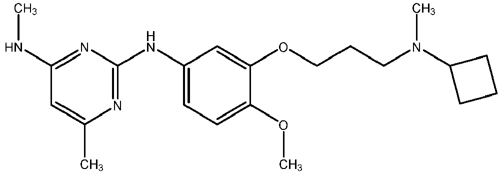 |
| 552 | 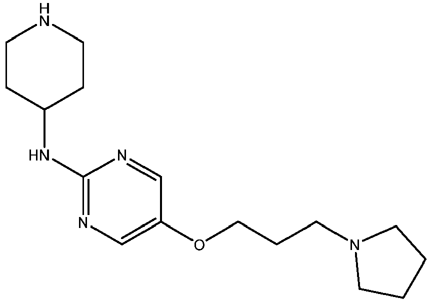 |
| 553 | 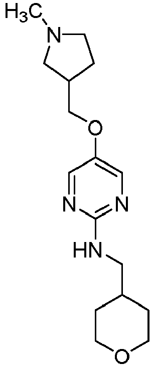 |
| 554 | 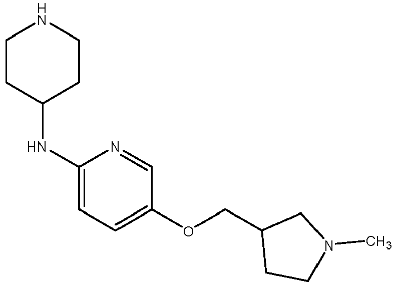 |
| 555 | 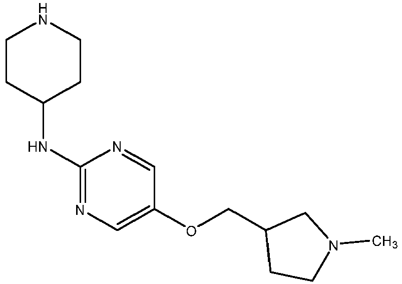 |
| 556 | 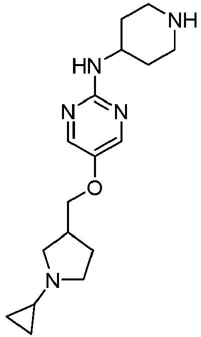 |
| 557 | 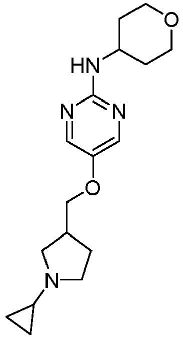 |
| 558 | 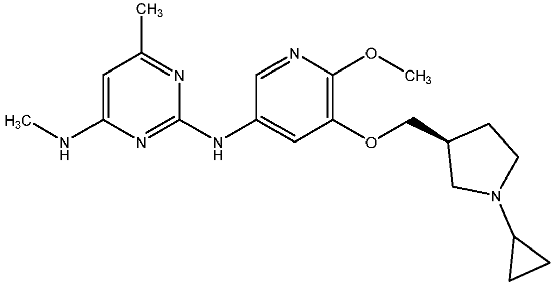 |
| 559 | 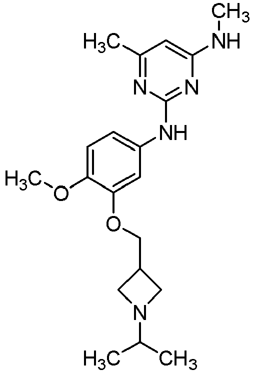 |
| 560 | 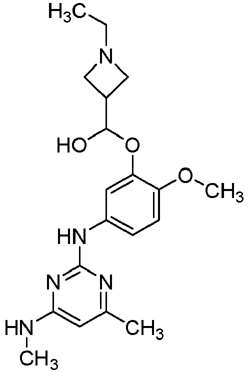 |
| 561 | 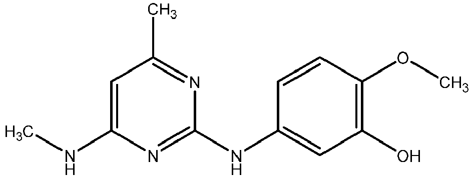 |
| 562 | 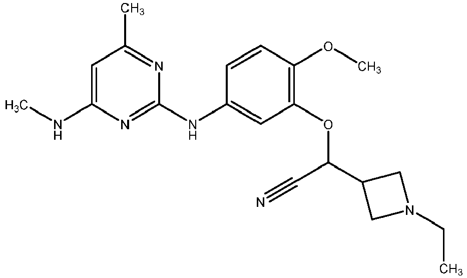 |
| 563 | 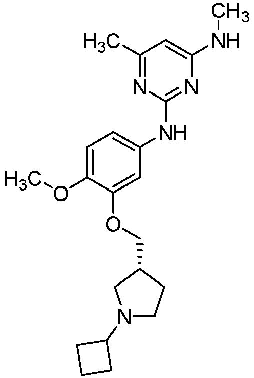 |
| 564 | 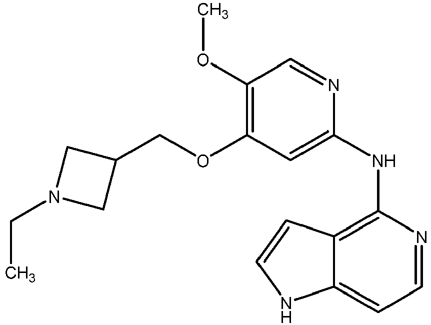 |
| 565 | 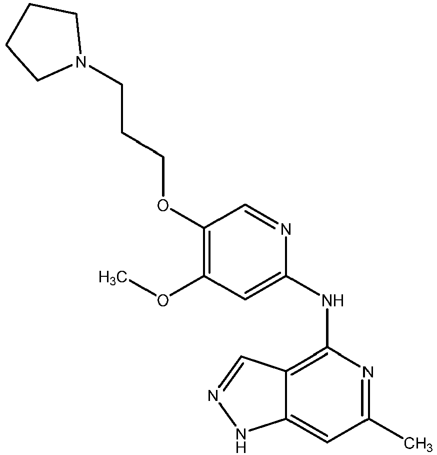 |
| 566 | 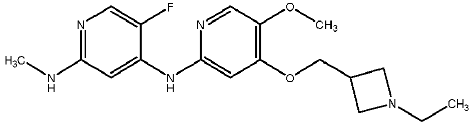 |
| 567 | 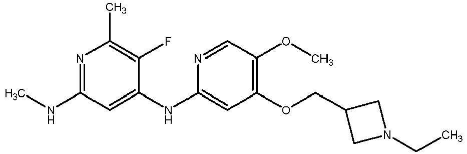 |
| 568 | 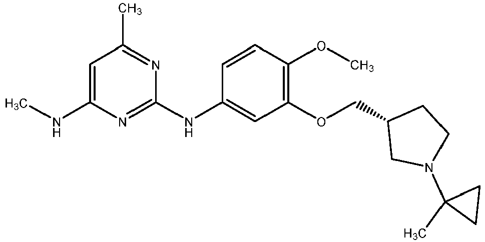 |
| 569 | 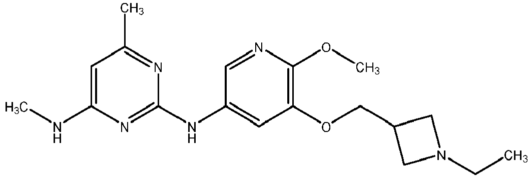 |
| 570 | 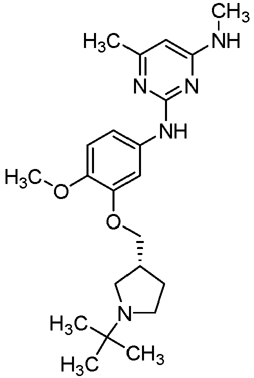 |
| 571 | 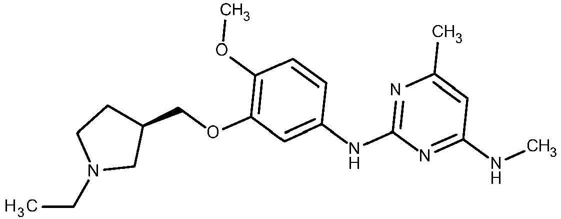 |
| 572 | 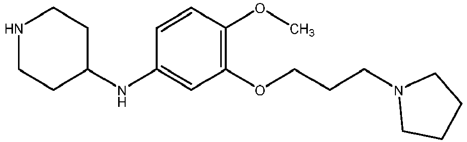 |
| 573 | 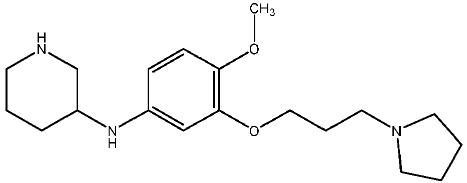 |
| 574 | 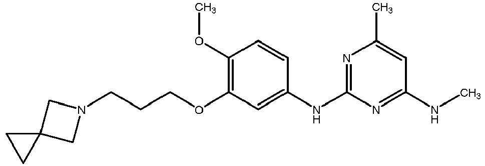 |
| 575 | 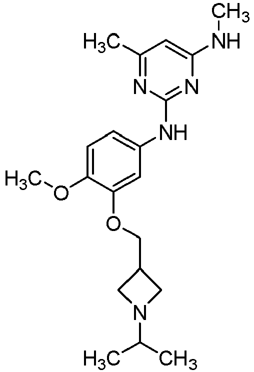 |
| 576 | 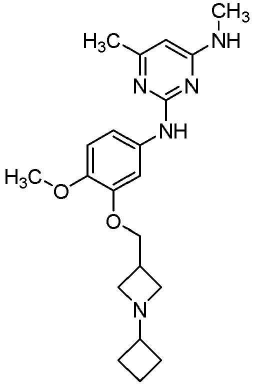 |
| 577 | 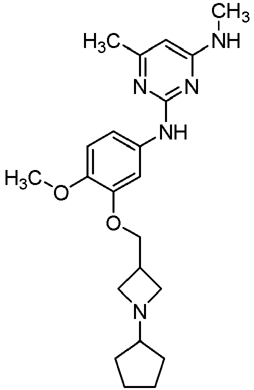 |
| 578 | 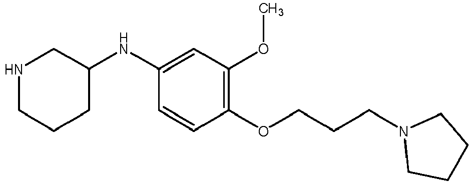 |
| 579 | 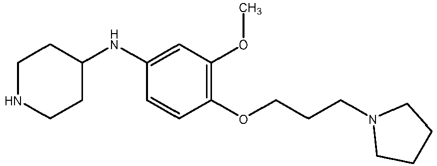 |
| 580 | 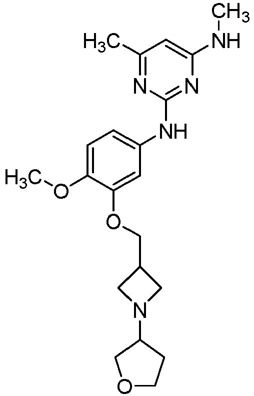 |
| 581 | 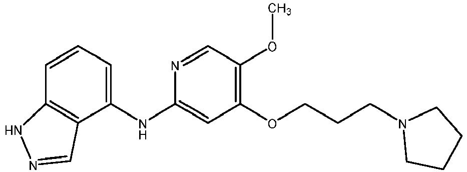 |
| 582 | 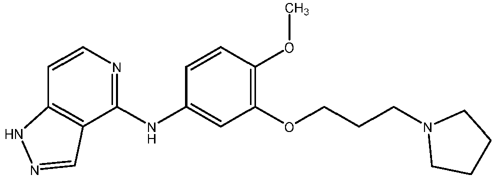 |
| 583 | 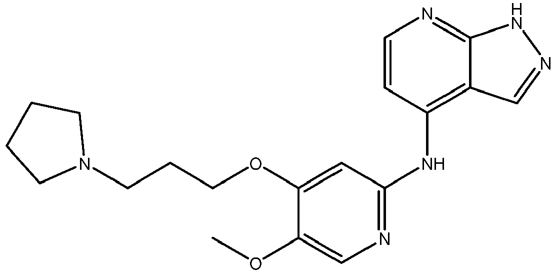 |
| 584 | 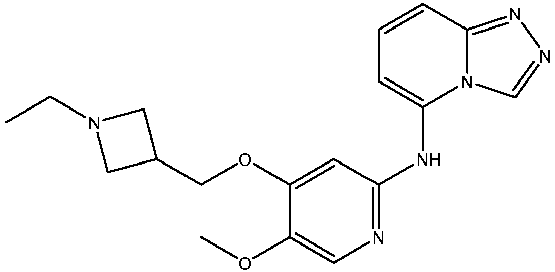 |
| 585 | 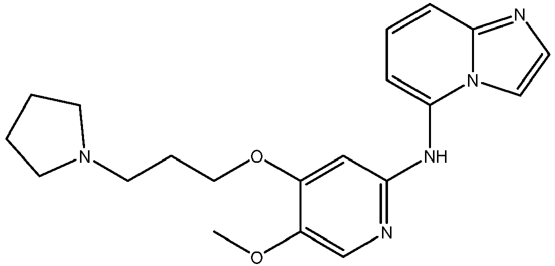 |
| 586 | 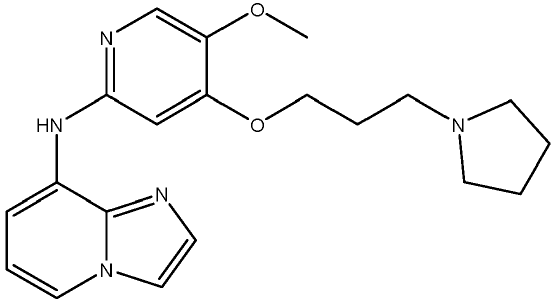 |
| 587 | 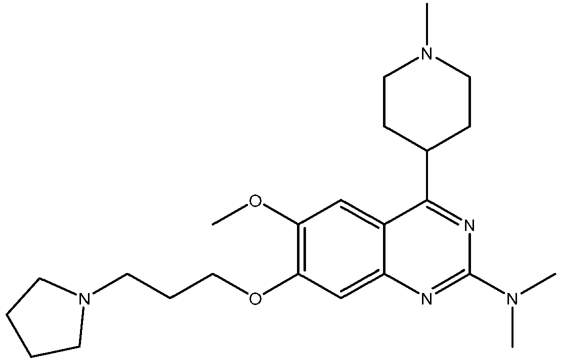 |
| 588 | 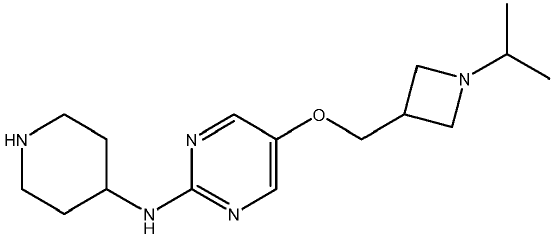 |
| 589 | 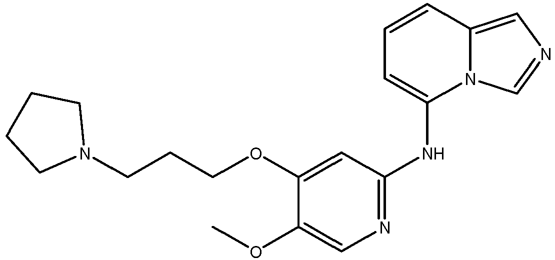 |
| 590 | 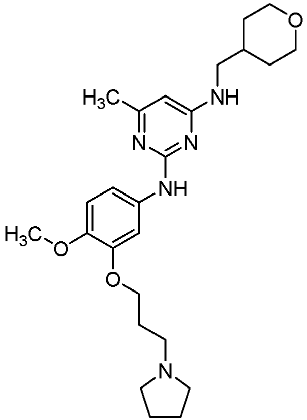 |
| | 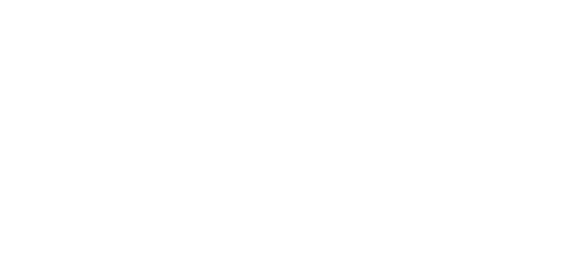 |
| 591 | 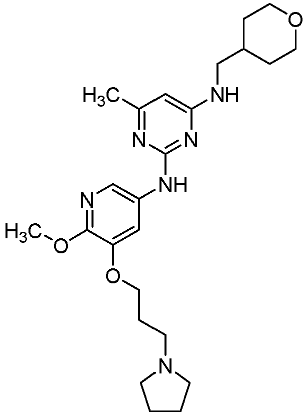 |
| 592 | 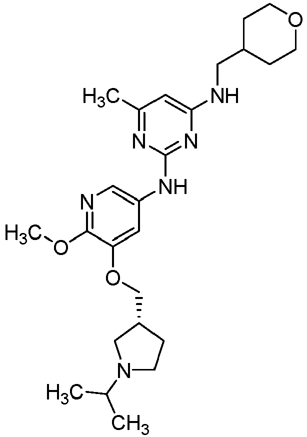 |
| 593 | 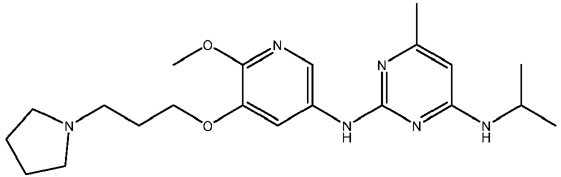 |
| 594 | 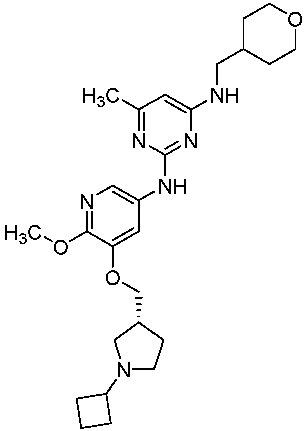 |
| 595 | 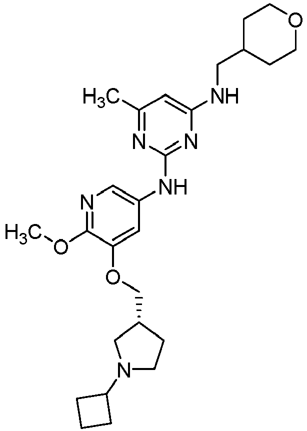 |
| 596 | 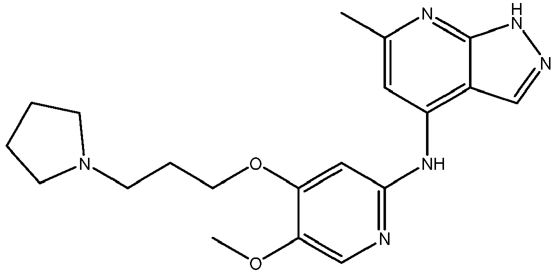 |
| 597 | 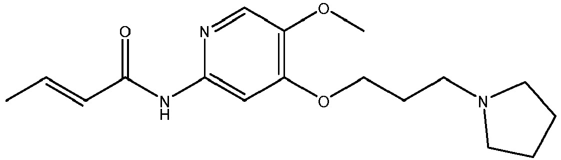 |
| 598 | 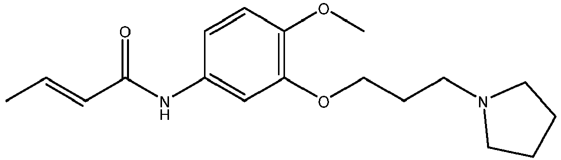 |
| 599 | 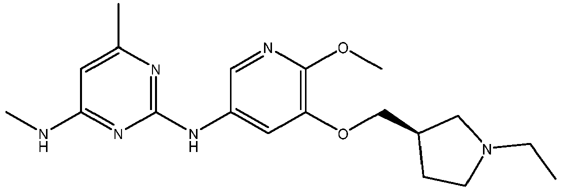 |
| 600 | 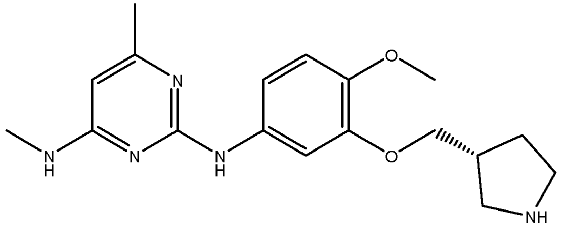 |
| 601 | 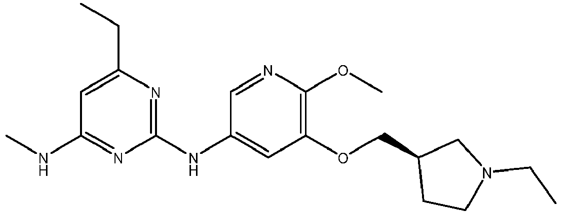 |
| 602 | 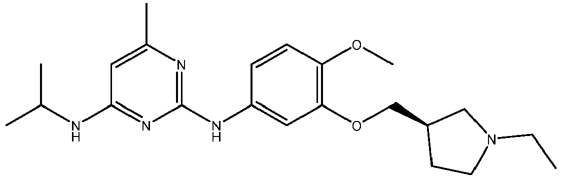 |
| 603 | 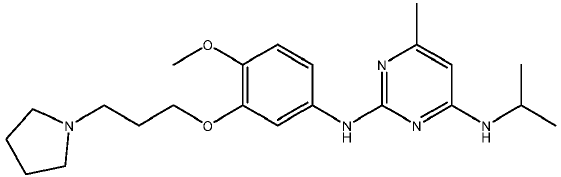 |
| 604 | 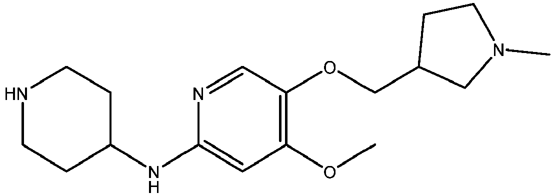 |
| 605 | 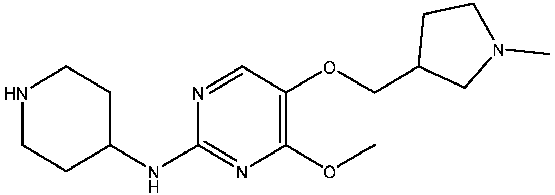 |
| 606 | 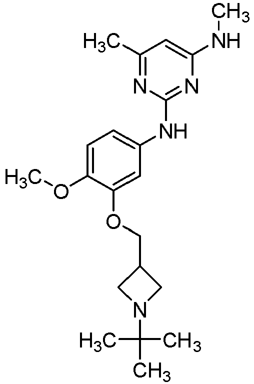 |
| 607 | 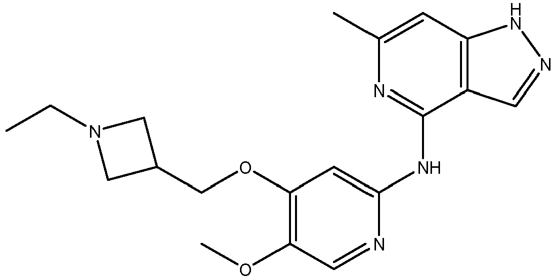 |
| 608 | 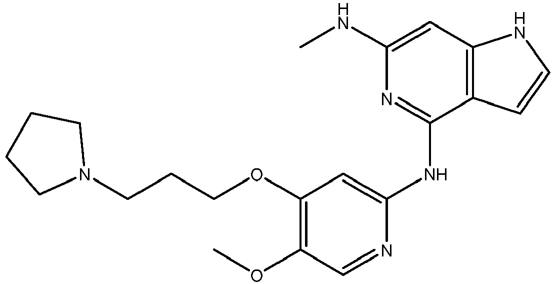 |
| 609 | 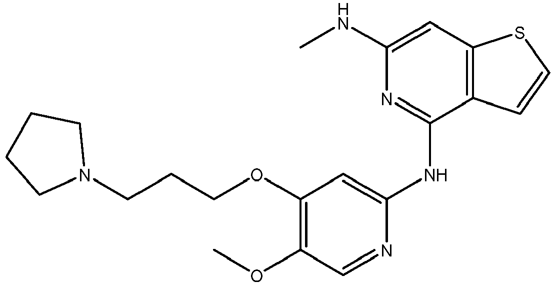 |
| 610 | 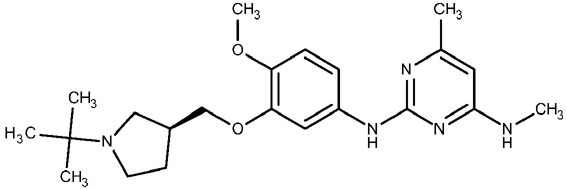 |
| 611 | 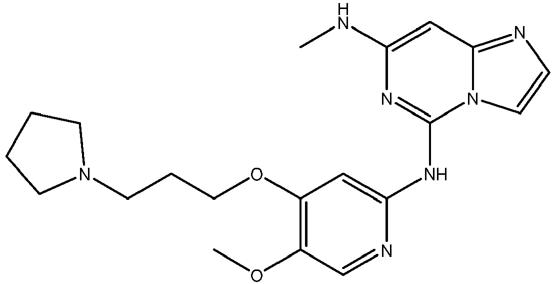 |
| 612 | 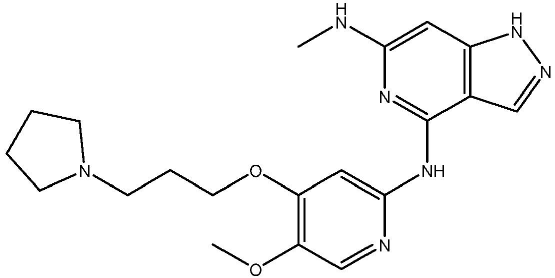 |
| 613 | 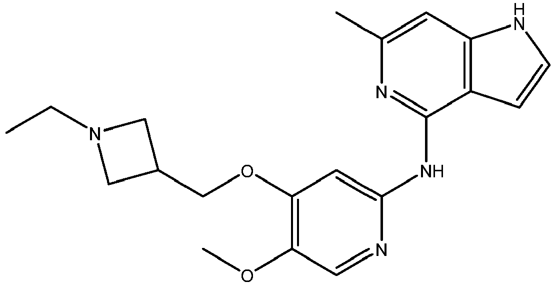 |
| 614 | 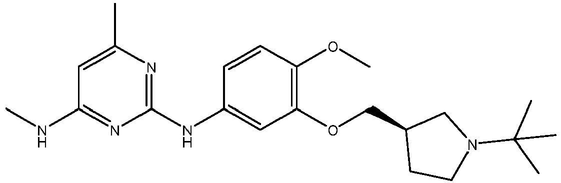 |
| 616 | 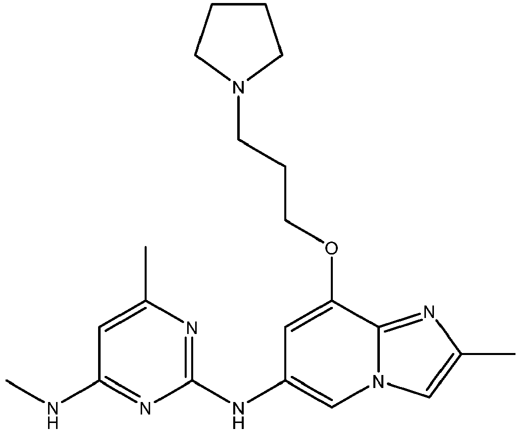 |
| 617 | 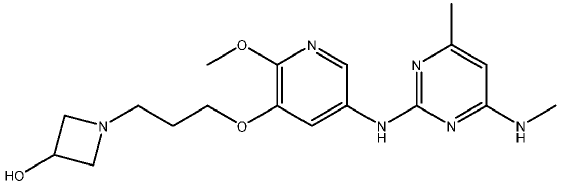 |
| 618 | 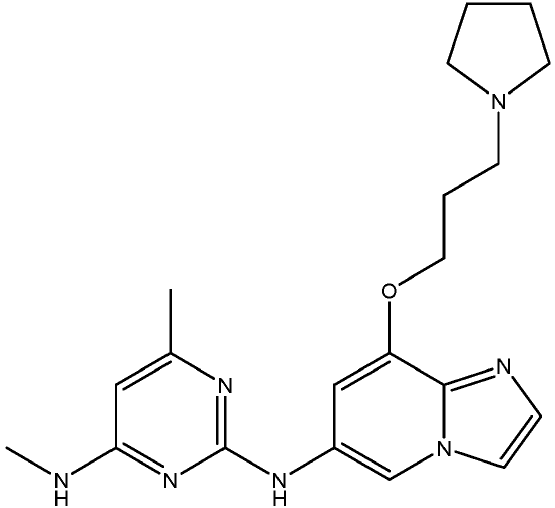 |
| 619 | 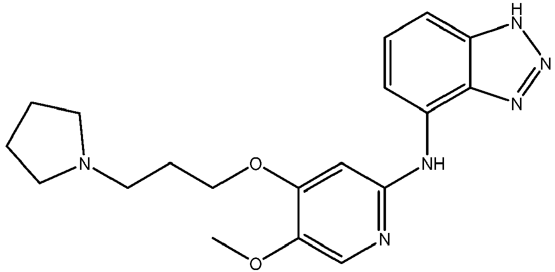 |
| 620 | 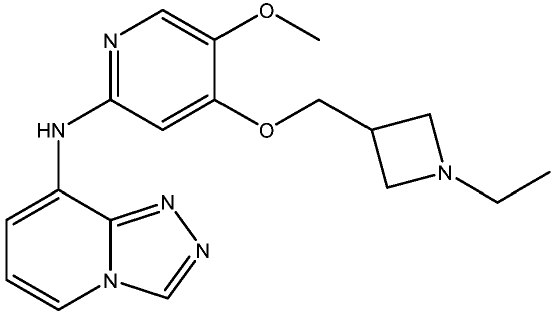 |
| 621 | 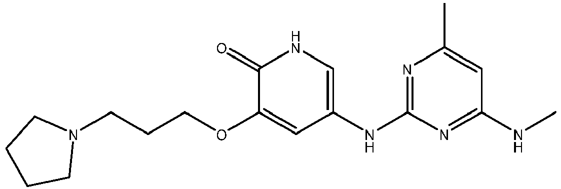 |
| 622 | 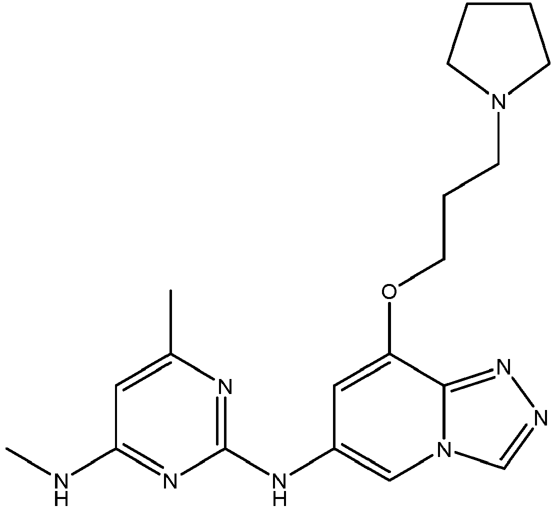 |
| 623 | 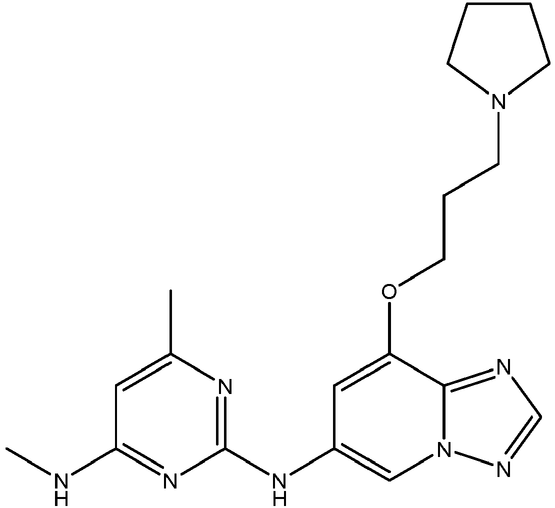 |
| 624 | 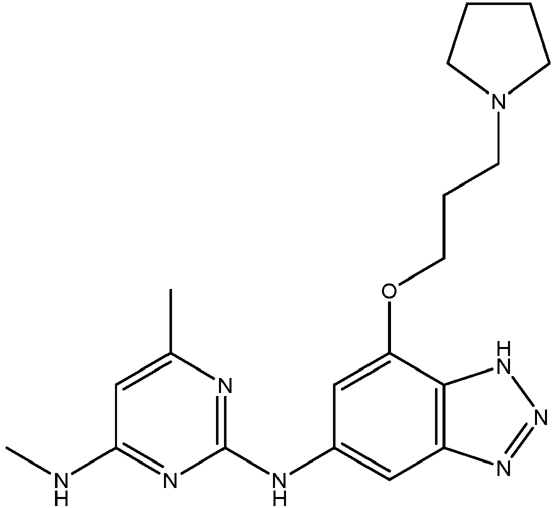 |
| 625 | 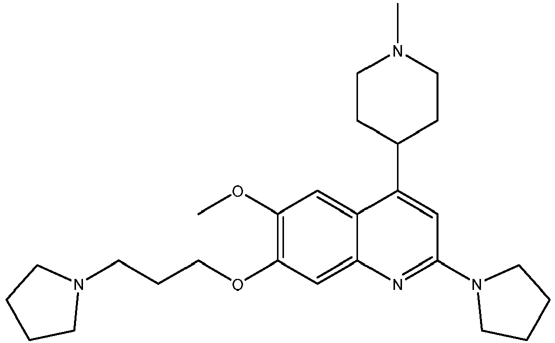 |
| 626 | 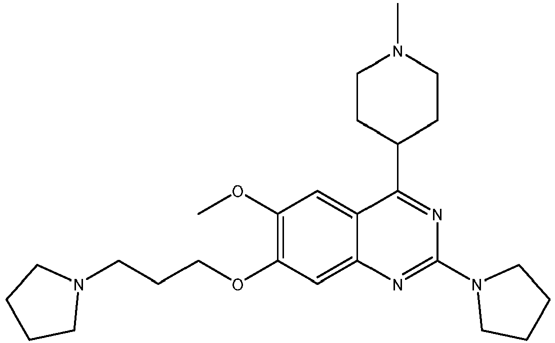 |
| 627 | 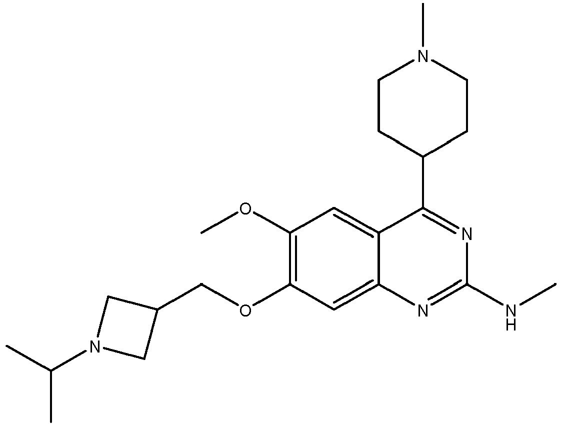 |
| 628 | 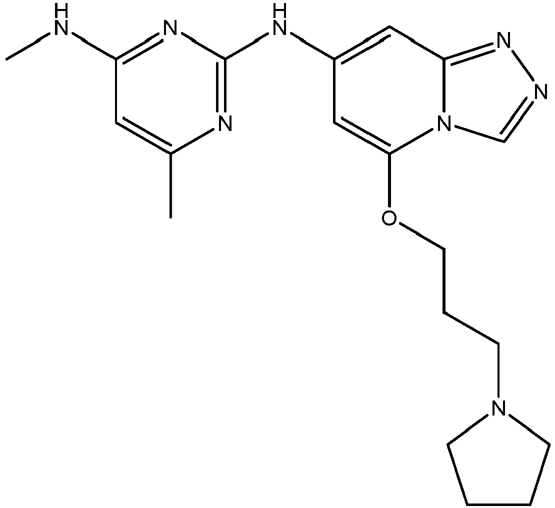 |
| 629 | 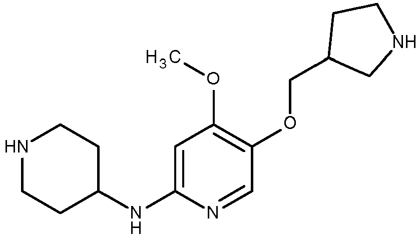 |
| 630 | 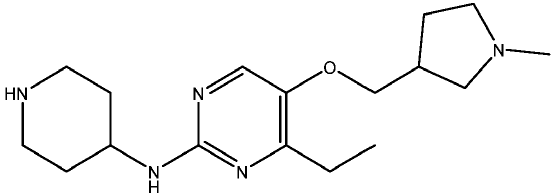 |
| 631 | 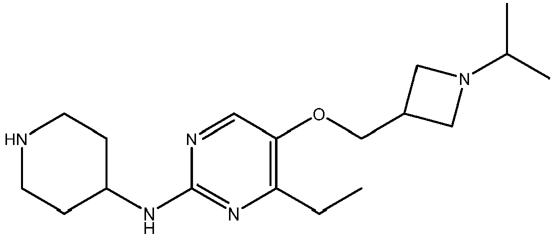 |
| 632 | 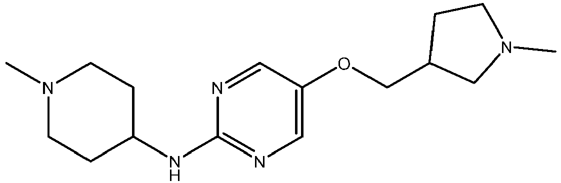 |
| 633 | 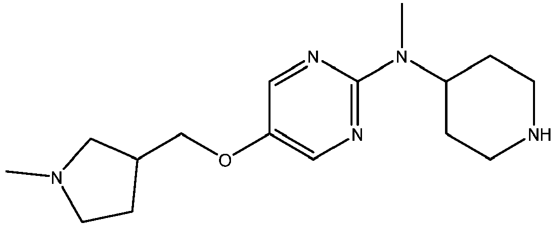 |
| 634 | 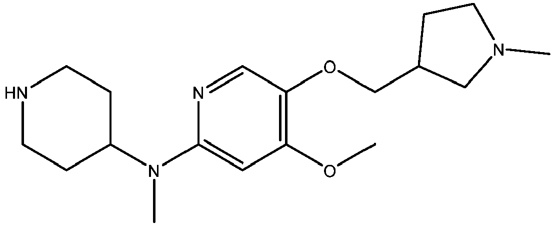 |
| 635 | 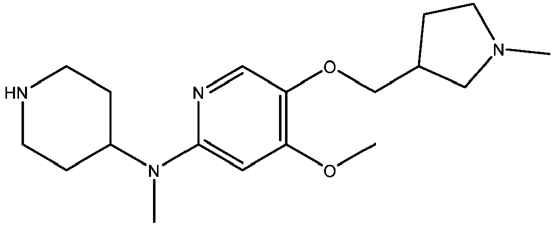 |
| 636 | 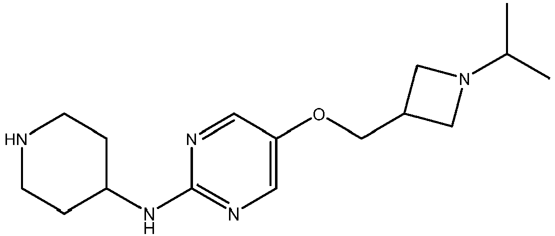 |
| 637 | 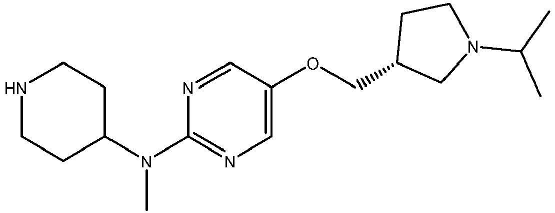 |
| 638 | 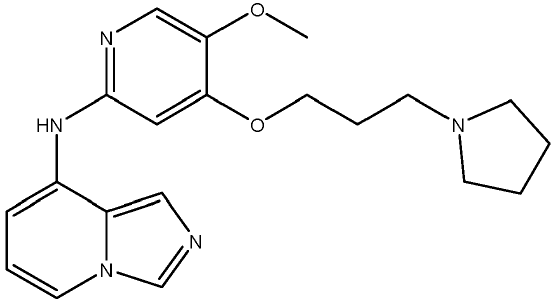 |
| 639 | 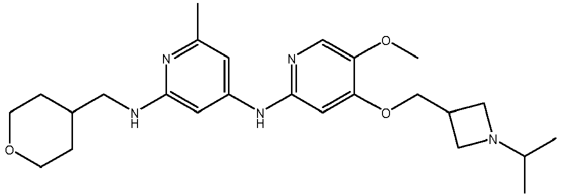 |
| 640 | 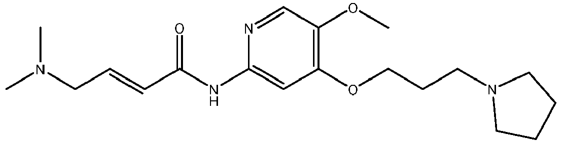 |
| 641 | 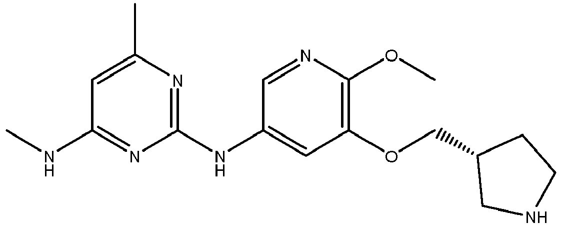 |
| 642 | 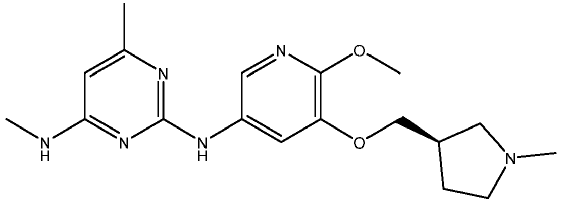 |
| 643 | 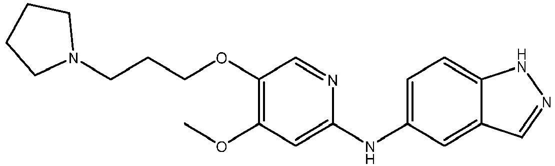 |
| 644 | 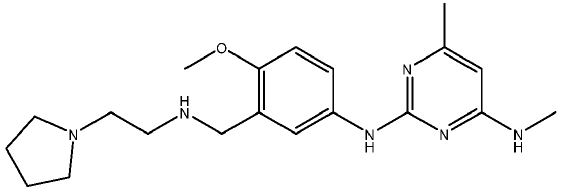 |
| 645 | 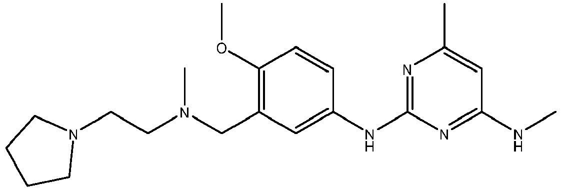 |
| 646 | 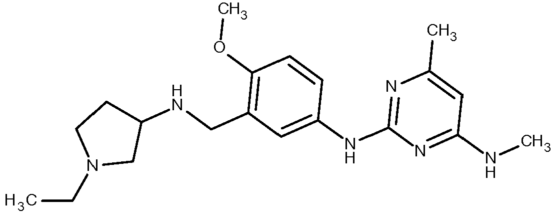 |
| 647 | 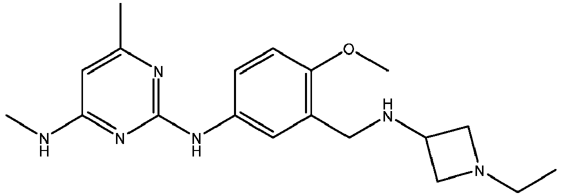 |
| 648 | 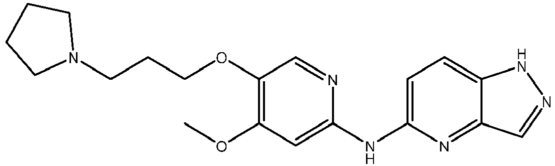 |
| 649 | 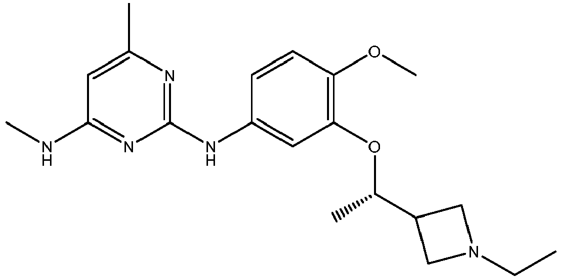 |
| 650 | 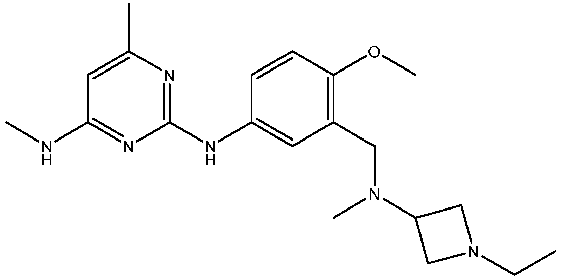 |
| 651 | 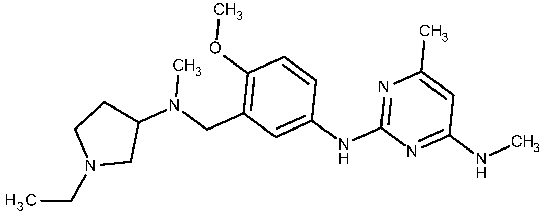 |
| 652 | 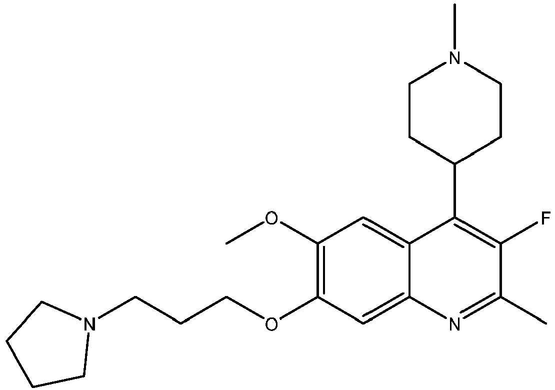 |
| 653 | 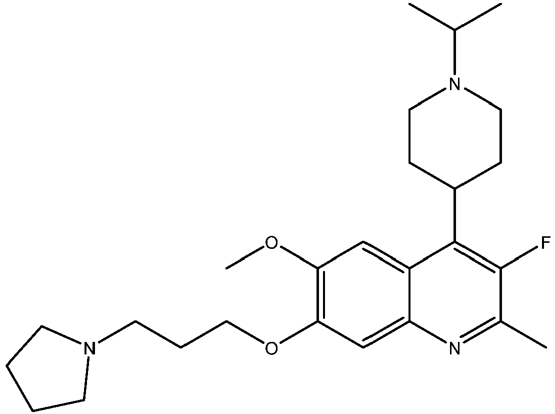 |
| 654 | 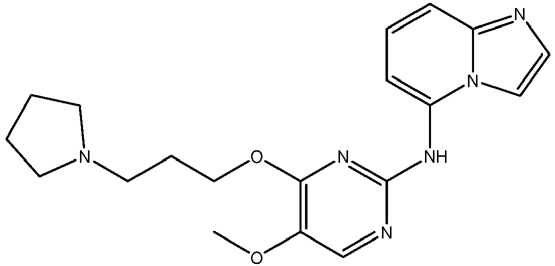 |
| 655 | 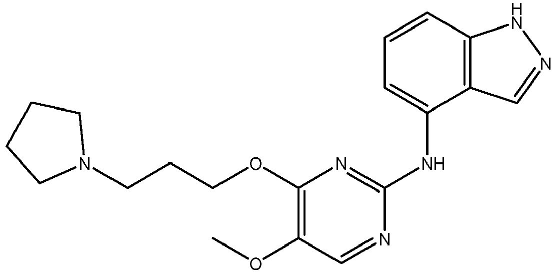 |
| 656 | 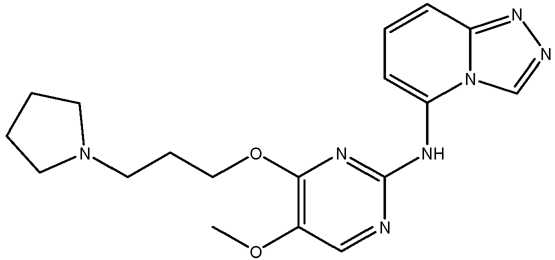 |
| 657 | 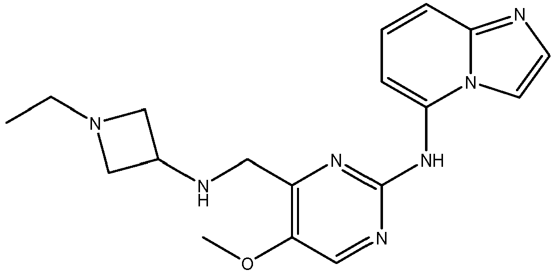 |
| 658 | 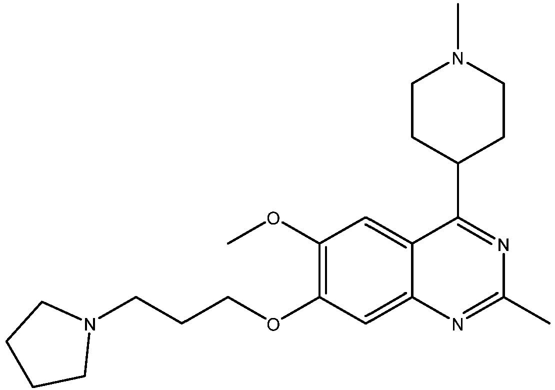 |
| 659 | 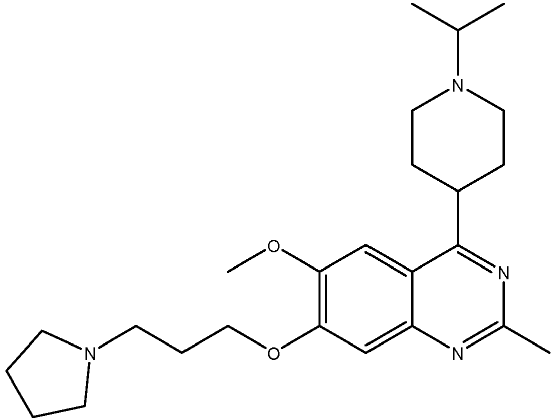 |
| 660 | 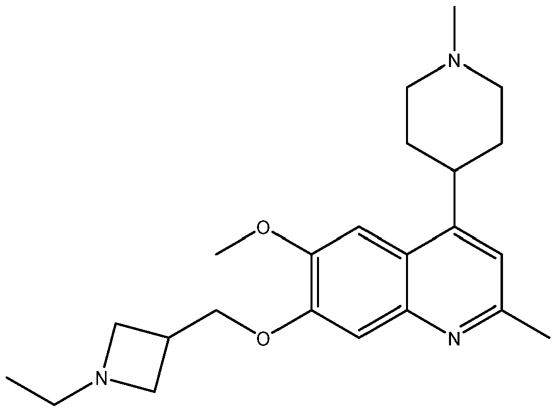 |
| 661 | 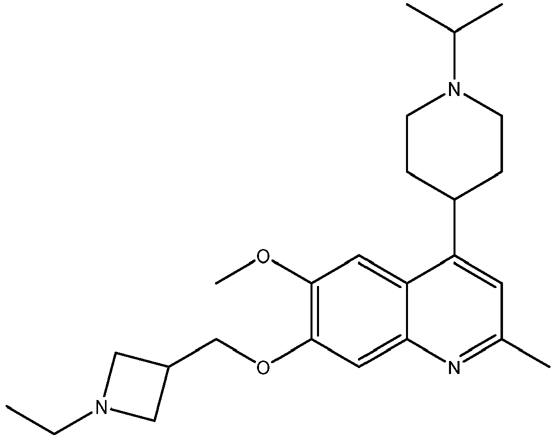 |
| 662 | 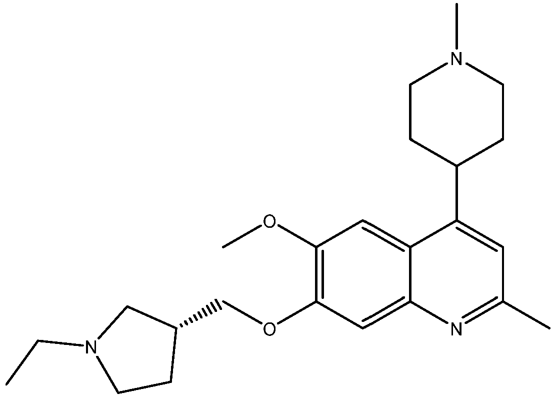 |
| 663 | 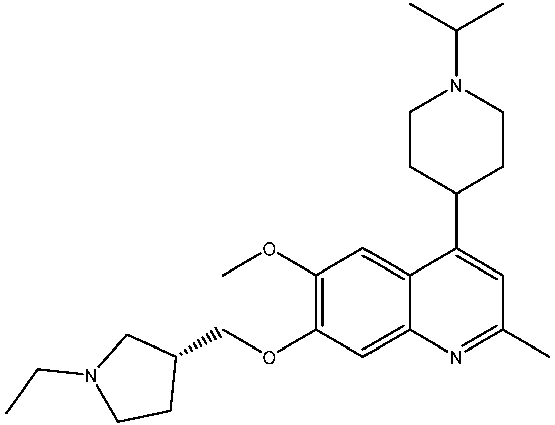 |
| 664 | 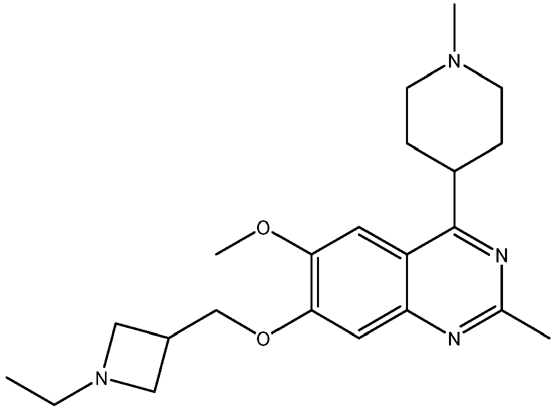 |
| 665 | 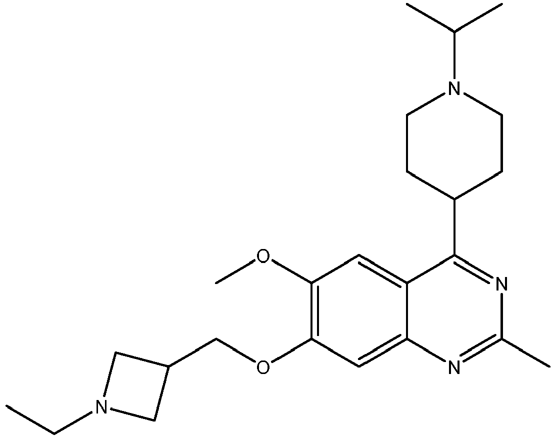 |
| 666 | 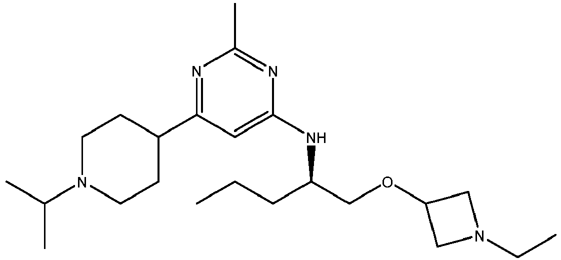 |
| 667 | 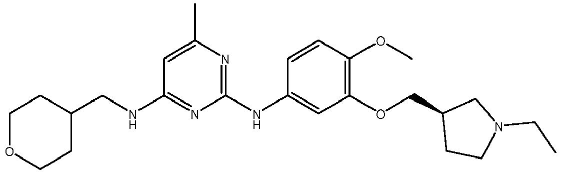 |
| 668 | 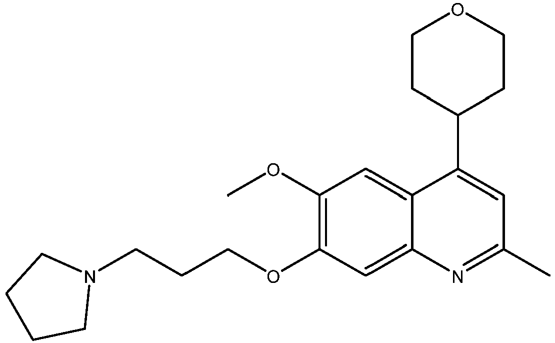 |
| 669 | 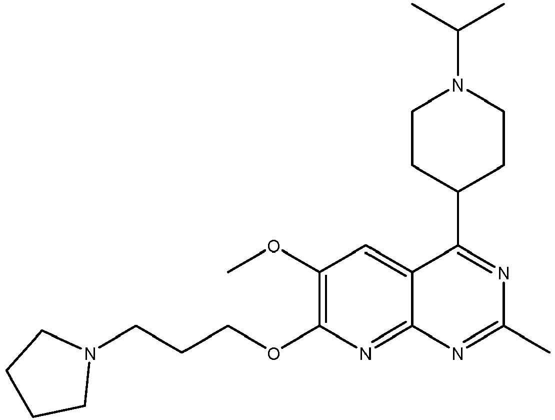 |
| 670 | 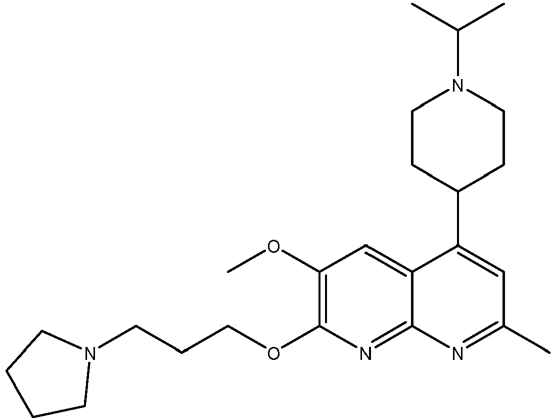 |
| 671 | 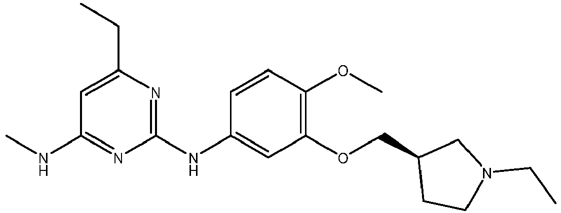 |
| 672 | 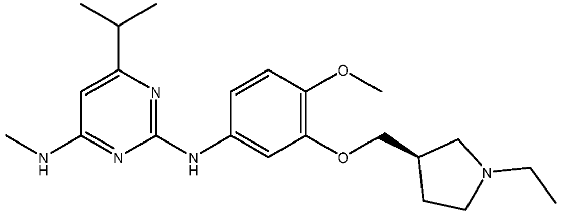 |
| 673 | 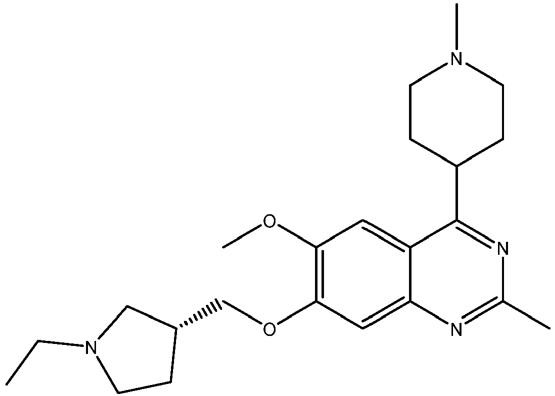 |
| 674 | 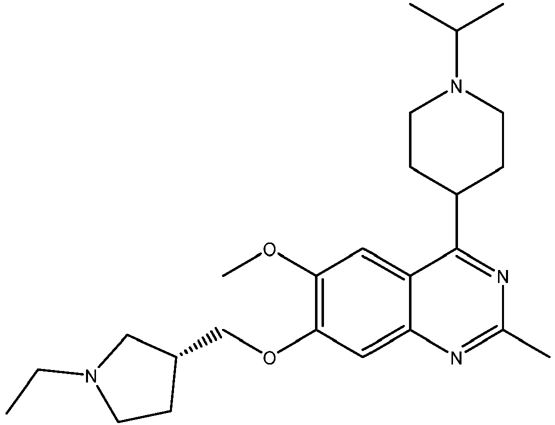 |
| 675 | 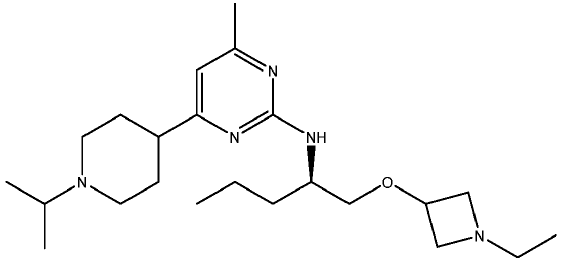 |
| 676 | 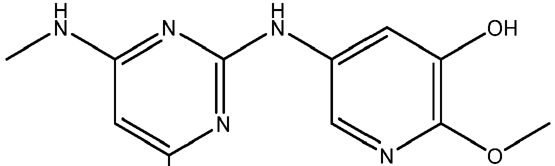 |
| 677 | 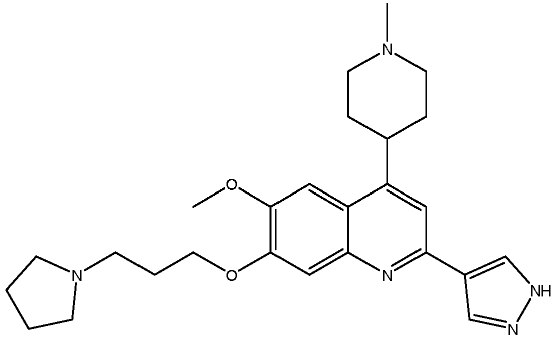 |
| 678 | 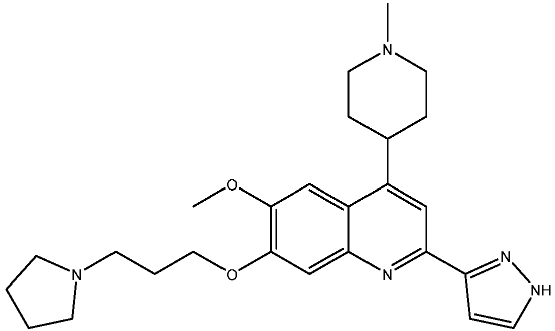 |
| 679 | 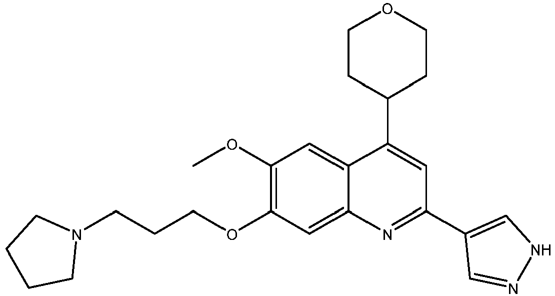 |
| 680 | 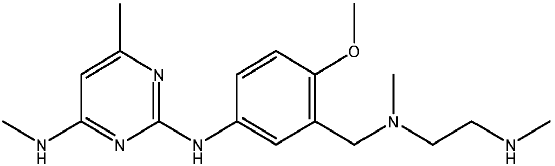 |
| 681 | 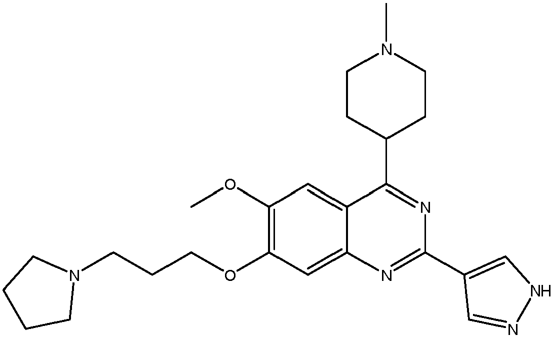 |
| 682 | 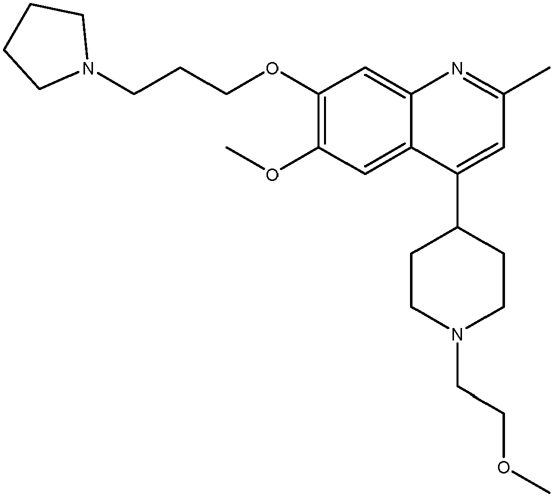 |
| 683 | 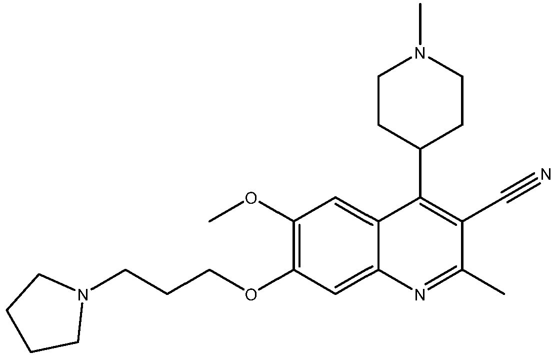 |
| 684 | 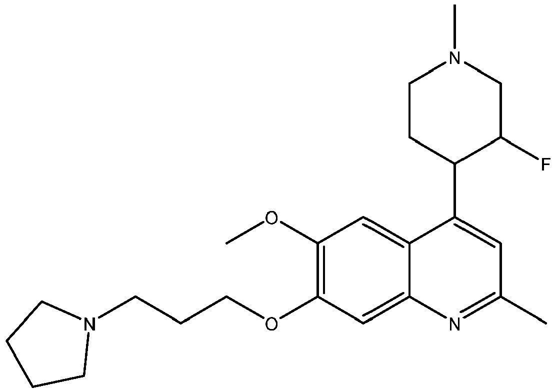 |
| 685 | 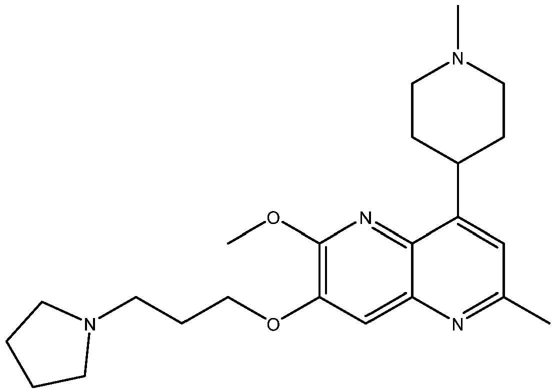 |
| 686 | 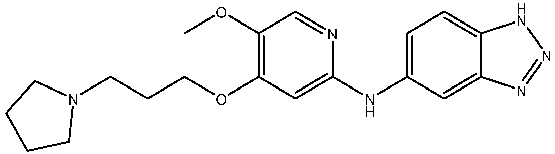 |
| 687 | 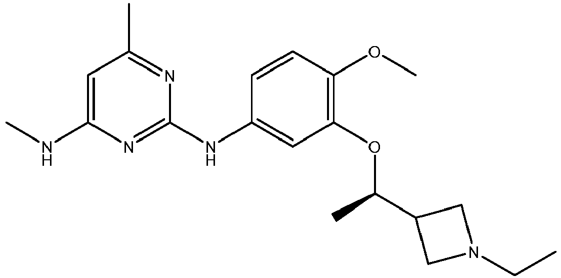 |
| 688 | 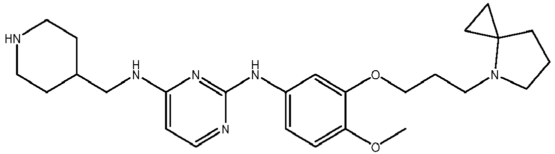 |
| 689 | 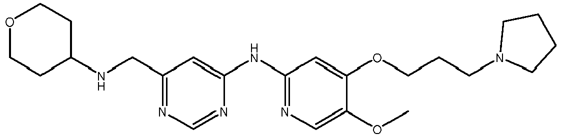 |
| 690 | 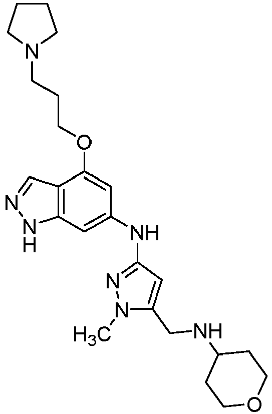 |
| 691 | 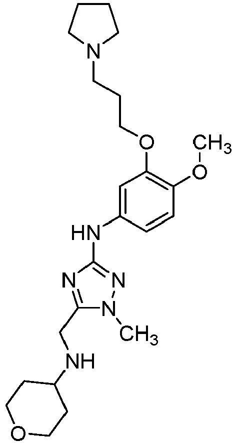 |
| 692 | 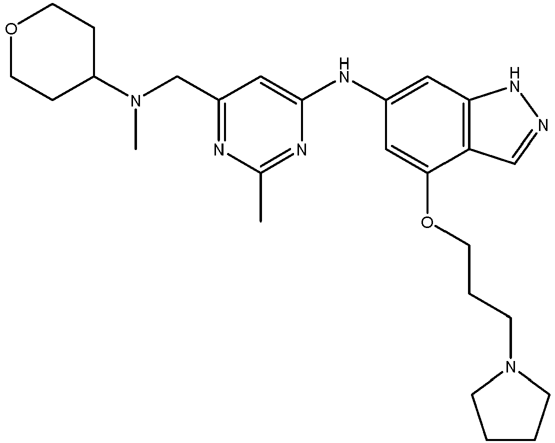 |
| 693 | 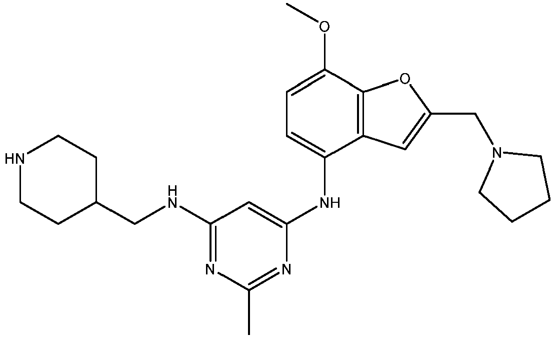 |
| 694 | 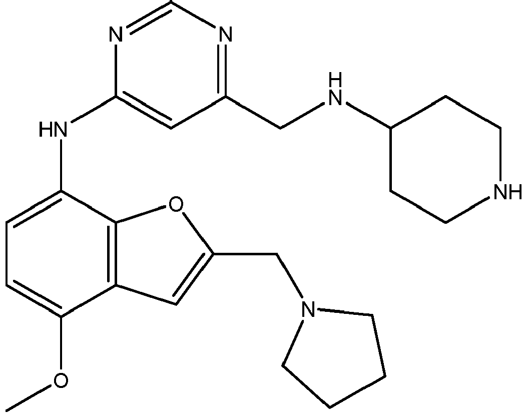 |
| 695 | 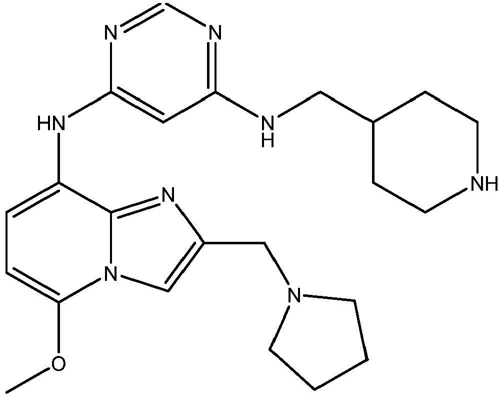 |
| 696 | 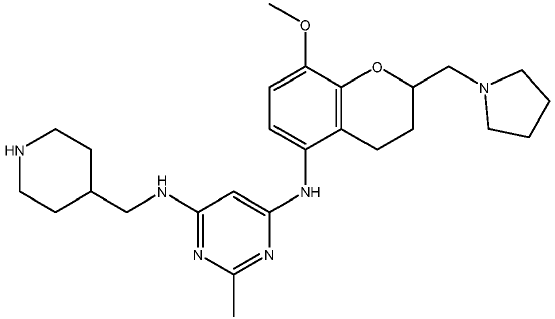 |
| 697 | 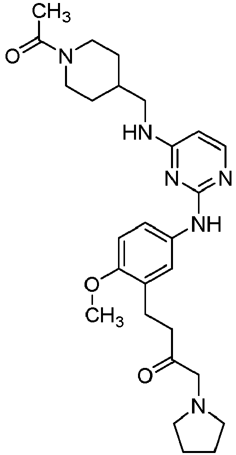 |
| 698 | 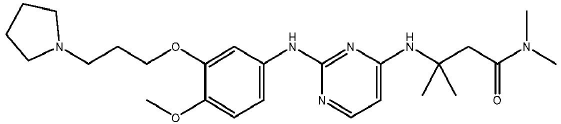 |
| 699 | 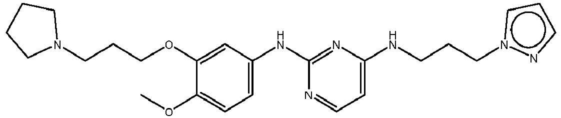 |
| 700 | 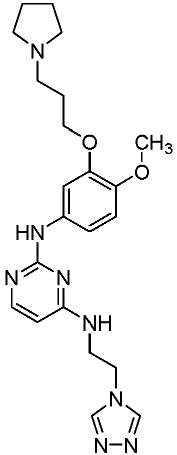 |
| 701 | 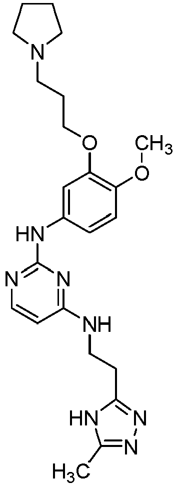 |
| 702 | 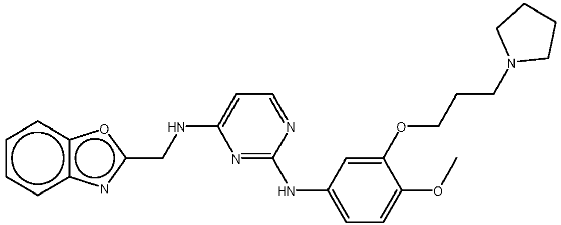 |
| 703 | 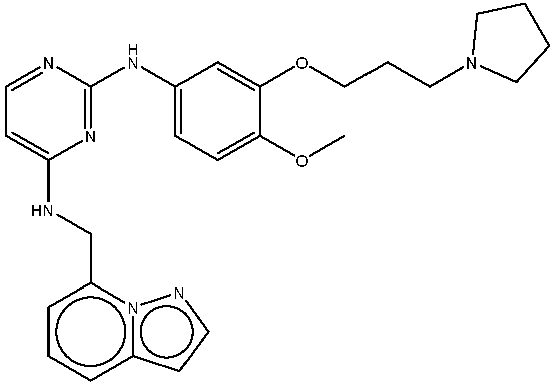 |
| 704 | 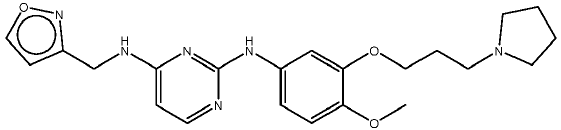 |
| 705 | 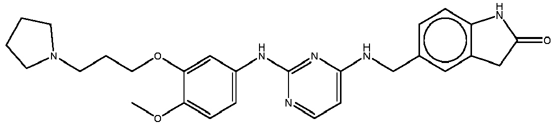 |
| 706 | 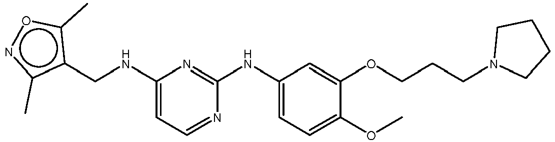 |
| 707 | 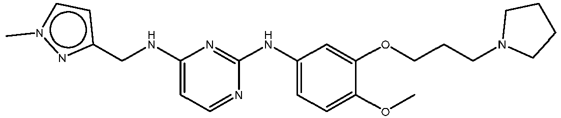 |
| 708 | 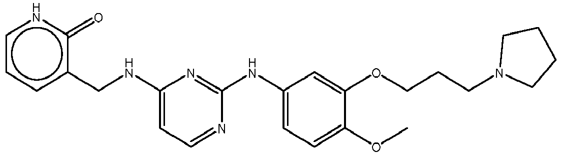 |
| 709 | 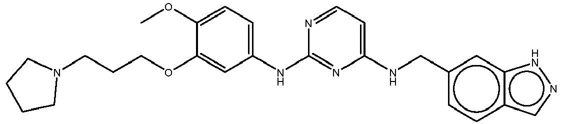 |
| 710 | 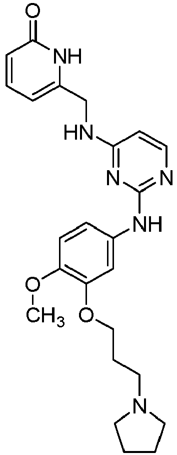 |
| 711 | 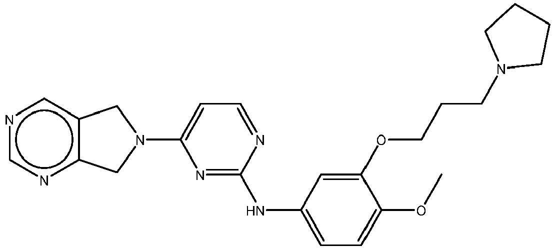 |
| 712 | 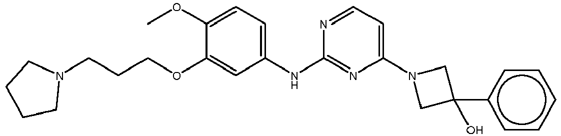 |
| 713 | 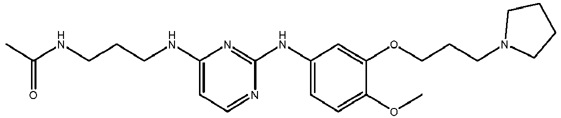 |
| 714 | 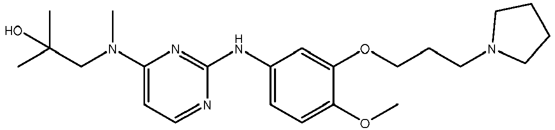 |
| 715 | 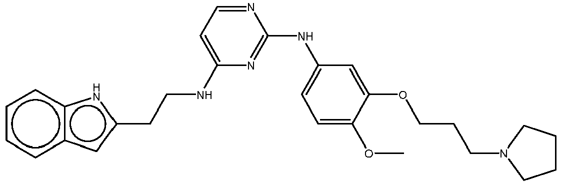 |
| 716 | 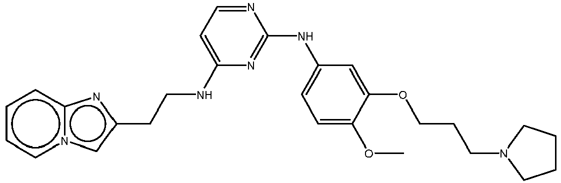 |
| 717 | 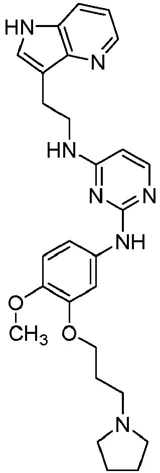 |
| 718 | 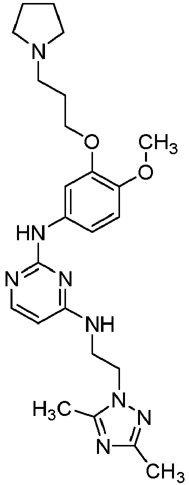 |
| 719 | 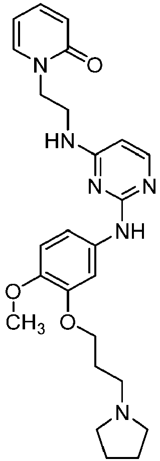 |
| 720 | 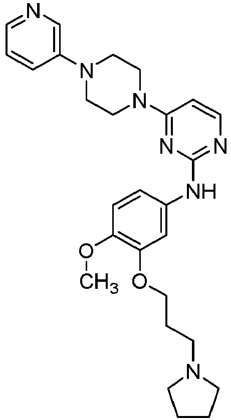 |
| 721 | 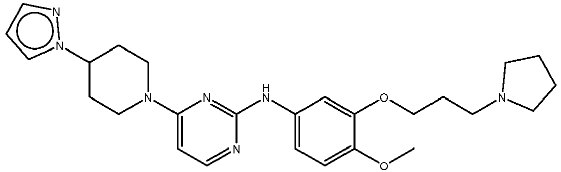 |
| 722 | 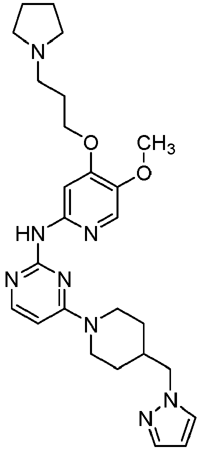 |
| 723 | 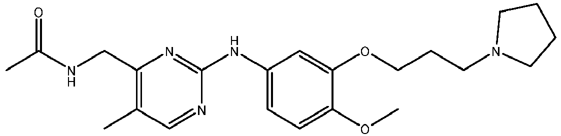 |
| 724 | 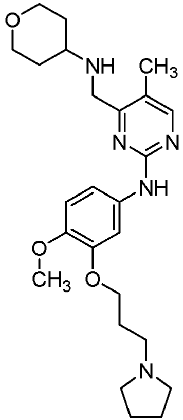 |
| 725 | 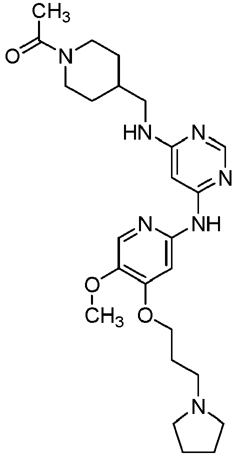 |
| 726 | 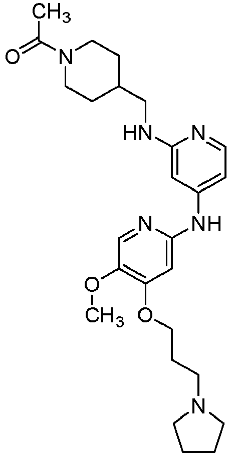 |
| 727 | 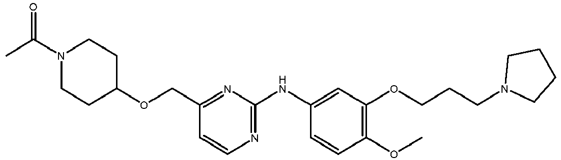 |
| 728 | 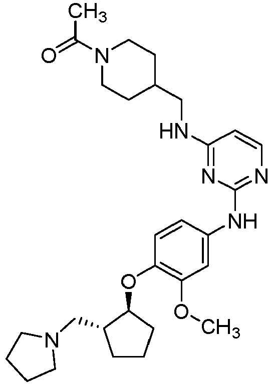 |
| 729 | 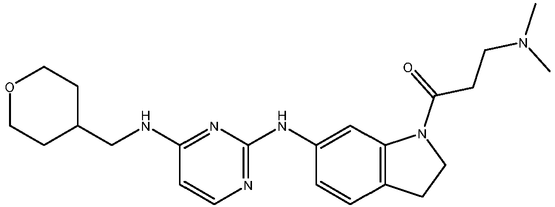 |
| 730 | 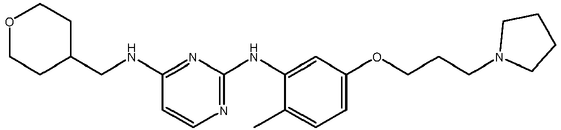 |
| 731 | 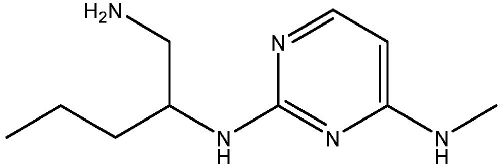 |
| 732 | 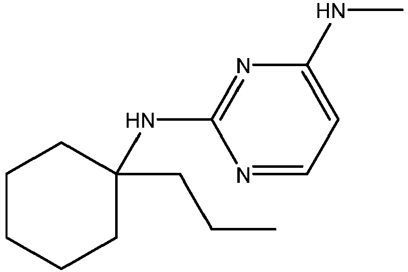 |
| 733 | 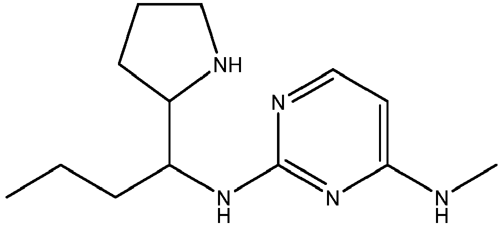 |
| 734 | 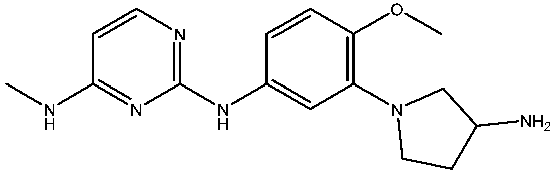 |
| 735 | 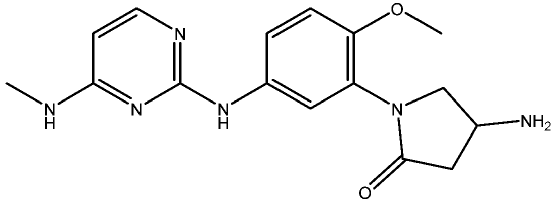 |
| 736 | 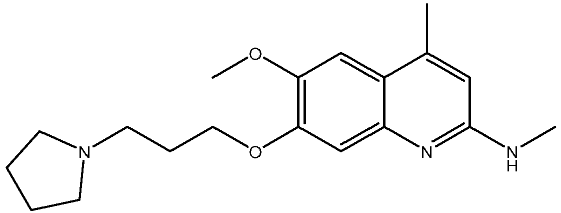 |
| 737 | 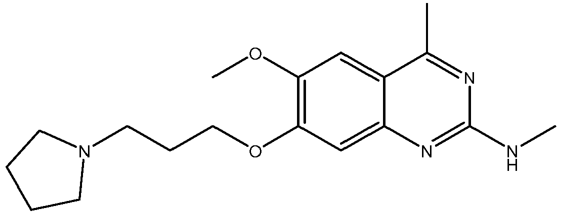 |
| 738 | 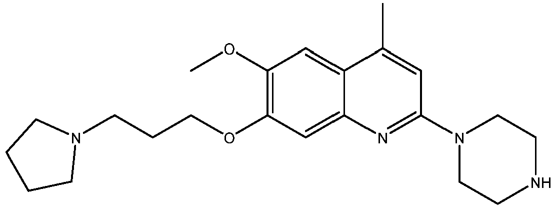 |
| 739 | 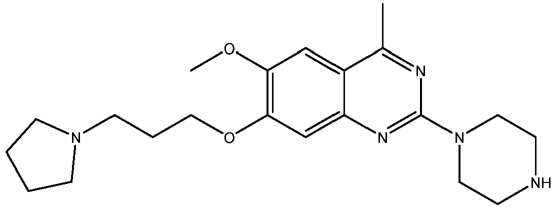 |
| 740 | 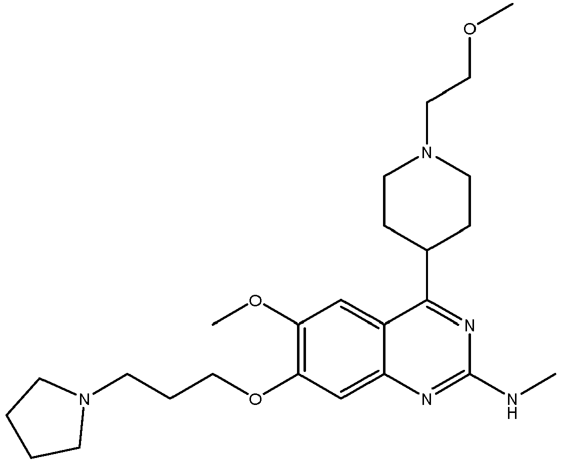 |
| 741 | 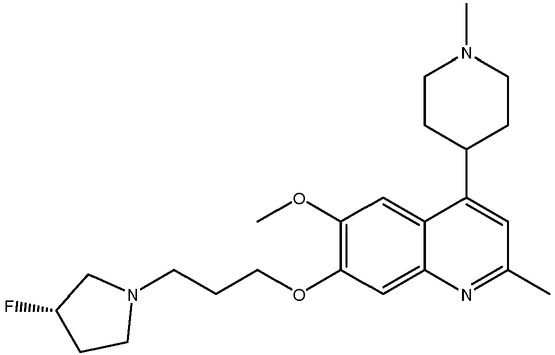 |
| 742 | 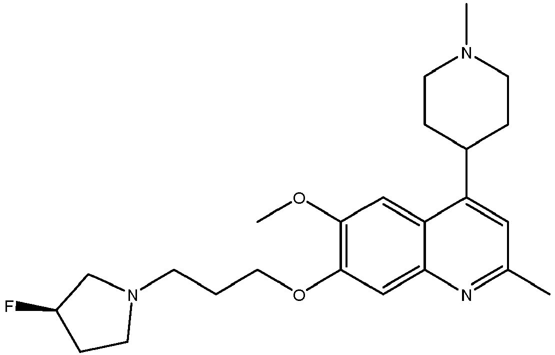 |
| 743 | 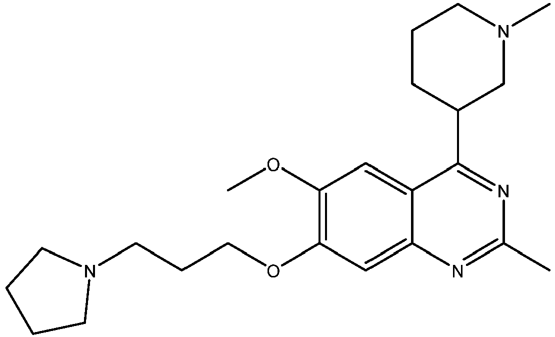 |
| 744 | 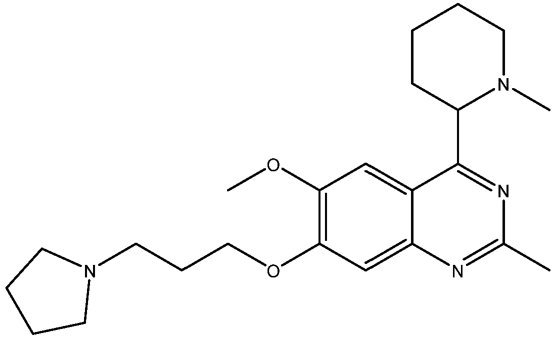 |
| 745 | 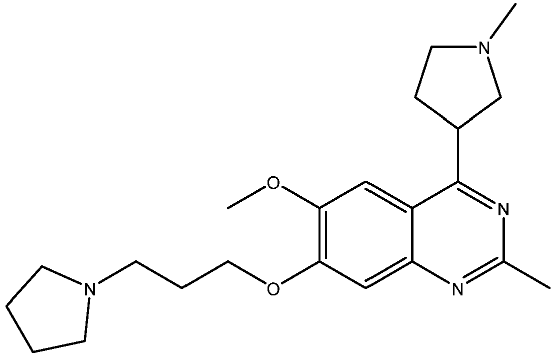 |
| 746 | 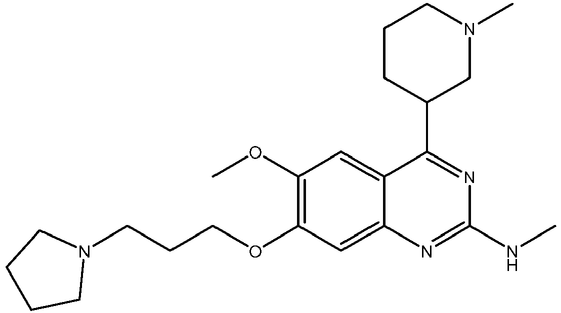 |
| 747 | 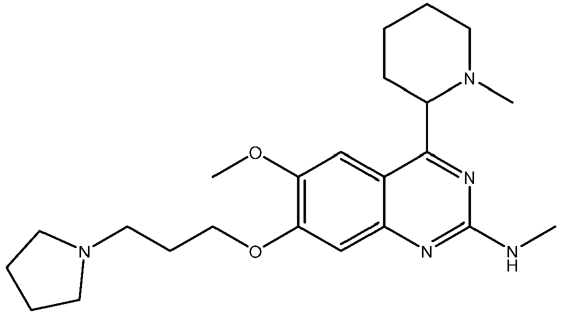 |
| 748 | 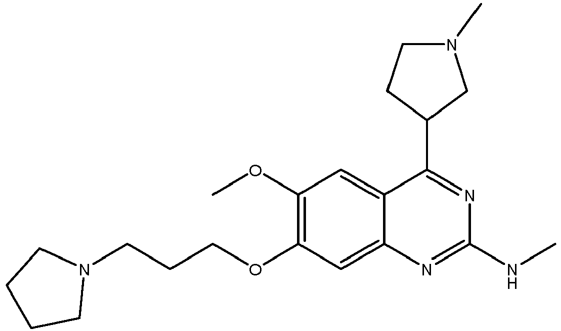 |
| 749 | 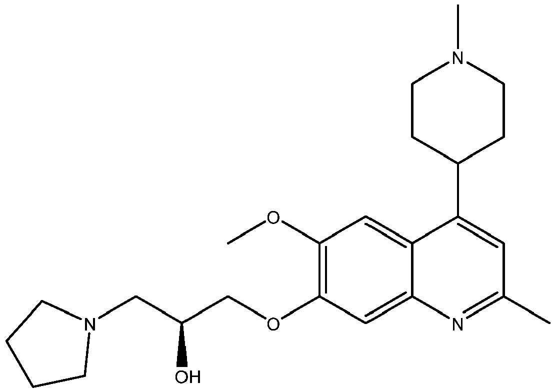 |
| 750 | 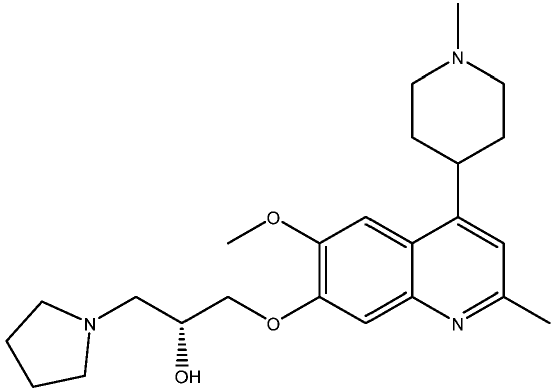 |
| 751 | 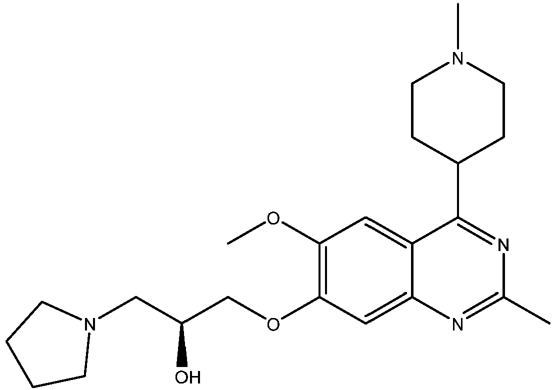 |
| 752 | 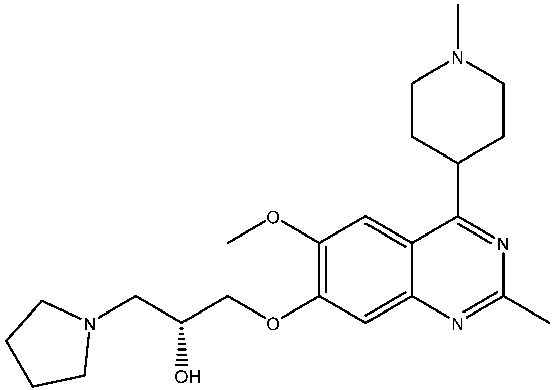 |
| 753 | 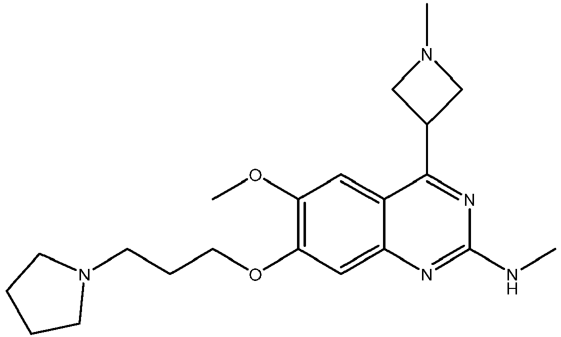 |
| 754 | 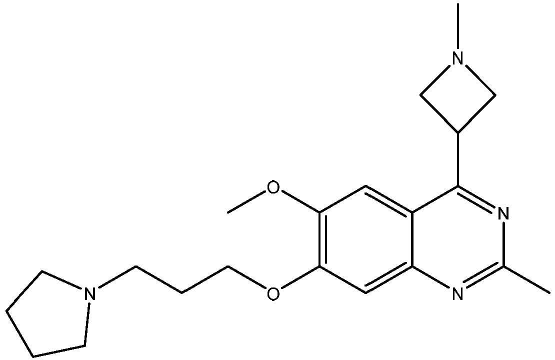 |
| 755 | 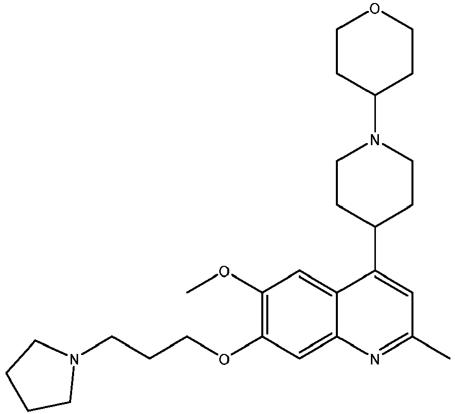 |
| 756 | 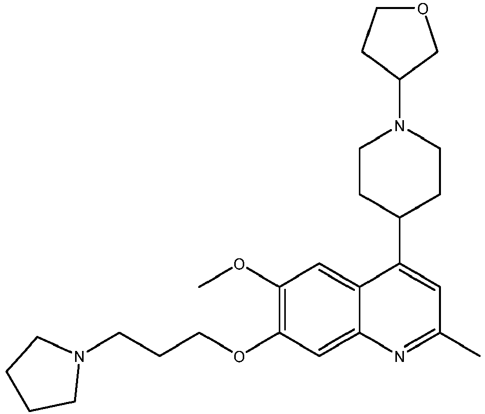 |
| 757 | 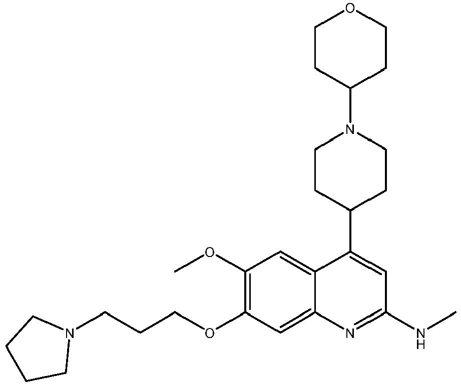 |
| 758 | 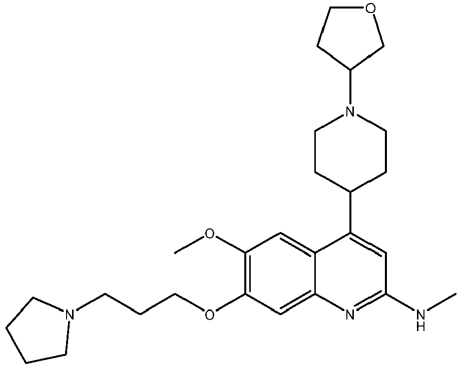 |
| 759 | 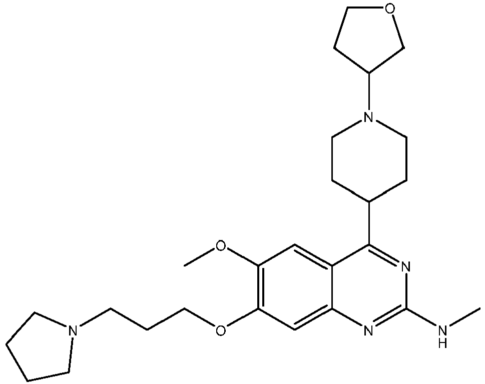 |
| 760 | 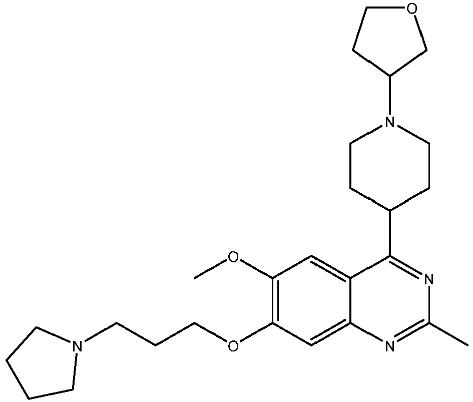 |
| 761 | 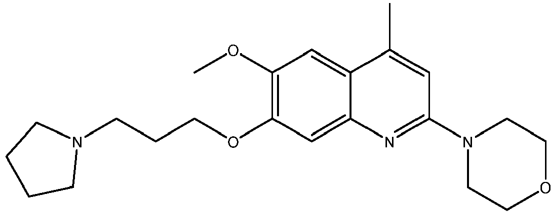 |
| 762 | 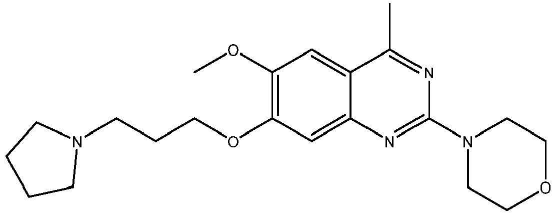 |
| 763 | 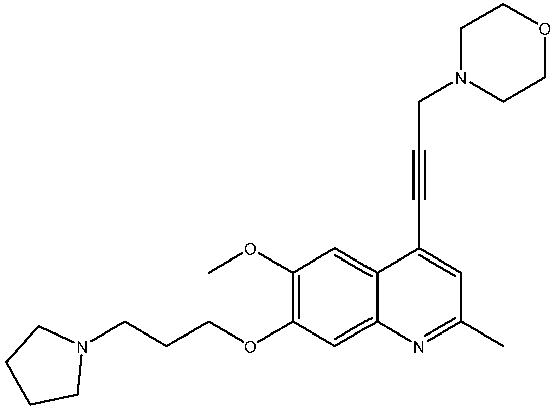 |
| 764 | 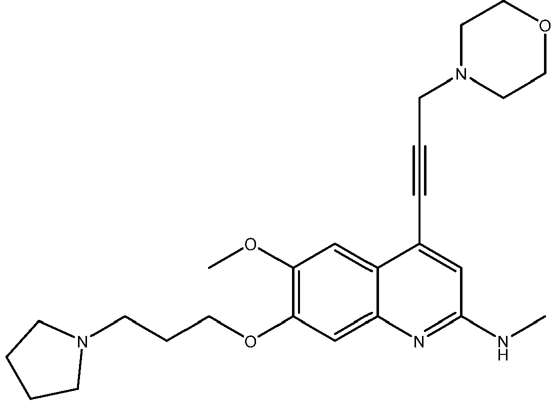 |
| 765 | 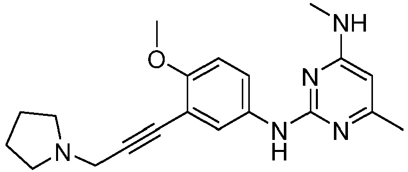 |

**Table 4**

| **Compound No.** | **Structure** |
|---|---|
| 784 | 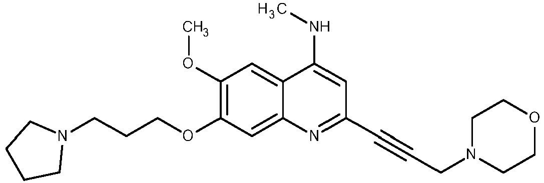 |
| 786 | 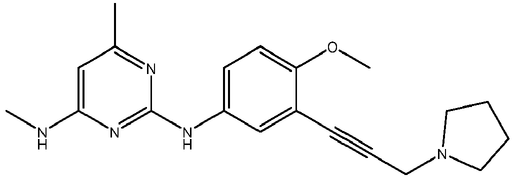 |
| 787 | 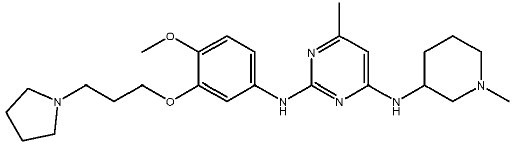 |
| 788 | 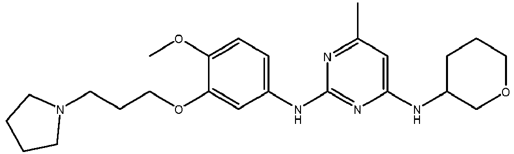 |
| 789 | 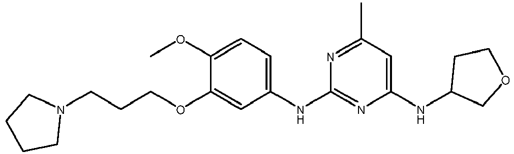 |
| 790 | 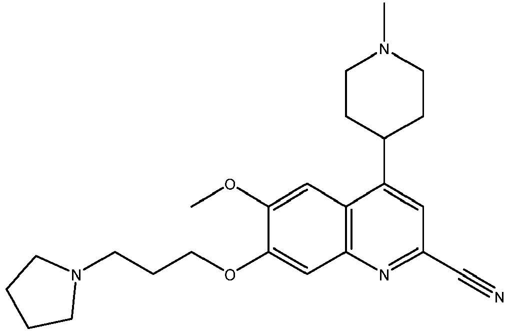 |
| 791 | 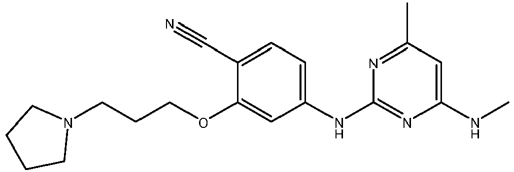 |
| 792 | 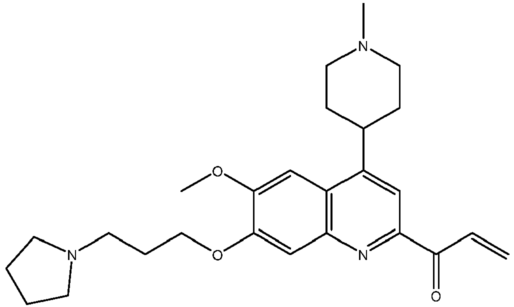 |
| 793 | 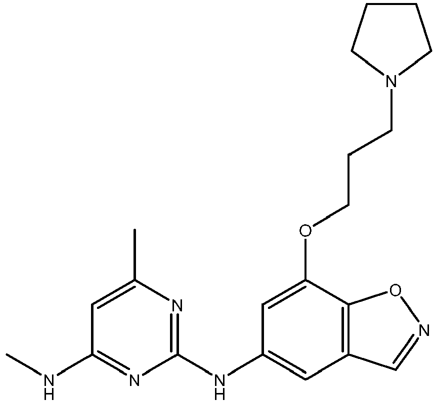 |
| 794 | 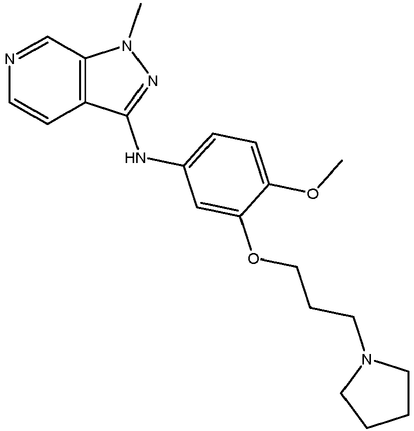 |
| 795 | 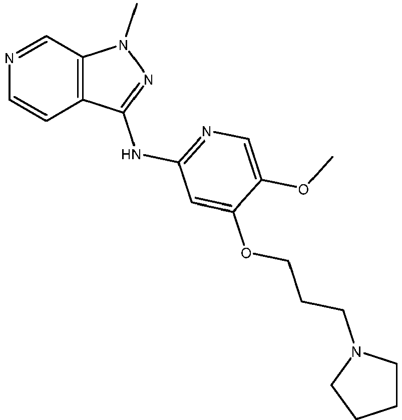 |
| 796 | 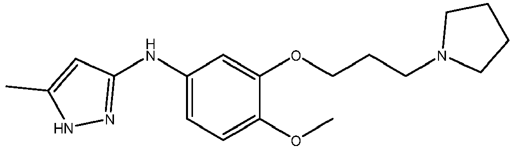 |
| 797 | 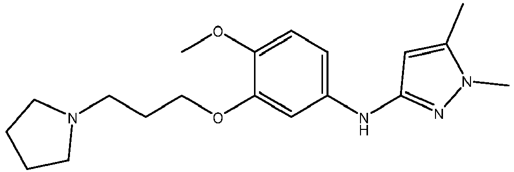 |
| 798 | 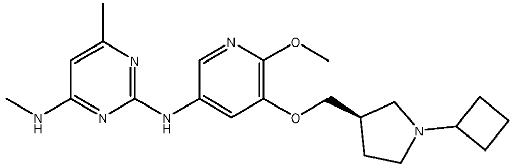 |
| 799 | 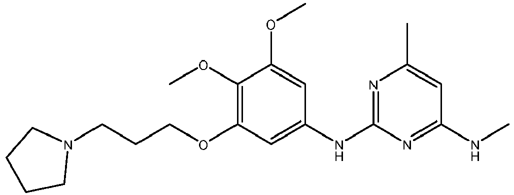 |
| 800 | 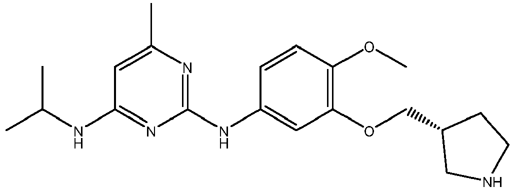 |
| 801 | 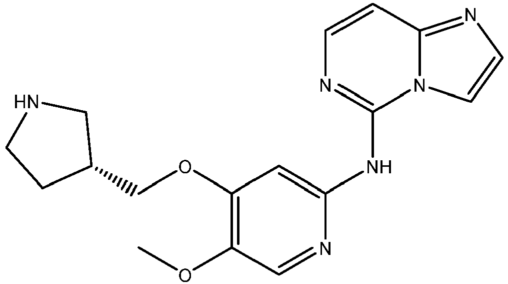 |
| 802 | 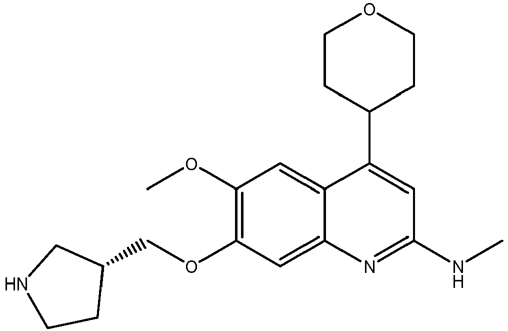 |
| 803 | 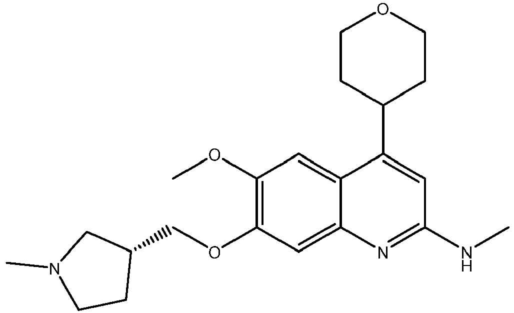 |
| 804 | 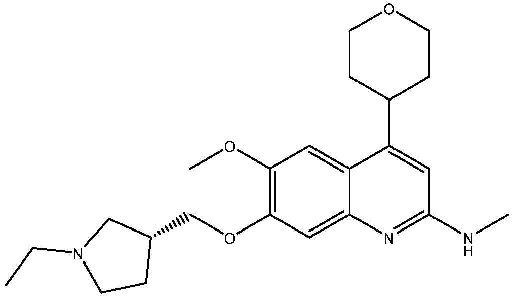 |
| 805 | 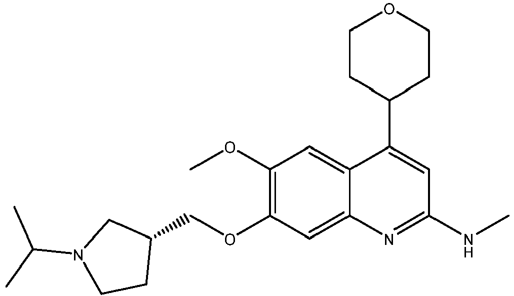 |
| 806 | 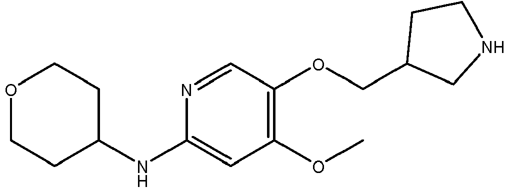 |
| 807 | 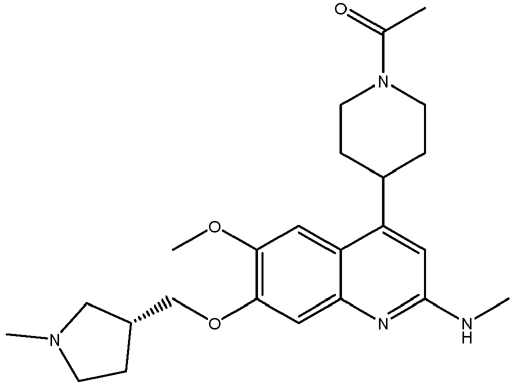 |
| 808 | 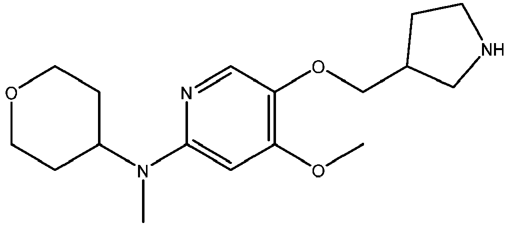 |
| 809 | 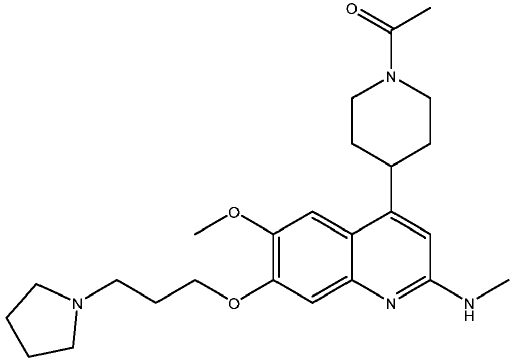 |
| 810 | 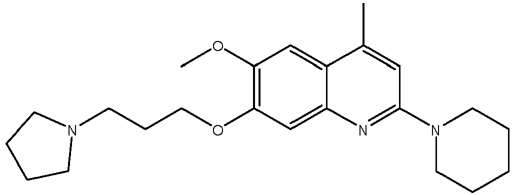 |
| 811 | 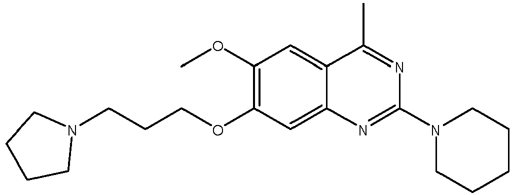 |
| 812 | 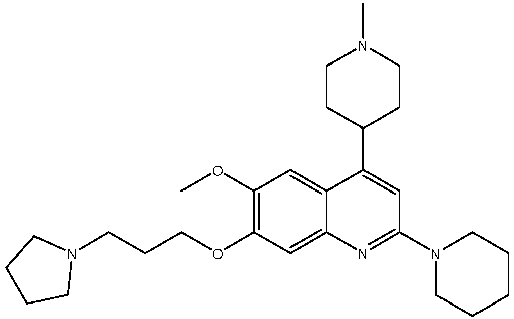 |
| 813 | 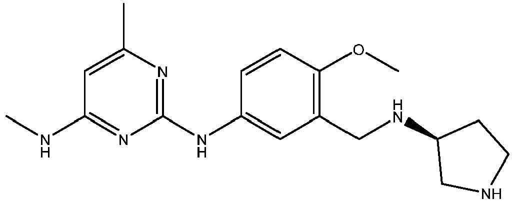 |
| 814 | 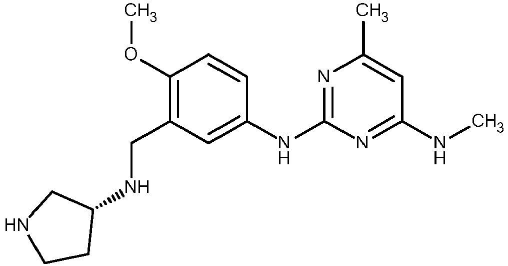 |
| 815 | 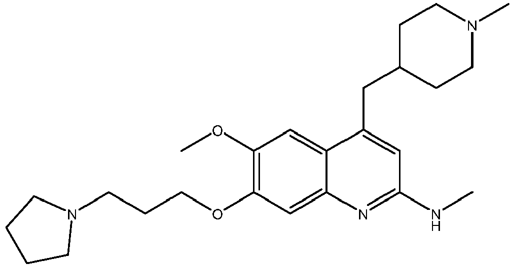 |
| 816 | 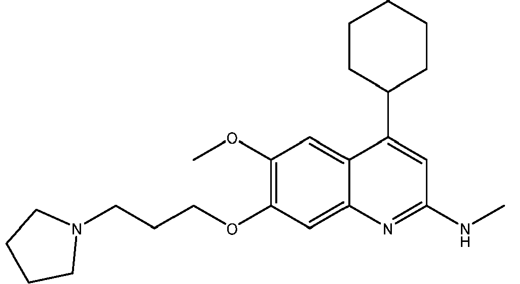 |
| 817 | 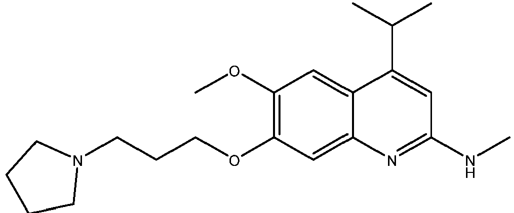 |
| 820 | 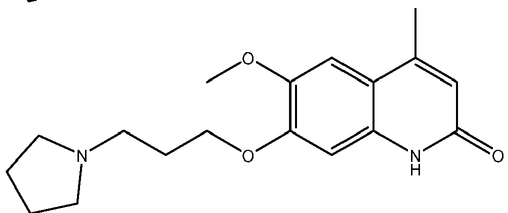 |
| 821 | 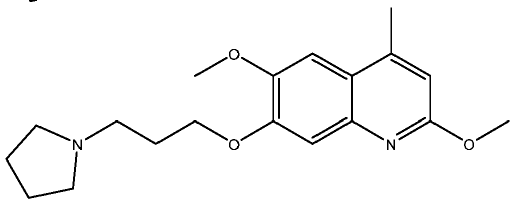 |
| 822 | 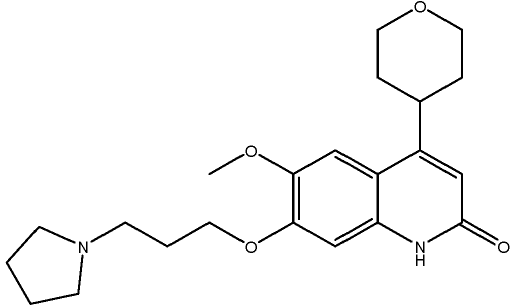 |
| 823 | 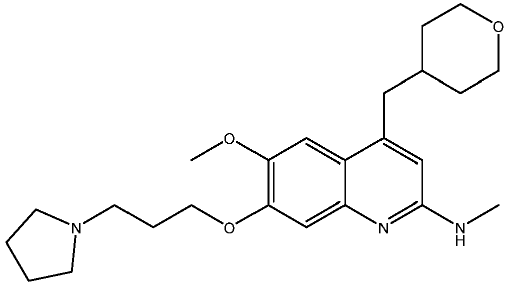 |
| 824 | 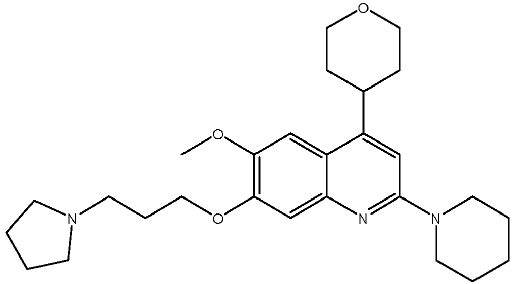 |
| 825 | 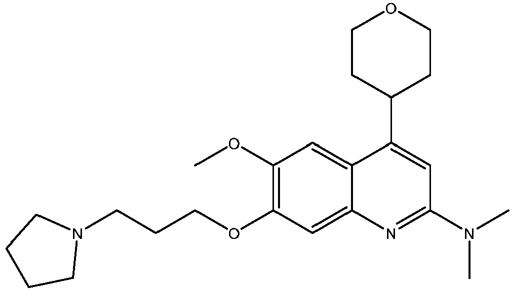 |
| 826 | 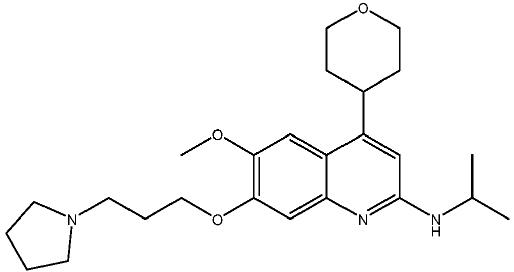 |
| 827 | 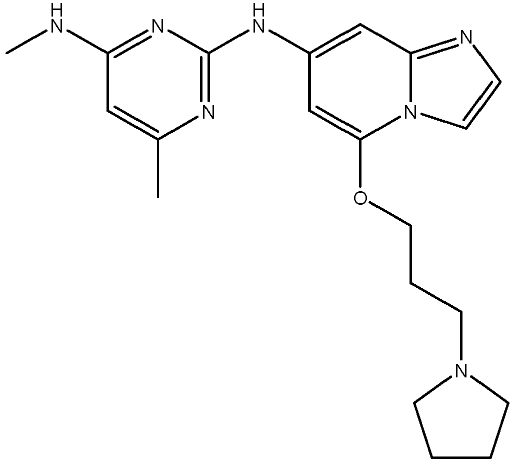 |
| 828 | 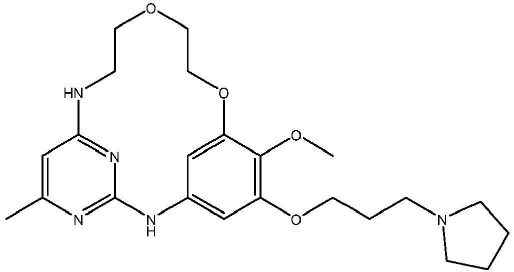 |
| 832 | 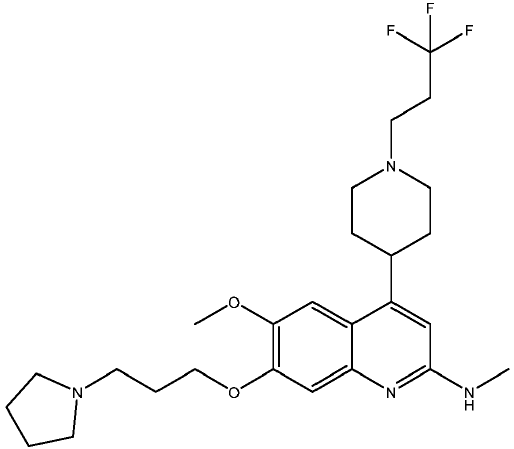 |
| 833 | 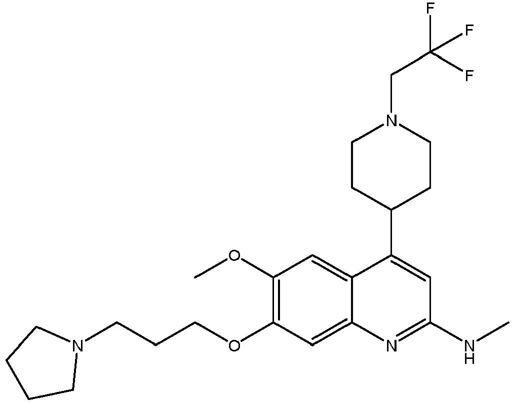 |
| 834 | 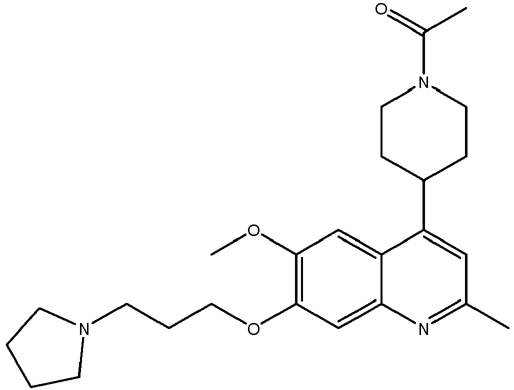 |
| 836 | 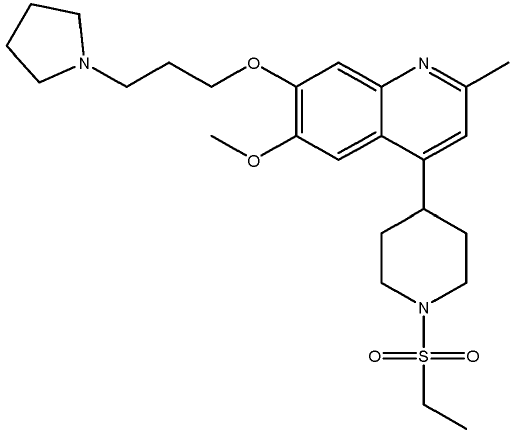 |
| 837 | 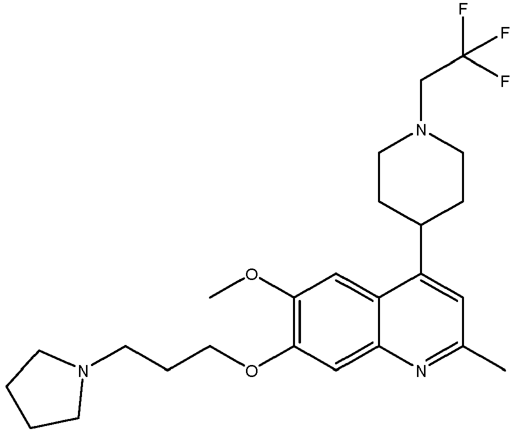 |
| 838 | 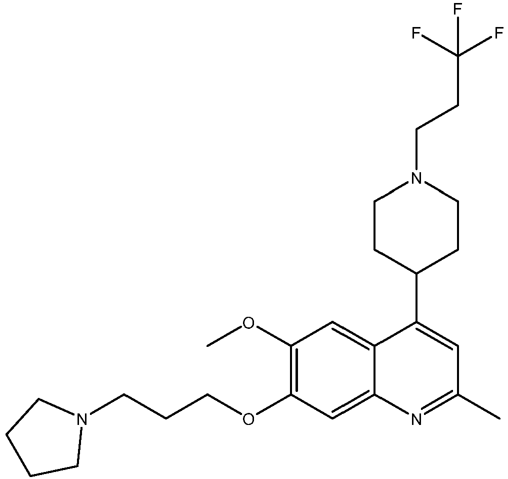 |
| 839 | 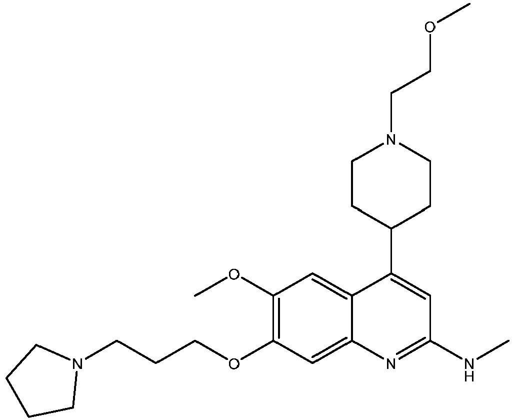 |
| 840 | 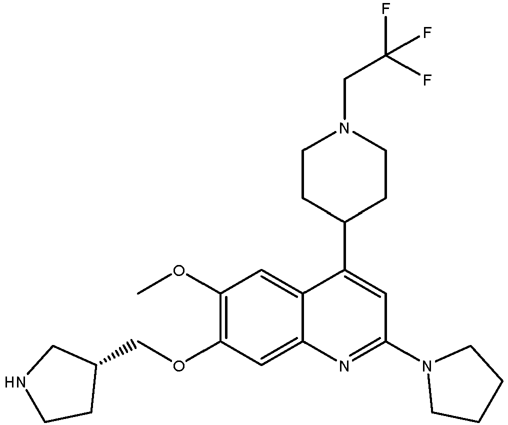 |
| 841 | 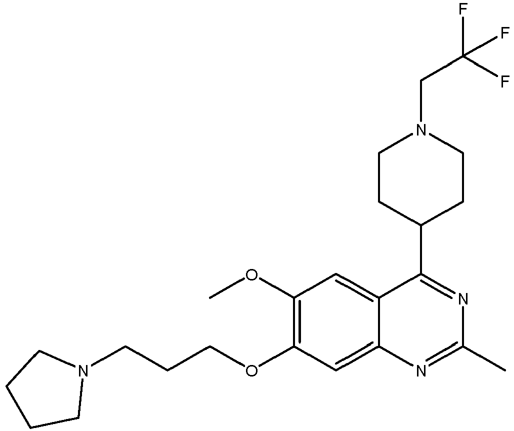 |
| 842 | 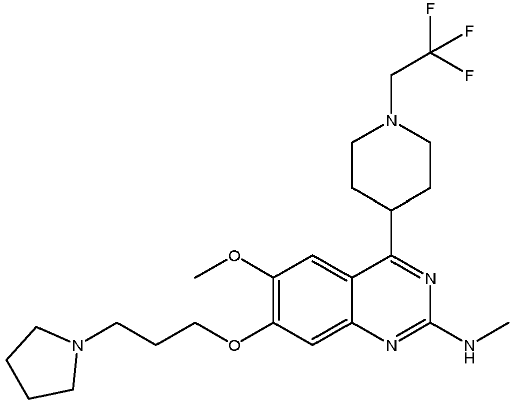 |
| 844 | 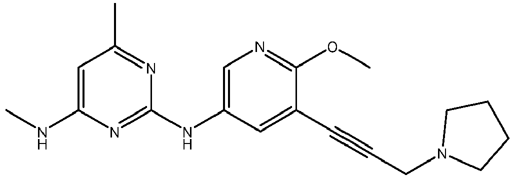 |
| 845 | 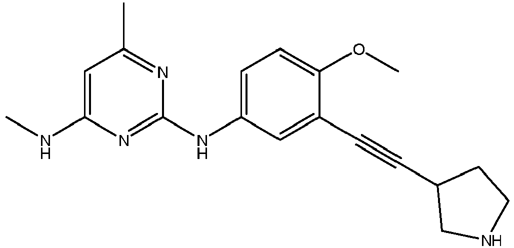 |
| 846 | 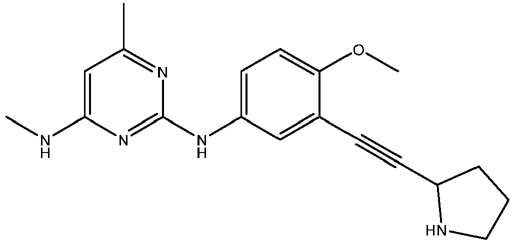 |
| 847 | 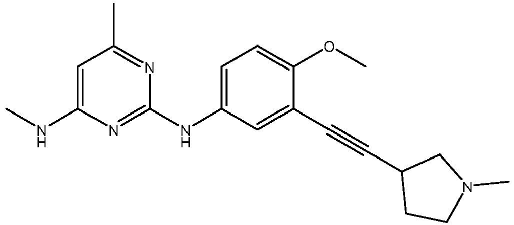 |
| 848 | 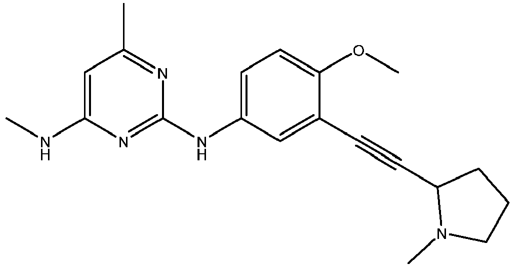 |
| 849 | 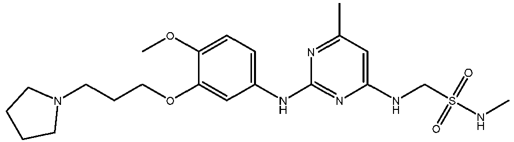 |
| 850 | 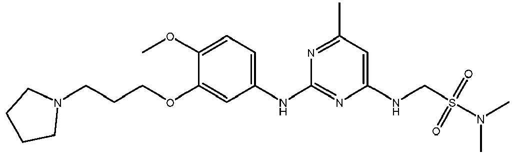 |
| 851 | 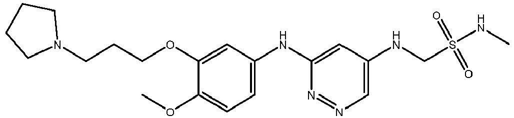 |
| 852 | 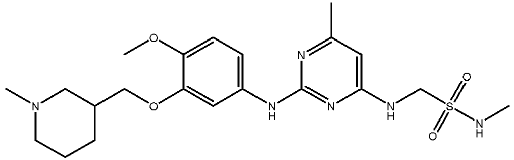 |
| 853 | 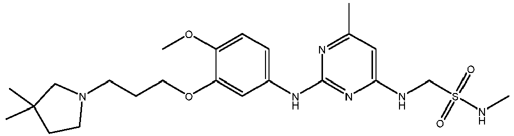 |
| 854 | 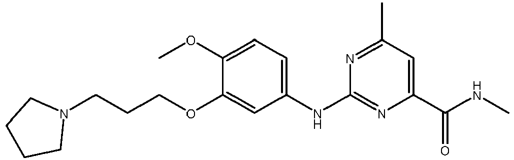 |
| 855 | 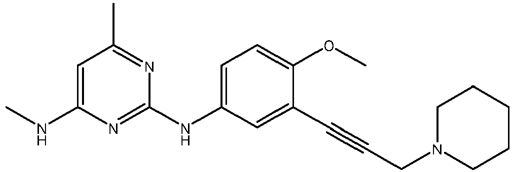 |
| 856 | 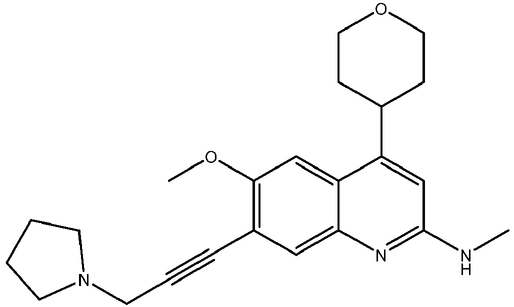 |
| 857 | 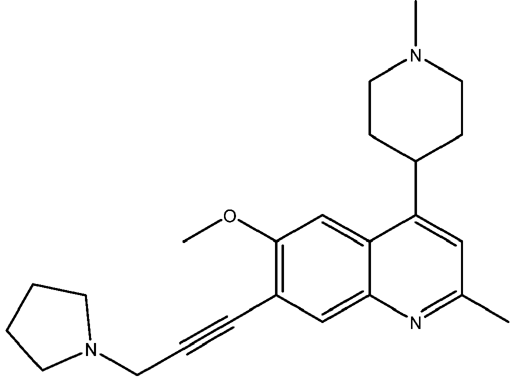 |
| 858 | 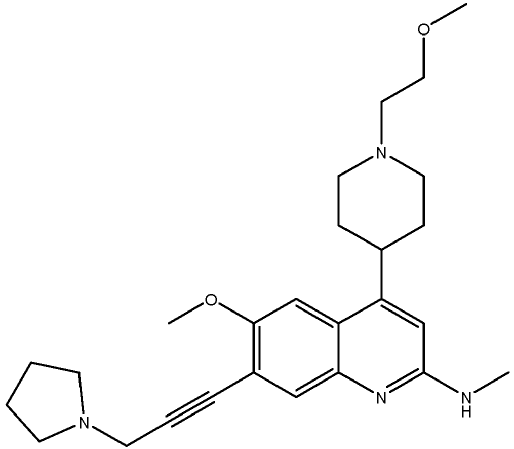 |
| 859 | 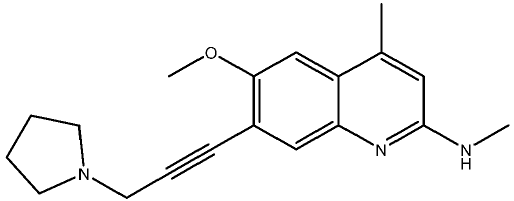 |
| 860 | 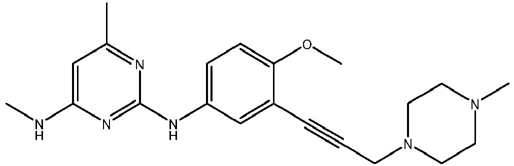 |
| 861 | 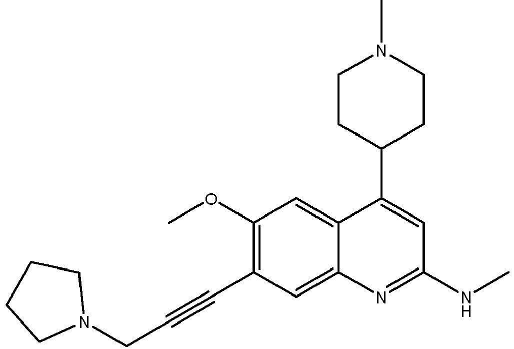 |
| 862 | 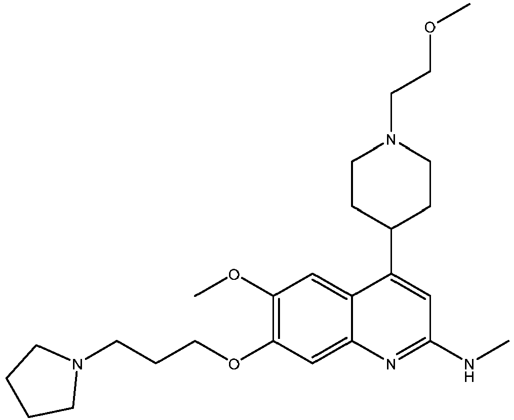 |
| 863 | 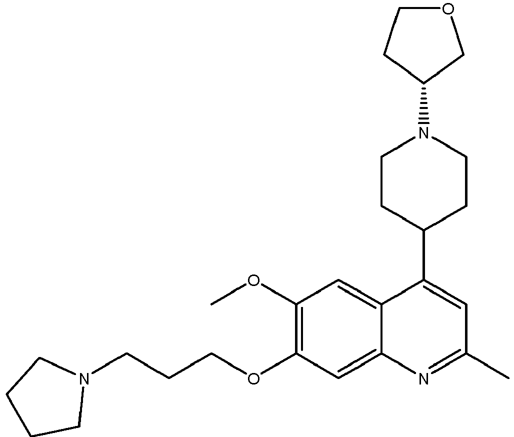 |
| 864 | 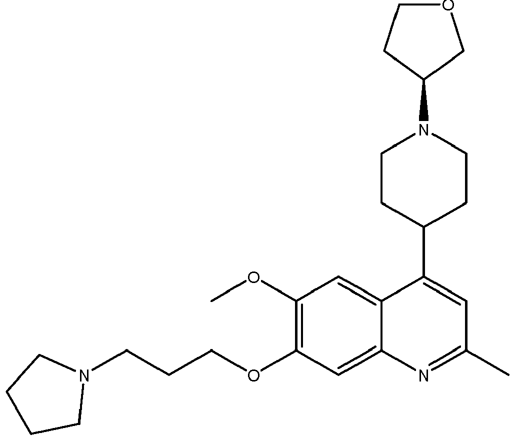 |
| 865 | 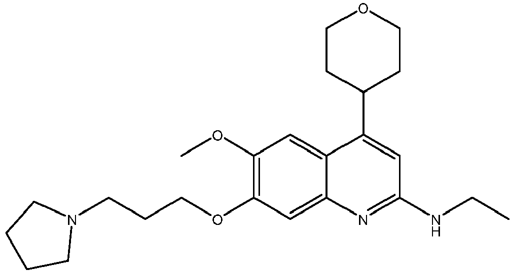 |
| 866 | 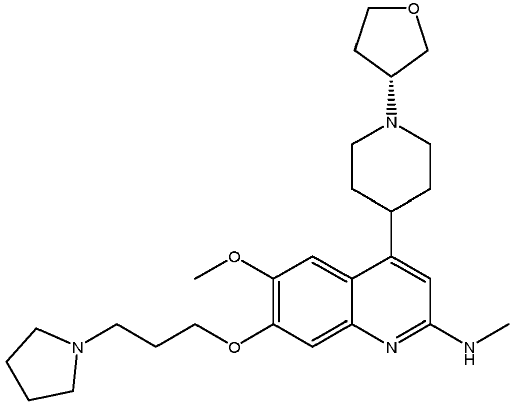 |
| 867 | 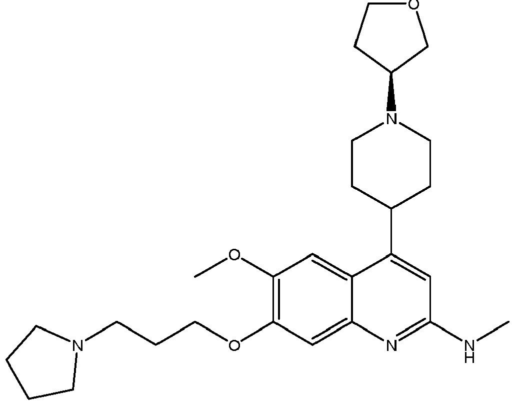 |
| 868 | 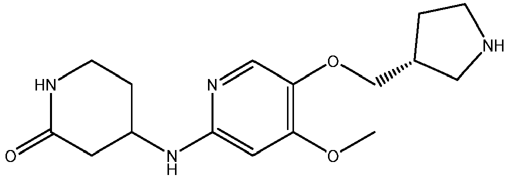 |
| 869 | 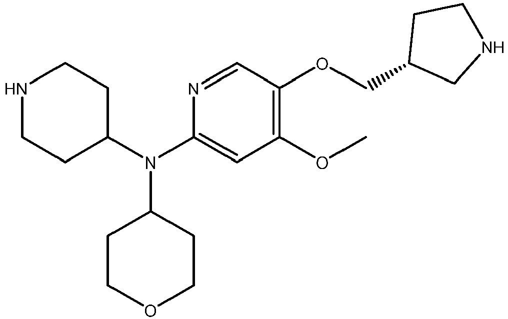 |
| 870 | 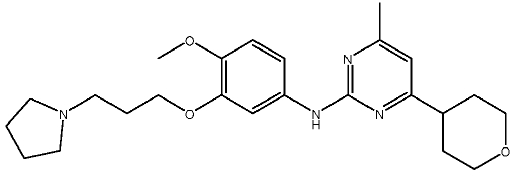 |
| 871 | 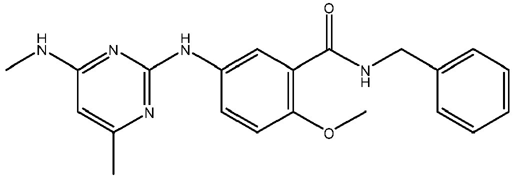 |
| 872 | 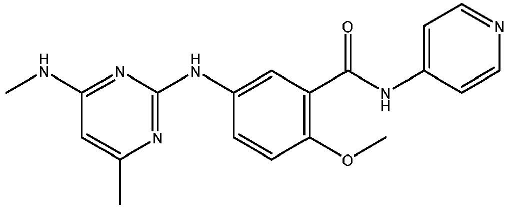 |
| 873 | 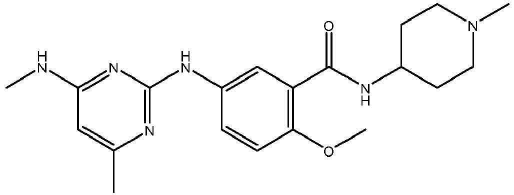 |
| 874 | 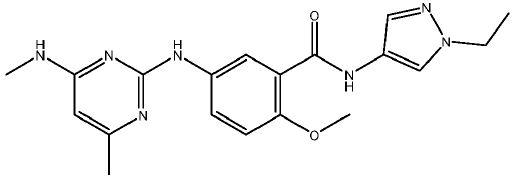 |
| 875 | 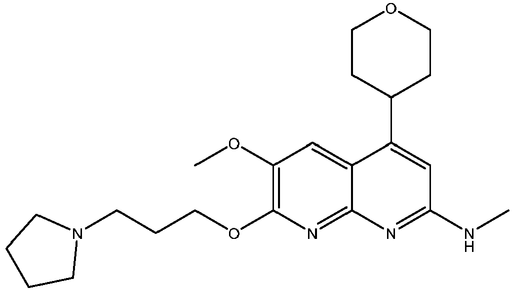 |
| 876 | 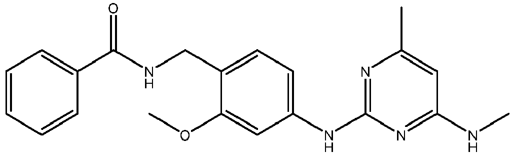 |
| 877 | 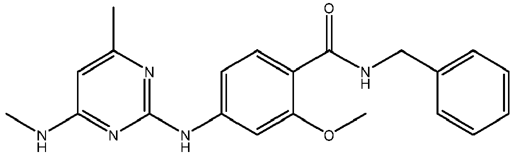 |
| 878 | 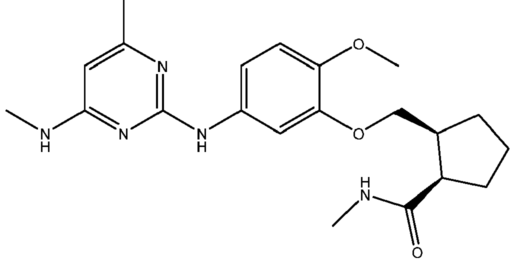 |
| 879 | 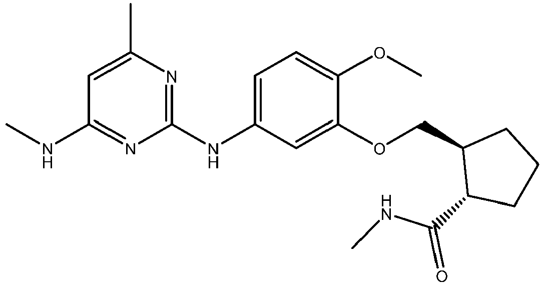 |
| 881 | 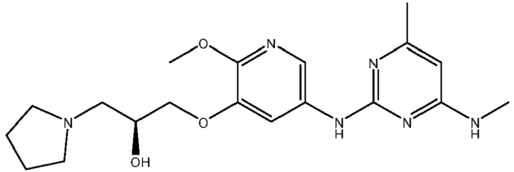 |
| 882 | 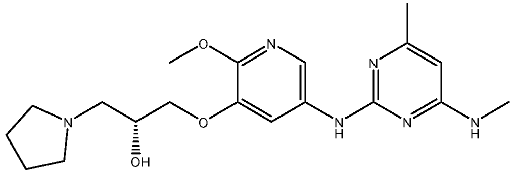 |
| 883 | 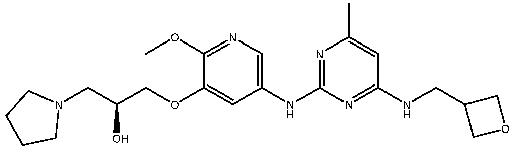 |
| 884 | 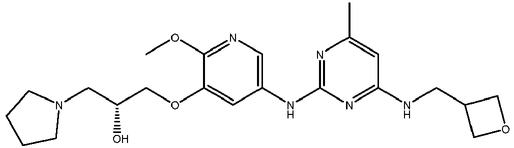 |
| 885 | 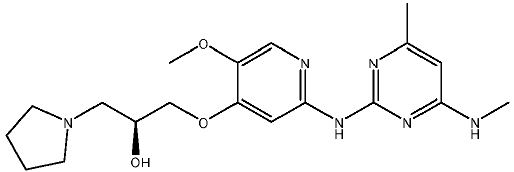 |
| 886 | 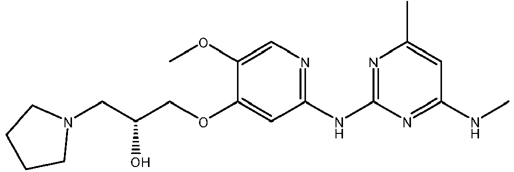 |
| 887 | 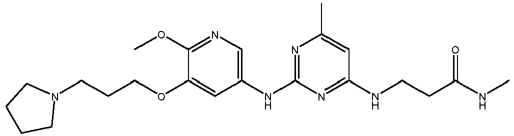 |
| 888 | 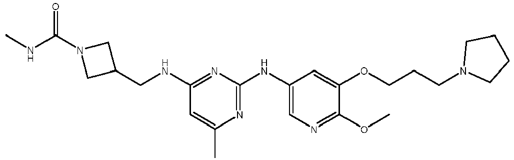 |
| 890 | 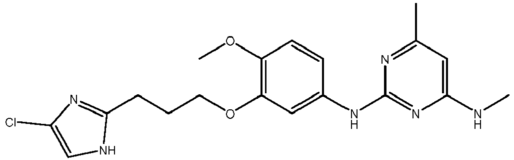 |
| 891 | 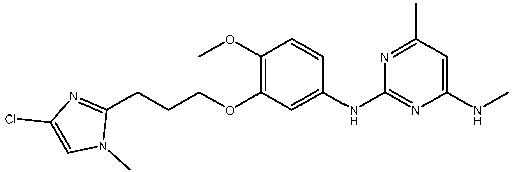 |
| 892 | 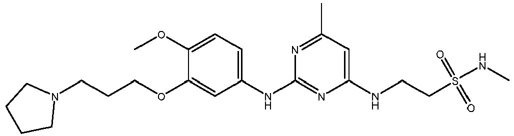 |
| 893 | 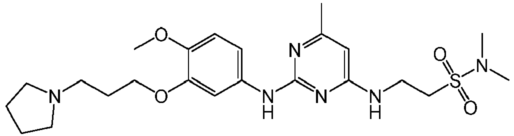 |
| 894 | 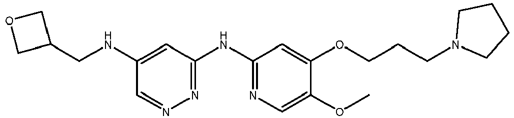 |
| 895 | 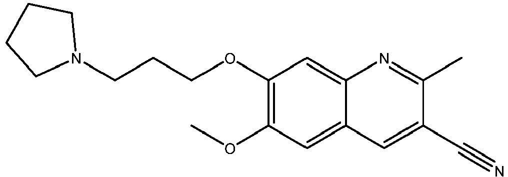 |
| 896 | 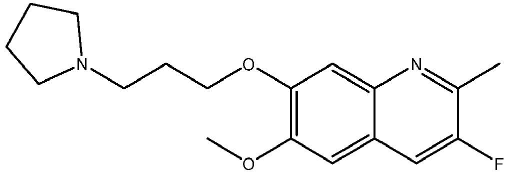 |
| 897 | 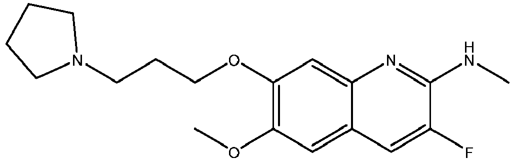 |
| 898 | 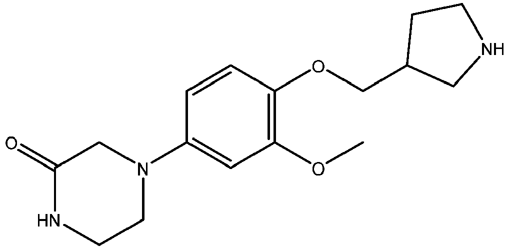 |
| 899 | 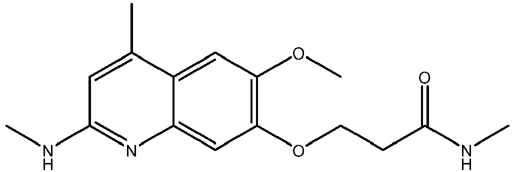 |
| 900 | 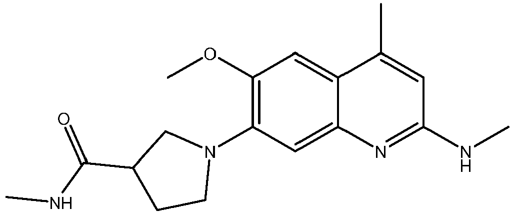 |
| 901 | 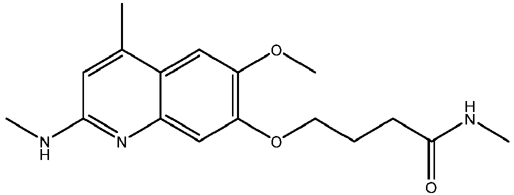 |
| 902 | 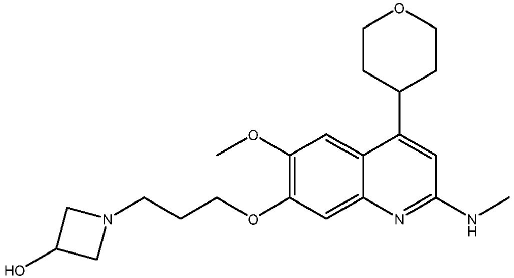 |
| 903 | 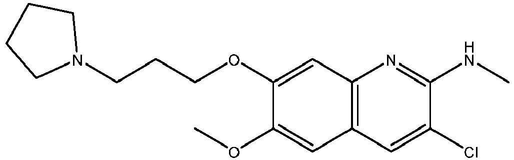 |
| 904 | 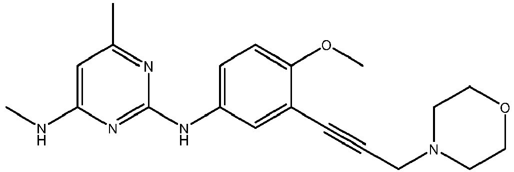 |
| 905 | 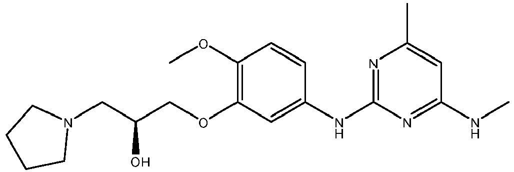 |
| 906 | 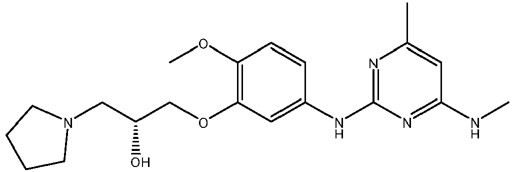 |
| 907 | 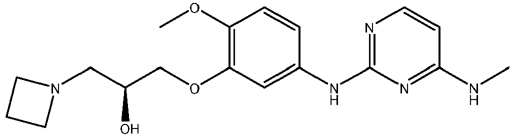 |
| 908 | 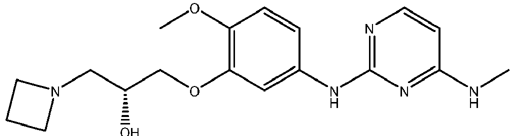 |
| 909 | 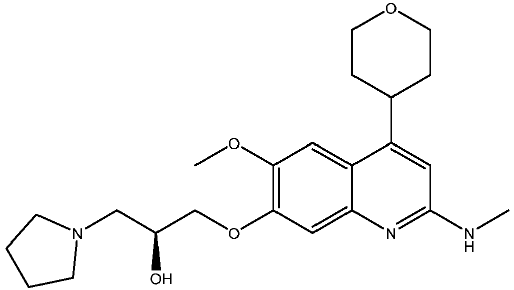 |
| 910 | 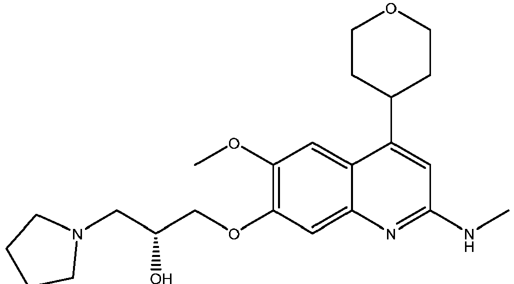 |
| 911 | 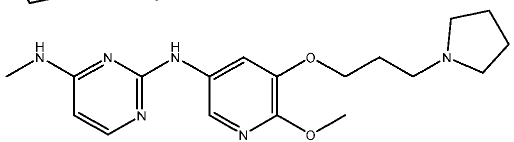 |
| 912 | 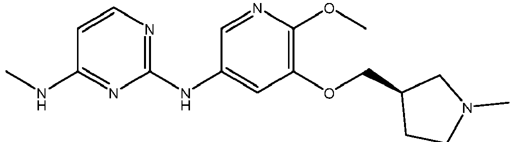 |
| 913 | 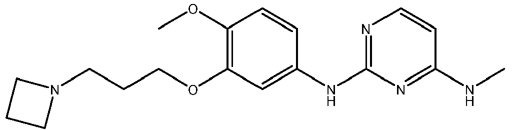 |
| 914 | 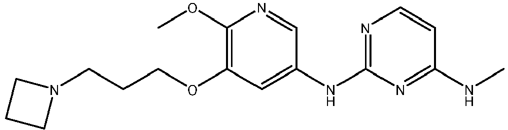 |
| 915 | 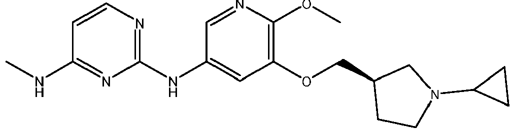 |
| 916 | 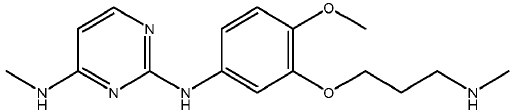 |
| 917 | 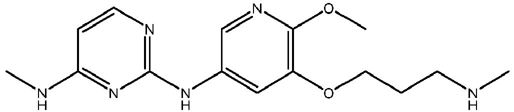 |
| 918 | 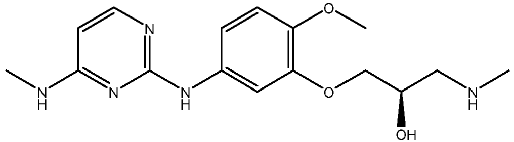 |
| 919 | 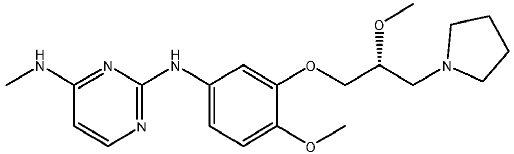 |
| 920 | 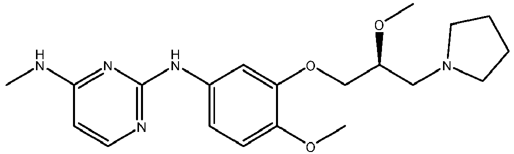 |
| 921 | 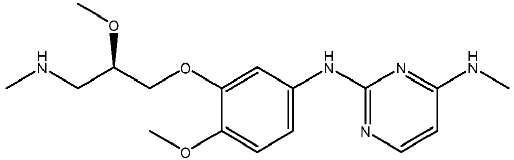 |
| 922 | 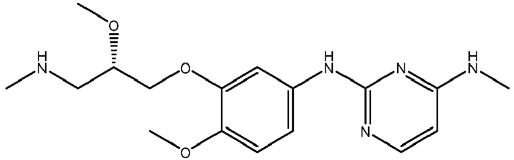 |
| 927 | 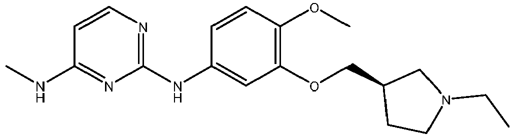 |
| 928 | 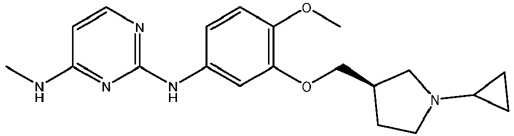 |
| 929 | 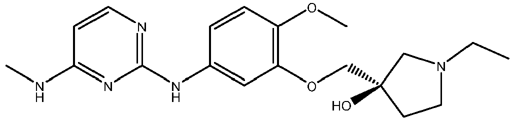 |
| 930 | 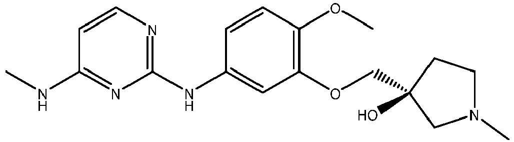 |
| 931 | 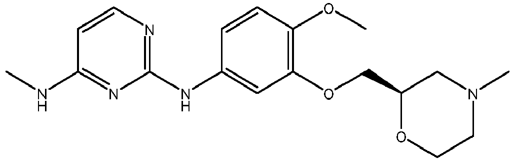 |
| 932 | 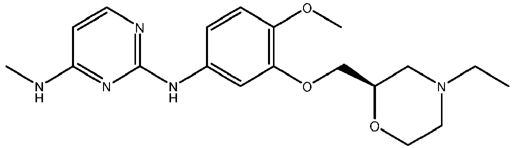 |
| 933 | 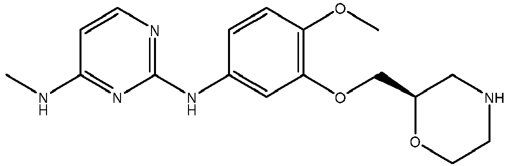 |
| 934 | 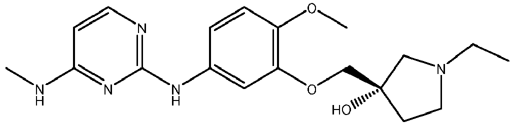 |
| 935 | 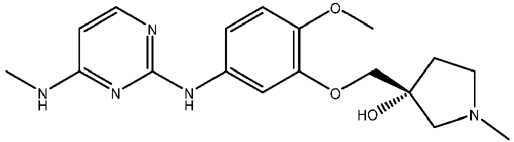 |
| 936 | 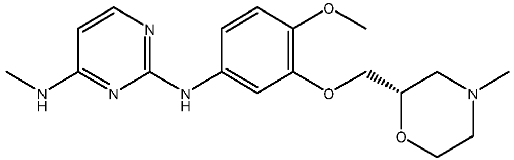 |
| 937 | 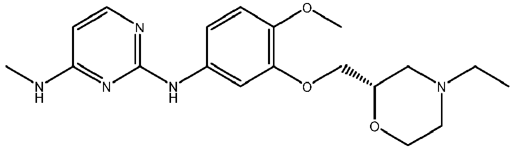 |
| 938 | 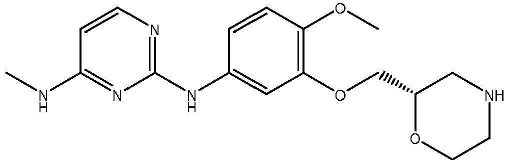 |
| 939 | 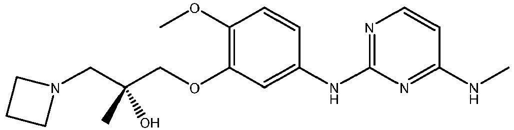 |
| 940 | 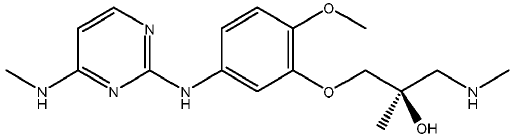 |
| 941 | 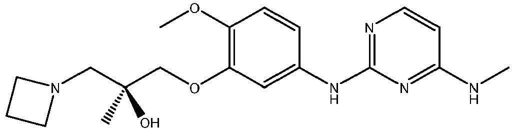 |
| 942 | 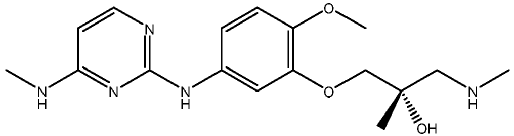 |
| 943 | 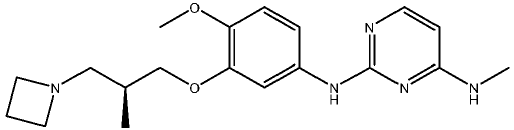 |
| 944 | 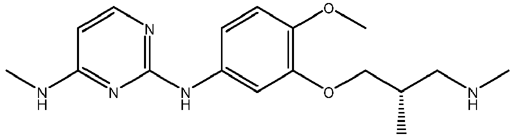 |
| 945 | 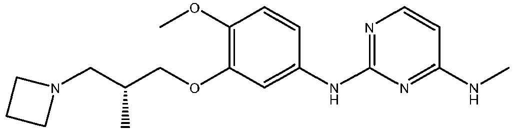 |
| 946 | 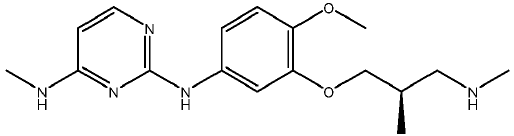 |
| 947 | 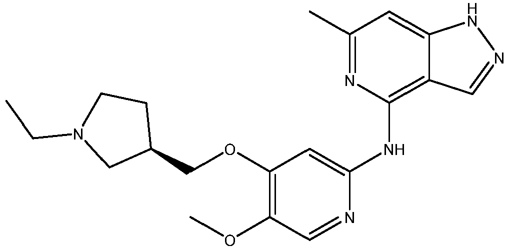 |
| 948 | 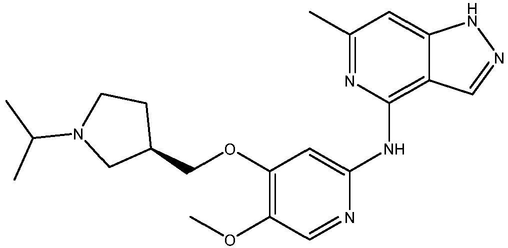 |
| 949 | 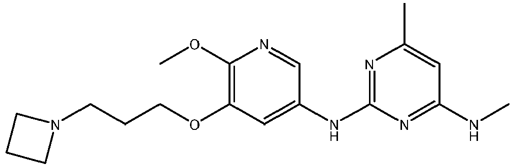 |
| 950 | 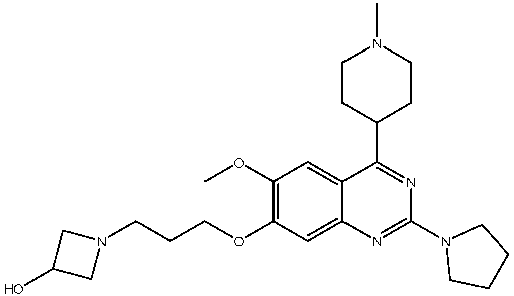 |
| 951 | 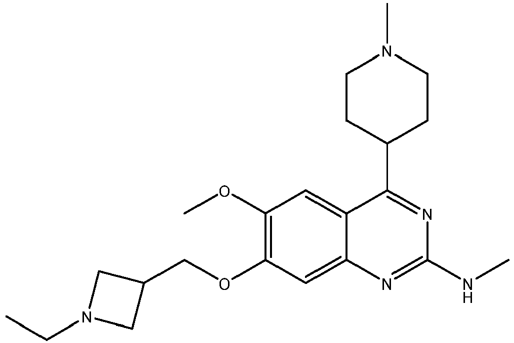 |
| 961 | 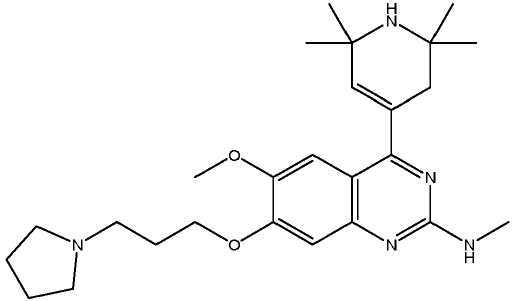 |
| 962 | 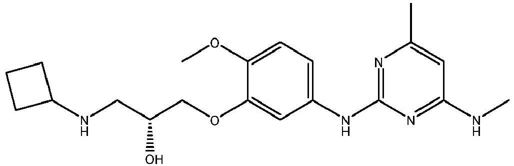 |
| 963 | 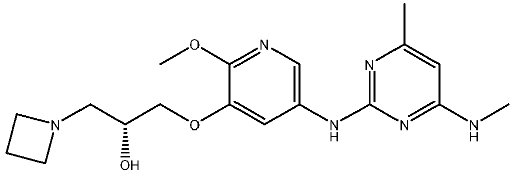 |
| 964 | 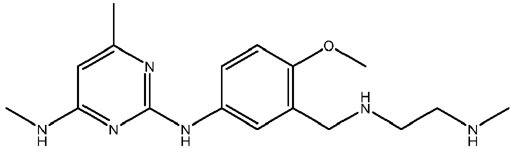 |
| 965 | 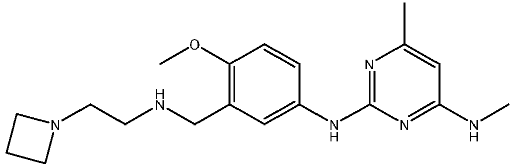 |
| 966 | 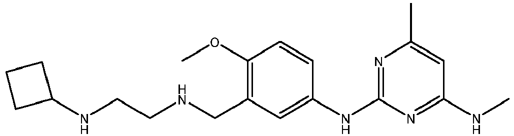 |
| 967 | 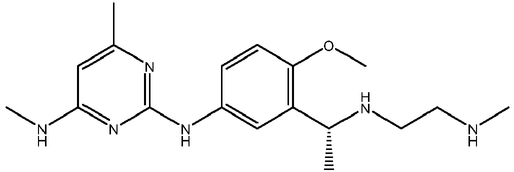 |
| 968 | 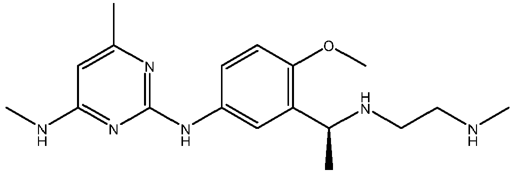 |
| 969 | 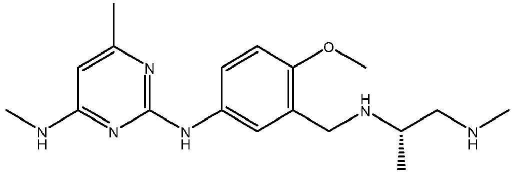 |
| 970 | 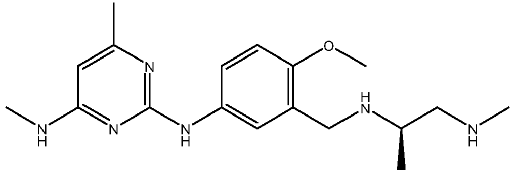 |
| 971 | 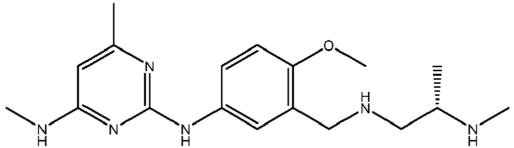 |
| 972 | 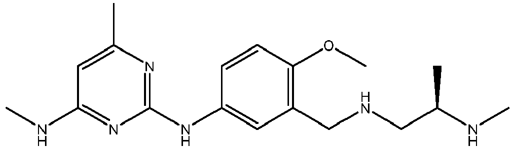 |
| 974 | 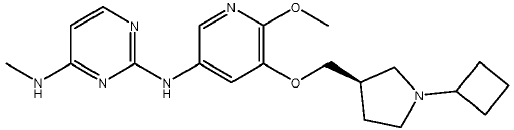 |
| 975 | 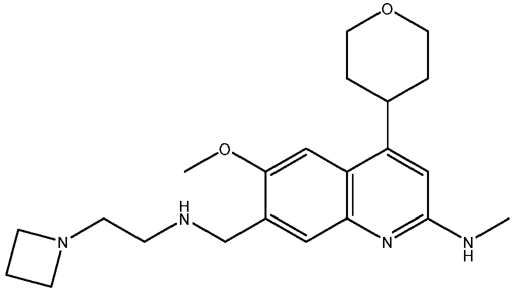 |
| 976 | 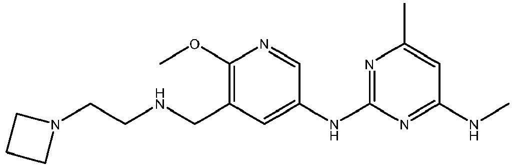 |
| 977 | 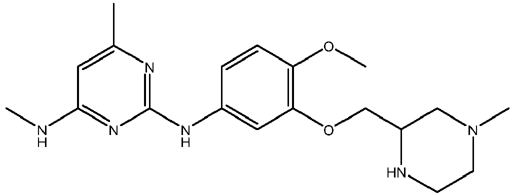 |
| 983 | 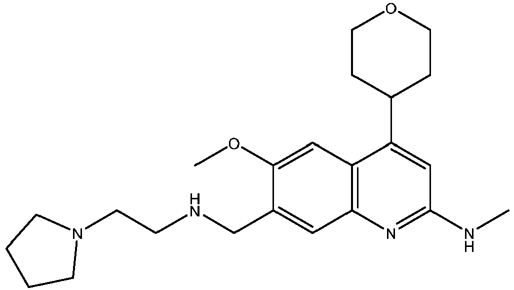 |
| 985 | 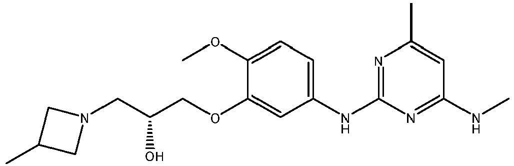 |
| 986 | 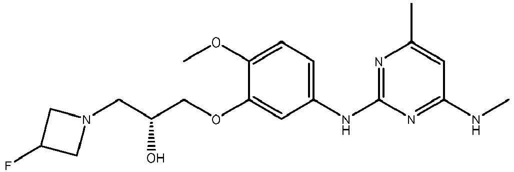 |
| 989 | 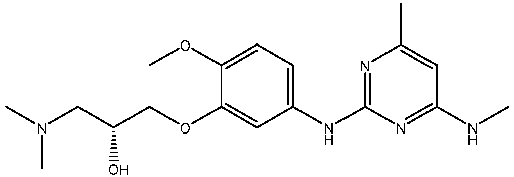 |
| 990 | 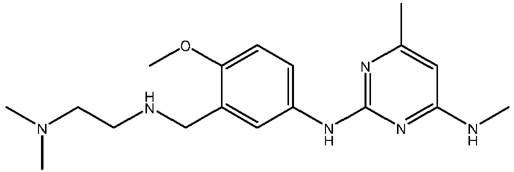 |
| 991 | 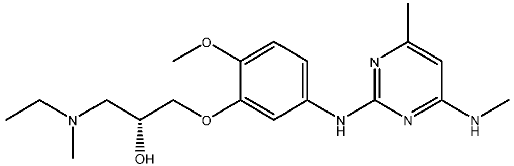 |
| 992 | 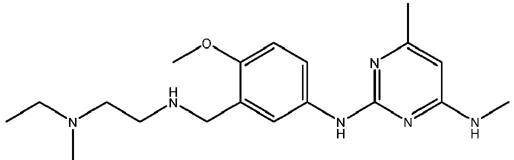 |
| 993 | 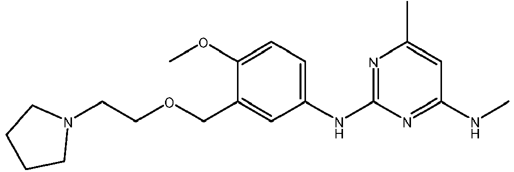 |
| 994 | 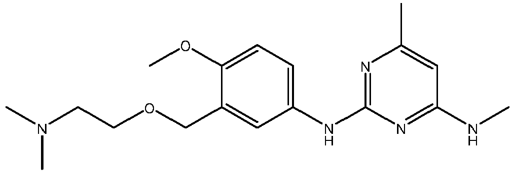 |
| 997 | 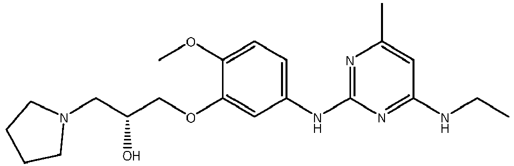 |
| 998 | 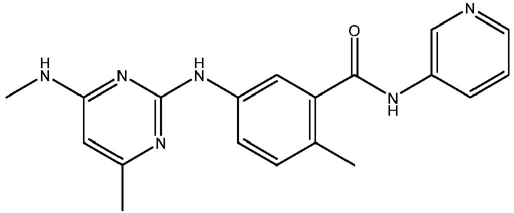 |
| 999 | 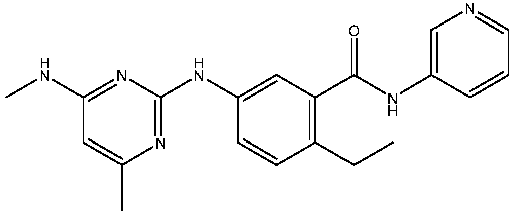 |
| 1000 | 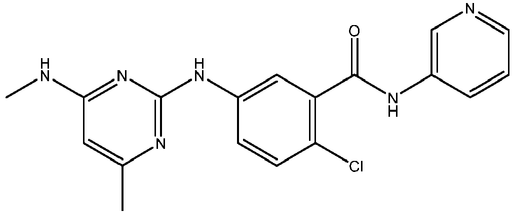 |
| 1001 | 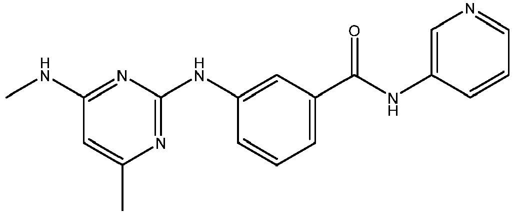 |
| 1002 | 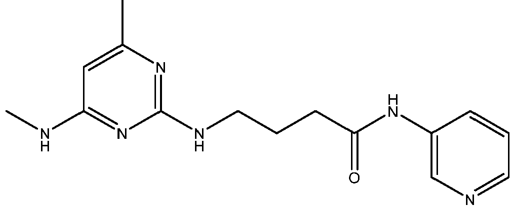 |
| 1004 | 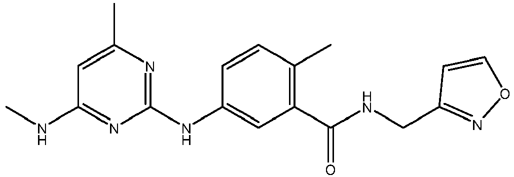 |
| 1005 | 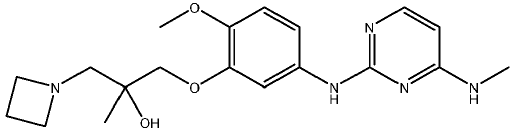 |
| 1006 | 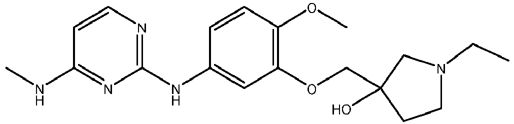 |
| 1007 | 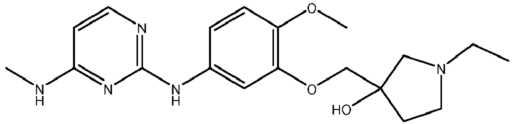 |
| 1008 | 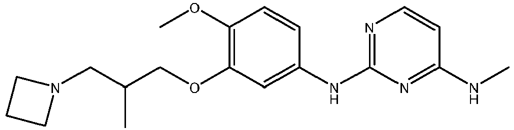 |
| 1009 | 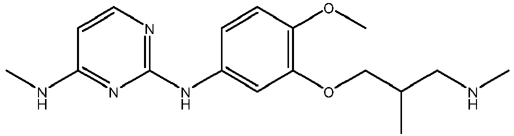 |
| 1010 | 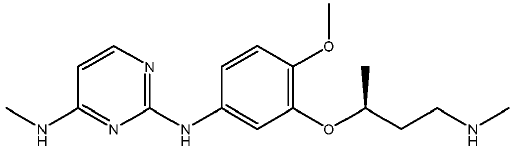 |
| 1011 | 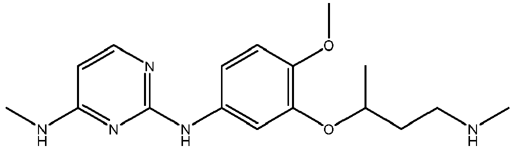 |
| 1012 | 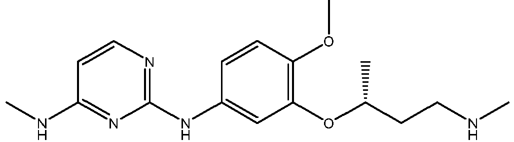 |
| 1013 | 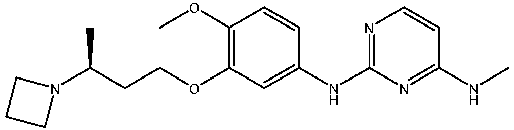 |
| 1014 | 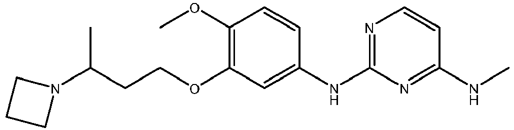 |
| 1015 | 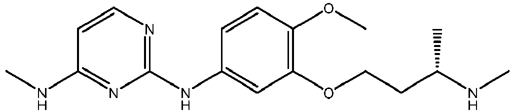 |
| 1016 | 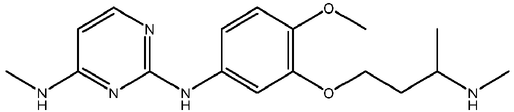 |
| 1017 | 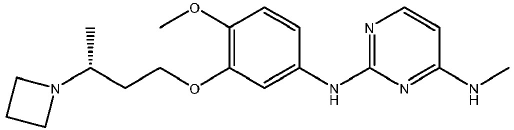 |
| 1018 | 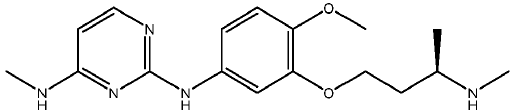 |
| 1019 | 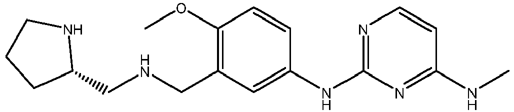 |
| 1020 | 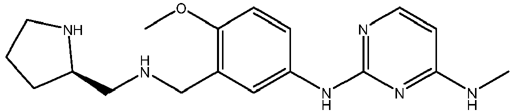 |
| 1021 | 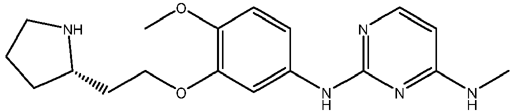 |
| 1022 | 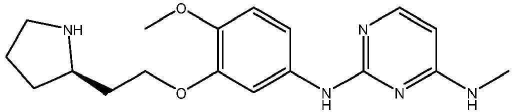 |
| 1023 | 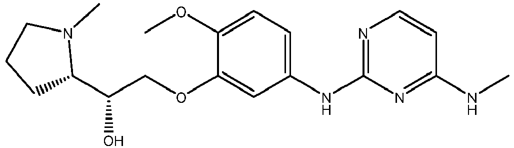 |
| 1024 | 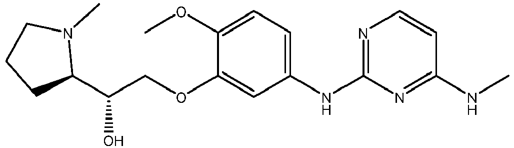 |
| 1025 | 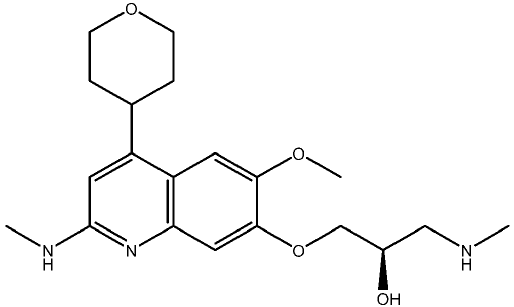 |
| 1026 | 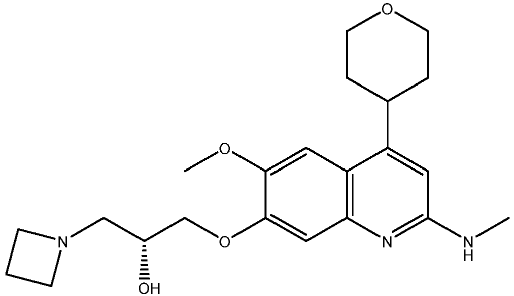 |
| 1027 | 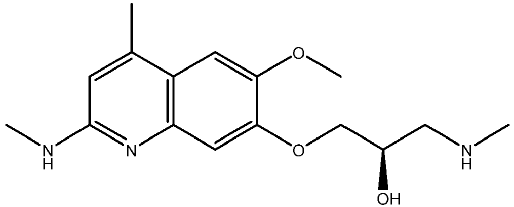 |
| 1028 | 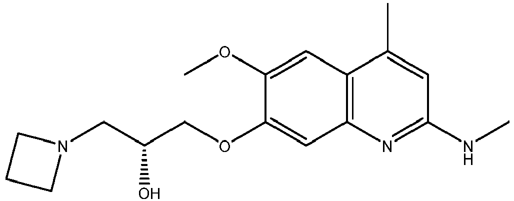 |
| 1029 | 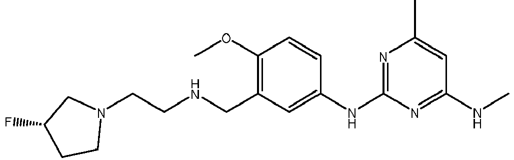 |
| 1030 | 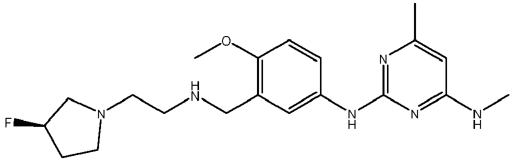 |
| 1031 | 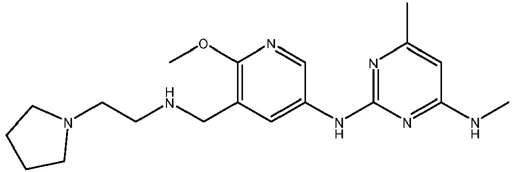 |
| 1032 | 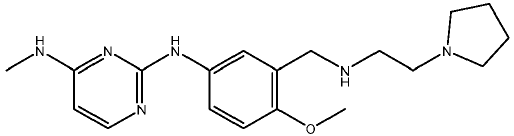 |
| 1033 | 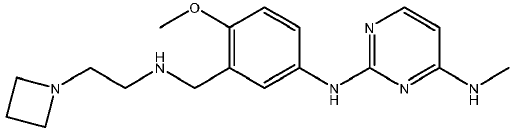 |
| 1034 | 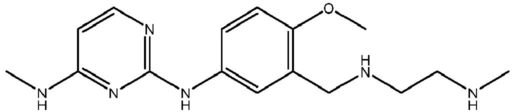 |
| 1035 | 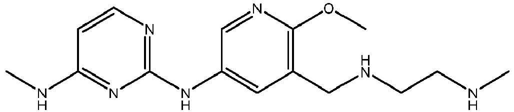 |
| 1036 | 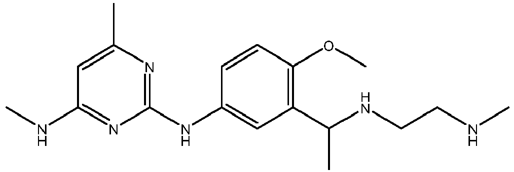 |
| 1037 | 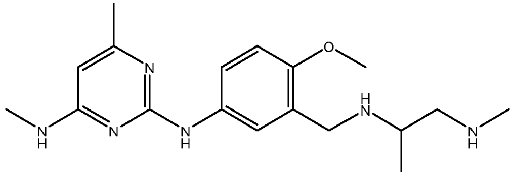 |
| 1038 | 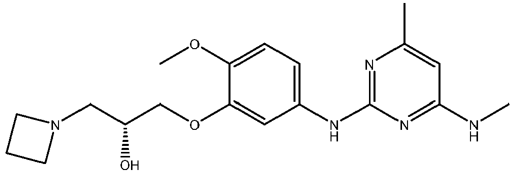 |
| 1039 | 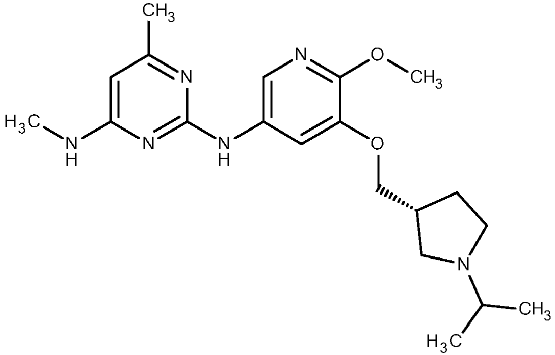 |
| 1040 | 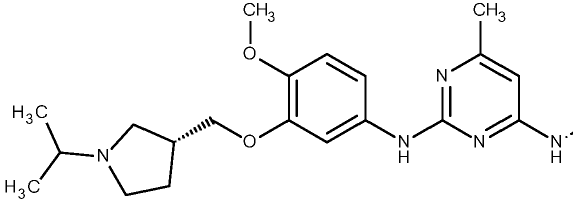 |
| 1041 | 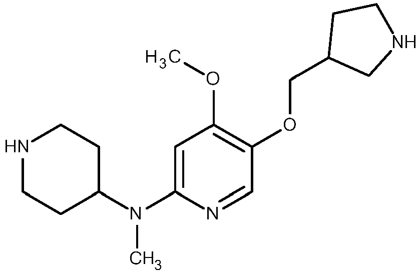 |
| 1042 | 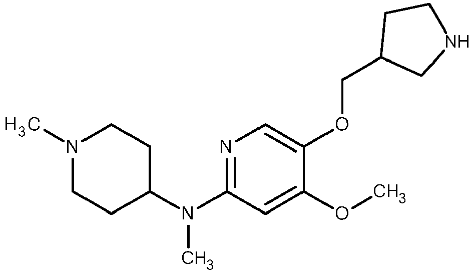 |

**Table 5**

| **Compound No.** | **Structure** |
|---|---|
| 1043 | 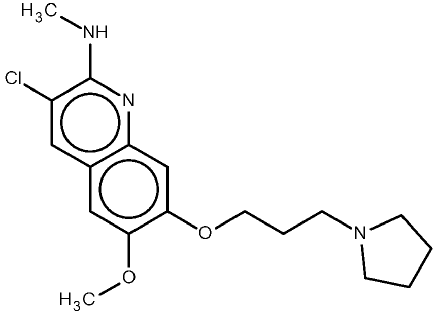 |
| 1044 | 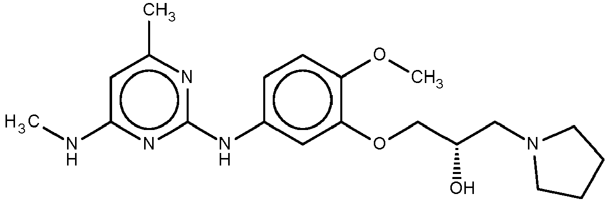 |
| 1045 | 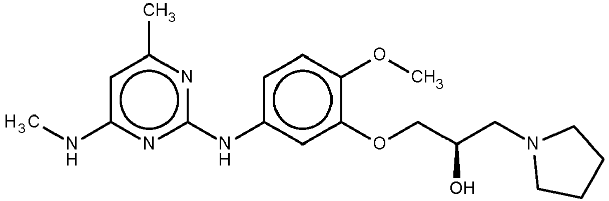 |
| 1046 | 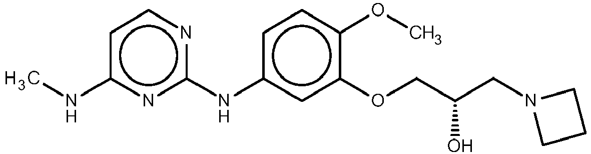 |
| 1047 | 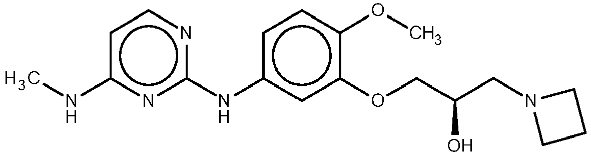 |
| 1048 | 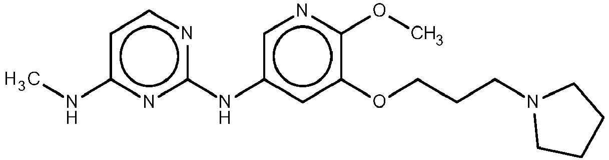 |
| 1049 | 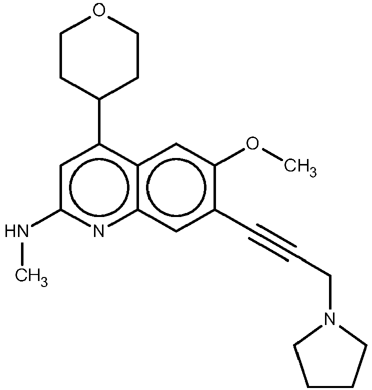 |
| 1050 | 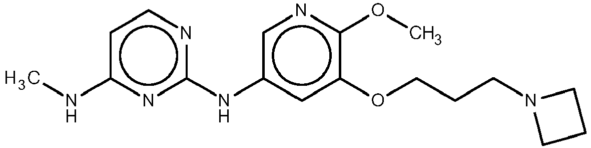 |
| 1051 | 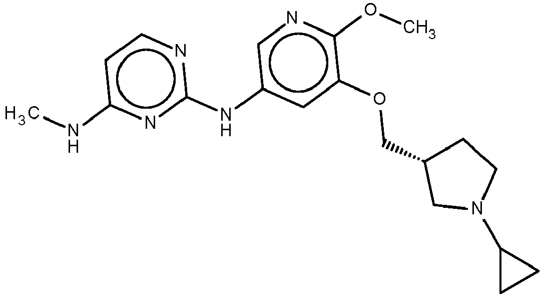 |
| 1052 | 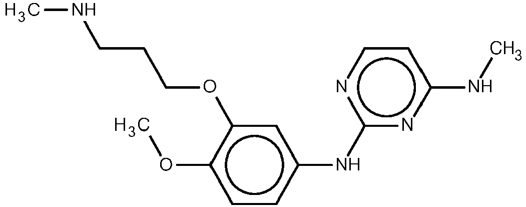 |
| 1053 | 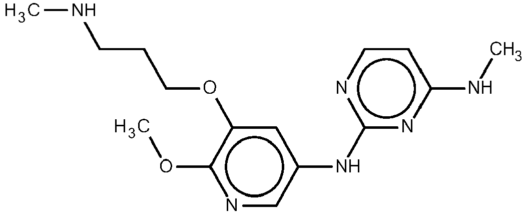 |
| 1054 | 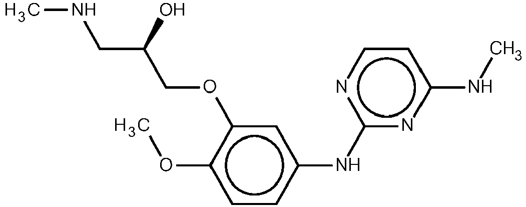 |
| 1055 | 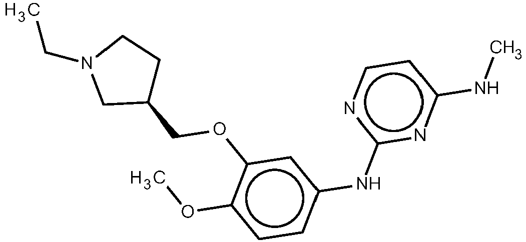 |
| 1056 | 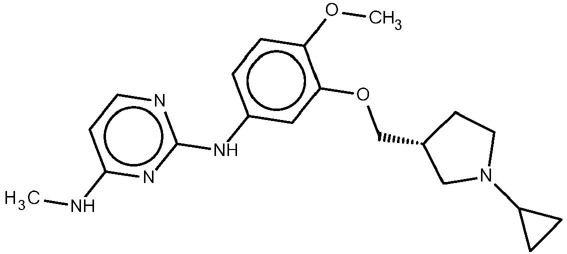 |
| 1057 | 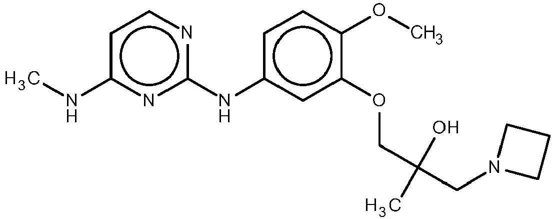 |
| 1058 | 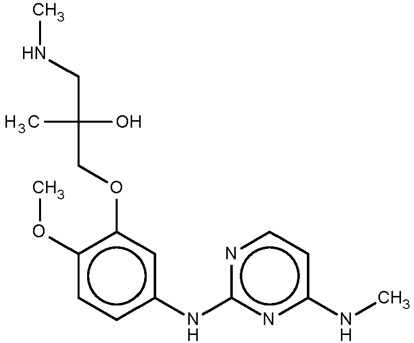 |
| 1059 | 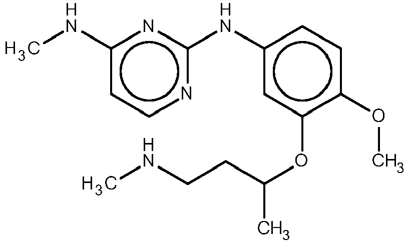 |
| 1060 | 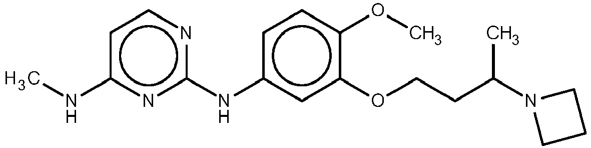 |
| 1061 | 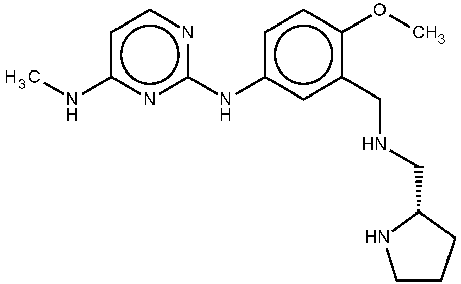 |
| 1062 | 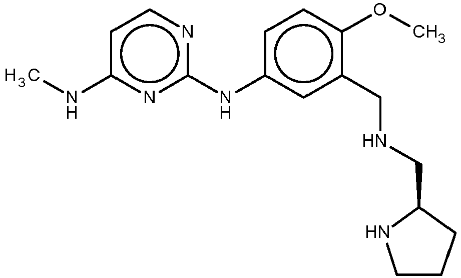 |
| 1063 | 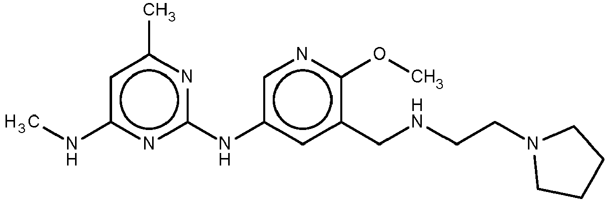 |
| 1064 | 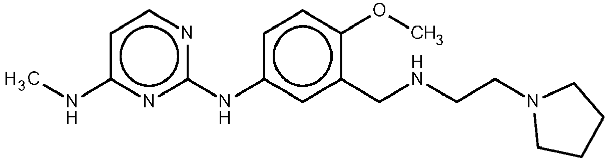 |
| 1065 | 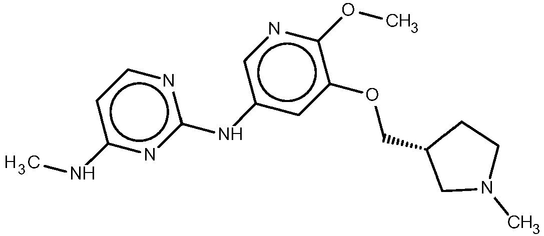 |
| 1066 | 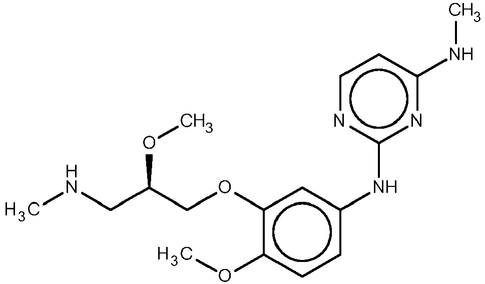 |
| 1067 | 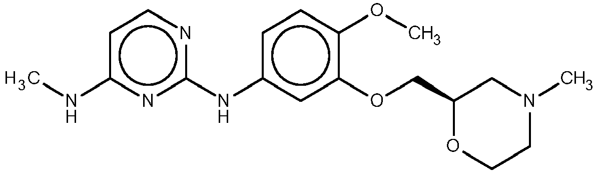 |
| 1068 | 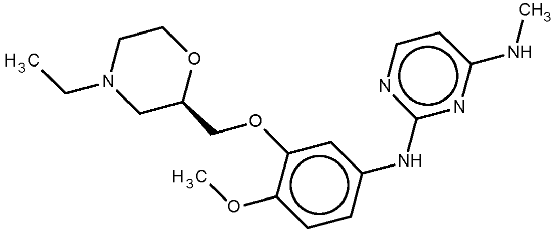 |
| 1069 | 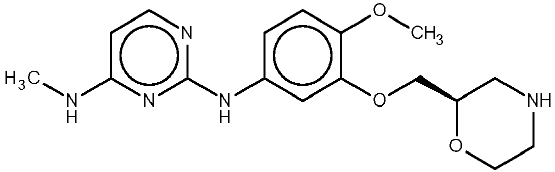 |
| 1070 | 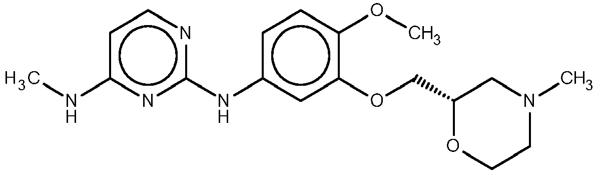 |
| 1071 | 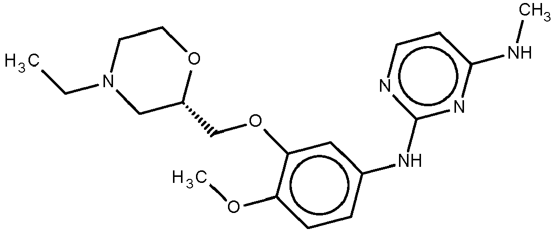 |
| 1072 | 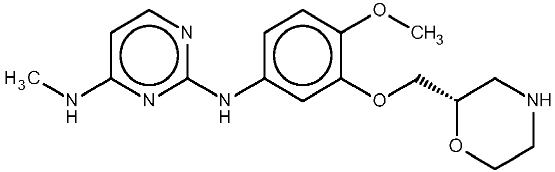 |
| 1073 | 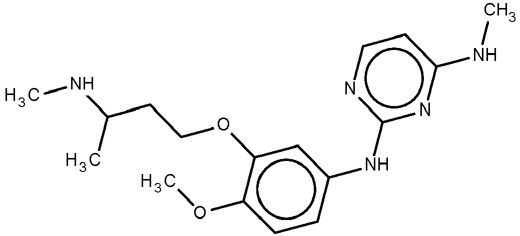 |
| 1074 | 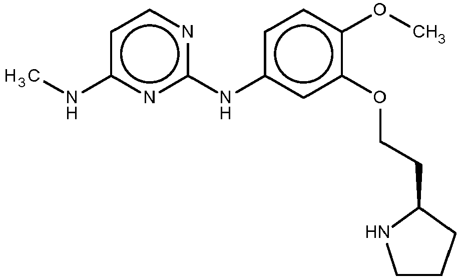 |
| 1075 | 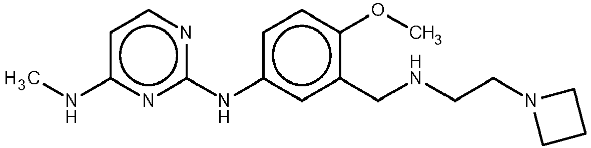 |
| 1076 | 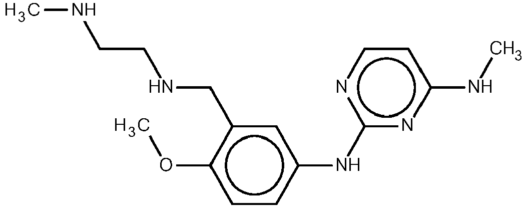 |
| 1077 | 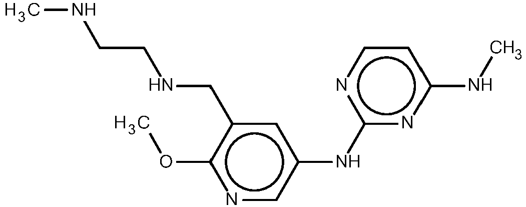 |
| 1078 | 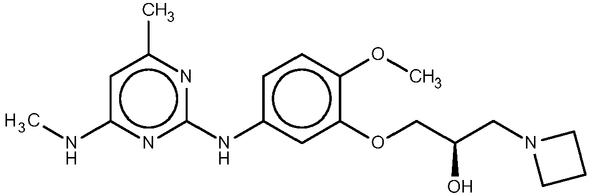 |
| 1079 | 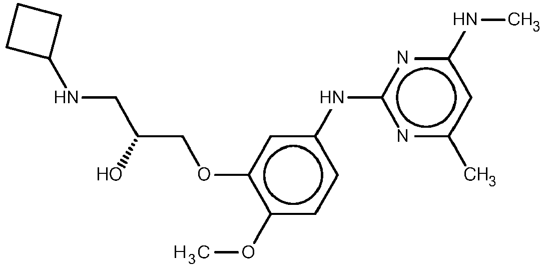 |
| 1080 | 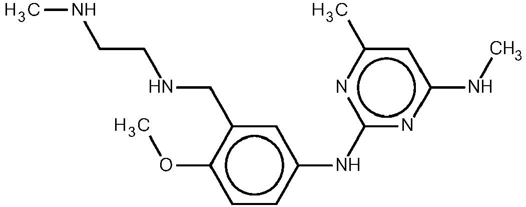 |
| 1081 | 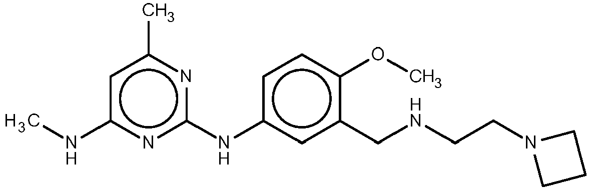 |
| 1082 | 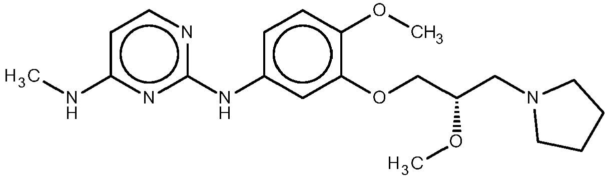 |
| 1083 | 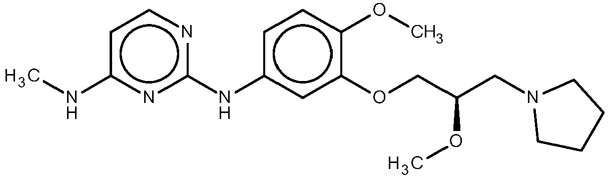 |
| 1084 | 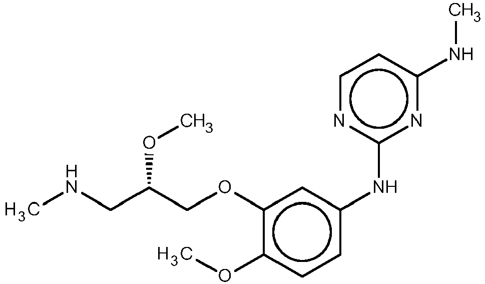 |
| 1085 | 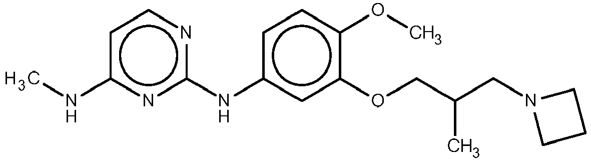 |
| 1086 | 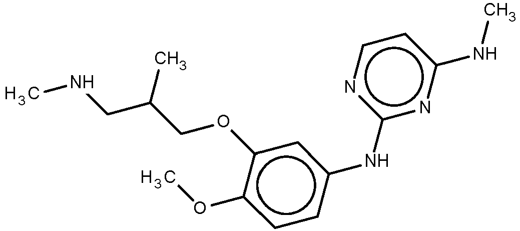 |
| 1087 | 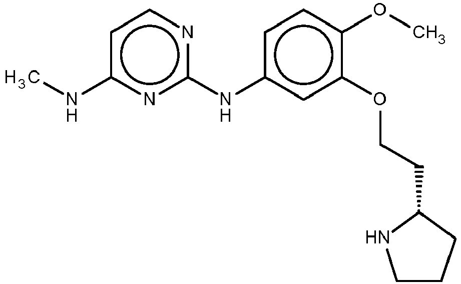 |
| 1088 | 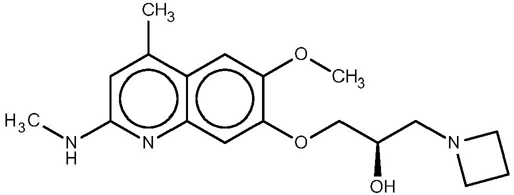 |
| 1089 | 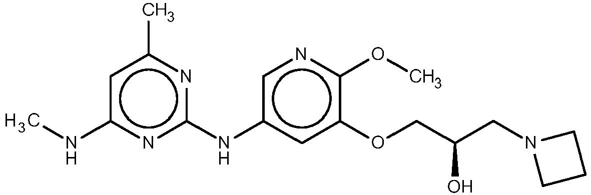 |
| 1090 | 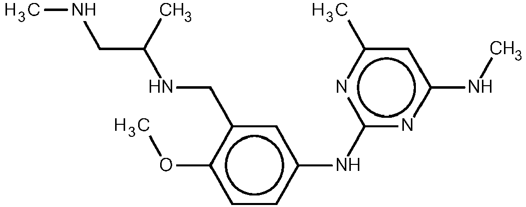 |
| 1091 | 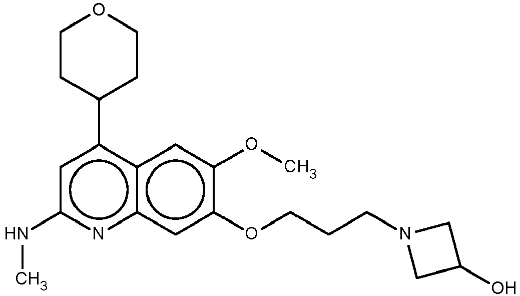 |
| 1092 | 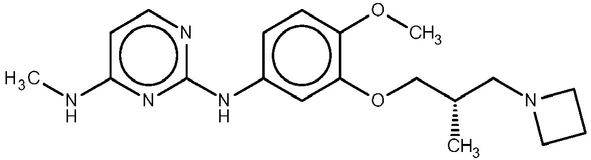 |
| 1093 | 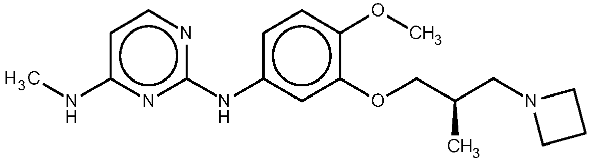 |
| 1094 | 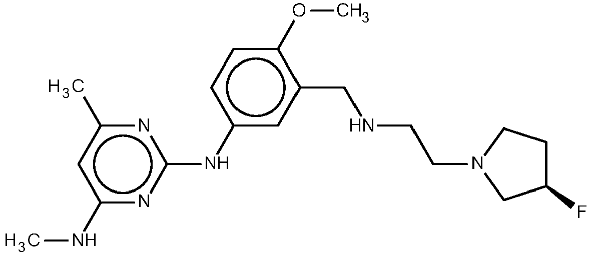 |
| 1095 | 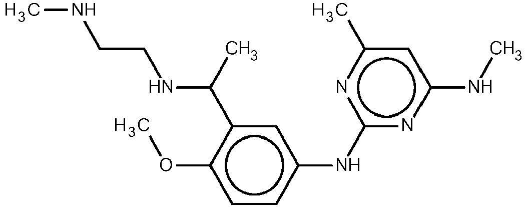 |
| 1096 | 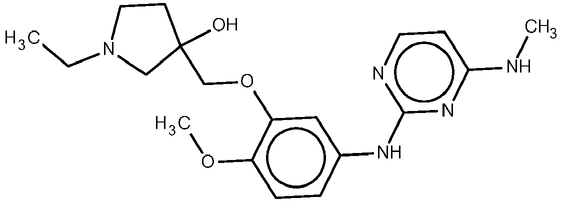 |
| 1097 | 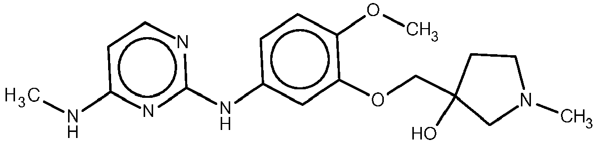 |
| 1098 | 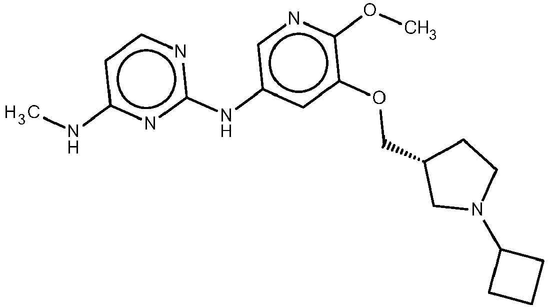 |
| 1099 | 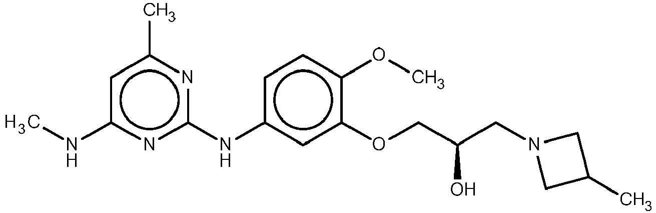 |
| 1100 | 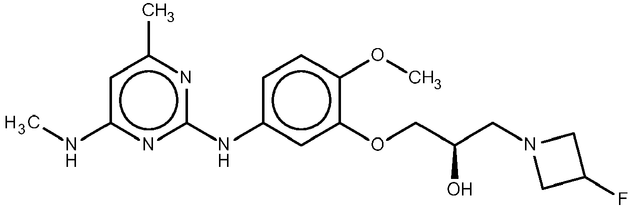 |
| 1101 | 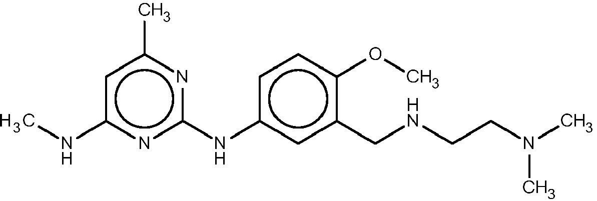 |
| 1102 | 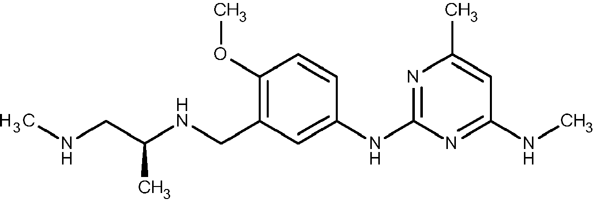 |
| 1103 | 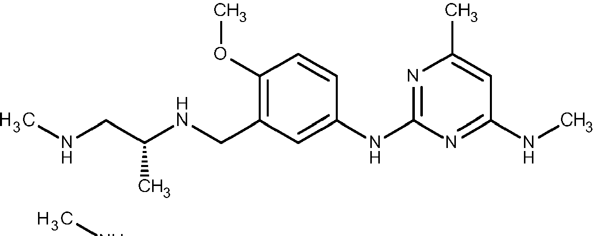 |
| 1104 | 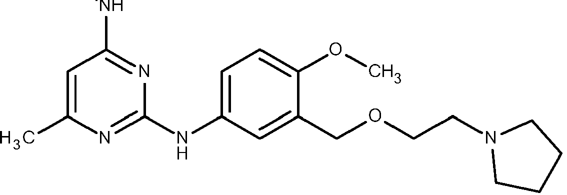 |
| 1105 | 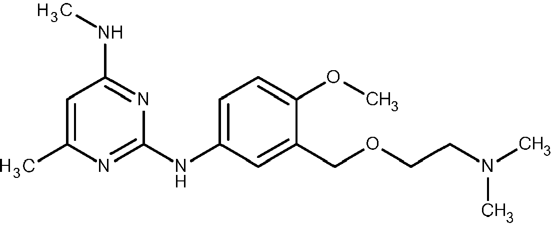 |
| 1106 | 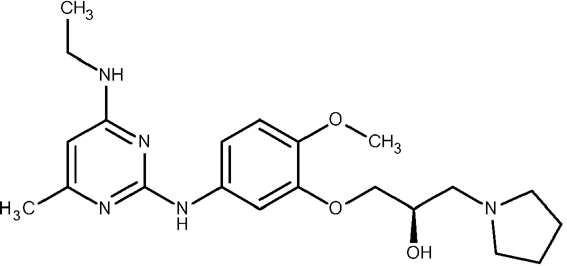 |
| 1107 | 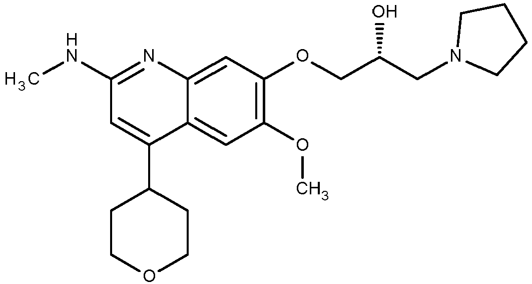 |
| 1108 | 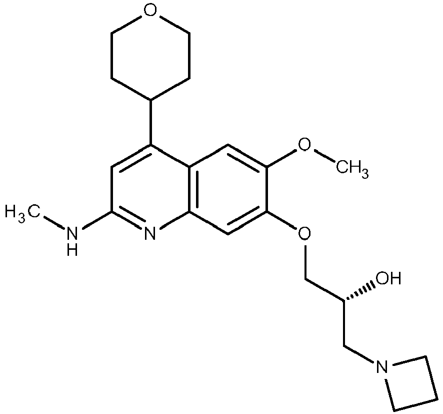 |
| 1109 | 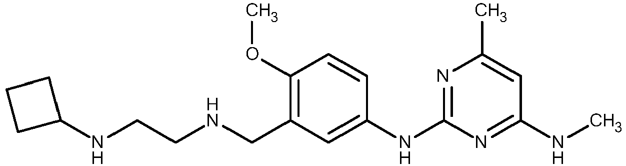 |
| 1110 | 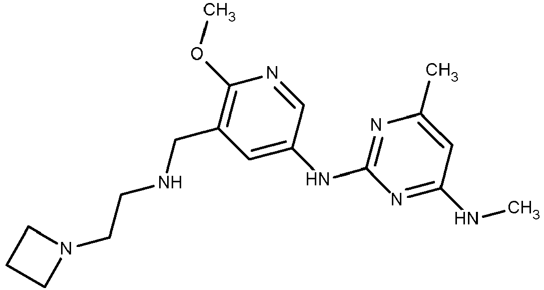 |
| 1111 | 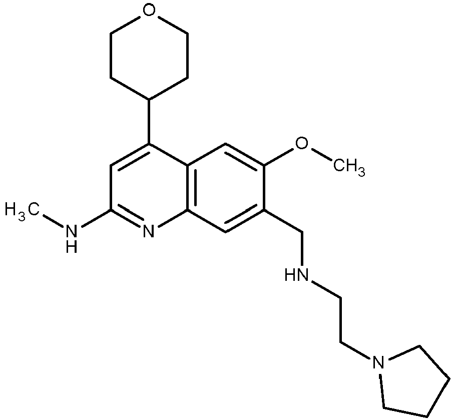 |
| 1112 | 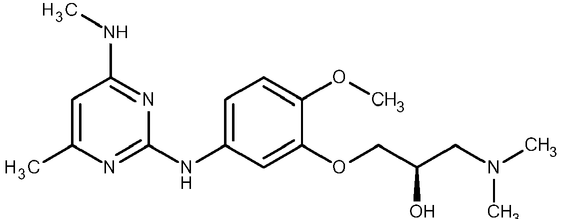 |
| 1113 | 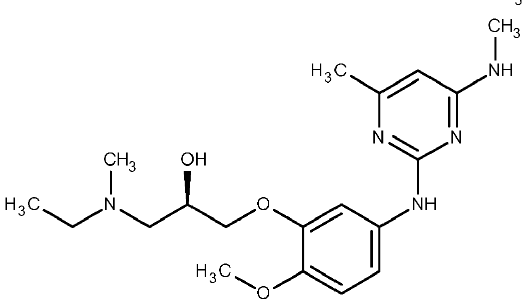 |
| 1114 | 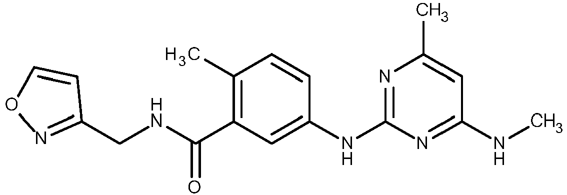 |
| 1115 | 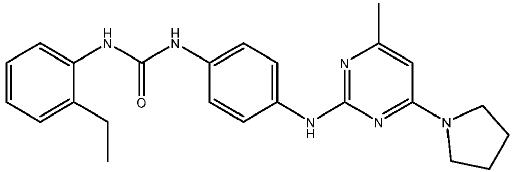 |
| 1116 | 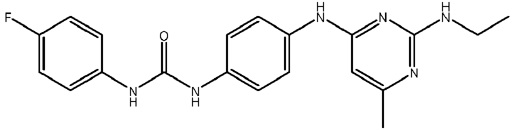 |
| 1117 | 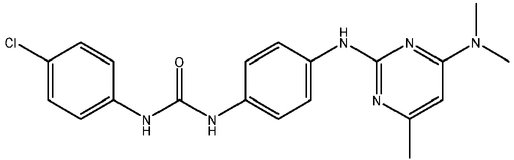 |
| 1118 | 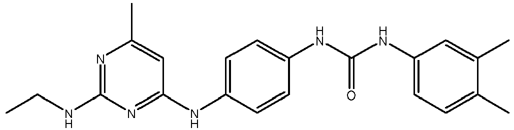 |
| 1119 | 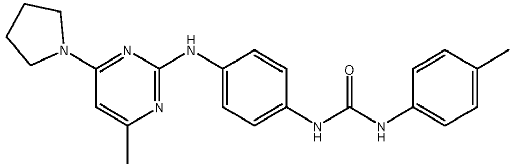 |

As used herein, "alkyl", "C₁, C₂, C₃, C₄, C₅ or C₆ alkyl" or "C₁-C₆ alkyl" is intended to include C₁, C₂, C₃, C₄, C₅ or C₆ straight chain (linear) saturated aliphatic hydrocarbon groups and C₃, C₄, C₅ or C₆ branched saturated aliphatic hydrocarbon groups. For example, C₁-C₆ alkyl is intended to include C₁, C₂, C₃, C₄, C₅ and C₆ alkyl groups. Examples of alkyl include, moieties having from one to six carbon atoms, such as, but not limited to, methyl, ethyl, n-propyl, i-propyl, n-butyl, s-butyl, t-butyl, n-pentyl, s-pentyl or n-hexyl.

In certain embodiments, a straight chain or branched alkyl has six or fewer carbon atoms (*e.g*., C₁-C₆ for straight chain, C₃-C₆ for branched chain), and in another embodiment, a straight chain or branched alkyl has four or fewer carbon atoms.

As used herein, the term "cycloalkyl" refers to a saturated or unsaturated nonaromatic hydrocarbon mono- or multi-ring (e.g., fused, bridged, or spiro rings) system having 3 to 30 carbon atoms (e.g., C₃-C₁₂, C₃-C₁₀, or C₃-C₈). Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, 1,2,3,4-tetrahydronaphthalenyl, and adamantyl. The term "heterocycloalkyl" refers to a saturated or unsaturated nonaromatic 3-8 membered monocyclic, 7-12 membered bicyclic (fused, bridged, or spiro rings), or 11-14 membered tricyclic ring system (fused, bridged, or spiro rings) having one or more heteroatoms (such as O, N, S, P, or Se), e.g., 1 or 1-2 or 1-3 or 1-4 or 1-5 or 1-6 heteroatoms, or *e.g.*, 1, 2, 3, 4, 5, or 6 heteroatoms, independently selected from the group consisting of nitrogen, oxygen and sulfur, unless specified otherwise. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofuran]-yl, 7'H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like. In the case of multicyclic non-aromatic rings, only one of the rings needs to be non-aromatic (*e.g*., 1,2,3,4-tetrahydronaphthalenyl or 2,3-dihydroindole).

The term "optionally substituted alkyl" refers to unsubstituted alkyl or alkyl having designated substituents replacing one or more hydrogen atoms on one or more carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

As used herein, "alkyl linker" or "alkylene linker" is intended to include C₁, C₂, C₃, C₄, C₅ or C₆ straight chain (linear) saturated divalent aliphatic hydrocarbon groups and C₃, C₄, C₅ or C₆ branched saturated aliphatic hydrocarbon groups. For example, C₁-C₆ alkylene linker is intended to include C₁, C₂, C₃, C₄, C₅ and C₆ alkylene linker groups. Examples of alkylene linker include, moieties having from one to six carbon atoms, such as, but not limited to, methyl (-CH₂-), ethyl (-CH₂CH₂-), n-propyl (-CH₂CH₂CH₂-), i-propyl (-CHCH₃CH₂-), n-butyl (-CH₂CH₂CH₂CH₂-), s-butyl (-CHCH₃CH₂CH₂-), i-butyl (-C(CH₃) ₂CH₂-), n-pentyl (-CH₂CH₂CH₂CH₂CH₂-), s-pentyl (-CHCH₃CH₂CH₂CH₂-) or n-hexyl (-CH₂CH₂CH₂CH₂CH₂CH₂-).

"Alkenyl" includes unsaturated aliphatic groups analogous in length and possible substitution to the alkyls described above, but that contain at least one double bond. For example, the term "alkenyl" includes straight chain alkenyl groups (*e.g*., ethenyl, propenyl, butenyl, pentenyl, hexenyl, heptenyl, octenyl, nonenyl, decenyl), and branched alkenyl groups.

In certain embodiments, a straight chain or branched alkenyl group has six or fewer carbon atoms in its backbone (*e.g*., C₂-C₆ for straight chain, C₃-C₆ for branched chain). The term "C₂-C₆" includes alkenyl groups containing two to six carbon atoms. The term "C₃-C₆" includes alkenyl groups containing three to six carbon atoms.

The term "optionally substituted alkenyl" refers to unsubstituted alkenyl or alkenyl having designated substituents replacing one or more hydrogen atoms on one or more hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, heterocyclyl, alkylaryl, or an aromatic or heteroaromatic moiety.

"Alkynyl" includes unsaturated aliphatic groups analogous in length and possible substitution to the alkyls described above, but which contain at least one triple bond. For example, "alkynyl" includes straight chain alkynyl groups (e.g., ethynyl, propynyl, butynyl, pentynyl, hexynyl, heptynyl, octynyl, nonynyl, decynyl), and branched alkynyl groups. In certain embodiments, a straight chain or branched alkynyl group has six or fewer carbon atoms in its backbone (*e.g*., C₂-C₆ for straight chain, C₃-C₆ for branched chain). The term "C₂-C₆" includes alkynyl groups containing two to six carbon atoms. The term "C₃-C₆" includes alkynyl groups containing three to six carbon atoms. As used herein, "C₂-C₆ alkenylene linker" or "C₂-C₆ alkynylene linker" is intended to include C₂, C₃, C₄, C₅ or C₆ chain (linear or branched) divalent unsaturated aliphatic hydrocarbon groups. For example, C₂-C₆ alkenylene linker is intended to include C₂, C₃, C₄, C₅ and C₆ alkenylene linker groups.

As used herein, the terms "heteroalkyl", "heteroalkylene linker", "heteroalkenyl", "heteroalkenylene linker", "heteroalkynyl", and "heteroalkynylene linker", are intended to refer to aliphatic hydrocarbon groups that include, e.g., C₁ to C₁₀ carbon atoms and one or more heteroatoms, *e.g.,* 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur. These aliphatic hydrocarbon groups can either be linear or branched, saturated or unsaturated.

The term "optionally substituted alkynyl" refers to unsubstituted alkynyl or alkynyl having designated substituents replacing one or more hydrogen atoms on one or more hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Other optionally substituted moieties (such as optionally substituted heteroalkyl, cycloalkyl, heterocycloalkyl, aryl, or heteroaryl) include both the unsubstituted moieties and the moieties having one or more of the designated substituents. For example, substituted heterocycloalkyl includes those substituted with one or more alkyl groups, such as 2,2,6,6-tetramethyl-piperidinyl and 2,2,6,6-tetramethyl-1,2,3,6-tetrahydropyridinyl.

"Aryl" includes groups with aromaticity, including "conjugated," or multicyclic systems with one or more aromatic rings and do not contain any heteroatom in the ring structure. Examples include phenyl, naphthalenyl, etc.

"Heteroaryl" groups are aryl groups, as defined above, except having from one to four heteroatoms in the ring structure, and may also be referred to as "aryl heterocycles" or "heteroaromatics." As used herein, the term "heteroaryl" is intended to include a stable 5-, 6-, or 7-membered monocyclic or 7-, 8-, 9-, 10-, 11- or 12-membered bicyclic aromatic heterocyclic ring which consists of carbon atoms and one or more heteroatoms, *e.g*., 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur. The nitrogen atom may be substituted or unsubstituted (*i.e*., N or NR wherein R is H or other substituents, as defined). The nitrogen and sulfur heteroatoms may optionally be oxidized (*i.e*., N→O and S(O)ₚ, where p = 1 or 2). It is to be noted that total number of S and O atoms in the aromatic heterocycle is not more than 1.

Examples of heteroaryl groups include pyrrole, furan, thiophene, thiazole, isothiazole, imidazole, triazole, tetrazole, pyrazole, oxazole, isoxazole, pyridine, pyrazine, pyridazine, pyrimidine, and the like.

Furthermore, the terms "aryl" and "heteroaryl" include multicyclic aryl and heteroaryl groups, *e.g.,* tricyclic, bicyclic, *e.g.,* naphthalene, benzoxazole, benzodioxazole, benzothiazole, benzoimidazole, benzothiophene, quinoline, isoquinoline, naphthrydine, indole, benzofuran, purine, benzofuran, deazapurine, indolizine.

The cycloalkyl, heterocycloalkyl, aryl, or heteroaryl ring can be substituted at one or more ring positions (e.g., the ring-forming carbon or heteroatom such as N) with such substituents as described above, for example, alkyl, alkenyl, alkynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl and heteroaryl groups can also be fused or bridged with alicyclic or heterocyclic rings, which are not aromatic so as to form a multicyclic system (e.g., tetralin, methylenedioxyphenyl such as benzo[d][1,3]dioxole-5-yl).

As used herein, "carbocycle" or "carbocyclic ring" is intended to include any stable monocyclic, bicyclic or tricyclic ring having the specified number of carbons, any of which may be saturated, unsaturated, or aromatic. Carbocycle includes cycloalkyl and aryl. For example, a C₃-C₁₄ carbocycle is intended to include a monocyclic, bicyclic or tricyclic ring having 3, 4, 5, 6, 7, 8, 9, 10, 11, 12, 13 or 14 carbon atoms. Examples of carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, fluorenyl, phenyl, naphthyl, indanyl, adamantyl and tetrahydronaphthyl. Bridged rings are also included in the definition of carbocycle, including, for example, [3.3.0]bicyclooctane, [4.3.0]bicyclononane, and [4.4.0] bicyclodecane and [2.2.2] bicyclooctane. A bridged ring occurs when one or more carbon atoms link two non-adjacent carbon atoms. In one embodiment, bridge rings are one or two carbon atoms. It is noted that a bridge always converts a monocyclic ring into a tricyclic ring. When a ring is bridged, the substituents recited for the ring may also be present on the bridge. Fused (e.g., naphthyl, tetrahydronaphthyl) and spiro rings are also included.

As used herein, "heterocycle" or "heterocyclic group" includes any ring structure (saturated, unsaturated, or aromatic) which contains at least one ring heteroatom (*e.g*., 1-4 heteroatoms selected from N, O and S). Heterocycle includes heterocycloalkyl and heteroaryl. Examples of heterocycles include, but are not limited to, morpholine, pyrrolidine, tetrahydrothiophene, piperidine, piperazine, oxetane, pyran, tetrahydropyran, azetidine, and tetrahydrofuran.

Examples of heterocyclic groups include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl (e.g., benzo[d][1,3]dioxole-5-yl), morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2,4-oxadiazol5(4H)-one, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl and xanthenyl.

The term "substituted," as used herein, means that any one or more hydrogen atoms on the designated atom is replaced with a selection from the indicated groups, provided that the designated atom's normal valency is not exceeded, and that the substitution results in a stable compound. When a substituent is oxo or keto (*i.e*., =O), then 2 hydrogen atoms on the atom are replaced. Keto substituents are not present on aromatic moieties. Ring double bonds, as used herein, are double bonds that are formed between two adjacent ring atoms (*e.g*., C=C, C=N or N=N). "Stable compound" and "stable structure" are meant to indicate a compound that is sufficiently robust to survive isolation to a useful degree of purity from a reaction mixture, and formulation into an efficacious therapeutic agent.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such formula. Combinations of substituents and/or variables are permissible, but only if such combinations result in stable compounds.

When any variable (*e.g*., R) occurs more than one time in any constituent or formula for a compound, its definition at each occurrence is independent of its definition at every other occurrence. Thus, for example, if a group is shown to be substituted with 0-2 R moieties, then the group may optionally be substituted with up to two R moieties and R at each occurrence is selected independently from the definition of R. Also, combinations of substituents and/or variables are permissible, but only if such combinations result in stable compounds.

The term "hydroxy" or "hydroxyl" includes groups with an -OH or -O⁻.

As used herein, "halo" or "halogen" refers to fluoro, chloro, bromo and iodo. The term "perhalogenated" generally refers to a moiety wherein all hydrogen atoms are replaced by halogen atoms. The term "haloalkyl" or "haloalkoxyl" refers to an alkyl or alkoxyl substituted with one or more halogen atoms.

The term "carbonyl" includes compounds and moieties which contain a carbon connected with a double bond to an oxygen atom. Examples of moieties containing a carbonyl include, but are not limited to, aldehydes, ketones, carboxylic acids, amides, esters, anhydrides, etc.

The term "carboxyl" refers to -COOH or its C₁-C₆ alkyl ester.

"Acyl" includes moieties that contain the acyl radical (R-C(O)-) or a carbonyl group. "Substituted acyl" includes acyl groups where one or more of the hydrogen atoms are replaced by, for example, alkyl groups, alkynyl groups, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

"Aroyl" includes moieties with an aryl or heteroaromatic moiety bound to a carbonyl group. Examples of aroyl groups include phenylcarboxy, naphthyl carboxy, etc.

"Alkoxyalkyl," "alkylaminoalkyl," and "thioalkoxyalkyl" include alkyl groups, as described above, wherein oxygen, nitrogen, or sulfur atoms replace one or more hydrocarbon backbone carbon atoms.

The term "alkoxy" or "alkoxyl" includes substituted and unsubstituted alkyl, alkenyl and alkynyl groups covalently linked to an oxygen atom. Examples of alkoxy groups or alkoxyl radicals include, but are not limited to, methoxy, ethoxy, isopropyloxy, propoxy, butoxy and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy and trichloromethoxy.

The term "ether" or "alkoxy" includes compounds or moieties which contain an oxygen bonded to two carbon atoms or heteroatoms. For example, the term includes "alkoxyalkyl," which refers to an alkyl, alkenyl, or alkynyl group covalently bonded to an oxygen atom which is covalently bonded to an alkyl group.

The term "ester" includes compounds or moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, etc.

The term "thioalkyl" includes compounds or moieties which contain an alkyl group connected with a sulfur atom. The thioalkyl groups can be substituted with groups such as alkyl, alkenyl, alkynyl, halogen, hydroxyl, alkylcarbonyloxy, arylcarbonyloxy, alkoxycarbonyloxy, aryloxycarbonyloxy, carboxylate, carboxyacid, alkylcarbonyl, arylcarbonyl, alkoxycarbonyl, aminocarbonyl, alkylaminocarbonyl, dialkylaminocarbonyl, alkylthiocarbonyl, alkoxyl, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties.

The term "thiocarbonyl" or "thiocarboxy" includes compounds and moieties which contain a carbon connected with a double bond to a sulfur atom.

The term "thioether" includes moieties which contain a sulfur atom bonded to two carbon atoms or heteroatoms. Examples of thioethers include, but are not limited to alkthioalkyls, alkthioalkenyls, and alkthioalkynyls. The term "alkthioalkyls" include moieties with an alkyl, alkenyl, or alkynyl group bonded to a sulfur atom which is bonded to an alkyl group. Similarly, the term "alkthioalkenyls" refers to moieties wherein an alkyl, alkenyl or alkynyl group is bonded to a sulfur atom which is covalently bonded to an alkenyl group; and alkthioalkynyls" refers to moieties wherein an alkyl, alkenyl or alkynyl group is bonded to a sulfur atom which is covalently bonded to an alkynyl group.

As used herein, "amine" or "amino" refers to -NH₂. "Alkylamino" includes groups of compounds wherein the nitrogen of -NH₂ is bound to at least one alkyl group. Examples of alkylamino groups include benzylamino, methylamino, ethylamino, phenethylamino, etc. "Dialkylamino" includes groups wherein the nitrogen of -NH₂ is bound to two alkyl groups. Examples of dialkylamino groups include, but are not limited to, dimethylamino and diethylamino. "Arylamino" and "diarylamino" include groups wherein the nitrogen is bound to at least one or two aryl groups, respectively. "Aminoaryl" and "aminoaryloxy" refer to aryl and aryloxy substituted with amino. "Alkylarylamino," "alkylaminoaryl" or "arylaminoalkyl" refers to an amino group which is bound to at least one alkyl group and at least one aryl group. "Alkaminoalkyl" refers to an alkyl, alkenyl, or alkynyl group bound to a nitrogen atom which is also bound to an alkyl group. "Acylamino" includes groups wherein nitrogen is bound to an acyl group. Examples of acylamino include, but are not limited to, alkylcarbonylamino, arylcarbonylamino, carbamoyl and ureido groups.

The term "amide" or "aminocarboxy" includes compounds or moieties that contain a nitrogen atom that is bound to the carbon of a carbonyl or a thiocarbonyl group. The term includes "alkaminocarboxy" groups that include alkyl, alkenyl or alkynyl groups bound to an amino group which is bound to the carbon of a carbonyl or thiocarbonyl group. It also includes "arylaminocarboxy" groups that include aryl or heteroaryl moieties bound to an amino group that is bound to the carbon of a carbonyl or thiocarbonyl group. The terms "alkylaminocarboxy", "alkenylaminocarboxy", "alkynylaminocarboxy" and "arylaminocarboxy" include moieties wherein alkyl, alkenyl, alkynyl and aryl moieties, respectively, are bound to a nitrogen atom which is in turn bound to the carbon of a carbonyl group. Amides can be substituted with substituents such as straight chain alkyl, branched alkyl, cycloalkyl, aryl, heteroaryl or heterocycle. Substituents on amide groups may be further substituted.

Compounds of the present disclosure that contain nitrogens can be converted to N-oxides by treatment with an oxidizing agent (e.g., 3-chloroperoxybenzoic acid (mCPBA) and/or hydrogen peroxides) to afford other compounds of the present disclosure. Thus, all shown and claimed nitrogen-containing compounds are considered, when allowed by valency and structure, to include both the compound as shown and its N-oxide derivative (which can be designated as N-->O or N⁺-O⁻). Furthermore, in other instances, the nitrogens in the compounds of the present disclosure can be converted to N-hydroxy or N-alkoxy compounds. For example, N-hydroxy compounds can be prepared by oxidation of the parent amine by an oxidizing agent such as m-CPBA. All shown and claimed nitrogen-containing compounds are also considered, when allowed by valency and structure, to cover both the compound as shown and its N-hydroxy (*i.e.,* N-OH) and N-alkoxy (*i.e.,* N-OR, wherein R is substituted or unsubstituted C₁-C₆ alkyl, C₁-C₆ alkenyl, C₁-C₆ alkynyl, 3-14-membered carbocycle or 3-14-membered heterocycle) derivatives.

In the present specification, the structural formula of the compound represents a certain isomer for convenience in some cases, but the present disclosure includes all isomers, such as geometrical isomers, optical isomers based on an asymmetrical carbon, stereoisomers, tautomers, and the like, it being understood that not all isomers may have the same level of activity. In addition, a crystal polymorphism may be present for the compounds represented by the formula. It is noted that any crystal form, crystal form mixture, or anhydride or hydrate thereof is included in the scope of the present disclosure.

"Isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

A carbon atom bonded to four nonidentical substituents is termed a "chiral center."

"Chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

"Geometric isomer" means the diastereomers that owe their existence to hindered rotation about double bonds or a cycloalkyl linker (e.g., 1,3-cylcobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present disclosure may be depicted as different chiral isomers or geometric isomers. It should also be understood that when compounds have chiral isomeric or geometric isomeric forms, all isomeric forms are intended to be included in the scope of the present disclosure, and the naming of the compounds does not exclude any isomeric forms, it being understood that not all isomers may have the same level of activity.

Furthermore, the structures and other compounds discussed in this disclosure include all atropic isomers thereof, it being understood that not all atropic isomers may have the same level of activity. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertible by tautomerizations is called tautomerism.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

Common tautomeric pairs are: ketone-enol, amide-nitrile, lactam-lactim, amide-imidic acid tautomerism in heterocyclic rings (*e*.*g*., in nucleobases such as guanine, thymine and cytosine), imine-enamine and enamine-enamine. Examples of lactam-lactim tautomerism are as shown below.

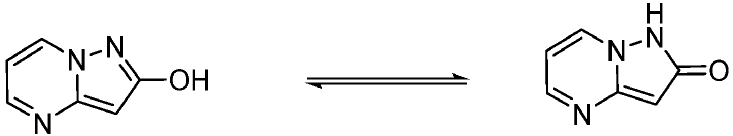

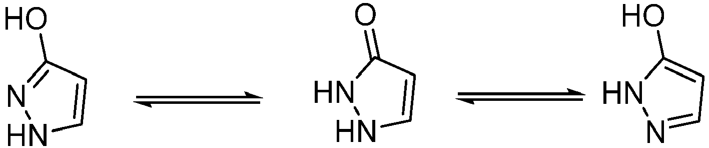

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

The term "crystal polymorphs", "polymorphs" or "crystal forms" means crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

The compounds of any Formula described herein include the compounds themselves, as well as their salts, and their solvates, if applicable. A salt, for example, can be formed between an anion and a positively charged group (e.g., amino) on a substituted benzene compound. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (e.g., trifluoroacetate). The term "pharmaceutically acceptable anion" refers to an anion suitable for forming a pharmaceutically acceptable salt. Likewise, a salt can also be formed between a cation and a negatively charged group (e.g., carboxylate) on a substituted benzene compound. Suitable cations include sodium ion, potassium ion, magnesium ion, calcium ion, and an ammonium cation such as tetramethylammonium ion. The substituted benzene compounds also include those salts containing quaternary nitrogen atoms.

Additionally, the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative" refers to compounds that have a common core structure, and are substituted with various groups as described herein. For example, all of the compounds represented by Formula (I) are amine-substituted aryl or heteroaryl compounds, and have Formula (I) as a common core.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include, but are not limited to, acyl sulfonimides, tetrazoles, sulfonates and phosphonates. See, *e*.*g*., Patani and LaVoie, Chem. Rev. 96, 3147-3176, 1996.

The present disclosure is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

The present disclosure provides methods for the synthesis of the compounds of any of the Formulae described herein. The present disclosure also provides detailed methods for the synthesis of various disclosed compounds of the present disclosure according to the following schemes as shown in the Examples.

In the descriptions and claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or all, of the group members are present in, employed in, or otherwise relevant to a given product or process. As used herein, the expressions "one or more of A, B, or C," "one or more A, B, or C," "one or more of A, B, and C," "one or more A, B, and C", "selected from A, B, and C," "selected from the group consisting of A, B, and C," and the like are used interchangeably and all refer to a selection from a group consisting of A, B, and /or C, i.e., one or more As, one or more Bs, one or more Cs, or any combination thereof, unless otherwise specified.

It is also noted that the term "comprising" is intended to be open and permits but does not require the inclusion of additional elements or steps. When the term "comprising" is used herein, the terms "consisting essentially of" and "consisting of" are thus also encompassed and disclosed. Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

The synthetic processes of the disclosure can tolerate a wide variety of functional groups, therefore various substituted starting materials can be used. The processes generally provide the desired final compound at or near the end of the overall process, although it may be desirable in certain instances to further convert the compound to a pharmaceutically acceptable salt thereof.

Compounds of the present disclosure can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry: Reactions, Mechanisms, and Structure, 5th edition, John Wiley & Sons: New York, 2001; Greene, T.W., Wuts, P.G. M., Protective Groups in Organic Synthesis, 3rd edition, John Wiley & Sons: New York, 1999; R. Larock, Comprehensive Organic Transformations, VCH Publishers (1989); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), are useful and recognized reference textbooks of organic synthesis known to those in the art. The following descriptions of synthetic methods are designed to illustrate, but not to limit, general procedures for the preparation of compounds of the present disclosure.

Compounds of the present disclosure can be conveniently prepared by a variety of methods familiar to those skilled in the art. The compounds of this disclosure having any of the Formulae described herein may be prepared according to the procedures illustrated in Schemes 1-9 below, from commercially available starting materials or starting materials which can be prepared using literature procedures. The variables (such as n, R³, R⁷, R⁸, and R⁹, etc.) in Schemes 1-9 are as defined in any Formula described herein, unless otherwise specified.

One of ordinary skill in the art will note that, during the reaction sequences and synthetic schemes described herein, the order of certain steps may be changed, such as the introduction and removal of protecting groups.

One of ordinary skill in the art will recognize that certain groups may require protection from the reaction conditions via the use of protecting groups. Protecting groups may also be used to differentiate similar functional groups in molecules. A list of protecting groups and how to introduce and remove these groups can be found in Greene, T.W., Wuts, P.G. M., Protective Groups in Organic Synthesis, 3rd edition, John Wiley & Sons: New York, 1999.

Preferred protecting groups include, but are not limited to:
For a hydroxyl moiety: TBS, benzyl, THP, Ac
For carboxylic acids: benzyl ester, methyl ester, ethyl ester, allyl ester
For amines: Cbz, BOC, DMB
For diols: Ac (x2) TBS (x2), or when taken together acetonides
For thiols: Ac
For benzimidazoles: SEM, benzyl, PMB, DMB
For aldehydes: di-alkyl acetals such as dimethoxy acetal or diethyl acetyl.

In the reaction schemes described herein, multiple stereoisomers may be produced. When no particular stereoisomer is indicated, it is understood to mean all possible stereoisomers that could be produced from the reaction. A person of ordinary skill in the art will recognize that the reactions can be optimized to give one isomer preferentially, or new schemes may be devised to produce a single isomer. If mixtures are produced, techniques such as preparative thin layer chromatography, preparative HPLC, preparative chiral HPLC, or preparative SFC may be used to separate the isomers.

The following abbreviations are used throughout the specification and are defined below:
- ACN: acetonitrile
- Ac: acetyl
- AcOH: acetic acid
- AlCl₃: aluminum chloride
- BINAP: (2,2'-bis(diphenylphosphino)-1,1'-binaphthyl)
- t-BuOK: potassium t-butoxide
- tBuONa or t-BuONa: sodium t-butoxide
- br: broad
- BOC: tert-butoxy carbonyl
- Cbz: benzyloxy carbonyl
- CDCl₃CHCl₃: chloroform
- CH₂Cl₂: dichloromethane
- CH₃CN: acetonitrile
- CsCO₃: cesium carbonate
- CH₃NO₃: nitromethane
- d: doublet
- dd: doublet of doublets
- dq: doublet of quartets
- DCE: 1,2 dichloroethane
- DCM: dichloromethane
- Δ: heat
- δ: chemical shift
- DIEA: N,N-diisopropylethylamine (Hunig's base)
- DMB: 2,4 dimethoxy benzyl
- DMF: N,N-Dimethylformamide
- DMSO: Dimethyl sulfoxide
- DMSO-*d6*: deuterated dimethyl sulfoxide
- EA or EtOAc: Ethyl acetate
- ES: electro spray
- Et₃N: triethylamine
- equiv: equivalents
- g: grams
- h: hours
- H₂O: water
- HCl: hydrogen chloride or hydrochloric acid
- HPLC: High performance liquid chromatography
- Hz: Hertz
- IPA: isopropyl alcohol
- i-PrOH: isopropyl alcohol
- J: NMR coupling constant
- K₂CO₃: potassium carbonate
- HI: potassium iodide
- KCN: potassium cyanide
- LCMS or LC-MS: Liquid chromatography mass spectrum
- M: molar
- m: multiplet
- mg: milligram
- MHz: megahertz
- mL: milliliter
- mm: millimeter
- mmol: millimole
- mol: mole
- [M+1]: molecular ion plus one mass unit
- m/z: mass/charge ratio
- m-CPBA: meta-chloroperbenzoic acid
- MeCN: Acetonitrile
- MeOH: methanol
- MeI: Methyl iodide
- min: minutes
- µm: micron
- MsCl: Mesyl chloride
- MW: microwave irradiation
- N: normal
- Na₂SO₄: sodium sulfate
- NH₃: ammonia
- NaBH(AcO)₃: sodium triacetoxyborohydride
- NaI: sodium iodide
- Na₂SO₄: sodium sulfate
- NH₄Cl: ammonium chloride
- NH₄HCO₃: ammonium bicarbonate
- nm: nanometer
- NMP: N-methylpyrrolidinone
- NMR: Nuclear Magnetic Resonance
- Pd(OAc)₂: palladium (II) acetate
- Pd/C: Palladium on carbon
- Pd₂(dba)₃: Tris(dibenzylideneacetone)dipalladium(0)
- PMB: para methoxybenzyl
- ppm: parts per million
- POCl₃: phosphoryl chloride
- prep-HPLC: preparative High Performance Liquid Chromatography
- PTSA: para-toluenesulfonic acid
- p-TsOH: para-toluenesulfonic acid
- RT: retention time
- rt: room temperature
- s: singlet
- t: triplet
- t-BuXPhos: 2-Di-*tert*-butylphosphino-2', 4', 6'-triisopropylbiphenyl
- TEA: Triethylamine
- TFA: trifluoroacetic acid
- TfO: triflate
- THP: tetrahydropyran
- TsOH: tosic acid
- UV: ultraviolet

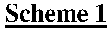

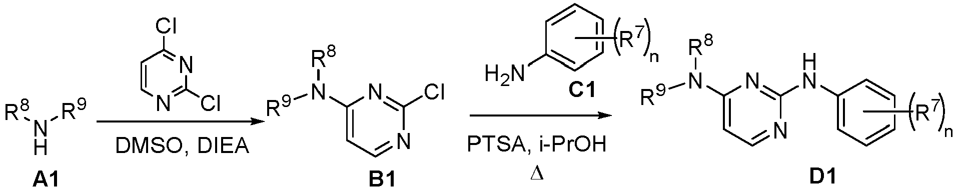

Scheme 1 shows the synthesis of *N*²-phenylpyrimidine-2,4-diamine compounds **D1** following a general route. 2,4-Dichloropyrimidine is combined in an organic solvent (*e*.*g*., DMSO) with a dialkylamine **A1** and a base (*e*.*g*. DIEA). The resulting 2-chloro- pyrimidine-4-amine **B1** is heated with a substituted aniline **C1** and an acid (*e*.*g*., PTSA) in an organic solvent (*e*.*g*., i-PrOH) and heated to afford the *N*²-phenylpyrimidine-2,4-diamine **D1.**

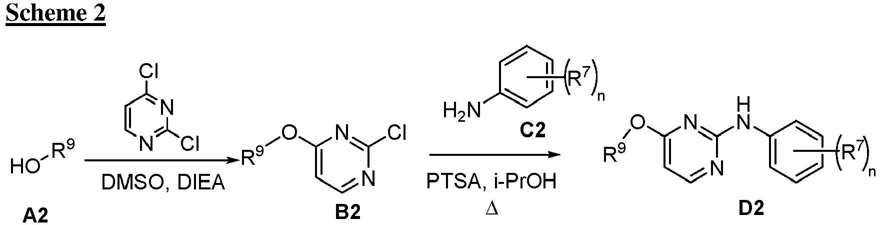

Scheme 2 shows the synthesis of phenylpyrimidine-2- amine compounds **D2** following a general route. 2,4-Dichloropyrimidine is combined in an organic solvent (*e*.*g*., DMSO) with an alcohol **A2** and a base (e.g. DIEA). The resulting 2-chloropyrimidine **B2** is heated with a substituted aniline **C2** and an acid (*e*.*g*., PTSA) in an organic solvent (*e*.*g*., i-PrOH) and heated to afford the phenylpyrimidine-2- amine **D2.**

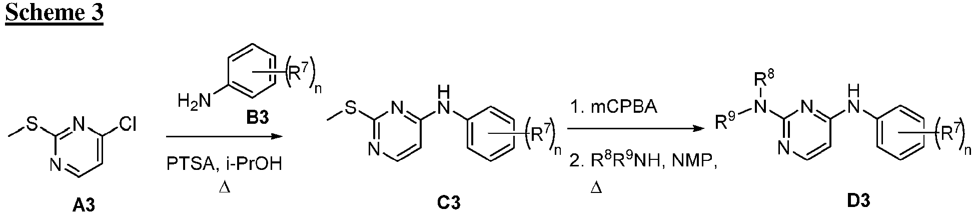

Scheme 3 shows the synthesis of *N*⁴-phenylpyrimidine-2,4-diamine compounds **D3** following a general route. 2-Chloro-4-(methylthio)pyrimidine **A3** is heated in an organic solvent (*e*.*g*., i-PrOH) with a substituted aniline **B3** and an acid (*e*.*g*., PTSA). The resulting substituted 2-(methylthio)-N-phenylpyrimidin-4-amine **C3** is treated with an oxidizing agent (*e.g.,* mCPBA), and then heated with an amine (e.g., NHR⁸R⁹) in an organic solvent (*e.g.,* NMP) to afford the *N*⁴-phenylpyrimidine-2,4-diamine **D3.**

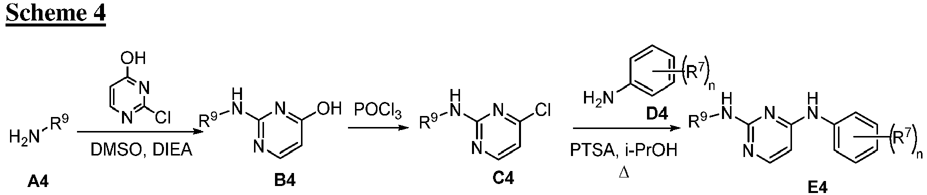

Scheme 4 shows the synthesis of *N*⁴-phenylpyrimidine-2,4-diamine compounds **E4** following a general route. 2-Chloropyrimidin-4-ol is combined with a primary amine **A4** and a base (*e.g.,* DIEA) in an organic solvent (*e.g.,* DMSO) to afford 2-aminopyrimidin-4-ol **B4,** which is then treated with a chlorinating agent (*e*.*g*., phosphoryl chloride). The resulting 4-chloropyrimidin-2-amine **C4** is heated with a substituted aniline **D4** and an acid (*e*.*g*., PTSA) in an organic solvent (*e*.*g*., i-PrOH) to afford the *N*⁴-phenylpyrimidine-2,4-diamine **E4**.

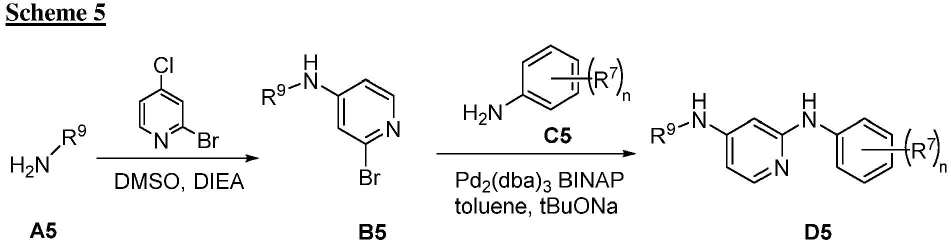

Scheme 5 shows the synthesis of *N*²-phenylpyridine-2,4-diamine compounds **D5** following a general route. 2-Bromo-4-chloropyridine is combined with a primary amine **A5** and a base (*e.g.,* DIEA) in an organic solvent (*e.g.,* DMSO). The resulting 2-bromo-pyridin-4-amine **B5** is coupled with a substituted aniline **C5** in an organic solvent (*e*.*g*., toluene) via a Buchwald-Hartwig amination employing a catalyst (*e.g.,* Pd₂(dba)₃), a ligand (*e.g.,* BINAP), and a base (*e*.*g*., tBuONa) to afford the *N*²-phenylpyridine-2,4-diamine **D5.**

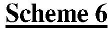

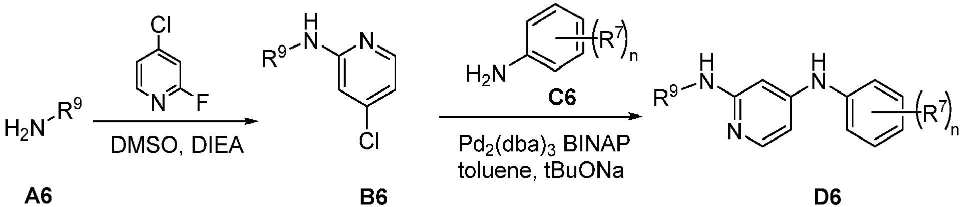

Scheme 6 shows the synthesis of *N*⁴-phenylpyridine-2,4-diamine compounds D6 following a general route. 4-Chloro-2-fluoropyridine is combined with a primary amine A6 and a base (e.g., DIEA) in an organic solvent (e.g., DMSO). The resulting 4-chloro-pyridin-2-amine B6 is coupled with a substituted aniline C6 in an organic solvent (e.g., toluene) via a Buchwald-Hartwig amination employing a catalyst (e.g., Pd₂(dba)₃), a ligand (e.g., BINAP), and a base (e.g., tBuONa) to afford the *N*⁴-phenylpyridine-2,4-diamine **D6**.

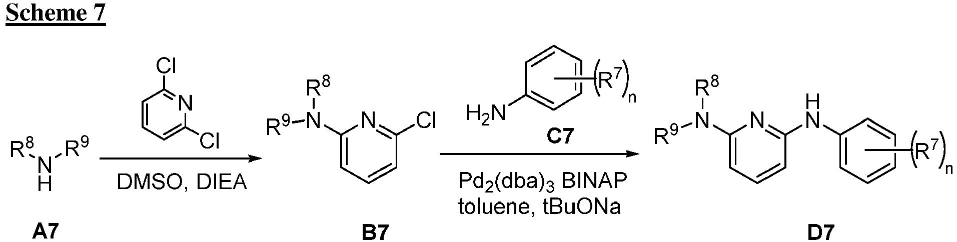

Scheme 7 shows the synthesis of *N*²-phenylpyridine-2,6-diamine compounds D7 following a general route. 2,6-Dichloropyridine is combined with an amine **A7** and a base (*e.g.,* DIEA) in an organic solvent (*e*.*g*., DMSO). The resulting 6-chloro-pyridin-2-amine **B7** is coupled with a substituted aniline **C7** in an organic solvent (*e*.*g*., toluene) via a Buchwald-Hartwig amination employing a catalyst (*e*.*g*., Pd₂(dba)₃), a ligand (*e*.*g*., BINAP), and a base (*e*.*g*., tBuONa) to afford the *N*²-phenylpyridine-2,6-diamine **D7**.

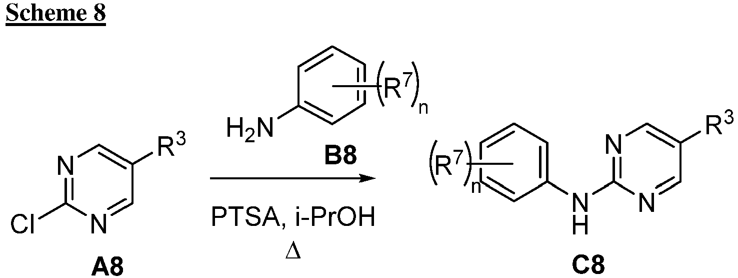

Scheme 8 shows the synthesis of N-phenylpyrimidin-2-amine compounds **C8** following a general route. 2-Chloro-pyrimidine **A8** is heated with a substituted aniline **B8** and an acid (*e.g.,* PTSA) in an organic solvent (*e.g.,* i-PrOH) to afford the *N-*phenylpyrimidin-2-amine **C8.**

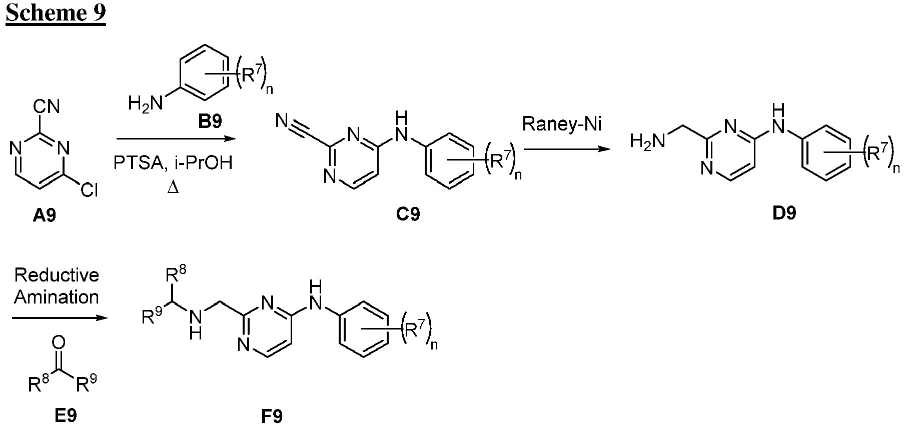

Scheme 9 shows the synthesis of 2-(alkylaminomethyl)-*N*-phenylpyrimidin-4-amine compounds **F9** following a general route. 4-Chloropyrimidine-2-carbonitrile **A9** is heated with a substituted aniline **B9** and an acid (*e.g.,* PTSA) in an organic solvent (*e.g.,* i-PrOH). The resulting 4-(phenylamino)pyrimidine-2-carbonitrile **C9** is treated with a reducing agent (*e*.*g*., Raney-Ni) to give the 2-(aminomethyl)-*N*-phenylpyrimidin-4-amine **D9.** Reductive amination with a carbonyl compound **E9** affords the 2-(alkylamino)methyl)-*N-*phenylpyrimidin-4-amine **F9.**

A person of ordinary skill in the art will recognize that in the above schemes the order of many of the steps are interchangeable.

Compounds of the present disclosure inhibit the histone methyltransferase activity of G9a, also known as KMT1C (lysine methyltransferase 1C) or EHMT2 (euchromatic histone methyltransferase 2), or a mutant thereof and, accordingly, , certain compounds disclosed herein are candidates for use in treating, or preventing certain conditions, diseases, and disorders in which EHMT2 plays a role. The present disclosure provides compounds for use in methods for treating conditions and diseases the course of which can be influenced by modulating the methylation status of histones or other proteins, wherein said methylation status is mediated at least in part by the activity of EHMT2. Modulation of the methylation status of histones can in turn influence the level of expression of target genes activated by methylation, and/or target genes suppressed by methylation. The method includes administering to a subject in need of such treatment, a therapeutically effective amount of a compound of the present disclosure, or a pharmaceutically acceptable salt, polymorph, solvate, or stereoisomer thereof.

Unless otherwise stated, any description of a method of treatment includes compounds for use in such treatment or prophylaxis as is described herein, as well as use of the compounds to prepare a medicament to treat or prevent such condition. The treatment includes treatment of human or non-human animals including rodents and other disease models.

This disclosure relates to compounds for use in a method of modulating the activity of EHMT2, which catalyzes the dimethylation of lysine 9 on histone H3 (H3K9) in a subject in need thereof. For example, the method comprises the step of administering to a subject having a cancer expressing a mutant EHMT2 a therapeutically effective amount of a compound described herein, wherein the compound(s) inhibits histone methyltransferase activity of EHMT2, thereby treating the cancer.

For example, the EHMT2-mediated cancer is selected from the group consisting of leukemia, prostate carcinoma, hepatocellular carcinoma, and lung cancer.

For example, the compounds disclosed herein can be used for treating cancer. For example, the cancer is a hematological cancer.

For example, the cancer is selected from the group consisting of brain and central nervous system (CNS) cancer, head and neck cancer, kidney cancer, ovarian cancer, pancreatic cancer, leukemia, lung cancer, lymphoma, myeloma, sarcoma, breast cancer, and prostate cancer. Preferably, a subject in need thereof is one who had, is having or is predisposed to developing brain and CNS cancer, kidney cancer, ovarian cancer, pancreatic cancer, leukemia, lymphoma, myeloma, and/or sarcoma. Exemplary brain and central CNS cancer includes medulloblastoma, oligodendroglioma, atypical teratoid/rhabdoid tumor, choroid plexus carcinoma, choroid plexus papilloma, ependymoma, glioblastoma, meningioma, neuroglial tumor, oligoastrocytoma, oligodendroglioma, and pineoblastoma. Exemplary ovarian cancer includes ovarian clear cell adenocarcinoma, ovarian endomethrioid adenocarcinoma, and ovarian serous adenocarcinoma. Exemplary pancreatic cancer includes pancreatic ductal adenocarcinoma and pancreatic endocrine tumor. Exemplary sarcoma includes chondrosarcoma, clear cell sarcoma of soft tissue, ewing sarcoma, gastrointestinal stromal tumor, osteosarcoma, rhabdomyosarcoma, and not otherwise specified (NOS) sarcoma. Alternatively, cancers to be treated by the compounds of the present invention are non NHL cancers.

For example, the cancer is selected from the group consisting of acute myeloid leukemia (AML) or chronic lymphocytic leukemia (CLL), medulloblastoma, oligodendroglioma, ovarian clear cell adenocarcinoma, ovarian endomethrioid adenocarcinoma, ovarian serous adenocarcinoma, pancreatic ductal adenocarcinoma, pancreatic endocrine tumor, malignant rhabdoid tumor, astrocytoma, atypical teratoid/rhabdoid tumor, choroid plexus carcinoma, choroid plexus papilloma, ependymoma, glioblastoma, meningioma, neuroglial tumor, oligoastrocytoma, oligodendroglioma, pineoblastoma, carcinosarcoma, chordoma, extragonadal germ cell tumor, extrarenal rhabdoid tumor, schwannoma, skin squamous cell carcinoma, chondrosarcoma, clear cell sarcoma of soft tissue, ewing sarcoma, gastrointestinal stromal tumor, osteosarcoma, rhabdomyosarcoma, and not otherwise specified (NOS) sarcoma. Preferably, the cancer is acute myeloid leukemia (AML), chronic lymphocytic leukemia (CLL), medulloblastoma, ovarian clear cell adenocarcinoma, ovarian endomethrioid adenocarcinoma, pancreatic ductal adenocarcinoma, malignant rhabdoid tumor, atypical teratoid/rhabdoid tumor, choroid plexus carcinoma, choroid plexus papilloma, glioblastoma, meningioma, pineoblastoma, carcinosarcoma, extrarenal rhabdoid tumor, schwannoma, skin squamous cell carcinoma, chondrosarcoma, ewing sarcoma, epithelioid sarcoma, renal medullary carcinoma, diffuse large B-cell lymphoma, follicular lymphoma and/or NOS sarcoma.

For example, the EHMT2-mediated disorder is a hematological disorder.

The compound(s) of the present disclosure inhibit the histone methyltransferase activity of EHMT2 or a mutant thereof and, accordingly, the compounds of the present disclosure are used in methods for treating conditions and diseases the course of which can be influenced by modulating the methylation status of histones or other proteins, wherein said methylation status is mediated at least in part by the activity of EHMT2. Certain compounds disclosed herein are candidates for use in treating, or preventing certain conditions, diseases, and disorders. Modulation of the methylation status of histones can in turn influence the level of expression of target genes activated by methylation, and/or target genes suppressed by methylation. The method includes administering to a subject in need of such treatment, a therapeutically effective amount of a compound of the present disclosure.

As used herein, a "subject" is interchangeable with a "subject in need thereof, both of which refer to a subject having a disorder in which EHMT2-mediated protein methylation plays a part, or a subject having an increased risk of developing such disorder relative to the population at large. A "subject" includes a mammal. The mammal can be *e.g.,* a human or appropriate non-human mammal, such as primate, mouse, rat, dog, cat, cow, horse, goat, camel, sheep or a pig. The subject can also be a bird or fowl. In one embodiment, the mammal is a human. A subject in need thereof can be one who has been previously diagnosed or identified as having cancer or a precancerous condition. A subject in need thereof can also be one who has (e.g., is suffering from) cancer or a precancerous condition. Alternatively, a subject in need thereof can be one who has an increased risk of developing such disorder relative to the population at large (*i.e.,* a subject who is predisposed to developing such disorder relative to the population at large). A subject in need thereof can have a precancerous condition. A subject in need thereof can have refractory or resistant cancer (i.e., cancer that doesn't respond or hasn't yet responded to treatment). The subject may be resistant at start of treatment or may become resistant during treatment. In some embodiments, the subject in need thereof has cancer recurrence following remission on most recent therapy. In some embodiments, the subject in need thereof received and failed all known effective therapies for cancer treatment. In some embodiments, the subject in need thereof received at least one prior therapy. In a preferred embodiment, the subject has cancer or a cancerous condition. For example, the cancer is leukemia, prostate carcinoma, hepatocellular carcinoma, and lung cancer.

As used herein, "candidate compound" refers to a compound of the present disclosure, or a pharmaceutically acceptable salt, polymorph or solvate thereof, that has been or will be tested in one or more *in vitro* or *in vivo* biological assays, in order to determine if that compound is likely to elicit a desired biological or medical response in a cell, tissue, system, animal or human that is being sought by a researcher or clinician. A candidate compound is a compound of the present disclosure, or a pharmaceutically acceptable salt, polymorph or solvate thereof. The biological or medical response can be the treatment of cancer. The biological or medical response can be treatment or prevention of a cell proliferative disorder. The biological response or effect can also include a change in cell proliferation or growth that occurs *in vitro* or in an animal model, as well as other biological changes that are observable *in vitro. In vitro* or *in vivo* biological assays can include, but are not limited to, enzymatic activity assays, electrophoretic mobility shift assays, reporter gene assays, *in vitro* cell viability assays, and the assays described herein.

For example, an *in vitro* biological assay that can be used includes the steps of (1) mixing a histone substrate (*e*.*g*., an isolated histone sample or an isolated histone peptide representative of human histone H3 residues 1-15) with recombinant EHMT2 enzymes; (2) adding a compound of the disclosure to this mixture; (3) adding non-radioactive and ³H-labeled S-Adenosyl methionine (SAM) to start the reaction; (4) adding excessive amount of non-radioactive SAM to stop the reaction; (4) washing off the free non-incorporated ³H-SAM; and (5) detecting the quantity of ³H-labeled histone substrate by any methods known in the art (*e*.*g*., by a PerkinElmer TopCount plate reader).

For example, an *in vitro* study that can be used includes the steps of (1) treating cancer cells (*e*.*g*., breast cancer cells) with a compound of this disclosure; (2) incubating the cells for a set period of time; (3) fixing the cells; (4) treating the cells with primary antibodies that bind to dimethylated histone substrates; (5) treating the cells with a secondary antibody (*e*.*g*. an antibody conjugated to an infrared dye); (6) detecting the quantity of bound antibody by any methods known in the art (*e*.*g*., by a Licor Odyssey Infrared Scanner).

As used herein, "treating" or "treat" describes the management and care of a patient for the purpose of combating a disease, condition, or disorder and includes the administration of a compound of the present disclosure, or a pharmaceutically acceptable salt, polymorph or solvate thereof, to alleviate the symptoms or complications of a disease, condition or disorder, or to eliminate the disease, condition or disorder. The term "treat" can also include treatment of a cell *in vitro* or an animal model.

A compound of the present disclosure, or a pharmaceutically acceptable salt, polymorph or solvate thereof, can or may also be used to prevent a relevant disease, condition or disorder, or used to identify suitable candidates for such purposes. As used herein, "preventing," "prevent," or "protecting against" describes reducing or eliminating the onset of the symptoms or complications of such disease, condition or disorder.

One skilled in the art may refer to general reference texts for detailed descriptions of known techniques discussed herein or equivalent techniques. These texts include Ausubel et al., Current Protocols in Molecular Biology, John Wiley and Sons, Inc. (2005); Sambrook et al., Molecular Cloning, A Laboratory Manual (3rd edition), Cold Spring Harbor Press, Cold Spring Harbor, New York (2000); Coligan et al., Current Protocols in Immunology, John Wiley & Sons, N.Y.; Enna et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, PA, 18th edition (1990). These texts can, of course, also be referred to in making or using an aspect of the disclosure.

As used herein, "combination therapy" or "co-therapy" includes the administration of a compound of the present disclosure, or a pharmaceutically acceptable salt, polymorph or solvate thereof, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents.

The present disclosure also provides pharmaceutical compositions comprising a compound of any of the Formulae described herein in combination with at least one pharmaceutically acceptable excipient or carrier.

A "pharmaceutical composition" is a formulation containing the compounds of the present disclosure in a form suitable for administration to a subject. In one embodiment, the pharmaceutical composition is in bulk or in unit dosage form. The unit dosage form is any of a variety of forms, including, for example, a capsule, an IV bag, a tablet, a single pump on an aerosol inhaler or a vial. The quantity of active ingredient (*e*.*g*., a formulation of the disclosed compound or salt, hydrate, solvate or isomer thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this disclosure include powders, sprays, ointments, pastes, creams, lotions, gels, solutions, patches and inhalants. In one embodiment, the active compound is mixed under sterile conditions with a pharmaceutically acceptable carrier, and with any preservatives, buffers, or propellants that are required.

As used herein, the phrase "pharmaceutically acceptable" refers to those compounds, anions, cations, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

A pharmaceutical composition of the disclosure is formulated to be compatible with its intended route of administration. Examples of routes of administration include parenteral, *e.g.,* intravenous, intradermal, subcutaneous, oral (*e.g.,* inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

A compound or pharmaceutical composition of the disclosure can be administered to a subject in many of the well-known methods currently used for chemotherapeutic treatment. For example, for treatment of cancers, a compound of the disclosure may be injected directly into tumors, injected into the blood stream or body cavities or taken orally or applied through the skin with patches. The dose chosen should be sufficient to constitute effective treatment but not so high as to cause unacceptable side effects. The state of the disease condition (e.g., cancer, precancer, and the like) and the health of the patient should preferably be closely monitored during and for a reasonable period after treatment.

The term "therapeutically effective amount", as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is cancer. In another aspect, the disease or condition to be treated is a cell proliferative disorder.

For any compound, the therapeutically effective amount can be estimated initially either in cell culture assays, *e*.*g*., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, *e*.*g*., ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

The pharmaceutical compositions containing active compounds of the present disclosure may be manufactured in a manner that is generally known, *e*.*g*., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol and sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds can be prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

In therapeutic applications, the dosages of the pharmaceutical compositions used in accordance with the disclosure vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the growth of the tumors and also preferably causing complete regression of the cancer. Dosages can range from about 0.01 mg/kg per day to about 5000 mg/kg per day. In preferred aspects, dosages can range from about 1 mg/kg per day to about 1000 mg/kg per day. In an aspect, the dose will be in the range of about 0.1 mg/day to about 50 g/day; about 0.1 mg/day to about 25 g/day; about 0.1 mg/day to about 10 g/day; about 0.1 mg to about 3 g/day; or about 0.1 mg to about 1 g/day, in single, divided, or continuous doses (which dose may be adjusted for the patient's weight in kg, body surface area in m², and age in years). An effective amount of a pharmaceutical agent is that which provides an objectively identifiable improvement as noted by the clinician or other qualified observer. For example, regression of a tumor in a patient may be measured with reference to the diameter of a tumor. Decrease in the diameter of a tumor indicates regression. Regression is also indicated by failure of tumors to reoccur after treatment has stopped. As used herein, the term "dosage effective manner" refers to amount of an active compound to produce the desired biological effect in a subject or cell.

The pharmaceutical compositions can be included in a container, pack, or dispenser together with instructions for administration.

The compounds of the present disclosure are capable of further forming salts. All of these forms are also contemplated within the scope of the claimed disclosure.

As used herein, "pharmaceutically acceptable salts" refer to derivatives of the compounds of the present disclosure wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like. In the salt form, it is understood that the ratio of the compound to the cation or anion of the salt can be 1:1, or any ration other than 1:1, e.g., 3:1, 2:1, 1:2, or 1:3.

It should be understood that all references to pharmaceutically acceptable salts include solvent addition forms (solvates) or crystal forms (polymorphs) as defined herein, of the same salt.

The compounds of the present disclosure can also be prepared as esters, for example, pharmaceutically acceptable esters. For example, a carboxylic acid function group in a compound can be converted to its corresponding ester, *e.g.,* a methyl, ethyl or other ester. Also, an alcohol group in a compound can be converted to its corresponding ester, *e.g.,* acetate, propionate or other ester.

The compounds, or pharmaceutically acceptable salts thereof, are administered orally, nasally, transdermally, pulmonary, inhalationally, buccally, sublingually, intraperitoneally, subcutaneously, intramuscularly, intravenously, rectally, intrapleurally, intrathecally and parenterally. For example, the compound may be administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Techniques for formulation and administration of the disclosed compounds of the disclosure can be found in Remington: the Science and Practice of Pharmacy, 19th edition, Mack Publishing Co., Easton, PA (1995). The compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

All percentages and ratios used herein, unless otherwise indicated, are by weight. Other features and advantages of the present disclosure are apparent from the different examples. The provided examples illustrate different components and methodology useful in practicing the present disclosure. The examples do not limit the claimed disclosure. Based on the present disclosure the skilled artisan can identify and employ other components and methodology useful for practicing the present disclosure.

In the synthetic schemes described herein, compounds may be drawn with one particular configuration for simplicity. Such particular configurations are not to be construed as limiting the disclosure to one or another isomer, tautomer, regioisomer or stereoisomer, nor does it exclude mixtures of isomers, tautomers, regioisomers or stereoisomers; however, it will be understood that a given isomer, tautomer, regioisomer or stereoisomer may have a higher level of activity than another isomer, tautomer, regioisomer or stereoisomer.

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### Example 1: Synthesis of Compound 1 (Reference)

### Synthesis of N²-(4-methoxy-3-(3-(pyrrolidin-1-yl)propoxy)phenyl)-N⁴-methylpyrimidine-2,4-diamine:

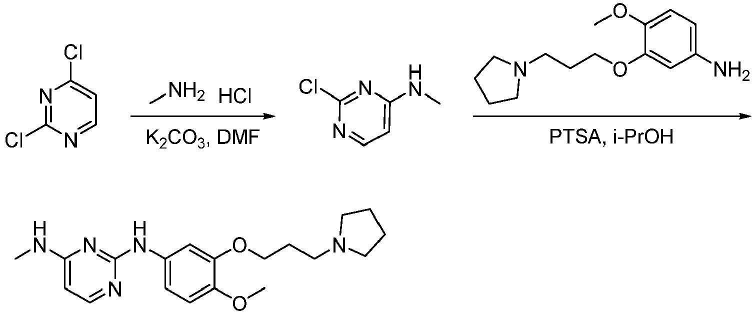

### Step 1: Synthesis of 2-chloro-N-methylpyrimidin-4-amine:

Into a 50-mL round-bottom flask, was placed 2,4-dichloropyrimidine (1.1 g, 7.38 mmol, 1.00 equiv.), methanamine hydrochloride (498 mg, 7.38 mmol, 1.00 equiv.), potassium carbonate (3.07 g, 22.21 mmol, 3.00 equiv.), N,N-dimethylformamide (10 mL). The resulting solution was stirred for 18 h at 20 °C. The resulting solution was diluted with 60 mL of H₂O. The resulting solution was extracted with 3x80 mL of ethyl acetate and the organic layers combined. The resulting mixture was washed with 3x100 mL of brine. The resulting mixture was concentrated under vacuum. The residue was applied onto a silica gel column with ethyl acetate/petroleum ether (1/2). This resulted in 0.67 g (63%) of 2-chloro-N-methylpyrimidin-4-amine as a white solid.

### Step 2: Synthesis of N²-(4-methoxy-3-(3-(pyrrolidin-1-yl)propoxy)phenyl)-N⁴-methylpyrimidine-2,4-diamine:

Into a 50-mL round-bottom flask, was placed 2-chloro-N-methylpyrimidin-4-amine (200 mg, 1.39 mmol, 1.00 equiv.), 4-methoxy-3-[3-(pyrrolidin-1-yl)propoxy]aniline (350 mg, 1.40 mmol, 1.00 equiv.), 4-methylbenzene-1-sulfonic acid (476 mg, 2.76 mmol, 2.00 equiv.), isopropanol (10 mL). The resulting solution was stirred for 3 h at 85 °C. The resulting mixture was concentrated under vacuum. The residue was applied onto a silica gel column with ACN/H₂O (1/5). This resulted in 66.3 mg (13%) of 2-N-[4-methoxy-3-[3-(pyrrolidin-1-yl)propoxy]phenyl]-4-N-methylpyrimidine-2,4-diamine as a pink solid.

### Example 2: Synthesis of Compound 2 (Reference)

### Synthesis of N⁴-((1-(2,2-difluoroethyl)piperidin-4-yl)methyl)-N²-(4-methoxy-3-(3-(pyrrolidin-1-yl)propoxy)phenyl)pyrimidine-2,4-diamine:

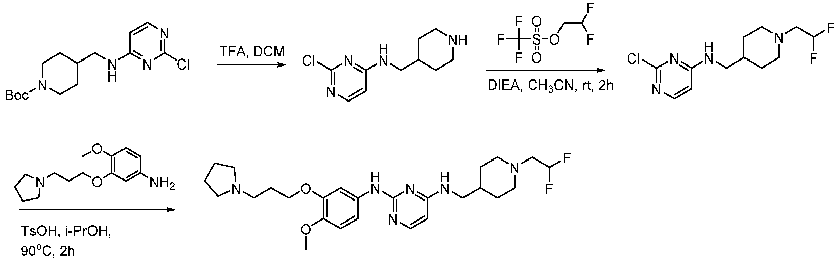

### Step 1: Synthesis of 2-chloro-N-(piperidin-4-ylmethyl)pyrimidin-4-amine:

Into a 50-mL 3-necked round-bottom flask, was placed tert-butyl 4-[[(2-chloropyrimidin-4-yl)amino]methyl]piperidine-1-carboxylate (1.1 g, 3.37 mmol, 1.00 equiv.), trifluoroacetic acid (3 mL), dichloromethane (10 mL). The resulting solution was stirred for 2 h at room temperature. The resulting mixture was concentrated under vacuum. The residue was purified by flash chromatography with H₂O/M_{C}CN/NH₄HCO₃. This resulted in 1.5 g (crude) of 2-chloro-N-(piperidin-4-ylmethyl)pyrimidin-4-amine as an off-white solid.

### Step 2: Synthesis of 2-chloro-N-((1-(2,2-difluoroethyl)piperidin-4-yl)methyl)pyrimidin-4-amine:

Into a 100-mL 3-necked round-bottom flask, was placed 2-chloro-N-(piperidin-4-ylmethyl)pyrimidin-4-amine (1.5 g, 6.62 mmol, 1.00 equiv.), 2,2-difluoroethyl trifluoromethanesulfonate (1.56 g, 7.29 mmol, 1.10 equiv.), DIEA (1.7 g, 2.00 equiv.), MeCN (20 mL). The resulting solution was stirred for 2 h at room temperature. The resulting mixture was concentrated under vacuum. The residue was purified by flash chromatography. This resulted in 1.1 g (57%) of 2-chloro-N-[[1-(2,2-difluoroethyl)piperidin-4-yl]methyl]pyrimidin-4-amine as a yellow solid.

### Step 3: Synthesis of N⁴-((1-(2,2-difluoroethyl)piperidin-4-yl)methyl)-N²-(4-methoxy-3-(3-(pyrrolidin-1-yl)propoxy)phenyl)pyrimidine-2,4-diamine:

Into a 50-mL round-bottom flask, was placed 2-chloro-N-[[1-(2,2-difluoroethyl)piperidin-4-yl]methyl]pyrimidin-4-amine (291 mg, 1.00 mmol, 1.00 equiv.), 4-methoxy-3-[3-(pyrrolidin-1-yl)propoxy] aniline (250 g, 998.66 mmol, 1.00 equiv.), TsOH·H₂O (380 mg, 2.00 mmol, 2.00 equiv.), isopropanol (5 mL). The resulting solution was stirred for 5 h at 90°C in an oil bath. The resulting mixture was concentrated under vacuum. The crude product was purified by Prep-HPLC. This resulted in 144.1 mg (29%) of 4-N-[[1-(2,2-difluoroethyl)piperidin-4-yl] methyl] -2-N- [4-methoxy-3- [3-(pyrrolidin-1-yl)propoxy]phenyl]pyrimidine-2,4-diamine as a white solid.

### Example 3: Synthesis of Compound 3 (Reference)

### Synthesis N²-(4-methoxy-3-(3-(pyrrolidin-1-yl)propoxy)phenyl)-N⁴-((tetrahydro-2H-pyran-4-yl)methyl)pyrimidine-2,4-diamine:

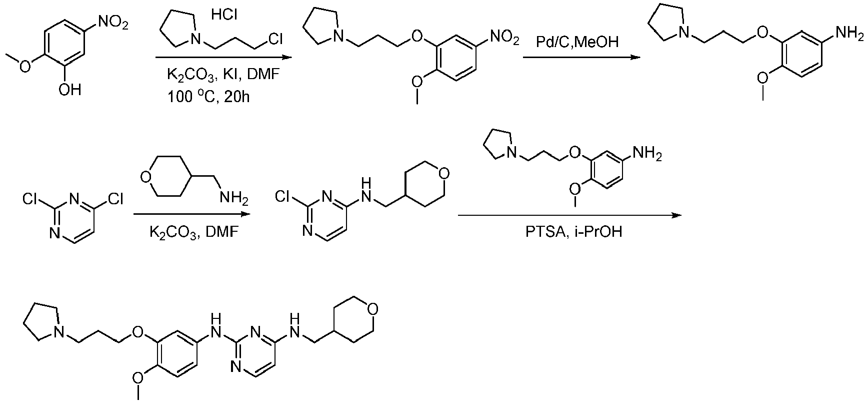

### Step 1: Synthesis of 1-(3-(2-methoxy-5-nitrophenoxy)propyl)pyrrolidine:

Into a 250-mL round-bottom flask, was placed 2-methoxy-5-nitrophenol (10 g, 59.12 mmol, 1.00 equiv.), 1-(3-chloropropyl)pyrrolidine hydrochloride (10.8 g, 58.66 mmol, 1.00 equiv.), Cs₂CO₃ (58 g, 178.01 mmol, 3.00 equiv.), NaI (8.9 g, 1.00 equiv.), N,N-dimethylformamide (100 mL). The resulting solution was stirred for 2 h at 110 °C. The resulting solution was diluted with 300 mL of H₂O. The resulting solution was extracted with 3x400 mL of ethyl acetate and the organic layers combined. The resulting mixture was washed with 5x400 mL of brine. The resulting mixture was concentrated under vacuum. This resulted in 14 g (84%) of 1-[3-(2-methoxy-5-nitrophenoxy)propyl] pyrrolidine as a yellow solid.

### Step 2: Synthesis of 4-methoxy-3-(3-(pyrrolidin-1-yl)propoxy)aniline:

Into a 250-mL round-bottom flask, was placed 1-[3-(2-methoxy-5-nitrophenoxy)propyl]pyrrolidine (14 g, 49.94 mmol, 1.00 equiv.), methanol (100 mL), Palladium carbon (2 g). The mixture underwent three hydrogen/air exchange cycles. The resulting solution was stirred for 15 h at 20 °C. The solids were filtered out. The resulting mixture was concentrated under vacuum. This resulted in 12.1 g (97%) of 4-methoxy-3-[3-(pyrrolidin-1-yl)propoxy]aniline as brown oil.

### Step 3: Synthesis of 2-chloro-N-((tetrahydro-2H-pyran-4-yl)methyl)pyrimidin-4-amine:

Into a 25-mL round-bottom flask, was placed 2,4-dichloropyrimidine (500 mg, 3.36 mmol, 1.00 equiv.), oxan-4-ylmethanamine (389 mg, 3.38 mmol, 1.00 equiv.), potassium carbonate (932 mg, 6.74 mmol, 2.00 equiv.), N,N-dimethylformamide (3 mL). The resulting solution was stirred for 1 h at 20 °C. The solids were filtered out. The residue was applied onto a silica gel column with ACN/H₂O (1/5). This resulted in 0.44 g (58%) of 2-chloro-N-(oxan-4-ylmethyl)pyrimidin-4-amine as a white solid.

### Step 4: Synthesis of N²-(4-methoxy-3-(3-(pyrrolidin-1-yl)propoxy)phenyl)-N4-((tetrahydro-2H-pyran-4-yl)methyl)pyrimidine-2,4-diamine:

Into a 50-mL round-bottom flask, was placed 2-chloro-N-(oxan-4-ylmethyl)pyrimidin-4-amine (150 mg, 0.66 mmol, 1.00 equiv.), 1-methanesulfonyl-4-methylbenzene (181 mg, 1.06 mmol, 1.10 equiv.), 4-methoxy-3-[3-(pyrrolidin-1-yl)propoxy]aniline (566 mg, 2.26 mmol, 5.00 equiv.), isopropanol (5 mL). The resulting solution was stirred for 12 h at 85 °C in an oil bath. The resulting solution was extracted with 3x30 mL of dichloromethane and the organic layers combined and dried over anhydrous sodium sulfate and concentrated under vacuum. The crude product was purified by Flash-Prep-HPLC with the following conditions (IntelFlash-1): Column, C18 silica gel; mobile phase, CH3CN/H₂O(0.05%NH₃.H₂O)=17% increasing to CH₃CN/H₂O(0.05%NH₃.H₂O)=30% within 10 min; Detector, UV 254 nm. This resulted in 116 mg (40%) of 2-N-[4-methoxy-3-[3-(pyrrolidin-1-yl)propoxy]phenyl]-4-N-(oxan-4-ylmethyl)pyrimidine-2,4-diamine as a white solid.

### Example 4: Synthesis of Compound 4 (Reference)

### Synthesis of N²-(4-methoxy-3-(3-(pyrrolidin-1-yl)propoxy)phenyl)-N⁴-((1-(2,2,2-trifluoroethyl)piperidin-4-yl)methyl)pyrimidine-2,4-diamine:

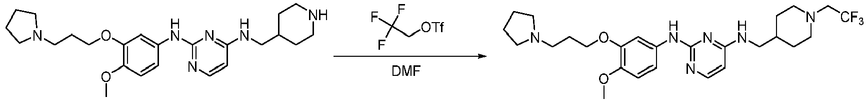

Into a 8-mL vial, was placed 2-N-[4-methoxy-3-[3-(pyrrolidin-1-yl)propoxy]phenyl]-4-N-(piperidin-4-ylmethyl)pyrimidine-2,4-diamine (250 mg, 0.57 mmol, 1.00 equiv.), 2,2,2-trifluoroethyl trifluoromethanesulfonate (197 mg, 0.85 mmol, 1.50 equiv.), N,N-dimethylformamide (3 mL). The resulting solution was stirred for overnight at room temperature. After concentration, the residue was purified by flash chromatography with H₂O/MeCN/NH₄HCO₃. This resulted in 77.6 mg (26%) of 2-N-[4-methoxy-3-[3-(pyrrolidin-1-yl)propoxy]phenyl]-4-N-[[1-(2,2,2-trifluoroethyl)piperidin-4--yl]methyl]pyrimidine-2,4-diamine as a white solid.

### Example 5: Synthesis of Compound 5 (Reference)

### Synthesis of N²-(4-methoxy-3-(3-(pyrrolidin-1-yl)propoxy)phenyl)-N⁴-(tetrahydro-2H-pyran-4-yl)pyrimidine-2,4-diamine:

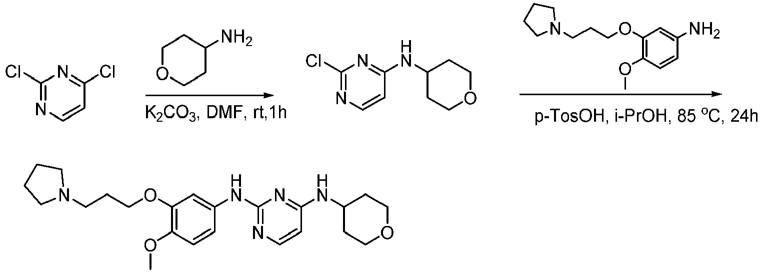

### Step 1: Synthesis of 2-chloro-N-(tetrahydro-2H-pyran-4-yl)pyrimidin-4-amine:

Into a 50-mL round-bottom flask, was placed 2,4-dichloropyrimidine (500 mg, 3.36 mmol, 1.00 equiv.), oxan-4-amine (341.2 mg, 3.37 mmol, 1.00 equiv.), potassium carbonate (932.4 mg, 6.75 mmol, 2.00 equiv.), N,N-dimethylformamide (3 mL). The resulting solution was stirred for 1 h at 20 °C. The solids were filtered out. The residue was applied onto a silica gel column with dichloromethane/methanol (4:1). This resulted in 250 mg (35%) of 2-chloro-N-(oxan-4-yl)pyrimidin-4-amine as a white solid.

### Step 2: Synthesis of N²-(4-methoxy-3-(3-(pyrrolidin-1-yl)propoxy)phenyl)-N4-(tetrahydro-2H-pyran-4-yl)pyrimidine-2,4-diamine:

Into a 50-mL round-bottom flask, was placed 2-chloro-N-(oxan-4-yl)pyrimidin-4-amine (150 mg, 0.70 mmol, 1.00 equiv.), 4-methoxy-3-[3-(pyrrolidin-1-yl)propoxy]aniline (192 mg, 0.77 mmol, 1.10 equiv.), 4-methylbenzene-1-sulfonic acid (603 mg, 3.50 mmol, 5.00 equiv.), i-prOH (5 mL). The resulting solution was stirred for 12 h at 85 °C in an oil bath. The resulting solution was extracted with 3x30 mL of dichloromethane and the organic layers combined and dried over anhydrous sodium sulfate and concentrated under vacuum. The crude product was purified by Prep-HPLC with the following conditions : Column: X Bridge RP, 19*150 mm, 5 um; Mobile Phase A:Water/10mmol NH4HCO3 , Mobile Phase B: ACN; Flow rate: 30 mL/min; Gradient: 16%B to 45%B in 10 min; 254nm.This resulted in 51.8 mg (17%) of 2-N-[4-methoxy-3-[3-(pyrrolidin-1-yl)propoxy]phenyl]-4-N-(oxan-4-yl)pyrimidine-2,4-diamine as a white solid.

### Example 6: Synthesis of Compound 6 (Reference)

### Synthesis of N⁴-(tert-butyl)-N²-(4-methoxy-3-(3-(pyrrolidin-1-yl)propoxy)phenyl)pyrimidine-2,4-diamine:

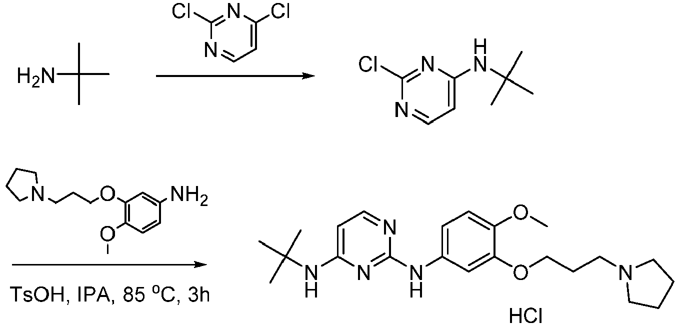

### Step 1: Synthesis of N-(tert-butyl)-2-chloropyrimidin-4-amine:

Into a 25-mL round-bottom flask, was placed 2,4-dichloropyrimidine (1 g, 6.71 mmol, 1.00 equiv.), 2-methylpropan-2-amine (496 mg, 6.78 mmol, 1.01 equiv.), potassium carbonate (2.8 g, 20.26 mmol, 3.02 equiv.), N,N-dimethylformamide (5 mL). The resulting solution was stirred for 3 h at room temperature. The solids were filtered out. The residue was applied onto a silica gel column with ACN:water (45:100). This resulted in 600 mg (48%) of N-tert-butyl-2-chloropyrimidin-4-amine as a white solid.

### Step 2: Synthesis of N⁴-(tert-butyl)-N²-(4-methoxy-3-(3-(pyrrolidin-1-yl)propoxy)phenyl)pyrimidine-2,4-diamine:

Into a 25-mL round-bottom flask, was placed N-tert-butyl-2-chloropyrimidin-4-amine (200 mg, 1.08 mmol, 1.00 equiv.), 4-methoxy-3-[3-(pyrrolidin-1-yl)propoxy]aniline (325 mg, 1.30 mmol, 1.21 equiv.), TsOH (185.7 mg, 1.08 mol, 1 equiv.), isopropanol (2 mL). The resulting solution was stirred for 3 h at 85 °C in an oil bath. The crude product was purified by Prep-HPLC with the following conditions (2#-AnalyseHPLC-SHIMADZU (HPLC-10)): Column, XSelect CSH Prep C18 OBD Column, 5um, 19*150mm; mobile phase, Waters (0.05%HCl) and ACN (5.0% ACN up to 15.0% in 6 min); Detector, UV 220nm. This resulted in 82.6 mg (18%) of 4-N-tert-butyl-2-N-[4-methoxy-3-[3-(pyrrolidin-1-yl)propoxy]phenyl]pyrimidine-2,4-diamine hydrochloride as a brown solid.

### Example 7: Synthesis of Compound 7 (Reference)

### Synthesis of tert-butyl 4-(((4-((4-methoxy-3-(3-(pyrrolidin-1-yl)propoxy)phenyl)amino)pyrimidin-2-yl)amino)methyl)piperidine-1-carboxylate:

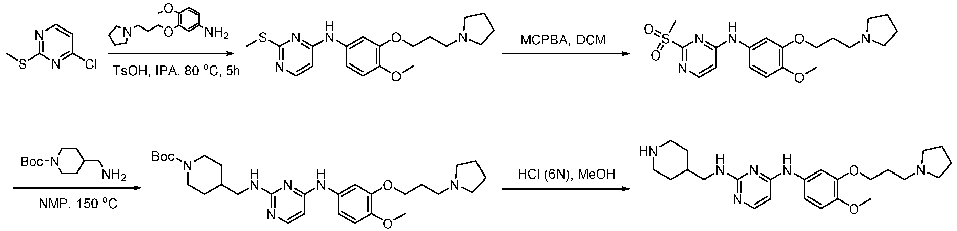

### Step 1: Synthesis of N-(4-methoxy-3-(3-(pyrrolidin-1-yl)propoxy)phenyl)-2-(methylthio)pyrimidin-4-amine:

Into a 50-mL 3-necked round-bottom flask, was placed 4-methoxy-3-[3-(pyrrolidin-1-yl)propoxy]aniline (1 g, 3.99 mmol, 1.00 equiv.), 4-chloro-2-(methylsulfanyl)pyrimidine (640 mg, 3.98 mmol, 1.00 equiv.), TsOH·H₂O (1.52 g, 2.00 equiv.), isopropanol (10 mL). The resulting solution was stirred for 3 h at 70 °C in an oil bath. The resulting mixture was concentrated under vacuum. The residue was purified by flash chromatography with H₂O/MeCN. This resulted in 950 mg (64%) of N-[4-methoxy-3-[3-(pyrrolidin-1-yl)propoxy]phenyl]-2-(methylsulfanyl)pyrimidin-4-amine as a black solid.

### Step 2: Synthesis of N-(4-methoxy-3-(3-(pyrrolidin-1-yl)propoxy)phenyl)-2-(methylsulfonyl)pyrimidin-4-amine:

Into a 50-mL 3-necked round-bottom flask, was placed N-[4-methoxy-3-[3-(pyrrolidin-1-yl)propoxy]phenyl]-2-(methylsulfanyl)pyrimidin-4-amine (800 mg, 2.14 mmol, 1.00 equiv.), mCPBA (736 mg, 4.27 mmol, 2.00 equiv.), dichloromethane (10 mL). The resulting solution was stirred for 2 h at room temperature. The resulting mixture was concentrated under vacuum. The residue was purified by flash chromatography with MeCN/H₂O. This resulted in 500 mg (58%) of 2-methanesulfonyl-N-[4-methoxy-3-[3-(pyrrolidin-1-yl)propoxy]phenyl]pyrimidin-4-amine as a dark red solid.

### Step 3: Synthesis of tert-butyl 4-(((4-((4-methoxy-3-(3-(pyrrolidin-1-yl)propoxy)phenyl)amino)pyrimidin-2-yl)amino)methyl)piperidine-1-carboxylate:

Into a 10-mL sealed tube, was placed 2-methanesulfonyl-N-[4-methoxy-3-[3-(pyrrolidin-1-yl)propoxy]phenyl]pyrimidin-4-amine (200 mg, 0.49 mmol, 1.00 equiv.), tert-butyl 4-(aminomethyl)piperidine-1-carboxylate (115.7 mg, 0.54 mmol, 1.10 equiv.), NMP (2 mL). The resulting solution was stirred for overnight at 150°C in an oil bath. The residue was purified by flash chromatography with H₂O/MeCN. This resulted in 130 mg (49%) of tert-butyl 4-([[4-([4-methoxy-3-[3-(pyrrolidin-1-yl)propoxy]phenyl]amino)pyrimidin-2-yl]amino]methyl)piperidine-1-carboxylate as yellow oil.

### Step 4: Synthesis of tert-butyl 4-(((4-((4-methoxy-3-(3-(pyrrolidin-1-yl)propoxy)phenyl)amino)pyrimidin-2-yl)amino)methyl)piperidine-1-carboxylate:

Into a 10-mL sealed tube, was placed tert-butyl 4-([[4-([4-methoxy-3-[3-(pyrrolidin-1-yl)propoxy]phenyl] amino)pyrimidin-2-yl] amino methyl)piperidine-1-carboxylate (130 mg, 0.24 mmol, 1.00 equiv.), hydrogen chloride (6N, 0.5 mL), methanol (0.5 mL). The resulting solution was stirred for 30 min at room temperature. The resulting mixture was concentrated under vacuum. The residue was purified by Prep-HPLC with the following conditions (2#-AnalyseHPLC-SHIMADZU (HPLC-10)): Column, XSelect CSH Prep C18 OBD Column, 5um, 19^{∗}150mm; mobile phase, Waters (0.05%HCl) and ACN (3.0% ACN up to 15.0% in 6 min); Detector, UV 220nm. This resulted in 9.9 mg (9%) of 4-N-[4-methoxy-3-[3-(pyrrolidin-1-yl)propoxy]phenyl]-2-N-(piperidin-4-ylmethyl)pyrimidine-2,4-diamine hydrochloride as an off-white solid.

### Example 8: Synthesis of Compound 8 (Reference)

### Synthesis of 1-(4-(((2-((4-chloro-3-(3-(pyrrolidin-1-yl)propoxy)phenyl)amino)pyrimidin-4-yl)amino)methyl)piperidin-1-yl)ethan-1-one:

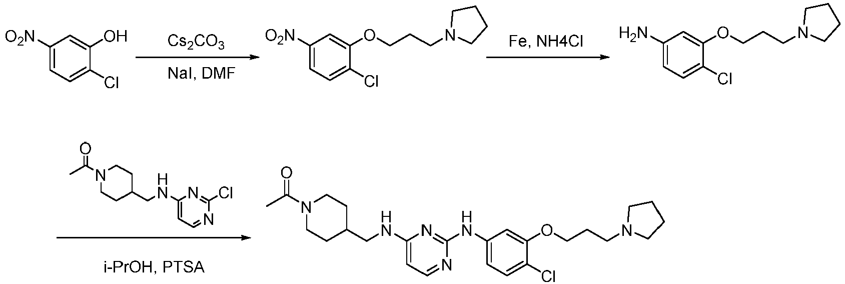

### Step 1: Synthesis of 1-(3-(2-chloro-5-nitrophenoxy)propyl)pyrrolidine:

Into a 50-mL round-bottom flask, was placed 2-chloro-5-nitrophenol (1 g, 5.76 mmol, 1.00 equiv.), N,N-dimethylformamide (10 mL), Cs₂CO₃ (5.6 g, 17.13 mmol, 3.00 equiv.), NaI (867 mg, 5.78 mmol, 1.00 equiv.), 1-(3-chloropropyl) pyrrolidine (1.1 g, 7.45 mmol, 1.00 equiv.). The resulting solution was stirred for 2 h at 110 °C in an oil bath. The reaction was quenched by the addition of water/ice. The resulting solution was extracted with 3x10 mL of ethyl acetate and the organic layers combined. The resulting mixture was washed with 2x10 mL of Brine, drying with Na₂SO₄.The resulting mixture was concentrated under vacuum. This resulted in 1.3 g (79%) of 1-[3-(2-chloro-5-nitrophenoxy)propyl] pyrrolidine as a yellow solid.

### Step 2: Synthesis of 4-chloro-3-(3-(pyrrolidin-1-yl)propoxy)aniline:

Into a 100-mL round-bottom flask, was placed 1-[3-(2-chloro-5-nitrophenoxy)propyl] pyrrolidine (900 mg, 3.16 mmol, 1.00 equiv.), methanol (20 mL), Fe (530.5 mg, 9.47 mmol, 3.00 equiv.), NH₄Cl (502 mg, 9.38 mmol, 3.00 equiv.), water(1 mL). The resulting solution was stirred for 12 h at 100 °C in an oil bath. The solids were filtered out. The resulting mixture was concentrated under vacuum. The residue was applied onto a silica gel column with CH₃CN/H₂O (1:19). The collected fractions were combined and concentrated under vacuum. This resulted in 420 mg (52%) of 4-chloro-3-[3-(pyrrolidin-1-yl)propoxy] aniline as yellow oil.

### Step 3: Synthesis of 1-(4-(((2-((4-chloro-3-(3-(pyrrolidin-1-yl)propoxy)phenyl)amino)pyrimidin-4-yl)amino)methyl)piperidin-1-yl)ethan-1-one:

Into a 10-mL sealed tube, was placed 4-chloro-3-[3-(pyrrolidin-1-yl)propoxy]aniline (400 mg, 1.57 mmol,1.00 equiv.), PTSA (541.7 mg, 3.15 mmol, 2.00 equiv.), 1-(4-[(2-chloropyrimidin-4-yl)amino]methylpiperidin-1-yl)ethan-1-one (422 mg, 1.57 mmol, 1.00 equiv.), isopropanol (5 mL). The resulting solution was stirred for 10 h at 85°C in an oil bath. The resulting mixture was concentrated under vacuum. The crude product (150 mg) was purified by Flash-Prep-HPLC with the following conditions (IntelFlash-1): Column, C18 silica gel; mobile phase, CH₃CN/H₂O(0.05%NH₃.H₂O)=15% increasing to CH₃CN/H₂O(0.05%NH₃.H₂O)=85% within 7 min; Detector, UV 254 nm. 78.3 mg product was obtained. This resulted in 78.3 mg (10%) of 1-[4-([[2-([4-chloro-3-[3-(pyrrolidin-1-yl)propoxy]phenyl]amino)pyrimidin-4-yl]amino]methyl)piperidin-1-yl]ethan-1-one as a white solid.

### Example 9: Synthesis of Compound 9 (Reference)

### Synthesis of 2-((4-methoxy-3-(3-(pyrrolidin-1-yl)propoxy)phenyl)amino)-N-methylpyrimidine-5-carboxamide:

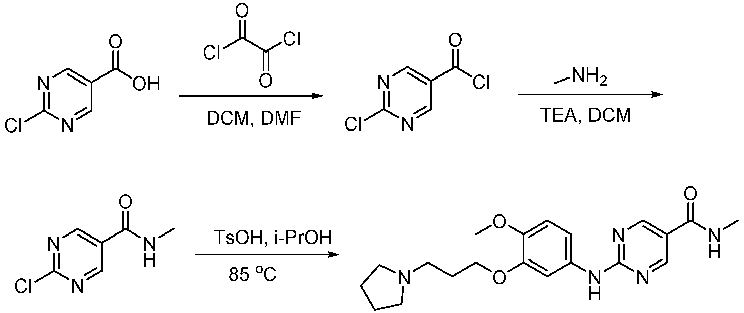

### Step 1: Synthesis of 2-chloropyrimidine-5-carbonyl chloride:

Into a 100-mL round-bottom flask, was placed 2-chloropyrimidine-5-carboxylic acid (1 g, 6.31 mmol, 1.00 equiv.), dichloromethane (20 mL), N,N-dimethylformamide (0.2 mL). This was followed by the addition of oxalic dichloride (1.2 g, 9.45 mmol, 1.50 equiv.) dropwise with stirring at 0 °C. The resulting solution was stirred for 2 h at 20 °C. The resulting mixture was concentrated under vacuum. This resulted in 1.11 g (99%) of 2-chloropyrimidine-5-carbonyl chloride as a yellow solid.

### Step 2: Synthesis of 2-chloro-N-methylpyrimidine-5-carboxamide:

Into a 100-mL round-bottom flask, was placed methanamine hydrochloride (509 mg, 7.54 mmol, 1.20 equiv.), dichloromethane (20 mL), TEA (1.92 g, 18.97 mmol, 3.00 equiv.). This was followed by the addition of 2-chloropyrimidine-5-carbonyl chloride (1.11 g, 6.27 mmol, 1.00 equiv.) dropwise with stirring at 0 °C. The resulting solution was stirred for 2 h at 20 °C. The residue was applied onto a silica gel column with ethyl acetate/petroleum ether (1:3). This resulted in 760 mg (71%) of 2-chloro-N-methylpyrimidine-5-carboxamide as a light yellow solid.

### Step 3: Synthesis of 2-((4-methoxy-3-(3-(pyrrolidin-1-yl)propoxy)phenyl)amino)-N-methylpyrimidine-5-carboxamide:

Into a 50-mL round-bottom flask, was placed 2-chloro-N-methylpyrimidine-5-carboxamide (750 mg, 4.37 mmol, 1.00 equiv.), 4-methoxy-3-[3-(pyrrolidin-1-yl)propoxy]aniline (1.09 g, 4.35 mmol, 1.00 equiv.), TsOH (2.24 g, 13.18 mmol, 3.00 equiv.), isopropanol (10 mL). The resulting solution was stirred for 3 h at 85 °C in an oil bath. The crude product was purified by Prep-HPLC with the following conditions (2#-AnalyseHPLC-SHIMADZU(HPLC-10)): Column, XBridge Prep C18 OBD Column, 19x150mm 5um; mobile phase, Waters(0.05%NH₃H₂O) and ACN (10.0% ACN up to 35.0% in 9 min); Detector, UV 220254nm. This resulted in 24.4 mg (1%) of 2-([4-methoxy-3-[3-(pyrrolidin-1-yl)propoxy]phenyl]amino)-N-methylpyrimidine-5-carboxamide as an off-white solid.

### Example 10: Synthesis of Compound 10 (Reference)

### Synthesis of N²-(4-methoxy-3-(3-(pyrrolidin-1-yl)propoxy)phenyl)-N⁴-(piperidin-4-ylmethyl)pyridine-2,4-diamine:

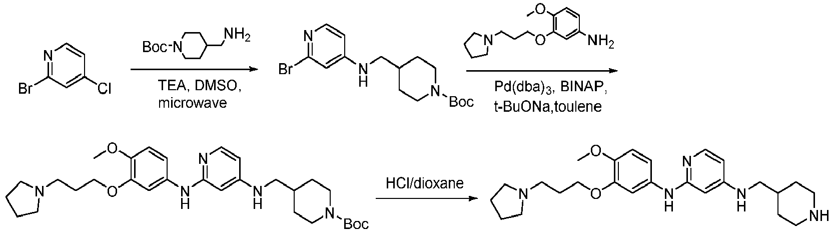

### Step 1: Synthesis of tert-butyl 4-(((2-bromopyridin-4-yl)amino)methyl)piperidine-1-carboxylate:

Into a 20-mL sealed tube, was placed 2-bromo-4-chloropyridine (1 g, 5.20 mmol, 1.00 equiv.), tert-butyl 4-(aminomethyl)cyclohexane-1-carboxylate (1.4 g, 6.56 mmol, 1.20 equiv.), TEA (1.1 g, 10.87 mmol, 2.00 equiv.), DMSO (10 mL). The final reaction mixture was irradiated with microwave radiation for 1 h at 120 °C. The resulting solution was extracted with 3x50 mL of ethyl acetate and the organic layers combined and concentrated under vacuum. This resulted in 510 mg (27%) of tert-butyl 4-[[(2-bromopyridin-4-yl)amino]methyl]cyclohexane-1-carboxylate as a light yellow solid.

### Step 2: Synthesis of tert-butyl 4-(((2-((4-methoxy-3-(3-(pyrrolidin-1-yl)propoxy)phenyl)amino)pyridin-4-yl)amino)methyl)piperidine-1-carboxylate:

Into a 50-mL 3-necked round-bottom flask, was placed tert-butyl 4-[[(2-bromopyridin-4-yl)amino]methyl]piperidine-1-carboxylate (510 mg, 1.38 mmol, 1.00 equiv.), 4-methoxy-3-[3-(pyrrolidin-1-yl)propoxy] aniline (346 mg, 1.38 mmol, 1.00 equiv.), Pd₂(dba)₃CHCl₃ (127 mg, 0.14 mmol, 0.10 equiv.), BINAP (172 mg, 0.28 mmol, 0.20 equiv.), t-BuONa (265 mg, 2.76 mmol, 2.00 equiv.), toluene (10 mL). The resulting solution was stirred for 3 h at 85 °C in an oil bath. The resulting solution was extracted with 3x50 mL of dichloromethane and the organic layers combined and concentrated under vacuum. The residue was purified by flash chromatography with H₂O/MeCN. This resulted in 410 mg (55%) of tert-butyl 4-([[2-([4-methoxy-3-[3-(pyrrolidin-1-yl)propoxy]phenyl]amino)pyridin-4-yl]amino]methyl)piperidine-1-carboxylate as a light yellow solid.

### Step 3: Synthesis of N²-(4-methoxy-3-(3-(pyrrolidin-1-yl)propoxy)phenyl)-N⁴-(piperidin-4-ylmethyl)pyridine-2,4-diamine:

Into a 50-mL round-bottom flask, was placed tert-butyl 4-([[2-([4-methoxy-3-[3-(pyrrolidin-1-yl)propoxy]phenyl] amino)pyridin-4-yl]amino]methyl)piperidine-1-carboxylate (390 mg, 0.72 mmol, 1.00 equiv.), dioxane (4 mL), hydrogen chloride (2 mL). The resulting solution was stirred for 2 h at room temperature. The resulting mixture was concentrated under vacuum. The pH value of the solution was adjusted to 9 with potassium carbonate (1 mol/L). The crude product was purified by Flash-Prep-HPLC with the following conditions (IntelFlash-1): Column: X Bridge RP, 19*150 mm, 5 um; Mobile Phase A:Water/10mmol NH4HCO3 , Mobile Phase B: ACN; Flow rate: 30 mL/min; Gradient: 10%B to 40%B in 10 min; 254nm.This resulted in 28.6 mg (9%) of 2-N-[4-methoxy-3-[3-(pyrrolidin-1-yl)propoxy]phenyl]-4-N-(piperidin-4-ylmethyl)pyridine-2,4-diamine as a brown solid.

### Example 11: Synthesis of Compound 11 (Reference)

### Synthesis of N⁴-(4-methoxy-3-(3-(pyrrolidin-1-yl)propoxy)phenyl)-N²-(piperidin-4-ylmethyl)pyridine-2,4-diamine:

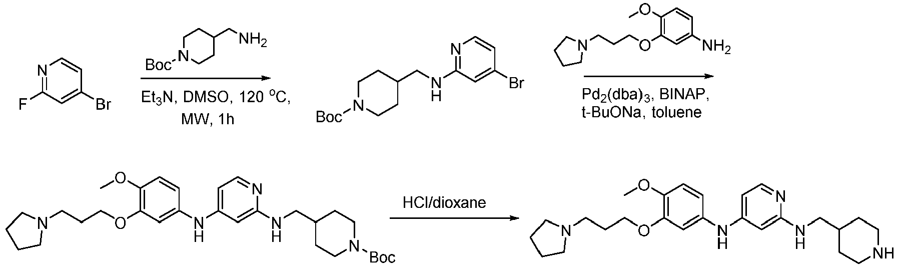

### Step 1: Synthesis of tert-butyl 4-(((4-bromopyridin-2-yl)amino)methyl)piperidine-1-carboxylate:

Into a 25-mL sealed tube, was placed tert-butyl 4-(aminomethyl)piperidine-1-carboxylate (500 mg, 2.33 mmol, 1.00 equiv.), 4-bromo-2-fluoropyridine (727 mg, 4.13 mmol, 1.00 equiv.), DMSO (8 mL), triethylamine (687 mg, 6.79 mmol, 2.0 equiv.). The final reaction mixture was irradiated with microwave radiation for 1 h at 120 °C. The reaction mixture was cooled to 20 degree C. The resulting solution was diluted with 30 mL of H₂O. The resulting solution was extracted with 3x30 mL of ethyl acetate and the organic layers combined and dried over anhydrous sodium sulfate. The solids were filtered out. The resulting mixture was concentrated under vacuum. The residue was applied onto a silica gel column with H₂O/MeCN (1-0). This resulted in 400 mg (47%) of 2-chloro-N-[4-methoxy-3-[3-(pyrrolidin-1-yl)propoxy]phenyl]pyridin-4-amine as a solid.

### Step 2: Synthesis of tert-butyl 4-(((4-((4-methoxy-3-(3-(pyrrolidin-1-yl)propoxy)phenyl)amino)pyridin-2-yl)amino)methyl)piperidine-1-carboxylate:

Into a 50-mL round-bottom flask, was placed 4-methoxy-3-[3-(pyrrolidin-1-yl)propoxy]aniline (270 mg, 1.08 mmol, 1.00 equiv.), tert-butyl 4-[[(4-bromopyridin-2-yl)amino]methyl]piperidine-1-carboxylate (400 mg, 1.08 mmol, 1.00 equiv.), BINAP (135 mg, 0.22 mmol, 0.20 equiv.), t-BuONa (201 mg, 2.09 mmol, 2.00 equiv.), Pd₂(dba)₃CHCl₃ (112 mg, 0.11 mmol, 0.10 equiv.), toluene. The resulting solution was stirred for 2.5 h at 85 °C in an oil bath. The reaction mixture was cooled to 20 degree C. The resulting solution was diluted with 20 mL of H₂O. The resulting solution was extracted with 3x15 mL of dichloromethane and the organic layers combined and dried over anhydrous sodium sulfate and concentrated under vacuum. The residue was applied onto a silica gel column with H₂O/MeCN (3/1). This resulted in 320 mg (55%) of tert-butyl 4-([[4-([4-methoxy-3-[3-(pyrrolidin-1-yl)propoxy]phenyl]amino)pyridin-2-yl]amino]methyl)piperidine-1-carboxylate as a solid.

### Step 3: Synthesis of N⁴-(4-methoxy-3-(3-(pyrrolidin-1-yl)propoxy)phenyl)-N²-(piperidin-4-ylmethyl)pyridine-2,4-diamine:

Into a 100-mL round-bottom flask, was placed tert-butyl 4-([[4-([4-methoxy-3-[3-(pyrrolidin-1-yl)propoxy]phenyl]amino)pyridin-2-yl]amino]methyl)piperidine-1-carboxylate (300 mg, 0.56 mmol, 1.00 equiv.), dioxane (15 mL), hydrogen chloride/dioxane (15 mL). The resulting solution was stirred for 16 h at 20 °C. The resulting mixture was concentrated under vacuum. The pH value of the solution was adjusted to 8-9 with potassium carbonate. The residue was applied onto a silica gel column with H₂O/MeCN (1-0). The crude product (200 mg) was purified by Prep-HPLC with the following conditions (2#-AnalyseHPLC-SHIMADZU(HPLC-10)): Column, XBridge Shield RP18 OBD Column,, 5um,19*150mm; mobile phase, Waters(0.05%NH₃H₂O) and ACN (3.0% ACN up to 70.0% in 6 min); Detector, UV 254/220nm. 23.5 . This resulted in 23.5 mg (10%) of 4-N-[4-methoxy-3-[3-(pyrrolidin-1-yl)propoxy]phenyl]-2-N-(piperidin-4-ylmethyl)pyridine-2,4-diamine as a light yellow solid.

### Example 12: Synthesis of Compound 12 (Reference)

### Synthesis of 5-methoxy-4-(3-(pyrrolidin-1-yl)propoxy)-N-(2-(((tetrahydro-2H-pyran-4-yl)amino)methyl)pyridin-4-yl)pyridin-2-amine:

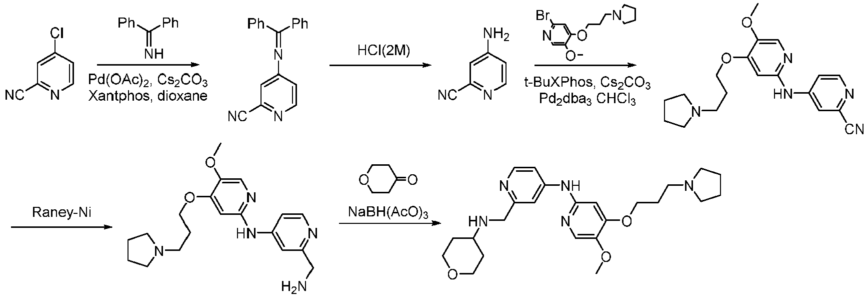

### Step 1: Synthesis of 4-((diphenylmethylene)amino)picolinonitrile:

Into a 100-mL round-bottom flask, was placed 4-chloropyridine-2-carbonitrile (1 g, 7.22 mmol, 1.00 equiv.), diphenylmethanimine (1.9 g, 10.48 mmol, 1.50 equiv.), Cs₂CO₃ (7 g, 21.48 mmol, 3.00 equiv.), Xantphos (0.832 g, 0.20 equiv.), Pd(OAc)₂ (346 mg, 1.54 mmol, 0.10 equiv.), dioxane (30 mL). The resulting solution was stirred for 2 h at 80 °C in an oil bath. The reaction was then quenched by the addition of 100 mL of water. The resulting solution was extracted with 3x50 mL of ethyl acetate and the organic layers combined. The resulting mixture was washed with 1x100 mL of brine. The mixture was dried over anhydrous sodium sulfate. The solids were filtered out. The resulting mixture was concentrated under vacuum. The crude product (1.9 g) was purified by Flash-Prep-HPLC with the following conditions (IntelFlash-1): Column, C18 silica gel; mobile phase, MeCN:Water=0:5 increasing to MeCN:Water=3:1 within 120 min; Detector, UV 254 nm. This resulted in 1.3 g (33% yield) of 4-(diphenylamino)pyridine-2-carbonitrile as a yellow solid.

### Step 2: Synthesis of 4-aminopicolinonitrile:

Into a 50-mL round-bottom flask, was placed 4-[(diphenylmethylidene)amino] pyridine-2-carbonitrile (1.23 g, 2.17 mmol, 1.00 equiv., 50%), hydrogen chloride(2M) (8 mL, 2.00 equiv.), methanol (24 mL). The resulting solution was stirred for 120 min at room temperature. The resulting mixture was concentrated under vacuum. The resulting solution was extracted with 2x10 mL of dichloromethane and the water phase combined. The pH value of the water phase was adjusted to 8 with sodium bicarbonate (sat. solution). The resulting solution was extracted with 3x10 mL of ethyl acetate and the organic layers combined. The resulting mixture was washed with 1x20 mL of brine, dried over Na₂SO₄, the solids were filtered out. The residue was evaporated and the result solid was dried in an oven under reduced pressure. This resulted in 0.28 g (81%) of 4-aminopyridine-2-carbonitrile as a yellow solid.

### Step 3: Synthesis of 4-((5-methoxy-4-(3-(pyrrolidin-1-yl)propoxy)pyridin-2-yl)amino)picolinonitrile:

Into a 30-mL sealed tube, was placed 2-bromo-5-methoxy-4-[3-(pyrrolidin-1-yl)propoxy]pyridine (314 mg, 1.00 mmol, 1.00 equiv.), 4-aminopyridine-2-carbonitrile (240 mg, 2.01 mmol, 2.00 equiv.), Pd₂(dba)₃-chloroform (0.1 g, 0.10 equiv.), t-BuXPhos (0.085 g, 0.20 equiv.), Cs₂CO₃ (970 mg, 2.97 mmol, 3.00 equiv.), dioxane (10 mL). The resulting solution was stirred for 16 h at 110 °C in an oil bath. The reaction was then quenched by the addition of 50 mL of water. The resulting solution was extracted with 3x30 mL of ethyl acetate and the organic layers combined. The organic layer was washed with 1x50 mL of brine, dried over anhydrous sodium sulfate. The solids were filtered out. The resulting mixture was concentrated under vacuum. The crude product (3 mL) was purified by Flash-Prep-HPLC with the following conditions (CombiFlash-1): Column, C18 silica gel; mobile phase, MeCN:Water=1:5 increasing to MeCN:Water=10:1 within 120 min; Detector, UV 254 nm. 70 mg product was obtained. This resulted in 70 mg (20%) of 4-([5-methoxy-4-[3-(pyrrolidin-1-yl)propoxylpyridin-2-yllamino)pyridine-2-carbonitrile as an off-white solid.

### Step 4: Synthesis of N-(2-(aminomethyl)pyridin-4-yl)-5-methoxy-4-(3-(pyrrolidin-1-yl)propoxy)pyridin-2-amine:

Into a 25-mL round-bottom flask, was placed 4-([5-methoxy-4-[3-(pyrrolidin-1-yl)propoxy]pyridin-2-yl]amino)pyridine-2-carbonitrile (70 mg, 0.20 mmol, 1.00 equiv.), Raney-Ni (0.2 g), methanol (5 mL). The mixture underwent three hydrogen/air exchange cycles. The resulting solution was stirred for 120 min at room temperature. The solids were filtered out. The resulting mixture was concentrated under vacuum. This resulted in 65 mg (92%) of N-[2-(aminomethyl)pyridin-4-yl]-5-methoxy-4-[3-(pyrrolidin-1-yl)propoxy]pyridin-2-amine as an off-white solid

### Step 5: Synthesis of 5-methoxy-4-(3-(pyrrolidin-1-yl)propoxy)-N-(2-(((tetrahydro-2H-pyran-4-yl)amino)methyl)pyridin-4-yl)pyridin-2-amine:

Into a 25-mL round-bottom flask, was placed N-[2-(aminomethyl)pyridin-4-yl]-5-methoxy-4-[3-(pyrrolidin-1-yl)propoxy]pyridin-2-amine (66 mg, 0.18 mmol, 1.00 equiv.), 1-(sodioboranyl)ethan-1-one acetyl acetate dihydrate (84 mg, 0.39 mmol, 3.50 equiv.), oxan-4-one (18 mg, 0.18 mmol, 1.00 equiv.), dichloromethane (3 mL). The resulting solution was stirred for 120 min at room temperature. The solids were filtered out. The resulting mixture was concentrated under vacuum. The crude product (1 mL) was purified by Prep-HPLC with the following conditions: Column, XBride Prep C18 OBD Column19X150 mm 5umC-0013; mobile phase, Phase A:Waters(HCl) Phase B: ACN =1:1; Detector, 254/220nm. 30.1 mg product was obtained. This resulted in 30.1 mg (34%) of 5-methoxy-N-(2-[[(oxan-4-yl)amino]methyl]pyridin-4-yl)-4-[3-(pyrrolidin-1-yl)propoxy]pyridin-2-amine hydrochloride as a white solid.

### Example 13: Synthesis of Compound 13 (Reference)

### Synthesis of 1-(4-(((4-((4-methoxy-3-(3-(pyrrolidin-1-yl)propoxy)phenyl)amino)pyridin-2-yl)(methyl)amino)methyl)piperidin-1-yl)ethan-1-one:

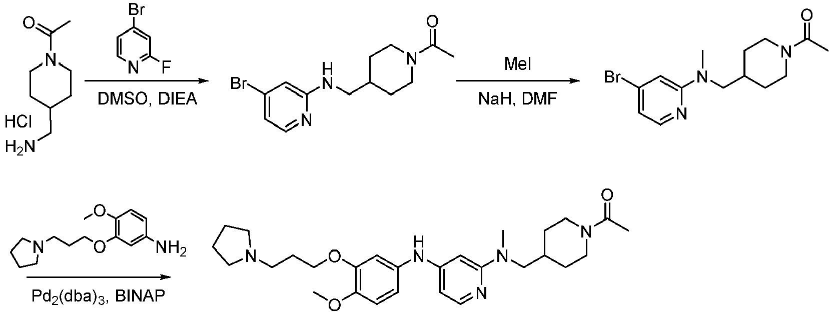

### Step 1: Synthesis of 1-(4-(((4-bromopyridin-2-yl)amino)methyl)piperidin-1-yl)ethan-1-one:

Into a 50-mL round-bottom flask, was placed 1-[4-(aminomethyl)piperidin-1-yl]ethan-1-one hydrochloride (500 mg, 2.59 mmol, 1.00 equiv.), 4-bromo-2-fluoropyridine (500 mg, 2.84 mmol, 1.10 equiv.), DIEA (0.546 g, 2.00 equiv.), DMSO (10 mL). The resulting solution was stirred for 180 min at 120 °C in an oil bath. The reaction was then quenched by the addition of 50 mL of water. The resulting solution was extracted with 3x30 mL of ethyl acetate and the organic layers combined. The resulting mixture was washed with 3x50 mL of BRINE. The mixture was dried over anhydrous sodium sulfate. The solids were filtered out. The resulting mixture was concentrated under vacuum. The crude product (3 mL) was purified by Flash-Prep-HPLC with the following conditions (IntelFlash-1): Column, C18 silica gel; mobile phase, MeCN:Water=1:10 increasing to MeCN:Water=10:1 within 60 min; Detector, UV 254 nm. 0.34 g product was obtained. This resulted in 0.34 g (40%) of 1-(4-[[(4-bromopyridin-2-yl)amino]methyl]piperidin-1-yl)ethan-1-one as off-white oil.

### Step 2: Synthesis of 1-(4-(((4-bromopyridin-2-yl)(methyl)amino)methyl)piperidin-1-yl)ethan-1-one:

Into a 25-mL round-bottom flask, was placed 1-(4-[[(4-bromopyridin-2-yl)amino]methyl]piperidin-1-yl)ethan-1-one (340 mg, 1.09 mmol, 1.00 equiv.), N,N-dimethylformamide (3 mL). The mixture was cooled to 0 °C, then sodium hydride (28 mg, 1.20 mol, 1.10 equiv.) was added in one portion and stirred at 0 °C for 30 min, iodomethane (250 mg, 1.76 mmol, 1.50 equiv.) was added drop wise. The resulting solution was stirred for 120 min at 0 °C in a water/ice bath. The reaction was then quenched by the addition of 25 mL of water. The resulting solution was extracted with of dichloromethane and the organic layers combined and concentrated under vacuum. The crude product (3 mL) was purified by Flash-Prep-HPLC with the following conditions (IntelFlash-1): Column, C18 silica gel; mobile phase, MeCN:Water=1:10 increasing to MeCN:Water=10:1 within 120 min; Detector, UV 254 nm. 0.31 g product was obtained. This resulted in 0.31 g (87%) of 1-(4-[[(4-bromopyridin-2-yl)(methyl)amino]methyl] piperidin-1-yl)ethan-1-one as a white solid.

### Step 3: Synthesis of 1-(4-(((4-((4-methoxy-3-(3-(pyrrolidin-1-yl)propoxy)phenyl)amino)pyridin-2-yl)(methyl)amino)methyl)piperidin-1-yl)ethan-1-one:

Into a 100-mL round-bottom flask, was placed 1-(4-[[(4-bromopyridin-2-yl)(methyl)amino]methyl]piperidin-1-yl)ethan-1-one (290 mg, 0.89 mmol, 1.00 equiv.), 4-methoxy-3-[3-(pyrrolidin-1-yl)propoxy] aniline (440 mg, 1.76 mmol, 2.00 equiv.), Pd₂(dba)₃ chloroform (0.092 g, 0.10 equiv.), BINAP (116 mg, 0.19 mmol, 0.20 equiv.), tBuONa (256 mg, 2.67 mmol, 3.00 equiv.), toluene (15 mL). The resulting solution was stirred for 16 h at 100 °C in an oil bath. The reaction was then quenched by the addition of 100 of water. The resulting solution was extracted with 3x50 mL of ethyl acetate and the organic layers combined. The resulting mixture was washed with 1x100 mL of BRINE. The mixture was dried over anhydrous sodium sulfate. The solids were filtered out. The resulting mixture was concentrated under vacuum. The crude product (1.5 mL) was purified by Prep-HPLC with the following conditions: Column, Xbridge Prep C18 OBD Column 19*150mm 5umC-0013; mobile phase, Phase A:Waters(0.05%NH₃H₂O) Phase B:ACN Gradient; Detector, 254/220. 60.5 mg product was obtained. This resulted in 60.5 mg (14%) of 1-[4-([[4-([4-methoxy-3-[3-(pyrrolidin-1-yl)propoxy]phenyl]amino)pyridin-2-yl](methyl)amino]methyl)piperidin-1-yl]ethan-1-one as a white solid.

### Example 14: Synthesis of Compound 14 (Reference)

### Synthesis of 1-(4-(((2-((4-methoxy-3-(3-(pyrrolidin-1-yl)propoxy)phenyl)amino)pyrimidin-4-yl)oxy)methyl)piperidin-1-yl)ethan-1-one:

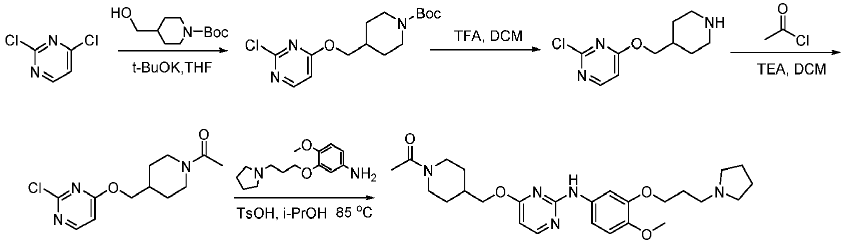

### Step 1: Synthesis of tert-butyl 4-(((2-chloropyrimidin-4-yl)oxy)methyl)piperidine-1-carboxylate:

Into a 100-mL round-bottom flask, was placed 2,4-dichloropyrimidine (1 g, 6.71 mmol, 1.00 equiv.), tert-butyl 4-(hydroxymethyl)piperidine-1-carboxylate (1.45 g, 6.74 mmol, 1.00 equiv.), tetrahydrofuran (20 mL), t-BuOK (1.51 g, 13.46 mmol, 2.00 equiv.). The resulting solution was stirred for 12 h at 20 °C. The resulting mixture was concentrated under vacuum. The residue was applied onto a silica gel column with ethyl acetate/petroleum ether (1:1). This resulted in 1.5 g (68%) of tert-butyl 4-[[(2-chloropyrimidin-4-yl)oxy]methyl]piperidine-1-carboxylate as an off-white solid.

### Step 2: Synthesis of 2-chloro-4-(piperidin-4-ylmethoxy)pyrimidine:

Into a 100-mL round-bottom flask, was placed tert-butyl 4-[[(2-chloropyrimidin-4-yl)oxy]methyl]piperidine-1-carboxylate (1.5 g, 4.58 mmol, 1.00 equiv.), dichloromethane (15 mL), trifluoroacetic acid (5 mL). The resulting solution was stirred for 12 h at 20 °C. The resulting mixture was concentrated under vacuum. The crude product was purified by Flash-Prep-HPLC with the following conditions (IntelFlash-1): Column, silica gel; mobile phase, ; Detector, UV 254 nm. This resulted in 1.8 mg (crude) of 2-chloro-4-(piperidin-4-ylmethoxy)pyrimidine as yellow crude oil.

### Step 3: Synthesis of 1-(4-(((2-chloropyrimidin-4-yl)oxy)methyl)piperidin-1-yl)ethan-1-one:

Into a 100-mL 3-necked round-bottom flask, was placed 2-chloro-4-(piperidin-4-ylmethoxy)pyrimidine (1.5 g, 6.59 mmol, 1.00 equiv.), dichloromethane (10 mL), TEA (2 g, 19.76 mmol, 3.00 equiv.). This was followed by the addition of acetyl chloride (780 mg, 9.94 mmol, 1.50 equiv.) dropwise with stirring at 0 °C. The resulting solution was stirred for 2 h at 20 °C. The resulting mixture was concentrated under vacuum. This resulted in 1 g (56%) of 1-(4-[[(2-chloropyrimidin-4-yl)oxy]methyl]piperidin-1-yl)ethan-1-one as yellow oil.

### Step 4: Synthesis of 1-(4-(((2-((4-methoxy-3-(3-(pyrrolidin-1-yl)propoxy)phenyl)amino)pyrimidin-4-yl)oxy)methyl)piperidin-1-yl)ethan-1-one:

Into a 50-mL round-bottom flask, was placed 4-methoxy-3-[3-(pyrrolidin-1-yl)propoxy]aniline (278.8 mg, 1.11 mmol, 1.00 equiv.), 1-(4-[[(2-chloropyrimidin-4-yl)oxy]methyl]piperidin-1-yl)ethan-1-one (300 mg, 1.11 mmol, 1.00 equiv.), TosOH (568.8 mg, 3.35 mmol, 3.00 equiv.), isopropanol (5 mL). The resulting solution was stirred for 2 h at 85 °C in an oil bath. The crude product was purified by Flash-Prep-HPLC with the following conditions (IntelFlash-1): Column, silica gel; mobile phase, ; Detector, UV 254 nm. This resulted in 71.6 mg (13%) of 1-[4-([[2-([4-methoxy-3-[3-(pyrrolidin-1-yl)propoxy]phenyl]amino)pyrimidin-4-yl]oxy]methyl)piperidin-1-yl]ethan-1-one as a pink solid.

### Example 15: Synthesis of Compound 15 (Reference)

### Synthesis of 1-(2-methoxy-5-((4-((piperidin-4-ylmethyl)amino)pyrimidin-2-yl)amino)phenoxy)-3-(pyrrolidin-1-yl)propan-2-ol:

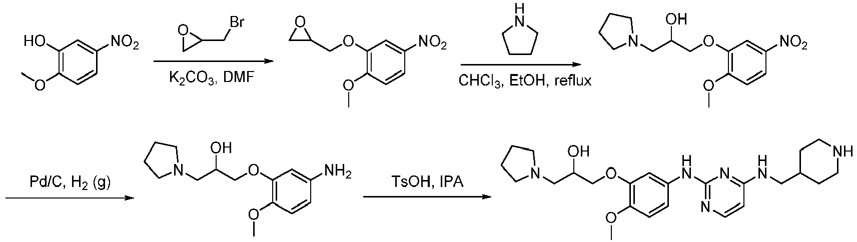

### Step 1: Synthesis of 2-((2-methoxy-5-nitrophenoxy)methyl)oxirane:

Into a 100-mL 3-necked round-bottom flask, was placed a solution of 2-methoxy-5-nitrophenol (2 g, 11.82 mmol, 1.00 equiv.) in N,N-dimethylformamide (20 mL), 2-(bromomethyl)oxirane (2.43 g, 17.74 mmol, 1.50 equiv.), potassium carbonate (3.27 g, 23.66 mmol, 2.00 equiv.). The resulting solution was stirred for overnight at room temperature. The resulting solution was diluted with 50 mL of EA, washed with 3x50 mL of brine, concentrated under vacuum. The residue was applied onto a silica gel column with ethyl acetate/petroleum ether (1:10-1:1). The collected fractions were combined and concentrated under vacuum. This resulted in 1.71 g (64%) of 2-(2-methoxy-5-nitrophenoxymethyl)oxirane as an off-white solid.

### Step 2: Synthesis of 1-(2-methoxy-5-nitrophenoxy)-3-(pyrrolidin-1-yl)propan-2-ol:

Into a 50-mL 3-necked round-bottom flask, was placed a solution of 2-(2-methoxy-5-nitrophenoxymethyl)oxirane (1.4 g, 6.22 mmol, 1.00 equiv.) in ethanol (8 mL), pyrrolidine (1.1 g, 15.47 mmol, 2.50 equiv.), chloroform (8 mL). The resulting solution was stirred for 2 h at 40 °C in an oil bath. The resulting mixture was concentrated under vacuum. The residue was applied onto a silica gel column with dichloromethane/methanol (100:-10:1). The collected fractions were combined and concentrated under vacuum. This resulted in 1.7 g (92%) of 1-(2-methoxy-5-nitrophenoxy)-3-(pyrrolidin-1-yl)propan-2-ol as yellow oil.

### Step 3: Synthesis of 1-(5-amino-2-methoxyphenoxy)-3-(pyrrolidin-1-yl)propan-2-ol:

Into a 100-mL round-bottom flask, was placed 1-(2-methoxy-5-nitrophenoxy)-3-(pyrrolidin-1-yl)propan-2-ol (1.7 g, 5.74 mmol, 1.00 equiv.), Palladium carbon(10%) (200 mg) and Methanol (20 mL). The mixture underwent three hydrogen/air exchange cycles. The resulting solution was stirred for 4 h at room temperature. The solids were filtered out. The resulting mixture was concentrated under vacuum. This resulted in 1.6 g (105%) of 1-(5-amino-2-methoxyphenoxy)-3-(pyrrolidin-1-yl)propan-2-ol as dark red oil.

### Step 4: Synthesis of 1-(2-methoxy-5-((4-((piperidin-4-ylmethyl)amino)pyrimidin-2-yl)amino)phenoxy)-3-(pyrrolidin-1-yl)propan-2-ol:

Into a 50 mL 3-necked round-bottom flask, was placed 1-(5-amino-2-methoxyphenoxy)-3-(pyrrolidin-1-yl)propan-2-ol (310 mg, 1.16 mmol, 1.00 equiv.), isopropanol (10 mL), tert-butyl 4-[[(2-chloropyrimidin-4-yl)amino]methyl]piperidine-1-carboxylate (381 mg, 1.17 mmol, 1.00 equiv.), TsOH (1H₂O) (1.107 g, 5.83 mmol, 5.00 equiv.). The resulting solution was stirred for 4 h at 85 °C in an oil bath. The resulting mixture was concentrated under vacuum. The resulting solution was dissolved in 20 mL of H₂O. Sodium carbonate was employed to adjust the pH to 9. The resulting solution was extracted with 3x20 mL of chloroform/ isopropanol (1/1) and the organic layers combined and dried over anhydrous sodium sulfate and concentrated under vacuum. The residue was purified by Flash chromatography with MeCN/H₂O(40%). The collected fractions were combined and concentrated under vacuum. This resulted in 77.9 mg (15%) of 1-[2-methoxy-5-([4-((piperidin-4-ylmethyl)amino]pyrimidin-2-yl)amino)phenoxy]-3-(pyrrolidin-1-yl)propan-2-ol as a white solid.

### Example 16: Synthesis of Compound 16 (Reference)

### Synthesis of N2-(4-methoxy-3-(3-morpholinopropoxy)phenyl)-N4-(piperidin-4-ylmethyl)pyrimidine-2,4-diamine:

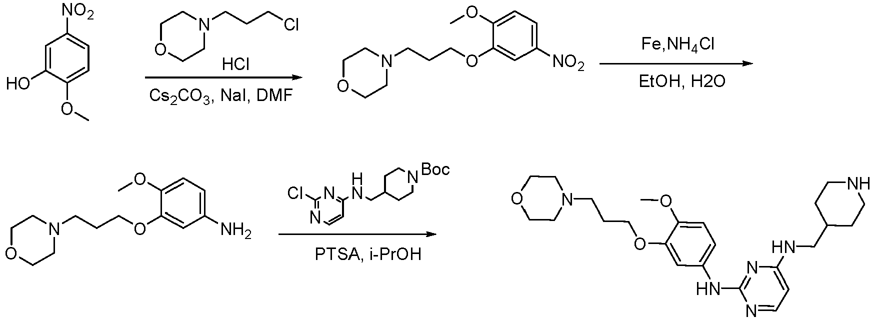

### Step 1: Synthesis of 4-(3-(2-methoxy-5-nitrophenoxy)propyl)morpholine:

Into a 50-mL round-bottom flask, was placed 2-methoxy-5-nitrophenol (1.1 g, 6.50 mmol, 1.00 equiv.), 4-(3-chloropropyl)morpholine hydrochloride (1.3 g, 6.50 mmol, 1.00 equiv.), Cs₂CO₃ (6.39 g, 19.61 mmol, 3.00 equiv.), NaI (980 mg, 1.00 equiv.), N,N-dimethylformamide (10 mL). The resulting solution was stirred for 16 h at 110 °C. The resulting solution was diluted with 50 mL of H₂O. The resulting solution was extracted with 100 mL of ethyl acetate and the organic layers combined. The resulting mixture was washed with 5x100 mL of brine. The resulting mixture was concentrated under vacuum. This resulted in 1.17 g (61%) of 4-[3-(2-methoxy-5-nitrophenoxy)propyl]morpholine as a yellow solid

### Step 2: Synthesis of 4-methoxy-3-(3-morpholinopropoxy)aniline:

Into a 50-mL round-bottom flask, was placed 4-[3-(2-methoxy-5-nitrophenoxy)propyl]morpholine (450 mg, 1.52 mmol, 1.00 equiv.), NH₄Cl (242 mg, 4.52 mmol, 3.00 equiv.), ethanol (12 mL), water(3 mL), Fe (256 mg, 4.57 mmol, 3.00 equiv.). The resulting solution was stirred for 12 h at 85 °C in an oil bath. The solids were filtered out. The resulting mixture was concentrated under vacuum. The crude product was purified by C18 Flash: ACN/H₂O(1/4).This resulted in 200 mg (49%) of 4-methoxy-3-[3-(morpholin-4-yl)propoxy] aniline as a yellow oil.

### Step 3: Synthesis of N²-(4-methoxy-3-(3-morpholinopropoxy)phenyl)-N⁴-(piperidin-4-ylmethyl)pyrimidine-2,4-diamine:

Into a 50-mL round-bottom flask, was placed 4-methoxy-3-[3-(morpholin-4-yl)propoxy]aniline (200 mg, 0.75 mmol, 1.00 equiv.), tert-butyl 4-[(2-chloropyrimidin-4-yl)amino]methylpiperidine-1-carboxylate (184 mg, 0.56 mmol, 1.00 equiv.), PTSA (485 mg, 2.82 mmol, 5.00 equiv.), isopropanol (8 mL). The resulting solution was stirred for 2 h at 85 °C in an oil bath. The resulting solution was extracted with 3x30 mL of ethyl acetate and the organic layers combined and concentrated under vacuum. The crude product was purified by Prep-HPLC with the following conditions : Column: X Bridge RP, 19*150 mm, 5 um; Mobile Phase A:Water/10mmol NH₄HCO₃ , Mobile Phase B: ACN; Flow rate: 30 mL/min; Gradient: 15%B to 43%B in 10 min; 254nm.This resulted in 31.1 mg (9%) of 2-N-[4-methoxy-3-[3-(morpholin-4-yl)propoxy]phenyl]-4-N-(piperidin-4-ylmethyl)pyrimidine-2,4-diamine as a white solid.

### Example 17: Synthesis of Compound 17 (Reference)

### Synthesis of N²-(3-(2-((cyclopentylmethyl)amino)ethyl)-4-methoxyphenyl)-N⁴-(piperidin-4-ylmethyl)pyrimidine-2,4-diamine:

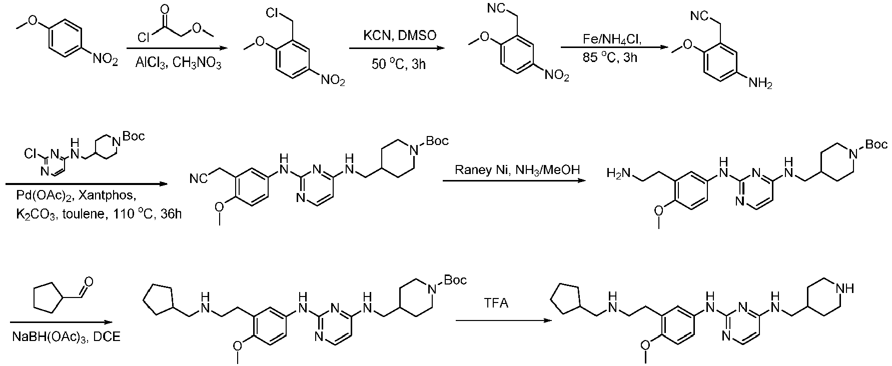

### Step 1: Synthesis of 2-(chloromethyl)-1-methoxy-4-nitrobenzene:

Into a 250-mL 3-necked round-bottom flask, was placed 1-methoxy-4-nitrobenzene (12 g, 78.36 mmol, 1.00 equiv.), CH₃NO₃ (100 mL), AlCl₃ (25.9 g, 2.50 equiv.). This was followed by the addition of 2-methoxyacetyl chloride (9.32 g, 85.88 mmol, 1.10 equiv.) dropwise with stirring at 0 °C. The resulting solution was stirred for 5 h at 20 °C. The reaction was then quenched by the addition of 100 mL of water/ice. The resulting solution was extracted with 2x120 mL of ethyl acetate and the organic layers combined and concentrated under vacuum. The residue was applied onto a silica gel column with dichloromethane/petroleum ether (1/4). The collected fractions were combined and concentrated under vacuum. This resulted in 6.9 g (44%) of 2-(chloromethyl)-1-methoxy-4-nitrobenzene as an off-white solid.

### Step 2: Synthesis of 2-(2-methoxy-5-nitrophenyl)acetonitrile:

Into a 250-mL round-bottom flask, was placed 2-(chloromethyl)-1-methoxy-4-nitrobenzene (6.9 g, 34.23 mmol, 1.00 equiv.), DMSO (100 mL), KCN (13.4 g, 205.77 mmol, 6.00 equiv.). The resulting solution was stirred for 2 h at 50 °C. The resulting solution was diluted with 200 mL of H₂O. The resulting solution was extracted with 2x200 mL of ethyl acetate and the organic layers combined. The resulting mixture was washed with 4x300 mL of brine. The resulting mixture was concentrated under vacuum. The residue was applied onto a silica gel column with dichloromethane/petroleum ether (1/1). This resulted in 4.58 g (70%) of 2-(2-methoxy-5-nitrophenyl)acetonitrile as an off-white solid.

### Step 3: Synthesis of 2-(5-amino-2-methoxyphenyl)acetonitrile:

Into a 250-mL round-bottom flask, was placed 2-(2-methoxy-5-nitrophenyl)acetonitrile (4.58 g, 23.83 mmol, 1.00 equiv.), Fe (4 g, 3.00 equiv.), NH4Cl (3.79 g, 70.85 mmol, 3.00 equiv.), ethanol (100 mL), water (20 mL). The resulting solution was stirred for 2 h at 85 °C. The solids were filtered out. The resulting mixture was concentrated under vacuum. This resulted in 3.6 g (93%) of 2-(5-amino-2-methoxyphenyl)acetonitrile as a brown solid.

### Step 4: Synthesis of tert-butyl 4-(((2-((3-(cyanomethyl)-4-methoxyphenyl)amino)pyrimidin-4-yl)amino)methyl)piperidine-1-carboxylate:

Into a 250-mL round-bottom flask purged and maintained with an inert atmosphere of nitrogen, was placed 2-(5-amino-2-methoxyphenyl)acetonitrile (1.8 g, 11.10 mmol, 1.00 equiv.), tert-butyl 4-[[(2-chloropyrimidin-4-yl)amino]methyl]piperidine-1-carboxylate (3.62 g, 11.08 mmol, 1.00 equiv.), Pd(OAc)2 (249 mg, 1.11 mmol, 0.10 equiv.), Xantphos (642 mg, 1.11 mmol, 0.20 equiv.), potassium carbonate (4.6 g, 33.28 mmol, 3.00 equiv.), Toluene (100 mL). The resulting solution was stirred for 16 h at 115 °C. The solids were filtered out. The resulting mixture was concentrated under vacuum. The residue was applied onto a silica gel column with ACN/H₂O (3/2). This resulted in 2.52 g (50%) of tert-butyl 4-[[(2-[[3-(cyanomethyl)-4-methoxyphenyl]amino]pyrimidin-4-yl)amino]methyl]piperidine-1-carboxylate as a yellow solid.

### Step 5: Synthesis of tert-butyl 4-(((2-((3-(2-aminoethyl)-4-methoxyphenyl)amino)pyrimidin-4-yl)amino)methyl)piperidine-1-carboxylate:

Into a 250-mL round-bottom flask, was placed tert-butyl 4-[[(2-[[3-(cyanomethyl)-4-methoxyphenyl]amino]pyrimidin-4-yl)amino]methyl]piperidine-1-carboxylate (2.52 g, 5.57 mmol, 1.00 equiv.), NH3/MeOH (20 mL), ethyl acetate (10 mL), Raney Ni (1 g). The mixture underwent three hydrogen/air exchange cycles. The resulting solution was stirred for 18 h at 20 °C. The solids were filtered out. The resulting mixture was concentrated under vacuum. This resulted in 2.4 g (94%) of tert-butyl 4-[[(2-[[3-(2-aminoethyl)-4-methoxyphenyl]amino]pyrimidin-4-yl)amino]methyl] piperidine-1-carboxylate as brown oil.

### Step 6: Synthesis of tert-butyl 4-(((2-((3-(2-((cyclopentylmethyl)amino)ethyl)-4-methoxyphenyl)amino)pyrimidin-4-yl)amino)methyl)piperidine-1-carboxylate:

Into a 50-mL round-bottom flask, was placed tert-butyl 4-[[(2-[[3-(2-aminoethyl)-4-methoxyphenyl]amino]pyrimidin-4-yl)amino]methyl]piperidine-1-carboxylate (400 mg, 0.88 mmol, 1.00 equiv.), cyclopentanecarbaldehyde (69 mg, 0.70 mmol, 0.80 equiv.), dichloromethane (20 mL)and was stirred for 0.5h at 20 °C. This was followed by the addition of acetyl ethaneperoxoate sodioboranyl acetate (1.1 g, 5.19 mmol, 6.00 equiv.), in portions at 0 °C. The resulting solution was stirred for 1 h at 20 °C. The resulting mixture was concentrated under vacuum. The residue was applied onto a silica gel column with ACN/H₂O (2/3). This resulted in 130 mg (28%) of tert-butyl 4-[([2-[(3-[2-[(cyclopentylmethyl)amino]ethyl]-4-methoxyphenyl)amino]pyrimidin-4-yl]amino)methyl]piperidine-1-carboxylate as yellow oil.

### Step 7: Synthesis of N²-(3-(2-((cyclopentylmethyl)amino)ethyl)-4-methoxyphenyl)-N⁴-(piperidin-4-ylmethyl)pyrimidine-2,4-diamine:

Into a 50-mL round-bottom flask, was placed tert-butyl 4-[([2-[(3-[2-[(cyclopentylmethyl)amino]ethyl]-4-methoxyphenyl)amino]pyrimidin-4-yl]amino)methyl]piperidine-1-carboxylate (130 mg, 0.24 mmol, 1.00 equiv.), dichloromethane (10 mL), trifluoroacetic acid (1.5 mL). The resulting solution was stirred for 1 h at 20 °C. The resulting mixture was concentrated under vacuum. The resulting solution was diluted with 10 mL of H₂O. The pH value of the solution was adjusted to 8 with sodium bicarbonate. The resulting mixture was concentrated under vacuum. The residue was applied onto a silica gel column with ACN/H₂O (1/6). This resulted in 48.2 mg (46%) of 2-N-(3-[2-[(cyclopentylmethyl)amino]ethyl]-4-methoxyphenyl)-4-N-(piperidin-4-ylmethyl)pyrimidine-2,4-diamine as a white solid.

### Example 18: Synthesis of Compound 18 (Reference)

### Synthesis of 1-(4-(((2-((4-methoxy-3-((1-methylpyrrolidin-3-yl)methoxy)phenyl)amino)pyrimidin-4-yl)amino)methyl)piperidin-1-yl)ethan-1-one:

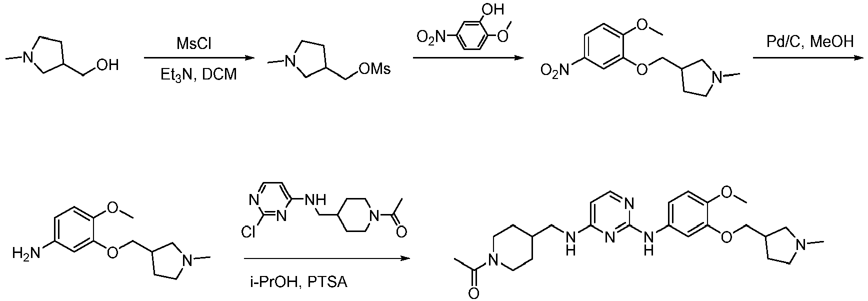

### Step 1: Synthesis of (1-methylpyrrolidin-3-yl)methyl methanesulfonate:

Into a 100-mL round-bottom flask, was placed (1-methylpyrrolidin-3-yl)methanol (500 mg, 4.34 mmol, 1.00 equiv.), TEA (1.3 g, 12.85 mmol, 3.00 equiv.), dichloromethane (20 mL). MsCl (743 mg, 1.50 equiv.) was added drop wise at 0 °C. The resulting solution was stirred for 2 h at 25 °C. The resulting solution was quenched with 20 mL of water, extracted with 3x100 mL of dichloromethane and the organic layers combined. The resulting mixture was washed with 3x50 mL of Brine. The mixture was dried over anhydrous sodium sulfate and concentrated under vacuum. This resulted in 700 mg (83%) of (1-methylpyrrolidin-3-yl)methyl methanesulfonate as yellow oil.

### Step 2: Synthesis of 3-((2-methoxy-5-nitrophenoxy)methyl)-1-methylpyrrolidine:

Into a 50-mL round-bottom flask, was placed (1-methylpyrrolidin-3-yl)methyl methanesulfonate (150 mg, 0.78 mmol, 1.00 equiv.), Cs₂CO₃ (760 mg, 2.33 mmol, 3.00 equiv.), N,N-dimethylformamide (10 mL), 2-methoxy-5-nitrophenol (132 mg, 0.78 mmol, 1.00 equiv.). The resulting solution was stirred for 2 h at 80 °C in an oil bath. The solids were filtered out. The residue was washed with 10 mL of water, extracted with 3x20 mL of ethyl acetate, the organic phase was combined and washed with 3x50 mL of brine, dried over Na₂SO₄, the solid was filtered out, the residue was evaporated, The crude product was purified by Flash-Prep-HPLC with the following conditions (IntelFlash-1): Column, silica gel; mobile phase, ACN/H₂O=6/1; Detector, UV 254 nm. This resulted in 180 mg (87%) of 3-(2-methoxy-5-nitrophenoxymethyl)-1-methylpyrrolidine as yellow oil.

### Step 3: Synthesis of 4-methoxy-3-((1-methylpyrrolidin-3-yl)methoxy)aniline:

Into a 100-mL round-bottom flask, was placed 3-(2-methoxy-5-nitrophenoxymethyl)-1-methylpyrrolidine (250 mg, 0.94 mmol, 1.00 equiv.), Palladium carbon, methanol (30 mL), The mixture underwent three hydrogen/air exchange cycles. The resulting solution was stirred for 4 h at 25 °C. The solids were filtered out. The resulting mixture was concentrated under vacuum. This resulted in 200 mg (90%) of 4-methoxy-3-[(1-methylpyrrolidin-3-yl)methoxy]aniline as a white solid.

### Step 4: Synthesis of 1-(4-(((2-((4-methoxy-3-((1-methylpyrrolidin-3-yl)methoxy)phenyl)amino)pyrimidin-4-yl)amino)methyl)piperidin-1-yl)ethan-1-one:

Into a 50-mL round-bottom flask, was placed 4-methoxy-3-[(1-methylpyrrolidin-3-yl)methoxy]aniline (150 mg, 0.63 mmol, 1.00 equiv.), isopropanol (20 mL), p-TsOH (327 mg, 3.00 equiv.), 1-(4-[(2-chloropyrimidin-4-yl)amino]methylpiperidin-1-yl)ethan-1-one (170 mg, 0.63 mmol, 1.00 equiv.). The resulting solution was stirred for 4 h at 85 °C in an oil bath. The resulting mixture was concentrated under vacuum. The crude product was purified by Prep-HPLC with the following conditions (Column: X Select C18, 19*150 mm, 5 um): Column; mobile phase, Mobile Phase A:Water/0.05% NH₄HCO₃, Mobile Phase B: ACN; Detector. This resulted in 156.6 mg (53%) of 1-[4-([[2-([4-methoxy-3-[(1-methylpyrrolidin-2-yl)methoxy]phenyl]amino)pyrimidin-4-yl]amino]methyl)piperidin-1-yl]ethan-1-one as a white solid.

### Example 19: Synthesis of Compound 19 (Reference)

### Synthesis of 1-(4-(((2-((4-methoxy-3-(3-(pyrrolidin-1-yl)propoxy)phenyl)amino)-6-methylpyrimidin-4-yl)amino)methyl)piperidin-1-yl)ethan-1-one:

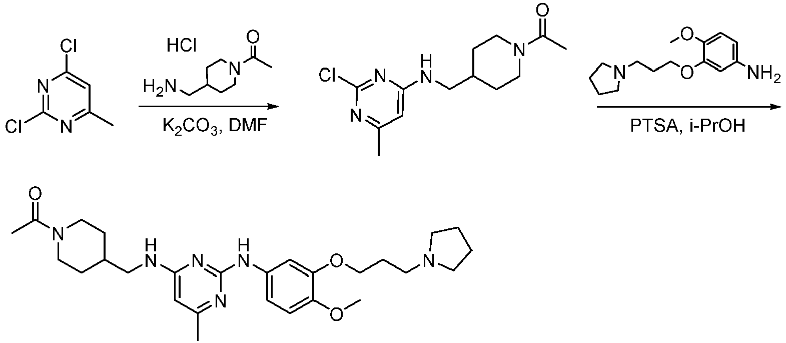

### Step 1: Synthesis of 1-(4-(((2-chloro-6-methylpyrimidin-4-yl)amino)methyl)piperidin-1-yl)ethan-1-one:

Into a 25-mL round-bottom flask, was placed 1-[4-(aminomethyl)piperidin-1-yl]ethan-1-one (300 mg, 1.92 mmol, 1.00 equiv.), N,N-dimethylformamide (5 mL), potassium carbonate (651 mg, 4.71 mmol, 3.00 equiv.), 2,4-dichloro-6-methylpyrimidine (251 mg, 1.54 mmol, 1.00 equiv.). The resulting solution was stirred for 7 h at 60 °C in an oil bath. The solids were filtered out. The resulting mixture was concentrated under vacuum. The residue was applied onto a silica gel column with H₂O/MeCN (1-0). This resulted in 0.21 g (48%) of 1-(4-[[(2-chloro-6-methylpyrimidin-4-yl)amino]methyl] piperidin-1-yl)ethan-1-one as a white solid.

### Step 2: Synthesis of 1-(4-(((2-((4-methoxy-3-(3-(pyrrolidin-1-yl)propoxy)phenyl)amino)-6-methylpyrimidin-4-yl)amino)methyl)piperidin-1-yl)ethan-1-one:

Into a 25-mL round-bottom flask, was placed 1-(4-[[(2-chloro-6-methylpyrimidin-4-yl)amino]methyl]piperidin-1-yl)ethan-1-one (200 mg, 0.71 mmol, 1.00 equiv.), 4-methoxy-3-[3-(pyrrolidin-1-yl)propoxy]aniline (195 mg, 0.78 mmol, 1.10 equiv.), TsOH (269 mg, 1.42 mmol, 2.00 equiv.), isopropanol (5 mL). The resulting solution was stirred for 7 h at 85 °C in an oil bath. The resulting mixture was concentrated under vacuum. The residue was applied onto a silica gel column with H₂O/MeCN (1-0). This resulted in 61.2 mg (17%) of 1-[4-([[2-([4-methoxy-3-[3-(pyrrolidin-1-yl)propoxy]phenyl]amino)-6-methylpyrimidin-4-yl]amino]methyl)piperidin-1-yl]ethan-1-one as a pink solid.

### Example 20: Synthesis of Compound 47 (Reference)

### Compound 47: Synthesis of 1-(4-(((2-((4-methoxy-3-(3-(pyrrolidin-1-yl)propoxy)phenyl)amino)pyrimidin-4-yl)amino)methyl)piperidin-1-yl)ethan-1-one

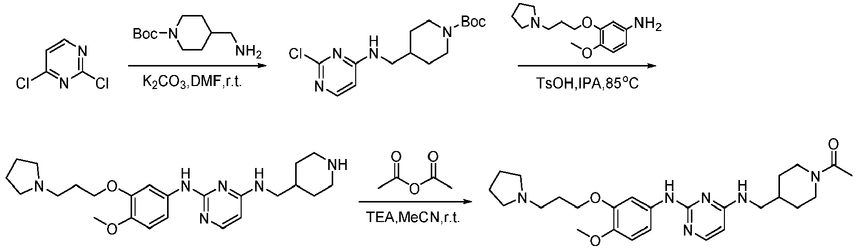

### Step 1: Synthesis of tert-butyl 4-[[(2-chloropyrimidin-4-yl)amino]methyl]piperidine-1-carboxylate:

Into a 250-mL 3-necked round-bottom flask, was placed a solution of 2,4-dichloropyrimidine (5.0 g, 33.56 mmol, 1 equiv) in N,N-dimethylformamide (50 mL), tert-butyl 4-(aminomethyl)piperidine-1-carboxylate (7.18 g, 33.50 mmol, 1 equiv), potassium carbonate (9.26 g, 2.00 equiv). The resulting solution was stirred for 2 h at RT. The resulting solution was diluted with 50 mL of EA, washed with 3x50 mL of brine, dried over anhydrous sodium sulfate and concentrated under vacuum. The residue was applied onto a silica gel column with ethyl acetate/petroleum ether (1:10-1:2). The collected fractions were combined and concentrated under vacuum. This resulted in 8.1 g (74%) of the title compound as colorless oil.

Analytical Data: (ES, *m*/*z*)*:* RT = 1.449 min, LCMS 28: *m*/*z* = 327 [M+1]. H-NMR: (400 MHz, Methanol-*d₄*) δ 8.83 - 8.68 (m, 1H), 7.26 (d, *J* = 5.8 Hz, 1H), 5.01 - 4.56 (m, 2H), 4.06 - 3.80 (m, 2H), 3.49 (s, 2H), 2.58 - 2.37 (m, 3H), 2.20 (s, 9H), 1.95 - 1.69 (m, 2H).

### Step 2: Synthesis of 2-N-[4-methoxy-3-[3-(pyrrolidin-1-yl)propoxy]phenyl]-4-N-(piperidin-4-ylmethyl)pyrimidine-2,4-diamine:

Into a 50-mL 3-necked round-bottom flask, was placed 4-methoxy-3-[3-(pyrrolidin-1-yl)propoxy]aniline (764 mg, 3.05 mmol, 1 equiv), tert-butyl 4-[[(2-chloropyrimidin-4-yl)amino]methyl]piperidine-1-carboxylate (1 g, 3.06 mmol, 1 equiv), TsOH (2.9 g, 15.26 mmol, 5.00 equiv), IPA (10 mL). The resulting solution was stirred for 4 h at 85 °C in an oil bath. The resulting mixture was concentrated under vacuum. The residue was purified by flash chromatography with ACN/H₂O(1/10). This resulted in 800 mg (59%) of the title compound as a solid.

Analytical Data: LC-MS: (ES, *m*/*z):* RT = 0.900 min, LCMS 07: *m*/*z* = 441 [M+1].

### Step 3: Synthesis of 1-[4-([[2-([4-methoxy-3-[3-(pyrrolidin-1-yl)propoxy]phenyl]amino)pyrimidin-4-yl]amino]methyl)piperidin-1-yl]ethan-1-one:

Into a 10-mL sealed tube, was placed 2-N-[4-methoxy-3-[3-(pyrrolidin-1-yl)propoxy]phenyl]-4-N-(piperidin-4-ylmethyl)pyrimidine-2,4-diamine (250 mg, 0.57 mmol, 1 equiv), acetyl acetate (63.6 mg, 0.62 mmol, 1.10 equiv), TEA (114.5 mg, 2.00 equiv), ACN (3 mL). The resulting solution was stirred for 4 h at RT. The resulting mixture was concentrated under vacuum. The residue was purified by flash chromatography with ACN/H₂O (1/10). This resulted in 61.2 mg (22%) of 1-(4-(((2-((4-methoxy-3-(3-(pyrrolidin-1-yl)propoxy)phenyl)amino)pyrimidin-4-yl)amino)methyl)piperidin-1-yl)ethan-1-one as a white solid.

### Example 21: Synthesis of Compound 205

### Compound 205: Synthesis of N²-(4-methoxy-3-(3-(pyrrolidin-1-yl)propoxy)phenyl)-N⁴,6-dimethylpyrimidine-2,4-diamine

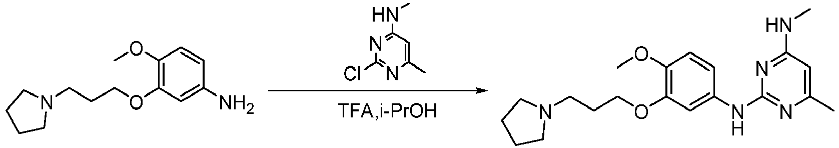

### Step 1: Synthesis of N²-(4-methoxy-3-(3-(pyrrolidin-1-yl)propoxy)phenyl)-N⁴,6-dimethylpyrimidine-2,4-diamine:

Into a 250-mL round-bottom flask, was placed 2-chloro-N,6-dimethylpyrimidin-4-amine (1.5 g, 9.52 mmol, 1 equiv), 4-methoxy-3-[3-(pyrrolidin-1-yl)propoxy]aniline (864 mg, 3.45 mmol, 1 equiv), trifluoroacetic acid (684 mg, 6.05 mmol, 1 equiv), isopropanol (50 mL). The resulting solution was stirred overnight at 85 °C. The resulting mixture was concentrated under vacuum. The crude product was purified by Prep-HPLC G. This resulted in 1.295 g (32%) of N²-(4-methoxy-3-(3-(pyrrolidin-1-yl)propoxy)phenyl)-N⁴,6-dimethylpyrimidine-2,4-diamine as a solid.

### Example 22: Synthesis of Compound 207 (Reference)

### Compound 207: Synthesis of N⁴-methyl-N²-(3-(3-(pyrrolidin-1-yl)propoxy)phenyl)pyrimidine-2,4-diamine

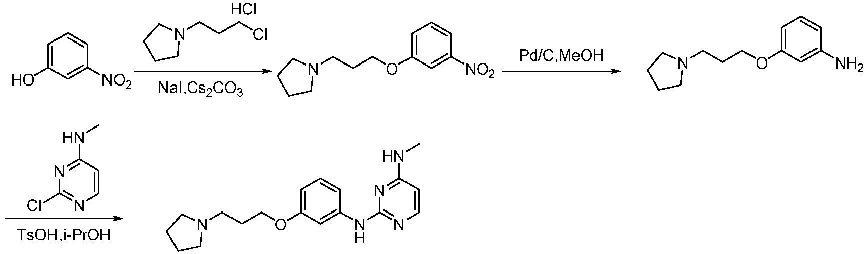

### Step 1: Synthesis of 1-[3-(3-nitrophenoxy)propyl]pyrrolidine:

Into a 50-mL round-bottom flask, was placed 3-nitrophenol (600 mg, 4.31 mmol, 1 equiv), 1-(3-chloropropyl)pyrrolidine hydrochloride (790 mg, 4.29 mmol, 1 equiv), Cs2CO3 (4.22 g, 12.95 mmol, 3.00 equiv), NaI (647 mg, 4.31 mmol, 1 equiv), N,N-dimethylformamide (10 mL). The resulting solution was stirred for 4 h at 110 °C. The resulting solution was diluted with 30 mL of H₂O. The resulting solution was extracted with 3x60 mL of ethyl acetate and the organic layers combined. The resulting mixture was washed with 4x60 mL of brine. The resulting mixture was concentrated under vacuum. This resulted in 1 g (93%) of the title compound as brown oil.

Analytical Data: LC-MS: (ES, *mlz):* RT = 0.975 min, LCMS 53: *mlz* = 251 [M+1].

### Step 2: Synthesis of 3-[3-(pyrrolidin-1-yl)propoxy]aniline:

Into a 100-mL round-bottom flask, was placed 1-[3-(3-nitrophenoxy)propyl]pyrrolidine (1 g, 4.00 mmol, 1 equiv), methanol (20 mL), Pd/C (0.5 g). The resulting solution was stirred for 1 h at 20 °C. The solids were filtered out. The resulting mixture was concentrated under vacuum. This resulted in 0.7 g (80%) of the title compound as an oil.

Analytical Data: LC-MS: (ES, *m*/*z*): RT = 0.586 min, LCMS 07: *m*/*z* = 221 [M+1].

### Step 3: Synthesis of N⁴-methyl-N²-(3-(3-(pyrrolidin-1-yl)propoxy)phenyl)pyrimidine-2,4-diamine:

Into a 50-mL round-bottom flask, was placed 3-[3-(pyrrolidin-1-yl)propoxy]aniline (400 mg, 1.82 mmol, 1 equiv), 2-chloro-N-methylpyrimidin-4-amine (259 mg, 1.80 mmol, 1 equiv), 4-methylbenzene-1-sulfonic acid (464 mg, 2.69 mmol, 1.50 equiv), isopropanol (10 mL). The resulting solution was stirred for 2 h at 85 °C. The resulting mixture was concentrated under vacuum. The residue was applied onto a silica gel column with ACN/H2O (1/5). This resulted in 104.7 mg (18%) of N⁴-methyl-N²-(3-(3-(pyrrolidin-1-yl)propoxy)phenyl)pyrimidine-2,4-diamine as a white solid.

### Example 23: Synthesis of Compound 209 (Reference)

### Compound 209: Synthesis of N²-(4-methoxy-3-(3-(pyrrolidin-1-yl)propoxy)phenyl)-N⁴-methylpyridine-2,4-diamine

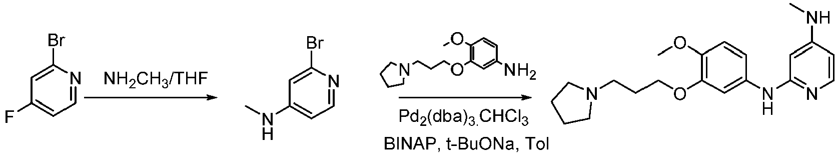

### Step 1: Synthesis of 2-bromo-N-methylpyridin-4-amine:

Into a 60-mL sealed tube, was placed 2-bromo-4-fluoropyridine (500 mg, 2.84 mmol, 1 equiv), NH₂CH₃-THF (20 mL). The resulting solution was stirred for 16 h at 80 °C in an oil bath. The resulting mixture was concentrated under vacuum. This resulted in 480 mg (90%) of the title compound as colorless oil.

Analytical Data: LC-MS: (ES, *m*/*z*): RT = 0.758 min, LCMS 27: *m*/*z* = 187 [M+1].

### Step 2: Synthesis of N²-(4-methoxy-3-(3-(pyrrolidin-1-yl)propoxy)phenyl)-N⁴-methylpyridine-2,4-diamine:

Into a 16-mL sealed tube purged and maintained with an inert atmosphere of nitrogen, was placed 2-bromo-N-methylpyridin-4-amine (400 mg, 2.14 mmol, 1 equiv), 4-methoxy-3-[3-(pyrrolidin-1-yl)propoxy]aniline (640 mg, 2.56 mmol, 1.20 equiv), t-BuONa (616 mg, 6.41 mmol, 3.00 equiv), BINAP (133 mg, 0.21 mmol, 0.10 equiv), Pd₂(dba)₃CHCl₃ (220 mg, 0.10 equiv), toluene (8 mL). The resulting solution was stirred for 3 h at 100 °C in an oil bath. The solids were filtered out. The crude product was purified by Flash-Prep-HPLC with the following conditions (CombiFlash-1): Column, C18 silica gel; mobile phase, methanol/H2O increasing to methanol/H2O=9/1 within min; Detector, UV 254 nm product was obtained. The crude product was purified by Prep-HPLC H. This resulted in 57.5 mg (7%) of N²-(4-methoxy-3-(3-(pyrrolidin-1-yl)propoxy)phenyl)-N⁴-methylpyridine-2,4-diamine as a solid.

### Example 24: Synthesis of Compound 256 (Reference)

### Compound 256: Synthesis of N²-(4-methoxy-3-(3-(pyrrolidin-1-yl)propoxy)phenyl)-N⁴-(oxetan-3-ylmethyl)pyrimidine-2,4-diamine

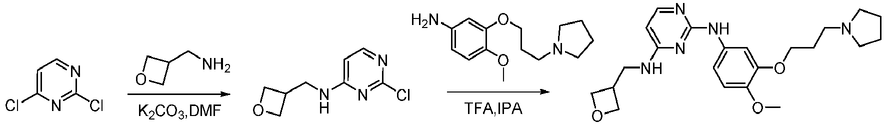

### Step 1: Synthesis of 2-chloro-N-(oxetan-3-ylmethyl)pyrimidin-4-amine:

Into a 20-mL vial, was placed N,N-dimethylformamide (3 mL), 2,4-dichloropyrimidine (595 mg, 3.99 mmol, 1 equiv), oxetan-3-ylmethanamine (350 mg, 4.02 mmol, 1.01 equiv), potassium carbonate (555 mg, 4.02 mmol, 1.01 equiv). The resulting solution was stirred for 2 h at 20 °C. The residue was applied onto a silica gel column with ACN/H₂O (1:9). The collected fractions were combined and concentrated under vacuum. This resulted in 570 mg (71%) of the title compound as a white solid.

Analytical Data: LC-MS: (ES, *m*/*z*)*:* RT = 0.84min, LCMS33: m/z = 200 [M+1].

### Step 2: Synthesis of N²-(4-methoxy-3-(3-(pyrrolidin-1-yl)propoxy)phenyl)-N⁴-methylpyrimidine-2,4-diamine:

Into a 20-mL round-bottom flask, was placed 2-chloro-N-(oxetan-3-ylmethyl)pyrimidin-4-amine (200 mg, 1 mmol, 1 equiv), 4-methoxy-3-[3-(pyrrolidin-1-yl)propoxy]aniline (251 mg, 1 mmol, 1 equiv), trifluoroacetic acid (195 mg, 1.73 mmol, 2.00 equiv), propan-2-ol (2 mL). The resulting solution was stirred for 2 h at 85 °C in an oil bath. The resulting mixture was concentrated under vacuum. The crude product (200 mg) was purified by Prep-HPLC C. This resulted in 20.1 mg (4%) of N²-(4-methoxy-3-(3-(pyrrolidin-1-yl)propoxy)phenyl)-N⁴-(oxetan-3-ylmethyl)pyrimidine-2,4-diamine as a yellow solid.

### Example 25: Synthesis of Compound 257 (Reference)

### Compound 257: Synthesis of N-(4-methoxy-3-(3-(pyrrolidin-1-yl)propoxy)phenyl)-1-methyl-1H-pyrazolo[3,4-b]pyridin-6-amine

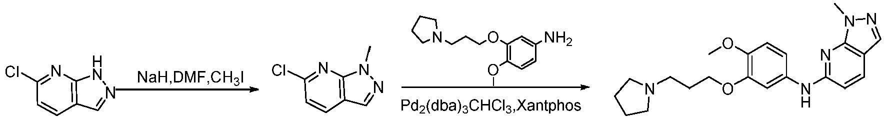

### Step 1: Synthesis of 6-chloro-1-methyl-1H-pyrazolo[3,4-b]pyridine:

Into a 100-mL round-bottom flask, was placed N,N-dimethylformamide (10 mL), sodium hydride (235 mg, 9.79 mmol, 1.50 equiv).This was followed by addition of 6-chloro-1H-pyrazolo[3,4-b]pyridine (1 g, 6.51 mmol, 1 equiv) at 0 °C. The resulting solution was stirred for 30 min at 0 °C. To this above, iodomethane (1.02g, 7.19 mmol, 1.10 equiv) was added. The reaction was allowed to react for 2h at 0 °C. The reaction was then quenched by the addition of 5 mL of water. The residue was applied onto a C18 column with Water/ACN (7:3). This resulted in 500 mg (46%) of the title compound as a white solid.

Analytical Data: LC-MS: (ES, *m*/*z*): RT = 0.729min, LCMS 27: *m*/*z* = 168 [M+1].

### Step 2: Synthesis of N²-(4-methoxy-3-(3-(pyrrolidin-1-yl)propoxy)phenyl)-N⁴-(oxetan-3-ylmethyl)pyrimidine-2,4-diamine:

Into a 100-mL round-bottom flask purged and maintained with an inert atmosphere of nitrogen, was placed dioxane (10 mL), 6-chloro-1-methyl-1H-pyrazolo[3,4-b]pyridine (200 mg, 1.19 mmol, 1 equiv), 4-methoxy-3-[3-(pyrrolidin-1-yl)propoxy] aniline (300 mg, 1.20 mmol, 1 equiv), Pd₂(dba)₃CHCl₃ (186 mg, 0.18 mmol, 0.15 equiv), xantphos (210 mg, 0.36 mmol, 0.30 equiv), Cs2CO3 (780 mg, 2.39 mmol, 2.01 equiv). The resulting solution was stirred for 14 h at 80 °C. The resulting mixture was concentrated under vacuum. The crude product (200 mg) was purified by Prep-HPLC D. This resulted in 97.0 mg (19%) of N-(4-methoxy-3-(3-(pyrrolidin-1-yl)propoxy)phenyl)-1-methyl-1H-pyrazolo[3,4-b]pyridin-6-amine as a solid.

### Example 26: Synthesis of Compound 258 (Reference)

### Compound 258: Synthesis of N-(4-methoxy-3-(3-(pyrrolidin-1-yl)propoxy)phenyl)-1-methyl-1H-imidazo[4,5-c]pyridin-6-amine

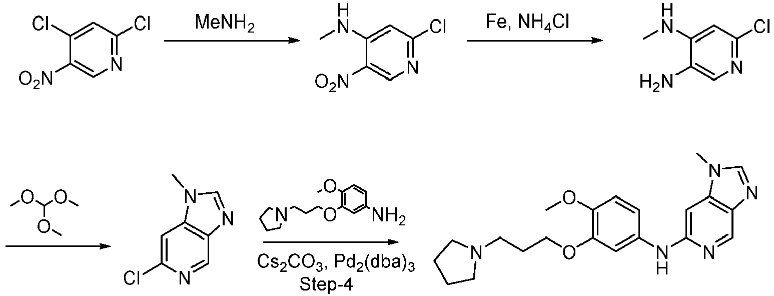

### Step 1: Synthesis of 2-chloro-N-methyl-5-nitropyridin-4-amine:

Into a 30-mL sealed tube, was placed 2,4-dichloro-5-nitropyridine (2 g, 10.36 mmol, 1 equiv), DIEA (2.69 g, 20.81 mmol, 2.00 equiv), tetrahydrofuran (20 mL), NH₂CH₃-HCl (1.06 g, 2.00 equiv). The resulting solution was stirred for 12 h at 25 °C. The crude product was purified by Flash-Prep-HPLC A. This resulted in 1.2 g (62%) of as a yellow solid.

Analytical Data: LC-MS: (ES, *m*/*z*): 188 [M+1], R: 1.12 min.

### Step 2: Synthesis of 6-chloro-4-N-methylpyridine-3,4-diamine:

Into a 100-mL round-bottom flask, was placed 2-chloro-N-methyl-5-nitropyridin-4-amine (2 g, 10.66 mmol, 1 equiv), Fe (2.99 g, 5.00 equiv), NH₄Cl (5.7 g, 106.56 mmol, 10.00 equiv), methanol (20 mL), water (20 mL). The resulting solution was stirred for 12 h at 80 °C in an oil bath. The solids were filtered out. The resulting mixture was concentrated under vacuum. This resulted in 600 mg (36%) of the title compound as a solid.

Analytical Data: LC-MS: (ES, *m*/*z)*: 158 [M+1], R: 0.982 min.

### Step 3: Synthesis of 6-chloro-1-methyl-1H-imidazo[4,5-c]pyridine:

Into a 100-mL round-bottom flask, was placed 6-chloro-4-N-methylpyridine-3,4-diamine (500 mg, 3.17 mmol, 1 equiv), trimethoxymethane (20 mL). The resulting solution was stirred for 4 h at 100 °C in an oil bath. The resulting mixture was concentrated under vacuum. The crude product was purified by Flash-Prep-HPLC A. This resulted in 200 mg (38%) of the title compound as a solid.

Analytical Data: LC-MS: (ES, *m*/*z):* 168 [M+1], R: 0.841 min.

### Step 4: Synthesis of N-[4-methoxy-3-[3-(pyrrolidin-1-yl)propoxy]phenyl]-1-methyl-1H-imidazo[4,5-c]pyridin-6-amine:

Into a 30-mL sealed tube, was placed 6-chloro-1-methyl-1H-imidazo[4,5-c]pyridine (300 mg, 1.79 mmol, 1 equiv), Cs2CO3 (1.76 g, 5.40 mmol, 3.00 equiv), Pd₂(dba)₃-CHCl₃ (100 mg), X-phos (100 mg), 1,4-dioxane (15 mL), 4-methoxy-3-[3-(pyrrolidin-1-yl)propoxy]aniline (540 mg, 2.16 mmol, 1.20 equiv). The resulting solution was stirred for 4 h at 100 °C in an oil bath. The solids were filtered out. The crude product was purified by Prep-HPLC E. This resulted in 54.2 mg (7%) of N-(4-methoxy-3-(3-(pyrrolidin-1-yl)propoxy)phenyl)-1-methyl-1H-imidazo[4,5-c]pyridin-6-amine as a yellow solid.

### Example 27: Synthesis of Compound 259 (Reference)

### Compound 259: Synthesis of N²-(4-methoxy-3-((2-methyl-2-azaspiro[4.5]decan-8-yl)oxy)phenyl)-N⁴-((tetrahydro-2H-pyran-4-yl)methyl)pyrimidine-2,4-diamine

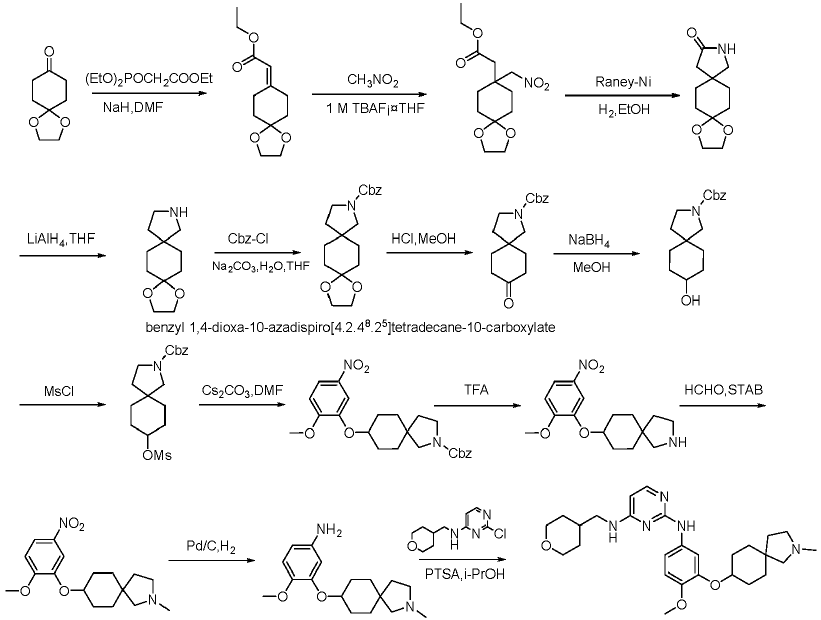

### Step 1: Synthesis of ethyl 2-[1,4-dioxaspiro[4.5]decan-8-ylidene]acetate:

Into a 250-mL round-bottom flask, was placed ethyl 2-(diethoxyphosphoryl)acetate (14.4 g, 64.23 mmol, 1 equiv), tetrahydrofuran (150 mL), sodium hydride (5.12 g, 213.33 mmol, 3.33 equiv), 1,4-dioxaspiro[4.5]decan-8-one (10 g, 64.03 mmol, 1 equiv). The resulting solution was stirred overnight at 0 °C. The reaction was then quenched by the addition of 50 mL of water. The resulting solution was extracted with 3x50 mL of ethyl acetate and the organic layers combined. The resulting mixture was washed with 3x50 mL of H₂O. The mixture was dried over anhydrous sodium sulfate and concentrated under vacuum. The residue was applied onto a silica gel column with ethyl acetate/petroleum ether (1:5). This resulted in 12 g (83%) of as a yellow liquid.

Analytical Data: ¹H NMR (300 MHz, Chloroform-*d*) δ 5.67 (p, *J=* 1.1 Hz, 1H), 4.15 (q, *J* = 7.1 Hz, 2H), 3.98 (s, 4H), 3.00 (ddd, *J* = 7.8, 5.1, 1.2 Hz, 2H), 2.44 - 2.32 (m, 2H), 1.84 - 1.70 (m, 4H), 1.28 (t, *J* = 7.1 Hz, 3H).

### Step 2: Synthesis of ethyl 2-[8-(nitromethyl)-1,4-dioxaspiro[4.5]decan-8-yl]acetate:

Into a 500-mL round-bottom flask, was placed ethyl 2-[1,4-dioxaspiro[4.5]decan-8-ylidene]acetate (12 g, 53.03 mmol, 1 equiv), tetrahydrofuran (150 mL), nitromethane (13 g, 212.98 mmol, 4.02 equiv), TBAF tetrahydrofuran (80 mL). The resulting solution was stirred overnight at 70 °C. The resulting solution was extracted with 3x30 mL of dichloromethane and the organic layers combined. The resulting mixture was washed with 3x30 mL of H₂O. The mixture was dried over anhydrous sodium sulfate and concentrated under vacuum. The residue was applied onto a silica gel column with ethyl acetate/petroleum ether (1:5). This resulted in 11 g (72%) of the title compound as a yellow liquid.

Analytical Data: ¹H NMR (300 MHz, Chloroform-*d*) δ 4.73 (s, 2H), 4.17 (q, *J* = 7.1 Hz, 2H), 3.95 (s, 4H), 2.57 (s, 2H), 1.85 - 1.63 (m, 6H), 1.35 - 1.18 (m, 3H).

### Step 3: Synthesis of 1,4-dioxa-10-azadispiro[4.2.4⁸.2⁵]tetradecan-11-one:

Into a 500-mL round-bottom flask, was placed ethyl 2-[8-(nitromethyl)-1,4-dioxaspiro[4.5]decan-8-yl]acetate (5 g, 17.40 mmol, 1 equiv), methanol (200 mL), Raney-Ni (1 g), TEA (5 g, 49.41 mmol, 2.84 equiv), hydrogen (500 mL). The resulting solution was stirred overnight at RT. The solids were filtered out. The resulting mixture was concentrated under vacuum. This resulted in 4 g (100%) of the title compound as a white solid.

Analytical Data: ¹H NMR (300 MHz, Chloroform-*d*) δ 6.35 (s, 1H), 3.96 (s, 4H), 3.21 (s, 2H), 2.23 (s, 2H), 1.79 - 1.60 (m, 8H).

### Step 4: Synthesis of 1,4-dioxa-10-azadispiro[4.2.4⁸.2⁵]tetradecane:

Into a 1-L round-bottom flask, was placed 1,4-dioxa-10-azadispiro[4.2.4⁸.2⁵]tetradecan-11-one (5.28 g, 24.99 mmol, 1 equiv), tetrahydrofuran (500 mL), LAH (2.85 g, 75.10 mmol, 3.00 equiv) at 0 °C. After 1 h, the resulting solution was stirred overnight at 50 °C. The reaction was then quenched by the addition of 2.85 g of water, 2.85 g of 15% NaOH, 8.55 g of water The solids were filtered out. The resulting mixture was concentrated under vacuum. This resulted in 3.6 g (73%) of the title compound as a colorless oil.

Analytical Data: ¹H NMR (300 MHz, Chloroform-d) δ 5.14 (s, 1H), 3.96 (s, 4H), 3.06 (t, *J* = 7.2 Hz, 2H), 2.81 (s, 2H), 1.65 (d, *J* = 6.2 Hz, 10H).

### Step 5: Synthesis of benzyl 1,4-dioxa-10-azadispiro[4.2.4⁸.2⁵]tetradecane-10-carboxylate:

Into a 250-mL round-bottom flask, was placed 1,4-dioxa-10-azadispiro[4.2.4⁸.2⁵]tetradecane (3.6 g, 18.25 mmol, 1 equiv), sodium carbonate (7.3 g, 68.87 mmol, 3.77 equiv), water(20 mL), tetrahydrofuran (20 mL), benzyl chloroformate (3.7 g, 21.69 mmol, 1.19 equiv). The resulting solution was stirred overnight at RT. The resulting solution was extracted with 3x50 mL of dichloromethane and the organic layers combined. The resulting mixture was washed with 3x50 mL of H2O. The solid was dried in an oven under reduced pressure. The residue was applied onto a silica gel column with ethyl acetate/petroleum ether (2:1). This resulted in 3.5 g (58%) of the title compound as a colorless liquid.

¹H NMR (300 MHz, Chloroform-d) δ 7.47 - 7.30 (m, 5H), 3.96 (s, 4H), 3.49 (t, *J* = 7.2 Hz, 2H), 3.28 (s, 2H), 1.86 - 1.51 (m, 10H).

### Step 6: Synthesis of benzyl 8-oxo-2-azaspiro[4.5]decane-2-carboxylate:

Into a 250-mL round-bottom flask, was placed benzyl 1,4-dioxa-10-azadispiro[4.2.4^[8].2^[5]]tetradecane-10-carboxylate (2.5 g, 7.54 mmol, 1 equiv), methanol (50 mL). This was followed by the addition of HCl (10 mL). 2N The resulting solution was stirred overnight at RT. The resulting solution was extracted with 3x30 mL of dichloromethane and the organic layers combined. The resulting mixture was washed with 3x30 mL of H₂O. The solid was dried in an oven under reduced pressure. This resulted in 2.0 g (83%) of the title compound as a colorless liquid.

Analytical Data: LC-MS: (ES, *m*/*z*): RT=0.877min, LCMS 45, *m*/*z* =288 [M+1].

### Step 7: Synthesis of benzyl 8-hydroxy-2-azaspiro[4.5]decane-2-carboxylate:

Into a 100-mL round-bottom flask, was placed benzyl 8-oxo-2-azaspiro[4.5]decane-2-carboxylate (1.77 g, 6.16 mmol, 1 equiv), methanol (30 mL), NaBH₄ (350 mg, 9.25 mmol, 1.50 equiv). The resulting solution was stirred for 1 h at RT. The reaction was then quenched by the addition of 10 mL of water. The resulting solution was extracted with 3x20 mL of dichloromethane and the organic layers combined. The resulting mixture was washed with 3x20 mL of H₂O. The mixture was dried over anhydrous sodium sulfate and concentrated under vacuum. This resulted in 1.6 g (81%) of the title compound as a colorless liquid.

LC-MS: (ES, *m*/*z*): RT=0.876 min, LCMS 45, *m*/*z* =290 [M+1].

### Step 8: Synthesis of benzyl 8-(methanesulfonyloxy)-2-azaspiro[4.5]decane-2-carboxylate:

Into a 250-mL round-bottom flask, was placed benzyl 8-hydroxy-2-azaspiro[4.5]decane-2-carboxylate (1.87 g, 6.46 mmol, 1 equiv), dichloromethane (50 mL), TEA (1.96 g, 19.37 mmol, 3.00 equiv), MsCl (885 mg). The resulting solution was stirred for 1 h at 0 °C. The reaction was then quenched by the addition of 20 mL of water. The resulting solution was extracted with 3x30 mL of dichloromethane and the organic layers combined. The resulting mixture was washed with 3x30 mL of H2O. The mixture was dried over anhydrous sodium sulfate and concentrated under vacuum. This resulted in 2.0 g (76%) of the title compound as a colorless liquid.

Analytical Data: LC-MS: (ES, *m*/*z*): RT=1.301 min, LCMS 53, *m*/*z* =368 [M+1].

### Step 9: Synthesis of benzyl 8-(2-methoxy-5-nitrophenoxy)-2-azaspiro[4.5]decane-2-carboxylate:

Into a 100-mL round-bottom flask, was placed benzyl 8-(methanesulfonyloxy)-2-azaspiro[4.5]decane-2-carboxylate (2.5 g, 6.80 mmol, 1 equiv), 2-methoxy-5-nitrophenol (1.27 g, 7.51 mmol, 1.10 equiv), Cs₂CO₃ (4.5 g, 13.81 mmol, 2.03 equiv), N,N-dimethylformamide (25 mL). The resulting solution was stirred for 5 h at 80 °C. The reaction was then quenched by the addition of 50 mL of water. The resulting solution was extracted with 3x50 mL of ether and the organic layers combined. The resulting mixture was washed with 3x50 mL of H₂O. The mixture was dried over anhydrous sodium sulfate and concentrated under vacuum. The residue was applied onto a silica gel column with ethyl acetate/petroleum ether (2:1). This resulted in 970 mg (31%) of the title compound as a solid.

Analytical Data: LC-MS: (ES, *mlz*): RT=1.486 min, LCMS 53, *m*/*z* =441 [M+1].

### Step 10: Synthesis of 8-(2-methoxy-5-nitrophenoxy)-2-azaspiro[4.5]decane:

Into a 100-mL round-bottom flask, was placed benzyl 8-(2-methoxy-5-nitrophenoxy)-2-azaspiro[4.5]decane-2-carboxylate (900 mg, 2.04 mmol, 1 equiv), trifluoroacetic acid (5 mL). The resulting solution was stirred for 2 h at 60 °C. The resulting mixture was concentrated under vacuum. This resulted in 900 mg (129%) of the title compound as a yellow liquid.

Analytical Data: LC-MS: (ES, *m*/*z*): RT=0.968 min, LCMS 34, *m*/*z* =307 [M+1].

### Step 11: Synthesis of 8-(2-methoxy-5-nitrophenoxy)-2-methyl-2-azaspiro[4.5]decane:

Into a 100-mL round-bottom flask, was placed 8-(2-methoxy-5-nitrophenoxy)-2-azaspiro[4.5]decane (800 mg, 2.61 mmol, 1 equiv), methanol (20 mL), NaBH₃CN (832 mg, 13.24 mmol, 5.07 equiv), HCHO (780 mg). The resulting solution was stirred overnight at RT. The reaction was then quenched by the addition of 20 mL of water. The resulting solution was extracted with 3x50 mL of dichloromethane and the organic layers combined. The resulting mixture was washed with 3x50 mL of H₂O. The mixture was dried over anhydrous sodium sulfate. The crude product was purified by Flash-Prep-HPLC A MeOH. This resulted in 300 mg (32%) of the title compound as a solid.

Analytical Data: LC-MS: (ES, *m*/*z*): RT=0.777 min, LCMS 45, *m*/*z* =321 [M+1].

### Step 12: Synthesis of 4-methoxy-3-([2-methyl-2-azaspiro[4.5]decan-8-yl]oxy)aniline:

Into a 100-mL round-bottom flask, was placed 8-(2-methoxy-5-nitrophenoxy)-2-methyl-2-azaspiro[4.5]decane (300 mg, 0.94 mmol, 1 equiv), methanol (20 mL), Pd/Cl (50 mg), hydrogen (100 mL). The resulting solution was stirred for 2 h at RT. The solids were filtered out. The resulting mixture was concentrated under vacuum. This resulted in 220 mg (73%) of the title compound as a light red liquid.

Analytical Data: LC-MS: (ES, *m*/*z*): RT=0.773 min, LCMS 28, *m*/*z* =291 [M+1].

### Step 13: Synthesis of N2-(4-methoxy-3-((2-methyl-2-azaspiro[4.5]decan-8-yl)oxy)phenyl)-N4-((tetrahydro-2H-pyran-4-yl)methyl)pyrimidine-2,4-diamine:

Into a 10-mL round-bottom flask, was placed 4-methoxy-3-([2-methyl-2-azaspiro[4.5]decan-8-yl]oxy)aniline (120 mg, 0.41 mmol, 1 equiv), 2-chloro-N-(oxan-4-ylmethyl)pyrimidin-4-amine (94 mg, 0.41 mmol, 1 equiv), trifluoroacetic acid (50 mg, 0.44 mmol, 1.07 equiv), isopropanol (5 mL). The resulting solution was stirred for 2 h at 85 °C. The resulting mixture was concentrated under vacuum. The crude product was purified by Prep-HPLC C-HCl. This resulted in 14.0 mg (6%) of N²-(4-methoxy-3-((2-methyl-2-azaspiro[4.5]decan-8-yl)oxy)phenyl)-N⁴-((tetrahydro-2H-pyran-4-yl)methyl)pyrimidine-2,4-diamine as a white solid.

### Example 28: Synthesis of Compound 260 (Reference)

### Compound 260: Synthesis of N²-(4-methoxy-3-((2-methyl-2-azaspiro[3.5]nonan-7-yl)oxy)phenyl)-N⁴-((tetrahydro-2H-pyran-4-yl)methyl)pyrimidine-2,4-diamine

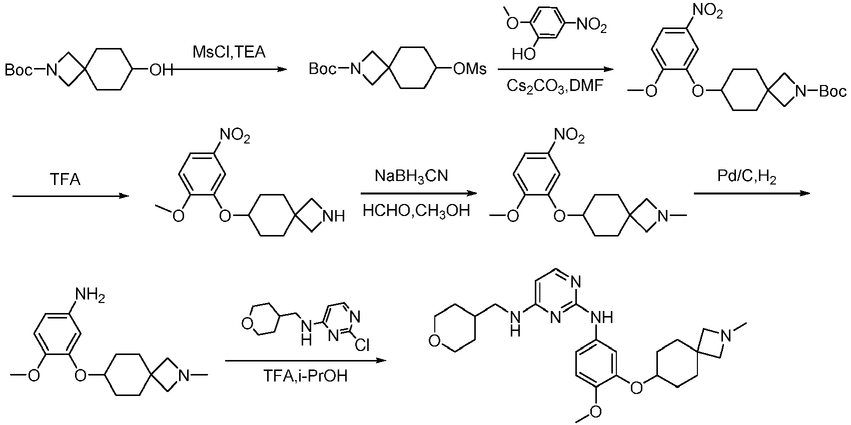

### Step 1: Synthesis of tert-butyl 7-(methanesulfonyloxy)-2-azaspiro[3.5]nonane-2-carboxylate:

Into a 100-mL round-bottom flask, was placed tert-butyl 7-hydroxy-2-azaspiro[3.5]nonane-2-carboxylate (300 mg, 1.24 mmol, 1 equiv), dichloromethane (10 mL), triethylamine (377 mg, 3.73 mmol, 3.00 equiv), methanesulfonyl chloride (286 mg, 2.50 mmol, 2.01 equiv). The resulting solution was stirred for 2 h at RT. The reaction was then quenched by the addition of water. The resulting solution was extracted with 3x50 mL of dichloromethane and the organic layers combined. The resulting mixture was washed with brine. The mixture was dried over anhydrous sodium sulfate and concentrated under vacuum. This resulted in 350 mg (88%) of the title compound as a light yellow solid.

Analytical Data: LC-MS: (ES, *m*/*z*): RT = 1.279min, LCMS 31: *m*/*z*= 320.45[M+1].

### Step 2: Synthesis of tert-butyl 7-(2-methoxy-5-nitrophenoxy)-2-azaspiro[3.5]nonane-2-carboxylate:

Into a 50-mL round-bottom flask, was placed tert-butyl 7-(methanesulfonyloxy)-2-azaspiro[3.5]nonane-2-carboxylate (450 mg, 1.41 mmol, 1 equiv), Cs2CO3 (1.38 g, 4.24 mmol, 3.01 equiv), N,N-dimethylformamide (5 mL), 2-methoxy-5-nitrophenol (358 mg, 2.12 mmol, 1.50 equiv). The resulting solution was stirred for 2 h at 80 °C. The solids were filtered out. The crude product was purified by Flash-Prep-HPLC A Grad. This resulted in 220 mg (40%) of the title compound as a solid.

Analytical Data: LC-MS: (ES, *m*/*z*): RT = 2.215min, LCMS 45: *m*/*z=* 378.20[M+1]. ¹H NMR (300 MHz, Chloroform-d) δ 7.90 (dd, *J* = 9.0, 2.6 Hz, 1H), 7.74 (d, *J* = 2.7 Hz, 1H), 6.92 (d, *J* = 9.0 Hz, 1H), 4.40 - 4.28 (m, 1H), 3.95 (s, 3H), 3.64 (d, *J* = 7.1 Hz, 4H), 2.05 - 1.92 (m, 4H), 1.77 - 1.58 (m, 4H), 1.45 (s, 9H).

### Step 3: Synthesis of 7-(2-methoxy-5-nitrophenoxy)-2-azaspiro[3.5]nonane:

Into a 50-mL round-bottom flask, was placed tert-butyl 7-(2-methoxy-5-nitrophenoxy)-2-azaspiro[3.5]nonane-2-carboxylate (220 mg, 0.56 mmol, 1 equiv), trifluoroacetic acid (5 mL), dichloromethane (10 mL). The resulting solution was stirred for 30 min at RT. The resulting mixture was concentrated under vacuum. This resulted in 150 mg (92%) of the title compound as yellow oil.

Analytical Data: LC-MS: (ES, *mlz):* RT = 0.864 min, LCMS 45: *m*/*z=* 293.10 [M+1]. ¹H NMR (300 MHz, Chloroform-d) δ 10.24 (s, 1H), 7.91 (dd, *J* = 9.0, 1.9 Hz, 1H), 7.75 - 7.71 (m, 1H), 6.93 (d, *J* = 9.0 Hz, 1H), 4.40 - 4.28 (m, 1H), 3.94 (s, 3H), 3.90 - 3.80 (m, 4H), 2.22 - 2.10 (m, 2H), 1.99 - 1.82 (m, 2H), 1.81 - 1.49 (m, 4H).

### Step 4: Synthesis of 7-(2-methoxy-5-nitrophenoxy)-2-methyl-2-azaspiro[3.5]nonane:

Into a 50-mL round-bottom flask, was placed 7-(2-methoxy-5-nitrophenoxy)-2-azaspiro[3.5]nonane (150 mg, 0.51 mmol, 1 equiv), HCHO (23 mg), methanol (5 mL), NaBH₃CN (162 mg, 2.58 mmol, 5.02 equiv). The resulting solution was stirred for 1 h at RT. The reaction was then quenched by the addition of water. The resulting solution was extracted with 3x50 mL of dichloromethane and the organic layers combined. The resulting mixture was washed with brine. The mixture was dried over anhydrous sodium sulfate and concentrated under vacuum. This resulted in 100 mg (64%) of the title compound as a light yellow solid.

Analytical Data: LC-MS: (ES, *m*/*z*): RT = 0.817min, LCMS 45: *m*/*z=* 307.15 [M+1]. ¹H NMR (300 MHz, Chloroform-d) δ 8.00 - 7.88 (m, 1H), 7.76 (d, *J* = 2.7 Hz, 1H), 6.94 (d, *J* = 9.0 Hz, 1H), 4.40 - 4.31 (m, 1H), 3.96 (s, 3H), 3.44 (d, *J=* 5.7 Hz, 4H), 2.62 (s, 3H), 2.20 - 2.04 (m, 2H), 2.02 - 1.88 (m, 2H), 1.80 - 1.61 (m, 4H).

### Step 5: Synthesis of 4-methoxy-3-([2-methyl-2-azaspiro[3.5]nonan-7-yl]oxy)aniline:

Into a 50-mL round-bottom flask purged and maintained with H₂, was placed 7-(2-methoxy-5-nitrophenoxy)-2-methyl-2-azaspiro[3.5]nonane (100 mg, 0.33 mmol, 1 equiv), Pd/C(20 mg), methanol (10 mL). The resulting solution was stirred for 1 h at RT. The solids were filtered out. The resulting mixture was concentrated under vacuum. This resulted in 80 mg (89%) of the title compound as a light yellow liquid.

Analytical Data: LC-MS: (ES, *m*/*z*): RT = 0.734min, LCMS 15: *m*/*z=* 277.10 [M+1]. ¹H NMR (300 MHz, Chloroform-d) δ 6.72 (d, *J* = 8.4 Hz, 1H), 6.38 - 6.22 (m, 2H), 4.19 - 4.08 (m, 1H), 3.77 (s, 3H), 3.31 (d, *J=* 11.4 Hz, 4H), 2.53 (s, 3H), 2.16 - 2.01 (m, 2H), 1.96 - 1.81 (m, 2H), 1.73 - 1.52 (m, 4H).

### Step 6: Synthesis of N2-(4-methoxy-3-((2-methyl-2-azaspiro[3.5]nonan-7-yl)oxy)phenyl)-N4-((tetrahydro-2H-pyran-4-yl)methyl)pyrimidine-2,4-diamine:

Into a 50-mL round-bottom flask, was placed 4-methoxy-3-([2-methyl-2-azaspiro[3.5]nonan-7-yl]oxy)aniline (75 mg, 0.27 mmol, 1 equiv), isopropanol (5 mL), trifluoroacetic acid (62 mg, 0.54 mmol, 2.00 equiv), 2-chloro-N-(oxan-4-ylmethyl)pyrimidin-4-amine (62 mg, 0.27 mmol, 1 equiv). The resulting solution was stirred for 2 h at 80 °C. The crude product was purified by Prep-HPLC F. This resulted in 86.1 mg (63%) of N²-(4-methoxy-3-((2-methyl-2-azaspiro[3.5]nonan-7-yl)oxy)phenyl)-N⁴-((tetrahydro-2H-pyran-4-yl)methyl)pyrimidine-2,4-diamine as a solid.

### Example 29: Synthesis of Compound 261

### Compound 261: Synthesis of 6-methoxy-N²-(4-methoxy-3-(3-(pyrrolidin-1-yl)propoxy)phenyl)-N⁴-methylpyrimidine-2,4-diamine

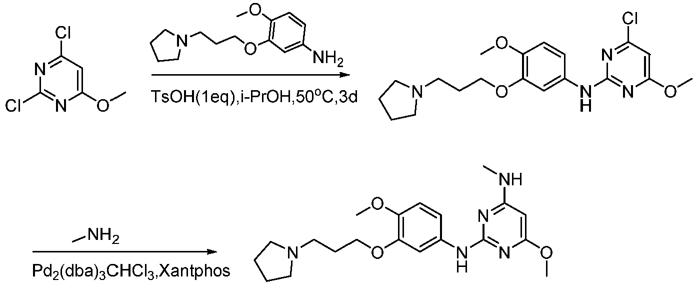

### Step 1: Synthesis of 4-chloro-6-methoxy-N-[4-methoxy-3-[3-(pyrrolidin-1-yl)propoxy]phenyl]pyrimidin-2-amine:

Into a 50-mL round-bottom flask, was placed 4-methoxy-3-[3-(pyrrolidin-1-yl)propoxy]aniline (800 mg, 3.20 mmol, 1 equiv), 2,4-dichloro-6-methoxypyrimidine (573 mg, 3.20 mmol, 1 equiv), TsOH (608 mg, 3.20 mmol, 1 equiv), isopropanol (10 mL). The resulting solution was stirred for 3d at 50 °C in an oil bath. The resulting mixture was concentrated under vacuum. The residue was purified by flash chromatography with H₂O/ACN/NH₄HCO₃. This resulted in 120 mg (10%) as an oil.

Analytical Data: LC-MS: (ES, *m*/*z*): RT = 1.113 min, LCMS 28: m/z =393 [M+1].

### Step 2: Synthesis of 6-methoxy-N²-(4-methoxy-3-(3-(pyrrolidin-1-yl)propoxy)phenyl)-N⁴-methylpyrimidine-2,4-diamine:

Into a 100-mL round-bottom flask purged and maintained with an inert atmosphere of nitrogen, was placed 4-chloro-6-methoxy-N-[4-methoxy-3-[3-(pyrrolidin-1-yl)propoxy]phenyl]pyrimidin-2-amine (350 mg, 0.89 mmol, 1 equiv), Methylamine 2M in tetrahydrofuran (0.9 mg, 2.00 equiv), Pd₂(dba)₃CHCl₃ (93 mg, 0.10 equiv), BINAP (111 mg, 0.18 mmol, 0.20 equiv), t-BuONa (256 mg, 2.66 mmol, 3.00 equiv), Toluene (10 mL). The resulting solution was stirred for overnight at 80 °C in an oil bath under N₂ (g) atmosphere. The resulting mixture was concentrated under vacuum. The solids were filtered out. The residue was purified by flash chromatography with ACN/H₂O(1/10). This resulted in 40.3 mg (12%) of 6-methoxy-N²-(4-methoxy-3-(3-(pyrrolidin-1-yl)propoxy)phenyl)-N⁴-methylpyrimidine-2,4-diamine as an off-white solid.

### Example 30: Synthesis of Compounds 262a and 262b (Reference)

### Compound 262a and 262b: Synthesis of N⁴-methyl-N²-((1R,3S)-3-(3-(pyrrolidin-1-yl)propoxy)cyclohexyl)pyrimidine-2,4-diamine and N⁴-methyl-N²-((1S,3R)-3-(3-(pyrrolidin-1-yl)propoxy)cyclohexyl)pyrimidine-2,4-diamine

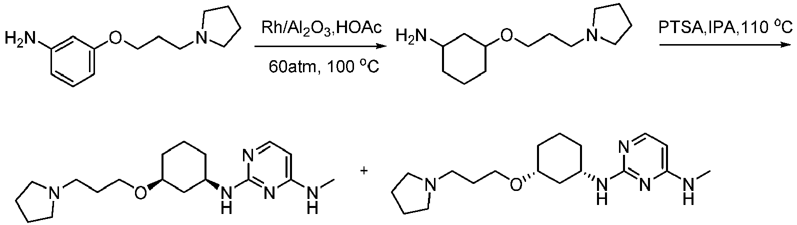

### Step 1: Synthesis of 3-[3-(pyrrolidin-1-yl)propoxy]cyclohexan-1-amine:

Into a 30-mL pressure tank reactor (60 atm), was placed 3-[3-(pyrrolidin-1-yl)propoxy]aniline (500 mg, 2.27 mmol, 1 equiv), acetic acid (15 mL), Rh/Al₂O₃ (0.3 g), hydrogen (1 g). The resulting solution was stirred for 5 h at 100 °C in an oil bath. The solids were filtered out. The resulting mixture was concentrated under vacuum. This resulted in 1.9 g (crude) of as an oil.

Analytical Data: LC-MS: (ES, *m*/*z):* MS = 227 [M+1].

### Step 2: Synthesis of N⁴-methyl-N²-((1R,3S)-3-(3-(pyrrolidin-1-yl)propoxy)cyclohexyl)pyrimidine-2,4-diamine and N⁴-methyl-N²-((1S,3R)-3-(3-(pyrrolidin-1-yl)propoxy)cyclohexyl)pyrimidine-2,4-diamine:

Into a 30-mL pressure tank reactor, was placed 2-chloro-N-methylpyrimidin-4-amine (550 mg, 3.83 mmol, 1.20 equiv), 3-[3-(pyrrolidin-1-yl)propoxy]cyclohexan-1-amine (720 mg, 3.18 mmol, 1 equiv), PTSA (1 g, 5.81 mmol, 2.00 equiv), IPA (10 mL). The resulting solution was stirred for 12 h at 110 °C in an oil bath. The resulting mixture was concentrated under vacuum. The crude product was purified by Flash-Prep-HPLC A MeOH. The crude product 120mg was purified by Chiral-Prep-HPLC This resulted in 42.6 mg (3%) of enantiomer 1 (randomly assigned) as yellow oil and 32.0 mg (2%) enantiomer 2 (randomly assigned) as an oil.

### Example 31: Synthesis of Compound 263

### Compound 263: Synthesis of N²-(4-methoxy-3-(3-(pyrrolidin-1-yl)propoxy)phenyl)-N⁴-methylquinazoline-2,4-diamine

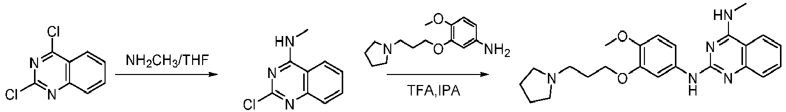

### Step 3: Synthesis of 2-chloro-N-methylquinazolin-4-amine:

Into a 50-mL round-bottom flask, was placed 2,4-dichloroquinazoline (1 g, 5.02 mmol, 1 equiv), tetrahydrofuran (10 mL), TEA (772 mg, 7.63 mmol, 1.50 equiv), CH₃NH₂.THF (7.5 mL, 3.00 equiv). The resulting solution was stirred for 1 h at 0 °C in a water/ice bath. The resulting mixture was concentrated under vacuum. The residue was applied onto a silica gel column with CH₃CN/H₂O (1:7). This resulted in 900 mg (93%) of the title compound as a white solid.

Analytical Data: LC-MS: (ES, *m*/*z*): RT = 1.33min, LCMS33: m/z = 194 [M+1]. ¹H NMR (400 MHz, Methanol-d4) δ 8.08 - 8.00 (m, 1H), 7.78 (m, 1H), 7.63-7.61 (m, 1H), 7.54-7.49 (m, 1H), 3.13 (s, 3H).

### Step 4: Synthesis of N²-(4-methoxy-3-(3-(pyrrolidin-1-yl)propoxy)phenyl)-N⁴-methylquinazoline-2,4-diamine:

Into a 25-mL round-bottom flask, was placed 2-chloro-N-methylquinazolin-4-amine (300 mg, 1.55 mmol, 1 equiv), trifluoroacetic acid (354.4 mg, 3.14 mmol, 2.00 equiv), 4-methoxy-3-[3-(pyrrolidin-1-yl)propoxy] aniline (388.6 mg, 1.55 mmol, 1 equiv), propan-2-ol (5 mL). The resulting solution was stirred for 2 h at 85 °C in an oil bath. The resulting mixture was concentrated under vacuum. The crude product (300 mg) was purified by Prep-HPLC D. This resulted in 64.7 mg (10%) of N²-(4-methoxy-3-(3-(pyrrolidin-1-yl)propoxy)phenyl)-N⁴-methylquinazoline-2,4-diamine as an off-white solid.

### Example 32: Synthesis of Compound 264 (Reference)

### Compound 264: Synthesis of 7-((4-(methylamino)pyrimidin-2-yl)amino)-2-(2-(pyrrolidin-1-yl)ethyl)-3,4-dihydroisoquinolin-1(2H)-one

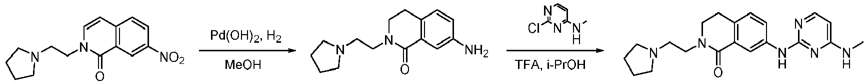

### Step 1: Synthesis of 7-amino-2-[2-(pyrrolidin-1-yl)ethyl]-1,2,3,4-tetrahydroisoquinolin- 1-one:

Into a 100-mL round-bottom flask, was placed 7-nitro-2-[2-(pyrrolidin-1-yl)ethyl]-1,2-dihydroisoquinolin-1-one (100 mg, 0.35 mmol, 1 equiv), methanol (20 mL), Pd(OH₂), hydrogen. The resulting solution was stirred for 16 h at 25 °C. The solids were filtered out. The resulting mixture was concentrated under vacuum. This resulted in 60 mg (66%) of the title compound as an oil.

Analytical Data: LC-MS: (ES, m/z): RT=0.302min, LCMS 31, m/z =260 [M+1].

### Step 2: Synthesis of 7-((4-(methylamino)pyrimidin-2-yl)amino)-2-(2-(pyrrolidin-1-yl)ethyl)-3,4-dihydroisoquinolin-1 (2H)-one:

Into a 25-mL round-bottom flask, was placed 7-amino-2-[2-(pyrrolidin-1-yl)ethyl]-1,2,3,4-tetrahydroisoquinolin-1-one (60 mg, 0.23 mmol, 1 equiv), 2-chloro-N-methylpyrimidin-4-amine (34 mg, 0.24 mmol, 1 equiv), isopropanol (6 mL), trifluoroacetic acid (52 mg, 0.46 mmol, 2.00 equiv). The resulting solution was stirred for 4 h at 85 °C in an oil bath. The resulting mixture was concentrated under vacuum. The crude product was purified by Prep-HPLC C TFA. This resulted in 45.3 mg (41%) of the title compound as a solid.

### Example 33: Synthesis of Compound 265 (Reference)

### Compound 265: Synthesis of N-(4-methoxy-3-(3-(pyrrolidin-1-yl)propoxy)phenyl)-1H-indol-4-amine

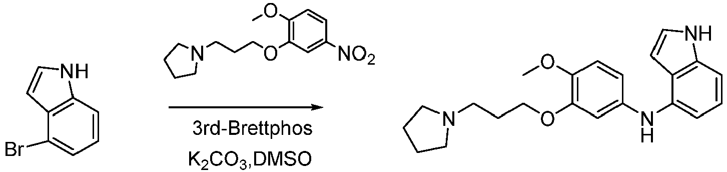

### Step 1: Synthesis of N-(4-methoxy-3-(3-(pyrrolidin-1-yl)propoxy)phenyl)-1H-indol-4-amine:

Into a 50-mL round-bottom flask purged and maintained with an inert atmosphere of nitrogen, was placed 4-bromo-1H-indole (200 mg, 1.02 mmol, 1 equiv), 3rd-BrettPhos (46 mg, 0.05 mmol, 0.05 equiv), potassium methaneperoxoate (283 mg, 2.03 mmol, 2.00 equiv), 4-methoxy-3-[3-(pyrrolidin-1-yl)propoxy] aniline (256.4 mg, 1.02 mmol, 1 equiv), DMSO (5 mL). The resulting solution was stirred for 6 h at 85 °C in an oil bath. The resulting solution was diluted with 10 mL of H₂O. The pH value of the solution was adjusted to 8 with sodium carbonate. The resulting solution was extracted with 3x10 mL of dichloromethane and the organic layers combined. HCl (aq) was employed to adjust the pH to 4. The resulting mixture was washed with 3x10 mL of H₂O. The resulting mixture was concentrated under vacuum. The crude product (200 mg) was purified by Prep-HPLC C TFA. This resulted in 91.9 mg (19%) of N-(4-methoxy-3-(3-(pyrrolidin-1-yl)propoxy)phenyl)-1H-indol-4-amine as a white solid.

### Example 34: Synthesis of Compound 266 (Reference)

### Compound 266: Synthesis of N-(4-methoxy-3-(3-(pyrrolidin-1-yl)propoxy)phenyl)-1H-pyrrolo[3,2-c]pyridin-4-amine

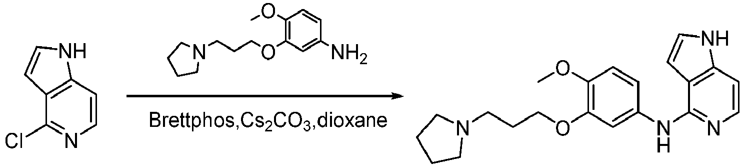

### Step 1: Synthesis of N-[4-methoxy-3-[3-(pyrrolidin-1-yl)propoxy]phenyl]-1H-pyrrolo[3,2-c]pyridin-4-amine:

Into a 20-mL vial, was placed dioxane (2 mL), 4-chloro-1H-pyrrolo[3,2-c]pyridine (200 mg, 1.31 mmol, 1 equiv), 4-methoxy-3-[3-(pyrrolidin-1-yl)propoxy]aniline (329 mg, 1.31 mmol, 1 equiv), Brettphos (230 mg), Cs₂CO₃ (781 mg, 2.40 mmol, 1.83 equiv). The vial was purged and maintained with N₂. The resulting solution was stirred for 12 h at 100°C. The resulting mixture was concentrated under vacuum. The crude product was purified by Chiral-Prep-HPLC D TFA. This resulted in 74.1 mg (12%) of N-(4-methoxy-3-(3-(pyrrolidin-1-yl)propoxy)phenyl)-1H-pyrrolo[3,2-c]pyridin-4-amine as a an solid.

### Example 35: Synthesis of Compound 267 (Reference)

### Compound 267: Synthesis of N⁴-methyl-N²-(6-((2-(pyrrolidin-1-yl)ethoxy)methyl)pyridin-2-yl)pyridine-2,4-diamine

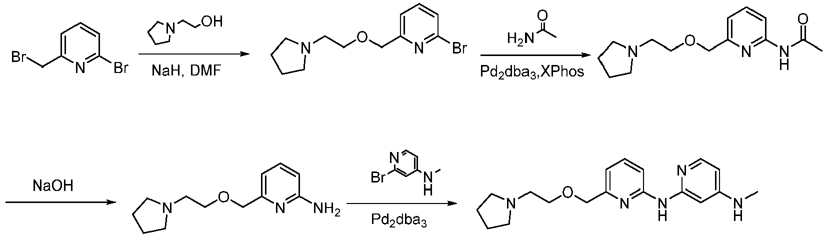

### Step 1: Synthesis of 2-bromo-6-[[2-(pyrrolidin-1-yl)ethoxy]methyl]pyridine:

Into a 250-mL round-bottom flask, was placed 2-bromo-6-(bromomethyl)pyridine (2 g, 7.97 mmol, 1 equiv), sodium hydride (956 mg, 39.83 mmol, 5.00 equiv), N,N-dimethylformamide (80 mL), 2-(pyrrolidin-1-yl)ethan-1-ol (1.1 g, 9.55 mmol, 1.20 equiv). The resulting solution was stirred for 1 h at 0 °C in a water/ice bath. The resulting solution was extracted with 3x100 mL of ethyl acetate and the organic layers combined. The resulting mixture was washed with 3x100 mL of brine. The mixture was dried over anhydrous sodium sulfate. The crude product was purified by Flash-Prep-HPLC A 1:1. This resulted in 910 mg (40%) of the title compound as colorless oil.

Analytical Data: LC-MS: (ES, *m*/*z*): RT= 0.85 min, LCMS 34: m/z = 285 [M+1].

### Step 2: Synthesis of N-(6-[[2-(pyrrolidin-1-yl)ethoxy]methyl]pyridin-2-yl)acetamide:

Into a 250-mL round-bottom flask, was placed 2-bromo-6-[[2-(pyrrolidin-1-yl)ethoxy]methyl]pyridine (910 mg, 3.19 mmol, 1 equiv), X-phos (100 mg), Cs₂CO₃ (3.134 g, 9.62 mmol, 3.00 equiv), dioxane (10 mL), Pd₂(dba)₃.CHCl₃ (100 mg), acetamide (567 mg, 9.60 mmol, 3.00 equiv). The resulting solution was stirred for 2 h at 80 °C in an oil bath. The crude product was purified by Flash-Prep-HPLC A 1:1. This resulted in 460 mg (55%) of the title compound as yellow oil.

Analytical Data: LC-MS: (ES, *m*/*z*): RT= 0.72 min, LCMS 28: m/z = 264 [M+1].

### Step 3: Synthesis of 6-[[2-(pyrrolidin-1-yl)ethoxy]methyl]pyridin-2-amine:

Into a 100-mL round-bottom flask, was placed N-(6-[[2-(pyrrolidin-1-yl)ethoxy]methyl]pyridin-2-yl)acetamide (460 mg, 1.75 mmol, 1 equiv), sodiumol (350 mg, 8.75 mmol, 5.00 equiv), methanol (20 mL), water(20 mL). The resulting solution was stirred for 12 h at 70 °C. The crude product was purified by Flash-Prep-HPLC A 1:1. This resulted in 230 mg (59%) of the title compound as colorless oil.

Analytical Data: LC-MS: (ES, *m*/*z):* RT= 0.62 min, LCMS 53: m/z = 222 [M+1].

### Step 4: Synthesis of N⁴-methyl-N²-(6-((2-(pyrrolidin-1-yl)ethoxy)methyl)pyridin-2-yl)pyridine-2,4-diamine:

Into a 50-mL round-bottom flask, was placed 2-bromo-N-methylpyridin-4-amine (180 mg, 0.96 mmol, 1 equiv), 6-[[2-(pyrrolidin-1-yl)ethoxy]methyl]pyridin-2-amine (255.3 mg, 1.15 mmol, 1.20 equiv), Cs₂CO₃ (939 mg, 2.88 mmol, 3.00 equiv), Pd₂dba₃-CHCl₃ (10 mg), X-phos (10 mg), 1,4-dioxane (10 mL). The resulting solution was stirred for 10 h at 100 °C. The crude product was purified by Flash-Prep-HPLC A 1:1.This resulted in 39.3 mg (11%) of N⁴-methyl-N²-(6-((2-(pyrrolidin-1-yl)ethoxy)methyl)pyridin-2-yl)pyridine-2,4-diamine as a light yellow solid.

### Example 36: Synthesis of Compound 268 (Reference)

### Compound 268: Synthesis of N²-methyl-N⁴-(6-((2-(pyrrolidin-1-yl)ethoxy)methyl)pyridin-2-yl)pyridine-2,4-diamine

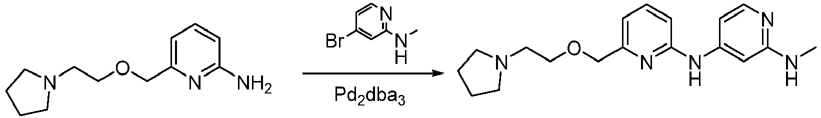

### Step 1: Synthesis of N²-methyl-N⁴-(6-((2-(pyrrolidin-1-yl)ethoxy)methyl)pyridin-2-yl)pyridine-2,4-diamine:

Into a 50-mL round-bottom flask, was placed 6-[[2-(pyrrolidin-1-yl)ethoxy]methyl]pyridin-2-amine (220 mg, 0.99 mmol, 1 equiv), 4-bromo-N-methylpyridin-2-amine (224 mg, 1.20 mmol, 1.20 equiv), Cs₂CO₃ (978 mg, 3.00 mmol, 3.00 equiv), Pd₂dba₃-CHCl₃ (50 mg), Xantphos (50 mg), 1,4-dioxane (10 mL). The resulting solution was stirred for 4 h at 100 °C. The crude product was purified by Flash-Prep-HPLC with the following conditions (IntelFlash-1): Column, silica gel; mobile phase, ACN/H2O=1/1; Detector, UV 254 nm. This resulted in 56.5 mg (17%) of N²-methyl-N⁴-(6-((2-(pyrrolidin-1-yl)ethoxy)methyl)pyridin-2-yl)pyridine-2,4-diamine as a solid.

### Example 37: Synthesis of Compound 272 (Reference)

### Compound 272: Compound Synthesis of 5-fluoro-N²-(4-methoxy-3-(3-(pyrrolidin-1-yl)propoxy)phenyl)-N⁴-methylpyridine-2,4-diamine

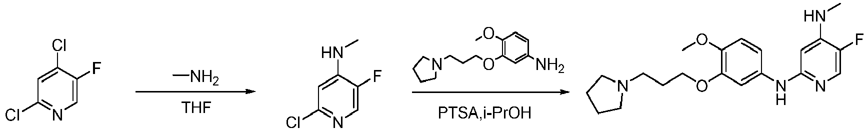

### Step 1: Synthesis of 2-chloro-5-fluoro-N-methylpyridin-4-amine:

Into a 20-mL vial, was placed tetrahydrofuran (8 mL), 2,4-dichloro-5-fluoropyridine (300 mg, 1.81 mmol, 1 equiv), a solution of methanamine (113 mg, 3.64 mmol, 2.01 equiv) in tetrahydrofuran (1.82 mL). The resulting solution was stirred for 18 h at 80 °C. The resulting mixture was concentrated under vacuum. The residue was applied onto a silica gel column with ethyl acetate/petroleum ether (1:10). This resulted in 200 mg (69%) of as a white solid.

Analytical Data: LC-MS: (ES, *m*/*z*): RT = 0.469 min, LCMS 32: *m*/*z* = 161 [M+1].

### Step 2: Synthesis of 5-fluoro-N²-(4-methoxy-3-(3-(pyrrolidin-1-yl)propoxy)phenyl)-N⁴-methylpyridine-2,4-diamine:

Into a 40-mL vial purged and maintained with an inert atmosphere of nitrogen, was placed toluene (10 mL), 2-chloro-5-fluoro-N-methylpyridin-4-amine (190 mg, 1.18 mmol, 1 equiv), 4-methoxy-3-[3-(pyrrolidin-1-yl)propoxy]aniline (327 mg, 1.31 mmol, 1.10 equiv), Pd₂(dba)₃-CHCl₃ (184 mg, 0.18 mmol, 0.15 equiv), BINAP (222 mg, 0.36 mmol, 0.30 equiv), t-BuONa (342 mg, 3.56 mmol, 3.01 equiv). The resulting solution was stirred for 13 h at 100 °C. The resulting mixture was concentrated under vacuum. The residue was applied onto a silica gel column with Water (0.05% HCl)/ACN (5:1). This resulted in 89.1 mg (18%) of 5-fluoro-N²-(4-methoxy-3-(3-(pyrrolidin-1-yl)propoxy)phenyl)-N⁴-methylpyridine-2,4-diamine as a light yellow solid.

### Example 38: Synthesis of Compound 276 (Reference)

### Compound 276: Synthesis of N²-(3-(2-fluoro-3-(pyrrolidin-1-yl)propoxy)phenyl)-N⁴-methylpyrimidine-2,4-diamine

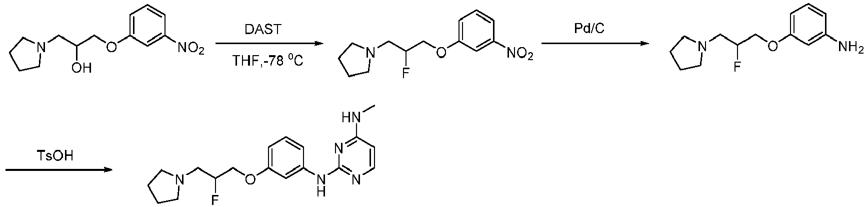

### Step 1: Synthesis of 1-[2-fluoro-3-(3-nitrophenoxy)propyl]pyrrolidine:

Into a 50-mL 3-necked round-bottom flask, was placed 1-(3-nitrophenoxy)-3-(pyrrolidin-1-yl)propan-2-ol (500 mg, 1.88 mmol, 1 equiv), dichloromethane (15 mL). This was followed by the addition of a solution of DAST (363 mg, 2.25 mmol, 1.20 equiv) in dichloromethane (3 mL) dropwise with stirring at -78 °C in 1 min. The resulting solution was stirred overnight at RT. The reaction was then quenched by the addition of 5M/M mL of water. The resulting solution was extracted with 3x10 mL of dichloromethane and the aqueous layers combined and concentrated under vacuum. This resulted in 400 mg (71%) of the title compound as a light yellow solid.

Analytical Data: LC-MS: (ES, *m*/*z*): RT=0.950 min, LCMS 31, *m*/*z* =269.0 [M+1].

### Step 2: Synthesis of 3-[2-fluoro-3-(pyrrolidin-1-yl)propoxy]aniline:

Into a 100-mL round-bottom flask, was placed 1-[2-fluoro-3-(3-nitrophenoxy)propyl]pyrrolidine (400 mg, 1.49 mmol, 1 equiv), methanol (5 mL), hydrogen (100 mL), Pd/C (100 mg). The resulting solution was stirred for 2 h at RT. The solids were filtered out. The resulting mixture was concentrated under vacuum. This resulted in 370 mg (104%) of the title compound as a yellow liquid.

LC-MS: (ES, *mlz):* RT=1.040 min, LCMS 34, *m*/*z* =239.0 [M+1]. ¹H NMR: (300 MHz, Chloroform-d) δ 7.08 (t, *J=* 8.0 Hz, 1H), 6.43 - 6.25 (m, 3H), 4.83 (d, *J* = 4.4 Hz, 1H), 4.75 - 4.63 (m, 1H), 4.28 - 4.07 (m, 2H), 3.69 (s, 2H), 3.18 - 2.69 (m, 5H), 2.01 - 1.77 (m, 4H).

### Step 3: Synthesis of N²-(3-(2-fluoro-3-(pyrrolidin-1-yl)propoxy)phenyl)-N⁴-methylpyrimidine-2,4-diamine:

Into a 8-mL round-bottom flask, was placed 2-chloro-N-methylpyrimidin-4-amine (120 mg, 0.84 mmol, 1 equiv), 3-[2-fluoro-3-(pyrrolidin-1-yl)propoxy]aniline (80 mg, 0.34 mmol, 0.40 equiv), trifluoroacetic acid (0.2 mL), isopropanol (3 mL). The resulting solution was stirred overnight at 85 °C. The crude product was purified by Prep-HPLC C TFA. This resulted in 37.4 mg (11%) of N²-(3-(2-fluoro-3-(pyrrolidin-1-yl)propoxy)phenyl)-N⁴-methylpyrimidine-2,4-diamine as a white solid.

### Example 39: Synthesis of Compound 277 (Reference)

### Compound 277: Synthesis of N²-(3-(2,2-difluoro-3-(pyrrolidin-1-yl)propoxy)phenyl)-N⁴-methylpyrimidine-2,4-diamine

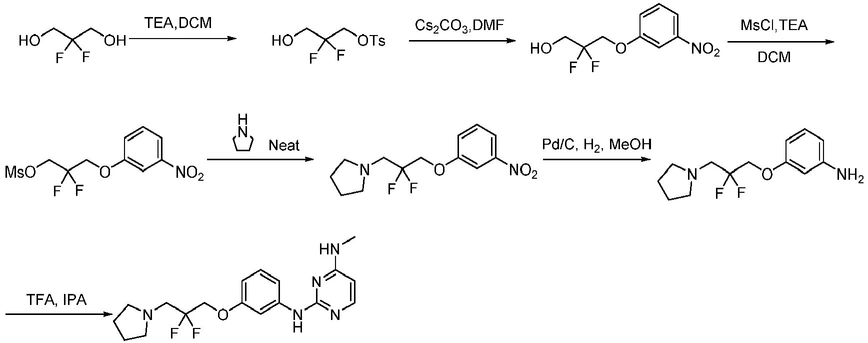

### Step 1: Synthesis of 2,2-difluoro-3-[[(4-methylbenzene)sulfonyl]oxy]propan-1-ol:

Into a 100-mL round-bottom flask, was placed 2,2-difluoropropane-1,3-diol (600 mg, 5.35 mmol, 1 equiv), TEA (1.4 g, 13.84 mmol, 3.00 equiv), dichloromethane (50 mL), 4-methylbenzene-1-sulfonyl chloride (1.02 g, 5.35 mmol, 1 equiv). The resulting solution was stirred for 12 h at 25 °C. The resulting solution was extracted with 3x100 mL of dichloromethane and the organic layers combined. The resulting mixture was washed with 3x100 mL of Brine. The mixture was dried over anhydrous sodium sulfate and concentrated under vacuum. The crude product was purified by Flash-Prep-HPLC A 1:1. This resulted in 500 mg (35%) of the title compound as yellow oil.

Analytical Data: LC-MS: (ES, *m*/*z*): R: 1.19 min, 267 [M+1].

### Step 2: Synthesis of 2,2-difluoro-3-(3-nitrophenoxy)propan-1-ol:

Into a 100-mL round-bottom flask, was placed 2,2-difluoro-3-[[(4-methylbenzene)sulfonyl]oxy]propan-1-ol (550 mg, 2.07 mmol, 1 equiv), Cs2CO3 (2 g, 6.14 mmol, 3.00 equiv), N,N-dimethylformamide (50 mL), 3-nitrophenol (431 mg, 3.10 mmol, 1.50 equiv). The resulting solution was stirred for 12 h at 100 °C in an oil bath. The resulting solution was extracted with 3x100 mL of ethyl acetate and the organic layers combined. The resulting mixture was washed with 3x100 mL of brine. The mixture was dried over anhydrous sodium sulfate and concentrated under vacuum. The crude product was purified by Flash-Prep-HPLC A 1:1. This resulted in 60 mg (12%) of the title compound as yellow oil.

Analytical Data: LC-MS: (ES, *m*/*z*): R: 1.086 min, 234 [M+1]. ¹H-NMR:
(Chloroform-*d*, *ppm*): δ 7.91-7.92 (m, 1H), 7.80 (t, *J* = 2.4 Hz, 1H), 7.50 (t, *J* = 8.2 Hz, 1H), 7.31-7.33 (m, 1H), 4.38 (t, *J* = 11.6 Hz, 2H), 4.03 (t, *J* = 12.5 Hz, 2H).

### Step 3: Synthesis of 2,2-difluoro-3-(3-nitrophenoxy)propyl methanesulfonate:

Into a 100-mL round-bottom flask, was placed 2,2-difluoro-3-(3-nitrophenoxy)propan-1-ol (50 mg, 0.21 mmol, 1 equiv), MsCl (37 mg, 1.50 equiv), TEA (65 mg, 0.64 mmol, 3.00 equiv), dichloromethane (50 mL). The resulting solution was stirred for 2 h at 25 °C. The resulting solution was extracted with 3x100 mL of dichloromethane and the organic layers combined. The resulting mixture was washed with 3x50 mL of brine. The mixture was dried over anhydrous sodium sulfate and concentrated under vacuum. This resulted in 60 mg (90%) of the title compound as yellow oil.

Analytical Data: LC-MS: (ES, *m*/*z*): R: 1.266 min, 312 [M+1]. ¹H-NMR: (DMSO-*d₆*, ppm): δ 7.96 - 7.85 (m, 2H), 7.71 - 7.49 (m, 2H), 4.82 - 4.57 (m, 4H), 3.33 (s, 3H).

### Step 4: Synthesis of 1-[2,2-difluoro-3-(3-nitrophenoxy)propyl]pyrrolidine:

Into a 20-mL sealed tube, was placed 2,2-difluoro-3-(3-nitrophenoxy)propyl methanesulfonate (60 mg, 0.19 mmol, 1 equiv), pyrrolidine (10 mL). The resulting solution was stirred for 12 h at 80 °C in an oil bath. The resulting mixture was concentrated under vacuum. The resulting solution was extracted with 3x100 mL of ethyl acetate and the organic layers combined. The resulting mixture was washed with 3x50 mL of brine. The mixture was dried over anhydrous sodium sulfate. This resulted in 50 mg (91%) of as yellow oil.

Analytical Data: LC-MS: (ES, *m*/*z*): 287 [M+1], R: 0.962 min.

### Step 5: Synthesis of 3-[2,2-difluoro-3-(pyrrolidin-1-yl)propoxy]aniline:

Into a 100-mL round-bottom flask, was placed 1-[2,2-difluoro-3-(3-nitrophenoxy)propyl]pyrrolidine (50 mg, 0.17 mmol, 1 equiv), Raney-Ni, hydrogen, methanol (10 mL). The resulting solution was stirred for 4 h at 25 °C. The solids were filtered out. The resulting mixture was concentrated under vacuum. This resulted in 40 mg (89%) of as yellow oil.

Analytical Data: LC-MS: (ES, *m*/*z*): 257 [M+1], R: 0.734 min.

### Step 6: Synthesis of N²-(3-(2,2-difluoro-3-(pyrrolidin-1-yl)propoxy)phenyl)-N⁴-methylpyrimidine-2,4-diamine:

Into a 50-mL round-bottom flask, was placed 3-[2,2-difluoro-3-(pyrrolidin-1-yl)propoxy]aniline (40 mg, 0.16 mmol, 1 equiv), trifluoroacetic acid (35 mg, 0.31 mmol, 2.00 equiv), isopropanol (10 mL), 2-chloro-N-methylpyrimidin-4-amine (27 mg, 0.19 mmol, 1.20 equiv). The resulting solution was stirred for 4 h at 80 °C in an oil bath. The resulting mixture was concentrated under vacuum. The crude product was purified by Prep-HPLC F TFA. This resulted in 39.2 mg (53%) of N²-(3-(2,2-difluoro-3-(pyrrolidin-1-yl)propoxy)phenyl)-N⁴-methylpyrimidine-2,4-diamine as a white solid.

### Example 40: Synthesis of Compound 279 (Reference)

### Compound 279: Synthesis of N⁴-methyl-N²-(3-(1-(2-(pyrrolidin-1-yl)ethoxy)ethyl)phenyl) pyrimidine-2,4-diamine

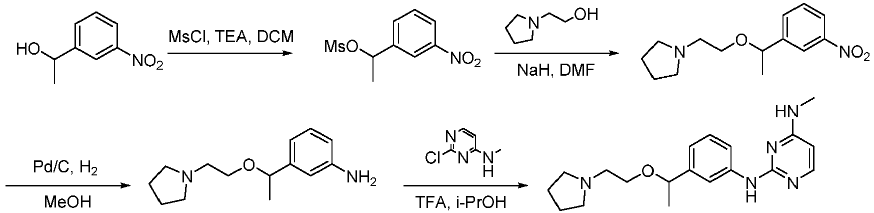

### Step 1: Synthesis of 1-(3-nitrophenyl)ethyl methanesulfonate:

Into a 50-mL 3-necked round-bottom flask purged and maintained with an inert atmosphere of nitrogen, was placed 1-(3-nitrophenyl)ethan-1-ol (1 g, 5.98 mmol, 1 equiv), dichloromethane (15 mL, 1.50 equiv), TEA (1.8 g, 17.79 mmol, 3.00 equiv). This was followed by the addition of MsCl (1.1 g) dropwise with stirring at 0 °C. The resulting solution was stirred for 3 h at 0 °C in a water/ice bath. The reaction was then quenched by the addition of water. The resulting solution was extracted with 3x100 mL of ethyl acetate and the organic layers combined. The resulting mixture was washed with 2x100 mL of water and 2x50 mL of brine. The mixture was dried over anhydrous sodium sulfate and concentrated under vacuum. This resulted in 1.4 g (95%) of the title compound as yellow oil.

Analytical Data: LC-MS: (ES, *m*/*z*): RT=0.801 min. ¹H NMR (300 MHz, DMSO-d6) δ 8.36 - 8.15 (m, 2H), 8.02 - 7.87 (m, 1H), 7.79 - 7.65 (m, 1H), 5.97 (q, *J* = 6.5 Hz, 1H), 3.20 (s, 3H), 1.67 (d, *J=* 6.5 Hz, 3H).

### Step 2: Synthesis of 1-[2-[1-(3-nitrophenyl)ethoxy]ethyl]pyrrolidine:

Into a 100-mL 3-necked round-bottom flask purged and maintained with an inert atmosphere of nitrogen, was placed sodium hydride (1.14 g, 28.50 mmol, 7.00 equiv, 60%), N,N-dimethylformamide (5 mL). This was followed by the addition of a solution of 2-(pyrrolidin-1-yl)ethan-1-ol (2.82 g, 24.48 mmol, 6.00 equiv) in N,N-dimethylformamide (8 mL) dropwise with stirring at -20 °C. The resulting solution was stirred for 0.5 h at -20 °C in an ice/salt bath. To this was added a solution of 1-(3-nitrophenyl)ethyl methanesulfonate (1 g, 4.08 mmol, 1 equiv) in N,N-dimethylformamide (7 mL) dropwise with stirring at -20 °C. The resulting solution was stirred for 1 h at -20 °C in an ice/salt bath. The reaction was then quenched by the addition of water/ice. The resulting solution was extracted with 3x100 mL of ethyl acetate and the organic layers combined. The resulting mixture was washed with 3x50 mL of water and 2x50 mL of brine. The mixture was dried over anhydrous sodium sulfate and concentrated under vacuum. The crude product was purified by Flash-Prep-HPLC A Grad. This resulted in 400 mg (37%) of the title compound as yellow oil.

Analytical Data: LC-MS: (ES, *mlz*): RT=0.740min, LCMS 40, *m*/*z* =265 [M+1]. ¹H NMR (300 MHz, DMSO-*d*₆) δ 8.23 - 8.07 (m, 2H), 7.85 - 7.74 (m, 1H), 7.76 - 7.60 (m, 1H), 4.64 (q, *J* = 6.4 Hz, 1H), 3.55 - 3.39 (m, 1H), 3.41 - 3.25 (m, 1H), 2.68 - 2.30 (m, 6H), 1.73 - 1.54 (m, 4H), 1.37 (d, *J* = 6.5 Hz, 3H).

### Step 3: Synthesis of 3-[1-[2-(pyrrolidin-1-yl)ethoxy]ethyl]aniline:

Into a 100-mL round-bottom flask, was placed 1-[2-[1-(3-nitrophenyl)ethoxy]ethyl]pyrrolidine (430 mg, 1.63 mmol, 1 equiv), methanol (30 mL), Pd/Cl, hydrogen. The resulting solution was stirred for 4 h at 25 °C. The solids were filtered out. The resulting mixture was concentrated under vacuum. This resulted in 370 mg (97%) of the title compound as yellow oil.

Analytical Data: LC-MS: (ES, *mlz*): RT=0.742min, LCMS 45, *m*/*z* =235 [M+1].

### Step 4: Synthesis of N⁴-methyl-N²-(3-(1-(2-(pyrrolidin-1-yl)ethoxy)ethyl)phenyl) pyrimidine-2,4-diamine:

Into a 100-mL round-bottom flask, was placed 3-[1-[2-(pyrrolidin-1-yl)ethoxy]ethyl]aniline (350 mg, 1.49 mmol, 1 equiv), 2-chloro-N-methylpyrimidin-4-amine (214 mg, 1.49 mmol, 1 equiv), isopropanol (20 mL), trifluoroacetic acid (341 mg, 3.02 mmol, 2.00 equiv). The resulting solution was stirred for 3 h at 90 °C in an oil bath. The resulting mixture was concentrated under vacuum. The crude product was purified by Prep-HPLC C TFA. This resulted in 171.5 mg (25%) of N⁴-methyl-N²-(3-(1-(2-(pyrrolidin-1-yl)ethoxy)ethyl)phenyl) pyrimidine-2,4-diamine as a semisolid.

### Example 41: Synthesis of Compound 280 (Reference)

### Compound 280: Synthesis of N²-(3-(3-(diethylamino)propoxy)-4-methoxyphenyl)-N⁴-methylpyrimidine-2,4-diamine

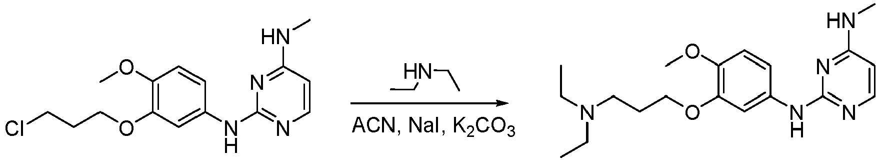

### Step 1: Synthesis of N²-(3-(3-(diethylamino)propoxy)-4-methoxyphenyl)-N⁴-methylpyrimidine-2,4-diamine:

Into a 16-mL sealed tube, was placed 2-N-[3-(3-chloropropoxy)-4-methoxyphenyl]-4-N-methylpyrimidine-2,4-diamine (200 mg, 0.62 mmol, 1 equiv), NaI (100 mg, 1 equiv), potassium carbonate (180 mg, 1.30 mmol, 2.00 equiv), ACN (8 mL), diethylamine (100 mg, 1.37 mmol, 2.00 equiv). The resulting solution was stirred for 3 h at 85 °C in an oil bath. The solids were filtered out. The resulting mixture was concentrated under vacuum. The crude product was purified by Prep-HPLC D HCl. This resulted in 73.7 mg (30%) of N²-(3-(3-(diethylamino)propoxy)-4-methoxyphenyl)-N⁴-methylpyrimidine-2,4-diamine as a light yellow solid.

### Example 42: Synthesis of Compound 283 (Reference)

### Compound 283: Synthesis of N²-(4-methoxy-3-(3-(3-methoxypyrrolidin-1-yl)propoxy)phenyl)-N⁴-methylpyrimidine-2,4-diamine

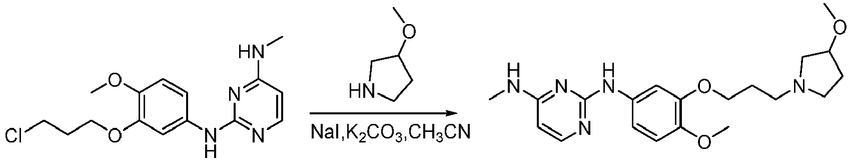

### Step 1: Synthesis of N²-(4-methoxy-3-(3-(3-methoxypyrrolidin-1-yl)propoxy)phenyl)-N⁴-methylpyrimidine-2,4-diamine:

Into a 8-mL round-bottom flask, was placed 2-N-[3-(3-chloropropoxy)-4-methoxyphenyl]-4-N-methylpyrimidine-2,4-diamine (300 mg, 0.93 mmol, 1 equiv), 3-methoxypyrrolidine (303 mg, 3.00 mmol, 3.22 equiv), NaI (150 mg), potassium carbonate (414 mg, 3.00 mmol, 3.22 equiv), CH₃CN (5 mL). The resulting solution was stirred overnight at 70 °C. The crude product was purified by Prep-HPLC C NH3. This resulted in 59.7 mg (17%) of N²-(4-methoxy-3-(3-(3-methoxypyrrolidin-1-yl)propoxy)phenyl)-N⁴-methylpyrimidine-2,4-diamine as a white solid.

### Example 43: Synthesis of Compound 285 (Reference)

### Compound 285: Synthesis of N²-(4-methoxy-3-(3-(3-(trifluoromethyl)pyrrolidin-1-yl)propoxy)phenyl)-N⁴-methylpyrimidine-2,4-diamine

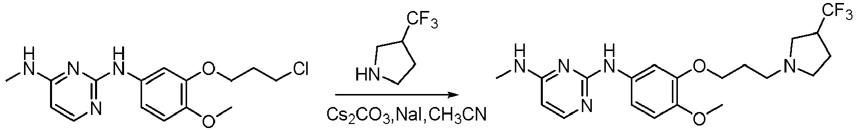

### Step 1: Synthesis of N²-(4-methoxy-3-(3-(3-(trifluoromethyl)pyrrolidin-1-yl)propoxy)phenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (300 mg, 0.93 mmol, 1 equiv), Cs₂CO₃ (911 mg, 2.80 mmol, 3.00 equiv), NaI (13.98 mg, 0.10 equiv), 3-(trifluoromethyl)pyrrolidine (388.5 mg, 2.79 mmol, 3.00 equiv), CH₃CN (6 mL). The resulting solution was stirred for 16 h at 85 °C in an oil bath. The resulting mixture was concentrated under vacuum. The crude product (200 mg) was purified by Prep-HPLC C TFA. This resulted in 88 mg (18%) of t N²-(4-methoxy-3-(3-(3-(trifluoromethyl)pyrrolidin-1-yl)propoxy)phenyl)-N⁴-methylpyrimidine-2,4-diamine as a white solid.

### Example 44: Synthesis of Compound 286 (Reference)

### Compound 286: Synthesis of 1-(3-(2-methoxy-5-((4-(methylamino)pyrimidin-2-yl)amino)phenoxy)propyl)pyrrolidin-3-ol

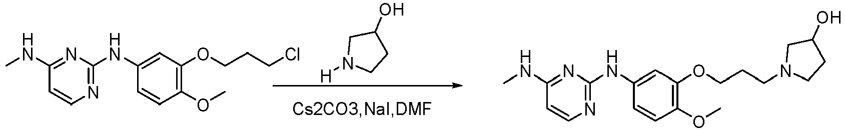

### Step 1: Synthesis of 1-(3-(2-methoxy-5-((4-(methylamino)pyrimidin-2-yl)amino)phenoxy)propyl)pyrrolidin-3-ol:

Into a 20-mL vial, was placed N,N-dimethylformamide (5 mL), 2-N-[3-(3-chloropropoxy)-4-methoxyphenyl]-4-N-methylpyrimidine-2,4-diamine (250 mg, 0.77 mmol, 1 equiv), pyrrolidin-3-ol (135 mg, 1.55 mmol, 2.00 equiv), Cs₂CO₃ (506 mg, 1.55 mmol, 2.01 equiv), NaI (117 mg). The resulting solution was stirred for 2 h at 80 °C. The solids were filtered out. The crude product was purified by Prep-HPLC C NH3. This resulted in 64.7mg (22%) of 1-(3-(2-methoxy-5-((4-(methylamino)pyrimidin-2-yl)amino)phenoxy)propyl)pyrrolidin-3-ol as a white solid.

### Example 45: Synthesis of Compound 287 (Reference)

### Compound 287: Synthesis of 1-(3-(2-methoxy-5-((4-(methylamino)pyrimidin-2-yl)amino)phenoxy)propyl)pyrrolidine-3-carbonitrile

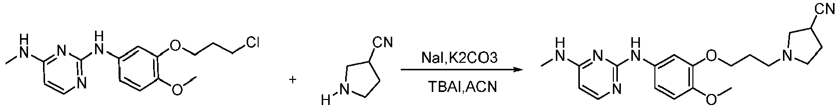

### Step 1: Synthesis of 1-(3-(2-methoxy-5-((4-(methylamino)pyrimidin-2-yl)amino)phenoxy)propyl)pyrrolidine-3-carbonitrile:

Into a 20-mL vial, was placed ACN (3 mL), 2-N-[3-(3-chloropropoxy)-4-methoxyphenyl]-4-N-methylpyrimidine-2,4-diamine (300 mg, 0.93 mmol, 1 equiv), pyrrolidine-3-carbonitrile (98 mg, 1.02 mmol, 1.10 equiv), NaI (140 mg), potassium carbonate (257 mg, 1.86 mmol, 2.00 equiv), TBAI (34 mg, 0.09 mmol, 0.10 equiv). The resulting solution was stirred for 14 h at 80 °C. The residue was applied onto a silica gel column with H2O/ACN (4:1). The collected fractions were combined and concentrated under vacuum. The crude product (100 mg) was purified by Prep-HPLC D HCl. This resulted in 43 mg (11%) of 1-(3-(2-methoxy-5-((4-(methylamino)pyrimidin-2-yl)amino)phenoxy)propyl)pyrrolidine-3-carbonitrile as a white solid.

### Example 46: Synthesis of Compound 288 (Reference)

### Compound 288: Synthesis of N²-(4-methoxy-3-(2-(1-methylpyrrolidin-2-yl)ethoxy)phenyl)-N⁴-methylpyrimidine-2,4-diamine

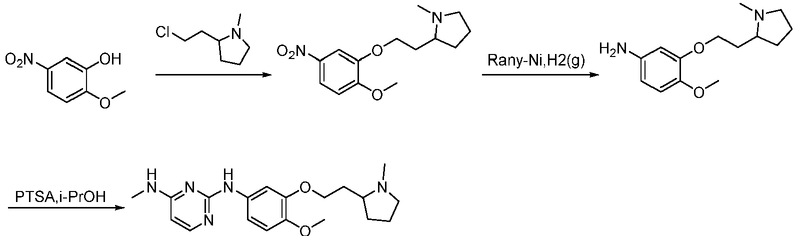

### Step 1: Synthesis of 2-[2-(2-methoxy-5-nitrophenoxy)ethyl]-1-methylpyrrolidine:

Into a 50-mL round-bottom flask, was placed N,N-dimethylformamide (10 mL), 2-methoxy-5-nitrophenol (500 mg, 2.96 mmol, 1 equiv), Cs₂CO₃ (1.93 g, 5.92 mmol, 2.00 equiv), NaI (444 mg, 2.96 mmol, 1 equiv), 2-(2-chloroethyl)-1-methylpyrrolidine (870 mg, 5.89 mmol, 1.99 equiv). The resulting solution was stirred for 2 h at 80 °C. The resulting solution was diluted with 10 mL of H₂O. The resulting solution was extracted with 3x10 mL of ethyl acetate and the organic layers combined. The resulting mixture was washed with 3x10 mL of water and 3x10 mL of brine. The mixture was dried over anhydrous sodium sulfate. The solids were collected by filtration. The resulting mixture was concentrated under vacuum. This resulted in 540 mg (65%) of the title compound as an oil.

Analytical Data: LC-MS: (ES, *mlz):* RT = 0.836 min, LCMS 27: *m*/*z* = 281 [M+1].

### Step 2: Synthesis of 4-methoxy-3-[2-(1-methylpyrrolidin-2-yl)ethoxy]aniline:

Into a 100-mL round-bottom flask, was placed methanol (30 mL), 2-[2-(2-methoxy-5-nitrophenoxy)ethyl]-1-methylpyrrolidine (520 mg, 1.86 mmol, 1 equiv), Rany-Ni (100 mg).The flask was purged and maintained with H₂.The resulting solution was stirred for 3 h at 20 °C. The solids were filtered out. The resulting mixture was concentrated under vacuum. This resulted in 460 mg (99%) of the title compound as a light red oil.

Analytical Data: LC-MS: (ES, *mlz):* RT = 0.398 min, LCMS 32: *m*/*z* = 251 [M+1].

### Step 3: Synthesis of N²-(4-methoxy-3-(2-(1-methylpyrrolidin-2-yl)ethoxy)phenyl)-N⁴-methylpyrimidine-2,4-diamine:

Into a 100-mL round-bottom flask, was placed isopropanol (10 mL), 2-chloro-N-methylpyrimidin-4-amine (252 mg, 1.76 mmol, 1 equiv), 4-methoxy-3-[2-(1-methylpyrrolidin-2-yl)ethoxy]aniline (440 mg, 1.76 mmol, 1 equiv), PTSA (303 mg, 1.76 mmol, 1 equiv). The resulting solution was stirred for 2 h at 85 °C. The crude product (600 mg) was purified by Prep-HPLC D HCl. 310 mg product was obtained. This resulted in 310 mg (45%) of N²-(4-methoxy-3-(2-(1-methylpyrrolidin-2-yl)ethoxy)phenyl)-N⁴-methylpyrimidine-2,4-diamine as light yellow oil.

### Example 47: Synthesis of Compound 289 (Reference)

### Compound 289: Synthesis of N²-(4-methoxy-3-((1-methylpyrrolidin-2-yl)methoxy)phenyl)-N⁴-methylpyrimidine-2,4-diamine

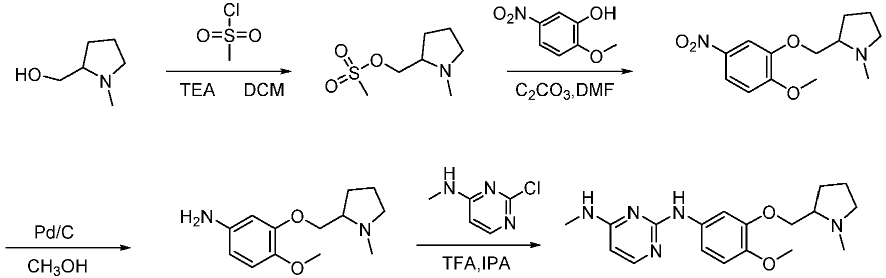

### Step 1: Synthesis of (1-methylpyrrolidin-2-yl)methyl methanesulfonate:

Into a 50-mL round-bottom flask, was placed (1-methylpyrrolidin-2-yl)methanol (1 g, 8.68 mmol, 1 equiv), TEA (2.66 g, 26.29 mmol, 3.00 equiv), dichloromethane (10 mL), methanesulfonyl chloride (1.29 g, 11.26 mmol, 1.30 equiv). The resulting solution was stirred for 1 h at 20 °C. This resulted in 2 g (119%) of the title compound as yellow oil.

### Step 2: Synthesis of 2-(2-methoxy-5-nitrophenoxymethyl)-1-methylpyrrolidine

Into a 50-mL sealed tube, was placed (1-methylpyrrolidin-2-yl)methyl methanesulfonate (1 g, 5.17 mmol, 1 equiv), Cs₂CO₃ (3.75 g, 11.51 mmol, 2.00 equiv), 2-methoxy-5-nitrophenol (876 mg, 5.18 mmol, 1 equiv), N,N-dimethylformamide (10 mL). The resulting solution was stirred for 16 h at 90 °C in an oil bath. The residue was applied onto a silica gel column with H₂O:CH₃CN (1:5). This resulted in 500 mg (36%) of the title compound as yellow oil.

Analytical Data: LC-MS: (ES, *m*/*z*): RT = 0.73min, LCMS40: m/z = 267.25 [M+1].

### Step 3: Synthesis of 4-methoxy-3-[(1-methylpyrrolidin-2-yl)methoxy]aniline:

Into a 50-mL round-bottom flask, was placed 2-(2-methoxy-5-nitrophenoxymethyl)-1-methylpyrrolidine (500 mg, 1.88 mmol, 1 equiv), methanol (20 mL), Pd/C (1 g, 1 equiv), hydrogen. The resulting solution was stirred for 1 h at 20 °C. The solids were filtered out. The resulting mixture was concentrated under vacuum. This resulted in 350 mg (79%) of the title compound as a yellow solid.

Analytical Data: LC-MS: (ES, *m*/*z*): RT = 0.39min, LCMS07: m/z = 237.25 [M+1].

### Step 4: Synthesis of N²-(4-methoxy-3-((1-methylpyrrolidin-2-yl)methoxy)phenyl)-N⁴-methylpyrimidine-2,4-diamine:

Into a 50-mL round-bottom flask, was placed 4-methoxy-3-[(1-methylpyrrolidin-2-yl)methoxy]aniline (350 mg, 1.48 mmol, 1 equiv), trifluoroacetic acid (338 mg, 2.99 mmol, 2.00 equiv), 2-chloro-N-methylpyrimidin-4-amine (212 mg, 1.48 mmol, 1 equiv), propan-2-ol (10 mL). The resulting solution was stirred for 6 h at 85 °C in an oil bath. The resulting mixture was concentrated under vacuum. The crude product (200 mg) was purified by Prep-HPLC C NH3. This resulted in 64.7 mg (13%) of 4-methoxy-3-[(1-methylpyrrolidin-2-yl)methoxy] aniline as an off-white solid.

### Example 48: Synthesis of Compound 290 (Reference)

### Compound 290: Synthesis of N²-(4-methoxy-3-(3-(2-methylpyrrolidin-1-yl)propoxy)phenyl)-N⁴-methylpyrimidine-2,4-diamine

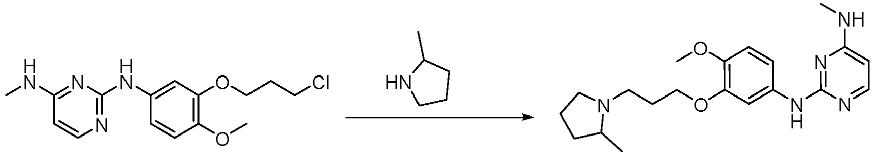

### Step 1: Synthesis of N²-(4-methoxy-3-(3-(2-methylpyrrolidin-1-yl)propoxy)phenyl)-N⁴-methylpyrimidine-2,4-diamine:

Into a 25-mL round-bottom flask, was placed N,N-dimethylformamide (5 mL), 2-N-[3-(3-chloropropoxy)-4-methoxyphenyl]-4-N-methylpyrimidine-2,4-diamine (200 mg, 0.62 mmol, 1 equiv), 2-methylpyrrolidine (53 mg, 0.62 mmol, 1 equiv), Cs₂CO₃ (405 mg, 1.24 mmol, 2.01 equiv), NaI (93 mg, 0.62 mmol, 1 equiv). The resulting solution was stirred for 4 h at 80 °C. The solids were filtered out. The crude product (200 mg) was purified by Prep-HPLC D HCl. 39.7 mg light yellow solid N²-(4-methoxy-3-(3-(2-methylpyrrolidin-1-yl)propoxy)phenyl)-N⁴-methylpyrimidine-2,4-diamine was obtained.

### Example 49: Synthesis of Compound 291 (Reference)

### Compound 291: Synthesis of N-ethyl-4-((4-(methylamino)pyrimidin-2-yl)amino)picolinamide

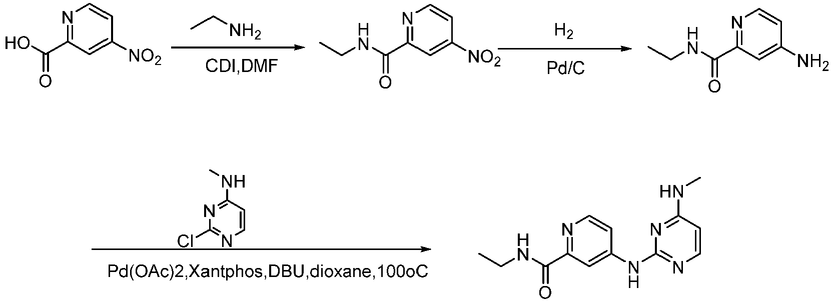

### Step 1: Synthesis of N-ethyl-4-nitropyridine-2-carboxamide:

Into a 50-mL round-bottom flask, was placed 4-nitropyridine-2-carboxylic acid (400 mg, 2.38 mmol, 1 equiv), CDI (582 mg, 3.59 mmol, 1.50 equiv), N,N-dimethylformamide (10 mL), ethanamine (1.2 mL). The resulting solution was stirred for 6 h at 25 °C. The resulting mixture was concentrated under vacuum. The reaction was then quenched by the addition of 10 mL of water. The resulting solution was extracted with 3x50 mL of ethyl acetate and the organic layers combined. This resulted in 464mg (99%) of the title compound as a yellow solid.

Analytical Data: LC-MS: (ES, *m*/*z):* RT = 0.871 min, LCMS 34: m/z = 195 [M+1].

### Step 2: Synthesis of 4-amino-N-ethylpyridine-2-carboxamide:

Into a 50-mL round-bottom flask, was placed N-ethyl-4-nitropyridine-2-carboxamide (464 mg, 2.38 mmol, 1 equiv), Pd/C (156.3 mg), hydrogen. The resulting solution was stirred for 4 h at 25 °C. The solids were collected by filtration. This resulted in 370 mg (94%) of the title compound as a yellow liquid.

Analytical Data: LC-MS: (ES, *m*/*z*): RT = 0.684 min, LCMS 34: m/z = 166 [M+1].

### Step 3: Synthesis of N-ethyl-4-((4-(methylamino)pyrimidin-2-yl)amino)picolinamide:

Into a 50-mL round-bottom flask, was placed 4-amino-N-ethylpyridine-2-carboxamide (200 mg, 1.21 mmol, 1 equiv), 2-chloro-N-methylpyrimidin-4-amine (174 mg, 1.21 mmol, 1 equiv), Xantphos (140.3 mg, 0.24 mmol, 0.20 equiv), DBU (368.5 mg, 2.42 mmol, 2.00 equiv), dioxane (10 mL), Pd(OAc)₂ (27.1 mg, 0.12 mmol, 0.10 equiv). The resulting solution was stirred for 24 h at 100 °C in an oil bath. The resulting solution was extracted with 3x10 mL of water and the organic layers combined. The crude product was purified by (ACN/H₂O=1/20). This resulted in 30.2 mg (8%) of N-ethyl-4-((4-(methylamino)pyrimidin-2-yl)amino)picolinamide as a white solid.

### Example 50: Synthesis of Compound 293 (Reference)

### Compound 293: Synthesis of N²-(4-methoxy-3-(3-(3-methylpyrrolidin-1-yl)propoxy)phenyl)-N⁴-methylpyrimidine-2,4-diamine

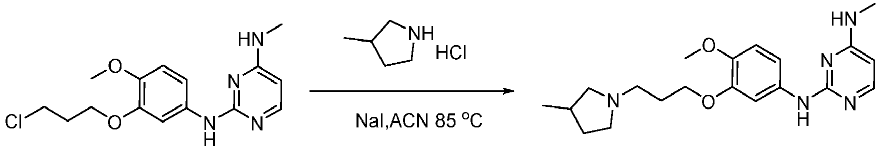

### Step 1: Synthesis of N²-(4-methoxy-3-(3-(3-methylpyrrolidin-1-yl)propoxy)phenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (300 mg, 0.93 mmol, 1 equiv), 3-methylpyrrolidine hydrochloride (112.7 mg, 0.93 mmol, 1 equiv), Cs₂CO₃ (939 mg, 2.88 mmol, 3.00 equiv), NaI (279.5 mg, 2.00 equiv), CH₃CN (10 mL). The mixture solution was stirred for 20 h at 85 °C. The resulting solution was diluted with 20 mL of water and extracted with 3x30 mL of ethyl acetate and the organic layers combined. The crude product was purified by Prep-HPLC C NH₄HCO₃. The resulting solution was stirred for 24 h at 85 °C in an oil bath. This resulted in 39.3mg (11%) of N²-(4-methoxy-3-(3-(3-methylpyrrolidin-1-yl)propoxy)phenyl)-N⁴-methylpyrimidine-2,4-diamine as a white solid.

### Example 51: Synthesis of Compound 298 (Reference)

### Compound 298: Synthesis of N²-(3-(3-(3-azabicyclo[3.1.0]hexan-3-yl)propoxy)-4-methoxyphenyl)-N⁴-methylpyrimidine-2,4-diamine

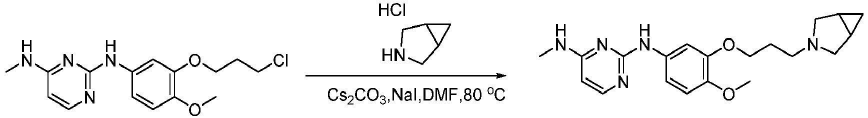

### Step 1: Synthesis of N²-(3-(3-(3-azabicyclo[3.1.0]hexan-3-yl)propoxy)-4-methoxyphenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (300 mg, 0.93 mmol, 1 equiv), 3-azabicyclo[3.1.0]hexane hydrochloride (166.3 mg, 1.39 mmol, 1.50 equiv), Cs₂CO₃ (609 mg, 1.87 mmol, 2.00 equiv), NaI (279 mg, 1.86 mmol, 2.00 equiv), N,N-dimethylformamide (5 mL). The resulting solution was stirred for 12 h at 80 °C in an oil bath. The solids were filtered out. The crude product was purified by Prep-HPLC D NH3. This resulted in 33.5 mg (10%) of N²-(3-(3-(3-azabicyclo[3.1.0]hexan-3-yl)propoxy)-4-methoxyphenyl)-N⁴-methylpyrimidine-2,4-diamine as a white solid.

### Example 52: Synthesis of Compound 299 (Reference)

### Compound 299: Synthesis of (R)-1-(2-methoxy-5-((4-(methylamino)pyrimidin-2-yl)amino)phenoxy)-3-(pyrrolidin-1-yl)propan-2-ol

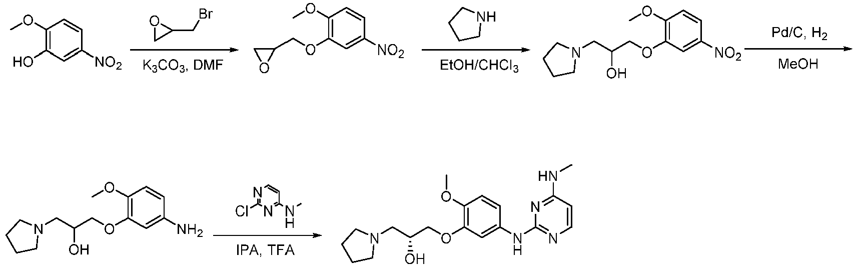

### Step 1: Synthesis of 2-(2-methoxy-5-nitrophenoxymethyl)oxirane:

Into a 250-mL round-bottom flask, was placed 2-methoxy-5-nitrophenol (3.5 g, 20.69 mmol, 1 equiv), 2-(bromomethyl)oxirane (2.84 g, 20.73 mmol, 1 equiv), potassium carbonate (5.7 g, 41.24 mmol, 2.00 equiv), N,N-dimethylformamide (80 mL). The resulting solution was stirred for 16 h at 25 °C. The resulting solution was allowed to react, with stirring, for an additional 3 h while the temperature was maintained at 50 °C in an oil bath. The resulting mixture was washed with 1x100 mL of H2O. The resulting solution was extracted with 3x300 mL of ethyl acetate and the organic layers combined. The resulting mixture was washed with 3x200 mL of water and 2x100 mL of brine. The mixture was dried over anhydrous sodium sulfate and concentrated under vacuum. This resulted in 4.2 g (crude) of the title compound as a yellow solid.

Analytical Data: LC-MS: (ES, m/z): RT=1.02 min. ¹H NMR (300 MHz, DMSO-d6) δ 7.93 (dd, *J* = 9.0, 2.7 Hz, 1H), 7.78 (d, *J* = 2.7 Hz, 1H), 7.20 (d, *J =* 9.0 Hz, 1H), 4.49 (dd, *J* = 11.4, 2.4 Hz, 1H), 3.99 - 3.86 (m, 4H), 3.43 - 3.28 (m, 1H), 2.91 - 2.81 (m, 1H), 2.78 - 2.68 (m, 1H).

### Step 2: Synthesis of 1-(2-methoxy-5-nitrophenoxy)-3-(pyrrolidin-1-yl)propan-2-ol:

Into a 50-mL round-bottom flask, was placed 2-(2-methoxy-5-nitrophenoxymethyl)oxirane (500 mg, 2.22 mmol, 1 equiv), ethanol (10 mL), chloroform (10 mL), pyrrolidine (394 mg, 5.54 mmol, 2.50 equiv). The resulting solution was stirred for 3 h at 60 °C in an oil bath. The resulting mixture was concentrated under vacuum. The crude product was purified by Flash-Prep-HPLC A DCM/MeOH. This resulted in 600 mg (91%) of the title compound as yellow oil.

Analytical Data: LC-MS: (ES, m/z): RT=0.927min, LCMS 31, m/z =297[M+1]. ¹H NMR (400 MHz, DMSO-d6) δ 7.90 (dd, *J* = 9.0, 2.7 Hz, 1H), 7.79 (d, *J* = 2.7 Hz, 1H), 7.18 (d, *J* = 9.0 Hz, 1H), 4.99 (s, 1H), 4.19 - 4.05 (m, 1H), 4.04 - 3.87 (m, 5H), 2.71 - 2.41 (m, 6H), 1.76 - 1.61 (m, 4H).

### Step 3: Synthesis of 1-(5-amino-2-methoxyphenoxy)-3-(pyrrolidin-1-yl)propan-2-ol:

Into a 100-mL round-bottom flask, was placed 1-(2-methoxy-5-nitrophenoxy)-3-(pyrrolidin-1-yl)propan-2-ol (700 mg, 2.36 mmol, 1 equiv), methanol (40 mL), Pd/Cl, hydrogen. The resulting solution was stirred for 16 h at 25 °C. The solids were filtered out. The resulting mixture was concentrated under vacuum. This resulted in 600 mg (95%) of the title compound as yellow oil.

LC-MS: (ES, *mlz*): RT=0.671min, LCMS 31, *m*/*z* =267 [M+1].

### Step 4: Synthesis of (R)-1-(2-methoxy-5-((4-(methylamino)pyrimidin-2-yl)amino)phenoxy)-3-(pyrrolidin-1-yl)propan-2-ol:

Into a 50-mL round-bottom flask, was placed 1-(5-amino-2-methoxyphenoxy)-3-(pyrrolidin-1-yl)propan-2-ol (600 mg, 1 equiv), 2-chloro-N-methylpyrimidin-4-amine (324 mg, 2.26 mmol, 1 equiv), isopropanol (10 mL), trifluoroacetic acid (514 mg, 4.55 mmol, 2.00 equiv). The resulting solution was stirred for 3 h at 90°C in an oil bath. The resulting mixture was concentrated under vacuum. The crude product was purified by Chiral-Prep-HPLC ID. This resulted in 42 mg (5%) of 1-(5-amino-2-methoxyphenoxy)-3-(pyrrolidin-1-yl)propan-2-ol (600 mg, 1 equiv), 2-chloro-N-methylpyrimidin-4-amine as a light yellow solid.

### Example 53: Synthesis of Compound 300 (Reference)

### Compound 300: Synthesis of (S)-1-(2-methoxy-5-((4-(methylamino)pyrimidin-2-yl)amino)phenoxy)-3-(pyrrolidin-1-yl)propan-2-ol

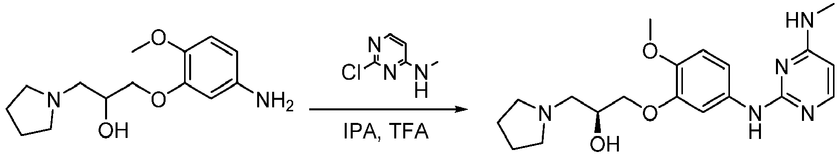

### Step 1: Synthesis of (S)-1-(2-methoxy-5-((4-(methylamino)pyrimidin-2-yl)amino)phenoxy)-3-(pyrrolidin-1-yl)propan-2-ol:

Into a 50-mL round-bottom flask, was placed 1-(5-amino-2-methoxyphenoxy)-3-(pyrrolidin-1-yl)propan-2-ol (600 mg, 2.25 mmol, 1 equiv), 2-chloro-N-methylpyrimidin-4-amine (324 mg, 2.26 mmol, 1 equiv), isopropanol (10 mL), trifluoroacetic acid (514 mg, 4.55 mmol, 2.00 equiv). The resulting solution was stirred for 2 h at 90 °C in an oil bath. The resulting mixture was concentrated under vacuum. The crude product was purified by Chiral-Prep-HPLC IB4. This resulted in 41.1 mg (5%) of (S)-1-(2-methoxy-5-((4-(methylamino)pyrimidin-2-yl)amino)phenoxy)-3-(pyrrolidin-1-yl)propan-2-ol as a light yellow solid.

### Example 54: Synthesis of Compound 301

### Compound 301: Synthesis of N2-(3-fluoro-4-methoxy-5-(3-(pyrrolidin-1-yl)propoxy)phenyl)-N4,6-dimethylpyrimidine-2,4-diamine

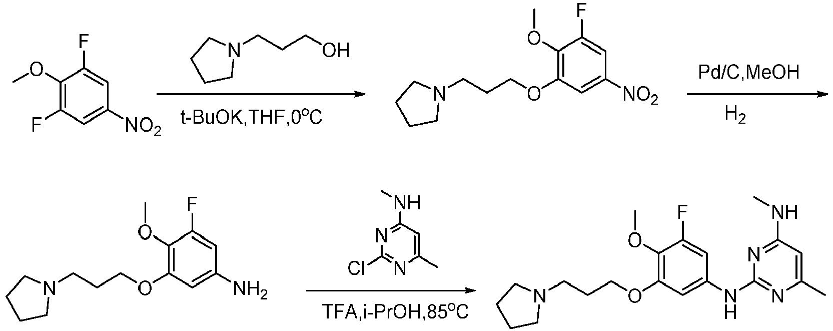

### Step 1: Synthesis of 1-[3-(3-fluoro-2-methoxy-5-nitrophenoxy)propyl]pyrrolidine:

Into a 250-mL round-bottom flask, was placed 1,3-difluoro-2-methoxy-5-nitrobenzene (1 g, 5.29 mmol, 1 equiv), 3-(pyrrolidin-1-yl)propan-1-ol (683 mg, 5.29 mmol, 1 equiv), t-BuOK (10.6 mL, 2.00 equiv), tetrahydrofuran (15 mL). The resulting solution was stirred for 1 h at 0 °C in a water/ice bath. The resulting mixture was concentrated under vacuum. The crude product (5 mL) was purified by ACN/H₂O(1/1).This resulted in 550mg (35%) of as a yellow solid.

Analytical Data: LC-MS: (ES, m/z): RT = 0.986min, LCMS 53: m/z = 299 [M+1]. ¹H NMR (400 MHz, Methanol-d4) δ 7.75 (s, 1H), 7.73 (s, 1H), 4.28 - 4.25 (m, 2H), 4.03 - 3.99 (m, 6H), 2.78 - 2.66 (m, 2H), 2.65 - 2.62 (m, 2H), 2.03 - 1.99 (s, 3H), 1.88 - 1.85 (m, 2H).

### Step 2: Synthesis of 3-fluoro-4-methoxy-5-[3-(pyrrolidin-1-yl)propoxy]aniline:

Into a 250-mL round-bottom flask, was placed 1-[3-(3-fluoro-2-methoxy-5-nitrophenoxy)propyl]pyrrolidine (450 mg, 1.51 mmol, 1 equiv), methanol (10 mL), Pd/C (150 mg), hydrogen. The resulting solution was stirred for 2 h at 25 °C. The solids were filtered out. The resulting mixture was concentrated under vacuum. This resulted in 350 mg (86%) of the title compound as yellow oil.

Analytical Data: LC-MS: (ES, *m*/*z*): RT = 0.803min, LCMS 34: m/z = 269 [M+1].

### Step 3: Synthesis of N²-(3-fluoro-4-methoxy-5-(3-(pyrrolidin-1-yl)propoxy)phenyl)-N⁴,6-dimethylpyrimidine-2,4-diamine:

Into a 100-mL round-bottom flask, was placed 3-fluoro-4-methoxy-5-[3-(pyrrolidin-1-yl)propoxy]aniline (300 mg, 1.12 mmol, 1 equiv), 2-chloro-N,6-dimethylpyrimidin-4-amine (176 mg, 1.12 mmol, 1 equiv), trifluoroacetic acid (255.2 mg, 2.26 mmol, 2.00 equiv), isopropanol (15 mL). The resulting solution was stirred for 24 h at 85 °C in an oil bath. The resulting mixture was concentrated under vacuum. The crude product was purified by Prep-HPLC C NH3. This resulted in 9.7 mg (2%) of N²-(3-fluoro-4-methoxy-5-(3-(pyrrolidin-1-yl)propoxy)phenyl)-N⁴,6-dimethylpyrimidine-2,4-diamine as a yellow solid.

### Example 55: Synthesis of Compound 302

### Compound 302: Synthesis of N²-(2-fluoro-4-methoxy-5-(3-(pyrrolidin-1-yl)propoxy)phenyl)-N⁴,6-dimethylpyrimidine-2,4-diamine

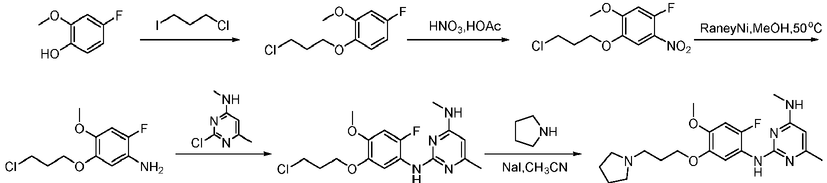

### Step 1: Synthesis 1-(3-chloropropoxy)-4-fluoro-2-methoxybenzene:

Into a 50-mL round-bottom flask, was placed 4-fluoro-2-methoxyphenol (1 g, 7.04 mmol, 1 equiv), 1-chloro-3-iodopropane (2.87 g, 14.04 mmol, 2.00 equiv), potassium carbonate (2.92 g, 21.13 mmol, 3.00 equiv), ACN (15 mL). The resulting solution was stirred for 14 h at 85 °C. The solids were filtered out. The resulting mixture was concentrated under vacuum. This resulted in 1.5 g (98%) of as yellow oil.

### Step 2: Synthesis of 1-(3-chloropropoxy)-4-fluoro-2-methoxy-5-nitrobenzene:

Into a 100-mL round-bottom flask, was placed 1-(3-chloropropoxy)-4-fluoro-2-methoxybenzene (1.53 g, 7.00 mmol, 1 equiv), acetyl acetate (25 mL). This was followed by the addition of HNO₃ (2.56 g, 4.00 equiv) dropwise with stirring at 0 °C. The resulting solution was stirred for 16 h at 20 °C. The reaction was then quenched by the addition of water/ice. The resulting solution was extracted with 2x80 mL of ethyl acetate and the organic layers combined. The resulting mixture was washed with 2x100 mL of sodium bicarbonate and 2x100 mL of brine. The resulting mixture was washed and the filtrate was concentrated under vacuum. The residue was applied onto a silica gel column with ethyl acetate/petroleum ether (1/5). This resulted in 1.62 g (88%) of the title compound as a yellow solid.

Analytical Data: ¹H NMR (400 MHz, Methanol-d4) δ 7.70 (s, 1H), 7.06 (s, 1H), 4.20 (t, *J* = 5.9 Hz, 2H), 3.79 (t, *J* = 6.4 Hz, 2H), 2.26 (q, *J* = 6.1 Hz, 2H), 2.03 (s, 3H).

### Step 3: Synthesis of 5-(3-chloropropoxy)-2-fluoro-4-methoxyaniline:

Into a 100-mL round-bottom flask, was placed 1-(3-chloropropoxy)-4-fluoro-2-methoxy-5-nitrobenzene (200 mg, 0.76 mmol, 1 equiv), RaneyNi (0.1 g), methanol (20 mL). The resulting solution was stirred for 16 h at 50 °C. The solids were filtered out. The resulting mixture was concentrated under vacuum. This resulted in 170 mg (96%) of the title compound as a brown oil.

Analytical Data: LC-MS: (ES, *m*/*z*): RT = 1.032 min; LCMS34: m/z = 234 [M+1].

### Step 4: Synthesis of 2-N-[5-(3-chloropropoxy)-2-fluoro-4-methoxyphenyl]-4-N,6-dimethylpyrimidine-2,4-diamine:

Into a 50-mL round-bottom flask, was placed 5-(3-chloropropoxy)-2-fluoro-4-methoxyaniline (150 mg, 0.64 mmol, 1 equiv), 2-chloro-N,6-dimethylpyrimidin-4-amine (101 mg, 0.64 mmol, 1 equiv), trifluoroacetic acid (125 mg, 1.11 mmol, 2.00 equiv), isopropanol (10 mL). The resulting solution was stirred for 16 h at 85 °C. The resulting mixture was concentrated under vacuum. The residue was applied onto a silica gel column with ACN/H2O (1/1). This resulted in 180 mg (79%) of the title compound as brown oil.

Analytical Data: LC-MS: (ES, *m*/*z*): RT = 1.153 min; LCMS34: m/z = 255 [M+1].

### Step 5: Synthesis N²-(2-fluoro-4-methoxy-5-(3-(pyrrolidin-1-yl)propoxy)phenyl)-N⁴,6-dimethylpyrimidine-2,4-diamine:

Into a 50-mL round-bottom flask, was placed 2-N-[5-(3-chloropropoxy)-2-fluoro-4-methoxyphenyl]-4-N,6-dimethylpyrimidine-2,4-diamine (162 mg, 0.46 mmol, 1 equiv), pyrrolidine (64 mg, 0.90 mmol, 2.00 equiv), NaI (69 mg, 0.46 mmol, 1 equiv), Cs₂CO₃ (298 mg, 0.91 mmol, 2.00 equiv), CH₃CN (10 mL). The resulting solution was stirred for 16 h at 85 °C. The resulting mixture was concentrated under vacuum. The residue was applied onto a silica gel column with ACN/H₂O (1/1). This resulted in 46 mg (26%) of N²-(2-fluoro-4-methoxy-5-(3-(pyrrolidin-1-yl)propoxy)phenyl)-N⁴,6-dimethylpyrimidine-2,4-diamine as a solid.

### Example 56: Synthesis of Compound 303 (Reference)

### Compound 303: Synthesis of N²-(5-methoxy-4-(3-(pyrrolidin-1-yl)propoxy)pyridin-2-yl)-N⁴,6-dimethylpyrimidine-2,4-diamine

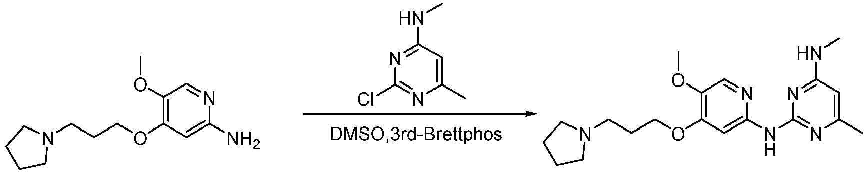

### Step 1: Synthesis of N²-(5-methoxy-4-(3-(pyrrolidin-1-yl)propoxy)pyridin-2-yl)-N⁴,6-dimethylpyrimidine-2,4-diamine:

Into a 50-mL round-bottom flask purged and maintained with an inert atmosphere of nitrogen, was placed 5-methoxy-4-[3-(pyrrolidin-1-yl)propoxy]pyridin-2-amine (200 mg, 0.80 mmol, 1 equiv), 2-chloro-N,6-dimethylpyrimidin-4-amine (125.1 mg, 0.79 mmol, 1 equiv), Cs₂CO₃ (779.3 mg, 2.39 mmol, 3.00 equiv), 3rd-BrettPhos (72.2 mg, 0.08 mmol, 0.20 equiv), Pd₂(dba)3-CHCl3 (41.2 mg, 0.04 mmol, 0.10 equiv), DMSO (5 mL). The resulting solution was stirred for 2 h at 100 °C in an oil bath. The solids were filtered out. The crude product was purified by Prep-HPLC C TFA. This resulted in 133.8 mg (35%) of N²-(5-methoxy-4-(3-(pyrrolidin-1-yl)propoxy)pyridin-2-yl)-N⁴,6-dimethylpyrimidine-2,4-diamine as a white solid.

### Example 57: Synthesis of Compound 305 (Reference)

### Compound 305: Synthesis of N²-(5-methoxy-4-(3-(pyrrolidin-1-yl)propoxy)pyridin-2-yl)-N⁴-methylpyridine-2,4-diamine

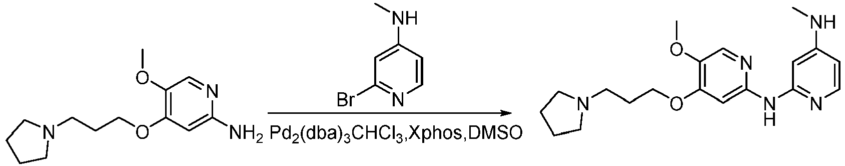

### Step 1: Synthesis of N²-(5-methoxy-4-(3-(pyrrolidin-1-yl)propoxy)pyridin-2-yl)-N⁴-methylpyridine-2,4-diamine:

Into a 100-mL round-bottom flask, was placed 5-methoxy-4-[3-(pyrrolidin-1-yl)propoxy]pyridin-2-amine (135 mg, 0.54 mmol, 1 equiv), 2-bromo-N-methylpyridin-4-amine (100 mg, 0.53 mmol, 1 equiv), Xphos (51.2 mg, 0.20 equiv), Cs₂CO₃ (350.5 mg, 1.08 mmol, 2.00 equiv), DMSO (5 mL), Pd₂(dba)₃-CHCl₃ (55.6 mg, 0.10 equiv). The resulting solution was stirred for 24 h at 100 °C in an oil bath. The resulting mixture was concentrated under vacuum. The crude product (120 mg) was purified by Flash-Prep-HPLC A Grad. This resulted in 18.6 mg (7%) of N²-(5-methoxy-4-(3-(pyrrolidin-1-yl)propoxy)pyridin-2-yl)-N⁴-methylpyridine-2,4-diamine as a yellow solid.

### Example 58: Synthesis of Compound 306 (Reference)

### Compound 306: Synthesis of N⁴-(5-methoxy-4-(3-(pyrrolidin-1-yl)propoxy)pyridin-2-yl)-N²,6-dimethylpyrimidine-2,4-diamine

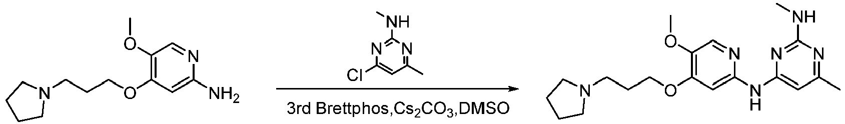

### Step 1: Synthesis of N⁴-(5-methoxy-4-(3-(pyrrolidin-1-yl)propoxy)pyridin-2-yl)-N²,6-dimethylpyrimidine-2,4-diamine:

Into a 100-mL round-bottom flask purged and maintained with an inert atmosphere of nitrogen, was placed 5-methoxy-4-[3-(pyrrolidin-1-yl)propoxy]pyridin-2-amine (200 mg, 0.80 mmol, 1 equiv), 3rd Brettphos (130 mg, 0.14 mmol, 0.10 equiv), Cs₂CO₃ (650 mg, 1.99 mmol, 2.00 equiv), 4-chloro-N,6-dimethylpyrimidin-2-amine (140 mg, 0.89 mmol, 1 equiv), DMSO (10 mL). The resulting solution was stirred for 3 h at 100 °C in an oil bath. The solids were filtered out. The crude product was applied onto a silica gel column with TFA/H₂O:ACN (10:1),Detector, UV 254 nm. This resulted in 88.6 mg (22%) of N⁴-(5-methoxy-4-(3-(pyrrolidin-1-yl)propoxy)pyridin-2-yl)-N²,6-dimethylpyrimidine-2,4-diamine as a white solid.

Analytical Data: LC-MS: (ES, *mlz*): RT = 0.83 min, LCMS 53: *m*/*z* = 373.0 [M+1]. ¹H NMR (400 MHz, Methanol-*d₄*) δ 8.04 (s, 1H), 7.37 (s, 1H), 6.46 (s, 1H), 4.33 (s, 2H), 3.96 (d, *J=* 1.3 Hz, 3H), 3.91 - 3.73 (m, 2H), 3.48 (t, *J* = 7.3 Hz, 2H), 3.24 - 3.12 (m, 2H), 3.09 (d, *J=* 1.8 Hz, 3H), 2.38 - 2.35 (m, 5H), 2.29 - 2.17 (m, 2H), 2.10 - 2.09 (m, 2H).

### Example 59: Synthesis of Compound 307 (Reference)

### Compound 307: Synthesis of N²-(5-methoxy-4-(3-(pyrrolidin-1-yl)propoxy)pyridin-2-yl)-N⁴,6-dimethylpyridine-2,4-diamine

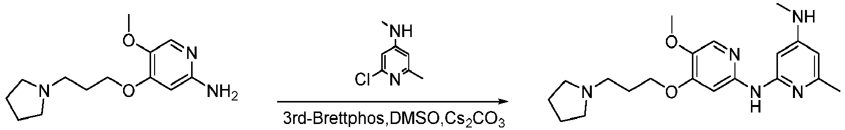

### Step 1: Synthesis of N²-(5-methoxy-4-(3-(pyrrolidin-1-yl)propoxy)pyridin-2-yl)-N⁴,6-dimethylpyridine-2,4-diamine:

Into a 100-mL round-bottom flask purged and maintained with an inert atmosphere of nitrogen, was placed 5-methoxy-4-[3-(pyrrolidin-1-yl)propoxy]pyridin-2-amine (200 mg, 0.80 mmol, 1 equiv), Cs₂CO₃ (75 mg, 0.23 mmol, 3 equiv), 3rd-Brettphos (140 mg, 0.20 equiv), 2-chloro-N,6-dimethylpyridin-4-amine (130 mg, 0.83 mmol, 1 equiv), DMSO (15 mL). The resulting solution was stirred for 3 h at 100 °C in an oil bath. The solids were filtered out. The crude product (200 mg) was applied onto a silica gel column with TFA/H₂O:ACN (8:1). This resulted in 64.6 mg (21%) of N²-(5-methoxy-4-(3-(pyrrolidin-1-yl)propoxy)pyridin-2-yl)-N⁴,6-dimethylpyridine-2,4-diamine as a white solid.

### Example 60: Synthesis of Compound 308 (Reference)

### Compound 308: Synthesis of N⁴-(5-methoxy-4-(3-(pyrrolidin-1-yl)propoxy)pyridin-2-yl)-N²,6-dimethylpyridine-2,4-diamine

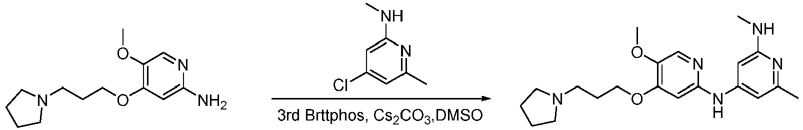

### Step 1: Synthesis of N⁴-(5-methoxy-4-(3-(pyrrolidin-1-yl)propoxy)pyridine-2-yl)-N²,6-dimethylpyridine-2,4-diamine:

Into a 100-mL round-bottom flask purged and maintained with an inert atmosphere of nitrogen, was placed 5-methoxy-4-[3-(pyrrolidin-1-yl)propoxy]pyridin-2-amine (200 mg, 0.80 mmol, 1 equiv), 3rd Brettphos (140 mg, 0.15 mmol, 0.20 equiv), Cs₂CO₃ (750 mg, 2.30 mmol, 3.00 equiv), 4-chloro-N,6-dimethylpyridin-2-amine (130 mg, 0.83 mmol, 1 equiv), DMSO (15 mL). The resulting solution was stirred for 3 h at 100 °C in an oil bath. The solids were filtered out. The crude product (300 mg) was applied onto a silica gel column with TFA/H₂O:ACN (10:1),Detector, UV 254 nm. This resulted in 121.9 mg (30%) of N⁴-(5-methoxy-4-(3-(pyrrolidin-1-yl)propoxy)pyridine-2-yl)-N²,6-dimethylpyridine-2,4-diamine as a white solid.

### Example 61: Synthesis of Compound 309 (Reference)

### Compound 309: Synthesis of 5-fluoro-N²-(5-methoxy-4-(3-(pyrrolidin-1-yl)propoxy)pyridin-2-yl)-N⁴,6-dimethylpyridine-2,4-diamine

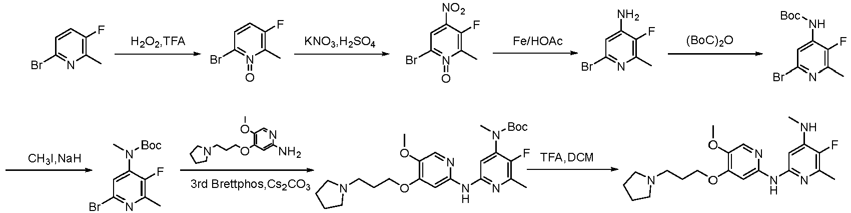

### Step 1: Synthesis 6-bromo-3-fluoro-2-methylpyridine 1-oxide:

Into a 50-mL round-bottom flask, was placed 6-bromo-3-fluoro-2-methylpyridine (1 g, 5.26 mmol, 1 equiv), H₂O₂ (4 mL), trifluoroacetic acid (10 mL). The resulting solution was stirred for 20 h at 70 °C. The resulting mixture was concentrated under vacuum. The residue was applied onto a silica gel column with dichloromethane/methanol (100/0). This resulted in 1.09 g (101%) of the title compound as yellow oil.

Analytical Data: LC-MS: (ES, *m*/*z*): RT = 0.714 min; LCMS40: m/z = 206 [M+1]. ¹H NMR (400 MHz, Chloroform-*d*) δ7.61 (d, *J* = 9.1 Hz, 1H), 7.13 (d, *J* = 9.2 Hz, 1H), 2.61 (s, 3H).

### Step 2: Synthesis of 6-bromo-3-fluoro-2-methyl-4-nitropyridine 1-oxide:

Into a 50-mL round-bottom flask, was placed 6-bromo-3-fluoro-2-methylpyridine 1-oxide (1 g, 4.85 mmol, 1 equiv), sulfuric acid (10 mL), potassium nitrate (1.97 g, 4.00 equiv). The resulting solution was stirred for 6 h at 120 °C. The reaction mixture was cooled with a water/ice bath. The resulting solution was extracted with 2x50 mL of ethyl acetate and the organic layers combined. The resulting mixture was washed with 2x50 mL of sodium bicarbonate. The resulting mixture was washed with 100 mL of brine. The resulting mixture was concentrated under vacuum. This resulted in 650 mg (53%) of the title compound as a yellow solid.

Analytical Data: LC-MS: (ES, *m*/*z*): RT = 1.068 min; LCMS33: m/z = 251 [M+1].

### Step 3: Synthesis of 6-bromo-3-fluoro-2-methylpyridin-4-amine:

Into a 50-mL round-bottom flask, was placed 6-bromo-3-fluoro-2-methyl-4-nitropyridine 1-oxide (600 mg, 2.39 mmol, 1 equiv), acetic acid (10 mL), Fe (672 mg, 5.00 equiv). The resulting solution was stirred for 1 h at 100 °C. The reaction was then quenched by the addition of water/ice. The resulting solution was extracted with 100 mL of ethyl acetate and the organic layers combined and concentrated under vacuum. The residue was applied onto a silica gel column with ACN/H₂O (1/10). This resulted in 260 mg (53%) of the title compound as an off-white solid.

Analytical Data: LC-MS: (ES, *m*/*z*): RT = 0.774 min; LCMS33: m/z = 205 [M+1]. ¹H NMR (400 MHz, DMSO-*d*6) δ 6.67 (d, *J* = 5.9 Hz, 1H), 6.46 (s, 2H), 2.24 (s, 3H).

### Step 4: Synthesis of tert-butyl N-(6-bromo-3-fluoro-2-methylpyridin-4-yl)carbamate:

Into a 50-mL round-bottom flask, was placed a solution of 6-bromo-3-fluoro-2-methylpyridin-4-amine (250 mg, 1.22 mmol, 1 equiv) in dichloromethane (10 mL), 4-dimethylaminopyridine (299 g, 2.45 mol, 2.00 equiv), (Boc)₂O (536 mg, 2.46 mmol, 2.00 equiv), TEA (0.34 mL). The resulting solution was stirred for 16 h at 20 °C. The reaction was then quenched by the addition of 10 mL of 10% NaOH. The resulting solution was extracted with 20 mL of dichloromethane and the organic layers combined and concentrated under vacuum. The residue was applied onto a silica gel column with dichloromethane/petroleum ether (1/1). This resulted in 0.3 g (81%) of as an off-white solid.

Analytical Data: LC-MS: (ES, *m*/*z*): RT = 1.458 min; LCMS53: m/z = 305 [M+1]. ¹H NMR (400 MHz, Methanol-d4) δ 8.19 (s, 1H), 2.52 (s, 1H), 2.42 (s, 3H), 1.56 (d, *J =* 2.2 Hz, 9H).

### Step 5: Synthesis of tert-butyl N-(6-bromo-3-fluoro-2-methylpyridin-4-yl)-N-methylcarbamate:

Into a 50-mL round-bottom flask, was placed a solution of tert-butyl N-(6-bromo-3-fluoro-2-methylpyridin-4-yl)carbamate (278 mg, 0.91 mmol, 1 equiv) in tetrahydrofuran (10 mL). This was followed by the addition of sodium hydride (110 mg, 3.00 equiv), in portions at 0 °C in 1 hr. To this was added CH₃I (388 mg, 2.73 mmol, 3.00 equiv) dropwise with stirring at 0 °C. The resulting solution was stirred for 18 h at 20 °C. The reaction was then quenched by the addition of 30 mL of water. The resulting solution was extracted with 2x50 mL of ethyl acetate and the organic layers combined and concentrated under vacuum. The residue was applied onto a silica gel column with dichloromethane/petroleum ether (1/1). This resulted in 150 mg (52%) of as a yellow oil.

Analytical Data: LC-MS: (ES, *m*/*z):* RT = 1.449 min; LCMS53: m/z = 319 [M+1].

### Step 6: Synthesis of tert-butyl N-[3-fluoro-6-([5-methoxy-4-[3-(pyrrolidin-1-yl)propoxy]pyridin-2-yl]amino)-2-methylpyridin-4-yl]-N-methylcarbamate:

Into a 50-mL round-bottom flask purged and maintained with an inert atmosphere of nitrogen, was placed 5-methoxy-4-[3-(pyrrolidin-1-yl)propoxy]pyridin-2-amine (112 mg, 0.45 mmol, 1 equiv), tert-butyl N-(6-bromo-3-fluoro-2-methylpyridin-4-yl)-N-methylcarbamate (140 mg, 0.44 mmol, 1 equiv), 3^{rd} Brettphos (40 mg, 0.10 equiv), Cs₂CO₃ (287 mg, 0.88 mmol, 2.00 equiv), DMSO (4 mL). The resulting solution was stirred for 4 h at 100 °C. The resulting solution was diluted with 15 mL of H₂O. The resulting solution was extracted with 2x50 mL of ethyl acetate and the organic layers combined. The resulting mixture was washed with 3x50 mL of brine. The resulting mixture was concentrated under vacuum. The residue was applied onto a silica gel column with ACN/H₂O (1/5). This resulted in 80 mg (37%) of as colorless crude oil.

Analytical Data: LC-MS: (ES, *m*/*z*): RT = 1.015 min; LCMS53: m/z = 490 [M+1].

### Step 7: Synthesis of 5-fluoro-N²-(5-methoxy-4-(3-(pyrrolidin-1-yl)propoxy)pyridin-2-yl)-N⁴,6-dimethylpyridine-2,4-diamine:

Into a 50-mL round-bottom flask, was placed tert-butyl N-[3-fluoro-6-([5-methoxy-4-[3-(pyrrolidin-1-yl)propoxy]pyridin-2-yl] amino)-2-methylpyridin-4-yl]-N-methylcarbamate (80 mg, 0.16 mmol, 1 equiv), dichloromethane (6 mL), trifluoroacetic acid (1.5 mL). The resulting solution was stirred for 16 h at 20 °C. The resulting mixture was concentrated under vacuum. The residue was applied onto a silica gel column with ACN/H₂O (1/5). This resulted in 33.1 mg (40%) of 5-fluoro-N²-(5-methoxy-4-(3-(pyrrolidin-1-yl)propoxy)pyridin-2-yl)-N⁴,6-dimethylpyridine-2,4-diamine as a brown solid.

### Example 62: Synthesis of Compound 311

### Compound 311: Synthesis of N²-(4-methoxy-3-(2-methoxyethoxy)phenyl)-N⁴,6-dimethylpyrimidine-2,4-diamine

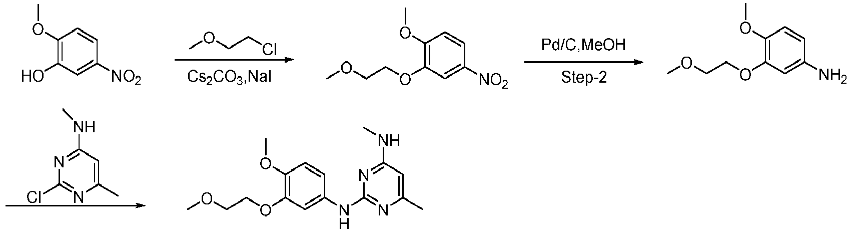

### Step 1: Synthesis of 1-methoxy-2-(2-methoxyethoxy)-4-nitrobenzene:

Into a 100-mL round-bottom flask, was placed 2-methoxy-5-nitrophenol (1 g, 5.91 mmol, 1 equiv), Cs₂CO₃ (3.8 g, 11.66 mmol, 2.00 equiv), NaI (1.8 g, 12.00 mmol, 2.00 equiv), N,N-dimethylformamide (40 mL), 1-chloro-2-methoxyethane (850 mg, 8.99 mmol, 1.5 equiv). The resulting solution was stirred for 2 h at 100 °C in an oil bath. The reaction was then quenched by the addition of 50 mL of NaHSO₃. The resulting solution was extracted with 3x50 mL of ethyl acetate and the organic layers were washed with 3x20 mL of sodium chloride. The resulting mixture was concentrated under vacuum. This resulted in 1.18 g (86%) of the title compound as a light yellow solid.

Analytical Data: LC-MS: (ES, *mlz*): RT = 1.21 min, LCMS 33: *m*/*z* = 228.0 [M+1]. ¹H NMR (300 MHz, DMSO-*d*₆) δ 7.91 (q, *J* = 9.0 Hz, 1H), 7.76 (d, *J* = 2.7 Hz, 1H), 7.19 (d, *J* = 9.0 Hz, 1H), 4.27 - 4.17 (m, 2H), 3.92 (s, 3H), 3.76 - 3.65 (m, 2H), 3.34 - 3.32 (s, 3H).

### Step 2: Synthesis of 4-methoxy-3-(2-methoxyethoxy)aniline:

Into a 100-mL round-bottom flask, was placed 1-methoxy-2-(2-methoxyethoxy)-4-nitrobenzene (580 mg, 2.55 mmol, 1 equiv), Pd/C (200 mg), methanol (25 mL). The resulting solution was stirred for 1 h at 25 °C. The solids were filtered out. The resulting mixture was concentrated under vacuum. This resulted in 430 mg (85%) of the title compound as a solid.

Analytical Data: LC-MS: (ES, *mlz*): RT = 0.72 min, LCMS 33: *m*/*z* = 198.0 [M+1].

### Step 3: Synthesis of N²-(4-methoxy-3-(2-methoxyethoxy)phenyl)-N⁴,6-dimethylpyrimidine-2,4-diamine:

Into a 100-mL round-bottom flask, was placed 4-methoxy-3-(2-methoxyethoxy)aniline (430 mg, 2.18 mmol, 1 equiv), TsOH (825 mg, 4.79 mmol, 2.00 equiv), 2-chloro-N,6-dimethylpyrimidin-4-amine (340 mg, 2.16 mmol, 1 equiv), isopropanol (23 mL). The resulting solution was stirred for 3 h at 90 °C in an oil bath. The resulting mixture was concentrated under vacuum. The crude product (300 mg) was applied onto a silica gel column with NH₄HCO3:ACN (1:1), Detector, UV 254 nm. 75 mg product was obtained. This resulted in 75 mg (11%) of N²-(4-methoxy-3-(2-methoxyethoxy)phenyl)-N⁴,6-dimethylpyrimidine-2,4-diamine as a solid.

### Example 63: Synthesis of Compound 312

### Compound 312: Synthesis of N²-(4-methoxy-3-(3-methoxypropoxy)phenyl)-N⁴,6-dimethylpyrimidine-2,4-diamine

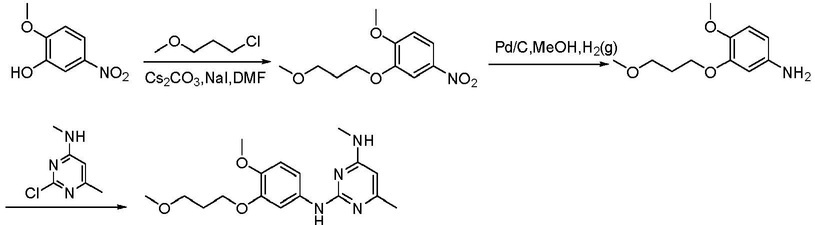

### Step 1: Synthesis of 1-methoxy-2-(3-methoxypropoxy)-4-nitrobenzene:

Into a 100-mL round-bottom flask, was placed 2-methoxy-5-nitrophenol (1 g, 5.91 mmol, 1 equiv), 1-chloro-3-methoxypropane (645 mg, 5.94 mmol, 1 equiv), Cs₂CO₃ (3.8 g, 11.66 mmol, 2.00 equiv), NaI (1.3 g, 1.50 equiv), N,N-dimethylformamide (20 mL). The resulting solution was stirred for 2 h at 100 °C in an oil bath. The resulting solution was diluted with 50 mL of EA. The resulting mixture was washed with 3x50 mL of brine. The mixture was dried over anhydrous sodium sulfate and concentrated under vacuum. This resulted in 1.4 g (98%) of as a yellow solid.

Analytical Data: LC-MS: (ES, *m*/*z*): RT = 1.317 min, LCMS 33: *m*/*z* = 242 [M+1].

### Step 2: Synthesis of 4-methoxy-3-(3-methoxypropoxy)aniline:

Into a 50-mL round-bottom flask, was placed 1-methoxy-2-(3-methoxypropoxy)-4-nitrobenzene (500 mg, 2.07 mmol, 1 equiv), Pd/C (10%) (100 mg), methanol (10 mL). The resulting solution was stirred for 1 h at RT under H₂ (g) atmosphere. The solids were filtered out. The resulting mixture was concentrated under vacuum. This resulted in 410 mg (94%) of the title compound as an oil.

Analytical Data: LC-MS: (ES, *m*/*z*): RT = 0.792 min, LCMS 33: *m*/*z* = 212 [M+1].

### Step 3: Synthesis of N²-(4-methoxy-3-(3-methoxypropoxy)phenyl)-N⁴,6-dimethylpyrimidine-2,4-diamine:

Into a 50-mL round-bottom flask, was placed 4-methoxy-3-(3-methoxypropoxy)aniline (350 mg, 1.66 mmol, 1 equiv), 2-chloro-N,6-dimethylpyrimidin-4-amine (262 mg, 1.66 mmol, 1 equiv), CF₃COOH (378 mg, 3.32 mmol, 2.00 equiv), isopropanol (5 mL). The resulting solution was stirred for overnight at 80 °C in an oil bath. The resulting mixture was concentrated under vacuum. The residue was purified by flash chromatography with H₂O/NH₄HCO₃/ACN (41%). This resulted in 315.0 mg (57%) of N²-(4-methoxy-3-(3-methoxypropoxy)phenyl)-N⁴,6-dimethylpyrimidine-2,4-diamine as a white solid.

### Example 64: Synthesis of Compound 313

### Compound 313: Synthesis of N²-(4-cyclopropyl-3-(3-(pyrrolidin-1-yl)propoxy)phenyl)-N⁴,6-dimethylpyrimidine-2,4-diamine

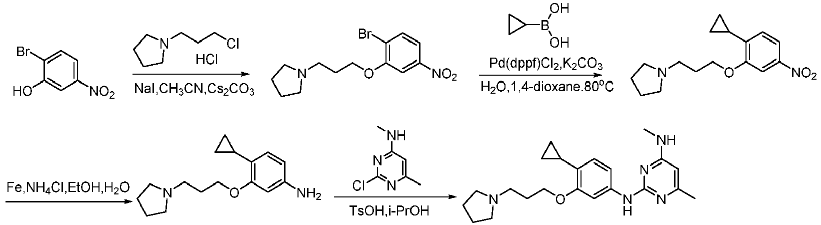

### Step 1: Synthesis of 1-[3-(2-bromo-5-nitrophenoxy)propyl]pyrrolidine:

Into a 100-mL round-bottom flask, was placed 2-bromo-5-nitrophenol (2 g, 9.17 mmol, 1 equiv), 1-(3-chloropropyl)pyrrolidine hydrochloride (1.69 g, 9.18 mmol, 1 equiv), NaI (1.65 g, 1.20 equiv), Cs₂CO₃ (5.96 g, 18.29 mmol, 2.00 equiv), CH₃CN (30 mL). The resulting solution was stirred for 5 h at 80 °C in an oil bath. The solids were filtered out. The resulting mixture was concentrated under vacuum. The residue was purified by flash chromatography with ACN/H₂O(28%). This resulted in 2.4 g (79%) of as a yellow solid.

Analytical Data: LC-MS: (ES, *mlz*): RT = 0.964 min, LCMS33: *m*/*z* = 329 [M+1].

### Step 2: Synthesis of 1-[3-(2-cyclopropyl-5-nitrophenoxy)propyl]pyrrolidine:

Into a 250-mL 3-necked round-bottom flask, was placed 1-[3-(2-bromo-5-nitrophenoxy)propyl]pyrrolidine (1.9 g, 5.77 mmol, 1 equiv), cyclopropylboronic acid (745 mg, 8.67 mmol, 1.50 equiv), Pd(dppf)Cl₂ (845 mg, 1.15 mmol, 0.20 equiv), potassium carbonate (1.59 g, 11.50 mmol, 2.00 equiv), water(2 mL), 1,4-dioxane (20 mL). The resulting solution was stirred for 16 h at 80 °C in an oil bath under N₂ (g) atmosphere. The resulting mixture was concentrated under vacuum. The solids were filtered out. The resulting mixture was concentrated under vacuum. The residue was purified by flash chromatography with H₂O/ACN (32%). This resulted in 440 mg (26%) of as an oil.

Analytical Data: LC-MS: (ES, *mlz*): RT = 0.956 min, LCMS39 : *m*/*z* = 291 [M+1].

### Step 3: Synthesis of 4-cyclopropyl-3-[3-(pyrrolidin-1-yl)propoxy]aniline:

Into a 100-mL round-bottom flask, was placed 1-[3-(2-cyclopropyl-5-nitrophenoxy)propyl]pyrrolidine (400 mg, 1.38 mmol, 1 equiv), Fe (385 mg, 6.88 mmol, 5.00 equiv), NH₄Cl (368 mg, 6.88 mmol, 5.00 equiv), water (6 mL), ethanol (12 mL). The resulting solution was stirred for 3 h at 80 °C in an oil bath. The solids were filtered out. The resulting mixture was concentrated under vacuum. This resulted in 1.1 g of the title compound as a yellow crude solid.

Analytical Data: LC-MS: (ES, *mlz):* RT = 0.794 min, LCMS33 : *m*/*z* = 261 [M+1].

### Step 4: Synthesis of N²-(4-cyclopropyl-3-(3-(pyrrolidin-1-yl)propoxy)phenyl)-N⁴,6-dimethylpyrimidine-2,4-diamine:

Into a 5-mL round-bottom flask, was placed 4-cyclopropyl-3-[3-(pyrrolidin-1-yl)propoxy]aniline (300 mg, 1.15 mmol, 1 equiv), 2-chloro-N,6-dimethylpyrimidin-4-amine (181.2 mg, 1.15 mmol, 1 equiv), CF₃COOH (263.1 mg, 2.31 mmol, 2.00 equiv), isopropanol (5 mL). The resulting solution was stirred for overnight at 80 °C in an oil bath. The resulting mixture was concentrated under vacuum. The crude product was purified by Flash-Prep-HPLC C NH3. This resulted in 40.6 mg of N²-(4-cyclopropyl-3-(3-(pyrrolidin-1-**yl)propoxy)phenyl)-N⁴,6-dimethylpyrimidine-2,4-diamine** as a white solid.

### Example 65: Synthesis of Compound 314 (Reference)

### Compound 314: Synthesis of N²-(4-cyclopropyl-3-(3-(pyrrolidin-1-yl)propoxy)phenyl)-N⁴-methylpyrimidine-2,4-diamine

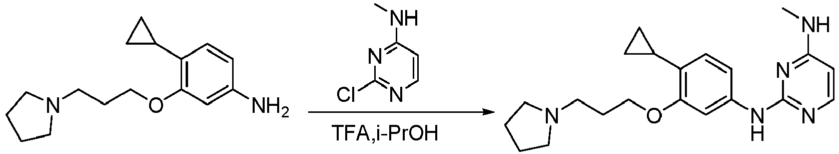

### Step 1: Synthesis of N²-(4-cyclopropyl-3-(3-(pyrrolidin-1-yl)propoxy)phenyl)-N⁴-methylpyrimidine-2,4-diamine:

Into a 50-mL round-bottom flask, was placed 4-cyclopropyl-3-[3-(pyrrolidin-1-yl)propoxy]aniline (300 mg, 1.15 mmol, 1 equiv), 2-chloro-N-methylpyrimidin-4-amine (166 mg, 1.16 mmol, 1 equiv), CF₃COOH (263 mg, 2.31 mmol, 2.00 equiv), isopropanol (5 mL). The resulting solution was stirred for overnight at 80 °C in an oil bath. The resulting mixture was concentrated under vacuum. The crude product was purified by Prep-HPLC C TFA. This resulted in 21.9 mg of N²-(4-cyclopropyl-3-(3-(pyrrolidin-1-yl)propoxy)phenyl)-N⁴-methylpyrimidine-2,4-diamine as a white solid.

### Example 66: Synthesis of Compound 315 (Reference)

### Compound 315: Synthesis of N⁴-methyl-N²-(3-(3-(pyrrolidin-1-yl)propoxy)-4-(trifluoromethoxy)phenyl)pyrimidine-2,4-diamine

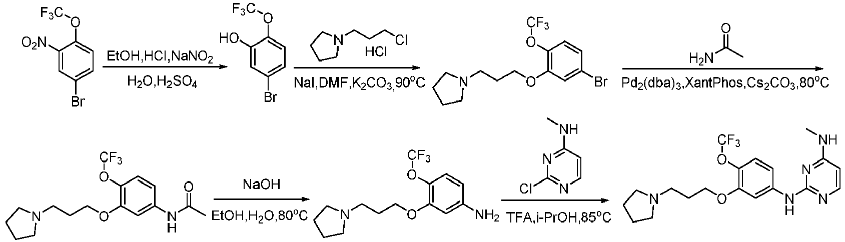

### Step 1: Synthesis of 5-bromo-2-(trifluoromethoxy)phenol:

Into a 250-mL 3-necked round-bottom flask, was placed 5-bromo-2-(trifluoromethoxy) aniline (2 g, 7.81 mmol, 1 equiv), ethanol (20 mL), HCl (2 mL). This was followed by the addition of NaNO₂ (595 mg, 8.62 mmol, 1.10 equiv) dropwise with stirring at 0 °C. To this was added water (110 mL), sulfuric acid (5.5 mL). The resulting solution was stirred for 1.5 h at 0 °C in a water/ice bath. The resulting solution was allowed to react, with stirring, for an additional 12 h while the temperature was maintained at 100 °C in an oil bath. The resulting solution was extracted with 3x100 mL of ethyl acetate and the organic layers combined. The resulting mixture was washed with 3x50 mL of sodium bicarbonate. The mixture was dried over anhydrous sodium sulfate. This resulted in 1 g (50%) of the title compound as an oil.

Analytical Data: LC-MS: (ES, *m*/*z*): RT =1.715min, LCMS 53: m/z = 257 [M+1]. ¹H NMR (400 MHz, DMSO-*d*₆) δ 13.29 (s, 1H), 7.96 (s, 1H), 7.77 - 7.51 (m, 1H), 7.44 (d, *J* = 8.5 Hz, 1H).

### Step 2: Synthesis of 1-[3-[5-bromo-2-(trifluoromethoxy)phenoxy]propyl]pyrrolidine:

Into a 100-mL round-bottom flask, was placed 5-bromo-2-(trifluoromethoxy)phenol (1000 mg, 3.89 mmol, 1 equiv), 1-(3-chloropropyl)pyrrolidine hydrochloride (720 mg, 3.91 mmol, 1 equiv), Cs₂CO₃ (2550 mg, 7.83 mmol, 2.00 equiv), NaI (589 mg, 1 equiv), N,N-dimethylformamide (10 mL). The resulting solution was stirred for 2 h at 90 °C in an oil bath. The resulting solution was extracted with 3x50 mL of ethyl acetate and the organic layers combined. The resulting mixture was washed with 3x30 mL of brine. The mixture was dried over anhydrous sodium sulfate and concentrated under vacuum. This resulted in 1.4 g (98%) of the title compound as red oil.

Analytical Data: LC-MS: (ES, *m*/*z*): RT =1.346min, LCMS 53: m/z =368 [M+1].

### Step 3: Synthesis of N-[3-[3-(pyrrolidin-1-yl)propoxy]-4-(trifluoromethoxy)phenyl] acetamide:

Into a 100-mL round-bottom flask purged and maintained with an inert atmosphere of nitrogen, was placed 1-[3-[5-bromo-2-(trifluoromethoxy)phenoxy] propyl]pyrrolidine (700 mg, 1.90 mmol, 1 equiv), acetamide (228.9 mg, 3.88 mmol, 2.00 equiv), Cs₂CO₃ (1.24 g, 3.81 mmol, 2.00 equiv), XantPhos (220.5 mg, 0.38 mmol, 0.20 equiv), Pd₂(dba)₃-CHCl₃ (197.4 mg, 0.10 equiv), dioxane (20 mL). The resulting solution was stirred for 12 h at 80 °C in an oil bath. The solids were collected by filtration. The crude product was purified by ACN/H₂O=2/5. This resulted in 450 mg (68%) of the title compound as yellow oil.

Analytical Data: LC-MS: (ES, *m*/*z*): RT =0.940min, LCMS 33: m/z =347[M+1]. ¹H NMR (300 MHz, Methanol-*d₄*) δ 7.63 (d, *J* = 2.4 Hz, 1H), 7.21 (d, *J=* 8.4 Hz, 1H), 7.05 (d, *J* = 2.4 Hz, 1H), 4.14 (t, *J* = 5.9 Hz, 2H), 3.63 (q, *J =* 7.0 Hz, 1H), 2.97 - 2.83 (m, 5H), 2.19 - 2.09 (m, 4H), 1.94 - 1.83 (m, 4H), 1.21 (t, *J* = 7.1 Hz, 1H).

### Step 4: Synthesis of 3-[3-(pyrrolidin-1-yl)propoxy]-4-(trifluoromethoxy)aniline:

Into a 50-mL round-bottom flask, was placed N-[3-[3-(pyrrolidin-1-yl)propoxy]-4-(trifluoromethoxy)phenyl]acetamide (450 mg, 1.30 mmol, 1 equiv), ethanol (6 mL), water(2 mL), sodium hydroxide (208 mg, 5.20 mmol, 4.00 equiv). The resulting solution was stirred for 12 h at 80 °C in an oil bath. The resulting mixture was concentrated under vacuum. The crude product was purified by ACN/H₂O = 1/20. This resulted in 350 mg (89%) of the title compound as yellow oil.

Analytical Data: LC-MS: (ES, *m*/*z):* RT =0.930min, LCMS 31: m/z =305 [M+1].

### Step 5: Synthesis of N⁴-methyl-N²-(3-(3-(pyrrolidin-1-yl)propoxy)-4-(trifluoromethoxy)phenyl)pyrimidine-2,4-diamine:

Into a 50-mL round-bottom flask, was placed 3-[3-(pyrrolidin-1-yl)propoxy]-4-(trifluoromethoxy)aniline (350 mg, 1.15 mmol, 1 equiv), 2-chloro-N-methylpyrimidin-4-amine (164.7 mg, 1.15 mmol, 1 equiv), trifluoroacetic acid (262.5 mg, 2.32 mmol, 2.00 equiv), isopropanol (6 mL). The resulting solution was stirred for 4 h at 80 °C in an oil bath. The resulting mixture was concentrated under vacuum. The crude product (450 mg) was purified by Prep-HPLC C NH3. This resulted in 104 mg (17%) of N⁴-methyl-N²-(3-(3-(pyrrolidin-1-yl)propoxy)-4-(trifluoromethoxy)phenyl)pyrimidine-2,4-diamine as a white solid.

### Example 67: Synthesis of Compounds 329 and 317

### Compound 329 and 317: Synthesis of Diastereomer 1: N²-(3-((1r,3r)-3-(dimethylamino)cyclobutoxy)-4-methoxyphenyl)-N⁴,6-dimethylpyrimidine-2,4-diamine and Diastereomer 2: N²-(3-((1s,3s)-3-(dimethylamino)cyclobutoxy)-4-methoxyphenyl)-N⁴,6-dimethylpyrimidine-2,4-diamine

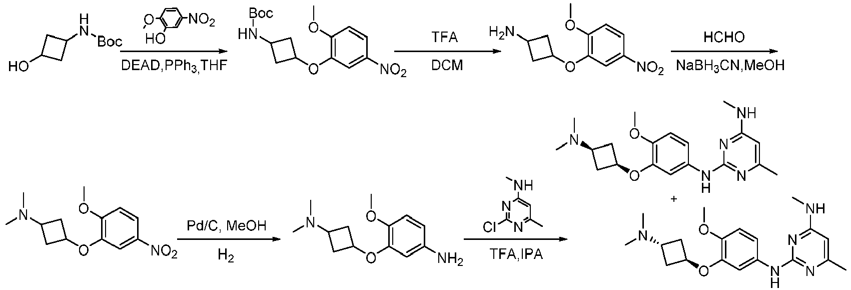

### Step 1: Synthesis of tert-butyl N-[3-(2-methoxy-5-nitrophenoxy)cyclobutyl]carbamate:

Into a 100-mL 3-necked round-bottom flask purged and maintained with an inert atmosphere of nitrogen, was placed tert-butyl N-(3-hydroxycyclobutyl)carbamate (500 mg, 2.67 mmol, 1 equiv), 2-methoxy-5-nitrophenol (452 mg, 2.67 mmol, 1 equiv), PPh₃ (1.541 g, 5.88 mmol, 2.20 equiv), tetrahydrofuran (20 mL). This was followed by the addition of a solution of DEAD (1.188 g, 5.88 mmol, 2.20 equiv) in tetrahydrofuran (5 mL) dropwise with stirring at 0 °C. The resulting solution was stirred for 10 min at 0 °C. The resulting solution was stirred for 16 h at 25 °C. The resulting mixture was concentrated under vacuum. The crude product was purified by Flash-Prep-HPLC A EA/PE. This resulted in 900 mg (100%) of as yellow oil.

Analytical Data: LC-MS: (ES, *m*/*z*): RT=0.707min, LCMS 40, *mlz* =239 [M+1]. ¹H NMR (300 MHz, DMSO-*d*₆) δ 8.98 (s, 1H), 8.00 - 7.69 (m, 1H), 7.62 - 7.47 (m, 1H), 7.28 - 7.13 (m, 1H), 5.01 - 4.46 (m, 1H), 3.91 (d, *J* = 3.0 Hz, 3H), 2.89 - 2.68 (m, 1H), 2.45 - 2.28 (m, 2H), 2.10 - 1.93 (m, 2H), 1.39 (d, *J* = 3.3 Hz, 9H).

### Step 2: Synthesis of 3-(2-methoxy-5-nitrophenoxy)cyclobutan-1-amine:

Into a 50-mL round-bottom flask, was placed tert-butyl N-[3-(2-methoxy-5-nitrophenoxy)cyclobutyl]carbamate (900 mg, 2.66 mmol, 1 equiv), dichloromethane (10 mL), trifluoroacetic acid (5 mL). The resulting solution was stirred for 30 min at 25 °C. This resulted in 1.2 g (crude) of as yellow oil.

Analytical Data: LC-MS: (ES, *m*/*z*): RT=0.557min, LCMS 30, *mlz* =239[M+1]. ¹H NMR (300 MHz, DMSO-*d*₆) δ 8.17 (s, 2H), 8.00 - 7.86 (m, 1H), 7.64 - 7.42 (m, 2H), 5.20 - 4.62 (m, 1H), 3.93 (s, 3H), 3.89 - 3.34 (m, 1H), 2.97 - 2.57 (m, 2H), 2.38 - 2.15 (m, 2H).

### Step 3: Synthesis of 3-(2-methoxy-5-nitrophenoxy)-N,N-dimethylcyclobutan-1-amine:

Into a 50-mL round-bottom flask, was placed 3-(2-methoxy-5-nitrophenoxy)cyclobutan-1-amine; trifluoroacetic acid (942 mg, 2.67 mmol, 1 equiv), methanol (20 mL), formaldehyde (241 mg, 8.03 mmol, 3.00 equiv), NaBH₃CN (843 mg, 13.42 mmol, 5.00 equiv). The resulting solution was stirred for 6 h at 25 °C. The resulting mixture was concentrated under vacuum. The crude product was purified by Flash-Prep-HPLC A MeOH/H₂O. This resulted in 330 mg (46%) of the title compound as yellow oil.

Analytical Data: LC-MS: (ES, *m*/*z*): RT=0.901min, LCMS 15, *mlz* =267 [M+1]. ¹H NMR (300 MHz, DMSO-*d*₆) δ 7.92 (dd, *J* = 9.0, 2.7 Hz, 1H), 7.63 - 7.42 (m, 1H), 7.20 (dd, *J* = 9.1, 2.5 Hz, 1H), 4.94 - 4.51 (m, 1H), 3.92 (s, 3H), 2.92 - 2.56 (m, 2H), 2.46 - 2.12 (m, 2H), 2.07 (d, *J* = 6.8 Hz, 6H), 1.94 - 1.77 (m, 1H).

### Step 4: Synthesis of 3-[3-(dimethylamino)cyclobutoxy]-4-methoxyaniline:

Into a 50-mL round-bottom flask, was placed 3-(2-methoxy-5-nitrophenoxy)-N,N-dimethylcyclobutan-1-amine (330 mg, 1.24 mmol, 1 equiv), methanol (20 mL), Pd/C, hydrogen. The resulting solution was stirred for 1 h at 25 °C. The solids were filtered out. The resulting mixture was concentrated under vacuum. This resulted in 285 mg (97%) of the title compound as yellow oil.

Analytical Data: LC-MS: (ES, *m*/*z*): RT=0.261min, LCMS 31, *mlz* =237 [M+1]. ¹H NMR (300 MHz, DMSO-*d*₆) δ 6.64 (dd, *J* = 8.3, 1.8 Hz, 1H), 6.25 - 6.00 (m, 2H), 4.72 - 4.51 (m, 2H), 4.36 - 4.14 (m, 1H), 3.61 (d, *J* = 2.7 Hz, 3H), 2.85 - 2.54 (m, 2H), 2.38 - 1.98 (m, 8H), 1.87 - 1.72 (m, 1H).

### Step 5: Synthesis of Diastereomer 1: N²-(3-((1r,3r)-3-(dimethylamino)cyclobutoxy)-4-methoxyphenyl)-N⁴,6-dimethylpyrimidine-2,4-diamine and Diastereomer 2: N²-(3-((1s,3s)-3-(dimethylamino)cyclobutoxy)-4-methoxyphenyl)-N⁴,6-dimethylpyrimidine-2,4-diamine:

Into a 50-mL round-bottom flask, was placed 3-[3-(dimethylamino)cyclobutoxy]-4-methoxyaniline (250 mg, 1.06 mmol, 1 equiv), 2-chloro-N,6-dimethylpyrimidin-4-amine (167 mg, 1.06 mmol, 1 equiv), IPA (10 mL), trifluoroacetic acid (242 mg, 2.14 mmol, 2.00 equiv). The resulting solution was stirred for 2 h at 90 °C in an oil bath. The resulting mixture was concentrated under vacuum. The crude product was purified by Chiral-Prep-HPLC IF. The crude product was purified by Prep-HPLC C HCl. This resulted in 49.6 mg (12%) of N²-(3-((1r,3r)-3-(dimethylamino)cyclobutoxy)-4-methoxyphenyl)-N⁴,6-dimethylpyrimidine-2,4-diamine diastereomer 1 (randomly assigned) as an off-white solid. And 69.4 mg (17%) of N²-(3-((1s,3s)-3-(dimethylamino)cyclobutoxy)-4-methoxyphenyl)-N⁴6-dimethylpyrimidine-2,4-diamine diastereomer 2 (randomly assigned) as an off-white solid.

### Example 68: Synthesis of Compound 318

### Compound 318: Synthesis of N²-(3-((1s,3s)-3-((dimethylamino)methyl) cyclobutoxy)-4-methoxyphenyl)-N⁴,6-dimethylpyrimidine-2,4-diamine

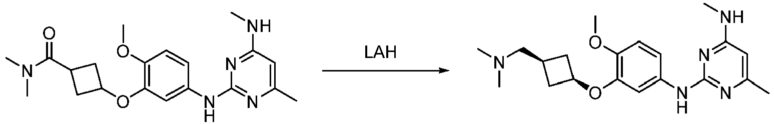

### Step 1: Synthesis of N²-(3-((1s,3s)-3-((dimethylamino)methyl)cyclobutoxy)-4-methoxyphenyl)-N⁴,6-dimethylpyrimidine-2,4-diamine:

Into a 50-mL round-bottom flask, was placed 3-(2-methoxy-5-[[4-methyl-6-(methylamino)pyrimidin-2-yl] amino]phenoxy)-N,N-dimethylcyclobutane-1-carboxamide (200 mg, 0.52 mmol, 1 equiv), LAH (78.96 mg, 2.08 mmol, 4.00 equiv), oxolane (10 mL). The resulting solution was stirred for 2 h at 0 °C in a water/ice bath. The reaction was then quenched by the addition of 200 mg of water/ice. The pH value of the solution was adjusted to 8 with sodium hydroxide (aq) (10 %). The resulting solution was diluted with 2 mL of H₂O. The resulting solution was extracted with 20 mL of ethyl acetate and the organic layers combined and dried over anhydrous sodium sulfate. The solids were filtered out. The crude product was purified by Prep-HPLC A. This resulted in 61.8 mg (32%) of N²-(3-((1s,3s)-3-((dimethylamino)methyl)cyclobutoxy)-4-methoxyphenyl)-N⁴,6-dimethylpyrimidine-2,4-diamine as a white solid.

### Example 69: Synthesis of Compound 319

### Compound 319: Synthesis of 6-ethyl-5-fluoro-N²-(4-methoxy-3-(3-(pyrrolidin-1-yl)propoxy)phenyl)-N⁴-methylpyrimidine-2,4-diamine

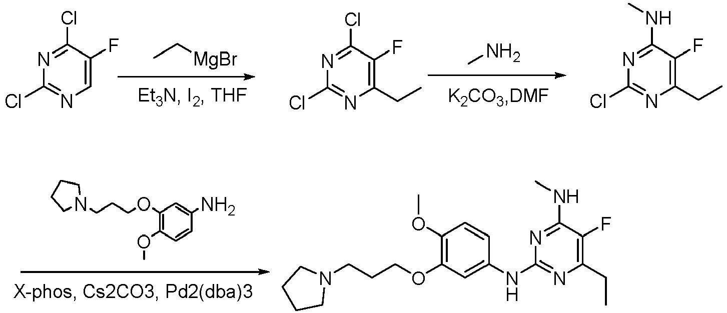

### Step 1: Synthesis of 2,4-dichloro-6-ethyl-5-fluoropyrimidine:

Into a 100-mL 3-necked round-bottom flask, was placed 2,4-dichloro-5-fluoropyrimidine (1 g, 5.99 mmol, 1 equiv), GDE (3 mL), I2 (1.5 g, 1 equiv), tetrahydrofuran (8 mL), TEA (605 mg, 5.98 mmol, 1 equiv), bromo(ethyl)magnesium (1.2 g, 9.00 mmol, 1.50 equiv). The resulting solution was stirred for 1 h at 0 °C in a water/ice bath. The resulting solution was extracted with 3x100 mL of ethyl acetate and the organic layers combined. The resulting mixture was washed with 3x50 mL of NaHSO3. The mixture was dried over anhydrous sodium sulfate and concentrated under vacuum. The residue was applied onto a silica gel column with ethyl acetate/petroleum ether (1/10). This resulted in 600 mg (51%) of the title compound as yellow oil.

Analytical Data: LC-MS: (ES, *m*/*z*): 195 [M+1], RT: 1.38 min.

### Step 2: Synthesis of 2-chloro-6-ethyl-5-fluoro-N-methylpyrimidin-4-amine:

Into a 50-mL round-bottom flask, was placed 2,4-dichloro-6-ethyl-5-fluoropyrimidine (300 mg, 1.54 mmol, 1 equiv), CH₃NH₂-HCl (206 mg, 2.00 equiv), Cs₂CO₃ (1 g, 3.07 mmol, 2.00 equiv), N,N-dimethylformamide (10 mL). The resulting solution was stirred for 12 h at 80 °C. The crude product was purified by Flash-Prep-HPLC A 1:1. This resulted in 150 mg (51%) of the title compound as a light yellow solid.

Analytical Data: LC-MS: (ES, *m*/*z*): 190 [M+1], R: 0.79 min.

### Step 3: Synthesis of 6-ethyl-5-fluoro-N²-(4-methoxy-3-(3-(pyrrolidin-1-yl)propoxy)phenyl)-N⁴-methylpyrimidine-2,4-diamine:

Into a 50-mL round-bottom flask, was placed 2-chloro-6-ethyl-5-fluoro-N-methylpyrimidin-4-amine (100 mg, 0.53 mmol, 1 equiv), 4-methoxy-3-[3-(pyrrolidin-1-yl)propoxy]aniline (198 mg, 0.79 mmol, 1.50 equiv), Cs₂CO₃ (508 mg, 1.56 mmol, 3.00 equiv), Pd₂(dba)₃.CHCl₃ (50 mg), X-phos (50 mg), 1,4-dioxane (10 mL). The resulting solution was stirred for 4 h at 100 °C. The crude product was purified by Flash-Prep-HPLC with the following conditions (IntelFlash-1): Column, silica gel; mobile phase, ACN/H2O=1/1; Detector, UV 254 nm product was obtained. This resulted in 44.1 mg (21%) of 6-ethyl-5-fluoro-N²-(4-methoxy-3-(3-(pyrrolidin-1-yl)propoxy)phenyl)-N⁴-methylpyrimidine-2,4-diamine as a light yellow solid.

### Example 70: Synthesis of Compound 320

### Compound 320: Synthesis of 6-cyclopropyl-N²-(4-methoxy-3-(3-(pyrrolidin-1-yl)propoxy)phenyl)-N⁴-methylpyrimidine-2,4-diamine

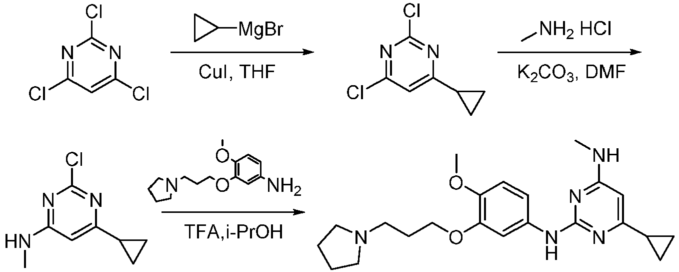

### Step 1: Synthesis of 2,4-dichloro-6-cyclopropylpyrimidine:

Into a 50-mL 3-necked round-bottom flask purged and maintained with an inert atmosphere of nitrogen, was placed 2,4,6-trichloropyrimidine (1 g, 5.45 mmol, 1 equiv), tetrahydrofuran (20 mL), CuI (110 mg, 0.58 mmol, 0.10 equiv). This was followed by the addition of bromo(cyclopropyl)magnesium (5.5 mL, 1 equiv) dropwise with stirring at 0 °C. The resulting solution was stirred for 2 h at 0 °C in a water/ice bath. The resulting solution was allowed to react, with stirring, for an additional 2 h at 25 °C. The reaction was then quenched by the addition of NH₄Cl. The resulting mixture was concentrated under vacuum. The resulting solution was extracted with 3x100 mL of ethyl acetate and the organic layers combined. The resulting mixture was washed with 2x100 mL of water and 2x100 mL of Brine. The mixture was dried over anhydrous sodium sulfate. The resulting mixture was concentrated under vacuum. The crude product was purified by Flash-Prep-HPLC A EA/PE. This resulted in 300 mg (29%) of the title compound as a yellow solid.

Analytical Data: LC-MS: (ES, *m*/*z*): RT=1.355min, LCMS 53, *m*/*z* =189 [M+1]. ¹H NMR (300 MHz, DMSO-d6) δ 7.77 (s, 1H), 2.27 - 2.13 (m, 1H), 1.29 - 1.03 (m, 4H).

### Step 2: Synthesis of 2-chloro-6-cyclopropyl-N-methylpyrimidin-4-amine:

Into a 8-mL sealed tube, was placed 2,4-dichloro-6-cyclopropylpyrimidine (200 mg, 1.06 mmol, 1 equiv), N,N-dimethylformamide (4 mL), potassium carbonate (365 mg, 2.64 mmol, 2.50 equiv), methanamine hydrochloride (72 mg, 1.07 mmol, 1 equiv). The resulting solution was stirred for 2 h at 0 °C in a water/ice bath. The resulting solution was allowed to react, with stirring, for an additional 2 h at 25 °C. The solids were filtered out. The crude product (4 mL) was purified by Flash-Prep-HPLC A Grad. This resulted in 80 mg (41%) of the title compound as a yellow solid.

Analytical Data: LC-MS: (ES, *m*/*z*): RT=0.682min, LCMS 30, *m*/*z* =184[M+1]. ¹H NMR (300 MHz, DMSO-d6) δ 7.61 (s, 1H), 6.33 (s, 1H), 2.76 (d, *J* = 4.8 Hz, 3H), 1.91 - 1.85 (m, 1H), 0.95 - 0.85 (m, 4H).

### Step 3: Synthesis of 6-cyclopropyl-N²-(4-methoxy-3-(3-(pyrrolidin-1-yl)propoxy)phenyl)-N⁴-methylpyrimidine-2,4-diamine:

Into a 8-mL sealed tube, was placed 2-chloro-6-cyclopropyl-N-methylpyrimidin-4-amine (80 mg, 0.44 mmol, 1 equiv), 4-methoxy-3-[3-(pyrrolidin-1-yl)propoxy]aniline (109 mg, 0.44 mmol, 1 equiv), isopropanol (5 mL), trifluoroacetic acid (100 mg, 0.88 mmol, 2.00 equiv). The resulting solution was stirred for 3 h at 90 °C in an oil bath. The resulting solution was extracted with of ethyl acetate and the organic layers combined. The crude product (5mL) was purified by Prep-HPLC C HCl. This resulted in 91.9 mg (49%) of 6-cyclopropyl-N²-(4-methoxy-3-(3-(pyrrolidin-1-yl)propoxy)phenyl)-N⁴-methylpyrimidine-2,4-diamine as a solid.

### Example 71: Synthesis of Compound 321 (Reference)

### Compound 321: Synthesis of N-(5-methoxy-4-(3-(pyrrolidin-1-yl)propoxy)pyridin-2-yl)imidazo[1,2-a]pyridin-3-amine

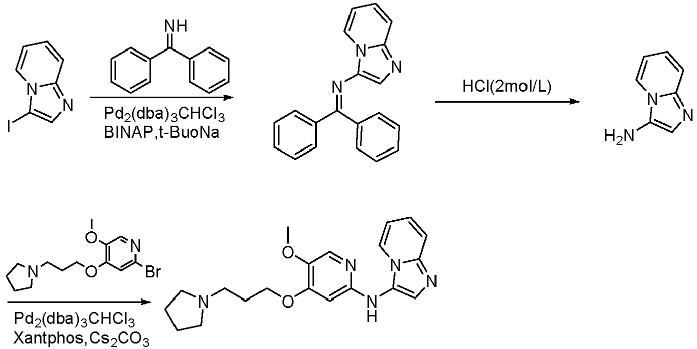

### Step 1: Synthesis of N-(diphenylmethylidene)imidazo[1,2-a]pyridin-3-amine:

Into a 100-mL 3-necked round-bottom flask, was placed toluene (20 mL), 3-iodoimidazo[1,2-a]pyridine (2 g, 8.20 mmol, 1 equiv), diphenylmethanimine (1.5 g, 8.28 mmol, 1.01 equiv), Pd₂(dba)₃CHCl₃ (1.3 g), BINAP (1.5 g, 2.41 mmol, 0.29 equiv), t-BuONa (2.4 g, 24.97 mmol, 3.05 equiv). The resulting solution was stirred for 5 h at 80 °C. The resulting solution was diluted with 10 mL of H₂O. The 3-necked round-bottom flask was purged and maintained with N₂. The resulting solution was extracted with 3x20 mL of dichloromethane and the organic layers combined and dried over anhydrous sodium sulfate and concentrated under vacuum. This resulted in 1.5 g (62%) of as a yellow solid.

Analytical Data: LC-MS: (ES, *m*/*z*): RT = 0.769 min, LCMS 32: *m*/*z* = 297 [M+1].

### Step 2: Synthesis of imidazo[1,2-a]pyridin-3-amine:

Into a 250-mL round-bottom flask, was placed HCl(2M) (30 mL), N-(diphenylmethylidene)imidazo[1,2-a]pyridin-3-amine (1.5 g, 5.04 mmol, 1 equiv). The resulting solution was stirred for 12 h at 20 °C. The resulting solution was extracted with 3x10 mL of dichloromethane and the organic layers combined. The pH value of the solution was adjusted to 10 with sodium hydroxide. The resulting mixture was washed with 3x20 mL of chloromethane2. The mixture was dried over anhydrous sodium sulfate and concentrated under vacuum. This resulted in 370 mg (55%) of the title compound as a yellow solid.

Analytical Data: LC-MS: (ES, *m*/*z*): RT = 0.290 min, LCMS 40: *m*/*z* = 133 [M+1].

### Step 3: Synthesis of N-[imidazo[1,2-a]pyridin-3-yl]-5-methoxy-4-[3-(pyrrolidin-1-yl)propoxy]pyridin-2-amine:

Into a 40-mL vial, was placed dioxane (20 mL), imidazo[1,2-a]pyridin-3-amine (180 mg, 1.35 mmol, 1 equiv), 2-bromo-5-methoxy-4-[3-(pyrrolidin-1-yl)propoxy]pyridine (469 mg, 1.49 mmol, 1.10 equiv), Pd₂(dba)3-CHCl3 (1035 mg), Xantphos (247 mg, 0.43 mmol, 0.32 equiv), Cs₂CO₃ (880 mg, 2.70 mmol, 2.00 equiv). The vial was purged and maintained with N2.The resulting solution was stirred for 12 h at 80 °C. The resulting mixture was concentrated under vacuum. The crude product (300 mg) was purified by Prep-HPLC C TFA. This resulted in 262.2 mg (40%) of N-[imidazo[1,2-a]pyridin-3-yl]-5-methoxy-4-[3-(pyrrolidin-1-yl)propoxy]pyridin-2-amine as a light brown solid.

### Example 72: Synthesis of Compound 322 (Reference)

### Compound 322: Synthesis of N³-(5-methoxy-4-(3-(pyrrolidin-1-yl)propoxy)pyridin-2-yl)-N⁵-methylpyridazine-3,5-diamine

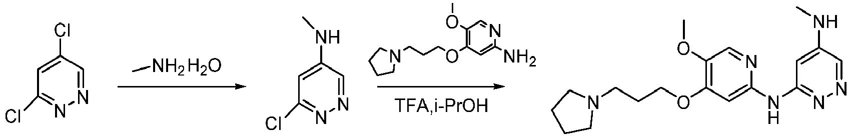

### Step 1: Synthesis of 6-chloro-N-methylpyridazin-4-amine:

Into a 20-mL sealed tube, was placed 3,5-dichloropyridazine (1 g, 6.71 mmol, 1 equiv), CH₃NH₂-H₂O (2 mL), dioxane (2 mL). The resulting solution was stirred for 2 h at 50 °C in an oil bath. The resulting solution was diluted with 2 mL of methanol. The residue was applied onto a silica gel column with CH₃CN:H₂O (1:10). This resulted in 620 mg (64%) of the title compound as a white solid.

Analytical Data: LC-MS: (ES, *m*/*z*): RT = 0.62min, LCMS07: m/z = 144.00 [M+1].

### Step 2: Synthesis of N³-(5-methoxy-4-(3-(pyrrolidin-1-yl)propoxy)pyridin-2-yl)-N⁵-methylpyridazine-3,5-diamine:

Into a 50-mL round-bottom flask, was placed 6-chloro-N-methylpyridazin-4-amine (300 mg, 2.09 mmol, 1 equiv), trifluoroacetic acid (604 mg, 5.34 mmol, 3.00 equiv), 5-methoxy-4-[3-(pyrrolidin-1-yl)propoxy]pyridin-2-amine (526.6 mg, 2.10 mmol, 1 equiv), isopropanol (5 mL). The resulting solution was stirred for 2 h at 85 °C in an oil bath. The resulting mixture was concentrated under vacuum. The crude product (300 mg) was purified by Prep-HPLC G. This resulted in 83.1 mg (8%) of N³-(5-methoxy-4-(3-(pyrrolidin-1-yl)propoxy)pyridin-2-yl)-N⁵-methylpyridazine-3,5-diamine as a white solid.

### Example 73: Synthesis of Compound 323 (Reference)

### Compound 323: Synthesis of N⁵-(5-methoxy-4-(3-(pyrrolidin-1-yl)propoxy)pyridin-2-yl)-N³-methylpyridazine-3,5-diamine

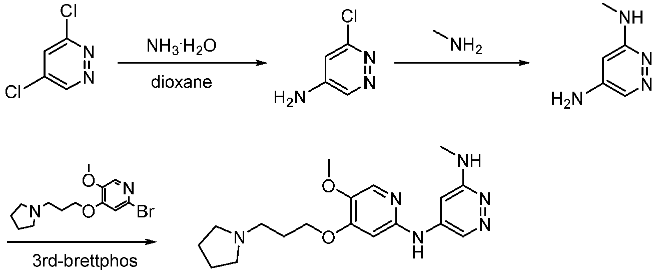

### Step 1: Synthesis 6-chloropyridazin-4-amine:

Into a 25-mL round-bottom flask, was placed 3,5-dichloropyridazine (1 g, 6.71 mmol, 1 equiv), ammonia (8 mL), dioxane (2 mL). The resulting solution was stirred overnight at 100 °C. The solids were collected by filtration. This resulted in 570 mg (62%) of the title compound as a brown solid.

Analytical Data: LC-MS: (ES, *m*/*z*): RT= 0.434 min, LCMS 53, *mlz* = 130 [M+1].

### Step 2: Synthesis of 3-N-methylpyridazine-3,5-diamine:

Into a 50-mL round-bottom flask, was placed 6-chloropyridazin-4-amine (570 mg, 4.40 mmol, 1 equiv), dioxane (20 mL), CH3NH2-H2O (4 mL). The resulting solution was stirred overnight at 140 °C. The resulting mixture was concentrated under vacuum. The crude product was purified by Flash-Prep-HPLC A. This resulted in 320 mg (59%) of the title compound as a yellow solid.

Analytical Data: LC-MS: (ES, *m*/*z*): RT= 0.187 min, LCMS 45, *m*/*z* = 125 [M+1].

### Step 3: Synthesis of N⁵-(5-methoxy-4-(3-(pyrrolidin-1-yl)propoxy)pyridin-2-yl)-N³-methylpyridazine-3,5-diamine:

Into a 100-mL round-bottom flask, was placed 3-N-methylpyridazine-3,5-diamine (250 mg, 2.01 mmol, 1 equiv), 2-bromo-5-methoxy-4-[3-(pyrrolidin-1-yl)propoxy]pyridine (628 mg, 1.99 mmol, 0.99 equiv), 3rd-Brettphos (181.2 mg), Cs₂CO₃ (1.3 g, 3.99 mmol, 1.98 equiv), DMSO (25 mL). The resulting solution was stirred for 1 h at 80 °C. The crude product was purified by Prep-HPLC C HCl. This resulted in 31.2 mg (4%) of N⁵-(5-methoxy-4-(3-(pyrrolidin-1-yl)propoxy)pyridin-2-yl)-N³-methylpyridazine-3,5-diamine as a light yellow solid.

### Example 74: Synthesis of Compound 324 (Reference)

### Compound 324: Synthesis of N⁴-(5-methoxy-4-(3-(pyrrolidin-1-yl)propoxy)pyridin-2-yl)-N⁶-methylpyrimidine-4,6-diamine

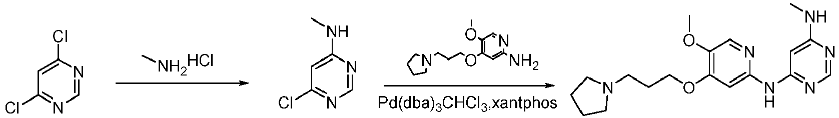

### Step 1: Synthesis of 6-chloro-N-methylpyrimidin-4-amine:

Into a 100-mL round-bottom flask, was placed N,N-dimethylformamide (10 mL), 4,6-dichloropyrimidine (1 g, 6.71 mmol, 1 equiv), Cs₂CO₃ (4.4 g, 13.50 mmol, 2.01 equiv), methanamine hydrochloride (905 mg, 13.40 mmol, 2.00 equiv). The resulting solution was stirred for 14 h at 80 °C. The resulting solution was diluted with 10 mL of H₂O. The resulting solution was extracted with 4x10 mL of ethyl acetate and the organic layers combined. The resulting mixture was washed with 1x10 mL of H₂O. The resulting mixture was concentrated under vacuum. The residue was applied onto a silica gel column with ethyl acetate/petroleum ether (1:1). This resulted in 750 mg (78%) of as a white solid.

Analytical Data: LC-MS: (ES, *m*/*z*): RT = 0.476min, LCMS 32: *m*/*z* = 144 [M+1].

### Step 2: Synthesis of N⁴-(5-methoxy-4-(3-(pyrrolidin-1-yl)propoxy)pyridin-2-yl)-N⁶-methylpyrimidine-4,6-diamine:

Into a 40-mL vial purged and maintained with an inert atmosphere of nitrogen, was placed dioxane (10 mL), 6-chloro-N-methylpyrimidin-4-amine (114 mg, 0.79 mmol, 1 equiv), 5-methoxy-4-[3-(pyrrolidin-1-yl)propoxy]pyridin-2-amine (200 mg, 0.80 mmol, 1 equiv), Pd₂(dba)3-CHCl3 (123 mg, 0.12 mmol, 0.15 equiv), xantphos (138 mg, 0.24 mmol, 0.30 equiv), Cs₂CO₃ (520 mg, 1.60 mmol, 2.01 equiv). The resulting solution was stirred for 14 h at 80 °C. The resulting mixture was concentrated under vacuum. The resulting solution was diluted with 5 mL of H₂O. The resulting solution was extracted with 3x10 mL of dichloromethane and the organic layers combined and concentrated under vacuum. The crude product (200 mg) was purified by Prep-HPLC D TFA. This resulted in 40.6 mg (11%) of N⁴-(5-methoxy-4-(3-(pyrrolidin-1-yl)propoxy)pyridin-2-yl)-N⁶-methylpyrimidine-4,6-diamine as a white solid.

### Example 75: Synthesis of Compound 325 (Reference)

### Compound 325: Synthesis of N⁴-(5-methoxy-4-(3-(pyrrolidin-1-yl)propoxy)pyridin-2-yl)-N⁶,2-dimethylpyrimidine-4,6-diamine

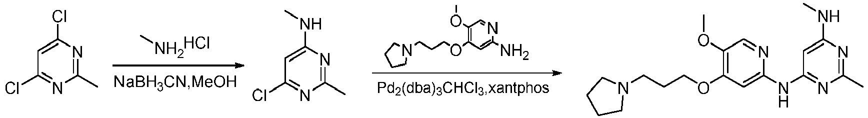

### Step 1: Synthesis of 6-chloro-N,2-dimethylpyrimidin-4-amine:

Into a 40-mL vial, was placed N,N-dimethylformamide (10 mL), 4,6-dichloro-2-methylpyrimidine (500 mg, 3.07 mmol, 1 equiv), methanamine hydrochloride (411 mg, 6.09 mmol, 1.98 equiv), Cs₂CO₃ (1.9 g, 5.83 mmol, 1.90 equiv). The resulting solution was stirred for 12 h at 80 °C. The resulting solution was diluted with 10 mL of H2O. The resulting solution was extracted with 3x20 mL of dichloromethane and the organic layers combined. The resulting mixture was washed with 3x10 mL of brine. The mixture was dried over anhydrous sodium sulfate and concentrated under vacuum. This resulted in 450 mg (93%) of the title compound as a yellow solid.

Analytical Data: LC-MS: (ES, *m*/*z*): RT = 0.763 min, LCMS 07: *m*/*z* = 157 [M+1].

### Step 2: Synthesis of N⁴-(5-methoxy-4-(3-(pyrrolidin-1-yl)propoxy)pyridin-2-yl)-N⁶,2-dimethylpyrimidine-4,6-diamine:

Into a 20-mL vial, was placed dioxane (10 mL), 6-chloro-N,2-dimethylpyrimidin-4-amine (114 mg, 0.72 mmol, 1 equiv), 5-methoxy-4-[3-(pyrrolidin-1-yl)propoxy]pyridin-2-amine (200 mg, 0.80 mmol, 1.10 equiv), Pd₂(dba)3-CHCl3 (112 mg), Xantphos (133 mg, 0.23 mmol, 0.32 equiv), Cs₂CO₃ (472 mg, 1.45 mmol, 2.00 equiv).The vial was purged and maintained with N₂. The resulting solution was stirred for 12 h at 80 °C. The resulting mixture was concentrated under vacuum. The crude product was purified by Prep-HPLC D TFA. This resulted in 48.8 mg (14%) of N⁴-(5-methoxy-4-(3-(pyrrolidin-1-yl)propoxy)pyridin-2-yl)-N⁶,2-dimethylpyrimidine-4,6-diamine as a white solid.

### Example 76: Synthesis of Compound 409 (Reference)

### Compound 409: Synthesis of N²-(3-((1-isopropylpyrrolidin-3-yl)methoxy)-4-methoxyphenyl)-N⁴-methylpyrimidine-2,4-diamine

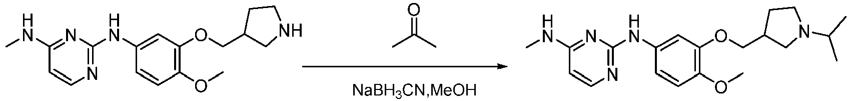

Compound 409 was synthesized as illustrated above.

### Example 77: Synthesis of Compound 326 (Reference)

### Compound 326: Synthesis of N-(5-methoxy-4-(3-(pyrrolidin-1-yl)propoxy)pyridin-2-yl)-2-methyl-7H-pyrrolo[2,3-d]pyrimidin-4-amine

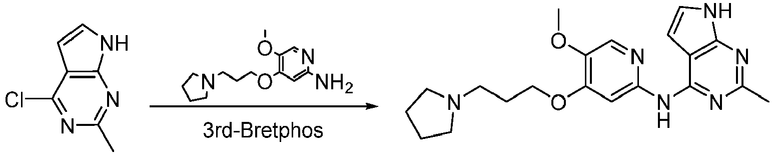

### Step 1: Synthesis of N-(5-methoxy-4-(3-(pyrrolidin-1-yl)propoxy)pyridin-2-yl)-2-methyl-7H-pyrrolo[2,3-d]pyrimidin-4-amine:

Into a 40-mL vial purged and maintained with an inert atmosphere of nitrogen, was placed dioxane (10 mL), 5-methoxy-4-[3-(pyrrolidin-1-yl)propoxy]pyridin-2-amine (200 mg, 0.80 mmol, 1 equiv), 4-chloro-2-methyl-7H-pyrrolo[2,3-d]pyrimidine (133 mg, 0.79 mmol, 1 equiv), Pd₂(dba)3-CHCl3 (124 mg, 0.12 mmol, 0.15 equiv), xantphos (138 mg, 0.24 mmol, 0.30 equiv), Cs₂CO₃ (519 mg, 1.59 mmol, 2.00 equiv). The resulting solution was stirred for 14 h at 80 °C. The solids were filtered out. The resulting mixture was concentrated under vacuum. The crude product (200 mg) was purified by Prep-HPLC D TFA. This resulted in 47.2 mg (12%) of N-(5-methoxy-4-(3-(pyrrolidin-1-yl)propoxy)pyridin-2-yl)-2-methyl-7H-pyrrolo[2,3-d]pyrimidin-4-amine as a white solid.

### Example 78: Synthesis of Compound 328

### Compound 328: Synthesis of N²-(4-methoxy-3-(3-(pyrrolidin-1-yl)propoxy)phenyl)-N⁴-methyl-6,7-dihydro-5H-cyclopenta[d]pyrimidine-2,4-diamine

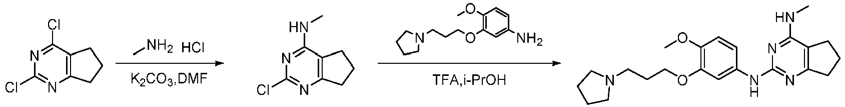

### Step 1: Synthesis of 2-chloro-N-methyl-5H,6H,7H-cyclopenta[d]pyrimidin-4-amine:

Into a 50-mL round-bottom flask, was placed 2,4-dichloro-5H,6H,7H-cyclopenta[d]pyrimidine (850 mg, 4.50 mmol, 1 equiv), potassium carbonate (1.87 g, 13.53 mmol, 3.01 equiv), N,N-dimethylformamide (5 mL), methanamine hydrochloride (303 mg, 4.49 mmol, 1 equiv). The resulting solution was stirred for 1 h at 0 °C. The solids were filtered out. The crude product was purified by Flash-Prep-HPLC A Grad. This resulted in 500 mg (61%) of the title compound as an off white solid.

Analytical Data: LC-MS: (ES, *m*/*z*): RT = 0.856 min, LCMS 45: *m*/*z* = 184.0 [M+1].¹H NMR (300 MHz, DMSO-d6) δ 8.19 (s, 1H), 2.86 (s, 3H), 2.82 - 2.70 (m, 2H), 2.63 (t, *J* = 7.5 Hz, 2H), 2.12 - 1.95 (m, 2H).

### Step 2: Synthesis of N²-(4-methoxy-3-(3-(pyrrolidin-1-yl)propoxy)phenyl)-N⁴-methyl-6,7-dihydro-5H-cyclopenta[d]pyrimidine-2,4-diamine:

Into a 50-mL round-bottom flask, was placed 2-chloro-N-methyl-5H,6H,7H-cyclopenta[d]pyrimidin-4-amine (200 mg, 1.09 mmol, 1 equiv), isopropanol (5 mL), trifluoroacetic acid (249 mg, 2.18 mmol, 2.01 equiv), 4-methoxy-3-[3-(pyrrolidin-1-yl)propoxy]aniline (273 mg, 1.09 mmol, 1 equiv). The resulting solution was stirred for 2 h at 80 °C. The crude product was purified by Prep-HPLC A. This resulted in 93.2 mg (20%) of N²-(4-methoxy-3-(3-(pyrrolidin-l-yl)propoxy)phenyl)-N⁴-methyl-6,7-dihydro-5H-cyclopenta[d]pyrimidine-2,4-diamine as an off white solid.

### Example 79: Synthesis of Compound 331

### Compound 331: Synthesis of N²-(3-((1r,3r)-3-((dimethylamino)methyl)cyclobutoxy)-4-methoxyphenyl)-N⁴,6-dimethylpyrimidine-2,4-diamine

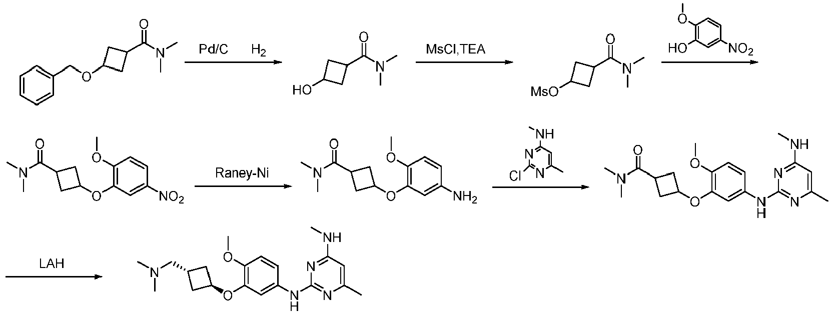

### Step 1: Synthesis of 3-hydroxy-N,N-dimethylcyclobutane-1-carboxamide:

Into a 250-mL round-bottom flask, was placed 3-(benzyloxy)-N,N-dimethylcyclobutane-1-carboxamide (3 g, 12.86 mmol, 1 equiv), methanol (100 mL), Pd/C, hydrogen . The resulting solution was stirred for 3 h at 50 °C in an oil bath. The solids were filtered out. The resulting mixture was concentrated under vacuum. This resulted in 1.8 g (98%) of the title compound as a yellow oil.

Analytical Data: LC-MS: (ES, *m*/*z*): RT = 0.56min, LCMS07: m/z =144 [M+1].

### Step 2: Synthesis of 3-(dimethylcarbamoyl)cyclobutyl methanesulfonate:

Into a 50-mL round-bottom flask, was placed 3-hydroxy-N,N-dimethylcyclobutane-1-carboxamide (900 mg, 6.29 mmol, 1 equiv), dichloromethane (10 mL), MsCl (2.1 g, 3.00 equiv), TEA (1.9 g, 18.78 mmol, 3.00 equiv). The resulting solution was stirred for 2 h at 20 °C. The reaction was then quenched by the addition of water. The resulting solution was extracted with 3x10 mL of dichloromethane and the organic layers combined. The resulting mixture was washed with 3x10 mL of H₂O. The mixture was dried over anhydrous sodium sulfate and concentrated under vacuum. This resulted in 1.5 g (108%) of the title compound as yellow oil.

Analytical Data: LC-MS: (ES, *m*/*z*): RT = 0.85min, LCMS07: m/z =222 [M+1].

### Step 3: Synthesis of 3-(2-methoxy-5-nitrophenoxy)-N,N-dimethylcyclobutane-1-carboxamide:

Into a 50-mL round-bottom flask, was placed 3-(dimethylcarbamoyl)cyclobutyl methanesulfonate (1.3 g, 5.88 mmol, 1 equiv), Cs₂CO₃ (5.75 g, 17.59 mmol, 3.00 equiv), 2-methoxy-5-nitrophenol (994 mg, 5.88 mmol, 1 equiv), N,N-dimethylformamide (10 mL). The resulting solution was stirred for 10 h at 80 °C in an oil bath. The resulting solution was diluted with 10 mL of H₂O. The resulting solution was extracted with 3x10 mL of ethyl acetate and the organic layers combined. The resulting mixture was washed with 2x10 mL of H₂O. The resulting mixture was washed with 2x10 mL of sodium chloride(aq). The mixture was dried over anhydrous sodium sulfate. The residue was applied onto a silica gel column with dichloromethane/methanol (10:1). This resulted in 1.2 g (69%) of the title compound as yellow oil.

Analytical Data: LC-MS: (ES, *m*/*z*): RT = 0.82min, LCMS32: m/z =295 [M+1].

### Step 4: Synthesis of 3-(5-amino-2-methoxyphenoxy)-N,N-dimethylcyclobutane-1-carboxamide:

Into a 250-mL round-bottom flask, was placed 3-(2-methoxy-5-nitrophenoxy)-N,N-dimethylcyclobutane-1-carboxamide (600 mg, 2.04 mmol, 1 equiv), methanol (150 mL), Raney-Ni, hydrogen. The resulting solution was stirred for 1 h at 20 °C. The solids were filtered out. The resulting mixture was concentrated under vacuum. This resulted in 480 mg (89%) of the title compound as blue green oil.

Analytical Data: LC-MS: (ES, *m*/*z*): RT = 0.79min, LCMS33: m/z =265 [M+1]. ¹H NMR (400 MHz, Methanol-d4) δ 6.76 (dd, *J* = 8.3, 3.8 Hz, 1H), 6.43 - 6.21 (m, 2H), 4.78 - 4.54 (m, 1H), 3.75 (d, *J* = 8.4 Hz, 3H), 3.55 - 3.47 (m, 1H), 3.06 - 2.92 (m, 6H), 2.77 - 2.64 (m, 2H), 2.50-2.31 (m, 2H).

### Step 5: Synthesis of 3-(2-methoxy-5-[[4-methyl-6-(methylamino)pyrimidin-2-yl]amino]phenoxy)-N,N-dimethylcyclobutane-1-carboxamide:

Into a 50-mL round-bottom flask, was placed 3-(5-amino-2-methoxyphenoxy)-N,N-dimethylcyclobutane-1-carboxamide (467 mg, 1.77 mmol, 1 equiv), 2-chloro-N,6-dimethylpyrimidin-4-amine (277 mg, 1.76 mmol, 1 equiv), IPA (10 mL), trifluoroacetic acid (514.7 mg, 4.55 mmol, 3.00 equiv). The resulting solution was stirred for 2 h at 85 °C in an oil bath. The resulting mixture was concentrated under vacuum. This resulted in 967 mg (>100% crude) of the title compound as yellow oil.

Analytical Data: LC-MS: (ES, *m*/*z*): RT = 0.89min, LCMS07: m/z =386 [M+1].

### Step 6: Synthesis of N²-(3-((1r,3r)-3-((dimethylamino)methyl)cyclobutoxy)-4-methoxyphenyl)-N⁴,6-dimethylpyrimidine-2,4-diamine:

Into a 50-mL round-bottom flask, was placed 3-(2-methoxy-5-[[4-methyl-6-(methylamino)pyrimidin-2-yl] amino]phenoxy)-N,N-dimethylcyclobutane-1-carboxamide (200 mg, 0.52 mmol, 1 equiv), oxolane (0 mg), LAH (78.96 mg, 2.08 mmol, 4.00 equiv). The resulting solution was stirred for 2 h at 0 °C in a water/ice bath. The reaction was then quenched by the addition of 200 mg of water/ice. The pH value of the solution was adjusted to 8 with sodium hydroxide(aq) (10 mol/L). The resulting solution was extracted with 20 mL of ethyl acetate and the organic layers combined and dried in an oven under reduced pressure. The solids were filtered out. The crude product (400 mg) was purified by Prep-HPLC C HCl. The crude product (300 mg) was purified by Chiral-Prep-HPLC IC. This resulted in 66.4 mg (34%) of N²-(3-((1r,3r)-3-((dimethylamino)methyl)cyclobutoxy)-4-methoxyphenyl)-N⁴,6-dimethylpyrimidine-2,4-diamine as a white solid.

### Example 80: Synthesis of Compound 332 (Reference)

### Compound 332: Synthesis of N²-(6-methoxy-5-(3-(pyrrolidin-1-yl)propoxy)pyridin-3-yl)-N⁴,6-dimethylpyrimidine-2,4-diamine

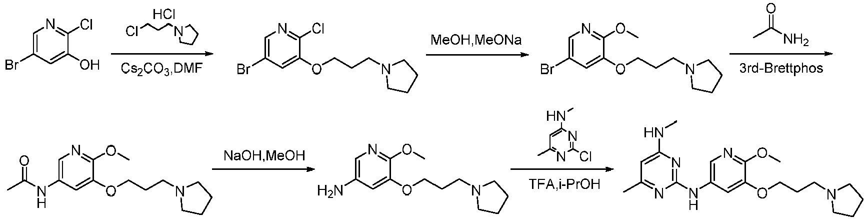

### Step 1: Synthesis of 5-bromo-2-chloro-3-[3-(pyrrolidin-1-yl)propoxy]pyridine:

Into a 100-mL round-bottom flask, was placed 5-bromo-2-chloropyridin-3-ol (1.1 g, 5.28 mmol, 1 equiv), Cs₂CO₃ (5.3 g, 16.27 mmol, 3.08 equiv), N,N-dimethylformamide (10 mL), 1-(3-chloropropyl)pyrrolidine hydrochloride (1 g, 5.43 mmol, 1.03 equiv). The resulting solution was stirred for 2 h at 80 °C. The reaction was then quenched by the addition of water. The resulting solution was extracted with 3x50 mL of ethyl acetate and the organic layers combined. The resulting mixture was washed with 50 mL of brine. The mixture was dried over anhydrous sodium sulfate and concentrated under vacuum. This resulted in 1.05 g (62%) of as a light yellow solid.

Analytical Data: LC-MS: (ES, *m*/*z*): RT = 0.827min, LCMS 45: *mlz*= 319.00 [M+1]. ¹H-NMR: (300 MHz, Chloroform-d) δ 8.05 (d, *J* = 2.0 Hz, 1H), 7.41 (d, *J* = 2.0 Hz, 1H), 4.15 (t, *J* = 6.3 Hz, 2H), 2.73 - 2.46 (m, 6H), 2.17 - 1.93 (m, 2H), 1.92 - 1.73 (m, 4H).

### Step 2: Synthesis of 5-bromo-2-methoxy-3-[3-(pyrrolidin-1-yl)propoxy]pyridine:

Into a 50-mL round-bottom flask, was placed 5-bromo-2-chloro-3-[3-(pyrrolidin-1-yl)propoxy]pyridine (1 g, 3.13 mmol, 1 equiv), methanol (10 mL), methoxysodium (849 mg, 15.72 mmol, 5.02 equiv). The resulting solution was stirred for 48 h at 70 °C. The reaction was then quenched by the addition of water. The resulting solution was extracted with 3x100 mL of ethyl acetate and the organic layers combined. The resulting mixture was washed with 100 mL of sodium chloride. The mixture was dried over anhydrous sodium sulfate and concentrated under vacuum. This resulted in 910 mg (92%) of as a light yellow liquid.

Analytical data: LC-MS: (ES, *m*/*z*): RT = 13min, LCMS 31: *m*/*z*= 315.35 [M+1]. ¹H-NMR: (300 MHz, Chloroform-d) δ 7.77 (d, *J* = 2.0 Hz, 1H), 7.23 (d, *J* = 2.1 Hz, 1H), 4.12 (t, *J* = 6.5 Hz, 2H), 3.98 (s, 3H), 2.62 - 2.51 (m, 6H), 2.15 - 2.02 (m, 2H), 1.85 - 1.78 (m, 4H).

### Step3: Synthesis of N-[6-methoxy-5-[3-(pyrrolidin-1-yl)propoxy]pyridin-3-yl]acetamide:

Into a 50-mL round-bottom flask purged and maintained with an inert atmosphere of nitrogen, was placed 5-bromo-2-methoxy-3-[3-(pyrrolidin-1-yl)propoxy]pyridine (870 mg, 2.76 mmol, 1 equiv), Cs₂CO₃ (2.7 g, 8.29 mmol, 3.00 equiv), 3rd-Brettphos (251 mg), dioxane (5 mL), acetamide (245 mg, 4.15 mmol, 1.50 equiv). The resulting solution was stirred for 16 h at 65 °C. The solids were filtered out. The crude product was purified by Flash-Prep-HPLC A Grad. This resulted in 200 mg (25%) of as a light yellow solid.

Analytical Data: LC-MS: (ES, *m*/*z*): RT = 0.541min, LCMS45: *mlz*= 294.10 [M+1]. ¹H-NMR-PH-EPISOK-350-4: (300 MHz, Deuterium Oxide) δ 7.57 (d, *J* = 2.1Hz, 1H), 7.33 (d, *J* = 2.1 Hz, 1H), 4.09 - 3.97 (m, 1H), 3.85 (s, 3H), 3.08 - 2.87 (m, 2H), 2.23 - 1.80 (m, 9H).

### Step 4: Synthesis of 6-methoxy-5-[3-(pyrrolidin-1-yl)propoxy]pyridin-3-amine:

Into a 100-mL round-bottom flask, was placed N-[6-methoxy-5-[3-(pyrrolidin-1-yl)propoxy]pyridin-3-yl]acetamide (180 mg, 0.61 mmol, 1 equiv), potassium hydroxide (172 mg, 3.07 mmol, 5.00 equiv), water (5 mL), methanol (5 mL). The resulting solution was stirred for 16 h at 60 °C. The resulting solution was extracted with 4x50 mL of dichloromethane and the organic layers combined and dried over anhydrous sodium sulfate and concentrated under vacuum. This resulted in 110 mg (71%) of the title compound as a light yellow solid.

Analytical Data: LC-MS: (ES, *m*/*z*): RT = 0.414min, LCMS53: *mlz*= 252.20 [M+1]. ¹H-NMR: (300 MHz, Methanol-d4) δ 7.19 (d, *J* = 2.4 Hz, 1H), 6.81 (d, *J* = 2.3 Hz, 1H), 4.15 - 3.92 (m, 2H), 3.86 (s, 3H), 2.78 - 2.51 (m, 4H), 2.04 (m, 2H), 1.95 - 1.70 (m, 9H).

### Step 5: Synthesis of N²-(6-methoxy-5-(3-(pyrrolidin-1-yl)propoxy)pyridin-3-yl)-N⁴,6-dimethylpyrimidine-2,4-diamine:

Into a 25-mL round-bottom flask, was placed 6-methoxy-5-[3-(pyrrolidin-1-yl)propoxy]pyridin-3-amine (100 mg, 0.40 mmol, 1 equiv), isopropanol (5 mL), trifluoroacetic acid (91 mg, 0.80 mmol, 2.01 equiv), 2-chloro-N,6-dimethylpyrimidin-4-amine (63 mg, 0.40 mmol, 1 equiv). The resulting solution was stirred for 2 h at 80 °C. The crude product was purified by Prep-HPLC C HCl. This resulted in 71 mg (44%) of N²-(6-methoxy-5-(3-(pyrrolidin-1-yl)propoxy)pyridin-3-yl)-N⁴,6-dimethylpyrimidine-2,4-diamine as an off-white solid.

### Example 81: Synthesis of Compound 334

### Compound 334: Synthesis of N²-(3-((1-ethylazetidin-3-yl)methoxy)-4-methoxyphenyl)-N⁴,6-dimethylpyrimidine-2,4-diamine

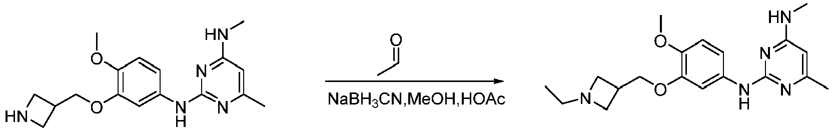

### Step 1: Synthesis of N²-(3-((1-ethylazetidin-3-yl)methoxy)-4-methoxyphenyl)-N⁴,6-dimethylpyrimidine-2,4-diamine:

Into a 100-mL round-bottom flask, was placed 2-N-[3-(azetidin-3-ylmethoxy)-4-methoxyphenyl]-4-N,6-dimethylpyrimidine-2,4-diamine (300 mg, 0.91 mmol, 1 equiv), acetaldehyde (32.1 mg, 0.73 mmol, 0.80 equiv), methanol (15 mL), NaBH₃CN (344.68 mg, 5.49 mmol, 6.00 equiv), HOAC (0.002 mL). The resulting solution was stirred for 20 min at 25 °C. The resulting solution was allowed to react, with stirring, for an additional 2 h at 25 °C. The resulting mixture was concentrated under vacuum. The crude product was purified by Prep-HPLC D TFA. This resulted in 32.4 mg (8%) of N²-(3-((1-ethylazetidin-3-yl)methoxy)-4-methoxyphenyl)-N⁴,6-dimethylpyrimidine-2,4-diamine as a white solid.

### Example 82: Synthesis of Compound 335 (Reference)

### Compound 335: Synthesis of N-(5-methoxy-4-(3-(pyrrolidin-1-yl)propoxy)pyridine-2-yl)-1H-pyrrolo[2,3-b]pyridin-4-amine

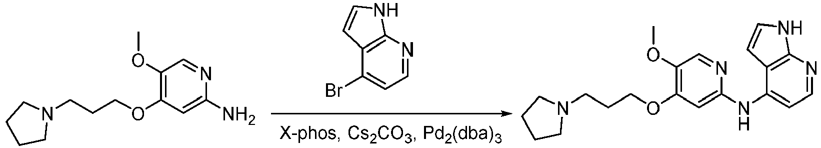

### Step 1: Synthesis of N-(5-methoxy-4-(3-(pyrrolidin-1-yl)propoxy)pyridin-2-yl)-1H-pyrrolo[2,3-b]pyridin-4-amine:

Into a 50-mL round-bottom flask, was placed 5-methoxy-4-[3-(pyrrolidin-1-yl)propoxy]pyridin-2-amine (128 mg, 0.51 mmol, 1 equiv), 4-bromo-1H-pyrrolo[2,3-b]pyridine (100 mg, 0.51 mmol, 1 equiv), Cs₂CO₃ (496 mg, 1.52 mmol, 3.00 equiv), Pd₂(dba)₃-CHCl₃ (50 mg), X-phos (50 mg), 1,4-dioxane (10 mL). The resulting solution was stirred for 4 h at 100 °C. The crude product was purified by Flash-Prep A 1:1. This resulted in 35.8 mg (15%) of N-(5-methoxy-4-(3-(pyrrolidin-1-yl)propoxy)pyridin-2-yl)-1H-pyrrolo[2,3-b]pyridin-4-amine as a white solid.

### Example 83: Synthesis of Compound 336 (Reference)

### Compound 336: Synthesis of N-(5-methoxy-4-(3-(pyrrolidin-1-yl)propoxy)pyridin-2-yl)-6-methyl-1H-pyrrolo[2,3-b]pyridin-4-amine

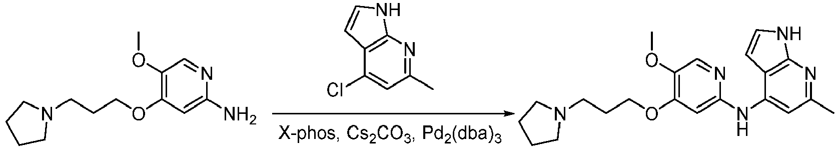

### Step 1: Synthesis of N-(5-methoxy-4-(3-(pyrrolidin-1-yl)propoxy)pyridin-2-yl)-6-methyl-1H-pyrrolo[2,3-b]pyridin-4-amine:

Into a 50-mL round-bottom flask, was placed 4-chloro-6-methyl-1H-pyrrolo[2,3-b]pyridine (150 mg, 0.90 mmol, 1 equiv), 5-methoxy-4-[3-(pyrrolidin-1-yl)propoxy]pyridin-2-amine (228 mg, 0.91 mmol, 1 equiv), Cs₂CO₃ (884 mg, 2.71 mmol, 3.00 equiv), Pd₂(dba)₃-CHCl₃ (50 mg), X-phos (50 mg), 1,4-dioxane (10 mL). The resulting solution was stirred for 4 h at 100 °C. The crude product was purified by Flash-Prep-HPLC A 1:1. This resulted in 35.9 mg (9.5%) of N-(5-methoxy-4-(3-(pyrrolidin-1-yl)propoxy)pyridin-2-yl)-6-methyl-1H-pyrrolo[2,3-b]pyridin-4-amine as a light yellow solid.

### Example 84: Synthesis of Compound 388 (Reference)

### Compound 388: Synthesis of N²-(1-(3-(dimethylamino)propyl)-1H-indazol-6-yl)-N⁴-methylpyrimidine-2,4-diamine

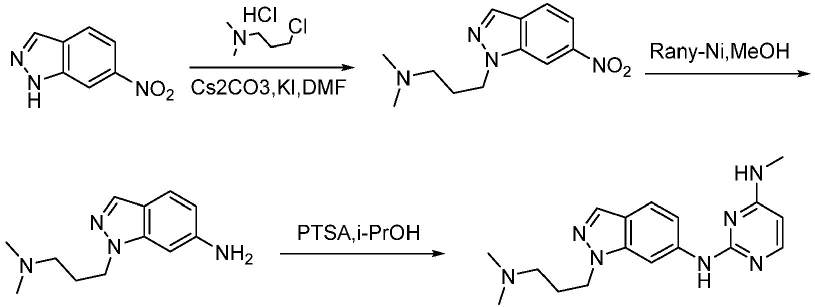

### Step 1: Synthesis of dimethyl[3-(6-nitro-2H-indazol-2-yl)propyl]amine:

Into a 40-mL vial, was placed N,N-dimethylformamide (20 mL), 6-nitro-1H-indazole (1 g, 6.13 mmol, 1 equiv), (3-chloropropyl)dimethylamine hydrochloride (963 mg, 6.09 mmol, 0.99 equiv), Cs₂CO₃ (4 g, 12.28 mmol, 2.00 equiv), KI (1 g). The resulting solution was stirred for 12 h at 60 °C. The resulting solution was diluted with 20 mL of H2O. The resulting solution was extracted with 3x20 mL of ethyl acetate and the organic layers combined. The resulting mixture was washed with 3x20 mL of water and 3x20 mL of brine. The resulting mixture was concentrated under vacuum. The residue was applied onto a silica gel column with dichloromethane/methanol (50:1). This resulted in 200 mg (13%) of the title compound as yellow oil.

Analytical Data: LC-MS: (ES, *m*/*z*): RT =0.861 min, LCMS 07: *m*/*z* = 249 [M+1].

### Step 2: Synthesis of 2-[3-(dimethylamino)propyl]-2H-indazol-6-amine:

Into a 100-mL round-bottom flask, was placed methanol (30 mL), Raney-Ni (40 mg), dimethyl[3-(6-nitro-2H-indazol-2-yl)propyl]amine (200 mg, 0.81 mmol, 1 equiv), hydrogen. The resulting solution was stirred for 2 h at 20 °C. The solids were filtered out. The flask was purged and maintained with H₂.The resulting mixture was concentrated under vacuum. This resulted in 190 mg (108%) of the title compound as yellow oil.

Analytical Data: LC-MS: (ES, *m*/*z*): RT =0.322 min, LCMS 33: *m*/*z* = 219 [M+1].

### Step 3: Synthesis of N²-(1-(3-(dimethylamino)propyl)-1H-indazol-6-yl)-N⁴-methylpyrimidine-2,4-diamine:

Into a 20-mL vial, was placed isopropanol (2 mL), 2-[3-(dimethylamino)propyl]-2H-indazol-6-amine (150 mg, 0.69 mmol, 1 equiv), 2-chloro-N-methylpyrimidin-4-amine (109 mg, 0.76 mmol, 1.10 equiv), PTSA (118 mg, 0.69 mmol, 1 equiv). The resulting solution was stirred for 12 h at 80 °C. The resulting mixture was concentrated under vacuum. The crude product (100 mg) was purified by Prep-HPLC G. This resulted in 35.1 mg (14%) of N²-(1-(3-(dimethylamino)propyl)-1H-indazol-6-yl)-N⁴-methylpyrimidine-2,4-diamine as a yellow solid.

### Example 85: Synthesis of Compound 404 (Reference)

### Compound 404: Synthesis of N²-(2-(2-(dimethylamino)ethyl)-2H-indazol-6-yl)-N⁴-methylpyrimidine-2,4-diamine

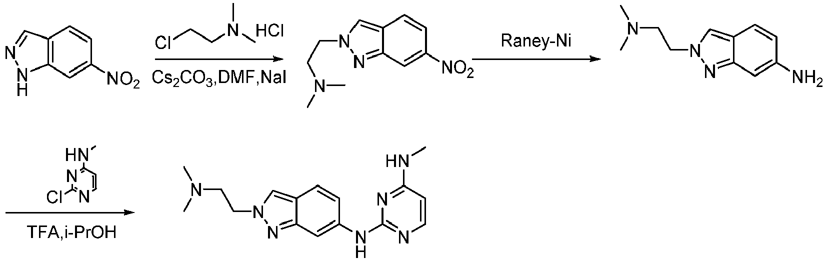

### Step 1: Synthesis of dimethyl[2-(6-nitro-2H-indazol-2-yl)ethyl]amine:

Into a 100-mL round-bottom flask, was placed 6-nitro-1H-indazole (1 g, 6.13 mmol, 1 equiv), N,N-dimethylformamide (10 mL), Cs₂CO₃ (8 g, 24.48 mmol, 3.99 equiv), iodosodium (920 mg, 6.14 mmol, 1 equiv). Reaction 30 min at RT. And then added (2-chloroethyl)dimethylamine hydrochloride (1.75 g, 12.15 mmol, 1.98 equiv). The resulting solution was stirred for 16 h at 60 °C. The reaction was then quenched by the addition of water. The resulting solution was extracted with 3x100 mL of ethyl acetate and the organic layers combined. The resulting mixture was washed with 3x mL of sodium chloride. The mixture was dried over anhydrous sodium sulfate and concentrated under vacuum. This resulted in 480 mg (33%) of the title compound as a light yellow solid.

Analytical Data: LC-MS: (ES, *m*/*z*): RT=0.629min, LCMS45: *mlz*= 235.10 [M+1].

### Step 2: Synthesis of 2-[2-(dimethylamino)ethyl]-2H-indazol-6-amine:

Into a 100-mL round-bottom flask purged and maintained with H₂, was placed dimethyl[2-(6-nitro-2H-indazol-2-yl)ethyl]amine (480 mg, 2.05 mmol, 1 equiv), Raney-Ni (50 mg), methanol (10 mL). The resulting solution was stirred for 2 h at RT. The solids were filtered out. The resulting mixture was concentrated under vacuum. This resulted in 380 mg (91%) of the title compound as a light yellow solid.

Analytical Data: LC-MS: (ES, *m*/*z*): RT = 0.373min, LCMS31: *mlz*= 205.48 [M+1].

### Step 3: Synthesis of N²-(2-(2-(dimethylamino)ethyl)-2H-indazol-6-yl)-N⁴-methylpyrimidine-2,4-diamine:

Into a 50-mL round-bottom flask, was placed 2-[2-(dimethylamino)ethyl]-2H-indazol-6-amine (370 mg, 1.81 mmol, 1 equiv), isopropanol (5 mL), trifluoroacetic acid (414 mg, 3.63 mmol, 2.00 equiv), 2-chloro-N-methylpyrimidin-4-amine (259 mg, 1.80 mmol, 1 equiv). The resulting solution was stirred for 2 h at 80 °C. The crude product was purified by Prep-HPLC F HCl. This resulted in 73 mg (12%) of N²-(2-(2-(dimethylamino)ethyl)-2H-indazol-6-yl)-N⁴-methylpyrimidine-2,4-diamine as a light yellow solid.

### Example 86: Synthesis of Compound 407 (Reference)

### Compound 407: Synthesis of N²-(4-methoxy-3-(((1-methylpyrrolidin-3-yl)oxy)methyl)phenyl)-N⁴-methylpyrimidine-2,4-diamine

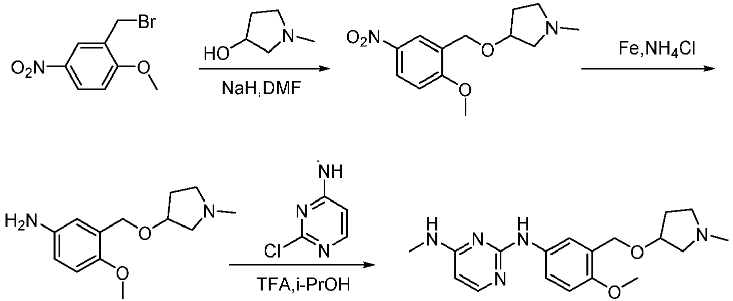

### Step 1: Synthesis of 3-[(2-methoxy-5-nitrophenyl)methoxy]-1-methylpyrrolidine:

Into a 250-mL round-bottom flask, was placed 1-methylpyrrolidin-3-ol (900 mg, 8.90 mmol, 1.10 equiv), N,N-dimethylformamide (30 mL). This was followed by the addition of sodium hydride (1.96 g, 81.67 mmol, 6.00 equiv) in several batches at 0 °C. 60%.The resulting solution was stirred for 30 min at 0 °C in a water/ice bath. To this was added 2-(bromomethyl)-1-methoxy-4-nitrobenzene (2 g, 8.13 mmol, 1 equiv). The resulting solution was allowed to react, with stirring, for an additional 2 h while the temperature was maintained at 20 °C in an oil bath. The reaction was then quenched by the addition of 60 mL of water. The resulting solution was extracted with 3x100 mL of ethyl acetate and the organic layers combined and dried in an oven under reduced pressure. This resulted in 740 mg (34%) of the title compound as an oil.

Analytical Data: LC-MS: (ES, *m*/*z*): RT = 0.923 min, LCMS34: m/z = 267.2 [M+1]. ¹H NMR (300 MHz, Methanol-*d*₄) δ 8.36 - 8.14 (m, 2H), 7.17 - 7.07 (m, 1H), 4.63 - 4.47 (m, 2H), 3.99 (d, *J* = 1.2 Hz, 3H), 2.85 - 2.70 (m, 2H), 2.58 - 2.36 (m, 5H), 2.31 - 2.12 (m, 2H), 2.09 - 1.90 (m, 1H).

### Step 2: Synthesis of 4-methoxy-3-[[(1-methylpyrrolidin-3-yl)oxy]methyl]aniline:

Into a 50-mL round-bottom flask, was placed 3-[(2-methoxy-5-nitrophenyl)methoxy]-1-methylpyrrolidine (700 mg, 2.63 mmol, 1 equiv), Fe (735.0 mg, 13.12 mmol, 5.00 equiv), NH₄Cl (714 mg, 13.35 mmol, 5.00 equiv), water(3 mL), ethanol (10 mL). The resulting solution was stirred for 2 h at 80 °C in an oil bath. The solids were filtered out. The crude product was purified by Flash-Prep-HPLC A. This resulted in 1.5 g (crude) of the title compound as a crude solid.

Analytical Data: LC-MS: (ES, *m*/*z*): RT = 0.687 min, LCMS53: m/z = 237.2 [M+1].

### Step 3: Synthesis of N²-(4-methoxy-3-(((1-methylpyrrolidin-3-yl)oxy)methyl)phenyl)-N⁴-methylpyrimidine-2,4-diamine:

Into a 50-mL round-bottom flask, was placed 4-methoxy-3-[[(1-methylpyrrolidin-3-yl)oxy]methyl]aniline (200 mg, 0.85 mmol, 1 equiv), 2-chloro-N-methylpyrimidin-4-amine (121.2 mg, 0.84 mmol, 1 equiv), isopropanol (5 mL), trifluoroacetic acid (193.2 mg, 1.71 mmol, 2.00 equiv). The resulting solution was stirred for 12 h at 85 °C in an oil bath. The crude product was purified by Prep-HPLC C HCl. This resulted in 49.0 mg (15%) of N²-(4-methoxy-3-(((1-methylpyrrolidin-3-yl)oxy)methyl)phenyl)-N⁴-methylpyrimidine-2,4-diamine as an oil.

### Example 87: Synthesis of Compound 408 (Reference)

### Compound 408: Synthesis of N²-(3-((1-ethylpyrrolidin-3-yl)methoxy)-4-methoxyphenyl)-N⁴-methylpyrimidine-2,4-diamine

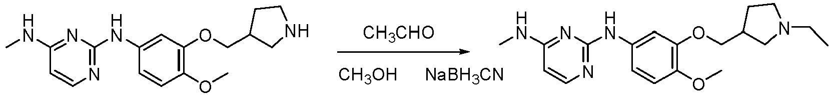

### Step 1: Synthesis of N²-(3-((1-ethylpyrrolidin-3-yl)methoxy)-4-methoxyphenyl)-N⁴-methylpyrimidine-2,4-diamine:

Into a 50-mL round-bottom flask, was placed 2-N-[4-methoxy-3-(pyrrolidin-3-ylmethoxy)phenyl]-4-N-methylpyrimidine-2,4-diamine (200 mg, 0.61 mmol, 1 equiv), methanol (10 mL), NaBH₃CN (114.9 mg, 1.83 mmol, 3.00 equiv), acetaldehyde (26.7 mg, 0.61 mmol, 1 equiv). The resulting solution was stirred for 2 h at 20 °C. The reaction was then quenched by the addition of water/ice. The resulting mixture was concentrated under vacuum. The crude product (200 mg) was purified by Prep-HPLC C TFA. This resulted in 29 mg (12%) of N²-(3-((1-ethylpyrrolidin-3-yl)methoxy)-4-methoxyphenyl)-N⁴-methylpyrimidine-2,4-diamine as a white solid.

### Example 88: Synthesis of Compound 410 (Reference)

### Compound 410: Synthesis of N²-(4-methoxy-3-((1-(2-methoxyethyl)pyrrolidin-3-yl)methoxy)phenyl)-N⁴-methylpyrimidine-2,4-diamine

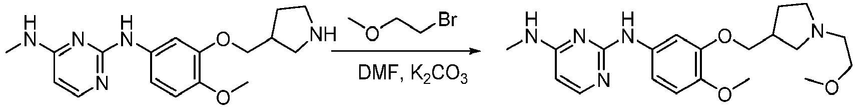

### Step 1: Synthesis of N²-(4-methoxy-3-((1-(2-methoxyethyl)pyrrolidin-3-yl)methoxy)phenyl)-N⁴-methylpyrimidine-2,4-diamine:

Into a 30-mL sealed tube, was placed 2-N-[4-methoxy-3-(pyrrolidin-3-ylmethoxy)phenyl]-4-N-methylpyrimidine-2,4-diamine (200 mg, 0.61 mmol, 1 equiv), N,N-dimethylformamide (10 mL), potassium carbonate (252 mg, 1.82 mmol, 3.00 equiv), 1-bromo-2-methoxyethane (101 mg, 0.73 mmol, 1.20 equiv). The resulting solution was stirred for 12 h at 50 °C in an oil bath. The crude product was purified by Prep-HPLC C TFA. This resulted in 60.9 mg (20%) of N²-(4-methoxy-3-((1-(2-methoxyethyl)pyrrolidin-3-yl)methoxy)phenyl)-N⁴-methylpyrimidine-2,4-diamine as a white solid.

### Example 89: Synthesis of Compound 411 (Reference)

### Compound 411: Synthesis of N²-(3-((1-cyclopropylpyrrolidin-3-yl)methoxy)-4-methoxyphenyl)-N⁴-methylpyrimidine-2,4-diamine

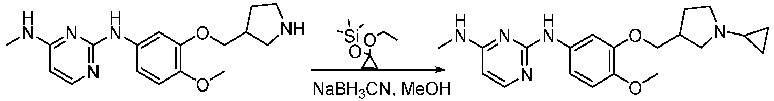

### Step 1: Synthesis of N²-(3-((1-cyclopropylpyrrolidin-3-yl)methoxy)-4-methoxyphenyl)-N⁴-methylpyrimidine-2,4-diamine:

Into a 25-mL round-bottom flask, was placed 2-N-[4-methoxy-3-(pyrrolidin-3-ylmethoxy)phenyl]-4-N-methylpyrimidine-2,4-diamine (250 mg, 0.76 mmol, 1 equiv), methanol (10 mL), (1-ethoxycyclopropoxy)trimethylsilane (200 mg, 1.15 mmol, 1.50 equiv), NaBH₃CN (144 mg, 2.29 mmol, 3.00 equiv), AcOH (0.2 mL). The resulting solution was stirred for 16 h at 65 °C in an oil bath. The reaction was then quenched by the addition of water. The resulting mixture was concentrated under vacuum. The crude product was purified by Prep-HPLC C TFA. This resulted in 95.3 mg (26%) of N²-(3-((1-cyclopropylpyrrolidin-3-yl)methoxy)-4-methoxyphenyl)-N⁴-methylpyrimidine-2,4-diamine as a white solid.

### Example 90: Synthesis of Compound 412 (Reference)

### Compound 412: Synthesis of N²-(4-methoxy-3-((1-methylpiperidin-3-yl)methoxy)phenyl)-N⁴-methylpyrimidine-2,4-diamine

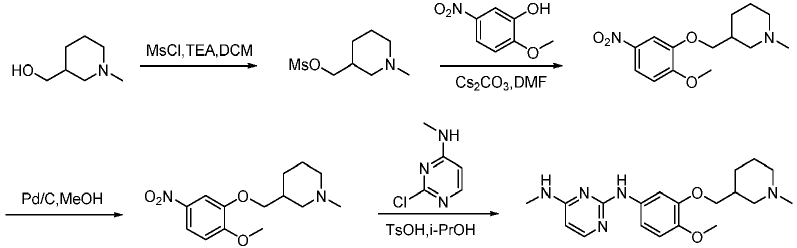

### Step 1: Synthesis of (1-methylpiperidin-3-yl)methyl methanesulfonate:

Into a 100-mL round-bottom flask, was placed (1-methylpiperidin-3-yl)methanol (1 g, 7.74 mmol, 1 equiv), dichloromethane (20 mL), TEA (2.349 g, 23.21 mmol, 3.00 equiv), MsCl (1.326 g, 11.63 mmol, 1.50 equiv). The resulting solution was stirred for 2 h at 20 °C. The resulting mixture was concentrated under vacuum. This resulted in 1.6 g (100%) of the title compound as a yellow solid.

### Step 2: Synthesis of 3-(2-methoxy-5-nitrophenoxymethyl)-1-methylpiperidine:

Into a 50-mL round-bottom flask, was placed 2-methoxy-5-nitrophenol (1.09 g, 6.44 mmol, 1 equiv), (1-methylpiperidin-3-yl)methyl methanesulfonate (1.6 g, 7.72 mmol, 1.20 equiv), Cs₂CO₃ (4.21 g, 12.92 mmol, 2.00 equiv), N,N-dimethylformamide (10 mL). The resulting solution was stirred for 24 h at 90 °C in an oil bath. The resulting solution was extracted with 3x50 mL of ethyl acetate and the organic layers combined. The crude product was purified by Flash-Prep-HPLC A Grad. This resulted in 900 mg (50%) of the title compound as an oil.

Analytical Data: LC-MS: (ES, *m*/*z*): RT = 0.901 min, LCMS07: m/z = 281.15 [M+1]. ¹H NMR (300 MHz, Methanol-*d*₄) δ 7.96 - 7.92 (m, 1H), 7.80 (d, *J* = 2.7 Hz, 1H), 7.13 (d, *J* = 9.0 Hz, 1H), 4.08 - 3.88 (m, 5H), 3.11 - 3.07 (m, 1H), 2.89 - 2.85 (m, 1H), 2.33 (s, 3H), 2.26 - 1.49 (m, 6H), 1.29 - 1.14 (m, 1H).

### Step 3: Synthesis of 4-methoxy-3-[(1-methylpiperidin-3-yl)methoxy]aniline:

Into a 100-mL round-bottom flask purged and maintained with an inert atmosphere of H₂, was placed 3-(2-methoxy-5-nitrophenoxymethyl)-1-methylpiperidine (900 mg, 3.21 mmol, 1 equiv), methanol (20 mL), Pd/C (300 mg). The resulting solution was stirred for 2 h at 20 °C. The solids were filtered out. The resulting mixture was concentrated under vacuum. This resulted in 800 mg (100%) of as dark red oil.

Analytical Data: LC-MS: (ES, *m*/*z*): RT = 0.149 min, LCMS48: m/z = 281.2 [M+1].

### Step 4: Synthesis of N²-(4-methoxy-3-((1-methylpiperidin-3-yl)methoxy)phenyl)-N⁴-methylpyrimidine-2,4-diamine:

Into a 50-mL round-bottom flask, was placed 4-methoxy-3-[(1-methylpiperidin-3-yl)methoxy]aniline (300 mg, 1.20 mmol, 1 equiv), 2-chloro-N-methylpyrimidin-4-amine (170.9 mg, 1.19 mmol, 1 equiv), isopropanol (5 mL), trifluoroacetic acid (272.5 mg, 2.41 mmol, 2.00 equiv). The resulting solution was stirred for 12 h at 85 °C in an oil bath. The crude product was purified by Prep-HPLC C HCl. This resulted in 93.5 mg (20%) of N²-(4-methoxy-3-((1-methylpiperidin-3-yl)methoxy)phenyl)-N⁴-methylpyrimidine-2,4-diamine as an off-white solid.

### Example 91: Synthesis of Compound 413

### Compound 413: Synthesis of N²-(4-methoxy-3-((1-methylpiperidin-3-yl)methoxy)phenyl)-N⁴,6-dimethylpyrimidine-2,4-diamine

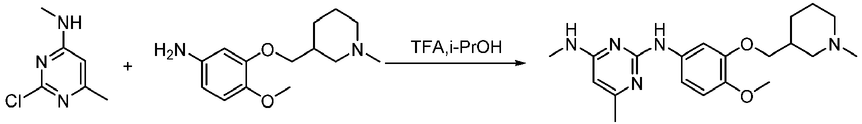

### Step 1: Synthesis of N²-(4-methoxy-3-((1-methylpiperidin-3-yl)methoxy)phenyl)-N⁴,6-dimethylpyrimidine-2,4-diamine:

Into a 50-mL round-bottom flask, was placed 4-methoxy-3-[(1-methylpiperidin-3-yl)methoxy]aniline (300 mg, 1.20 mmol, 1 equiv), 2-chloro-N,6-dimethylpyrimidin-4-amine (187.6 mg, 1.19 mmol, 1 equiv), isopropanol (5 mL), trifluoroacetic acid (272.5 mg, 2.41 mmol, 2.00 equiv). The resulting solution was stirred for 12 h at 85 °C in an oil bath. The crude product was purified by Prep-HPLC G NH₄HCO₃. This resulted in 43.1 mg (10%) of N²-(4-methoxy-3-((1-methylpiperidin-3-yl)methoxy)phenyl)-N⁴,6-dimethylpyrimidine-2,4-diamine as an off-white solid.

### Example 92: Synthesis of Compound 414 (Reference)

### Compound 414: Synthesis of 1-(3-(2-methoxy-5-((4-(methylamino)pyrimidin-2-yl)amino)phenoxy)propyl)azetidin-3-ol

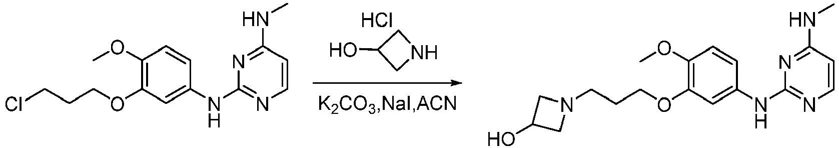

### Step 1: Synthesis of 1-(3-(2-methoxy-5-((4-(methylamino)pyrimidin-2-yl)amino)phenoxy)propyl)azetidin-3-ol:

Into a 50-mL round-bottom flask, was placed 2-N-[3-(3-chloropropoxy)-4-methoxyphenyl]-4-N-methylpyrimidine-2,4-diamine (200 mg, 0.62 mmol, 1 equiv), NaI (93 mg), potassium carbonate (514 mg, 3.72 mmol, 6.00 equiv), ACN (10 mL), azetidin-3-ol hydrochloride (203 mg, 1.85 mmol, 2.99 equiv). The resulting solution was stirred for 16 h at 80 °C. The solids were filtered out. The crude product was purified by Prep-HPLC C HCl. This resulted in 59.3 mg (24%) of 1-(3-(2-methoxy-5-((4-(methylamino)pyrimidin-2-yl)amino)phenoxy)propyl)azetidin-3-ol as a light yellow solid.

### Example 93: Synthesis of Compounds 415 and 416 (Reference)

### Compound 415 and 416: Synthesis of (S)-N²-(4-methoxy-3-((1-methylpyrrolidin-3-yl)methoxy)phenyl)-N⁴-methylpyrimidine-2,4-diamine and (R)-N²-(4-methoxy-3-((1-methylpyrrolidin-3-yl)methoxy)phenyl)-N⁴-methylpyrimidine-2,4-diamine

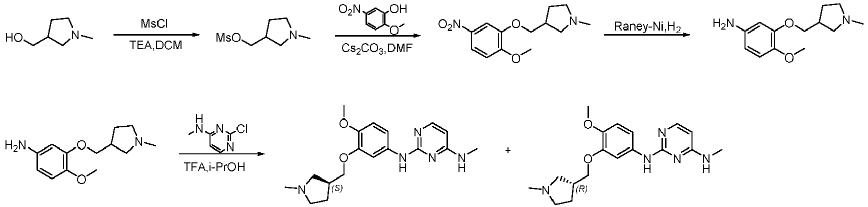

### Step 1: Synthesis of (1-methylpyrrolidin-3-yl)methyl methanesulfonate:

Into a 100-mL round-bottom flask, was placed (1-methylpyrrolidin-3-yl)methanol (1.5 g, 13.02 mmol, 1 equiv), TEA (4.0 g, 39.53 mmol, 3.00 equiv), dichloromethane (15 mL), methanesulfonyl chloride (2.23 mg, 0.02 mmol, 1.5 equiv). The resulting solution was stirred for 3 h at 25 °C. The reaction was then quenched by the addition of 20 mL of water. The resulting solution was extracted with 15 mL of dichloromethane and the organic layers combined and concentrated under vacuum. This resulted in 1.78 g (crude) of the title compound as a brown solid.

Analytical Data: LC-MS: (ES, *m*/*z*): RT = 0.34 min, LCMS 33: *m*/*z* = 194.0 [M+1].

### Step 2: Synthesis of 3-(2-methoxy-5-nitrophenoxymethyl)-1-methylpyrrolidine:

Into a 100-mL round-bottom flask, was placed (1-methylpyrrolidin-3-yl)methyl methanesulfonate (1.78 g, 9.21 mmol, 1 equiv), Cs₂CO₃ (9 g, 27.62 mmol, 3.00 equiv), 2-methoxy-5-nitrophenol (2.3 g, 13.60 mmol, 1.5 equiv), N,N-dimethylformamide (40 mL). The resulting solution was stirred for 2 h at 80 °C in an oil bath. The resulting solution was extracted with 3x40 mL of ethyl acetate and the organic layers combined. The resulting mixture was washed with 2x30 mL of sodium chloride. The residue was applied onto a silica gel column with dichloromethane/methanol (20:1). This resulted in 1.46 g (58%) of the title compound as a white solid.

Analytical Data: LC-MS: (ES, *m*/*z*): RT = 0.89 min, LCMS 07: *m*/*z* = 267.0 [M+1]. ¹H NMR (300 MHz, Methanol-*d*₄) δ 7.93 (q, *J* = 2.7 Hz, 1H), 7.80 (d, *J* = 2.7 Hz, 1H), 7.12 (d, *J* = 9.0 Hz, 1H), 4.11 - 3.99 (m, 2H), 3.97 (s, 3H), 2.94 - 2.64 (m, 4H), 2.56 (q, *J* = 9.4 Hz, 1H), 2.43 (s, 3H), 2.23 - 2.02 (m, 1H), 1.72 - 1.69 (m, 1H).

### Step 3: Synthesis of 4-methoxy-3-[(1-methylpyrrolidin-3-yl)methoxy]aniline:

Into a 100-mL round-bottom flask, was placed 3-(2-methoxy-5-nitrophenoxymethyl)-1-methylpyrrolidine (1.46 g, 5.48 mmol, 1 equiv), Raney-Ni (300 mg), methanol (25 mL). The resulting solution was stirred for 1 h at 25 °C. The solids were filtered out. The resulting mixture was concentrated under vacuum. This resulted in 630 mg (42%) of the title compound as an oil.

Analytical Data: LC-MS: (ES, *m*/*z*): RT = 0.60 min, LCMS 07: *m*/*z* = 237.0 [M+1].

### Step 4: Synthesis of (S)-N²-(4-methoxy-3-((1-methylpyrrolidin-3-yl)methoxy)phenyl)-N⁴-methylpyrimidine-2,4-diamine (E1) and (R)-N²-(4-methoxy-3-((1-methylpyrrolidin-3-yl)methoxy)phenyl)-N⁴-methylpyrimidine-2,4-diamine (E2):

Into a 100-mL round-bottom flask, was placed 4-methoxy-3-[(1-methylpyrrolidin-3-yl)methoxy]aniline (300 mg, 1.27 mmol, 1 equiv), trifluoroacetic acid (290 mg, 2.57 mmol, 2.00 equiv, 98%), 2-chloro-N-methylpyrimidin-4-amine (182 mg, 1.27 mmol, 1 equiv), isopropanol (15 mL). The resulting solution was stirred for 3 h at 90 °C in an oil bath. The resulting mixture was concentrated under vacuum. The crude product was applied onto a silica gel column with NH₄HCO₃:ACN (1:1), Detector, UV 254 nm. This resulted in 23 mg (5%) of (S)-N²-(4-methoxy-3-((1-methylpyrrolidin-3-yl)methoxy)phenyl)-N⁴-methylpyrimidine-2,4-diamine E1 (arbitrarily assigned, S) and 22.7 mg (5%) of (R)-N²-(4-methoxy-3-((1-methylpyrrolidin-3-yl)methoxy)phenyl)-N⁴-methylpyrimidine-2,4-diamine E2 (arbitrarily assigned, R) as a white solid.

### Example 94: Synthesis of Compounds 417 and 418

### Compound 417 and 418: Synthesis of (S)-N²-(4-methoxy-3-((1-methylpyrrolidin-3-yl)methoxy)phenyl)-N⁴,6-dimethylpyrimidine-2,4-diamine and (R)-N²-(4-methoxy-3-((1-methylpyrrolidin-3-yl)methoxy)phenyl)-N⁴,6-dimethylpyrimidine-2,4-diamine

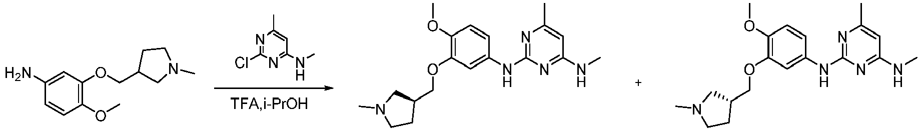

### Step 1: Synthesis of (S)-N²-(4-methoxy-3-((1-methylpyrrolidin-3-yl)methoxy)phenyl)-N⁴,6-dimethylpyrimidine-2,4-diamine (E1) and (R)-N²-(4-methoxy-3-((1-methylpyrrolidin-3-yl)methoxy)phenyl)-N⁴,6-dimethylpyrimidine-2,4-diamine (E2):

Into a 100-mL round-bottom flask, was placed 4-methoxy-3-[(1-methylpyrrolidin-3-yl)methoxy]aniline (300 mg, 1.27 mmol, 1 equiv), 2-chloro-N,6-dimethylpyrimidin-4-amine (200 mg, 1.27 mmol, 1 equiv), trifluoroacetic acid (290 mg, 2.57 mmol, 2.00 equiv, 98%), isopropanol (15 mL). The resulting solution was stirred for 3 h at 90 °C in an oil bath. The resulting mixture was concentrated under vacuum. The crude product (300 mg) was applied onto a silica gel column with NH₄HCO₃:ACN (1:1),Detector, UV 254 nm. This resulted in 82.4 mg (18%) of (S)-N²-(4-methoxy-3-((1-methylpyrrolidin-3-yl)methoxy)phenyl)-N⁴,6-dimethylpyrimidine-2,4-diamine E1 (arbitrarily assigned, S) as a white solid. And 49.6 mg (11%) of (R)-N²-(4-methoxy-3-((1-methylpyrrolidin-3-yl)methoxy)phenyl)-N⁴,6-dimethylpyrimidine-2,4-diamine E1 (arbitrarily assigned, R) as a white solid.

### Example 95: Synthesis of Compound 419

### Compound 419: Synthesis of N²-(3-(((1-ethylpyrrolidin-3-yl)oxy)methyl)-4-methoxyphenyl)-N⁴,6-dimethylpyrimidine-2,4-diamine

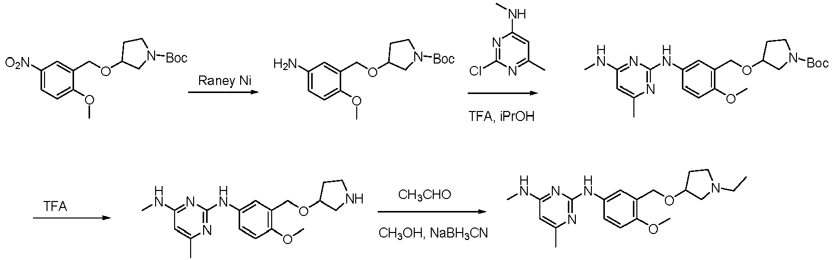

### Step 1: Synthesis of tert-butyl 3-(5-amino-2-methoxyphenoxymethyl)pyrrolidine-1-carboxylate:

Into a 250-mL round-bottom flask, was placed tert-butyl 3-(2-methoxy-5-nitrophenoxymethyl)pyrrolidine-1-carboxylate (600 mg, 1.70 mmol, 1 equiv), methanol (50 mL), Raney-Ni, hydrogen. The resulting solution was stirred for 1 h at 20 °C. The solids were filtered out. The resulting mixture was concentrated under vacuum. This resulted in 496 mg (90%) of as a solid.

Analytical Data: LC-MS: (ES, *m*/*z*): RT = 0.76min, LCMS33: m/z = 322 [M+1]. ¹H NMR (300 MHz, Methanol-d4) δ 6.77 (d, *J* = 8.5 Hz, 1H), 6.47 (d, *J* = 2.6 Hz, 1H), 6.33 (dd, *J* = 8.5, 2.5 Hz, 1H), 4.02 - 3.85 (m, 2H), 3.76 (s, 3H), 3.63 - 3.48 (m, 2H), 3.26 (dd, *J* = 20.0, 11.5 Hz, 2H), 2.77 - 2.66 (m, 1H), 2.10 (d, *J* = 10.7 Hz, 1H), 1.85 (dd, *J* = 13.9, 6.7 Hz, 1H), 1.48 (s, 9H).

### Step 2: Synthesis of tert-butyl 3-(2-methoxy-5-[[4-methyl-6-(methylamino)pyrimidin-2-yl]amino]phenoxymethyl)pyrrolidine-1-carboxylate:

Into a 100-mL round-bottom flask, was placed tert-butyl 3-(5-amino-2-methoxyphenoxymethyl)pyrrolidine-1-carboxylate (496 mg, 1.54 mmol, 1 equiv), 2-chloro-N,6-dimethylpyrimidin-4-amine (242 mg, 1.54 mmol, 1 equiv), trifluoroacetic acid (445 mg, 3.94 mmol, 3.00 equiv), isopropanol (10 mL). The resulting solution was stirred for 2 h at 85 °C in an oil bath. The resulting mixture was concentrated under vacuum. The residue was applied onto a silica gel column with CH₃CN/H₂O (1:5). This resulted in 560 mg (82%) of the title compound as a solid.

Analytical Data: LC-MS: (ES, *m*/*z*): RT = 1.15min, LCMS28: m/z = 444 [M+1]. ¹H NMR (300 MHz, Methanol-d4) δ 7.29 (s, 1H), 7.13 - 6.99 (m, 2H), 5.99 (d, *J* = 1.2 Hz, 1H), 5.51 (s, 1H), 4.02 (q, *J* = 8.0, 6.8 Hz, 2H), 3.87 (s, 3H), 3.68 - 3.38 (m, 3H), 3.00 (s, 3H), 2.73 (s, 1H), 2.29 (s, 3H), 2.12 (s, 1H), 1.86 (s, 1H), 1.48 (s, 9H).

### Step 3: Synthesis of 2-N-[4-methoxy-3-(pyrrolidin-3-ylmethoxy)phenyl]-4-N,6-dimethylpyrimidine-2,4-diamine:

Into a 50-mL round-bottom flask, was placed tert-butyl 3-(2-methoxy-5-[[4-methyl-6-(methylamino)pyrimidin-2-yl]amino]phenoxymethyl)pyrrolidine-1-carboxylate (560 mg, 1.26 mmol, 1 equiv), trifluoroacetic acid (364 mg, 3.22 mmol, 3.00 equiv), dichloromethane (10 mL). The resulting solution was stirred for 3 h at 20 °C. The resulting mixture was concentrated under vacuum. TEA was employed to adjust the pH to 8. The resulting mixture was concentrated under vacuum. This resulted in 900 mg (>100%) of the title compound as a solid.

Analytical Data: LC-MS: (ES, *m*/*z*): RT = 0.78min, LCMS07: m/z = 344 [M+1]. ¹H NMR (300 MHz, Methanol-d4) δ 7.28 (d, *J* = 2.5 Hz, 1H), 7.19 (dd, *J* = 8.7, 2.5 Hz, 1H), 7.04 (d, *J* = 8.7 Hz, 1H), 5.99 (d, *J* = 1.1 Hz, 1H), 4.19 - 4.04 (m, 2H), 3.88 (s, 3H), 3.71 - 3.47 (m, 3H), 3.01 (s, 5H), 2.37 - 2.22 (m, 4H), 2.12-1.94 (m, 1H).

### Step 4: Synthesis of N²-(3-(((1-ethylpyrrolidin-3-yl)oxy)methyl)-4-methoxyphenyl)-N⁴,6-dimethylpyrimidine-2,4-diamine:

Into a 50-mL round-bottom flask, was placed 2-N-[4-methoxy-3-(pyrrolidin-3-ylmethoxy)phenyl]-4-N,6-dimethylpyrimidine-2,4-diamine (300 mg, 0.87 mmol, 1 equiv), NaBH₃CN (165.3 mg, 2.63 mmol, 3.00 equiv), acetaldehyde (38.5 mg, 0.87 mmol, 1 equiv), methanol (10 mL). The resulting solution was stirred for 2 h at 20 °C. The reaction was then quenched by the addition of water/ice. The resulting mixture was concentrated under vacuum. The crude product (300 mg) was purified by Prep-HPLC C HCl. This resulted in 77.6 mg (22%) of N²-(3-(((1-ethylpyrrolidin-3-yl)oxy)methyl)-4-methoxyphenyl)-N⁴,6-dimethylpyrimidine-2,4-diamine as a white solid.

### Example 96: Synthesis of Compound 420 (Reference)

### Compound 420: Synthesis of N²-(4-methoxy-3-((1-propylpyrrolidin-3-yl)methoxy)phenyl)-N⁴-methylpyrimidine-2,4-diamine

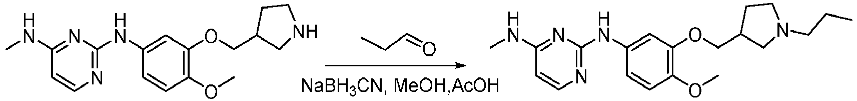

### Step 1: Synthesis of N²-(4-methoxy-3-((1-propylpyrrolidin-3-yl)methoxy)phenyl)-N⁴-methylpyrimidine-2,4-diamine:

Into a 50-mL round-bottom flask, was placed 2-N-[4-methoxy-3-(pyrrolidin-3-ylmethoxy)phenyl]-4-N-methylpyrimidine-2,4-diamine (300 mg, 0.91 mmol, 1 equiv), propanal (60 mg, 1.03 mmol, 1.10 equiv), methanol (15 mL), NaBH₃CN (172 mg, 2.74 mmol, 3.00 equiv), AcOH (0.2 mL). The resulting solution was stirred for 2 h at 25 °C. The reaction was then quenched by the addition of water. The resulting mixture was concentrated under vacuum. The crude product was purified by Prep-HPLC C TFA. This resulted in 144.6 mg (33%) of N²-(4-methoxy-3-((1-propylpyrrolidin-3-yl)methoxy)phenyl)-N⁴-methylpyrimidine-2,4-diamine as an off-white solid.

### Example 97: Synthesis of Compound 421

### Compound 421: Synthesis of N²-(4-methoxy-3-(((1-methylpyrrolidin-3-yl)oxy)methyl)phenyl)-N⁴,6-dimethylpyrimidine-2,4-diamine

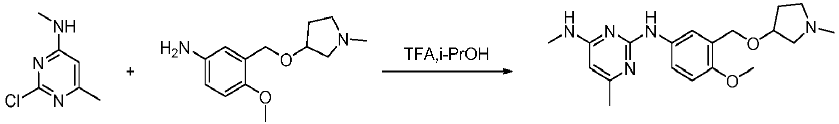

### Step 1: Synthesis of N²-(4-methoxy-3-(((1-methylpyrrolidin-3-yl)oxy)methyl)phenyl)-N⁴,6-dimethylpyrimidine-2,4-diamine:

Into a 50-mL round-bottom flask, was placed 4-methoxy-3-[[(1-methylpyrrolidin-3-yl)oxy]methyl]aniline (200 mg, 0.85 mmol, 1 equiv), 2-chloro-N,6-dimethylpyrimidin-4-amine (133.0 mg, 0.84 mmol, 1 equiv), isopropanol (5 mL), trifluoroacetic acid (193.2 mg, 1.71 mmol, 2.00 equiv). The resulting solution was stirred for 12 h at 85 °C in an oil bath. The crude product was purified by Prep-HPLC G NH₄HCO₃. This resulted in 54.2 mg (18%) of N²-(4-methoxy-3-(((1-methylpyrrolidin-3-yl)oxy)methyl)phenyl)-N⁴,6-dimethylpyrimidine-2,4-diamine as an off-white solid.

### Example 98: Synthesis of Compound 422 (Reference)

### Compound 422: Synthesis of N²-(3-((1-(cyclopropylmethyl)pyrrolidin-3-yl)methoxy)-4-methoxyphenyl)-N⁴-methylpyrimidine-2,4-diamine

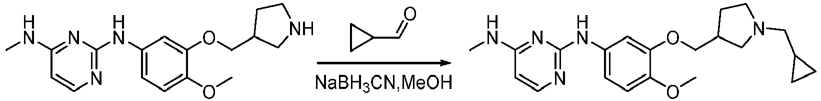

### Step 1: Synthesis of N²-(3-((1-(cyclopropylmethyl)pyrrolidin-3-yl)methoxy)-4-methoxyphenyl)-N⁴-methylpyrimidine-2,4-diamine:

Into a 50-mL round-bottom flask, was placed 2-N-[4-methoxy-3-(pyrrolidin-3-ylmethoxy)phenyl]-4-N-methylpyrimidine-2,4-diamine (200 mg, 0.61 mmol, 1 equiv), cyclopropanecarbaldehyde (64 mg, 0.91 mmol, 1.50 equiv), methanol (10 mL). After 10 min, added NaBH₃CN (191 mg, 3.04 mmol, 5.01 equiv). The resulting solution was stirred for 2 h at RT. The reaction was then quenched by the addition of water. The crude product was purified by Prep-HPLC C HCl. This resulted in 66.4 mg (26%) of N²-(3-((1-(cyclopropylmethyl)pyrrolidin-3-yl)methoxy)-4-methoxyphenyl)-N⁴-methylpyrimidine-2,4-diamine as an off-white solid.

### Example 99: Synthesis of Compound 423 (Reference)

### Compound 423: Synthesis of N²-(4-methoxy-3-(3-(3-methylazetidin-1-yl)propoxy)phenyl)-N⁴-methylpyrimidine-2,4-diamine

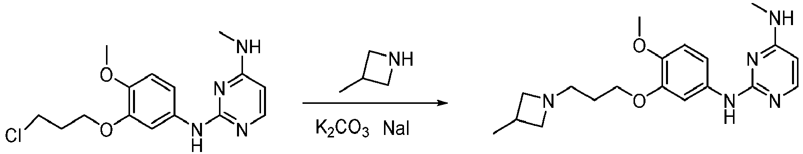

### Step 1: Synthesis of N²-(4-methoxy-3-(3-(3-methylazetidin-1-yl)propoxy)phenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (200 mg, 0.62 mmol, 1 equiv), potassium methaneperoxoate (257.1 mg, 1.85 mmol, 3.00 equiv), acetonitrile (10 mL), 3-methylazetidine hydrochloride (132.9 mg, 1.24 mmol, 2.00 equiv), iodosodium (93.2 mg, 0.62 mmol, 1 equiv). The resulting solution was stirred for 12 h at 85 °C. The solids were filtered out. The resulting mixture was concentrated under vacuum. The crude product (200 mg) was purified by Prep-HPLC C TFA. This resulted in 75.3 mg (26%) of N²-(4-methoxy-3-(3-(3-methylazetidin-1-yl)propoxy)phenyl)-N⁴-methylpyrimidine-2,4-diamine as a white solid.

### Example 100: Synthesis of Compound 424 (Reference)

### Compound 424: Synthesis of N²-(3-(3-((cyclopropylmethyl)(methyl)amino) propoxy)-4-methoxyphenyl)-N⁴-methylpyrimidine-2,4-diamine

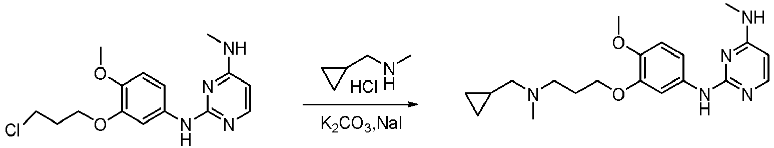

### Step 1: Synthesis of N²-(3-(3-((cyclopropylmethyl)(methyl)amino)propoxy)-4-methoxyphenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (200 mg, 0.62 mmol, 1 equiv), potassium carbonate (257 mg, 1.86 mmol, 3.00 equiv), NaI (93 mg, 1 equiv), CH₃CN (10 mL), (cyclopropylmethyl)(methyl)amine (150 mg, 1.76 mmol, 2.00 equiv). The resulting solution was stirred for 12 h at 85 °C in an oil bath. The solids were filtered out. The resulting mixture was concentrated under vacuum. The crude product (200 mg) was purified by Flash-Prep-HPLC A. This resulted in 41.8 mg (14%) of N²-(3-(3-((cyclopropylmethyl)(methyl)amino)propoxy)-4-methoxyphenyl)-N⁴-methylpyrimidine-2,4-diamine as an off-white solid.

### Example 101: Synthesis of Compound 425 (Reference)

### Compound 425: Synthesis of N²-(4-methoxy-3-(3-(3-methoxyazetidin-1-yl)propoxy)phenyl)-N⁴-methylpyrimidine-2,4-diamine

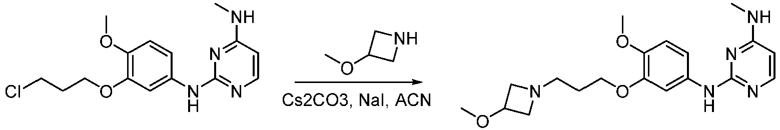

### Step 1: Synthesis of N²-(4-methoxy-3-(3-(3-methoxyazetidin-1-yl)propoxy)phenyl)-N⁴-methylpyrimidine-2,4-diamine:

Into a 50-mL round-bottom flask, was placed 2-N-[3-(3-chloropropoxy)-4-methoxyphenyl]-4-N-methylpyrimidine-2,4-diamine (200 mg, 0.62 mmol, 1 equiv), 3-methoxyazetidine hydrochloride (228 mg, 1.84 mmol, 3.00 equiv), NaI (93 mg, 1 equiv), potassium carbonate (513 mg, 3.71 mmol, 6.00 equiv), ACN (10 mL). The resulting solution was stirred for 12 h at 80 °C. The crude product was purified by Flash-Prep-HPLC A 1:1. This resulted in 74.9 mg (25%) of N²-(4-methoxy-3-(3-(3-methoxyazetidin-1-yl)propoxy)phenyl)-N⁴-methylpyrimidine-2,4-diamine as a white solid.

### Example 102: Synthesis of Compound 426 (Reference)

### Compound 426: Synthesis of N²-(3-((5-cyclopropylisoxazol-3-yl)methoxy)-4-methoxyphenyl)-N⁴-methylpyrimidine-2,4-diamine

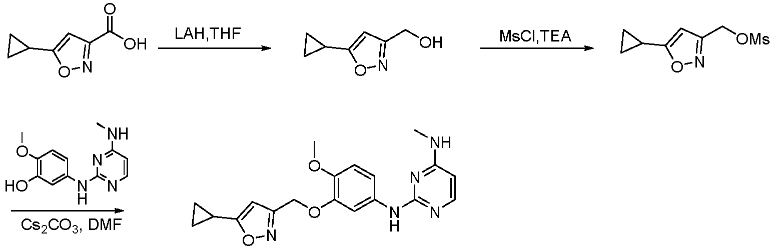

### Step 1: Synthesis of (5-cyclopropyl-1,2-oxazol-3-yl)methanol:

Into a 100-mL 3-necked round-bottom flask, was placed tetrahydrofuran (20 mL), LAH (1.99 g, 52.44 mmol, 4.00 equiv). This was followed by the addition of a solution of 5-cyclopropyl-1,2-oxazole-3-carboxylic acid (2 g, 13.06 mmol, 1 equiv) in tetrahydrofuran (5 mL) dropwise with stirring at 0 °C. The resulting solution was stirred for 3 h at 0 °C. The reaction was then quenched by the addition of 2 mL of water. The resulting solution was diluted with 100 mL of EA. The mixture was dried over anhydrous sodium sulfate. The solids were filtered out. The resulting mixture was concentrated under vacuum. This resulted in 1.8 g (99%) of the title compound as an oil.

Analytical Data: LC-MS: (ES, *m*/*z*): RT =0.572 min, LCMS 32: *m*/*z* = 140 [M+1].

### Step 2: Synthesis of (5-cyclopropyl-1,2-oxazol-3-yl)methyl methanesulfonate:

Into a 100-mL round-bottom flask, was placed dichloromethane (50 mL), (5-cyclopropyl-1,2-oxazol-3-yl)methanol (1.8 g, 12.94 mmol, 1 equiv), TEA (3.96 g, 39.13 mmol, 3.03 equiv). This was followed by the addition of MsCl (1.9 g, 16.67 mmol, 1.29 equiv) dropwise with stirring at 0 °C. The resulting solution was stirred for 14 h at 20 °C. The resulting mixture was washed with 3x10 mL of H₂O. The mixture was dried over anhydrous sodium sulfate. The solids were filtered out. The resulting mixture was concentrated under vacuum. This resulted in 1.2 g (43%) of the title compound as an oil.

Analytical Data: LC-MS: (ES, *m*/*z*): RT =0.834 min, LCMS 07

### Step 3: Synthesis of N²-(3-((5-cyclopropylisoxazol-3-yl)methoxy)-4-methoxyphenyl)-N⁴-methylpyrimidine-2,4-diamine:

Into a 40-mL vial, was placed N,N-dimethylformamide (5ml), (5-cyclopropyl-1,2-oxazol-3-yl)methyl methanesulfonate (200 mg, 0.92 mmol, 1 equiv), 2-methoxy-5-[[4-(methylamino)pyrimidin-2-yl]amino]phenol (270 mg, 1.10 mmol, 1.19 equiv), Cs2CO3 (600 mg, 1.84 mmol, 2.00 equiv). The resulting solution was stirred for 4 h at 80 °C. The solids were filtered out. The crude product (200 mg) was purified by Prep-HPLC C HCl. This resulted in 72.6 mg (20%) of N²-(3-((5-cyclopropylisoxazol-3-yl)methoxy)-4-methoxyphenyl)-N⁴-methylpyrimidine-2,4-diamine as an off-white solid.

### Example 103: Synthesis of Compound 428

### Compound 428: Synthesis of N²-(4-methoxy-3-(3-(pyrrolidin-1-yl)propoxy)phenyl)-N⁴-methyl-6-(2,2,2-trifluoroethyl)pyrimidine-2,4-diamine

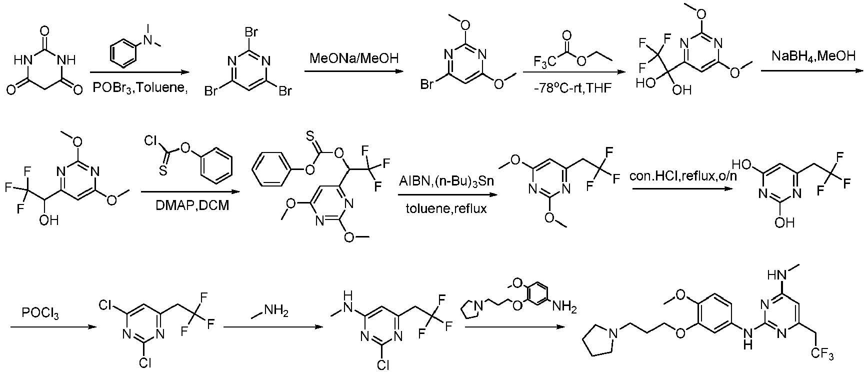

### Step 1: Synthesis of 2,4,6-tribromopyrimidine:

Into a 1-L 3-necked round-bottom flask, was placed 1,3-diazinane-2,4,6-trione (30 g, 234.22 mmol, 1 equiv), N,N-dimethylaniline (42.54 g, 351.05 mmol, 1.50 equiv), POBr₃ (263 g, 4.00 equiv), toluene (300 mL). The resulting solution was stirred for 3 h at 110 °C. The resulted mixture was cooled into RT, the yellow organic layer decanted off. The red gum was rinse once with EA. The combined organic layer was washed with 3x500 mL of Saturated sodium bicarbonate, 3x500 mL of brine and 2x500 mL of water. The mixture was dried over anhydrous sodium sulfate and concentrated under vacuum. This resulted in 54 g of the title compound as a yellow crude solid.

### Step 2: Synthesis of 4-bromo-2,6-dimethoxypyrimidine:

Into a 2-L 3-necked round-bottom flask, was placed 2,4,6-tribromopyrimidine (54 g, 170.47 mmol, 1 equiv), methanol (500 mL), diethyl ether (500 mL), and MeONa/MeOH (30%) (76.7 g, 2.50 equiv) was added dropwise. The resulting solution was stirred for 2 h at RT. The resulting mixture was concentrated under vacuum. The resulting solution was diluted with 1 L of EA. The resulting mixture was washed with 3x500 mL of brine. The resulting mixture was concentrated under vacuum. The residue was applied onto a silica gel column with ethyl acetate/petroleum ether (1:100-1:10). The collected fractions were combined and concentrated under vacuum. This resulted in 23 g (62%) of the title compound as a white solid.

Analytical Data: LC-MS: (ES, *m*/*z*): RT = 1.287 min, LCMS 28: *m*/*z* = 219 [M+1].

### Step 3: Synthesis of 1-(2,6-dimethoxypyrimidin-4-yl)-2,2,2-trifluoroethane-1,1-diol:

Into a 1-L 3-necked round-bottom flask purged and maintained with an inert atmosphere of nitrogen, was placed 4-bromo-2,6-dimethoxypyrimidine (23 g, 105.01 mmol, 1 equiv), tetrahydrofuran (250 mL), Diethyl ether (250 mL). And n-BuLi(2.5M) (46.2 mg, 0.72 mmol, 1.10 equiv) was added dropwise at -78 °C. After stirred for 5 min at -78 °C, ethyl 2,2,2-trifluoroacetate (16.4 g, 115.43 mmol, 1.10 equiv) was added dropwise. After stirred for 30 min at -78 °C, the resulting solution was stirred for overnight at RT. The reaction was then quenched by the addition of 200 mL of saturated NH₄Cl. Sodium carbonate was employed to adjust the pH to 8. The resulting solution was diluted with 1 L of EA. The resulting mixture was washed with 3x500 mL of brine. The resulting mixture was concentrated under vacuum. The residue was applied onto a silica gel column with ethyl acetate/petroleum ether (1:100-1:10). The collected fractions were combined and concentrated under vacuum. This resulted in 15 g (56%) of the title compound as an off-white solid.

Analytical Data: LC-MS: (ES, *m*/*z*): RT = 0.739 min, LCMS 32: *m*/*z* = 255 [M+1].

### Step 4: Synthesis of 1-(2,6-dimethoxypyrimidin-4-yl)-2,2,2-trifluoroethan-1-ol:

Into a 500-mL 3-necked round-bottom flask, was placed 1-(2,6-dimethoxypyrimidin-4-yl)-2,2,2-trifluoroethane-1,1-diol (15 g, 59.02 mmol, 1 equiv), methanol (150 mL), and NaBH4 (8.98 g, 237.38 mmol, 4.00 equiv) was added portionwise at 0 °C. The resulting solution was stirred for 1h at RT. The reaction was then quenched by the addition of 50 mL of saturated NH₄Cl. The resulting solution was diluted with 500 mL of EA. The resulting mixture was washed with 3x500 mL of brine. The mixture was dried over anhydrous sodium sulfate and concentrated under vacuum. The residue was applied onto a silica gel column with ethyl acetate/petroleum ether (1:100-1:10). The collected fractions were combined and concentrated under vacuum. This resulted in 14 g of the title compound as an off-white solid.

Analytical Data: LC-MS: (ES, *m*/*z*): RT = 0.806 min, LCMS 32: *m*/*z* = 239 [M+1]. ¹H NMR (300 MHz, DMSO-*d*₆) δ 7.14 (d, *J* = 6.4 Hz, 1H), 6.72 (s, 1H), 5.01-4.95 (m, 1H), 3.92 (d, *J* = 5.4 Hz, 6H).

### Step 5: Synthesis of [1-(2,6-dimethoxypyrimidin-4-yl)-2,2,2-trifluoroethoxy](phenoxy) methanethione:

Into a 500-mL 3-necked round-bottom flask, was placed 1-(2,6-dimethoxypyrimidin-4-yl)-2,2,2-trifluoroethan-1-ol (14 g, 58.78 mmol, 1 equiv), 4-dimethylaminopyridine (21.53 g, 176.23 mmol, 3.00 equiv), dichloromethane (200 mL). And phenyl chloromethanethioate (10.76 g, 62.33 mmol, 1.50 equiv) was added dropwise at 0 °C. The resulting solution was stirred for 2 h at RT. The resulting mixture was concentrated under vacuum. The resulting solution was diluted with 200 mL of EA. The resulting mixture was washed with 3x200 mL of brine. The mixture was dried over anhydrous sodium sulfate and concentrated under vacuum. This resulted in 37 g of the title compound as a yellow crude solid.

Analytical Data: LC-MS: (ES, *m*/*z*): RT = 0.806 min, LCMS 32: *m*/*z* = 375 [M+1].

### Step 6: Synthesis of 2,4-dimethoxy-6-(2,2,2-trifluoroethyl)pyrimidine:

Into a 1-L 3-necked round-bottom flask, was placed [1-(2,6-dimethoxypyrimidin-4-yl)-2,2,2-trifluoroethoxy](phenoxy)methanethione (37 g, 98.84 mmol, 1 equiv), AIBN (3.2 g, 19.49 mmol, 0.20 equiv), (n-Bu)₃SnH (114.76 g, 4.00 equiv), Toluene (500 mL). The resulting solution was stirred for 2 h at 110 °C in an oil bath. The resulting solution was diluted with 1 L of EA. The resulting mixture was washed with 3×1 L of brine. The resulting mixture was concentrated under vacuum. The residue was applied onto a silica gel column with ethyl acetate/petroleum ether (1:100-1:10). The collected fractions were combined and concentrated under vacuum. This resulted in 13 g (59%) of the title compound as yellow crude oil.

Analytical Data: LC-MS: (ES, *m*/*z*): RT = 1.271 min, LCMS 28: *m*/*z* = 223 [M+1].

### Step 7: Synthesis of 6-(2,2,2-trifluoroethyl)pyrimidine-2,4-diol:

Into a 500-mL 3-necked round-bottom flask, was placed 2,4-dimethoxy-6-(2,2,2-trifluoroethyl)pyrimidine (11 g, 49.51 mmol, 1 equiv), Conc. HCl (150 mL). The resulting solution was stirred for 6 h at 105 °C in an oil bath. The resulting mixture was concentrated under vacuum. This resulted in 7.7 g (80%) of the title compound as an off-white solid.

Analytical Data: LC-MS: (ES, *m*/*z*): RT = 0.773 min, LCMS 15: *m*/*z* = 195 [M+1].

### Step 8: Synthesis of 2,4-dichloro-6-(2,2,2-trifluoroethyl)pyrimidine:

Into a 50-mL round-bottom flask, was placed 6-(2,2,2-trifluoroethyl)pyrimidine-2,4-diol (2.2 g, 11.33 mmol, 1 equiv), phosphoroyl trichloride (5 mL). The resulting solution was stirred for 3 h at 120°C in an oil bath. The resulted mixture was poured into ice/water. The resulting solution was extracted with 2x50 mL of ethyl acetate and the organic layers combined. The resulting mixture was washed with 2x50 mL of brine. The mixture was dried over anhydrous sodium sulfate and concentrated under vacuum. This resulted in 760 mg (29%) of the title compound as colorless oil.

Analytical Data: ¹H NMR (300 MHz, Chloroform-d) δ 7.41 (s, 1H), 3.63 (q, *J* = 10.2 Hz, 2H).

### Step 9: Synthesis of 2-chloro-N-methyl-6-(2,2,2-trifluoroethyl)pyrimidin-4-amine:

Into a 50-mL round-bottom flask, was placed 2,4-dichloro-6-(2,2,2-trifluoroethyl)pyrimidine (720 mg, 3.12 mmol, 1 equiv), methanamine hydrochloride (318 mg, 4.71 mmol, 1.50 equiv), potassium carbonate (1.29 g, 9.33 mmol, 3.00 equiv), N,N-dimethylformamide (10 mL). The resulting solution was stirred for 4 h at RT. The resulting solution was diluted with 50 mL of EA. The resulting mixture was washed with 3x50 mL of brine. The mixture was dried over anhydrous sodium sulfate and concentrated under vacuum. The residue was applied onto a silica gel column with ethyl acetate/petroleum ether (1:100-1:10). The collected fractions were combined and concentrated under vacuum. This resulted in 300 mg (43%) of the title compound as an off-white solid.

Analytical Data: LC-MS: (ES, *m*/*z*): RT = 0.799 min, LCMS 32: *m*/*z* = 226 [M+1]. ¹H NMR (400 MHz, DMSO-*d*₆) δ 8.06 (d, *J* = 5.5 Hz, 1H), 6.52 (m, 1H), 3.76 - 3.52 (m, 2H), 2.94 - 2.70 (m, 3H).

### Step 10: Synthesis of N²-(4-methoxy-3-(3-(pyrrolidin-1-yl)propoxy)phenyl)-N⁴-methyl-6-(2,2,2-trifluoroethyl)pyrimidine-2,4-diamine:

Into a 50-mL round-bottom flask, was placed 2-chloro-N-methyl-6-(2,2,2-trifluoroethyl)pyrimidin-4-amine (260 mg, 1.15 mmol, 1 equiv), 4-methoxy-3-[3-(pyrrolidin-1-yl)propoxy]aniline (288 mg, 1.15 mmol, 1 equiv), CF3COOH (393 mg, 3.45 mmol, 3.00 equiv), isopropanol (5 mL). The resulting solution was stirred for overnight at 80 °C in an oil bath. The resulting mixture was concentrated under vacuum. The residue was purified by flash chromatography with H₂O/ACN/NH₄HCO₃. This resulted in 72.6 mg (14%) of N²-(4-methoxy-3-(3-(pyrrolidin-1-yl)propoxy)phenyl)-N⁴-methyl-6-(2,2,2-trifluoroethyl)pyrimidine-2,4-diamine as an off-white solid.

### Example 104: Synthesis of Compound 429 (Reference)

### Compound 429: Synthesis of N²-(4-methoxy-3-(3-(2-methylazetidin-1-yl)propoxy)phenyl)-N⁴-methylpyrimidine-2,4-diamine

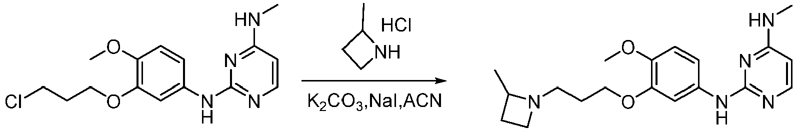

### Step 1: Synthesis of N²-(4-methoxy-3-(3-(2-methylazetidin-1-yl)propoxy)phenyl)-N⁴-methylpyrimidine-2,4-diamine:

Into a 16-mL sealed tube, was placed 2-N-[3-(3-chloropropoxy)-4-methoxyphenyl]-4-N-methylpyrimidine-2,4-diamine (200 mg, 0.62 mmol, 1 equiv), ACN (8 mL), NaI (93 mg, 1 equiv), potassium carbonate (214 mg, 1.55 mmol, 2.50 equiv), 2-methylazetidine hydrochloride (100 mg, 0.93 mmol, 1.50 equiv). The resulting solution was stirred for 3 h at 80 °C in an oil bath. The solids were filtered out. The crude product was purified by Prep-HPLC C TFA. This resulted in 36.7 mg (13%) of N²-(4-methoxy-3-(3-(2-methylazetidin-1-yl)propoxy)phenyl)-N⁴-methylpyrimidine-2,4-diamine as a white solid.

### Example 105: Synthesis of Compound 430 (Reference)

### Compound 430: Synthesis of N²-(4-ethoxy-3-(3-(pyrrolidin-1-yl)propoxy)phenyl)-N⁴-methylpyrimidine-2,4-diamine

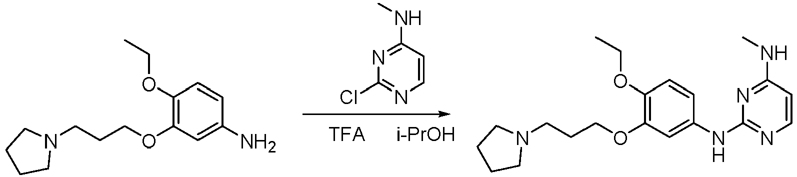

### Step 1: Synthesis of N²-(4-ethoxy-3-(3-(pyrrolidin-1-yl)propoxy)phenyl)-N⁴-methylpyrimidine-2,4-diamine:

Into a 50-mL round-bottom flask, was placed 4-ethoxy-3-[3-(pyrrolidin-1-yl)propoxy] aniline (250 mg, 0.95 mmol, 1 equiv), 2-chloro-N-methylpyrimidin-4-amine (135.4 mg, 0.94 mmol, 1 equiv), propan-2-ol (5 mL), trifluoroacetic acid (275.6 mg, 2.44 mmol, 3.00 equiv). The resulting solution was stirred for 2 h at 80v°C in an oil bath. The resulting mixture was concentrated under vacuum. The crude product (250 mg) was purified by Prep-HPLC C HCl. This resulted in 87.6 mg (23%) of N²-(4-ethoxy-3-(3-(pyrrolidin-1-yl)propoxy)phenyl)-N⁴-methylpyrimidine-2,4-diamine as a white solid.

### Example 106: Synthesis of Compound 432

### Compound 432: Synthesis of N²-(4-ethoxy-3-(3-(pyrrolidin-1-yl)propoxy)phenyl)-N⁴,6-dimethylpyrimidine-2,4-diamine

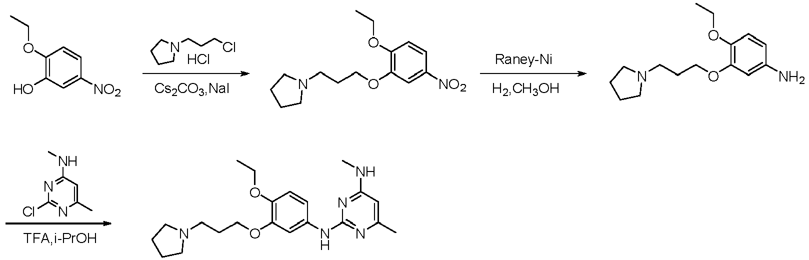

### Step 1: Synthesis of 1-[3-(2-ethoxy-5-nitrophenoxy)propyl]pyrrolidine:

Into a 50-mL round-bottom flask, was placed 2-ethoxy-5-nitrophenol (1 g, 5.46 mmol, 1 equiv), N,N-dimethylformamide (5 mL), Cs₂CO₃ (5.3 g, 16.22 mmol, 3.00 equiv), iodosodium (819.7 mg, 5.47 mmol, 1 equiv), 1-(3-chloropropyl)pyrrolidine hydrochloride (1 g, 5.43 mmol, 1 equiv). The resulting solution was stirred for 2 h at 110 °C in an oil bath. The reaction was then quenched by the addition of water/ice. The resulting solution was extracted with 3x10 mL of ethyl acetate and the organic layers combined. The resulting mixture was washed with 2x10 mL of sodium chloride (aq). The resulting mixture was washed with 2x10 mL of H₂O. The mixture was dried over anhydrous sodium sulfate and concentrated under vacuum. This resulted in 1.48 g (92%) of the title compound as an oil.

Analytical Data: LC-MS: (ES, *m*/*z*): RT = 0.96min, LCMS07: m/z = 295.05 [M+1]. ¹H NMR (300 MHz, Methanol-d4) δ 7.36 (dd, *J* = 2.5, 0.9 Hz, 1H), 7.24 - 7.14 (m, 2H), 4.15 (q, *J* = 7.0 Hz, 2H), 4.05 (t, *J* = 6.1 Hz, 2H), 2.75 - 2.57 (m, 6H), 2.08- 1.98(m, 2H), 1.90 - 1.79 (m, 4H), 1.41 (t, *J* = 7.0 Hz, 3H).

### Step 2: Synthesis of 4-ethoxy-3-[3-(pyrrolidin-1-yl)propoxy]aniline:

Into a 250-mL round-bottom flask, was placed 1-[3-(2-ethoxy-5-nitrophenoxy)propyl]pyrrolidine (800 mg, 2.72 mmol, 1 equiv), Raney-Ni, methanol (100 mL). The resulting solution was stirred for 2 h at 20 °C. The solids were filtered out. The resulting mixture was concentrated under vacuum. This resulted in 700 mg (97%) of the title compound as a solid.

Analytical Data: LC-MS: (ES, *m*/*z*): RT = 0.77min, LCMS33: m/z = 265.19 [M+1]. ¹H NMR: (400 MHz, Methanol-d4) δ 6.72 (d, *J* = 8.7 Hz, 1H), 6.40 (d, *J* = 2.9 Hz, 1H), 6.23 (dd, *J* = 8.7, 2.9 Hz, 1H), 4.12 - 3.85 (m, 4H), 2.70 - 2.56 (m, 6H), 2.05 - 1.73 (m, 6H), 1.40 (t, *J* = 7.0 Hz, 3H).

### Step 3: Synthesis of N²-(4-ethoxy-3-(3-(pyrrolidin-1-yl)propoxy)phenyl)-N⁴,6-dimethylpyrimidine-2,4-diamine:

Into a 50-mL round-bottom flask, was placed 4-ethoxy-3-[3-(pyrrolidin-1-yl)propoxy]aniline (250 mg, 0.95 mmol, 1 equiv), trifluoroacetic acid (275.6 mg, 2.44 mmol, 3.00 equiv), 2-chloro-N,6-dimethylpyrimidin-4-amine (148.7 mg, 0.94 mmol, 1 equiv), propan-2-ol (5 mL). The resulting solution was stirred for 2 h at 80 °C in an oil bath. The resulting mixture was concentrated under vacuum. The crude product (250 mg) was purified by Prep-HPLC C HCl. This resulted in 111 mg (28%) of N²-(4-ethoxy-3-(3-(pyrrolidin-1-yl)propoxy)phenyl)-N⁴,6-dimethylpyrimidine-2,4-diamine as a white solid.

### Example 107: Synthesis of Compound 433 (Reference)

### Compound 433: Synthesis of N²-(3-((5-cyclopropyl-1,2,4-oxadiazol-3-yl)methoxy)-4-methoxyphenyl)-N⁴-methylpyrimidine-2,4-diamine

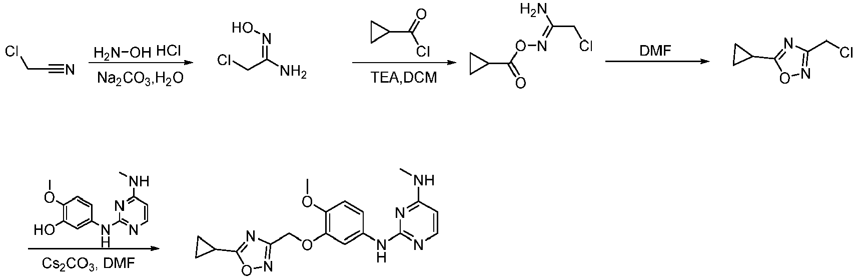

### Step 1: Synthesis of (E)-2-chloro-N'-hydroxyethenimidamide

Into a 100-mL round-bottom flask, was placed water (20 g), 2-chloroacetonitrile (5 g, 66.23 mmol, 1 equiv), hydroxylamine hydrochloride (4.6 g, 66.20 mmol, 1 equiv). This was followed by the addition of sodium carbonate (3.5 g, 33.02 mmol, 0.50 equiv), in portions. The resulting solution was stirred for 2 h at 20 °C. The resulting solution was extracted with 4x20 mL of ether and the organic layers combined and dried over anhydrous sodium sulfate. The solids were filtered out. The resulting mixture was concentrated under vacuum. This resulted in 3.9 g (54%) of the title compound as a white solid. Analytical Data: LC-MS: (ES, *m*/*z*): RT =0.259 min, LCMS 33: *m*/*z* = 109 [M+1].

### Step 2: Synthesis of (Z)-(1-amino-2-chloroethylidene)amino cyclopropanecarboxylate:

Into a 250-mL round-bottom flask, was placed dichloromethane (100 mL), cyclopropanecarbonyl chloride (5.5 g, 52.61 mmol, 1.50 equiv), (E)-2-chloro-N'-hydroxyethenimidamide (3.8 g, 35.01 mmol, 1 equiv).The resulting solution was stirred for 30 min at 20 °C. This was followed by addition of TEA (3.9 g, 38.54 mmol, 1.10 equiv). The resulting solution was allowed to react, with stirring, for an additional 1 h at 20 °C. The resulting mixture was washed with 2x50 mL of water and 1x50 mL of brine. The mixture was dried over anhydrous sodium sulfate. The solids were filtered out. The resulting mixture was concentrated under vacuum. This resulted in 5.2 g (84%) of the title compound as light red oil. Analytical Data: LC-MS: (ES, *m*/*z*): RT =0.504 min, LCMS 32: *m*/*z* = 177 [M+1].

### Step 3: Synthesis of 3-(chloromethyl)-5-cyclopropyl-1,2,4-oxadiazole:

Into a 20-mL sealed tube, was placed N,N-dimethylformamide (10 mL), (Z)-(1-amino-2-chloroethylidene)amino cyclopropanecarboxylate (1.5 g, 8.49 mmol, 1 equiv). The resulting solution was stirred for 3 h at 135 °C. The resulting solution was diluted with 10 mL of H₂O. The resulting solution was extracted with 3x10 mL of ethyl acetate and the organic layers combined. The resulting mixture was washed with 3x10 mL of water and 2x10 mL of brine. The mixture was dried over anhydrous sodium sulfate. The solids were filtered out. The resulting mixture was concentrated under vacuum. This resulted in 550 mg (41%) of the title compound as an oil. Analytical Data: LC-MS: (ES, *m*/*z*): RT =0.775 min, LCMS 32: *m*/*z* = 159 [M+1].

### Step 4: Synthesis of N²-(3-((5-cyclopropyl-1,2,4-oxadiazol-3-yl)methoxy)-4-methoxyphenyl)-N⁴-methylpyrimidine-2,4-diamine:

Into a 50-mL round-bottom flask, was placed N,N-dimethylformamide (5 mL), 3-(chloromethyl)-5-cyclopropyl-1,2,4-oxadiazole (550 mg, 3.47 mmol, 4.27 equiv), 2-methoxy-5-[[4-(methylamino)pyrimidin-2-yl]amino]phenol (200 mg, 0.81 mmol, 1 equiv), Cs₂CO₃ (530 mg, 1.63 mmol, 2.00 equiv), NaI (122 mg). The resulting solution was stirred for 8 h at 80 °C. The solids were filtered out. The crude product (300 mg) was purified by Prep-HPLC C NH₄HCO₃. This resulted in 36.8 mg (12%) of N²-(3-((5-cyclopropyl-1,2,4-oxadiazol-3-yl)methoxy)-4-methoxyphenyl)-N⁴-methylpyrimidine-2,4-diamine as a light yellow solid.

### Example 108: Synthesis of Compound 434 (Reference)

### Compound 434: Synthesis of N²-(3-((1-cyclopropyl-1H-1,2,3-triazol-4-yl)methoxy)-4-methoxyphenyl)-N⁴-methylpyrimidine-2,4-diamine

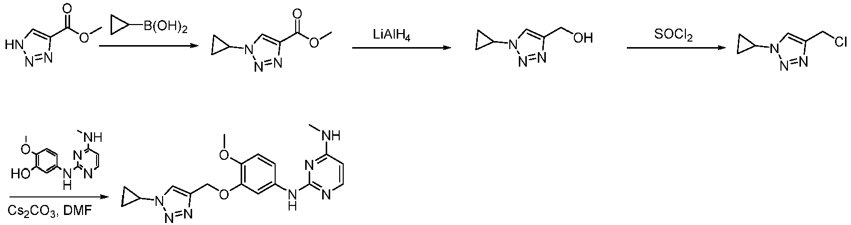

### Step 1: Synthesis of methyl 1-cyclopropyl-1H-1,2,3-triazole-4-carboxylate:

Into a 250-mL round-bottom flask, was placed methyl 1H-1,2,3-triazole-4-carboxylate (2 g, 15.74 mmol, 1 equiv), Cu(OAc)₂ (8.6 g, 47.35 mmol, 3.00 equiv), pyridine (12.4 g, 156.76 mmol, 10.00 equiv), tetrahydrofuran (100 mL), cyclopropylboronic acid (2.7 g, 31.43 mmol, 2.00 equiv). The resulting solution was stirred for 72 h at 55 °C in an oil bath. The resulting solution was extracted with 3x200 mL of ethyl acetate and the organic layers combined. The resulting mixture was washed with 3x100 mL of brine. The mixture was dried over anhydrous sodium sulfate and concentrated under vacuum. The crude product was purified by Flash-Prep-HPLC A 1:1. This resulted in 150 mg (6%) of the title compound as a yellow solid. Analytical Data: LC-MS: (ES, *m*/*z*): 168 [M+1], R: 1.065 min. ¹H-NMR: (DMSO-*d*₆, *ppm*): δ 8.84 (s, 1H), 4.06-4.08 (m, 1H), 3.83 (s, 3H), 1.31 - 1.06 (m, 4H).

### Step 2: Synthesis of (1-cyclopropyl-1H-1,2,3-triazol-4-yl)methanol:

Into a 100-mL round-bottom flask, was placed methyl 1-cyclopropyl-1H-1,2,3-triazole-4-carboxylate (120 mg, 0.72 mmol, 1 equiv), LiAlH4 (144 mg, 3.79 mmol, 5.00 equiv), tetrahydrofuran (20 mL). The resulting solution was stirred for 1 h at 0 °C in a water/ice bath. The reaction was then quenched by the addition of water. The solids were filtered out. The crude product was purified by Flash-Prep-HPLC A. This resulted in 30 mg (30%) of the title compound as a white solid. Analytical Data: LC-MS: (ES, *m*/*z*): 140 [M+1], R: 0.856 min.

### Step 3: Synthesis of 4-(chloromethyl)-1-cyclopropyl-1H-1,2,3-triazole:

Into a 100-mL round-bottom flask, was placed (1-cyclopropyl-1H-1,2,3-triazol-4-yl)methanol (20 mg, 0.14 mmol, 1 equiv), phosphoroyl trichloride (4 mL). The resulting solution was stirred for 3 h at 100 °C in an oil bath. The resulting mixture was concentrated under compound as a white solid. Analytical Data: LC-MS: (ES, *m*/*z*): 158 [M+1], R: 1.267 min.

### Step 4: Synthesis of N²-(3-((1-cyclopropyl-1H-1,2,3-triazol-4-yl)methoxy)-4-methoxyphenyl)-N⁴-methylpyrimidine-2,4-diamine:

Into a 50-mL round-bottom flask, was placed 4-(chloromethyl)-1-cyclopropyl-1H-1,2,3-triazole (100 mg, 0.63 mmol, 1 equiv), Cs₂CO₃ (619 mg, 1.90 mmol, 3.00 equiv), N,N-dimethylformamide (10 mL), 2-methoxy-5-[4-(methylamino)pyrimidin-2-yl]aminophenol (156 mg, 0.63 mmol, 1 equiv). The resulting solution was stirred for 2 h at 50 °C in an oil bath. The solids were filtered out. The crude product was purified by Prep-HPLC C TFA. This resulted in 21.3 mg (7%) of N²-(3-((1-cyclopropyl-1H-1,2,3-triazol-4-yl)methoxy)-4-methoxyphenyl)-N⁴-methylpyrimidine-2,4-diamine as a white solid.

### Example 109: Synthesis of Compound 435

### Compound 435: Synthesis of N²-(4-methoxy-3-((1-methylpiperidin-4-yl)methoxy)phenyl)-N⁴,6-dimethylpyrimidine-2,4-diamine

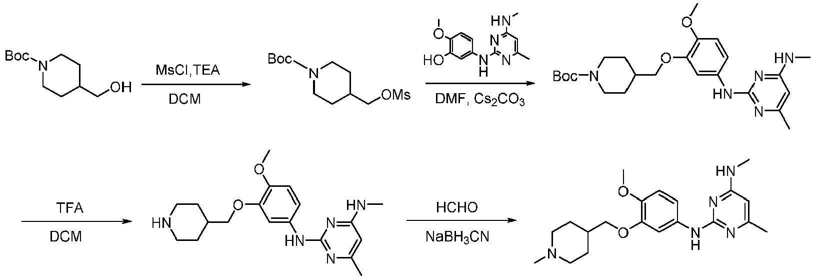

### Step 1: Synthesis of tert-butyl 4-[(methanesulfonyloxy)methyl]piperidine-1-carboxylate:

Into a 250-mL round-bottom flask, was placed tert-butyl 4-(hydroxymethyl)piperidine-1-carboxylate (500 mg, 2.32 mmol, 1 equiv), methanesulfonyl chloride (530 mg, 4.63 mmol, 2.00 equiv), TEA (704 mg, 6.96 mmol, 3.00 equiv), dichloromethane (15 mL). The resulting solution was stirred for 1 h at 25 °C. The resulting solution was extracted with 3x100 mL of ethyl acetate and the organic layers combined. The crude product was purified by Flash-Prep-HPLC A 1:1. This resulted in 550 mg (81%) of as light yellow oil.

Analytical Data: LC-MS: (ES, *m*/*z*): RT= 1.26 min, LCMS 53: m/z = 294 [M+1]. ¹H-NMR: (DMSO-*d*₆, *ppm*): δ 4.07 (d, *J* = 6.4 Hz, 2H), 3.96 (d, *J* = 13.0 Hz, 2H), 3.18 (s, 3H), 2.72 (s, 2H), 1.92 - 1.78 (m, 1H), 1.72 - 1.57 (m, 2H), 1.40 (s, 9H), 1.18 - 0.99 (m, 4H).

### Step 2: Synthesis of tert-butyl 4-(2-methoxy-5-[[4-methyl-6-(methylamino)pyrimidin-2-yl]amino]phenoxymethyl)piperidine-1-carboxylate:

Into a 250-mL round-bottom flask, was placed 2-methoxy-5-[[4-methyl-6-(methylamino)pyrimidin-2-yl]amino]phenol; trifluoroacetic acid (730 mg, 1.95 mmol, 1 equiv), tert-butyl 4-[(methanesulfonyloxy)methyl]piperidine-1-carboxylate (720 mg, 2.45 mmol, 1.20 equiv), Cs₂CO₃ (2 g, 6.14 mmol, 3.00 equiv), N,N-dimethylformamide (30 mL). The resulting solution was stirred for 12 h at 80 °C. The resulting solution was extracted with 3x100 mL of ethyl acetate and the organic layers combined. The resulting mixture was washed with x mL of sodium chloride. The crude product was purified by Flash-Prep-HPLC A 1:1. This resulted in 600 mg (67%) of the title compound as light yellow oil.

Analytical Data: LC-MS: (ES, *m*/*z*): RT= 1.14 min, LCMS 15: m/z = 458 [M+1].¹H-NMR: (DMSO-*d*₆, *ppm*):δ 8.78 - 8.71 (m, 1H), 7.81 (s, 1H), 7.19 - 7.09 (m, 1H), 6.93 (s, 1H), 6.80 (d, *J* = 8.7 Hz, 1H), 5.75 (s, 1H), 3.98 (d, *J* = 12.7 Hz, 2H), 3.79 (d, *J* = 6.4 Hz, 2H), 3.69 (s, 3H), 2.83 (d, *J* = 4.5 Hz, 3H), 2.74 (s, 2H), 2.10 (s, 3H), 1.97 - 1.87 (m, 1H), 1.76 (d, *J* = 13.3 Hz, 2H), 1.40 (s, 9H), 1.25-1.03 (m, 2H).

### Step 3: Synthesis of 2-N-[4-methoxy-3-(piperidin-4-ylmethoxy)phenyl]-4-N,6-dimethylpyrimidine-2,4-diamine:

Into a 100-mL round-bottom flask, was placed tert-butyl 4-(2-methoxy-5-[[4-methyl-6-(methylamino)pyrimidin-2-yl]amino]phenoxymethyl)piperidine-1-carboxylate (600 mg, 1.31 mmol, 1 equiv), trifluoroacetic acid (10 mL), dichloromethane (20 mL). The resulting solution was stirred for 1 h at 25 °C. The crude product was purified by Flash-Prep-HPLC A 1:1. This resulted in 420 mg (90%) of the title compound as a solid.

Analytical Data: LC-MS: (ES, *m*/*z*): RT= 1.14 min, LCMS 15: m/z = 358 [M+1].

### Step 4: Synthesis of N²-(4-methoxy-3-((1-methylpiperidin-4-yl)methoxy)phenyl)-N⁴,6-dimethylpyrimidine-2,4-diamine:

Into a 100-mL round-bottom flask, was placed 2-N-[4-methoxy-3-(piperidin-4-ylmethoxy)phenyl]-4-N,6-dimethylpyrimidine-2,4-diamine (210 mg, 0.59 mmol, 1 equiv), HCHO (364 mg, 10.00 equiv), NaBH₃CN (280 mg, 4.46 mmol, 16.00 equiv), methanol (15 mL). The resulting solution was stirred for 4 h at 25 °C. The solids were filtered out. The crude product was purified by Flash-Prep-HPLC A. This resulted in 94.0 mg (39%) of N²-(4-methoxy-3-((1-methylpiperidin-4-yl)methoxy)phenyl)-N⁴,6-dimethylpyrimidine-2,4-diamine as a white solid.

### Example 110: Synthesis of Compound 436

### Compound 436: Synthesis of N²-(3-((1-(cyclopropylmethyl)piperidin-4-yl)methoxy)-4-methoxyphenyl)-N⁴,6-dimethylpyrimidine-2,4-diamine

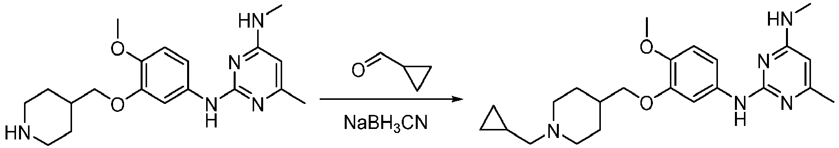

### Step 1: Synthesis of N²-(3-((1-(cyclopropylmethyl)piperidin-4-yl)methoxy)-4-methoxyphenyl)-N⁴,6-dimethylpyrimidine-2,4-diamine:

Into a 100-mL round-bottom flask, was placed 2-N-[4-methoxy-3-(piperidin-4-ylmethoxy)phenyl]-4-N,6-dimethylpyrimidine-2,4-diamine (270 mg, 0.76 mmol, 1 equiv), cyclopropanecarbaldehyde (847 mg, 12.08 mmol, 16 equiv), NaBH₃CN (476 g, 7.57 mol, 10.00 equiv), methanol (15 mL), HOAC (0.5 mL). The resulting solution was stirred for 2 h at 25°C. The crude product was purified by Flash-Prep-HPLC A 1:1. This resulted in 111.1 mg (3.3%) of N²-(3-((1-(cyclopropylmethyl)piperidin-4-yl)methoxy)-4-methoxyphenyl)-N⁴,6-dimethylpyrimidine-2,4-diamine as a white solid.

### Example 111: Synthesis of Compound 437

### Compound 437: Synthesis of N²-(3-(azetidin-3-ylmethoxy)-4-methoxyphenyl)-N⁴,6-dimethylpyrimidine-2,4-diamine

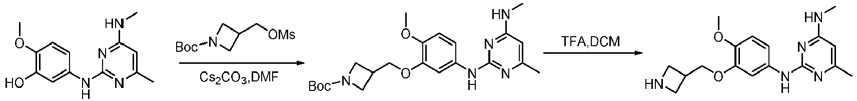

### Step 1: Synthesis of tert-butyl 3-(2-methoxy-5-[[4-methyl-6-(methylamino)pyrimidin-2-yl]amino]phenoxymethyl)azetidine-1-carboxylate:

Into a 100-mL round-bottom flask, was placed 2-methoxy-5-[[4-methyl-6-(methylamino)pyrimidin-2-yl]amino]phenol (2 g, 7.68 mmol, 1 equiv), tert-butyl 3-[(methanesulfonyloxy)methyl]azetidine-1-carboxylate (2.4 g, 9.05 mmol, 1.20 equiv), Cs₂CO₃ (5.0 g, 15.35 mmol, 2.00 equiv), N,N-dimethylformamide (20 mL). The resulting solution was stirred for 12 h at 80 °C in an oil bath. The resulting solution was extracted with 3x50 mL of ethyl acetate and the organic layers combined. The crude product was purified by Flash-Prep-HPLC A. This resulted in 0.5 g (15%) of the title compound as a light brown solid.

Analytical Data: LC-MS: (ES, *m*/*z*): RT = 1.130 min, LCMS53: m/z = 430.2 [M+1].

### Step 2: Synthesis of N²-(3-(azetidin-3-ylmethoxy)-4-methoxyphenyl)-N⁴,6-dimethylpyrimidine-2,4-diamine:

Into a 100-mL round-bottom flask, was placed tert-butyl 3-(2-methoxy-5-[[4-methyl-6-(methylamino)pyrimidin-2-yl]amino]phenoxymethyl)azetidine-1-carboxylate (500 mg, 1.16 mmol, 1 equiv), dichloromethane (10 mL), trifluoroacetic acid (2 mL). The resulting solution was stirred for 3 h at 20 °C. The resulting mixture was concentrated under vacuum. The crude product was purified by Flash-Prep-HPLC A. This resulted in 39.4 mg (10%) of N²-(3-(azetidin-3-ylmethoxy)-4-methoxyphenyl)-N⁴,6-dimethylpyrimidine-2,4-diamine as a light brown solid.

### Example 112: Synthesis of Compounds 438 and 439 (Reference)

### Compound 438 and 439: Synthesis of N2-(3-(((3S,4S)-1,4-dimethylpyrrolidin-3-yl)methoxy)-4-methoxyphenyl)-N4-methylpyrimidine-2,4-diamine and N2-(3-(((3R,4R)-1,4-dimethylpyrrolidin-3-yl)methoxy)-4-methoxyphenyl)-N4-methylpyrimidine-2,4-diamine

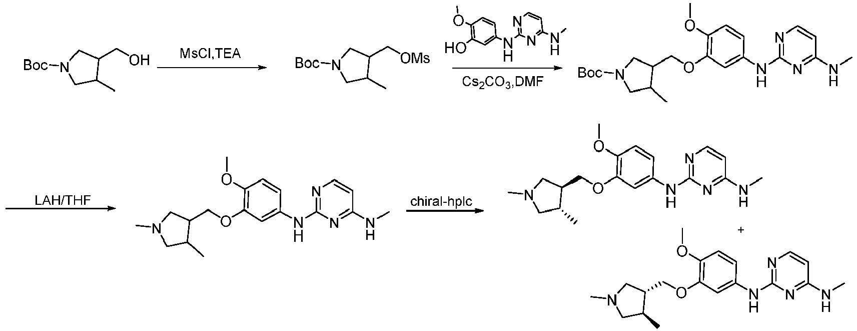

### Step 1: Synthesis of tert-butyl 3-[(methanesulfonyloxy)methyl]-4-methylpyrrolidine- 1-carboxylate:

Into a 100-mL round-bottom flask, was placed dichloromethane (10 mg, 0.12 mmol, 0.05 equiv), tert-butyl 3-(hydroxymethyl)-4-methylpyrrolidine-1-carboxylate (500 mg, 2.32 mmol, 1 equiv), TEA (712 mg, 7.04 mmol, 3.03 equiv).This was followed by addition of MsCl (345 mg, 3.03 mmol, 1.30 equiv)at 0 °C. The resulting solution was stirred for 2 h at 20 °C. The resulting solution was diluted with 10 mL of DCM. The resulting mixture was washed with 2x10 mL of water and 1x10 mL of brine. The mixture was dried over anhydrous sodium sulfate. The solids were filtered out. The resulting mixture was concentrated under vacuum. This resulted in 800 mg (N/A) of the title compound as off-white oil.

Analytical Data: LC-MS: (ES, *m*/*z*): LCMS 32: *m*/*z* = 294 [M+1].

### Step 2: Synthesis of tert-butyl 3-(2-methoxy-5-[[4-(methylamino)pyrimidin-2-yl] amino]phenoxymethyl)pyrrolidine-1-carboxylate:

Into a 100-mL round-bottom flask, was placed N,N-dimethylformamide (20 L), tert-butyl 3-[(methanesulfonyloxy)methyl]-4-methylpyrrolidine-1-carboxylate (800 mg, 2.73 mmol, 1 equiv), 2-methoxy-5-[[4-(methylamino)pyrimidin-2-yl]amino]phenol (739 mg, 3.00 mmol, 1.10 equiv), Cs₂CO₃ (1.78 g, 5.46 mmol, 2.00 equiv). The resulting solution was stirred for 4 h at 80 °C. The resulting solution was diluted with 20 mL of H2O. The resulting solution was extracted with 3x20 mL of ethyl acetate and the organic layers combined. The resulting mixture was washed with 3x10 mL of water and 3x10 mL of brine. The mixture was dried over anhydrous sodium sulfate. The solids were filtered out. The resulting mixture was concentrated under vacuum. The residue was applied onto a silica gel column with ethyl acetate/petroleum ether (7:3). This resulted in 900 mg (74%) of the title compound as a solid.

Analytical Data: LC-MS: (ES, *m*/*z*): RT =0.848 min, LCMS 32: *m*/*z* = 444 [M+1].

### Step 3: Synthesis of 2-N-[3-[(1,4-dimethylpyrrolidin-3-yl)methoxy]-4-methoxyphenyl]-4-N-methylpyrimidine-2,4-diamine:

Into a 100-mL round-bottom flask, was placed tetrahydrofuran (20 mL), LAH (386 mg, 10.17 mmol, 5.01 equiv).This was followed by addition of a solution of tert-butyl 3-(2-methoxy-5-[[4-(methylamino)pyrimidin-2-yl]amino]phenoxymethyl)-4-methylpyrrolidine-1-carboxylate (900 mg, 2.03 mmol, 1 equiv) in tetrahydrofuran (2 mL) at 0 °C. The resulting solution was stirred for 5 h at 80 °C. The reaction was then quenched by the addition of 0.4 mL of water and 0.4 mL as a solution of NaOH in H₂O (0.4ml). The resulting solution was diluted with 50 mL of EA. The mixture was dried over anhydrous sodium sulfate. The solids were filtered out. The resulting mixture was concentrated under vacuum. This resulted in 440 mg (61%) of the title compound as a solid.

Analytical Data: LC-MS: (ES, *m*/*z*): RT =0.589 min, LCMS 32: *m*/*z* = 358 [M+1].

### Step 4: Synthesis of N²-(3-(((3S,4S)-1,4-dimethylpyrrolidin-3-yl)methoxy)-4-methoxyphenyl)-N⁴-methylpyrimidine-2,4-diamine and N²-(3-(((3R,4R)-1,4-dimethylpyrrolidin-3-yl)methoxy)-4-methoxyphenyl)-N⁴-methylpyrimidine-2,4-diamine:

Into a 100-mL round-bottom flask, was placed 2-N-[3-[(1,4-dimethylpyrrolidin-3-yl)methoxy]-4-methoxyphenyl]-4-N-methylpyrimidine-2,4-diamine (450 mg, 1.26 mmol, 1 equiv). This resulted in 48.8 mg (11%) of N²-(3-(((3S,4S)-1,4-dimethylpyrrolidin-3-yl)methoxy)-4-methoxyphenyl)-N⁴-methylpyrimidine-2,4-diamine E1 (randomly assigned) as a light yellow solid. And 75.0 mg (17%) of N²-(3-(((3R,4R)-1,4-dimethylpyrrolidin-3-yl)methoxy)-4-methoxyphenyl)-N⁴-methylpyrimidine-2,4-diamine E2 (randomly assigned) as a light yellow solid.

### Example 113: Synthesis of Compound 440 (Reference)

### Compound 440: Synthesis of N²-(3-(2-(cyclopentyloxy)ethoxy)-4-methoxyphenyl)-N⁴-methylpyrimidine-2,4-diamine

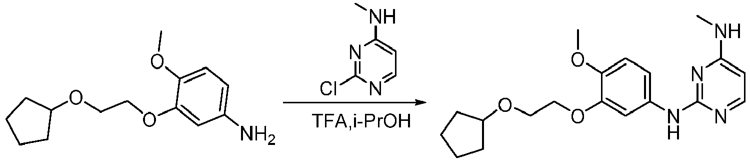

### Step 1: Synthesis of N²-(3-(2-(cyclopentyloxy)ethoxy)-4-methoxyphenyl)-N⁴-methylpyrimidine-2,4-diamine:

Into a 20-mL vial, was placed isopropanol (2 mL), 3-[2-(cyclopentyloxy)ethoxy]-4-methoxyaniline (150 mg, 0.60 mmol, 1 equiv), 2-chloro-N-methylpyrimidin-4-amine (103 mg, 0.72 mmol, 1.20 equiv), trifluoroacetic acid (136 mg, 1.20 mmol, 2.02 equiv). The resulting solution was stirred for 2 h at 80 °C. The crude product was purified by Prep-HPLC B TFA. This resulted in 124.7 mg (44%) of N²-(3-(2-(cyclopentyloxy)ethoxy)-4-methoxyphenyl)-N⁴-methylpyrimidine-2,4-diamine as a white solid.

### Example 114: Synthesis of Compound 441

### Compound 441: Synthesis of N²-(4-methoxy-3-(3-(pyrrolidin-1-yl)propoxy)phenyl)-6-(methoxymethyl)-N⁴-methylpyrimidine-2,4-diamine

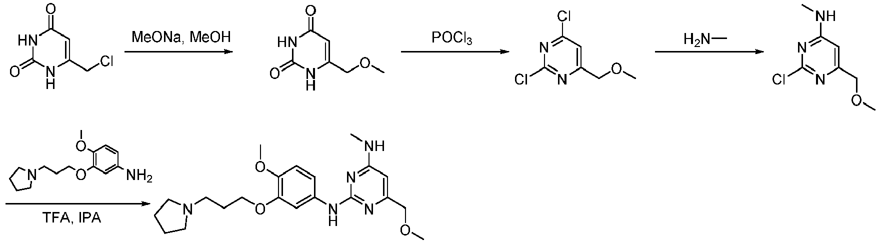

### Step 1: Synthesis of 6-(methoxymethyl)-1,2,3,4-tetrahydropyrimidine-2,4-dione:

Into a 20-mL round-bottom flask, was placed 6-(chloromethyl)-1,2,3,4-tetrahydropyrimidine-2,4-dione (2 g, 12.46 mmol, 1 equiv), methanol; methoxysodium (10 mL). The resulting solution was stirred for 3 h at 70 °C. The resulting mixture was concentrated under vacuum. The residue was dissolved in 30 mL of H₂O. The pH value of the solution was adjusted to 7 with HCl (2 mmol). The resulting solution was extracted with 3x30 mL of ethyl acetate and the organic layers combined. This resulted in 350 mg (17%) of the title compound as a white solid.

Analytical Data: LC-MS: (ES, *m*/*z*): RT= 0.467 min, LCMS 07, *m*/*z* =157 [M+1]. ¹H NMR (300 MHz, Deuterium Oxide) δ 5.70 (d, *J* = 1.2 Hz, 1H), 4.23 (d, *J* = 1.0 Hz, 2H), 3.33 (s, 3H).

### Step 2: Synthesis of 2,4-dichloro-6-(methoxymethyl)pyrimidine:

Into a 100-mL round-bottom flask, was placed 6-(methoxymethyl)-1,2,3,4-tetrahydropyrimidine-2,4-dione (350 mg, 2.24 mmol, 1 equiv), phosphoroyl trichloride (5 mL). The resulting solution was stirred for 5 h at 120 °C. The resulting mixture was concentrated under vacuum. The reaction was then quenched by the addition of 30 mL of water. The pH value of the solution was adjusted to 7 with sodium bicarbonate-H₂O (100 %). The resulting solution was extracted with 3x20 mL of dichloromethane and the organic layers combined. The resulting mixture was washed with 3x20 mL of H₂O. The mixture was dried over anhydrous sodium sulfate. This resulted in 360 mg (75%) of the title compound as a light yellow liquid.

Analytical Data: LC-MS: (ES, *m*/*z*): RT= 0.914 min, LCMS07, *mlz* =193 [M+1].

### Step 3: Synthesis of 2-chloro-6-(methoxymethyl)-N-methylpyrimidin-4-amine:

Into a 50-mL round-bottom flask, was placed 2,4-dichloro-6-(methoxymethyl)pyrimidine (350 mg, 1.81 mmol, 1 equiv), TEA (545 mg, 5.39 mmol, 2.97 equiv), tetrahydrofuran (10 mL), MeNH₂-THF (2.7 mL). The resulting solution was stirred for 2 h at 0 °C. The resulting mixture was concentrated under vacuum. The crude product was purified by Flash-Prep-HPLC A. This resulted in 160 mg (42%) of the title compound as a white solid.

Analytical Data: LC-MS: (ES, *m*/*z*): RT= 0.650 min, LCMS 45, *mlz* =188 [M+1]. ¹H NMR (300 MHz, Chloroform-*d*) δ 6.44 (s, 1H), 4.41 (s, 2H), 3.51 (s, 3H), 3.06 - 2.97 (m, 3H).

### Step 4: Synthesis of N²-(4-methoxy-3-(3-(pyrrolidin-1-yl)propoxy)phenyl)-6-(methoxymethyl)-N⁴-methylpyrimidine-2,4-diamine:

Into a 25-mL round-bottom flask, was placed 2-chloro-6-(methoxymethyl)-N-methylpyrimidin-4-amine (158 mg, 0.84 mmol, 1 equiv), 4-methoxy-3-[3-(pyrrolidin-1-yl)propoxy]aniline (210 mg, 0.84 mmol, 1 equiv), isopropanol (5 mL), trifluoroacetic acid (287 mg, 2.54 mmol, 3.02 equiv). The resulting solution was stirred for 2 h at 80 °C. The resulting mixture was concentrated under vacuum. The crude product was purified by Prep-HPLC C HCl. This resulted in 61.2 mg (16%) of N²-(4-methoxy-3-(3-(pyrrolidin-1-yl)propoxy)phenyl)-6-(methoxymethyl)-N⁴-methylpyrimidine-2,4-diamine as a light brown solid.

### Example 115: Synthesis of Compound 442

### Compound 442: Synthesis of N²-(4-methoxy-3-((1-methylazetidin-3-yl)methoxy)phenyl)-N⁴,6-dimethylpyrimidine-2,4-diamine

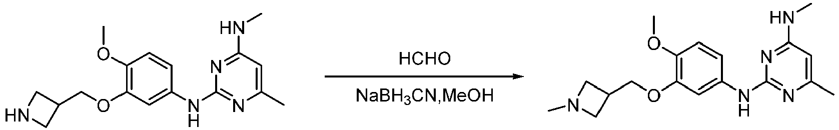

### Step 1: Synthesis of N²-(4-methoxy-3-((1-methylazetidin-3-yl)methoxy)phenyl)-N⁴,6-dimethylpyrimidine-2,4-diamine:

Into a 50-mL round-bottom flask, was placed 2-N-[3-(azetidin-3-ylmethoxy)-4-methoxyphenyl]-4-N,6-dimethylpyrimidine-2,4-diamine (300 mg, 0.91 mmol, 1 equiv), methanol (10 mL), HCHO (90 mg, 3.00 mmol, 1 equiv), NaBH₃CN (360 mg, 5.73 mmol, 6.00 equiv). The resulting solution was stirred for 2 h at 20 °C. The crude product was purified by Prep-HPLC C HCl. This resulted in 30.4 mg (9%) of N²-(4-methoxy-3-((1-methylazetidin-3-yl)methoxy)phenyl)-N⁴,6-dimethylpyrimidine-2,4-diamine as an off-white solid.

### Example 116: Synthesis of Compound 445

### Compound 445: Synthesis of N²-(3-((1-(cyclopropylmethyl)azetidin-3-yl)methoxy)-4-methoxyphenyl)-N⁴,6-dimethylpyrimidine-2,4-diamine

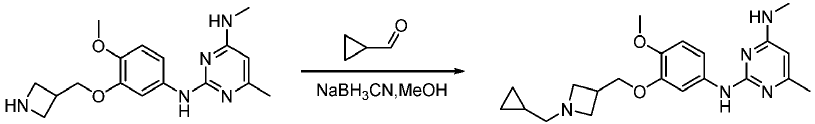

### Step 1: Synthesis of N²-(3-((1-(cyclopropylmethyl)azetidin-3-yl)methoxy)-4-methoxyphenyl)-N⁴,6-dimethylpyrimidine-2,4-diamine:

Into a 50-mL round-bottom flask, was placed 2-N-[3-(azetidin-3-ylmethoxy)-4-methoxyphenyl]-4-N,6-dimethylpyrimidine-2,4-diamine (300 mg, 0.91 mmol, 1 equiv), methanol (10 mL), cyclopropanecarbaldehyde (63.8 mg, 0.91 mmol, 1 equiv), NaBH₃CN (361 mg, 5.74 mmol, 6.00 equiv). The resulting solution was stirred for 2 h at 20 °C. The crude product was purified by Prep-HPLC C TFA. This resulted in 65.8 mg (15%) of N²-(3-((1-(cyclopropylmethyl)azetidin-3-yl)methoxy)-4-methoxyphenyl)-N⁴,6-dimethylpyrimidine-2,4-diamine as a white solid.

### Example 117: Synthesis of Compound 446

### Compound 446: Synthesis of N²-(3-((1-cyclobutylpyrrolidin-3-yl)methoxy)-4-methoxyphenyl)-N⁴,6-dimethylpyrimidine-2,4-diamine

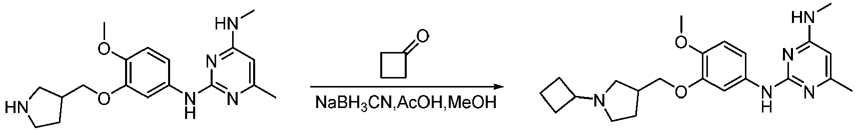

### Step 1: Synthesis of N²-(3-((1-cyclobutylpyrrolidin-3-yl)methoxy)-4-methoxyphenyl)-N⁴,6-dimethylpyrimidine-2,4-diamine:

N²-(3-((1-cyclobutylpyrrolidin-3-yl)methoxy)-4-methoxyphenyl)-N⁴,6-dimethylpyrimidine-2,4-diamine was prepared as for N²-(3-(((1-ethylpyrrolidin-3-yl)oxy)methyl)-4-methoxyphenyl)-N⁴,6-dimethylpyrimidine-2,4-diamine using cyclobutanone in place of acetaldehyde in the final step.

### Example 118: Synthesis of Compounds 447 and 448 (Reference)

### Compound 447 and 448: Synthesis of 2-((4-methoxy-3-(3-(pyrrolidin-1-yl)propoxy)phenyl)amino)-6-methylpyrimidin-4-ol and 4-((4-methoxy-3-(3-(pyrrolidin-1-yl)propoxy)phenyl)amino)-6-methylpyrimidin-2-ol

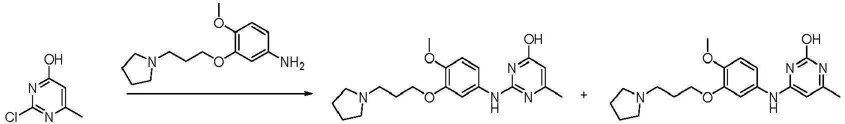

### Step 1: Synthesis of 2-((4-methoxy-3-(3-(pyrrolidin-1-yl)propoxy)phenyl)amino)-6-methylpyrimidin-4-ol and 4-((4-methoxy-3-(3-(pyrrolidin-1-yl)propoxy)phenyl)amino)-6-methylpyrimidin-2-ol:

Into a 100-mL round-bottom flask, was placed 2-chloro-6-methylpyrimidin-4-ol (360 mg, 2.49 mmol, 1 equiv), 4-methoxy-3-[3-(pyrrolidin-1-yl)propoxy]aniline (600 mg, 2.40 mmol, 1 equiv), TsOH (900 mg, 5.23 mmol, 2.00 equiv, 96%), isopropanol (40 mL). The resulting solution was stirred for 3 h at 85 °C in an oil bath. The resulting mixture was concentrated under vacuum. The crude product was applied onto a silica gel column with NH₄HCO₃:ACN (1:1),Detector, UV 254 nm. This resulted in 84.4 mg (9%) of 2-((4-methoxy-3-(3-(pyrrolidin-1-yl)propoxy)phenyl)amino)-6-methylpyrimidin-4-ol, regioisomer 1 as a solid. And 30.6 mg (3%) of 4-((4-methoxy-3-(3-(pyrrolidin-1-yl)propoxy)phenyl)amino)-6-methylpyrimidin-2-ol, regioisomer 2 as a solid.

### Example 119: Synthesis of Compound 449

### Compound 449: Synthesis of N²-(4-methoxy-3-((1-(oxetan-3-yl)pyrrolidin-3-yl)methoxy)phenyl)-N⁴,6-dimethylpyrimidine-2,4-diamine

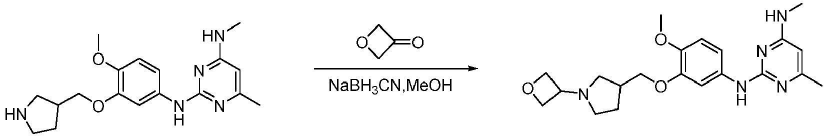

### Step 1: Synthesis of N²-(4-methoxy-3-((1-(oxetan-3-yl)pyrrolidin-3-yl)methoxy)phenyl)-N⁴,6-dimethylpyrimidine-2,4-diamine:

N²-(4-methoxy-3-((1-(oxetan-3-yl)pyrrolidin-3-yl)methoxy)phenyl)-N⁴,6-dimethylpyrimidine-2,4-diamine was prepared as for N²-(3-(((1-ethylpyrrolidin-3-yl)oxy)methyl)-4-methoxyphenyl)-N⁴,6-dimethylpyrimidine-2,4-diamine using oxetan-3-one in place of acetaldehyde in the final step.

### Example 120: Synthesis of Compound 450

### Compound 450: Synthesis of N²-(4-methoxy-3-(3-(pyrrolidin-1-yl)propoxy)phenyl)-N⁴-methyl-6-(pyrrolidin-1-yl)pyrimidine-2,4-diamine

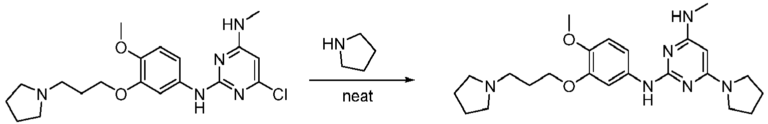

### Step 1: Synthesis of N²-(4-methoxy-3-(3-(pyrrolidin-1-yl)propoxy)phenyl)-N⁴-methyl-6-(pyrrolidin-1-yl)pyrimidine-2,4-diamine:

Into a 10-mL vial, was placed 6-chloro-2-N-[4-methoxy-3-[3-(pyrrolidin-1-yl)propoxy]phenyl]-4-N-methylpyrimidine-2,4-diamine (150 mg, 0.38 mmol, 1 equiv), pyrrolidine (2 mL). The resulting solution was stirred for 3 h at 100 °C in an oil bath. The crude product (150 mg) was purified by Prep-HPLC C HCl. This resulted in 96.1 mg (54%) of N²-(4-methoxy-3-(3-(pyrrolidin-1-yl)propoxy)phenyl)-N⁴-methyl-6-(pyrrolidin-1-yl)pyrimidine-2,4-diamine as a solid.

### Example 121: Synthesis of Compound 451 (Reference)

### Compound 451: Synthesis of N²-(4-methoxy-3-((1-methylpyrrolidin-3-yl)methoxy)phenyl)-6-methyl-N⁴-((tetrahydro-2H-pyran-4-yl)methyl)pyrimidine-2,4-diamine

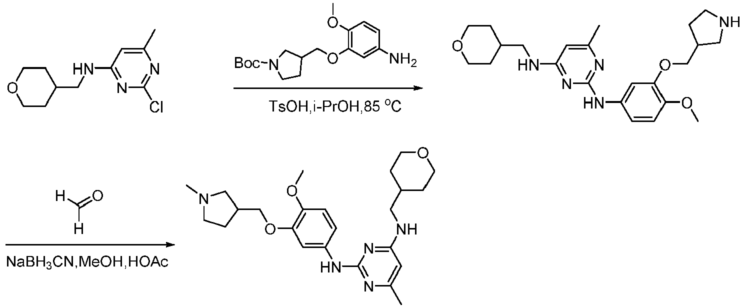

### Step 1: Synthesis of N²-(4-methoxy-3-(pyrrolidin-3-ylmethoxy)phenyl)-6-methyl-N⁴-((tetrahydro-2H-pyran-4-yl)methyl)pyrimidine-2,4-diamine:

Into a 100-mL round-bottom flask, was placed 2-chloro-6-methyl-N-(oxan-4-ylmethyl)pyrimidin-4-amine (870 mg, 3.60 mmol, 1 equiv), tert-butyl 3-(5-amino-2-methoxyphenoxymethyl)pyrrolidine-1-carboxylate (1.163 g, 3.61 mmol, 1 equiv), TsOH (1.242 g, 7.21 mmol, 2.00 equiv), isopropanol (20 mL). The resulting solution was stirred for 24 h at 85 °C in an oil bath. The crude product was purified by (CH₃OH/H₂O=1/10). This resulted in 1.1 g (71%) of the title compound as yellow oil.

Analytical Data: LC-MS: (ES, *m*/*z*): RT =0.885 min, LCMS28: m/z = 428 [M+1].

### Step 2: Synthesis of N²-(4-methoxy-3-((1-methylpyrrolidin-3-yl)methoxy)phenyl)-6-methyl-N⁴-((tetrahydro-2H-pyran-4-yl)methyl)pyrimidine-2,4-diamine:

Into a 100-mL round-bottom flask, was placed N²-(4-methoxy-3-(pyrrolidin-3-ylmethoxy)phenyl)-6-methyl-N⁴-((tetrahydro-2H-pyran-4-yl)methyl)pyrimidine-2,4-diamine (300 mg, 0.70 mmol, 1 equiv). This was followed by the addition of HCHO (70.3 mg, 2.34 mmol, 1 equiv, 30% aq), methanol (20 mL)was stirred for 0.5h at 20 °C. Then NaBH₃CN (265.6 mg, 4.23 mmol, 6.00 equiv), HOAC (0.2 mL) was added and stirred for 2h at 25 °C. The resulting mixture was concentrated under vacuum. The crude product was purified by Prep-HPLC D TFA. This resulted in 71.2 mg (18%) of N²-(4-methoxy-3-((1-methylpyrrolidin-3-yl)methoxy)phenyl)-6-methyl-N⁴-((tetrahydro-2H-pyran-4-yl)methyl)pyrimidine-2,4-diamine as a white solid.

### Example 122: Synthesis of Compound 452

### Compound 452: Synthesis of N²-(4-methoxy-3-((tetrahydrofuran-2-yl)methoxy)phenyl)-N⁴,6-dimethylpyrimidine-2,4-diamine

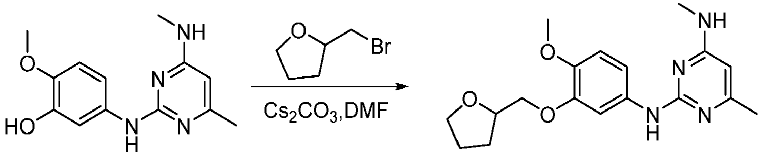

### Step 1: Synthesis of N²-(4-methoxy-3-((tetrahydrofuran-2-yl)methoxy)phenyl)-N⁴,6-dimethylpyrimidine-2,4-diamine:

Into a 20-mL vial, was placed N,N-dimethylformamide (5 mL), 2-methoxy-5-[[4-methyl-6-(methylamino)pyrimidin-2-yl]amino]benzene-1-peroxol (200 mg, 0.72 mmol, 1 equiv), 2-(bromomethyl)oxolane (330 mg, 2.00 mmol, 2.76 equiv), Cs₂CO₃ (330 mg, 1.01 mmol, 1.40 equiv). Adding 2-(bromomethyl)oxolane every two minutes. The resulting solution was stirred for 12 h at 20 °C. The resulting mixture was concentrated under vacuum. The crude product was purified by Prep-HPLC C HCl. This resulted in 95.4 mg (35%) of N²-(4-methoxy-3-((tetrahydrofuran-2-yl)methoxy)phenyl)-N⁴,6-dimethylpyrimidine-2,4-diamine as a light brown solid.

### Example 123: Synthesis of Compound 453 (Reference)

### Compound 453: Synthesis of N²-(3-(2-cyclopropoxyethoxy)-4-methoxyphenyl)-N⁴-methylpyrimidine-2,4-diamine

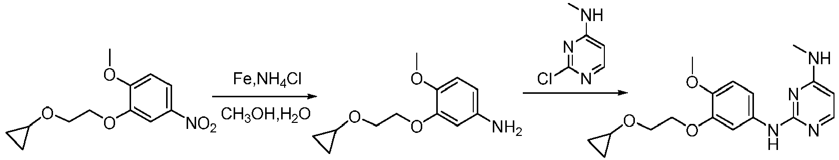

### Step 1: Synthesis of 3-(2-cyclopropoxyethoxy)-4-methoxyaniline

Into a 50-mL round-bottom flask, was placed 2-(2-cyclopropoxyethoxy)-1-methoxy-4-nitrobenzene (300 mg, 1.18 mmol, 1 equiv), NH₄Cl (192 mg, 3.59 mmol, 3.00 equiv), iron (199 mg, 3.56 mmol, 3.00 equiv), methanol (5 mL), water (1 mL). The resulting solution was stirred for 12 h at 90 °C in an oil bath. The solids were filtered out. The resulting mixture was concentrated under vacuum. The residue was applied onto a silica gel column with methanol/H₂O (1:4). This resulted in 200 mg (76%) of the title compound as a light brown solid.

Analytical Data: LC-MS: (ES, *m*/*z*): RT = 0.82min, LCMS07: m/z = 224 [M+1].

### Step 2: Synthesis of N²-(3-(2-cyclopropoxyethoxy)-4-methoxyphenyl)-N⁴-methylpyrimidine-2,4-diamine:

Into a 50-mL round-bottom flask, was placed 3-(2-cyclopropoxyethoxy)-4-methoxyaniline (200 mg, 0.90 mmol, 1 equiv), trifluoroacetic acid (306.7 mg, 2.71 mmol, 3.00 equiv), IPA (10 mL), 2-chloro-N-methylpyrimidin-4-amine (129 mg, 0.90 mmol, 1 equiv). The resulting solution was stirred for 5 h at 85 °C in an oil bath. The resulting mixture was concentrated under vacuum. The crude product (200 mg) was purified by Prep-HPLC D TFA. This resulted in 65 mg (16%) of N²-(3-(2-cyclopropoxyethoxy)-4-methoxyphenyl)-N⁴-methylpyrimidine-2,4-diamine as a white solid.

### Example 124: Synthesis of Compound 456

### Compound 456: Synthesis of N²-(3-((1-cyclopentylpyrrolidin-3-yl)methoxy)-4-methoxyphenyl)-N⁴,6-dimethylpyrimidine-2,4-diamine

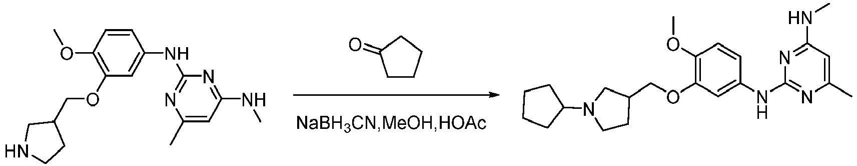

### Step 1: Synthesis of N²-(3-((1-cyclopentylpyrrolidin-3-yl)methoxy)-4-methoxyphenyl)-N⁴,6-dimethylpyrimidine-2,4-diamine:

N²-(3-((1-cyclopentylpyrrolidin-3-yl)methoxy)-4-methoxyphenyl)-N⁴,6-dimethylpyrimidine-2,4-diamine was prepared as for N²-(3-(((1-ethylpyrrolidin-3-yl)oxy)methyl)-4-methoxyphenyl)-N⁴,6-dimethylpyrimidine-2,4-diamine using cyclopentanone in place of acetaldehyde in the final step.

### Example 125: Synthesis of Compound 458

### Compound 458: Synthesis of 6-cyclopentyl-N²-(4-methoxy-3-(3-(pyrrolidin-1-yl)propoxy)phenyl)-N⁴-methylpyrimidine-2,4-diamine

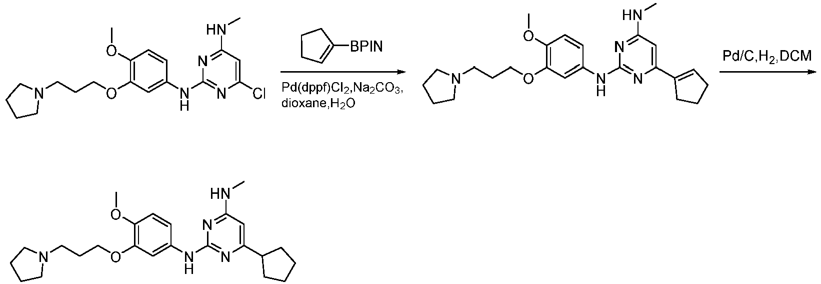

### Step 1: Synthesis of 6-(cyclopent-1-en-1-yl)-2-N-[4-methoxy-3-[3-(pyrrolidin-1-yl)propoxy]phenyl]-4-N-methylpyrimidine-2,4-diamine:

Into a 40-mL round-bottom flask, was placed 6-chloro-2-N-[4-methoxy-3-[3-(pyrrolidin-1-yl)propoxy]phenyl]-4-N-methylpyrimidine-2,4-diamine (300 mg, 0.77 mmol, 1 equiv), Pd(dppf)Cl₂ (127 mg, 0.17 mmol, 0.23 equiv), sodium carbonate (245 mg, 2.31 mmol, 3.02 equiv), dioxane (9 mL), water(3 mL), LiCl (37 mg), [cyclopent-1-en-1-yl(iodo)boranyl]phosphanimine (173 mg, 0.69 mmol, 0.90 equiv). The resulting solution was stirred overnight at 80 °C. The resulting mixture was concentrated under vacuum. The resulting solution was diluted with 10 mL of H₂O. The resulting solution was extracted with 3x20 mL of dichloromethane and the organic layers combined and dried in an oven under reduced pressure. The resulting mixture was washed with 3x50 mL of 1N HCl/H₂O. The pH value of the solution was adjusted to 9 with sodium carbonate (100 %). The resulting solution was extracted with 3x50 mL of dichloromethane and the organic layers combined dried in an oven under reduced pressure. The resulting mixture was concentrated under vacuum. This resulted in 500 mg (crude) of as a brown solid.

Analytical Data: LC-MS: (ES, *m*/*z*): RT= 0.914 min, LCMS 07, *m*/*z* =424 [M+1].

### Step 2: Synthesis of 6-cyclopentyl-N²-(4-methoxy-3-(3-(pyrrolidin-1-yl)propoxy)phenyl)-N⁴-methylpyrimidine-2,4-diamine:

Into a 100-mL round-bottom flask, was placed 6-(cyclopent-1-en-1-yl)-2-N-[4-methoxy-3-[3-(pyrrolidin-1-yl)propoxy]phenyl]-4-N-methylpyrimidine-2,4-diamine (500 mg, 1.18 mmol, 1 equiv), Pd/C (100 mg), hydrogen (100 mL), dichloromethane (20 mL). The resulting solution was stirred overnight at RT. The solids were filtered out. The resulting mixture was concentrated under vacuum. The crude product was purified by Prep-HPLC C HCl. This resulted in 50.9 mg (9%) of 6-cyclopentyl-N²-(4-methoxy-3-(3-(pyrrolidin-1-yl)propoxy)phenyl)-N⁴-methylpyrimidine-2,4-diamine as a white solid.

### Example 126: Synthesis of Compound 459

### Compound 459: Synthesis of N²-(4-methoxy-3-(3-(pyrrolidin-1-yl)propoxy)phenyl)-N⁴-methyl-6-(tetrahydro-2H-pyran-4-yl)pyrimidine-2,4-diamine

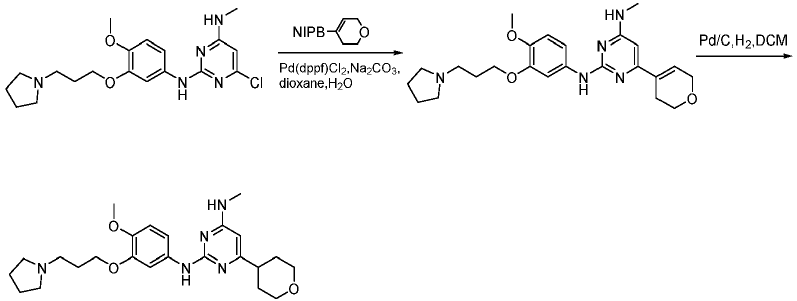

### Step 1: Synthesis of N²-(4-methoxy-3-(3-(pyrrolidin-1-yl)propoxy)phenyl)-N⁴-methyl-6-(tetrahydro-2H-pyran-4-yl)pyrimidine-2,4-diamine:

N²-(4-methoxy-3-(3-(pyrrolidin-1-yl)propoxy)phenyl)-N⁴-methyl-6-(tetrahydro-2H-pyran-4-yl)pyrimidine-2,4-diamine was prepared as for 6-cyclopentyl-N²-(4-methoxy-3-(3-(pyrrolidin-1-yl)propoxy)phenyl)-N⁴-methylpyrimidine-2,4-diamine using 2-(3,6-dihydro-2H-pyran-4-yl)-4,4,5,5-tetramethyl-1,3,2-dioxaborolane in place of 2-(cyclopent-1-en-1-yl)-4,4,5,5-tetramethyl-1,3,2-dioxaborolane in step 1.

### Example 127: Synthesis of Compound 460 (Reference)

### Compound 460: Synthesis of N-(5-methoxy-4-(3-(pyrrolidin-1-yl)propoxy)pyridine-2-yl)-6-methyl-1H-pyrazolo[4,3-c]pyridine-4-amine

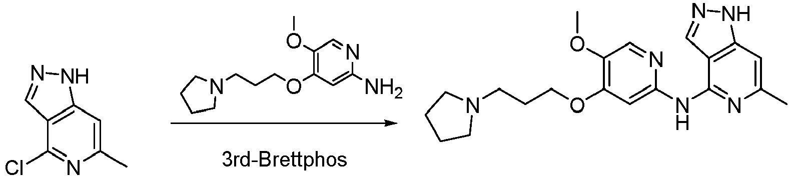

### Step 1: Synthesis of N-(5-methoxy-4-(3-(pyrrolidin-1-yl)propoxy)pyridine-2-yl)-6-methyl-1H-pyrazolo[4,3-c]pyridine-4-amine:

Into a 40-mL vial purged and maintained with an inert atmosphere of nitrogen, was placed DMSO (10 mL), 4-chloro-6-methyl-1H-pyrazolo[4,3-c]pyridine (100 mg, 0.60 mmol, 1 equiv), 5-methoxy-4-[3-(pyrrolidin-1-yl)propoxy]pyridin-2-amine (180 mg, 0.72 mmol, 1.20 equiv), 3rd-Brettphos (81 mg, 0.09 mmol, 0.15 equiv), Cs₂CO₃ (390 mg, 1.20 mmol, 2.01 equiv). The resulting solution was stirred for 4 h at 80 °C. The resulting solution was diluted with 10 mL of H₂O. The resulting solution was extracted with 3x10 mL of ethyl acetate and the organic layers combined. The resulting mixture was washed with 3x10 mL of water and 3x10 mL of brine. The mixture was dried over anhydrous sodium sulfate. The solids were filtered out. The resulting mixture was concentrated under vacuum. The crude product (100 mg) was purified by Prep-HPLC D TFA. This resulted in 30.4 mg (10%) of N-(5-methoxy-4-(3-(pyrrolidin-1-yl)propoxy)pyridine-2-yl)-6-methyl-1H-pyrazolo[4,3-c]pyridine-4-amine as a white solid.

### Example 128: Synthesis of Compound 461

### Compound 461: Synthesis of 2-(3-((2-methoxy-5-((4-methyl-6-(methylamino)pyrimidin-2-yl)amino)phenoxy)methyl)pyrrolidin-1-yl)ethan-1-ol

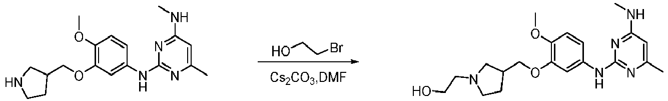

### Step 1: Synthesis of 2-(3-((2-methoxy-5-((4-methyl-6-(methylamino)pyrimidin-2-yl)amino)phenoxy)methyl)pyrrolidin-1-yl)ethan-1-ol:

Into a 100-mL round-bottom flask, was placed 2-N-[4-methoxy-3-(pyrrolidin-3-ylmethoxy)phenyl]-4-N,6-dimethylpyrimidine-2,4-diamine (200 mg, 0.58 mmol, 1 equiv), 2-bromoethan-1-ol (70 mg, 0.56 mmol, 1 equiv), Cs₂CO₃ (380 mg, 1.17 mmol, 2.00 equiv), NaI (170 mg, 2.00 equiv), ACN (15 mL). The resulting solution was stirred for 4 h at 80 °C in an oil bath. The solids were filtered out. The residue was applied onto a silica gel column with TFA:ACN (5:1). This resulted in 44.2 mg (15%) of 2-(3-((2-methoxy-5-((4-methyl-6-(methylamino)pyrimidin-2-yl)amino)phenoxy)methyl)pyrrolidin-1-yl)ethan-1-ol as a solid.

### Example 129: Synthesis of Compound 462 (Reference)

### Compound 462: Synthesis of N²-(3-((1-cyclopropylpyrrolidin-3-yl)methoxy)-4-methoxyphenyl)-6-methyl-N⁴-((tetrahydro-2H-pyran-4-yl)methyl)pyrimidine-2,4-diamine

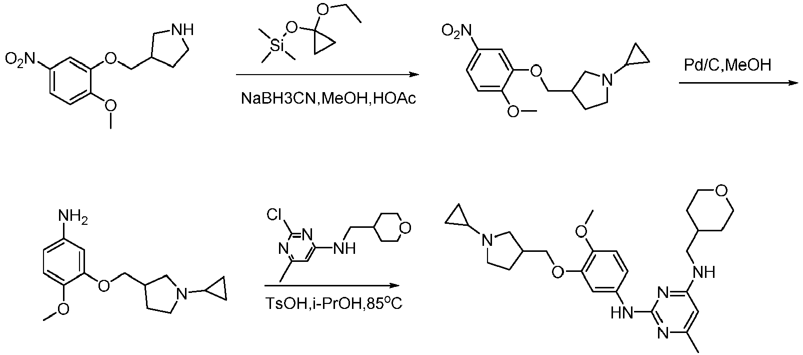

### Step 1: Synthesis 1-cyclopropyl-3-(2-methoxy-5-nitrophenoxymethyl)pyrrolidine:

Into a 100-mL round-bottom flask, was placed 3-(2-methoxy-5-nitrophenoxymethyl)pyrrolidine (340 mg, 1.35 mmol, 1 equiv), (1-ethoxycyclopropoxy)trimethylsilane (354 mg, 2.03 mmol, 1.50 equiv), methanol (20 mL), NaBH₃CN (512 mg, 8.15 mmol, 6.00 equiv), HOAc (0.02 mL). The resulting solution was stirred for 30 min at 25 °C. The resulting solution was allowed to react, with stirring, for an additional 24 h while the temperature was maintained at 65 °C in an oil bath. The resulting mixture was concentrated under vacuum. The crude product was purified by (H₂O/ACN=1/1). This resulted in 300 mg (76%) of the title compound as yellow oil.

Analytical Data: LC-MS: (ES, *m*/*z*): RT =0.930min, LCMS 27: m/z = 293 [M+1].

### Step 2: Synthesis of3-[(1-cyclopropylpyrrolidin-3-yl)methoxy]-4-methoxyaniline:

Into a 100-mL round-bottom flask, was placed 1-cyclopropyl-3-(2-methoxy-5-nitrophenoxymethyl)pyrrolidine (280 mg, 0.96 mmol, 1 equiv), Pd/C (100 mg, 0.30 equiv), methanol (15 mL), hydrogen. The resulting solution was stirred for 1 h at 25 °C. The solids were filtered out and concentrated under vacuum. This resulted in 243 mg (97%) of the title compound as yellow oil.

Analytical Data: LC-MS: (ES, *m*/*z*): RT =0.702min, LCMS 07: m/z = 263 [M+1].

### Step 3: Synthesis of N²-(3-((1-cyclopropylpyrrolidin-3-yl)methoxy)-4-methoxyphenyl)-6-methyl-N⁴-((tetrahydro-2H-pyran-4-yl)methyl)pyrimidine-2,4-diamine:

Into a 100-mL round-bottom flask, was placed 3-[(1-cyclopropylpyrrolidin-3-yl)methoxy]-4-methoxyaniline (200 mg, 0.76 mmol, 1 equiv), TsOH (257 mg, 1.49 mmol, 2.00 equiv), 2-chloro-6-methyl-N-(oxan-4-ylmethyl)pyrimidin-4-amine (180 mg, 0.74 mmol, 1 equiv), isopropanol (15 mL). The resulting solution was stirred for 4 h at 85 °C in an oil bath. The crude product was purified by (H₂O/ACN=1/1). This resulted in 107.4 mg (24%) of N²-(3-((1-cyclopropylpyrrolidin-3-yl)methoxy)-4-methoxyphenyl)-6-methyl-N⁴-((tetrahydro-2H-pyran-4-yl)methyl)pyrimidine-2,4-diamine as a white solid.

### Example 130: Synthesis of Compound 463

### Compound 463: Synthesis of N²-(3-(3-(cyclopropyl(methyl)amino)propoxy)-4-methoxyphenyl)-N⁴,6-dimethylpyrimidine-2,4-diamine

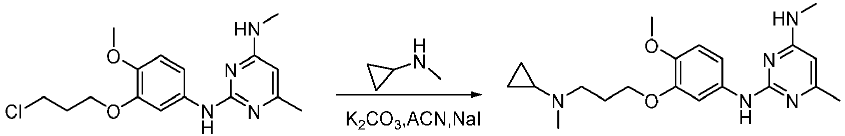

### Step 1: Synthesis of N²-(3-(3-(cyclopropyl(methyl)amino)propoxy)-4-methoxyphenyl)-N⁴,6-dimethylpyrimidine-2,4-diamine:

Into a 40-mL round-bottom flask, was placed 2-N-[3-(3-chloropropoxy)-4-methoxyphenyl]-4-N,6-dimethylpyrimidine-2,4-diamine (300 mg, 0.89 mmol, 1 equiv), N-methylcyclopropanamine (76 mg, 1.07 mmol, 1.20 equiv), potassium carbonate (368 mg, 2.66 mmol, 2.99 equiv), CH₃CN (20 mL), NaI (134 mg). The resulting solution was stirred overnight at 80 °C. The solids were filtered out. The crude product was purified by Prep-HPLC C HCl. This resulted in 38.5 mg (11%) of N²-(3-(3-(cyclopropyl(methyl)amino)propoxy)-4-methoxyphenyl)-N⁴,6-dimethylpyrimidine-2,4-diamine as a white solid.

### Example 131: Synthesis of Compound 464 (Reference)

### Compound 464: Synthesis of N²-(5-methoxy-4-(3-(pyrrolidin-1-yl)propoxy)pyridin-2-yl)-6-methyl-N⁴-((tetrahydro-2H-pyran-4-yl)methyl)pyridine-2,4-diamine

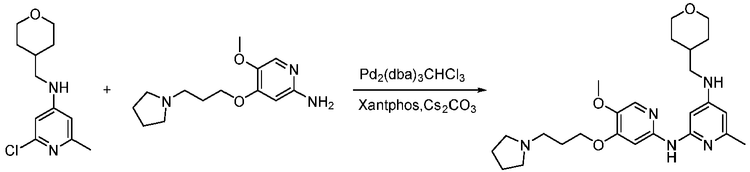

### Step 1: Synthesis of N²-(5-methoxy-4-(3-(pyrrolidin-1-yl)propoxy)pyridin-2-yl)-6-methyl-N⁴-((tetrahydro-2H-pyran-4-yl)methyl)pyridine-2,4-diamine:

Into a 20-mL vial, was placed DMSO (10 mg, 0.13 mmol, 0.15 equiv), 2-chloro-6-methyl-N-(oxan-4-ylmethyl)pyridin-4-amine (200 mg, 0.83 mmol, 1 equiv), 5-methoxy-4-[3-(pyrrolidin-1-yl)propoxy]pyridin-2-amine (250 mg, 0.99 mmol, 1.20 equiv), Pd₂(dba)₃-CHCl₃ (130 mg), Xantphos (150 mg, 0.26 mmol, 0.31 equiv), Cs₂CO₃ (54 mg, 0.17 mmol, 0.20 equiv). The vial was purged and maintained with N₂. The resulting solution was stirred for 12 h at 80°C. The resulting mixture was concentrated under vacuum. The residue was applied onto a silica gel column with H₂O/ACN (9:1). The crude product was purified by Prep-HPLC D HCl. This resulted in 30.2 mg (7%) of N²-(5-methoxy-4-(3-(pyrrolidin-1-yl)propoxy)pyridin-2-yl)-6-methyl-N⁴-((tetrahydro-2H-pyran-4-yl)methyl)pyridine-2,4-diamine as a white solid.

### Example 132: Synthesis of Compound 465

### Compound 465: Synthesis of 1-(3-(2-methoxy-5-((4-methyl-6-(methylamino)pyrimidin-2-yl)amino)phenoxy)propyl)azetidin-3-ol

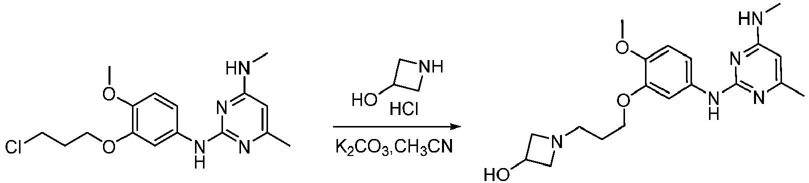

### Step 1: Synthesis of 1-(3-(2-methoxy-5-((4-methyl-6-(methylamino)pyrimidin-2-yl)amino)phenoxy)propyl)azetidin-3-ol:

Into a 50-mL round-bottom flask, was placed 2-N-[3-(3-chloropropoxy)-4-methoxyphenyl]-4-N,6-dimethylpyrimidine-2,4-diamine (200 mg, 0.59 mmol, 1 equiv), potassium methaneperoxoate (246.4 mg, 1.77 mmol, 3.00 equiv), azetidin-3-ol hydrochloride (129.8 mg, 1.18 mmol, 2.00 equiv), acetonitrile (10 mL). The resulting solution was stirred for 12 h at 85 °C in an oil bath. The resulting mixture was concentrated under vacuum. The crude product (200 mg) was purified by Prep-HPLC D TFA. This resulted in 72.5 mg (25%) of 1-(3-(2-methoxy-5-((4-methyl-6-(methylamino)pyrimidin-2-yl)amino)phenoxy)propyl)azetidin-3-ol as a solid.

### Example 133: Synthesis of Compound 466

### Compound 466: Synthesis of N²-(3-((1-cyclopropylpiperidin-4-yl)oxy)-4-methoxyphenyl)-N⁴,6-dimethylpyrimidine-2,4-diamine

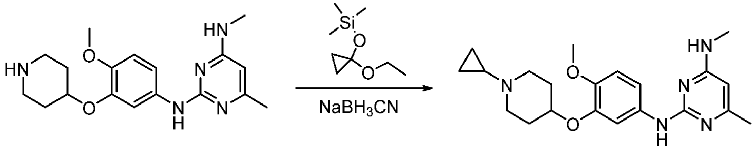

### Step 1: Synthesis of N²-(3-((1-cyclopropylpiperidin-4-yl)oxy)-4-methoxyphenyl)-N⁴,6-dimethylpyrimidine-2,4-diamine:

Into a 50-mL round-bottom flask, was placed 2-N-[4-methoxy-3-(piperidin-4-yloxy)phenyl]-4-N,6-dimethylpyrimidine-2,4-diamine (150 mg, 0.44 mmol, 1 equiv), NaBH₃CN (86 mg, 1.37 mmol, 3.00 equiv), methanol (5 mL), (1-ethoxycyclopropoxy) trimethylsilane (118.9 mg, 0.68 mmol, 1.50 equiv), acetic acid (10 mg, 0.17 mmol, 0.38 equiv). The resulting solution was stirred for 6 h at 65 °C in an oil bath. The resulting mixture was concentrated under vacuum. The crude product (150 mg) was purified by Prep-HPLC D TFA. This resulted in 55.7 mg (26%) of N²-(3-((1-cyclopropylpiperidin-4-yl)oxy)-4-methoxyphenyl)-N⁴,6-dimethylpyrimidine-2,4-diamine as a white solid.

### Example 134: Synthesis of Compounds 481 and 482

### Compound 481 and 482: Synthesis of (S)-N²-(3-((1-cyclopropylpyrrolidin-3-yl)methoxy)-4-methoxyphenyl)-N⁴,6-dimethylpyrimidine-2,4-diamine and (R)-N²-(3-((1-cyclopropylpyrrolidin-3-yl)methoxy)-4-methoxyphenyl)-N⁴,6-dimethylpyrimidine-2,4-diamine

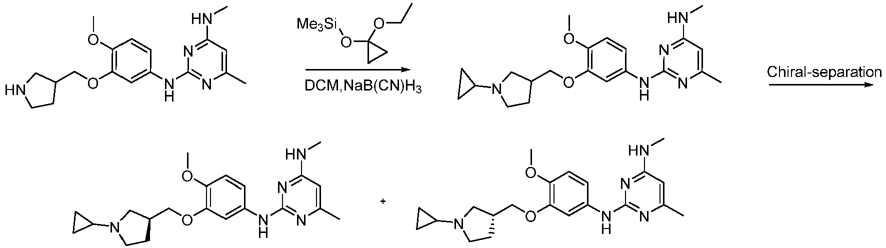

### Step 1: Synthesis of (S)-N²-(3-((1-cyclopropylpyrrolidin-3-yl)methoxy)-4-methoxyphenyl)-N⁴,6-dimethylpyrimidine-2,4-diamine and (R)-N²-(3-((1-cyclopropylpyrrolidin-3-yl)methoxy)-4-methoxyphenyl)-N⁴,6-dimethylpyrimidine-2,4-diamine:

Into a 100-mL round-bottom flask, was placed 2-N-[4-methoxy-3-(pyrrolidin-3-ylmethoxy)phenyl]-4-N,6-dimethylpyrimidine-2,4-diamine (400 mg, 1.16 mmol, 1 equiv), (1-ethoxycyclopropoxy)trimethylsilane (300 mg, 1.72 mmol, 1.50 equiv), AcOH (0.4 mL), methanol (20 mL), NaBH₃CN (330 mg, 5.25 mmol, 3.00 equiv). The resulting solution was stirred for 24 h at 65 °C in an oil bath. The resulting mixture was concentrated under vacuum. The residue was applied onto a silica gel column with TFA:ACN (5:1). This resulted in 31.9 mg (3%) of the racemic mixture as a white solid.

The product was Prep-Chiral-HPLC: Column: Chiralpak ID-2, 2x25cm, 5um;Mobile Phase A:Hex 0.1%DEA) HPLC, Mobile Phase B: IPA--HPLC; Flow rate: 20 mL/min; Gradient: 20 B to 20 B in 30 min; 220/254 nm. This resulted in 27.7 mg (2%) of (S)-N²-(3-((1-cyclopropylpyrrolidin-3-yl)methoxy)-4-methoxyphenyl)-N⁴,6-dimethylpyrimidine-2,4-diamine E1 (randomly assigned S) and 25.5mg (2%) (R)-N²-(3-((1-cyclopropylpyrrolidin-3-yl)methoxy)-4-methoxyphenyl)-N⁴,6-dimethylpyrimidine-2,4-diamine E2 (randomly assigned R).

### Example 135: Synthesis of Compound 498

### Compound 498: Synthesis of N²-(3-(3-(5-azaspiro[2.4]heptan-5-yl)propoxy)-4-methoxyphenyl)-N⁴,6-dimethylpyrimidine-2,4-diamine

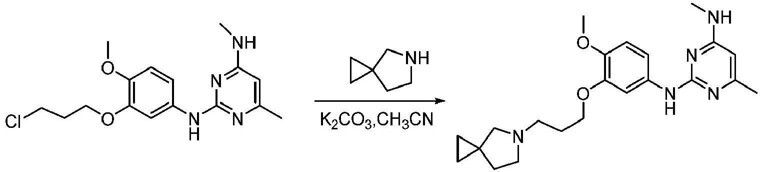

### Step 1: Synthesis of N²-(3-(3-(5-azaspiro[2.4]heptan-5-yl)propoxy)-4-methoxyphenyl)-N⁴,6-dimethylpyrimidine-2,4-diamine:

Into a 20-mL round-bottom flask, was placed 2-N-[3-(3-chloropropoxy)-4-methoxyphenyl]-4-N,6-dimethylpyrimidine-2,4-diamine (300 mg, 0.89 mmol, 1 equiv), potassium carbonate (300 mg, 2.17 mmol, 2.44 equiv), CH₃CN (5 mL), NaI (135 mg), 5-azaspiro[2.4]heptane (372 mg, 3.83 mmol, 4.30 equiv). The resulting solution was stirred for 48 h at 80 °C. The crude product was purified by Prep-HPLC D TFA. This resulted in 75.7 mg (16%) of N²-(3-(3-(5-azaspiro[2.4]heptan-5-yl)propoxy)-4-methoxyphenyl)-N⁴,6-dimethylpyrimidine-2,4-diamine as a white solid.

### Example 136: Synthesis of Compound 504

### Compound 504: Synthesis of N²-(4-methoxy-3-(3-(2-methylpyrrolidin-1-yl)propoxy)phenyl)-N⁴,6-dimethylpyrimidine-2,4-diamine

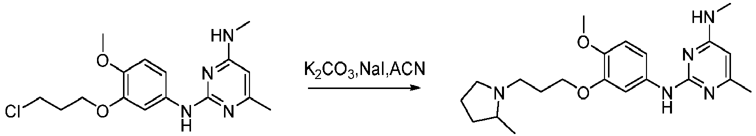

### Step 1: Synthesis of N²-(4-methoxy-3-(3-(2-methylpyrrolidin-1-yl)propoxy)phenyl)-N⁴,6-dimethylpyrimidine-2,4-diamine:

Into a 50-mL round-bottom flask, was placed 2-N-[3-(3-chloropropoxy)-4-methoxyphenyl]-4-N,6-dimethylpyrimidine-2,4-diamine (200 mg, 0.59 mmol, 1 equiv), 2-methylpyrrolidine (101 mg, 1.19 mmol, 2.00 equiv), NaI (89 mg, 1 equiv), potassium carbonate (246 mg, 1.78 mmol, 3.00 equiv), ACN (10 mL). The resulting solution was stirred for 12 h at 85 °C in an oil bath. The solids were filtered out. The crude product was purified by Flash-Prep-HPLC A 1:1. This resulted in 78.7 mg (31%) of N²-(4-methoxy-3-(3-(2-methylpyrrolidin-1-yl)propoxy)phenyl)-N⁴,6-dimethylpyrimidine-2,4-diamine as a white solid.

### Example 137: Synthesis of Compound 518 (Reference)

### Compound 518: Synthesis of 4-cyclopentyl-6-methoxy-N-methyl-7-(3-(pyrrolidin-1-yl)propoxy)quinazolin-2-amine

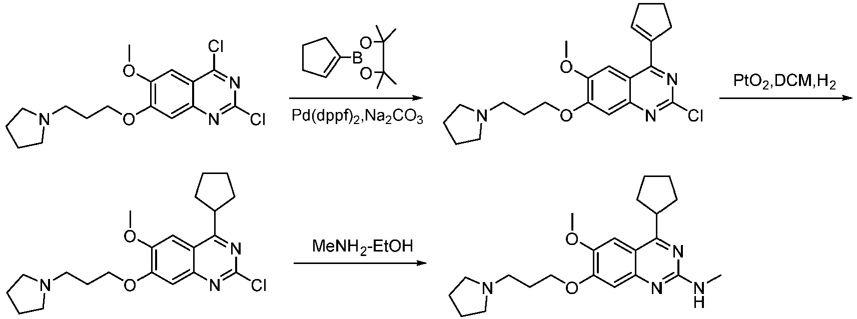

### Step 1: Synthesis of 2-chloro-4-(cyclopent-1-en-1-yl)-6-methoxy-7-[3-(pyrrolidin-1-yl)propoxy] quinazoline:

Into a 20-mL vial purged and maintained with an inert atmosphere of nitrogen, was placed 2,4-dichloro-6-methoxy-7-[3-(pyrrolidin-1-yl)propoxy]quinazoline (500 mg, 1.40 mmol, 1 equiv), Pd(dppf)₂ (115 mg, 0.10 equiv), 2-(cyclopent-1-en-1-yl)-4,4,5,5-tetramethyl-1,3,2-dioxaborolane (273 mg, 1.41 mmol, 1 equiv), sodium methaneperoxoate (447.9 mg, 4.19 mmol, 3.00 equiv), dioxane (8 mL), water (2 mL). The resulting solution was stirred for 4 h at 60 °C in an oil bath. The resulting solution was diluted with 5 mL of H₂O. The resulting solution was extracted with 3x10 mL of dichloromethane and the organic layers combined. The resulting mixture was washed with 3x10 mL of H₂O. The resulting mixture was concentrated under vacuum. The residue was applied onto a silica gel column with methanol/H₂O (10:1). This resulted in 300 mg (55%) of as a solid.

Analytical Data: LC-MS: (ES, *m*/*z*): RT = 1.03min, m/z = 388 [M+1].

### Step 2: Synthesis of 2-chloro-4-cyclopentyl-6-methoxy-7-[3-(pyrrolidin-1-yl)propoxy]quinazoline:

Into a 250-mL round-bottom flask, was placed 2-chloro-4-(cyclopent-1-en-1-yl)-6-methoxy-7-[3-(pyrrolidin-1-yl)propoxy]quinazoline (300 mg, 0.77 mmol, 1 equiv), dichloromethane (100 mL), dioxoplatinum, hydrogen . The resulting solution was stirred for 12 h at 20 °C. The solids were filtered out. The resulting mixture was concentrated under vacuum. The residue was applied onto a silica gel column with CH₃CN/H₂O (1:5). This resulted in 200 mg (66%) of the title compound as a brown solid.

Analytical Data: LC-MS: (ES, *m*/*z*): RT = 0.871min, m/z =390 [M+1].

### Step 3: Synthesis of 4-cyclopentyl-6-methoxy-N-methyl-7-[3-(pyrrolidin-1-yl)propoxy] quinazolin-2-amine:

Into a 10-mL sealed tube, was placed 2-chloro-4-cyclopentyl-6-methoxy-7-[3-(pyrrolidin-1-yl)propoxy]quinazoline (130 mg, 0.33 mmol, 1 equiv), ethanol; methanamine (2 mL). The resulting solution was stirred for 3 h at 80°C in an oil bath. The resulting mixture was concentrated under vacuum. The crude product (130 mg) was purified by Flash-Prep-HPLC A Grad. This resulted in 31.2 mg (19%) of 4-cyclopentyl-6-methoxy-N-methyl-7-[3-(pyrrolidin-1-yl)propoxy]quinazolin-2-amine as a yellow solid.

### Example 138: Synthesis of Compound 523 (Reference)

### Compound 523: Synthesis of 4-cyclohexyl-6-methoxy-N-methyl-7-(3-(pyrrolidin-1-yl)propoxy)quinazolin-2-amine

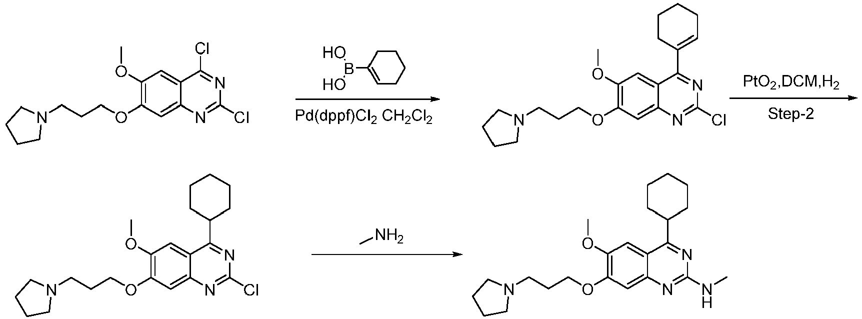

### Step 1: Synthesis of 2-chloro-4-(cyclohex-1-en-1-yl)-6-methoxy-7-[3-(pyrrolidin-1-yl)propoxy]quinazoline:

Into a 100-mL round-bottom flask purged and maintained with an inert atmosphere of nitrogen, was placed 2,4-dichloro-6-methoxy-7-[3-(pyrrolidin-1-yl)propoxy]quinazoline (300 mg, 0.84 mmol, 1 equiv), (cyclohex-1-en-1-yl)boronic acid (116 mg, 0.92 mmol, 1.1 equiv), Pd(dppf)Cl₂ dichloromethane (69 mg, 0.10 equiv), sodium carbonate (179 mg, 1.69 mmol, 2.00 equiv), dioxane (16 mL), water(4 mL). The resulting solution was stirred for 7 h at 60 °C in an oil bath. The solids were filtered out. The resulting mixture was concentrated under vacuum. The crude product (350 mg) was purified by Flash HPLC MeOH. This resulted in 220 mg (64%) of the title compound as yellow oil.

Analytical Data: LC-MS: (ES, *mlz*): RT = 1.08 min, LCMS 53: *m*/*z* = 402.0 [M+1]. ¹H NMR (400 MHz, Methanol-*d*₄) δ 7.47 (s, 1H), 7.25 (s, 1H), 6.25 - 6.22 (m, 1H), 4.29 (t, *J=* 6.1 Hz, 2H), 3.98 (s, 3H), 2.82 - 2.74 (m, 2H), 2.67 - 2.60 (m, 4H), 2.56 - 2.49 (m, 2H), 2.39 - 2.20 (m, 2H), 2.17 - 2.10 (m, 2H), 1.97 - 1.78 (m, 8H).

### Step 2: Synthesis of 2-chloro-4-cyclohexyl-6-methoxy-7-[3-(pyrrolidin-1-yl)propoxy] quinazoline:

Into a 100-mL round-bottom flask, was placed 2-chloro-4-(cyclohex-1-en-1-yl)-6-methoxy-7-[3-(pyrrolidin-1-yl)propoxy]quinazoline (220 mg, 0.55 mmol, 1 equiv), PtO₂ (200 mg), methanol (15 mL). The resulting solution was stirred for 12 h at 25 °C under H₂(g). The solids were filtered out. The resulting mixture was concentrated under vacuum. This resulted in 218 mg (87%) of as a yellow solid.

Analytical Data: LC-MS: (ES, *mlz*): RT = 1.15 min, LCMS 53: *m*/*z* = 404.0 [M+1].

### Step 3: Synthesis of 4-cyclohexyl-6-methoxy-N-methyl-7-[3-(pyrrolidin-1-yl)propoxy] quinazolin-2-amine:

Into a 50-mL round-bottom flask, was placed 2-chloro-4-cyclohexyl-6-methoxy-7-[3-(pyrrolidin-1-yl)propoxy]quinazoline (200 mg, 0.50 mmol, 1 equiv), Methylamine ethanol solution(32%) (15 mL, 1 equiv). The resulting solution was stirred for 1 h at 80 °C in an oil bath. The resulting mixture was concentrated under vacuum. The crude product (210mg) was purified by Flash HPLC A Grad. This resulted in 71.8 mg (35%) of 4-cyclohexyl-6-methoxy-N-methyl-7-[3-(pyrrolidin-1-yl)propoxy]quinazolin-2-amine as a yellow solid.

### Example 139: Synthesis of Compound 538 (Reference)

### Compound 538: Synthesis of N⁴-methyl-N²-(4-(3-(pyrrolidin-1-yl)propoxy)-1H-indazol-6-yl)pyrimidine-2,4-diamine

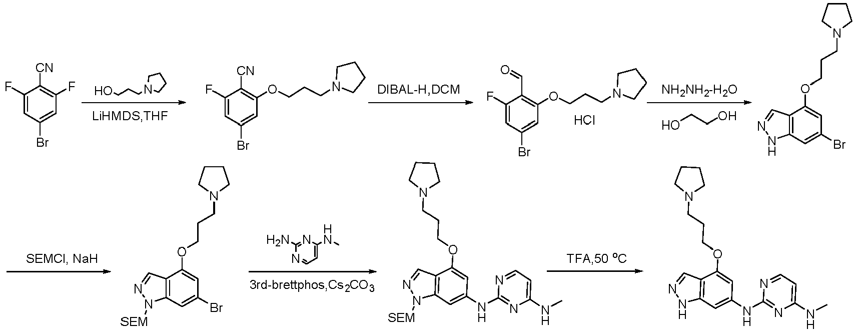

### Step 1: Synthesis of 4-bromo-2-fluoro-6-[3-(pyrrolidin-1-yl)propoxy]benzonitrile:

Into a 100-mL round-bottom flask, was placed 4-bromo-2,6-difluorobenzonitrile (1 g, 4.59 mmol, 1 equiv), LiHMDS (5.5 mL), tetrahydrofuran (30 mL), 3-(pyrrolidin-1-yl)propan-1-ol (710 mg, 5.50 mmol, 1.20 equiv). The resulting solution was stirred for 30 min at 25 °C. The resulting solution was allowed to react, with stirring, for an additional 2 h at 25 °C. The reaction was then quenched by the addition of water. The resulting solution was extracted with 2x100 mL of dichloromethane and the organic layers combined and concentrated under vacuum. The crude product was purified by Flash-Prep-HPLC A. This resulted in 0.18 g of the title compound as yellow oil.

Analytical Data: ¹H NMR (300 MHz, Chloroform-d) δ 7.13 - 6.95 (m, 2H), 4.22 (t, *J=* 6.3 Hz, 2H), 2.65 - 2.35 (m, 4H), 2.11 - 1.76 (m, 6H).

### Step 2: Synthesis of 4-bromo-2-fluoro-6-[3-(pyrrolidin-1-yl)propoxy]benzaldehyde:

Into a 250-mL round-bottom flask, was placed 4-bromo-2-fluoro-6-[3-(pyrrolidin-1-yl)propoxy]benzonitrile (2.3 g, 7.03 mmol, 1 equiv), DIBAL-H (12 mL), dichloromethane (50 mL). The resulting solution was stirred for 1 h at 25 °C. The resulting solution was allowed to react, with stirring, for an additional 2 h while the temperature was maintained at 40 °C in an oil bath. The reaction was then quenched by the addition of HCl. The resulting mixture was concentrated under vacuum. The crude product was purified by Flash-Prep-HPLC and this resulted in 1 g (39%) of the title compound as a yellow solid.

Analytical Data: ¹H NMR (300 MHz, DMSO-*d*₆) δ 10.29 (d, *J=* 1.4 Hz, 1H), 10.14 (s, 1H), 7.32 (d, *J* = 10.0 Hz, 2H), 4.28 (t, *J* = 5.8 Hz, 2H), 3.65 - 6.48 (m, 2H), 3.02 - 2.98 (m, 2H), 2.26 - 1.82 (m, 6H).

### Step 3: Synthesis of 6-bromo-4-[3-(pyrrolidin-1-yl)propoxy]-1H-indazole:

Into a 50-mL round-bottom flask, was placed 4-bromo-2-fluoro-6-[3-(pyrrolidin-1-yl)propoxy]benzaldehyde (1 g, 3.03 mmol, 1 equiv), NH₂NH₂H2O (3 mL), ethylene glycol (5 mL). The resulting solution was stirred for 2 h at 120 °C in an oil bath. The resulting solution was extracted with 2x100 mL of dichloromethane and the organic layers combined. The crude product was purified by Flash-Prep-HPLC and this resulted in 0.45 g (41%) of the title compound as a light yellow solid.

Analytical Data: ¹H NMR (300 MHz, Chloroform-d) δ 10.35 (s, 1H), 8.08 (d, *J =* 1.0 Hz, 1H), 7.26 (t, *J =* 1.2 Hz, 1H), 6.63 (d, *J =* 1.2 Hz, 1H), 4.21 (t, *J =* 6.2 Hz, 2H), 2.79 (t, *J=* 7.5 Hz, 2H), 2.70 (s, 4H), 2.18 (p, *J=* 6.6 Hz, 2H), 1.89 (p, *J=* 3.3 Hz, 4H).

### Step 4: Synthesis of 6-bromo-4-[3-(pyrrolidin-1-yl)propoxy]-1-[[2-(trimethylsilyl)ethoxy]methyl]-1H-indazole:

Into a 250-mL round-bottom flask, was placed 6-bromo-4-[3-(pyrrolidin-1-yl)propoxy]-1H-indazole (400 mg, 1.23 mmol, 1 equiv), sodium hydride (300 mg, 12.50 mmol, 10.13 equiv), tetrahydrofuran (40 mL), SEMCl (0.6 g). The resulting solution was stirred for 20 min at 0 °C in a water/ice bath. The resulting solution was allowed to react, with stirring, for an additional 3 h at 25 °C. The reaction was then quenched by the addition of water. The resulting solution was extracted with 2x100 mL of dichloromethane and the organic layers combined. The crude product was purified by Flash-Prep-HPLC and this resulted in 0.22 g (39%) of the title compound as yellow oil.

Analytical Data: ¹H NMR (300 MHz, Chloroform-d) δ 8.14 - 8.02(m, 1H), 7.49 - 7.35(m, 1H), 6.67 - 6.48 (m, 1H), 5.66 (d, *J=* 2.6 Hz, 2H), 4.25 - 4.15(m, 2H), 3.69 - 3.47 (m, 2H), 3.08 - 2.48 (m, 6H), 2.25 - 2.12 (m, 2H), 1.27 (d, *J* = 1.6 Hz, 1H), 1.03 - 0.84 (m, 2H), 0.10 - 0.01 (m, 12H).

### Step 5: Synthesis of 4-N-methyl-2-N-[4-[3-(pyrrolidin-1-yl)propoxy]-1-[[2-(trimethylsilyl)ethoxy]methyl]-1H-indazol-6-yl]pyrimidine-2,4-diamine:

Into a 10-mL round-bottom flask, was placed 4-N-methylpyrimidine-2,4-diamine (200 mg, 1.61 mmol, 4.07 equiv), 6-bromo-4-[3-(pyrrolidin-1-yl)propoxy]-1-[[2-(trimethylsilyl)ethoxy]methyl]-1H-indazole (180 mg, 0.40 mmol, 1 equiv), 3rd-brettphos (50 mg), Cs₂CO₃ (300 mg, 0.92 mmol, 2.32 equiv), dioxane (5 mL). The resulting solution was stirred for 12 h at 110 °C in an oil bath. The resulting solution was extracted with 2x50 mL of dichloromethane and the organic layers combined. The resulting mixture was washed with 1x100 mL of brine. The mixture was dried over anhydrous sodium sulfate and concentrated under vacuum. This resulted in 0.22 g of the title compound as an oil.

### Step 6: Synthesis of N⁴-methyl-N²-(4-(3-(pyrrolidin-1-yl)propoxy)-1H-indazol-6-yl)pyrimidine-2,4-diamine:

Into a 25-mL round-bottom flask, was placed 4-N-methyl-2-N-[4-[3-(pyrrolidin-1-yl)propoxy]-1-[[2-(trimethylsilyl)ethoxy]methyl]-1H-indazol-6-yl]pyrimidine-2,4-diamine (40 mg, 0.08 mmol, 1 equiv), trifluoroacetic acid (3 mL). The resulting solution was stirred for 30 min at 50 °C in an oil bath. The resulting mixture was concentrated under vacuum. The crude product was purified by Prep-HPLC B. This resulted in 10 mg (33%) of N⁴-methyl-N²-(4-(3-(pyrrolidin-1-yl)propoxy)-1H-indazol-6-yl)pyrimidine-2,4-diamine as yellow oil.

### Example 140: Synthesis of Compound 541 (Reference)

### Compound 541: Synthesis of N⁴-methyl-N²-(piperidin-3-yl)pyrimidine-2,4-diamine

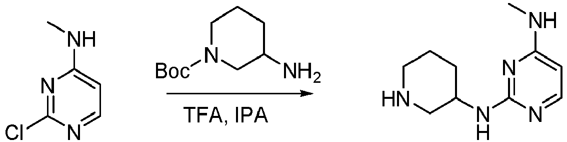

### Step 1: Synthesis of N⁴-methyl-N²-(piperidin-3-yl)pyrimidine-2,4-diamine:

Into a 50-mL round-bottom flask, was placed 2-chloro-N-methylpyrimidin-4-amine (150 mg, 1.04 mmol, 1 equiv), tert-butyl 3-aminopiperidine-1-carboxylate (220 mg, 1.10 mmol, 1.05 equiv), trifluoroacetic acid (380 mg, 3.36 mmol, 3.00 equiv), IPA (5 mL). The resulting solution was stirred for 16 h at 90 °C in an oil bath. The crude product was purified by Prep-HPLC C NH₄HCO₃. This resulted in 132.4 mg (61%) of N⁴-methyl-N²-(piperidin-3-yl)pyrimidine-2,4-diamine as a white powder.

### Example 141: Synthesis of Compound 542 (Reference)

### Compound 542: Synthesis of N⁴-methyl-N²-(piperidin-4-yl)pyrimidine-2,4-diamine

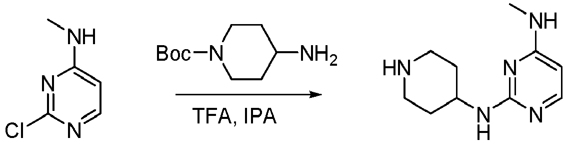

### Step 1: Synthesis of N⁴-methyl-N²-(piperidin-4-yl)pyrimidine-2,4-diamine:

Into a 50-mL round-bottom flask, was placed 2-chloro-N-methylpyrimidin-4-amine (150 mg, 1.04 mmol, 1 equiv), trifluoroacetic acid (480 mg, 4.25 mmol, 4.00 equiv), IPA (5 mL), tert-butyl 4-aminopiperidine-1-carboxylate (250 mg, 1.25 mmol, 1.19 equiv). The resulting solution was stirred for 16 h at 90 °C in an oil bath. The crude product was purified by Prep-HPLC C NH₄HCO₃. This resulted in 42.2 mg (19%) of N⁴-methyl-N²-(piperidin-4-yl)pyrimidine-2,4-diamine as light yellow oil.

### Example 142: Synthesis of Compound 543 (Reference)

### Compound 543: Synthesis of N²-butyl-N⁴-methylpyrimidine-2,4-diamine

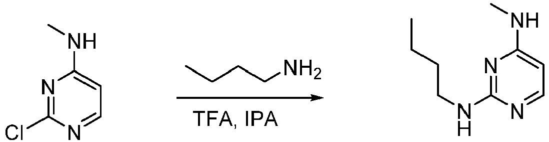

### Step 1: Synthesis of N²-butyl-N⁴-methylpyrimidine-2,4-diamine:

Into a 50-mL round-bottom flask, was placed 2-chloro-N-methylpyrimidin-4-amine (150 mg, 1.04 mmol, 1 equiv), butan-1-amine (80 mg, 1.09 mmol, 1.05 equiv), trifluoroacetic acid (380 mg, 3.36 mmol, 3.00 equiv), IPA (5 mL). The resulting solution was stirred for 16 h at 90 °C in an oil bath. The crude product was purified by Prep-HPLC C NH₄HCO₃. This resulted in 35.1 mg (19%) of N²-butyl-N⁴-methylpyrimidine-2,4-diamine as white oil.

Analytical Data: LC-MS: (ES, *mlz*): RT = 1.15 min, LCMS 07: *m*/*z* = 181.1 [M+1]. ¹H NMR (300 MHz, Methanol-*d*₄) δ 7.61 (d, *J* = 6.0 Hz, 1H), 5.77 (d, *J=* 6.0 Hz, 1H), 3.34 (t, *J=* 4.5 Hz, 1H), 3.32 (t, *J=* 1.5 Hz, 1H), 2.87 (s, 3H), 1.63 - 1.53 (m, 2H), 1.48 - 1.36 (m, 2H), 0.98 (t, *J* = 7.2 Hz, 3H).

### Example 143: Synthesis of Compound 546 (Reference)

### Compound 546: Synthesis of N⁴-methyl-N²-(3-methylpiperidin-3-yl)pyrimidine-2,4-diamine

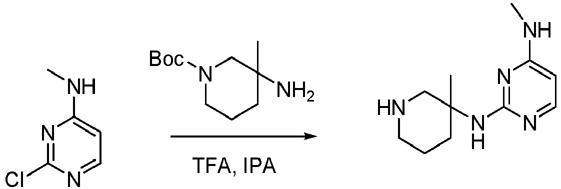

### Step 1: Synthesis of N⁴-methyl-N²-(3-methylpiperidin-3-yl)pyrimidine-2,4-diamine:

Into a 50-mL round-bottom flask, was placed 2-chloro-N-methylpyrimidin-4-amine (200 mg, 1.39 mmol, 1 equiv), tert-butyl 3-amino-3-methylpiperidine-1-carboxylate (357 mg, 1.67 mmol, 1.20 equiv), trifluoroacetic acid (791 mg, 7.00 mmol, 5.02 equiv), IPA (4 mL). The resulting solution was stirred for 16 h at 90 °C in an oil bath. The crude product was purified by Prep-HPLC C NH₄HCO₃. This resulted in 52.4 mg (17%) N⁴-methyl-N²-(3-methylpiperidin-3-yl)pyrimidine-2,4-diamine as a light yellow solid.

### Example 144: Synthesis of Compound 547 (Reference)

### Compound 547: Synthesis of N⁴-methyl-N²-(4-methylpiperidin-4-yl)pyrimidine-2,4-diamine

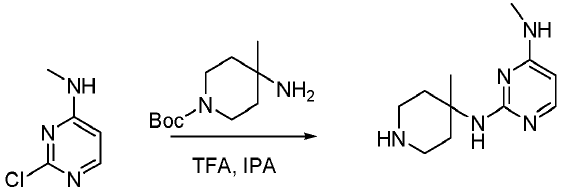

### Step 1: Synthesis of N⁴-methyl-N²-(4-methylpiperidin-4-yl)pyrimidine-2,4-diamine:

Into a 50-mL round-bottom flask, was placed 2-chloro-N-methylpyrimidin-4-amine (400 mg, 2.79 mmol, 1 equiv), trifluoroacetic acid (1109 mg, 9.81 mmol, 4.00 equiv), IPA (10 mL), tert-butyl 4-amino-4-methylpiperidine-1-carboxylate (573 mg, 2.67 mmol, 1.10 equiv). The resulting solution was stirred for 16 h at 90 °C in an oil bath. The crude product was purified by Prep-HPLC C NH₄HCO₃. This resulted in 34 mg (6%) of N⁴-methyl-N²-(4-methylpiperidin-4-yl)pyrimidine-2,4-diamine as a white semisolid.

### Example 145: Synthesis of Compound 548 (Reference)

### Compound 548: Synthesis of N²-((1R,3S)-3-aminocyclopentyl)-N⁴-methylpyrimidine-2,4-diamine

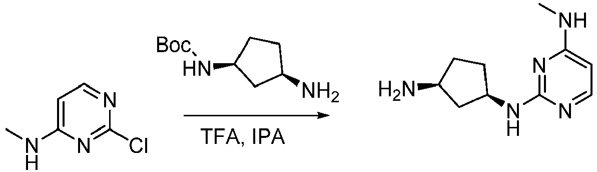

### Step 1: Synthesis of N²-((1R,3S)-3-aminocyclopentyl)-N⁴-methylpyrimidine-2,4-diamine:

Into a 100-mL round-bottom flask, was placed 2-chloro-N-methylpyrimidin-4-amine (300 mg, 2.09 mmol, 1 equiv), trifluoroacetic acid (2.375 g, 21.01 mmol, 10.06 equiv), IPA (5 mL), tert-butyl N-[(1S,3R)-3-aminocyclopentyl]carbamate (459 mg, 2.29 mmol, 1.10 equiv). The resulting solution was stirred for 16 h at 90 °C in an oil bath. The crude product was purified by Prep-HPLC C TFA. This resulted in 33.2 mg (5%) of N²-((1R,3S)-3-aminocyclopentyl)-N⁴-methylpyrimidine-2,4-diamine as light yellow oil.

### Example 146: Synthesis of Compound 549 (Reference)

### Compound 549: Synthesis of N²-(1-butyl-3-methylpiperidin-3-yl)-N⁴-methylpyrimidine-2,4-diamine

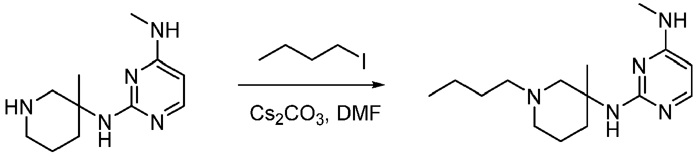

### Step 1: Synthesis of N²-(1-butyl-3-methylpiperidin-3-yl)-N⁴-methylpyrimidine-2,4-diamine:

Into a 50-mL round-bottom flask, was placed 4-N-methyl-2-N-(3-methylpiperidin-3-yl)pyrimidine-2,4-diamine (150 mg, 0.68 mmol, 1 equiv), CsCO3 (231 mg, 2.50 equiv), N,N-dimethylformamide (2 mL), 1-iodobutane (187 mg, 1.02 mmol, 1.50 equiv). The resulting solution was stirred for 3 days at 20 °C. The crude product was purified by Prep-HPLC C TFA. This resulted in 53.6 mg (20%) of N²-(1-butyl-3-methylpiperidin-3-yl)-N⁴-methylpyrimidine-2,4-diamine as a light yellow oil.

### Example 147: Synthesis of Compound 550 (Reference)

### Compound 550: Synthesis of N²-(1-butyl-4-methylpiperidin-4-yl)-N⁴-methylpyrimidine-2,4-diamine

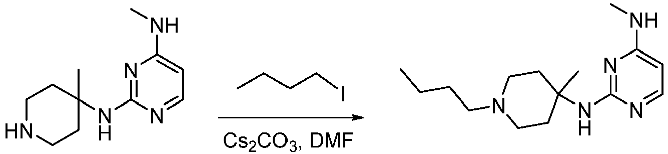

### Step 1: Synthesis of N²-(1-butyl-4-methylpiperidin-4-yl)-N⁴-methylpyrimidine-2,4-diamine:

Into a 50-mL round-bottom flask, was placed 4-N-methyl-2-N-(4-methylpiperidin-4-yl)pyrimidine-2,4-diamine (220 mg, 0.99 mmol, 1 equiv), Cs₂CO₃ (338.5 mg), N,N-dimethylformamide (3 mL), 1-iodobutane (275 mg, 1.49 mmol, 1.50 equiv). The resulting solution was stirred for 2 days at 20 °C. The residue was applied onto a silica gel column with CH₃CN/H₂O (40%). This resulted in 62.1 mg (23%) of N²-(1-butyl-4-methylpiperidin-4-yl)-N⁴-methylpyrimidine-2,4-diamine as light yellow oil.

### Example 148: Synthesis of Compound 551

### Compound 551: Synthesis of N²-(3-(3-(cyclobutyl(methyl)amino)propoxy)-4-methoxyphenyl)-N⁴,6-dimethylpyrimidine-2,4-diamine

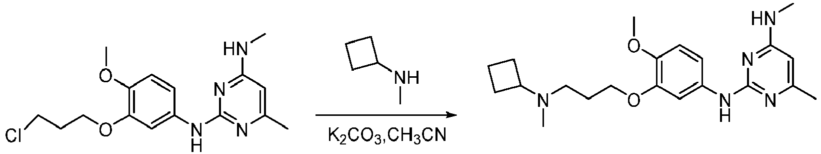

### Step 1: Synthesis of N²-(3-(3-(cyclobutyl(methyl)amino)propoxy)-4-methoxyphenyl)-N⁴,6-dimethylpyrimidine-2,4-diamine:

Into a 100-mL round-bottom flask, was placed 2-N-[3-(3-chloropropoxy)-4-methoxyphenyl]-4-N,6-dimethylpyrimidine-2,4-diamine (200 mg, 0.59 mmol, 1 equiv), potassium carbonate (246 mg, 1.78 mmol, 3.00 equiv), NaI (89 mg, 1 equiv), N-methylcyclobutanamine (144 mg, 1.69 mmol, 2.00 equiv), CH₃CN (20 mL). The resulting solution was stirred for 10 h at 85 °C in an oil bath. The solids were filtered out. The resulting mixture was concentrated under vacuum. The crude product (200 mg) was purified by Prep-HPLC C HCl. This resulted in 82.3 mg (33%) of N²-(3-(3-(cyclobutyl(methyl)amino)propoxy)-4-methoxyphenyl)-N⁴,6-dimethylpyrimidine-2,4-diamine as a white solid.

### Example 149: Synthesis of Compound 642 (Reference)

### Compound 642: Synthesis of 2-N-(6-methoxy-5-[[(3R)-1-methylpyrrolidin-3-yl]methoxy]pyridin-3-yl)-4-N,6-dimethylpyrimidine-2,4-diamine

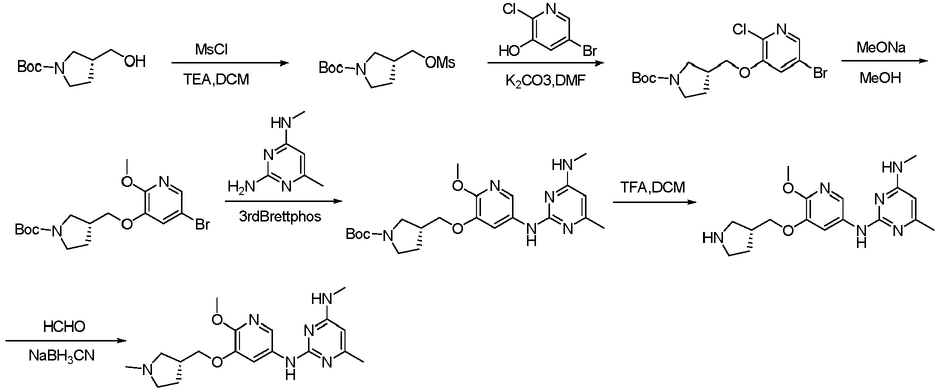

### Step 1: Synthesis of tert-butyl (3R)-3-[(methanesulfonyloxy)methyl]pyrrolidine-1 -carboxylate:

Into a 100-mL round-bottom flask, was placed tert-butyl (3R)-3-(hydroxymethyl)pyrrolidine-1-carboxylate (1 g, 4.97 mmol, 1.00 equiv), dichloromethane (10 mL), TEA (1.5 g, 14.82 mmol, 3.00 equiv), MsCl (850 mg, 7.46 mmol, 1.50 equiv). The resulting solution was stirred for 2 h at 25 °C. The resulting mixture was concentrated under vacuum. This resulted in 2 g (crude) of the title compound as yellow crude oil.

### Step 2: Synthesis of tert-butyl (3R)-3-[[(5-bromo-2-chloropyridin-3-yl)oxy]methyl]pyrrolidine-1-carboxylate:

Into a 100-mL round-bottom flask, was placed 5-bromo-2-chloropyridin-3-ol (1.04 g, 4.99 mmol, 1.00 equiv), tert-butyl (3R)-3-[(methanesulfonyloxy)methyl]pyrrolidine-1-carboxylate (1.4 g, 5.01 mmol, 1.00equiv), potassium carbonate (2.06 g, 14.90 mmol, 3.00 equiv), N,N-dimethylformamide (10 mL). The resulting solution was stirred for 12 h at 80 °C in an oil bath. The resulting solution was extracted with 3x50 mL of ethyl acetate and the organic layers combined. The resulting mixture was washed with 3x30 mL of brine. The residue was applied onto a silica gel column with ethyl acetate/petroleum ether (1:3). This resulted in 1.4 g (72%) of the title compound as a white solid.

Analytical Data: LCMS: (ES, *m*/*z*): RT = 1.469 min, LCMS15: m/z = 393 [M+1]. ¹H NMR: (400 MHz, Methanol-*d*₄) δ 8.07 (d, *J =* 2.0 Hz, 1H), 7.75 (d, *J =* 2.0 Hz, 1H), 4.19 - 4.09 (m, 2H), 3.69 - 2.69 (m, 8H), 2.24 - 1.76 (m, 2H), 1.48 (s, 9H).

### Step 3: Synthesis of : tert-butyl (3R)-3-[[(5-bromo-2-methoxypyridin-3-yl)oxy]methyl]pyrrolidine-1-carboxylate

Into a 25-mL round-bottom flask, was placed tert-butyl (3R)-3-[[(5-bromo-2-chloropyridin-3-yl)oxy]methyl]pyrrolidine-1-carboxylate (1.4 g, 3.57 mmol, 1.00 equiv), methanol (4 mL), NaOCH₃/MeOH (2 mL, 1.00 equiv). The resulting solution was stirred for 12 h at 70 °C in an oil bath. The resulting mixture was concentrated under vacuum. The resulting solution was extracted with 3x30 mL of ethyl acetate and the organic layers combined. This resulted in the title compound 1.4 g (crude) of as colorless oil.

Analytical Data: LC-MS: (ES, *m*/*z*): RT = 1.491 min, LCMS28: m/z = 387 [M+1].

### Step 4: Synthesis of tert-butyl (3R)-3-[[(2-methoxy-5-[[4-methyl-6-(methylamino)pyrimidin-2-yl] amino]pyridin-3-yl)oxy] methyl] pyrrolidine-1 -carboxylate:

Into a 50-mL round-bottom flask purged and maintained with an inert atmosphere of nitrogen, was placed tert-butyl (3R)-3-[[(5-bromo-2-methoxypyridin-3-yl)oxy]methyl]pyrrolidine-1-carboxylate (500 mg, 1.29 mmol, 1.00 equiv), 4-N,6-dimethylpyrimidine-2,4-diamine (196.6 mg, 1.42 mmol, 1.10 equiv), Cs₂CO₃ (1.26 g, 3.87 mmol, 3.00 equiv), 3rd-Brettphos (117.4 mg, 0.13 mmol, 0.10 equiv), DMSO (5 mL). The resulting solution was stirred for 12 h at 100 °C in an oil bath. The solids were filtered out. The crude product was purified by Flash-Prep-HPLC with the following conditions (IntelFlash-1): Column, silica gel; mobile phase, H₂O:ACN = 40% ; Detector, UV 254 nm. This resulted in 340 mg (59%) of the title compound as a white solid.

Analytical Data: LC-MS: (ES, *m*/*z*): RT = 1.077 min, LCMS53: m/z = 445 [M+1].

### Step 5: Synthesis of 2-N-[6-methoxy-5-[(3R)-pyrrolidin-3-ylmethoxy]pyridin-3-yl]-4-N,6-dimethylpyrimidine-2,4-diamine:

Into a 50-mL round-bottom flask, was placed tert-butyl (3R)-3-[[(2-methoxy-5-[[4-methyl-6-(methylamino)pyrimidin-2-yl]amino]pyridin-3-yl)oxy]methyl]pyrrolidine-1-carboxylate (340 mg, 0.76 mmol, 1.00 equiv), dichloromethane (5 mL), trifluoroacetic acid (1 mL). The resulting solution was stirred for 2 h at 25 °C. The resulting mixture was concentrated under vacuum. This resulted in 1 g (crude) of the title compound as yellow crude oil.

Analytical Data: LC-MS: (ES, *m*/*z*): RT = 0.814 min, LCMS33: m/z = 345 [M+1].

### Step 6: Synthesis of 2-N-(6-methoxy-5-[[(3R)-1-methylpyrrolidin-3-yl]methoxy]pyridin-3-yl)-4-N,6-dimethylpyrimidine-2,4-diamine

Into a 50-mL round-bottom flask, was placed 2-N-[6-methoxy-5-[(3R)-pyrrolidin-3-ylmethoxy]pyridin-3-yl]-4-N,6-dimethylpyrimidine-2,4-diamine (100 mg, 0.29 mmol, 1.00 equiv), methanol (5 mL), HCHO (29 mg, 0.97 mmol, 1.00 equiv), NaBH₃CN (115 mg, 1.83 mmol, 6.00 equiv). The resulting solution was stirred for 2 h at 25 °C. The crude product was purified by Prep-HPLC with Method C NH4HCO3. This resulted in 55.8 mg (54%) of the title compound as a white solid.

### Example 150: Synthesis of Compound 644

### Compound 644: Synthesis of :2-N-[4-methoxy-3-([[2-(pyrrolidin-1-yl)ethyl]amino]methyl)phenyl]-4-N,6-dimethylpyrimidine-2,4-diamine

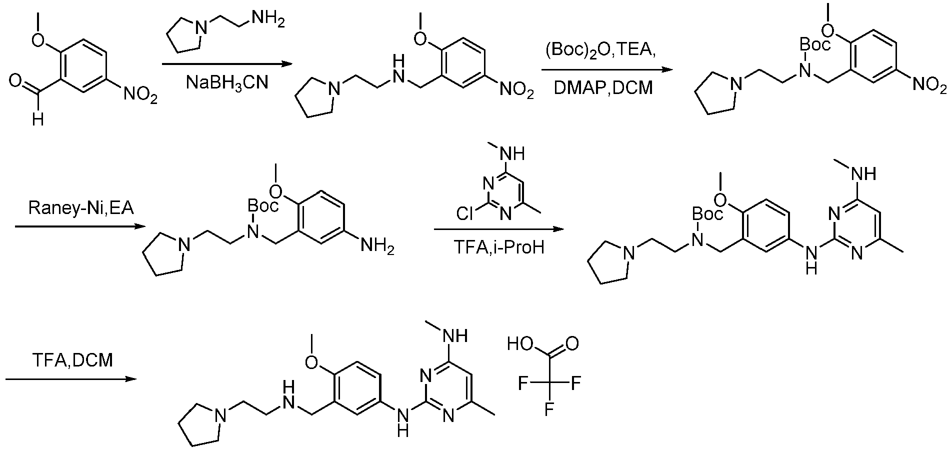

### Step 1: Synthesis of [(2-methoxy-5-nitrophenyl)methyl][2-(pyrrolidin-1-yl)ethyl] amine:

Into a 100-mL round-bottom flask, was placed methanol (50 mL), 2-methoxy-5-nitrobenzaldehyde (1 g, 5.52 mmol, 1.00 equiv), 2-(pyrrolidin-1-yl)ethan-1-amine (630 mg, 5.52 mmol, 1.00 equiv), NaBH₃CN (1 g, 15.91 mmol, 2.88 equiv). The resulting solution was stirred for 1 h at 20 °C. The resulting mixture was concentrated under vacuum. The residue was applied onto a silica gel column with H2O/ACN (10:1). This resulted in 240 mg (16%) of the title as yellow oil.

Analytical Data: LC-MS: (ES, *m*/*z*): RT = 0.861min, LCMS 69: *m*/*z* = 280 [M+1].

### Step 2: Synthesis of tert-butyl N-[(2-methoxy-5-nitrophenyl)methyl]-N-[2-(pyrrolidin-1-yl)ethyl]carbamate:

Into a 50-mL round-bottom flask, was placed dichloromethane (10 mL), [(2-methoxy-5-nitrophenyl)methyl][2-(pyrrolidin-1-yl)ethyl]amine (240 mg, 0.86 mmol, 1.00 equiv), Boc₂O (281 mg, 1.29 mmol, 1.50 equiv), TEA (261 mg, 2.58 mmol, 3.00 equiv), 4-dimethylaminopyridine (10 mg, 0.08 mmol, 0.10 equiv). The resulting solution was stirred for 12 h at 20 °C. The resulting mixture was concentrated under vacuum. The residue was applied onto a silica gel column with H2O/ACN (1:1). This resulted in 170 mg (52%) of the title compound as yellow oil.

Analytical Data: LC-MS: (ES, *m*/*z*): RT = 0.76 min, LCMS 45: *m*/*z* = 380 [M+1].

### Step 3: Synthesis of tert-butyl N-[(5-amino-2-methoxyphenyl)methyl]-N-[2-(pyrrolidin-1-yl)ethyl]carbamate:

Into a 100-mL round-bottom flask, was placed ethyl acetate (10 mL), tert-butyl N-[(2-methoxy-5-nitrophenyl)methyl]-N-[2-(pyrrolidin-1-yl)ethyl]carbamate (170 mg, 0.45 mmol, 1.00 equiv), Raney-Ni (20 mg). The flask was purged and maintained with H₂.The resulting solution was stirred for 1 h at 20°C. The solids were filtered out. The resulting mixture was concentrated under vacuum. This resulted in 110 mg (70%) of the title compound as yellow oil.

Analytical Data: LC-MS: (ES, *m*/*z*): RT = 0.86 min, LCMS 28: *m*/*z* = 350 [M +1]. ¹H NMR (300 MHz, Methanol-*d*₄) δ 6.78 (d, *J=* 8.5 Hz, 1H), 6.74 - 6.60 (m, 2H), 4.41 (s, 2H), 3.76 (s, 3H), 2.57 (d, *J =* 13.2 Hz, 7H), 1.85 - 1.74 (m, 5H), 1.48 (d, *J =* 17.1 Hz, 9H).

### Step 4: Synthesis of tert-butyl N-[(2-methoxy-5-[[4-methyl-6-(methylamino)pyrimidin-2-yl]amino]phenyl)methyl]-N-[2-(pyrrolidin-1-yl)ethyl]carbamate:

Into a 100-mL round-bottom flask, was placed isopropanol (10 mL), tert-butyl N-[(5-amino-2-methoxyphenyl)methyl]-N-[2-(pyrrolidin-1-yl)ethyl]carbamate (110 mg, 0.31 mmol, 1.00 equiv), 2-chloro-N,6-dimethylpyrimidin-4-amine (49 mg, 0.31 mmol, 0.99 equiv), trifluoroacetic acid (61 mg, 0.54 mmol, 1.71 equiv). The resulting solution was stirred for 2 h at 20°C. The resulting mixture was concentrated under vacuum. This resulted in 248 mg (167%) of as yellow oil.

Analytical Data: LC-MS: (ES, *m*/*z*): RT = 1.45 min, LCMS 33: *m*/*z* = 471 [M+1].

### Step 5: Synthesis of 2-N-[4-methoxy-3-([[2-(pyrrolidin-1-yl)ethyl]amino]methyl)phenyl]-4-N,6-dimethylpyrimidine-2,4-diamine:

Into a 100-mL round-bottom flask, was placed dichloromethane (2 mL), tert-butyl N-[(2-methoxy-5-[[4-methyl-6-(methylamino)pyrimidin-2-yl]amino]phenyl)methyl]-N-[2-(pyrrolidin-1-yl)ethyl]carbamate (248 mg, 0.53 mmol, 1.00 equiv), trifluoroacetic acid (2 mL). The resulting solution was stirred for 1 h at 20 °C. The resulting mixture was concentrated under vacuum. The crude product (200 mg) was purified by Prep-HPLC with Method C TFA. This resulted in 70.6 mg (28%) of the title compound as a trifluoroacetic acid as an off-white solid.

### Example 151: Synthesis of Compound 524 (Reference)

### Compound 524: Synthesis of 6-methoxy-N-methyl-4-(oxan-4-yl)-7-[3-(pyrrolidin-1-yl)propoxylquinolin-2-amine

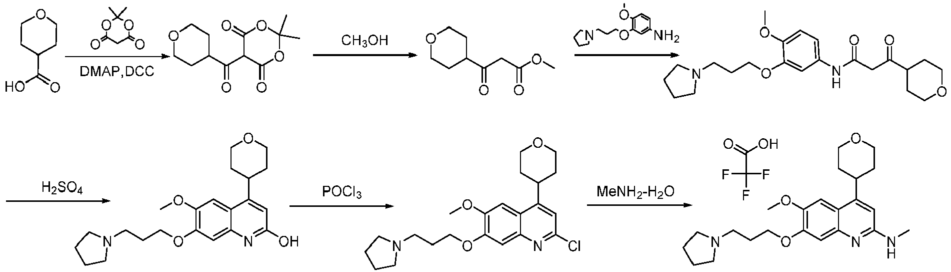

### Step 1: Synthesis of 2,2-dimethyl-5-[(oxan-4-yl)carbonyl]-1,3-dioxane-4,6-dione:

Into a 250-mL round-bottom flask, was placed oxane-4-carboxylic acid (6 g, 46.10 mmol, 1.00 equiv), 4-dimethylaminopyridine (8.4 g, 68.76 mmol, 1.49 equiv), DCC (9.6 g, 46.53 mmol, 1.01 equiv), dichloromethane (50 mL), 2,2-dimethyl-1,3-dioxane-4,6-dione (6.6 g, 45.79 mmol, 0.99 equiv). The resulting solution was stirred for 1 overnight at 0 °C. The resulting solution was extracted with of dichloromethane and the organic layers combined and concentrated under vacuum. This resulted in 9.2 g (78%) of the title compound as a light yellow solid.

Analytical Data: LC-MS: (ES, *m*/*z*): RT=0.422 min, LCMS34, m/z=255 [M+1].

### Step 2: Synthesis of methyl 3-(oxan-4-yl)-3-oxopropanoate:

Into a 100-mL round-bottom flask, was placed 2,2-dimethyl-5-[(oxan-4-yl)carbonyl]-1,3-dioxane-4,6-dione (5 g, 19.51 mmol, 1.00 equiv), methanol (20 mL). The resulting solution was stirred for 1 overnight at 60 °C. The resulting mixture was concentrated under vacuum. The residue was applied onto a silica gel column with ethyl acetate/petroleum ether (31/69). This resulted in 3.2 g (88%) of the title compound as an off-white liquid.

Analytical Data: LC-MS: (ES, *m*/*z*): RT=0.735 min, LCMS34, m/z=185 [M-1]. ¹H NMR: (300 MHz, Chloroform-d) δ 4.08 - 3.96 (m, 2H), 3.75 (s, 3H), 3.52 (s, 2H), 3.49 - 3.38 (m, 2H), 2.79 - 2.65 (m,1H), 1.87 - 1.62 (m, 4H).

### Step 3: N-[4-methoxy-3-[3-(pyrrolidin-1-yl)propoxy]phenyl]-3-(oxan-4-yl)-3-oxopropanamide:

Into a 10-mL vial, was placed methyl 3-(oxan-4-yl)-3-oxopropanoate (500 mg, 2.69 mmol, 1.00 equiv), AlMe₃ (0.4 mL, 3.00 equiv), toluene (2 mL), 4-methoxy-3-[3-(pyrrolidin-1-yl)propoxy]aniline (672 mg, 2.68 mmol, 1.00 equiv). The resulting solution was stirred for 48 h at 80 °C. The resulting solution was extracted with of dichloromethane and the organic layers combined and concentrated under vacuum. This resulted in 880 mg (81%) of the title compound as a brown oil.

Analytical Data: LC-MS: (ES, *m*/*z*): RT=0.600 min, LCMS45, m/z=405 [M+1].

### Step 4: Synthesis of 6-methoxy-4-(oxan-4-yl)-7-[3-(pyrrolidin-1-yl)propoxy] quinolin-2-ol

Into a 100-mL round-bottom flask, was placed N-[4-methoxy-3-[3-(pyrrolidin-1-yl)propoxy]phenyl]-3-(oxan-4-yl)-3-oxopropanamide (1 g, 2.47 mmol, 1.00 equiv), sulfuric acid (5 mL). The resulting solution was stirred for 0.5 h at 50 °C. The resulting solution was extracted with of dichloromethane and the organic layers combined and concentrated under vacuum. This resulted in 960 mg (98%) of the title compound as a gray solid.

Analytical Data: LC-MS: (ES, *m*/*z*): RT=0.837 min, LCMS07, m/z=387 [M+1].

### Step 5: Synthesis of 2-chloro-6-methoxy-4-(oxan-4-yl)-7-[3-(pyrrolidin-1-yl)propoxy] quinoline:

Into a 50-mL round-bottom flask, was placed 6-methoxy-4-(oxan-4-yl)-7-[3-(pyrrolidin-1-yl)propoxy]quinolin-2-ol (50 mg, 0.13 mmol, 1.00 equiv), phosphoroyl trichloride (2 mL). The resulting solution was stirred for 2 h at 110 °C. The resulting solution was extracted with of dichloromethane and the organic layers combined and concentrated under vacuum. This resulted in 38 mg (73%) of the title compound as a gray solid.

Analytical Data: LC-MS: (ES, *m*/*z*): RT=0.758 min, LCMS45, m/z = 405 [M+1]. ¹H NMR: (300 MHz, Chloroform-d) δ 7.39 (s, 1H), 7.17 (d, J = 13.9 Hz, 2H), 4.30 - 4.12 (m, 4H), 4.02 (s, 3H), 3.82 - 3.61 (m, 2H), 3.49 - 3.34 (m, 1H), 2.83 - 2.51 (m, 6H), 2.32 - 1.68 (m, 10H).

### Step 6: Synthesis of 6-methoxy-N-methyl-4-(oxan-4-yl)-7-[3-(pyrrolidin-1-yl)propoxy]quinolin-2-amine:

Into a 10-mL vial, was placed 2-chloro-6-methoxy-4-(oxan-4-yl)-7-[3-(pyrrolidin-1-yl)propoxy]quinoline (300 mg, 0.74 mmol, 1.00 equiv), MeNH₂-H₂O (5 g). The resulting solution was stirred for 48 h at 100 °C. The resulting mixture was concentrated under vacuum. The crude product (165.1 mg) was purified by Prep-HPLC with Method D TFA. This resulted in 165.1 mg (43%) of the title compound trifluoroacetic acid as a solid.

### Example 152: Synthesis of Compound 906

### Compound 906: Synthesis of (2S)-1-(2-methoxy-5-[[4-methyl-6-(methylamino)pyrimidin-2-yl]amino]phenoxy)-3-(pyrrolidin-1-yl)propan-2-ol

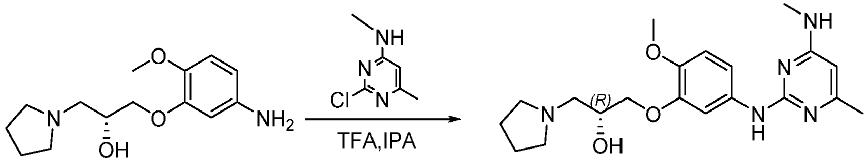

### Step 1: Synthesis of (2R)-1-(5-amino-2-methoxyphenoxy)-3-(pyrrolidin-1-yl)propan-2-ol:

Synthesis as for Compound 1038 starting with (2R)-2-(2-methoxy-5-nitrophenoxymethyl)oxirane and using pyrrolidine in place of azetidine.

### Step 2: Synthesis of (2S)-1-(2-methoxy-5-[[4-methyl-6-(methylamino)pyrimidin-2-yl] amino]phenoxy)-3-(pyrrolidin-1-yl)propan-2-ol:

Into a 20-mL round-bottom flask, was placed (2R)-1-(5-amino-2-methoxyphenoxy)-3-(pyrrolidin-1-yl)propan-2-ol (267 mg, 1.00 mmol, 1.00 equiv), 2-chloro-N,6-dimethylpyrimidin-4-amine (157 mg, 1.00 mmol, 0.99 equiv), trifluoroacetic acid (342 mg, 3.03 mmol, 3.02 equiv), IPA (10 mL). The resulting solution was stirred for 1 h at 8 °C. The solids were collected by filtration. The crude product was purified by Prep-HPLC with Method B TFA. This resulted in 12.3 mg of the title compound as a white solid.

### Example 153: Synthesis of Compound 1038

### Compound 1038: Synthesis of (2R)-1-(azetidin-1-yl)-3-(2-methoxy-5-[[4-methyl-6-(methylamino)pyrimidin-2-yl]amino]phenoxy)propan-2-ol

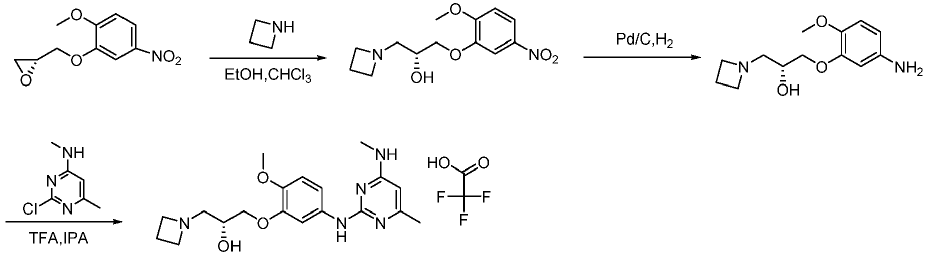

### Step 1: Synthesis of (2R)-1-(azetidin-1-yl)-3-(2-methoxy-5-nitrophenoxy)propan-2-ol:

Into a 40-mL round-bottom flask, was placed (2R)-2-(2-methoxy-5-nitrophenoxymethyl)oxirane (1 g, 4.44 mmol, 1.00 equiv), ethanol (10 mL), chloroform (10 mL), azetidine (507 mg, 8.88 mmol, 1.50 equiv). The resulting solution was stirred for 2 h at 75 °C in an oil bath. The resulting mixture was concentrated under vacuum. The residue was applied onto a silica gel column with ethyl acetate/petroleum ether (1:2). This resulted in 650 mg (52%) of the title compound as a yellow solid.

Analytical Data: LC-MS: (ES, *m*/*z*): RT=0.84 min, LCMS07 : m/z= 283.15 [M+1].

### Step 2: Synthesis of (2R)-1-(5-amino-2-methoxyphenoxy)-3-(azetidin-1-yl)propan-2-ol:

Into a 100-mL round-bottom flask, was placed (2R)-1-(azetidin-1-yl)-3-(2-methoxy-5-nitrophenoxy)propan-2-ol (600 g, 2.13 mol, 1.00 equiv), ethyl acetate (50 mL), Palladium carbon, hydrogen. The resulting solution was stirred for 1 h at 20 °C. The solids were filtered out. This resulted in the title compound 400 mg (75%) of as yellow oil.

Analytical Data: LC-MS: (ES, *m*/*z*): RT=0.35min, LCMS15: m/z=253.15 [M+1].

### Step 3: Synthesis of (2R)-1-(azetidin-1-yl)-3-(2-methoxy-5-[[4-methyl-6-(methylamino)pyrimidin-2-yl]amino]phenoxy)propan-2-ol:

Into a 20-mL round-bottom flask, was placed (2R)-1-(5-amino-2-methoxyphenoxy)-3-(azetidin-1-yl)propan-2-ol (400 mg, 1.59 mmol, 1.00 equiv), trifluoroacetic acid (538 mg, 4.76 mmol, 3.00 equiv), IPA (8 mL), 2-chloro-N,6-dimethylpyrimidin-4-amine (199 mg, 1.26 mmol, 0.80 equiv). The resulting solution was stirred for 2 h at 80 °C in an oil bath. The solids were filtered out. The resulting mixture was concentrated under vacuum. This resulted in 294.3 mg (38%) of the title compound as a trifluoroacetic acid as a pink solid.

### Example 154: Synthesis of Compound 965

### Compound 965: Synthesis of 2-N-[3-([[2-(azetidin-1-yl)ethyl]amino]methyl)-4-methoxyphenyl]-4-N-methylpyrimidine-2,4-diamine

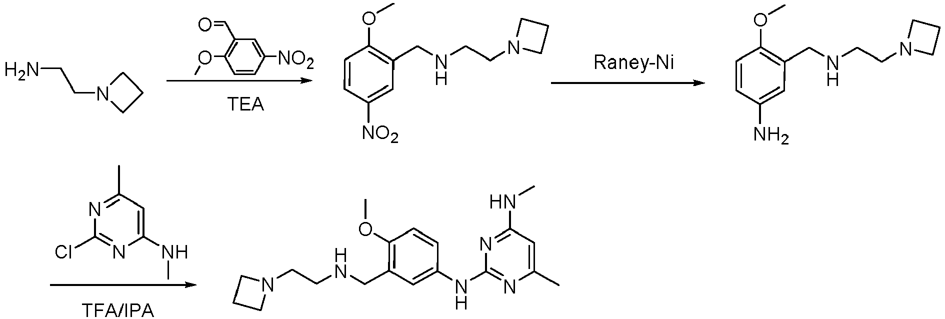

### Step 1: Synthesis of [2-(azetidin-1-yl)ethyl][(2-methoxy-5-nitrophenyl)methyl] amine:

Into a 250-mL round-bottom flask, was placed 2-(azetidin-1-yl)ethan-1-amine (500 mg, 4.99 mmol, 1.00 equiv), 2-methoxy-5-nitrobenzaldehyde (905 mg, 5.00 mmol, 1.00 equiv) in DCE (50 mL) and stirred for 15 min at 25 °C. Then NaBH(OAc)₃ (6.36 g) was added and stirred for 2 h at 25 °C. The resulting solution was extracted with 3x30 mL of dichloromethane and the organic layers combined and concentrated under vacuum. This resulted in 500 mg (38%) of the title compound as yellow oil.

Analytical Data: LC-MS: (ES, *m*/*z*): RT = 0.726min, LCMS07: m/z = 266 [M+1].

### Step 2: Synthesis of 3-((2-(azetidin-1-yl)ethylamino)methyl)-4-methoxybenzenamine:

Into a 100-mL round-bottom flask purged and maintained with an inert atmosphere of nitrogen, was placed Raney-Ni (100 mg), [2-(azetidin-1-yl)ethyl][(2-methoxy-5-nitrophenyl)methyl]amine (400 mg, 1.51 mmol, 1.00 equiv), methanol (50 mL). The resulting solution was stirred for 2 h at 25 degrees. The resulting solution was filtered and concentrated under vacuum. This resulted in 200 mg (56%) of the title compound as yellow oil.

Analytical Data: LC-MS: (ES, *m*/*z*): RT = 0.285min, LCMS15: m/z = 236 [M+1].

### Step 3: Synthesis of 2-N-[3-([[2-(azetidin-1-yl)ethyl]amino]methyl)-4-methoxyphenyl]-4-N-methylpyrimidine-2,4-diamine:

Into a 100-mL round-bottom flask, was placed 3-([[2-(azetidin-1-yl)ethyl]amino]methyl)-4-methoxyaniline (100 mg, 0.42 mmol, 1.00 equiv), 2-chloro-N,6-dimethylpyrimidin-4-amine (67 mg, 0.43 mmol, 1.00 equiv), trifluoroacetic acid (97 mg, 0.86 mmol, 2.02 equiv), IPA (10 mL). The resulting solution was stirred for 3 h at 80 °C. The resulting solution was extracted with 3x10 mL of dichloromethane and the organic layers combined and concentrated under vacuum. The crude product was purified by Prep-HPLC with Method C NH4HCO3. This resulted in 75.8 mg (52%) of the title compound as a light brown solid.

### Example 155: HPLC Methods for Compound Purification

**Method A. Column: IntelFlash-1, C18 silica gel; Detector, UV 254 nm**
   A. Mobile phase, H2O/ACN
   A MeOH. Mobile phase, methanol
   A Grad. (IntelFlash-1): Mobile phase, H2O/ACN=100/0 increasing to H2O/ACN=30/70 within 30 min
   A 1:1. Mobile phase, ACN/H2O=1/1
   A DCM/MeOH. Mobile phase, DCM/MeOH
   A EA/PE. Mobile phase, EA/PE
**Method B. Column, XBridge Prep C18 OBD Column, 30×100mm,5um; Detector, UV 254 nm**
   B HCl. Mobile phase, Water (0.05% HCl) and ACN (Gradient)
   B TFA. Mobile phase, Water (0.05% TFA) and ACN (Gradient)
**Method C. Column, SunFire Prep C18 OBD Column, 19x150mm 5um 10nm; Detector, UV 254/220nm**
   C HCl. Mobile phase, Water (0.05% HCl) and ACN (Gradient)
   C TFA. Mobile phase, Water (0.1% TFA) and CAN (Gradient)
   C NH3. Mobile phase, Water (0.05% NH3-H2O) and ACN (Gradient)
   C. NH4HCO3. Mobile Phase, Water with 10mmol NH4HCO3 and ACN (Gradient)
**Method D. Column, XSelect CSH Prep C18 OBD Column, 19x250mm, 5um; Detector, uv 254nm**
   D HCl. Mobile phase, Water (0.05% HCl) and ACN (Gradient);
   D TFA. Mobile phase, Water (0.06% TFA) and ACN (Gradient); Detector 254 nm.
   D NH3. Mobile phase, Water (0.05% NH3-H¬2O) and ACN (20.0% ACN up to 60.0% in 7 min); Detector, UV 220nm
   D NH4HCO3. Mobile Phase, Water with 10mmol NH4HCO3 and CAN (Gradient)
**Method E. Column: X Select C18, 19x150 mm, 5 um; Mobile Phase A: Water/0.05% HCl, Mobile Phase B: ACN; Detector 254 nm.**
**Method F. Column: X Bridge RP, 19x150 mm, 5 um; Detector 254 nm.**
   F HCl. Mobile phase Water (0.05% HCl) and ACN (Gradient)
   F TFA. Mobile phase Water (0.05% TFA) and ACN (Gradient)
**Method G. Column: GeminisoNX C18 AXAI Packed, 21.2x150mm 5um; Detector, UV 254nm.**
   G HCl Mobile phase, Water (0.05% HCl) and ACN (3.0% ACN up to 10.0% in 10 min)
   G NH4HCO3. Mobile Phase, Water with 10mmol NH4HCO3 and ACN (Gradient)
**Method H. Column: Sunfire Prep C18 OBD Column, 10um, 19x250mm; Mobile phase, Water (0.05% HCl) and methanol (3.0% methanol-up to 20.0% in 8 min); Detector, UV 254nm.**
**Method Chiral IC. Column: Chiralpak IC, 2x25cm, 5um; Mobile phase, Hex0.1%DEA- and IPA- (hold 25.0% IPA- in 21 min); Detector, UV 220/254nm.**
**Method Chiral ID. Column: Chiralpak ID-2, 2x25cm, 5um; Mobile phase, Hex(0.1%DEA)- and ethanol- (hold 50.0% ethanol- in 14 min); Detector, UV 220/254nm**
**Method Chiral IB4. Column: Chiralpak IB4.6x250,5umHPLC Chiral-A(IB)001IB00CE-LA026; Mobile phase, Hex (0.1%DEA):EtOH=50:50; Detector, 254nm**
**Method Chiral IF. Column: CHIRALPAK IF, 2x25cm,5um; Mobile phase, Hex(0.2%DEA)- and IPA- (hold 30.0% IPA- in 22 min); Detector, UV 220/254nm**

Other compounds were synthesized in the similar manner and the characterization data are listed in Tables IA and IB below.

**Table IA**

| Cpd No. | Data |
|---|---|
| 1 **(Reference)** | LC-MS: (ES, m/z): RT = 1.224 min, LCMS: m/z = 358.20 [M+1]. 1H NMR (400 MHz, Methanol-d4) δ 7.71 (s, 1H), 7.48 (s, 1H), 7.09 (d, J = 8.8 Hz, 1H), 6.89 (d, J = 8.8 Hz, 1H), 5.91 (d, J = 6.0 Hz, 1H), 4.08 (t, J = 5.6 Hz, 2H), 3.82 (s, 3H), 2.93 (s, 3H), 2.76 (t, J = 6.4 Hz, 2H), 2.67 - 2.69 (m, 4H), 2.07 - 2.02(m, 2H), 1.86 - 1.85 (m, 4H). |
| 2 **(Reference)** | LC-MS: (ES, m/z): RT = 1.035 min, LCMS: m/z = 505 [M+1]. 1H NMR (400 MHz, Methanol-d4) δ 7.70 (s, 1H), 7.35 (s, 1H), 7.14 (d, J = 2.4 Hz, 1H), 6.90 (d, J = 8.4 Hz, 1H), 6.12 - 5.84 (m, 2H), 4.10 (t, J = 6.0 Hz, 2H), 3.82 (s, 3H), 3.34 - 3.33 (m, 2H), 3.00 - 2.97 (m, 2H), 2.82 - 2.69 (m, 8H), 2.20 (t, J = 9.6 Hz, 2H), 2.09 - 2.05 (m, 2H), 1.89 - 1.86 (m, 4H), 1.78 - 1.75 (m, 2H), 1.70 - 1.60 (m, 1H), 1.35 (q, J = 3.2 Hz, 2H). |
| 3 **(Reference)** | LC-MS: (ES, m/z): RT = 1.124min; LCMS: m/z = 442 [M+1]. 1H NMR (300 MHz, Methanol-d4) δ 7.72 (s, 1H), 7.36 (s, 1H), 7.13 (d, J = 8.7Hz, 1H), 6.89 (d, J = 8.7 Hz, 1H), 5.93 (d, J = 6.0 Hz, 1H), 4.10 (t, J = 6.3 Hz, 2H), 3.97 (d, J = 1.9 Hz, 2H), 3.83 (s, 3H), 3.49 - 3.34 (m, 4H), 2.77 - 2.72 (m, 2H), 2.72 - 2.64 (m, 4H), 2.14 - 1.98 (m, 2H), 2.00 - 1.78 (m, 5H), 1.77 - 1.65 (m, 2H), 1.44 - 1.23 (m, 2H). |
| 4 **(Reference)** | LC-MS: (ES, m/z): RT = 1.367 min, LCMS: m/z = 523 [M+1]. 1H NMR (300 MHz, Methanol-d4) δ 7.68 (d, J = 6.1 Hz, 1H), 7.33 (d, J = 2.4 Hz, 1H), 7.19 - 7.15 (m, 1H), 6.90 (d, J = 8.7 Hz, 1H), 5.93 (d, J = 6.0 Hz, 1H), 4.14 (t, J = 6.3 Hz, 2H), 3.83 (s, 3H), 3.33 - 3.32 (m, 2H), 3.15 - 2.97 (m, 10H), 2.37 - 2.30 (m, 2H), 2.22 - 2.15 (m, 2H), 2.03 - 1.98 (m, 4H), 1.76 - 1.66 (m, 2H), 1.63 - 1.62 (m, 1H), 1.39 - 1.30 (m, 2H). |
| 5 **(Reference)** | LC-MS: (ES, m/z): RT = 1.115 min, LCMS: m/z = 428 [M+1]. 1H NMR (400 MHz, Methanol-d4) δ 7.71 (d, J = 6.0 Hz, 1H), 7.27 (d, J = 2.4 Hz, 1H), 7.15 (d, J = 2.4 Hz, 1H), 6.89 (d, J = 8.8 Hz, 1H), 5.92 (d, J = 6.0 Hz, 1H), 4.10 - 4.07 (m, 3H), 3.99 - 3.82 (m, 2H), 3.83 (s, 3H), 3.57 - 3.50 (m, 2H), 2.81 - 2.71 (m, 2H), 2.66 - 2.65 (m, 4H), 2.12 - 1.93 (m, 4H), 1.92 - 1.80 (m, 4H), 1.57 (m, 2H). |
| 6 **(Reference)** | LC-MS: (ES, m/z): RT = 1.321 min, LCMS: m/z = 400.3 [M-HCl+1]. 1H NMR (300 MHz, Methanol-d4) δ 7.53 (d, J = 7.3 Hz, 1H), 7.13 - 6.99 (m, 3H), 6.19 (d, J = 7.3 Hz, 1H), 4.21 (t, J = 5.5 Hz, 2H), 3.91 (s, 3H), 3.87 - 3.78 (m, 2H), 3.49 (t, J = 7.1 Hz, 2H), 3.18 (dt, J = 12.2, 7.2 Hz, 2H), 2.40 - 2.29 (m, 2H), 2.28 - 2.16 (m, 2H), 2.15 - 2.04 (m, 2H), 1.45 (s, 9H). |
| 7 **(Reference)** | LC-MS: (ES, m/z): RT = 1.013 min, LCMS: m/z = 441 [M+1]. 1H NMR (300 MHz, Deuterium Oxide) δ 7.52 (d, J = 7.2 Hz, 1H), 7.19 (s, 1H), 7.02 (d, J = 8.7 Hz, 2H), 6.13 (d, J = 6.9 Hz, 1H), 4.11 (t, J = 5.7 Hz, 2H), 3.79 (s, 3H), 3.63 - 3.62 (m, 2H), 3.35 - 3.20 (m, 6H), 3.06 - 2.97 (m, 2H), 2.81 - 2.80 (m, 2H), 2.19 - 2.04 (m, 4H), 2.01 - 1.84 (m, 5H), 1.36 - 1.18 (m, 2H). |
| 8 **(Reference)** | LC-MS: (ES, m/z): RT = 1.43 min, LCMS: m/z = 487 [M+1]. 1H NMR (300 MHz, Methanol-d4) δ 7.77 (s, 1H), 7.51 (d, J = 2.2 Hz, 1H), 7.33 - 7.17 (m, 2H), 5.99 (d, J = 6.0 Hz, 1H), 4.53 (d, J = 13.3 Hz, 1H), 4.17 (t, J = 5.8 Hz, 2H), 3.94 (d, J = 13.7 Hz, 1H), 3.19 - 2.93 (m, 7H), 2.72 - 2.57 (m, 1H), 2.19 - 2.12 (m, 2H), 2.10 (s, 3H), 2.02 - 1.76 (m, 8H), 1.34 - 1.07 (m, 2H). |
| 9 **(Reference)** | LC-MS: (ES, m/z): RT= 1.976 min, LCMS: m/z = 386 [M+1]. 1H NMR (300 MHz, Methanol-d4) δ 8.77 (s, 2H), 7.38 (d, J = 2.4 Hz, 1H), 7.18 (d, J = 6.0 Hz, 1H), 6.92 (d, J = 8.7 Hz, 1H), 4.07 (t, J = 6.3 Hz, 2H), 3.82 (s, 3H), 2.90 (s, 3H), 2.73 (d, J = 7.5 Hz, 2H), 2.63 - 2.60 (m, 4H), 2.07 - 2.02 (m, 2H), 1.86 - 1.81 (m, 4H). |
| 10 **(Reference)** | LC-MS: (ES, m/z): RT =1.509 min, LCMS: m/z = 440 [M+1]. 1H NMR (400 MHz, Methanol-d4) δ 7.57 (d, J = 6.2 Hz, 1H), 7.00 (d, J = 2.4 Hz, 1H), 6.95 - 6.92 (m, 1H), 6.85 - 6.82 (m, 1H), 6.11 (d, J = 6.4 Hz, 1H), 5.92 (s, 1H), 4.08 (t, J = 6.0 Hz, 2H), 3.84 (s, 3H), 3.24 - 3.21 (m, 2H), 3.02 (d, J = 6.4 Hz, 2H), 2.86 - 2.74 (m, 8H), 2.11 - 2.04 (m, 2H), 1.93 - 1.80 (m, 7H), 1.35 - 1.29 (m, 2H). |
| 11 **(Reference)** | LC-MS: (ES, m/z): RT = 1.776 min, LCMS: m/z = 440 [M+1]. 1H NMR (400 MHz, Methanol-d4) δ 7.58 (d, J = 7.6 Hz, 1H), 7.05 (d, J = 9.6 Hz, 1H), 6.85 - 6.83 (m, 2H), 6.38 (d, J = 5.2 Hz, 1H), 6.11 (d, J = 5.2 Hz, 1H), 4.07 (t, J = 5.6 Hz, 2H), 3.80 (s, 3H), 3.60 - 3.58 (m, 2H), 3.33 - 3.28 (m, 4H), 3.13 - 3.00 (m, 4H), 2.91 - 2.72 (m, 2H), 2.20 - 1.97 (m, 4H), 1.90 - 1.85 (m, 5H), 1.42 - 1.23 (m, 2H). |
| 12 **(Reference)** | LC-MS: RT= 0.918 min, LCMS: m/z = 442.30 [M+1]. 1H NMR (300 MHz, Deuterium Oxide, ppm) δ: 8.28 (d, J = 7.0 Hz, 1H), 7.98 (s, 1H), 7.63 (d, J = 2.5 Hz, 1H), 7.45 (dd, J = 7.0, 2.5 Hz, 1H), 7.00 (s, 1H), 4.46 (s, 2H), 4.28 (t, J = 5.6 Hz, 2H), 4.02 (dd, J = 11.8, 4.6 Hz, 2H), 3.90 (s, 3H), 3.72-3.51 (m, 3H), 3.50-3.30 (m, 4H), 3.10-2.97 (m, 2H), 2.24-2.22 (m, 2H), 2.10-2.05 (m, 4H), 1.95-1.91 (m, 2H), 1.72-1.67 (m, 2H). |
| 13 **(Reference)** | LC-MS: RT= 1.19 min, LCMS: m/z = 496.30 [M+1]. 1H-NMR (400 MHz, Chloroform-d, ppm) δ: 7.88 (d, J = 5.7 Hz, 1H), 6.90 - 6.79 (m, 2H), 6.75 (dd, J = 8.5, 2.4 Hz, 1H), 6.11 (dd, J = 5.7, 1.9 Hz, 1H), 5.94 - 5.74 (m, 2H), 4.61 (d, J = 13.5 Hz, 1H), 4.07 (t, J = 6.7 Hz, 2H), 3.88 (s, 3H), 3.82 - 3.79 (m, 1H), 3.49 (dd, J = 14.2, 7.4 Hz, 1H), 3.35 (dd, J = 14.2, 7.0 Hz, 1H), 2.98 (s, 4H), 2.65 (t, J = 7.3 Hz, 2H), 2.60 - 2.39 (m, 5H), 2.09 (s, 5H), 2.03 - 2.01 (m, 1H), 1.84 - 1.60 (m, 6H), 1.21 - 1.15 (m, 2H). |
| 14 **(Reference)** | LC-MS: (ES, m/z): RT = 2.434 min, LCMS: m/z = 484 [M+1]. 1H NMR (300 MHz, DMSO-d6) δ 9.29 (s, 1H), 8.14 (d, J = 5.6 Hz, 1H), 7.49 (d, J = 2.4 Hz, 1H), 7.17 (d, J = 2.4 Hz, 1H), 6.85 (d, J = 8.8 Hz, 1H), 6.20 (d, J = 5.6 Hz, 1H), 4.43- 4.38 (m, 1H), 4.20 (d, J = 6.6 Hz, 2H), 3.98 (t, J = 6.3 Hz, 2H), 3.86 - 3.73 (m, 1H), 3.71 (s, 3H), 3.07 - 2.99 (m, 1H), 2.56 - 2.52 (m, 2H), 2.50 - 2.42 (m, 5H), 1.98 (s, 4H), 1.88 - 1.83 (m, 2H), 1.79 - 1.66 (m, 6H), 1.29 - 1.06 (m, 2H). |
| 15 **(Reference)** | LC-MS: (ES, m/z): RT = 0.957 min, LCMS: m/z = 457 [M+1]. 1H NMR (300 MHz, Chloroform-d) δ 7.90 (d, J = 5.7 Hz, 1H), 7.43 (d, J = 2.5 Hz, 1H), 7.03 - 6.99 (m, 1H), 6.81 (d, J = 8.7 Hz, 2H), 5.80 (d, J = 5.7 Hz, 1H), 4.92 (br s, 1H), 4.18 - 4.13 (m, 1H), 4.12 - 4.01 (t, J = 1.5 Hz, 2H), 3.83 (s, 3H), 3.22 - 3.21 (m, 2H), 3.14 - 3.10 (m, 2H), 2.82 - 2.54 (m, 10H), 1.79 - 1.67 (m, 7H), 1.30 - 1.16 (m, 2H). |
| 16 **(Reference)** | LC-MS: (ES, m/z): RT = 1.423 min, LCMS: m/z = 457 [M+1]. 1H NMR (400 MHz, Methanol-d4) δ 7.70 (s, 1H), 7.33 (d, J = 2.4 Hz, 1H), 7.14 (d, J = 2.4 Hz, 1H), 6.89 (d, J = 8.7 Hz, 1H), 5.92 (d, J = 6.0 Hz, 1H), 4.10 (t, J = 6.0 Hz, 2H), 3.82 (s, 3H), 3.72 (d, J = 4.4 Hz, 4H), 3.32 - 3.35 (m, 2H), 3.16 - 3.09 (m, 2H), 2.68 - 2.49 (m, 8H), 2.02 - 1.99 (m, 2H), 1.85 - 1.77 (m, 3H), 1.24 - 1.12 (m, 2H). |
| 17 **(Reference)** | LC-MS: (ES, m/z): RT = 2.234 min, LCMS: m/z = 439 [M+1]. 1H NMR (400 MHz, Methanol-d4) δ 7.70 (s, 1H), 7.44 (d, J = 1.2 Hz, 1H), 7.37 (d, J = 2.4 Hz, 1H), 6.90 (d, J = 8.8 Hz, 1H), 5.91 (d, J = 6.0 Hz, 1H), 3.84 (s, 3H), 3.30 -3.28 (m, 2H), 3.13 - 3.10 (m, 2H), 2.92 - 2.87 (m, 4H), 2.66 - 2.60 (m, 4H), 2.07 - 2.05 (m, 1H), 1.85 - 1.79 (m,5H), 1.79 - 1.57 (m, 4H), 1.26 - 1.15 (m, 4H). |
| 18 **(Reference)** | LC-MS: (ES, m/z): RT = 1.13 min, LCMS: m/z = 469 [M+1]. 1H NMR (300 MHz, Methanol-d4) δ 7.70 (s, 1H), 7.27 (d, J = 2.5 Hz, 1H), 7.15 (dd, J = 8.7, 2.5 Hz, 1H), 6.88 (d, J = 8.7 Hz, 1H), 5.91 (d, J = 6.0 Hz, 1H), 4.48 (d, J = 13.3 Hz, 1H), 4.02 - 3.85 (m, 3H), 3.81 (s, 3H), 3.16 - 2.99 (m, 1H), 2.85-2.46 (m, 7H), 2.40 (s, 3H), 2.21 - 1.58 (m, 9H), 1.32 - 1.00 (m, 2H). |
| 19 **(Reference)** | LC-MS: (ES, m/z): RT = 2.264 min, LCMS: m/z = 497 [M+1]. 1H NMR (400 MHz, Methanol-d4) δ 7.34 (s, 1H), 7.05 (d, J = 2.1 Hz, 1H), 6.80 (d, J = 8.7 Hz, 1H), 6.67 (s, 1H), 5.69 (s, 1H), 4.76 (s, 1H), 4.67 - 4.62 (m, 1H), 4.11 (t, J = 6.6 Hz, 2H), 3.81- 3.97 (m, 4H), 3.24 (s, 2H), 3.06 - 2.97 (m, 1H), 2.69 (s, 2H), 2.69 - 2.45 (m, 4H), 2.24 (s, 3H), 2.13 - 2.03 (m, 5H), 1.81 - 1.63 (m, 7H), 1.20- 1.88 (m, 2H). |
| 20 **(Reference)** | 1H NMR (300 MHz, Methanol-d4) δ 7.74 - 7.65 (m, 1H), 7.44 (s, 1H), 7.09 (d, J = 8.6 Hz, 1H), 6.94 - 6.84 (m, 1H), 5.93 - 5.84 (m, 1H), 4.23 (d, J = 9.8 Hz, 1H), 4.08 (d, J = 6.8 Hz, 2H), 3.82 (s, 3H), 2.77 (t, J = 7.7 Hz, 2H), 2.66 - 2.64 (m, 4H), 2.14 - 1.94 (m, 2H), 1.91 - 1.81 (m, 4H), 1.39 - 1.02 (m, 6H). |
| 21 **(Reference)** | LC-MS: (ES, m/z): RT = 0.801 min, LCMS: m/z = 434 [M+H]. 1H NMR (400 MHz, Methanol-d4) δ 7.62 (d, J = 7.2 Hz, 1H), 7.42 - 7.27 (m, 5H), 7.17 (s, 1H), 7.10 - 6.96 (m, 2H), 6.24 (d, J = 7.3 Hz, 1H), 4.69 (s, 2H), 4.03 (t, J = 5.5 Hz, 2H), 3.90 (s, 3H), 3.82 - 3.77 (m, 2H), 3.41 (t, J = 7.2 Hz, 2H), 3.17 - 3.10 (m, 2H), 2.24 - 2.17 (m, 4H), 2.10 - 2.05 (m, 2H). |
| 22 **(Reference)** | LC-MS: (ES, m/z): RT = 1.160 min, LCMS: m/z = 416 [M+H]. 1H NMR (400 MHz, Methanol-d4) δ 7.72 (s, 1H), 7.40 (s, 1H), 7.11 (dd, J = 8.7, 2.5 Hz, 1H), 6.86 (d, J = 8.7 Hz, 1H), 5.90 (d, J = 6.0 Hz, 1H), 4.04 (t, J = 6.2 Hz, 2H), 3.80 (s, 3H), 3.46 (t, J = 6.1 Hz, 4H), 3.32 (s, 3H), 2.72 - 2.63 (m, 2H), 2.62 - 2.52 (m, 4H), 2.07 - 1.95 (m, 2H), 1.91 - 1.75 (m, 6H). |
| 23 **(Reference)** | LC-MS: (ES, m/z): RT = 0.643 min, LCMS: m/z = 435 [M+1]. 1H NMR (300 MHz, Chloroform-d) δ 8.59 (d, J = 1.5 Hz, 1H), 7.92 (d, J = 6.0 Hz, 1H), 7.70 - 7.64 (m, 1H), 7.38 (d, J = 2.4 Hz, 1H), 7.29 (d, J = 7.8 Hz, 1H), 7.23 - 7.19 (m, 1H), 7.01 - 6.97 (m, 1H), 6.82 (d, J = 8.7 Hz, 1H), 6.75 (br s, 1H), 5.93 (d, J = 5.7 Hz, 2H), 4.70 (d, J = 4.8 Hz, 2H), 4.11 (t, J = 6.6 Hz, 2H), 3.84 (s, 3H), 2.69 - 2.66 (m, 2H), 2.58-2.54 (m, 4H), 2.16 - 2.02 (m, 2H), 1.80 - 1.76 (m, 4H). |
| 24 **(Reference)** | LC-MS: (ES, m/z): RT = 1.016 min, LCMS: m/z = 435 [M+1]. 1H NMR (300 MHz, Methanol-d4) δ 8.52 - 8.43 (m, 2H), 7.78 (d, J = 6.0 Hz, 1 H), 7.42 - 7.39 (m, 2H), 7.23 (d, J = 3.0 Hz, 1H), 6.96 (d, J = 8.9 Hz, 1H), 6.80 (d, J = 8.7 Hz, 1H), 6.02 (d, J = 5.7 Hz, 1H), 4.68 (s, 2H), 3.95 (t, J = 6.0 Hz, 2H), 3.80 (s, 3H), 2.70 - 2.65 (m, 6H), 2.04 - 1.94 (m, 2H), 1.91 - 1.88 (m, 4H). |
| 25 **(Reference)** | LC-MS: (ES, m/z): RT = 0.528 min, LCMS: m/z = 435 [M+1]. 1H NMR (400 MHz, Methanol-d4) δ 8.55 (d, J = 2.1 Hz, 1H), 8.43 (d, J = 1.6 Hz, 1H), 7.83 (d, J = 8.0 Hz, 1H), 7.76 (d, J = 6.0 Hz, 1H), 7.42 - 7.39 (m, 1H), 7.31 (d, J = 2.4 Hz, 1H), 7.03 (d, J = 2.4 Hz, 1H), 6.85 (d, J = 8.8 Hz, 1H), 5.99 (d, J = 6.0 Hz, 1H), 4.67 (s, 2H), 3.99 (t, J = 6.1 Hz, 2H), 3.83 (s, 3H), 2.79 - 2.72 (m, 6H), 2.04 - 1.99 (m, 2H), 1.94 - 1.82 (m, 4H). |
| 26 **(Reference)** | LC-MS: (ES, m/z): RT = 1.301 min, LCMS: m/z = 464 [M+1]. 1H NMR (400 MHz, Methanol-d4) δ 7.75 (d, J = 6.0 Hz, 1H), 7.50 (s, 1H), 7.27-7.23 (t, J = 8.1 Hz, 1H), 6.95 - 6.93 (m, 3H), 6.86 - 6.81 (m, 2H), 5.97 (d, J = 4.0 Hz, 1H), 4.62 (s, 2H), 3.92 (s, 2H), 3.80 (s, 3H), 3.76 (s, 3H), 2.61 (m, 6H), 1.95 (q, J = 7.2 Hz, 2H), 1.87 - 1.80 (m, 4H). |
| 27 **(Reference)** | LC-MS: (ES, m/z): RT = 1.674 min, LCMS: m/z = 402 [M+1]. 1H NMR (400 MHz, Chloroform-d) δ 7.92 (d, J = 5.6 Hz, 1H), 7.35 (d, J = 1.6 Hz, 1H), 7.03 - 7.01 (m, 1H), 6.84 (d, J = 8.8 Hz, 1H), 6.74 (s, 1H), 5.85 (d, J = 5.6 Hz, 1H), 5.06 (br s, 1H), 4.12 (t, J = 6.6 Hz, 2H), 3.86 (s, 3H), 3.58 (s, 4H), 3.41 (s, 3H), 2.67 (t, J = 7.6 Hz, 2H), 2.55 (br s, 4H), 2.14 - 2.07 (m, 2H), 1.81 - 1.77 (m, 4H). |
| 28 **(Reference)** | LC-MS: (ES, m/z): RT = 0.799 min, LCMS: m/z = 464 [M+1]. 1H NMR (400 MHz, Methanol-d4) δ 7.73 (d, J = 6.0 Hz, 1H), 7.56 (br s, 1H), 7.29 (d, J = 8.8 Hz, 2H), 6.96 - 6.85 (m, 4H), 5.95 (d, J = 6.0 Hz, 1H), 4.58 (s, 2H), 3.92 (d, J = 6.4 Hz, 2H), 3.80 (d, J = 6.4 Hz, 6H), 2.60 - 2.58 (m, 6H), 1.94 (q, J = 7.6 Hz, 2H), 1.85 - 1.82 (m, 4H). |
| 29 **(Reference)** | LC-MS: (ES, m/z): RT = 0.991 min, LCMS: m/z = 483 [M+1]. 1H NMR (400 MHz, Methanol-d4) δ 7.61 (d, J = 5.2 Hz, 1H), 7.16 (d, J = 6.8 Hz, 1H), 7.07 (d, J = 8.4 Hz, 2H), 6.19 (d, J = 7.2 Hz, 1H), 4.19 (t, J = 5.6 Hz, 2H), 3.90 (s, 3H), 3.51 - 3.46 (m, 9H), 3.41 - 3.32 (m, 2H), 3.06 - 2.99 (m, 2H), 2.31 - 2.25 (m, 2H), 2.15 (s, 4H), 2.10 - 1.95 (m, 3H), 1.62 (q, J = 7.6 Hz, 2H), 1.36 (d, J = 6.6 Hz, 6H). |
| 30 **(Reference)** | LC-MS: (ES, m/z): RT = 1.999 min, LCMS: m/z = 438 [M+1]. 1H NMR (300 MHz, Chloroform-d) δ 7.93 (d, J = 1.5 Hz, 1H), 7.61 (s, 1H), 7.45 - 7.32 (m, 2H), 7.02 (d, J = 2.5 Hz, 1H), 6.83 (d, J = 8.4 Hz, 1H), 6.74 (br s, 1H), 5.83 (d, J = 5.7 Hz, 1H), 4.86 (br s, 1H), 4.42 (d, J = 5.1 Hz, 2H), 4.07 (t, J = 6.6 Hz, 2H), 3.86 (s, 3H), 3.84 (s, 3H), 2.63 - 2.61 (m, 2H), 2.58 - 2.52 (m, 4H), 2.11 - 2.02 (m, 2H), 1.82 - 1.73 (m, 4H). |
| 31 **(Reference)** | LC-MS: (ES, m/z): RT = 1.492 min, LCMS: m/z = 474 [M+1]. 1H NMR (400 MHz, Methanol-d4) δ 7.82 (d, J = 6.0 Hz, 1H), 7.55 - 7.52 (m, 2H), 7.28 (s, 1H), 7.25 - 7.21 (m, 2H), 6.89 (d, J = 8.8 Hz, 1H), 6.68 (d, J = 8.4Hz, 1H), 6.07 (d, J = 6.0 Hz, 1H), 4.87 (s, 2H), 3.83 (d, J = 5.6 Hz, 2H), 3.75 (s, 3H), 2.60 - 2.56 (m, 6H), 1.87 - 1.82 (m, 6H). |
| 32 **(Reference)** | LC-MS: (ES, m/z): RT = 1.175 min, LCMS: m/z = 455 [M+1]. 1H NMR (300 MHz, Chloroform-d) δ 7.88 (d, J = 5.7 Hz, 1H), 7.31 (d, J = 2.1 Hz, 1H), 7.05 - 6.91 (m, 2H), 6.82 (d, J = 9.0 Hz, 1H), 5.83 (d, J = 6.0 Hz, 1H), 5.02 (br s, 1H), 4.11 (t, J = 3.0 Hz, 2H), 3.83 (s, 3H), 3.26 - 3.24 (m, 2H), 2.99 - 2.91 (m, 8H), 2.36 (s, 3H), 2.24 - 2.19 (m, 2H), 2.17 - 2.01 (m, 2H), 1.90 - 1.94 (m, 4H), 1.80 - 1.76 (m, 2H), 1.65 - 1.49 (m, 1H), 1.49 - 1.25 (m, 2H). |
| | |
| 33 **(Reference)** | LC-MS: (ES, m/z): RT = 5.231 min, LCMS: m/z = 517 [M+1]. 1H NMR (400 MHz, Methanol-d4) δ 7.70 (s, 1H), 7.38 (s, 1H), 7.25 - 7.21 (m, 2H), 7.12 (d, J = 8.8 Hz, 1H), 7.00 (d, J = 8.0 Hz, 2H), 6.90 - 6.82 (m, 2H), 5.94 (d, J = 6.0 Hz, 1H), 4.11 (t, J = 6.0 Hz, 2H), 3.82 (s, 3H), 3.71 - 3.68 (m, 2H), 3.43 - 3.35 (m, 2H), 2.85 - 2.81 (m, 2H), 2.73 - 2.66 (m, 6H), 2.11 (q, J = 6.0 Hz, 2H), 1.91 - 1.87 (m, 6H), 1.81 - 1.80 (m, 1H), 1.48 - 1.41 (m, 2H). |
| 34 **(Reference)** | LC-MS: (ES, m/z): RT = 1.313 min, LCMS: m/z = 426.15 [M+1]. 1H NMR (300 MHz, Methanol-d4) δ 7.74 - 7.65 (m, 1H), 7.43 (s, 1H), 7.09 (dd, J = 8.7, 2.5 Hz, 1H), 6.88 (d, J = 8.7 Hz, 1H), 5.92 (d, J = 6.1 Hz, 1H), 4.08 (t, J = 6.1 Hz, 2H), 3.82 (s, 3H), 3.34 - 3.30 (m, 2H), 2.78 - 2.56 (m, 6H), 2.30 - 2.16 (m, 1H), 2.13 - 1.97 (m, 2H), 1.93 - 1.53 (m, 10H), 1.36 - 1.22 (m, 2H). |
| 35 **(Reference)** | LC-MS: (ES, m/z): RT = 1.203 min, LCMS: m/z = 398.1 [M+1]. 1H NMR (300 MHz, Methanol-d4) δ 7.71 (d, J = 5.8 Hz, 1H), 7.44 (s, 1H), 7.07 (dd, J = 8.6, 2.5 Hz, 1H), 6.95 - 6.84 (m, 1H), 5.95 - 5.80 (m, 1H), 4.50 (br s, 1H), 4.12 (t, J = 6.1 Hz, 2H), 3.82 (s, 3H), 2.75 - 2.64 (m, 6H), 2.42 - 2.34 (m, 2H), 2.10 - 1.94 (m, 5H), 1.90 - 1.81 (m, 5H). |
| 36 **(Reference)** | LC-MS: (ES, m/z): RT = 0.843 min, LCMS: m/z = 464 [M+1]. 1H NMR (300 MHz, Methanol-d4) δ 7.72 (d, J = 6.0 Hz, 1H), 7.48 (s, 1H), 7.27 - 7.22 (m, 2H), 7.00 - 6.81 (m, 4H), 5.96 (d, J = 6.0 Hz, 1H), 4.60 (s, 2H), 3.96 (t, J = 5.7 Hz, 2H), 3.87 (s, 3H), 3.80 (s, 3H), 2.76 - 2.70 (m, 6H), 2.02 - 1.95 (m, 2H), 1.93 - 1.83 (m, 4H). |
| 37 **(Reference)** | LC-MS: (ES, m/z): RT = 1.015 min, LCMS: m/z = 449 [M+1]. 1H NMR (300 MHz, Methanol-d4) δ 8.45 - 8.35 (m, 2H), 7.70 (t, J = 6.8 Hz, 2H), 7.41 - 7.30 (m, 2H), 7.11 (dd, J = 8.7, 2.5 Hz, 1H), 6.89 (d, J = 8.7 Hz, 1H), 5.89 (d, J = 6.0 Hz, 1H), 4.04 (t, J = 6.1 Hz, 2H), 3.83 (s, 3H), 3.66 (t, J = 7.2 Hz, 2H), 2.96 (t, J = 7.2 Hz, 2H), 2.79 - 2.66 (m, 6H), 2.16 - 1.78 (m, 6H). |
| 38 **(Reference)** | LC-MS: (ES, m/z): RT = 1.008 min, LCMS: m/z = 429.10 [M-HCl+1]. 1H NMR (300 MHz, Methanol-d4) δ 7.84 - 7.70 (m, 1H), 7.20 (s, 1H), 7.16 - 7.02 (m, 2H), 6.24 (d, J = 7.2 Hz, 1H), 4.25 (t, J = 5.4 Hz, 2H), 3.94 (s, 3H), 3.90 - 3.83 (m, 2H), 3.64 (dd, J = 6.6, 5.0 Hz, 2H), 3.53 (t, J = 7.0 Hz, 2H), 3.45 (dd, J = 6.6, 5.0 Hz, 2H), 3.26 - 3.08 (m, 2H), 2.41 - 2.19 (m, 4H), 2.19 - 2.05 (m, 2H), 1.90 (s, 3H). |
| 39 **(Reference)** | LC-MS: (ES, m/z): RT = 1.005 min, LCMS: m/z = 449 [M-HCl+1]. 1H NMR (400 MHz, Deuterium Oxide) δ 8.36 (d, J = 5.7 Hz, 2H), 7.61 (d, J = 5.9 Hz, 2H), 7.42 (d, J = 7.3 Hz, 1H), 7.11 - 6.87 (m, 3H), 6.04 (d, J = 7.3 Hz, 1H), 4.05 (t, J = 5.7 Hz, 2H), 3.82 (s, 3H), 3.74 - 3.58 (m, 4H), 3.31 (t, J = 7.5 Hz, 2H), 3.14 - 2.88 (m, 4H), 2.22 - 1.81 (m, 6H). |
| 40 **(Reference)** | LC-MS: (ES, m/z): RT = 1.421 min, LCMS: m/z = 464.3 [M-HCl+1]. 1H NMR (400 MHz, Methanol-d4) δ 7.65 - 7.60 (m, 1H), 7.29 (t, J = 7.7 Hz, 2H), 7.24 - 7.20 (m, 1H), 7.08 - 7.01 (m, 2H), 6.97 - 6.94 (m, 3H), 6.26 (d, J = 6.8 Hz, 1H), 4.19 - 4.16 (m, 4H), 3.89 - 3.80 (m, 5H), 3.80 (t, J = 8.9 Hz, 2H), 3.44 (d, J = 6.6 Hz, 2H), 3.17 - 3.07 (m, 2H), 2.22 - 2.01 (m, 4H), 2.07 (d, J = 5.9 Hz, 2H). |
| 41 **(Reference)** | LC-MS: (ES, m/z): RT = 1.414 min, LCMS: m/z = 415 [M+1]. 1H NMR (300 MHz, Chloroform-d) δ 7.93 (d, J = 5.7 Hz, 1H), 7.40 (s, 1H), 6.92 - 6.89 (m, 1H), 6.88 - 6.76 (m, 2H), 6.57 (br s, 1H), 5.91 (d, J = 6.0 Hz, 1H), 5.41 (br s, 1H), 4.15 (t, J = 6.4 Hz, 2H), 4.08 (d, J = 5.2 Hz, 2H), 3.84 (s, 3H), 2.80 - 2.67 (m, 9H), 2.19 (q, J = 6.9 Hz, 2H), 1.86 - 1.84 (m, 4H). |
| 42 **(Reference)** | LC-MS: (ES, m/z): RT = 1.67 min, LCMS: m/z = 416.25 [M-HCl+1]. 1H NMR (300 MHz, Deuterium Oxide) δ 7.64 - 7.40 (m, 1H), 7.09 - 6.93 (m, 3H), 6.14 (d, J = 7.3 Hz, 1H), 4.10 (t, J = 5.6 Hz, 2H), 3.82 (s, 3H), 3.73 - 3.57 (m, 2H), 3.46 - 3.26 (m, 4H), 3.13 - 2.95 (m, 2H), 2.24 - 2.02 (m, 4H), 2.02 - 1.82 (m, 2H), 1.17 (s, 6H). |
| 43 **(Reference)** | LC-MS: (ES, m/z): RT = 1.105 min, LCMS: m/z = 479 [M+1]. 1H NMR (400 MHz, Methanol-d4) δ 7.76 (d, J = 6.0 Hz, 1H), 7.24 (d, J = 2.0 Hz, 1H), 7.19 (d, J = 6.4 Hz, 1H), 6.92 (d, J = 8.8 Hz, 1H), 5.95 (d, J = 5.6 Hz, 1H), 4.07 (t, J = 6.0 Hz, 2H), 3.84 - 3.81 (m, 5H), 3.34 - 3.29 (m, 2H), 2.81 (s, 6H), 2.72 (t, J = 6.0 Hz, 2H), 2.61 (d, J = 6.4 Hz, 4H), 2.08 - 2.01 (m, 2H), 1.85 - 1.83 (m, 4H). |
| 44 **(Reference)** | LC-MS: (ES, m/z): RT = 1.063 min, LCMS: m/z = 457.15 [M-HCl+1]. |
| | 1H NMR (300 MHz, Methanol-d4) δ 7.62 (t, J = 6.8 Hz, 1H), 7.20 - 7.05 (m, 3H), 6.26 - 6.16 (m, 1H), 4.21 (q, J = 5.5 Hz, 2H), 3.94 - 3.81 (m, 5H), 3.51 - 3.42 (m, 6H), 3.22 - 3.12 (m, 2H), 3.05 (s, 2H), 2.89 (s, 1H), 2.34 - 2.20 (m, 4H), 2.15 - 2.01 (m, 5H), 1.95 - 1.84 (m, 2H). |
| 45 **(Reference)** | LC-MS: (ES, m/z): RT = 2.140 min, LCMS: m/z = 456.3 [M+1]. 1H NMR (300 MHz, Methanol-d4) δ 7.72 (d, J = 6.0 Hz, 1H), 7.33 (d, J = 2.5 Hz, 1H), 7.08 - 7.01 (m, 1H), 6.91 (d, J = 8.7 Hz, 1H), 5.99 (d, J = 6.0 Hz, 1H), 4.11 (t, J = 6.1 Hz, 2H), 3.83 (s, 3H), 3.47 (br s, 2H), 2.76 (t, J = 7.8 Hz, 2H), 2.67 - 2.65 (m, 4H), 2.11 - 2.00 (m, 2H), 1.89 - 1.79 (m, 4H), 1.68 - 1.45 (m, 9H), 1.39 - 1.21 (m, 1H). |
| 46 **(Reference)** | LC-MS: (ES, m/z): RT = 1.146 min, LCMS: m/z = 424.3 [M+1]. 1H NMR (400 MHz, Methanol-d4) δ 7.75 (d, J = 6.0 Hz, 1H), 7.60 - 7.54 (m, 2H), 7.00 (d, J = 8.6, 1H), 6.88 (d, J = 8.8 Hz, 1H), 6.29 (d, J = 2.2 Hz, 1H), 5.98 (d, J = 6.0, 1H), 4.66 (s, 2H), 3.98 (t, J = 5.6 Hz, 2H), 3.81 (s, 3H), 2.78 - 2.59 (m, 6H), 2.01 - 1.86 (m, 6H). |
| 47 **(Reference)** | LC-MS: (ES, m/z): RT = 1.135 min, LCMS: m/z = 483 [M+1]. 1H NMR (300 MHz, Methanol-d4) δ 7.90 (d, J = 5.7 Hz, 1H), 7.25 (d, J = 5.4 Hz, 1H), 7.05 (d, J = 8.4 Hz, 1H), 6.81 (d, J = 8.4 Hz, 1H), 6.68 (s, 1H), 5.82 (d, J = 5.7 Hz, 1H), 4.85 (br s, 1H), 4.67 - 4.62 (m, 1H), 4.11 (t, J = 6.6 Hz, 2H), 3.83 - 3.80 (m, 4H), 3.26 - 3.25 (m, 2H), 3.07 - 2.98 (m, 1H), 2.72 - 2.50 (m, 7H), 2.17 - 2.02 (m, 5H), 1.83 - 1.76 (m, 7H), 1.24 - 1.12 (m, 2H). |
| 48 **(Reference)** | LC-MS: (ES, m/z): RT = 1.203 min, LCMS: m/z = 483 [M+1]. 1H NMR (400 MHz, Methanol-d4) δ 7.69 (s, 1H), 7.58 (d, J = 2.4 Hz, 1H), 7.53 - 7.50 (m, 1H), 6.91 (d, J = 8.8 Hz, 1H), 5.91 (d, J = 6.0 Hz, 1H), 4.57 (s, 2H), 4.49 - 4.48 (m, 1H), 3.92 - 3.89 (m, 1H), 3.83 (s, 3H), 3.70 (t, J = 5.6 Hz, 2H), 3.45 - 3.30 (m, 2H), 3.12 - 3.06 (m, 1H), 2.88 - 2.85 (m, 2H), 2.79 - 2.72 (m, 4H), 2.64 - 2.62 (m, 1H), 2.09 (s, 3H), 1.89 - 1.95 (m, 1H), 1.92 - 1.77 (m, 6H), 1.25 - 1.12 (m, 2H). |
| 49 **(Reference)** | LC-MS: (ES, m/z): RT = 1.815 min, LCMS: m/z = 458.2 [M-HCl+1]. |
| | 1H NMR (300 MHz, Methanol-d4) δ 7.62 (d, J = 7.0 Hz, 1H), 7.18 - 7.06 (m, 3H), 6.30 (d, J = 6.9 Hz, 1H), 4.27 - 4.18 (m, 2H), 3.91 (s, 3H), 3.85 - 3.66 (m, 6H), 3.58 (s, 2H), 3.49 (d, J = 6.7 Hz, 2H), 3.19 (d, J = 8.9 Hz, 2H), 2.38 - 2.15(m, 4H), 2.13 - 2.05 (m, 2H), 1.72 - 1.62 (m, 2H), 1.54 - 1.48 (m, 2H). |
| 50 **(Reference)** | LC-MS: (ES, m/z): RT = 1.314 min, LCMS: m/z = 426.20 [M+1]. 1H NMR (300 MHz, Methanol-d4) δ 7.66 (d, J = 6.1 Hz, 1H), 7.30 (d, J = 2.5 Hz, 1H), 7.14 (dd, J = 8.7, 2.5 Hz, 1H), 6.88 (d, J = 8.7 Hz, 1H), 5.87 (d, J = 6.1 Hz, 1H), 4.10 (t, J = 6.1 Hz, 2H), 3.91 - 3.82 (m, 4H), 2.81 (t, J = 7.7 Hz, 2H), 7.73 - 7.71 (m, 4H), 2.14 - 1.96 (m, 4H), 1.93 - 1.75 (m, 6H), 1.69 - 1.59 (m, 1H), 1.51 - 1.16 (m, 5H). |
| 51 **(Reference)** | LC-MS: (ES, m/z): RT = 1.020 min, LCMS: m/z = 471 [M+1]. 1H NMR (400 MHz, Methanol-d4) δ 7.77 (d, J = 5.6 Hz, 1H), 7.18 (s, 1H), 7.14 - 7.11 (m, 1H), 6.91 (d, J = 8.8 Hz, 1H), 6.04 (d, J = 6.0 Hz, 1H), 4.26 (s, 2H), 4.10 (t, J = 6.0 Hz, 2H), 3.85 (s, 3H), 3.84 - 3.67 (m, 4H), 3.59 - 3.53 (m, 4H), 2.95 - 2.91 (m, 2H), 2.86 - 2.76 (m, 4H), 2.14 - 2.07 (m, 2H), 1.96 - 1.93 (m, 4H). |
| 52 **(Reference)** | LC-MS: (ES, m/z): RT = 1.220 min, LCMS: m/z = 491 [M+1]. 1H NMR (400 MHz, Chloroform-d) δ 7.86 (d, J = 5.6 Hz, 1H), 7.51 - 7.42 (m, 3H), 7.26 - 7.24 (m, 2H), 7.08 - 7.05 (m, 1H), 6.93 (br s, 1H), 6.69 - 6.65 (m, 2H), 5.84 (br s, 1H), 5.72 (t, J = 4.4 Hz, 1H), 4.02 (t, J = 6.8 Hz, 2H), 3.85 (s, 5H), 3.33 (s, 3H), 2.64 (t, J = 7.6 Hz, 2H), 2.52 - 2.50 (m, 4H), 2.09 - 2.02 (m, 2H), 1.81 - 1.77 (m, 4H). |
| 53 **(Reference)** | LC-MS: (ES, m/z): RT = 1.189 min, LCMS: m/z = 436.3 [M-HCl+1]. 1H NMR (300 MHz, Deuterium Oxide) δ 7.66 (dd, J = 7.4, 2.8 Hz, 1H), 7.49 (d, J = 10.5 Hz, 1H), 7.26 - 6.87 (m, 3H), 6.23 (d, J = 7.5 Hz, 1H), 4.53 (d, J = 7.3 Hz, 4H), 4.09 - 3.98 (m, 2H), 3.78 - 3.58 (m, 5H), 3.38 - 3.28 (m, 2H), 3.12 - 2.96 (m, 2H), 2.21 - 1.89 (m, 6H). |
| 54 **(Reference)** | LC-MS: (ES, m/z): RT = 1.12 min, LCMS: m/z = 469 [M+1]. 1H NMR (300 MHz, Methanol-d4) δ 7.71 (d, J = 6.0 Hz, 1H), 7.25 (d, J = 2.5 Hz, 1H), 7.15 (dd, J = 8.7, 2.5 Hz, 1H), 6.89 (d, J = 8.7 Hz, 1H), 5.91 (d, J = 6.0 Hz, 1H), 4.51 - 4.42 (m, 1H), 4.10 - 4.06 (m, 3H), 3.98 -3.84 - 3.82 (m, 1H), 3.81 (s, 3H), 3.29 - 3.20 (m, 1H), 2.97 - 2.84 (m, 1H), 2.81 - 2.54 (m, 6H), 2.19 - 1.97 (m, 7H), 1.88 - 1.72 (m, 4H), 1.54 - 1.32 (m, 2H). |
| 55 **(Reference)** | LC-MS: (ES, m/z): RT = 1.152 min, LCMS: m/z = 497 [M+1]. 1H NMR (400 MHz, Methanol-d4) δ 7.56 (s, 1H), 7.26 (d, J = 2.8 Hz, 1H), 7.19 (d, J = 2.4 Hz, 1H), 6.87 (d, J = 8.4Hz, 1H), 4.49 - 4.46 (m, 1H), 4.07 (t, J = 6.0 Hz, 2H), 3.90 - 3.88 (m, 1H), 3.81 (s, 3H), 3.46 - 3.35 (m, 2H), 3.06 (t, J = 5.6 Hz, 1H), 2.72 (t, J = 5.6 Hz, 2H), 2.65 - 2.62 (m, 5H), 2.09 (s, 3H), 2.07 - 2.00 (m, 3H), 1.97 (s, 3H), 1.84 - 1.77 (m, 6H), 1.23 - 1.11 (m, 2H). |
| 56**(Reference)** | LC-MS: (ES, m/z): RT = 1.34 min, LCMS: m/z = 449 [M+1]. 1H NMR (400 MHz, Methanol-d4) δ 8.50 (d, J = 1.6 Hz, 2H), 7.94 (d, J = 6.1 Hz, 1H), 7.35 - 7.28 (m, 2H), 7.26 (br s, 1H), 7.00 (d, J = 8.8 Hz, 1H), 6.84 (d, J = 8.7 Hz, 1H), 6.18 (d, J = 6.1 Hz, 1H), 4.93 - 4.90 (m, 2H), 3.99 - 3.95 (m, 2H), 3.82 (s, 3H), 3.25 - 3.11 (m, 9H), 2.15 - 2.02 (m, 6H). |
| 57 **(Reference)** | LC-MS: (ES, m/z): RT = 1.18 min, LCMS: m/z = 487.3 [M+1]. 1H NMR (300 MHz, Chloroform-d) δ 7.90 (d, J = 5.9 Hz, 1H), 7.54 (dd, J = 8.8, 2.7 Hz, 1H), 7.45 (d, J = 2.7 Hz, 1H), 6.98 (br s, 1H), 6.81 (d, J = 8.8 Hz, 1H), 5.81 (d, J = 5.9 Hz, 1H), 5.21 - 4.91 (m , 2H), 4.68 - 4.61 (m, 1H), 4.55 (s, 2H), 3.90 - 3.62 (m, 6H), 3.55 (t, J = 5.5 Hz, 2H), 3.29 - 3.13 (m, 4H), 3.11 - 2.95 (m, 1H), 2.74 (t, J = 5.4 Hz, 2H), 2.54 - 2.41 (m, 1H), 2.09 (s, 3H), 1.85 - 1.75 (m, 3H), 1.19 - 1.15 (m, 2H). |
| 102 **(Reference)** | LC-MS: (ES, m/z): RT = 1.88 min, LCMS 07: m/z = 414 [M+1]. 1H NMR (300 MHz, Chloroform-d) δ 8.00 (d, J = 6.0 Hz, 1H), 7.29 (d, J = 2.4 Hz, 1H), 6.96 - 6.91 (m, 1H), 6.82 (d, J = 8.7 Hz, 1H), 6.75 (s, 1H), 5.96 (d, J = 6.0 Hz, 1H), 4.07 (t, J = 6.6 Hz, 2H), 3.86 (s, 3H), 3.78 - 3.70 (m, 4H), 3.61 - 3.44 (m, 4H), 3.66 - 3.64 (m, 2H), 3.54 - 3.62 (m, 4H), 2.12 - 2.05 (m, 2H), 1.78 - 2.03 (m, 4H). |
| 103 **(Reference)** | LC-MS: (ES, m/z): RT = 1.245 min, LCMS28: m/z = 510.35 [M+1]. 1H NMR (300 MHz, Chloroform-d) δ 7.57 (d, J = 2.4 Hz, 1H), 7.27 - 7.26 (m, 1H), 6.98 - 6.92 (m, 1H), 6.80 (d, J = 8.7 Hz, 1H), 6.63 (s, 1H), 4.84 (s, 1H), 4.54 (s, 1H), 4.12 (t, J = 6.6 Hz, 2H), 3.83 (s, 3H), 3.52 - 3.42 (m, 4H), 3.26 - 3.10 (m, 4H), 2.85 - 2.60 (m, 8H), 2.24 - 2.17 (m, 2H), 2.01 - 1.93 (m, 4H), 1.94 - 1.57 (m, 7H), 1.39 - 1.27 (m, 2H). |
| 104 **(Reference)** | LC-MS: (ES, m/z): RT=1.410 min; LCMS15: m/z = 441 [M+1]. 1H NMR (300 MHz, Methanol-d4) δ 8.32 (d, J = 5.1 Hz, 1H), 7.39 (d, J = 2.4 Hz, 1H), 7.29 - 7.16 (m, 1H), 6.93 (d, J = 8.7 Hz, 1H), 6.78 (d, J = 5.1 Hz, 1H), 4.09 (t, J = 6.3 Hz, 2H), 3.87 (s, 3H), 3.82 (s, 2H), 3.24 - 3.20 (m, 2H), 2.90 - 2.71 (m, 9H), 2.14 - 2.02 (m, 4H), 1.97 - 1.87 (m, 4H), 1.54 - 1.38 (m, 2H). |
| 105 **(Reference)** | LC-MS: (ES, m/z): RT = 0.645 min, LCMS48: m/z = 410.3 [M+1]. 1H NMR (400 MHz, Methanol-d4) δ 7.78 - 7.70 (m, 2H), 7.59 (d, J = 7.2 Hz, 1H), 6.96 (d, J = 8.0 Hz, 1H), 6.10 (d, J = 7.2 Hz, 1H), 3.72 - 3.69 (m, 2H), 3.64 (t, J = 5.8 Hz, 2H), 3.50 - 3.42 (m, 3H), 3.42 - 3.40 (m, 1H), 340 - 3.30 (m, 2H), 3.03 - 2.97 (m, 2H), 2.05 - 1.94 (m, 3H), 1.56 - 1.47 (m, 2H). |
| 106 **(Reference)** | LC-MS: (ES, m/z): RT = 1.477 min; LCMS 15: m/z = 483 [M+1]. 1H NMR (400 MHz, Methanol-d4) δ 8.34 (s, 1H), 7.25 (s, 1H), 7.17 - 7.15 (m, 3H), 4.70 (s, 3H), 4.25 (t, J = 5.6 Hz, 2H), 4.13 - 4.12 (m, 1H), 3.93 (s, 3H), 3.85 - 3.80 (m, 2H), 3.65 (s, 1H), 3.49(t, J = 7.2 Hz, 2H), 3.34 - 3.14 (m, 4H), 2.80 - 2.65 (m, 1H), 2.34 - 2.26 (m, 3H), 2.24 - 2.18 (m, 2H), 2.07 (s, 3H), 2.12 - 2.07 (m, 2H), 1.76 (s, 1H), 1.60 (s, 1H). |
| 107 **(Reference)** | LC-MS: (ES, m/z): RT = 1.712 min, LCMS 07: m/z = 442 [M+1]. 1H NMR (400 MHz, Methanol-d4) δ 8.31 (d, J = 5.2 Hz, 1H), 7.40 (d, J = 2.4 Hz, 1H), 7.18 (d, J = 2.4 Hz, 1H), 6.93 (d, J = 8.8 Hz, 1H), 6.77 (d, J = 5.2 Hz, 1H), 4.11 (t, J = 6.0 Hz, 2H), 3.98 - 3.95 (m, 2H), 3.83 (d, J = 9.8 Hz, 5H), 3.44 (t, J = 2.0 Hz, 2H), 2.93 - 2.84 (m, 2H), 2.80 - 2.75 (m, 5H), 2.17 - 2.05 (m, 2H), 1.98 - 1.85 (m, 6H), 1.51 - 1.42 (m, 2H). |
| 108 **(Reference)** | LC-MS: (ES, m/z): RT = 1.45 min, LCMS 33: m/z = 441 [M+1]. 1H NMR (400 MHz, Methanol-d4) δ 8.22 (s, 1H), 7.09 (d, J = 8.6 Hz, 1H), 7.03 (s, 1H), 6.98 (s, 1H), 5.90 (s, 1H), 4.22 (t, J = 5.6 Hz, 2H), 3.91 (s, 3H), 3.82 (dd, J = 10.9, 5.1 Hz, 3H), 3.53 - 3.40 (m, 5H), 3.18 (dd, J = 12.4, 6.5 Hz, 2H), 3.02 (t, J = 12.7 Hz, 2H), 2.38 - 2.16 (m, 4H), 2.17 - 1.94 (m, 5H), 1.52 (t, J = 12.7 Hz, 2H). |
| 109 **(Reference)** | LC-MS: (ES, m/z): RT = 5.062 min, LCMS33: m/z = 440 [M+1]. 1H NMR (300 MHz, Chloroform-d) δ 7.92 (d, J = 5.7 Hz, 1H), 7.32 - 7.20 (m, 1H), 7.05 - 7.02 (m, 1H), 6.82 (d, J = 8.7 Hz, 1H), 6.70 (s, 1H), 5.82 (d, J = 5.8 Hz, 1H), 4.77 (d, J = 6.3 Hz, 1H), 4.02 (t, J = 6.9 Hz, 2H), 3.85 (s, 3H), 3.24 - 3.17 (m, 2H), 3.17 - 3.13 (m, 2H), 2.66 - 2.58 (m, 2H), 2.00 - 1.37 (m, 15H), 1.39 - 1.05 (m, 4H). |
| 110 **(Reference)** | LC-MS: (ES, m/z): RT = 2.24 min; m/z = 442.10 [M+1]. 1H NMR (300 MHz, CD3OD) δ: 8.13 (d, J = 7.0 Hz, 1H), 7.77 (s, 1H), 7.45 (s, 1H), 7.34 (s, 1H), 6.90 (d, J = 6.0 Hz, 1H), 4.17 (t, J = 6.0 Hz, 2H), 4.01 - 3.92 (m, 2H), 3.86 (s, 3H), 3.79 (s, 2H), 3.47 - 3.35 (m, 2H), 2.78 - 2.60 (m, 7H), 2.18 - 2.02 (m, 2H), 1.92 - 1.81 (m, 6H), 1.51-1.38 (m, 2H). |
| 111 **(Reference)** | LC-MS: (ES, m/z): RT = 1.401 min, LCMS 07: m/z = 455 [M+1]. 1H NMR (400 MHz, Methanol-d4) δ 8.30 (d, J = 5.2 Hz, 1H), 7.38 (d, J = 2.4 Hz, 1H), 7.17 (d, J = 2.4 Hz, 1H), 6.93 (d, J = 8.8 Hz, 1H), 6.77 (d, J = 5.2 Hz, 1H), 4.10 (t, J = 6.0 Hz, 2H), 3.84 (s, 3H), 3.80 (s, 2H), 2.90 - 2.87 (m, 2H), 2.84 - 2.79 (m, 2H), 2.73 (s, 4H), 2.60 - 2.52 (m, 1H), 2.28 (s, 3H), 2.10 - 2.05 (m, 4H), 1.98 - 1.92 (m, 2H), 1.88 (s, 4H), 1.53 - 1.40 (m, 2H). |
| 112 **(Reference)** | LC-MS: (ES, m/z): RT = 1.401 min, LCMS 07: m/z = 455 [M+1]. 1H NMR (400 MHz, Methanol-d4) δ 8.31 (d, J = 5.2 Hz, 1H), 7.40 (d, J = 2.4 Hz, 1H), 7.18 (d, J = 2.4 Hz, 1H), 6.95 - 6.85 (m, 2H), 4.10 (t, J = 6.0 Hz, 2H), 4.02 - 3.98 (m, 2H), 3.83 (s, 3H), 3.64 (s, 2H), 3.42 - 3.36 (m, 2H), 2.80 - 2.71 (m, 2H), 2.70 - 2.68 (m, 5H), 2.35 (s, 3H), 2.09 - 2.05 (m, 2H), 1.88 - 1.83 (m, 6H), 1.64 - 1.60 (m, 2H). |
| 113 **(Reference)** | LC-MS: (ES, m/z): RT = 1.70 min, LCMS 15: m/z = 440 [M+1]. 1H NMR (300 MHz, Methanol-d4) δ 7.22 - 7.17 (m, 2H), 7.09 - 7.06 (m, 1H), 6.87 (d, J = 8.7 Hz, 1H), 5.96 (d, J = 7.8 Hz, 1H), 5.87 (d, J = 8.1 Hz, 1H), 4.09 (t, J = 6.0 Hz, 2H),, 3.81 (s, 3H), 3.56- 3.08 (m, 4H), 2.85 - 2.74 (m, 8H), 2.11 - 2.02 (m, 2H), 2.07 - 2.02 (m, 7H), 1.97- 1.89 (m, 2H). |
| 114 **(Reference)** | LC-MS: (ES, m/z): RT = 0.944 min, LCMS27: m/z = 466.1 [M+1]. 1H NMR (300 MHz, Methanol-d4) δ 8.00 (s, 1H), 7.66 (s, 1H), 7.38 - 7.25 (m, 3H), 6.15 (d, J = 6.6 Hz, 1H), 4.87 - 4.42 (m, 1H), 3.91 - 3.86 (m, 1H), 3.65 - 3.38 (m, 7H), 3.14 - 2.82 (m, 4H), 2.70 - 2.56 (m, 1H), 2.13 - 2.08 (m, 7H), 2.03 - 1.84 (m, 1H), 1.88 - 1.71 (m, 2H), 1.25 - 1.11 (m, 2H). |
| 115 **(Reference)** | LC-MS: (ES, m/z): RT = 1.23 min, LCMS 15: m/z = 500 [M+1]. 1H NMR (300 MHz, DMSO-d6) δ 10.68 (s, 1H), 9.95 (s, 1H), 8.91 (s, 1H), 8.26 (s, 1H), 7.84 - 7.78 (m, 2H), 7.78 - 7.76 (m, 1H), 7.41 (s, 1H), 7.20 - 7.13 (m, 3H), 6.91 (d, J = 8.7 Hz, 1H), 5.95 (d, J = 6.0 Hz,1H), 4.30 - 4.26 (m, 1H), 3.72- 3.68 (m, 1H), 3.58 (s, 2H), 3.25 (s, 2H), 2.91 - 2.72 (m, 1H), 2.51 - 2.45 (m, 1H), 1.92 (s, 3H), 1.78 - 1.63 (m, 3H), 1.08 -0.99 (m, 2H). |
| 116 **(Reference)** | LC-MS: (ES, m/z): RT = 1.238 min, LCMS 28: m/z = 531 [M+1]. 1H-NMR: (400 MHz, Methanol-d4) δ 7.66 - 7.57 (m, 1H), 7.43 - 7.23 (m, 5H), 7.11 (s, 3H), 6.31- 6.22 (m, 1H), 4.25 - 4.17 (m, 1H), 4.17 - 4.13 (m, 1H), 3.97 - 3.85 (m, 4H), 3.85 - 3.72 (m, 1H), 3.72-3.60 (m, 1H), 3.53 - 3.42 (m, 4H), 3.42 - 3.34 (m, 3H), 3.06 - 2.95 (m, 3H), 2.40 - 2.33 (m, 2H), 2.21 - 2.10 (m, 3H), 2.07 - 1.86 (m, 4H), 1.57 - 1.42 (m, 2H). |
| 117 **(Reference)** | LC-MS: (ES, m/z): RT = 1.725 min, LCMS 15: m/z = 501 [M+1]. 1H NMR (400 MHz, Methanol-d4) δ 7.70 (s, 1H), 7.32 (d, J = 2.4 Hz, 1H), 7.12 (d, J = 2.4 Hz, 1H), 6.88 (d, J = 8.8 Hz, 1H), 5.92 (d, J = 6.0 Hz, 1H), 5.30 - 5.20 (m, 1H), 4.51 - 4.48 (m, 1H), 4.08 (t, J = 6.0 Hz, 2H), 3.93 - 3.90 (m, 1H), 3.82 (s, 3H), 3.32 - 3.28 (m, 2H), 3.08 - 2.94 (m, 3H), 2.77 - 2.63 (m, 4H), 2.48 - 2.46 (m, 1H), 2.31 - 2.12 (m, 1H), 2.10 (s, 3H), 2.09 - 1.93 (m, 4H), 1.86 - 1.78 (m, 2H), 1.25 - 1.15 (m, 2H). |
| 118 **(Reference)** | LC-MS: (ES, m/z): RT= 1.111 min, LCMS 15: m/z = 501 [M+1]. 1H NMR (400 MHz, Methanol-d4) δ 7.69 (s, 1H), 7.31 (d, J = 2.4 Hz, 1H), 7.12 (d, J = 2.4 Hz, 1H), 6.87 (d, J = 8.7 Hz, 1H), 5.91 (d, J = 6.0 Hz, 1H), 5.32 - 5.09 (m, 1H), 4.50 - 4.46 (m, 1H), 4.07 (t, J = 6.3 Hz, 2H), 3.92 - 3.88 (m, 1H), 3.81 (s, 3H), 3.32 - 3.22 (m, 2H), 3.07 - 2.92 (m, 3H), 2.76 - 2.62 (m, 4H), 2.47 - 2.44 (m, 1H), 2.32 - 2.13 (m, 1H), 2.08 - 1.92 (m, 7H), 1.85 - 1.76 (m, 2H), 1.19 - 1.14 (m, 2H). |
| 119 **(Reference)** | LC-MS: (ES, m/z): RT = 1.357 min, LCMS31: m/z = 497.4 [M+1]. 1H NMR (400 MHz, Methanol-d4) δ 7.70 (s, 1H), 7.31 (d, J = 2.4 Hz, 1H), 7.14 - 7.12 (m, 1H), 6.89 (d, J = 8.8 Hz, 1H), 5.93 (d, J = 6.0 Hz, 1H), 4.52 - 4.48 (m, 1H), 4.05 (t, J = 6.0 Hz, 2H), 3.94 - 3.91 (m, 1H), 3.83 (s, 3H), 3.56 - 3.50 (m, 4H), 3.36 - 3.33 (m, 2H), 3.15 - 3.03 (m, 1H), 2.67 - 2.61 (m, 1H), 2.38 (t, J = 8.0 Hz, 2H), 2.12 - 1.99 (m, 7H), 1.96 - 1.94 (m, 1H), 1.90 - 1.76 (m, 2H), 1.33 - 1.07 (m, 2H). |
| 120 **(Reference)** | LC-MS: (ES, m/z): RT = 0.82 min, LCMS 15: m/z = 469 [M+1]. 1H NMR (300 MHz, Chloroform-d) δ 7.90 (d, J = 5.8 Hz, 1H), 7.26 -7.05 (m, 1H), 6.91 - 6.74 (m, 2H), 5.80 (d, J = 6.0 Hz, 1H), 4.79 (s, 1H), 4.67 (d, J = 1.8 Hz, 1H), 4.06 - 4.04 (m, 1H), 3.99 - 3.78 (m, 5H), 3.28 (s, 2H), 3.19 - 2.96 (m, 2H), 2.85 - 2.65 (m, 1H), 2.63 - 2.50 (m, 3H), 2.32 - 2.30 (m, 2H), 2.14 - 2.08 (m, 5H), 1.81 - 1.76 (m, 5H), 1.12 - 1.13 (m, 2H). |
| 121 **(Reference)** | LC-MS: (ES, m/z): RT = 1.22 min, LCMS 07: m/z = 496 [M+1]. 1H NMR (400 MHz, Methanol-d4) δ 8.47 (d, J = 2.6 Hz, 1H), 7.68 (s, 1H), 7.26 (dd, J = 8.8, 2.6 Hz, 1H), 6.93 (d, J = 8.8 Hz, 1H), 5.91 (d, J = 6.1 Hz, 1H), 4.44 (d, J = 13.3 Hz, 1H), 3.88 (s, 4H), 3.35 (s, 2H), 3.06 (td, J = 13.4, 13.0, 2.8 Hz, 1H), 2.88 (t, J = 6.6 Hz, 2H), 2.74 - 2.58 (m, 7H), 2.08 (s, 3H), 1.97 - 1.71 (m, 7H), 1.29 - 1.07 (m, 2H). |
| 122 **(Reference)** | LC-MS: (ES, m/z): RT = 1.554 min, LCMS 28: m/z = 476.2 [M+1]. 1H NMR (300 MHz, Methanol-d4) δ 8.27 (s, 1H), 7.75 (s, 1H), 7.40 - 7.16 (m, 3H), 6.49 (s, 1H), 5.97 (d, J = 6.0 Hz, 1H), 4.46 (d, J = 12.9 Hz, 1H), 3.87 (d, J = 13.5 Hz, 1H), 3.34 - 3.33 (m, 2H), 3.11 - 2.96 (m, 1H), 2.68 - 2.55 (m, 1H), 2.22 (t, J = 8.4, 1H), 2.08 (s, 3H), 1.99 - 1.70 (m, 3H), 1.31 - 1.07 (m, 4H), 1.07 - 0.97 (m, 2H). |
| 123 **(Reference)** | LC-MS: (ES, m/z): RT = 3.287 min, LCMS 27: m/z = 476.1 [M+1]. 1H NMR (300 MHz, Methanol-d4) δ 8.51 (s, 1H), 8.26 (s, 1H), 7.80 - 7.70 (m, 1H), 7.36 - 7.20 (m, 3H), 5.97 (d, J = 6.0 Hz, 1H), 4.42 (d, J = 13.2 Hz, 1H), 4.12 - 3.97 (m, 1H), 3.87 (d, J = 13.7 Hz, 1H), 3.50 - 3.36 (m, 2H), 3.14 - 2.97 (m, 1H), 2.60 (t, J = 12.1 Hz, 1H), 2.06 (s, 3H), 2.01- 1.70 (m, 3H), 1.42 - 1.02 (m, 6H). |
| 124 **(Reference)** | LC-MS: (ES, m/z): RT = 1.277 min, LCMS 33: m/z = 398.3 [M+1]. 1H NMR (300 MHz, Methanol-d4) δ 7.81 (d, J = 6.1 Hz, 1H), 7.55 (d, J = 2.5 Hz, 1H), 7.10 (dd, J = 8.7, 2.5 Hz, 1H), 6.89 (d, J = 8.7 Hz, 1H), 5.93 (d, J = 6.1 Hz, 1H), 4.09 (t, J = 6.2 Hz, 2H), 3.82 (s, 3H), 3.70 - 3.40 (m, 4H), 2.82 - 2.57 (m, 6H), 2.14 - 1.80 (m, 10H). |
| 125 **(Reference)** | LC-MS: (ES, m/z): RT = 4.209 min,UFLC05, LCMS 48: m/z = 412.3 [M+1]. 1H NMR (300 MHz, Methanol-d4) δ 7.82 (d, J = 6.3 Hz, 1H), 7.40 (d, J = 2.4 Hz, 1H), 7.00 (dd, J = 8.7, 2.4 Hz, 1H), 6.88 (d, J = 8.7 Hz, 1H), 6.15 (d, J = 6.3 Hz, 1H), 4.07 (t, J = 6.1 Hz, 2H), 3.81 (s, 3H), 3.72 - 3.62 (m, 4H), 2.83 - 2.63 (m, 6H), 2.14 - 1.55 (m, 12H). |
| 126 **(Reference)** | LC-MS: (ES, m/z): RT = 1.028 min, LCMS 28: m/z = 443 [M+1]. 1H-NMR: (300 MHz, Deuterium Oxide) δ 6.97 - 6.88 (m, 1H), 6.80 (d, J = 2.4 Hz, 1H), 6.75-6.67 (m, 1H), 6.03 (s, 1H), 4.32 (s, 2H), 4.07 (t, J = 5.7 Hz, 2H), 3.73 (s, 3H), 3.70 - 3.44 (m, 8H), 3.32 (t, J = 7.5 Hz, 2H), 3.12 - 2.90 (m, 4H), 2.45 - 2.29 (m, 2H), 2.19 - 1.99 (m, 4H), 1.99 - 1.74 (m, 4H). |
| 127 **(Reference)** | LC-MS: (ES, m/z): RT = 1.102 min, LCMS 28: m/z = 444 [M+1]. 1H-NMR: (400 MHz, Methanol-d4) δ 6.94 (d, J = 2.4 Hz, 1H), 6.84 (d, J = 8.8 Hz, 1H), 6.75 - 6.70 (m, 1H), 5.88 (s, 1H), 4.08 (t, J = 6.0 Hz, 2H), 3.99 - 3.92 (m, 2H), 3.80 (d, J = 6.4 Hz, 5H), 3.74 (s, 3H), 3.48 - 3.38 (m, 2H), 2.88 (t, J = 7.6 Hz, 2H), 2.82 - 2.73 (m, 5H), 2.14 - 2.04 (m, 2H), 1.95 - 2.82 (m, 6H), 1.52 - 1.39 (m, 2H). |
| 128 **(Reference)** | LC-MS: (ES, m/z): RT = 1.08 min; LCMS 27: m/z = 450.30 [M+1]. 1H NMR (300 MHz, DMSO-d6) δ 12.54 (s, 1H), 8.87 (s, 1H), 7.96 (d, J = 6.0 Hz, 1H), 7.56 (d, J = 2.5 Hz, 1H), 7.12 (d, J = 8.5 Hz, 1H), 6.86 (d, J = 8.7 Hz, 1H), 6.30 (d, J = 6.1 Hz, 1H), 4.65 (s, 2H), 4.13 - 3.88 (m, 4H), 3.72 (s, 3H), 2.76 (d, J = 5.9 Hz, 2H), 2.56 (d, J = 7.0 Hz, 2H), 2.49 - 2.38 (m, 6H), 1.92 (t, J = 6.8 Hz, 2H), 1.72 - 1.61 (m, 4H). |
| 129 **(Reference)** | LC-MS: (ES, m/z): RT = 1.15 min; LCMS 33: m/z = 423.24 [M+1]. 1H NMR (300 MHz, Methanol-d4) δ 8.30 (d, J = 5.3 Hz, 1H), 7.33 (d, J = 2.5 Hz, 1H), 7.16 (dd, J = 8.7, 2.5 Hz, 1H), 6.95 - 6.78 (m, 2H), 4.04 (t, J = 6.2 Hz, 2H), 3.81 (s, 3H), 2.74 - 2.51 (m, 6H), 2.45 (s, 6H), 2.09 - 1.93 (m, 2H), 1.89 - 1.73 (m, 4H). |
| 130 **(Reference)** | LC-MS: (ES, m/z): RT = 8.151min; m/z = 424.24 [M+1]. 1H NMR (300 MHz, Methanol-d4) δ 8.41 (d, J = 5.2 Hz, 1H), 7.33 (d, J = 2.5 Hz, 1H), 7.19 (dd, J = 8.7, 2.5 Hz, 1H), 6.98 - 6.85 (m, 2H), 4.07 (t, J = 6.2 Hz, 2H), 3.83 (s, 3H), 2.78 - 2.57 (m, 9H), 2.46 (s, 3H), 2.15 - 1.96 (m, 2H), 1.93 - 1.76 (m, 4H). |
| 131 **(Reference)** | LC-MS: (ES, m/z): RT = 1.14 min; LCMS 33: m/z = 409.20 [M+1]. 1H NMR (300 MHz, Methanol-d4) δ 8.26 (d, J = 5.3 Hz, 1H), 8.09 (s, 1H), 7.34 (d, J = 2.5 Hz, 1H), 7.18 (dd, J = 8.7, 2.5 Hz, 1H), 7.00 - 6.87 (m, 2H), 4.07 (t, J = 6.2 Hz, 2H), 3.82 (s, 3H), 2.78 - 2.56 (m, 9H), 2.10 - 1.90 (m, 2H), 1.91 - 1.75 (m, 4H). |
| 132 **(Reference)** | LC-MS: (ES, m/z): RT = 1.725 min, LCMS07: m/z = 441.1 [M+1]. 1H NMR (400 MHz, Methanol-d4) δ 7.95 - 7.93 (m, 1H), 7.36 (d, J = 2.0 Hz, 1H), 7.07 - 7.05 (m, 1H), 6.95 - 6.87 (m, 1H), 6.25 - 6.16 (m, 1H), 4.31 - 4.24 (m, 2H), 4.15 - 4.05 (m, 2H), 3.96 - 3.80 (m, 5H), 3.57 - 3.48 (m, 2H), 3.04 (s, 3H), 2.89 - 2.39 (m, 6H), 2.15 - 2.07 (m, 2H), 1.91 - 1.80 (m, 4H). |
| 133 **(Reference)** | LC-MS: (ES, m/z): RT = 2.345 min, LCMS27: m/z = 441.1 [M+1]. 1H NMR (300 MHz, Methanol-d4) δ 7.91 (d, J = 6.3 Hz, 1H), 7.31 (d, J = 2.4 Hz, 1H), 7.12 - 7.08 (m, 1H), 6.90 (d, J = 8.6 Hz, 1H), 6.22 (d, J = 6.3 Hz, 1H), 4.33 (s, 2H), 4.08 (t, J = 6.3 Hz, 2H), 4.00 - 3.90 (m, 2H), 3.81 (s, 3H), 3.38 - 3.31 (m, 2H), 2.76 - 2.68 (m, 2H), 2.65 - 2.60 (m, 4H), 2.12 - 1.96 (m, 2H), 1.94 - 1.76 (m, 6H). |
| 134 **(Reference)** | LC-MS: (ES, m/z): RT = 1.133 min, LCMS 28: m/z = 478.3 [M+1]. 1H NMR (300 MHz, Deuterium Oxide) δ8.24 - 8.16 (m, 1H), 7.67 (d, J = 7.6 Hz, 1H), 7.16 - 6.92 (m, 3H), 6.60 - 6.46 (m, 1H), 4.65 (s, 1H), 4.48 (s, 1H), 4.18 - 4.04 (m, 3H), 3.95 - 3.80 (m, 4H), 3.73 - 3.65 (m, 2H), 3.38 (t, J = 7.4 Hz, 2H), 3.73 - 3.65 (m, 2H), 3.73 - 3.65 (m, 3H), 2.27 - 1.84 (m, 6H). |
| 135 **(Reference)** | LC-MS: (ES, m/z): RT = 0.992 min, LCMS 33: m/z = 449.6 [M+1]. 1H NMR (400 MHz, Methanol-d4) δ 8.53 - 8.43 (m, 2H), 7.92 (d, J = 6.1 Hz, 1H), 7.77 - 7.74 (m, 1H), 7.44 - 7.40 (m, 1H), 7.33 (d, J = 2.5 Hz, 1H), 7.00 (dd, J = 8.7, 2.5 Hz, 1H), 6.83 (d, J = 8.6 Hz, 1H), 6.16 (d, J = 6.2 Hz, 1H), 4.94 (s, 2H), 3.91 (t, J = 6.1 Hz, 2H), 3.79 (s, 3H), 3.10 (s, 3H), 2.69 - 2.57 (m, 6H), 2.00 - 1.78 (m, 6H). |
| 136 **(Reference)** | LC-MS: (ES, m/z): RT = 1.07 min, LCMS 33: m/z = 449.6 [M+1]. 1H NMR (300 MHz, Methanol-d4) δ8.55 - 8.52 (m, 1H), 7.90 (d, J = 6.1 Hz, 1H), 7.82 - 7.76 (m, 1H), 7.39 - 7.21 (m, 3H), 7.02 - 6.91 (m, 1H), 6.80 (d, J = 8.7 Hz, 1H), 6.15 (d, J = 6.2 Hz, 1H), 4.97 (s, 2H), 3.91 (t, J = 6.0 Hz, 2H), 3.79 (s, 3H), 3.16 (s, 3H), 2.73 - 2.57 (m, 6H), 2.03 - 1.79 (m, 6H). |
| 137 **(Reference)** | LC-MS: (ES, m/z): RT = 1.071 min, LCMS 33: m/z = 400 [M+1]. 1H NMR (300 MHz, Methanol-d4) δ 7.99 (d, J = 6.4 Hz, 1H), 7.25 (s, 1H), 7.11 (q, J = 8.8 Hz, 2H), 6.54 - 6.23 (m, 1H), 4.64 (s, 2H), 4.43 (s, 1H), 4.25 - 4.16 (m, 2H), 3.89 - 3.58 (d, 7H), 3.47 (d, J = 6.6 Hz, 2H), 3.15 (d, J = 11.1 Hz, 2H), 2.33 - 2.05 (m, 6H). |
| 138 **(Reference)** | LC-MS: (ES, m/z): RT = 0.676min, LCMS 30: m/z = 469.2 [M+1]. 1H NMR (300 MHz, Methanol-d4) δ 7.75 (d, J = 13.6 Hz, 1H), 7.13 - 6.98 (m, 3H), 6.56 (dd, J = 14.4, 7.6 Hz, 1H), 4.30-4.20 (m, 2H), 4.13 - 3.94 (m, 2H), 3.91 (s, 3H), 3.88 - 3.73 (m, 6H), 3.71 - 3.57 (m, 2H), 3.55-3.45 (m, 2H), 3.22 - 3.12 (m, 2H), 2.30 (s, 2H), 2.27-2.15 (m, 2H), 2.16 - 2.02 (m, 5H), 2.00-1.82 (m, 2H). |
| 139 **(Reference)** | LC-MS: (ES, m/z): RT = 1.04min, LCMS07: m/z = 455.15 [M+1]. 1H NMR (400 MHz, Methanol-d4) δ 7.92 (d, J = 6.2 Hz, 1H), 7.33 (d, J = 2.5 Hz, 1H), 7.07 (dd, J = 8.7, 2.4 Hz, 1H), 6.92 (d, J = 8.7 Hz, 1H), 6.22 (d, J = 6.2 Hz, 1H), 4.09 (t, J = 6.1 Hz, 2H), 3.83 (s, 3H), 3.81 - 3.73 (m, 2H), 3.73 - 3.62 (m, 6H), 2.83 (t, J = 7.7 Hz, 2H), 2.75 (s, 4H), 2.17 (s, 3H), 2.10 (q, J = 6.9 Hz, 2H), 1.93 - 1.85 (m, 4H). |
| 140 **(Reference)** | LC-MS: (ES, m/z): RT = 1.09 min, LCMS 33: m/z = 416 [M+1]. 1H NMR (400 MHz, DMSO-d6) δ 8.77 (s, 1H), 7.88 (d, J = 6.0 Hz, 1H), 7.50 (s, 1H), 7.25 (d, J = 8.6 Hz, 1H), 6.84 (d, J = 8.8 Hz, 1H), 6.06 (d, J = 6.1 Hz, 1H), 4.54 (s, 1H), 3.98 (t, J = 6.3 Hz, 2H), 3.71 (s, 3H), 3.45 (t, J = 6.2 Hz, 2H), 3.33 (s, 2H), 3.04 (s, 3H), 2.90-2.60 (m, 6H), 1.99 (q, J = 7.0 Hz, 2H), 1.78 (s, 4H), 1.76 - 1.64 (m, 2H). |
| 141 **(Reference)** | LC-MS: (ES, m/z): RT = 1.15mm, LCMS33: m/z = 442 [M+1]. 1H NMR (400 MHz, Methanol-d4) δ 7.69 (d, J = 7.6 Hz, 1H), 7.16 - 7.00 (m, 3H), 6.60 (d, J = 7.7 Hz, 1H), 4.63 (d, J = 13.3 Hz, 1H), 4.19 (t, J = 5.5 Hz, 2H), 3.92 - 3.81 (m, 6H), 3.59 (m, 1H), 3.49 (t, J = 7.0 Hz, 3H), 3.16 (s, 2H), 2.31-2.11 (m, 4H), 2.11-2.07 (m, 2H), 1.77 - 1.61 (m, 4H), 1.29 (s, 3H). |
| 142 **(Reference)** | LC-MS: (ES, m/z): RT = 1.874 min, LCMS 07: m/z = 442.10 [M+1]. 1H NMR (300 MHz, Methanol-d4) δ 7.80 - 7.69 (m, 1H), 7.31 - 7.01 (m, 3H), 6.71 - 6.47 (m, 1H), 5.20 - 4.99 (m, 1H), 4.28 - 4.14 (m, 2H), 4.06 - 3.98 (m, 2H), 3.91 (s, 3H), 3.85 - 3.71 (m, 2H), 3.62 - 3.51 (m, 1H), 3.55 - 3.36 (m, 3H), 3.21 - 3.09 (m, 5H), 2.34 - 1.85 (m, 8H), 1.81 - 1.68 (m, 2H). |
| 143 **(Reference)** | LC-MS: (ES, m/z): RT = 1.255 min, LCMS 28: m/z = 430.3 [M+1]. 1H NMR (300 MHz, Methanol-d4) δ 7.84 (d, J = 6.2 Hz, 1H), 7.36 (d, J = 2.4 Hz, 1H), 7.15 (dd, J = 8.7, 2.5 Hz, 1H), 6.91 (d, J = 8.7 Hz, 1H), 6.10 (d, J = 6.3 Hz, 1H), 4.11 (t, J = 6.1 Hz, 2H), 3.84 (s, 3H), 3.66 (t, J = 7.1 Hz, 2H), 3.46 - 3.35 (m, 5H), 3.11 (s, 3H), 2.85 - 2.66 (m, 6H), 2.16 - 2.04 (m, 2H), 1.94 - 1.84 (m, 6H). |
| 144 **(Reference)** | LC-MS: (ES, m/z): RT = 1.07 min, LCMS 53: m/z = 400 [M+1]. 1H NMR (300 MHz, Chloroform-d) δ 8.31 (d, J = 5.1 Hz, 1H), 7.32 (d, J = 3.9 Hz, 1H), 7.04 - 7.01 (m, 1H), 6.95 - 6.91 (m, 1H), 6.85 (d, J = 8.7 Hz, 1H), 6.63 (d, J = 5.1 Hz, 2H), 4.41 (d, J = 4.8 Hz, 2H), 4.12 (t, J = 6.6 Hz, 2H), 3.88 (s, 3H), 2.72 - 2.54 (m, 6H), 2.18 - 2.13 (m, 2H), 2.11 (s, 3H), 2.01-1.82(m,4H). |
| 145 **(Reference)** | LC-MS: (ES, m/z): RT = 2.06 min, LCMS 33: m/z = 467 [M+1]. 1H NMR (400 MHz, Methanol-d4) δ 7.60 (d, J = 7.3 Hz, 1H), 7.23 (d, J = 8.2 Hz, 1H), 7.03 (d, J = 7.1 Hz, 2H), 6.21 (d, J = 7.3 Hz, 1H), 4.47 (d, J = 13.3 Hz, 1H), 4.16 (t, J = 5.8 Hz, 2H), 3.91 (d, J = 13.8 Hz, 1H), 3.80-3.70 (m, 2H), 3.51 - 3.39 (m, 4H), 3.23 - 3.05 (m, 3H), 2.65 (t, J = 11.8 Hz, 1H), 2.31 (t, J = 7.6 Hz, 2H), 2.26 (s, 3H), 2.22-2.15 (m, 2H), 2.14 - 1.88 (m, 6H), 1.79-1.70 (m, 2H), 1.34 - 1.06 (m, 2H). |
| 146 **(Reference)** | LC-MS: (ES, m/z): RT = 1.212 min, LCMS 33: m/z = 456 [M+1]. 1H NMR (300 MHz, Methanol-d4) δ 7.84 (d, J = 6.2 Hz, 1H), 7.29 (s, 1H), 7.16 (dd, J = 8.7, 2.5 Hz, 1H), 6.89 (d, J = 8.7 Hz, 1H), 6.08 (d, J = 6.2 Hz, 1H), 4.09 (t, J = 6.2 Hz, 2H), 4.00 - 3.89 (m, 2H), 3.82 (s, 3H), 3.58 - 3.35 (m, 4H), 3.11 (s, 3H), 2.80 - 2.60 (m, 6H), 2.14 - 1.98 (m, 3H), 1.92 - 1.78 (m, 4H), 1.62 - 1.52 (m, 2H), 1.42 - 1.26 (m, 2H). |
| 147 **(Reference)** | LC-MS: (ES, m/z): RT = 1.271 min, LCMS 28: m/z = 470.3 [M+1]. 1H NMR (300 MHz, Methanol-d4) δ 7.84 (d, J = 6.2 Hz, 1H), 7.44 (d, J = 2.4 Hz, 1H), 7.01 (dd, J = 8.7, 2.4 Hz, 1H), 6.90 (d, J = 8.7 Hz, 1H), 6.19 (d, J = 6.3 Hz, 1H), 4.57 (d, J = 13.1 Hz, 2H), 4.09 (t, J = 6.1 Hz, 2H), 3.83 (s, 3H), 2.92 - 2.74 (m, 4H), 2.68 (d, J = 6.3 Hz, 4H), 2.08 (d, J = 15.5 Hz, 2H), 1.87 (p, J = 2.9 Hz, 6H), 1.62 (t, J = 12.1 Hz, 1H), 1.40 - 1.25 (m, 2H), 1.18 (s, 6H). |
| 148 **(Reference)** | LC-MS: (ES, m/z): RT = 1.11 min, LCMS 33: m/z = 428.3 [M+1]. 1H NMR (300 MHz, Methanol-d4) δ 7.71 (dd, J = 7.9, 3.1 Hz, 1H), 7.18 - 6.98 (m, 3H), 6.60 (d, J = 7.7 Hz, 1H), 4.40 - 4.33 (m, 1H), 4.19 (t, J = 5.5 Hz, 2H), 4.03 - 3.97 (m, 2H), 3.91 (s, 3H), 3.86 - 3.80 (m, 2H), 3.74 - 3.54 (m, 2H), 3.49 (t, J = 7.1 Hz, 2H), 3.21 - 3.14 (m, 2H), 2.42 - 2.19 (m, 4H), 2.13 - 2.06 (m, 2H), 2.02 - 1.93 (m, 2H), 1.63 - 1.56 (m, 2H). |
| 149 **(Reference)** | LC-MS: (ES, m/z): RT = 1.180min, LCMS 07: m/z = 442.25 [M+1]. 1H NMR (300 MHz, Methanol-d4) δ 7.70 (d, J = 7.6 Hz, 1H), 7.15 - 6.98 (m, 3H), 6.59 (d, J = 7.7 Hz, 1H), 4.19 (t, J = 5.5 Hz, 3H), 3.91 (s, 7H), 3.69 - 3.51 (m, 2H), 3.48 (d, J = 7.1 Hz, 2H), 3.42 (s, 3H), 3.29 - 3.08 (m, 2H), 2.31 - 2.25 (m, 4H), 2.10 - 1.70 (m, 6H). |
| 150 **(Reference)** | LC-MS: (ES, m/z): RT = 1.121 min, LCMS28: m/z = 402.2 [M+1]. 1H NMR (300 MHz, Deuterium Oxide) δ 7.56 (dd, J = 15.3, 7.6 Hz, 1H), 7.14 - 6.93 (m, 3H), 6.42 - 6.28 (m, 1H), 4.10 (t, J = 5.7 Hz, 2H), 3.86 - 3.58 (m, 9H), 3.36 (t, J = 7.5 Hz, 2H), 3.18 - 2.98 (m, 5H), 2.25 - 1.86 (m, 6H). |
| 151 **(Reference)** | LC-MS: (ES, m/z): RT = 1.199 min, LCMS 28: m/z = 428.3 [M+1]. 1H NMR (300 MHz, Methanol-d4) δ 7.75 (d, J = 7.6 Hz, 1H), 7.10 (dd, J = 9.7, 4.6 Hz, 3H), 6.57 (dd, J = 12.5, 7.6 Hz, 1H), 4.20 (t, J = 5.6 Hz, 2H), 4.14 - 4.00 (m, 2H), 3.97 - 3.74 (m, 11H), 3.50 (t, J = 7.1 Hz, 2H), 3.18 (s, 2H), 2.42 - 1.88 (m, 8H). |
| 152 **(Reference)** | LC-MS: (ES, m/z): RT = 1.72 min, LCMS 33: m/z = 476.2 [M+1]. 1H NMR (300 MHz, Methanol-d4) δ 7.60-7.80 (m, 1H), 7.11-7.30 (m, 3H), 6.22 (d, J = 7.3 Hz, 1H), 4.15-4.25 (m, 3H), 3.92 (s, 3H), 3.87 - 3.77 (m, 2H), 3.52 - 3.47 (m, 2H), 3.27 - 3.12 (m, 6H), 2.46 - 2.04 (m, 10H). |
| 153 **(Reference)** | LC-MS: (ES, m/z): RT = 1.04min, LCMS07: m/z = 455.20 [M+1]. 1H NMR (300 MHz, Methanol-d4) δ 7.71 (d, J = 7.6 Hz, 1H), 7.19 - 6.97 (m, 3H), 6.60 (d, J = 7.7 Hz, 1H), 4.30 - 4.14 (m, 3H), 3.91 (s, 5H), 3.49 (t, J = 7.1 Hz, 2H), 3.35 (s, 1H), 3.17 (q, J = 12.3 Hz, 3H), 2.37 - 2.15 (m, 4H), 2.16 - 1.88 (m, 4H), 1.74 (q, J = 12.3 Hz, 2H). |
| 154 **(Reference)** | LC-MS: (ES, m/z): RT = 1.313 min, LCMS 28: m/z = 475.3 [M+1]. 1H NMR (300 MHz, Methanol-d4) δ 7.69 (d, J = 7.6 Hz, 1H), 7.37 (d, J = 7.7 Hz, 1H), 7.17 (t, J = 7.7 Hz, 1H), 7.10 - 7.00 (m, 3H), 6.86 (dd, J = 14.2, 8.0 Hz, 2H), 6.67 (t, J = 7.2 Hz, 1H), 4.81 (d, J = 12.6 Hz, 2H), 4.20 (q, J = 5.9 Hz, 3H), 3.97 (s, 3H), 3.83 (s, 2H), 3.59 - 3.43 (m, 2H), 3.35 (s, 3H), 3.30 - 3.02 (m, 2H), 2.32 - 2.04 (m, 6H). |
| 155 **(Reference)** | LC-MS: (ES, m/z): RT = 1.01 min, LCMS 33: m/z = 450.6 [M+1]. 1H NMR (300 MHz, Deuterium Oxide) δ 8.50-8.60 (m, 1H), 7.64-7.84 (m, 1H), 7.11 - 6.91 (m, 3H), 6.51-6.70 (m, 1H), 4.91 (s, 1H), 4.74 (s, 1H), 4.15 - 4.06 (m, 3H), 3.96 (t, J = 5.7 Hz, 1H), 3.82 (s, 3H), 3.69 - 3.63 (m, 2H), 3.34 (t, J = 7.5 Hz, 2H), 3.07 - 2.98 (m, 2H), 2.94 - 2.79 (m, 2H), 2.24 - 1.99 (m, 4H), 2.00 - 1.86 (m, 2H). |
| 156 **(Reference)** | LC-MS: (ES, m/z): RT = 0.541 min, LCMS 48: m/z = 462.3 [M+1]. 1H NMR (400 MHz, Methanol-d4) δ 8.96 (s, 1H), 8.66 (s, 1H), 7.96 (d, J = 6.2 Hz, 1H), 7.35 (d, J = 2.5 Hz, 1H), 7.09 (dd, J = 8.7, 2.5 Hz, 1H), 6.94 (d, J = 8.7 Hz, 1H), 6.35 (d, J = 6.2 Hz, 1H), 4.89 (s, 2H), 4.08 - 4.12 (m, 4H), 3.84 (s, 3H), 3.08 (t, J = 5.9 Hz, 2H), 2.82 - 2.73 (m, 2H), 2.71 - 2.62 (m, 4H), 2.14 - 2.02 (m, 2H), 1.92 - 1.79 (m, 4H). |
| 157 **(Reference)** | LC-MS: (ES, m/z): RT = 1.112 min, LCMS 15: m/z = 483.30 [M+1]. 1H NMR (300 MHz, Chloroform-d) δ 7.83 (d, J = 5.8 Hz, 1H), 6.87 (d, J = 9.2 Hz, 1H),6.71-6.83 (m, 2H), 6.34 (dd, J = 5.8, 2.1 Hz, 1H), 6.08 (d, J = 2.0 Hz, 1H), 5.83 (s, 1H), 4.64 (d, J = 13.7 Hz, 1H), 4.15 - 4.03 (m, 4H), 3.75 -3.88 (s, 4H), 2.97 -3.14 (m, 1H),2.80- 2.47 (m, 7H), 2.18 -1.96 (m, 6H), 1.93- 1.74 (m, 6H), 1.36 - 1.19 (m, 2H). |
| 158 **(Reference)** | LC-MS: (ES, m/z): RT = 1.143 min, LCMS07: m/z = 497.3 [M+1]. 1H NMR (300 MHz, Methanol-d4) δ 7.63 (d, J = 6.0 Hz, 1H), 6.96 (d, J = 8.4 Hz, 1H), 6.88 (d, J = 2.4 Hz, 1H), 6.83 - 6.79 (m, 1H), 5.81 (d, J = 6.0 Hz, 1H), 4.42 - 4.32 (m, 1H), 4.04 (t, J = 6.2 Hz, 2H), 3.84 - 3.78 (m, 4H), 3.38 (s, 3H), 3.16 - 2.94 (m, 3H), 2.73 - 2.60 (m, 7H), 2.08 - 2.00 (m, 5H), 1.85 - 1.80 (m, 5H), 1.71 - 1.55 (m, 2H), 1.12 - 0.90 (m, 2H). |
| 159 **(Reference)** | LC-MS: (ES, m/z): RT = 1.56 min, LCMS 27: m/z = 477.1 [M+1]. 1H NMR (300 MHz, Methanol-d4) δ7.69 (d, J = 6.1 Hz, 1H), 7.39 (d, J = 2.2 Hz, 1H), 7.21 - 7.05 (m, 3H), 6.90 (d, J = 8.7 Hz, 1H), 6.76 - 6.58 (m, 3H), 5.91 (d, J = 6.0 Hz, 1H), 4.17 (t, J = 5.9 Hz, 2H), 3.85 - 3.25 (m, 4H), 2.85 (d, J = 11.5 Hz, 2H), 2.24 (s, 3H), 2.16 - 2.06 (m, 2H), 2.05 - 1.90 (m, 2H), 1.75 (d, J = 13.3 Hz, 2H), 1.70 - 1.54 (m, 1H), 1.40 - 1.21 (m, 2H). |
| 160 **(Reference)** | LC-MS: (ES, m/z): RT = 1.31 min; LCMS 33: m/z = 505.30 [M+1]. 1H NMR (300 MHz, Methanol-d4) δ 7.70 (d, J = 6.0 Hz, 1H), 7.39 (d, J = 2.5 Hz, 1H), 7.18 - 7.02 (m, 3H), 6.89 (dd, J = 8.8, 1.3 Hz, 1H), 6.73 - 6.56 (m, 3H), 5.92 (d, J = 6.0 Hz, 1H), 4.45 (d, J = 13.3 Hz, 1H), 4.17 - 4.14 (m, 2H), 3.89 - 3.84 (m, 4H), 3.38 - 3.20 (m, 4H), 3.09 - 2.93 (m, 1H), 2.71 - 2.51 (m, 1H), 2.16 - 2.01 (m, 5H), 1.95 - 1.68 (m, 3H), 1.23 - 1.00 (m, 2H). |
| 161 **(Reference)** | LC-MS: (ES, m/z): RT = 1.106 min, LCMS 33: m/z = 469 [M+1]. 1H NMR (300 MHz, Methanol-d4) δ 7.86 (d, J = 6.2 Hz, 1H), 7.39 (d, J = 2.5 Hz, 1H), 7.02 (dd, J = 8.7, 2.4 Hz, 1H), 6.90 (d, J = 8.7 Hz, 1H), 6.21 (d, J = 6.3 Hz, 1H), 4.42 (d, J = 13.6 Hz, 2H), 4.13 - 3.89 (m, 3H), 3.82 (s, 3H), 3.16 - 3.00 (m, 2H), 2.81 - 2.58 (m, 6H), 2.18 - 1.68 (m, 11H), 1.52 - 1.34 (m, 2H). |
| 162 **(Reference)** | LC-MS: (ES, m/z): RT = 1.78min, LCMS07: m/z = 491.20 [M+1]. 1H NMR (400 MHz, Methanol-d4) δ 7.92 (d, J = 6.1 Hz, 1H), 7.35 (d, J = 2.5 Hz, 1H), 7.04 (d, J = 8.7 Hz, 1H), 6.92 (d, J = 8.7 Hz, 1H), 6.25 (d, J = 6.2 Hz, 1H), 4.09 (t, J = 6.2 Hz, 2H), 3.85-3.81 (m, 7H), 3.50 - 3.29 (m, 4H), 2.87 (s, 3H), 2.84 - 2.76 (m, 2H), 2.71(d, J = 5.9 Hz, 4H), 2.14 - 2.02 (m, 2H), 1.92 - 1.84 (m, 4H). |
| 163 **(Reference)** | LC-MS: (ES, m/z): RT = 1.18 min, LCMS 33: m/z = 496 [M+1]. 1H NMR (400 MHz, DMSO-d6) δ 7.57 (d, J = 7.3 Hz, 1H), 7.04 (d, J = 8.6 Hz, 1H), 6.96 - 6.81 (m, 2H), 6.61 (s, 1H), 5.93 (s, 1H), 4.37 (d, J = 13.0 Hz, 1H), 4.05 (t, J = 6.0 Hz, 2H), 3.88-3.78 (m, 4H), 3.61 - 3.53 (m, 2H), 3.47 - 3.22 (m, 4H), 3.07 - 2.90 (m, 6H), 2.44 (t, J = 12.4 Hz, 1H), 2.18-2.08 (m, 2H), 2.01 (d, J = 6.8 Hz, 2H), 1.98 (s, 3H), 1.92-1.82 (m, 3H), 1.61 - 1.51 (m, 2H), 1.20 - 1.10 (m, 1H), 1.09 - 0.96 (m, 1H). |
| 164 **(Reference)** | LC-MS: RT = 2.26min, LCMS07: m/z = 474 [M+1]. 1H NMR (400 MHz, Methanol-d4) δ 9.12 (s, 1H), 8.56 (d, J = 5.4 Hz, 1H), 7.73 - 7.64 (m, 2H), 7.51 (d, J = 5.4 Hz, 1H), 7.47 - 7.37 (m, 2H), 7.26 (dd, J = 8.7, 2.5 Hz, 1H), 7.00 (d, J = 8.7 Hz, 1H), 4.15 (t, J = 6.1 Hz, 2H), 3.89 - 3.85 (m, 5H), 2.84- 2.75 (m, 2H), 2.67 (t, J = 6.2 Hz, 4H), 2.15 -2.08 (m, 2H), 1.90 - 1.81 (m, 4H). |
| 165 **(Reference)** | LC-MS: (ES, m/z): RT = 0.61 min, LCMS 32: m/z = 451.4 [M+1]. 1H NMR (300 MHz, Methanol-d4) δ 7.75 (d, J = 6.0 Hz, 1H), 7.65 (dd, J = 9.4, 2.6 Hz, 1H), 7.44 - 7.33 (m, 2H), 7.06 (dd, J = 8.7, 2.5 Hz, 1H), 6.89 (d, J = 8.7 Hz, 1H), 6.53 (dd, J = 9.3, 0.8 Hz, 1H), 5.95 (d, J = 6.0 Hz, 1H), 4.41 (s, 2H), 4.05 (t, J = 6.0 Hz, 2H), 3.83 (s, 3H), 2.87 - 2.78 (m, 6H), 2.14 - 1.98 (m, 2H), 1.96 - 1.88 (m, 4H). |
| 166 **(Reference)** | LC-MS: (ES, m/z): RT = 0.826 min, LCMS 30: m/z = 497 [M+1]. 1H NMR (400 MHz, Chloroform-d) δ 7.70 (s, 1H), 7.36 (d, J = 2.0 Hz, 1H), 7.09 (d, J = 6.4 Hz, 1H), 6.85 (d, J = 8.8 Hz, 1H), 5.90 (d, J = 6.0 Hz, 1H), 4.45 - 4.40 (m, 1H), 4.30 (t, J = 6.0 Hz, 2H), 3.90 - 3.86 (m, 1H), 3.77 (s, 3H), 3.61 (t, J = 6.8 Hz, 2H), 3.42 (t, J = 6.8 Hz, 2H), 3.32 -3.30 (m, 2H), 3.06 (t, J = 1.2 Hz, 1H), 2.80 (t, J = 6.4 Hz, 2H), 2.60 (t, J = 2.8 Hz, 1H), 2.06 (s, 3H), 1.99 - 1.75 (m, 7H), 1.23 - 1.10 (m, 2H). |
| 167 **(Reference)** | LC-MS: (ES, m/z): RT = 1.356 min, LCMS 15: m/z = 449.25 [M+1]. 1H NMR (300 MHz, Methanol-d4) δ 8.69 - 8.59 (m, 1H), 8.50 (t, J = 7.9 Hz, 1H), 7.97 (d, J = 7.9, 5.8 Hz, 2H), 7.63 (d, J = 7.2 Hz, 1H), 7.15 - 7.07 (m, 2H), 7.03 (d, J = 8.6, 2.4 Hz, 1H), 6.22 (d, J = 7.3 Hz, 1H), 4.23 (t, J = 5.6 Hz, 2H), 4.00 - 3.89 (m, 5H), 3.89 - 3.75 (m, 2H), 3.61 - 3.49 (m, 4H), 3.22 - 3.08 (m, 2H), 2.35 - 2.16 (m, 4H), 2.15 - 2.00 (m, 2H). |
| 168 **(Reference)** | LC-MS: (ES, m/z): RT = 1.165 min, LCMS53: m/z = 490.30 [M+1]. 1H NMR (300 MHz, Deuterium Oxide) δ 8.04 -7.96 (m, 1H),7.88 - 7.78 (m, 1H), 7.64 (d, J = 7.6 Hz, 1H), 7.18 (d, J = 9.3 Hz, 1H), 7.13- 6.98 (m, 3H), 6.97 - 6.89 (m, 1H), 6.40 (d, J = 7.6 Hz, 1H), 4.10 (t, J = 5.9 Hz, 4H), 3.89 (s, 4H), 3.82 (s, 5H), 3.72 - 3.59 (m, 2H), 3.36 (t, J = 7.5 Hz, 2H), 3.12 - 2.96 (m, 2H), 2.25 - 2.02 (m, 4H), 1.94 (m,2H). |
| 169 **(Reference)** | LC-MS: (ES, m/z): RT = 1.081 min, LCMS 33: m/z = 462 [M+1]. 1H-NMR: (400 MHz, Methanol-d4) δ 7.99 (d, J = 6.0 Hz, 1H), 7.27 (d, J = 2.4 Hz, 1H), 7.07 - 7.03 (m, 1H), 6.4 (d, J = 8.8 Hz, 1H), 6.35 (d, J = 6.4 Hz, 1H), 4.28 - 4.08 (m, 4H), 4.06 (t, J = 6.2 Hz, 2H), 3.83 (s, 3H), 3.20-3.11(m, 4H), 2.78 - 2.69 (m, 2H), 2.62 (q, J = 4.4 Hz, 4H), 2.11- 1.99 (m, 2H), 1.90 - 1.80 (m, 4H). |
| 170 **(Reference)** | LC-MS: (ES, m/z): RT = 1.068 min, LCMS 07: m/z = 455 [M+1]. 1H-NMR: (400 MHz, Methanol-d4) δ 7.86 (d, J = 6.0 Hz, 1H), 7.53 (d, J = 2.4 Hz, 1H), 7.08 - 7.03 (m, 1H), 6.89 (d, J = 8.8 Hz, 1H), 5.83 (d, J = 6.0 Hz, 1H), 4.29 (t, J = 8.8 Hz, 2H), 4.25 - 4.20 (m, 2H), 4.09 (t, J = 6.0 Hz, 2H), 3.98 - 3.90 (m, 1H), 3.82 (s, 3H), 3.01 (d, J = 8.4 Hz, 6H), 2.81 - 2.75 (m, 2H), 2.72 - 2.62 (m, 4H), 2.11 - 2.02 (m, 2H), 1.92 - 1.80 (m, 4H). |
| 171 **(Reference)** | LC-MS: (ES, m/z): RT = 1.74 min, LCMS 53: m/z = 372 [M+1]. 1H NMR (400 MHz, Chloroform-d) δ 7.93 (d, J = 8.0 Hz, 1H), 7.43 (s, 1H), 6.98 - 6.95 (m, 1H), 6.82 - 6.80 (d, J = 8.8 Hz, 2H), 5.92 (d, J = 6.0 Hz, 1H), 4.09 (t, J = 6.8 Hz, 2H), 3.86 (s, 3H), 3.12 (s, 6H), 2.66 - 2.62 (t, J = 8.0 Hz, 2H), 2.54 (d, J = 6.1 Hz, 4H), 2.12 - 2.05 (m, 2H), 1.83 - 1.79 (m, 4H). |
| 172 **(Reference)** | LC-MS: (ES, m/z): RT = 2.821 min, LCMS07: m/z = 497.25 [M+1]. 1H NMR (300 MHz, Methanol-d4) δ 7.83 (d, J = 6.3 Hz, 1H), 7.24 (s, 1H), 7.17 - 7.13 (m, 1H), 6.88 (d, J = 8.7 Hz, 1H), 6.07 (d, J = 6.3 Hz, 1H), 4.43 - 4.39 (m, 1H), 4.08 (t, J = 6.1 Hz, 2H), 3.87 - 3.81 (m, 4H), 3.60 - 3.50(m, 1H), 3.50 - 3.40 (m, 1H), 3.09 - 3.00 (m, 4H), 2.82 - 2.74 (m, 6H), 2.68 - 2.60 (m, 1H), 2.14 - 1.98 (m, 6H), 1.93 - 1.83 (m, 4H), 1.80 - 1.60 (m, 2H), 1.33 - 1.06 (m, 2H). |
| 173 **(Reference)** | LC-MS: (ES, m/z): RT = 1.16 min, LCMS 33: m/z =483 [M+1]. 1H NMR (300 MHz, Methanol-d4) δ 7.74 (d, J = 7.3 Hz, 1H), 7.14 (d, J = 8.5 Hz, 1H), 7.07 - 6.94 (m, 2H), 6.67 (dd, J = 7.3, 2.3 Hz, 1H), 6.49 (d, J = 2.4 Hz, 1H), 4.61 (d, J = 13.3 Hz, 1H), 4.22 (t, J = 5.5 Hz, 2H), 4.10 (d, J = 6.0 Hz, 3H), 3.92 (s, 3H), 3.93 - 3.71 (m, 2H), 3.49 (t, J = 7.1 Hz, 2H), 3.31 - 3.09 (m, 3H), 2.84 (t, J = 12.8 Hz, 1H), 2.38 - 2.18 (m, 8H), 2.18 - 1.88 (m, 4H), 1.55-1.28 (m, 2H). |
| 174 **(Reference)** | LC-MS: (ES, m/z): RT = 1.14 min, LCMS 15: m/z = 343 [M+1]. 1H NMR (300 MHz, Chloroform-d) δ 8.24 (d, J = 4.8 Hz, 1H), 7.38 (d, J = 2.7 Hz, 1H), 7.04 - 7.00 (m, 1H), 6.93 (s, 1H), 6.84 (d, J = 8.4 Hz, 1H), 6.56 (d, J = 5.1 Hz, 1H), 4.12 (t, J = 6.6 Hz, 2H), 3.86 (s, 3H), 2.72 (t, J = 7.2 Hz, 2H), 2.68 - 2.60 (m, 4H), 2.39 (s, 3H), 2.17 - 2.07 (m, 2H), 1.92 - 1.76 (m, 4H). |
| 175 **(Reference)** | LC-MS: (ES, m/z): RT = 1.15 min, LCMS 15: m/z = 357 [M+1]. 1H NMR (300 MHz, Chloroform-d) δ 7.54 (d, J = 2.4 Hz, 1H), 7.07 - 6.98 (m, 1H), 6.91 (s, 1H), 6.82 (d, J = 8.7 Hz, 1H), 6.48 (s, 1H), 4.12 (t, J = 6.6 Hz, 2H), 3.86 (s, 3H), 2.69 (t, J = 7.5 Hz, 2H), 2.58 (q, J = 5.2 Hz, 4H), 2.34 (s, 6H), 2.15 - 2.06 (m, 2H), 1.81 - 1.77 (m, 4H). |
| 176 **(Reference)** | LC-MS: (ES, m/z): RT= 1.152, LCMS m/z = 357.30 [M+1]. 1H NMR (300 MHz, Methanol-d4, ppm) δ 7.34 (d, J = 2.5 Hz, 1H), 7.22 (t, J = 7.9 Hz, 1H), 6.97 (dd, J = 8.6, 2.4 Hz, 1H), 6.86 (d, J = 8.6 Hz, 1H), 5.99 (d, J = 7.5 Hz, 1H), 5.85 (d, J = 8.0 Hz, 1H), 4.05 (t, J = 6.2 Hz, 2H), 3.80 (s, 3H), 2.87 (s, 3H), 2.72 - 2.65 (m, 2H), 2.61 - 2.57 (m, 4H), 2.07 - 1.97 (m, 2H), 1.87 - 1.75 (m, 4H). |
| 177 **(Reference)** | LC-MS: (ES, m/z): RT = 1.04 min, LCMS 33: m/z = 416 [M+1]. 1H NMR (300 MHz, Methanol-d4) δ 7.79 - 7.63 (m, 1H), 7.15 (d, J = 12.9 Hz, 1H), 7.10 - 7.03 (m, 2H), 6.60 - 6.45 (m, 1H), 4.20 (t, J = 5.5 Hz, 2H), 3.99 - 3.75 (m, 7H), 3.70 - 3.60 (m, 2H), 3.49 (t, J = 7.1 Hz, 2H), 3.35 (d, J = 6.5 Hz, 3H), 3.28 (d, J = 9.7 Hz, 3H), 3.17-3.10 (m, 2H), 2.42 - 2.01 (m, 6H). |
| 178 **(Reference)** | LC-MS: (ES, m/z): RT = 1.14 min, LCMS 07: m/z = 456 [M+1]. 1H NMR (300 MHz, Methanol-d4) δ 7.59 (d, J = 7.3 Hz, 1H), 7.14 - 7.01 (m, 3H), 6.18 (d, J = 7.3 Hz, 1H), 4.20 (t, J = 5.6 Hz, 2H), 3.96 - 3.75 (m, 7H), 3.57 - 3.34 (m, 6H), 3.20-3.10 (m, 2H), 2.37 - 2.15 (m, 4H), 2.18 - 2.02 (m, 2H), 1.66 - 1.51 (m, 5H), 1.30-1.15 (m, 2H) . |
| 179 **(Reference)** | LC-MS: (ES, m/z): RT = 0.572 min, LCMS 32: m/z = 427 [M+1]. 1H-NMR: (400 MHz, Methanol-d4) δ 7.86 (d, J = 5.6 Hz, 1H), 7.54 (d, J = 2.4 Hz, 1H), 7.07 - 7.03 (m, 1H), 6.90 (d, J = 8.8 Hz, 1H), 5.83 (d, J = 6.0 Hz, 1H), 4.26 (t, J = 8.6 Hz, 2H), 4.22 - 4.15 (m, 2H), 4.11 (t, J = 6.0 Hz, 2H), 3.82 (s, 3H), 3.65 - 3.55 (m, 1H), 2.92 (t, J = 7.6 Hz, 2H), 2.85 (d, J = 6.2 Hz, 4H), 2.16 - 2.05 (m, 2H), 1.96 - 1.86 (m, 4H). |
| 180 **(Reference)** | LC-MS: (ES, m/z): RT = 1.462 min, LCMS33: m/z = 409 [M+1]. 1H NMR (300 MHz, Methanol-d4) δ 8.35 (s, 1H), 8.28 (d, J = 6.6 Hz, 1H), 7.83 (s, 1H), 7.54 - 7.44 (m, 1H), 7.22 (s, 1H), 7.20 - 7.10 (m, 2H), 4.23 (t, J = 5.4 Hz, 2H), 3.93 (s, 3H), 3.87 - 3.80 (m, 2H), 3.50 (t, J = 7.2 Hz, 2H), 3.25 - 3.10 (m, 2H), 2.35 - 2.23 (m, 3H), 2.21 - 2.20 (m, 4H), 2.18 - 2.05 (m, 2H). |
| 181 **(Reference)** | LC-MS: (ES, m/z): RT = 1.070 min, LCMS07: m/z = 359.0 [M+1]. 1H NMR (300 MHz, Methanol-d4) δ 8.38 (s, 2H), 7.38 (d, J = 2.5 Hz, 1H), 7.16 - 7.12 (m, 1H), 6.91 (d, J = 8.7 Hz, 1H), 4.49 (s, 2H), 4.09 (t, J = 6.1 Hz, 2H), 3.82 (s, 3H), 2.84 - 2.76 (m, 2H), 2.76 - 2.66 (m, 4H), 2.15 - 1.99 (m, 2H), 1.95 - 1.79 (m, 4H). |
| 182 **(Reference)** | LC-MS: (ES, m/z): RT = 2.06 min, LCMS 33: m/z = 483 [M+1]. 1H NMR (300 MHz, DMSO-d6) δ 7.90-7.70 (m, 2H), 6.70-6.45 (m, 2H), 6.09 (s, 1H), 4.39 (d, J = 12.8 Hz, 1H), 4.16 (t, J = 5.8 Hz, 2H), 3.83 (s, 4H), 3.65-3.50 (m, 3H), 3.29 (t, J = 7.6 Hz, 2H), 3.20-2.90 (m, 5H), 2.20 (t, J = 7.3 Hz, 2H), 2.10-1.95 (m, 5H), 1.94 - 1.83 (m, 3H),1.83- 1.73(m, 2H), 1.33 - 0.85 (m, 2H). |
| 183 **(Reference)** | LC-MS: (ES, m/z): RT = 0.714 min, LCMS 32: m/z = 497 [M+1]. 1H-NMR: (300 MHz, Chloroform-d) δ 7.92 (d, J = 6.0 Hz, 1H), 7.18 (d, J = 2.4 Hz, 1H), 7.12 - 7.06 (m, 1H), 6.93 - 6.75 (m, 2H), 5.80 (d, J = 6.0 Hz, 1H), 4.89 (s, 1H), 4.69 - 4.50 (m, 1H), 4.18 - 4.05 (m, 1H), 3.91 - 3.70 (m, 5H), 3.35 - 3.16 (m, 2H), 3.10 - 2.90 (m, 1H), 2.66 - 2.42 (m, 6H), 2.41 - 2.19 (m, 2H), 2.09 (s, 3H), 1.93 - 1.68 (m, 7H), 1.31- 1.02 (m, 5H). |
| 184 **(Reference)** | LC-MS: (ES, m/z): RT = 1.00 min, LCMS 15: m/z = 482.4 [M+1]. 1H NMR: (400 MHz, Methanol-d4) δ 7.48 - 7.41 (m, 2H), 7.08 - 7.01 (m, 2H), 6.93 - 6.83 (m, 2H), 4.57 (d, J = 13.4 Hz, 1H), 4.20 (t, J = 5.5 Hz, 2H), 4.02 (d, J = 13.6 Hz, 1H), 3.89 (s, 3H), 3.87 - 3.79 (m, 2H), 3.49 (t, J = 7.1 Hz, 2H), 3.37 (s, 1H), 3.26 - 3.09 (m, 3H), 3.08 (d, J = 6.4 Hz, 2H), 2.70-2.80 (m, 1H), 2.35 - 2.02 (m, 9H), 2.02 - 1.84 (m, 3H), 1.39 - 1.14 (m, 2H). |
| 185 **(Reference)** | LC-MS: (ES, m/z): RT = 0.962 min, LCMS 53: m/z = 483.35 [M+1]. 1H NMR: (300 MHz, Methanol-d4) δ 8.04 (d, J = 2.4 Hz, 1H), 7.19 (t, J = 1.4 Hz, 1H), 6.93 (d, J = 1.3 Hz, 2H), 5.99 (d, J = 2.5 Hz, 1H), 4.62 - 4.49 (m, 1H), 4.08 (t, J = 6.1 Hz, 2H), 4.01 - 3.91 (m, 1H), 3.83 (s, 3H), 3.20 - 2.98 (m, 3H), 2.81 - 2.55 (m, 7H), 2.14 - 1.98 (m, 5H), 1.95 - 1.75 (m, 7H), 1.35 - 1.05 (m, 2H). |
| 186 | LC-MS: (ES, m/z): RT = 0.997 min, LCMS53: m/z = 390.30 [M+1]. 1H NMR: (300 MHz, DMSO-d6) δ 8.73 (s, 1H), 7.76 (s, 1H), 7.14 (dd, J = 8.7, 2.2 Hz, 1H), 6.91 (s, 1H), 6.79 (d, J = 8.7 Hz, 1H), 5.73 (s, 1H), 5.29 - 5.06 (m, 1H),3.96 (t, J = 6.5 Hz, 2H), 3.68 (s, 3H), 2.89 - 2.70 (m, 5H), 2.68 - 2.48 (m, 3H), 2.34 - 2.26 (m, 1H), 2.26 - 2.02 (m, 4H), 1.98 - 1.74 (m, 3H). |
| 187 **(Reference)** | LC-MS: RT= 1.025, m/z = 376.30 [M+1]. 1H NMR: (300 MHz, Methanol-d4, ppm) δ: 7.54 (d, J = 4.0 Hz, 1H), 7.33 (d, J = 2.5 Hz, 1H), 6.97 (dd, J = 8.7, 2.5 Hz, 1H), 6.75 (d, J = 8.7 Hz, 1H), 3.93 (t, J = 6.2 Hz, 2H), 3.69 (s, 3H), 2.89 (s, 3H), 2.62 - 2.57 (m, 2H), 2.52 - 2.46 (m, 4H), 1.96 - 1.87 (m, 2H), 1.77 - 1.72 (m, 4H). |
| 188 **(Reference)** | LC-MS: (ES, m/z): RT = 0.955 min, LCMS 28: m/z = 358.2 [M+1]. 1H-NMR: 1H NMR (300 MHz, Methanol-d4) δ 8.03 (d, J = 2.5 Hz, 1H), 7.10 (d, J = 8.5 Hz, 1H), 7.00 - 6.89 (m, 2H), 6.37 (d, J = 2.5 Hz, 1H), 4.22 (t, J = 5.5 Hz, 2H), 3.90 (s, 3H), 3.86 - 3.75 (m, 2H), 3.49 (t, J = 7.1 Hz, 2H), 3.17 (d, J = 10.8Hz, 2H), 2.96 (s, 3H), 2.35 - 2.01 (m, 6H). |
| 190 **(Reference)** | LC-MS: (ES, m/z): RT = 0.871 min, LCMS 53: m/z = 442.3 [M+1]. 1H NMR (300 MHz, Methanol-d4) δ 8.24 (d, J = 6.3 Hz, 1H), 7.25 - 7.11 (m, 2H), 7.04 (d, J = 8.7 Hz, 1H), 6.76 - 6.67 (m, 1H), 4.40 - 4.32 (m, 2H), 4.20 (t, J = 5.5 Hz, 2H), 4.07- 4.10 (m, 2H), 3.90 (s, 3H), 3.88 - 3.77 (m, 2H), 3.62 - 3.37 (m, 5H), 3.25 - 3.09 (m, 2H), 2.34 - 2.17 (m, 4H), 2.15 - 2.03 (m, 4H), 1.86 - 1.66 (m, 2H). |
| 191 **(Reference)** | LC-MS: (ES, m/z): RT = 0.840 min, LCMS 07: m/z = 474.20 [M+1]. 1H NMR (300 MHz, Methanol-d4) δ 8.88 (d, J = 20.6 Hz, 1H), 8.29 - 8.16 (m, 1H), 8.09 (d, J = 1.9 Hz, 1H), 8.03 - 7.90 (m, 2H), 7.68 (d, J = 7.5 Hz, 1H), 7.14 - 6.96 (m, 3H), 6.37 - 6.29 (m, 1H), 4.82 (s, 2H), 4.28 - 4.12 (m, 2H), 3.90 (s, 3H), 3.86 - 3.73 (m, 2H), 3.48 (t, J = 7.1 Hz, 2H), 3.15 (t, J = 8.3, 2H), 2.37 - 1.99 (m, 6H). |
| 192 **(Reference)** | LC-MS: (ES, m/z): RT = 1.253 min, LCMS07: m/z = 441.10 [M+1]. 1H NMR (300 MHz, DMSO-d6) δ 10.63 (s, 1H), 9.77 (s, 1H), 9.60 (t, J = 4.1 Hz, 1H), 7.84 (d, J = 7.5 Hz, 1H), 7.20 - 7.00 (m, 3H), 6.23 (d, J = 7.2 Hz, 1H), 4.60 - 4.48 (m, 1H), 4.39 (t, J = 8.3 Hz, 1H), 4.16 - 4.00 (m, 4H), 3.85 - 3.56 (m, 6H), 3.36 - 3.26 (m, 2H), 3.10 - 2.96 (m, 2H), 2.22 - 1.73 (m, 9H). |
| 193 **(Reference)** | LC-MS: (ES, m/z): RT = 0.987 min, LCMS 33: m/z = 439 [M+1]. 1H NMR (300 MHz, Methanol-d4) δ 7.63 - 7.48 (m, 3H), 7.11 (d, J = 7.2 Hz, 3H), 6.30 (t, J = 2.1 Hz, 1H), 6.15 (d, J = 7.3 Hz, 1H), 4.40 (t, J = 6.0 Hz, 2H), 4.19 (t, J = 5.5 Hz, 2H), 3.95 - 3.77 (m, 7H), 3.48 (t, J = 7.1 Hz, 2H), 3.19 - 3.01 (m, 2H), 2.30-2.15 (m, 4H), 2.10-2.00 (m, 2H). |
| 194 **(Reference)** | LC-MS: (ES, m/z): RT = 1.00 min, LCMS 15: m/z = 386 [M+1]. 1H NMR (400 MHz, Methanol-d4) δ 8.57 (s, 2H), 7.39 (s, 1H), 7.13 (d, J = 6.4 Hz, 1H), 6.91 (d, J = 8.8 Hz, 1H), 4.09 (t, J = 6.2 Hz, 2H), 3.82 (s, 3H), 2.76 - 2.72 (m, 2H), 2.69 - 2.64 (m, 4H), 2.14 (s, 3H), 2.12 - 2.00 (m, 2H), 1.88 - 1.81 (m, 4H). |
| 195 **(Reference)** | LC-MS: (ES, m/z): RT = 1.007 min, LCMS 15: m/z = 497 [M+1]. 1H NMR (400 MHz, Methanol-d4) δ 7.70 (s, 1H), 7.32 (s, 1H), 7.18 (d, J = 2.4 Hz, 1H), 6.90 (d, J = 8.8 Hz, 1H), 5.92 (d, J = 6.0 Hz, 1H), 4.55 - 4.39 (m, 2H), 3.97 - 3.87 (m, 1H), 3.82 (s, 3H), 3.32 - 3.30 (m, 1H), 3.29 - 3.25 (m, 1H), 3.09 (t, J = 1.2 Hz, 1H), 2.80 - 2.75 (m, 1H), 2.70 - 2.56 (m, 6H), 2.09 (s, 3H), 2.05 - 1.76 (m, 9H), 1.32 (d, J = 6.0 Hz, 3H), 1.28 - 1.15 (m, 2H). |
| 196 **(Reference)** | LC-MS: (ES, m/z): RT = 0.902 min, LCMS 30: m/z = 483.36 [M+H]. 1H NMR (300 MHz, Methanol-d4) δ 8.31 (d, J = 5.4 Hz, 1H), 7.33 - 7.15 (m, 2H), 7.02 (d, J = 8.7 Hz, 1H), 6.82 (d, J = 5.4 Hz, 1H), 4.20 (t, J = 5.5 Hz, 2H), 3.93 - 3.76 (m, 8H), 3.66 (t, J = 6.8 Hz, 2H), 3.51 - 3.40 (m, 6H), 3.29 - 3.11 (m, 5H), 2.36 - 2.01 (m, 9H). |
| 197 **(Reference)** | LC-MS: (ES, m/z): RT = 1.207 min, LCMS15: m/z = 381.2 [M+1]. 1H NMR (300 MHz, Methanol-d4) δ 8.14 (s, 1H), 7.15 - 7.10 (m, 1H), 7.03 - 7.00 (m, 1H), 6.94 (d, J = 8.4 Hz, 1H), 6.88 (d, J = 2.4 Hz, 1H), 6.79 - 6.78 (m, 1H), 6.62 - 6.59 (m, 1H), 4.18 (s, 3H), 4.05 (t, J = 6.0 Hz, 2H), 3.84 (s, 3H), 2.75 (t, J = 7.5Hz, 2H), 2.68 - 2.63 (m, 4H), 2.13 - 1.97 (m, 2H), 1.93 - 1.77 (m, 4H). |
| 199 **(Reference)** | LC-MS: (ES, m/z): RT = 1.06 min, LCMS 53: m/z = 519.3 [M+1]. 1H NMR (300 MHz, Methanol-d4) δ 7.61 (d, J = 7.3 Hz, 1H), 7.19 - 6.99 (m, 3H), 6.20 (d, J = 7.3 Hz, 1H), 4.21 (t, J = 5.5 Hz, 2H), 3.91 (s, 3H), 3.80 - 3.71 (m, 4H), 3.55 - 3.38 (m, 4H), 3.32 - 3.18 (m, 2H), 2.84 (s, 3H), 2.80 - 2.65 (m, 2H), 2.38 - 2.01 (m, 6H), 1.89 - 1.72 (m, 3H), 1.39 - 1.24 (m, 2H). |
| 200 **(Reference)** | LC-MS: (ES, m/z): RT = 0.98 min, LCMS 33: m/z = 381 [M+1]. 1H NMR (300 MHz, Methanol-d4) δ 9.14 (s, 1H), 7.63 (dd, J = 8.9, 0.7 Hz, 1H), 7.32 - 7.20 (m, 2H), 7.07 - 6.97 (m, 1H), 6.96 - 6.85 (m, 2H), 4.19 (t, J = 5.6 Hz, 2H), 4.02 (s, 3H), 3.89 -3.79 (m, 5H), 3.50 (t, J = 7.0 Hz, 2H), 3.25 - 3.10 (m, 2H), 2.36 - 2.02 (m, 6H). |
| 201 **(Reference)** | LC-MS: (ES, m/z): RT = 0.78 min, LCMS 48: m/z = 368.2 [M+1]. 1H NMR (300 MHz, Methanol-d4) δ 8.99 (s, 1H), 8.47 (dd, J = 6.5, 1.0 Hz, 1H), 7.49 - 7.31 (m, 2H), 7.12 - 6.89 (m, 3H), 4.20 (t, J = 5.6 Hz, 2H), 3.90 (s, 3H), 3.88 - 3.78 (m, 2H), 3.50 (t, J = 7.1 Hz, 2H), 3.26 - 3.09 (m, 2H), 2.37 - 1.99 (m, 6H). |
| 202 **(Reference)** | LC-MS: (ES, m/z): RT = 0.99 min ; LCMS 33: m/z = 383.21 [M+1]. 1H NMR (300 MHz, Methanol-d4) δ 8.76 (s, 1H), 7.24 - 7.07 (m, 3H), 6.54 (s, 1H), 4.24 (t, J = 5.5 Hz, 2H), 3.95 (s, 3H), 3.90 - 3.77 (m, 2H), 3.50 (t, J = 7.2 Hz, 2H), 3.25 - 3.10 (m, 2H), 2.60 (d, J = 0.5 Hz, 3H), 2.39 - 2.02 (m, 6H). |
| 203 **(Reference)** | LC-MS: (ES, m/z): RT = 1.17 min; LCMS 33: m/z = 513.30 [M+1]. 1H NMR (300 MHz, Methanol-d4) δ 7.70 (d, J = 6.0 Hz, 1H), 7.33 (d, J = 2.5 Hz, 1H), 7.13 (dd, J = 8.7, 2.5 Hz, 1H), 6.88 (d, J = 8.7 Hz, 1H), 5.92 (d, J = 6.0 Hz, 1H), 4.19 - 4.03 (m, 6H), 3.82 (s, 3H), 3.30 (s, 2H), 2.89 - 2.61 (m, 8H), 2.06 (m, 2H), 1.92 - 1.72 (m, 7H), 1.32 - 1.06 (m, 5H). |
| 204 **(Reference)** | LC-MS: (ES, m/z): RT = 1.285 min, LCMS 07: m/z = 444.1 [M+1]. 1H NMR (300 MHz, Methanol-d4) δ 7.70 (s, 1H), 7.35 (d, J = 2.5 Hz, 1H), 7.15 (d, J = 8.7, 2.5 Hz, 1H), 6.90 (d, J = 8.7 Hz, 1H), 5.93 (d, J = 6.0 Hz, 1H), 4.19 (t, J = 5.5 Hz, 2H), 4.02 - 3.92 (m, 2H), 3.82 (s, 3H), 3.76 - 3.69 (m, 4H), 3.48 - 3.37 (m, 2H), 3.32 (d, J = 1.6 Hz, 2H), 2.84 (t, J = 5.5 Hz, 2H), 2.71 - 2.57 (m, 4H), 1.99 - 1.79 (m, 1H), 1.74 - 1.64 (m, 2H), 1.43 - 1.24 (m, 2H). |
| 205 | LC-MS: (ES, m/z): RT= 0.970 min, LCMS 33, m/z =372 [M+1]. 1H NMR (300 MHz, Deuterium Oxide) δ7.20 - 6.90 (m, 3H), 5.91 (s, 1H), 4.09 (t, J = 5.6 Hz, 2H), 3.80 (s, 3H), 3.72 - 3.60 (m, 2H), 3.40 - 3.28 (m, 2H), 3.10 - 2.95 (m, 2H), 2.84 (s, 3H), 2.29 - 1.85 (m, 9H). |
| 206 **(Reference)** | LC-MS: (ES, m/z): RT = 0.96 min ; LCMS 33: m/z = 357.20 [M+1]. 1H NMR (300 MHz, Methanol-d4) δ 7.53 (d, J = 7.3 Hz, 1H), 7.08 (d, J = 8.3 Hz, 1H), 6.91 (m, 2H), 6.44 - 6.34 (m, 1H), 6.02 (d, J = 2.2 Hz, 1H), 4.20 (t, J = 5.5 Hz, 2H), 3.90 (s, 3H), 3.88 - 3.78 (m, 2H), 3.49 (t, J = 7.1 Hz, 2H), 3.25 - 3.09 (m, 2H), 2.89 (s, 3H), 2.37 - 1.99 (m, 6H). |
| 207 **(Reference)** | LC-MS: (ES, m/z): RT =1.540 min, LCMS 15: m/z = 328 [M+1]. 1H NMR (400 MHz, Methanol-d4) δ 7.74 (d, J = 5.2 Hz, 1H), 7.49 (s, 1H), 7.17 - 7.11 (m, 2H), 6.55 - 6.52 (m, 1H), 5.95 (d, J = 6.0 Hz, 1H), 4.06 (t, J = 6.1 Hz, 2H), 2.95 (s, 3H), 2.74 (t, J = 8.0 Hz, 2H), 2.66 (d, J = 5.6 Hz, 4H), 2.07 - 2.00 (m, 2H), 1.91 - 1.88 (m, 4H). |
| 208 **(Reference)** | LC-MS: (ES, m/z): RT = 1.07min, LCMS07: m/z = 372.10 [M+1]. 1H NMR (400 MHz, Methanol-d4) δ 7.52 (s, 1H), 7.35 - 7.21 (m, 1H), 7.03 (d, J = 8.8 Hz, 1H), 6.10 (s, 1H), 4.21 (t, J = 5.5 Hz, 2H), 3.94 - 3.78 (m, 5H), 3.50 (t, J = 7.0 Hz, 2H), 3.17 (q, J = 8.1 Hz, 2H), 3.04 (s, 3H), 2.40 - 2.16 (m, 7H), 2.11 - 2.06 (m, 2H). |
| 209 **(Reference)** | LC-MS: (ES, m/z): RT = 0.963 min, LCMS 53: m/z = 357.25 [M+1]. 1H NMR (300 MHz, Methanol-d4) δ 7.41 (s, 1H), 7.10 (d, J = 8.4 Hz, 1H), 7.00 - 6.89 (m, 2H), 6.31 (s, 1H), 5.83 (s, 1H), 4.21 (t, J = 5.6 Hz, 2H) 3.91 (s, 3H), 3.88 - 3.77 (m, 2H), 3.49 (t, J = 7.1 Hz, 2H), 3.25 - 3.09 (m, 2H), 2.86 (s, 3H), 2.37 - 2.02 (m, 6H). |
| 210 **(Reference)** | LC-MS: (ES, m/z): RT = 0.918 min, LCMS33: m/z = 374 [M+1]. 1H-NMR: (300 MHz, Methanol-d4) δ 7.70 (d, J = 6.0 Hz, 1H), 7.47 (s, 1H), 7.10 - 7.02 (m, 1H), 6.87 (d, J = 8.7 Hz, 1H), 5.90 (d, J = 6.0 Hz, 1H), 4.07 (t, J = 6.4 Hz, 2H), 3.80 (s, 3H), 3.75 - 3.64 (m, 4H), 2.92 (s, 3H), 2.64 - 2.41 (m, 6H), 2.08 - 1.91 (m, 2H). |
| 211 **(Reference)** | LC-MS: (ES, m/z): RT= 0.997, m/z = 392.20 [M+1]. 1H NMR (300 MHz, Chloroform-d, ppm) δ: 7.87 (s, 1H), 7.40 (d, J = 2.5 Hz, 1H), 7.05 - 7.00 (m, 2H), 6.83 (d, J = 8.7 Hz, 1H), 5.32 (d, J = 5.0 Hz, 1H), 4.11 (t, J = 5.5 Hz, 2H), 3.94 - 3.76 (m, 4H), 3.58 - 3.40 (m, 1H), 3.34 - 3.29 (m, 2H), 3.22 - 2.95 (m, 5H), 2.49 - 2.40 (m, 2H), 2.15 (s, 4H). |
| 212 **(Reference)** | LC-MS: (ES, m/z): RT =0.906 min, LCMS 33: m/z = 344 [M+1]. 1H-NMR-PH-EPI-K-244-0: (300 MHz, Methanol-d4) δ 7.74 (d, J = 6.0 Hz, 1H), 7.54 (d, J = 2.4 Hz, 1H), 7.20 - 7.06 (m, 2H), 6.63 - 6.52 (m, 1H), 5.94 (d, J = 6.0 Hz, 1H), 4.14 - 4.07 (m, 1H), 4.06 - 3.86 (m, 2H), 2.94 (s, 3H), 2.83 - 2.77 (m, 1H), 2.74 - 2.56 (m, 5H), 1.92 - 1.72 (m, 4H). |
| 213 **(Reference)** | LC-MS: (ES, m/z): RT = 1.40 min, LCMS15: m/z = 344 [M+1]. 1H NMR (300 MHz, Chloroform-d) δ 8.03 (s, 2H), 7.24 (d, J = 2.4 Hz, 1H), 7.04 - 7.01 (m, 1H), 6.85 (d, J = 8.7 Hz, 1H), 6.76 (s, 1H), 4.12 (t, J = 6.6 Hz, 2H), 3.86 (s, 3H), 3.29 (s, 2H), 2.72 - 2.67 (m, 6H), 2.16 - 2.07 (m, 2H), 1.94 - 1.78 (m, 4H). |
| 214 **(Reference)** | LC-MS: (ES, m/z): RT = 1.25 min, LCMS53: m/z = 423.2 [M+1]. 1HNMR (300 MHz, Chloroform-d) δ 8.38 (d, J = 5.7 Hz, 1H), 7.29 (d, J = 5.7 Hz, 1H), 7.17 (d, J = 2.4 Hz, 1H), 7.07 (d, J = 2.4 Hz, 1H), 6.87 (d, J = 8.7 Hz, 2H), 6.00 (d, J = 0.9 Hz, 1H), 4.12 (t, J = 6.6 Hz, 2H), 3.86 (s, 3H), 2.68 - 2.49 (m, 9H), 2.30 (s, 3H), 2.12 - 2.04 (m, 2H), 1.79 (s, 4H). |
| 215 **(Reference)** | LC-MS: (ES, m/z): RT = 0.864 min, LCMS07: m/z = 372.1 [M+1]. 1H NMR (400 MHz, Methanol-d4) δ 8.67 (s, 2H), 7.31 (d, J = 2.4 Hz, 1H), 7.19 - 7.17 (m, 1H), 7.07 (d, J = 8.8 Hz, 1H), 4.26 - 4.19 (m, 4H), 3.90 (s, 3H), 3.86 - 3.80 (m, 2H), 3.50 (t, J = 7.2 Hz, 2H), 3.21 - 3.14 (m, 2H), 2.79 (s, 3H), 2.31 - 2.27 (m, 2H), 2.25 - 2.21 (m, 2H), 2.17 - 2.02 (m, 2H). |
| 216 **(Reference)** | LC-MS: (ES, m/z): RT = 1.465min, LCMS07: m/z = 483.15 [M+1]. 1H NMR (400 MHz, Methanol-d4) δ 7.75 (d, J = 6.1 Hz, 1H), 7.33 (d, J = 2.4 Hz, 1H), 7.25 - 7.18 (m, 1H), 6.97 (d, J = 8.7 Hz, 1H), 6.01 (d, J = 6.1 Hz, 1H), 4.49 (d, J = 13.3 Hz, 1H), 4.19 (t, J = 5.5 Hz, 2H), 3.87 (s, 4H), 3.45 (t, J = 6.8 Hz, 6H), 3.30 (d, J = 6.4 Hz, 2H), 3.15 - 3.03 (m, 1H), 2.64 - 2.63 (m, 1H), 2.32 - 2.21 (m, 2H), 2.20 - 2.07 (m, 7H), 1.98 - 1.76 (m, 3H), 1.38 - 1.06 (m, 3H). |
| 217 **(Reference)** | LC-MS: (ES, m/z): RT = 1.25min, LCMS07: m/z = 381.05 [M+1]. 1H NMR (400 MHz, Methanol-d4) δ 7.82 (s, 1H), 7.55 (d, J = 8.7 Hz, 1H), 7.00 (d, J = 8.4 Hz, 1H), 6.94 - 6.82 (m, 4H), 4.18 (t, J = 5.5 Hz, 2H), 3.93 - 3.78 (m, 8H), 3.49 (t, J = 6.9 Hz, 2H), 3.20 - 3.13 (m, 2H), 2.29 - 2.19 (m, 4H), 2.13 - 2.06 (m, 2H). |
| 218 **(Reference)** | LC-MS: (ES, m/z): RT = 0.96 min; LCMS07: m/z = 382.05 [M+1]. 1H NMR (300 MHz, Methanol-d4) δ 9.15 (s, 1H), 7.95 (d, J = 9.1 Hz, 1H), 7.64 (d, J = 2.5 Hz, 1H), 7.36 (dd, J = 8.7, 2.5 Hz, 1H), 7.03 - 6.91 (m, 2H), 4.26 (t, J = 5.5 Hz, 2H), 4.06 (s, 3H), 3.89 - 3.60 (m, 5H), 3.52 (t, J = 7.0 Hz, 2H), 3.27 - 3.11 (m, 2H), 2.40 - 2.03 (m, 6H). |
| 219 **(Reference)** | LC-MS: (ES, m/z): RT = 0.663 min, LCMS 32: m/z = 383 [M+1]. 1H-NMR: (300 MHz, Methanol-d4) δ 8.84 (s, 2H), 7.38 (d, J = 2.4 Hz, 1H), 7.31 - 2.25 (m, 1H), 7.06 (d, J = 8.7 Hz, 1H), 4.23 (t, J = 5.4 Hz, 2H), 3.90 (d, J = 0.9 Hz, 6H), 3.88 - 3.78 (m, 2H), 3.50 (t, J = 7.2 Hz, 2H), 3.20 - 2.13 (m, 2H), 2.36 - 2.27 (m, 2H), 2.27 - 2.16 (m, 2H), 2.15 - 2.00 (m, 2H). |
| 220 **(Reference)** | LC-MS: (ES, m/z): RT = 1.016 min, LCMS 33: m/z = 368 [M+1]. 1H-NMR: (300 MHz, Methanol-d4) δ 8.37 (s, 1H), 7.56 - 7.50 (m, 1H), 7.14 - 6.95 (m, 4H), 6.48 - 6.40 (m, 1H), 4.09 (t, J = 6.3 Hz, 2H), 3.88 (s, 3H), 2.79 - 2.70 (m, 2H), 2.70 - 2.58 (m, 4H), 2.12 - 2.00 (m, 2H), 1.90 - 1.78 (m, 4H). |
| 221 **(Reference)** | LC-MS: (ES, m/z): RT = 1.81 min, LCMS 53: m/z = 381.25 [M+1]. 1H NMR (300 MHz, DMSO-d6) δ 10.80 (s, 1H), 8.09 (s, 1H), 7.61 - 7.44 (m, 1H), 7.21 (s, 1H), 7.04 (dd, J = 9.2, 2.1 Hz, 1H), 6.89 (d, J = 8.6 Hz, 1H), 6.74 (d, J = 2.5 Hz, 1H), 6.66 (dd, J = 8.6, 2.5 Hz, 1H), 4.11 (s, 3H), 4.01 (t, J = 6.0 Hz, 2H), 3.73 (s, 3H), 3.62 - 3.47 (m, 2H), 3.32 - 3.20 (m, 2H), 3.07 - 2.90 (m, 2H), 2.23 - 1.80 (m, 6H). |
| 222 **(Reference)** | LC-MS: (ES, m/z): RT = 1.33 min; LCMS 33:m/z = 381.20 [M+1]. 1H NMR (300 MHz, DMSO-d6) δ 8.16 (s, 1H), 7.07 (d, J = 8.3 Hz, 1H), 6.98 - 6.73 (m, 5H), 4.11 (s, 3H), 4.01 (t, J = 5.8 Hz, 2H), 3.72 (s, 3H), 3.64 - 3.50 (m, 2H), 3.27 (t, J = 7.5 Hz, 2H), 3.06 - 2.91 (m, 2H), 2.17 - 1.99 (m, 4H), 2.00 - 1.87 (m, 2H). |
| 223 **(Reference)** | LC-MS: (ES, m/z): RT = 1.02 min ; LCMS 33: m/z = 382.22 [M+1]. 1H NMR (300 MHz, Methanol-d4) δ 8.01 (d, J = 9.8 Hz, 1H), 7.74 (d, J = 1.3 Hz, 1H), 7.59 (d, J = 2.5 Hz, 1H), 7.46 - 7.31 (m, 2H), 7.04 (d, J = 8.8 Hz, 1H), 4.26 (t, J = 5.5 Hz, 2H), 3.89 - 3.70(m, 5H), 3.52 (t, J = 7.0 Hz, 2H), 3.20 (s, 2H), 2.61 (m, 3H), 2.42 - 2.02 (m, 6H). |
| 224 **(Reference)** | LC-MS: (ES, m/z): RT = 1.02 min, LCMS 33: m/z = 409 [M+1]. 1H NMR (400 MHz, Methanol-d4) δ 8.52 (s, 1H), 8.23 (s, 1H), 8.14 (d, J = 6.7 Hz, 1H), 7.31 (d, J = 6.7 Hz, 1H), 7.22 - 7.12 (m, 3H), 4.23 (t, J = 5.6 Hz, 2H), 4.02 (s, 3H), 3.93 (s, 3H), 3.88-3.80 (m, 2H), 3.50 (t, J = 7.1 Hz, 2H), 3.23 - 3.12 (m, 2H), 2.36 - 2.02 (m, 6H). |
| 225 **(Reference)** | LC-MS: (ES, m/z): RT = 1.13mm, LCMS33: m/z = 438 [M+1]. 1H NMR (400 MHz, Methanol-d4) δ 7.82 (s, 1H), 7.73 (s, 1H), 7.38 - 7.30 (m, 2H), 7.09 (d, J = 8.6 Hz, 3H), 6.93 (t, J = 8.1 Hz, 1H), 6.45 (d, J = 7.2 Hz, 1H), 4.13 (d, J = 5.9 Hz, 2H), 3.93 (s, 3H), 3.87 - 3.76 (m, 2H), 3.46 (t, J = 7.1 Hz, 2H), 3.19-3.12 (m, 2H), 2.31 - 2.16 (m, 4H), 2.16 - 2.03 (m, 2H). |
| 226 **(Reference)** | LC-MS: (ES, m/z): RT = 1.032 min, LCMS 53: m/z = 441.35 [M+1]. 1H NMR (300 MHz, Deuterium Oxide) δ 7.70 - 7.31 (m, 2H), 7.30 - 7.09 (m, 2H), 6.13 (d, J = 6.1 Hz, 1H), 4.01 - 3.81 (m, 5H), 3.72 - 3.52 (m, 2H), 3.52 - 3.16 (m, 8H), 3.12 - 2.91 (m, 2H), 2.24 - 1.75 (m, 7H), 1.73 - 1.44 (m, 2H), 1.38 - 1.02 (m, 2H). |
| 227 **(Reference)** | LC-MS: (ES, m/z): RT = 1.00min, LCMS07: m/z = 442.27 [M+1]. 1H NMR (300 MHz, Methanol-d4) δ 8.22 (d, J = 3.0 Hz, 1H), 7.74 (d, J = 6.0 Hz, 1H), 6.88 (d, J = 8.8 Hz, 1H), 6.49 (dd, J = 8.8, 3.0 Hz, 1H), 5.98 (d, J = 6.0 Hz, 1H), 4.12 - 3.86 (m, 7H), 3.49 - 3.33 (m, 4H), 2.78 - 2.58 (m, 6H), 2.09 - 1.93 (m, 3H), 1.94 - 1.74 (m, 6H), 1.46 - 1.25 (m, 2H). |
| 228 **(Reference)** | LC-MS: (ES, m/z): RT = 0.918 min, LCMS 53: m/z = 441.35 [M+1]. 1H NMR (300 MHz, Methanol-d4) δ 8.05 - 7.92 (m, 1H), 7.69 (d, J = 7.3 Hz, 1H), 7.53 - 7.23 (m, 2H), 6.39-6.60 (m, 1H), 4.09 - 3.89 (m, 5H), 3.79 - 3.68 (m, 2H),3.63 - 3.51 (m, 2H), 3.50 - 3.36 (m, 6H), 3.22 - 3.07 (m, 2H), 2.44 - 1.85 (m, 7H), 1.85 - 1.62 (m, 2H), 1.49 - 1.25 (m, 2H). |
| 229 **(Reference)** | LC-MS: (ES, m/z): RT = 0.96 min, LCMS 53: m/z = 376.2 [M+1]. 1H NMR (300 MHz, Chloroform-d) δ 7.92 - 7.83 (m, 1H), 7.49 - 7.35 (m, 2H), 7.00 (d, J = 8.7 Hz, 1H), 6.84 (d, J = 8.7 Hz, 1H), 5.87 (d, J = 6.1 Hz, 1H), 5.13 (s, 2H), 4.16 (t, J = 6.5 Hz, 2H), 3.86 (s, 3H), 3.13 - 2.88 (m, 6H), 2.82 (t, J = 7.3 Hz, 2H), 2.72 - 2.62 (m, 1H), 2.36 - 2.00 (m, 4H). |
| 230 **(Reference)** | LC-Ms: (ES, m/z): RT = 0.94 min, LCMS 53: m/z = 362.2 [M+1]. 1H NMR (300 MHz, Chloroform-d) δ 7.91 (d, J = 6.0 Hz, 1H), 7.40 (d, J = 2.4 Hz, 1H), 7.10 - 6.95 (m, 2H), 6.84 (d, J = 8.7 Hz, 1H), 5.86 (d, J = 6.0 Hz, 1H), 5.10 - 4.92 (m, 2H), 4.10 (t, J = 6.6 Hz, 2H), 3.86 (s, 3H), 3.79 - 3.62 (m, 2H), 3.28 - 3.05 (m, 2H), 2.99 (d, J = 5.1 Hz, 3H), 2.71 (t, J = 7.2 Hz, 2H), 2.06 - 1.89 (m, 2H). |
| 231 **(Reference)** | LC-MS: (ES, m/z): RT = 1.35min, LCMS 07: m/z = 358 [M+1]. 1H NMR (400 MHz, Methanol-d4) δ 7.70 (d, J = 7.2 Hz, 1H), 7.54 (s, 1H), 7.37 - 7.29 (m, 1H), 7.13 - 6.89 (m, 1H), 6.30 (d, J = 7.2 Hz, 1H), 4.21 (t, J = 8.2, 2H), 3.93 - 3.78 (m, 5H), 3.50 (t, J = 7.0 Hz, 2H), 3.24 - 3.12 (m, 2H), 3.03 (s, 3H), 2.36 - 2.16 (m, 4H), 2.15 - 2.05 (m, 2H). |
| 232 | LC-MS: (ES, m/z): RT = 0.987 min, LCMS 33: m/z =360 [M+1]. 1H NMR (300 MHz, Methanol-d4) δ7.51-7.69 (m, 1H),7.20 (t, J = 10.2 Hz, 1H), 6.78 - 6.92 (m, 1H), 6.08 (s, 1H), 4.14 (t, J = 5.8 Hz, 2H), 3.78-3.62 (m, 2H), 3.43-3.35 (m,2H), 3.16 - 3.02(m, 2H), 3.00 (s, 3H), 2.34 (s, 3H), 2.32 - 2.17 (m, 4H), 2.11 - 1.98 (m, 2H). |
| 233 **(Reference)** | LC-MS: RT= 1.076, m/z= 383.25 [M+1]. 1H NMR (300 MHz, Methanol-d4, ppm) δ: 8.37 (s, 1H), 7.21 - 7.10 (m, 3H), 4.21 (t, J = 5.5 Hz, 2H), 3.97 (s, 3H), 3.91 - 3.79 (m, 2H), 3.49 (t, J = 7.1 Hz, 2H), 3.24 - 3.09 (m, 5H), 2.34 - 2.15 (m, 6H). |
| 234 **(Reference)** | LC-MS: (ES, m/z): RT = 1.07 min, LCMS 33: m/z = 426 [M+1]. 1H NMR (400 MHz, Methanol-d4) δ 8.15 (s, 1H), 7.41 - 6.89 (m, 3H), 4.21 (t, J = 5.5 Hz, 2H), 3.91 (s, 3H), 3.90-3.80 (m, 2H), 3.49 (t, J = 7.1 Hz, 2H), 3.23 - 3.08 (m, 5H), 2.36 - 2.17 (m, 4H), 2.15 - 2.05 (m, 2H). |
| 235 **(Reference)** | LC-MS: (ES, m/z): RT = 1.01 min, LCMS 07: m/z = 426 [M+1]. 1H NMR (300 MHz, Methanol-d4) δ 8.51 - 7.92 (m, 1H), 7.21 - 7.03 (m, 3H), 4.19 (t, J = 5.5 Hz, 2H), 3.95 - 3.76 (m, 5H), 3.49 (t, J = 7.1 Hz, 2H), 3.20 - 3.10 (m, 2H), 2.93 (s, 3H), 2.37 - 2.14 (m, 4H), 2.10 - 1.98 (m, 2H). |
| 236 | LC-MS: RT= 1.035, m/z = 390.30 [M+1]. 1H NMR (300 MHz, Methanol-d4, ppm) δ: 7.54 (d, J = 2.5 Hz, 1H), 7.08 (dd, J = 8.7, 2.5 Hz, 1H), 6.88 (d, J = 8.7 Hz, 1H), 4.10 (t, J = 6.1 Hz, 2H), 3.82 (s, 3H), 3.00 (s, 3H), 2.89 - 2.70 (m, 6H), 2.21 (d, J = 2.9 Hz, 3H), 2.12 - 2.03 (m, 2H), 1.93 - 1.87 (m, 4H). |
| 237 **(Reference)** | LC-MS: (ES, m/z): RT = 0.938 min, LCMS 28: m/z = 360.15 [M+1]. 1H NMR (300 MHz, Methanol-d4) δ 7.58 (d, J = 7.3 Hz, 1H), 7.32 - 7.19 (m, 2H), 7.11 (d, J = 8.8 Hz, 1H), 6.17 (d, J = 7.3 Hz, 1H), 4.50 - 4.36 (m, 2H), 4.22 - 3.98 (m, 2H), 3.92 - 3.72 (m, 5H), 3.72 - 3.62 (m, 4H), 3.43 - 3.30 (m, 2H), 3.03 (s, 3H). |
| 238 | LC-MS: (ES, m/z): RT = 1.356 min, LCMS 07: m/z = 426 [M+1]. 1H-NMR: (400 MHz, Methanol-d4) δ 7.69 (s, 1H), 7.13 - 7.05 (m, 1H), 6.89 (d, J = 8.8 Hz, 1H), 6.23 (s, 1H), 4.10 (t, J = 6.2 Hz, 2H), 3.82 (s, 3H), 2.98 (s, 3H), 2.78 - 2.68 (m, 2H), 2.64 (q, J = 4.8 Hz, 4H), 2.8 - 2.00 (m, 2H), 1.91 - 1.78 (m, 4H). |
| 239 **(Reference)** | LC-MS: (ES, m/z): RT= 0.911 min, LCMS33: m/z = 341 [M+1]. 1H NMR (300 MHz, Methanol-d4) δ 8.53 (s, 1H), 7.59 (d, J = 7.2 Hz, 1H), 7.32 (d, J = 7.8 Hz, 1H), 7.20 - 7.16 (m, 1H), 6.19 (d, J = 7.2 Hz, 1H), 4.23 (t, J = 8.4 Hz, 2H), 3.52 (t, J = 6.3 Hz, 2H), 3.28 (t, J = 8.1 Hz, 2H), 3.13 - 2.99 (m, 5H), 2.97 (s, 6H). |
| 240 | LC-MS: (ES, m/z): RT = 1.044 min, LCMS07: m/z = 386.15 [M+1]. 1H NMR (400 MHz, Methanol-d4) δ 7.61 (s, 1H), 7.08 - 7.05 (m, 1H), 6.89 (d, J = 8.8 Hz, 1H), 5.81 (s, 1H), 4.10 (t, J = 6.0 Hz, 2H), 3.82 (s, 3H), 2.93 (s, 3H), 2.80 - 2.71 (m, 2H), 2.71 - 2.61 (m, 4H), 2.47 (q, J = 7.6 Hz, 2H), 2.12 - 2.00 (m, 2H), 1.92 - 1.80 (m, 4H), 1.25 (t, J = 7.6 Hz, 3H). |
| 241 **(Reference)** | LC-MS: (ES, m/z): RT = 1.17 min; LCMS 33: m/z = 464.3 [M+1]. 1H NMR (300 MHz, Methanol-d4) δ 8.43 (d, J = 5.1 Hz, 1H), 7.63 - 7.44 (m, 5H), 7.39 (d, J = 2.5 Hz, 1H), 7.26 (dd, J = 8.7, 2.5 Hz, 1H), 7.01 (d, J = 8.8 Hz, 1H), 6.82 (d, J = 5.1 Hz, 1H), 4.35 (s, 2H), 4.26 (t, J = 5.6 Hz, 2H), 4.07 (dd, J = 12.0, 4.6 Hz, 2H), 3.84 (s, 3H), 3.69 (t, J = 6.8 Hz, 2H), 3.53 - 3.40 (m, 3H), 2.29 - 2.21 (m, 2H), 2.14 - 2.05 (m, 2H), 1.80 - 1.65 (m, 2H). |
| 242 **(Reference)** | LC-MS: (ES, m/z): RT = 1.067 min, LCMS 33: m/z = 483 [M+1]. 1H NMR (300 MHz, Deuterium Oxide) δ 7.87 (d, J = 2.4 Hz, 1H), 7.04 (d, J = 8.4 Hz, 1H), 6.93 - 6.82 (m, 2H), 6.12 (d, J = 2.5 Hz, 1H), 4.27 (d, J = 12.9 Hz, 1H), 4.08 (t, J = 5.6 Hz, 2H), 3.92 - 3.58 (m, 6H), 3.34 (t, J = 7.5 Hz, 2H), 3.16 - 2.95 (m, 5H), 2.59 (dd, J = 14.1, 11.3 Hz, 1H), 2.25 - 1.60 (m, 12H), 1.20 - 0.90 (m, 2H). |
| 243 **(Reference)** | LC-MS: (ES, m/z): UFLC 06:RT = 6.901 min, LCMS 53: m/z = 440.3 [M+1]. |
| | 1H NMR (300 MHz, Deuterium Oxide) δ 7.42 (d, J = 7.3 Hz, 1H), 7.00 (d, J = 1.6 Hz, 2H), 6.85 - 6.71 (m, 1H), 6.05 (dd, J = 7.4, 1.7 Hz, 1H), 4.64 - 4.56 (m, 1H), 4.33 (t, J = 12.7, 2.3 Hz, 2H), 4.08 - 3.97 (m, 2H), 3.96 - 3.82 (m, 2H), 3.77 (s, 3H), 3.46 - 3.25 (m, 2H), 3.25 - 3.12 (m, 2H), 2.89 - 2.72 (m, 5H), 2.46 - 2.31 (m, 2H), 1.88 - 1.72 (m, 1H), 1.50 (d, J = 13.2 Hz, 2H), 1.25 - 1.06 (m, 2H). |
| 244 **(Reference)** | LC-MS: (ES, m/z): RT = 0.95min, LCMS33: m/z = 346 [M+1]. 1H NMR (300 MHz, Methanol-d4) δ 7.66 (d, J = 7.3 Hz, 1H), 7.45 (dd, J = 6.3, 3.0 Hz, 1H), 7.22 (m, 1H), 6.93 (m, J = 9.2, 3.5 Hz, 1H), 6.25 (d, J = 7.3 Hz, 1H), 4.14 (t, J = 5.8 Hz, 2H), 3.72 (d, J = 5.8 Hz, 2H), 3.50 - 3.33 (m, 2H), 3.15 (t, J = 13.0 Hz, 2H), 3.02 (s, 3H), 2.24 (m, 4H), 2.16 - 2.02 (m, 2H). |
| 245 **(Reference)** | LC-MS: (ES, m/z): RT = 1.004 min, LCMS 07: m/z = 344.15 [M+1]. 1H NMR (300 MHz, Methanol-d4) δ 7.65 (d, J = 7.3 Hz, 1H), 7.16 (s, 1H), 7.07 (d, J = 1.3 Hz, 2H), 6.20 (d, J = 7.3 Hz, 1H), 4.21 (t, J = 5.5 Hz, 2H), 3.90 (s, 3H), 3.81 (s, 2H), 3.49 (t, J = 7.1 Hz, 2H), 3.29 - 3.02 (m, 2H), 2.34 - 2.01 (m, 6H). |
| 246 **(Reference)** | LC-MS: (ES, m/z): RT = 0.907 min, LCMS 07: m/z = 358.05 [M+1]. 1H NMR (300 MHz, Methanol-d4) δ 8.01 (d, J = 2.5 Hz, 1H), 7.25 - 7.14 (m, 1H), 6.94 (d, J = 1.3 Hz, 2H), 5.96 (d, J = 2.4 Hz, 1H), 4.09 (t, J = 6.1 Hz, 2H), 3.84 (s, 3H), 2.78 (s, 5H), 2.70 (s, 4H), 2.12 - 1.99 (m, 2H), 1.90 - 1.80 (m, 4H). |
| 247 **(Reference)** | LC-MS: (ES, m/z): RT = 1.41 min, LCMS 07: m/z = 358 [M+1]. 1H NMR (300 MHz, Methanol-d4) δ 7.44 (d, J = 2.5 Hz, 1H), 7.30 - 7.13 (m, 3H), 7.07 - 6.98 (m, 1H), 4.22 (t, J = 5.5 Hz, 2H), 4.02 - 3.77 (m, 5H), 3.50 (t, J = 7.0 Hz, 2H), 3.22-3.12 (m, 2H), 3.03 (s, 3H), 2.37 - 2.02 (m, 6H). |
| 248 **(Reference)** | LC-MS: (ES, m/z):RT = 0.871 min, LCMS 07: m/z = 358 [M+1]. 1H NMR (300 MHz, Methanol-d4) δ 8.03 (s, 1H), 7.77 (d, J = 6.9 Hz, 1H), 7.27 (s, 1H), 6.81 (s, 1H), 6.73 (d, J = 7.2 Hz, 1H), 4.53 (s, 2H), 4.00 (s, 3H), 3.89 - 3.75 (m, 2H), 3.49 (t, J = 7.0 Hz, 2H), 3.18 - 3.10 (m, 2H), 3.00 (s, 3H), 2.40 - 2.39 (m, 2H), 2.30 - 2.06 (m, 4H). |
| 249 **(Reference)** | LC-MS: (ES, m/z): RT = 0.577 min, LCMS 30: m/z = 368.2 [M+1]. 1H NMR (300 MHz, Methanol-d4) δ 8.30 (s, 1H), 7.56 (d, J = 7.1 Hz, 1H), 7.26 - 7.07 (m, 4H), 4.23 (t, J = 5.6 Hz, 2H), 3.96 (s, 3H), 3.82 (s, 2H), 3.49 (t, J = 7.2 Hz, 2H), 3.21 - 3.15 (m, 2H), 2.38 - 2.23 (m, 2H), 2.21 - 2.14 (m, 4H). |
| 250 **(Reference)** | LC-MS: (ES, m/z): RT = 0.974 min, LCMS 33: m/z = 367.21 [M+1]. 1H NMR (300 MHz, Methanol-d4) δ 7.90 (d, J = 7.1 Hz, 1H), 7.30 (d, J = 3.6 Hz, 1H), 7.14 (d, J = 8.2 Hz, 1H), 7.04 (d, J = 8.2 Hz, 2H), 6.81 - 6.69 (m, 2H), 4.21 (t, J = 5.5 Hz, 2H), 3.94 (s, 3H), 3.90 - 3.76 (m, 2H), 3.49 (t, J = 7.1 Hz, 2H), 3.25 - 3.10 (m, 2H), 2.37 - 2.04 (m, 6H) |
| 251 **(Reference)** | LC-MS: (ES, m/z): RT = 1.07 min, LCMS 53: m/z = 374.2 [M+1]. 1H NMR (300 MHz, Chloroform-d) δ 7.94 (d, J = 5.7 Hz, 1H), 7.40 (t, J = 2.2 Hz, 1H), 7.17 (t, J = 8.1 Hz, 1H), 7.08 - 7.04 (m, 1H), 6.99 (s, 1H), 6.55 - 6.51 (m, 1H), 5.84 (d, J = 5.7 Hz, 1H), 5.30 - 5.05 (m, 1H), 4.66 (d, J = 7.8 Hz, 1H), 4.06 (t, J = 6.3 Hz, 3H), 3.03 - 2.64 (m, 5H), 2.57 - 2.42 (m, 1H), 2.32 - 1.95 (m, 4H), 1.28 (d, J = 6.3 Hz, 6H). |
| 252 **(Reference)** | LC-MS: (ES, m/z): RT = 0.943 min, LCMS 28: m/z = 344 [M+1]. 1H-NMR: (300 MHz, Methanol-d4) δ 7.74 (d, J = 6.0 Hz, 1H), 7.53 (d, J = 2.4 Hz, 1H), 7.22 - 7.09 (m, 2H), 6.64 - 6.52 (m, 1H), 5.94 (d, J = 6.0 Hz, 1H), 4.18-4.06 (m, 1H), 4.04 - 3.89 (m, 2H), 2.94 (s, 3H), 2.84 - 2.78 (m, 1H), 2.77 - 2.57 (m, 5H), 1.96 - 1.74 (m, 4H). |
| 253 **(Reference)** | LC-MS: (ES, m/z): RT = 0.943 min, LCMS 28: m/z = 344 [M+1]. 1H-NMR: (300 MHz, Methanol-d4) δ 7.74 (d, J = 5.7 Hz, 1H), 7.53 (d, J = 2.4 Hz, 1H), 7.22 - 7.05 (m, 2H), 6.61 - 6.52 (m, 1H), 5.94 (d, J = 6.0 Hz, 1H), 4.19 - 4.06 (m, 1H), 4.05 - 3.90 (m, 2H), 2.94 (s, 3H), 2.84 - 2.96 (m, 1H), 2.76 - 2.57 (m, 5H), 1.93 - 1.75 (m, 4H). |
| 256 **(Reference)** | LC-MS: (ES, m/z): RT = 0.83min, LCMS33: m/z = 414 [M+1]. 1H NMR (300 MHz, Methanol-d4) δ 7.75 (d, J = 6.7 Hz, 1H), 7.21 (s, 1H), 7.16 - 7.04 (m, 2H), 6.41 (d, J = 6.5 Hz, 1H), 4.39-4.36 (m, 1H), 4.22 (t, J = 5.5 Hz, 2H), 3.99-3.92 (m, 4H), 3.89 - 3.64 (m, 5H), 3.51-3.42 (m, 3H), 3.27 - 3.09 (m, 2H), 2.60-2.07 (m, 7H). |
| 257 **(Reference)** | LC-MS: (ES, m/z): RT = 1.22min, LCMS 33: m/z = 382 [M+1]. 1H NMR (300 MHz, Methanol-d4) δ 8.20 (s, 1H), 8.05 (d, J = 9.1 Hz, 1H), 7.55 (d, J = 2.5 Hz, 1H), 7.30 (dd, J = 8.7, 2.4 Hz, 1H), 7.06 (d, J = 8.7 Hz, 1H), 6.81 (d, J = 9.1 Hz, 1H), 4.25 (t, J = 5.5 Hz, 2H), 4.09 (s, 3H), 3.90 (s, 5H), 3.50 (t, J = 7.0 Hz, 2H), 3.25 - 3.09 (m, 2H), 2.39 - 2.04 (m, 6H). |
| 258 **(Reference)** | LC-MS: (ES, m/z): RT = 0.871 min, LCMS 33: m/z = 382 [M+1]. 1H NMR (Methanol-d4, ppm): δ 8.75 (s, 1H), 8.59 (s, 1H), 7.19 - 6.98 (m, 4H), 4.23 (t, J = 5.6 Hz, 2H), 3.98 - 3.76 (m, 8H), 3.49 (t, J = 7.1 Hz, 2H), 3.25 - 3.10 (m, 2H), 2.38 - 2.02 (m, 6H). |
| 259 **(Reference)** | LC-MS: (ES, m/z): RT= 1.801 min, LCMS 31, m/z = 482.5 [M+1]. 1H NMR (300 MHz, Methanol-d4) δ 7.57 (d, J = 7.3 Hz, 1H), 7.09 (d, J = 17.2 Hz, 3H), 6.17 (d, J = 7.3 Hz, 1H), 4.53 - 4.24 (m, 1H), 4.07 - 3.82 (m, 5H), 3.82 - 3.50 (m, 2H), 3.49 - 3.35 (m, 4H), 3.30 - 3.15 (m, 1H), 2.96 (d, J = 4.1 Hz, 4H), 2.25 - 1.18 (m, 15H). |
| 260 **(Reference)** | LC-MS: (ES, m/z): RT = 1.069 min; m/z = 468.35 [M+1]. 1H NMR (300 MHz, Deuterium Oxide) δ 7.37 (d, J = 7.3 Hz, 1H), 6.99 - 6.84 (m, 2H), 6.82 - 6.70 (m, 1H), 5.97 (d, J = 7.5 Hz, 1H), 4.15 - 3.92 (m, 3H), 3.88 - 3.61 (m, 7H), 3.37 - 3.13 (m, 2H), 3.12 - 2.98 (m, 2H), 2.81 (s, 3H), 2.05 - 1.81 (m, 2H), 1.85- 1.23 (m, 9H), 1.22 - 0.91 (m, 2H). |
| 261 | LC-MS: (ES, m/z): RT = 0.720 min, LCMS 32: m/z = 388 [M+1]. 1H NMR (300 MHz, Methanol-d4) δ 7.56 (d, J = 2.4 Hz, 1H), 7.10-7.06 (m, 1H), 6.86 (d, J = 8.7 Hz, 1H), 5.25 (s, 1H), 4.08 (t, J = 6.4 Hz, 2H), 3.85 (s, 3H), 3.80 (s, 3H), 2.87 (s, 3H), 2.77 - 2.68 (m, 2H), 2.62 (d, J = 5.7 Hz, 4H), 2.08-2.00 (m, 2H), 1.88-1.80 (m, 4H). |
| 262a **(Reference)** | LC-MS: (ES, m/z): RT = 0.964 min ; m/z = 334 [M+1]. 1H NMR (300 MHz, Methanol-d4) δ 7.59 (d, J = 6.2 Hz, 1H), 5.91 (d, J = 6.6 Hz, 1H), 3.91 (d, J = 8.6 Hz, 1H), 3.73 - 3.39 (m, 3H), 3.35 - 3.18 (m, 4H), 2.92 (s, 3H), 2.38 - 2.25 (m, 1H), 2.17 - 1.78 (m, 10H), 1.50 - 1.20 (m, 5H). |
| 262b **(Reference)** | LC-MS: (ES, m/z): RT = 0.964 min; m/z = 334 [M+1]. 1H NMR (300 MHz, Methanol-d4) δ 7.59 (d, J = 6.2 Hz, 1H), 5.87 (d, J = 6.6 Hz, 1H), 3.91 - 3.69 (m, 1H), 3.73 - 3.39 (m, 3H), 2.85 (s, 3H), 2.78 - 2.61 (m, 6H), 2.32 - 2.21 (m, 1H), 2.00 - 1.75 (m, 9H), 1.50 - 1.11 (m, 4H). |
| 263 | LC-MS: (ES, m/z): RT = 1.07min, LCMS07: m/z = 408.15[M+1]. 1H NMR (400 MHz, Methanol-d4) δ 8.12-8.09 (m,1H), 7.84-7.80 (m, 1H), 7.61 - 7.53 (m, 1H), 7.48-7.46 (m, 1H), 7.25 (s, 1H), 7.20 - 7.07 (m, 2H), 4.22 (t, J = 5.5 Hz, 2H), 3.93 (s, 3H), 3.89 - 3.78 (m, 2H), 3.50 (t, J = 7.1 Hz, 2H), 3.19-3.14 (m, 5H), 2.36 - 2.16 (m, 4H), 2.10-2.07 (m, 2H). |
| 264 **(Reference)** | LC-MS: (ES, m/z): RT=0.695min, LCMS 40, m/z =367 [M+1]. 1H NMR (300 MHz, Methanol-d4) δ 8.35 (d, J = 2.4 Hz, 1H), 7.75 - 7.60 (m, 2H), 7.39 (d, J = 8.4 Hz, 1H), 6.22 (d, J = 7.3 Hz, 1H), 3.96 (t, J = 6.1 Hz, 2H), 3.90 - 3.80 (m, 2H), 3.73 (t, J = 6.7 Hz, 2H), 3.53 (t, J = 6.0 Hz, 2H), 3.27 - 3.08 (m, 4H), 3.06 (s, 3H), 2.29 - 1.98 (m, 4H). |
| 265 **(Reference)** | LC-MS: (ES, m/z): RT = 2.07min, LCMS07: m/z = 366.20 [M+1]. 1H NMR (300 MHz, DMSO-d6) δ 10.96 (s, 1H), 9.38 (s, 1H), 7.70 (s, 1H), 7.31 - 7.15 (m, 1H), 6.90 (dd, J = 7.7, 3.0 Hz, 3H), 6.74 (d, J = 2.5 Hz, 1H), 6.68 - 6.65 (m, 1H), 6.55 (t, J = 2.5 Hz, 1H), 3.99 (t, J = 5.8 Hz, 2H), 3.74 (s, 3H), 3.62 (s, 2H), 3.34-3.26 (m, 2H), 3.06-3.03 (m, 2H), 2.19 - 1.98 (m, 4H), 1.93 - 1.79 (m, 2H). |
| 266 **(Reference)** | LC-MS: (ES, m/z): RT = 1.021 min, LCMS 33: m/z = 367 [M+1]. 1H NMR (300 MHz, Methanol-d4) δ 7.51 - 7.38 (m, 2H), 7.24 - 7.04 (m, 4H), 6.92 (d, 1H), 4.20 (t, J = 5.5 Hz, 2H), 3.95 (s, 3H), 3.82 (s, 2H), 3.49 (t, J = 7.2 Hz, 2H), 3.17 (t, J = 13.2 Hz, 2H), 2.36 - 2.02 (m, 6H). |
| 267 **(Reference)** | LC-MS: (ES, m/z): RT= 1.04 min, LCMS 45: m/z = 328 [M+1]. 1H-NMR: (Methanol-d4, ppm): δ 7.88-7.86 (m, 2H), 7.29 (d, J = 7.6 Hz, 1H), 7.00 (d, J = 8.3 Hz, 1H), 6.52 (s, 1H), 6.11 (s, 1H), 4.83 (s, 2H), 4.01 - 3.91 (m, 2H), 3.84-3.68 (m, 2H), 3.59 - 3.49 (m, 2H), 3.28 - 3.13 (m, 2H), 2.95 (s, 3H), 2.25 - 2.01 (m, 4H). |
| 268 **(Reference)** | LC-MS: (ES, m/z): RT= 0.94 min, LCMS 28: m/z = 328 [M+1]. 1H-NMR: (Methanol-d4, ppm): δ 7.71 (d, J = 6.0 Hz, 1H), 7.62-7.58 (m, 1H), 7.25 (d, J = 2.0 Hz, 1H), 6.98 (dd, J = 7.4, 1.1 Hz, 1H), 6.79 (d, J = 8.3 Hz, 1H), 6.69 (dd, J = 6.0, 2.0 Hz, 1H), 4.59 (s, 2H), 3.81-3.63 (m, 2H), 2.87 (s, 3H), 2.85-2.72 (m, 2H), 2.70 - 2.58 (m, 4H), 1.85-1.68 (m, 4H). |
| 272 **(Reference)** | LC-MS: (ES, m/z): RT = 0.985 min, LCMS 07: m/z = 375 [M+1]. 1H NMR (300 MHz, Methanol-d4) δ 7.62 (d, J = 5.8 Hz, 1H), 7.11 (d, J = 8.4 Hz, 1H), 7.02 - 6.90 (m, 2H), 5.96 (d, J = 7.2 Hz, 1H), 4.20 (t, J = 5.6 Hz, 2H), 3.91 (s, 3H), 3.85-3.75 (m, 2H), 3.49 (t, J = 7.1 Hz, 2H), 3.25 - 3.09 (m, 2H), 2.92 (s, 3H), 2.37 - 2.02 (m, 6H). |
| 276 **(Reference)** | LC-MS: (ES, m/z): RT=1.529 min, LCMS 33, m/z =346.0 [M+1]. 1H NMR (300 MHz, Deuterium Oxide) δ 7.49 (d, J = 7.4 Hz, 1H), 7.43 - 7.34 (m, 1H), 7.25 - 7.05 (m, 2H), 3.95 - 6.88 (m, 1H), 6.36 - 5.98 (m, 1H), 5.13 - 4.98 (m, 1H), 4.96 - 4.80 (m, 1H), 4.57 - 4.33 (m, 2H), 4.10 - 3.90 (m, 1H), 3.89 - 3.59 (m, 2H), 3.47 - 3.22 (m, 2H), 2.94 (s, 3H), 2.28 - 1.83 (m, 4H). |
| 277 **(Reference)** | LC-MS: (ES, m/z): RT = 1.348 min, LCMS 27: m/z = 364 [M+1]. 1H-NMR: (Methanol-d4, ppm): δ 7.64 (d, J = 7.3 Hz, 1H), 7.46 - 7.27 (m, 3H), 6.97 - 6.84 (m, 1H), 6.22 (d, J = 7.3 Hz, 1H), 4.48 (t, J = 12.2 Hz, 2H), 4.23 - 4.06 (m, 2H), 3.78 - 3.33 (m, 4H), 3.06 (s, 3H), 2.17 (s, 4H). |
| 279 **(Reference)** | LC-MS: (ES, m/z): RT=1.221min, LCMS 30, m/z =342 [M+1]. 1H NMR (400 MHz, Methanol-d4) δ 7.68 - 7.58 (m, 3H), 7.53 - 7.40 (m, 1H), 7.30 - 7.20 (m, 1H), 6.21 (d, J = 7.3 Hz, 1H), 4.58 (q, J = 6.4 Hz, 1H), 3.75 - 3.54 (m, 4H), 3.48 - 3.34 (m, 2H), 3.22 - 2.99 (m, 5H), 2.20 - 1.99 (m, 4H), 1.53 (d, J = 6.5 Hz, 3H). |
| 280 **(Reference)** | LC-MS: (ES, m/z): RT=0.969min, LCMS 15, m/z =360 [M+1].1H NMR (300 MHz, Methanol-d4) δ 7.57 (d, J = 7.3 Hz, 1H), 7.22 (s, 1H), 7.10 (d, J = 1.9 Hz, 2H), 6.19 (d, J = 7.3 Hz, 1H), 4.21 (t, J = 5.6 Hz, 2H), 3.91 (s, 3H), 3.51 - 3.28 (m, 6H), 3.03 (s, 3H), 2.36 - 2.20 (m, 2H), 1.39 (t, J = 7.3 Hz, 6H). |
| 283 **(Reference)** | LC-MS: (ES, m/z): RT=0.651min, LCMS 07, m/z =388.4 [M+1]. 1H NMR (300 MHz, Methanol-d4) δ 7.72 (d, J = 6.1 Hz, 1H), 7.52 - 7.43 (m, 1H), 7.08 (dd, J = 8.7, 2.5 Hz, 1H), 6.88 (d, J = 8.7 Hz, 1H), 5.91 (d, J = 6.0 Hz, 1H), 4.18 - 3.90 (m, 3H), 3.81 (s, 3H), 3.29 (s, 3H), 2.93 (s, 3H), 2.83 - 2.48 (m, 6H), 2.18 - 1.94 (m, 3H), 1.88 - 1.76 (m, 1H). |
| 285 **(Reference)** | LC-MS: (ES, m/z): RT = 0.57min, LCMS48: m/z = 426 [M+1]. 1H NMR (400 MHz, Methanol-d4) δ 7.56 (d, J = 7.3 Hz, 1H), 7.20 (s, 1H), 7.04-7.10 (m, 2H), 6.17 (d, J = 7.3 Hz, 1H), 4.19 (t, J = 5.4 Hz, 3H), 3.99 - 3.87 (m, 5H), 3.69-3.42 (m, 4H), 3.03 (s, 3H), 2.51 - 2.26 (m, 4H). |
| 286 **(Reference)** | LC-MS: (ES, m/z): RT = 0.934 min, LCMS 07: m/z = 374 [M+1]. 1H NMR (300 MHz, Methanol-d4) δ 7.72 (d, J = 5.9 Hz, 1H), 7.47 (d, J = 2.3 Hz, 1H), 7.08 (dd, J = 8.7, 2.5 Hz, 1H), 6.89 (d, J = 8.7 Hz, 1H), 5.91 (d, J = 6.0 Hz, 1H), 4.40-4.30 (m, 1H), 4.14 - 4.03 (m, 2H), 3.82 (s, 3H), 2.97 - 2.47 (m, 9H), 2.24 - 1.92 (m, 3H), 1.80-1.70 (m, 1H). |
| 287 **(Reference)** | LC-MS: (ES, m/z): RT = 1.021 min, LCMS 33: m/z = 383 [M+1]. 1H NMR (300 MHz, Methanol-d4) δ 7.56 (d, 1H), 7.20 (s, 1H), 7.09 (d, 2H), 6.18 (d, 1H), 4.35-4.10(m, 3H), 4.10-3.92 (m, 4H), 3.90-3.48 (s, 5H), 3.03 (s, 3H), 2.87-2.36 (s, 2H), 2.32 (s, 2H). |
| 288 **(Reference)** | LC-MS: (ES, m/z): RT = 2.22 min, LCMS 27: m/z = 358 [M+1]. 1H NMR (300 MHz, Methanol-d4) δ 7.80-7.70 (m, 1H), 7.50 (s, 1H), 7.10 - 7.00 (m, 1H), 6.90 (d, J = 8.7 Hz, 1H), 5.96 (d, J = 7.2 Hz, 1H), 4.15-4.05 (m, 2H), 3.80 (s, 3H), 3.15-3.05 (m,1H), 2.95 (s, 3H), 2.50 - 2.40 (m, 4H), 2.35 - 2.20 (m, 2H), 2.20 - 2.10 (m, 1H), 1.85 - 1.55 (m, 4H). |
| 289 **(Reference)** | LC-MS: (ES, m/z): RT = 0.92min, LCMS07: m/z = 344.10 [M+1]. 1H NMR (300 MHz, Methanol-d4) δ 7.72 (s, 1H), 7.52 (s, 1H), 7.08 (dd, J = 8.7, 2.5 Hz, 1H), 6.90 (d, J = 8.7 Hz, 1H), 5.92 (d, J = 6.0 Hz, 1H), 4.04 (d, J = 5.8 Hz, 2H), 3.82 (s, 3H), 3.21 - 3.08 (m, 1H), 2.94 (s, 3H), 2.94-2.83(m, 1H), 2.59 (d, J = 1.3 Hz, 3H), 2.40 (q, J = 9.0 Hz, 1H), 2.19 - 2.06 (m, 1H), 1.90-1.82 (m, 2H), 1.77-1.68 (m, 1H). |
| 290 **(Reference)** | 1H NMR (300 MHz, Methanol-d4) δ 7.56 (d, J = 7.3 Hz, 1H), 7.29 - 7.18 (m, 1H), 7.09 (d, J = 1.0 Hz, 2H), 6.18 (d, J = 7.3 Hz, 1H), 4.30 - 4.12 (m, 2H), 3.94 - 3.49 (m, 6H), 3.30-3.20(m, 2H), 3.03 (s, 3H), 2.48 - 2.03 (m, 5H), 1.90 - 1.70 (m, 1H), 1.51 (d, J = 6.5 Hz, 3H). |
| 291 **(Reference)** | LC-MS: (ES, m/z): RT = 1.412 min, LCMS 34: m/z = 273 [M+1]. 1H NMR (300 MHz, Methanol-d4) δ 8.73 (s, 2H), 8.26 (s, 1H), 7.90 (d, J = 7.3 Hz, 1H), 6.51 (d, J = 7.3 Hz, 1H), 3.53 (q, J = 7.3 Hz, 2H), 3.20 (s, 3H), 1.30 (t, J = 7.3 Hz, 3H). |
| 293 **(Reference)** | LC-MS: (ES, m/z): RT =1.055 min, LCMS 28: m/z = 372 [M+1]. 1H NMR (400 MHz, Methanol-d4) δ 7.72 (s, 1H), 7.48 (s, 1H), 7.08 (d, J = 6.4 Hz, 1H), 6.90 (d, J = 8.8 Hz, 1H), 5.92 (d, J = 6.0 Hz, 1H), 4.10 (t, J = 6.0 Hz, 2H), 3.82 (s, 3H), 3.02 (d, J = 8.8 Hz, 1H), 2.93 (s, 3H), 2.87 (s, 1H), 2.79 (d, J = 8.0 Hz, 2H), 2.68 (s, 1H), 2.34 - 2.28 (m, 1H), 2.21 (d, J = 8.0 Hz, 1H), 2.13 - 2.01 (m, 3H), 1.45 (d, J =8.0 Hz, 1H), 1.09 (d, J = 6.8 Hz, 3H). |
| 298 **(Reference)** | LC-MS: (ES, m/z): RT = 0.978 min, LCMS15: m/z = 370.2 [M+1]. 1H NMR (400 MHz, Methanol-d4) δ 7.72 (s, 1H), 7.50 - 7.44 (m, 1H), 7.08 - 7.06 (m, 1H), 6.89 (d, J = 8.6 Hz, 1H), 5.92 (d, J = 6.0 Hz, 1H), 4.10 - 4.02 (m, 2H), 3.82 (s, 3H), 3.08 (d, J = 9.2 Hz, 2H), 2.93 (s, 3H), 2.68 (t, J = 7.6 Hz, 2H), 2.46 (d, J = 9.2 Hz, 2H), 2.01 - 1.95 (m, 2H), 1.49 - 1.41 (m, 2H), 0.68 (q, J = 4.1 Hz, 1H), 0.43 (q, J = 7.2 Hz, 1H). |
| 299 **(Reference)** | LC-MS: (ES, m/z): RT=0.590min, LCMS 30, m/z =374 [M+1]. 1H NMR (300 MHz, Methanol-d4) δ 7.72 (d, J = 6.0 Hz, 1H), 7.53 (d, J = 2.4 Hz, 1H), 7.09 (dd, J = 8.7, 2.5 Hz, 1H), 6.91 (d, J = 8.7 Hz, 1H), 5.92 (d, J = 6.0 Hz, 1H), 4.22 - 4.09 (m, 1H), 4.09 - 3.91 (m, 2H), 3.85 (s, 3H), 2.94 (s, 3H), 2.90 - 2.65 (m, 6H), 1.92 - 1.78 (m, 4H). |
| 300 **(Reference)** | LC-MS: (ES, m/z): RT=0.592min, LCMS 30, m/z =374 [M+1]. 1H NMR (300 MHz, Methanol-d4) δ 7.72 (d, J = 6.0 Hz, 1H), 7.53 (s, 1H), 7.09 (dd, J = 8.7, 2.5 Hz, 1H), 6.91 (d, J = 8.7 Hz, 1H), 5.92 (d, J = 6.0 Hz, 1H), 4.23 - 3.90 (m, 3H), 3.85 (s, 3H), 2.94 (s, 3H), 2.89 - 2.64 (m, 6H), 1.92 - 1.78 (m, 4H). |
| 301 | LC-MS: (ES, m/z): RT = 1.302min, LCMS 27: m/z = 390 [M+1]. 1H NMR (400 MHz, Methanol-d4) δ 7.35 - 7.23 (m, 2H), 5.85 (s, 1H), 4.02 (t, J = 6.1 Hz, 2H), 3.87 (s, 3H), 2.94 (s, 3H), 2.84 - 2.75 (m, 2H), 2.72 - 2.62 (m, 4H), 2.20 (s, 3H), 2.03 - 1.81 (m, 6H). |
| 302 | LC-MS: (ES, m/z): RT = 1.013 min; LCMS15: m/z = 390 [M+1]. 1H NMR (300 MHz, Methanol-d4) δ 8.10 (s, 1H), 6.86 (d, J = 12.4 Hz, 1H), 5.85 (s, 1H), 4.07 (t, J = 6.2 Hz, 2H), 3.83 (s, 3H), 2.93 (s, 3H), 2.71 - 2.66 (m, 2H), 2.59 (s, 4H), 2.19 (s, 3H), 2.05 - 1.96 (m, 2H), 1.93 - 1.77 (m, 4H). |
| 303 **(Reference)** | LC-MS: (ES, m/z): RT = 0.529 min, LCMS48: m/z = 373.3 [M+1]. 1H NMR (400 MHz, Methanol-d4) δ 8.00 (s, 1H), 6.73 (s, 1H), 6.16 (d, J = 1.2 Hz, 1H), 4.28 (t, J = 5.6 Hz, 2H), 3.94 (s, 3H), 3.86 - 3.76 (m, 2H), 3.47 (t, J = 7.2 Hz, 2H), 3.22 - 3.10 (m, 2H), 3.04 (s, 3H), 2.47 - 2.42 (m, 3H), 2.41 - 2.29 (m, 2H), 2.26 - 2.24 (m, 2H), 2.24 - 2.19 (m, 2H). |
| 304 **(Reference)** | LC-MS: (ES, m/z): RT = 1.019 min, LCMS15: m/z = 368.2 [M+1]. 1H NMR (400 MHz, Methanol-d4) δ 8.05 (s, 1H), 7.90 (d, J = 6.9 Hz, 1H), 7.60 (d, J = 3.3 Hz, 1H), 7.37 (d, J = 7.0 Hz, 1H), 7.35 - 7.30 (m, 1H), 7.11 (s, 1H), 4.35 (t, J = 5.6 Hz, 2H), 3.98 (d, J = 1.1 Hz, 3H), 3.88 - 3.78 (m, 2H), 3.50 (t, J = 7.3 Hz, 2H), 3.24 - 3.13 (m, 2H), 2.43 - 2.36 (m, 2H), 2.28 - 2.23 (m, 2H), 2.19 - 2.04 (m, 2H). |
| 305 **(Reference)** | LC-MS: (ES, m/z): RT =0.958min, LCMS 28: m/z = 358 [M+1]. 1H NMR (400 MHz, Methanol-d4) δ 7.92 (s, 1H), 7.70 (s, 1H), 6.59 (s, 1H), 6.45 (d, J = 9.1 Hz, 1H), 6.00 (s, 1H), 4.26 (t, J = 5.5 Hz, 2H), 3.93 (s, 3H), 3.89 - 3.74 (m, 2H), 3.47 (t, J = 7.3 Hz, 2H), 3.24 - 3.08 (m, 2H), 2.93 (s, 3H), 2.46 - 2.30 (m, 2H), 2.23 (q, J = 9.0, 2H), 2.09 - 2.06 (m, 2H). |
| 306 **(Reference)** | LC-MS: (ES, m/z): RT = 1.30 min, LCMS 53: m/z = 372.3 [M+1]. 1H NMR (400 MHz, Methanol-d4) δ 7.95 (d, J = 1.3 Hz, 1H), 6.61 (s, 1H), 6.26 (s, 1H), 5.91 (s, 1H), 4.26 (t, J = 5.5 Hz, 2H), 3.93 (s, 3H), 3.87 - 3.76 (m, 2H), 3.47 (t, J = 7.3 Hz, 2H), 3.22 - 3.10 (m, 2H), 2.92 (s, 3H), 2.55 - 2.45 (m, 3H), 2.40 - 2.16 (m, 4H), 2.16 - 2.00 (m, 2H). |
| 307 **(Reference)** | LC-MS: (ES, m/z): RT = 1.30 min, LCMS 53: m/z = 372.3 [M+1]. 1H NMR (400 MHz, Methanol-d4) δ 7.95 (d, J = 1.3 Hz, 1H), 6.61 (s, 1H), 6.26 (s, 1H), 5.91 (s, 1H), 4.26 (t, J = 5.5 Hz, 2H), 3.93 (s, 3H), 3.87 - 3.76 (m, 2H), 3.47 (t, J = 7.3 Hz, 2H), 3.22 - 3.10 (m, 2H), 2.92 (s, 3H), 2.55 - 2.45 (m, 3H), 2.40 - 2.16 (m, 4H), 2.16 - 2.00 (m, 2H). |
| 308 **(Reference)** | LC-MS: (ES, m/z): RT = 2.72 min, LCMS 33: m/z = 372.3 [M+1]. 1H NMR (400 MHz, Methanol-d4) δ 7.95 (d, J = 1.3 Hz, 1H), 7.43 (s, 1H), 6.66 (s, 1H), 4.92 (s, 1H), 4.26 (t, J = 5.5 Hz, 2H), 3.93 (s, 3H), 3.83 - 3.78 (m, 2H), 3.47 (t, J = 7.3 Hz, 2H), 3.19 - 3.14 (m, 2H), 3.00 (s, 3H), 2.61 - 2.41 (m, 3H), 2.40 - 2.30 (m, 4H), 2.26 - 2.21 (m, 2H). |
| 309 **(Reference)** | LC-MS: (ES, m/z): RT = 1.107 min; LCMS53: m/z = 390 [M+1]. 1H NMR (300 MHz, Methanol-d4) δ 7.95 (s, 1H), 6.62 (s, 1H), 6.07 (d, J = 6.5 Hz, 1H), 4.29 (t, J = 5.5 Hz, 2H), 3.93 (s, 3H), 3.82 (s, 2H), 3.47 (t, J = 7.3 Hz, 2H), 3.16 (q, J = 8.6 Hz, 2H), 2.97 (s, 3H), 2.51 (d, J = 3.2 Hz, 3H), 2.39 - 2.37 (m, 2H), 2.21 (q, J = 6.7 Hz, 2H), 2.14 - 2.02 (m, 2H). |
| 310 **(Reference)** | LC-MS: (ES, m/z): RT = 1.802 min ; m/z = 382.2 [M+1]. 1H NMR (300 MHz, Deuterium Oxide) δ 6.97 - 6.72 (m, 2H), 6.46 - 6.15 (m, 2H), 6.01 - 5.82 (m, 1H), 3.75 - 2.65 (m, 11H), 2.29 - 1.66 (m, 9H). |
| 311 | LC-MS: (ES, m/z): RT = 2.10 min, LCMS 53: m/z = 319.2 [M+1]. 1H NMR (300 MHz, DMSO-d6) δ 8.74 (s, 1H), 7.70 (s, 1H), 7.20 (q, J = 8.7 Hz, 1H), 6.93 (s, 1H), 6.82 (d, J = 8.8 Hz, 1H), 5.75 (s, 1H), 4.08 - 3.98 (m, 2H), 3.73 - 3.62 (m, 5H), 3.32 (s, 3H), 2.82 (d, J = 4.5 Hz, 3H), 2.11 (s, 3H). |
| 312 | LC-MS: (ES, m/z): RT = 1.221 min, LCMS 33: m/z = 333 [M+H1]. 1H NMR (400 MHz, Methanol-d4) δ 7.60 (s, 1H), 7.06-7.02 (m, 1H), 6.89 (d, J = 8.7 Hz, 1H), 5.81 (d, J = 0.8 Hz, 1H), 4.11 (t, J = 6.4 Hz, 2H), 3.82 (s, 3H), 3.61 (t, J = 6.2 Hz, 2H), 3.37 (s, 3H), 2.93 (s, 3H), 2.19 (s, 3H), 2.10-2.04 (m, 2H). |
| 313 | UPLC: (ES, m/z): RT = 2.42 min, UPLC 07: m/z = 382 [M+1]. 1H NMR (400 MHz, Methanol-d4) δ 7.26 (d, J = 2.0 Hz, 1H), 7.12 - 7.06 (m, 1H), 6.92 (d, J = 8.3 Hz, 1H), 6.03 (d, J = 1.1 Hz, 1H), 4.19 (t, J = 5.8 Hz, 2H), 3.81 - 3.72 (m, 2H), 3.55 - 3.46 (m, 2H), 3.22 - 3.12 (m, 2H), 3.03 (s, 3H), 2.39 - 2.28 (m, 5H), 2.23 - 2.03 (m, 5H), 0.99 - 0.91 (m, 2H), 0.70 - 0.63 (m, 2H). |
| 314 **(Reference)** | LC-MS: (ES, m/z): RT = 1.167 min, LCMS 28: m/z = 368 [M+1]. 1H-NMR: (400 MHz, Methanol-d4) δ 7.58 (d, J = 7.3 Hz, 1H), 7.18 (s, 1H), 7.06 (d, J = 8.2 Hz, 1H), 6.95 (d, J = 8.3 Hz, 1H), 6.18 (d, J = 7.3 Hz, 1H), 4.18 (t, J = 5.7 Hz, 2H), 3.74 (s, 2H), 3.54 - 3.45 (m, 2H), 3.25 - 3.12 (m, 2H), 3.05 (s, 3H), 2.36 - 3.28 (m, 2H), 2.22 - 2.12 (m, 5H), 1 - 0.90 (m, 2H), 0.73 - 0.60 (m, 2H). |
| 315 **(Reference)** | LC-MS: (ES, m/z): RT =1.160min, LCMS 33: m/z =412 [M+1]. 1H NMR (300 MHz, Methanol-d4) δ 7.67 (d, J = 7.3 Hz, 1H), 7.52 (d, J = 1.8 Hz, 1H), 7.45 - 7.27 (m, 2H), 6.24 (d, J = 7.3 Hz, 1H), 4.24 (t, J = 5.7 Hz, 2H), 3.75 - 3.69 (m, 2H), 3.50 - 3.39 (m, 2H), 3.15 (q, J = 8.4 Hz, 2H), 3.07 (s, 3H), 2.31 - 2.29 (m, 2H), 2.20 (d, J = 9.1 Hz, 2H), 2.07 (t, J = 6.6 Hz, 2H). |
| 317 | LC-MS: (ES, m/z): RT=12min, LCMS 31, m/z =358 [M+1]. 1H NMR (300 MHz, Methanol-d4) δ 7.22 (dd, J = 8.7, 2.4 Hz, 1H), 7.12 - 6.99 (m, 2H), 6.01 (d, J = 1.0 Hz, 1H), 4.67 - 4.49 (m, 1H), 3.86 (s, 3H), 3.60 - 3.44 (m, 1H), 3.11 - 2.95 (m, 5H), 2.87 (s, 6H), 2.50 - 2.27 (m, 5H). |
| 318 | LC-MS: (ES, m/z): RT = 0.99min, LCMS33: m/z = 372 [M+1]. 1H NMR (300 MHz, Methanol-d4) δ 7.36 (d, J = 2.4 Hz, 1H), 7.10 (dd, J = 8.7, 2.5 Hz, 1H), 6.88 (d, J = 8.8 Hz, 1H), 5.81 (s, 1H), 4.68-4.65 (m, 1H), 3.82 (s, 3H), 2.93 (s, 3H), 2.78 - 2.69 (m, 2H), 2.62 (d, J = 6.9 Hz, 2H), 2.36 (s, 6H), 2.36-2.13 (m, 4H), 1.99 - 1.79 (m, 2H). |
| 319 | LC-MS: (ES, m/z): RT= 0.83 min, LCMS 45: m/z = 404 [M+1]. 1H-NMR: (Methanol-d4, ppm): δ 7.26 (d, J = 2.4 Hz, 1H), 7.14 (dd, J = 8.7, 2.4 Hz, 1H), 7.05 (d, J = 8.7 Hz, 1H), 4.20 (t, J = 5.5 Hz, 2H), 3.90-3.80 (m, 5H), 3.50 (t, J = 7.1 Hz, 2H), 3.07 (s, 5H), 2.78-2.56 (m, 2H), 2.37 - 2.02 (m, 6H), 1.33 (t, J = 7.6 Hz, 3H). |
| 320 | LC-MS: (ES, m/z): RT=1.780min, LCMS 31, m/z =398 [M+1]. 1H NMR (400 MHz, Methanol-d4) δ 7.34 (d, J = 2.4 Hz, 1H), 7.17 (dd, J = 8.7, 2.5 Hz, 1H), 7.04 (d, J = 8.7 Hz, 1H), 5.86 (s, 1H), 4.20 (t, J = 5.5 Hz, 2H), 3.89 (s, 3H), 3.87 - 3.78 (m, 2H), 3.49 (t, J = 7.0 Hz, 2H), 3.25 - 3.09 (m, 2H), 3.00 (s, 3H), 2.35 - 2.16 (m, 4H), 2.15 - 2.02 (m, 2H), 1.99 - 1.86 (m, 1H), 1.32 - 1.11 (m, 2H), 1.09 - 0.95 (m, 2H). |
| 321 **(Reference)** | LC-MS: (ES, m/z): RT = 0.758 min, LCMS 33: m/z = 368 [M+1]. 1H NMR (300 MHz, Methanol-d4) δ 8.71-8.59 (m,1H), 8.31-8.19 (m, 1H), 8.18-8.00(m, 2H), 7.65-7.55(m, 1H),7.61 - 7.50 (m, 1H), 6.88- 6.75 (m, 1H), 4.30 (s, 2H), 3.90 (s, 3H), 3.89-3.75 (m, 2H), 3.42 (s, 2H), 3.20-3.09 (m, 2H), 2.30 (s, 2H),2.28 - 2.02 (m, 4H). |
| 322 **(Reference)** | LC-MS: (ES, m/z): RT = 0.96min, LCMS33: m/z = 359.00 [M+1]. 1H NMR (300 MHz, Methanol-d4) δ 8.08 (s, 1H), 8.01 (s, 1H), 6.68 (s, 1H), 6.18 (s, 1H), 4.28 (t, J = 5.5 Hz, 2H), 3.95 (s, 3H), 3.95-3.75 (m, 2H), 3.48 (t, J = 7.4 Hz, 2H), 3.17 (s, 2H), 2.98 (s, 3H), 2.42 - 2.30 (m, 2H), 2.26-2.23 (m, 2H), 2.08 (t, J = 6.5 Hz, 2H). |
| 323 **(Reference)** | LC-MS: (ES, m/z): RT=0.836min, LCMS 15, m/z =359.2 [M+1]. 1H NMR (300 MHz, Deuterium Oxide) δ 8.08 - 7.92 (m, 2H), 7.09 (s, 1H), 6.26 (d, J = 2.4 Hz, 1H), 4.22 (t, J = 5.6 Hz, 2H), 3.89 (s, 3H), 3.73 - 3.56 (m, 2H), 3.36 (d, J = 17.0 Hz, 5H), 3.10 - 2.95 (m, 2H), 2.32 - 1.78 (m, 6H). |
| 324 **(Reference)** | LC-MS: (ES, m/z): RT = 0.888min, LCMS 33: m/z = 359 [M+1]. 1H NMR (300 MHz, Methanol-d4) δ 8.40 (s, 1H), 7.92 (s, 1H), 6.70 (s, 1H),6.10-5.90 (m, 1H), 4.31 (t, J = 5.6 Hz, 2H), 3.94 (s, 3H), 3.85-3.75 (m, 2H), 3.47 (t, J = 7.4 Hz, 2H), 3.25-3.15 (m, 2H), 3.05-2.95 (m, 3H), 2.40-2.30 (m, 2H), 2.22 (s, 2H), 2.10-2.00 (m, 2H). |
| 325 **(Reference)** | LC-MS: (ES, m/z): RT = 0.918 min, LCMS 33: m/z = 373 [M+1]. 1H NMR (300 MHz, Methanol-d4) δ 7.99 (s, 1H), 6.67 (s, 1H), 6.00 (s, 1H), 4.28 (s, 2H), 3.94 (s, 3H), 3.80 (s, 2H), 3.47 (s, 2H), 3.21 - 3.08 (m, 2H), 2.99 (s, 3H), 2.59 (s, 3H), 2.35 (s, 2H), 2.22 (s, 2H), 2.08 (s, 2H) |
| 326 **(Reference)** | LC-MS: (ES, m/z): RT = 0.958 min, LCMS 33: m/z = 383 [M+1]. 1H NMR (300 MHz, Methanol-d4) δ 8.14 (s, 1H), 7.48 (d, J = 3.6 Hz, 1H), 7.09 - 6.98 (m, 2H), 4.36 (t, J = 5.5 Hz, 2H), 4.00 (s, 3H), 3.90-3.80 (m, 2H), 3.50 (t, J = 7.4 Hz, 2H), 3.19 (s, 2H), 2.83 (s, 3H), 2.45-2.35 (m, 2H), 2.30-2.20 (m, 2H), 2.15-2.05 (m, 2H). |
| 328 | LC-MS: (ES, m/z): RT = 1.196 min; m/z = 398.3[M+1]. 1H NMR (300 MHz, Deuterium Oxide) δ7.22 - 7.12 (m, 1H), 7.07 - 6.92 (m, 2H), 4.05 (t, J = 5.6 Hz, 2H), 3.79 (s, 3H), 3.75 - 3.58 (m, 2H), 3.33 (t, J = 7.5 Hz, 2H), 3.11 - 2.95 (m, 2H), 2.89 (s, 3H), 2.72 (t, J = 7.7 Hz, 2H), 2.55 - 2.42 (m, 2H), 2.23 - 1.89 (m, 8H). |
| 329 | LC-MS: (ES, m/z): RT=1.021min, LCMS 31, m/z =358 [M+1]. 1H NMR (300 MHz, Methanol-d4) δ 7.18 (dd, J = 8.7, 2.4 Hz, 1H), 7.14 - 6.96 (m, 2H), 6.04 - 5.96 (m, 1H), 4.13 - 3.96 (m, 1H), 3.87 (s, 3H), 3.37 - 3.34 (m, 1H), 3.04 - 2.97 (m, 3H), 2.91 - 2.75 (m, 8H), 2.71 - 2.59 (m, 2H), 2.31 (d, J = 0.9 Hz, 3H). |
| 330 | LC-MS: (ES, m/z): RT = 1.115 min ; m/z = 372.0 [M+1]. 1H NMR (300 MHz, Deuterium Oxide) δ 7.19 - 7.06 (m, 1H), 6.96 - 6.29 (m, 2H), 5.86 - 5.40 (m, 1H), 3.95 - 3.76 (m, 1H), 3.76 - 3.38 (m, 5H), 2.79 - 2.43 (m, 9H), 2.41 - 2.25 (m, 2H), 2.25 - 2.02 (m, 2H), 1.97 - 1.83 (m, 3H). |
| 331 | LC-MS: (ES, m/z): RT = 0.99min, LCMS33: m/z =372 [M+1]. 1H NMR (300 MHz, Methanol-d4) δ 7.24 (d, J = 2.4 Hz, 1H), 7.09 (d, J = 2.4 Hz, 1H), 6.89 (d, J = 8.7 Hz, 1H), 5.81 (s, 1H), 4.83 - 4.75 (m, 1H), 3.83 (s, 3H), 2.92 (s, 3H), 2.68 (d, J = 4.7 Hz, 3H), 2.53 - 2.24 (m, 10H), 2.19 (s, 3H). |
| 332 **(Reference)** | LC-MS: (ES, m/z): RT = 2.247 min; m/z = 373.2 [M+1]. 1H NMR (300 MHz, Deuterium Oxide) δ 7.76 - 7.65 (m, 1H), 7.53 - 7.09 (m, 1H), 6.07 - 5.83 (m, 1H), 4.17 - 3.95 (m, 2H), 3.91 - 3.80 (s, 3H), 3.74 - 3.48 (m, 2H), 3.38 - 3.23 (m, 2H), 3.11 - 2.94 (m, 2H), 2.94 - 2.74 (m, 3H), 2.26 - 2.00 (m, 7H), 1.97 - 1.82 (m, 2H). |
| 333 **(Reference)** | LC-MS: (ES, m/z): RT = 1.032 min, LCMS 28: m/z = 355 [M+1]. 1H-NMR-PH-EPI-K-351-100: (300 MHz, Methanol-d4) δ 12.19 (s, 1H), 7.90 (d, J = 7.1 Hz, 1H), 7.64 - 7.49 (m, 1H), 7.35 -7.25 (m, 1H), 7.21 -7.13 (m, 1H), 6.32 (s, 1H), 4.76 (s, 2H), 4.33 (q, J = 7.2 Hz, 2H), 3.93 - 3.85 (m, 2H), 3.74 -3.66 (m, 2H), 3.53 - 3.45 (m, 2H), 3.24 - 3.08 (m, 2H), 2.25 - 2.11 (m, 2H), 2.11 - 1.96 (m, 2H), 1.53 (t, J = 7.2 Hz, 3H). |
| 334 | LC-MS: (ES, m/z): RT=0.955min, LCMS 40, m/z =358 [M+1]. 1H NMR (300 MHz, Methanol-d4) δ 7.42 - 7.29 (m, 1H), 7.27 - 6.99 (m, 2H), 6.06 - 5.98 (m, 1H), 4.52 - 4.04 (m, 6H), 3.95 - 3.86 (m, 3H), 3.49 (q, J = 7.3 Hz, 2H), 3.40 - 3.33 (m, 1H), 3.05 - 2.95 (m, 3H), 2.31 (s, 3H), 1.37 - 1.21 (m, 3H). |
| 335 **(Reference)** | LC-MS: (ES, m/z): RT= 0.90 min, LCMS 33:m/z = 368 [M+1]. 1H-NMR: (Methanol-d4, ppm): δ 8.27 (dd, J = 7.3, 2.2 Hz, 1H), 8.12 - 8.00 (m, 2H), 7.41 (d, J = 3.6 Hz, 1H), 7.09 - 6.98 (m, 2H), 4.30 (t, J = 5.5 Hz, 2H), 3.97 (s, 3H), 3.92-3.77 (m, 2H), 3.49 (t, J = 7.3 Hz, 2H), 3.24 - 3.09 (m, 2H), 2.43 - 2.01 (m, 6H). |
| 336 **(Reference)** | LC-MS: (ES, m/z): RT= 1.73 min, LCMS 53:m/z = 382 [M+]. 1H-NMR: (Methanol-d4, ppm):δ 8.14 (s, 1H), 7.48 - 7.35 (m, 3H), 6.92 (d, J = 3.5 Hz, 1H), 4.50-4.40 (m, 2H), 4.05 (s, 3H), 3.92-3.75 (m, 2H), 3.49 (t, J = 7.2 Hz, 2H), 3.22-3.05 (m, 2H), 2.74 (s, 3H), 2.47 - 2.36 (m, 2H), 2.22 - 2.09 (m, 4H). |
| 388 **(Reference)** | LC-MS: (ES, m/z): RT =0.859 min, LCMS 33: m/z = 326 [M+1]. 1H NMR (300 MHz, Methanol-d4) δ 8.45 (d, J = 0.9 Hz, 1H), 8.09 (s, 1H), 7.84 (dd, J = 9.0, 0.8 Hz, 1H), 7.67 (d, J = 7.4 Hz, 1H), 7.22 (dd, J = 8.9, 1.8 Hz, 1H), 6.26 (d, J = 7.3 Hz, 1H), 4.99 - 4.87 (m, 2H), 3.29 - 3.17 (m, 2H), 3.10 (s, 3H), 2.92 (s, 6H), 2.48 (s, 2H). |
| 404 **(Reference)** | LC-MS: (ES, m/z): RT = 0.598 min; m/z = 312.2 [M+1]. 1H NMR (300 MHz, Methanol-d4) δ 8.53 (d, J = 0.9 Hz, 1H), 8.13 - 8.06 (m, 1H), 7.93 - 7.77 (m, 1H), 7.68 (d, J = 7.3 Hz, 1H), 7.26 (dd, J = 9.0, 1.8 Hz, 1H), 6.27 (d, J = 7.3 Hz, 1H), 5.00 (t, J = 6.7, 5.1 Hz, 2H), 3.88 (t, J = 5.9 Hz, 2H), 3.12(s, 3H), 3.09 - 3.01(m, 6H). |
| 407 **(Reference)** | LC-MS: (ES, m/z): RT = 0.942 min, LCMS33: m/z = 344 [M+1]. 1H NMR (300 MHz, Methanol-d4) δ 7.61 - 7.37 (m, 3H), 7.12 - 7.08 (m, 1H), 6.19 - 6.17 (m, 1H), 4.70 - 4.62 (m, 2H), 4.45 (d, J = 5.4 Hz, 1H), 3.97 - 3.84 (m, 3H), 3.84 - 3.70 (m, 2H), 3.34 - 3.11 (m, 2H), 3.09 - 2.92 (m, 6H), 2.57 - 2.33 (m, 1H), 2.34 - 2.09 (m, 1H). |
| 408 **(Reference)** | LC-MS: (ES, m/z): RT = 0.95min, LCMS07: m/z = 358.20 [M+1]. 1H NMR (300 MHz, Methanol-d4) δ 7.55 (dd, J = 7.3, 1.8 Hz, 1H), 7.21 (d, J = 8.0 Hz, 1H), 7.09 (dd, J = 4.7, 1.3 Hz, 2H), 6.18 (d, J = 7.3 Hz, 1H), 4.20 - 4.07 (m, 2H), 3.96-3.84 (m, 4H), 3.75-3.70 (m, 1H), 3.38 (d, J = 7.4 Hz, 2H), 3.30 - 3.05 (m, 2H), 3.19-2.89 (m, 4H), 2.53 - 2.25 (m, 1H), 2.23 - 1.94 (m, 1H), 1.38 (t, J = 7.3 Hz, 3H). |
| 409 **(Reference)** | LC-MS: (ES, m/z):RT = 0.981 min, LCMS 33: m/z = 372 [M+1]. |
| 410 **(Reference)** | LC-MS: (ES, m/z): RT = 0.94 min, LCMS 07: m/z = 388 [M+1]. 1H-NMR: (Methanol-d4, ppm): δ 7.57 (d, J = 7.3 Hz, 1H), 7.25 (s, 1H), 7.16 (d, J = 9.4 Hz, 1H), 7.05 (d, J = 8.9 Hz, 1H), 6.16 (d, J = 7.3 Hz, 1H), 4.19 - 4.00 (m, 2H), 4.00 - 3.65 (m, 7H), 3.58 - 3.35 (m, 6H), 3.32 - 3.14 (m, 1H), 3.03-2.85 (m, 4H), 2.41-1.90 (m, 2H). |
| 411 **(Reference)** | LC-MS: (ES, m/z): RT=1.285min, LCMS 40, m/z =370 [M+1]. 1H NMR (300 MHz, Methanol-d4) δ 7.56 (d, J = 7.3 Hz, 1H), 7.29 - 6.99 (m, 3H), 6.17 (d, J = 7.3 Hz, 1H), 4.23 - 4.00 (m, 2H), 3.98 - 3.41 (m, 7H), 3.03 (s, 5H), 2.62 - 1.60 (m, 2H), 1.11 - 0.89 (m, 4H). |
| 412 **(Reference)** | LC-MS: (ES, m/z): RT = 0.919 min, LCMS07: m/z = 358.15 [M+1]. 1H NMR (300 MHz, Methanol-d4) δ 7.57 (d, J = 7.2 Hz, 1H), 7.20 (s, 1H), 7.13 - 7.01 (m, 2H), 6.26 - 6.13 (m, 1H), 4.09 - 4.04 (m, 1H), 3.99 - 3.84 (m, 4H), 3.75 - 3.71 (m, 1H), 3.57 - 3.53 (m, 1H), 3.07 - 2.90 (m, 8H), 2.42 (s, 1H), 2.10 - 1.89 (m, 3H), 1.59 - 1.40 (m, 1H). |
| 413 | LC-MS: (ES, m/z): RT = 0.969 min, LCMS07: m/z = 372.20 [M+1]. 1H NMR (400 MHz, Methanol-d4) δ 7.58 (s, 1H), 7.08 - 7.06 (m, 1H), 6.88 (d, J = 8.4Hz, 1H), 5.81 (s, 1H), 3.96 - 3.94 (m, 1H), 3.90 - 3.79 (m, 4H), 3.16 - 3.14 (m, 1H), 2.93 (s, 4H), 2.36 (d, J = 2.8 Hz, 3H), 2.18 (s, 5H), 2.00 - 1.97 (m, 1H), 1.92 - 1.76 (m, 2H), 1.72 - 1.67 (m, 1H), 1.23 - 1.09 (m, 1H). |
| 414 **(Reference)** | LC-MS: (ES, m/z): RT = 1.020 min, LCMS53: m/z = 360.2 [M+1]. 1H NMR (300 MHz, Deuterium Oxide) δ 7.65 - 7.29 (m, 1H), 7.18 - 6.82 (m, 3H), 7.06 (s, 2H), 6.20 - 5.87 (m, 1H), 4.60 - 4.37 (m, 2H), 4.25 - 3.88 (m, 4H), 3.88 - 3.68 (m, 4H), 3.48 - 3.25 (m, 2H), 2.98 - 2.68 (m, 3H), 2.10 - 1.82 (m, 2H). |
| 415 **(Reference)** | LC-MS: (ES, m/z): RT = 1.77 min, LCMS 07: m/z = 344.3 [M+1]. 1H NMR (400 MHz, Methanol-d4) δ 7.72 (s, 1H), 7.50 (s, 1H), 7.08 (d, J = 8.7 Hz, 1H), 6.90 (d, J = 8.7 Hz, 1H), 5.92 (d, J = 6.0 Hz, 1H), 3.96 - 3.86 (m, 2H), 3.82 (s, 3H), 2.94 (s, 3H), 2.86 (d, J = 9.1 Hz, 1H), 2.81 - 2.73 (m, 1H), 2.67 (t, J = 7.1 Hz, 2H), 2.56 (q, J = 7.7 Hz, 1H), 2.43 (s, 3H), 2.20 - 2.04 (m, 1H), 1.69 - 1.67 (m, 1H). |
| 416 **(Reference)** | LC-MS: (ES, m/z): RT = 1.71 min, LCMS 07: m/z = 344.3 [M+1]. 1H NMR (400 MHz, Methanol-d4) δ 7.74 (s, 1H), 7.55 (s, 1H), 7.09 (d, J = 8.7 Hz, 1H), 6.93 (d, J = 8.7 Hz, 1H), 5.97 (d, J = 6.0 Hz, 1H), 3.96 - 3.83 (m, 2H), 3.82 (s, 3H), 2.94 (s, 3H), 2.88 (d, J = 9.1 Hz, 1H), 2.84 - 2.72 (m, 1H), 2.66 (t, J = 7.1 Hz, 2H), 2.57 (q, J = 7.7 Hz, 1H), 2.41 (s, 3H), 2.22 - 2.01 (m, 1H), 1.68 - 1.64 (m, 1H). |
| 417 | LC-MS: (ES, m/z): RT = 1.64 min, LCMS 53: m/z = 358.3 [M+1]. 1H NMR (300 MHz, Chloroform-d) δ 7.54 (d, J = 2.5 Hz, 1H), 7.21 (s, 1H), 6.98 (d, J = 8.6 Hz, 1H), 6.83 (d, J = 8.7 Hz, 1H), 5.73 (d, J = 0.8 Hz, 1H), 4.94 (s, 1H), 4.00 (d, J = 6.5 Hz, 2H), 3.84 (s, 3H), 3.35 - 2.57 (m, 8H), 2.49 (s, 3H), 2.28 (s, 3H), 2.16 - 2.13 (m, 1H), 1.73 - 1.71 (m, 1H). |
| 418 | LC-MS: (ES, m/z): RT = 1.15 min, LCMS 53: m/z = 358.3 [M+1]. 1H NMR (400 MHz, Methanol-d4) δ 7.60 (s, 1H), 7.08 (d, J = 8.7 Hz, 1H), 6.89 (d, J = 8.7 Hz, 1H), 5.81 (s, 1H), 3.96 - 3.90 (m, 2H), 3.82 (s, 3H), 2.93 (s, 3H), 2.85 - 2.83 (m, 1H), 2.82 - 2.70 (m, 1H), 2.71 - 2.60 (m, 2H), 2.53 - 2.49 (m, 1H), 2.41 (d, J = 1.1 Hz, 3H), 2.18 (s, 3H), 2.16 - 2.03 (m, 1H), 1.67 - 1.60 (m, Hz, 1H). |
| 419 | LC-MS: (ES, m/z): RT =1.01min, LCMS33: m/z = 372 [M+1]. 1H NMR (300 MHz, DMSO-d6) δ 7.41 (s, 1H), 7.19 - 6.95 (m, 2H), 6.03 (s, 1H), 4.1-3.92 (m, 2H), 3.85-3.7 (m, 3H), 3.63-3.52 (m, 1H), 3.32 - 2.98 (m, 4H), 2.95 - 2.72 (m, 5H), 2.42 - 2.05 (m, 4H), 1.98-1.67 (m, 1H), 1.25 (t, J = 7.2 Hz, 3H). |
| 420 **(Reference)** | LC-MS: (ES, m/z): RT=0.774min, LCMS 40, m/z =372 [M+1]. 1H NMR (300 MHz, Methanol-d4) δ 7.56 (d, J = 7.3 Hz, 1H), 7.28 - 6.98 (m, 3H), 6.17 (d, J = 7.3 Hz, 1H), 4.19 - 3.99 (m, 2H), 3.89 (s, 4H), 3.78 - 3.65 (m, 1H), 3.46 - 3.36 (m, 1H), 3.30 - 3.10 (m, 3H), 3.08 - 2.87 (m, 4H), 2.53 - 1.91 (m, 2H), 1.89 - 1.71 (m, 2H), 1.07 (t, J = 7.4 Hz, 3H). |
| 421 | 1H NMR (400 MHz, Methanol-d4) δ 7.69 - 7.63 (m, 1H), 7.57 - 7.55 (m, 1H), 6.90 (d, J = 8.8 Hz, 1H), 5.79 (d, J = 0.8 Hz, 1H), 4.57 - 4.45 (m, 2H), 4.24 - 4.21 (m, 1H), 3.83 (s, 3H), 2.92 (s, 3H), 2.85 - 2.67 (m, 3H), 2.55 - 2.51 (m, 1H), 2.38 (s, 3H), 2.24 - 2.10 (m, 4H), 1.96 - 1.94 (m, 1H). |
| 422 **(Reference)** | LC-MS: (ES, m/z): RT = 0.979 min, LCMS 45: m/z = 384.2 [M+1]. 1H NMR (300 MHz, Deuterium Oxide) δ 7.65 - 7.33 (m, 1H), 7.24 - 7.12 (s, 1H), 7.03 - 6.89 (m, 2H), 6.19 - 5.98 (m, 1H), 4.08 - 3.52 (m, 7H), 3.39 - 2.80 (m, 8H), 2.38 - 2.07 (m, 1H), 2.05 - 1.72 (m, 1H), 1.12 - 0.95 (m, 1H), 0.70 - 0.53 (m, 2H), 0.29 - 0.21 (m, 2H). |
| 423 **(Reference)** | LC-MS: (ES, m/z): RT = 0.94min, LCMS07: m/z = 358.15 [M+1]. 1H NMR (300 MHz, Methanol-d4) δ 7.87-7.5 (m, 1H), 7.29 - 7.01 (m, 3H), 6.38-6.11 (m, 1H), 4.43 (t, J = 9.4 Hz, 1H), 4.27 - 4.10 (m, 3H), 4.08 - 4.00 (m, 1H), 3.94 (d, J = 2.2 Hz, 3H), 3.79 (t, J = 9.8 Hz, 1H), 3.50-3.41 (m, 2H), 3.10-2.9 (m, 4H), 2.2-2.02 (m, 2H), 1.41 (d, J = 7.1 Hz, 1H), 1.30 (d, J = 6.7 Hz, 2H). |
| 424 **(Reference)** | LC-MS: (ES, m/z): RT = 0.97min, LCMS07: m/z = 372.20 [M+1]. 1H NMR (300 MHz, Methanol-d4) δ 7.9-7.54 (m, 1H), 7.29 - 7.05 (m, 3H), 6.4-6.16 (m, 1H), 4.20 (t, J = 5.6 Hz, 2H), 3.91 (s, 3H), 3.62 - 3.49 (m, 1H), 3.48 - 3.39 (m, 1H), 3.29-3.19 (m, 1H), 3.17-2.9 (m, 7H), 2.31-2.27 (m, 2H), 1.31 - 1.15 (m, 1H), 0.83-0.80 (m, 2H), 0.59 - 0.39 (m, 2H). |
| 425 **(Reference)** | LC-MS: (ES, m/z): RT= 0.96 min, LCMS 45: m/z = 374 [M+1]. 1H-NMR: (Methanol-d4, ppm): δ 7.57 (d, J = 7.3 Hz, 1H), 7.24 - 7.03 (m, 3H), 6.17 (d, J = 7.3 Hz, 1H), 4.65-4.60 (m, 1H), 4.33 (s, 3H), 4.16-4.10 (m, 2H), 4.08 - 3.90 (m, 4H), 3.59-3.45 (m, 2H), 3.40-3.35 (m, 3H), 3.03 (s, 3H), 2.25-2.11 (m, 2H). |
| 426 **(Reference)** | LC-MS: (ES, m/z): RT = 1.34 min, LCMS 33: m/z = 368 [M+1]. 1H NMR (300 MHz, Methanol-d4) δ 7.53 (d, J = 7.4 Hz, 1H), 7.23 (s, 1H), 7.07 (s, 2H), 6.25 - 6.12 (m, 2H), 5.14 (s, 2H), 3.89 (s, 3H), 3.02 (s, 3H), 2.15-2.05 (m, 1H), 1.19 - 1.03 (m, 2H), 1.04 - 0.89 (m, 2H). |
| 428 | LC-MS: (ES, m/z): RT = 1.111 min, LCMS 33: m/z = 440 [M+1]. 1H NMR (300 MHz, Methanol-d4) δ 7.62 (s, 1H), 7.10 - 7.04 (m, 1H), 6.88 (d, J = 8.7 Hz, 1H), 5.96 (s, 1H), 4.09 (t, J = 6.2 Hz, 2H), 3.82 (s, 3H), 3.42 - 3.38 (m,1H), 3.31-3.28 (m, 1H), 2.94 (s, 3H), 2.80 - 2.70 (m, 2H), 2.68 - 2.60 (m, 4H), 2.10-2.02 (m, 2H), 1.90-1.80 (m, 4H). |
| 429 **(Reference)** | LC-MS: (ES, m/z): RT=0.996min, LCMS 31, m/z = 358 [M+1]. 1H NMR (300 MHz, Methanol-d4) δ 7.56 (d, J = 7.3 Hz, 1H), 7.24 - 7.00 (m, 3H), 6.17 (d, J = 7.3 Hz, 1H), 4.62 - 4.47 (m, 1H), 4.29 - 4.10 (m, 3H), 4.08 - 3.89 (m, 4H), 3.58 - 3.33 (m, 2H), 3.03 (s, 3H), 2.69 - 2.54 (m, 1H), 2.43 - 2.23 (m, 1H), 2.22 - 2.07 (m, 2H), 1.62 (d, J = 6.6 Hz, 3H). |
| 430 **(Reference)** | LC-MS: (ES, m/z): RT = 1.02min, LCMS33: m/z = 372 [M+1]. 1H NMR (300 MHz, DMSO-d6) δ 8.1-7.8 (m, 1H), 7.71 (s, 1H), 7.05 (d, J = 9.0 Hz, 1H), 6.76 (dd, J = 9.0, 3.0 Hz, 1H), 6.4-6.2 (m, 1H), 4.14 - 3.93 (m, 4H), 3.63 - 3.49 (m, 2H), 3.32 - 3.19 (m, 2H), 3.08 - 2.85 (m, 5H), 2.19 - 1.83 (m, 6H), 1.32 (t, J = 6.9 Hz, 3H). |
| 432 | LC-MS: (ES, m/z): RT = 1.06mm, LCMS33: m/z = 386 [M+1]. 1H NMR (300 MHz, DMSO-d6) δ 7.96 - 7.89 (m, 1H), 7.04 (d, J = 9.0 Hz, 1H), 6.70 (dd, J = 9.0, 3.0 Hz, 1H), 6.37-6.09 (m, 1H), 4.11-3.98 (m, 4H), 3.62-3.56 (m, 2H), 3.31 - 3.19 (m, 2H), 3.08 - 2.85 (m, 5H), 2.39 - 2.24 (m, 3H), 2.19 - 1.76 (m, 6H), 1.36 (t, J = 6.9 Hz, 3H). |
| 433 **(Reference)** | LC-MS: (ES, m/z): RT = 1.34 min, LCMS 33: m/z = 368 [M+1]. 1H NMR (300 MHz, Methanol-d4) δ 7.64 (s, 1H), 7.43 (d, J = 2.4 Hz, 1H), 7.11 (dd, J = 8.9, 2.5 Hz, 1H), 6.98 (d, J = 8.7 Hz, 1H), 6.01 (d, J = 6.5 Hz, 1H), 5.14 (s, 2H), 3.84 (s, 3H), 2.95 (s, 3H), 2.35-2.25 (m, 1H), 1.35 - 1.13 (m, 4H). |
| 435 | LC-MS: (ES, m/z): RT= 1.15 min, LCMS 53: m/z = 372 [M+1]. 1H-NMR: (Methanol-d4, ppm): δ 7.27 (s, 1H), 7.16 - 6.97 (m, 2H), 6.01 (s, 1H), 3.95-3.90 (m, 2H), 3.86 (s, 3H), 3.65 - 3.53 (m, 2H), 3.01-2.95 (m, 5H), 2.90 (s, 3H), 2.31 (s, 3H), 2.22 - 2.11 (m, 3H), 1.87-1.56 (m, 2H). |
| 436 | LC-MS: (ES, m/z): RT= 1.08 min, LCMS53:m/z = 412 [M+1]. 1H-NMR: (Methanol-d4, ppm): δ 7.27 (d, J = 2.4 Hz, 1H), 7.11 (dd, J = 8.7, 2.5 Hz, 1H), 7.02 (d, J = 8.8 Hz, 1H), 6.04 - 5.97 (m, 1H), 3.96 (d, J = 5.4 Hz, 2H), 3.85 (d, J = 3.7 Hz, 3H), 3.76 (d, J = 12.4 Hz, 2H), 3.17 - 2.97 (m, 6H), 2.32 - 2.20 (m, 5H), 1.91-1.76 (m, 2H), 1.33-1.05 (m, 1H), 0.87 - 0.74 (m, 2H), 0.56-0.36 (m, 2H). |
| 437 | LC-MS: (ES, m/z): RT = 0.957 min, LCMS15: m/z = 330.2 [M+1]. 1H NMR (400 MHz, DMSO-d6) δ 8.80 - 8.58 (m, 2H), 7.69 (s, 1H), 7.22 (d, J = 8.0 Hz, 1H), 6.91 (d, J = 8.4 Hz, 1H), 5.84 (s, 1H), 4.11 (d, J = 6.2 Hz, 2H), 4.06 (d, J = 9.7 Hz, 2H), 3.91 - 3.71 (m, 6H), 3.28 - 3.18 (m, 1H), 2.86 (s, 3H), 2.16 (s, 3H). |
| 438 **(Reference)** | LC-MS: (ES, m/z): RT = 1.54 min, LCMS 28: m/z = 358 [M+1]. 1H NMR (300 MHz, Methanol-d4) δ 7.72 (d, J = 5.8 Hz, 1H), 7.51 (s, 1H), 7.07 (dd, J = 8.7, 2.5 Hz, 1H), 6.89 (d, J = 8.7 Hz, 1H), 5.91 (d, J = 6.0 Hz, 1H), 4.08 - 3.90 (m, 2H), 3.82 (s, 3H), 2.98 - 2.62 (m, 6H), 2.37 (d, J = 2.5 Hz, 3H), 2.30-2.20 (m, 2H), 2.10-2.00 (m, 1H), 1.18 (d, J = 6.8 Hz, 3H). |
| 439 **(Reference)** | LC-MS: (ES, m/z): RT = 1.56 min, LCMS 28: m/z = 358 [M+1]. 1H NMR (300 MHz, Methanol-d4) δ 7.72 (s, 1H), 7.51 (s, 1H), 7.08 (dd, J = 8.7, 2.5 Hz, 1H), 6.89 (d, J = 8.8 Hz, 1H), 5.92 (d, J = 6.0 Hz, 1H), 4.09 - 3.90 (m, 2H), 3.82 (s, 3H), 2.94 (s, 4H), 2.90-2.80 (m, 2H), 2.39 (d, J = 4.9 Hz, 3H), 2.27 (d, J = 8.3 Hz, 2H), 2.08 (s, 1H), 1.18 (d, J = 6.8 Hz, 3H). |
| 440 **(Reference)** | LC-MS: (ES, m/z): RT = 1.400 min, LCMS 33: m/z = 359 [M+1]. 1H NMR (300 MHz, Methanol-d4) δ 7.53 (d, J = 7.2 Hz, 1H), 7.20 (s, 1H), 7.03 (d, J = 2.7 Hz, 2H), 6.16 (d, J = 7.3 Hz, 1H), 4.20 - 4.00 (m, 3H), 3.91 - 3.73 (m, 5H), 3.03 (s, 3H), 1.85 - 1.49 (m, 9H). |
| 441 | LC-MS: (ES, m/z): RT= 0.831 min, LCMS 32, m/z = 402.4 [M+1]. 1H NMR (400 MHz, Deuterium Oxide) δ 7.15 (d, J = 2.1 Hz, 1H), 7.09 - 6.90 (m, 2H), 6.02 (d, J = 1.7 Hz, 1H), 4.27 (s, 2H), 4.14 - 3.98 (m, 2H), 3.77 (s, 3H), 3.70 - 3.55 (m, 2H), 3.32 (d, J = 4.4 Hz, 5H), 3.08 - 2.92 (m, 2H), 2.83 (s, 3H), 2.24 - 1.73 (m, 6H). |
| 442 | LC-MS: (ES, m/z): RT = 0.930 min, LCMS07: m/z = 344.15 [M+1]. 1H NMR (300 MHz, Methanol-d4) δ 7.33 - 7.29 (m, 1H), 7.26 - 7.04 (m, 2H), 6.02 (d, J = 1.2 Hz, 1H), 4.63 - 4.40 (m, 2H), 4.26 - 4.03 (m, 4H), 3.93 (d, J = 1.5 Hz, 3H), 3.32 - 3.31 (m, 1H), 3.08 - 2.95 (m, 6H), 2.32 (s, 3H). |
| 444 | LC-MS: (ES, m/z): RT =0.957 min, LCMS 07: m/z = 358 [M+1]. 1H NMR (300 MHz, Methanol-d4) δ 7.35 (d, J = 14.3 Hz, 1H), 7.27 - 7.14 (m, 1H), 7.09 (q, J = 8.8 Hz, 1H), 6.01 (d, J = 1.1 Hz, 1H), 4.54 - 4.35 (m, 2H), 4.31 - 4.08 (m, 4H), 3.92 (s, 3H), 3.70 - 3.47 (m, 2H), 3.47 (s, 1H), 3.01 (s, 3H), 2.48 - 2.24 (m, 3H), 1.28 (t, J = 7.3 Hz, 3H). |
| 445 | LC-MS: (ES, m/z): RT = 1.067 min, LCMS15: m/z = 384.2 [M+1].1H NMR (400 MHz, Methanol-d4) δ 7.36 - 7.06 (m, 3H), 5.99 (s, 1H), 4.53 - 4.35 (m, 2H), 4.32 - 4.07 (m, 4H), 3.91 (s, 3H), 3.36 (d, J = 7.0 Hz, 2H), 3.18 (d, J = 7.3 Hz, 1H), 3.03 - 2.95 (m, 3H), 2.31 (s, 3H), 1.09 - 1.06 (m, 1H), 0.77 - 0.72 (m, 2H), 0.50 - 0.41 (m, 2H). |
| 446 | LC-MS: (ES, m/z): RT = 1.99 min, LCMS 33: m/z = 398.3 [M+1]. 1H NMR (300 MHz, Methanol-d4) δ 7.33 - 7.17 (m, 2H), 7.04 (d, J = 8.8 Hz, 1H), 5.98 (s, 1H), 4.26 - 3.86 (m, 6H), 3.80 - 3.46 (m, 2H), 2.99 (d, J = 11.3 Hz, 6H), 2.51 - 2.12 (m, 9H), 1.97 (q, J = 9.4 Hz, 2H). |
| 447 **(Reference)** | LC-MS: (ES, m/z): RT = 0.85 min, LCMS 33: m/z = 359.0 [M+1]. 1H NMR (400 MHz, DMSO-d6) δ 10.65 (s, 1H), 9.28 (s, 1H), 7.31 (s, 2H), 6.89 (d, J = 8.7 Hz, 1H), 5.59 (s, 1H), 3.96 (t, J = 6.5 Hz, 2H), 3.73 (s, 3H), 2.54 (d, J = 7.1 Hz, 2H), 2.42 - 2.39 (m, 4H), 2.05 (s, 3H), 1.89 - 1.84 (m, 2H), 1.68 - 1.63 (m, 4H). |
| 448 **(Reference)** | LC-MS: (ES, m/z): RT = 0.88 min, LCMS 33: m/z = 359.0 [M+1]. 1H NMR (400 MHz, DMSO-d6) δ 9.19 (s, 1H), 7.67 - 7.41 (m, 1H), 7.05 (q, J = 8.7 Hz, 1H), 6.88 (d, J = 8.7 Hz, 1H), 5.63 (s, 1H), 3.99 (t, J = 6.5 Hz, 2H), 3.72 (s, 3H), 2.57 (t, J = 7.1 Hz, 2H), 2.48 (s, 5H), 2.09 (s, 3H), 1.91 - 1.85 (m, 2H), 1.77 - 1.45 (m, 4H). |
| 449 | LC-MS: (ES, m/z): RT = 1.226 min; LCMS34: m/z = 400 [M+1]. 1H NMR (400 MHz, Methanol-d4) δ 7.35 - 7.29 (m, 1H), 7.23 (d, J = 8.7 Hz, 1H), 7.04 (d, J = 8.8 Hz, 1H), 5.98 (d, J = 1.1 Hz, 1H), 5.01 - 4.91 (m, 3H), 4.79 - 4.77 (m, 2H), 4.69 (s, 1H), 4.13 - 4.11 (m, 2H), 3.90 (s, 4H), 3.80 - 3.50 (m, 2H), 3.04 - 2.97 (m, 4H), 2.40 - 2.30 (m, 1H), 2.30 (d, J = 0.9 Hz, 3H), 2.17 (s, 1H). |
| 450 | LC-MS: (ES, m/z): RT = 1.18min, LCMS33: m/z = 427 [M+1]. 1H NMR (300 MHz, DMSO-d6) δ10.86-10.48 (m, 2H), 8.04-7.95 (m, 1H), 7.45 (d, J = 15.1 Hz, 1H), 7.08-6.91 (m, 2H), 5.04 (s, 1H), 4.07 (d, J = 6.6 Hz, 2H), 3.76 (s, 3H), 3.74-3.25 (m, 8H), 3.10-2.97 (m, 2H), 2.91 - 2.79 (m, 3H), 2.19 (q, J = 7.4, 6.8 Hz, 2H), 2.06 - 1.81 (m, 8H). |
| 451 **(Reference)** | LC-MS: (ES, m/z): RT =1.016min, LCMS 07: m/z = 442 [M+1]. 1H NMR (400 MHz, Methanol-d4) δ 7.30 - 7.12 (m, 2H), 7.05 (q, J = 4.9 Hz, 1H), 6.05 - 5.97 (m, 1H), 4.09 - 4.75 (m, 2H), 4.05 - 3.75 (m, 6H), 3.72 (m, 1H), 3.40 (q, J = 2.3 Hz, 3H), 3.28 - 3.00 (m, 5H), 2.99 - 2.89 (m, 1H), 2.54 - 2.41 (m, 1H), 2.31 (d, J = 0.9 Hz, 3H), 1.88 - 1.81 (m, 1H), 1.80 - 1.78 (m, 1H), 1.75 - 1.73 (m, 1H), 1.72 - 1.57 (m, 2H), 1.29 - 1.25 (m, 2H). |
| 452 | LC-MS: (ES, m/z): RT = 1.245 min, LCMS 33: m/z = 345 [M+1]. 1H NMR (300 MHz, Methanol-d4) δ 7.26 (s, 1H), 7.03 (d, 2H), 6.29-5.99 (m, 1H), 4.30(s, 1H), 4.11-3.82 (m, 7H), 3.00(s, 3H), 2.42-2.22 (m,3H), 2.18-1.81(m, 4H). |
| 453 **(Reference)** | LC-MS: (ES, m/z): RT = 1.25min, LCMS33: m/z = 331 [M+1]. 1H NMR (300 MHz, Methanol-d4) δ 7.76-7.50 (m, 1H), 7.16-7.11 (m, 1H), 7.10-7.01 (m, 2H), 6.39-6.1 (m, 1H), 4.2-4.02 (m, 2H), 3.93 - 3.83 (m, 5H), 3.52 - 3.44 (m, 1H), 3.06-2.95 (m, 3H), 0.67 - 0.55 (m, 2H), 0.52 (dd, J = 6.8, 4.6 Hz, 2H). |
| 456 | LC-MS: (ES, m/z): RT =0.764min, LCMS48: m/z = 412 [M+1]. 1H NMR (300 MHz, Methanol-d4) δ 7.33 (s, 1H), 7.24 - 7.20 (m, 1H), 7.04 (q, J = 3.5 Hz, 1H), 5.99 (q, J = 1.2 Hz, 1H), 4.10 - 4.06 (m, 2H), 4.01 - 3.86 (m, 4H), 3.86 - 3.57 (m, 2H), 3.54 - 3.38 (m, 1H), 3.23 - 3.18 (m, 1H), 3.00 (d, J = 12.0 Hz, 4H), 2.53 - 1.96 (m, 7H), 1.94 - 1.65 (m, 6H). |
| 458 | LC-MS: (ES, m/z): RT=3.213min, LCMS 28, m/z =426.3 [M+1]. 1H NMR (300 MHz, Methanol-d4) δ 7.35 (d, J = 2.4 Hz, 1H), 7.18 (dd, J = 8.7, 2.5 Hz, 1H), 7.05 (d, J = 8.8 Hz, 1H), 6.05 (s, 1H), 4.21 (t, J = 5.5 Hz, 2H), 3.86 (d, J = 19.0 Hz, 5H), 3.49 (t, J = 7.0 Hz, 2H), 3.25 - 2.88 (m,6H), 2.44 - 1.98 (m, 8H), 1.98 - 1.56 (m, 6H). |
| 459 | LC-MS: (ES, m/z): RT= 1.100min, LCMS 28, m/z = 442.2 [M+1]. 1H NMR (300 MHz, Deuterium Oxide) δ 7.16 (d, J = 1.8 Hz, 1H), 7.11 - 6.92 (m, 2H), 5.93 (s, 1H), 4.18 - 3.92 (m, 4H), 3.80 (s, 3H), 3.74 - 3.58 (m, 2H), 3.57 - 3.27 (m, 4H), 3.15 - 2.62 (m, 6H), 2.28 - 1.52 (m, 10H). |
| 460 **(Reference)** | LC-MS: (ES, m/z): RT = 0.998 min, LCMS 33: m/z = 383 [M+1]. 1H NMR (400 MHz, Methanol-d4) δ 8.64 (s, 1H), 8.14 (s, 1H), 7.20(s, 1H), 7.03 (s, 1H), 4.35 (t, J = 5.5 Hz, 2H), 4.00 (s, 3H), 3.82 (s, 2H), 3.50 (t, J = 7.4 Hz, 2H), 3.18 (s, 2H), 2.73 (s, 3H), 2.45-2.35 (m, 2H), 2.30-2.20 (m, 2H), 2.15-2.05 (m, 2H). |
| 461 | LC-MS: (ES, m/z): RT = 0.63 min, LCMS 53: m/z = 388.3 [M+1]. 1H NMR (300 MHz, Methanol-d4) δ 7.29 (d, J = 2.8 Hz, 1H), 7.20 (d, J = 8.5 Hz, 1H), 7.09 - 6.99 (m, 1H), 5.99 (s, 1H), 4.20 - 4.02 (m, 2H), 4.00 - 3.63 (m, 7H), 3.61 - 3.41 (m, 3H), 3.26 - 3.19 (m, 1H), 3.01 - 2.98 (m, 4H), 2.31 - 2.26 (m, 4H), 2.23 - 1.99 (m, 1H). |
| 462 **(Reference)** | LC-MS: (ES, m/z): RT =1.069min, LCMS07: m/z =468 [M+1]. 1H NMR (300 MHz, Methanol-d4) δ 7.36 - 7.11 (m, 2H), 7.03 (d, J = 8.6 Hz, 1H), 5.99 (s, 1H), 4.13 (s, 2H), 4.04 - 3.52 (m, 9H), 3.50 - 3.35 (m, 3H), 3.30 - 3.16 (m, 1H), 3.04 (d, J = 3.6 Hz, 2H), 2.31 (s, 5H), 1.88 - 1.82 (m, 1H), 1.73 - 1.52 (m, 2H), 1.44 - 1.18 (m, 2H), 1.13 - 0.68 (m, 4H). |
| 463 | LC-MS: (ES, m/z): RT=0.986 min, LCMS 07, m/z =372.20 [M+1]. 1H NMR (300 MHz, Methanol-d4) δ 7.46 - 6.91 (m, 3H), 6.02 (s, 1H), 4.19 (t, J = 5.6 Hz, 2H), 3.90 (s, 3H), 3.75 - 3.45 (m, 2H), 3.18 - 2.76 (m, 7H), 2.53 - 2.12 (m, 5H), 1.31 - 0.86 (m, 4H). |
| 464 **(Reference)** | LC-MS: (ES, m/z): RT = 1.128 min, LCMS 33: m/z = 456 [M+1]. 1H NMR (300 MHz, Methanol-d4) δ 7.96 (s, 1H),6.52-6.61 (m, 1H),6.20-6.40 (m, 1H), 5.90-6.08 (m, 1H), 4.28 (t, J = 5.6 Hz, 2H), 4.05 - 3.90 (m,5H), 3.81 (s, 2H), 3.52 - 3.41 (m, 4H), 3.21-3.11 (s, 4H), 2.59-2.40 (m, 3H), 2.35 (t, J = 6.6 Hz, 2H), 2.22 (s, 2H), 2.10 (s, 2H), 1.93 (s, 1H), 1.74 (s, 2H), 1.40 (s, 2H). |
| 465 | LC-MS: (ES, m/z): RT = 1.68mm, LCMS28: m/z = 374 [M+1]. 1H NMR (300 MHz, Methanol-d4) δ 7.29 (d, J = 2.4 Hz, 1H), 7.20 (dd, J = 8.7, 2.6 Hz, 1H), 7.06-7.04 (m, 1H), 6.2-5.99 (m, 1H), 4.76 - 4.52 (m, 2H), 4.39 - 4.11 (m, 4H), 4.05-3.85 (m, 4H), 3.61-3.43 (m, 2H), 3.05-2.9 (m, 3H), 2.45-2.23 (m, 3H), 2.20-2.06 (m, 2H). |
| 466 | LC-MS: (ES, m/z): RT = 1.64min, LCMS33: m/z = 384 [M+1]. 1H NMR (300 MHz, Methanol-d4) δ 7.40 - 7.27 (m, 2H), 7.12-6.95 (d, J = 9.1 Hz, 1H), 6.25-5.97 (m, 1H), 4.89-4.67 (m, 1H), 3.90 (d, J = 4.4 Hz, 3H), 3.77-3.45 (m, 4H), 3.09-2.94 (m, 4H), 2.40 (s, 1H), 2.33-2.23 (m, 4H), 2.14-1.9 (m, 2H), 1.25-0.9 (m,4H). |
| 473 **(Reference)** | LC-MS: (ES, m/z): RT = 0.962min, LCMS28: m/z = 359 [M+1]. 1H NMR (300 MHz, Methanol-d4) δ 8.05 (s, 1H), 7.09 (s, 1H), 6.30 (s, 1H), 4.60 - 4.24 (m, 2H), 4.12 - 3.62 (m, 5H), 3.44 - 3.34 (m, 1H), 3.28 - 2.95 (m, 8H), 2.78 (s, 3H), 2.60 - 2.30 (m, 1H), 2.27 - 1.85 (m, 1H). |
| 478 **(Reference)** | LC-MS: (ES, m/z): RT = 1.062 min, LCMS 53: m/z = 368.2 [M+1]. 1H NMR (400 MHz, DMSO-d6) δ 13.63 (s, 1H), 8.74 (s, 1H), 7.78 (s, 1H), 7.71 (s, 1H), 7.45 - 7.19 (m, 1H), 7.19 - 6.95 (m, 1H), 6.95 - 6.66 (m, 1H), 5.87 (d, J = 5.8 Hz, 1H), 4.88 (s, 2H), 3.69 (s, 3H), 2.82 (s, 3H), 2.04 (dd, J = 9.7, 5.2 Hz, 1H), 1.13 - 0.97 (m, 2H), 0.90 (d, J = 5.9 Hz, 5H). |
| 480 **(Reference)** | LC-MS: (ES, m/z): RT = 1.114 min ; m/z = 368.3 [M+1]. 1H NMR (300 MHz, Deuterium Oxide) δ 8.75 - 8.38 (m, 1H), 7.28 (dd, J = 7.3, 4.6 Hz, 1H), 7.17 - 7.00 (m, 1H), 6.86 - 6.66 (m, 2H), 6.12 -5.89 (m, 1H), 4.98 - 4.75 (m, 2H), 3.75 - 3.42 (m, 4H), 2.85 - 2.46 (m, 3H), 1.09 - 0.93 (m, 4H). |
| 481 | LC-MS: (ES, m/z): RT = 1.03 min, LCMS 33: m/z = 384.3 [M+1]. 1H NMR (300 MHz, Methanol-d4) δ 7.37 (d, J = 2.5 Hz, 1H), 7.13 (q, J = 8.7, 1H), 6.99 (d, J = 8.7 Hz, 1H), 5.94 (s, 1H), 4.05 - 4.01 (m, 2H), 3.86 (s, 3H), 3.48 (t, J = 9.9 Hz, 1H), 3.30 - 3.12 (m, 2H), 2.98 (s, 3H), 2.88 (q, J = 7.2 Hz, 1H), 2.55 (d, J = 8.4 Hz, 1H), 2.27 (s, 4H), 1.93 - 1.91 (m, 6.9 Hz, 1H), 0.80 (q, J = 5.1 Hz, 4H). |
| 482 | LC-MS: (ES, m/z): RT = 1.20 min, LCMS 27: m/z = 384.0 [M+1]. 1H NMR (300 MHz, Methanol-d4) δ 7.49 (s, 1H), 7.09 (q, J = 8.7 Hz, 1H), 7.00 - 6.91 (m, 1H), 5.87 (d, J = 2.9 Hz, 1H), 4.09 - 3.96 (m, 2H), 3.84 (s, 3H), 3.26 - 2.69 (m, 8H), 2.22 (s, 5H), 1.79 - 1.69 (m, 1H), 0.63 (q, J = 7.7 Hz, 4H). |
| 483 | LC-MS: (ES, m/z): RT = 1.16min, LCMS28: m/z = 400 [M+1]. 1H NMR (300 MHz, Deuterium Oxide) δ 7.27 (s, 1H), 6.92 (s, 2H), 5.84 (s, 1H), 4.14 - 4.02 (m, 2H), 3.75 (s, 3H), 3.26 (d, J = 7.8 Hz, 6H), 2.75 (s, 3H), 2.62 - 2.52 (m, 1H), 2.21 - 2.05 (m, 2H), 1.97 (s, 4H), 1.10 (d, J = 6.9 Hz, 6H). |
| 486 **(Reference)** | LC-MS: (ES, m/z): RT = 0.902 min, LCMS33: m/z = 382 [M+1]. 1H NMR (400 MHz, Methanol-d4) δ 8.08 (s, 1H), 7.81 (d, J = 2.4 Hz, 1H), 7.21 - 7.19 (m, 1H), 6.94 (d, J = 8.8 Hz, 1H), 6.83 (d, J = 0.8 Hz, 1H), 4.14 (t, J = 6.0 Hz, 2H), 3.84 (s, 3H), 2.82 - 2.73 (m, 2H), 2.66 (d, J = 4.8 Hz, 4H), 2.50 (d, J = 0.8 Hz, 3H), 2.16 - 2.04 (m, 2H), 1.89 - 1.85 (m, 4H). |
| 490 | LC-MS: (ES, m/z): RT = 0.969 min, LCMS 33: m/z = 358 [M+1]. 1H NMR (300 MHz, Methanol-d4) δ 7.25 (s, 1H), 7.20 - 7.11 (m, 1H),7.07 - 6.95 (s, 1H), 6.03 - 5.96 (m, 1H), 4.39 - 4.28 (m, 2H), 4.25 - 4.08 (m, 4H), 3.95 (s, 3H), 3.64 - 3.48 (m, 2H), 3.01 (s, 3H), 2.65 - 2.51 (m, 1H), 2.52 - 2.38 (m, 1H), 2.41 - 2.22 (m, 3H), 2.14 - 2.00 (m , 2H). |
| 496 | LC-MS: (ES, m/z): RT = 1.471min, m/z = 406.2 [M+1]. 1H NMR (300 MHz, Methanol-d4) δ 7.45 (d, J = 2.4 Hz, 1H), 7.41 - 7.21 (m, 1H), 6.23 - 6.07 (m, 1H), 4.23 (q, J = 5.9 Hz, 2H), 3.90 - 3.70 (m, 5H), 3.53 - 3.42 (m, 2H), 3.18 (m, 2H), 3.02 (s, 3H), 2.46 - 2.03 (m, 9H). |
| 497 | LC-MS: (ES, m/z): RT = 2.041 min, LCMS33: m/z = 370 [M+1]. 1H NMR (300 MHz, Methanol-d4) δ 7.29 - 7.09 (m, 3H), 6.29 - 5.89 (m, 1H), 4.17 (t, J = 5.7 Hz, 2H), 3.82 - 3.70 (m, 2H), 3.53 - 3.41 (m, 2H), 3.18 - 3.04 (m, 2H), 3.04 (s, 3H), 2.68 (q, J = 7.5 Hz, 2H), 2.45 - 2.17 (m, 7H), 2.17 - 2.03 (m, 2H), 1.29 - 1.14 (m, 3H). |
| 498 | LC-MS: (ES, m/z): RT= 1.120 min, LCMS 28, m/z =398.2 [M+1]. 1H NMR (400 MHz, Methanol-d4) δ 7.30 (d, J = 2.4 Hz, 1H), 7.25 - 7.18 (m, 1H), 7.05 (d, J = 8.8 Hz, 1H), 5.99 (d, J = 1.2 Hz, 1H), 4.20 (t, J = 5.5 Hz, 2H), 4.04 - 3.85 (m, 4H), 3.62 - 3.36 (m, 4H), 3.29 - 3.25 (m, 1H), 3.01 (s, 3H), 2.51 - 1.89 (m, 7H), 0.95 - 0.60 (m, 4H). |
| 502 | LC-MS: (ES, m/z): RT = 0.98min, LCMS33: m/z = 360 [M+1]. 1H NMR (300 MHz, Methanol-d4) δ 7.27 (s, 1H), 7.16 (dd, J = 8.7, 2.5 Hz, 1H), 7.02 (d, J = 8.8 Hz, 1H), 5.98 (s, 1H), 4.11 (t, J = 5.6 Hz, 2H), 3.88 (s, 3H), 3.35 - 3.30 (m, 2H), 2.97 - 2.88 (m, 9H), 2.30 (s, 3H), 2.06 - 1.89 (m, 4H). |
| 503 | LC-MS: (ES, m/z): RT = 1.140 min; LCMS27: m/z = 372 [M+1]. 1H NMR (400 MHz, Methanol-d4) δ 7.25 (d, J = 2.9 Hz, 1H), 7.16 (d, J = 8.6 Hz, 1H), 7.02 (d, J = 8.7 Hz, 1H), 5.98 (s, 1H), 4.16 - 4.13 (m, 1H), 4.13 - 4.05 (m, 1H), 3.87 (s, 4H), 3.79 - 3.62 (m, 1H), 3.54 - 3.42 (m, 1H), 3.26 - 3.05 (m, 1H), 3.04 - 2.77 (m, 7H), 2.75 - 2.55 (m, 1H), 2.35 (s, 3H), 2.10 - 1.75 (m, 3H). |
| 504 | LC-MS: (ES, m/z): RT= 1.03 min, LCMS 15:m/z = 386 [M+1]. 1H-NMR: (Methanol-d4, ppm): δ 7.34 - 7.26 (m, 1H), 7.16 (dd, J = 8.9, 2.3 Hz, 1H), 7.05 (d, J = 8.7 Hz, 1H), 6.02 (s, 1H), 4.35-4.18 (m, 2H), 3.98 - 3.78 (m, 4H), 3.74-3.65 (m, 1H), 3.65-3.50 (m, 1H), 3.42-3.08 (m, 2H), 3.05-2.99 (m, 3H), 2.30-2.40 (m, 6H), 2.24 - 2.05 (m, 2H), 1.93-1.75 (m, 1H), 1.61-1.51 (m, 3H). |
| 506 | LC-MS: (ES, m/z): RT = 1.03min, LCMS33: m/z = 384 [M+1]. 1H NMR (300 MHz, Methanol-d4) δ 7.37 - 7.17 (m, 2H), 7.11 - 7.01 (m, 1H), 5.98 (s, 1H), 4.17 (d, J = 5.4 Hz, 2H), 3.91 - 3.81 (m, 5H), 3.51 - 3.77 (m, 4H), 3.00 (s, 3H), 2.44 - 2.17 (m, 5H), 2.03 - 1.89 (m, 2H), 0.92 (q, J = 7.6 Hz, 1H), 0.73-0.58 (m, 1H). |
| 507 **(Reference)** | LC-MS: (ES, m/z): RT = 1.023min; LCMS33: m/z = 380 [M+1]. 1H NMR (300 MHz, Methanol-d4) δ 8.06 (s, 1H), 7.90 (d, J = 7.0 Hz, 1H), 7.61 (d, J = 3.4 Hz, 1H), 7.38 (d, J = 0.9 Hz, 1H), 7.26 (d, J = 0.9 Hz, 1H), 7.01 (s, 1H), 4.30 (t, J = 5.4 Hz, 2H), 3.99 (s, 3H), 3.97 - 3.80 (m, 2H), 3.48 - 3.40 (m, 4H), 2.35 - 2.33 (m, 2H), 1.91 - 1.89 (m, 2H), 1.01 - 0.87 (m, 1H), 0.73 - 0.62 (m, 1H). |
| 508 | LC-MS: (ES, m/z): RT = 0.97 min, LCMS 53: m/z = 358.0 [M+1]. 1H NMR (300 MHz, Methanol-d4) δ 7.23 (t, J = 3.1 Hz, 1H), 7.15 (t, J = 8.9 Hz, 1H), 7.01 (t, J = 1.3 Hz, 1H), 6.01 - 5.96 (m, 1H), 4.45 - 4.25 (m, 2H), 4.09 (q, J = 5.8 Hz, 3H), 3.97 (q, J = 9.3 Hz, 1H), 3.87 (d, J = 2.1 Hz, 3H), 3.27 - 3.06 (m, 1H), 2.99 (d, J = 5.0 Hz, 4H), 2.92 (s, 2H), 2.30 (d, J = 0.9 Hz, 3H), 2.25 - 2.11 (m, 2H). |
| 509 **(Reference)** | LC-MS: (ES, m/z): RT = 1.102 min; LCMS53: m/z = 402 [M+1]. 1H NMR (400 MHz, Methanol-d4) δ 7.93 (s, 1H), 6.62 (s, 1H), 6.09 (d, J = 6.5 Hz, 1H), 4.25 (t, J = 5.5 Hz, 2H), 3.94 (s, 3H), 3.89 - 3.87 (m, 2H), 3.50 - 3.38 (m, 4H), 2.96 (s, 3H), 2.50 (d, J = 3.1 Hz, 3H), 2.37 - 2.26 (m, 2H), 1.95 - 1.86 (m, 2H), 0.91 (q, J = 7.7 Hz, 1H), 0.69 - 0.62 (m, 1H). |
| 510 | LC-MS: (ES, m/z): RT = 1.075 min, LCMS28: m/z = 370 [M+1]. 1H-NMR: (300 MHz, Methanol-d4) δ 7.25-6.95 (m, 3H), 6.10 - 5.86 (m, 1H), 4.49 - 3.93 (m, 6H), 3.79 (s, 3H), 3.34 - 2.98 (m, 2H), 2.82 (s, 3H), 2.14 (s, 3H), 0.93 - 0.62 (m, 4H). |
| 514 **(Reference)** | LC-MS: (ES, m/z): RT = 1.197 min, LCMS 27: m/z = 385 [M+1]. 1H-NMR: (300 MHz, Methanol-d4) δ 7.95 (d, J = 2.2 Hz, 1H), 7.81 (s, 1H), 5.82 (d, J = 0.8 Hz, 1H), 4.05 (t, J = 6.2 Hz, 2H), 3.91 (s, 3H), 3.08 (d, J = 9.1 Hz, 2H), 2.91 (s, 3H), 2.74 - 2.64 (m, 2H), 2.50 - 2.44 (m, 2H), 2.18 (s, 3H), 2.04 - 1.94 (m, 2H), 1.49 - 1.41 (m, 2H), 0.70 - 0.32 (m, 1H), 0.46 - 0.38 (m, 1H). |
| 515 **(Reference)** | LC-MS: (ES, m/z): RT =0.870min, LCMS 33: m/z = 371 [M+1]. 1H NMR (300 MHz, Methanol-d4) δ 8.44 (s, 1H), 7.91 (d, J = 2.0 Hz, 1H), 5.98 (s, 1H), 4.20 (q, J = 6.2 Hz, 2H), 3.71 - 3.41 (m, 6H), 3.00 (s, 3H), 2.83 (q, J = 7.5 Hz, 2H), 2.41 - 2.25 (m, 5H), 2.20 - 2.09 (m, 4H), 1.26 (t, J = 7.6 Hz, 3H). |
| 517a | LC-MS: (ES, m/z): RT=1.041 min, LCMS 53, m/z =398 [M+1]. 1H NMR (300 MHz, Methanol-d4) δ 7.29 (d, J = 2.4 Hz, 1H), 7.20 - 6.98 (m, 2H), 6.01 (s, 1H), 4.12 - 3.81 (m, 6H), 3.75- 3.63 (m, 1H), 3.24 - 3.08 (m, 2H), 3.01 (s, 3H), 2.94 - 2.81 (m, 1H), 2.46 - 2.35 (m, 1H), 2.31 (d, J = 0.9 Hz, 3H), 2.14 - 1.78 (m, 3H), 1.63 - 1.43 (m, 1H), 1.17 - 0.95 (m, 4H). |
| 517b | LC-MS: (ES, m/z): RT = 1.094min, LCMS28: m/z = 398.2 [M+1]. 1H NMR (400 MHz, Deuterium Oxide) δ 7.28 - 6.78 (m, 3H), 6.19 - 5.72 (m, 1H), 3.96 (dd, J = 9.8, 4.7 Hz, 1H), 3.85 (dd, J = 9.8, 7.4 Hz, 1H), 3.80 - 3.70 (m, 4H), 3.58 (d, J = 13.7 Hz, 1H), 3.10 - 2.89 (m, 2H), 2.82 (s, 3H), 2.72 - 2.62 (m, 1H), 2.38 - 2.04 (m, 4H), 1.95 (s, 3H), 1.83 (d, J = 13.1 Hz, 1H), 1.70 - 1.56 (m, 1H), 1.44 - 1.20 (m, 1H), 0.94 - 0.75 (m, 4H). |
| 518 **(Reference)** | LC-MS: (ES, m/z): RT = 1.22min, m/z = 385 [M+1]. 1H NMR (300 MHz, Methanol-d4) δ 7.59 (s, 1H), 7.48-7.05 (m, 1H), 4.37 (t, J = 5.4 Hz, 2H), 4.20 - 3.80 (m, 4H), 3.92 - 3.72 (m, 2H), 3.50 (t, J = 7.3 Hz, 2H), 3.25 - 3.10 (m, 5H), 2.61 - 1.60 (m, 14H). |
| 523 **(Reference)** | LC-MS: (ES, m/z): RT = 1.27 min, LCMS 53: m/z = 399.0 [M+1]. 1H NMR (300 MHz, Methanol-d4) δ 7.54 (s, 1H), 7.31- 7.20 (m, 1H), 4.37 (t, J = 5.5 Hz, 2H), 4.03 (s, 3H), 3.84 - 3.82 (m, 2H), 3.71 - 3.44 (m, 3H), 3.18 (s, 5H), 2.40 - 2.36 (m, 2H), 2.32 - 2.03 (m, 4H), 2.04 - 1.51 (m, 9H), 1.39 - 1.40 (m, 1H). |
| 527 **(Reference)** | LC-MS: (ES, m/z): RT = 0.932min, LCMS28: m/z = 454.3 [M+1]. 1H-NMR: (CDCl3, ppm): 1H NMR (300 MHz, Deuterium Oxide) δ 7.43-7.31 (m, J = 11.7 Hz, 1H), 7.09 (s, 1H), 4.30-4.20 (m, 2H), 3.83-3.90 (m, 3H), 3.80 (s, 3H), 3.71 - 3.60 (m, 3H), 3.56 (d, J = 10.7 Hz, 2H), 3.30-3.39 (m, 3H), 3.24 - 3.11 (m, 2H), 3.11 - 2.94 (m, 2H), 2.80-2.90 (m, 3H), 2.30-2.20 (m, 3H), 2.20 - 2.02 (m, 7H), 2.00 - 1.84 (m, 4H). |
| 528 **(Reference)** | LC-MS: (ES, m/z): RT= 0.69 min, LCMS 15:m/z = 504 [M+1]. 1H-NMR: (Methanol-d4, ppm): δ 7.55 (s, 1H), 7.33 (s, 1H), 4.37 (t, J = 5.5 Hz, 2H), 4.26 - 4.15 (m, 4H), 4.05 (s, 3H), 3.98 - 3.89 (m, 1H), 3.85 - 3.81 (m, 2H), 3.71 - 3.70 (m, 2H), 3.50 (t, J = 7.2 Hz, 2H), 3.34 - 3.32 (m, 1H), 3.32 - 3.30 (m, 3H), 2.98 (s, 3H), 2.45 - 2.03 (m, 14H). |
| 529 **(Reference)** | LC-MS: (ES, m/z): RT=4.170min LCMS53, m/z =468 [M+1]. 1H NMR (300 MHz, Methanol-d4) δ 7.76 (s, 1H), 7.29 (s, 1H), 4.60 - 4.22 (m, 3H), 4.12 - 3.89 (m, 4H), 3.88 - 3.76 (m, 2H), 3.75 - 3.62 (m, 2H), 3.61 - 3.39 (m, 3H), 3.23 - 3.05 (m, 2H), 2.96 (s, 3H), 2.45 - 1.58 (m, 19H). |
| 538 **(Reference)** | LC-MS: (ES, m/z): RT = 0.666min, m/z = 368.2 [M+1]: 1H NMR (300 MHz, Methanol-d4) δ 8.60 (s, 1H), 7.83 (s, 1H), 7.72 (d, J = 6.9 Hz, 1H), 6.85 (s, 1H), 6.30 (d, J = 6.8 Hz, 1H), 4.38 (s, 2H), 3.78 (s, 2H), 3.55 (s, 2H), 3.21 (s, 2H), 3.14 (s, 3H), 2.41 (s, 2H), 2.22 - 2.10 (m, 4H). |
| 541 **(Reference)** | LC-MS: (ES, m/z): RT = 1.64 min, LCMS 27: m/z = 208.0 [M+1]. 1H NMR (300 MHz, Methanol-d4) δ 7.64 (d, J = 6.9 Hz, 1H), 6.07 (d, J = 6.9 Hz, 1H), 4.55 - 4.45 (m, 1H), 3.96 - 3.92 (m, 1H), 3.63 - 3.51 (m, 1H), 3.49 - 3.33 (m, 2H), 2.95 (s, 3H), 2.27 - 2.17 (m, 1H), 1.97 - 1.91 (m,1H), 1.89 - 1.69 (m, 2H). |
| 542 **(Reference)** | LC-MS: (ES, m/z): RT = 1.21 min, LCMS 27: m/z = 208.0 [M+1]. 1H NMR (300 MHz, Methanol-d4) δ 7.67 (d, J = 6.0 Hz, 1H), 5.76 (d, J = 6.0 Hz, 1H), 4.69 - 4.57 (m, 2H), 2.95 - 2.83 (m, 6H), 1.89 - 1.85 (m, 2H), 1.37 - 1.25 (m, 2H). |
| 543 **(Reference)** | LC-MS: (ES, m/z): RT = 1.15 min, LCMS 07: m/z = 181.1 [M+1]. 1H NMR (300 MHz, Methanol-d4) δ 7.61 (d, J = 6.0 Hz, 1H), 5.77 (d, J = 6.0 Hz, 1H), 3.34 (t, J = 4.5 Hz, 1H), 3.32 (t, J = 1.5 Hz, 1H), 2.87 (s, 3H), 1.63 - 1.53 (m, 2H), 1.48 - 1.36 (m, 2H), 0.98 (t, J = 7.2 Hz, 3H). |
| 545 **(Reference)** | LC-MS: (ES, m/z): RT = 0.86min, LCMS15: m/z = 250 [M+1]. 1H-NMR (300 MHz, Methanol-d4) δ 7.67 (d, J = 6.0 Hz, 1H), 5.75 (d, J = 6.0 Hz, 1H),4.07-3.88 (m, 2H), 3.76-3.54(m, 2H), 2.86 (s, 3H), 1.74 - 1.30 (m, 8H), 1.04-0.80 (m, 3H). |
| 546 **(Reference)** | LC-MS: (ES, m/z): RT = 1.31 min, LCMS 27: m/z = 222.0 [M+1]. 1H NMR (300 MHz, Methanol-d4) δ 7.67 (d, J = 6.0 Hz, 1H), 5.76 (d, J = 6.0 Hz, 1H), 3.86 - 3.80 (m, 1H), 3.69 - 3.58 (m, 1H), 3.57 - 3.48 (m, 2H), 2.88 (s, 3H), 1.76 - 1.55 (m, 4H), 1.14 (s, 3H). |
| 547 **(Reference)** | LC-MS: (ES, m/z): RT = 0.67 min, LCMS 27: m/z = 222.0 [M+1]. 1H NMR (300 MHz, Methanol-d4) δ 7.67 (d, J = 6.0 Hz, 1H), 5.76 (d, J = 6.0 Hz, 1H), 3.92 - 3.84 (m, 2H), 3.71 - 3.62 (m, 2H), 2.87 (s, 3H), 1.61 - 1.49 (m, 4H), 1.22 (s, 3H). |
| 548 **(Reference)** | LC-MS: (ES, m/z): RT = 0.98 min, LCMS28: m/z = 208.1 [M+1]. 1H NMR (300 MHz, Methanol-d4) δ 7.62 (s, 1H), 5.89 (d, J = 6.3 Hz, 1H), 4.31 (t, J = 7.2 Hz, 1H), 3.70 - 3.55 (m, 1H), 2.91 (s, 3H), 2.68 - 2.56 (m, 1H), 2.18 - 2.12 (m, 2H), 1.99 - 1.88 (m, 2H), 1.62 - 1.55 (m, 1H). |
| 549 **(Reference)** | LC-MS: (ES, m/z): RT = 1.12 min, LCMS 27: m/z = 278.1 [M+1]. 1H NMR (300 MHz, Methanol-d4) δ 7.62 (d, J = 7.2 Hz, 1H), 6.17 (d, J = 7.2 Hz, 1H), 4.14 - 4.02 (m, 2H), 3.79 - 3.71 (m, 1H), 3.68 - 3.54 (m, 1H), 3.34 - 3.32 (m, 2H), 3.24 - 3.01 (m, 3H), 2.00 - 1.91 (m, 4H), 1.72 - 1.65 (m, 2H), 1.52 - 1.39 (m, 5H), 1.02 (t, J = 7.3 Hz, 3H). |
| 550 **(Reference)** | LC-MS: (ES, m/z): RT = 1.13 min, LCMS 27: m/z = 278.1 [M+1]. 1H NMR (400 MHz, Methanol-d4) δ 7.68 (s, 1H), 5.78 (d, J = 6.0 Hz, 1H), 4.32 - 4.25 (m, 2H), 3.67 - 3.27 (m, 2H), 2.87 (s, 3H), 2.71 - 2.65 (m, 2H), 1.65 (t, J = 5.1 Hz, 4H), 1.59 - 1.49 (m, 2H), 1.49 - 1.39 (m, 2H), 1.29 (s, 3H), 0.98 (t, J = 7.2 Hz, 3H). |
| 551 | LC-MS: (ES, m/z): RT = 1.04 min, LCMS33: m/z = 387 [M+1]. 1H NMR (300 MHz, Deuterium Oxide) δ 7.18 (d, J = 2.0 Hz, 1H), 7.08 - 6.95 (m, 2H), 6.04-5.89 (m, 1H), 4.07 (t, J = 5.5 Hz, 2H), 3.85 - 3.77 (m, 3H), 3.68 (t, J = 8.1 Hz, 1H), 3.35-3.25 (m, 1H), 3.05-2.98 (m, 1H), 2.84 (d, J = 2.1 Hz, 3H), 2.69 (s, 3H), 2.35-2.19 (m, 3H), 2.19-2.07 (m, 6H), 1.72 (q, J = 9.3 Hz, 2H). |
| 552 **(Reference)** | LC-MS: (ES, m/z): RT = 0.681min, LCMS 27: m/z = 306 [M+1]. 1H-NMR (300 MHz, Methanol-d4) δ 8.27 (s, 2H), 4.20 - 4.03 (m, 3H), 3.74 - 3.69 (m, 2H), 3.55 - 3.37 (m, 4H), 3.25 - 3.06 (m, 4H), 2.33 - 2.13 (m, 6H), 2.07 - 2.03 (m, 2H), 1.93 - 1.73 (m, 2H). |
| 553 **(Reference)** | LC-MS: (ES, m/z): RT = 0.87 min, LCMS 33: m/z = 307 [M+1]. 1H NMR (400 MHz, Methanol-d4) δ 8.24 (s, 2H), 4.17 - 3.94 (m, 4H), 3.92 - 3.66 (m, 2H), 3.49 - 3.36 (m, 2H), 3.30 (d, J = 7.0 Hz, 2H), 3.27 - 2.88 (m, 6H), 2.50 - 2.22 (m, 1H), 2.19 - 1.86 (m, 2H), 1.71 - 1.58 (m, 2H), 1.33 - 1.08 (m, 2H). |
| 554 **(Reference)** | LC-MS: (ES, m/z): RT= 1.24 min, LCMS 28:m/z = 291 [M+1]. 1H-NMR: (Methanol-d4, ppm): δ 7.94 - 7.78 (m, 1H), 7.56 (t, J = 3.1 Hz, 1H), 7.19 - 7.11 (m, 1H), 4.20 - 3.98 (m, 3H), 3.92 - 3.65 (m, 2H), 3.58 - 3.44 (m 2H), 3.32 - 3.14 (m, 3H), 3.14 - 2.93 (m, 5H), 2.53 - 2.24 (m, 3H), 2.20 - 1.81 (m, 3H). |
| 555 **(Reference)** | LC-MS: (ES, m/z): RT = 0.80 min, LCMS 53: m/z = 292.3 [M+1]. 1H NMR (400 MHz, Methanol-d4) δ 8.20 (s, 2H), 4.17 - 3.95 (m, 3H), 3.94 - 3.66 (m, 2H), 3.46 (q, J = 3.8 Hz, 2H), 3.29 - 2.83 (m, 8H), 2.48 - 1.97 (m, 4H), 1.85 - 1.73 (m, 2H). |
| 556 **(Reference)** | LC-MS: (ES, m/z): RT =0.763min, LCMS33: m/z = 318 [M+1]. 1H-NMR (400 MHz, Methanol-d4) δ 8.20 (s, 2H), 4.04 - 3.99 (m, 3H), 3.76 (s, 2H), 3.46 - 3.41 (m, 3H), 3.15 - 3.05 (m, 3H), 2.95 - 8.83 (m, 2H), 2.50 - 2.00 (m, 4H), 1.95 - 1.76 (m, 2H), 1.13 - 0.90 (m, 4H). |
| 557 **(Reference)** | LC-MS: (ES, m/z): RT =1.315min, LCMS 28: m/z =319 [M+1]. 1H NMR (300 MHz, Methanol-d4) δ 8.09 (s, 2H), 3.94-3.89 (m, 5H), 3.54-3.49 (m, 2H), 2.97 - 2.93 (m, 1H), 2.90 - 2.52 (m, 4H), 2.15 - 1.91 (m, 3H), 1.83 - 1.48 (m, 4H), 0.59 - 0.40 (m, 4H). |
| 559 | LC-MS: (ES, m/z): RT = 1.030 min, LCMS 33: m/z = 372 [M+1]. 1H NMR (300 MHz, Methanol-d4) δ 7.40 - 7.29 (m, 1H), 7.27 - 7.11 (m, 1H), 7.11 - 6.99 (m, 1H), 6.29 -5.99 (m, 1H), 4.45 - 4.08 (m, 6H), 3.90 (s, 3H), 3.88 - 3.55 (m, 1H), 3.21 - 3.11 (m, 1H), 3.09 - 2.91 (m, 3H), 2.44- 2.28 (m, 3H), 1.40 -1.21 (m, 6H). |
| 563 | LC-MS: (ES, m/z): RT = 1.05 min, LCMS 53: m/z = 398 [M+1]. 1H NMR (400 MHz, Methanol-d4) δ 7.59 (s, 1H), 7.09 - 6.96 (m, 1H), 6.90 (q, J = 8.7, 1H), 5.81 (s, 1H), 3.99 - 3.96 (m, 2H), 3.83 (d, J = 1.1 Hz, 3H), 3.30 - 2.96 (m, 2H), 2.94 (s, 3H), 2.80 (q, J = 8.0 Hz, 3H), 2.63 (s, 1H), 2.19 (s, 3H), 2.17 - 1.97 (m, 5H), 1.89 - 1.68 (m, 3H). |
| 565 **(Reference)** | LC-MS: (ES, m/z): RT = 0.926 min, LCMS 33: m/z = 382 [M+1]. 1H NMR (300 MHz, Methanol-d4) δ 7.64 - 7.50 (m, 1H), 7.21 - 7.09 (m, 3H), 6.90 (s, 1H), 4.28 - 4.18 (m, 2H), 3.96 (s, 3H), 3.88 - 3.71 (m, 2H), 3.57 - 3.48 (m, 2H), 3.21 - 3.08 (m, 2H), 2.53 (s, 3H), 2.31 - 2.10 (m, 6H). |
| 570 | LC-MS: (ES, m/z): RT = 0.999 min, LCMS07: m/z = 386.25 [M+1]. 1H NMR (300 MHz, Methanol-d4) δ 7.59 (s, 1H), 7.12 - 7.05 (m, 1H), 6.90 - 6.86 (m, 1H), 5.80 (s, 1H), 4.00 - 3.91 (m, 2H), 3.81 (d, J = 1.7 Hz, 3H), 3.10 - 2.92 (m, 4H), 2.77 - 2.70 (m, 3H), 2.55 - 2.44 (m, 2H), 2.17 - 2.06 (m, 4H), 1. 70 - 1.66 (m, 1H), 1.22 - 1.10 (m, 6H). |
| 571 | LC-MS: (ES, m/z): RT = 1.000 min, LCMS33: m/z = 372 [M+1]. 1H NMR (400 MHz, Methanol-d4) δ 7.60 (s, 1H), 7.09 - 7.06 (m, 1H), 6.89 (d, J = 8.7 Hz, 1H), 5.81 (d, J = 0.8 Hz, 1H), 4.04 - 3.90 (m, 2H), 3.82 (s, 3H), 2.94 (s, 4H), 2.75 - 2.72 (m, 2H), 2.72 - 2.49 (m, 4H), 2.18 (s, 3H), 2.12 - 2.09 (m, 1H), 1.71 - 1.66 (m, 1H), 1.18 (t, J = 7.2 Hz, 3H). |
| 576 | LC-MS: (ES, m/z): RT=1.784min, LCMS 33, m/z =384 [M+1]. 1H NMR (300 MHz, Methanol-d4) δ 7.37 - 7.26 (m, 1H), 7.27 - 7.03 (m, 2H), 6.00 (d, J = 1.1 Hz, 1H), 4.48 - 4.03(m, 7H), 3.97- 3.84 (m, 3H), 3.32- 3.26 (m, 1H), 2.99 (s, 3H), 2.43 - 2.14 (m, 7H), 2.00 - 1.88 (m, 2H). |
| 580 | LC-MS: (ES, m/z): RT= 0.96 min, LCMS 27:m/z = 400 [M+1]. 1H-NMR: (Methanol-d4, ppm): δ 7.59 (s, 1H), 7.15-7.01 (m, 1H), 6.90 (d, J = 8.7 Hz, 1H), 5.82 (s, 1H), 4.13 (d, J = 6.2 Hz, 2H), 4.00 - 3.58 (m, 7H), 3.57 - 3.44 (m, 2H), 3.31 - 3.14 (m, 3H), 2.94-2.92 (m, 4H), 2.19 (s, 3H), 2.10-1.90 (m, 1H), 1.82-1.71 (m, 1H). |
| 590 **(Reference)** | LC-MS: (ES, m/z): RT = 1.073min, LCMS53: m/z = 456.4 [M+1]. 1H NMR (300 MHz, Deuterium Oxide) δ 7.29 - 6.72 (m, 3H), 6.22 - 5.67 (m, 1H), 4.06 (t, J = 5.6 Hz, 2H), 3.90 - 3.70 (m, 5H), 3.68 - 3.58 (m, 2H), 3.41 - 3.18 (m, 4H), 3.12 (d, J = 6.9 Hz, 2H), 3.06 - 2.92 (m, 2H), 2.02 - 1.62 (m, 10H), 1.63 - 1.32 (m, 2H), 1.26 - 1.00 (m, 2H). |
| 524 **(Reference)** | LC-MS: (ES, m/z): RT=1.553 min, LCMS53, m/z=400 [M+1]. 1H NMR: (300 MHz, Methanol-d4) δ 7.45 (d, J = 4.3 Hz, 2H), 6.82 (s, 1H), 4.32 (t, J = 5.5 Hz, 2H), 4.17 - 3.97 (m, 5H), 3.90 - 3.69 (m, 4H), 3.67 - 3.37 (m, 3H), 3.16 (s, 5H), 2.47 - 1.75 (m, 10H). |
| 535 **(Reference)** | LC-MS: (ES, m/z): RT=0.825 min,LCMS53, m/z=413 [M+1]. 1H NMR (300 MHz, Methanol-d4) δ 7.60 (s, 1H), 7.49 (s, 1H), 6.89 (s, 1H), 4.36 (t, J = 5.6 Hz, 2H), 4.06 (s, 3H), 3.91 - 3.64 (m, 5H), 3.58 -3.41(m, 4H), 3.26 - 3.07 (m, 5H), 2.98 (s, 3H), 2.47 - 1.99 (m, 10H). |
| 599 **(Reference)** | LC-MS: (ES, m/z): RT = 1.078 min, LCMS28: m/z = 373.2 [M+1]. 1H NMR (300 MHz, Methanol-d4) δ 8.04 - 7.88 (m, 1H), 7.69 - 7.49 (m, 1H), 6.07 (s, 1H), 4.20 - 4.10 (m, 2H), 4.06 - 3.91 (m, 2H), 3.91 - 3.65 (m, 2H), 3.48 - 3.24 (m, 2H), 3.24 - 3.12 (m, 1H), 3.12 - 2.92 (m, 6H), 2.54 - 2.43 (m, 1H), 2.43 - 2.24 (m, 3H), 2.25 - 1.90 (m, 1H), 1.43 (t, J = 9 Hz, 3H). |
| 604 **(Reference)** | LC-MS: (ES, m/z): RT=1.325min, LCMS 28, m/z =321.3 [M+1]. 1H NMR (400 MHz, D2O-d6) δ7.30 (d, J = 4.2, 1H), 6.25 (s, 1H), 4.05 - 3.50 (m, 8H), 3.48 - 3.38 (m, 2H), 3.30 - 2.80 (m, 8H), 2.40 - 1.70 (m, 6H). |
| 613 **(Reference)** | LC-MS: (ES, m/z): RT = 0.15min, LCMS28: m/z =368.20 [M+1]. 1H NMR (400 MHz, Methanol-d4) δ 8.14 (d, J = 3.3 Hz, 1H), 7.52 (d, J = 3.3 Hz, 1H), 7.27 - 7.13 (m, 2H), 7.08-7.01 (m, 1H), 4.57-4.41 (m, 2H), 4.45-4.35 (m, 1H), 4.30 - 4.17 (m, 2H), 4.16-4.09 (m, 1H), 4.01 (d, J = 9.7 Hz, 3H), 3.53 - 3.47 (m, 2H), 3.4-3.35 (m, 1H), 2.73 (s, 3H), 1.31 - 1.26 (m, 3H). |
| 622 **(Reference)** | LC-MS: (ES, m/z): RT = 0.886 min, LCMS33: m/z = 383 [M+1]. 1H NMR (300 MHz, Methanol-d4) δ 9.27 (s, 1H), 8.81 (s, 1H), 6.99 (d, J = 1.5 Hz, 1H), 6.08 (d, J = 1.1 Hz, 1H), 4.41 (t, J = 5.5 Hz, 2H), 3.83 - 3.73 (m, 2H), 3.62 - 3.50 (m, 2H), 3.25 - 3.15 (m, 2H), 3.07 (s, 3H), 2.48 - 2.31 (m, 5H), 2.30 - 2.15 (m, 2H), 2.15 - 2.00 (m, 2H). |
| 625 **(Reference)** | LC-MS: (ES, m/z): RT=0.876 min,LCMS53, m/z=453 [M+1]. 1H NMR (300 MHz, Methanol-d4) δ 7.52 (s, 1H), 7.47 (s, 1H), 6.82 (s, 1H),4.32 (t, J = 5.5 Hz, 2H), 4.03 (s, 3H), 3.91 - 3.64 (m, 9H), 3.49 (t, J = 7.2 Hz, 2H), 3.45 - 3.32 (m, 2H), 3.24 - 3.08 (m, 2H), 2.98 (s, 3H), 2.46 - 1.95 (m, 14H). |
| 628 **(Reference)** | LC-MS: (ES, m/z): RT = 2.127min; LCMS33: m/z = 383 [M+1]. 1H NMR (400 MHz, Methanol-d4) δ 9.30 (d, J = 2.9 Hz, 1H), 8.39 (s, 1H), 6.82 (s, 1H), 6.20 (s, 1H), 4.61 (t, J = 5.9 Hz, 2H), 3.77 (s, 2H), 3.60 - 3.51 (m, 2H), 3.21 - 3.19 (m, 5H), 2.51 - 2.38 (m, 5H), 2.23 (q, J = 7.7 Hz, 2H), 2.14 - 2.05 (m, 2H). |
| 641 **(Reference)** | LC-MS: (ES, m/z): RT = 0.868 min, LCMS28: m/z = 345.1 [M+1]. |
| 642 **(Reference)** | LC-MS: (ES, m/z): RT = 0.903 min, LCMS07: m/z = 359.20 [M+1]. 1H NMR (300 MHz, Methanol-d4) δ 7.97 (d, J = 2.4 Hz, 1H), 7.84 (s, 1H), 5.82 (d, J = 0.9 Hz, 1H), 4.04 - 3.87 (m, 5H), 2.92 (s, 3H), 2.86 - 2.75 (m, 2H), 2.75 - 2.59 (m, 2H), 2.57 - 2.47 (m, 1H), 2.40 (s, 3H), 2.21 - 2.02 (m, 4H), 1.71 - 1.61 (m, 1H). |
| 644 | LC-MS: (ES, m/z): RT = 1.961 min, HPLC05: m/z = 371 [M+1]. 1H NMR (300 MHz, Methanol-d4) δ 7.90-7.75 (m, 1H), 7.72-7.49 (m, 1H), 7.25-7.09 (m, 1H), 6.04 - 5.97 (m, 1H), 4.34 (s, 2H), 3.97 (s, 3H), 3.70 - 3.43 (m, 8H), 2.99 (s, 3H), 2.49-2.25 (m, 3H), 2.21-2.03 (m, 4H). |
| 906 | LC-MS: (ES, m/z): RT=1.001 min, LCMS28, m/z=388 [M+1]. 1H NMR: (300 MHz, Methanol-d4) δ 7.61 (d, J = 2.4 Hz, 1H), 7.07 (dd, J = 8.7, 2.5 Hz, 1H), 6.89 (d, J = 8.7 Hz, 1H), 5.79 (d, J = 0.8 Hz, 1H), 4.18 - 4.08 (m, 1H), 4.08 - 3.90 (m, 2H), 3.83 (s, 3H), 3.01 - 2.73 (m, 4H), 2.70 - 2.55 (m, 5H), 2.17 (s, 3H), 1.83-1.79 (m, 4H). |
| 949 **(Reference)** | LC-MS: (ES, m/z): RT = 1.509min; LCMS15: m/z = 358 [M+1]. 1H NMR (300 MHz, Methanol-d4) δ 7.97 (d, J = 2.2 Hz, 1H), 7.79 (d, J = 2.3 Hz, 1H), 5.82 (d, J = 0.8 Hz, 1H), 4.04 (t, J = 6.2 Hz, 2H), 3.92 (s, 3H), 3.33 - 3.28 (m, 4H), 2.91 (s, 3H), 2.67 (q, J = 8.1, 2H), 2.21 - 2.05 (m, 5H), 1.77 - 1.86 (m, 6.2 Hz, 2H). |
| 965 | LC-MS: (ES, m/z): RT = 0.834min, LCMS07: m/z = 357 [M+1]. 1H NMR: (400 MHz, Methanol-d4) δ 7.61 - 7.53 (m, 2H), 6.92 (d, J = 8.6 Hz, 1H), 5.80 (s, 1H), 3.86 (s, 3H), 3.74 (s, 2H), 3.22 (t, J = 6.8 Hz, 4H), 2.91 (s, 3H), 2.57 - 2.56 (m, 4H), 2.18 (s, 3H), 2.09 - 2.05 (m, 2H). |
| 1038 | LC-MS: (ES, m/z): RT=0.97min, LCMS28, m/z=374.21 [M+1]. 1H-NMR: (Methanol-d4) δ 7.30 (d, J = 2.4 Hz, 1H), 7.19 (dd, J = 8.7, 2.5 Hz, 1H), 7.10-7.01 (m, 1H), 6.25-5.92 (m, 1H), 4.40 - 4.15 (m, 5H), 4.13 - 3.98 (m, 2H), 3.92-3.88 (m, 3H), 3.54 (dd, J = 12.9, 3.2 Hz, 1H), 3.45-3.38 (m, 1H), 3.06-2.93 (m, 3H), 2.78-2.56 (m, 1H), 2.52-2.39 (m, 1H), 2.31 (d, J = 1.0 Hz, 3H). |

**Table IB**

| Cpd No. | Data |
|---|---|
| **648 (Reference)** | LC-MS: (ES, m/z): RT=1.015 min,LCMS28, m/z=369 [M+1]. 1H NMR (300 MHz, Methanol-d4) δ 8.25 (d, J = 0.9 Hz, 1H), 8.18 (dd, J = 9.1, 1.0 Hz, 1H), 7.94 (s, 1H), 7.18 (d, J = 9.1 Hz, 1H), 6.83 (s, 1H), 4.24 (t, J = 5.6 Hz, 2H), 4.11 (s, 3H), 3.86 - 3.76 (m, 2H), 3.47 (dd, J = 8.1, 6.8 Hz, 2H), 3.23 - 3.08 (m, 2H), 2.40 - 2.02 (m, 6H), 1.29 (s, 1H). |
| **652 (Reference)** | LC-MS: (ES, m/z): RT=0.541min LCMS 32, m/z =416 [M+1]. 1H NMR (400 MHz, Methanol-d4) δ 7.66 (s, 1H), 7.52 (s, 1H), 4.38 (t, J = 5.5 Hz, 2H), 4.12 (s, 3H), 4.02 - 3.90 (m, 1H), 3.90 - 3.79 (m, 2H), 3.77 - 3.68 (m, 2H), 3.51 (t, J = 7.2 Hz, 2H), 3.42 - 3.32 (m, 2H), 3.24 - 3.10 (m, 2H), 2.99 (s, 3H), 2.79 - 2.73 (m, 3H), 2.68 - 2.52 (m, 2H), 2.45 - 2.34 (m, 2H), 2.30 - 2.02 (m, 6H). |
| **656 (Reference)** | LC-MS: (ES, m/z): RT = 1.488 min; LCMS53: m/z = 370 [M+1]. 1H NMR (300 MHz, Methanol-d4) δ 8.59 (s, 1H), 8.23 (d, J = 8.1, 1H), 8.16 (s, 1H), 7.84 (d, J = 8.8, 1H), 7.42 (d, J = 8.8, 1.0 Hz, 1H), 4.65 (t, J = 5.7 Hz, 2H), 3.95 (s, 3H), 3.86 - 3.63 (m, 2H), 3.48 (t, J = 7.6 Hz, 2H), 3.18 (q, J = 9.0 Hz, 2H), 2.42 - 2.31 (m, 2H), 2.22 (q, J = 9.4 Hz, 2H), 2.08 (d, J = 3.7 Hz, 2H). |
| **659 (Reference)** | LC-MS: (ES, m/z): RT = 0.99min, LCMS28: m/z = 427.31 [M+1]. 1H NMR (300 MHz, Deuterium Oxide) δ 7.55 (s, 1H), 7.26 (s, 1H), 4.36 (t, J = 5.6 Hz, 2H), 4.18-3.95 (m, 4H), 3.76 - 3.43 (m, 5H), 3.42 - 3.12 (m, 4H), 3.1-2.96 (m, 2H), 2.91-2.81 (m, 3H), 2.35 - 2.15 (m, 6H), 2.13 - 1.79 (m, 4H), 1.41 - 1.19 (m, 6H). |
| **662 (Reference)** | LC-MS: (ES, m/z): RT = 1.087 min, LCMS 31, m/z = 398.27 [M+1]. 1H NMR (300 MHz, Methanol-d4) δ 7.80 - 7.68 (m, 2H), 7.53 (d, J = 2.2 Hz, 1H), 4.48 - 4.31 (m, 2H), 4.16 (d, J = 2.6 Hz, 3H), 4.14 - 3.79 (m, 2H), 3.80 - 3.65 (m, 3H), 3.57 - 3.35 (m, 5H), 3.27 - 3.05 (m, 2H), 3.00 (s, 3H), 2.93 (s, 3H), 2.62 - 1.96 (m, 6H), 1.49 - 1.35 (m, 3H). |
| **663 (Reference)** | LC-MS: (ES, m/z): RT = 1.047 min, LCMS 30, m/z = 426.3 [M+1]. 1H NMR (300 MHz, Methanol-d4) δ 7.76 - 7.64 (m, 2H), 7.57 (s, 1H), 4.47 - 4.25 (m, 2H), 4.19 - 3.80 (m, 5H), 3.84 - 3.59 (m, 4H), 3.56 - 3.12 (m,6H), 2.92 (s, 3H), 2.63 - 1.93 (m, 7H), 1.52 - 1.27 (m, 10H). |
| **664 (Reference)** | LC-MS: (ES, m/z): RT = 0.786 min; LCMS07: m/z = 385 [M+1]. 1H NMR (300 MHz, Methanol-d4) δ 7.67 (d, J = 5.7 Hz, 1H), 7.42 (d, J = 7.1 Hz, 1H), 4.61 - 4.41 (m, 2H), 4.40 - 4.24 (m, 2H), 4.26 - 3.87 (m, 7H), 3.83 - 3.56 (m, 2H), 3.49 (q, J = 7.3 Hz, 3H), 3.35 (d, J = 2.5 Hz, 1H), 2.98 (s, 3H), 2.83 (d, J = 1.5 Hz, 3H), 2.55 - 2.29 (m, 2H), 2.20 (d, J = 8.2 Hz, 2H), 1.76 - 0.93 (m, 3H). |
| **670 (Reference)** | LC-MS: (ES, m/z): RT=0.856 min,LCMS28, m/z=427 [M+1]. 1H NMR (300 MHz, Methanol-d4) δ 7.95 (s, 1H), 7.69 (s, 1H), 4.76 (t, J = 5.7 Hz, 2H), 4.14 (s, 3H), 4.04 - 3.90 (m, 1H), 3.79 (s, 2H), 3.74 - 3.58 (m, 3H), 3.54 - 3.35 (m, 4H), 3.16 (s, 2H), 2.89 (s, 3H), 2.47 - 1.96 (m, 10H), 1.46 (d, J = 6.7 Hz, 6H). |
| **673 (Reference)** | LC-MS: (ES, m/z): RT = 0.815 min; LCMS15: m/z = 399 [M+1]. 1H NMR (300 MHz, Methanol-d4) δ 7.43 (s, 1H), 7.19 (s, 1H), 4.23 - 4.06 (m, 2H), 4.02 (s, 3H), 3.62 - 3.49 (m, 1H), 3.09 (d, J = 5.9 Hz, 2H), 2.99 - 2.91 (m, 1H), 2.89 - 2.42 (m, 9H), 2.42 - 2.25 (m, 5H), 2.26 - 2.01 (m, 3H), 1.99 - 1.80 (m, 2H), 1.81-1.70 (m, 1H), 1.16 (t, J = 7.3 Hz, 3H). |
| **681 (Reference)** | LC-MS: (ES, m/z): RT = 1.013 min, LCMS 28, m/z=451.2 [M+1]. 1H NMR (300 MHz, Methanol-d4) δ 8.41 (s, 2H), 7.57 (s, 1H), 7.41 (d, J = 2.2 Hz, 1H), 4.39 (t, J = 5.5 Hz, 2H), 4.09 (s, 3H), 4.02 -3.92 (m, 1H), 3.90 - 3.80 (m, 2H), 3.78 - 3.68 (m, 2H), 3.64 - 3.47 (m, 2H), 3.43 - 3.33 (m, 2H), 3.26 - 3.10 (m, 2H), 2.99 (s, 3H), 2.52 - 2.34 (m, 4H), 2.33 - 2.17 (m, 4H), 2.15 - 2.02 (m, 2H). |
| **737 (Reference)** | LC-MS: (ES, m/z): RT = 0.920 min, LCMS 30, m/z = 331 [M+1]. 1H NMR (400 MHz, Methanol-d4) δ 7.49 (s, 1H), 7.24 (s, 1H), 4.41 - 4.33 (m, 2H), 4.02 (s, 3H), 3.84 (s, 2H), 3.54 - 3.45 (m, 2H), 3.16 (s, 5H), 2.89 (s, 3H), 2.45 - 2.33 (m, 2H), 2.23 (s, 2H), 2.13 - 2.05 (m, 2H). |
| **739 (Reference)** | LC-MS: (ES, m/z): RT = 0.898 min, LCMS 07: m/z = 386 [M+1]. 1H NMR (400 MHz, Methanol-d4) δ 7.47 (s, 1H), 7.39 (s, 1H), 4.38 (t, J = 5.6 Hz, 2H), 4.30 (t, J = 5.2 Hz, 4H), 4.03 (s, 3H), 3.85 (d, J = 9.8 Hz, 2H), 3.55 - 3.39 (m, 6H), 3.25 - 3.15 (m, 2H), 2.89 (s, 3H), 2.39 (p, J = 6.5 Hz, 2H), 2.23 (q, J = 7.5, 7.1 Hz, 2H), 2.10 (d, J = 7.8, 4.9 Hz, 2H). |
| **743 (Reference)** | LC-MS: (ES, m/z): RT = 0.88min, LCMS33: m/z = 399.27 [M+1]. 1H NMR (400 MHz, Deuterium Oxide) δ 7.59-7.45 (m, 1H), 7.26 (d, J = 1.4 Hz, 1H), 4.44 - 4.05 (m, 3H), 4.02-3.89(m, 3H), 3.72 - 3.59 (m, 2H), 3.57 - 3.31 (m, 5H), 3.10 - 2.97 (m, 3H), 2.95 - 2.79 (m, 6H), 2.33 - 2.02 (m, 6H), 2.01 - 1.46 (m, 4H). |
| **745 (Reference)** | LC-MS: (ES, m/z): RT = 0.89min, LCMS07: m/z = 385.28 [M+1]. 1H NMR (400 MHz, Deuterium Oxide) δ 7.54 (s, 1H), 7.26 (d, J = 2.5 Hz, 1H), 4.89-4.78 (m, 1H), 4.36 (t, J = 5.6 Hz, 2H), 4.25-4.17 (m, 1H), 4.12-3.95 (m, 3H), 3.89 - 3.76 (m, 1H), 3.72 - 3.61 (m, 2H), 3.52-3.41 (m, 1H), 3.39 - 3.34 (m, 2H), 3.32-3.21 (m, 1H), 3.11-2.95 (m, 5H), 2.94-2.79 (m, 4H), 2.37 - 2.19 (m, 2H), 2.14-2.01 (m, 3H), 2.01-1.82 (m, 2H). |
| **746 (Reference)** | HPLC: (ES, m/z): RT = 4.51min, HPLC07: m/z =414.28 [M+1]. 1H NMR (400 MHz, Methanol-d4) δ 7.58-7.42 (m, 1H), 7.29 (s, 1H), 4.45-4.31 (m, 2H), 4.21-4.01 (m, 4H), 3.95 - 3.57 (m, 4H), 3.57 - 3.43 (m, 3H), 3.26 - 3.05 (m, 6H), 3.18-2.98 (m, 3H), 2.47-2.32 (m, 2H), 2.31 - 2.02 (m, 7H), 1.93-1.82 (m, 1H). |
| **755 (Reference)** | LC-MS: (ES, m/z): RT= 0.94 min, LCMS 28: m/z = 468 [M+1]. 1H-NMR: (Methanol-d4, ppm):δ 7.69 (d, J = 8.1 Hz, 2H), 7.56 (s, 1H), 4.44 (t, J = 5.5 Hz, 2H), 4.21 - 4.10 (m, 5H), 4.09 - 3.96 (m, 1H), 3.87-3.81 (m, 4H), 3.64 - 3.38 (m, 7H), 3.26 - 3.10 (m, 2H), 2.93 (s, 3H), 2.46-2.42 (m, 2H), 2.35 - 2.20 (m, 6H), 2.19 - 2.09 (m, 4H), 2.07-1.92 (m, 2H). |
| **756 (Reference)** | LC-MS: (ES, m/z): RT = 1.316 min; LCMS53: m/z = 454 [M+1]. 1H NMR (300 MHz, Methanol-d4) δ 7.68 (d, J = 6.4 Hz, 2H), 7.54 (s, 1H), 4.43 (t, J = 5.5 Hz, 2H), 4.28 (d, J = 8.1 Hz, 1H), 4.14 (s, 6H), 3.62 - 3.96 (m, , 6H), 3.42 - 3.58 (m, 4H), 3.25 - 3.09 (m, 2H), 2.91 (s, 3H), 2.57 - 2.01 (m, 12H). |
| **757 (Reference)** | LC-MS: (ES, m/z): RT=0.887 min,LCMS53, m/z=483 [M-+1]. 1H NMR (300 MHz, Methanol-d4) δ 7.46 (s, 2H), δ 6.87 (s, 1H),4.33 (t, J = 5.5 Hz, 2H), 4.18 - 4.07 (m, 2H), 4.03 (s, 3H), 3.90 - 3.69 (m, 5H), 3.61 - 3.34 (m, 7H), 3.17 (s, 5H), 2.44 - 1.99 (m, 12H), 1.98 - 1.79 (m, 2H). |
| **758 (Reference)** | LC-MS: (ES, m/z): RT=0.877 min,LCMS53, m/z=469 [M+1]. 1H NMR (300 MHz, Methanol-d4) δ 7.46 (s, 2H), 6.88 (s, 1H), 4.38 - 4.20 (m, 3H), 4.19 - 3.99 (m, 5H), 3.97 - 3.56 (m, 7H), 3.55 - 3.36 (m, 4H), 3.17 (s, 5H), 2.55 - 2.01 (m, 12H). |
| **759 (Reference)** | HPLC: (ES, m/z): RT=3.82 min, HPLC07: m/z = 471 [M+1]. 1H-NMR: (Methanol-d4): δ 7.59 (s, 1H), 7.29 (s, 1H), 4.37 (t, J = 5.5 Hz, 2H), 4.28 - 3.95 (m, 7H), 3.96 - 3.59 (m, 6H), 3.54 - 3.38 (m, 4H), 3.27 - 3.08 (m, 5H), 2.68 - 2.01 (m, 12H). |
| **762 (Reference)** | LC-MS: (ES, m/z): RT = 1.130 min, LCMS 28, m/z = 387 [M+1]. 1H NMR (400 MHz, Methanol-d4) δ 7.50 (s, 1H), 7.31 (s, 1H), 4.37 (t, J = 5.5 Hz, 2H), 4.05 - 3.98 (m, 7H), 3.91 - 3.78 (m, 6H), 3.50 (t, J = 7.2 Hz, 2H), 3.23 - 3.11 (m, 2H), 2.91 (s, 3H), 2.45 - 2.34 (m, 2H), 2.30 - 2.16 (m, 2H), 2.15 - 2.05 (m, 2H). |
| **763 (Reference)** | LC-MS: (ES, m/z): RT = 1.107 min; LCMS53: m/z = 424.3 [M+1]. 1H NMR (300 MHz, Methanol-d4) δ 7.87 (s, 1H), 7.66 (s, 1H), 7.50 (s, 1H), 4.60 - 4.38 (m, 4H), 4.12 (s, 3H), 3.98 (s, 4H), 3.90 - 3.76 (m, 2H), 3.31 - 3.69 (m, 6H), 3.25 - 3.10 (m, 2H), 2.85 (s, 3H), 2.35 - 2.45 (m, 2H), 2.29 - 2.03 (m, 4H). |
| **786** | LC-MS: (ES, m/z): RT = 1.025 min, LCMS 33: m/z = 352 [M+1]. 1H NMR (300 MHz, Methanol-d4) δ 7.78 (d, J = 2.7 Hz, 1H), 7.54 (d, J = 2.7 Hz, 1H), 6.92 (d, J = 9.0 Hz, 1H), 5.81 (d, J = 0.9 Hz, 1H), 3.84 (s, 3H), 3.68 (s, 2H), 2.91 (s, 3H), 2.85 - 2.72 (m, 4H), 2.22 - 2.15 (m, 3H), 1.97 - 1.81 (m, 4H). |
| **787 (Reference)** | LC-MS: (ES, m/z): RT = 1.399 min; LCMS53: m/z = 455 [M+1]. 1H NMR (400 MHz, Methanol-d4) δ 7.33 (d, J = 2.5 Hz, 1H), 7.19 (d, J = 8.7, 1H), 6.92 (d, J = 8.7 Hz, 1H), 5.80 (s, 1H), 4.10 (t, J = 6.0 Hz, 3H), 3.82 (s, 3H), 2.99 (s, 1H), 2.88 - 2.34 (m, 7H), 2.29 (s, 3H), 2.17 (s, 3H), 2.13 - 2.01 (m, 3H), 1.95 (s, 2H), 1.91 - 1.75 (m, 5H), 1.69 (d, J = 3.6 Hz, 1H), 1.31 (d, J = 10.8 Hz, 1H). |
| **788 (Reference)** | LC-MS: (ES, m/z): RT = 1.041 min; LCMS07: m/z = 442 [M+1]. 1H NMR (400 MHz, Methanol-d4) δ 7.54 (s,1H), 7.03 - 6.94 (m,1H), 6.89 (d, J = 5.6 Hz, 1H), 5.81 (s, 1H), 4.11 (t, J = 7.2 Hz, 4H), 3.83 (d, J = 6.1 Hz, 4H), 3.45 (t, J = 3.2 Hz, 1H), 3.22 - 3.13 (m, 1H), 2.76 (d, J = 8.9 Hz, 6H), 2.14 (s, 3H), 2.05 (s, 3H), 1.86 (d, J = 7.6 Hz, 5H), 2.73 - 2.51 (m, 2H). |
| **789 (Reference)** | LC-MS: (ES, m/z): RT= 1.15 min, LCMS28: m/z = 428 [M+1]. 1H-NMR: (Methanol-d4, ppm):δ 7.42 (d, J = 2.5 Hz, 1H), 7.11 (dd, J = 8.7, 2.5 Hz, 1H), 6.88 (d, J = 8.7 Hz, 1H), 5.82 (d, J = 0.8 Hz, 1H), 4.56 (s, 1H), 4.12 - 3.77 (m, 8H), 3.68 - 3.53 (m, 1H), 2.80 - 2.57 (m, 6H), 2.37 - 2.25 (m, 1H), 2.22 (s, 3H),2.12 - 1.76 (m, 7H). |
| **790 (Reference)** | LC-MS: (ES, m/z): RT = 0.924 min, LCMS 07: m/z = 409 [M+1]. 1H NMR (400 MHz, Methanol-d4) δ 4.41 (t, J = 5.4 Hz, 2H), 4.18 (s, 3H), 3.92 - 3.82 (m, 1H), 3.81 (d, J = 10.9 Hz, 2H), 3.73 (d, J = 11.2 Hz, 2H), 3.51 (q, J = 9.9, 8.4 Hz, 4H), 3.19 (d, J = 14.4, 7.7 Hz, 2H), 3.01 (d, J = 2.7 Hz, 3H), 2.41 (q, J = 6.1 Hz, 2H), 2.33 - 2.18 (m, 4H), 2.19 - 2.06 (m, 4H). |
| **791** | LC-MS: (ES, m/z):RT = 1.673 min; LCMS53: m/z=367 [M+1]. 1H NMR (300 MHz, Methanol-d4) δ 8.01 (s, 1H), 7.45 (d, J = 8.6 Hz, 1H), 7.26 (d, J = 8.6 Hz, 1H), 5.94 (s, 1H), 4.23 (t, J = 6.0 Hz, 2H), 2.93 (s, 3H), 2.86 - 2.81 (m, 2H), 2.73 - 2.70 (m, 4H), 2.20 (s, 3H), 2.15 - 2.08 (m, 2H), 1.96 - 1.85 (m, 4H). |
| **793** | LC-MS: (ES, m/z): RT=1.706 min,LCMS53, m/z=383 [M+1]. 1H NMR (300 MHz, Methanol-d4) δ 7.56 (d, J = 2.5 Hz, 1H), 7.42 (d, J = 2.5 Hz, 1H), 6.00 (s, 1H), 4.28 (t, J = 5.5 Hz, 2H), 3.62 - 3.38 (m, 6H), 2.99 (s, 3H), 2.33 - 2.05 (m, 9H). |
| **794 (Reference)** | LC-MS: (ES, m/z): RT = 0.958 min, m/z = 382.15 [M+1]. 1H NMR (400 MHz, Chloroform-d) δ 8.85 (d, J = 1.2 Hz, 1H), 8.23 (d, J = 5.6 Hz, 1H), 7.59 - 7.49 (m, 1H), 7.17 (d, J = 2.7 Hz, 1H), 7.11 (dd, J = 8.6, 2.6 Hz, 1H), 6.88 (d, J = 8.7 Hz, 1H), 6.56 (s, 1H), 4.15 (t, J = 6.2 Hz, 2H), 4.07 (s, 3H), 3.86 (s, 3H), 3.11 (s, 6H), 2.42 - 2.26 (m, 2H), 2.06 (d, J = 7.1 Hz, 4H). |
| **795 (Reference)** | LC-MS: (ES, m/z): RT = 0.8 min, m/z = 383.25 [M+1]. 1H NMR (400 MHz, Methanol-d4) δ 9.74 (s, 1H), 8.79 (d, J = 6.5, 1.0 Hz, 1H), 8.50 (d, J = 6.4 Hz, 1H), 7.91 (s, 1H), 7.26 (s, 1H), 4.53 (t, J = 5.7 Hz, 2H), 4.43 (s, 3H), 3.99 (s, 3H), 3.87 - 3.76 (m, 2H), 3.51 (t, J = 7.4 Hz, 2H), 3.26 - 3.11 (m, 2H), 2.49 - 2.38 (m, 2H), 2.29 - 2.17 (m, 2H), 2.16 - 2.01 (m, 2H). |
| **796 (Reference)** | LC-MS: (ES, m/z): RT = 0.982min, LCMS33: m/z = 331 [M+1]. 1H NMR (300 MHz, Methanol-d4) δ 7.00 (d, J = 8.7 Hz, 1H), 6.90 (d, J = 2.3 Hz, 1H), 6.83 (dd, J = 8.5, 2.5 Hz, 1H), 4.19 (t, J = 5.5 Hz, 2H), 3.91 -3.73 (m, 5H), 3.49 (t, J = 7.0 Hz, 2H), 3.25 - 3.02(m, 2H), 2.34 (s, 3H), 2.29 - 2.04 (m, 7H). |
| **797 (Reference)** | LC-MS: (ES, m/z): RT = 1.218 min, LCMS28: m/z = 344 [M+1]. 1H NMR (400 MHz, Methanol-d4) δ 6.98 - 6.90 (m, 2H), 6.78 (dd, J = 8.6, 2.5 Hz, 1H), 4.18 (t, J = 5.5 Hz, 2H), 3.87 - 3.74 (m, 5H), 3.69 (s, 3H), 3.48 (t, J = 6.9 Hz, 2H), 3.22 - 3.11 (m, 2H), 2.32 - 2.17 (m, 7H), 2.10 - 2.01 (m, 2H). |
| **798 (Reference)** | LC-MS: (ES, m/z): RT= 1.103 min; LCMS15: m/z = 399 [M+1]. 1H NMR (300 MHz, Methanol-d4) δ 7.98 (d, J = 2.2 Hz, 1H), 7.85 (s, 1H), 5.83 (d, J = 0.8 Hz, 1H), 4.21 - 3.65 (m, 5H), 3.13 - 2.76 (m, 5H), 2.76 - 2.49 (m, 3H), 2.39 (d, J = 9.7, 6.2 Hz, 1H), 2.19 (s, 3H), 2.15 - 1.86 (m, 5H), 1.86 - 1.53 (m, 3H). |
| **799** | LC-MS: (ES, m/z): RT = 1.478 min; LCMS07: m/z = 402 [M+1]. 1H NMR (400 MHz, Methanol-d4) δ 7.18 (d, J = 2.4 Hz, 1H), 6.94 (d, J = 2.4 Hz, 1H), 6.02 (d, J = 1.1 Hz, 1H), 4.19 (t, J = 5.6 Hz, 2H), 3.89 (s, 3H), 3.82 (s, 3H), 3.78 (s, 2H), 3.58 - 3.39 (m, 2H), 3.23 (s, 2H), 3.06 (s, 3H), 2.33 (d, J = 1.0 Hz, 3H), 2.30 - 2.13 (m, 4H), 2.07 (d, J = 8.8 Hz, 2H). |
| **800** | LC-MS: (ES, m/z): RT = 2.007 min; LCMS53: m/z = 372 [M+1]. 1H NMR (300 MHz, Methanol-d4) δ 7.48 (d, J = 2.4 Hz, 1H), 7.12 (d, J = 8.7, 2.4 Hz, 1H), 6.89 (d, J = 8.7 Hz, 1H), 5.77 (d, J = 0.8 Hz, 1H), 4.15 (s,1H), 4.08 - 4.02(m,1H), 3.98 - 3.92 (m,1H), 3.8 (s, 3H), 3.31 - 3.19 (m,2H),3.18 - 3.00 (m, 2H), 2.81 - 2.70 (m, 1H), 2.23 - 2.07 (m, 4H), 1.82 (d, J = 6.8 Hz, 1H), 1.23 (d, J = 6.5 Hz, 6H). |
| **802** | LC-MS: (ES, m/z): RT=1.843min LCMS 31, m/z =372 [M+1]. 1H NMR (400 MHz, Methanol-d4) δ 7.49 - 7.43 (m, 2H), 6.83 (s, 1H), 4.35 - 4.27 (m, 1H), 4.27 - 4.18 (m, 1H), 4.17 - 4.08 (m, 2H), 4.02 (s, 3H), 3.83 - 3.72 (m, 2H), 3.66 - 3.51 (m, 3H), 3.46 - 3.28 (m, 2H), 3.18 (s, 3H), 3.07 - 2.95 (m, 1H), 2.43 - 2.29 (m, 1H), 2.14 - 2.00 (m, 1H), 1.98 - 1.82 (m, 4H). |
| **803 (Reference)** | LC-MS: (ES, m/z): RT=0.672min LCMS 32, m/z =386 [M+1]. 1H NMR (400 MHz, Methanol-d4) δ 7.27 (s, 1H), 7.19 (s, 1H), 6.55 (s, 1H), 4.15 - 3.99 (m, 4H), 3.94 (s, 3H), 3.80 - 3.69 (m, 2H), 3.52 - 3.36 (m, 1H), 2.99 (s, 3H), 2.96 - 2.90 (m, 1H), 2.89 - 2.76 (m, 1H), 2.76 - 2.66 (m, 2H), 2.63 - 2.55 (m, 1H), 2.44 (s, 3H), 2.24 - 2.10 (m, 1H), 1.95 - 1.70 (m, 5H). |
| **806 (Reference)** | LC-MS: (ES, m/z): RT=0.983 min,LCMS28, m/z=308 [M+1]. 1H NMR (300 MHz, Methanol-d4) δ 7.45 (s, 1H), 6.44 (s, 1H), 4.12 - 3.92 (m, 7H), 3.90 - 3.80 (m, 1H), 3.63 - 3.33 (m, 5H), 3.25 (dd, J = 11.9, 7.0 Hz, 1H), 2.97 - 2.77 (m, 1H), 2.35 - 2.18 (m,1H), 2.10 - 1.90 (m, 3H), 1.70 -1.50 (m, 2H). |
| **808 (Reference)** | LC-MS: (ES, m/z): RT = 0.85min, LCMS33: m/z = 322.21 [M+1]. 1H NMR (400 MHz, Methanol-d4) δ 7.50 (s, 1H), 6.64 (s, 1H), 4.31-4.15 (m, 1H), 4.14 - 3.95 (m, 7H), 3.71 - 3.55 (m, 2H), 3.54 - 3.42 (m, 2H), 3.41 - 3.35 (m, 1H), 3.28-3.21 (m, 1H), 3.10 (s, 3H), 2.95 - 2.87 (m, 1H), 2.41-2.22 (m, 1H), 2.09 - 1.92 (m, 3H), 1.80 - 1.70 (m, 2H). |
| **809 (Reference)** | LC-MS: (ES, m/z): RT=0.991 min,LCMS53, m/z=441 [M+1]. 1H NMR (300 MHz, Methanol-d4) δ 7.46 (d, J = 6.9 Hz, 2H), 6.79 (s, 1H), 4.82 - 4.68 (m, 1H), 4.33 (t, J = 5.5 Hz, 2H), 4.19 - 4.06 (m, 1H), 4.02 (s, 3H), 3.91 - 3.75 (m, 2H), 3.71 - 3.35 (m, 4H), 3.16 (s, 5H), 2.93 (td, J = 13.0, 2.6 Hz, 1H), 2.45- 1.96 (m, 11H), 1.85 - 1.55 (m, 2H). |
| **810 (Reference)** | LC-MS: (ES, m/z): RT =1.842min, LCMS15, m/z = 384 [M+1]. 1H NMR (300 MHz, Methanol-d4) δ 7.46 (s, 1H), 7.37 (s, 1H), 7.25 (d, J = 1.2 Hz, 1H), 4.33 (t, J = 5.5 Hz, 2H), 4.02 (s, 3H), 3.91 - 3.82 (m, 6H), 3.51 (t, J = 7.2 Hz, 2H), 3.23 - 3.10 (m, 2H), 2.73 (d, J = 1.0 Hz, 3H), 2.46 - 2.31 (m, 2H), 2.27 - 2.20 (m, 2H), 2.15 - 2.04 (m, 2H), 1.87 - 1.78 (m, 6H). |
| **812 (Reference)** | HPLC: (ES, m/z): RT = 8.367 min; HPLC07: m/z = 467 [M+1]. 1H NMR (300 MHz, Methanol-d4) δ 7.55 - 7.46 (m, 2H), 7.09 (s, 1H), 4.32 (t, J = 5.5 Hz, 2H), 4.03 (s, 3H), 3.92 - 3.66 (m, 9H), 3.55 - 3.32 (m, 3H), 3.20 - 3,12 (m, 2H), 2.97 (s, 4H), 2.44 - 2.00 (m, 10H), 1.82 (s, 6H). |
| **816 (Reference)** | LC-MS: (ES, m/z): RT = 1.246 min; LCMS15: m/z = 398 [M+1]. 1H NMR (400 MHz, Methanol-d4) δ 7.20 (d, J = 8.8 Hz, 2H), 6.52 (s, 1H), 4.18 (t, J = 6.1 Hz, 2H), 3.92 (s, 3H), 3.24 - 3.04 (m, 1H), 2.98 (s, 3H), 2.85 - 2.71 (m, 2H), 2.73 - 2.53 (m, 4H), 2.26 - 2.07 (m, 2H), 2.07 - 1.90 (m, 4H), 1.90 - 1.74 (m, 5H), 1.72 - 1.45 (m, 4H), 1.45 - 1.24 (m, 1H). |
| **817 (Reference)** | LC-MS: (ES, m/z): RT=2.004 min,LCMS28, m/z=358 [M+1]. 1H NMR (300 MHz, Methanol-d4) δ 7.53 (s, 1H), 7.46 (s, 1H), 6.85 (s, 1H), 4.37 (t, J = 5.5 Hz, 2H), 4.02 (s, 3H), 3.93 - 3.79 (m, 2H), 3.68 (p, J = 6.8 Hz, 1H), 3.53 (t, J = 7.2 Hz, 2H), 3.20 (s, 5H), 2.50 - 2.02 (m, 6H), 1.42 (d, J = 6.8 Hz, 7H). |
| **820 (Reference)** | LC-MS: (ES, m/z): RT = 0.695 min, LCMS 30, m/z =317 [M+1]. 1H NMR (300 MHz, Methanol-d4) δ 7.42 (s, 1H), 7.19 (s, 1H), 6.90 (d, J = 1.1 Hz, 1H), 4.37 (t, J = 5.6 Hz, 2H), 4.03 (s, 3H), 3.92 - 3.78 (m, 2H), 3.52 (t, J = 7.2 Hz, 2H), 3.27 - 3.11 (m, 2H), 2.77 (s, 3H), 2.48 - 2.33 (m, 2H), 2.32 - 2.03 (m, 4H). |
| **821 (Reference)** | LC-MS: (ES, m/z): RT = 1.047 min; LCMS07: m/z = 331 [M+1]. 1H NMR (400 MHz, Methanol-d4) δ 7.53 (s, 1H), 7.47 (s, 1H), 7.33 (s, 1H), 4.42 (t, J = 5.5 Hz, 2H), 4.34 (s, 3H), 4.08 (s, 3H), 3.90 - 3.80 (m, 2H), 3.52 (t, J = 7.2 Hz, 2H), 3.22 - 3.17 (m, 2H), 2.94 (s, 3H), 2.45 - 2.39 (m, 2H), 2.30 - 2.17 (m, 2H), 2.16 - 2.08 (m, 2H). |
| **822 (Reference)** | LC-MS: (ES, m/z): RT=1.815 min, LCMS53, m/z=387 [M+1]. 1H NMR (400 MHz, Methanol-d4) δ 7.59 (s, 1H), 7.31 (d, J = 1.1 Hz, 1H), 7.03 (d, J = 1.7 Hz, 1H), 4.41 (t, J = 5.7 Hz, 2H), 4.18 - 4.05 (m, 5H), 3.90 - 3.75 (m, 5H), 3.52 (t, J = 7.2 Hz, 2H), 3.25 - 3.14 (m, 2H), 2.48 - 2.36 (m, 2H), 2.31 - 2.02 (m, 4H), 2.00 - 1.83 (m, 4H). |
| **823 (Reference)** | LC-MS: (ES, m/z): RT = 1.008 min, LCMS 07: m/z = 414 [M+1]. 1H NMR (400 MHz, D2O) δ 7.19 - 7.07 (m, 2H), 7.01 - 6.93 (m, 1H), 4.31 - 4.21 (m, 2H), 3.91 (td, J = 10.3, 9.5, 4.7 Hz, 5H), 3.73 (d, J = 10.9, 5.2 Hz, 2H), 3.38 (d, J = 37.3, 8.9 Hz, 4H), 3.16 - 2.98 (m, 5H), 2.81 (s, 2H), 2.36 - 2.24 (m, 2H), 2.15 (d, J = 9.6, 5.6 Hz, 2H), 2.07 - 1.88 (m, 3H), 1.61 - 1.52 (m, 2H), 1.35 (q, J = 11.6 Hz, 2H). |
| **824 (Reference)** | LC-MS: (ES, m/z): RT=1.339 min,LCMS15, m/z=454 [M+1]. 1H NMR (400 MHz, Methanol-d4) δ 7.51 (s, 1H), 7.47 (s, 1H), 7.05 (s, 1H), 4.33 (t, J = 5.5 Hz, 2H), 4.18 - 4.09 (m, 2H), 4.03 (s, 3H), 3.91 - 3.73 (m, 8H), 3.70 - 3.55 (m, 1H), 3.50 (t, J = 7.2 Hz, 2H), 3.17 (dd, J = 11.5, 6.7 Hz, 2H), 2.44 - 2.33 (m, 2H), 2.30 - 2.16 (m, 2H), 2.16 - 2.02 (m, 2H), 2.02 - 1.87 (m, 4H), 1.86 - 1.72 (m, 6H). |
| **825 (Reference)** | LC-MS: (ES, m/z): RT=1.172 min,LCMS28, m/z=414 [M+1]. 1H NMR (400 MHz, Deuterium Oxide) δ7.30 - 7.10 (m, 2H), 6.72 - 6.60 (m, 1H), 4.30 - 4.10 (m, 2H), 4.07 - 3.99 (m, 2H), 3.90 - 3.85 (m, 3H), 3.75 - 3.60 (m, 4H), 3.45 - 3.30 (m, 3H), 3.30 - 3.20 (m, 6H), 3.10 - 3.00 (m, 2H), 2.30 - 1.70 (m, 10H). |
| **826 (Reference)** | LC-MS: (ES, m/z): RT=1.099 min,LCMS28, m/z=428 [M+1]. 1H NMR (400 MHz, Methanol-d4) δ 7.61 (s, 1H), 7.45 (s, 1H), 6.83 (s, 1H), 4.36 (t, J = 5.5 Hz, 2H), 4.30 - 4.20 (m, 1H), 4.17 - 4.07 (m, 2H), 4.02 (s, 3H), 3.89 - 3.75 (m, 4H), 3.60 - 3.45 (m, 3H), 3.20 - 3.10 (m, 2H), 2.42 - 2.30 (m, 2H), 2.30 - 2.17 (m, 2H), 2.17 - 2.02 (m, 2H), 1.96 - 1.81 (m, 4H), 1.40 (d, J = 6.3 Hz, 6H). |
| **832 (Reference)** | LC-MS: (ES, m/z): RT = 1.385 min; LCMS07: m/z = 495 [M+1]. 1H NMR (300 MHz, Methanol-d4) δ 7.49 - 7.41 (m, 2H), 6.87 (s, 1H), 4.33 (t, J = 5.5 Hz, 2H), 4.03 (s, 3H), 3.85 - 3.79 (m, 4H), 3.72 - 3.66 (m, 1H), 3.58 - 3.36 (m, 6H), 3.17 (s, 5H), 2.92 - 2.86 (m, 2H), 2.45 - 2.03 (m, 10H). |
| **833 (Reference)** | LC-MS: (ES, m/z): RT = 1.462 min; LCMS07: m/z = 481 [M+1]. 1H NMR (300 MHz, Methanol-d4) δ 7.46 - 7.41 (m, 2H), 7.43 (s, 1H), 4.32 (t, J = 5.5 Hz, 2H), 4.01 (s, 3H), 3.86 - 3.77 (m, 2H), 3.69 - 3.60 (m, 2H), 3.52 - 3.42 (m, 5H), 3.24 - 3.01 (m, 7H), 2.39 - 2.35 (m, 2H), 2.28 - 1.92 (m, 8H). |
| **839 (Reference)** | LC-MS: (ES, m/z): RT = 0.858 min; LCMS07: m/z = 457 [M+1]. 1H NMR (300 MHz, Methanol-d4) δ 7.22 - 7.17(m, 2H), 6.53 (s, 1H), 4.17 (t, J = 6.1 Hz, 2H), 3.91 (s, 3H), 3.63 - 3.57 (m, 2H), 3.37 (s, 3H), 3.23 - 3.06 (m, 3H), 2.97 (s, 3H), 2.83 - 2.60 (m, 8H), 2.39 - 2.33 (m, 2H), 2.15 - 2.10 (m, 2H), 2.01 - 1.75 (m, 8H). |
| **844 (Reference)** | LC-MS: (ES, m/z): RT = 1.012 min, LCMS28: m/z = 353.2 [M+1]. 1H NMR (300 MHz, Methanol-d4) δ 8.47 (s, 1H), 8.02 (s, 1H), 6.01 (s, 1H), 4.41 (s, 2H), 4.01 (s, 3H), 3.91 - 3.32 (m, 4H), 2.95 (s, 3H), 2.32 (s, 3H), 2.21 - 2.01 (m, 4H). |
| **846** | LC-MS: (ES, m/z): RT = 0.981 min, LCMS15: m/z = 338.3 [M+1]. 1H NMR (300 MHz, Methanol-d4) δ 7.74 (d, J = 2.7 Hz, 1H), 7.65 - 7.55 (m, 1H), 6.94 (d, J = 9.0 Hz, 1H), 5.81 (s, 1H), 4.33 - 4.32 (m, 1H), 3.84 (s, 3H), 3.32 - 3.31 (m, 1H), 3.30 - 3.07 (m, 1H), 2.90 (s, 3H), 2.32 - 2.30 (m, 1H), 2.28 - 2.02 (m, 6H). |
| **847** | LC-MS: (ES, m/z): RT = 1.003 min, LCMS 33: m/z = 352 [M+1]. 1H NMR (400 MHz, Methanol-d4) δ 7.64 - 7.45 (m, 2H), 7.06 (d, J = 8.8 Hz, 1H), 5.99 -5.96 (m, 1H), 3.89 (s, 6H), 3.45 (s, 1H), 3.27 (s, 1H), 2.99 (s, 6H), 2.63 (s, 1H), 2.44 - 2.14 (m, 4H). |
| **848** | LC-MS: (ES, m/z): RT = 1.655 min, LCMS28: m/z = 352.2 [M+1]. 1H NMR (300 MHz, Methanol-d4) δ 7.78 (s, 1H), 7.54 (d, J = 8.9 Hz, 1H), 6.93 (d, J = 9.0 Hz, 1H), 5.81 (s, 1H), 3.84 (s, 3H), 3.44 (t, J = 6.9 Hz, 1H), 3.03 - 2.88 (m, 4H), 2.30 - 2.92 (m, 4H), 2.35 - 2.08 (m, 4H), 2.00 - 1.92 (m, 3H). |
| **854 (Reference)** | LC-MS: (ES, m/z): RT = 1.18min, LCMS33: m/z = 400.14 [M+1]. 1H NMR (400 MHz, Methanol-d4) δ 7.50 (d, J = 2.5 Hz, 1H), 7.24 (s, 1H), 7.19 (dd, J = 8.7, 2.5 Hz, 1H), 6.97 (d, J = 8.7 Hz, 1H), 4.16 (t, J = 5.9 Hz, 2H), 3.86 (s, 3H), 3.12-3.02 (m, 2H), 3.01-2.91 (m, 7H), 2.49 (s, 3H), 2.23-2.12 (m, 2H), 2.04 - 1.87 (m, 4H). |
| **855** | LC-MS: (ES, m/z): RT=1.033min, LCMS15, m/z=366.3 [M+1]. 1H NMR (300 MHz, Methanol-d4) δ 7.66 (d, J = 7.4 Hz, 2H), 7.14 (d, J = 9.5 Hz, 1H), 6.02 (s, 1H), 4.32 (s, 2H), 3.93 (s, 3H), 3.72 (s,3H), 3.57 (d, J = 1.6 Hz, 3H), 2.99 (s, 3H), 2.32 (s, 3H), 1.88 (s,7H). |
| **863 (Reference)** | LC-MS: (ES, m/z): RT = 1.256 min; LCMS53: m/z = 454 [M+1]. 1H-NMR (300 MHz, Methanol-d4) δ7.68 - 7.64 (m, 2H), 7.52 (s, 1H), 4.42 (t, J = 6.0 Hz, 2H), 4.28 - 4.22 (m, 1H), 4.18 - 3.98 (m, 6H), 3.97 - 3.62 (m, 6H), 3.52 - 3.39 (m, 4H), 3.22-3.08 (m, 2H), 2.91 (s, 3H), 2.55 - 2.01 (m, 12H). |
| **864 (Reference)** | LC-MS: (ES, m/z): RT = 0.813 min; LCMS53: m/z = 454 [M+1]. 1H NMR (300 MHz, Methanol-d4) δ7.38 (s, 1H), 7.34 (s, 1H), 7.20 (s, 1H), 4.23 (t, J = 6.0 Hz, 2H), 4.06 - 3.88 (m, 5H), 3.87 - 3.65 (m, 2H), 3.43 - 3.32 (m, 1H), 3.26 - 3.08 (m, 4H), 3.02 - 3.00 (m, 2H), 2.91 - 2.71 (m, 4H), 2.64 (s, 3H), 2.59-2.38 (m, 2H), 2.27 - 2.09 (m, 3H), 2.05 - 1.80 (m, 8H). |
| **866 (Reference)** | LC-MS: (ES, m/z): RT = 1.406 min; LCMS07: m/z = 469 [M+1]. 1H NMR (300 MHz, Methanol-d4) δ 7.23 - 7.18 (m, 2H), 6.65 (s, 1H), 4.18 (t, J = 6.1 Hz, 2H), 4.06 - 3.88 (m, 5H), 3.87 - 3.65 (m, 2H), 3.24 - 2.92 (m, 7H), 2.81 - 2.75 (m, 2H), 2.80 - 2.30 (m, 6H), 2.25 - 2.05 (m, 3H), 2.01 - 1.72 (m, 9H). |
| **867 (Reference)** | LC-MS: (ES, m/z): RT = 1.405 min; LCMS07: m/z = 469 [M+1]. 1H NMR (300 MHz, Methanol-d4) δ 7.25 - 7.16 (m, 2H), 6.64 (s, 1H), 4.21 (t, J = 6.1 Hz, 2H), 4.05 - 3.87 (m, 5H), 3.87 - 3.65 (m, 2H), 3.24 - 2.86 (m, 7H), 2.81 - 2.75 (m, 2H), 2.70 - 2.33 (m, 6H), 2.26 - 2.08 (m, 3H), 2.06 - 1.72 (m, 9H). |
| **870 (Reference)** | LC-MS: (ES, m/z): RT = 1.182 min, LCMS 28: m/z = 427 [M+1]. 1H NMR (400 MHz, Methanol-d4) δ 7.64 (d, J = 2.5 Hz, 1H), 7.17 (dd, J = 8.8, 2.4 Hz, 1H), 7.00 (d, J = 8.8 Hz, 1H), 6.72 (s, 1H), 4.24 (t, J = 5.5 Hz, 2H), 4.11 - 4.01 (m, 2H), 3.95 - 3.79 (m, 5H), 3.73 - 3.54 (m, 2H), 3.53 - 3.46 (m, 2H), 3.23 - 3.14 (m, 2H), 3.06 - 2.83 (m, 1H), 2.43 (s, 3H), 2.35 - 2.16 (m, 4H), 2.16 - 2.01 (m, 2H), 2.01 - 1.79 (m, 4H). |
| **871** | LC-MS: (ES, m/z): RT=1.383min, LCMS15, m/z=378.2 [M+1]. 1H-NMR: δ 8.24 (d, J = 2.7 Hz, 1H), 7.65 (s, 1H), 7.45 - 7.17 (m, 4H), 6.03 (d, J = 1.3 Hz,2H), 5.09 (s, 1H), 4.65 (s, 2H), 4.01 (d, J = 1.6 Hz, 3H), 2.99 (s, 3H), 2.32 (d, J = 1.0 Hz, 3H). |
| **872** | LC-MS: RT=1.675min, LCMS15, m/z=365.3 [M+1]. 1H-NMR: δ 8.51 - 8.39 (m, 3H), 7.86 - 7.76 (m, 3H), 7.16 (dd, J = 9.0, 1.2 Hz, 1H), 5.84 (s, 1H), 4.05 (s, 3H), 2.95 (s, 3H), 2.21 (s, 3H). |
| **873** | LC-MS: (ES, m/z): RT = 0.939 min, LCMS 27: m/z = 385 [M+1]. 1H NMR (300 MHz, Methanol-d4) δ 8.19 (dd, J = 21.0, 2.9 Hz, 1H), 7.69 (dd, J = 8.9, 2.9 Hz, 1H), 7.22 (d, J = 9.0 Hz, 1H), 6.27 - 5.97 (m, 1H), 4.25 - 4.10 (m, 1H), 4.00 (s, 3H), 3.62 (d, J = 12.8 Hz, 2H), 3.29 - 3.14(m 2H), 3.17 - 3.07 (m, 6H), 2.52 - 2.06 (m, 5H), 1.70 - 1.90 (m, 2H). |
| **874** | LC-MS: (ES, m/z): RT = 1.864 min, LCMS 07: m/z = 382 [M+1]. 1H NMR (400 MHz, Methanol-d4) δ 8.26 (d, J = 10.6 Hz, 2H), 8.38 (d, J = 12.4 Hz, 1H), 7.76 - 7.64 (m, 1H), 7.32 - 7.22 (m, 1H), 6.04 (q, J = 1.0 Hz, 1H), 4.34 (q, J = 14.2, 10.4 Hz, 2H), 4.07 (d, J = 5.2 Hz, 3H), 3.00 (d, J = 9.2 Hz, 3H), 2.33 (s, 3H), 1.59 - 1.49 (m, 3H). |
| **876** | LC-MS: (ES, m/z): RT=1.300min, LCMS15, m/z=378.2 [M+1]. 1H NMR (300 MHz, Methanol-d4) δ 7.93 - 7.82 (m, 2H), 7.63 - 7.39 (m, 3H), 7.31 (d, J = 8.2 Hz, 1H), 7.07 (dd, J = 8.1, 2.0 Hz, 1H), 6.03 (s, 1H), 4.59 (s, 1H), 3.94 (s, 3H), 3.03 (s, 3H), 2.32 (s, 3H). |
| **877** | LC-MS: (ES, m/z): RT=1.375min, LCMS15, m/z=378.3 [M+1]. 1H NMR (300 MHz, DMSO-d6) δ 10.28 (s, 1H), 8.83 (s, 1H), 8.68 - 8.57 (m, 1H), 7.83 (d, J = 8.5 Hz, 1H), 7.72 (s, 1H), 7.39 - 7.10 (m, 6H), 6.08 (s, 1H), 4.51 (d, J = 6.1 Hz, 2H), 3.92 (s, 3H), 2.96 (d, J = 4.5 Hz, 3H), 2.28 (s, 3H). |
| **881 (Reference)** | LC-MS: (ES, m/z): RT=0.963min, LCMS28, m/z=389.2 [M+1]. 1H NMR (300 MHz, Methanol-d4) δ 8.03 (s, 1H), 7.57 (d, J = 2.3 Hz, 1H), 6.04 (d, J = 1.5 Hz, 1H), 4.43 - 4.31 (m, 1H), 4.14 - 3.98 (m, 2H), 3.78 (d, J = 10.2 Hz, 3H), 3.47 (d, J = 9.6 Hz, 2H), 3.23 (s,2H), 3.01 (s,2H), 2.33 (s, 3H), 2.21 (s, 3H), 2.15 - 2.01 (m, 4H). |
| **882 (Reference)** | LC-MS: (ES, m/z): RT=0.947min, LCMS28, m/z=389.2 [M+1]. 1H NMR (300 MHz, Methanol-d4) δ 8.05 (s, 1H), 7.58 (s, 1H), 6.07 - 6.00 (m, 1H), 4.43 - 4.31 (m, 1H), 4.09 (d, J = 4.8 Hz, 2H), 4.01 (s, 3H), 3.81 - 3.71 (m, 2H), 3.52 - 3.39 (m, 2H), 3.24 (s, 2H), 3.01 (s, 3H), 2.33 (s, 2H), 2.21 (s, 3H), 2.15 - 2.03 (m, 2H). |
| **883 (Reference)** | LC-MS: (ES, m/z): RT=0.897min, LCMS28, m/z=445.3 [M+1]. 1H NMR (300 MHz, Methanol-d4) δ 8.13 (s, 1H), 7.62 (d, J = 2.3 Hz, 1H), 6.24 (s, 1H), 4.44 - 4.20 (m, 2H), 4.17 - 4.03 (m, 2H), 3.99 (s, 3H), 3.94 - 3.37 (m, 8H), 3.35 - 3.15 (m, 3H), 2.47 (s, 4H), 2.28 - 2.15 (m, 2H), 2.14 - 2.03 (m, 2H). |
| **884 (Reference)** | LC-MS: (ES, m/z): RT=1.373min, LCMS28, m/z=445.2 [M+1]. 1H NMR (300 MHz, Methanol-d4) δ 8.14 (d, J = 2.1 Hz, 1H), 7.61 (s, 1H), 6.23 (s, 1H), 4.44 - 4.20 (m, 2H), 4.11 (dd, J = 5.1, 2.5 Hz, 2H), 3.99 (s, 3H), 3.94 - 3.38 (m, 8H), 3.24 (s, 3H), 2.47 (s, 3H), 2.40 (s, 2H), 2.09 (m, J = 9.2 Hz, 3H). |
| **885 (Reference)** | LC-MS: (ES, m/z): RT=0.962 min, LCMS 07, m/z=389 [M+1]. 1H NMR (400 MHz, Chloroform-d) δ 8.33 (s, 1H), 7.82 (s, 1H), 5.80 (s, 1H), 4.28 (d, J = 24.4, 9.5, 4.8 Hz, 2H), 4.16 (d, J = 9.9, 5.5 Hz, 1H), 3.88 (s, 3H), 3.01 - 2.87 (m, 6H), 2.779 (d, 3H), 2.30 (s, 3H), 1.94 - 1.86 (m, 4H), 1.21 (s, 1H). |
| **886 (Reference)** | LC-MS: (ES, m/z): RT=0.962 min, LCMS27, m/z=389 [M+1]. 1H NMR (400 MHz, Chloroform-d) δ 8.33 (s, 1H), 7.82 (s, 1H), 5.80 (s, 1H), 4.28 (d, J = 24.4, 9.5, 4.8 Hz, 2H), 4.16 (d, J = 9.9, 5.5 Hz, 1H), 3.88 (s, 3H), 3.01 - 2.87 (m, 6H), 2.779 (d, 3H), 2.30 (s, 3H), 1.94 - 1.86 (m, 4H), 1.21 (s, 1H). |
| **887 (Reference)** | LC-MS: (ES, m/z): RT = 0.983 min; LCMS33: m/z = 444 [M+1]. 1H NMR (300 MHz, Methanol-d4) δ 7.94 (d, J = 2.2 Hz, 1H), 7.78 (d, J = 2.3 Hz, 1H), 5.83 (s, 1H), 4.10 (t, J = 6.1 Hz, 2H), 3.92 (s, 3H), 3.64 (t, J = 6.8 Hz, 2H), 2.85 - 2.75 (m, 2H), 2.71 (d, J = 4.6 Hz, 7H), 2.48 (t, J = 6.8 Hz, 2H), 2.17 (s, 3H), 2.09 - 2.07 (m, 2H), 1.95 - 1.79 (m, 4H). |
| **888 (Reference)** | LC-MS: (ES, m/z): RT = 1.38 min, LCMS 33: m/z = 485.3 [M+1]. 1H NMR (300 MHz, Methanol-d4) δ 7.96 (d, J = 2.1 Hz, 1H), 7.55 (d, J = 2.1 Hz, 1H), 6.03 (d, J = 1.2 Hz, 1H), 4.21 (t, J = 5.4 Hz, 2H), 3.99 (d, J = 5.7 Hz, 5H), 3.84 (t, J = 8.4 Hz, 2H), 3.75 - 3.55 (m, 4H), 3.48 (t, J = 7.2 Hz, 2H), 3.17 (q, J = 9.3 Hz, 2H), 2.92 - 2.79 (m, 1H), 2.67 (s, 3H), 2.38 (s, 3H), 2.28 - 2.07 (m, 6H). |
| **890** | LC-MS: (ES, m/z): RT=1.059 min, LCMS27, m/z=403 [M+1]. 1H NMR (400 MHz, Methanol-d4) δ 7.46 (s, 1H), 7.27 (d, J = 2.5 Hz, 1H), 7.21 - 7.13 (m, 1H), 6.97 (d, J = 8.7 Hz, 1H), 5.97 (d, J = 1.1 Hz, 1H), 4.13 (t, J = 5.5 Hz, 2H), 3.83 (s, 3H), 3.16 (t, J = 7.0 Hz, 2H), 2.99 (s, 3H), 2.42 - 2.26 (m, 5H). |
| **891** | LC-MS: (ES, m/z): RT=2.388 min, LCMS07, m/z=417 [M+1]. 1H NMR (300 MHz, Methanol-d4) δ 7.30 (s, 1H), 7.22 (d, J = 2.5 Hz, 1H), 7.16 - 7.04 (m, 1H), 6.98 (d, J = 8.8 Hz, 1H), 5.97 (d, J = 0.9 Hz, 1H), 4.10 (t, J = 5.6 Hz, 2H), 3.83 (s, 3H), 3.73 (s, 3H), 3.08 (t, J = 7.1 Hz, 2H), 2.97 (d, J = 4.8 Hz, 3H), 2.34 - 2.19 (m, 5H). |
| **892 (Reference)** | LC-MS: (ES, m/z): RT=0.983min, LCMS15, m/z=479.3 [M+1]. 1H NMR (300 MHz, Methanol-d4) δ 7.40 (d, J = 2.5 Hz, 1H), 7.14 (dd, J = 8.7, 2.5 Hz, 1H), 6.94 (d, J = 8.8 Hz, 1H), 5.85 (s, 1H), 4.20 - 4.10 (m, 2H), 3.90 - 3.75 (m, 5H), 3.36 (s, 1H), 3.32 (d, J = 3.6 Hz, 1H), 3.08 - 2.91 (m, 6H), 2.65 (s, 3H), 2.23 - 2.07 (m, 5H), 2.05 - 1.89 (m, 4H). |
| **893 (Reference)** | LC-MS: (ES, m/z): RT=1.072min, LCMS28, m/z=493.3 [M+1]. 1H NMR (300 MHz, Methanol-d4) δ 7.39 (d, J = 2.5 Hz, 1H), 7.15 (dd, J = 8.7, 2.4 Hz, 1H), 6.94 (d, J = 8.8 Hz, 1H), 5.85 (s, 1H), 4.22 - 4.11 (m, 2H), 3.88 - 3.77 (m, 5H), 3.34 - 3.24 (m, 2H), 3.10 - 2.97 (m, 6H), 2.81 (s, 6H), 2.18 (d, J = 9.3 Hz, 5H), 2.04 - 1.93 (m, 4H). |
| **894 (Reference)** | LC-MS: (ES, m/z): RT = 1.798 min, LCMS33: m/z = 415 [M+1]. 1H NMR (300 MHz, Methanol-d4) δ 8.15 (s, 1H), 8.01 (s, 1H), 6.77 (s, 1H), 6.41 (s, 1H), 4.32 - 4.29 (m, 2H), 3.96 (s, 3H), 3.88 - 3.76 (m, 4H), 3.73 - 3.70 (m, 2H), 3.48 - 4.44 (m, 4H), 3.26 - 3.10 (m, 2H), 2.37 - 2.34 (m, 2H), 2.32 - 2.17 (m, 3H), 2.10 - 2.06 (m, 2H). |
| **895 (Reference)** | LC-MS: (ES, m/z): RT = 1.221 min; LCMS33: m/z = 326 [M+1]. 1H NMR (300 MHz, Methanol-d4) δ 8.51 (s, 1H), 7.30 (d, J = 5.6 Hz, 2H), 4.25 (t, J = 6.1 Hz, 2H), 3.98 (s, 3H), 2.86 - 2.71 (m, 5H), 2.70 - 2.59 (m, 4H), 2.21 - 2.12 (m, 2H), 1.94 - 1.77 (m, 4H). |
| **896 (Reference)** | LC-MS: (ES, m/z): RT=0.715min LCMS30, m/z =319 [M+1]. 1H NMR (400 MHz, Methanol-d4) δ 8.28 (d, J = 9.7 Hz, 1H), 7.53 - 7.41 (m, 2H), 4.37 (t, J = 5.5 Hz, 2H), 4.04 (s, 3H), 3.94 - 3.75 (m, 2H), 3.51 (t, J = 7.2 Hz, 2H), 3.25 - 3.09 (m, 2H), 2.77 (d, J = 2.6 Hz, 3H), 2.46 - 2.33 (m, 2H), 2.31 - 2.01 (m, 4H). |
| **897 (Reference)** | LC-MS: (ES, m/z): RT=0.990min LCMS 07, m/z =334 [M+1]. 1H NMR (400 MHz, Methanol-d4) δ 8.08 (d, J = 11.2 Hz, 1H), 7.48 (s, 1H), 7.37 (s, 1H), 4.32 (t, J = 5.5 Hz, 2H), 3.98 (s, 3H), 3.90 - 3.80 (m, 2H), 3.51 (t, J = 7.2 Hz, 2H), 3.27 - 3.11 (m, 5H), 2.44 - 2.32 (m, 2H), 2.30 - 2.17 (m, 2H), 2.16 - 2.03 (m, 2H). |
| **900 (Reference)** | LC-MS: (ES, m/z): RT=1.28min, LCMS33: m/z=329.19 [M+1]. 1H NMR (400 MHz, Methanol-d4) δ 7.18 (s, 1H), 6.90 (s, 1H), 6.57 (s,1H), 3.94 (s, 3H), 3.88 - 3.76 (m, 2H), 3.73 - 3.64 (m, 2H), 3.15-3.04 (m, 4H), 2.78 (s, 3H), 2.61 (d, J = 1.0 Hz, 3H), 2.32 - 2.13 (m, 2H). |
| **904** | LC-MS: (ES, m/z): RT=1.447 min, LCMS07, m/z=368 [M+1]. 1H NMR (400 MHz, Methanol-d4) δ 7.77 (s, 1H), 7.51 (dd, J = 9.0, 2.8 Hz, 1H), 6.92 (d, J = 9.0 Hz, 1H), 5.82 (s, 1H), 3.83 (s, 3H), 3.76 (t, J = 4.7 Hz, 4H), 3.57 (s, 2H), 2.92 (s, 3H), 2.70 (dd, J = 5.7, 3.6 Hz, 4H), 2.19 (s, 3H). |
| **905** | LC-MS: (ES, m/z): RT=1.720 min, LCMS28, m/z=388 [M+1]. 1H NMR (300 MHz, Methanol-d4) δ 7.66 - 7.58 (m, 1H), 7.08 (dd, J = 8.7, 2.5 Hz, 1H), 6.91 (d, J = 8.7 Hz, 1H), 5.81 (d, J = 0.8 Hz, 1H), 4.19-4.11 (m, 1H), 4.04 (dd, J = 9.7, 4.4 Hz, 1H), 3.96 (dd, J = 9.7, 6.3 Hz, 1H), 3.84 (s, 3H), 2.93 (s, 3H), 2.82 (dd, J = 12.6, 4.4 Hz, 1H), 2.75 - 2.59 (m, 5H), 2.19 (s, 3H), 1.91 - 1.75 (m, 4H). |
| **906** | LC-MS: (ES, m/z): RT=1.001 min, LCMS28, m/z=388 [M+1]. 1H NMR (300 MHz, Methanol-d4) δ 7.61 (d, J = 2.4 Hz, 1H), 7.07 (dd, J = 8.7, 2.5 Hz, 1H), 6.89 (d, J = 8.7 Hz, 1H), 5.79 (d, J = 0.8 Hz, 1H), 4.18 - 4.08 (m, 1H), 4.08 - 3.90 (m, 2H), 3.83 (s, 3H), 3.01 - 2.73 (m, 4H), 2.70 - 2.55 (m, 5H), 2.17 (s, 3H), 1.83-1.79 (m, 4H). |
| **907 (Reference)** | LC-MS: (ES, m/z): RT=1.01min, LCMS33: m/z=360.15[M+1]. 1H-NMR: (Methanol-d4) δ 7.72 (d, J = 5.8 Hz, 1H), 7.50 (d, J = 2.5 Hz, 1H), 7.09 (dd, J = 8.7, 2.5 Hz, 1H), 6.91 (d, J = 8.7 Hz, 1H), 5.92 (d, J = 6.0 Hz, 1H), 4.05 - 3.90 (m, 3H), 3.85 (s, 3H), 3.44 (t, J = 7.2 Hz, 4H), 2.94 (s, 3H), 2.82 (dd, J = 12.5, 3.7 Hz, 1H), 2.74 - 2.63 (m, 1H), 2.25-2.10(m, 2H). |
| **908 (Reference)** | LC-MS: (ES, m/z): RT=0.94min, LCMS28 : m/z=360.12 [M+1]. 1H-NMR: (Methanol-d4) δ 7.67-7.45 (m, 1H), 7.32 - 7.02 (m, 3H), 6.44 - 6.10 (m, 1H), 4.43 - 4.15 (m, 5H), 4.10 - 3.99 (m, 2H), 3.92-3.78 (m, 3H), 3.61 - 3.47 (m, 1H), 3.45-3.35 (m, 1H), 3.1-2.95 (m, 3H), 2.74 - 2.59 (m, 1H), 2.53-2.31 (m, 1H). |
| **911 (Reference)** | LC-MS: (ES, m/z): RT = 0.614 min; LCMS32: m/z = 359 [M+1]. 1H NMR (400 MHz, Methanol-d4) δ 7.96 (d, J = 2.2 Hz, 1H), 7.79 - 7.71 (m, 2H), 5.94 (d, J = 6.0 Hz, 1H), 4.10 (t, J = 6.1 Hz, 2H), 3.93 (s, 3H), 2.93 (s, 3H), 2.91 - 2.65 (m, 6H), 2.14 - 2.03 (m, 2H), 1.95 - 1.82 (m, 4H). |
| **912 (Reference)** | LC-MS: (ES, m/z): RT = 0.961 min; LCMS27: m/z = 345 [M+1]. 1H NMR (400 MHz, Methanol-d4) δ 7.96 (d, J = 2.2 Hz, 1H), 7.79 (s, 1H), 7.73 (s, 1H), 5.94 (d, J = 6.0 Hz, 1H), 4.03 - 3.90 (m, 5H), 2.94 (s, 3H), 2.91 - 2.62 (m, 4H), 2.68 - 2.44(m, 1H), 2.41 (s, 3H), 2.32 - 2.05(m, 1H), 1.75 - 1.62 (m, 1H). |
| **914 (Reference)** | LC-MS: (ES, m/z): RT= 0.96 min, LCMS 27: m/z = 345 [M+1]. 1H-NMR: (Methanol-d4, ppm): δ 7.97 (d, J = 2.1 Hz, 1H), 7.61 (d, J = 7.3 Hz, 1H), 7.51 (d, J = 2.2 Hz, 1H), 6.20 (d, J = 7.3 Hz, 1H), 4.45 - 4.30 (m, 2H), 4.24 - 4.07 (m, 4H), 4.03 (s, 3H), 3.45 (t, J = 6.9 Hz, 2H), 3.02 (s, 3H), 2.84 - 2.31 (m, 2H), 2.30 - 2.15 (m, 2H). |
| **915 (Reference)** | LC-MS: (ES, m/z): RT = 1.076 min; LCMS27: m/z = 371 [M+1]. 1H NMR (400 MHz, Methanol-d4) δ 8.03 - 7.86 (m, 1H), 7.79 (s, 1H), 7.74 (s, 1H), 5.94 (d, J = 6.0 Hz, 1H), 4.04 - 3.90 (m, 5H), 3.24 - 2.96 (m, 1H), 2.94 (s, 3H), 2.88 - 2.64 (m, 3H), 2.65 - 2.56 (m, 1H), 2.21 - 1.89 (m, 1H), 1.76 (d, J = 5.5 Hz, 1H), 1.69 - 1.62 (m, 1H), 0.56 - 0.41 (m, 4H). |
| **916 (Reference)** | LC-MS: (ES, m/z): RT = 1.124 min; LCMS39: m/z = 318 [M+1]. 1H NMR (400 MHz, Methanol-d4) δ 7.72 (s, 1H), 7.47 (s, 1H), 7.09 (dd, J = 8.7, 2.5 Hz, 1H), 6.99 - 6.73(m, 1H), 5.91 (d, J = 6.0 Hz, 1H), 4.11 (t, J = 6.0 Hz, 2H), 3.83 (s, 3H), 2.93 (s, 3H), 2.81 (t, J = 6.8 Hz, 2H), 2.44 (s, 3H), 2.03 (p, J = 6.4 Hz, 2H). |
| **917 (Reference)** | LC-MS: (ES, m/z): RT= 0.90 min, LCMS 28: m/z = 319 [M+1]. 1H-NMR: (Methanol-d4, ppm): δ 7.97 (d, J = 2.2 Hz, 1H), 7.81 - 7.71 (m, 2H), 5.95 (d, J = 6.0 Hz, 1H), 4.15 (t, J = 5.9 Hz, 2H), 3.95 (s, 3H), 2.98 - 2.80 (m, 5H), 2.55 (s, 3H), 2.20 - 2.03 (m,2H). |
| **918 (Reference)** | LC-MS: (ES, m/z): RT=0.837 min, LCMS 07, m/z=344.0[M+1]. 1H NMR (400 MHz, Methanol-d4) δ 7.60 - 7.53 (m, 1H), 7.25 (s, 1H), 7.16 - 7.03 (m, 2H), 6.17 (d, J = 7.3 Hz, 1H), 4.28 (t, J = 8.5, 4.2 Hz, 1H), 4.15 - 4.04 (m, 2H), 3.91 (d, J = 2.0, 1.1 Hz, 3H), 3.23 (d, J = 12.7, 8.4 Hz, 1H), 3.03 (s, 4H), 2.80 (s, 3H). |
| **919 (Reference)** | LC-MS: (ES, m/z): RT = 1.006 min, LCMS28: m/z = 388 [M+1]. 1H NMR (300 MHz, Methanol-d4) δ 7.57 (d, J = 7.3 Hz, 1H), 7.28 - 7.00 (m, 3H), 6.18 (d, J = 7.3 Hz, 1H), 4.31 - 4.10 (m, 2H), 4.02 (dq, J = 8.0, 3.8 Hz, 1H), 3.90 (s, 3H), 3.80 - 3.70 (m, 2H), 3.59 (s, 4H), 3.49 (dd, J = 13.2, 3.2 Hz, 1H), 3.24 (s, 2H), 3.04 (s, 3H), 2.21 (s, 2H), 2.11 (d, J = 8.4 Hz, 2H). |
| **920 (Reference)** | LC-MS: (ES, m/z): RT = 0.997 min, LCMS28: m/z = 388 [M+1]. 1H NMR (300 MHz, Methanol-d4) δ 7.56 (d, J = 7.3 Hz, 1H), 7.27 - 7.00 (m, 3H), 6.17 (d, J = 7.3 Hz, 1H), 4.30 - 4.09 (m, 2H), 4.08 - 3.95 (m, 1H), 3.89 (s, 3H), 3.74 (s, 2H), 3.58 (s, 4H), 3.48 (dd, J = 13.2, 3.2 Hz, 1H), 3.24 (t, J = 8.6 Hz, 2H), 3.04 (s, 3H), 2.24 - 2.03 (m, 4H). |
| **922 (Reference)** | LC-MS: (ES, m/z): RT = 0.966 min, LCMS28: m/z = 348 [M+1]. 1H NMR (300 MHz, Methanol-d4) δ 7.56 (d, J = 7.2 Hz, 1H), 7.24 (s, 1H), 7.20 - 7.03 (m, 2H), 6.17 (d, J = 7.3 Hz, 1H), 4.29 (dd, J = 10.4, 4.3 Hz, 1H), 4.16 (dd, J = 10.4, 3.6 Hz, 1H), 3.91 (s, 4H), 3.56 (s, 3H), 3.39 (td, J = 10.8, 8.8, 5.6 Hz, 2H), 3.03 (s, 3H), 2.80 (s, 3H). |
| **927 (Reference)** | LC-MS: (ES, m/z): RT = 1.437 min, LCMS 07: m/z = 358 [M+1]. 1H NMR (300 MHz, Methanol-d4) δ 7.57 (d, J = 3.2 Hz, 1H), 7.33 - 7.28 (m, 3H), 6.19 (d, J = 2.4 Hz, 1H), 4.13 - 4.10 (m, 2H), 4.00 - 3.78 (m, 4H), 3.72 - 3.68 (m, 1H), 3.44 - 3.35 (m, 2H), 3.29 - 3.10 (m, 1H),3.16 - 3.09(m, 1H) 3.03 (s, 4H), 2.53 - 1.93 (m, 2H), 1.41 - 1.39 (m, 3H). |
| **928 (Reference)** | LC-MS: (ES, m/z): RT = 1.369 min, LCMS 33: m/z = 370 [M+1]. 1H NMR (300 MHz, Methanol-d4) δ 7.56 (d, J = 2.4 Hz, 1H), 7.21 (s, 1H), 7.08 (d, J = 1.2 Hz, 2H), 6.18 (d, J = 1.8 Hz, 1H), 4.20 - 4.05 (m, 2H), 4.02 - 3.79 (m, 4H), 3.78 - 3.67 (m, 1H), 3.61 - 3.58 (m, 1H), 3.53 - 3.36 (m, 1H), 3.13 (s, 1H), 3.03 (s, 4H), 2.26 - 2.23 (m, 2H), 1.04-1.01 (m, 4H). |
| **931 (Reference)** | LC-MS: (ES, m/z): RT = 1.86 min, LCMS 53: m/z = 360 [M+1]. 1H NMR (300 MHz, Methanol-d4) δ 7.72 (d, J = 6.3 Hz, 1H), 7.51 (s, 1H), 7.09 (d, J = 8.7 Hz, 1H), 6.90 (d, J = 8.7 Hz, 1H), 5.92 (d, J = 6.0 Hz, 1H), 4.14 - 3.87 (m, 4H), 3.82 (s, 3H), 3.71 (t, J = 2.4 Hz, 1H), 3.04 - 2.90 (m, 4H), 2.74 (q, J = 1.8 Hz, 1H), 2.35 (s, 3H), 2.29 - 2.07 (m, 1H) , 2.05 - 2.00 (m, 1H). |
| **932 (Reference)** | LC-MS: (ES, m/z): RT = 0.92 min, LCMS 33: m/z = 374 [M+1]. 1H NMR (300 MHz, Methanol-d4) δ 7.72 (d, J = 6.0 Hz, 1H), 7.52 (d, J = 2.4 Hz, 1H), 7.09 (d, J = 8.7 Hz, 1H), 6.90 (d, J = 8.7 Hz, 1H), 5.92 (d, J = 6.0 Hz, 1H), 4.15 - 3.88 (m, 4H), 3.86 - 3.65 (m, 4H), 3.09 - 3.05 (m, 1H), 2.94 (s, 3H), 2.89 - 2.77 (m, 1H), 2.50 (q, J = 7.2 Hz, 2H), 2.26 - 1.97 (m, 2H), 1.15 (t, J = 7.2 Hz, 3H). |
| **933 (Reference)** | LC-MS: (ES, m/z): RT = 1.86 min, LCMS 53: m/z = 346 [M+1]. 1H NMR (300 MHz, Methanol-d4) δ 7.71 (d, J = 6.3 Hz, 1H), 7.50 (d, J = 2.4 Hz, 1H), 7.09 (q, J = 2.4 Hz, 1H), 6.90 (d, J = 8.4 Hz, 1H), 5.92 (d, J = 6.0 Hz, 1H), 4.12 - 3.99 (m, 4H), 3.84 (s, 3H), 3.69 - 3.64 (m, 1H), 3.10 - 3.08 (m, 1H), 2.97 (s, 3H), 2.90 - 2.71 (m, 3H). |
| **936 (Reference)** | LC-MS: (ES, m/z): RT = 0.912 min; LCMS33: m/z = 360 [M+1].1H NMR (300 MHz, Methanol-d4) δ 7.55 (d, J = 7.3 Hz, 1H), 7.22 (s, 1H), 7.18 - 6.99 (m, 2H), 6.17 (d, J = 7.3 Hz, 1H), 4.26 - 4.09 (m, 4H), 3.89 (s, 4H), 3.70 - 3.68(m, 1H), 3.51 - 3.50 (m, 1H), 3.22 - 3.20 (m, 2H), 3.02 (d, J = 7.7 Hz, 6H). |
| **937 (Reference)** | LC-MS: (ES, m/z): RT = 0.931 min; LCMS33: m/z = 374 [M+1]. 1H NMR (300 MHz, Methanol-d4) δ 7.72 (d, J = 5.9 Hz, 1H), 7.50 (d, J = 2.4 Hz, 1H), 7.10 (d, J = 8.7 Hz, 1H), 6.92 (d, J = 8.7 Hz, 1H), 5.94 (d, J = 6.1 Hz, 1H), 4.20 - 4.05 (m, |
| | 1H), 4.04 - 3.90 (m, 3H), 3.84 (s, 3H), 3.74 - 3.72 (m, 1H), 3.15 - 3.04 (m, 1H), 2.95 (s, 3H), 2.86 - 2.84 (m, 1H), 2.52 (q, J = 7.2 Hz, 2H), 2.29 - 2.00 (m, 2H), 1.16 (t, J = 7.2 Hz, 3H). |
| **938 (Reference)** | LC-MS: (ES, m/z): RT = 0.907 min; LCMS33: m/z = 346 [M+1]. 1H NMR (300 MHz, Methanol-d4) δ 7.72 (d, J = 6.0 Hz, 1H), 7.51 (s, 1H), 7.09 (d, J = 8.7 Hz, 1H), 6.90 (d, J = 8.7 Hz, 1H), 5.92 (d, J = 6.0 Hz, 1H), 4.05 - 4.03 (m, 1H), 4.00 - 3.84 (m, 3H), 3.82 (s, 3H), 3.67 - 3.64 (m, 1H), 3.12 - 3.01 (m, 1H), 2.94 (s, 3H), 2.88 - 2.79 (m, 2H), 2.72 - 2.68 (m, 1H). |
| **949 (Reference)** | LC-MS: (ES, m/z): RT = 1.509min; LCMS15: m/z = 358 [M+1]. 1H NMR (300 MHz, Methanol-d4) δ 7.97 (d, J = 2.2 Hz, 1H), 7.79 (d, J = 2.3 Hz, 1H), 5.82 (d, J = 0.8 Hz, 1H), 4.04 (t, J = 6.2 Hz, 2H), 3.92 (s, 3H), 3.33 - 3.28 (m, 4H), 2.91 (s, 3H), 2.67 (q, J = 8.1, 2H), 2.21 - 2.05 (m, 5H), 1.77 - 1.86 (m, 6.2 Hz, 2H). |
| **1005 (Reference)** | LC-MS: (ES, m/z): RT = 0.611min, LCMS 32, m/z = 374.2 [M+1]. 1H NMR (400 MHz, Methanol-d4) δ 7.58 (d, J = 7.3 Hz, 1H), 7.32 - 7.26 (m, 1H), 7.21 - 7.05 (m, 2H), 6.17 (d, J = 7.3 Hz, 1H), 4.50 - 4.19 (m, 4H), 4.06 - 3.92 (m, 5H), 3.57 (d, J = 13.3 Hz, 1H), 3.39 (d, J = 13.3 Hz, 1H), 3.04 (s, 3H), 2.75 - 2.60 (m, 1H), 2.50 - 2.35 (m, 1H), 1.36 (s, 3H). |
| **1011 (Reference)** | LC-MS: (ES, m/z): RT=0.982 min, LCMS 28, m/z =332.2 [M+1]. 1H NMR (400 MHz, Methanol-d4) δ 7.58 (d, J = 7.3 Hz, 1H), 7.29 - 7.17 (m, 2H), 7.10 (d, J = 8.7 Hz, 1H), 6.17 (d, J = 7.3 Hz, 1H), 4.60 - 4.49 (m, 1H), 3.92 (s, 3H), 3.32 - 3.25 (m, 2H), 3.02 (s, 3H), 2.78 (s, 3H), 2.17 - 2.00 (m, 2H), 1.37 (d, J = 6.1 Hz, 3H). |
| **1014 (Reference)** | LC-MS: (ES, m/z): RT=1.00min, LCMS28 : m/z=358.14 [M+1]. 1H-NMR (Methanol-d4) δ 7.95-7.52 (m, 1H), 7.45-6.89 (m, 3H), 6.44 - 6.10 (m, 1H), 4.59-4.37 (m,1H), 4.35 - 4.09 (m, 5H), 3.97-3.88 (m, 3H), 3.75-3.52 (m, 1H), 3.12-2.94 (m, 3H), 2.71 - 2.57 (m, 1H), 2.48-2.27 (m, 1H), 2.26-2.01 (m, 2H), 1.34 (d, J = 6.5 Hz, 3H). |
| **1019 (Reference)** | LC-MS: (ES, m/z): RT = 6.569 min; LCMS53: m/z = 343[M+1]. 1H NMR (300 MHz, Methanol-d4) δ 7.80 - 7.55 (m, 3H), 7.19 (d,1H), 6.19 (d, J = 7.3 Hz, 1H), 4.36 (s, 2H), 4.12 - 3.92 (m, 4H), 3.67 - 3.31 (m, 4H), 3.02 (s, 3H), 2.46 - 2.29 (m, 1H), 2.24 - 1.98 (m, 2H), 1.89 - 1.84 (m, 1H). |
| **1020 (Reference)** | LC-MS: (ES, m/z): RT = 8.458 min; LCMS53: m/z = 343[M+1]. 1H NMR (300 MHz, Methanol-d4) δ 7.82 - 7.55 (m, 3H), 7.19 (d, J = 8.9 Hz, 1H), 6.19 (d, J = 7.3 Hz, 1H), 4.36 (s, 2H), 4.10 - 3.92 (m, 4H), 3.64 - 3.35 (m, 5H), 3.02 (s, 3H), 2.38 (m, 1H), 2.22 - 1.98 (m, 2H), 1.86 - 1.83 (m, 1H). |
| **1031 (Reference)** | LC-MS: (ES, m/z): (ES, m/z): RT = 0.908min, LCMS07: m/z =372 [M +1]. 1H NMR (400 MHz, Methanol-d4) δ 8.67 - 8.62 (m, 1H), 8.02 (d, J = 2.6 Hz, 1H), 6.03 (d, J = 1.1 Hz, 1H), 4.35 (s, 2H), 4.06 (d, J = 7.1 Hz, 3H), 3.71 - 3.55 (m, 4H), 3.48 (s, 4H), 2.99 (s, 3H), 2.33 (d, J = 1.0 Hz, 3H), 2.18 - 2.10 (m, 4H). |
| **1032 (Reference)** | LC-MS: (ES, m/z): RT = 0.868 min, LCMS28: m/z =357 [M+1]. 1H NMR (400 MHz, DMSO-d6) δ 8.83 (s, 1H), 7.82 - 7.74 (m, 2H), 7.68 (d, J = 8.9 Hz, 1H), 7.10 (s, 1H), 6.96 (d, J = 8.9 Hz, 1H), 5.89 (d, J = 5.9 Hz, 1H), 3.95 (s, 2H), 3.79 (s, 3H), 2.96 - 2.78 (m, 7H), 2.77 - 2.69 (m, 4H), 1.84 - 1.72 (m, 4H). |

### Example 156: Bioactivity Assays

### MATERIALS AND EQUIPMENT:

Recombinant purified human EHMT2 913-1193 (55 µM) synthesized by Viva was used for all experiments. Biotinylated histone peptides were synthesized by Biopeptide and HPLC-purified to > 95% purity. Streptavidin Flashplates and seals were purchased from PerkinElmer and 384 Well V-bottom Polypropylene Plates were from Greiner. ³H-labeled S-adenosylmethionine (³H-SAM) was obtained from American Radiolabeled Chemicals with a specific activity of 80 Ci/mmol. Unlabeled SAM and S-adenosylhomocysteine (SAH) were obtained from American Radiolabeled Chemicals and Sigma-Aldrich respectively. Flashplates were washed in a Biotek ELx-405 with 0.1% Tween. 384-well Flashplates and 96-well filter binding plates were read on a TopCount microplate reader (PerkinElmer). Compound serial dilutions were performed on a Freedom EVO (Tecan) and spotted into assay plates using a Thermo Scientific Matrix PlateMate (Thermo Scientific). Reagent cocktails were added by Multidrop Combi (Thermo Scientific).

MDA-MB-231 cell line was purchased from ATCC (Manassas, VA, USA). RPMI/Glutamax medium, Penicillin-Streptomycin, Heat Inactivated Fetal Bovine Serum, and D-PBS were purchased from Life Technologies (Grand Island, NY, USA). Odyssey blocking buffer, 800CW goat anti-mouse IgG (H+L) antibody, and Licor Odyssey Infrared Scanner were purchased from Licor Biosciences, Lincoln, NE, USA. H3K9me2 mouse monoclonal antibody (Cat #1220) was purchased from Abcam (Cambridge, MA, USA). 16% Paraformaldehyde was purchased from Electron Microscopy Sciences, Hatfield, PA, USA).MDA-MB-231 cells were maintained in complete growth medium (RPMI supplemented with 10% v/v heat inactivated fetal bovine serum) and cultured at 37°C under 5% CO₂. UNC0638 was purchased from Sigma-Aldrich (St. Louis, MO, USA).

**General Procedure for EHMT2 Enzyme Assay on Histone Peptide Substrate.** 10-point curves of test compounds were made on a Freedom EVO (Tecan) using serial 3-fold dilutions in DMSO, beginning at 2.5 mM (final top concentration of compound was 50 µM and the DMSO was 2%). A 1 µL aliquot of the inhibitor dilution series was spotted in a polypropylene 384-well V-bottom plate (Greiner) using a Thermo Scientific Matrix PlateMate (Thermo Scientific). The 100% inhibition control consisted of 1 mM final concentration of the product inhibitor S-adenosylhomocysteine (SAH, Sigma-Aldrich). Compounds were incubated for 30 minutes with 40 µL per well of 0.031 nM EHMT2 (recombinant purified human EHMT2 913-1193, Viva) in 1X assay buffer (20 mM Bicine [pH 7.5], 0.002% Tween 20, 0.005% Bovine Skin Gelatin and 1 mM TCEP). 10 µL per well of substrate mix comprising assay buffer, ³H-SAM (³H-labeled S-adenosylmethionine, American Radiolabeled Chemicals, specific activity of 80 Ci/mmol), unlabeled SAM (American Radiolabeled Chemicals), and peptide representing histone H3 residues 1-15 containing C-terminal biotin (appended to a C-terminal amide-capped lysine, synthesized by Biopeptide and HPLC-purified to greater than 95% purity) were added to initiate the reaction (both substrates were present in the final reaction mixture at their respective Kₘ values, an assay format referred to as "balanced conditions"). Reactions were incubated for 60 minutes at room temperature and quenched with 10 µL per well of 400 µM unlabeled SAM, then transferred to a 384-well streptavidin Flashplate (PerkinElmer) and washed in a Biotek ELx-405 well washer with 0.1% Tween after 60 minutes. 384-well Flashplates were read on a TopCount microplate reader (PerkinElmer).

**General Procedure for MDA-MB-231 HEK9me2 in-cell Western** Assay. Compound (100 nL) was added directly to 384-well cell plate. MDA-MB-231 cells (ATCC) were seeded in assay medium (RPMI/Glutamax supplemented with 10% v/v heat inactivated fetal bovine serum and 1% Penicillin/Streptomycin, Life Technologies) at a concentration of 3,000 cells per well to a Poly-D-Lysine coated 384-well cell culture plate with 50 µL per well. Plates were incubated at 37°C, 5% CO₂ for 48 hours (BD Biosciences 356697). Plates were incubated at room temperature for 30 minutes and then incubated at 37°C, 5% CO₂ for additional 48 hours. After the incubation, 50 µL per well of 8% paraformaldehyde (Electron Microscopy Sciences) in PBS was added to the plates and incubated at room temperature for 20 minutes. Plates were transferred to a Biotek 406 plate washer and washed 2 times with 100 µL per well of wash buffer (1X PBS containing 0.3% Triton X-100 (v/v)). Next, 60 µL per well of Odyssey blocking buffer (Licor Biosciences) was added to each plate and incubated for 1 hour at room temperature. Blocking buffer was removed and 20 µL of monoclonal primary antibody α-H3K9me2 (Abcam) diluted 1:800 in Odyssey buffer with 0.1% Tween 20 (v/v) were added and plates were incubated overnight (16 hours) at 4 °C. Plates were washed 5 times with 100 µL per well of wash buffer. Next 20 µL per well of secondary antibody was added (1:500 800CW donkey anti-mouse IgG (H+L) antibody (Licor Biosciences), 1:1000 DRAQ5 (Cell Signaling Technology) in Odyssey buffer with 0.1% Tween 20 (v/v)) and incubated for 1 hour at room temperature. The plates were washed 5 times with 100 µL per well wash buffer then 2 times with 100 µL per well of water. Plates were allowed to dry at room temperature then imaged on a Licor Odyssey Infrared Scanner (Licor Biosciences) which measured integrated intensity at 700nm and 800nm wavelengths. Both 700 and 800 channels were scanned.

**% Inhibition Calculation.** First, the ratio for each well was determined by: $\left(\frac{H 3 K 9 m ∅ 2 800 nm value}{DRAQ 5 700 nm value}\right)$.

Each plate included fourteen control wells of DMSO only treatment (Minimum Inhibition) as well as fourteen control wells (background wells) for maximum inhibition treated with control compound UNC0638 (Background wells).

The average of the ratio values for each well was calculated and used to determine the percent inhibition for each test well in the plate. Control compound was serially diluted three-fold in DMSO for a total of 10 test concentrations beginning at 1 µM. Percent inhibition was calculated as: Percent Inhibition = 100- $\left(\left(\frac{\left(Individual Text Sample Ratio\right) - \left(Background Avg Ratio\right)}{\left(Minimum Inhibition Ratio\right) - \left(Background Average Ratio\right)}\right)∗100\right)$

IC₅₀ curves were generated using triplicate wells per concentration of compound. The IC₅₀ is the concentration of compound at which measured methylation is inhibited by 50% as interpolated from the dose response curves. IC₅₀ values were calculated using a non-linear regression (variable slope-four parameter fit model) with by the following formula: $\left(% inhibition=Bottom+\frac{Top - Bottom}{\left(1+{\left({IC}_{50}/\left[I\right]\right)}^{n}\right)}\right) ,$ where *Top* is fixed at 100% and *Bottom* is fixed to 0%, [1] = concentration of inhibitor, *IC*₅₀ = half maximal inhibitory concentration , and *n* = Hill Slope.

The IC₅₀ values are listed in Tables II-VII below ("A" means IC₅₀ < 100nM; "B" means IC₅₀ ranging between 100 nM and 1 µM; "C" means IC₅₀ ranging between > 1 µM and 10 µM; "D" means IC₅₀ >10 µM; "ND" means not determined).

**Table II**

| Compound No. | **EHMT2 PEP (IC50 µM)** | **EHMT1 PEP (IC50 µM)** | **EHMT2 ICW (IC50 µM)** |
|---|---|---|---|
| 1 (Reference) | A | A | A |
| 2 (Reference) | A | A | B |
| 3 (Reference) | A | A | B |
| 4 (Reference) | A | B | B |
| 5 (Reference) | B | B | B |
| 6 (Reference) | A | B | C |
| 7 (Reference) | A | A | C |
| 8 (Reference) | B | B | C |
| 9 (Reference) | C | D | D |
| 10 (Reference) | A | B | D |
| 11 (Reference) | A | A | ND |
| 12 (Reference) | C | C | D |
| 13 (Reference) | D | D | D |
| 14 (Reference) | D | D | D |
| 15 (Reference) | A | A | D |
| 16 (Reference) | B | B | D |
| 17 (Reference) | B | B | D |
| 18 (Reference) | B | B | C |
| 19 (Reference) | A | A | B |
| 20 (Reference) | A | B | B |
| 21 (Reference) | A | B | B |
| 22 (Reference) | A | A | B |
| 23 (Reference) | B | B | B |
| 24 (Reference) | A | A | B |
| 25 (Reference) | A | A | B |
| 26 (Reference) | A | B | B |
| 27 (Reference) | B | B | B |
| 28 (Reference) | A | A | B |
| 29 (Reference) | A | A | B |
| 30 (Reference) | A | B | B |
| 31 (Reference) | B | B | B |
| 32 (Reference) | A | A | B |
| 33 (Reference) | A | B | B |
| 34 (Reference) | A | B | B |
| 35 (Reference) | B | B | B |
| 36 (Reference) | A | B | B |
| 37 (Reference) | B | B | B |
| 38 (Reference) | A | B | B |
| 39 (Reference) | A | A | B |
| 40 (Reference) | B | B | C |
| 41 (Reference) | B | B | C |
| 42 (Reference) | B | B | C |
| 43 (Reference) | B | B | C |
| 44 (Reference) | B | B | C |
| 45 (Reference) | B | B | C |
| 46 (Reference) | A | B | C |
| 47 (Reference) | A | A | C |
| 48 (Reference) | B | B | C |
| 49 (Reference) | B | B | C |
| 50 (Reference) | B | B | C |
| 51 (Reference) | B | B | C |
| 52 (Reference) | C | C | C |
| 53 (Reference) | C | C | C |
| 54 (Reference) | B | B | C |
| 55 (Reference) | B | B | C |
| 56 (Reference) | C | C | C |
| 57 (Reference) | C | C | C |
| 58 (Reference) | D | D | ND |
| 59 (Reference) | D | D | ND |
| 60 (Reference) | C | C | ND |
| 61 (Reference) | D | D | ND |
| 62 (Reference) | D | D | ND |
| 63 (Reference) | D | D | ND |
| 64 (Reference) | D | D | ND |
| 65 (Reference) | D | D | ND |
| 66 (Reference) | D | D | ND |
| 67 (Reference) | D | D | ND |
| 68 (Reference) | D | D | ND |
| 69 (Reference) | D | D | ND |
| 70 (Reference) | D | D | ND |
| 71 (Reference) | D | D | ND |
| 72 (Reference) | D | D | ND |
| 73 (Reference) | D | D | ND |
| 74 (Reference) | D | D | ND |
| 75 (Reference) | D | D | ND |
| 76 (Reference) | D | D | ND |
| 77 (Reference) | D | D | ND |
| 78 (Reference) | D | D | ND |
| 79 (Reference) | D | C | ND |
| 80 (Reference) | D | D | ND |
| 81 (Reference) | D | D | ND |
| 82 (Reference) | D | D | ND |
| 83 (Reference) | D | D | ND |
| 84 (Reference) | D | D | ND |
| 85 (Reference) | D | D | ND |
| 86 (Reference) | C | C | ND |
| 87 (Reference) | C | C | ND |
| 88 (Reference) | B | B | C |
| 89 (Reference) | D | D | ND |
| 90 (Reference) | A | A | D |
| 91 (Reference) | D | C | ND |
| 92 (Reference) | D | D | ND |
| 93 (Reference) | D | D | ND |
| 94 (Reference) | C | C | ND |
| 95 (Reference) | D | D | ND |
| 96 (Reference) | C | C | ND |
| 97 (Reference) | C | C | ND |
| 98 (Reference) | D | D | ND |
| 99 (Reference) | D | D | ND |
| 100 (Reference) | D | D | ND |
| 101 (Reference) | C | D | ND |
| 102 (Reference) | D | D | ND |
| 103 (Reference) | B | B | D |
| 104 (Reference) | D | D | ND |
| 105 (Reference) | C | D | ND |
| 106 (Reference) | C | B | ND |
| 107 (Reference) | D | D | ND |
| 108 (Reference) | C | C | ND |
| 109 (Reference) | C | C | ND |
| 110 (Reference) | C | C | ND |
| 111 (Reference) | D | D | ND |
| 112 (Reference) | D | D | ND |
| 113 (Reference) | C | C | ND |
| 114 (Reference) | D | D | ND |
| 115 (Reference) | D | D | ND |
| 116 (Reference) | C | C | ND |
| 117 (Reference) | C | C | ND |
| 118 (Reference) | B | B | ND |
| 119 (Reference) | D | D | ND |
| 120 (Reference) | C | B | ND |
| 121 (Reference) | C | C | ND |
| 122 (Reference) | D | D | ND |
| 123 (Reference) | D | D | ND |
| 124 (Reference) | C | C | ND |
| 125 (Reference) | C | C | ND |
| 126 (Reference) | D | D | D |
| 127 (Reference) | D | D | D |
| 128 (Reference) | D | D | D |
| 129 (Reference) | D | D | D |
| 130 (Reference) | D | D | D |
| 131 (Reference) | D | D | D |
| 132 (Reference) | D | D | D |
| 133 (Reference) | C | D | D |
| 134 (Reference) | D | D | D |
| 135 (Reference) | C | C | D |
| 136 (Reference) | C | C | D |
| 137 (Reference) | C | C | D |
| 138 (Reference) | C | D | D |
| 139 (Reference) | D | D | D |
| 140 (Reference) | C | C | D |
| 141 (Reference) | D | D | D |
| 142 (Reference) | C | C | D |
| 143 (Reference) | C | C | D |
| 144 (Reference) | D | D | D |
| 145 (Reference) | C | C | D |
| 146 (Reference) | D | D | C |
| 147 (Reference) | C | C | D |
| 148 (Reference) | C | D | D |
| 149 (Reference) | D | D | D |
| 150 (Reference) | B | C | D |
| 151 (Reference) | D | D | D |
| 152 (Reference) | C | C | D |
| 153 (Reference) | C | C | D |
| 154 (Reference) | C | D | D |
| 155 (Reference) | C | D | D |
| 156 (Reference) | C | C | D |
| 157 (Reference) | C | C | D |
| 158 (Reference) | D | D | D |
| 159 (Reference) | D | D | C |
| 160 (Reference) | D | D | D |
| 161 (Reference) | D | D | D |
| 162 (Reference) | D | D | D |
| 163 (Reference) | D | D | D |
| 164 (Reference) | D | D | D |
| 165 (Reference) | B | B | D |
| 166 (Reference) | D | D | D |
| 167 (Reference) | B | B | B |
| 168 (Reference) | D | D | C |
| 169 (Reference) | D | D | D |
| 170 (Reference) | D | D | D |
| 171 (Reference) | C | C | C |
| 172 (Reference) | C | C | D |
| 173 (Reference) | C | C | D |
| 174 (Reference) | D | D | D |
| 175 (Reference) | D | D | D |
| 176 (Reference) | D | D | D |
| 177 (Reference) | C | D | D |
| 178 (Reference) | B | B | B |
| 179 (Reference) | D | D | D |
| 180 (Reference) | D | D | D |
| 181 (Reference) | D | D | D |
| 182 (Reference) | B | B | D |
| 183 (Reference) | C | C | C |
| 184 (Reference) | D | D | D |
| 185 (Reference) | C | C | D |
| 186 | B | B | B |
| 187 (Reference) | C | C | C |
| 188 (Reference) | B | B | D |
| 190 (Reference) | C | C | ND |
| 191 (Reference) | A | A | B |
| 192 (Reference) | B | B | C |
| 193 (Reference) | B | B | B |
| 194 (Reference) | D | D | ND |
| 195 (Reference) | C | C | C |
| 196 (Reference) | D | D | ND |
| 197 (Reference) | D | D | ND |
| 199 (Reference) | B | B | C |
| 200 (Reference) | D | D | ND |
| 201 (Reference) | D | D | ND |
| 202 (Reference) | D | D | ND |
| 203 (Reference) | B | B | B |
| 204 (Reference) | C | C | D |
| 205 | A | A | A |
| 206 (Reference) | B | B | D |
| 207 (Reference) | C | C | C |
| 208 (Reference) | B | B | C |
| 209 (Reference) | B | B | C |
| 210 (Reference) | C | C | C |
| 211 (Reference) | C | C | ND |
| 212 (Reference) | C | C | ND |
| 213 (Reference) | D | D | ND |
| 214 (Reference) | D | D | D |
| 215 (Reference) | D | D | D |
| 216 (Reference) | B | B | D |
| 217 (Reference) | D | D | D |
| 218 (Reference) | D | D | D |
| 219 (Reference) | D | D | D |
| 220 (Reference) | D | D | D |
| 221 (Reference) | D | D | D |
| 222 (Reference) | D | D | D |
| 223 (Reference) | C | C | D |
| 224 (Reference) | D | D | D |
| 225 (Reference) | C | C | C |
| 226 (Reference) | B | C | C |
| 227 (Reference) | D | D | D |
| 228 (Reference) | D | D | D |
| 229 (Reference) | B | B | C |
| 230 (Reference) | C | C | C |
| 231 (Reference) | B | B | B |
| 232 | D | D | D |
| 233 (Reference) | D | D | D |
| 234 (Reference) | D | D | D |
| 235 (Reference) | D | D | D |
| 236 | B | B | B |
| 237 (Reference) | C | C | D |
| 238 | D | D | D |
| 239 (Reference) | D | D | D |
| 240 (Reference) | A | A | B |
| 241 (Reference) | D | D | D |
| 242 (Reference) | C | C | D |
| 243 (Reference) | D | D | D |
| 244 (Reference) | D | D | D |
| 245 (Reference) | B | B | C |
| 246 (Reference) | B | B | D |
| 247 (Reference) | C | C | C |
| 248 (Reference) | A | A | B |
| 249 (Reference) | D | D | D |
| 250 (Reference) | C | C | D |
| 251 (Reference) | D | D | D |
| 252 (Reference) | C | C | C |
| 253 (Reference) | D | D | D |

**Table III**

| **Compound No.** | **EHMT2 PEP (IC50 µM)** | **EHMT1 PEP (IC50 µM)** | **EHMT2 ICW (IC50 µM)** |
|---|---|---|---|
| 256 (Reference) | D | D | ND |
| 257 (Reference) | D | D | D |
| 258 (Reference) | D | D | D |
| 259 (Reference) | D | D | D |
| 260 (Reference) | D | D | D |
| 261 | D | D | D |
| 262a and 262b (Reference) | D | D | D |
| 263 | A | A | ND |
| 264 (Reference) | D | D | D |
| 265 (Reference) | D | D | D |
| 266 (Reference) | C | C | C |
| 267 (Reference) | D | D | D |
| 268 (Reference) | D | D | D |
| 270 (Reference) | A | A | B |
| 271 (Reference) | C | B | D |
| 272 (Reference) | B | C | C |
| 273 (Reference) | D | D | D |
| 274 (Reference) | C | C | D |
| 275 (Reference) | D | D | D |
| 276 (Reference) | D | C | D |
| 277 (Reference) | D | D | D |
| 278 (Reference) | A | A | C |
| 279 (Reference) | D | D | D |
| 280 (Reference) | C | C | C |
| 281 (Reference) | A | ND | B |
| 282 (Reference) | D | D | D |
| 283 (Reference) | A | A | B |
| 284 (Reference) | D | D | D |
| 285 (Reference) | D | D | D |
| 286 (Reference) | A | B | B |
| 287 (Reference) | D | D | D |
| 288 (Reference) | B | B | C |
| 289 (Reference) | B | A | B |
| 290 (Reference) | B | B | C |
| 291 (Reference) | D | D | D |
| 293 (Reference) | C | B | C |
| 295 (Reference) | D | D | D |
| 296 (Reference) | D | D | D |
| 297 (Reference) | C | D | D |
| 298 (Reference) | A | A | B |
| 299 (Reference) | A | A | B |
| 300 (Reference) | B | B | B |
| 301 | D | D | D |
| 302 | C | C | D |
| 303 (Reference) | A | B | C |
| 304 (Reference) | A | A | B |
| 305 (Reference) | A | A | B |
| 306 (Reference) | C | D | D |
| 307 (Reference) | A | A | B |
| 308 (Reference) | B | B | C |
| 309 (Reference) | A | A | B |
| 310 (Reference) | B | B | D |
| 311 | D | D | D |
| 312 | D | D | D |
| 313 | B | B | C |
| 314 | B | B | C |
| 315 (Reference) | C | C | ND |
| 316 | C | B | C |
| 317 | C | D | C |
| 318 | D | D | D |
| 319 | B | B | C |
| 320 | A | A | B |
| 321 (Reference) | C | C | D |
| 322 (Reference) | A | A | B |
| 323 (Reference) | D | D | D |
| 324 (Reference) | C | C | D |
| 325 (Reference) | C | C | C |
| 326 (Reference) | B | B | C |
| 328 | B | B | C |
| 329 | C | C | D |
| 330 | D | C | D |
| 331 | D | D | D |
| 332 (Reference) | A | A | B |
| 333 (Reference) | C | C | D |
| 334 | A | A | B |
| 335 (Reference) | B | B | C |
| 336 (Reference) | B | B | C |
| 337 | C | B | C |
| 338 (Reference) | D | D | D |
| 339 (Reference) | D | D | D |
| 340 (Reference) | B | B | D |
| 341 (Reference) | D | D | D |
| 342 (Reference) | D | D | D |
| 343 (Reference) | D | D | D |
| 344 (Reference) | D | D | D |
| 345 (Reference) | D | D | D |
| 346 (Reference) | D | D | D |
| 347 (Reference) | C | C | D |
| 348 (Reference) | D | D | D |
| 349 (Reference) | C | C | C |
| 350 (Reference) | B | B | C |
| 351 (Reference) | D | D | D |
| 352 (Reference) | D | D | D |
| 353 (Reference) | B | B | C |
| 354 (Reference) | C | B | C |
| 355 (Reference) | A | A | B |
| 356 | D | D | D |
| 357 (Reference) | D | D | D |
| 358 (Reference) | D | D | D |
| 359 (Reference) | D | D | D |
| 360 | B | B | B |
| 361 (Reference) | C | C | D |
| 362 | D | D | D |
| 363 (Reference) | D | D | D |
| 364 | A | A | B |
| 365 (Reference) | D | D | D |
| 366 (Reference) | C | C | D |
| 367 (Reference) | D | D | D |
| 368 (Reference) | D | D | D |
| 369 (Reference) | B | B | C |
| 370 (Reference) | B | B | D |
| 371 (Reference) | C | C | D |
| 372 (Reference) | A | A | B |
| 373 (Reference) | D | D | D |
| 374 (Reference) | C | C | D |
| 375 (Reference) | D | D | D |
| 376 (Reference) | C | C | C |
| 377 (Reference) | C | D | D |
| 378 (Reference) | B | B | B |
| 379 (Reference) | B | C | C |
| 380 | D | D | ND |
| 381 (Reference) | C | D | D |
| 382 (Reference) | B | B | B |
| 383 (Reference) | C | C | D |
| 384 | D | D | D |
| 385 | B | B | C |
| 386 (Reference) | D | D | D |
| 387 (Reference) | B | A | B |
| 388 (Reference) | D | D | D |
| 389 (Reference) | D | D | D |
| 390 (Reference) | B | B | B |
| 391 | B | B | C |
| 392 (Reference) | B | B | C |
| 393 (Reference) | B | A | B |
| 394 (Reference) | D | D | D |
| 395 (Reference) | D | D | D |
| 396 (Reference) | D | C | D |
| 397 (Reference) | D | D | D |
| 398 (Reference) | B | B | C |
| 399 (Reference) | ND | ND | B |
| 400 (Reference) | C | D | D |
| 402 (Reference) | D | D | D |
| 404 (Reference) | D | D | D |
| 405 (Reference) | D | D | D |
| 407 (Reference) | B | B | B |
| 408 (Reference) | B | B | B |
| 409 (Reference) | A | A | B |
| 410 (Reference) | C | C | D |
| 411 (Reference) | A | A | B |
| 412 (Reference) | B | B | C |
| 413 | B | B | B |
| 414 (Reference) | A | A | B |
| 415 (Reference) | C | B | C |
| 416 (Reference) | A | A | B |
| 417 | B | B | C |
| 418 | A | A | A |
| 419 | A | A | B |
| 420 (Reference) | B | B | C |
| 421 | B | B | C |
| 422 (Reference) | B | B | C |
| 423 (Reference) | B | B | C |
| 424 (Reference) | C | C | C |
| 425 (Reference) | B | B | C |
| 426 (Reference) | D | D | D |
| 427 (Reference) | D | D | D |
| 428 | C | C | C |
| 429 (Reference) | B | B | C |
| 430 (Reference) | D | D | D |
| 431 (Reference) | D | D | D |
| 432 | D | D | D |
| 433 (Reference) | D | C | D |
| 434 (Reference) | D | D | D |
| 435 | C | C | C |
| 436 | C | D | D |
| 437 | B | A | C |
| 438 (Reference) | D | D | D |
| 439 (Reference) | B | B | B |
| 440 (Reference) | D | D | D |
| 441 | B | B | C |
| 442 | A | A | B |
| 443 | D | D | D |
| 444 | A | A | B |
| 445 | B | B | C |
| 446 | A | A | B |
| 447 (Reference) | D | D | D |
| 448 (Reference) | D | D | D |
| 449 | B | B | B |
| 450 | C | D | D |
| 451 (Reference) | B | A | C |
| 452 | D | C | D |

**Table IV**

| **Compound No.** | **EHMT2 PEP (IC50 µM)** | **EHMT1 PEP (IC50 µM)** | **EHMT2 ICW (IC50 µM)** |
|---|---|---|---|
| **453** (Reference) | D | D | D |
| **455** | D | D | D |
| **456** | C | C | D |
| **457** | A | A | A |
| **458** | B | B | C |
| **459** | C | C | D |
| **460** (Reference) | A | A | B |
| **461** | A | A | B |
| **462** (Reference) | A | A | B |
| **463** | A | A | B |
| **464** (Reference) | B | B | C |
| **465** | A | A | B |
| **466** | D | D | D |
| **468** | C | B | C |
| **470** | D | C | D |
| **471** (Reference) | C | C | D |
| **473** (Reference) | C | B | C |
| **475** (Reference) | B | A | B |
| **477** (Reference) | B | B | C |
| **478** (Reference) | D | D | D |
| **480** (Reference) | D | D | D |
| **481** | A | A | A |
| **482** | B | B | C |
| **483** | A | A | B |
| **485** (Reference) | A | A | B |
| **486** (Reference) | D | D | D |
| **487** | C | B | C |
| **488** | B | A | B |
| **489** | C | B | C |
| **490** | A | A | A |
| **491** | B | A | C |
| **492** | B | A | B |
| **494** (Reference) | A | A | A |
| **494a** (Reference) | B | A | B |
| **495** (Reference) | B | A | B |
| **496** | C | B | C |
| **497** | B | B | B |
| **498** | C | B | C |
| **502** | C | B | C |
| **503** | C | C | C |
| **504** | B | B | B |
| **506** | A | A | B |
| **507** (Reference) | A | A | B |
| **508** | C | C | D |
| **509** (Reference) | B | A | B |
| **510** | B | A | B |
| **512** (Reference) | A | A | A |
| **514** (Reference) | B | A | B |
| **515** (Reference) | C | C | C |
| **516** (Reference) | C | C | C |
| **517a** | B | B | B |
| **517b** | B | B | B |
| **518** (Reference) | C | C | C |
| **519** (Reference) | B | C | D |
| **520** (Reference) | A | A | C |
| **521** (Reference) | C | C | C |
| **522** (Reference) | C | C | C |
| **523** (Reference) | C | C | C |
| **524** (Reference) | A | A | A |
| **526** (Reference) | A | A | B |
| **527** (Reference) | A | A | B |
| **528** (Reference) | B | B | C |
| **529** (Reference) | A | A | B |
| **530** (Reference) | A | A | C |
| **532** (Reference) | A | A | B |
| **533** (Reference) | A | A | B |
| **534** (Reference) | C | C | D |
| **535** (Reference) | A | A | A |
| **536** (Reference) | A | A | B |

**Table V**

| **Compound No.** | **EHMT2 PEP (IC50 µM)** | **EHMT1 PEP (IC50 µM)** | **EHMT2 ICW (IC50 µM)** |
|---|---|---|---|
| **538** (Reference) | B | B | D |
| **539** (Reference) | C | D | D |
| **540** (Reference) | ND | ND | ND |
| **541** (Reference) | D | D | D |
| **542** (Reference) | D | D | D |
| **543** (Reference) | D | D | D |
| **544** (Reference) | D | D | D |
| **545** (Reference) | D | D | D |
| **546** (Reference) | D | D | D |
| **547** (Reference) | D | D | D |
| **548** (Reference) | D | D | D |
| **549** (Reference) | D | D | D |
| **550** (Reference) | D | D | D |
| **551** | B | B | B |
| **552** (Reference) | D | D | D |
| **553** (Reference) | D | D | D |
| **554** (Reference) | D | D | D |
| **555** (Reference) | D | D | D |
| **556** (Reference) | D | D | D |
| **557** (Reference) | D | D | D |
| **558** (Reference) | B | A | B |
| **559** | B | A | B |
| **560** | B | B | B |
| **561** | D | D | D |
| **562** | C | C | C |
| **563** | A | A | A |
| **564** (Reference) | A | A | D |
| **565** (Reference) | C | C | C |
| **566** (Reference) | C | C | D |
| **567** (Reference) | C | C | D |
| **568** | B | A | B |
| **569** (Reference) | B | A | B |
| **570** | C | C | C |
| **571** | A | A | B |
| **572** (Reference) | D | D | D |
| **573** (Reference) | C | C | D |
| **574** | C | C | D |
| **575** | C | B | C |
| **576** | B | A | B |
| **577** | C | B | D |
| **578** (Reference) | C | C | D |
| **579** (Reference) | C | C | D |
| **580** | D | D | D |
| **581** (Reference) | C | D | D |
| **582** (Reference) | D | D | D |
| **583** (Reference) | C | C | D |
| **584** (Reference) | D | D | D |
| **585** (Reference) | C | C | C |
| **586** (Reference) | C | C | D |
| **587** (Reference) | A | A | B |
| **588** (Reference) | D | D | D |
| **589** (Reference) | D | D | D |
| **590** (Reference) | A | A | B |
| **591** (Reference) | A | A | B |
| **592** (Reference) | B | B | B |
| **593** (Reference) | B | B | B |
| **594** (Reference) | B | A | B |
| **595** (Reference) | B | B | B |
| **596** (Reference) | C | C | D |
| **597** (Reference) | D | D | D |
| **598** (Reference) | D | D | D |
| **599** (Reference) | B | A | B |
| **600** | A | A | A |
| **601** (Reference) | B | A | B |
| **602** (Reference) | B | B | B |
| **603** (Reference) | A | A | B |

**Table VI**

| **Compound No.** | **EHMT2 PEP IC50 (µM)** | **EHMT1 PEP IC50 (µM)** | **EHMT2 ICW IC50 (µM)** |
|---|---|---|---|
| 604 (Reference) | B | B | D |
| 605 (Reference) | D | D | D |
| 607 (Reference) | B | A | C |
| 609 (Reference) | B | B | C |
| 610 | D | D | C |
| 611 (Reference) | C | C | C |
| 613 (Reference) | B | A | C |
| 616 (Reference) | C | B | C |
| 618 (Reference) | C | B | D |
| 619 (Reference) | D | D | D |
| 620 (Reference) | D | D | D |
| 621 (Reference) | D | C | D |
| 622 (Reference) | C | C | D |
| 623 (Reference) | C | C | C |
| 624 | B | A | C |
| 625 (Reference) | A | A | B |
| 628 (Reference) | D | D | D |
| 629 (Reference) | B | A | D |
| 630 (Reference) | D | D | D |
| 631 (Reference) | D | D | D |
| 632 (Reference) | D | D | D |
| 633 (Reference) | D | D | D |
| 634 (Reference) | B | B | D |
| 635 (Reference) | C | C | D |
| 636 (Reference) | D | D | D |
| 637 (Reference) | D | D | D |
| 638 (Reference) | D | D | D |
| 640 (Reference) | D | D | D |
| 641 (Reference) | A | A | B |
| 642 (Reference) | B | A | B |
| 643 (Reference) | D | D | D |
| 644 | A | A | B |
| 645 | D | C | C |
| 646 | C | C | C |
| 647 | D | C | D |
| 648 (Reference) | C | C | D |
| 649 | B | B | B |
| 650 | D | D | D |
| 651 | D | C | D |
| 652 (Reference) | B | A | C |
| 654 (Reference) | D | D | D |
| 655 (Reference) | D | D | D |
| 656 (Reference) | D | D | D |
| 658 (Reference) | B | B | D |
| 659 (Reference) | B | B | D |
| 660 (Reference) | A | A | C |
| 661 (Reference) | A | A | C |
| 662 (Reference) | A | A | B |
| 663 (Reference) | A | A | B |
| 664 (Reference) | C | C | D |
| 665 (Reference) | C | C | D |
| 667 (Reference) | B | A | B |
| 668 (Reference) | B | B | B |
| 670 (Reference) | B | B | D |
| 671 | B | A | B |
| 672 | A | A | B |
| 673 (Reference) | B | B | D |
| 674 (Reference) | C | C | D |
| 676 (Reference) | D | D | D |
| 677 (Reference) | A | A | D |
| 678 (Reference) | B | B | C |
| 679 (Reference) | C | C | C |
| 680 | C | C | D |
| 681 (Reference) | C | B | C |
| 682 (Reference) | A | A | B |
| 686 (Reference) | D | D | D |
| 687 | C | C | C |
| 736 (Reference) | A | A | A |
| 737 (Reference) | C | C | D |
| 738 (Reference) | B | B | C |
| 739 (Reference) | C | D | D |
| 740 (Reference) | A | A | C |
| 741 (Reference) | B | B | C |
| 742 (Reference) | A | A | C |
| 743 (Reference) | C | C | D |
| 745 (Reference) | B | B | D |
| 746 (Reference) | B | B | D |
| 747 (Reference) | B | B | C |
| 748 (Reference) | B | B | D |
| 753 (Reference) | A | A | C |
| 754 (Reference) | C | C | D |
| 755 (Reference) | A | A | B |
| 756 (Reference) | A | A | B |
| 757 (Reference) | A | A | A |
| 758 (Reference) | A | A | A |
| 759 (Reference) | A | A | C |
| 760 (Reference) | C | C | D |
| 761 (Reference) | C | C | C |
| 762 (Reference) | D | D | D |
| 763 (Reference) | D | D | C |
| 784 (Reference) | C | C | D |
| 786 | A | A | B |
| 787 (Reference) | A | A | B |
| 788 (Reference) | B | A | B |
| 789 (Reference) | A | A | B |
| 790 (Reference) | B | A | C |
| 791 | C | B | C |
| 793 | D | D | D |
| 794 (Reference) | D | D | D |
| 795 (Reference) | D | D | D |
| 796 (Reference) | D | D | D |
| 797 (Reference) | D | D | D |
| 798 (Reference) | B | A | B |
| 800 | A | A | B |
| 801 (Reference) | D | D | D |
| 802 (Reference) | A | A | C |
| 803 (Reference) | B | A | C |
| 806 (Reference) | C | C | D |
| 808 (Reference) | D | C | D |
| 809 (Reference) | A | A | B |
| 810 (Reference) | B | A | C |
| 811 (Reference) | D | D | D |
| 812 (Reference) | A | A | B |
| 813 | C | B | D |
| 814 | C | C | D |
| 816 (Reference) | A | A | B |
| 817 (Reference) | A | A | B |
| 822 (Reference) | D | D | D |
| 823 (Reference) | A | A | B |
| 824 (Reference) | B | B | C |
| 825 (Reference) | B | B | C |
| 832 (Reference) | A | A | A |
| 833 (Reference) | A | A | B |
| 834 (Reference) | B | B | C |
| 836 (Reference) | B | B | C |
| 837 (Reference) | B | B | C |
| 838 (Reference) | A | A | B |
| 839 (Reference) | A | A | A |
| 841 (Reference) | D | D | D |
| 844 (Reference) | B | B | C |
| 845 | B | A | B |
| 846 | A | A | B |
| 847 | B | A | C |
| 848 | A | A | B |
| 854 (Reference) | D | D | D |
| 855 | A | A | B |
| 859 (Reference) | A | A | B |
| 860 | D | D | D |
| 863 (Reference) | A | A | B |
| 864 (Reference) | A | A | B |
| 865 (Reference) | A | A | B |
| 866 (Reference) | A | A | A |
| 867 (Reference) | A | A | A |
| 870 (Reference) | D | D | D |
| 871 | D | D | D |
| 873 | D | D | D |
| 876 | D | D | D |
| 877 | D | D | D |
| 881 (Reference) | B | B | B |
| 882 (Reference) | B | B | C |
| 883 (Reference) | D | D | D |
| 884 (Reference) | D | D | D |
| 885 (Reference) | C | C | D |
| 886 (Reference) | C | C | D |
| 887 (Reference) | B | B | C |
| 888 (Reference) | B | B | D |
| 890 | D | D | D |
| 891 | D | D | D |
| 892 (Reference) | A | A | C |
| 893 (Reference) | B | A | B |
| 894 (Reference) | B | B | D |
| 895 (Reference) | D | D | D |
| 896 (Reference) | D | D | D |
| 897 (Reference) | D | C | D |
| 900 (Reference) | D | D | B |
| 902 (Reference) | A | A | B |
| 903 (Reference) | C | B | C |
| 904 | D | C | D |
| 905 | B | B | B |
| 906 | A | A | B |
| 907 (Reference) | C | B | C |
| 908 (Reference) | A | A | B |
| 911 (Reference) | B | A | B |
| 912 (Reference) | B | A | B |
| 914 (Reference) | B | A | B |
| 915 (Reference) | A | A | A |
| 916 (Reference) | B | A | C |
| 917 (Reference) | C | B | C |
| 918 (Reference) | C | B | C |
| 919 (Reference) | C | C | D |
| 920 (Reference) | B | B | B |
| 921 (Reference) | D | D | D |
| 922 (Reference) | C | C | D |
| 927 (Reference) | B | A | B |
| 928 (Reference) | B | A | A |
| 931 (Reference) | D | D | D |
| 932 (Reference) | D | C | D |
| 933 (Reference) | D | C | D |
| 936 (Reference) | D | D | D |
| 937 (Reference) | D | D | D |
| 938 (Reference) | D | D | D |
| 943 (Reference) | C | C | C |
| 945 (Reference) | B | A | B |
| 947 (Reference) | A | A | C |
| 949 (Reference) | B | A | B |
| 950 (Reference) | B | B | C |
| 951 (Reference) | B | A | C |
| 961 (Reference) | C | C | D |
| 962 (Reference) | B | B | C |
| 963 (Reference) | A | A | B |
| 964 (Reference) | B | A | C |
| 965 | A | A | A |
| 974 (Reference) | B | A | B |
| 985 | B | A | B |
| 986 | B | B | C |
| 990 | A | A | B |
| 1005 (Reference) | C | C | C |
| 1006 (Reference) | B | A | B |
| 1007 (Reference) | D | C | D |
| 1008 (Reference) | B | A | B |
| 1009 (Reference) | C | C | C |
| 1011 (Reference) | C | C | C |
| 1014 (Reference) | C | B | C |
| 1016 (Reference) | C | B | C |
| 1019 (Reference) | C | B | D |
| 1020 (Reference) | C | C | D |
| 1021 (Reference) | C | B | C |
| 1022 (Reference) | C | B | D |
| 1028 (Reference) | A | A | B |
| 1030 | B | A | B |
| 1031 (Reference) | B | A | B |
| 1032 (Reference) | A | A | B |
| 1033 (Reference) | A | A | B |
| 1034 (Reference) | B | B | C |
| 1035 (Reference) | B | B | C |
| 1036 | B | B | C |
| 1037 | B | B | B |
| 1038 | A | A | A |
| 1040 | B | B | A |
| 1041 (Reference) | B | B | D |
| 1042 (Reference) | C | C | D |

**Table VII**

| **Compound No.** | **EHMT2 PEP IC50 (µM)** | **EHMT1 PEP IC50 (µM)** | **EHMT2 ICW IC50 (µM)** |
|---|---|---|---|
| 1043 (Reference) | C | B | C |
| 1044 | B | B | B |
| 1045 | A | A | B |
| 1046 (Reference) | C | C | C |
| 1047 (Reference) | A | A | B |
| 1048 (Reference) | B | A | B |
| 1049 (Reference) | B | A | B |
| 1050 (Reference) | B | A | B |
| 1051 (Reference) | A | A | A |
| 1052 (Reference) | B | B | C |
| 1053 (Reference) | C | B | C |
| 1054 (Reference) | C | B | C |
| 1055 (Reference) | B | A | B |
| 1056 (Reference) | B | A | A |
| 1057 (Reference) | B | B | B |
| 1058 (Reference) | D | D | D |
| 1059 (Reference) | C | C | C |
| 1060 (Reference) | C | B | C |
| 1061 (Reference) | C | B | D |
| 1062 (Reference) | C | C | D |
| 1063 (Reference) | B | B | B |
| 1064 (Reference) | A | A | B |
| 1065 (Reference) | B | A | B |
| 1066 (Reference) | C | C | D |
| 1067 (Reference) | D | D | D |
| 1068 (Reference) | D | D | D |
| 1069 (Reference) | D | C | D |
| 1070 (Reference) | D | D | D |
| 1071 (Reference) | D | D | D |
| 1072 (Reference) | D | D | D |
| 1073 (Reference) | C | B | C |
| 1074 (Reference) | C | B | D |
| 1075 (Reference) | A | A | B |
| 1076 (Reference) | B | B | C |
| 1077 (Reference) | B | B | C |
| 1078 | A | A | A |
| 1079 | B | B | C |
| 1080 | B | A | C |
| 1081 | A | A | A |
| 1082 (Reference) | C | C | D |
| 1083 (Reference) | B | B | B |
| 1084 (Reference) | D | D | D |
| 1085 (Reference) | B | A | B |
| 1086 (Reference) | C | C | C |
| 1087 (Reference) | C | B | C |
| 1088 (Reference) | A | A | B |
| 1089 (Reference) | B | A | B |
| 1090 | B | B | B |
| 1091 (Reference) | B | A | B |
| 1092 (Reference) | C | C | C |
| 1093 (Reference) | B | A | B |
| 1094 | B | A | B |
| 1095 | B | B | C |
| 1096 (Reference) | D | D | D |
| 1097 (Reference) | D | C | D |
| 1098 (Reference) | A | A | B |
| 1099 | B | A | B |
| 1100 | B | B | C |
| 1101 | A | A | B |
| 1102 | A | A | B |
| 1103 | C | C | C |
| 1104 | A | A | B |
| 1105 | B | B | C |
| 1106 | A | A | B |
| 1107 (Reference) | A | A | B |
| 1108 (Reference) | A | A | B |
| 1109 | C | B | C |
| 1110 (Reference) | B | A | B |
| 1111 (Reference) | A | A | B |
| 1112 | B | A | C |
| 1113 | B | A | C |
| 1114 | D | D | D |
| 1115 (Reference) | C | C | ND |
| 1116 (Reference) | C | D | ND |
| 1117 | C | D | ND |
| 1118 (Reference) | C | D | ND |
| 1119 (Reference) | C | C | ND |

### Example 157: Bioactivity Assays

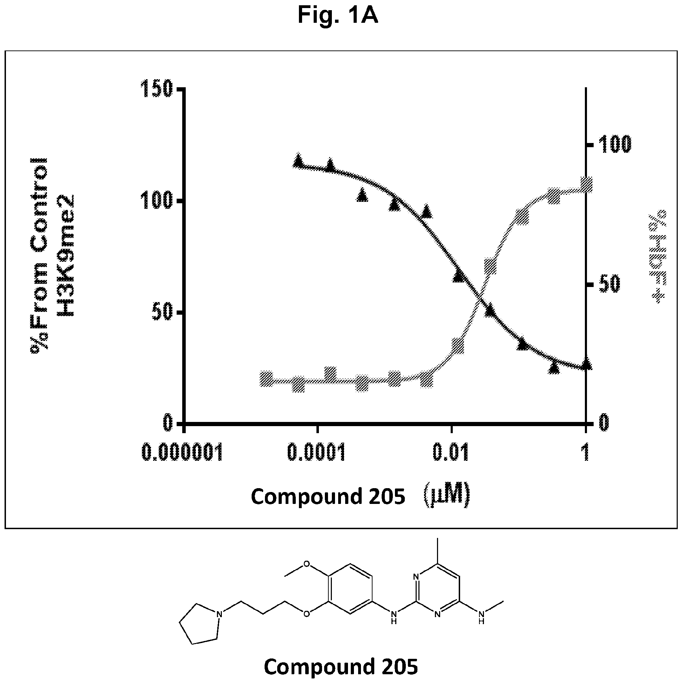

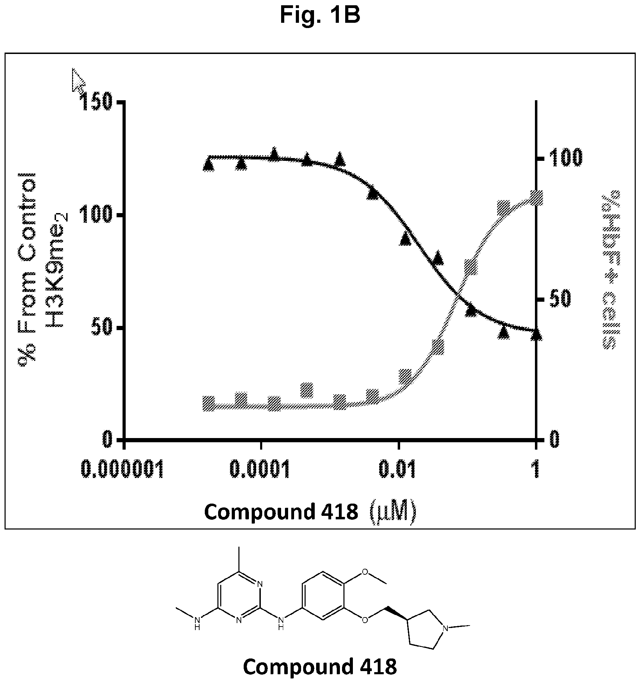

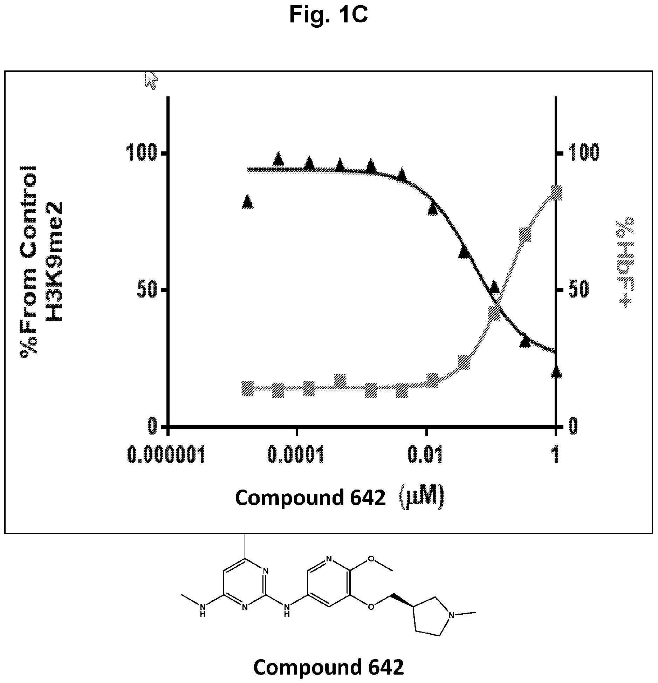

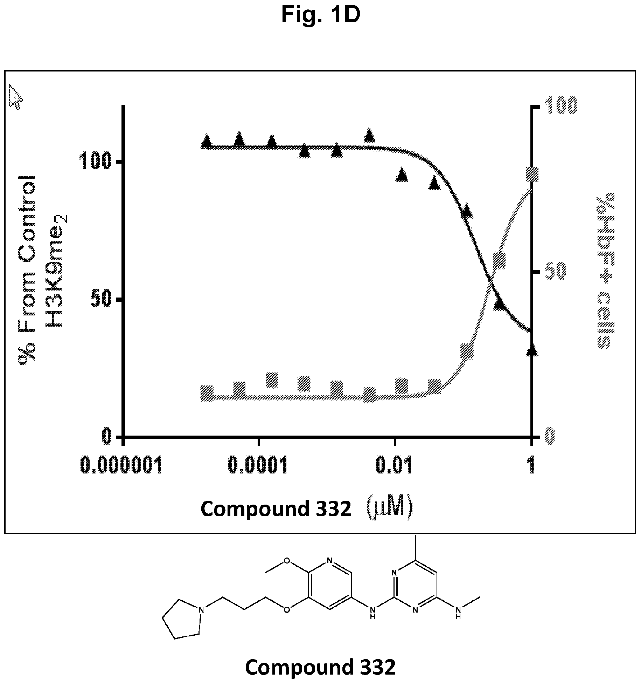

The following procedure and Figures 1A-1D, 2, and 3 describe the induction of fetal hemoglobin following treatment of cells with EHMT2 inhibitors defined herein.

### Cell Culture

Peripheral Blood Mononuclear Cells (PBMCs) where isolated from whole blood of healthy donors by Ficoll gradients. CD34+ cells were then magnetically isolated from the PBMC fraction. Cells were differentiated *in vitro* toward the erythroid lineage for 14 days using the first two weeks of the 3-phase culture method described by Giarratana, et al (Blood 2011). After isolation, cells were seeded at a density of 1×10⁵ cells/mL in phase 1 media. At day 7, cells were split in a ratio of 1:5 into Phase 2 media.

### Drug Treatment

Compounds were dissolved in Dimethyl sulfoxide (DMSO), and 1 µM stocks were prepared and diluted in a 1:3 series to generate an 11-point dose curve. 100 % DMSO served as control. Compound dilutions were added to cells on day 1 as 1:1000 dilutions. DMSO was equally added to control cells for a final concentration of 0.001%. Compound dilutions and DMSO were re-added on day 7, after the cell split described above.

### Flow cytometry

At day 14, around 10⁶ cells were fixed, permeabilized, and stained for cell surface markers CD235a, CD71, Human Fetal Hemoglobin, Human Histone 3, and Dimethyl-Lysine 9 Histone 3.

### RT-qPCR

At day 14, around 10⁶ cells were pelleted. RNA was isolated by traditional spin column methods and gene expression analysis carried out by 2-step RT-qPCR. Standard curves were generated using plasmids encoding each of Human Globin HBA, HBB and HBG and were used to calculate individual globin copy numbers. HBB and HBG were added to calculate the total β-Locus Globins copies. Reported results represent % HBG/Total mRNA copies.

### Mass Spectrometry

At day 14, around 10⁶ cells were pelleted. Protein was isolated, digested, and quantified by LC-PRM mass spectrometry analysis. Globin-specific label peptides were used for quantification of individual globins. HBB and HBG were added to calculate the total β-Locus Globin protein levels. Reported results represent % HBG/Total protein.

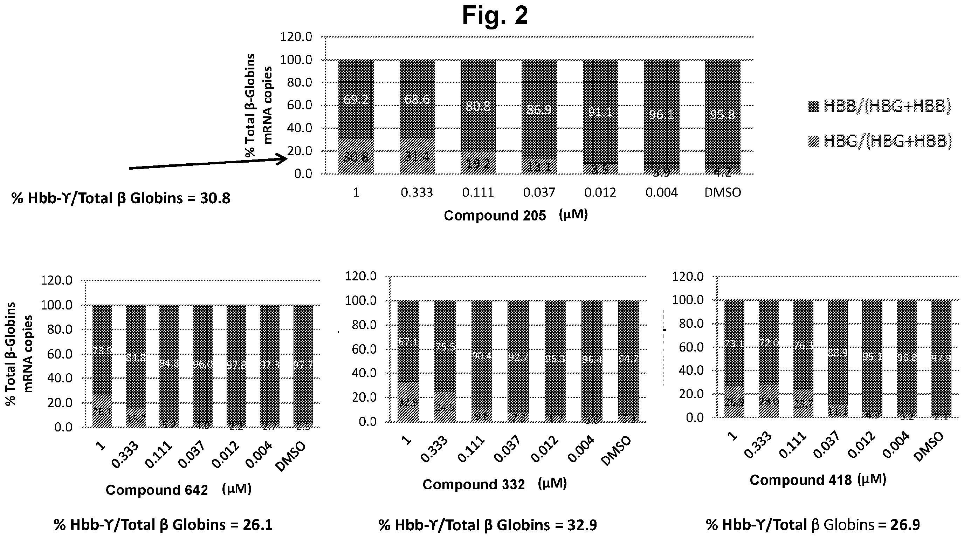

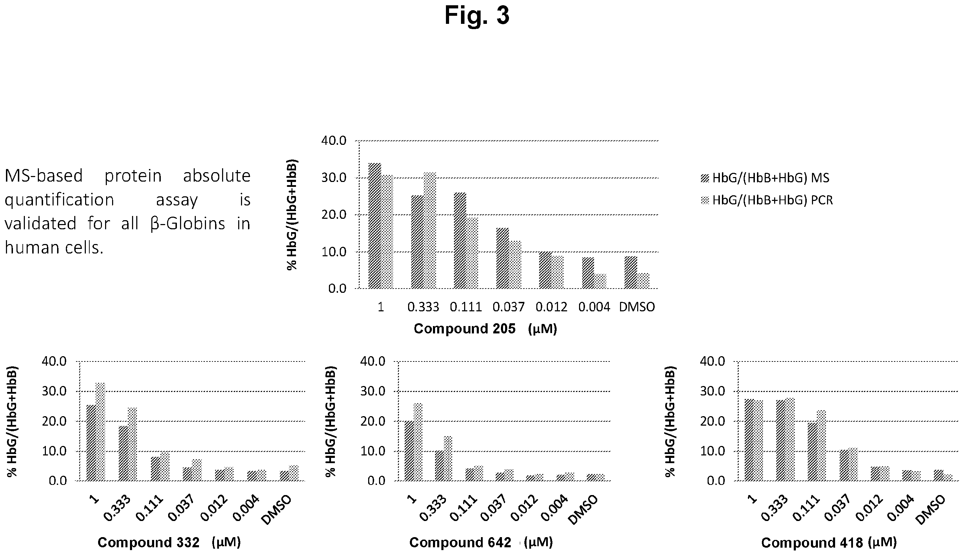

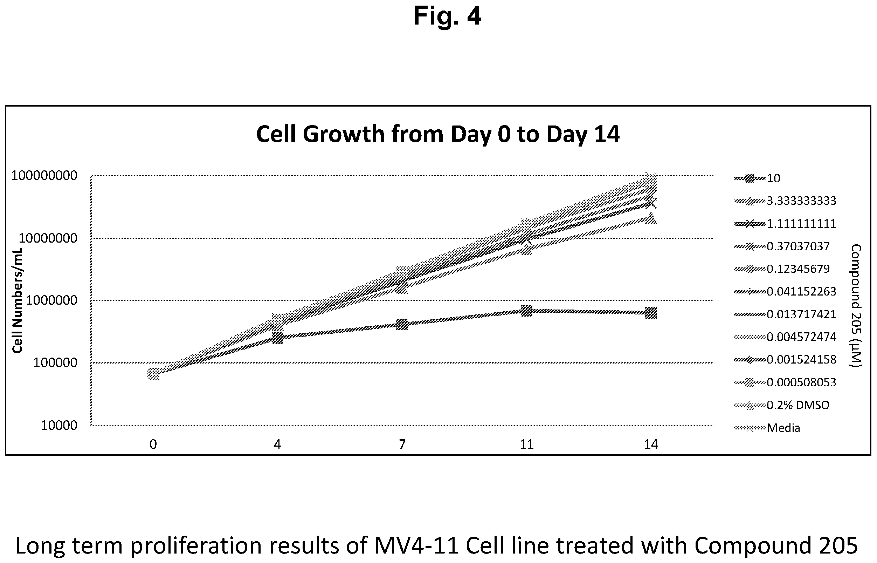

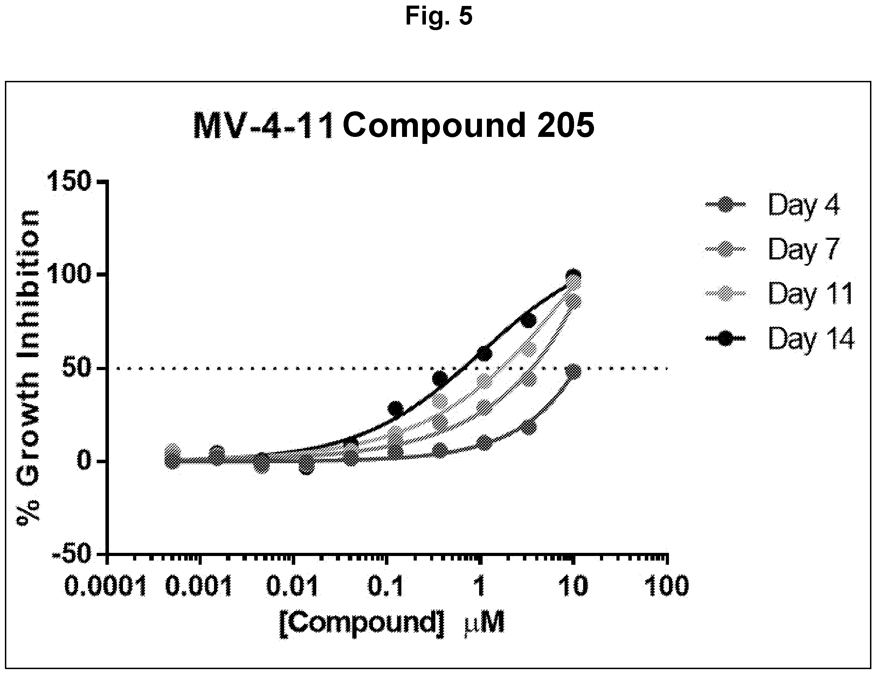

There was good correlation between in-cell Western (ICW) and fluorescence-activated cell sorting (FACs) data for K9 lysine dimethylation, and between fluorescence-activated cell sorting data for K9 lysine dimethylation and percent of cells containing fetal hemoglobin (HbF+ cells), as shown in Figures 1A-1D. As shown in Figures 2 and 3, all tested compounds showed around 30% Hbb-γ per total β-globins at the mRNA and protein level, with a 1:1 correlation between protein and mRNA data. A good correlation between potency, target engagement, and induction of HbF+ cells was observed. More potent compounds were shown to have a more sustained induction of Hbb-γ, which also correlated with the sustained induction of HbF+ cells as observed by FACs analysis. The data suggests that sickle-cell disease (SCD)-relevant levels of around 30% HbF/ total β-globins might be achievable for all tested EHMT2 inhibitors. The following procedure and Figures 4 and 5 describe the inhibition of MV4-11 human acute monocytic leukemia cells following treatment with an EHMT2 inhibitor defined herein.

### MATERIALS AND EQUIPMENT:

MV-4-11 leukemia cells were purchased from ATCC. IMDM, FBS, and Calcein-AM were purchased from Invitrogen. Flat 96-well plates were purchased from Corning, and Poly-D-Lysine 96-well Microplates, black/clear were purchased from BD BIOCOAT.

A 3-fold serial dilution of Compound 205 ("3*compounds") was prepared as follows: Compound 205 was dissolved in DMSO to give a 10 mM solution and stored at -20 °C. A 3-fold serial dilution of Compound 205 in DMSO to give solutions ranging in concentration from 5 mM to 0.25 µM.

The 3*compounds solutions were added to the cell plate via the following procedure: 1.2 µl of the compound solutions were transferred to a 96-well plate with 200 µl of media in each well, then mixed well by pipetting up and down to give 3*compounds in media. 50 µl of 3*compounds in media were then transferred to the cell plate.

### Day 0

In a flat bottom 96-well plate, 100µL of cells were added per well at a density of 1×10⁵ cells/mL. (Note: Only internal wells were used. PBS was placed in all outer wells to avoid evaporation of the internal wells.) 50 µL of 3*compounds were added to each well, to give a final volume of 150 µL per well.

### Days 1-3

The plates were incubated for 96 hours.

### Day 4

Cells were pipetted up and down to mix in each well. 20 µL of the cell suspension was aspirated from each well and added to a V-bottom plate. 80 µL of HBSS was added to each well of the V-bottom plate and mixed.

Next, 50 µL of cell suspension in the V-bottom plate was aspirated and added to a poly-D-lysine coated 96-well plate. To this was added 50 µL of HBSS containing 2 µM Calcein-AM, to give a final concentration of 1 µM. The cells were allowed to sit at room temperature for 10 minutes, then centrifuged to settle the cells on the bottom of the wells.

The plate was then incubated for an additional 40 minutes in the incubator to load Calcein AM and to give cells more time to attach.

The plate was removed and read on an Acumen plate reader, and cell numbers were calculated, taking into account the dilution factors. The master plate was split by taking the total viable cell count calculated, and cells were pipetted up and down in each well in order to mix. Then, cell suspension was aspirated from each well and added to a V-bottom plate.

The plate was centrifuged at 1100 rpm for 5 minutes, then the media was removed, being careful not to disturb the cell pellet.

The pellet was then resuspended in 200 µL fresh media. The cells were mixed in each well by pipetting up and down, then 100 µL of cell suspension was aspirated from each well and added to a new 96-well flat bottom plate, and 50 µL of 3*compounds solution was added.

### Days 4-6

The plates were incubated for 72 hours.

### Day 7

Cells were pipetted up and down to mix in each well. 20 µL of the cell suspension was aspirated from each well and added to a V-bottom plate. 80 µL of HBSS was added to each well of the V-bottom plate and mixed.

Next, 40 µL of cell suspension in the V-bottom plate was aspirated and added to a poly-D-lysine coated 96-well plate. To this was added 40 µL of HBSS containing 2 µM Calcein-AM, to give a final concentration of 1 µM. The cells were allowed to sit at room temperature for 10 minutes, then centrifuged to settle the cells on the bottom of the wells.

The plate was then incubated for an additional 40 minutes in the incubator to load Calcein AM and to give cells more time to attach.

The plate was removed and read on an Acumen plate reader, and cell numbers were calculated, taking into account the dilution factors. The master plate was split by taking the total viable cell count calculated, and cells were pipetted up and down in each well in order to mix. Then, 1.2* of the calculated cell suspension was aspirated from each well and added to a V-bottom plate.

The plate was centrifuged at 1100 rpm for 5 minutes, then the media was removed, being careful not to disturb the cell pellet.
The pellet was then resuspended in 120 µL fresh media. The cells were mixed in each well by pipetting up and down, then 100 µL of cell suspension was aspirated from each well and added to a new 96-well flat bottom plate, and 50 µL of 3*compounds solution was added.

### Days 7-10

The plates were incubated for 96 hours.

### Day 11

Cells were pipetted up and down to mix in each well. 20 µL of the cell suspension was aspirated from each well and added to a V-bottom plate. 80 µL of HBSS was added to each well of the V-bottom plate and mixed.

Next, 50 µL of cell suspension in the V-bottom plate was aspirated and added to a poly-D-lysine coated 96-well plate. To this was added 50 µL of HBSS containing 2 µM Calcein-AM, to give a final concentration of 1 µM. The cells were allowed to sit at room temperature for 10 minutes, then centrifuged to settle the cells on the bottom of the wells.

The plate was then incubated for an additional 40 minutes in the incubator to load Calcein AM and to give cells more time to attach.

The plate was removed and read on an Acumen plate reader, and cell numbers were calculated, taking into account the dilution factors. The master plate was split by taking the total viable cell count calculated, and cells were pipetted up and down in each well in order to mix and reduce variation caused by pipetting. Then, 1.2* of the calculated cell suspension was aspirated from each well and added to a V-bottom plate.

The plate was centrifuged at 1100 rpm for 5 minutes, then the media was removed, being careful not to disturb the cell pellet.

The pellet was then resuspended in 120 µL fresh media. The cells were mixed in each well by pipetting up and down, then 100 µL of cell suspension was aspirated from each well and added to a new 96-well flat bottom plate, and 50 µL of 3*compounds solution was added.

### Days 11-13

The plates were incubated for 72 hours.

### Day 14

Cells were pipetted up and down to mix in each well. 20 µL of the cell suspension was aspirated from each well and added to a V-bottom plate. 80 µL of HBSS was added to each well of the V-bottom plate and mixed.

Next, 40 µL of cell suspension in the V-bottom plate was aspirated and added to a poly-D-lysine coated 96-well plate. To this was added 40 µL of HBSS containing 2 µM Calcein-AM, to give a final concentration of 1 µM. The cells were allowed to sit at room temperature for 10 minutes, then centrifuged to settle the cells on the bottom of the wells.

The plate was then incubated for an additional 40 minutes in the incubator to load Calcein AM and to give cells more time to attach.

The plate was removed and read on an Acumen plate reader, and cell numbers were calculated, taking into account the dilution factors.

Growth was calculated for days 4, 7, 11, and 14 as follows: the split factor was calculated for day 4 to 7, day 7 to 11, and day 11-14 The split factor is the number of viable cells/mL on Day X (either 4, 7, or 11) divided by the density the cells are being split back to.

For growth of cells from day 4 to 7, the day 7 viable cells/mL density was multiplied by the split factor from day 4.

For growth of cells from day 7 to 11, the day 11 viable cells/mL density was multiplied by the day 4 and day 7 split factors.

For growth of cells from day 11 to 14, the day 14 viable cells/mL density was multiplied by the day 4, day 7, and day 11 split factors.

Growth was plotted on semi-log chart (viable cells/mL on Y axis, in log, and days on X axis).

The invention can be embodied in other specific forms without departing from the essential characteristics thereof. The foregoing embodiments are therefore to be considered in all respects illustrative rather than limiting on the invention described herein. Scope of the invention is thus indicated by the appended claims rather than by the foregoing description.

## Claims

1. A compound of Formula (IIa1) for use in treating or preventing a blood disorder: 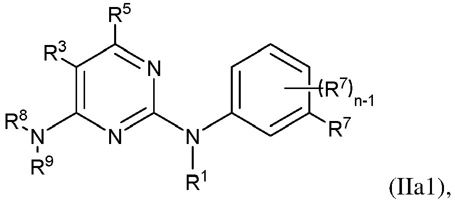 or a tautomer thereof, or a pharmaceutically acceptable salt of the compound or the tautomer, wherein
R¹ is H or C₁-C₄ alkyl;
R³ is selected from the group consisting of H, halo, cyano, C₁-C₆ alkoxyl, C₆-C₁₀ aryl, NR^{a}C(O)R^{b}, C₃-C₈ cycloalkyl, 4- to 7- membered heterocycloalkyl, 5- to 6-membered heteroaryl, and C₁-C₆ alkyl, wherein C₁-C₆ alkoxyl and C₁-C₆ alkyl are optionally substituted with one or more of halo, OR^{a}, or NR^{a}R^{b}, in which each of R^{a} and R^{b} independently is H or C₁-C₆ alkyl, or R³ is -Q¹-T¹, in which Q¹ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T¹ is H, halo, cyano, NR⁸R⁹, C(O)NR⁸R⁹, OR⁸, OR⁹, or R^{S1}, in which R^{S1} is C₃-C₈ cycloalkyl, phenyl, 4- to 12-membered heterocycloalkyl containing 1-4 heteroatoms selected from N, O, and S, or a 5- or 6-membered heteroaryl and R^{S1} is optionally substituted with one or more of halo, C₁-C₆ alkyl, hydroxyl, oxo, -C(O)R⁹, -SO₂R⁸, -SO₂N(R⁸)₂, -NR⁸C(O)R⁹, amino, mono- or di- alkylamino, or C₁-C₆ alkoxyl;
R⁵ is selected from the group consisting of F, Br, cyano, C₁-C₆ alkoxyl, C₆-C₁₀ aryl, NR^{a}R^{b}, C(O)NR^{a}R^{b}, NR^{a}C(O)R^{b}, C₃-C₈ cycloalkyl, 4- to 12-membered heterocycloalkyl containing 1-4 heteroatoms selected from N, O, and S, C₁-C₆ alkyl optionally substituted with one or more of halo, OR^{a} or NR^{a}R^{b}, and C₂-C₆ alkynyl optionally substituted with 4- to 12-membered heterocycloalkyl; wherein said C₃-C₈ cycloalkyl or 4- to 12-membered heterocycloalkyl are optionally substituted with one or more of halo, C(O)R^{a}, OR^{a}, NR^{a}R^{b}, 4-to 7-membered heterocycloalkyl, -C₁-C₆ alkylene-4- to 7-membered heterocycloalkyl, or C₁-C₄ alkyl optionally substituted with one or more of halo, OR^{a} or NR^{a}R^{b}, in which each of R^{a} and R^{b} independently is H or C₁-C₆ alkyl; or
R⁵ and R³ together with the atoms to which they are attached form phenyl or a 5- or 6-membered heteroaryl;
each R⁷ is independently oxo (=O) or -Q²-T², in which each Q² independently is a bond or C₁-C₆ alkylene, C₂-C₆ alkenylene, or C₂-C₆ alkynylene linker optionally substituted with one or more of halo, cyano, hydroxyl, amino, mono- or di- alkylamino, or C₁-C₆ alkoxyl, and each T² independently is H, halo, cyano, OR¹⁰, OR¹¹, C(O)R¹¹, NR¹⁰R¹¹, C(O)NR¹⁰R¹¹, NR¹⁰C(O)R¹¹, 5- to 10-membered heteroaryl, C₃-C₈ cycloalkyl, or 4- to 12-membered heterocycloalkyl containing 1-4 heteroatoms selected from N, O, and S, and wherein the 5- to 10-membered heteroaryl, C₃-C₈ cycloalkyl or 4- to 12-membered heterocycloalkyl is optionally substituted with one or more of halo, C₁-C₆ alkyl optionally substituted with NR^{x}R^{y}, hydroxyl, oxo, N(R⁸)₂, cyano, C₁-C₆ haloalkyl, -SO₂R⁸, or C₁-C₆ alkoxyl, each of R^{x} and R^{y} independently being H or C₁-C₆ alkyl; and R⁷ is not H or C(O)OR^{g}; or optionally, one R⁷ and R⁵ together form a C₃-C₁₀ alkylene, C₂-C₁₀ heteroalkylene, C₄-C₁₀ alkenylene, C₂-C₁₀ heteroalkenylene, C₄-C₁₀ alkynylene or C₂-C₁₀ heteroalkynylene linker optionally substituted with one or more of halo, cyano, hydroxyl, or C₁-C₆ alkoxyl;
each R⁸ independently is H or C₁-C₆ alkyl;
each R⁹ is independently -Q³-T³, in which Q³ is a bond or C₁-C₆ alkylene, and T³ is H;
each R¹⁰ is selected from the group consisting of H and C₁-C₆ alkyl;
each R¹¹ is -Q⁶-T⁶, in which Q⁶ is a bond or C₁-C₆ alkylene, C₂-C₆ alkenylene, or C₂-C₆ alkynylene linker optionally substituted with one or more of halo, cyano, hydroxyl, oxo, or C₁-C₆ alkoxyl, and T⁶ is H, halo, OR^{g}, NR^{g}R^{h}, NR^{g}C(O)R^{h}, C(O)NR^{g}R^{h}, C(O)R^{g}, S(O)₂R^{g}, or R^{S3}, in which each of R^{g} and R^{h} independently is H, phenyl, C₃-C₈ cycloalkyl, or C₁-C₆ alkyl optionally substituted with C₃-C₈ cycloalkyl, or R^{g} and R^{h} together with the nitrogen atom to which they are attached form a 4- to 12-membered heterocycloalkyl containing 1-4 heteroatoms selected from N, O, and S, and R^{S3} is C₃-C₈ cycloalkyl, C₆-C₁₀ aryl, 4- to 12-membered heterocycloalkyl containing 1-4 heteroatoms selected from N, O and S, or a 5- to 10-membered heteroaryl, and R^{S3} is optionally substituted with one or more -Q⁷-T⁷, wherein each Q⁷ independently is a bond or C₁-C₃ alkylene, C₂-C₃ alkenylene, or C₂-C₃ alkynylene linker each optionally substituted with one or more of halo, cyano, hydroxyl, or C₁-C₆ alkoxy, and each T⁷ independently is selected from the group consisting of H, halo, cyano, C₁-C₆ alkyl, C₃-C₈ cycloalkyl, C₆-C₁₀ aryl, 4- to 7-membered heterocycloalkyl containing 1-4 heteroatoms selected from N, O, and S, 5- to 6-membered heteroaryl, OR^{j}, C(O)R^{j}, NR^{j}R^{k}, C(O)NR^{j}R^{k}, S(O)₂R^{j}, and NRⁱC(O)R^{k}, each of R^{j} and R^{k} independently being H or C₁-C₆ alkyl optionally substituted with one or more halo; or -Q⁷-T⁷ is oxo; or
R¹⁰ and R¹¹ taken together with the nitrogen atom to which they are attached form a 4-to 12-membered heterocycloalkyl containing 1-4 heteroatoms selected from N, O, and S, which is optionally substituted with one or more of halo, C₁-C₆ alkyl, hydroxyl, or C₁-C₆ alkoxyl;
n is 0, 1, 2, 3, or 4.

2. A compound for use according to claim 1, wherein R³ is not H.

3. A compound for use according to any one of the preceding claims, wherein n is 1 or 2; and/or
wherein R¹ is H or CH₃; and/or
wherein n is 1 or 2, and at least one of R⁷ is -Q²-OR¹¹ in which R¹¹ is -Q⁶-R^{S3} and Q⁶ is optionally substituted C₂-C₆ alkylene, C₂-C₆ alkenylene, or C₂-C₆ alkynylene linker; and/or
wherein n is 1 or 2, and at least one of R⁷ is -Q²-NR¹⁰R¹¹ in which R¹¹ is -Q⁶-R^{S3}; and/or
wherein Q⁶ is C₂-C₆ alkylene, C₂-C₆ alkenylene, or C₂-C₆ alkynylene linker optionally substituted with a hydroxyl and R^{S3} is 4- to 7-membered heterocycloalkyl optionally substituted with one or more -Q⁷-T⁷; and/or
wherein Q⁶ is C₁-C₆ alkylene, C₂-C₆ alkenylene, or C₂-C₆ alkynylene linker optionally substituted with a hydroxyl and R^{S3} is C₃-C₆ cycloalkyl optionally substituted with one or more -Q⁷-T⁷; and/or
wherein each Q⁷ is independently a bond or a C₁-C₃ alkylene, C₂-C₃ alkenylene, or C₂-C₃ alkynylene linker and each T⁷ is independently H, halo, C₁-C₆ alkyl, or phenyl; and/or
wherein Q² is a bond or a C₁-C₄ alkylene, C₂-C₄ alkenylene, or C₂-C₄ alkynylene linker; and/or
wherein at least one of R⁷ is

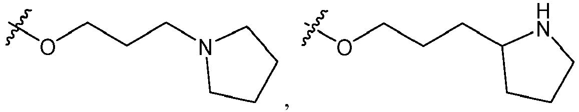

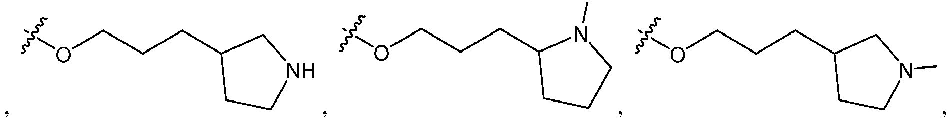

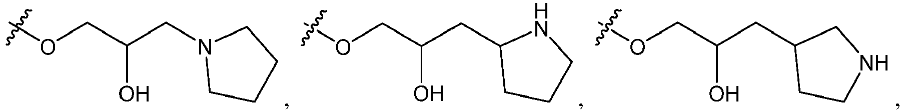

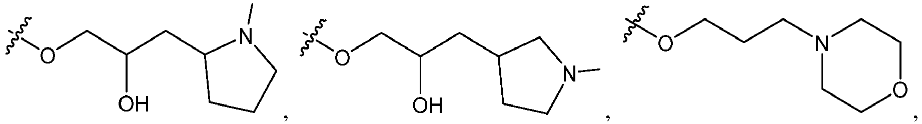

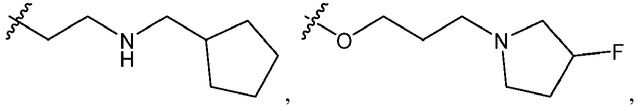

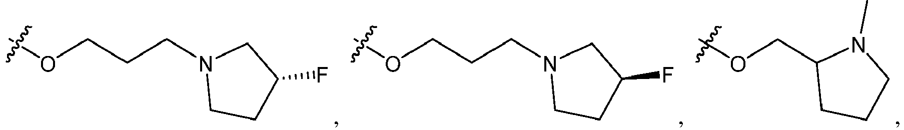

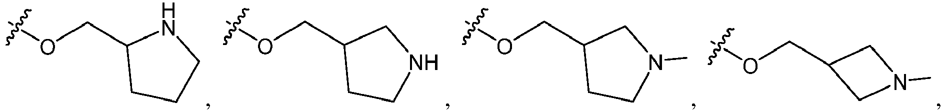

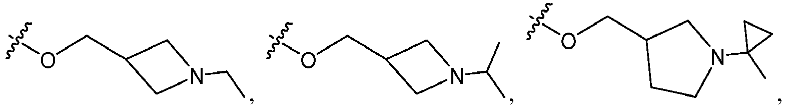

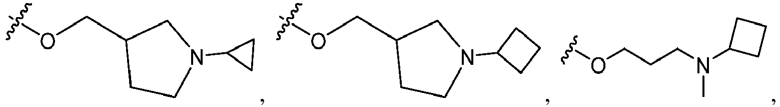

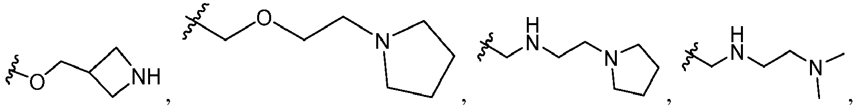

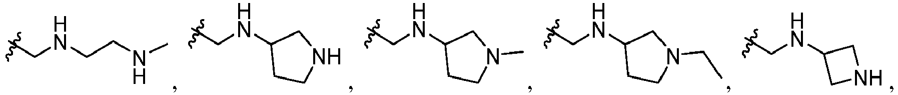

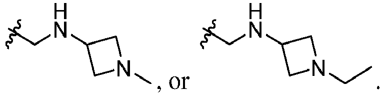

4. A compound for use according to any one of the preceding claims, wherein n is 2 and the compound further comprises another R⁷ selected from halo and methoxy.

5. A compound for use according to claim 1, being of Formula (VI): 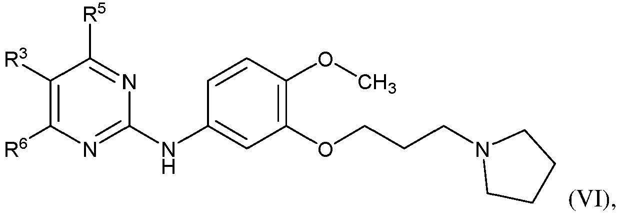 wherein
R⁵ is selected from the group consisting of C₁-C₆ alkyl and NR⁸R⁹; and R⁶ is NR⁸R⁹.

6. A compound for use according to any one of the preceding claims, being of Formula (Villa): 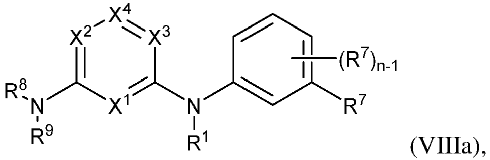 wherein
X¹ is N;
X² is CR³;
X³ is N;
X⁴ is CR⁵;
R³ is H; and
R⁵ is independently selected from the group consisting of C₃-C₈ cycloalkyl, and C₁-C₆ alkyl optionally substituted with one or more of halo or OR^{a}; or
R⁵ and R³ together with the atoms to which they are attached form phenyl or a 5- or 6-membered heteroaryl.

7. A compound for use according to any one of the preceding claims, being of Formula (VIIIb): 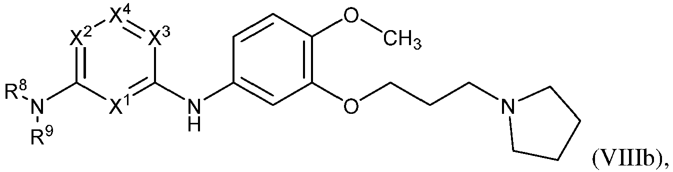 wherein
X¹ is N;
X² is CR³;
X³ is N;
X⁴ is CR⁵;
R⁵ is selected from the group consisting of C₃-C₈ cycloalkyl, and C₁-C₆ alkyl; or
R⁵ and R³ together with the atoms to which they are attached form phenyl or a 5- or 6-membered heteroaryl.

8. A compound for use according to any one of the preceding claims, being of Formula (VIIIc): 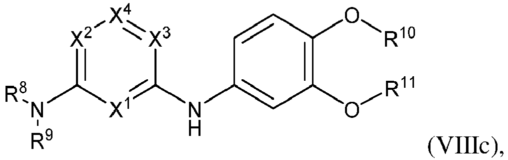 wherein
X¹ is N;
X² is CR³;
X³ is N;
X⁴ is CR⁵;
R⁵ is selected from the group consisting of C₃-C₈ cycloalkyl, and C₁-C₆ alkyl; or
R⁵ and R³ together with the atoms to which they are attached form phenyl or a 5- or 6-membered heteroaryl.

9. A compound for use according to any one of the preceding claims, wherein R⁵ is C₁₋₆ alkyl or R⁵ and R³ together with the atoms to which they are attached form phenyl or a 5- to 6-membered heteroaryl ring.

10. A compound for use according to any one of the preceding claims, wherein the compound is selected from the following compounds and pharmaceutically acceptable salts thereof:
| **Compound No.** | **Structure** |
|---|---|
| 186 | 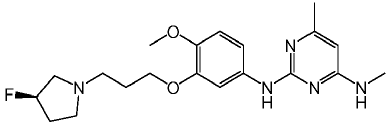 |
| 205 | 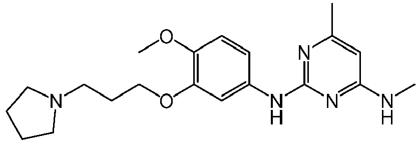 |
| 232 | 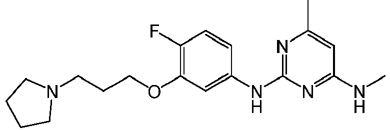 |
| 236 | 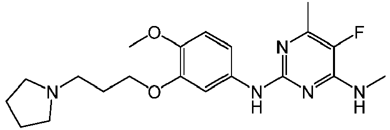 |
| 238 | 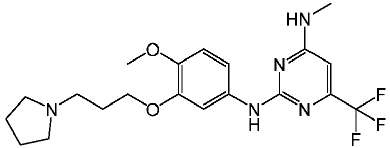 |
| 240 | 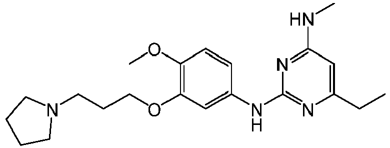 |
| 261 | 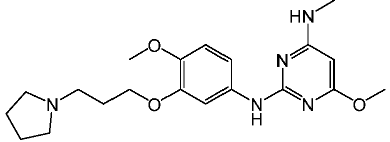 |
| 263 | 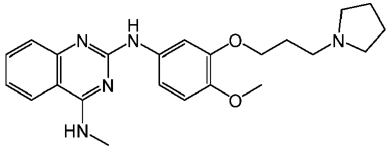 |
| 301 | 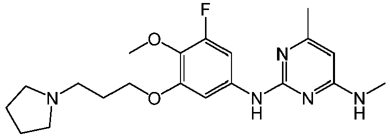 |
| 302 | 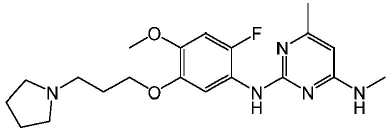 |
| 311 | 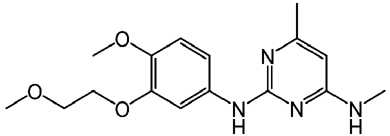 |
| 312 | 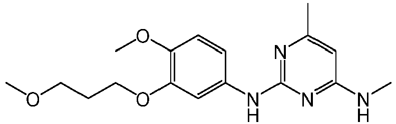 |
| 313 | 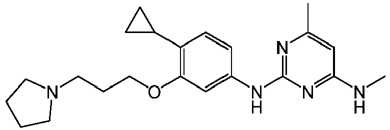 |
| 316 | 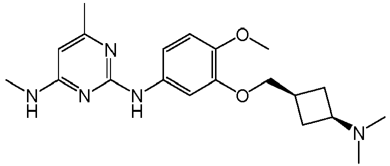 |
| 317 | 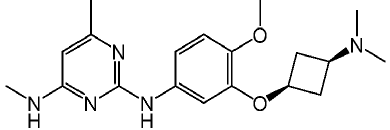 |
| 318 | 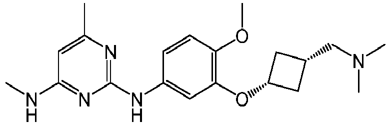 |
| 319 | 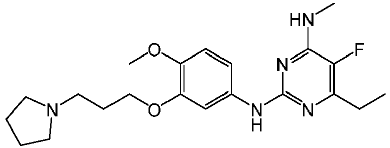 |
| 320 | 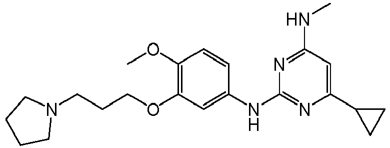 |
| 328 | 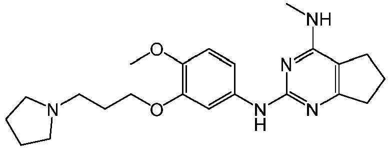 |
| 329 | 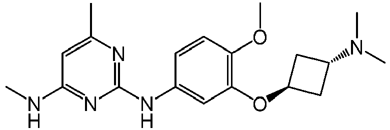 |
| 330 | 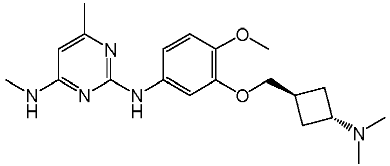 |
| 331 | 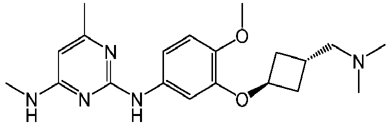 |
| 334 | 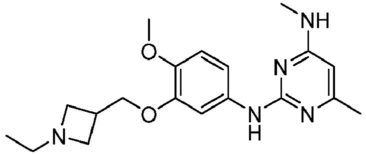 |
| 337 | 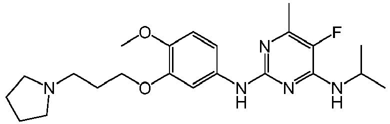 |
| 356 | 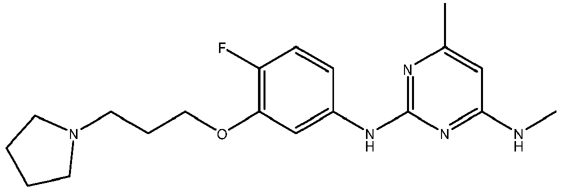 |
| 360 | 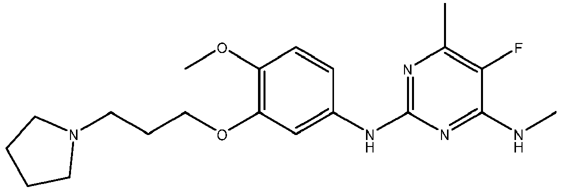 |
| 362 | 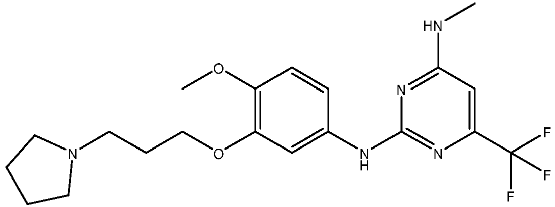 |
| 364 | 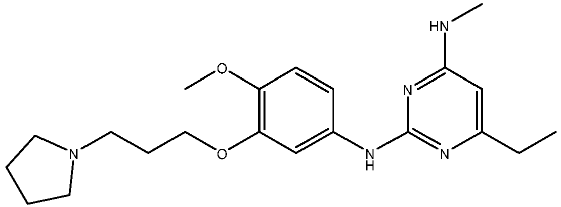 |
| 380 | 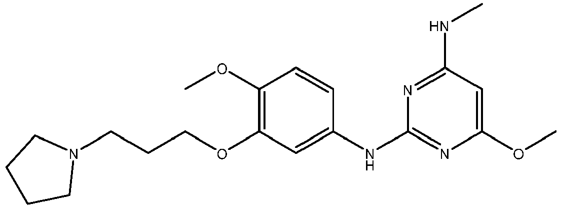 |
| 384 | 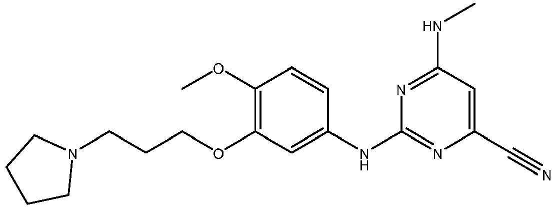 |
| 385 | 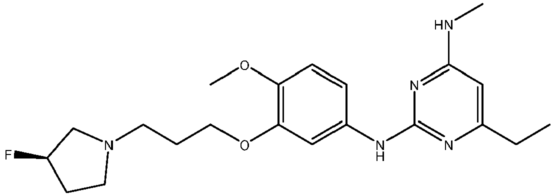 |
| 391 | 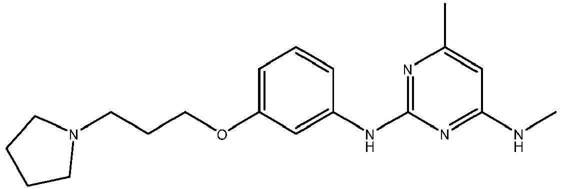 |
| 413 | 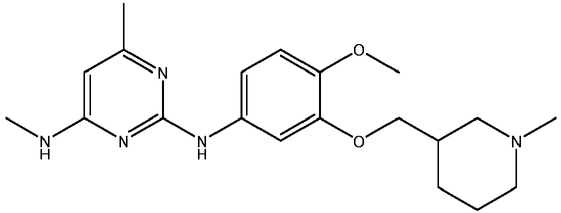 |
| 417 | 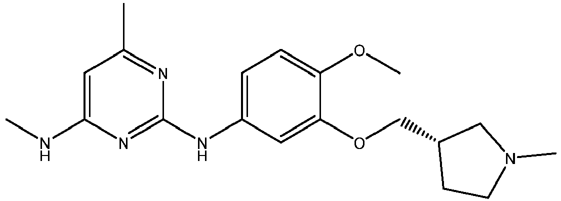 |
| 418 | 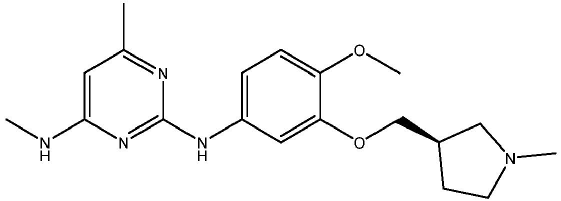 |
| 419 | 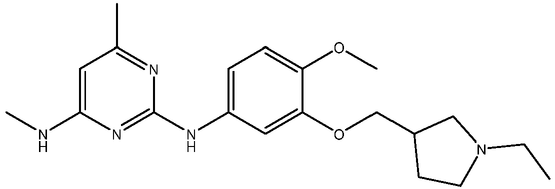 |
| 421 | 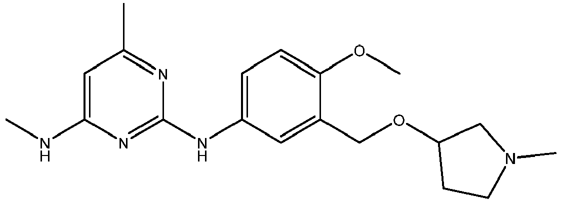 |
| 428 | 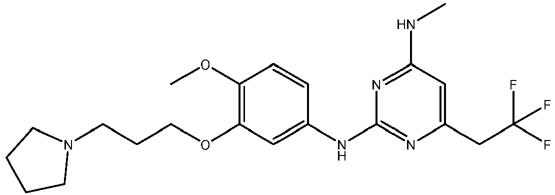 |
| 432 | 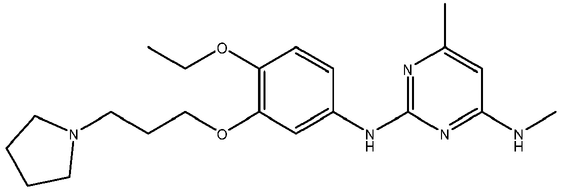 |
| 435 | 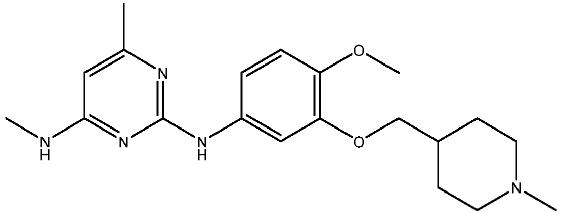 |
| 436 | 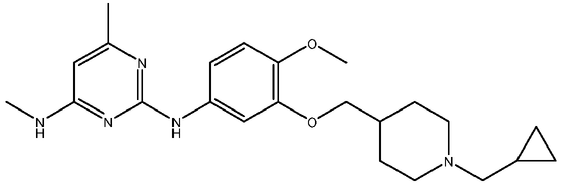 |
| 437 | 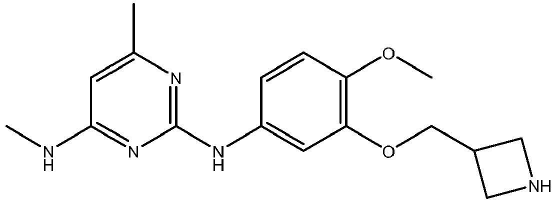 |
| 441 | 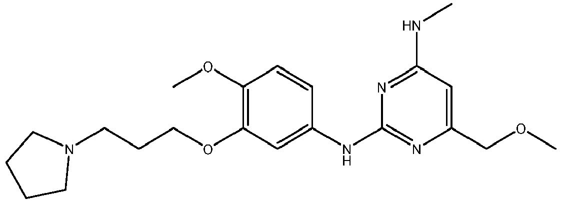 |
| 442 | 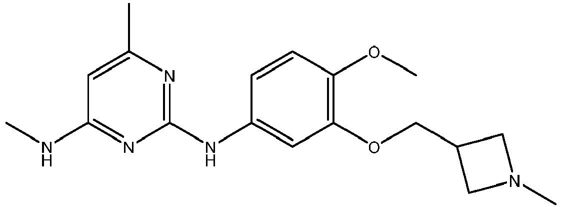 |
| 443 | 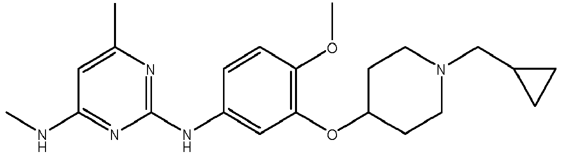 |
| 444 | 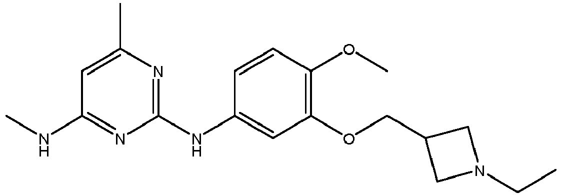 |
| 445 | 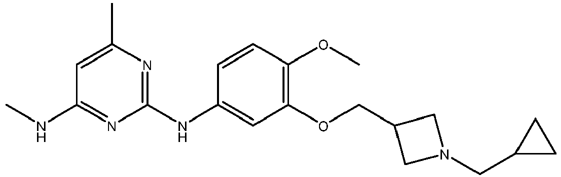 |
| 446 | 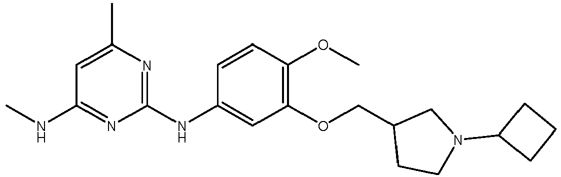 |
| 449 | 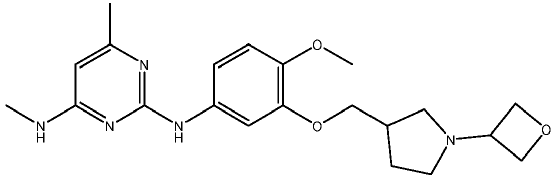 |
| 450 | 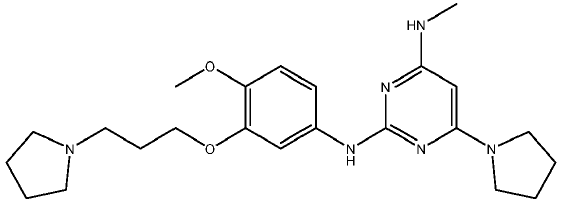 |
| 452 | 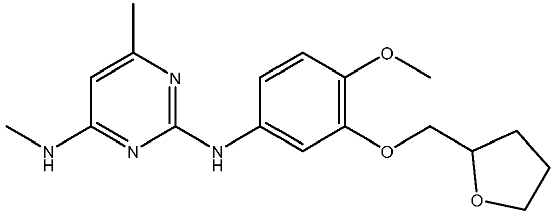 |
| 455 | 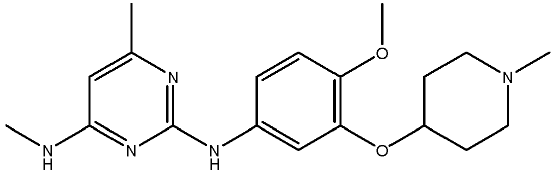 |
| 456 | 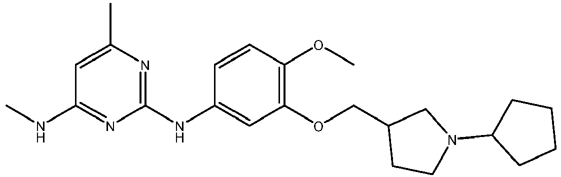 |
| 457 | 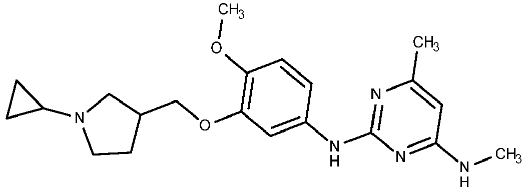 |
| 458 | 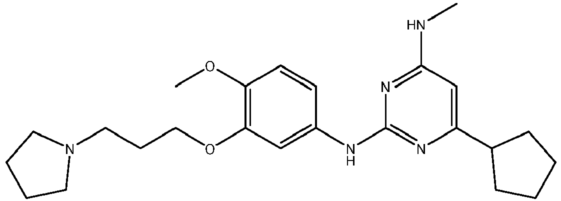 |
| 459 | 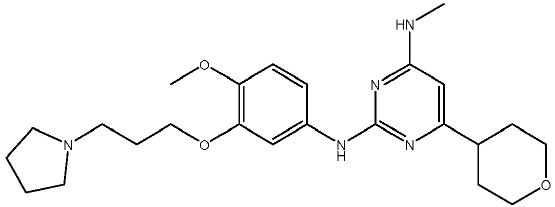 |
| 461 | 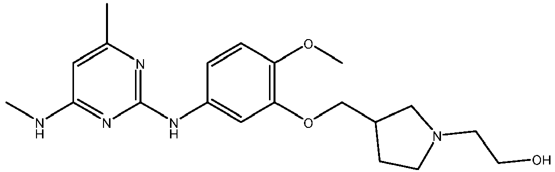 |
| 463 | 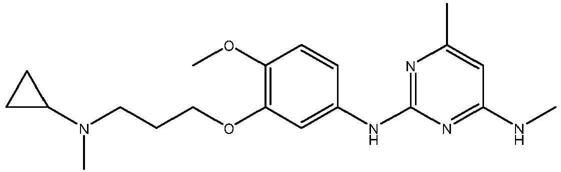 |
| 465 | 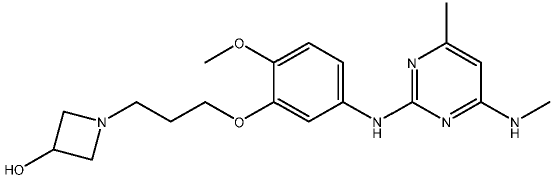 |
| 466 | 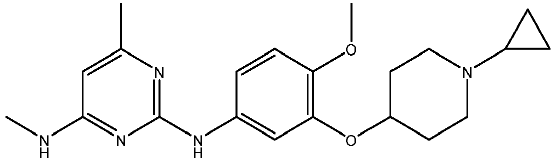 |
| 468 | 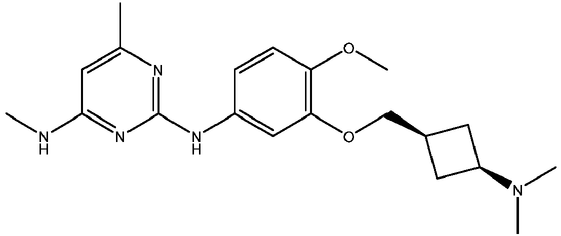 |
| 470 | 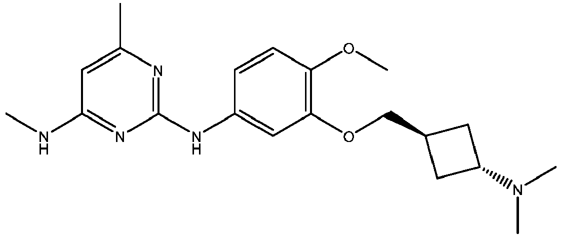 |
| 481 | 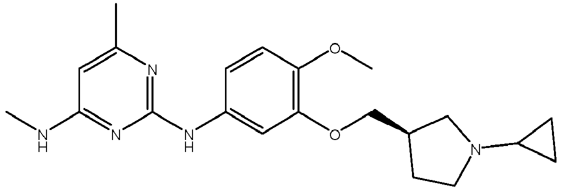 |
| 482 | 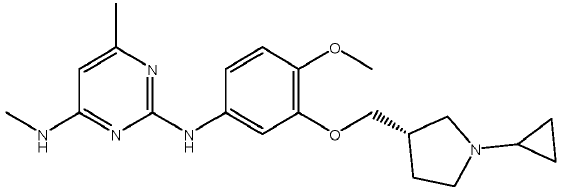 |
| 483 | 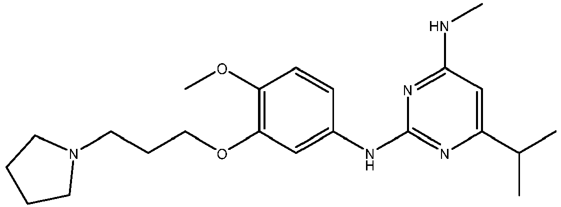 |
| 484 | 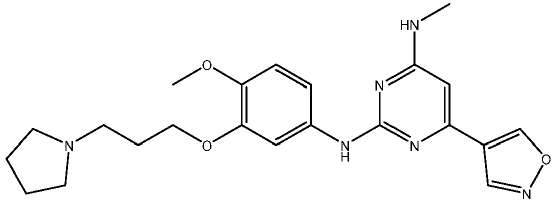 |
| 487 | 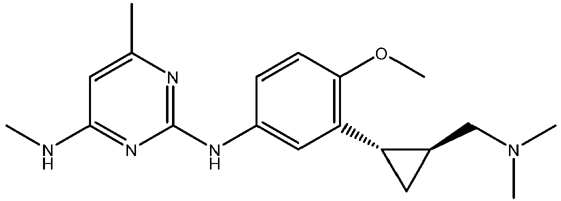 |
| 488 | 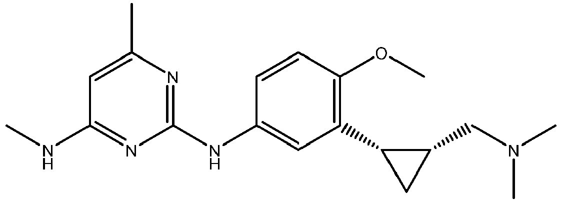 |
| 489 | 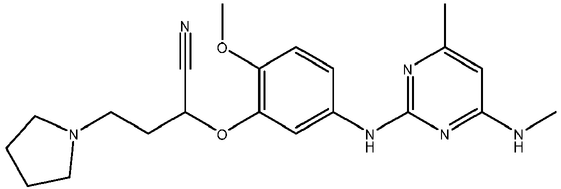 |
| 490 | 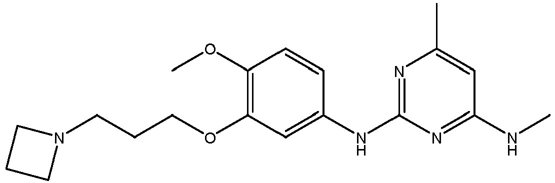 |
| 491 | 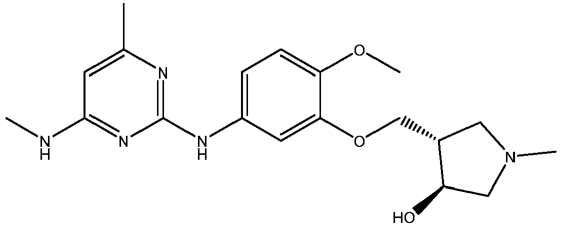 |
| 492 | 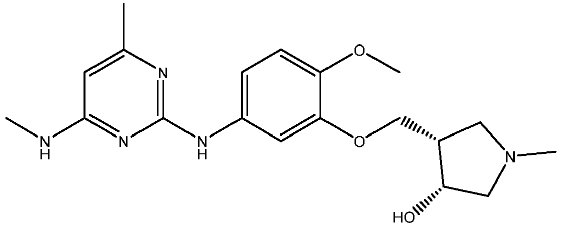 |
| 493 | 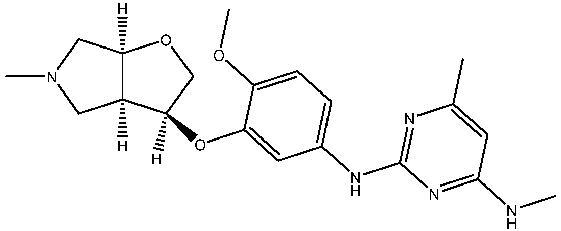 |
| 496 | 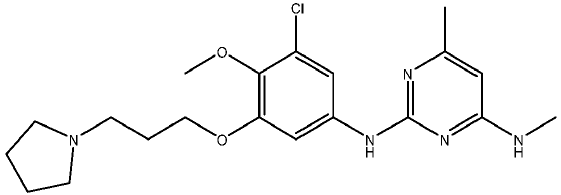 |
| 497 | 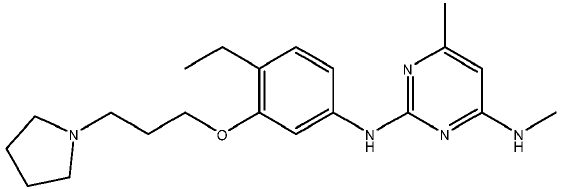 |
| 498 | 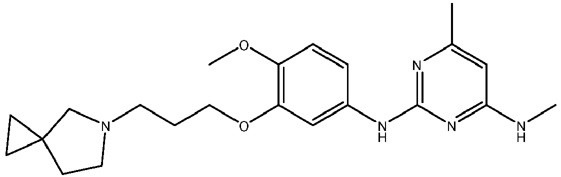 |
| 502 | 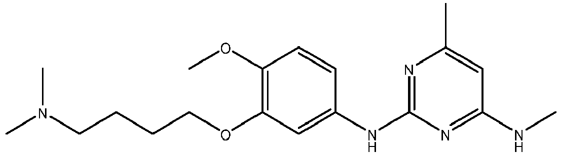 |
| 503 | 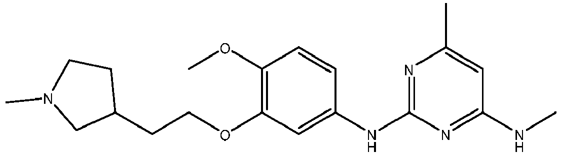 |
| 504 | 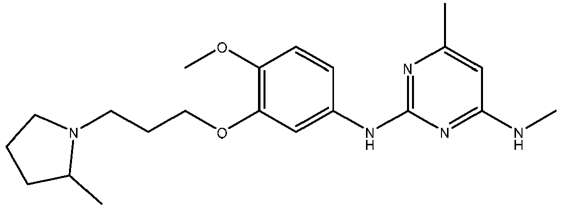 |
| 505 | 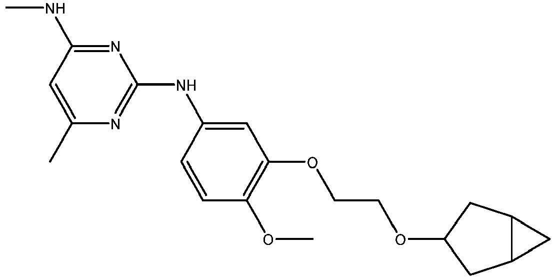 |
| 506 | 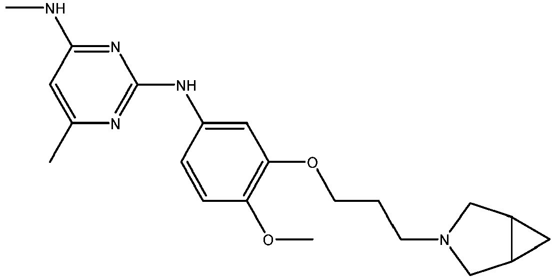 |
| 508 | 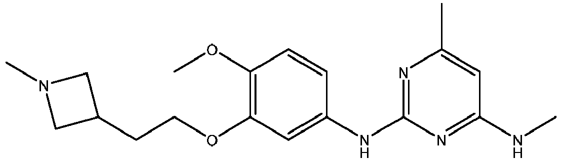 |
| 510 | 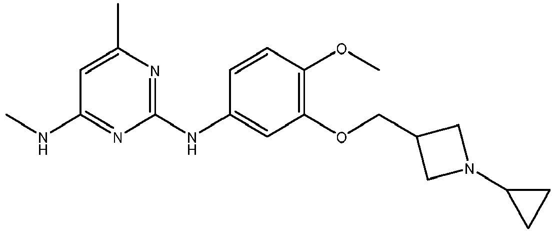 |
| 517a | 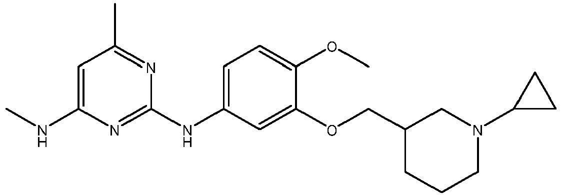 |
| 517b | 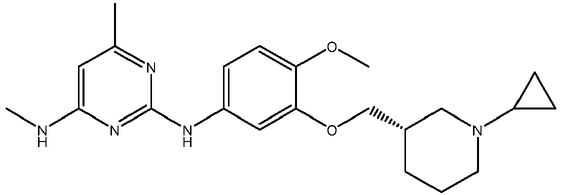 |
| 551 | 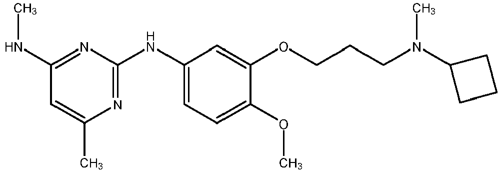 |
| 559 | 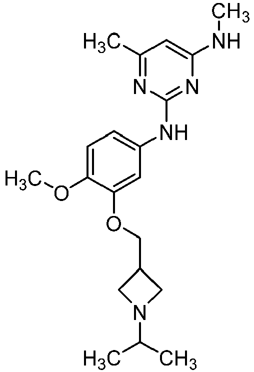 |
| 560 | 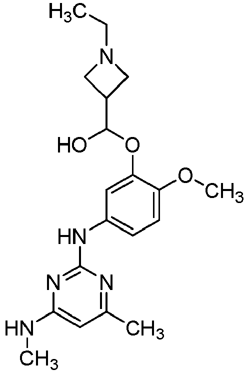 |
| 561 | 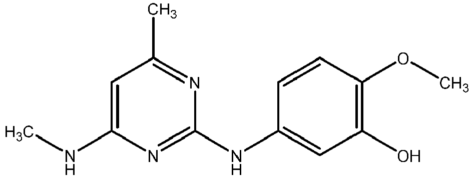 |
| 562 | 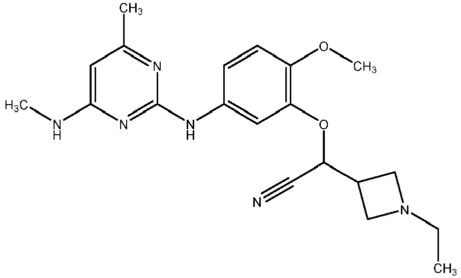 |
| 563 | 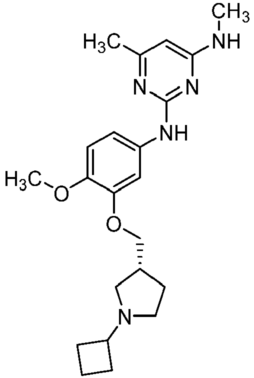 |
| 568 | 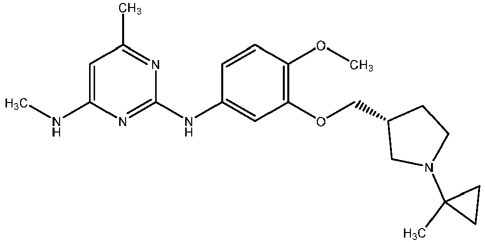 |
| 570 | 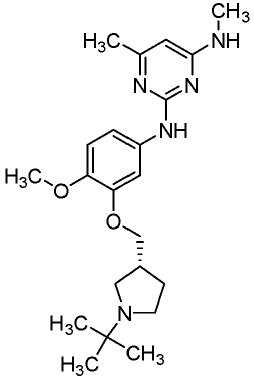 |
| 571 | 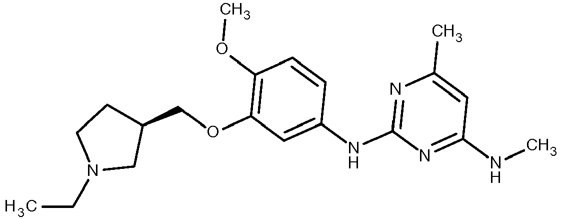 |
| 574 | 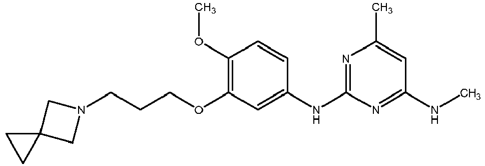 |
| 575 | 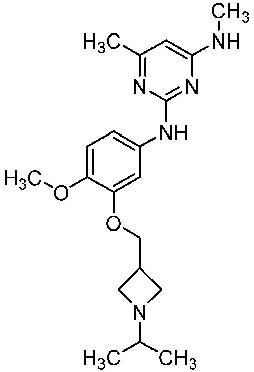 |
| 576 | 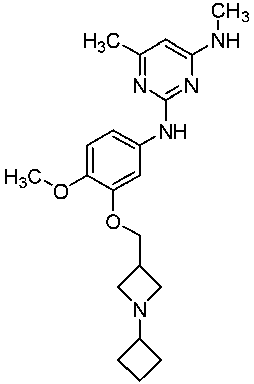 |
| 577 | 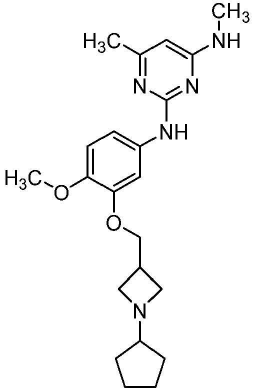 |
| 580 | 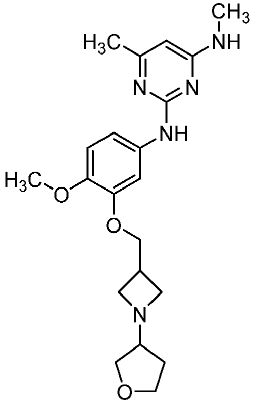 |
| 600 | 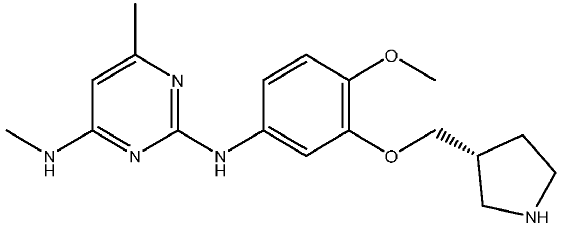 |
| 602 | 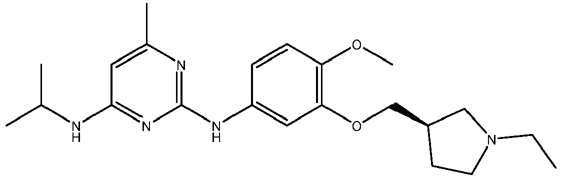 |
| 603 | 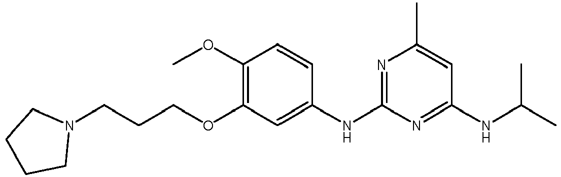 |
| 606 | 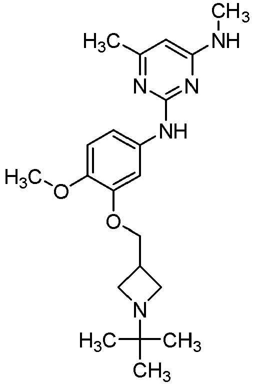 |
| 610 | 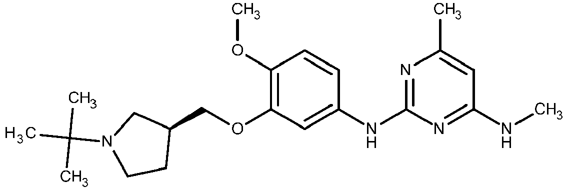 |
| 614 | 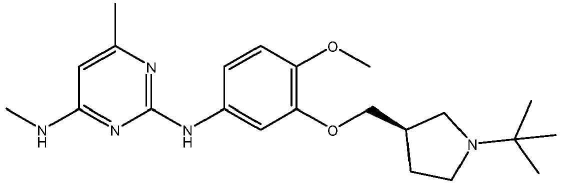 |
| 624 | 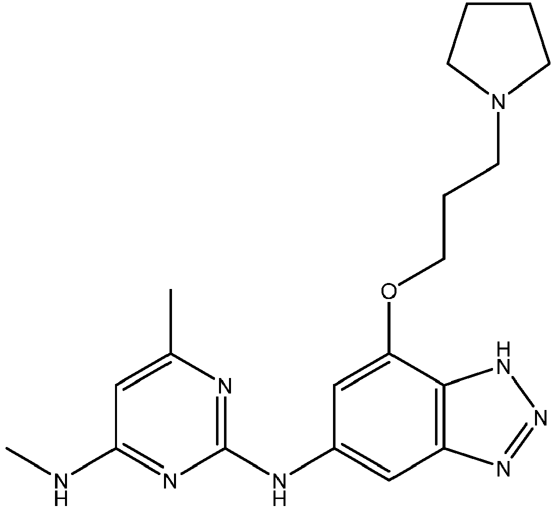 |
| 644 | 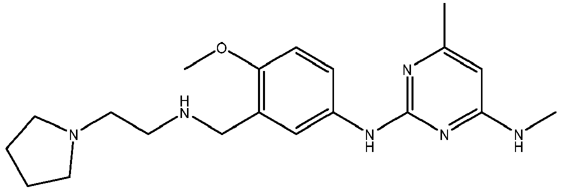 |
| 645 | 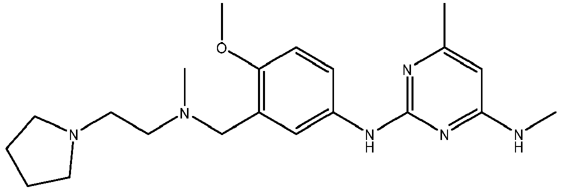 |
| 646 | 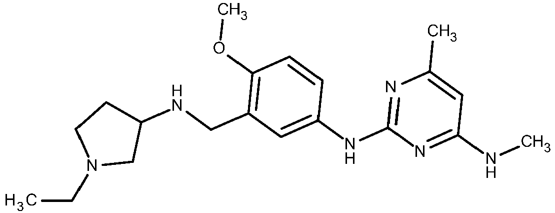 |
| 647 | 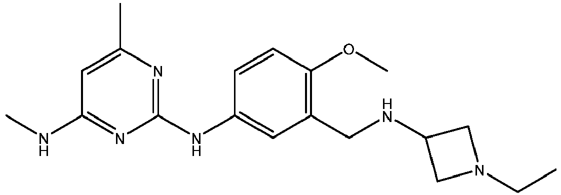 |
| 649 | 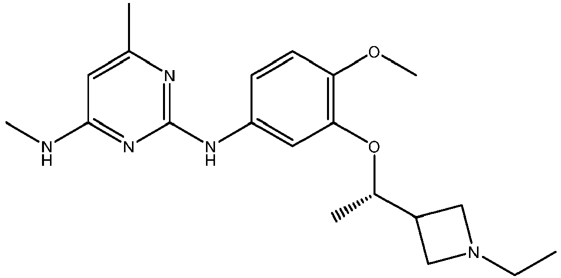 |
| 650 | 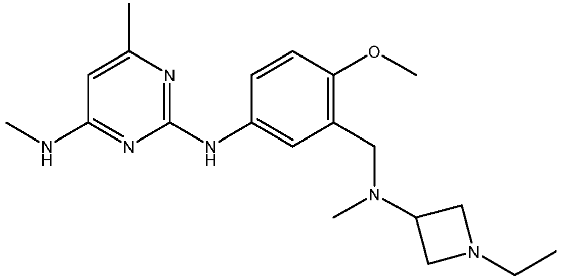 |
| 651 | 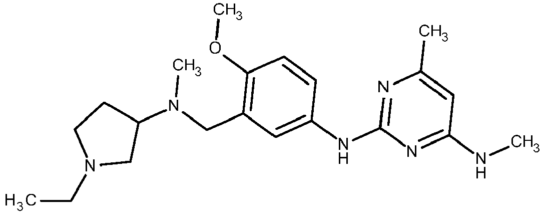 |
| 671 | 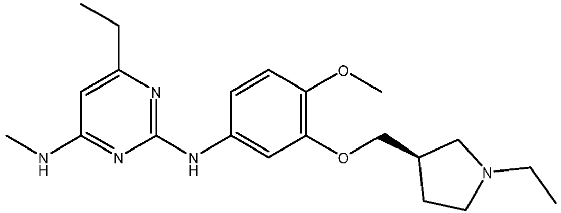 |
| 672 | 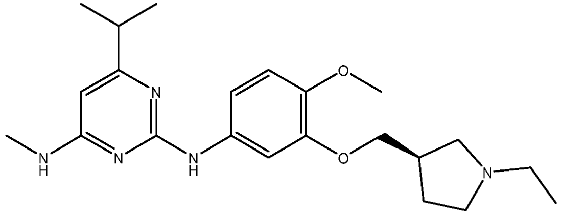 |
| 680 | 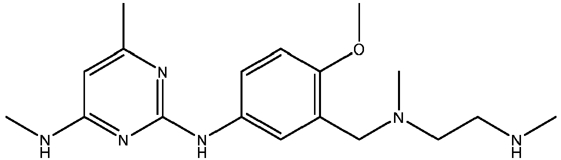 |
| 687 | 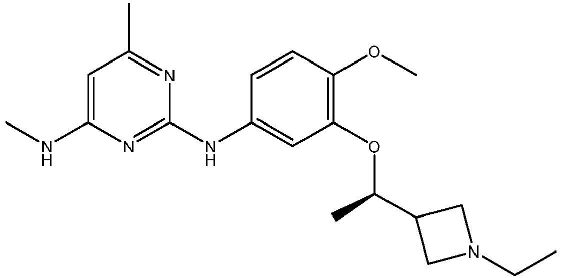 |
| 765 | 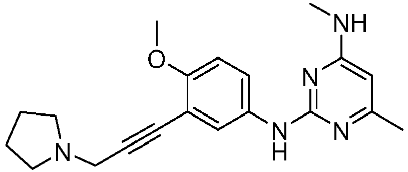 |
| 786 | 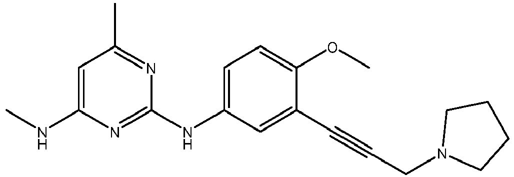 |
| 791 | 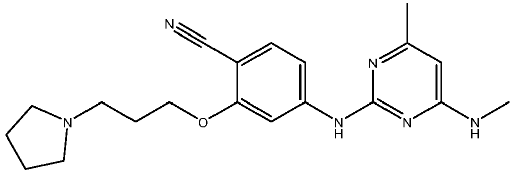 |
| 793 | 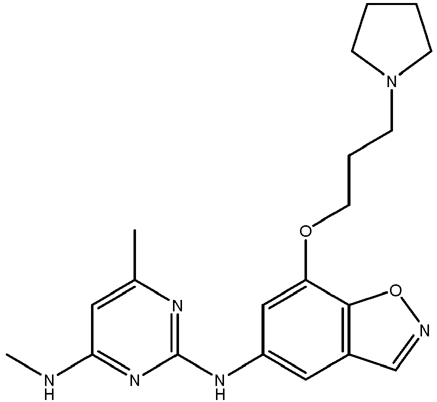 |
| 799 | 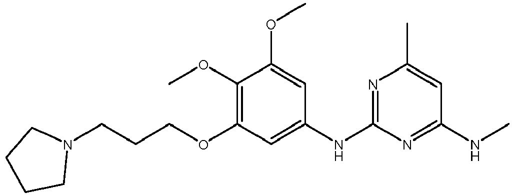 |
| 800 | 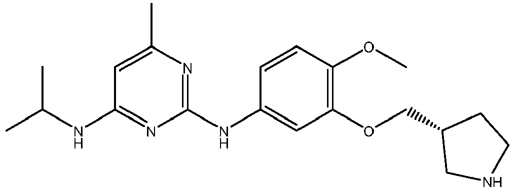 |
| 813 | 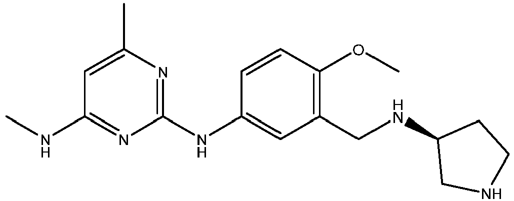 |
| 814 | 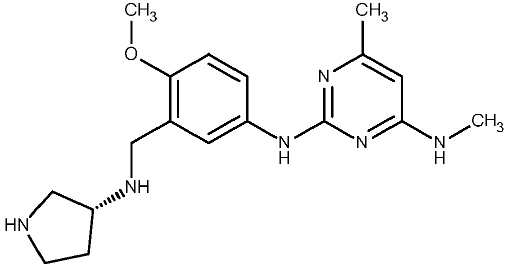 |
| 845 | 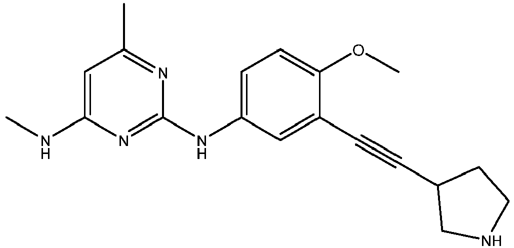 |
| 846 | 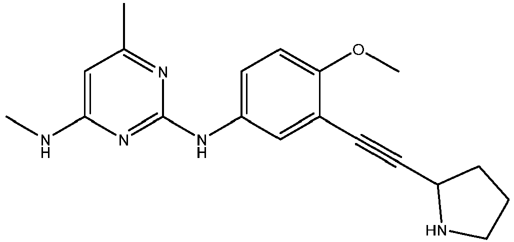 |
| 847 | 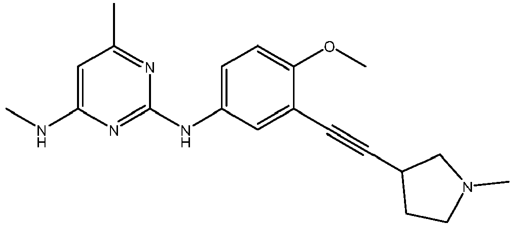 |
| 848 | 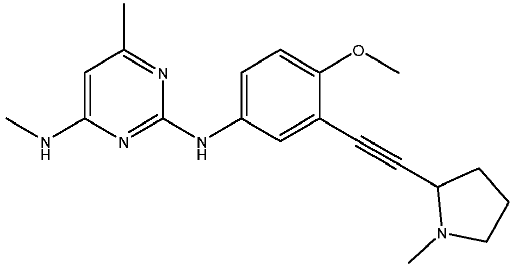 |
| 855 | 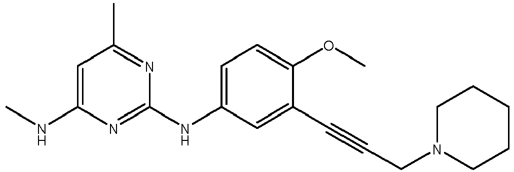 |
| 860 | 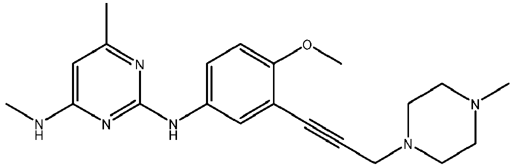 |
| 871 | 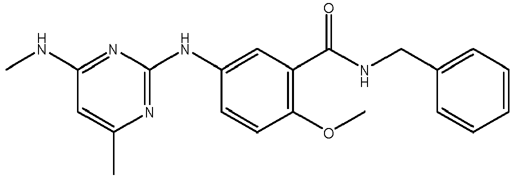 |
| 872 | 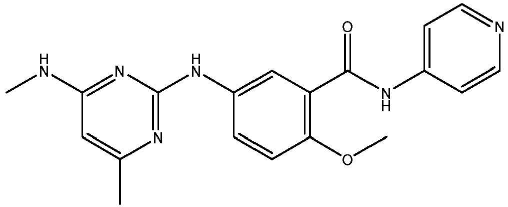 |
| 873 | 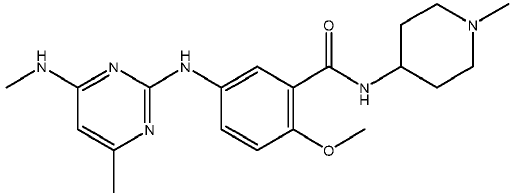 |
| 874 | 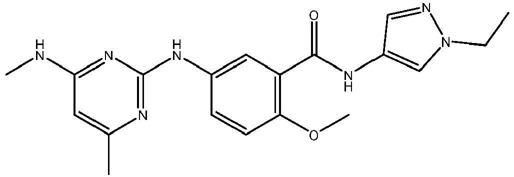 |
| 876 | 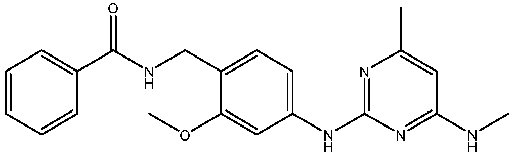 |
| 877 | 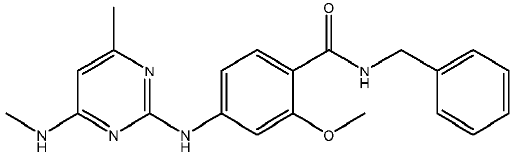 |
| 878 | 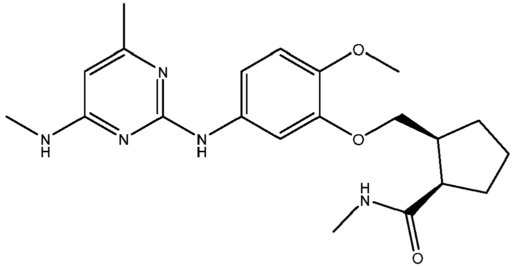 |
| 879 | 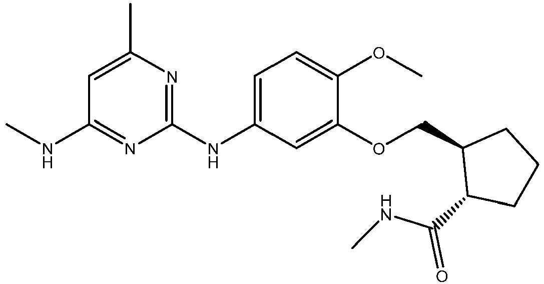 |
| 890 | 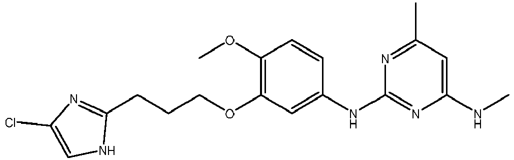 |
| 891 | 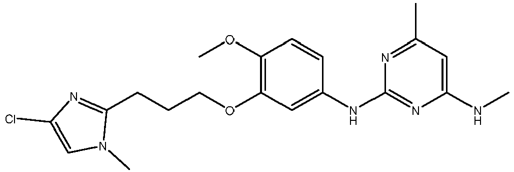 |
| 904 | 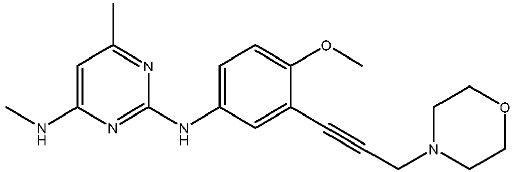 |
| 905 | 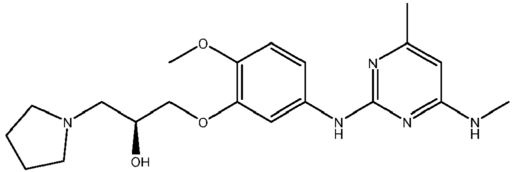 |
| 906 | 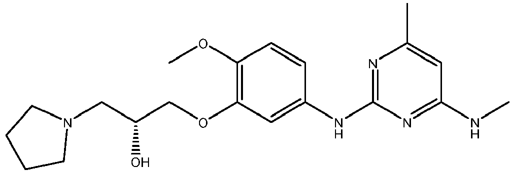 |
| 962 | 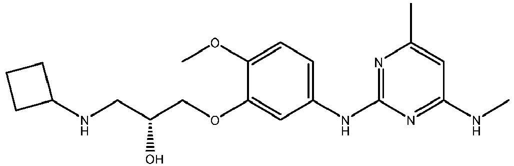 |
| 964 | 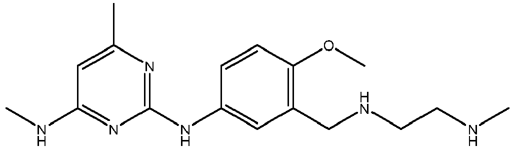 |
| 965 | 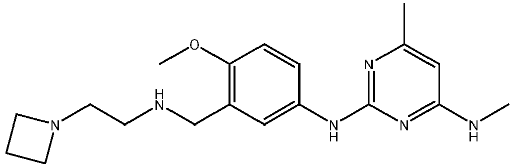 |
| 966 | 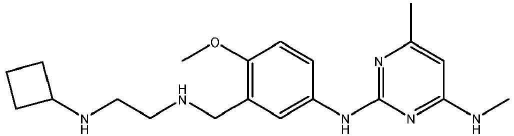 |
| 967 | 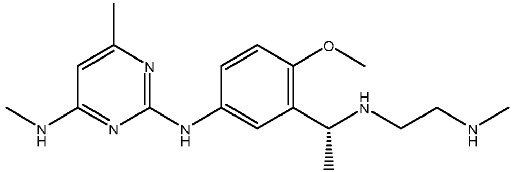 |
| 968 | 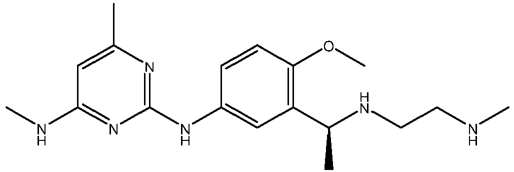 |
| 969 | 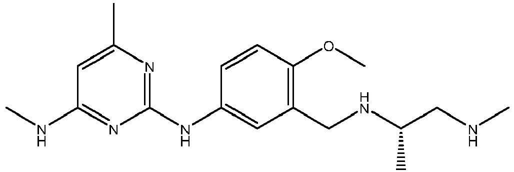 |
| 970 | 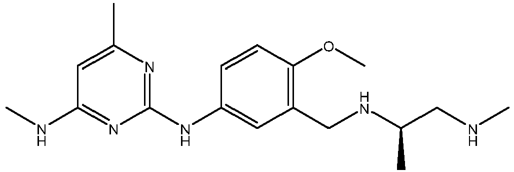 |
| 971 | 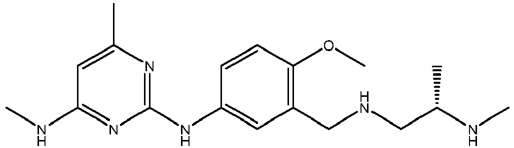 |
| 972 | 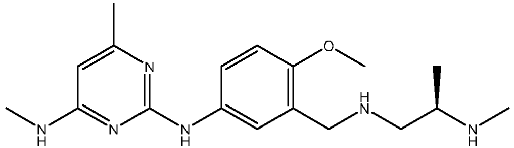 |
| 977 | 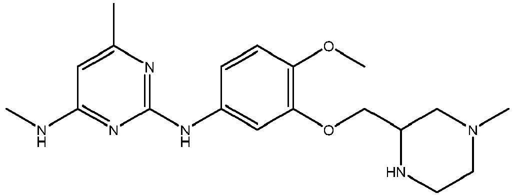 |
| 985 | 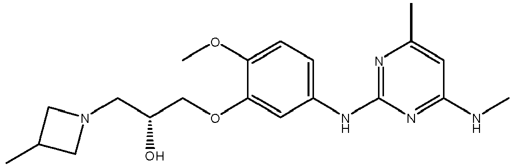 |
| 986 | 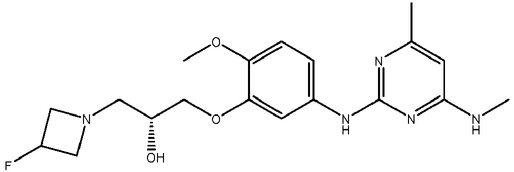 |
| 989 | 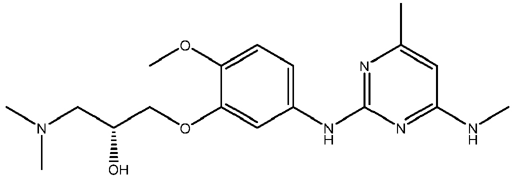 |
| 990 | 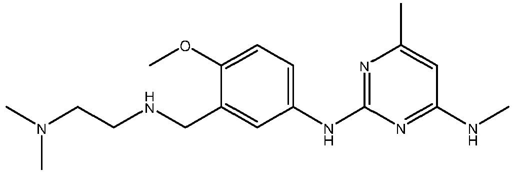 |
| 991 | 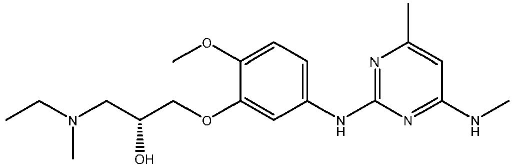 |
| 992 | 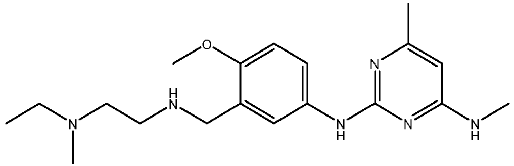 |
| 993 | 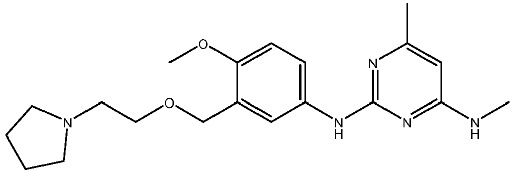 |
| 994 | 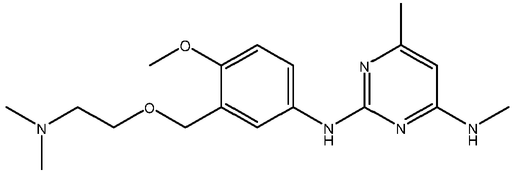 |
| 997 | 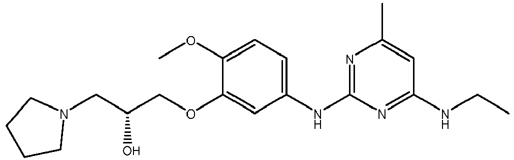 |
| 998 | 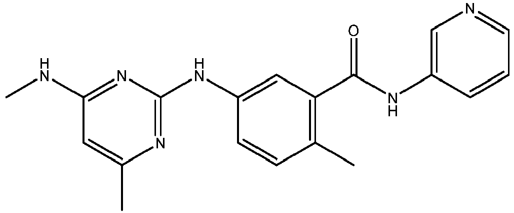 |
| 999 | 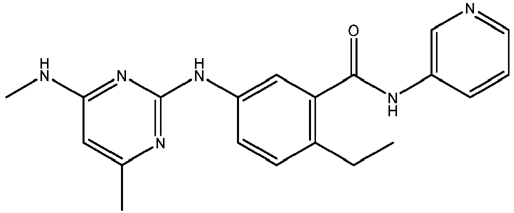 |
| 1000 | 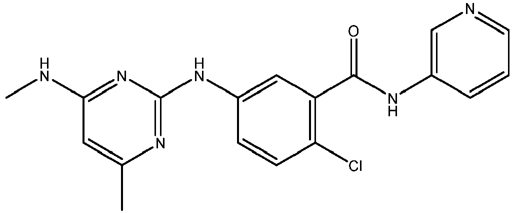 |
| 1001 | 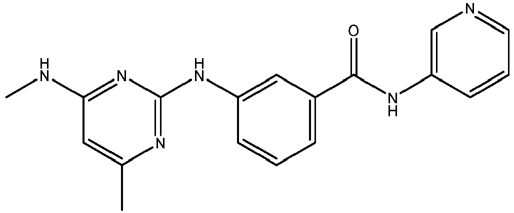 |
| 1004 | 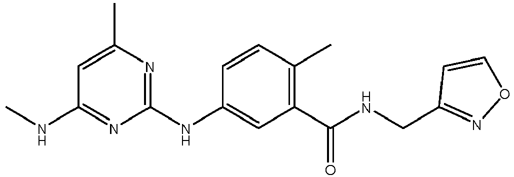 |
| 1029 | 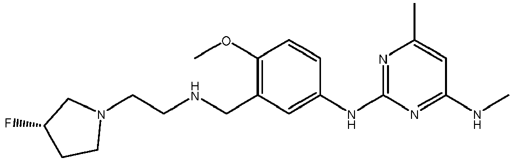 |
| 1030 | 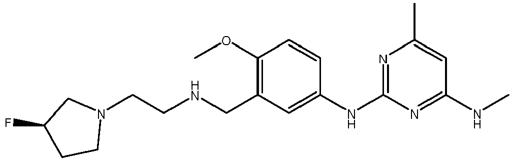 |
| 1036 | 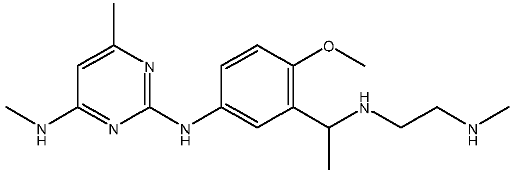 |
| 1037 | 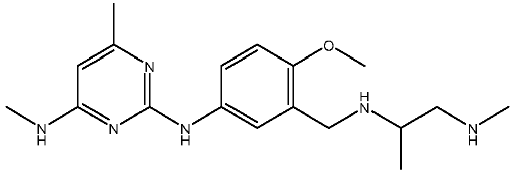 |
| 1038 | 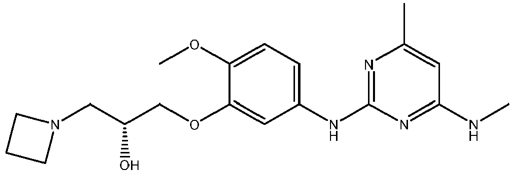 |
| 1040 | 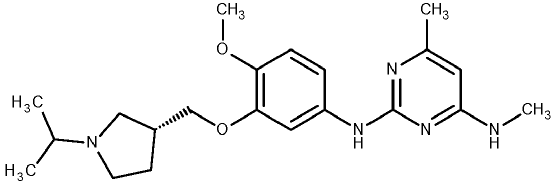 |
| 1044 | 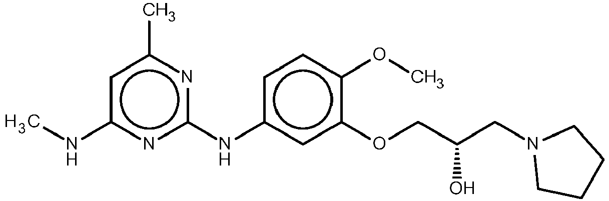 |
| 1045 | 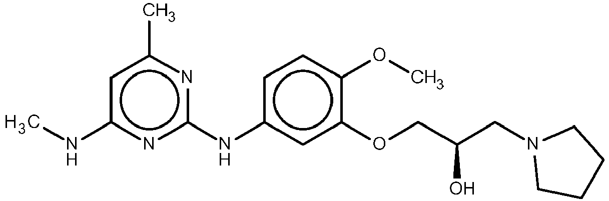 |
| 1078 | 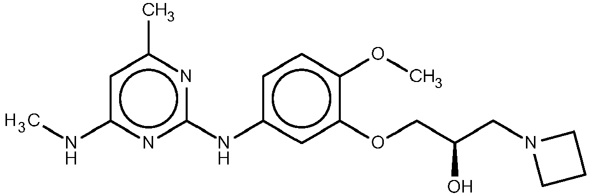 |
| 1079 | 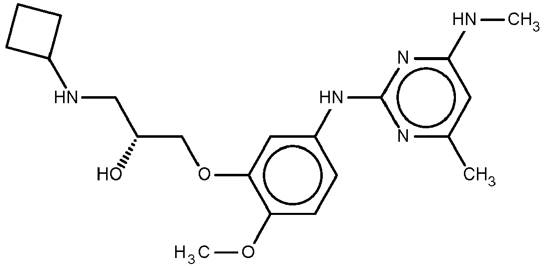 |
| 1080 | 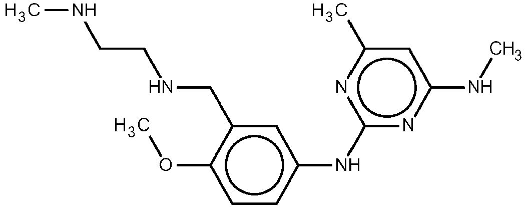 |
| 1081 | 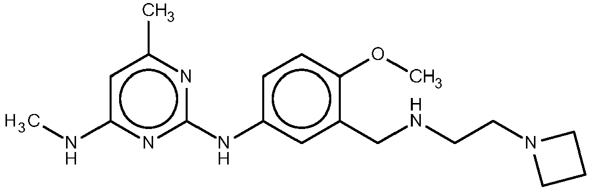 |
| 1090 | 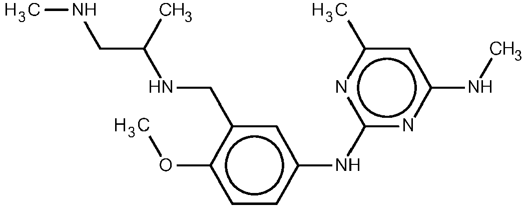 |
| 1094 | 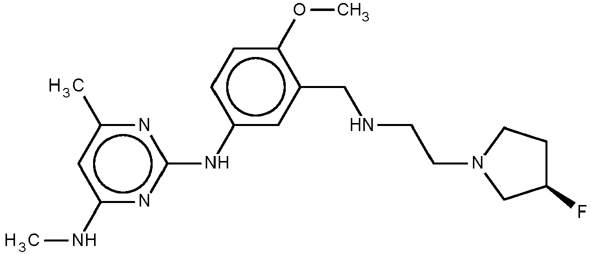 |
| 1095 | 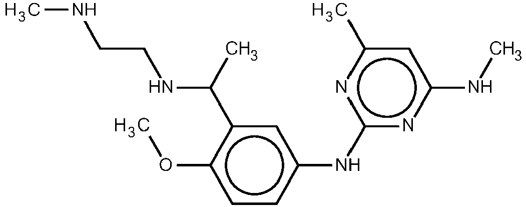 |
| 1099 | 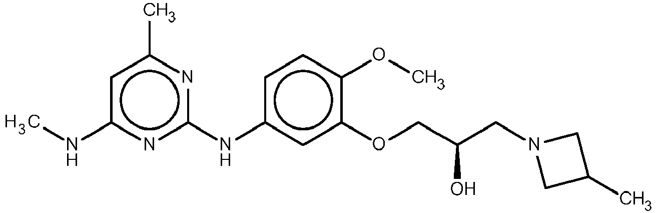 |
| 1100 | 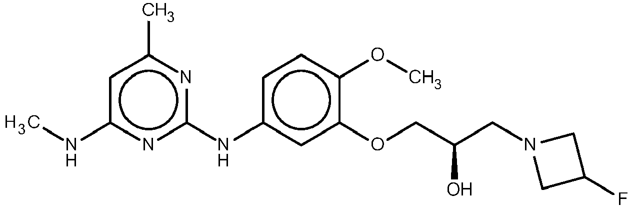 |
| 1101 | 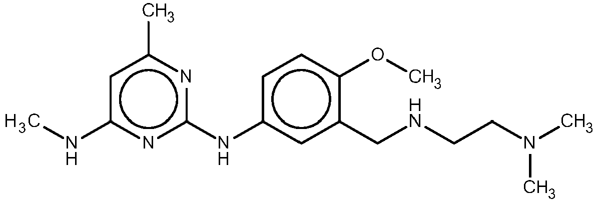 |
| 1102 | 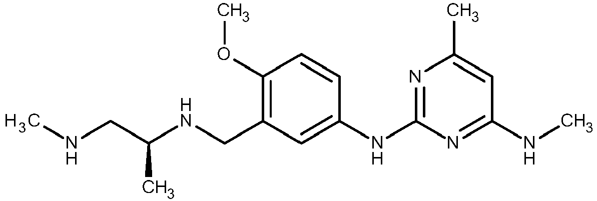 |
| 1103 | 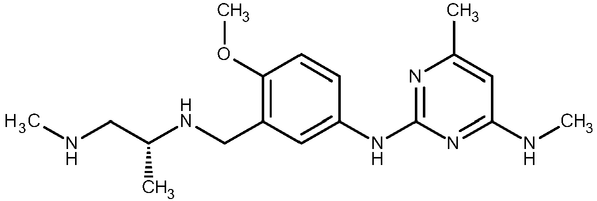 |
| 1104 | 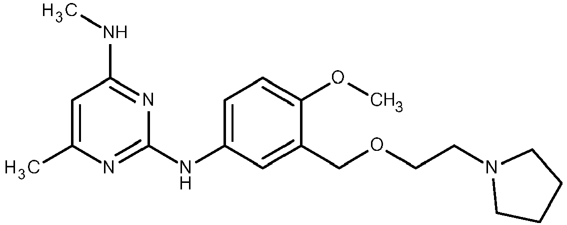 |
| 1105 | 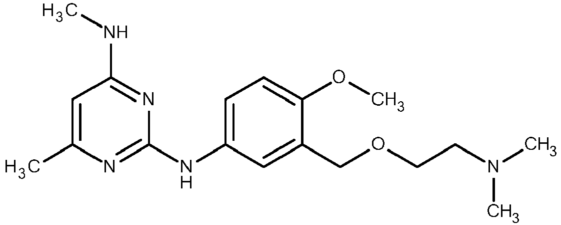 |
| 1106 | 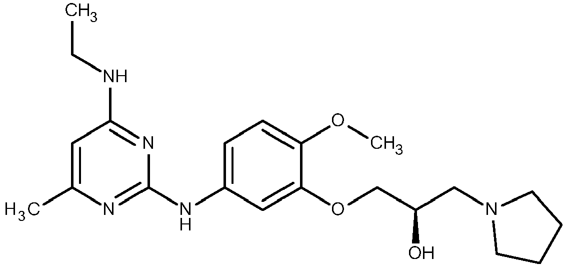 |
| 1109 | 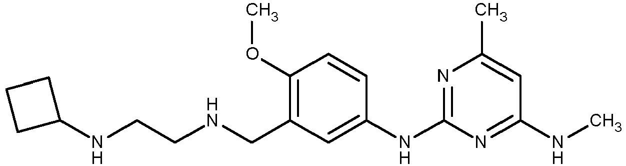 |
| 1112 | 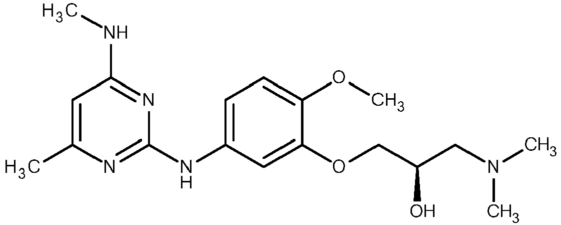 |
| 1113 | 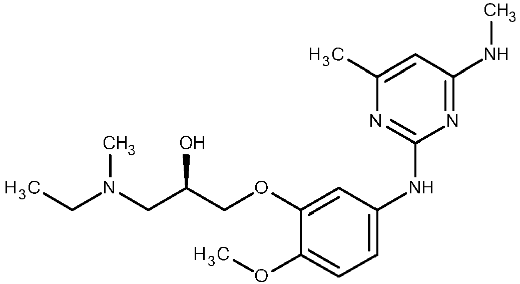 |
| 1114 | 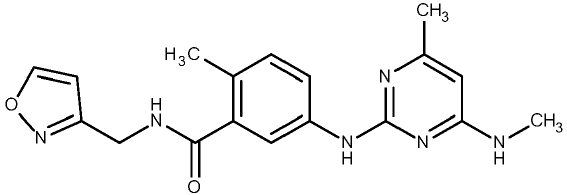 |
| 1117 | 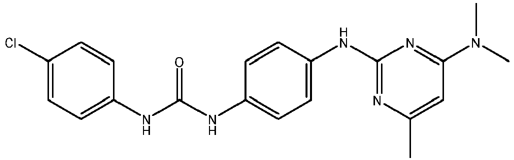 |

11. A compound for use as defined in any of claims 1-10, wherein the blood disorder is sickle cell anemia or β-thalassemia.

12. A compound for use as defined in any of claims 1-10, wherein the blood disorder is a hematological cancer.

13. A compound for use as defined in claim 12, wherein the hematological cancer is acute myeloid leukemia (AML) or chronic lymphocytic leukemia (CLL).

14. A compound for use as defined in any of the preceding claims, wherein the compound is a selective inhibitor of EHMT2.

## Patentansprüche

1. Verbindung der Formel (IIa1) zur Verwendung beim Behandeln oder Vorbeugen einer Blutkrankheit: 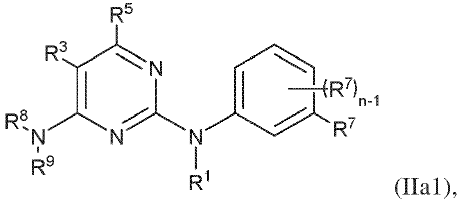
oder ein Tautomer davon oder ein pharmazeutisch verträgliches Salz der Verbindung oder des Tautomers, wobei
R¹ H oder C₁-C₄-Alkyl ist;
R³ aus der Gruppe bestehend aus H, Halogen, Cyano, C₁-C₆-Alkoxyl, C₆-C₁₀-Aryl, NR^{a}C(O)R^{b}, C₃-C₈-Cycloalkyl, 4- bis 7-gliedrigem Heterocycloalkyl, 5- bis 6-gliedrigem Heteroaryl und C₁-C₆-Alkyl ausgewählt ist, wobei C₁-C₆-Alkoxyl und C₁-C₆-Alkyl optional mit einem oder mehreren von Halogen, OR^{a} oder NR^{a}R^{b} substituiert sind, in denen jedes von R^{a} und R^{b} unabhängig H oder C₁-C₆-Alkyl sind oder R³ -Q¹-T¹ ist, wobei Q¹ eine Bindung oder ein C₁-C₆-Alkylen-, C₂-C₆-Alkenylen- oder C₂-C₆-Alkinylen-Linker ist, der optional mit einem oder mehreren von Halogen, Cyano, Hydroxyl, Oxo oder C₁-C₆-Alkoxyl substituiert ist, und T¹ H, Halogen, Cyano, NR⁸R⁹, C(O)NR⁸R⁹, OR⁸, OR⁹ oder R^{S1} ist, wobei R^{S1} C₃-C₈-Cycloalkyl, Phenyl, 4- bis 12-gliedriges Heterocycloalkyl, das 1-4 Heteroatome ausgewählt aus N, O und S enthält, oder ein 5- oder 6-gliedriges Heteroaryl und R^{S1} optional mit einem oder mehreren von Halogen, C₁-C₆-Alkyl, Hydroxyl, Oxo, -C(O)R⁹, -SO₂R⁸, -SO₂N(R⁸)₂, -NR⁸C(O)R⁹, Amino, Mono- oder Dialkylamino oder C₁-C₆-Alkoxyl substituiert ist;
R⁵ aus der Gruppe bestehend aus F, Br, Cyano, C₁-C₆-Alkoxyl, C₆-C₁₀-Aryl, NR^{a}R^{b}, C(O)NR^{a}R^{b}, NR^{a}C(O)R^{b}, C₃-C₈-Cycloalkyl, 4- bis 12-gliedrigem Heterocycloalkyl, das 1-4 Heteroatome ausgewählt aus N, O, und S enthält, C₁-C₆-Alkyl, das optional mit einem oder mehreren von Halogen, OR^{a} oder NR^{a}R^{b} substituiert ist, und C₂-C₆-Alkinyl, das optional mit 4-bis 12-gliedrigem Heterocycloalkyl substituiert ist, ausgewählt ist; wobei das Cs-Cs-Cycloalkyl oder das 4- bis 12-gliedrige Heterocycloalkyl optional mit einem oder mehreren von Halogen, C(O)R^{a}, OR^{a}, NR^{a}R^{b}, 4- bis 7-gliedrigem Heterocycloalkyl, -C₁-C₆-Alkylen-4- bis 7-gliedrigem Heterocycloalkyl oder C₁-C₄-Alkyl, das optional mit einem oder mehreren von Halogen, OR^{a} oder NR^{a}R^{b} ist, wobei jedes von R^{a} und R^{b} unabhängig H oder C₁-C₆-Alkyl ist, substituiert sind; oder
R⁵ und R³ zusammen mit den Atomen, an die sie gebunden sind, Phenyl oder ein 5- oder 6-gliedriges Heteroaryl bilden;
jedes R⁷ unabhängig Oxo (=O) oder -Q²-T² ist, wobei jedes Q² unabhängig eine Bindung oder ein C₁-C₆-Alkylen-, C₂-C₆-Alkenylen- oder C₂-C₆-Alkinylen-Linker ist, der optional mit einem oder mehreren von Halogen, Cyano, Hydroxyl, Amino, Mono- oder Dialkylamino oder C₁-C₆-Alkoxyl substituiert ist und jedes T² unabhängig H, Halogen, Cyano, OR¹⁰, OR¹¹, C(O)R¹¹, NR¹⁰R¹¹, C(O)NR¹⁰R¹¹, NR¹⁰C(O)R¹¹, 5- bis 10-gliedriges Heteroaryl, Cs-Cs-Cycloalkyl oder 4-bis 12-gliedriges Heterocycloalkyl, das 1-4 Heteroatome ausgewählt aus N, O und S enthält, ist, und wobei das 5- bis 10-gliedrige Heteroaryl, C₃-C₈-Cycloalkyl oder 4- bis 12-gliedrige Heterocycloalkyl optional mit einem oder mehreren von Halogen, C₁-C₆-Alkyl, das optional mit NR^{x}R^{y}, Hydroxyl, Oxo, N(R⁸)₂, Cyano, C₁-C₆-Halogenalkyl, -SO₂R⁸ oder C₁-C₆-Alkoxyl substituiert ist, substituiert ist, wobei jedes von R^{x} und R^{y} unabhängig H oder C₁-C₆-Alkyl ist; und R⁷ nicht H oder C(O)OR^{g} ist; oder optional ein R⁷ und R⁵ zusammen einen C₃-C₁₀-Alkylen-, C₂-C₁₀-Heteroalkylen-, C₄-C₁₀-Alkenylen-, C₂-C₁₀-Heteroalkenylen-, C₄-C₁₀-Alkinylen- oder C₂-C₁₀-Heteroalkinylen-Linker bilden, der optional mit einem oder mehreren von Halogen, Cyano, Hydroxyl oder C₁-C₆-Alkoxyl substituiert ist;
jedes R⁸ unabhängig H oder C₁-C₆-Alkyl ist;
jedes R⁹ unabhängig -Q³-T³ ist, wobei Q³ eine Bindung oder C₁-C₆-Alkylen ist, und T³ H ist;
jedes R¹⁰ aus der Gruppe bestehend aus H und C₁-C₆-Alkyl ausgewählt ist;
jedes R¹¹ -Q⁶-T⁶ ist, wobei Q⁶ eine Bindung oder ein C₁-C₆-Alkylen-, C₂-C₆-Alkenylen- oder C₂-C₆-Alkinylen-Linker ist, der optional mit einem oder mehreren von Halogen, Cyano, Hydroxyl, Oxo oder C₁-C₆ Alkoxyl substituiert ist, und T⁶ H, Halogen, OR^{g}, NR^{g}R^{h}, NR^{g}C(O)R^{h}, C(O)NR^{g}R^{h}, C(O)R^{g}, S(O)₂R^{g} oder R^{S3} ist, wobei jedes von R^{g} und R^{h} unabhängig H, Phenyl, C₃-Cs-Cycloalkyl oder C₁-C₆-Alkyl, das optional mit C₃-C₈-Cycloalkyl substituiert ist, ist, oder R^{g} und R^{h} zusammen mit dem Stickstoffatom, an das sie gebunden sind, ein 4- bis 12-gliedriges Heterocycloalkyl, das 1-4 Heteroatome ausgewählt aus N, O und S enthält, und R^{S3} C₃-C₈-Cycloalkyl, C₆-C₁₀-Aryl, 4- bis 12-gliedriges Heterocycloalkyl, das 1-4 Heteroatome ausgewählt aus N, O und S enthält, oder ein 5- bis 10-gliedriges Heteroaryl ist, und R^{S3} optional mit einem oder mehreren -Q⁷-T⁷ substituiert ist, wobei jedes Q⁷ unabhängig eine Bindung oder ein C₁-C₃-Alkylen-, C₂-C₃-Alkenylen- oder C₂-C₃-Alkinylen-Linker ist, die jeweils optional mit einem oder mehreren von Halogen, Cyano, Hydroxyl oder C₁-C₆-Alkoxy substituiert sind und jedes T⁷ unabhängig aus der Gruppe bestehend aus H, Halogen, Cyano, C₁-C₆-Alkyl, C₃-C₈-Cycloalkyl, C₆-C₁₀-Aryl, 4- bis 7-gliedrigem Heterocycloalkyl, das 1-4 Heteroatome ausgewählt aus N, O und S enthält, 5- bis 6-gliedrigem Heteroaryl, OR^{j}, C(O)R^{j}, NR^{j}R^{k}, C(O)NR^{j}R^{k}, S(O)₂R^{j} und NR^{j}C(O)R^{k} ausgewählt ist, wobei jedes von R^{j} und R^{k} unabhängig H oder C₁-C₆-Alkyl ist, das optional mit einem oder mehreren Halogenen substituiert ist; oder -Q⁷-T⁷ Oxo ist; oder
R¹⁰ und R¹¹ zusammen mit dem Stickstoffatom, an das sie gebunden sind, ein 4- bis 12-gliedriges Heterocycloalkyl bilden, das 1-4 Heteroatome ausgewählt aus N, O und S enthält, das optional mit einem oder mehreren von Halogen, C₁-C₆-Alkyl, Hydroxyl oder C₁-C₆-Alkoxyl substituiert ist;
n 0, 1, 2, 3 oder 4 ist.

2. Verbindung zur Verwendung nach Anspruch 1, wobei R³ nicht H ist.

3. Verbindung zur Verwendung nach einem der vorhergehenden Ansprüche, wobei n 1 oder 2 ist; und/oder
wobei R¹ H oder CH₃ ist; und/oder
wobei n 1 oder 2 ist und mindestens eines von R⁷ -Q²-OR¹¹ ist, wobei R¹¹ -Q⁶-R^{S3} ist und Q⁶ ein optional substituierter C₂-C₆-Alkylen-, C₂-C₆-Alkenylen- oder C₂-C₆-Alkinylen-Linker ist; und/oder
wobei n 1 oder 2 ist und mindestens eines von R⁷ -Q²-NR¹⁰R¹¹ ist, wobei R¹¹ -Q⁶-R^{S3} ist; und/oder
wobei Q⁶ ein C₂-C₆-Alkylen-, C₂-C₆-Alkenylen- oder C₂-C₆-Alkinylen-Linker, der optional mit einem Hydroxyl substituiert ist, und R^{S3} 4- bis 7-gliedriges Heterocycloalkyl ist, das optional mit einem oder mehreren -Q⁷-T⁷ substituiert ist; und/oder
wobei Q⁶ ein C₁-C₆-Alkylen-, C₂-C₆-Alkenylen- oder C₂-C₆-Alkinylen-Linker ist, der optional mit einem Hydroxyl substituiert ist, und R^{S3} C₃-C₆-Cycloalkyl ist, das optional mit einem oder mehreren -Q⁷-T⁷ substituiert ist; und/oder
wobei jedes Q⁷ unabhängig eine Bindung oder ein C₁-C₃-Alkylen-, C₂-C₃-Alkenylen- oder C₂-C₃-Alkinylen-Linker ist und jedes T⁷ unabhängig H, Halogen, C₁-C₆-Alkyl oder Phenyl ist; und/oder
wobei Q² eine Bindung oder ein C₁-C₄-Alkylen-, C₂-C₄-Alkenylen- oder C₂-C₄-Alkinylen-Linker ist; und/oder
wobei mindestens eines von R⁷ Folgendes ist:

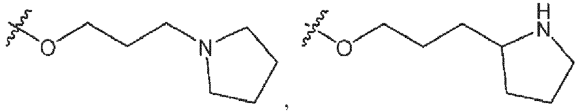

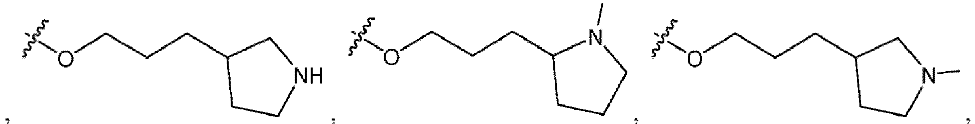

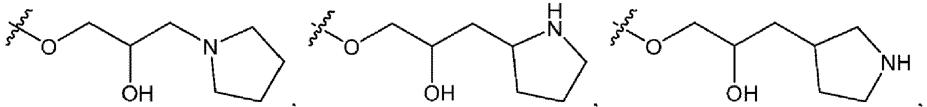

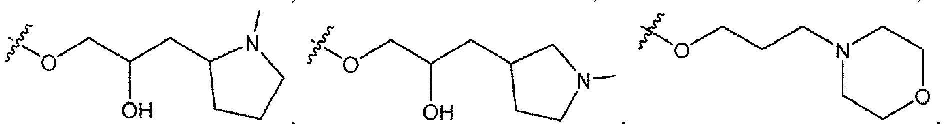

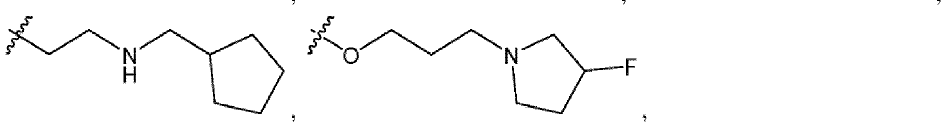

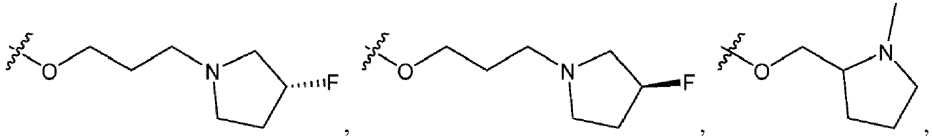

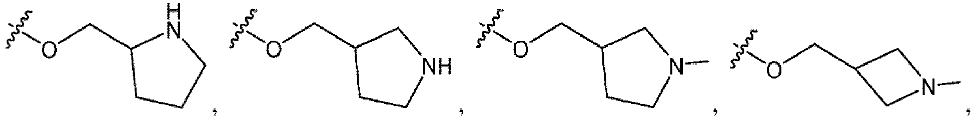

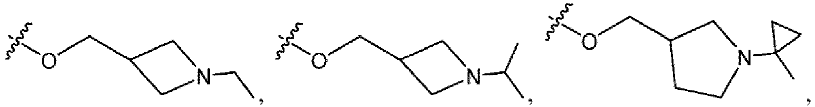

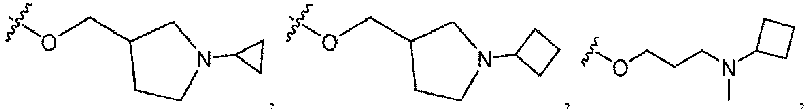

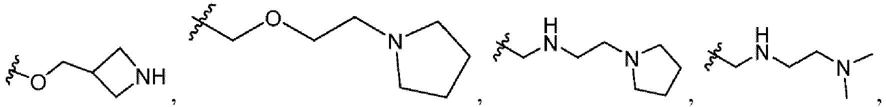

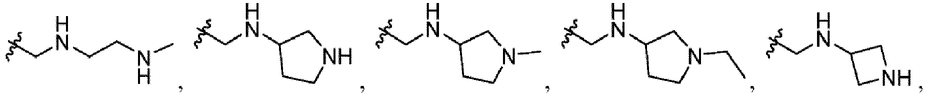

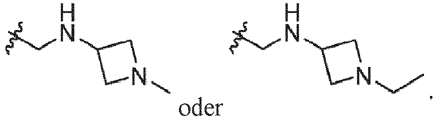

4. Verbindung zur Verwendung nach einem der vorhergehenden Ansprüche, wobei n 2 ist und die Verbindung ferner ein weiteres R⁷ umfasst, das aus Halogen und Methoxy ausgewählt ist.

5. Verbindung zur Verwendung nach Anspruch 1 der Formel (VI): 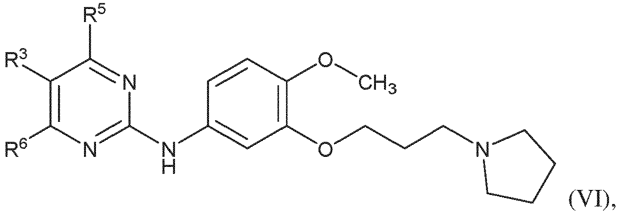 wobei
R⁵ aus der Gruppe bestehend aus C₁-C₆-Alkyl und NR⁸R⁹ ausgewählt ist; und R⁶ NR⁸R⁹ ist.

6. Verbindung zur Verwendung nach einem der vorhergehenden Ansprüche mit der Formel (VIIIa): 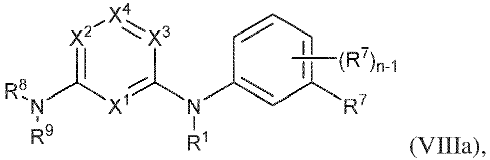 wobei
X¹ N ist;
X² CR³ ist;
X³ N ist;
X⁴ CR⁵ ist;
R³ H ist; und
R⁵ unabhängig aus der Gruppe bestehend aus Cs-Cs-Cycloalkyl und C₁-C₆-Alkyl, das optional mit einem oder mehreren von Halogen oder OR^{a} substituiert ist, ausgewählt ist; oder
R⁵ und R³ zusammen mit den Atomen, an die sie gebunden sind, Phenyl oder ein 5- oder 6- gliedriges Heteroaryl bilden.

7. Verbindung zur Verwendung nach einem der vorhergehenden Ansprüche mit der Formel (VIIIb): 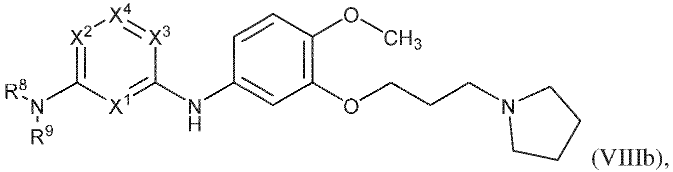 wobei
X¹ N ist;
X² CR³ ist;
X³ N ist;
X⁴ CR⁵ ist,
R⁵ aus der Gruppe bestehend aus Cs-Cs-Cycloalkyl und C₁-C₆-Alkyl ausgewählt ist; oder
R⁵ und R³ zusammen mit den Atomen, an die sie gebunden sind, Phenyl oder ein 5- oder 6- gliedriges Heteroaryl bilden.

8. Verbindung zur Verwendung nach einem der vorhergehenden Ansprüche mit der Formel (VIIIc): 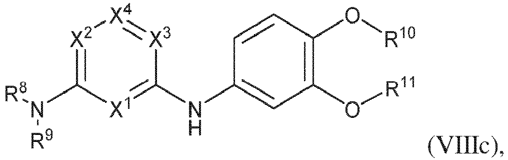 wobei
X¹ N ist;
X² CR³ ist;
X³ N ist;
X⁴ CR⁵ ist,
R⁵ aus der Gruppe bestehend aus Cs-Cs-Cycloalkyl und C₁-C₆-Alkyl ausgewählt ist; oder
R⁵ und R³ zusammen mit den Atomen, an die sie gebunden sind, Phenyl oder ein 5- oder 6- gliedriges Heteroaryl bilden.

9. Verbindung zur Verwendung nach einem der vorhergehenden Ansprüche, wobei C₁₋₆-Alkyl ist oder R⁵ und R³ zusammen mit den Atomen, an die sie gebunden sind, Phenyl oder einen 5- bis 6-gliedrigen Heteroarylring bilden.

10. Verbindung zur Verwendung nach einem der vorhergehenden Ansprüche, wobei die Verbindung aus den folgenden Verbindungen und pharmazeutisch verträglichen Salzen davon ausgewählt ist:
| Verbindung Nr. | Struktur |
|---|---|
| 186 | 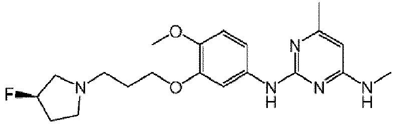 |
| 205 | 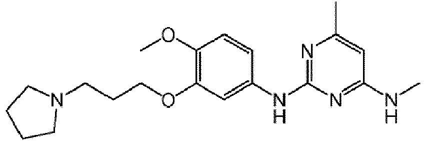 |
| 232 | 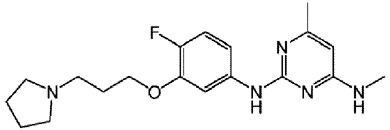 |
| 236 | 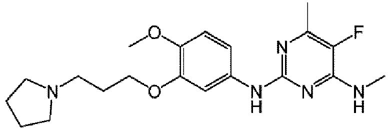 |
| 238 | 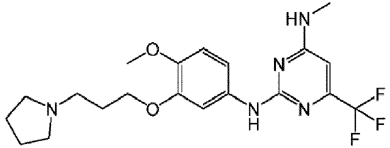 |
| 240 | 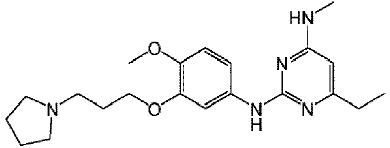 |
| 261 | 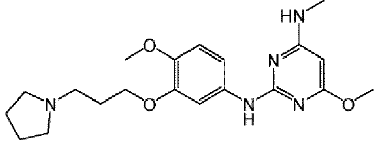 |
| 263 | 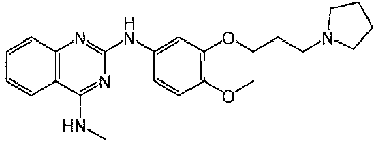 |
| 301 | 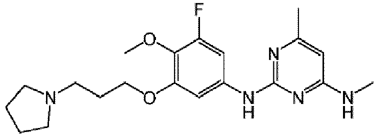 |
| 302 | 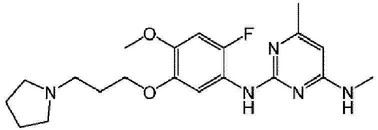 |
| 311 | 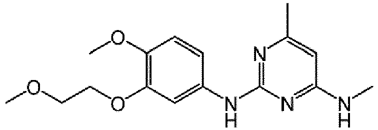 |
| 312 | 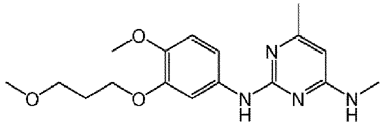 |
| 313 | 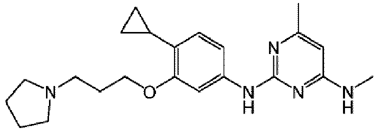 |
| 316 | 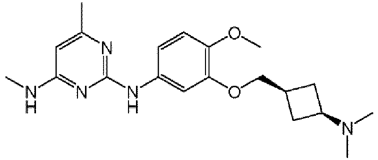 |
| 317 | 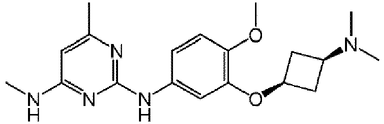 |
| 318 | 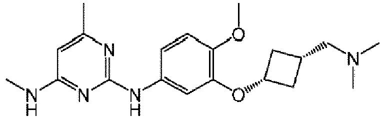 |
| 319 | 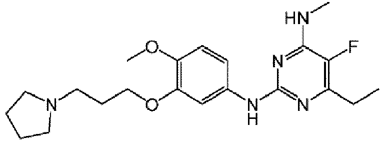 |
| 320 | 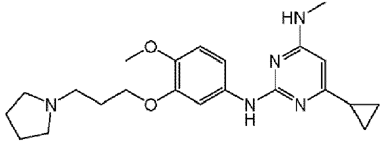 |
| 328 | 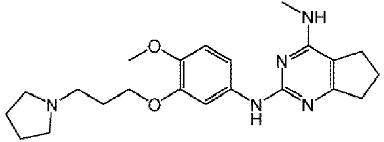 |
| 329 | 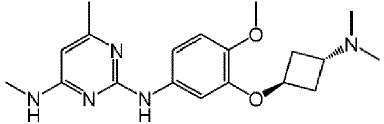 |
| 330 | 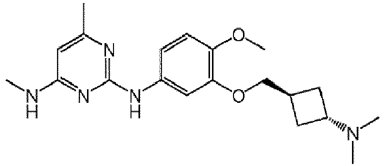 |
| 331 | 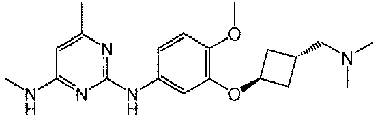 |
| 334 | 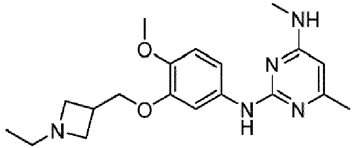 |
| 337 | 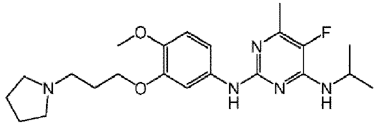 |
| 356 | 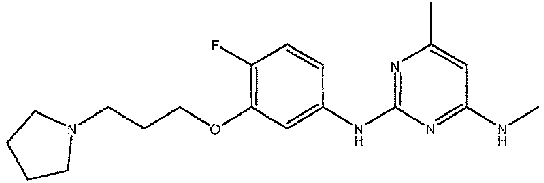 |
| 360 | 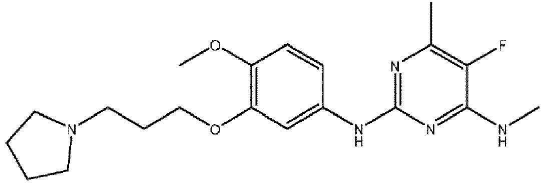 |
| 362 | 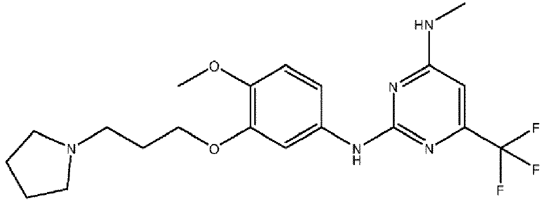 |
| 364 | 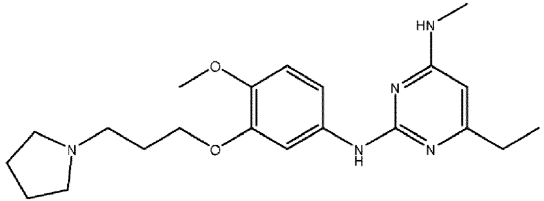 |
| 380 | 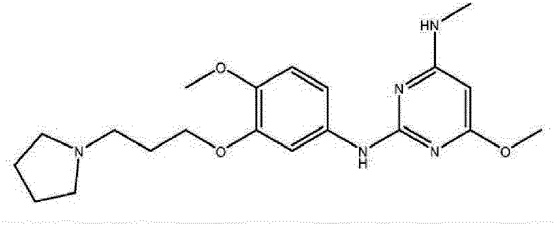 |
| 384 | 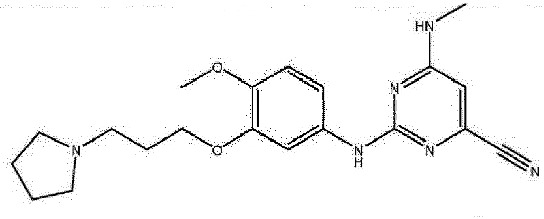 |
| 385 | 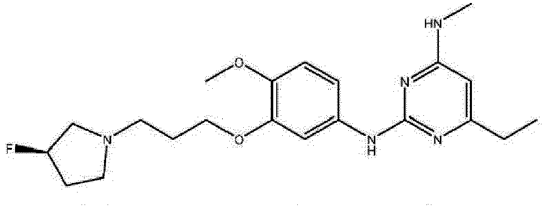 |
| 391 | 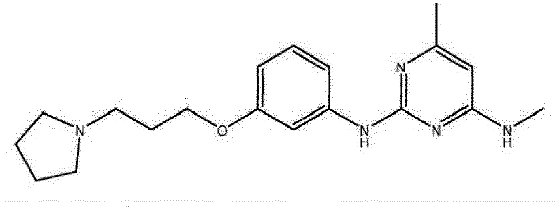 |
| 413 | 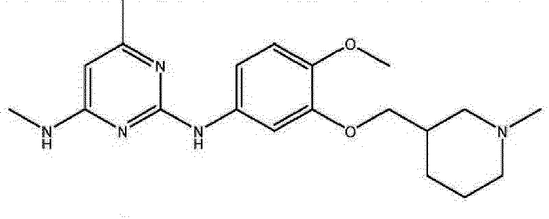 |
| 417 | 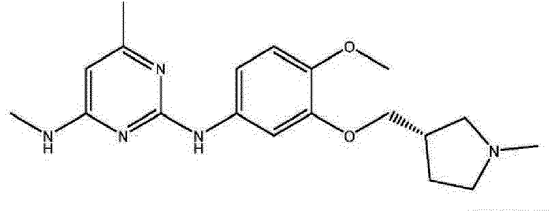 |
| 418 | 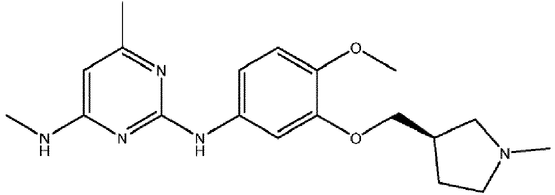 |
| 419 | 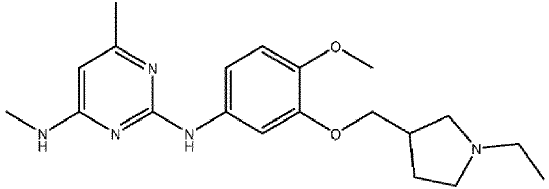 |
| 421 | 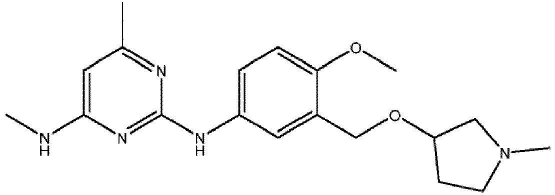 |
| 428 | 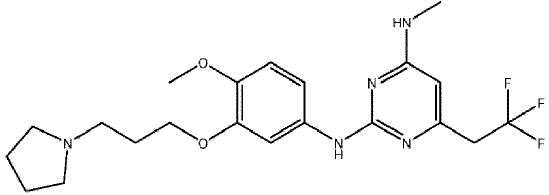 |
| 432 | 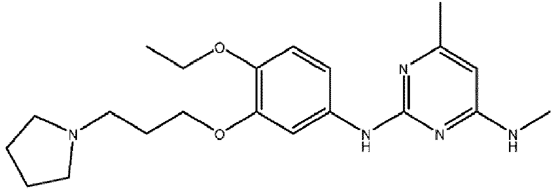 |
| 435 | 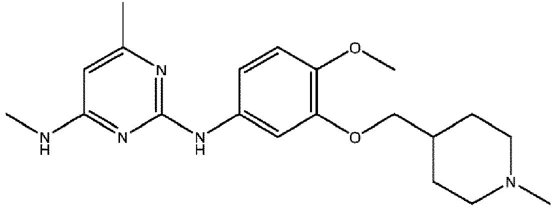 |
| 436 | 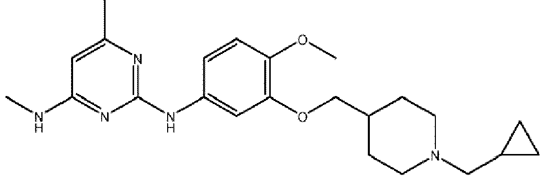 |
| 437 | 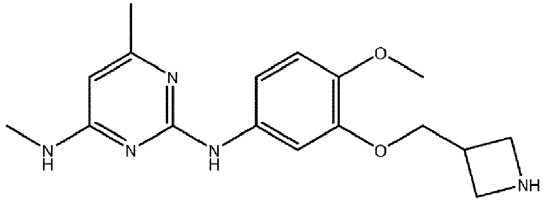 |
| 441 | 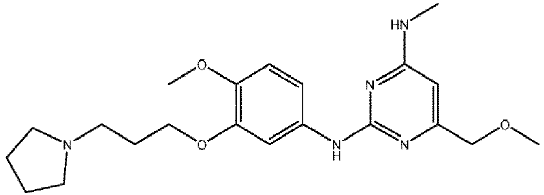 |
| 442 | 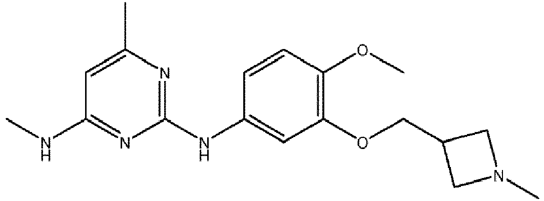 |
| 443 | 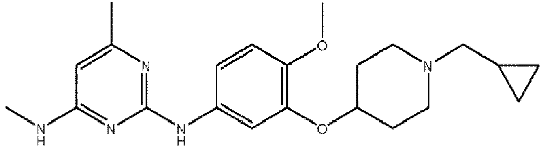 |
| 444 | 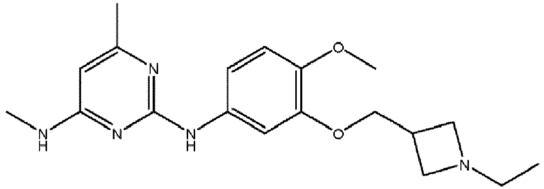 |
| 445 | 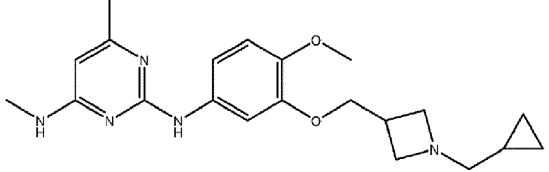 |
| 446 | 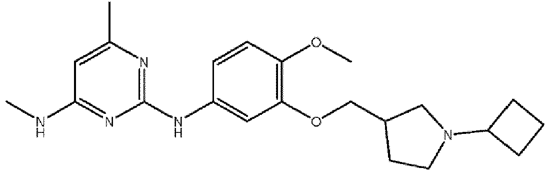 |
| 449 | 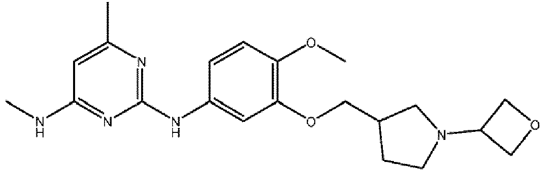 |
| 450 | 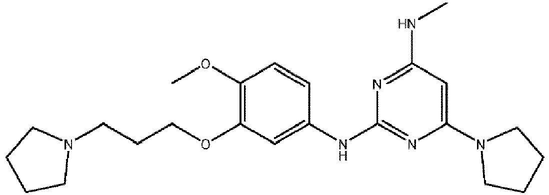 |
| 452 | 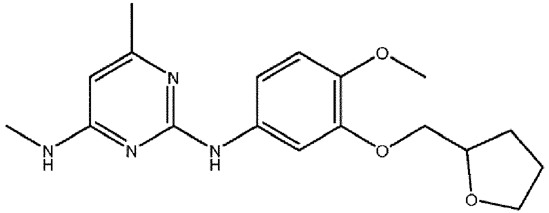 |
| 455 | 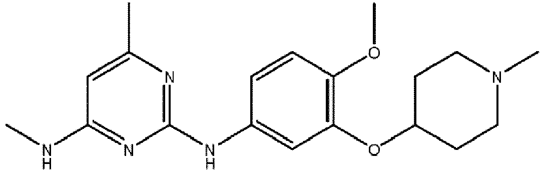 |
| 456 | 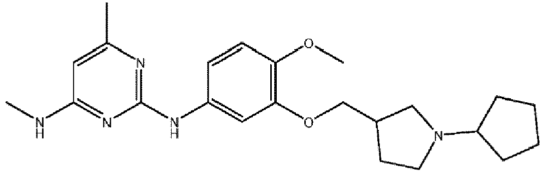 |
| 457 | 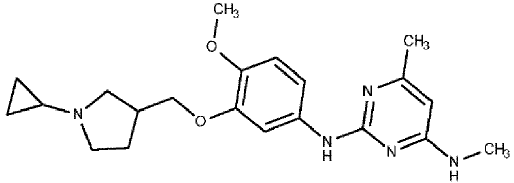 |
| 458 | 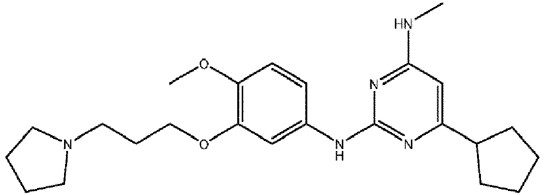 |
| 459 | 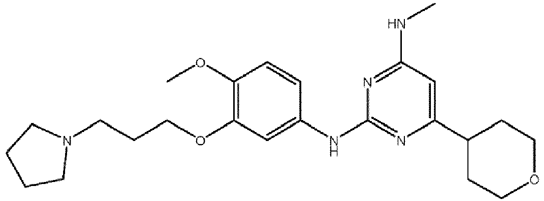 |
| 461 | 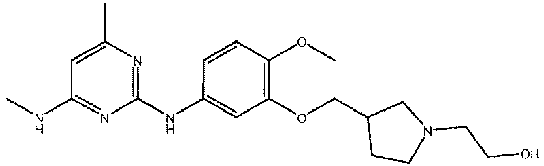 |
| 463 | 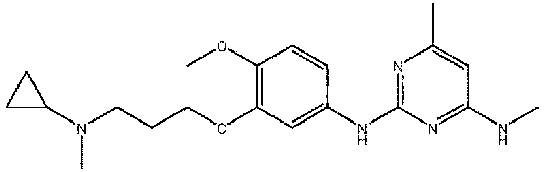 |
| 465 | 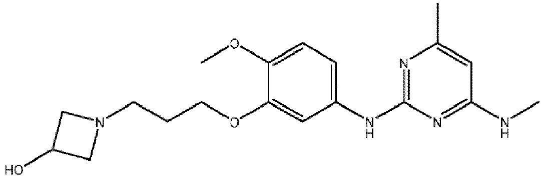 |
| 466 | 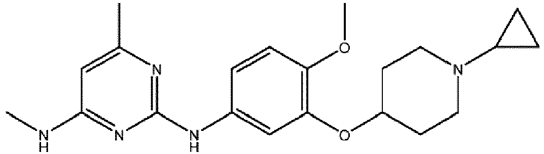 |
| 468 | 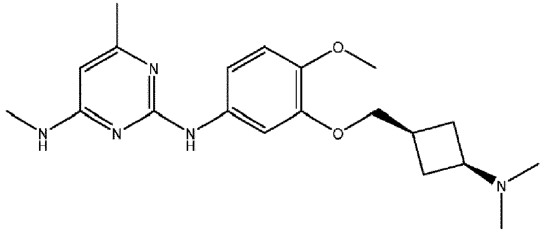 |
| 470 | 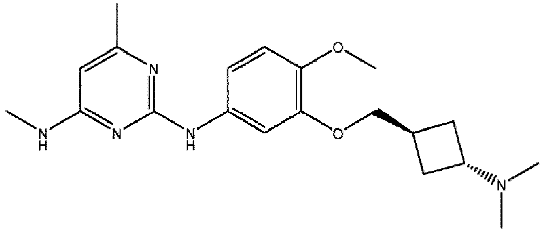 |
| 481 | 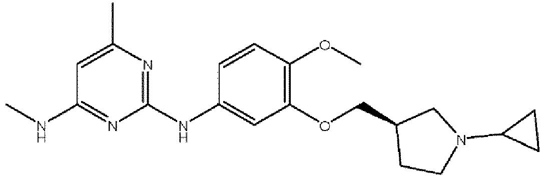 |
| 482 | 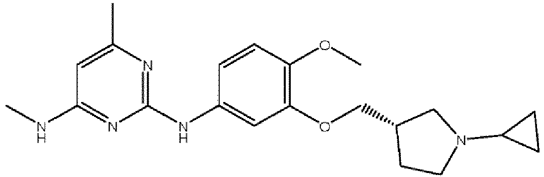 |
| 483 | 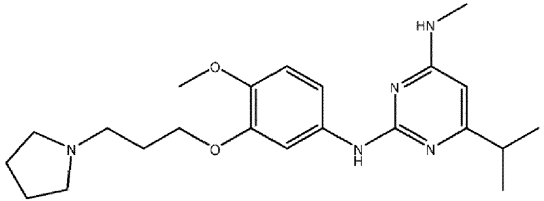 |
| 484 | 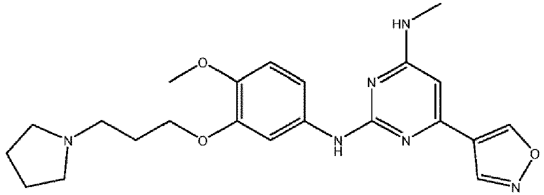 |
| 487 | 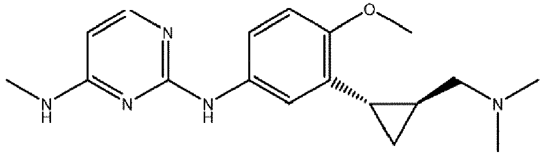 |
| 488 | 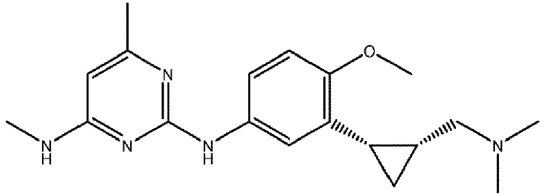 |
| 489 | 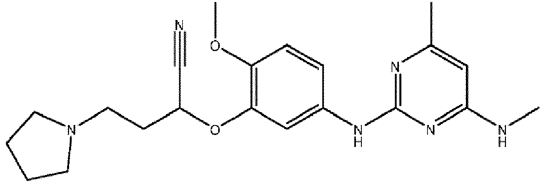 |
| 490 | 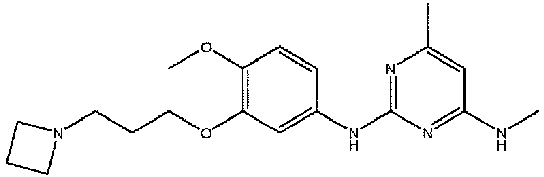 |
| 491 | 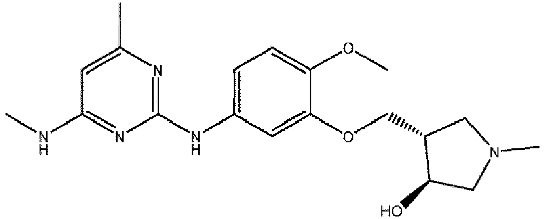 |
| 492 | 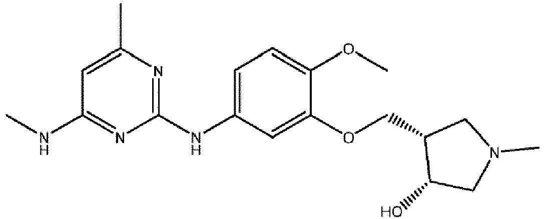 |
| 493 | 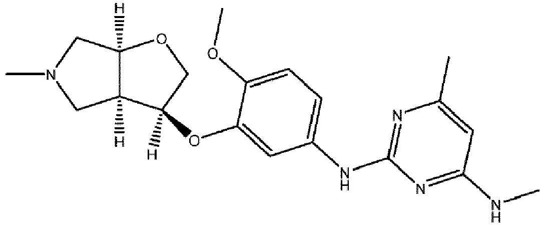 |
| 496 | 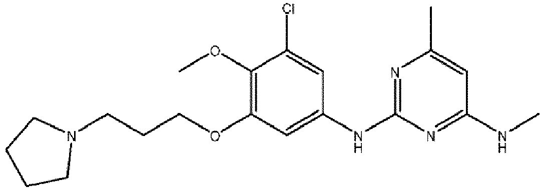 |
| 497 | 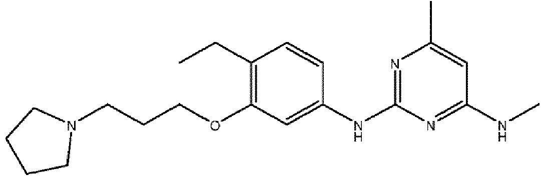 |
| 498 | 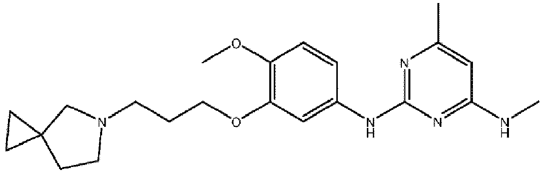 |
| 502 | 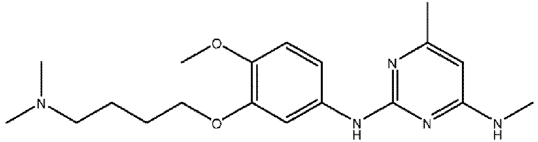 |
| 503 | 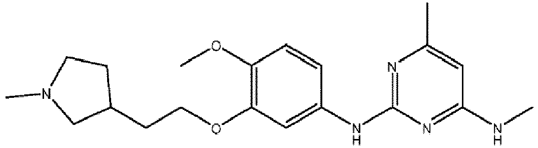 |
| 504 | 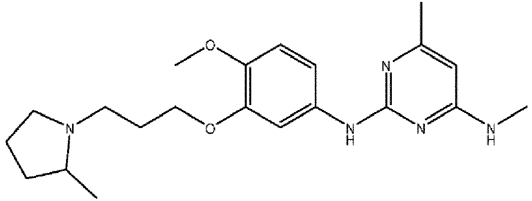 |
| 505 | 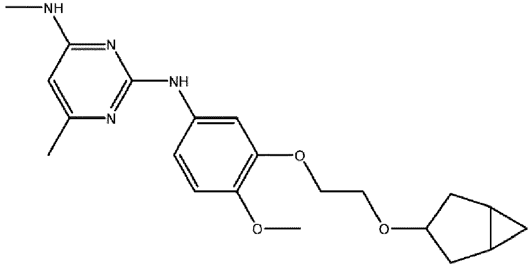 |
| 506 | 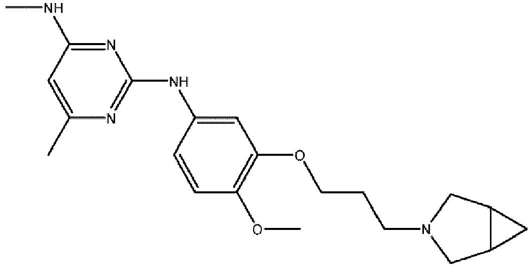 |
| 508 | 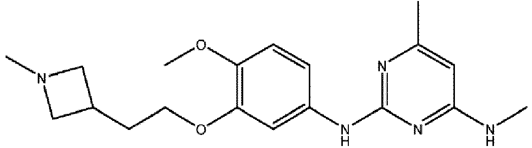 |
| 510 | 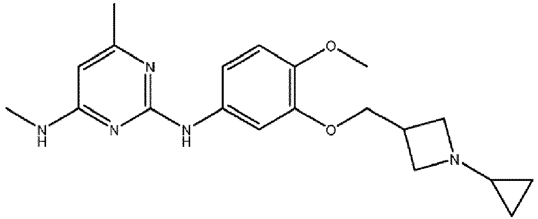 |
| 517a | 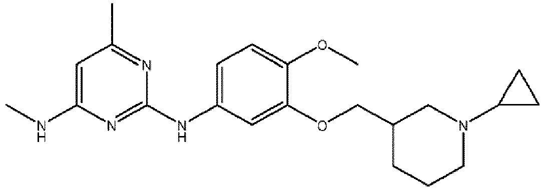 |
| 517b | 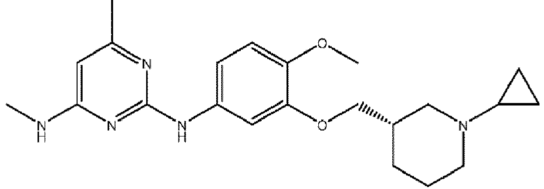 |
| 551 | 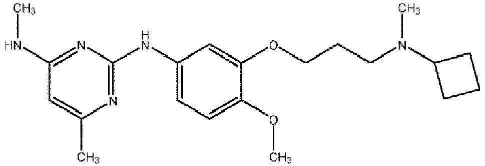 |
| 559 | 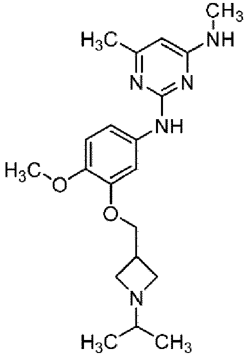 |
| 560 | 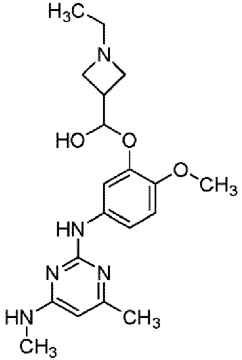 |
| 561 | 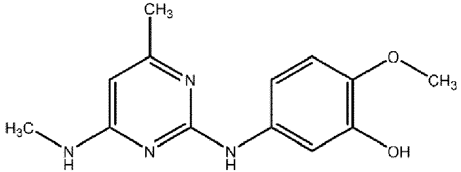 |
| 562 | 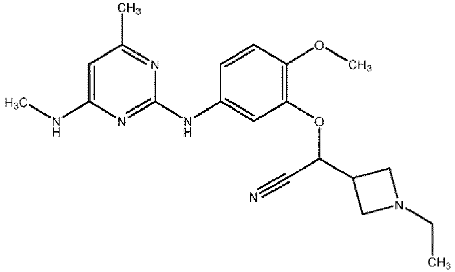 |
| 563 | 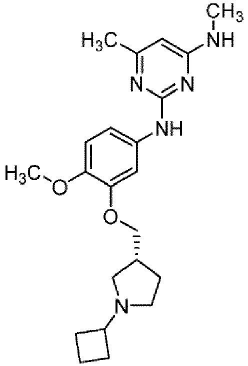 |
| 568 | 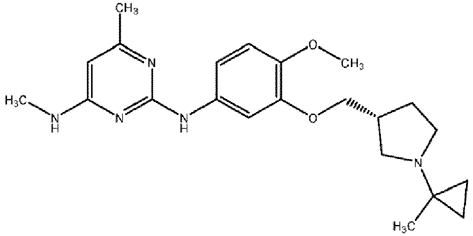 |
| 570 | 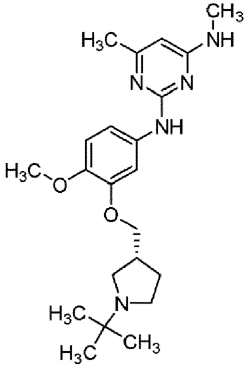 |
| 571 | 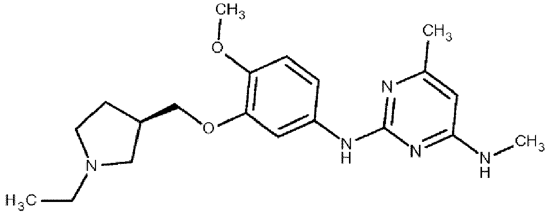 |
| 574 | 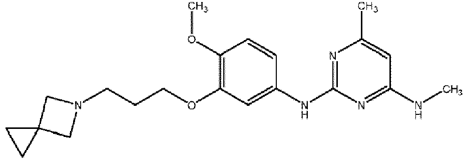 |
| 575 | 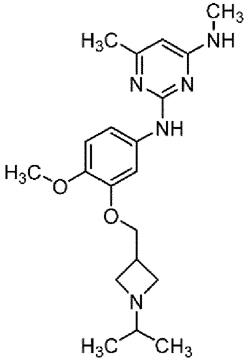 |
| 576 | 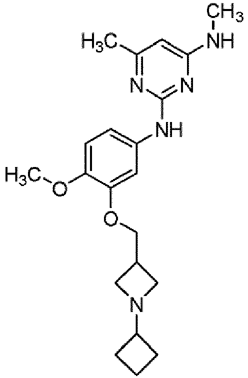 |
| 577 | 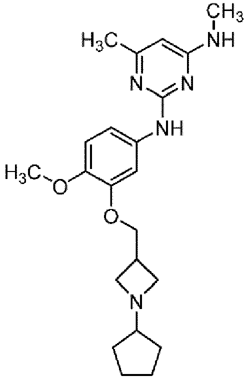 |
| 580 | 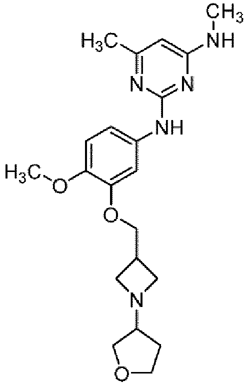 |
| 600 | 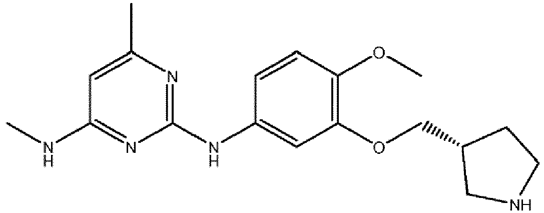 |
| 602 | 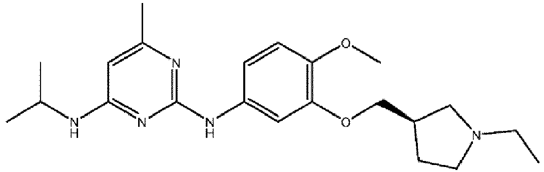 |
| 603 | 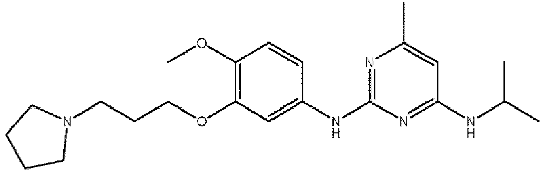 |
| 606 | 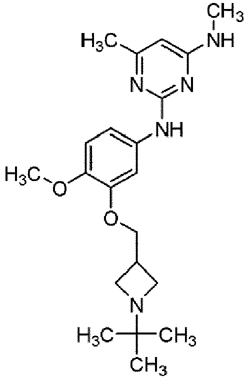 |
| 610 | 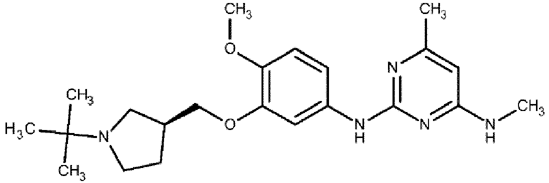 |
| 614 | 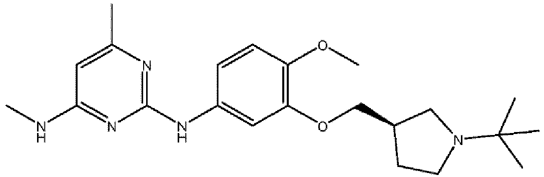 |
| 624 | 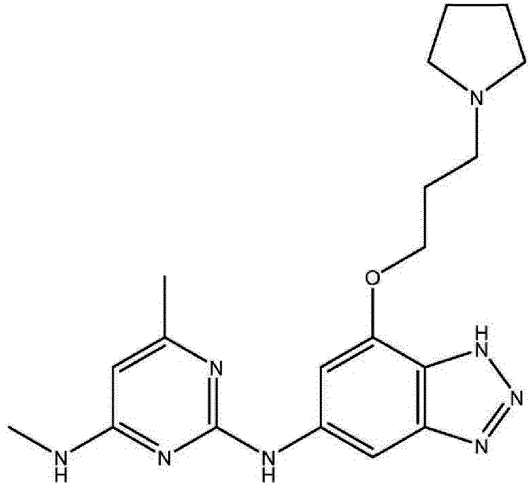 |
| 644 | 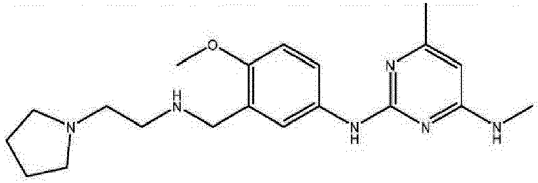 |
| 645 | 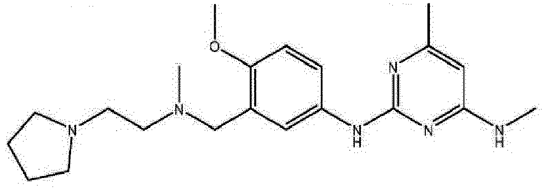 |
| 646 | 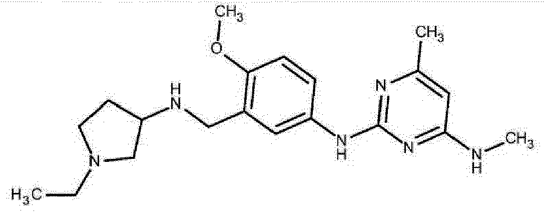 |
| 647 | 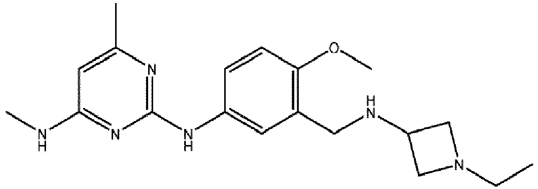 |
| 649 | 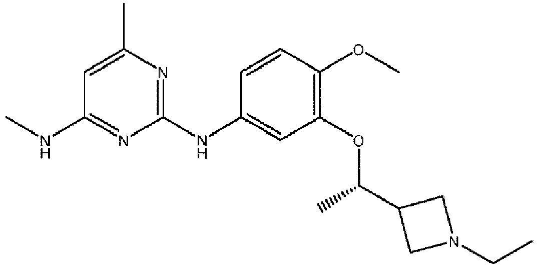 |
| 650 | 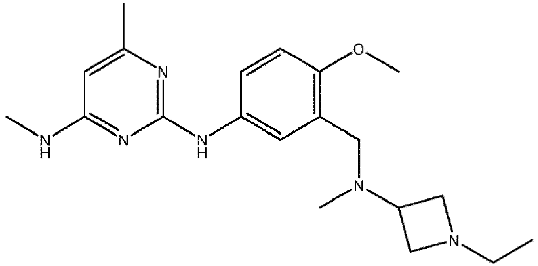 |
| 651 | 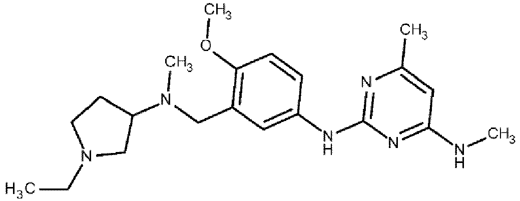 |
| 671 | 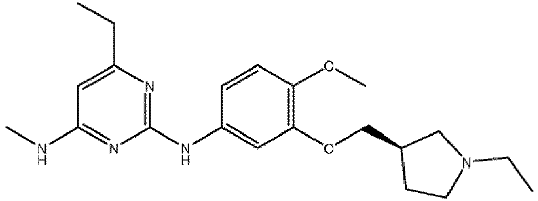 |
| 672 | 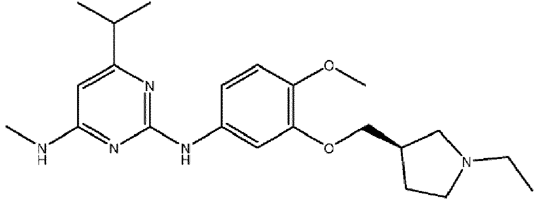 |
| 680 | 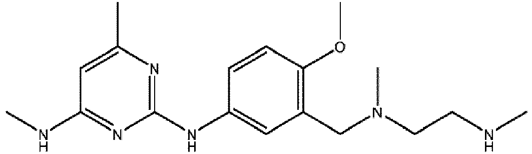 |
| 687 | 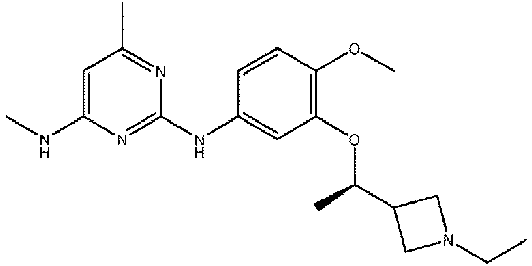 |
| 765 | 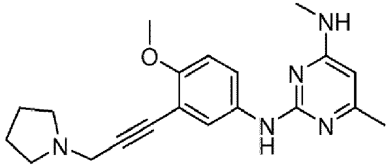 |
| 786 | 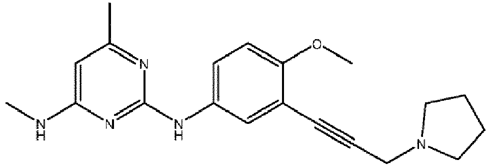 |
| 791 | 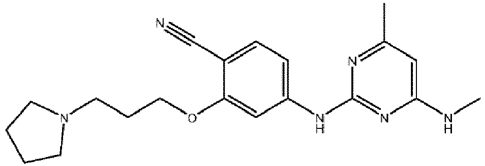 |
| 793 | 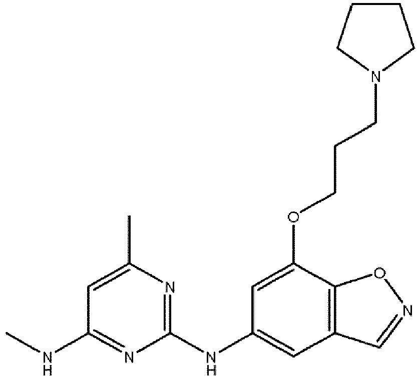 |
| 799 | 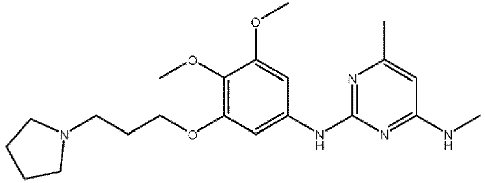 |
| 800 | 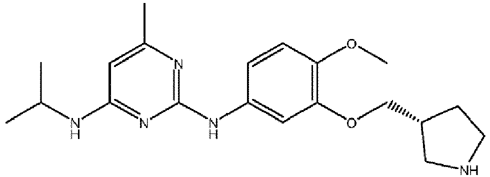 |
| 813 | 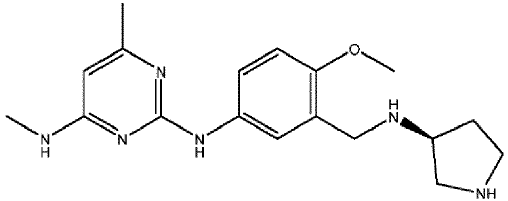 |
| 814 | 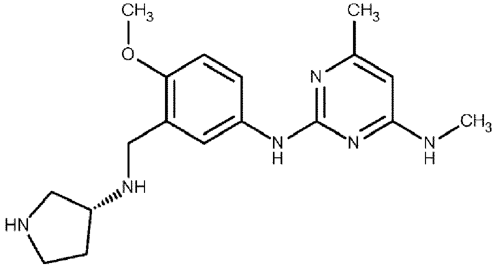 |
| 845 | 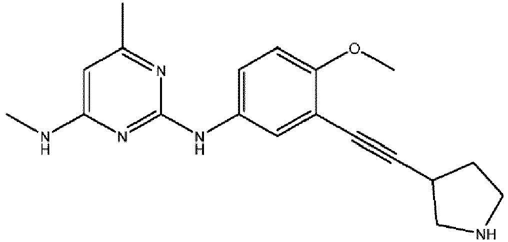 |
| 846 | 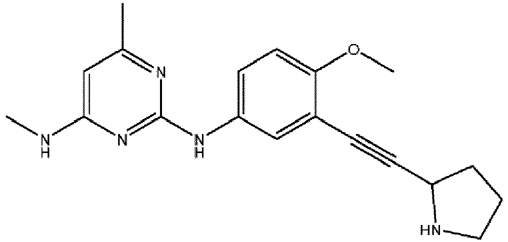 |
| 847 | 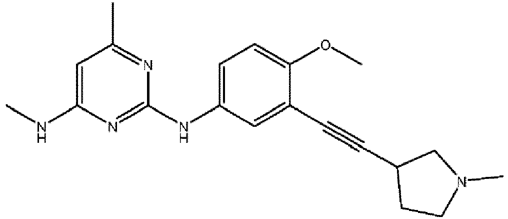 |
| 848 | 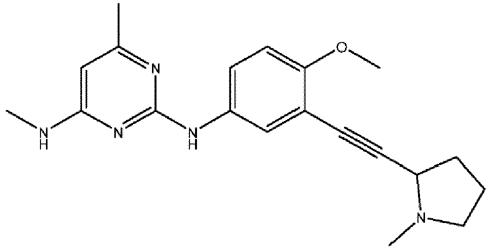 |
| 855 | 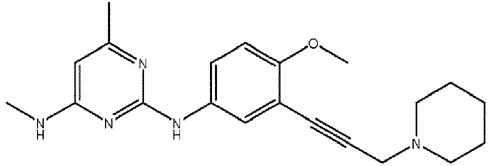 |
| 860 | 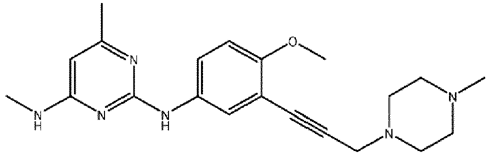 |
| 871 | 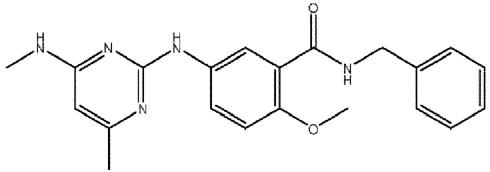 |
| 872 | 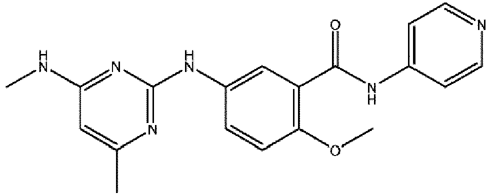 |
| 873 | 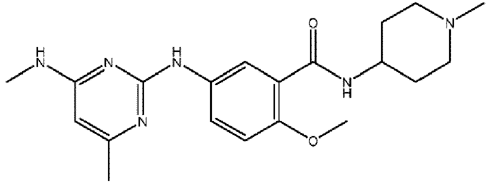 |
| 874 | 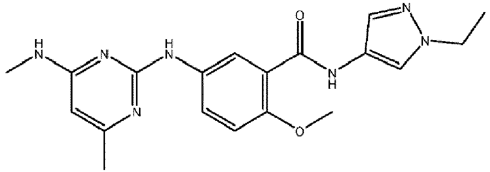 |
| 876 | 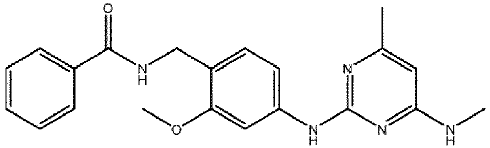 |
| 877 | 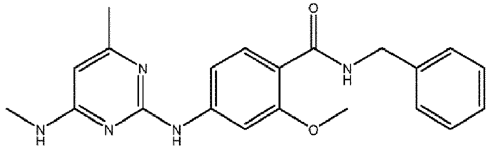 |
| 878 | 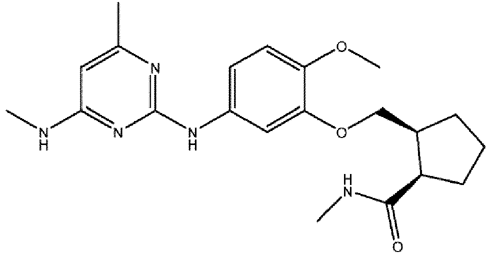 |
| 879 | 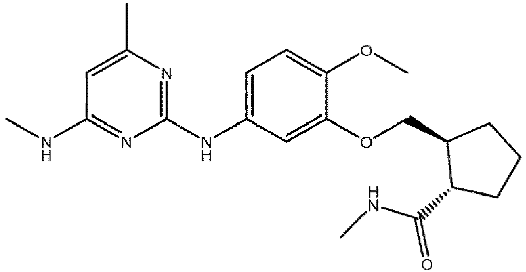 |
| 890 | 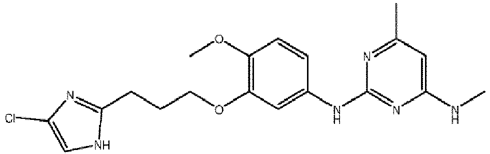 |
| 891 | 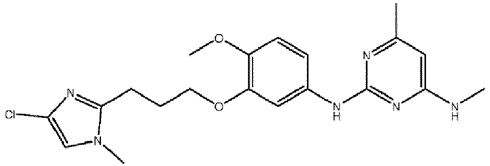 |
| 904 | 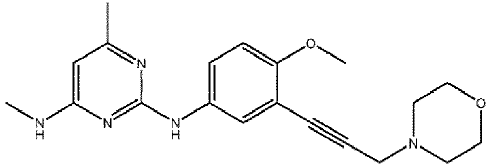 |
| 905 | 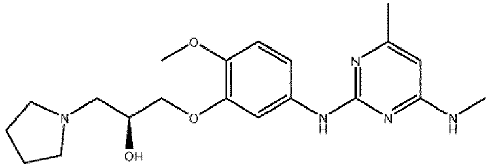 |
| 906 | 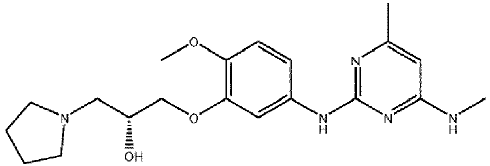 |
| 962 | 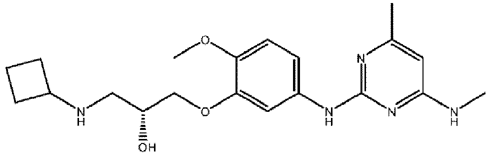 |
| 964 | 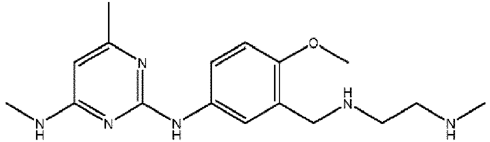 |
| 965 | 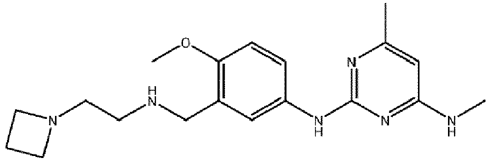 |
| 966 | 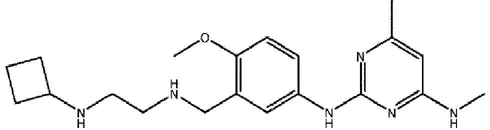 |
| 967 | 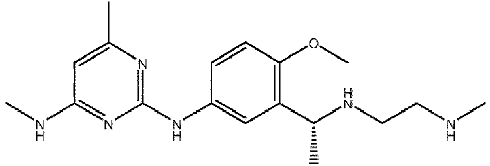 |
| 968 | 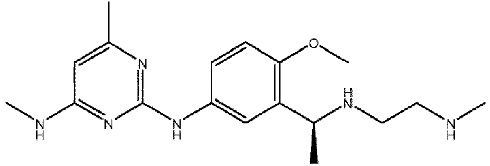 |
| 969 | 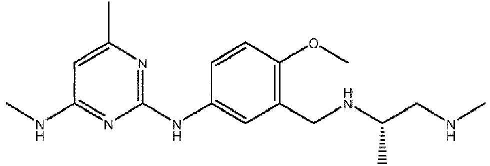 |
| 970 | 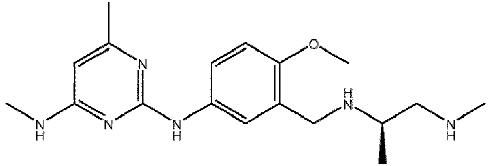 |
| 971 | 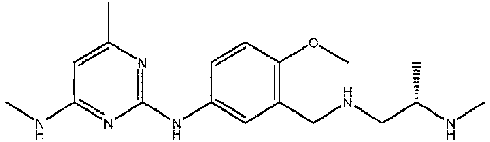 |
| 972 | 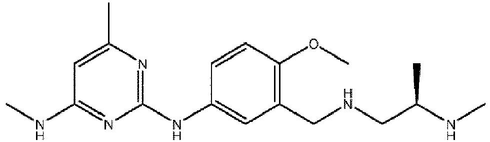 |
| 977 | 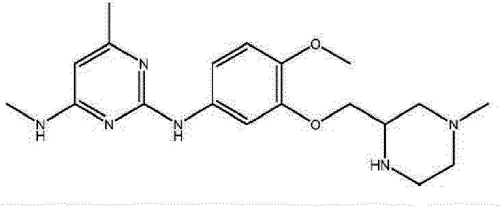 |
| 985 | 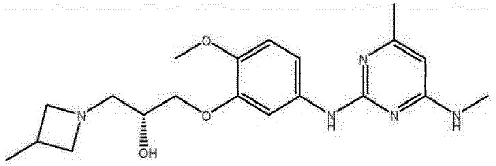 |
| 986 | 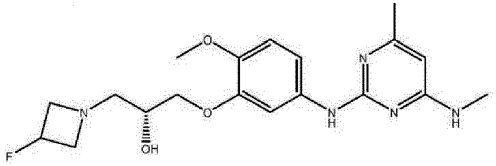 |
| 989 | 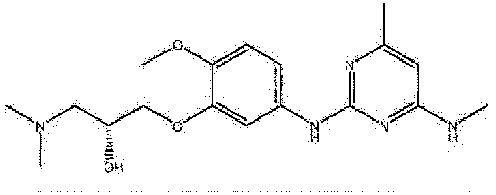 |
| 990 | 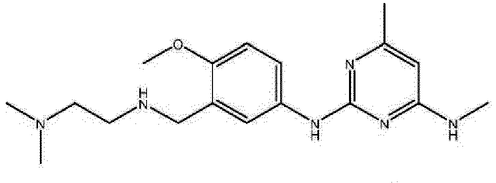 |
| 991 | 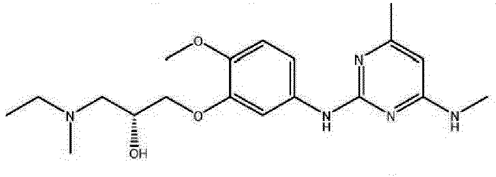 |
| 992 | 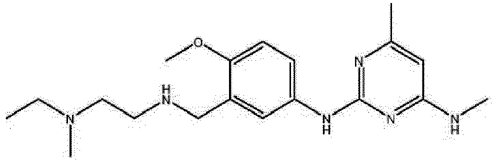 |
| 993 | 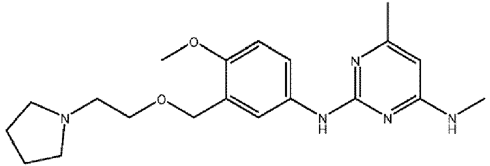 |
| 994 | 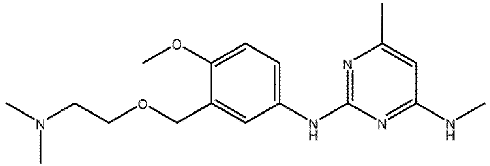 |
| 997 | 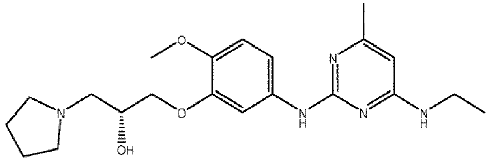 |
| 998 | 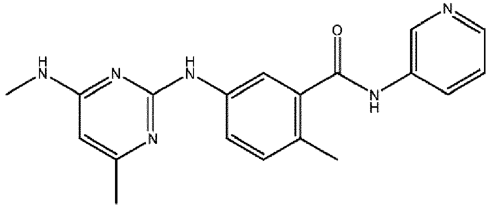 |
| 999 | 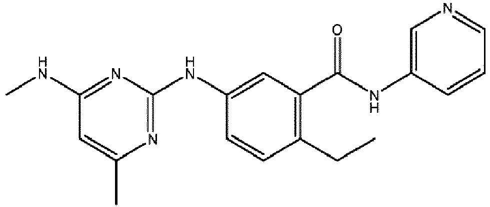 |
| 1000 | 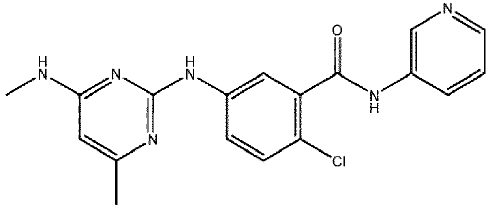 |
| 1001 | 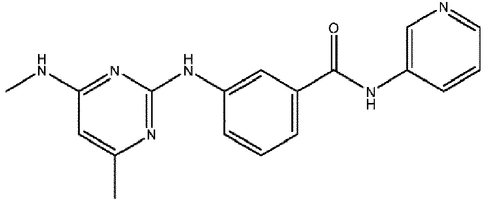 |
| 1004 | 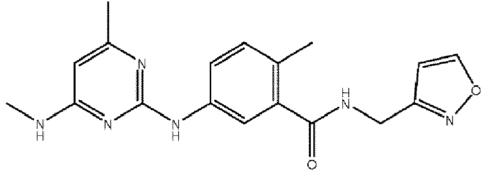 |
| 1029 | 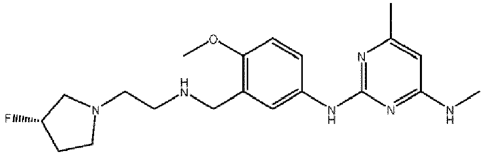 |
| 1030 | 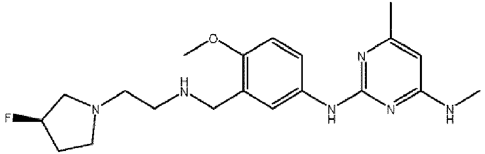 |
| 1036 | 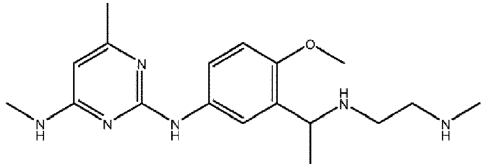 |
| 1037 | 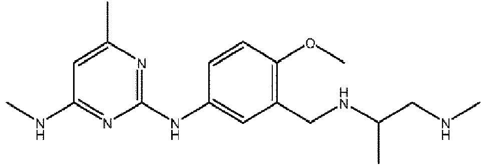 |
| 1038 | 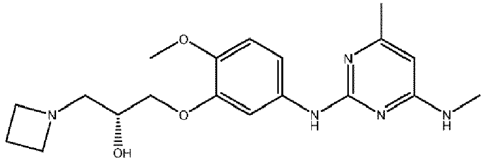 |
| 1040 | 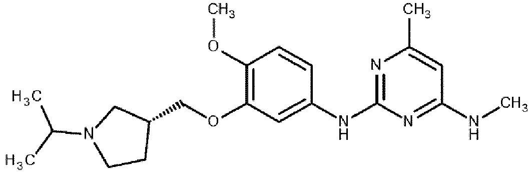 |
| 1044 | 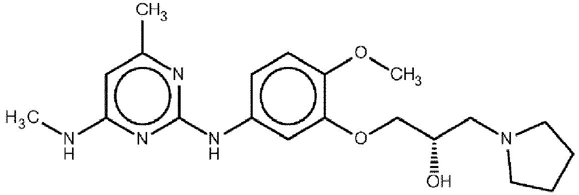 |
| 1045 | 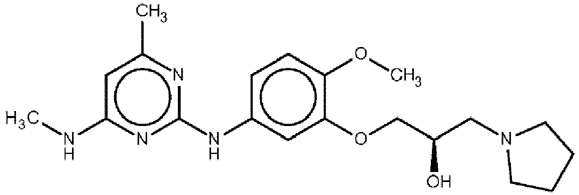 |
| 1078 | 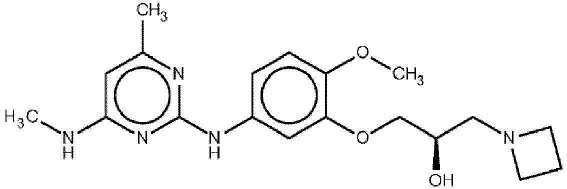 |
| 1079 | 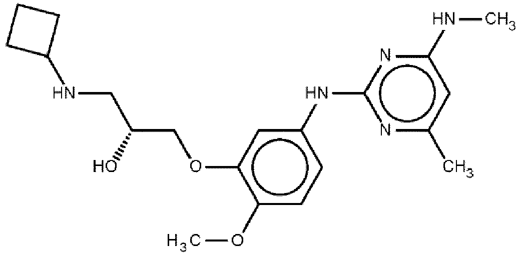 |
| 1080 | 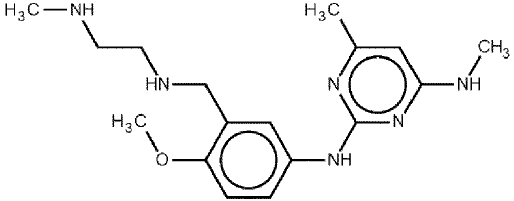 |
| 1081 | 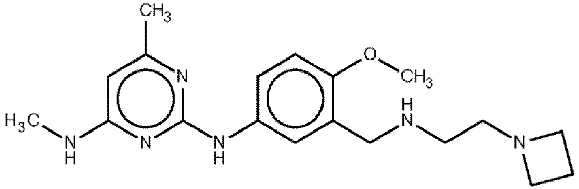 |
| 1090 | 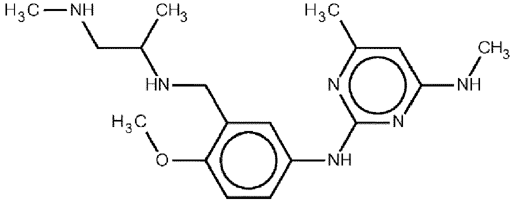 |
| 1094 | 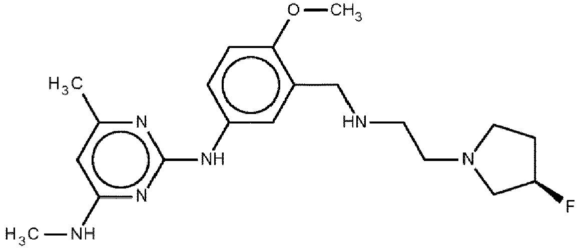 |
| 1095 | 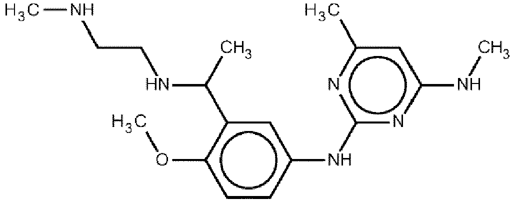 |
| 1099 | 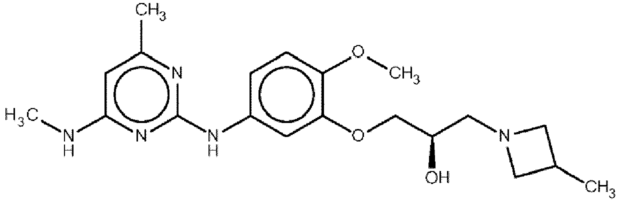 |
| 1100 | 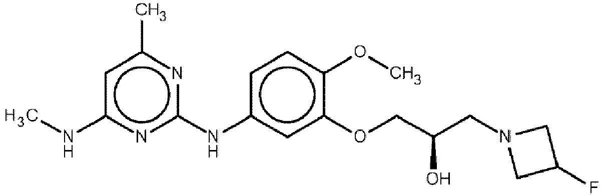 |
| 1101 | 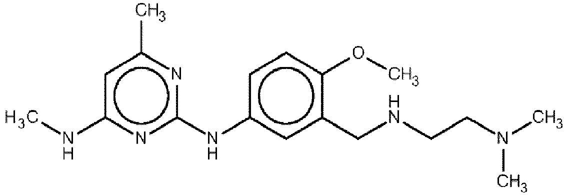 |
| 1102 | 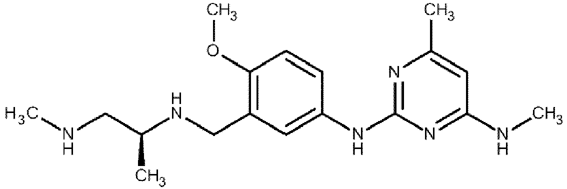 |
| 1103 | 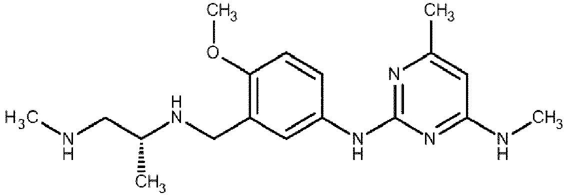 |
| 1104 | 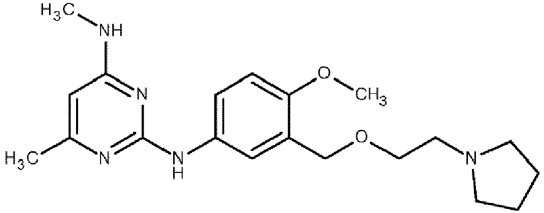 |
| 1105 | 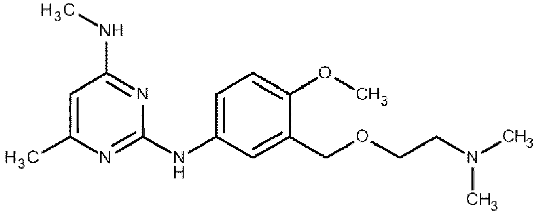 |
| 1106 | 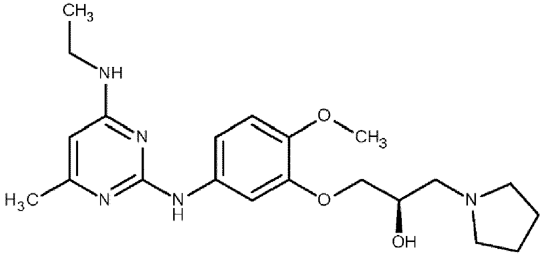 |
| 1109 | 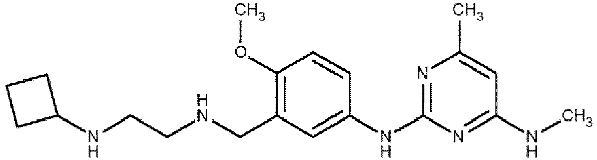 |
| 1112 | 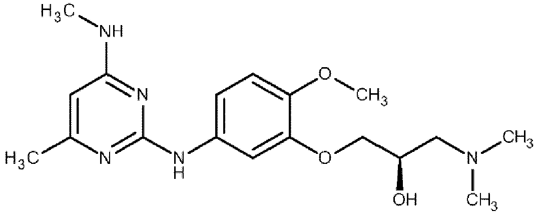 |
| 1113 | 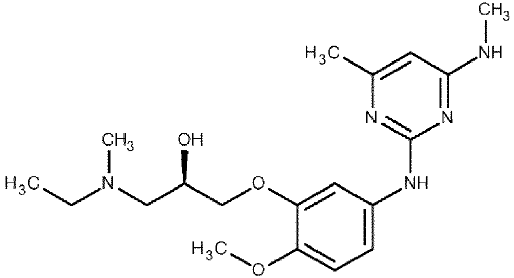 |
| 1114 | 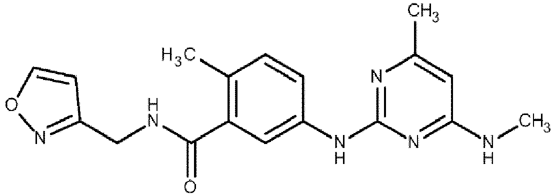 |
| 1117 | 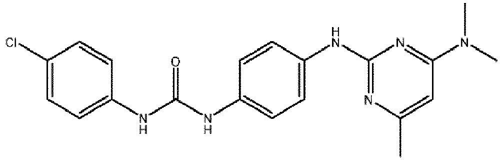 |

11. Verbindung zur Verwendung nach einem der Ansprüche 1-10, wobei die Blutkrankheit Sichelzellenanämie oder β-Thalassämie ist.

12. Verbindung zur Verwendung nach einem der Ansprüche 1-10, wobei die Blutkrankheit ein hämatologischer Krebs ist.

13. Verbindung zur Verwendung nach Anspruch 12, wobei der hämatologische Krebs akute myeloische Leukämie (AML) oder chronische lymphatische Leukämie (CLL) ist.

14. Verbindung zur Verwendung nach einem der vorhergehenden Ansprüche, wobei die Verbindung ein selektiver Inhibitor von EHMT2 ist.

## Revendications

1. Composé de formule (IIa1) destiné à être utilisé dans le traitement ou la prévention d'un trouble sanguin : 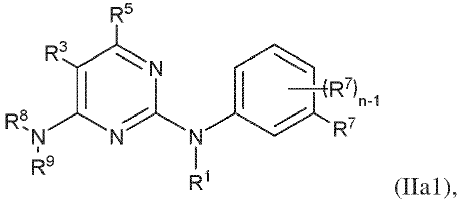 ou tautomère de celui-ci, ou sel pharmaceutiquement acceptable du composé ou du tautomère,
dans laquelle
R¹ représente un atome H ou un groupe C₁-C₄ alkyle ;
R³ est choisi dans le groupe constitué par un atome H, les groupes halogéno, cyano, C₁-C₆ alcoxy, C₆-C₁₀ aryle, NR^{a}C(O)R^{b}, C₃-C₈ cycloalkyle, hétérocycloalkyle de 4 à 7 chaînons, hétéroaryle de 5 à 6 chaînons, et C₁-C₆ alkyle, lesdits groupes C₁-C₆ alcoxy et C₁-C₆ alkyle étant éventuellement substitués par un ou plusieurs des groupes halogéno, OR^{a} ou NR^{a}R^{b}, dans lesquels chacun des groupes R^{a} et R^{b} représente indépendamment un atome H ou un groupe C₁-C₆ alkyle, ou R³ représente un groupe -Q¹-T¹, dans lequel Q¹ représente une liaison ou un groupe de liaison C₁-C₆ alkylène, C₂-C₆ alcénylène ou C₂-C₆ alcynylène éventuellement substitué par un ou plusieurs des groupes halogéno, cyano, hydroxy, oxo ou C₁-C₆ alcoxy, et T¹ représente un atome H, un groupe halogéno, cyano, NR⁸R⁹, C(O)NR⁸R⁹, OR⁸, OR⁹ ou R^{S1}, dans lequel R^{S1} représente un groupe C₃-C₈ cycloalkyle, phényle, hétérocycloalkyle de 4 à 12 chaînons contenant 1 à 4 hétéroatomes choisis parmi les atomes N, O et S, ou un groupe hétéroaryle de 5 à 6 chaînons et R^{S1} est éventuellement substitué par un ou plusieurs des groupes halogéno, C₁-C₆ alkyle, hydroxy, oxo, -C(O)R⁹, -SO₂R⁸, -SO₂N(R⁸)₂, -NR⁸C(O)R⁹, amino, mono- ou di- alkylamino, ou C₁-C₆ alcoxy ;
R⁵ est choisi dans le groupe constitué par un atome F, Br, les groupes cyano, C₁-C₆ alcoxy, C₆-C₁₀ aryle, NR^{a}R^{b}, C(O)NR^{a}R^{b}, NR^{a}C(O)R^{b}, C₃-C₈ cycloalkyle, hétérocycloalkyle de 4 à 12 chaînons contenant 1 à 4 hétéroatomes choisis parmi les atomes N, O, et S, C₁-C₆ alkyle éventuellement substitué par un ou plusieurs des groupes halogéno, OR^{a} ou NR^{a}R^{b}, et C₂-C₆ alcynyle éventuellement substitué par un groupe hétérocycloalkyle de 4 à 12 chaînons ; lesdits groupes C₃-C₈ cycloalkyle ou hétérocycloalkyle de 4 à 12 chaînons étant éventuellement substitués par un ou plusieurs des groupes halogéno, C(O)R^{a}, OR^{a}, NR^{a}R^{b}, hétérocycloalkyle de 4 à 7 chaînons, -C₁-C₆ alkylène-hétérocycloalkyle de 4 à 7 chaînons, ou C₁-C₄ alkyle éventuellement substitué par un plusieurs des groupes halogéno, OR^{a} ou NR^{a}R^{b}, dans lesquels chacun des groupes R^{a} et R^{b} représente indépendamment un atome H ou un groupe C₁-C₆ alkyle ; ou
R⁵ et R³ conjointement avec les atomes auxquels ils sont liés forment un groupe phényle ou un groupe hétéroaryle de 5 ou 6 chaînons ;
chaque groupe R⁷ représente indépendamment un groupe oxo (=O) ou -Q²-T², dans lequel chaque groupe Q² représente indépendamment une liaison ou un groupe de liaison C₁-C₆ alkylène, C₂-C₆ alcénylène ou C₂-C₆ alcynylène éventuellement substitué par un ou plusieurs des groupes halogéno, cyano, hydroxy, amino, mono- ou di- alkylamino, ou C₁-C₆ alcoxy, et chaque groupe T² représente indépendamment un atome H, un groupe halogéno, cyano, OR¹⁰, OR¹¹, C(O)R¹¹, NR¹⁰R¹¹, C(O)NR¹⁰R¹¹, NR¹⁰C(O)R¹¹, hétéroaryle de 5 à 10 chaînons, C₃-C₈ cycloalkyle, ou hétérocycloalkyle de 4 à 12 chaînons contenant 1 à 4 hétéroatomes choisis parmi les atomes N, O et S, et ledit groupe hétéroaryle de 5 à 10 chaînons, C₃-C₈ cycloalkyle, ou hétérocycloalkyle de 4 à 12 chaînons étant éventuellement substitué par un ou plusieurs des groupes halogéno, C₁-C₆ alkyle éventuellement substitué par un groupe NR^{x}R^{y}, hydroxy, oxo, N(R⁸)₂, cyano, C₁-C₆ halogénoalkyle, -SO₂R⁸ ou C₁-C₆ alcoxy, chacun des groupes R^{x} et R^{y} représentant indépendamment un atome H ou un groupe C₁-C₆ alkyle ; et R⁷ ne représente pas un atome H ou un groupe C(O)OR^{g}; ou éventuellement, un groupe R⁷ et un groupe R⁵ forment ensemble un groupe de liaison C₃-C₁₀ alkylène, C₂-C₁₀ hétéroalkylène, C₄-C₁₀ alcénylène, C₂-C₁₀ hétéroalcénylène, C₄-C₁₀ alcynylène ou C₂-C₁₀ hétéroalcynylène éventuellement substitué par un ou plusieurs des groupes halogéno, cyano, hydroxy ou C₁-C₆ alcoxy ;
chaque groupe R⁸ représente indépendamment un atome H ou un groupe C₁-C₆ alkyle ;
chaque groupe R⁹ représente indépendamment un groupe -Q³-T³, dans lequel Q³ représente une liaison ou un groupe C₁-C₆ alkylène, et T³ représente un atome H ;
chaque groupe R¹⁰ est choisi dans le groupe constitué par un atome H et un groupe C₁-C₆ alkyle ;
chaque groupe R¹¹ représente un groupe -Q⁶-T⁶, dans lequel Q⁶ représente une liaison ou un groupe de liaison C₁-C₆ alkylène, C₂-C₆ alcénylène ou C₂-C₆ alcynylène éventuellement substitué par un ou plusieurs des groupes halogéno, cyano, hydroxy, oxo ou C₁-C₆ alcoxy, et T⁶ représente un atome H, un groupe halogéno, OR^{g}, NR^{g}R^{h}, NR^{g}C(O)R^{h}, C(O)NR^{g}R^{h}, C(O)R^{g}, S(O)₂R^{g} ou R^{S3}, dans lesquelles chacun des groupes R^{g} et R^{h} représente indépendamment un atome H, un groupe phényle, C₃-C₈ cycloalkyle ou C₁-C₆ alkyle éventuellement substitué par un groupe C₃-C₈ cycloalkyle, ou R^{g} et R^{h} conjointement avec l'atome d'azote auquel ils sont liés forment un groupe hétérocycloalkyle de 4 à 12 chaînons contenant 1 à 4 hétéroatomes choisis parmi les atomes N, O et S, et R^{S3} représente un groupe C₃-C₈ cycloalkyle, C₆-C₁₀ aryle, hétérocycloalkyle de 4 à 12 chaînons contenant 1 à 4 hétéroatomes choisis parmi les atomes N, O et S, ou un groupe hétéroaryle de 5 à 10 chaînons, et R^{S3} est éventuellement substitué par un ou plusieurs groupes -Q⁷-T⁷, chaque Q⁷ représentant indépendamment une liaison ou un groupe de liaison C₁-C₃ alkylène, C₂-C₃ alcénylène ou C₂-C₃ alcynylène chacun éventuellement substitué par un ou plusieurs des groupes halogéno, cyano, hydroxy ou C₁-C₆ alcoxy, et chaque groupe T⁷ est indépendamment choisi dans le groupe constitué par un atome H, un groupe halogéno, cyano, C₁-C₆ alkyle, C₃-C₈ cycloalkyle, C₆-C₁₀ aryle, hétérocycloalkyle de 4 à 7 chaînons contenant 1 à 4 hétéroatomes choisis parmi les atomes N, O et S, et hétéroaryle de 5 à 6 chaînons, OR^{j}, C(O)R^{j}, NR^{j}R^{k}, C(O)NR^{j}R^{k}, S(O)₂R^{j} et NR^{j}C(O)R^{k}, chacun des groupes R^{j} et R^{k} représentant indépendamment un atome H ou un groupe C₁-C₆ alkyle éventuellement substitué par un ou plusieurs groupes halogéno ; ou -Q⁷-T⁷ représente un groupe oxo ; ou
R¹⁰ et R¹¹ pris conjointement avec l'atome d'azote auquel ils sont liés forment un groupe hétérocycloalkyle de 4 à 12 chaînons contenant 1 à 4 hétéroatomes choisis parmi les atomes N, O et S, qui est éventuellement substitué par un ou plusieurs des groupes halogéno, C₁-C₆ alkyle, hydroxy ou C₁-C₆ alcoxy ;
n vaut 0, 1, 2, 3 ou 4.

2. Composé destiné à être utilisé selon la revendication 1, R³ ne représentant pas un atome H.

3. Composé destiné à être utilisé selon l'une quelconque des revendications précédentes, n valant 1 ou 2 ; et/ou
R¹ représentant un atome H ou un groupe CH₃ ; et/ou
n valant 1 ou 2, et au moins l'un des groupes R⁷ représentant un groupe -Q²-OR¹¹ dans lequel R¹¹ représente un groupe -Q⁶-R^{S3} et Q⁶ représente éventuellement un groupe de liaison C₂-C₆ alkylène, C₂-C₆ alcénylène ou C₂-C₆ alcynylène substitué ; et/ou
n valant 1 ou 2, et au moins l'un des groupes R⁷ représentant un groupe -Q²-NR¹⁰R¹¹ dans lequel R¹¹ représente un groupe -Q⁶-R^{S3} ; et/ou
Q⁶ représentant un groupe de liaison C₂-C₆ alkylène, C₂-C₆ alcénylène ou C₂-C₆ alcynylène éventuellement substitué par un groupe hydroxy et R^{S3} représentant un groupe hétérocycloalkyle de 4 à 7 chaînons éventuellement substitué par un ou plusieurs groupes -Q⁷-T⁷ ; et/ou
Q⁶ représentant un groupe de liaison C₁-C₆ alkylène, C₂-C₆ alcénylène ou C₂-C₆ alcynylène éventuellement substitué par un groupe hydroxy et R^{S3} représentant un groupe C₃-C₆ cycloalkyle éventuellement substitué par un ou plusieurs groupes -Q⁷-T⁷ ; et/ou
chaque groupe Q⁷ représentant indépendamment une liaison ou un groupe de liaison C₁-C₃ alkylène, C₂-C₃ alcénylène ou C₂-C₃ alcynylène et chaque T⁷ représentant indépendamment un atome H, un groupe halogéno, C₁-C₆ alkyle ou phényle ; et/ou
Q² représentant une liaison ou un groupe de liaison C₁-C₄ alkylène, C₂-C₄ alcénylène ou C₂-C₄ alcynylène ; et/ou
au moins l'un des groupes R⁷ représentant

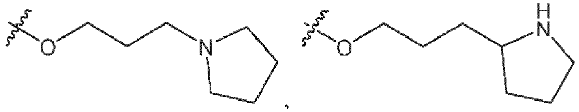

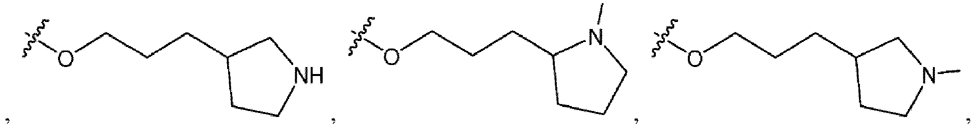

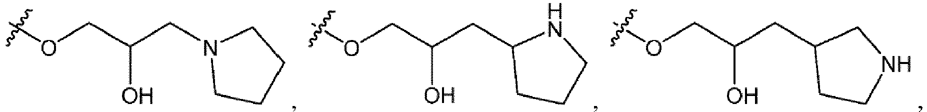

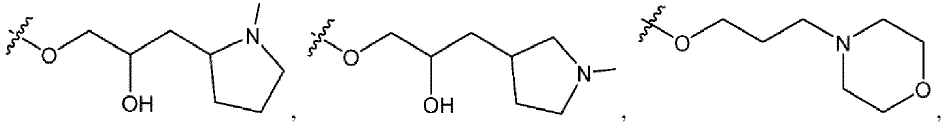

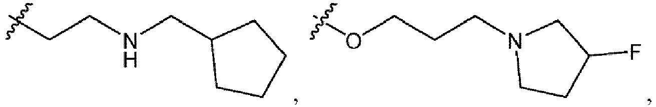

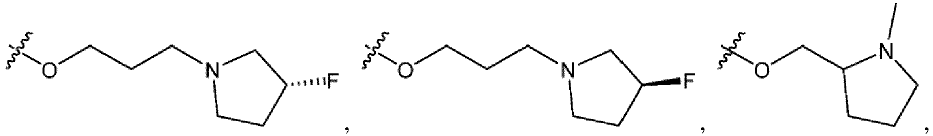

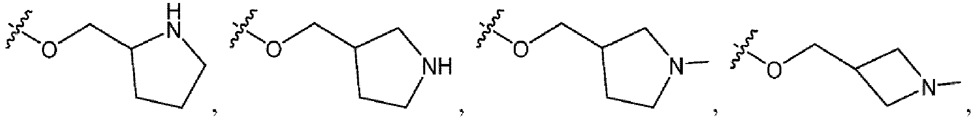

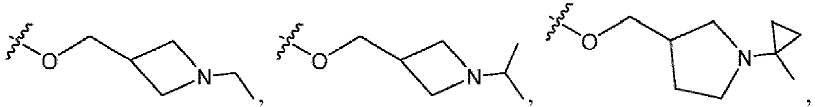

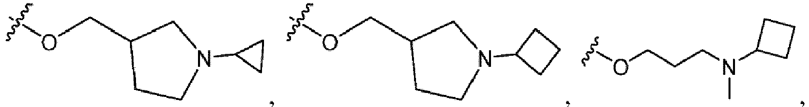

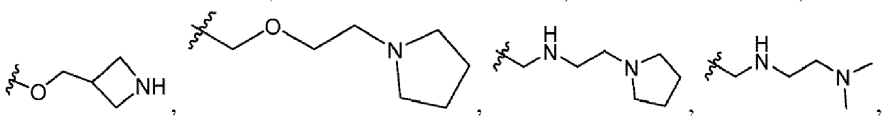

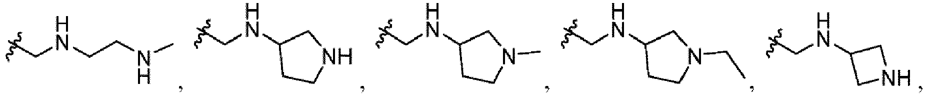

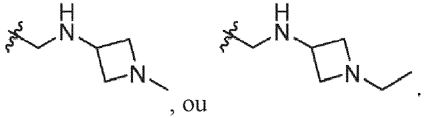

4. Composé destiné à être utilisé selon l'une quelconque des revendications précédentes, n valant 2 et ledit composé comprenant en outre un autre groupe R⁷ choisi parmi les groupes halogéno et méthoxy.

5. Composé destiné à être utilisé selon la revendication 1, étant de formule (VI) : 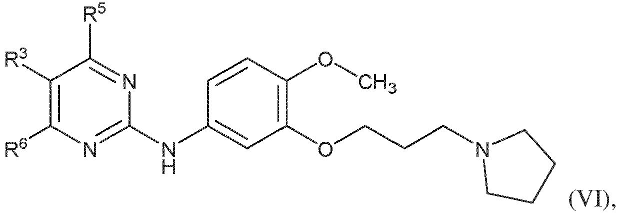 dans laquelle
R⁵ est choisi dans le groupe constitué par les groupes C₁-C₆ alkyle et NR⁸R⁹, et R⁶ représente un groupe NR⁸R⁹.

6. Composé destiné à être utilisé selon l'une quelconque des revendications précédentes, étant de formule (VIIIa) : 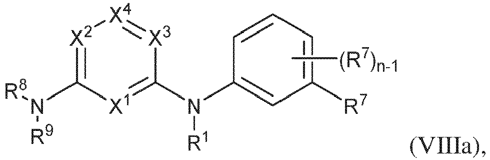 dans laquelle
X¹ représente un atome N ;
X² représente un groupe CR³ ;
X³ représente un atome N ;
X⁴ représente un groupe CR⁵ ;
R³ représente un atome H ; et
R⁵ est indépendamment choisi dans le groupe constitué par les groupes C₃-C₈ cycloalkyle et C₁-C₆ alkyle éventuellement substitués par un ou plusieurs groupes halogéno ou OR^{a} ; ou
R⁵ et R³ conjointement avec les atomes auxquels ils sont liés forment un groupe phényle ou un groupe hétéroaryle de 5 ou 6 chaînons.

7. Composé destiné à être utilisé selon l'une quelconque des revendications précédentes, étant de formule (VIIIb) : 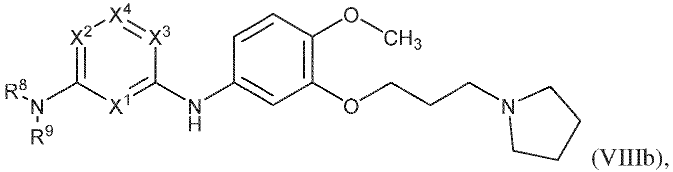 dans laquelle
X¹ représente un atome N ;
X² représente un groupe CR³ ;
X³ représente un atome N ;
X⁴ représente un groupe CR⁵ ;
R⁵ est choisi dans le groupe constitué par les groupes C₃-C₈ cycloalkyle et C₁-C₆ alkyle ; ou
R⁵ et R³ conjointement avec les atomes auxquels ils sont liés forment un groupe phényle ou un groupe hétéroaryle de 5 ou 6 chaînons.

8. Composé destine à être utilisé selon l'une quelconque des revendications précédentes, étant de formule (VIIIc) : 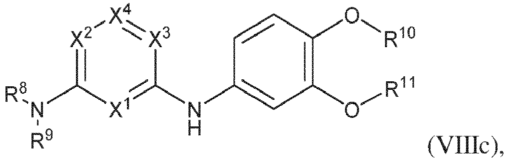 dans laquelle
X¹ représente un atome N ;
X² représente un groupe CR³ ;
X³ représente un atome N ;
X⁴ représente un groupe CR⁵ ;
R⁵ est choisi dans le groupe constitué par les groupes C₃-C₈ cycloalkyle et C₁-C₆ alkyle ; ou
R⁵ et R³ conjointement avec les atomes auxquels ils sont liés forment un groupe phényle ou un groupe hétéroaryle de 5 ou 6 chaînons.

9. Composé destiné à être utilisé selon l'une quelconque des revendications précédentes, R⁵ représentant un groupe C₁₋₆ alkyle ou R⁵ et R³ conjointement avec les atomes auxquels ils sont liés forment un groupe phényle ou un groupe hétéroaryle de 5 à 6 chaînons.

10. Composé destiné à être utilisé selon l'une quelconque des revendications précédentes, ledit composé étant choisi parmi les composés suivants et leurs sels pharmaceutiquement acceptables :
| **Composé n°** | **Structure** |
|---|---|
| 186 | 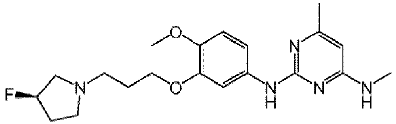 |
| 205 | 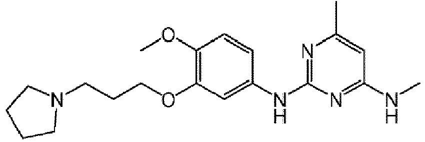 |
| 232 | 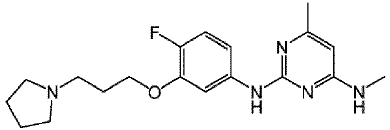 |
| 236 | 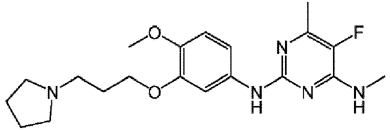 |
| 238 | 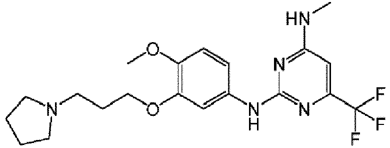 |
| 240 | 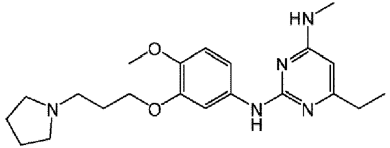 |
| 261 | 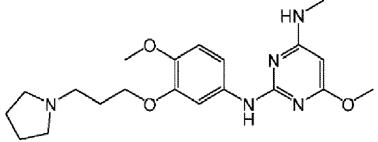 |
| 263 | 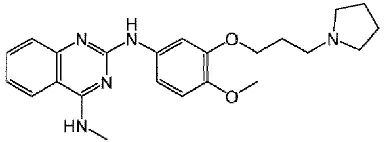 |
| 301 | 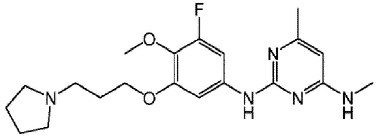 |
| 302 | 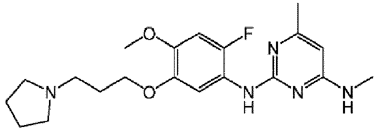 |
| 311 | 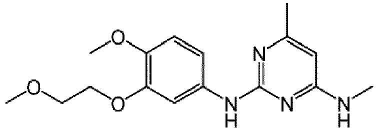 |
| 312 | 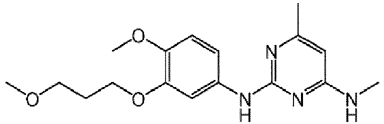 |
| 313 | 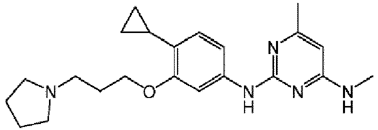 |
| 316 | 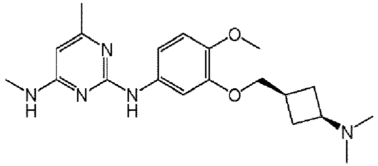 |
| 317 | 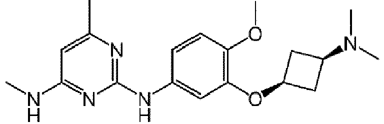 |
| 318 | 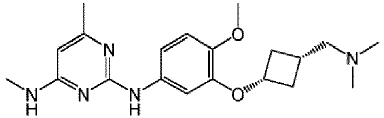 |
| 319 | 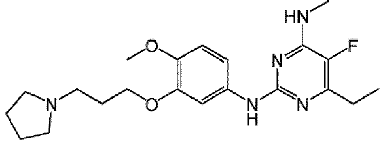 |
| 320 | 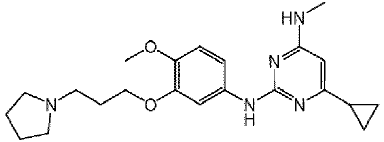 |
| 328 | 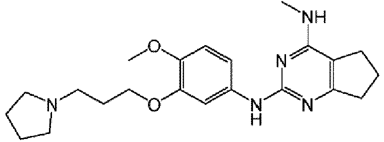 |
| 329 | 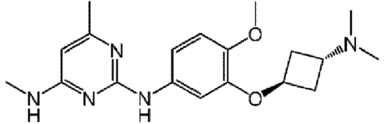 |
| 330 | 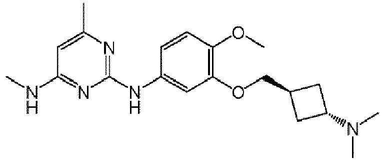 |
| 331 | 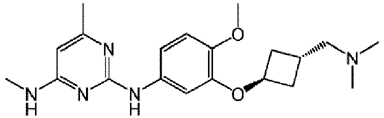 |
| 334 | 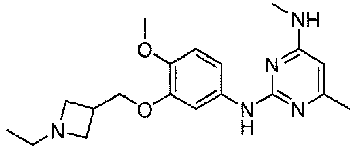 |
| 337 | 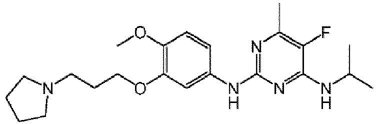 |
| 356 | 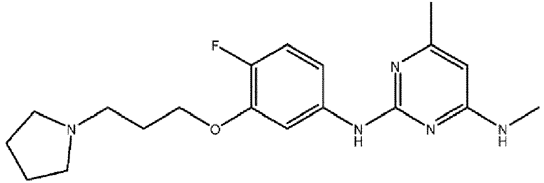 |
| 360 | 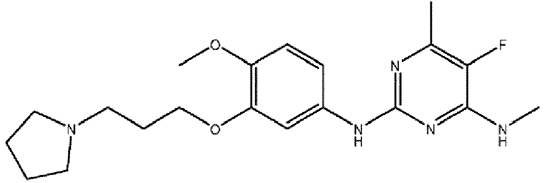 |
| 362 | 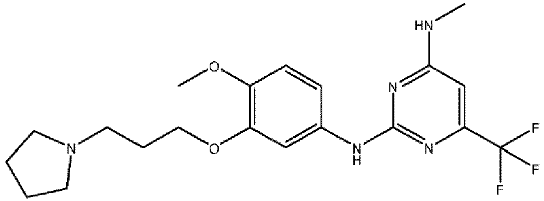 |
| 364 | 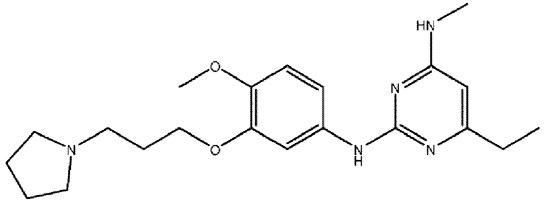 |
| 380 | 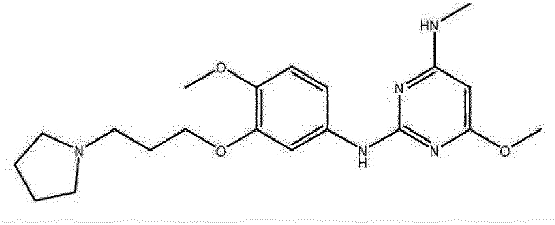 |
| 384 | 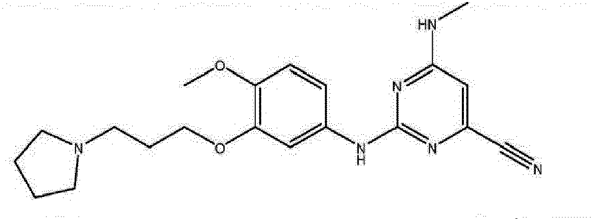 |
| 385 | 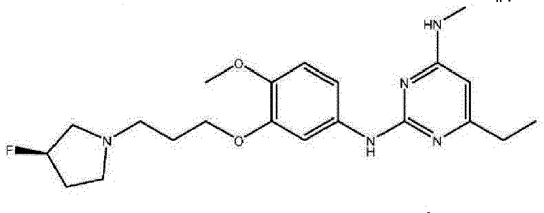 |
| 391 | 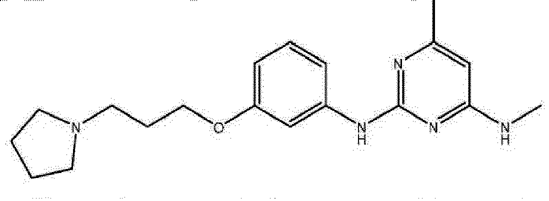 |
| 413 | 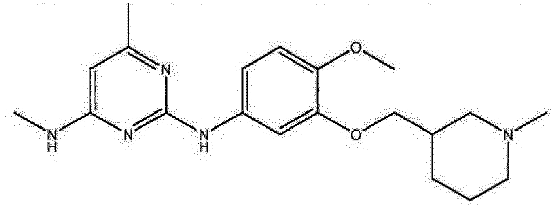 |
| 417 | 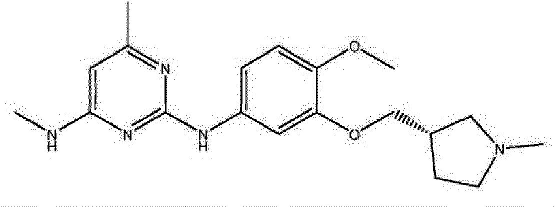 |
| 418 | 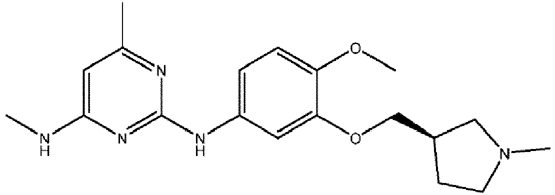 |
| 419 | 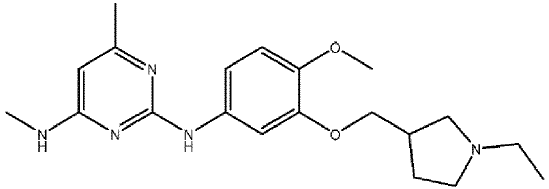 |
| 421 | 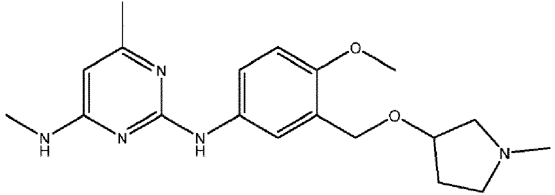 |
| 428 | 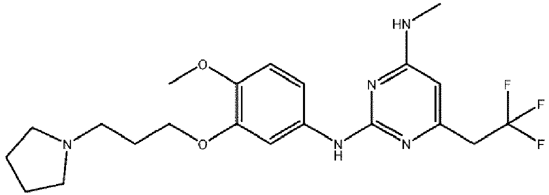 |
| 432 | 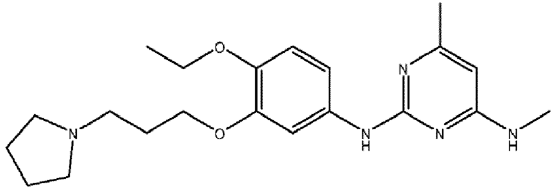 |
| 435 | 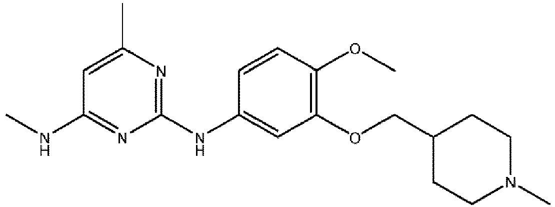 |
| 436 | 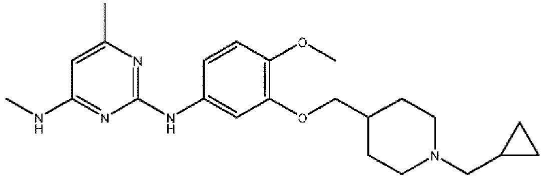 |
| 437 | 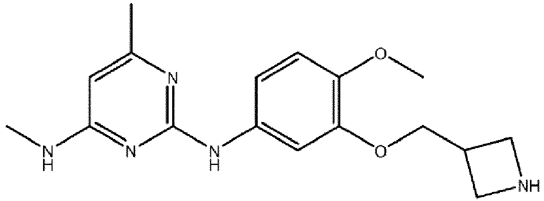 |
| 441 | 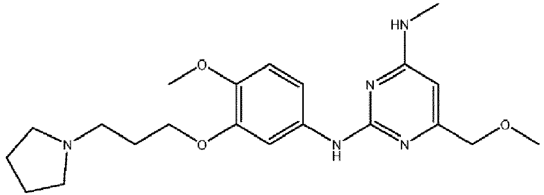 |
| 442 | 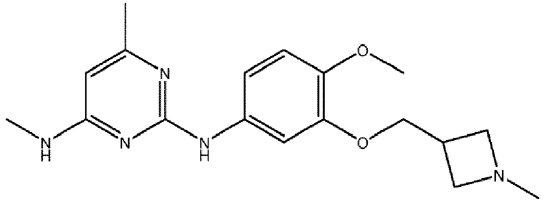 |
| 443 | 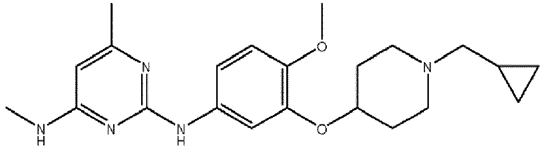 |
| 444 | 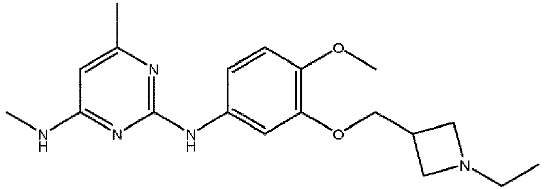 |
| 445 | 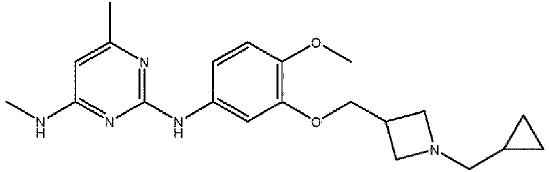 |
| 446 | 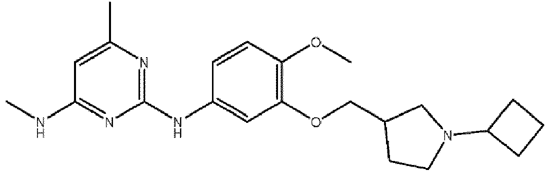 |
| 449 | 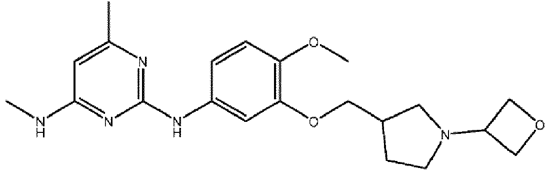 |
| 450 | 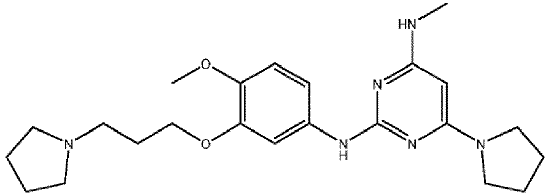 |
| 452 | 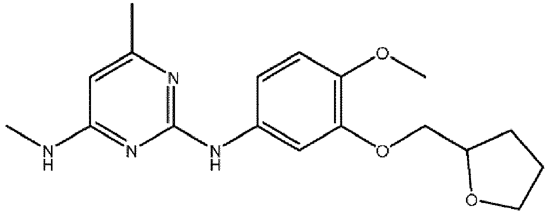 |
| 455 | 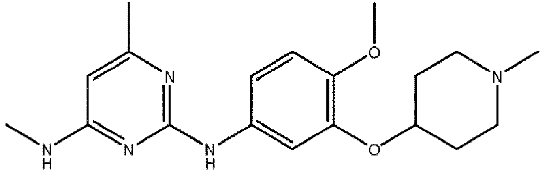 |
| 456 | 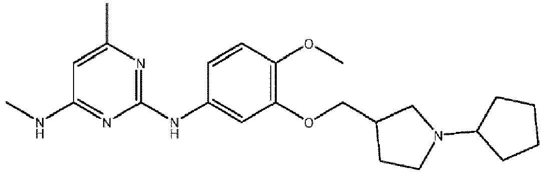 |
| 457 | 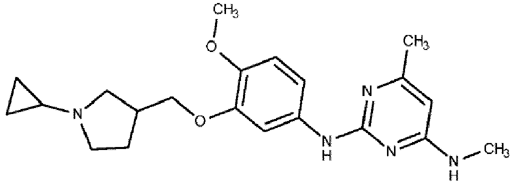 |
| 458 | 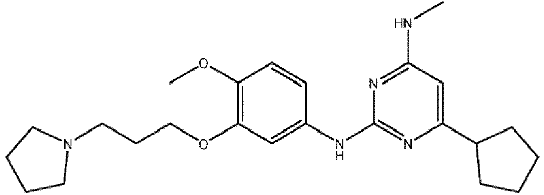 |
| 459 | 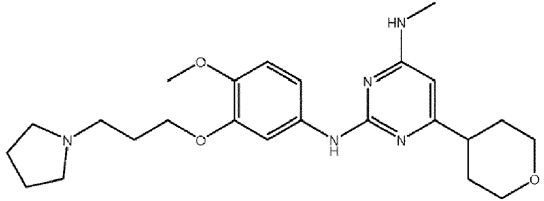 |
| 461 | 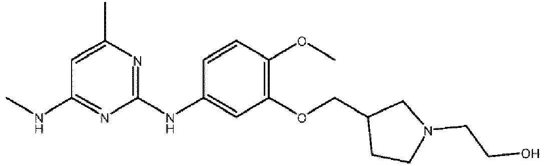 |
| 463 | 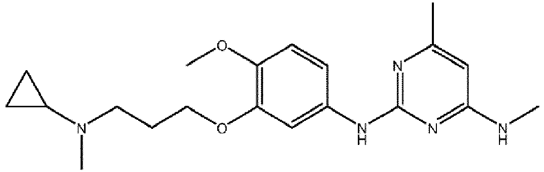 |
| 465 | 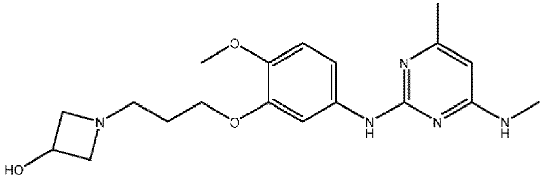 |
| 466 | 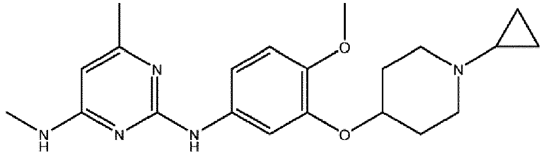 |
| 468 | 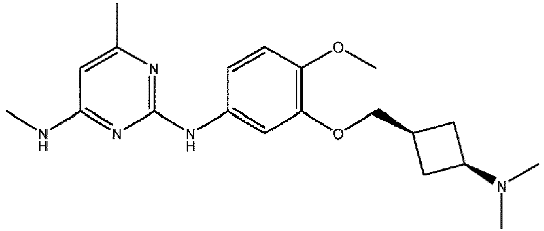 |
| 470 | 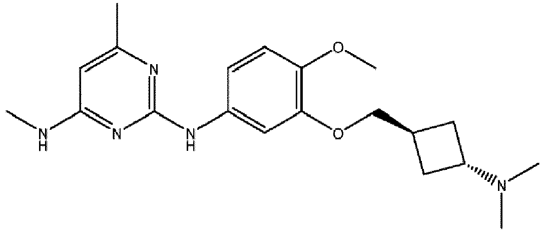 |
| 481 | 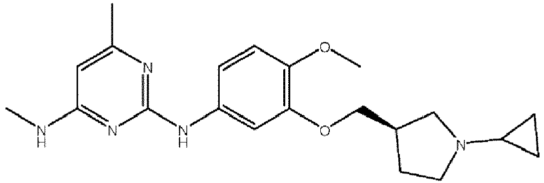 |
| 482 | 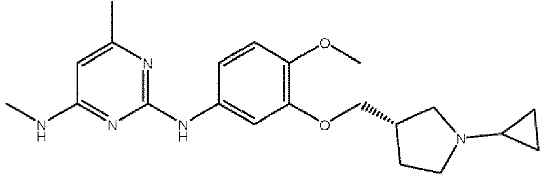 |
| 483 | 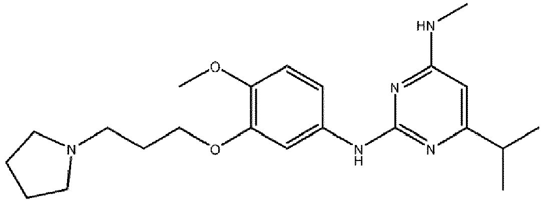 |
| 484 | 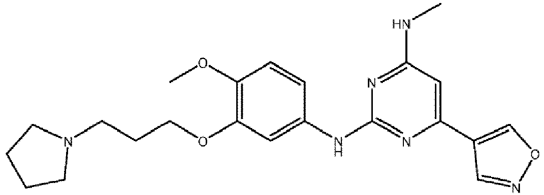 |
| 487 | 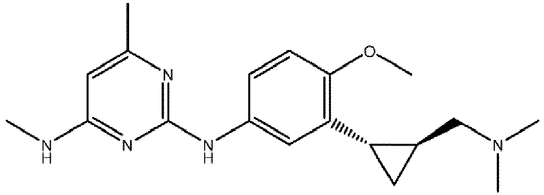 |
| 488 | 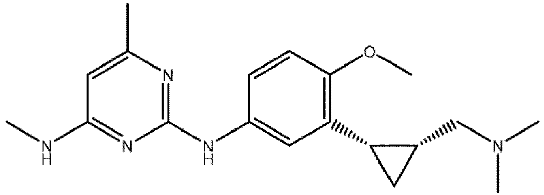 |
| 489 | 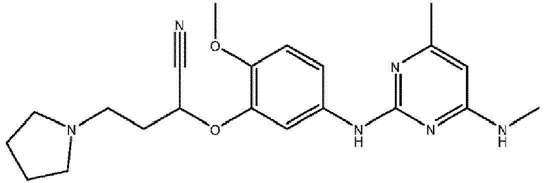 |
| 490 | 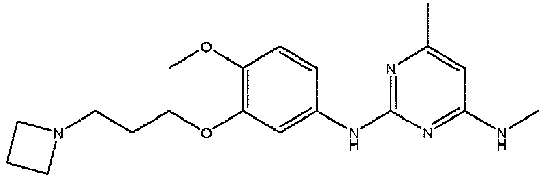 |
| 491 | 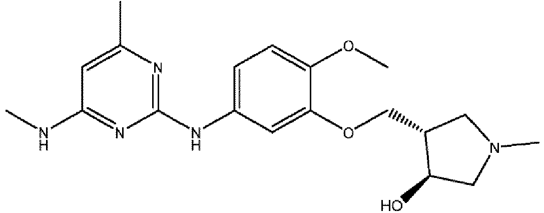 |
| 492 | 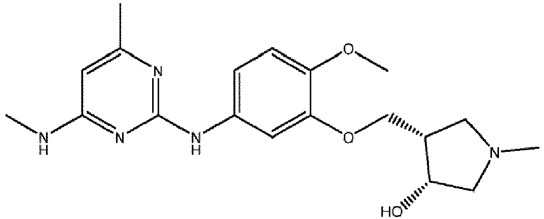 |
| 493 | 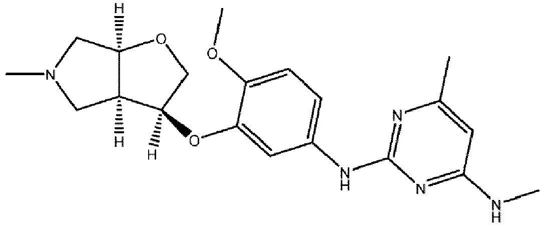 |
| 496 | 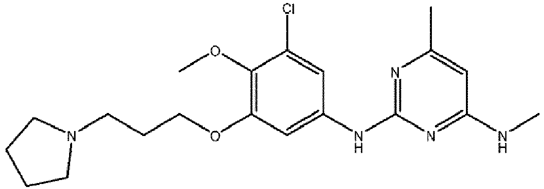 |
| 497 | 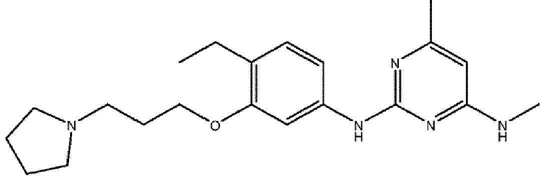 |
| 498 | 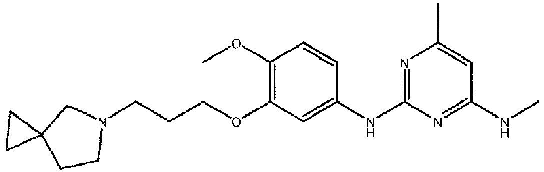 |
| 502 | 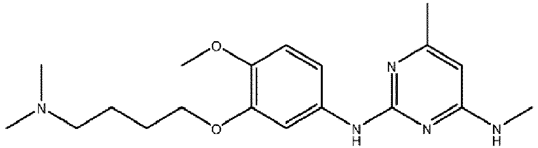 |
| 503 | 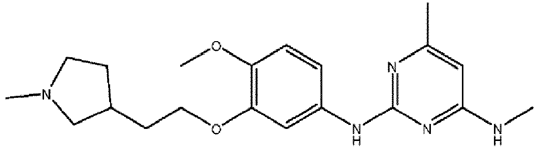 |
| 504 | 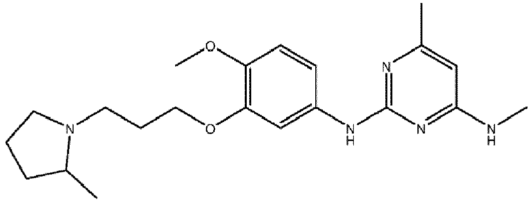 |
| 505 | 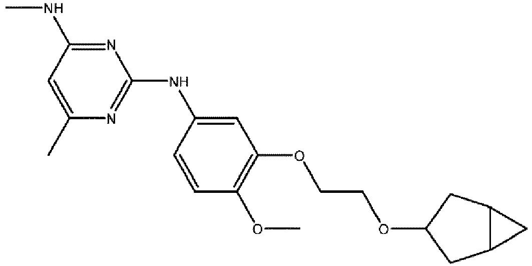 |
| 506 | 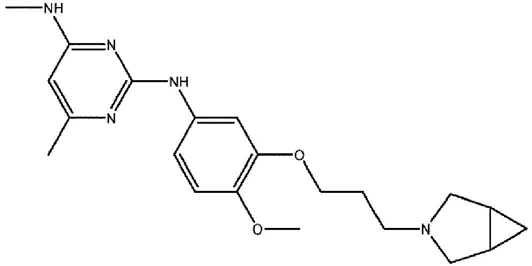 |
| 508 | 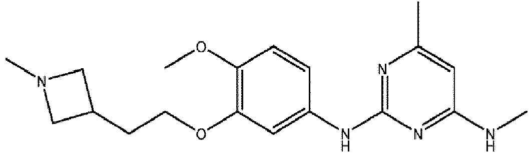 |
| 510 | 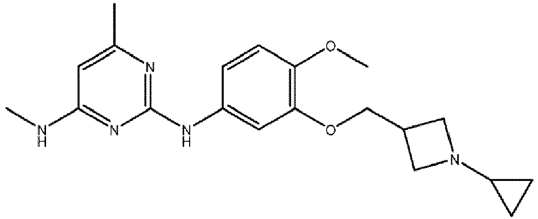 |
| 517a | 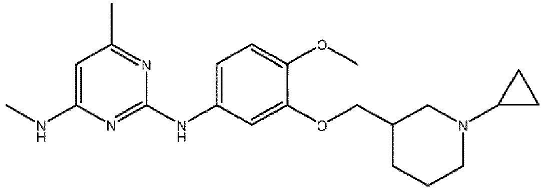 |
| 517b | 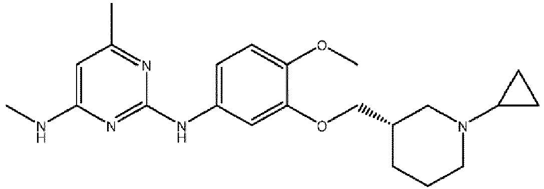 |
| 551 | 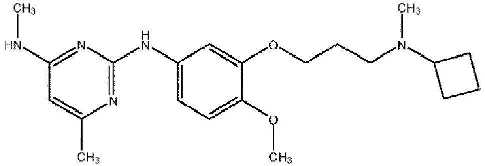 |
| 559 | 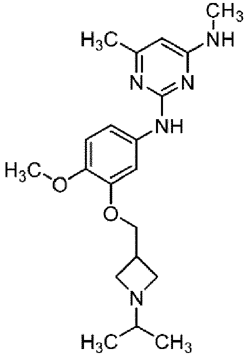 |
| 560 | 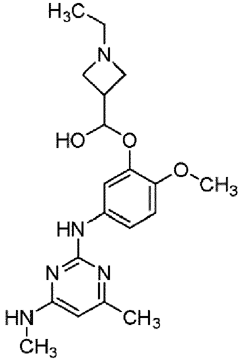 |
| 561 | 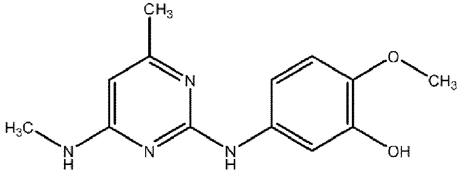 |
| 562 | 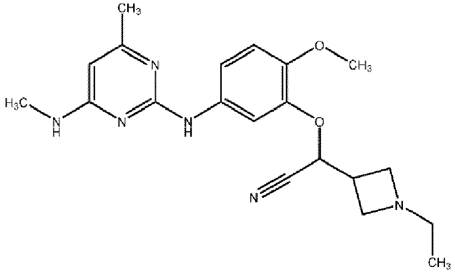 |
| 563 | 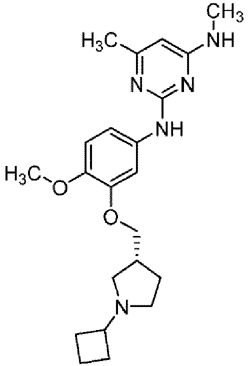 |
| 568 | 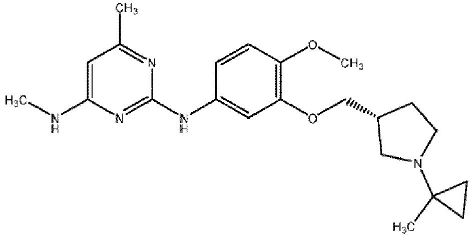 |
| 570 | 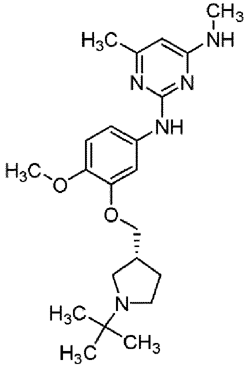 |
| 571 | 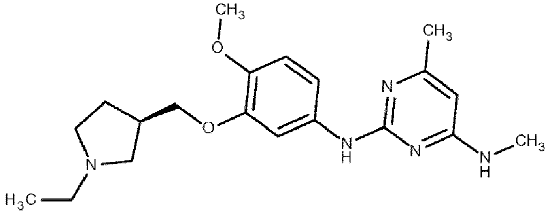 |
| 574 | 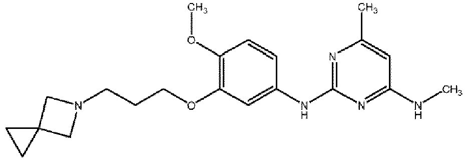 |
| 575 | 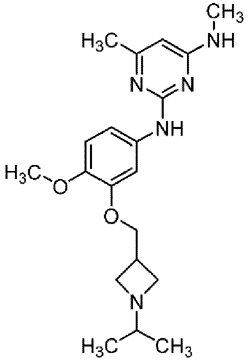 |
| 576 | 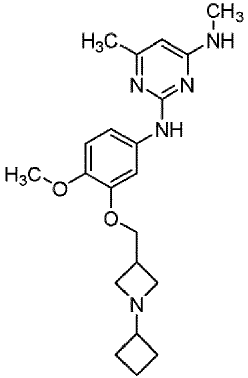 |
| 577 | 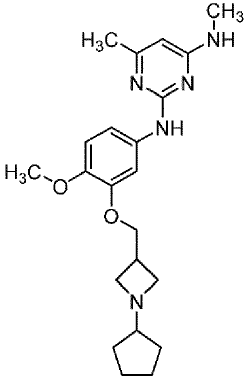 |
| 580 | 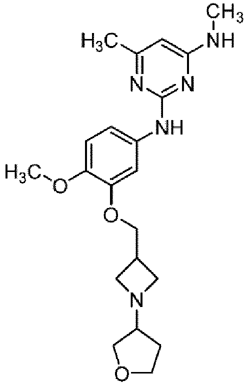 |
| 600 | 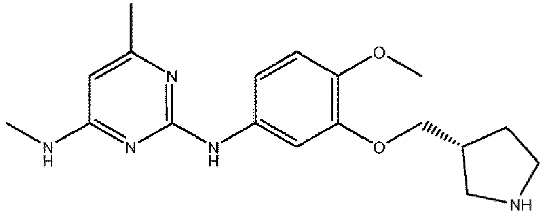 |
| 602 | 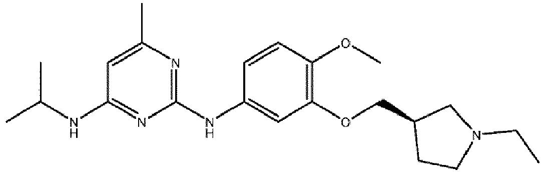 |
| 603 | 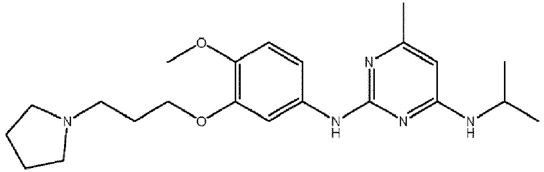 |
| 606 | 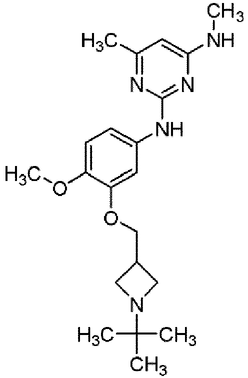 |
| 610 | 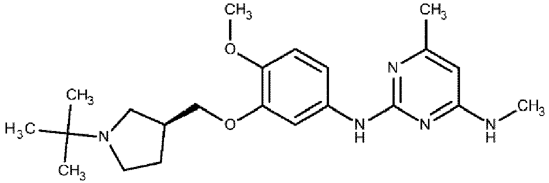 |
| 614 | 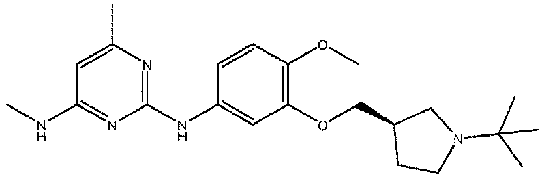 |
| 624 | 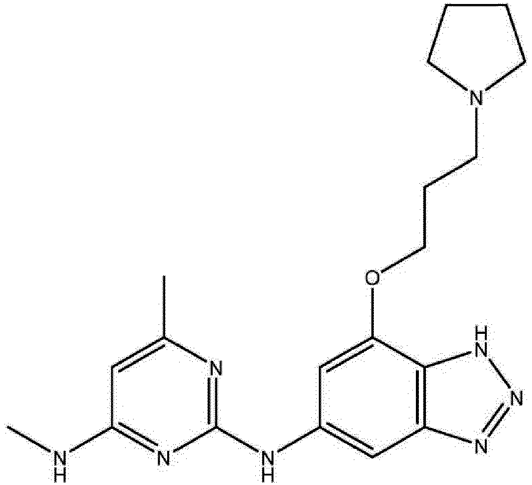 |
| 644 | 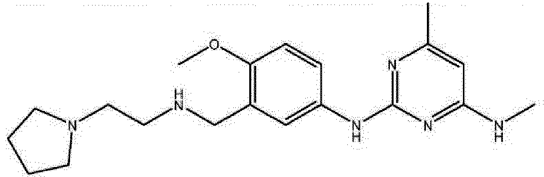 |
| 645 | 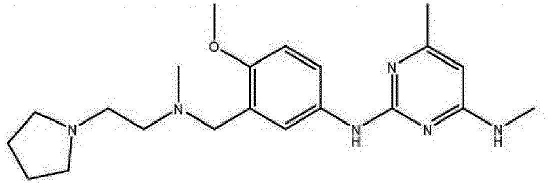 |
| 646 | 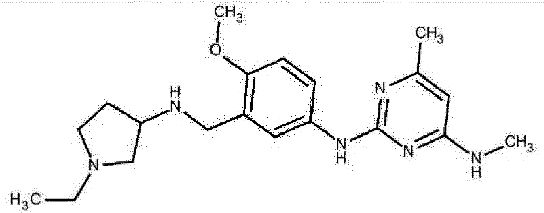 |
| 647 | 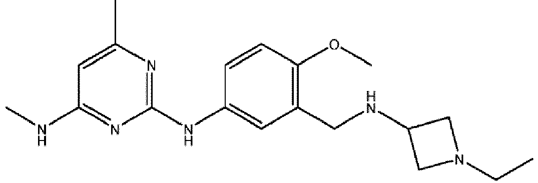 |
| 649 | 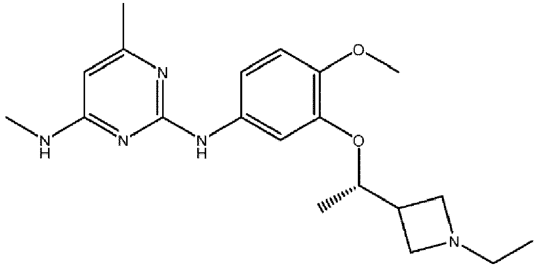 |
| 650 | 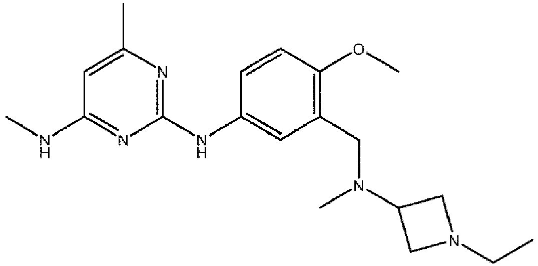 |
| 651 | 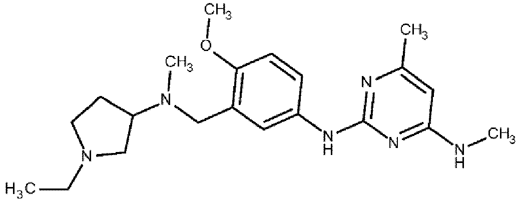 |
| 671 | 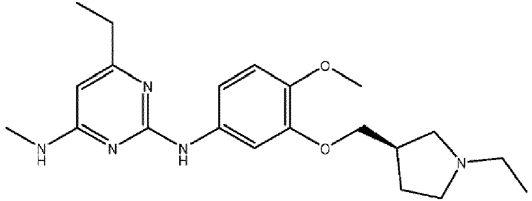 |
| 672 | 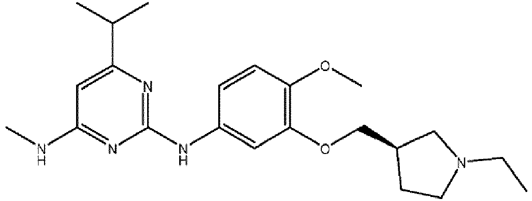 |
| 680 | 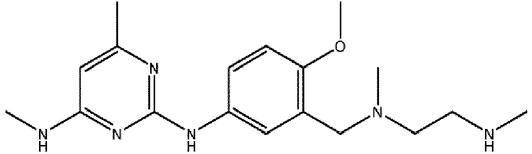 |
| 687 | 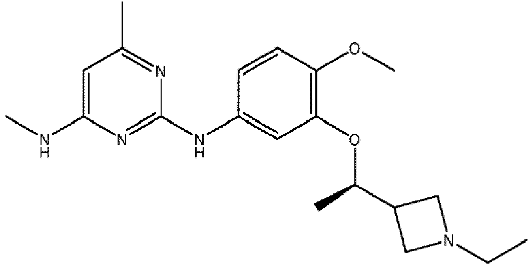 |
| 765 | 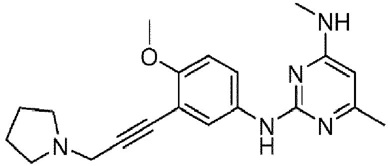 |
| 786 | 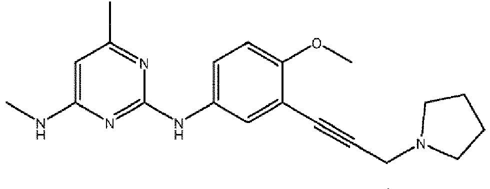 |
| 791 | 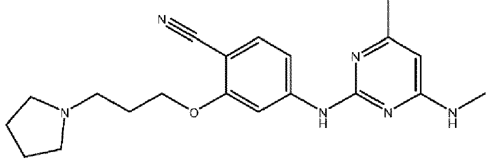 |
| 793 | 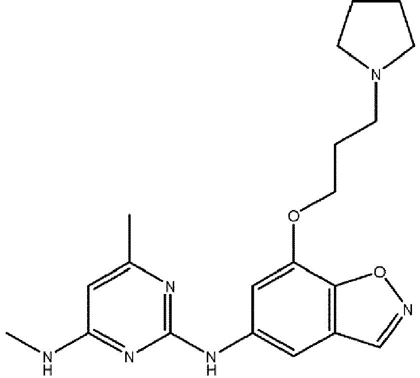 |
| 799 | 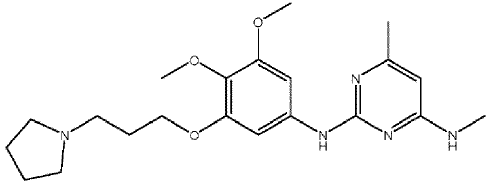 |
| 800 | 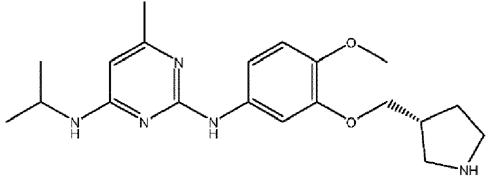 |
| 813 | 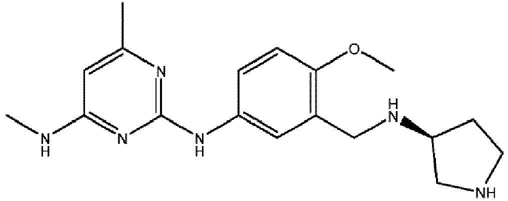 |
| 814 | 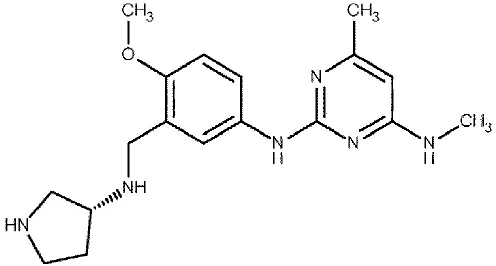 |
| 845 | 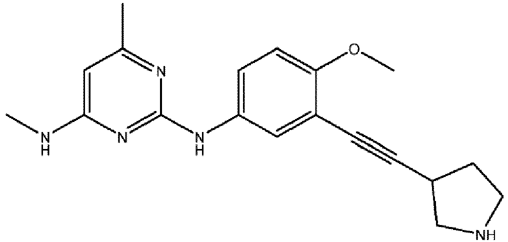 |
| 846 | 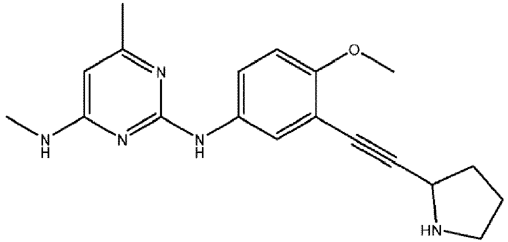 |
| 847 | 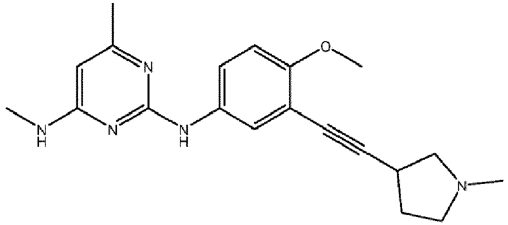 |
| 848 | 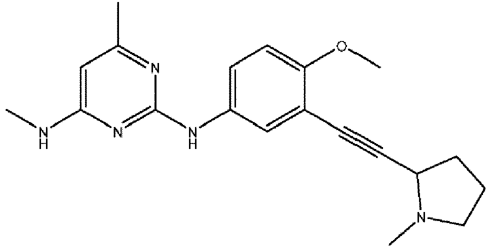 |
| 855 | 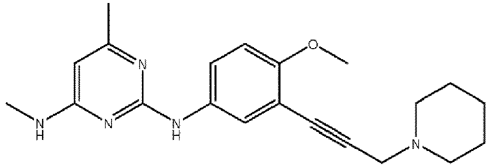 |
| 860 | 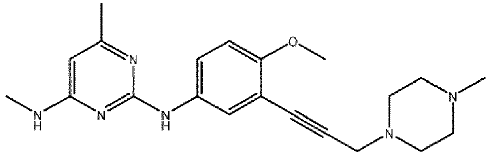 |
| 871 | 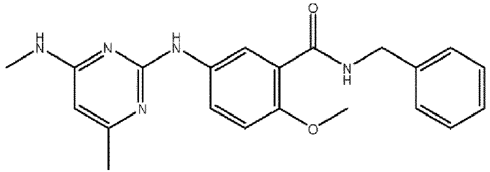 |
| 872 | 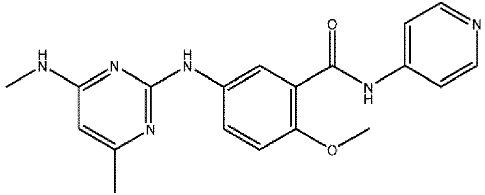 |
| 873 | 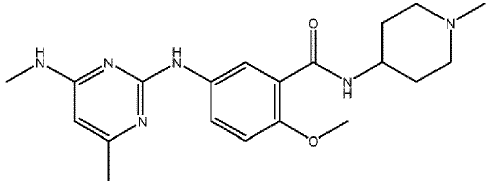 |
| 874 | 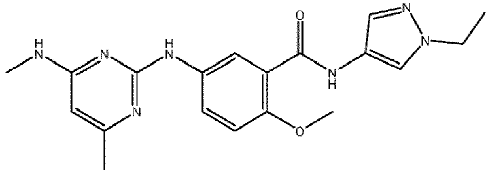 |
| 876 | 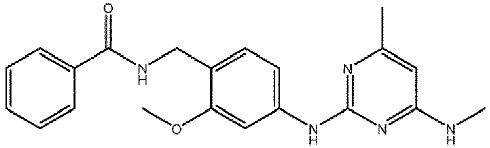 |
| 877 | 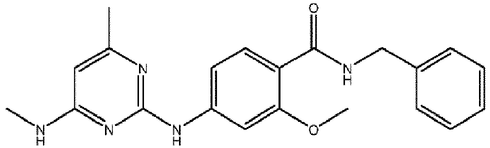 |
| 878 | 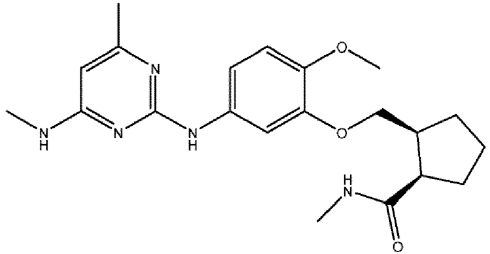 |
| 879 | 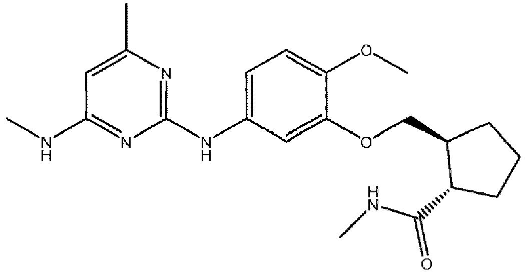 |
| 890 | 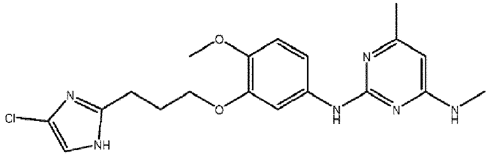 |
| 891 | 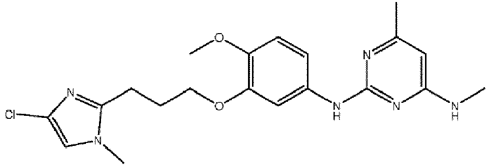 |
| 904 | 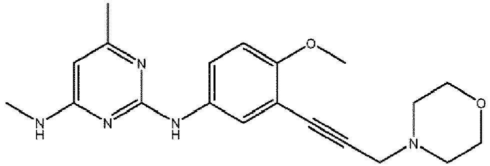 |
| 905 | 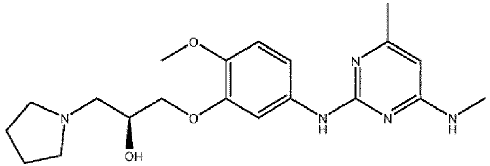 |
| 906 | 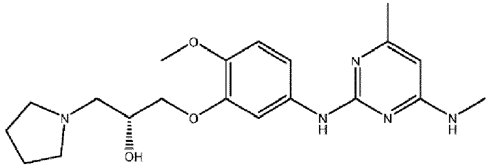 |
| 962 | 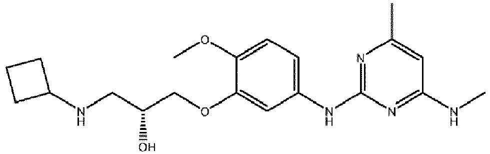 |
| 964 | 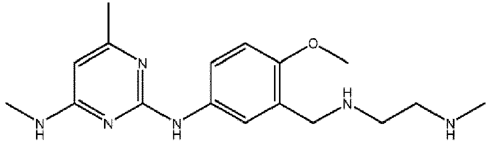 |
| 965 | 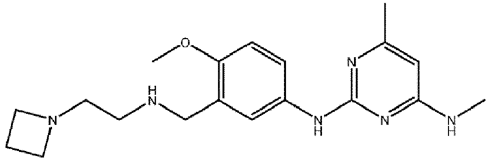 |
| 966 | 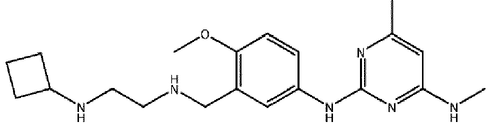 |
| 967 | 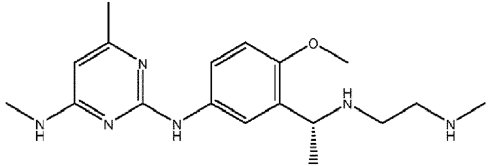 |
| 968 | 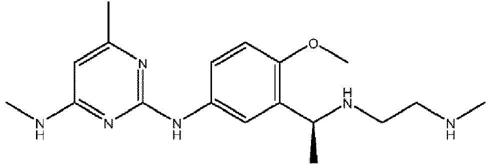 |
| 969 | 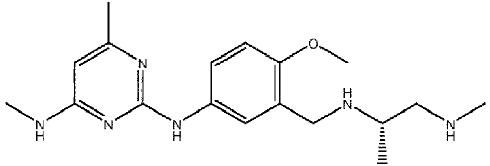 |
| 970 | 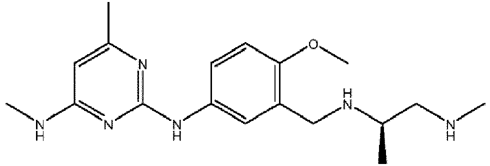 |
| 971 | 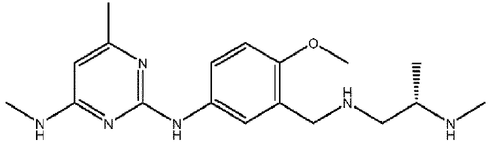 |
| 972 | 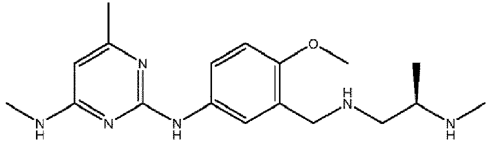 |
| 977 | 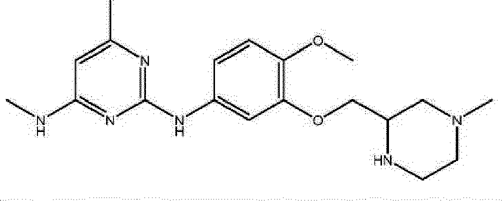 |
| 985 | 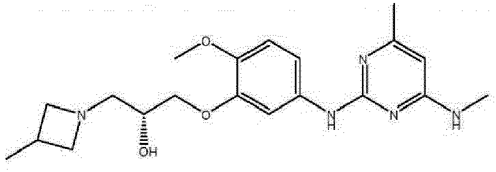 |
| 986 | 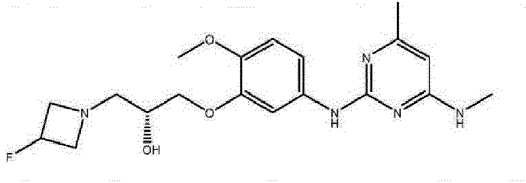 |
| 989 | 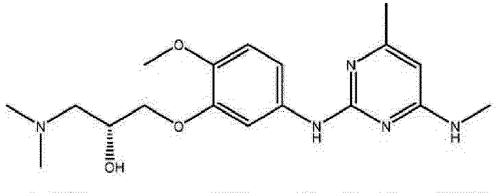 |
| 990 | 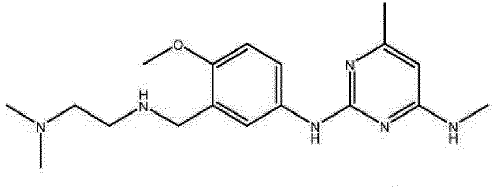 |
| 991 | 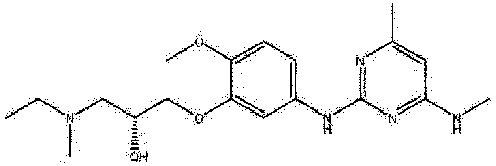 |
| 992 | 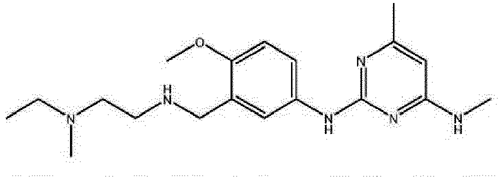 |
| 993 | 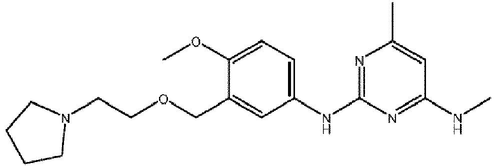 |
| 994 | 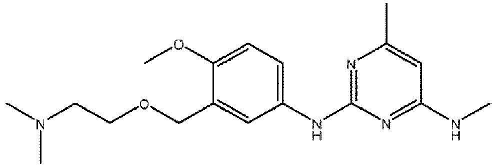 |
| 997 | 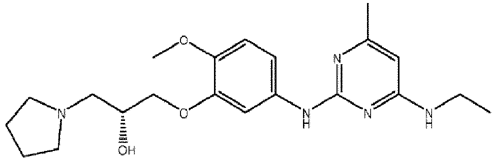 |
| 998 | 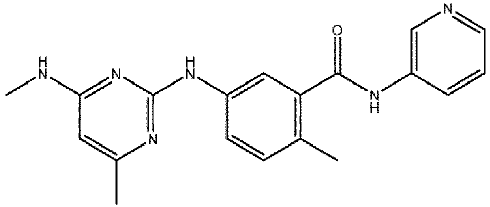 |
| 999 | 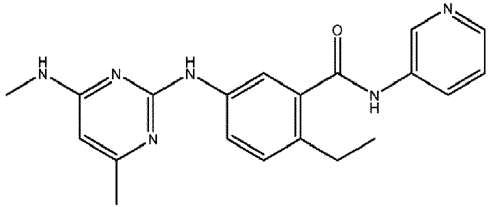 |
| 1000 | 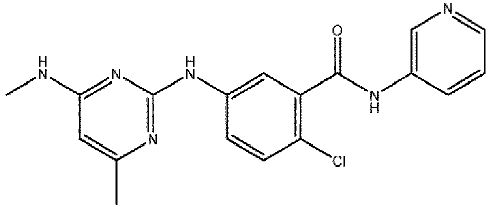 |
| 1001 | 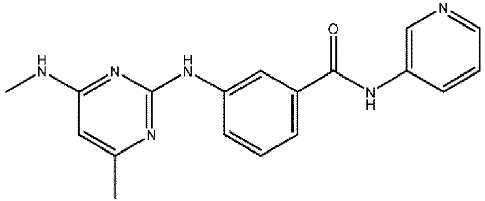 |
| 1004 | 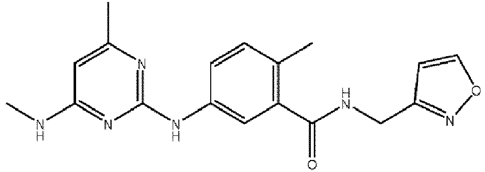 |
| 1029 | 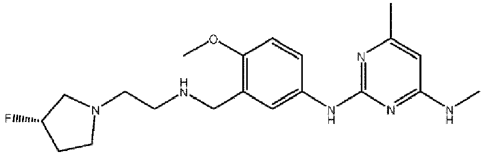 |
| 1030 | 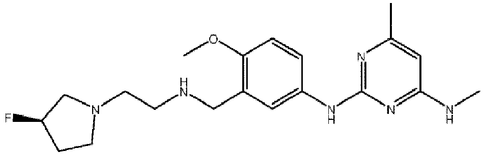 |
| 1036 | 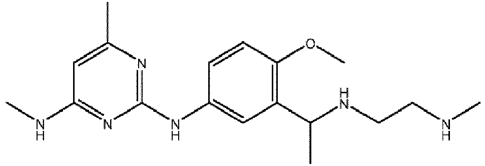 |
| 1037 | 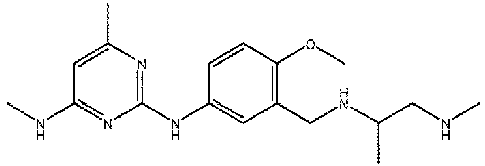 |
| 1038 | 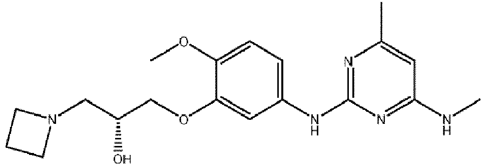 |
| 1040 | 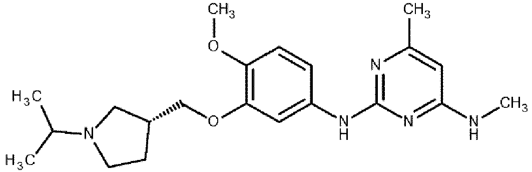 |
| 1044 | 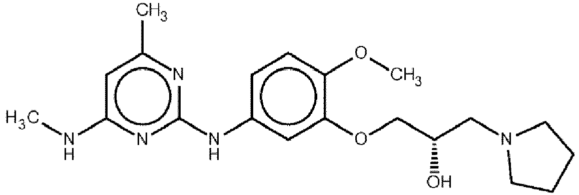 |
| 1045 | 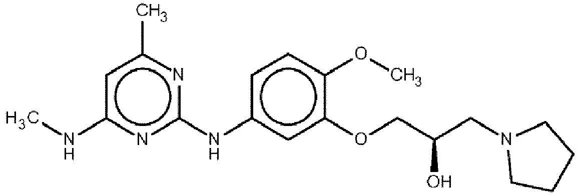 |
| 1078 | 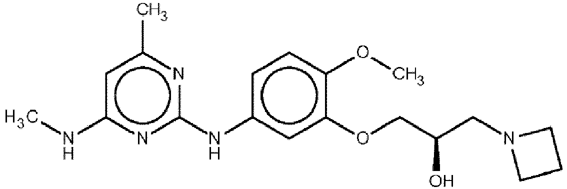 |
| 1079 | 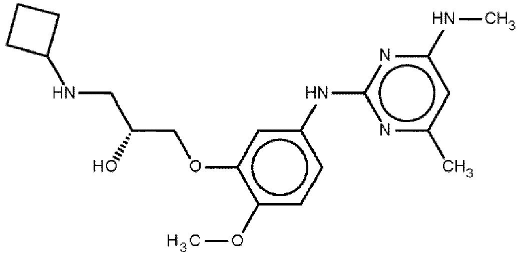 |
| 1080 | 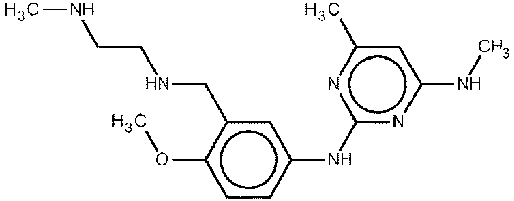 |
| 1081 | 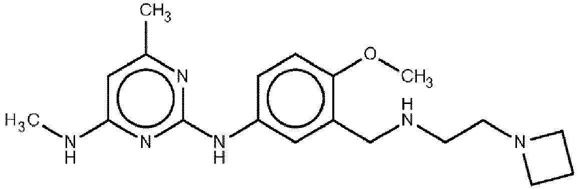 |
| 1090 | 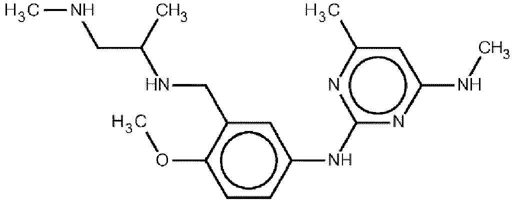 |
| 1094 | 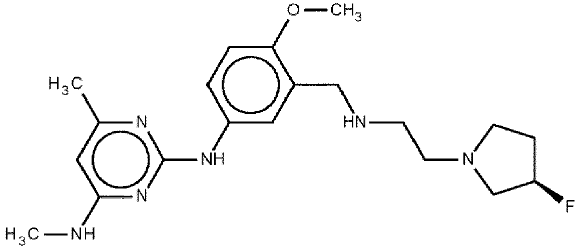 |
| 1095 | 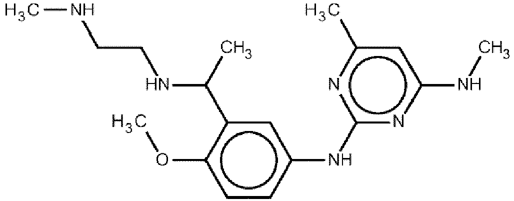 |
| 1099 | 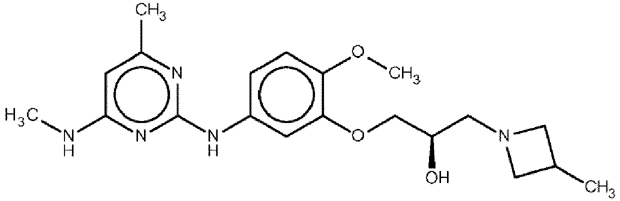 |
| 1100 | 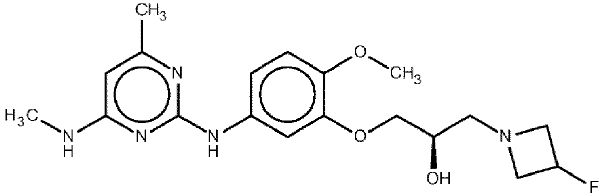 |
| 1101 | 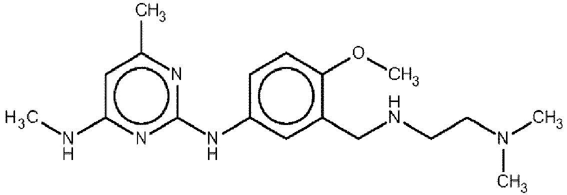 |
| 1102 | 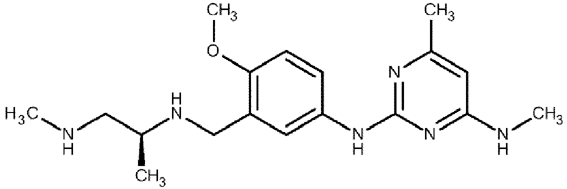 |
| 1103 | 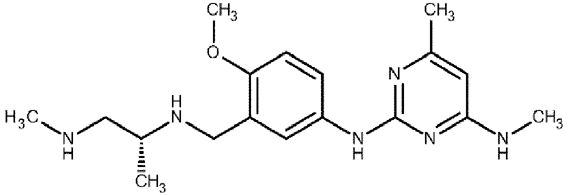 |
| 1104 | 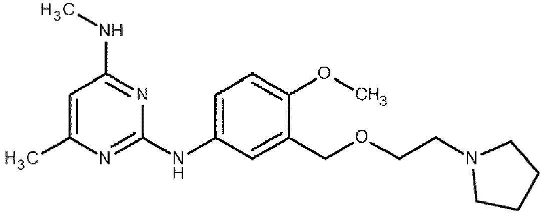 |
| 1105 | 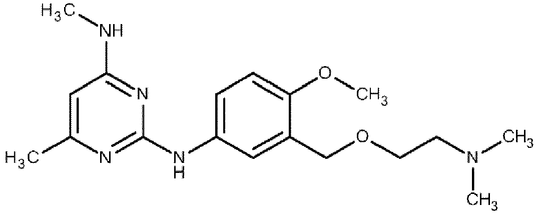 |
| 1106 | 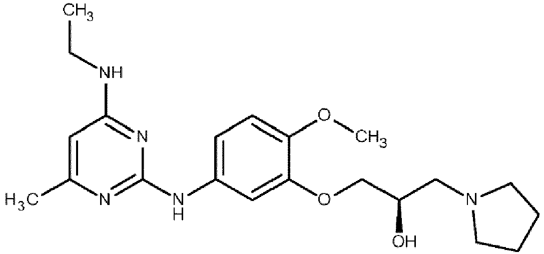 |
| 1109 | 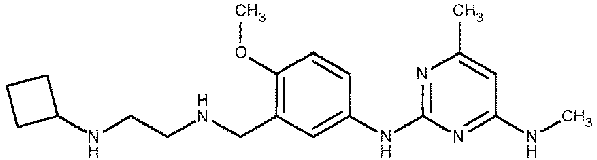 |
| 1112 | 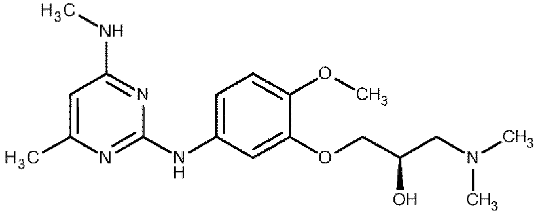 |
| 1113 | 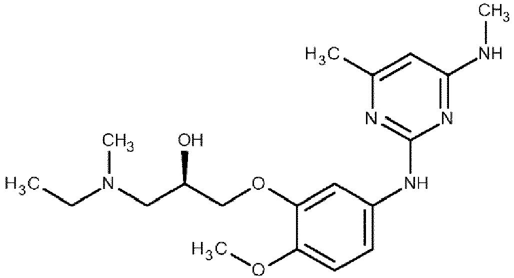 |
| 1114 | 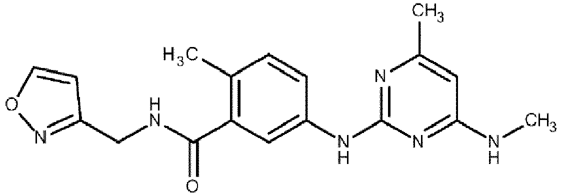 |
| 1117 | 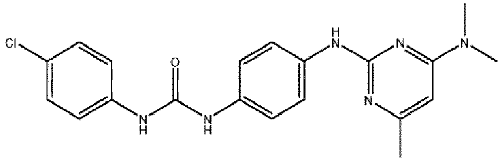 |

11. Composé destiné à être utilisé tel que défini dans l'une quelconque des revendications 1 à 10, ledit trouble sanguin étant une drépanocytose ou une β-thalassémie.

12. Composé destiné à être utilisé tel que défini dans l'une quelconque des revendications 1 à 10, ledit trouble sanguin étant un cancer hématologique.

13. Composé destiné à être utilisé tel que défini dans la revendication 12, ledit cancer hématologique étant une leucémie myéloïde aiguë (LMA) ou une leucémie lymphocytaire chronique (LLC).

14. Composé destiné à être utilisé tel que défini dans l'une quelconque des revendications précédentes, ledit composé étant un inhibiteur sélectif d'EHMT2.
